(19) Europäisches Patentamt
European Patent Office
Office européen des brevets

(11) EP 4 431 116 A1

(12) EUROPEAN PATENT APPLICATION
published in accordance with Art. 153(4) EPC

(43) Date of publication:
18.09.2024 Bulletin 2024/38

(21) Application number: 22889485.3

(22) Date of filing: 08.11.2022

(51) International Patent Classification (IPC):
*A61K 47/64* (2017.01) *A61K 47/65* (2017.01)
*A61K 47/54* (2017.01) *A61K 45/00* (2006.01)
*A61P 35/00* (2006.01) *A61P 35/02* (2006.01)
*C08G 69/48* (2006.01)

(52) Cooperative Patent Classification (CPC):
A61K 47/645; A61P 35/00; A61P 35/02;
C08G 69/10; C08G 69/48

(86) International application number:
PCT/CN2022/130704

(87) International publication number:
WO 2023/078464 (11.05.2023 Gazette 2023/19)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
KH MA MD TN

(30) Priority: 08.11.2021 CN 202111323754

(71) Applicant: Shanghai Best-Link Bioscience, LLC
Shanghai 201203 (CN)

(72) Inventors:
- ZHANG, Fuyao
  Shanghai 201203 (CN)
- WAN, Jiaxun
  Shanghai 201203 (CN)
- HUANG, Lei
  Shanghai 201203 (CN)

(74) Representative: Maiwald GmbH
Elisenhof
Elisenstraße 3
80335 München (DE)

# (54) EPSILON-POLY-L-LYSINE-BASED DRUG CONJUGATE, INTERMEDIATE THEREOF, AND APPLICATION THEREOF

(57) Disclosed are an epsilon-poly-L-lysine-based drug conjugate, an intermediate thereof, and an application thereof. The present invention provides an epsilon-poly-L-lysine derivative-drug conjugate that has a controllable group coupling number and controllable group coupling sites and that is represented by formula (I). The drug conjugate of the present invention has one or more among the effect advantages of low renal clearance rate, low liver and spleen clearance rate, long plasma half-life, rapid accumulation of effective drug concentration in lesions, and a better therapeutic effect.

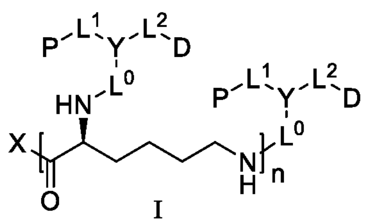

I

EP 4 431 116 A1

## Description

This application claims priority to Chinese Application No. 2021113237547, filed on Nov. 8, 2021, the entire disclosure of which is incorporated herein by reference.

TECHNICAL FIELD

The present invention relates to a poly(ε-L-lysine)-based drug conjugate, its intermediates and applications thereof.

BACKGROUND

Chemotherapy drugs usually have a few disadvantages, such as non-targeted organ toxicity and low tumor-specific accumulation. Therefore, a series of special delivery forms have been developed for chemotherapy drugs, such as self-assembled drug delivery systems, including liposomes, micelles, vesicles and microspheres; drug conjugation systems, including small molecule prodrugs, polymer drug conjugates, antibody drug conjugates, etc. Among them, the following forms of polymer drug conjugates have been reported:

a) PEG block co-polymer-drug conjugates, e.g., NC-6004 (PEG-b-poly(glutamic acid)-cisplatin) (British Journal of Cancer, 2011, 104, 593); K-912/NC-6300 (PEG-b-poly(aspartic acid)-epirubicin) (Investigational New Drugs, 2017, 35, 307);
b) core-cross-linked polymers, e.g., CriPec docetaxel (copolymeric micelles of docetaxel and PEG block polymers containing methacrylic acid groups ) (Biomaterials, 2015, 53, 370);
c) 4-arm-PEG-drug conjugates, e.g., NKTR102 (4-arm-PEG-Irinotecan conjugate) (Polymers, 2014, 6, 2186; Lancet Oncology, 2015, 16, 1556; Mol Cancer Ther, 2018, 17, 196); DFP-13318 (4-arm-PEG-SN-38 conjugate) (Journal of Medicinal Chemistry, 2014, 57, 2303);
d) drugs with a linear polymer as a backbone and pendant active functional groups for random conjugation, e.g., Opaxio (Polyglutamic acid-paclitaxel conjugate) (British Journal of Cancer, 2008, 98, 1608);
e) drugs and pharmacokinetic regulators with a linear polymer as a backbone and pendant active functional groups for random conjugation, e.g., OsteoDex (dextran-grafted alendronic acid) (Int J Oncol, 2010, 37, 563; Anticancer Research, 2016, 36, 6499);
f) CRLX101 (cyclodextrin PEGylated copolymer, pendant conjugated CPT) (Clinical Cancer Research, 2015, 21, 808; J Control Release, 2011, 153, 49); CRLX301 (cyclodextrin-PEGylated copolymer, pendant conjugated docetaxel) (Journal of Clinical Oncology, 2016, 34(15_suppl), 2526);
g) hyperbranched polymer-drug conjugates or dendrimer-drug conjugates, e.g., Starpharma's dendritic polylysine drug conjugates (Mol Pharm, 2018, 15, 4568).

The structure of poly(ε-L-lysine) hydrochloride is $[C_6H_{12}N_2O \cdot HCl]n \cdot H_2O$. It has been approved as a biological preservative by the Japanese Ministry of Health, the US FDA, South Korea, China and other countries and organizations. Currently, different forms of poly(ε-L-lysine) based drug delivery include: block copolymers (WO2019039544) and grafted copolymers (Mol Pharm, 2015, 12, 4226; Adv Mater, 2016, 28, 8162; CN109265680A). Among them, the block copolymer form only utilizes terminal functional groups of poly(ε-L-lysine), which has low degree of modification and tunability. The poly(ε-L-lysine)-grafted copolymer form obtained by random grafting has uncontrollable distribution of grafted functional groups on the polymer backbone, resulting in interchain heterogeneity of the polymer. These resulting grafted copolymers with different degrees of modification and positions of modification have different pharmacokinetics and pharmacodynamics in vivo. Therefore, for the existing poly(ε-L-lysine) random modification method, in addition to molecular weight, molecular weight distribution and average modification degree of a certain molecule, the quality standard also should involve investigatation of the modification distribution of the conjugated molecules between different batches, so the relevant quality standards are difficult to establish.

According to the published literature (Mol Pharm, 2018, 15, 4568), the molecular size of dendrimer-drug conjugates is strongly correlated with drug uptake, retention, release and clearance in tumors. When the size of dendrimer is small, the drug is released fast, but it is easily cleared through the kidneys. When the size of dendrimer is increased by increasing the dendrimer generation, steric hindrance of the outer layer increases, which is unfavorable for enzymatic cleavage of the linker to release drugs. Therefore, for dendrimer-drug conjugates, it is difficult to balance the relationship between clearance, retention and drug release, and it is difficult to achieve the optimal therapeutic effect.

For example, the polylysine dendrimer-drug conjugate developed by Starpharma, due to divergent synthesis of polylysine dendrimers, has high functional group density on its surface and large steric hindrance between the drug and the polylysine dendrimer backbone. The accessibility of enzymes to the linker between the drug and the dendrimer

backbone is highly related to the generation of dendrimer and surface PEG chain length. It is necessary to enhance the release rate of the drug by reducing the generation of dendrimer or surface PEG chain length (Mol Pharm, 2018, 15, 4568). According to the literature, only polylysine dendrimer-drug conjugates with small molecular weights ($G4_{PEG570}$, a 4th generation dendrimer having an average molecular weight of about $2.26\times10^4$, conjugated with PEG having an average molecular weight of 570) can obtain a fast drug release rate (48% released in 48 h). When the PEG chain length is increased ($G4_{PEG1100}$, a 4th generation dendrimer having an average molecular weight of about $2.86\times10^4$, conjugated with PEG having an average molecular weight of 1100) or the generation of dendrimer is increased ($G5_{PEG570}$, a 5th generation dendrimer having an average molecular weight of about $4.51\times10^4$, conjugated with PEG having an average molecular weight of 570), the drug release rate decreases (29% released in 48 h). When the generation of dendrimer and the PEG chain length are both increased ($G5_{PEG1100}$, a 5th generation dendrimer having an average molecular weight of about $6.11\times10^4$, conjugated with PEG having an average molecular weight of 1100), the drug release rate is greatly reduced (5% released in 48 h after dosing). Although the rate of drug release can be increased by reducing the size of the dendrimer, it would face the problem of renal clearance. According to the literature (Mol Pharm, 2008, 5, 449), the renal clearances of different polylysine dendrimers at 0-24 h were as follows: 40.9% for $Lys_{16}(PEG_{570})_{32}$ (average molecular weight $2.24\times10^4$), 6.6% for $Lys_8(PEG_{2000})_{16}$ (average molecular weight $3.41\times10^4$), and 0.6% for $Lys_{16}(PEG_{2000})_{32}$ (average molecular weight $6.8\times10^4$). In view of this, the polylysine dendrimer needs to be conjugated with PEG of higher molecular weight (such as $PEG_{2000}$) to obtain a polymer with a total molecular weight greater than 60,000 to minimize its renal clearance. The higher molecular weight of dendrimers and surface modification with longer PEG mean higher steric hindrance, which leads to lower drug release rate and efficacy. Therefore, the current drug delivery system based on dendrimers can only take a balance between in-vivo clearance rate of polymer and in-tumor drug release rate, and cannot achieve the best efficacy.

CONTENT OF THE PRESENT INVENTION

The technical problem to be solved by the present invention is the difficulty in balancing clearance, retention and drug release of dendrimer-drug conjugates in the prior art and therefore in achieving the best therapeutic efficacy. Thus, the present invention provides a poly(ε-L-lysine)-based drug conjugate, its intermediates and applications thereof. The drug conjugate of the present invention has one or more advantages of low renal clearance, low hepatosplenic clearance, long plasma half-life, rapid accumulation of effective drug concentration in tumors and better therapeutic effect.

The aim of the present invention is to provide a poly(ε-L-lysine) derivative-drug conjugate which has a controllable number of conjugates and a controllable position of conjugation, as shown in formula I:

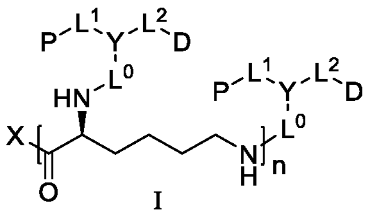

I

the poly(ε-L-lysine) derivative-drug conjugate includes:

poly(ε-L-lysine) residue, as shown in formula II, the number of repeating units of the poly(ε-L-lysine) being n,

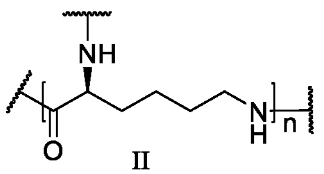

II

where the poly(ε-L-lysine) is obtained from biosynthesis or chemical synthesis;
n is an integer of 4 to 100;
Y, which is a branched center with at least three functionalities;
P, which is a pharmacokinetic regulator residue;
D, which is a pharmaceutically active agent residue;
X, which is a terminal group;
$L^0$, $L^1$ and $L^2$, which are each independently covalent bonds or $C_1$-$C_{40}$ linkers with or without heteroatoms, where

the heteroatoms are O, S, Se, N, P, Si or B, and the number of the heteroatoms is one or more; when the number is more than one, the heteroatoms could be the same or different; the linkers may or may not contain unsaturated groups; the $L^0$ links poly(ε-L-lysine) residue and Y, the $L^1$ links P and Y, and the $L^2$ links D and Y

In some embodiments, the number n of the poly(ε-L-lysine) repeating units is an integer of 4 to 100.

In some embodiments, the number n of the poly(ε-L-lysine) repeating units is an integer of 10 to 70.

In some embodiments, the number n of the poly(ε-L-lysine) repeating units is an integer of 20 to 40, such as 30.

In the poly(ε-L-lysine) derivative-drug conjugate of the present invention, Y is a branched center with at least three functionalities including the following structures, or a multifunctional branched center composed of two or more of the following structures:

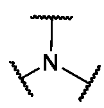 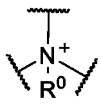 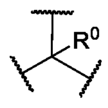 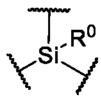 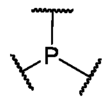 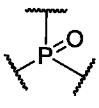 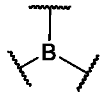

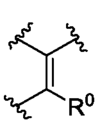 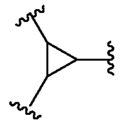 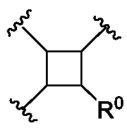 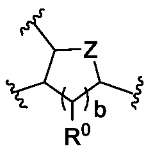 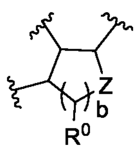 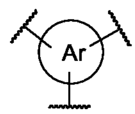

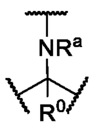 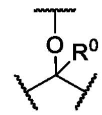 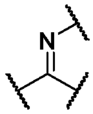 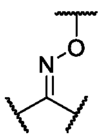 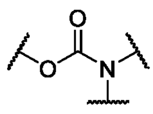 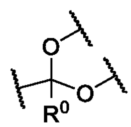 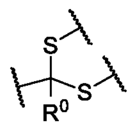

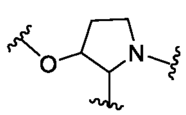 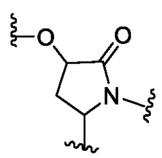 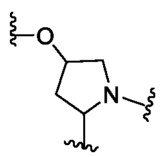

where Z is O, S, S(O), $S(O)_2$, $NR^a$ or $CHR^0$;

$R^0$ is selected from H, D, halogen, nitro, cyano, $C_1$~$C_{10}$ alkyl, $C_1$~$C_{10}$ alkoxy, $C_3$~$C_{10}$ alkenyl, $C_3$~$C_{10}$ alkynyl, $C_3$~$C_8$ cycloalkyl, $C_2$~$C_8$ heterocycloalkyl, $C_6$~$C_{10}$ aryl, $C_5$~$C_{10}$ heteroaryl or functional groups containing primary amine, secondary amine, tertiary amine, hydroxyl, sulfhydryl, carboxyl, ester group, amide, boronic acid, borate, phosphoric acid, sulfonic acid, sulfoxide, aldehyde group or ketone group; the heteroatoms in the $C_2$~$C_8$ heterocycloalkyl are O, S or N, and the number of the heteroatoms is one or more; when the number is more than one, the heteroatoms could be the same or different; the heteroatoms in the $C_5$~$C_{10}$ heteroaryl are O, S or N, and the number of the heteroatoms is one or more; when the number is more than one, the heteroatoms could be the same or different;

$R^a$ is selected from H, $C_1$~$C_{10}$ alkyl, $C_1$~$C_{10}$ alkoxy, $C_3$~$C_{10}$ alkenyl, $C_3$~$C_{10}$ alkynyl, $C_3$~$C_8$ cycloalkyl, $C_2$~$C_8$ heterocycloalkyl, $C_6$~$C_{10}$ aryl or $C_5$~$C_{10}$ heteroaryl; the heteroatoms in the $C_2$~$C_8$ heterocycloalkyl are O, S or N, and the number of the heteroatoms is one or more; when the number is more than one, the heteroatoms could be the same or different; the heteroatoms in the $C_5$~$C_{10}$ heteroaryl are O, S or N, and the number of the heteroatoms is one or more; when the number is more than one, the heteroatoms could be the same or different;

Ar is $C_6$~$C_{20}$ aryl or $C_5$~$C_{20}$ heteroaryl; the heteroatoms in the $C_5$~$C_{20}$ heteroaryl are O, S or N, and the number of the heteroatoms is one or more; when the number is more than one, the heteroatoms could be the same or different;

b is an integer of 0 to 3.

In some embodiments, the branched center Y is a substituted or non-substituted amino acid (residue) with at least 3 functionalities, the amino acid is a natural amino acid or an unnatural amino acid, and the configuration of the amino acid is D type or L type or D/L mixture; when the amino acid is a mixture of D/L configuration, the L configuration proportion is greater than 0% and less than 100%.

In some embodiments, the branched center Y is a substituted or non-substituted amino acid (residue) with at least 3 functionalities, the amino acid is one or more of aspartic acid, glutamic acid, lysine, ornithine, arginine, citrulline, histidine, serine, threonine, tryptophan, tyrosine, hydroxyproline, cystine or cysteine, and the configuration of the amino

acid is D type or L type or D/L mixture; when the amino acid is a mixture of D/L configuration, the L configuration proportion is greater than 0% and less than 100%.

In some embodiments, the linkers $L^0$ and $L^1$ are each independently covalent bonds, environmentally responsive linkers or environmentally non-responsive linkers; $L^2$ is an environmentally responsive linker, and its structure is $L^{2a}$-$L^{2b}$, where $L^{2a}$ is linked to Y, $L^{2b}$ is linked to D, and $L^{2a}$ or $L^{2b}$ may exist alone or exist together; $L^{2a}$ and $L^{2b}$ are each independently covalent bonds or environmentally responsive linkers; the structure of the poly(ε-L-lysine) derivative-drug conjugate is shown in formula III:

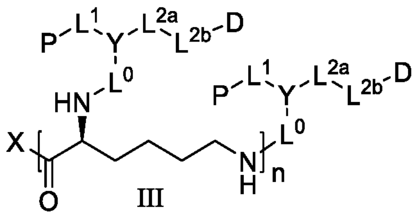

III

where X, Y, P, D, $L^0$ and $L^1$ are each defined as described in compound I.

In some embodiments, the linkers $L^0$ and $L^1$ are covalent bonds, $L^2$ is an environmentally responsive linker, and its structure is $L^{2a}$-$L^{2b}$, where $L^{2a}$ is linked to Y, $L^{2b}$ is linked to D, and $L^{2a}$ or $L^{2b}$ may exist alone or exist together; $L^{2a}$ and $L^{2b}$ are each independently covalent bonds or environmentally responsive linkers; the structure of the poly(ε-L-lysine) derivative-drug conjugate is shown in formula IV:

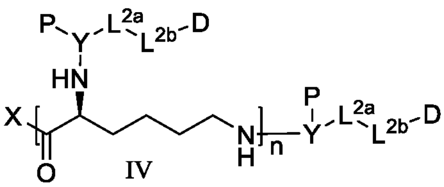

IV

where X, Y, P and D are each defined as described in compound I.

In some embodiments, when an electrophilic group in Y is linked to $L^2$, $L^{2a}$ does not exist; at this time, $L^2$ = $L^{2b}$, and the linking of the trifunctional branched center Y to the linkers $L^0$, $L^1$ and $L^2$ is selected from any of the following structures:

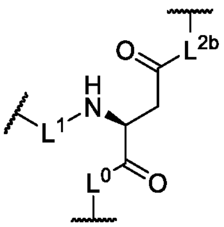
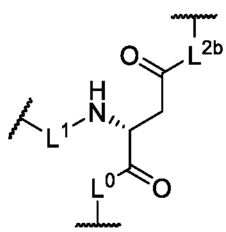
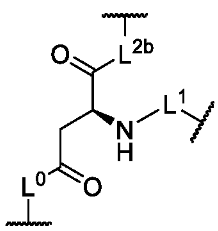
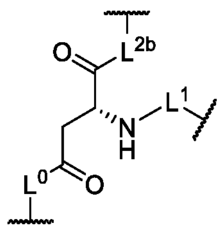

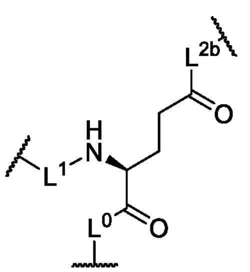
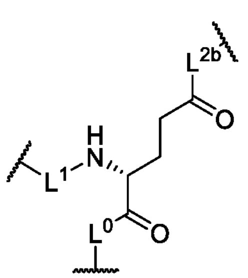
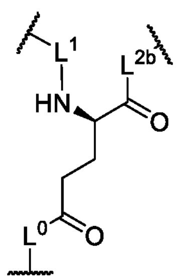
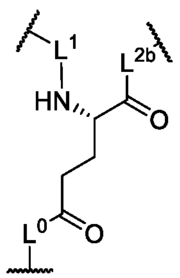
;

preferably

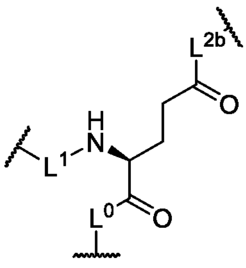 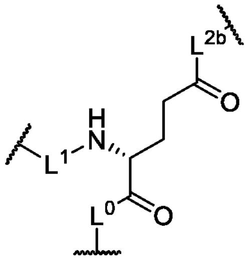 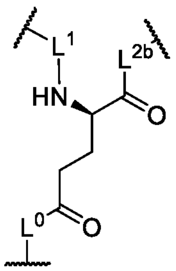 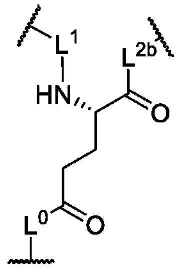 ;

more preferably

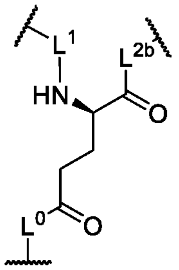 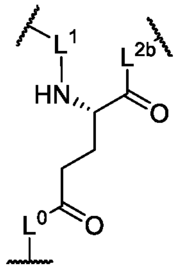 .

In some embodiments, when a nucleophilic group in Y is linked to $L^2$, $L^{2a}$ exists alone or $L^{2a}$ and $L^{2b}$ exist together; at this time, $L^2 = L^{2a}$ or $L^2 = L^{2a}-L^{2b}$, and the linking of the trifunctional branched center Y to the linkers $L^0$, $L^1$ and $L^2$ are selected from any of the following structures:

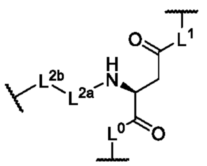 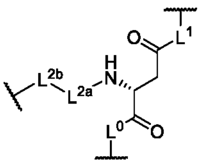 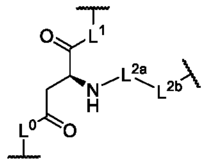 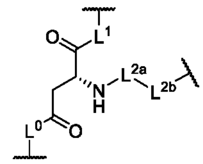

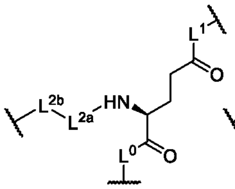 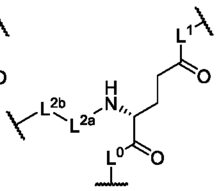 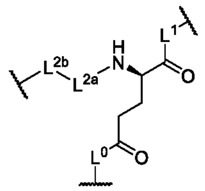 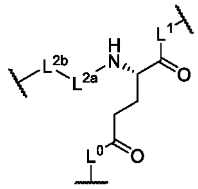

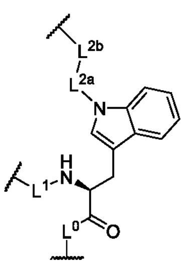 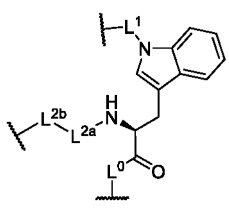 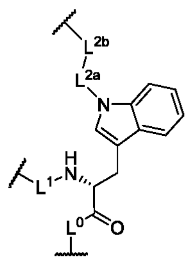 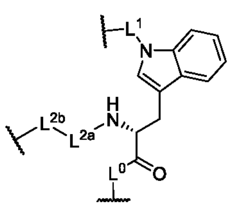

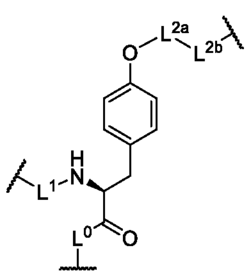
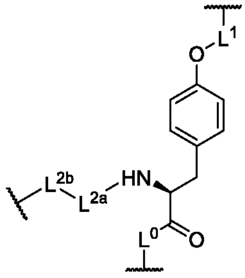
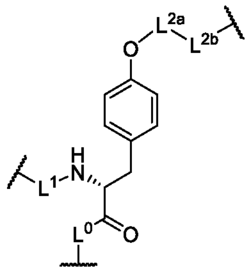
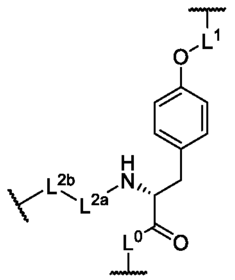

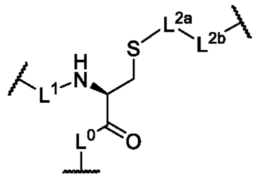
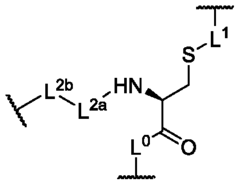
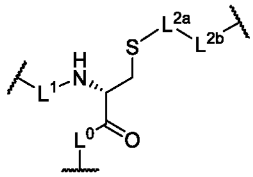
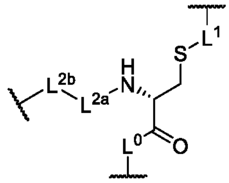

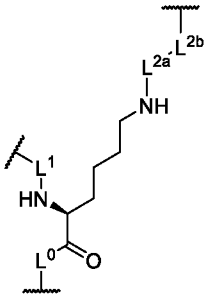
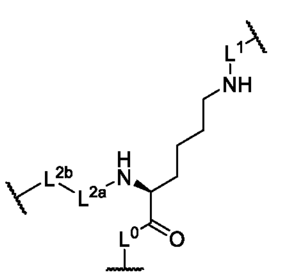
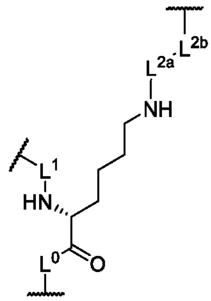
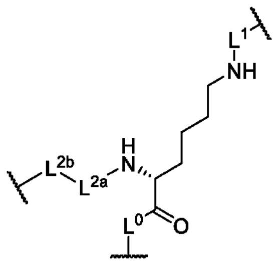

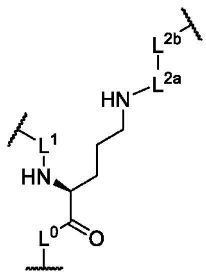
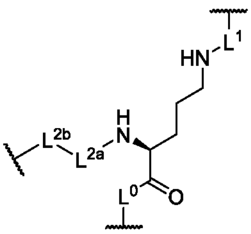
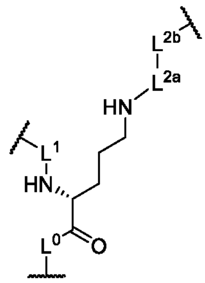
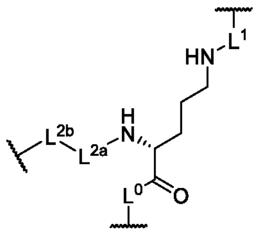

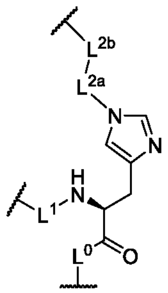
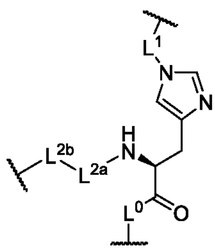
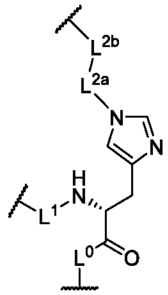
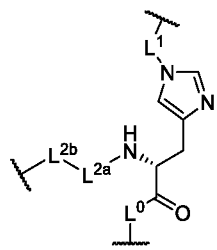

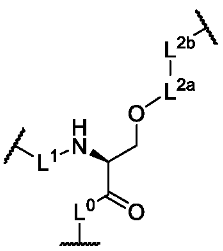 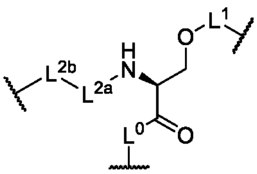 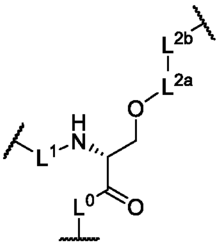 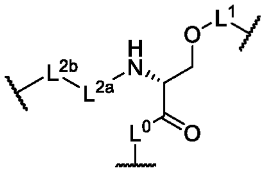

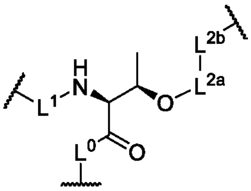 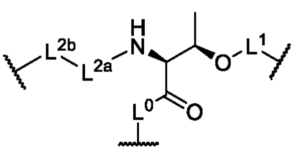 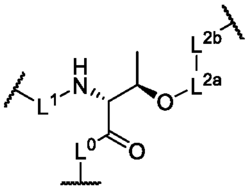 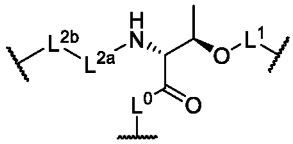

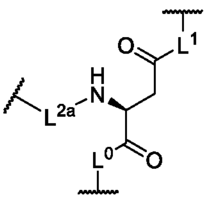 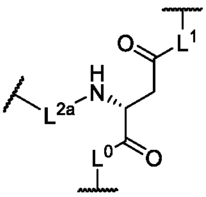 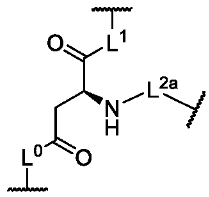 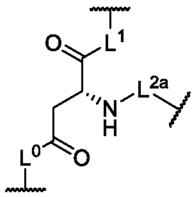

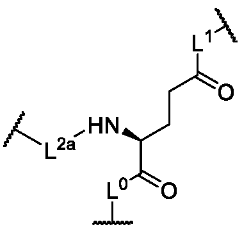 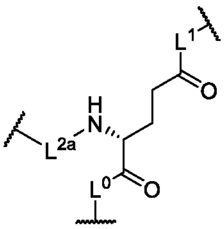 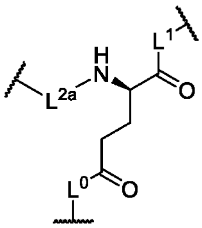 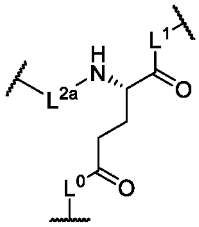

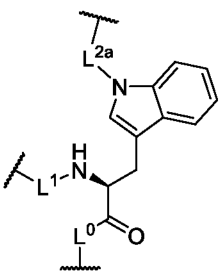 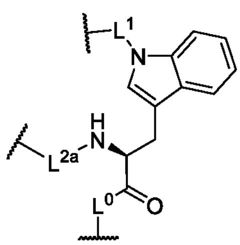 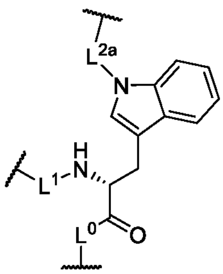 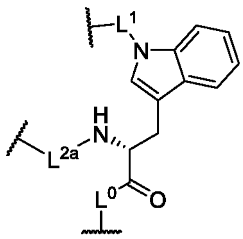

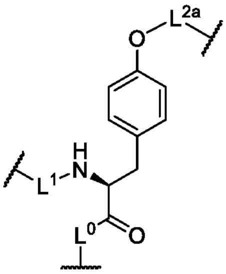 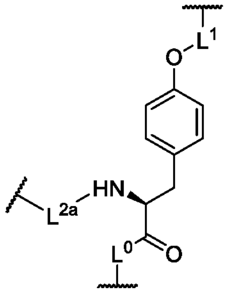 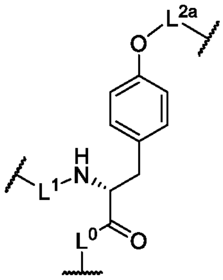 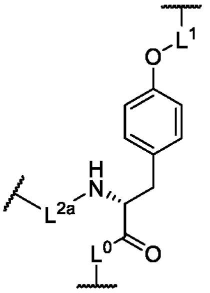

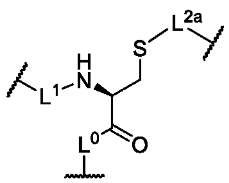 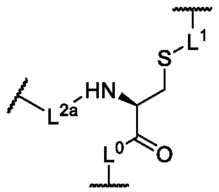 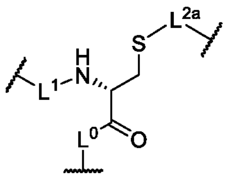 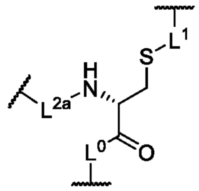

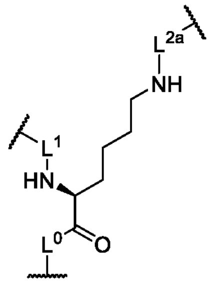 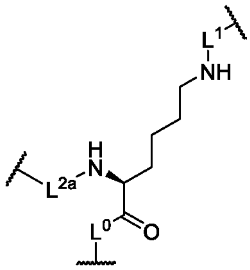 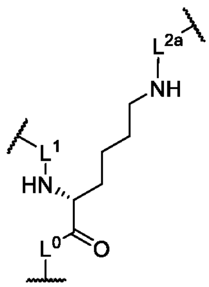 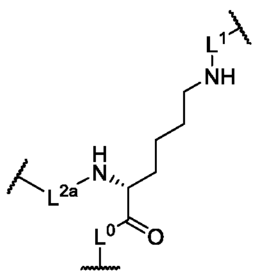

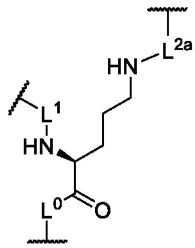 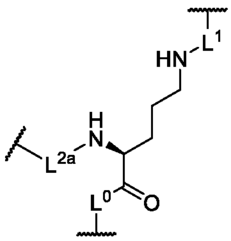 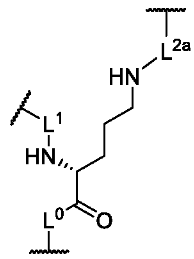 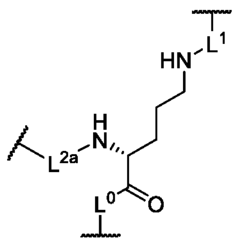

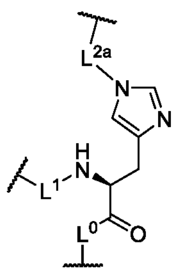 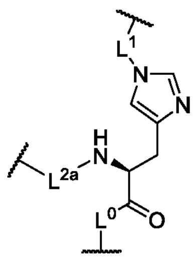 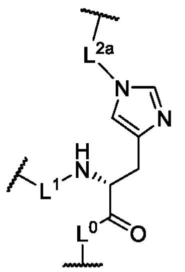 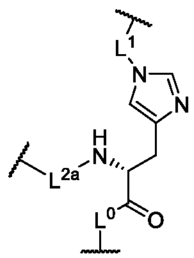

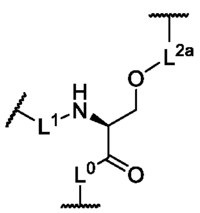 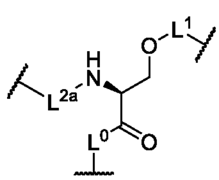 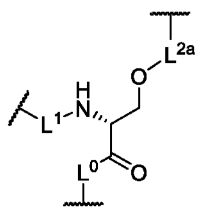 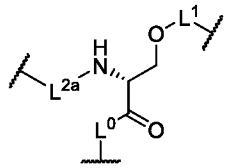

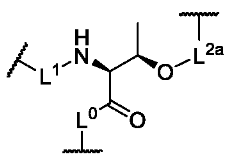 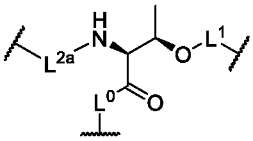 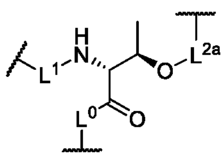 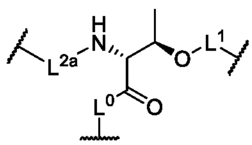

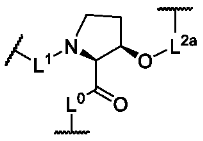 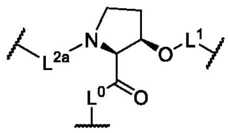 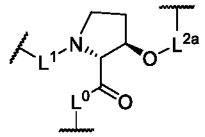 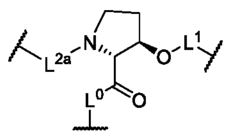

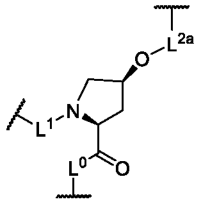 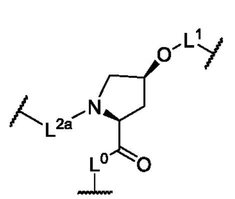 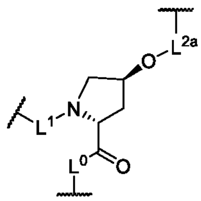 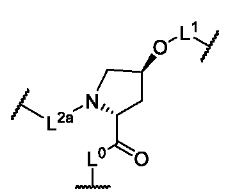 ;

preferably

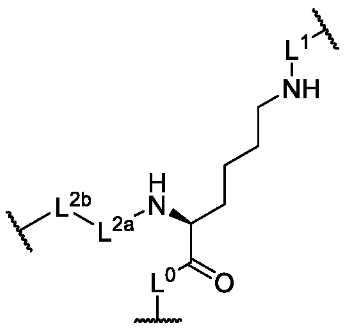 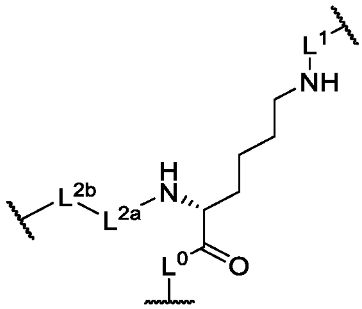 , 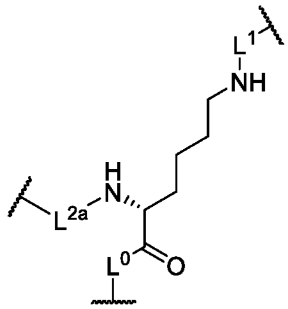

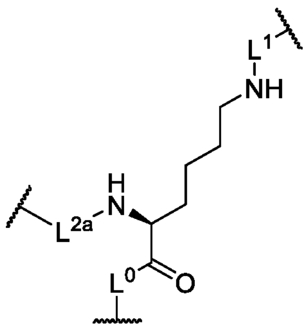 .

In some embodiments, the terminal group X is selected from OR, SR, $NR^1R^2$, carboxyl protection group or $L^{2b}$-D, and the R, $R^1$ and $R^2$ are independently selected from H, $C_1$~$C_{30}$ alkyl, $C_1$~$C_{30}$ alkoxy, $C_3$~$C_{30}$ alkenyl, $C_3$~$C_{30}$ alkynyl, $C_3$~$C_8$ cycloalkyl, $C_2$~$C_8$ heterocycloalkyl, $C_6$~$C_{20}$ aryl, $C_5$~$C_{20}$ heteroaryl; $R^1$, $R^2$ and N atoms linked thereto can form $C_2$~$C_8$ heterocycloalkyl; the heteroatoms in the $C_2$~$C_8$ heterocycloalkyl are O, S or N, and the number of the heteroatoms is one or more; when the number is more than one, the heteroatoms could be the same or different; the heteroatoms in the $C_5$~$C_{20}$ heteroaryl are O, S or N, and the number of the heteroatoms is one or more; when the number of the heteroatoms is more than one, the heteroatoms are the same or different.

In some embodiments, the terminal group X is selected from OR, SR, $NR^1R^2$, carboxyl protection group or $L^{2b}$-D, and the R, $R^1$ and $R^2$ are independently selected from H, $C_1$~$C_{10}$ alkyl, or $R^{1-1}$-substituted $C_1$~$C_{10}$ alkyl; the $R^{1-1}$ is $-NH_2$ or

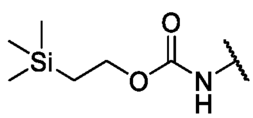 .

In some embodiments, the environmentally responsive linker is one or more of enzyme-responsive linker, pH-responsive linker, photo-responsive linker and redox-responsive linker.

In some embodiments, the enzyme-responsive linker can be cleaved by one or more of the following enzymes: secretory phospholipase A2, acid phosphatase, serum alkaline phosphatase, cytochrome P450, sulfatase, prostate-specific antigen, phospholipase A1, phospholipase A2, phospholipase B, phospholipase C, phospholipase D, neutrophil elastase, cysteine protease-3, cathepsin, matrix metalloproteinase, β-glucuronidase, β-galactosidase, DTP, nitroreductase, NADPH, aminopeptidase N, carboxylesterase, diaphorase, histone deacetylase, asparaginyl endopeptidase, urokinase-type plasminogen activator, urokinase-type plasminogen activator receptor and collagenase.

In some embodiments, the enzyme-responsive linker can be cleaved by one or more of the following enzymes: cysteine protease-3, cathepsin, matrix metalloproteinase and β-glucuronidase.

In some embodiments, the enzyme-responsive linker includes the following amino acid sequences (residues): Cit-Phe, Lys-Lys, Phe-Lys, Arg-Arg, Val-Cit, Val-Ala, Val-Lys, Ala-Lys, Phe-Cit, Leu-Cit, Ile-Cit, Trp-Cit, Phe-Arg, Asn-Pro-Val, Gly-Pro-Nle, Glu-Val-Cit, Glu-Val-Ala, Gly-Phe-Gly, Gly-Phe-Phe, Gly-Leu-Gly, Gly-Val-Ala, Gly-Phe-Leu-Gly, Gly-Phe-Phe-Leu, Gly-Leu-Leu-Gly, Gly-Phe-Tyr-Ala, Gly-Phe-Gly-Phe, Gly-Gly-Phe-Gly, Ala-Gly-Val-Phe, Gly-Phe-Phe-Gly, Asp-Glu-Val-Asp, Gly-Phe-Leu-Gly-Phe, Gly-Phe-Ala-Gly-Leu-Phe, Gly-Leu-Ala-Ala-Val-Ala, Gly-Gly-Phe-Leu-Gly-Phe or Gln-Ser-Phe-Arg-Phe-Lys.

In some embodiments, the enzyme-responsive linker includes the following structures:

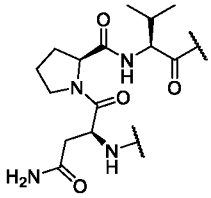
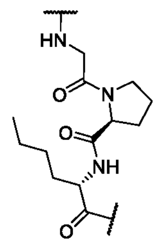
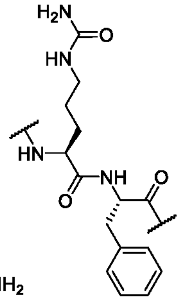
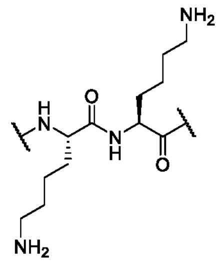
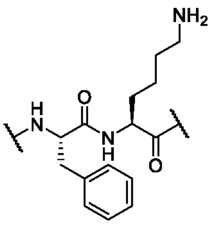
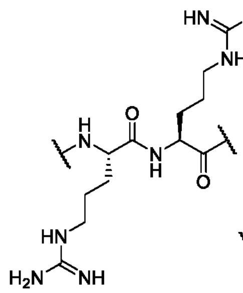
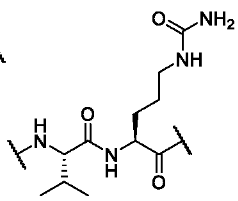
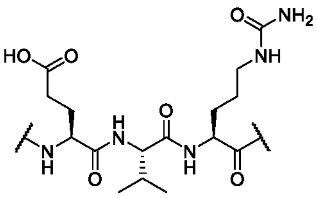
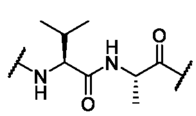
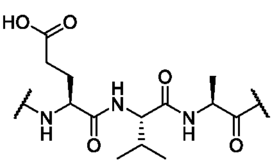
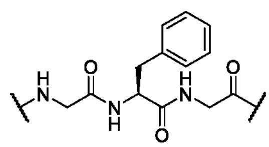
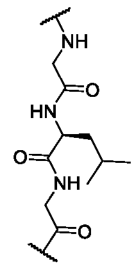
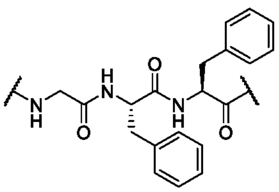
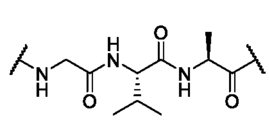
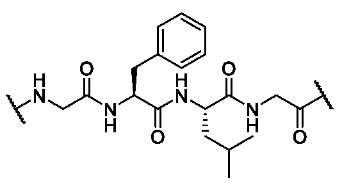

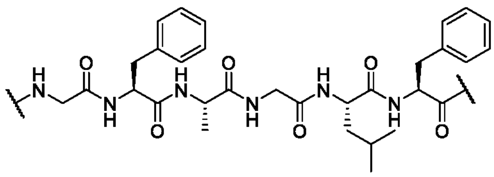

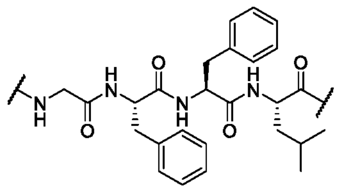

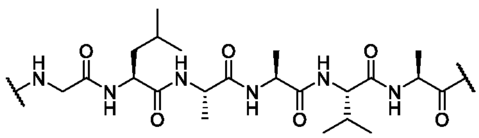

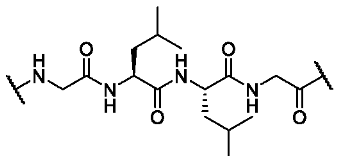

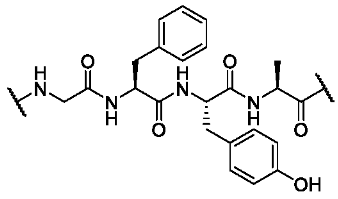

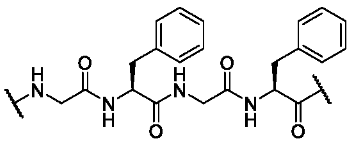

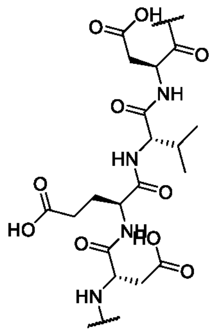

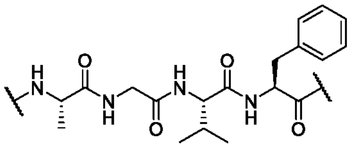

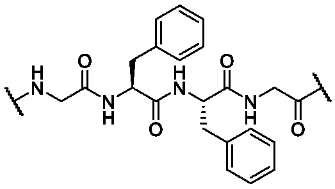

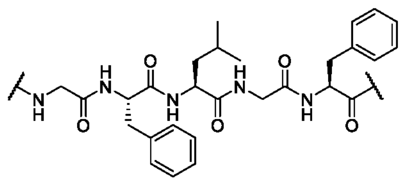

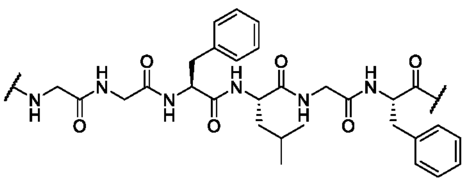

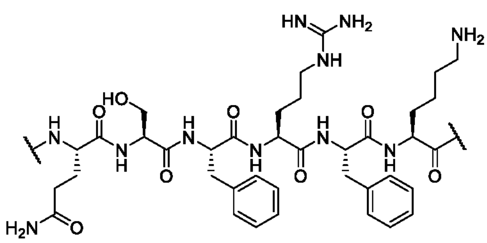

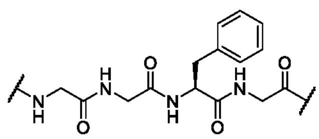

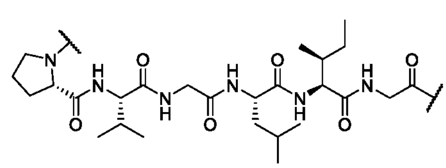

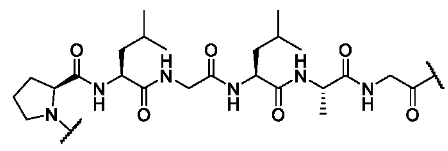

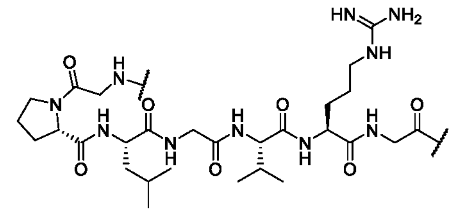

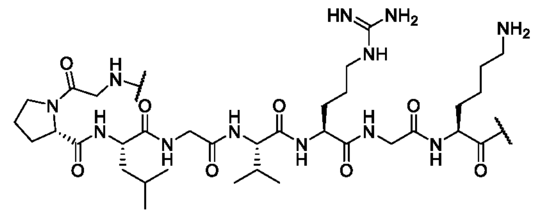

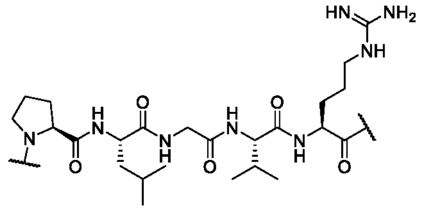

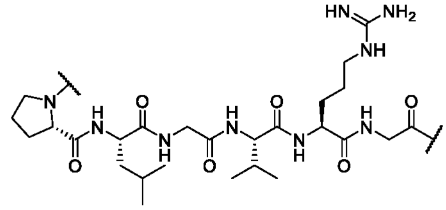

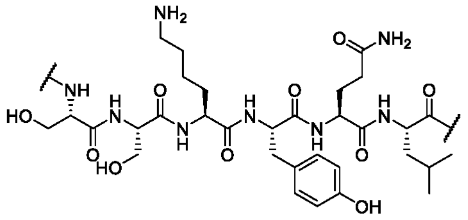

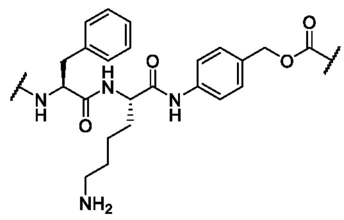

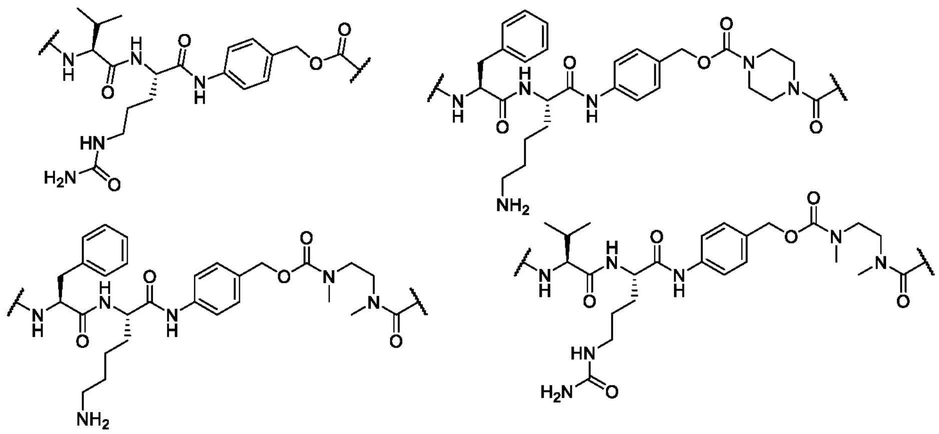

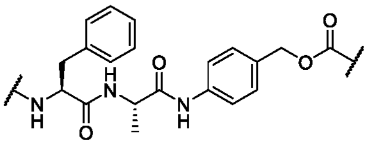
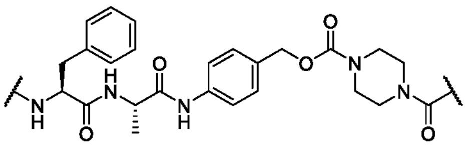
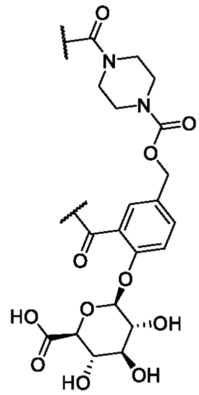
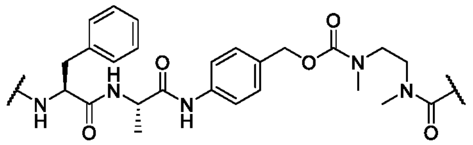
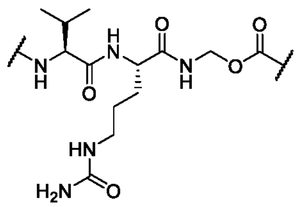
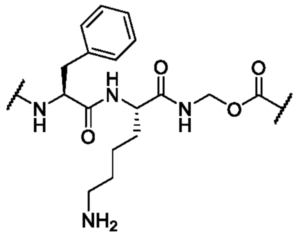
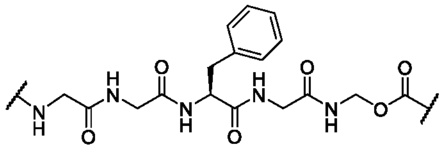
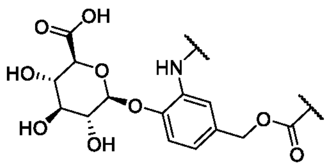
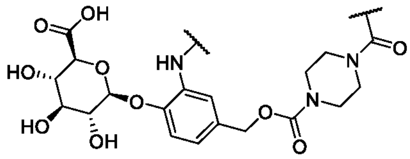
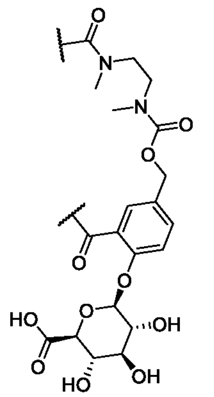
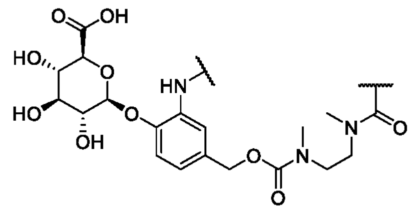
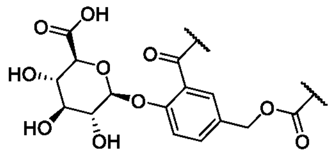

In some embodiments, the pH-responsive linker includes one or more of the following structures: hydrazone, imine, oxime, carboxylate, thioester, sulfate, sulfonate, orthoester, carbonate, carbamate, substituted carbamate, ketal, acetal, silyl ether, phosphate, borate, phosphoramidate or cis-aconitic acid groups.

In some embodiments, the pH-responsive linker includes the following structures:

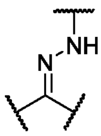
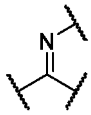
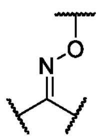
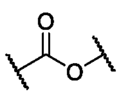
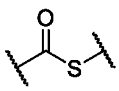
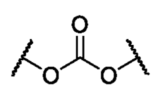
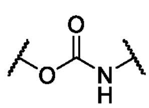
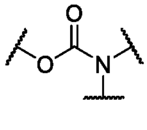
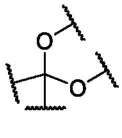
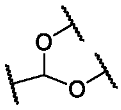
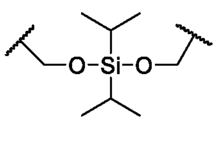
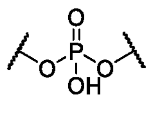
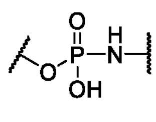
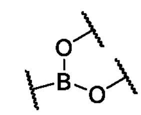
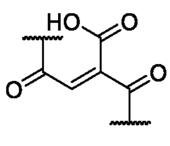
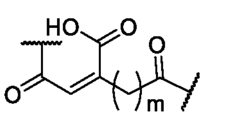

where m is 0 to 4.

In some embodiments, the photo-responsive linker includes one or more of the following structures: o-nitrobenzene, coumarin, benzoin, BODIPY or cyanine groups.

In some embodiments, the photo-responsive linker includes the following structures:

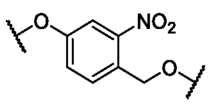 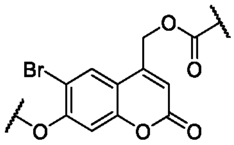 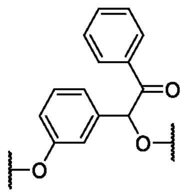

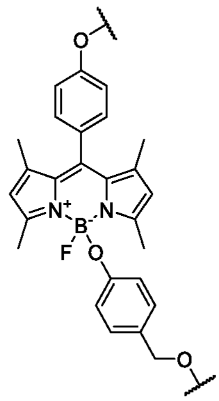 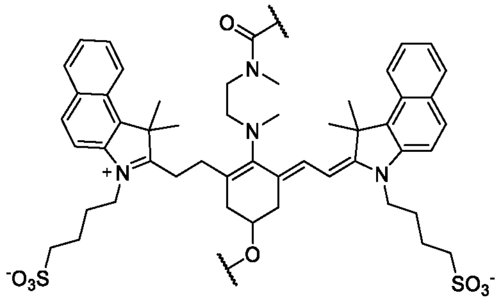

In some embodiments, the redox-responsive linker includes one or more of the following structures: thioketal, phenylborate, phenylboronic acid, oxalate, vinyl ether, thioether, amino acrylate, disulfide, diselenide, 2,4-dinitrobenzenesulfonate, 2-azidomethylbenzoate, 4-azidobenzyl, unsaturated acid ester or azobenzene groups.

In some embodiments, the redox-responsive linker includes the following structures:

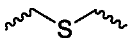 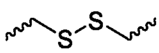 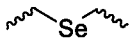 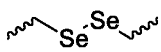 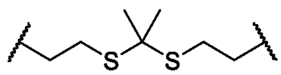

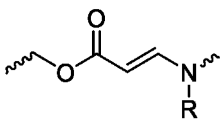 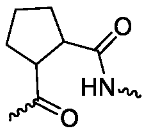 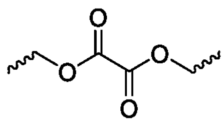 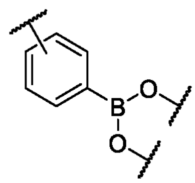

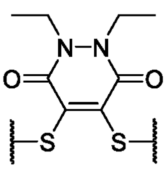 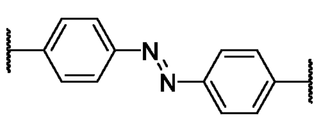

In some embodiments, the linker $L^{2a}$ is shown in formula V:

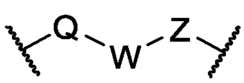

V

Q is selected from one of

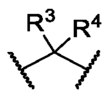 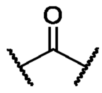 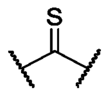 ;

where $R^3$ and $R^4$ are independently selected from H, D, Ci~Ce alkyl, Ci~Ce alkoxy, $C_3$~$C_6$ alkenyl, $C_3$~$C_6$ alkynyl, $C_3$~$C_8$ cycloalkyl, $C_2$~$C_8$ heterocycloalkyl, $C_6$~$C_{10}$ aryl or $C_5$~$C_{10}$ heteroaryl; $R^3$, $R^4$ and C atom linked thereto can form $C_3$~$C_8$ alkyl or heterocycloalkyl; the heteroatoms in the $C_2$~$C_8$ heterocycloalkyl are O, S or N, and the number of the heteroatoms is one or more; when the number is more than one, the heteroatoms could be the same or different; the heteroatoms in the $C_5$~$C_{10}$ heteroaryl are O, S or N, and the number of the heteroatoms is one or more; when the number is more than one, the heteroatoms could be the same or different;

W is a covalent bond or a $C_0$~$C_{20}$ fragment with or without heteroatoms, where the heteroatoms are O, S, Se, N, P, Si or B, and the number of the heteroatoms is one or more; when the number is more than one, the heteroatoms could be the same or different; the W fragment may or may not contain unsaturated bonds;

Z is selected from one of

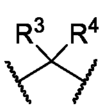 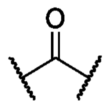 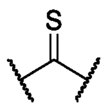 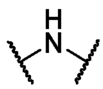 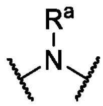 ;

where $R^a$ is defined as described in the definition of the branched center Y

In some embodiments, the linker $L^{2a}$ is selected from the following structures or a covalent bond:

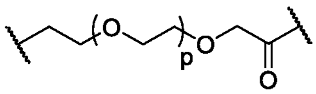 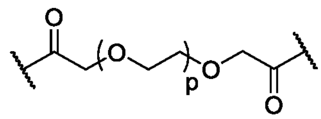 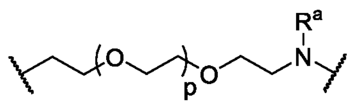

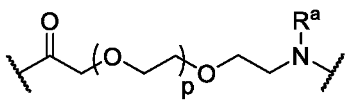 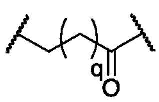 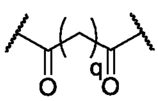 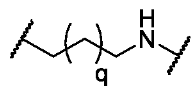 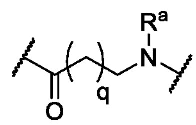

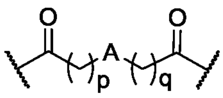 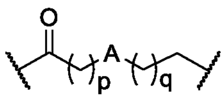 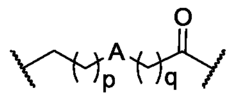

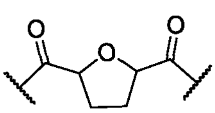 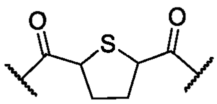 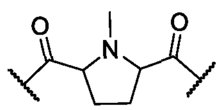 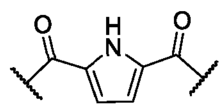 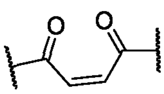

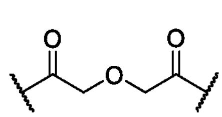 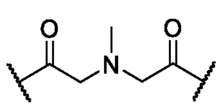 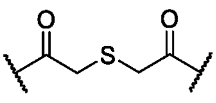 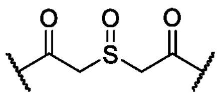 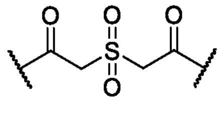

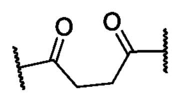 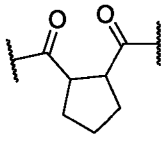 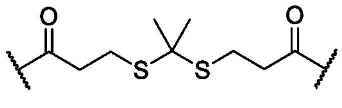 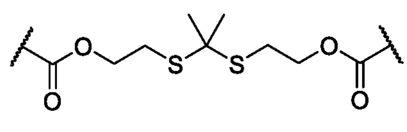

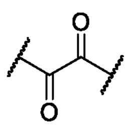 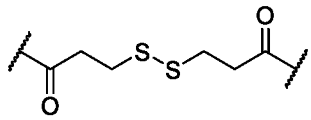 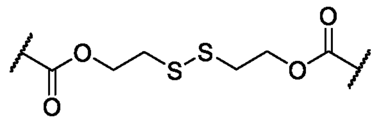 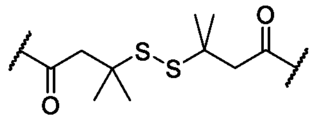

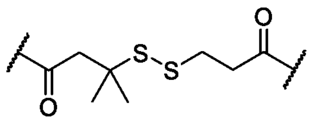 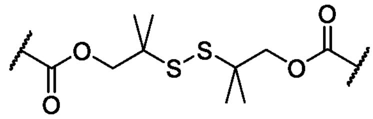 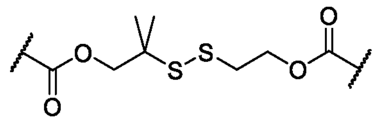

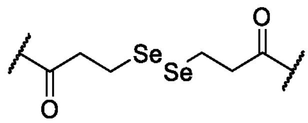 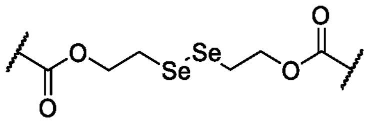 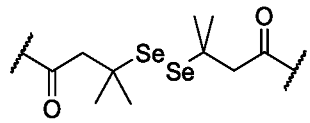

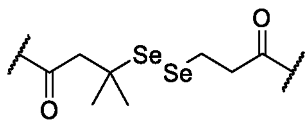 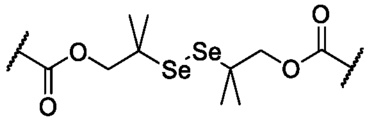 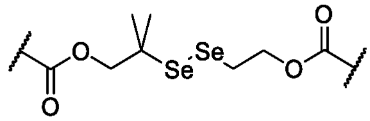

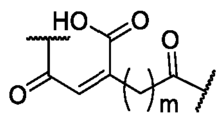

where

A is O, S, S(O), $S(O)_2$, $NR^a$ or $C(R^3R^4)$;
p is an integer of 0 to 16;
q is an integer of 0 to 16;
m is an integer of 0 to 4;
$R^a$ is defined as described in the definition of the branched center Y;
$R^3$ and $R^4$ are defined as described in formula V

The $L^{2a}$ is preferably the following structures:

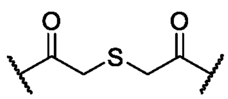, 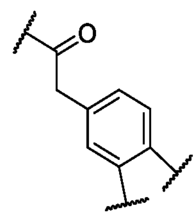,

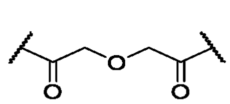, 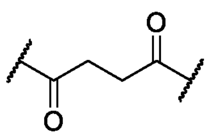, 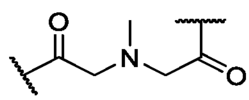, 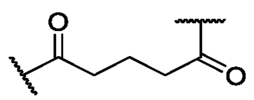,

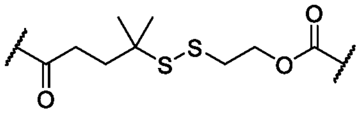, 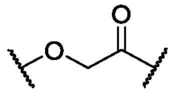, 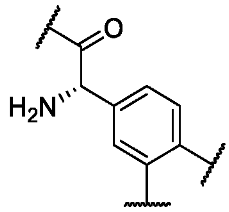, 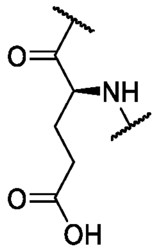, 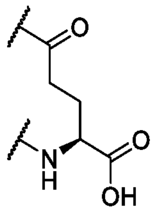;

in $L^{2a}$-$L^{2b}$-D, when $L^{2a}$ is

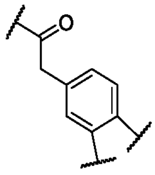 or 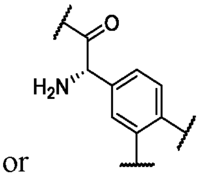,

bivalent aryl is linked to $L^{2b}$ or D (when $L^{2b}$) by carbon atoms to form ring structures, such as

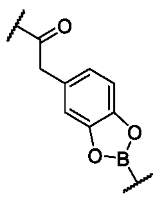 or 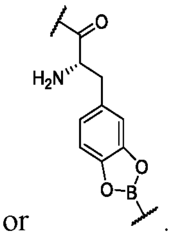.

In some embodiments, the linker $L^{2b}$ is selected from the following structures:

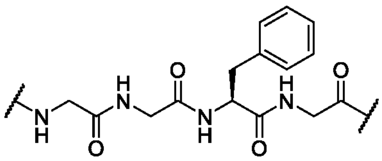 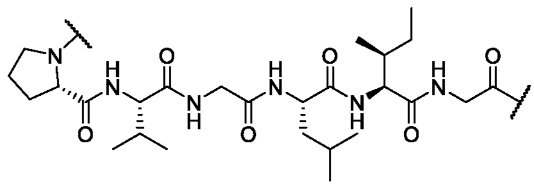

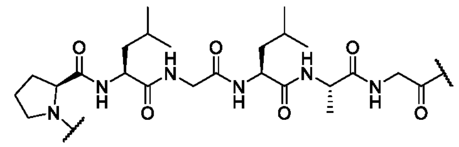
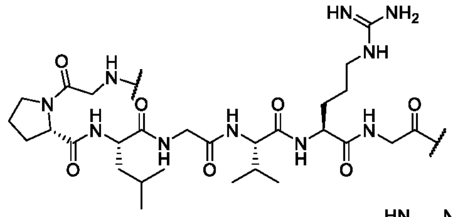
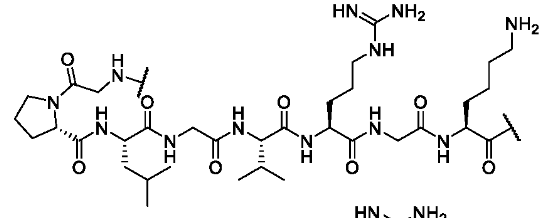
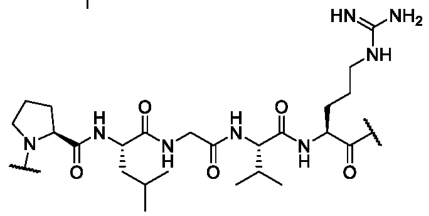
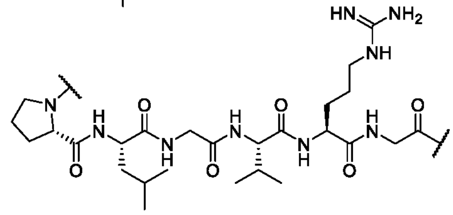
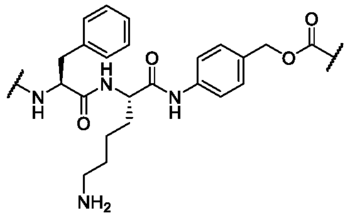
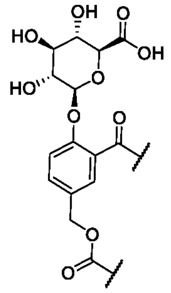
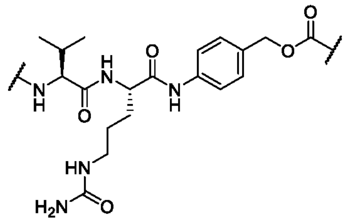
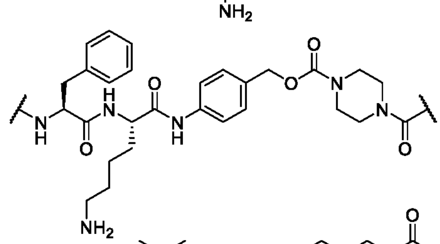
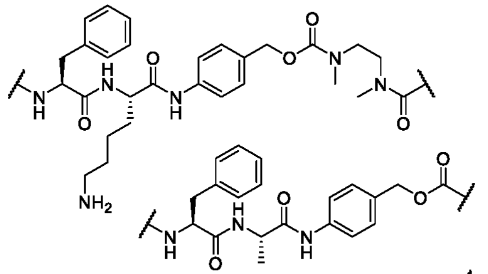
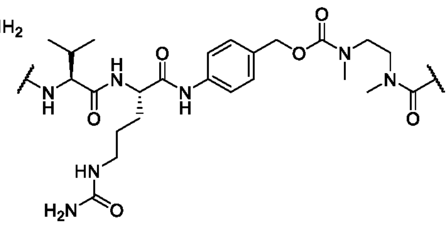
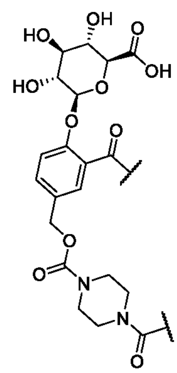
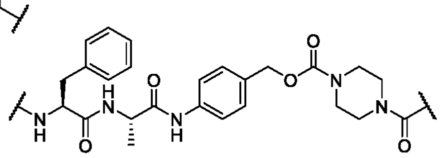
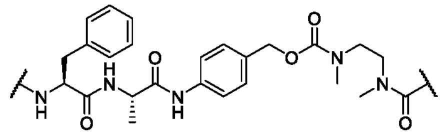
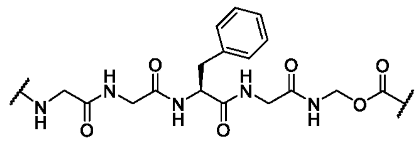
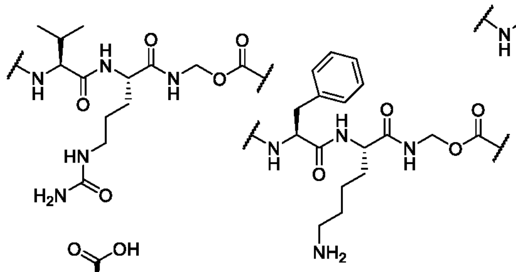
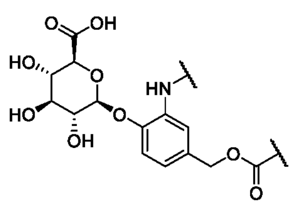
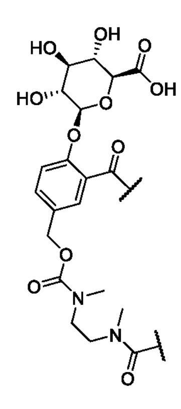
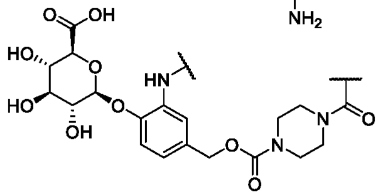
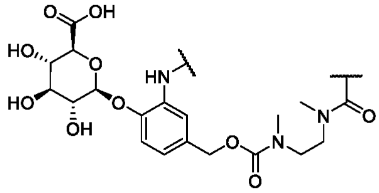

.

In some embodiments, the $L^{2b}$ is selected from the following structures:

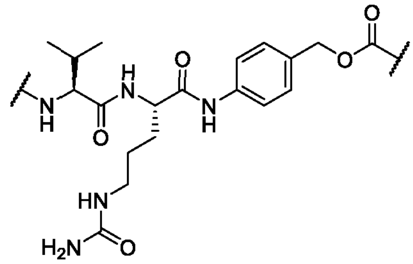

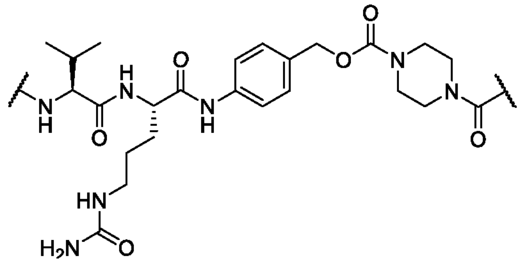

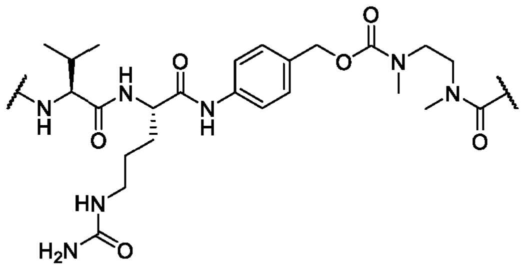

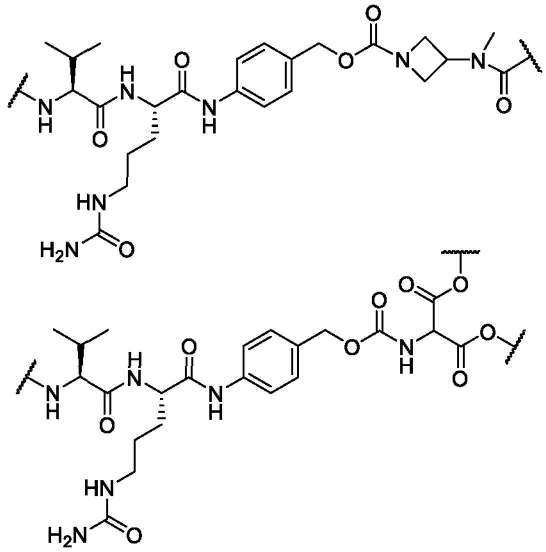

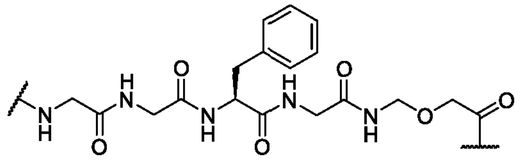

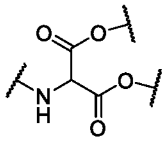

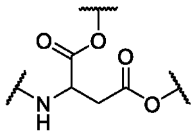

,

when $L^{2b}$ is

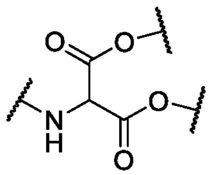 or 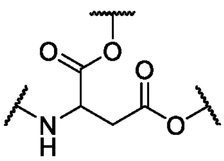, 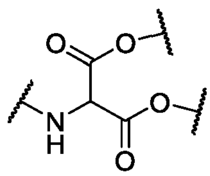 or 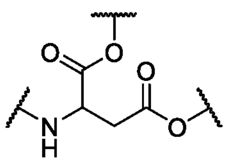

is linked to D by oxygen atoms to form ring structures, such as

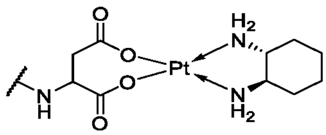

or

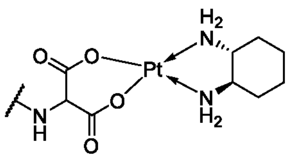

.

In some embodiments,

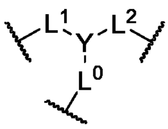 is 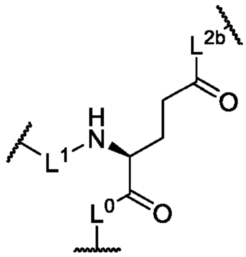 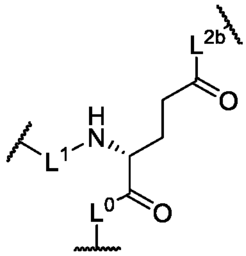

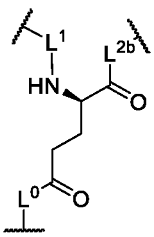 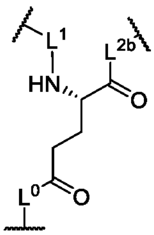 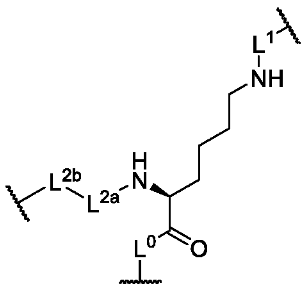 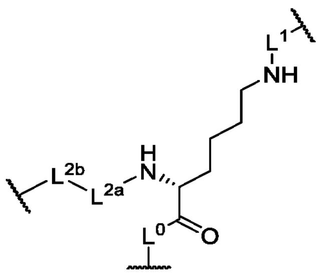 ,

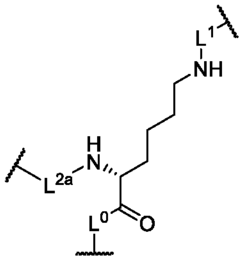 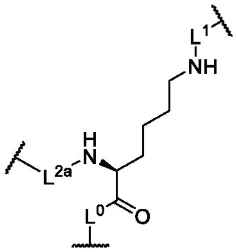 ,

such as

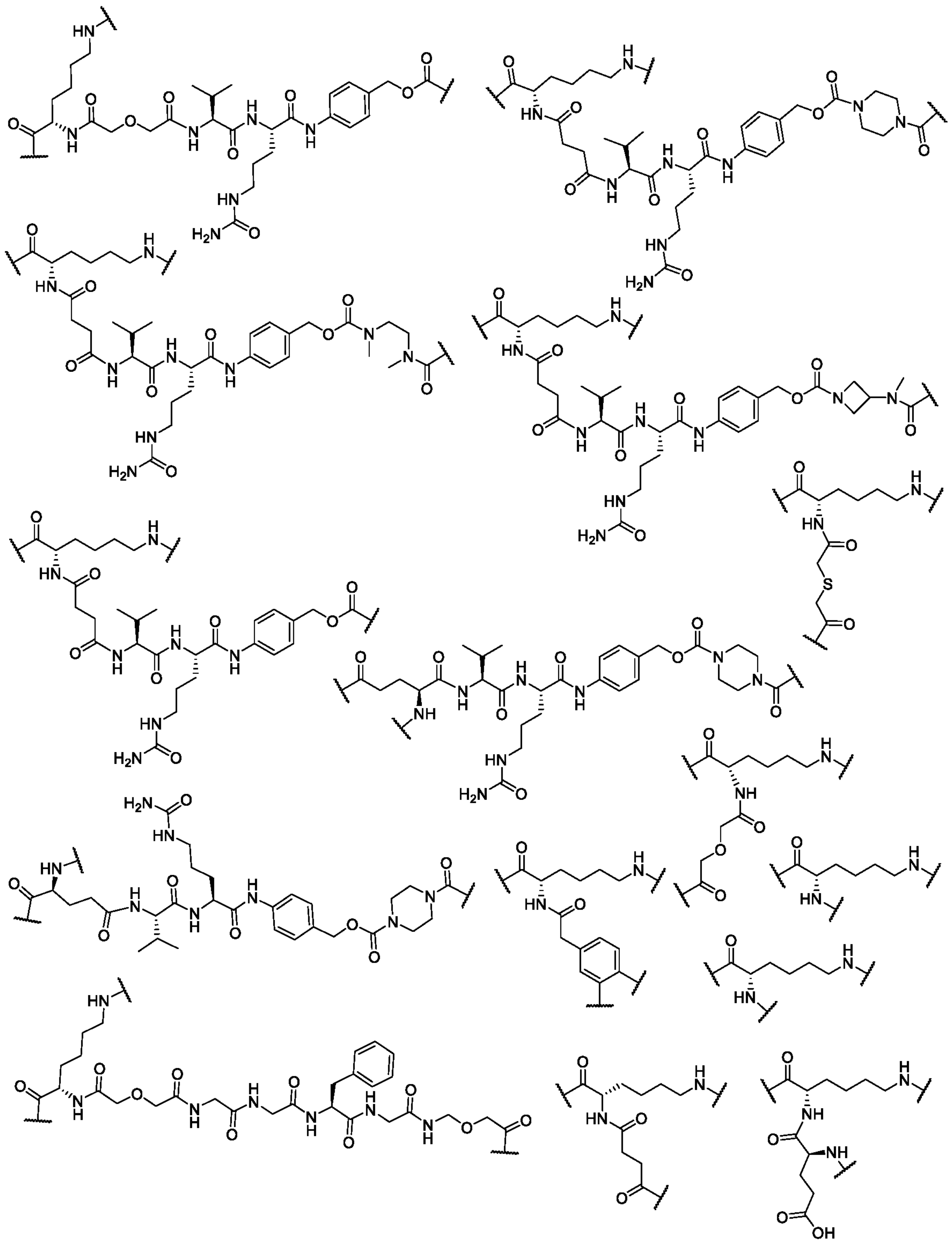

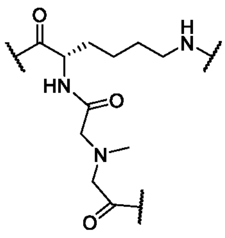
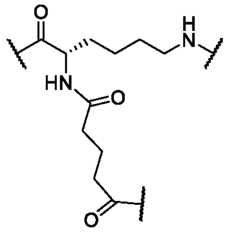
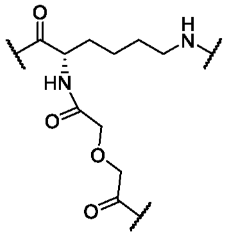
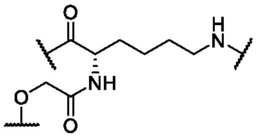

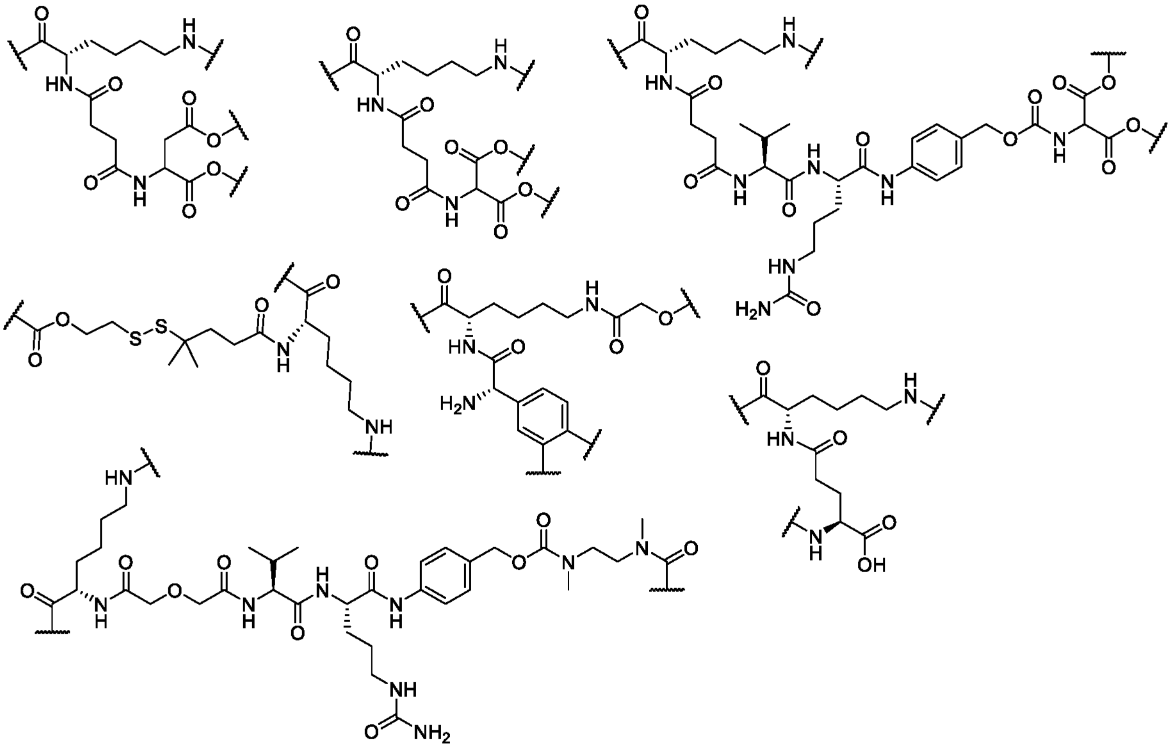

.

In some embodiments, the pharmacokinetic regulator residue P is selected from a polyethylene glycol derivative residue with the number of repeating units being a and a terminal group being $R^b$, a hyaluronic acid derivative residue with the number of repeating units being b, a polyphosphate residue with the number of repeating units being c, a polysarcosine residue with the number of repeating units being d or a polyoxazoline residue with the number of repeating units being f; where $R^b$ is H, $C_1$~$C_{10}$ alkyl, $C_1$~$C_{10}$ heteroalkyl, $C_3$~$C_{10}$ cycloalkyl, $C_3$~$C_{10}$ alkenyl, $C_3$~$C_{10}$ alkynyl or hydroxyl protecting group; a is an integer of 5 to 250, b is an integer of 5 to 250, c is an integer of 5 to 250, d is an integer of 5 to 250, e is an integer of 5 to 250, and f is an integer of 5 to 250.

In some embodiments, the pharmacokinetic regulator residue P is the polyethylene glycol derivative residue with the number of repeating units being a and a terminal group being $R^b$; where a is an integer of 5 to 150, and b is an integer of 5 to 150.

In some embodiments, the pharmacokinetic regulator residue P is the polyethylene glycol derivative residue with the number of repeating units being a and a terminal group being $R^b$; where a is an integer of 5 to 150, preferably an integer of 10 to 60, more preferably an integer of 15 to 50, such as 21, 43 or 44.

In some embodiments, the pharmacokinetic regulator residue P is the polyethylene glycol derivative residue with the number of repeating units being a and a terminal group being $R^b$; where $R^b$ is H, $C_1$~$C_{10}$ alkyl, preferably Ci~Ce alkyl, more preferably $C_1$~$C_3$ alkyl, such as methyl, ethyl, n-propyl or i-propyl.

In some specific embodiments, the pharmacokinetic regulator residue P is a methyl-terminated polyethylene glycol derivative residue, the number-average molecular weight of which is about 550, 1000, 2000, 3000, 4000 or 5000.

In some specific embodiments, the pharmacokinetic regulator residue P is a methyl-terminated polyethylene glycol derivative residue, the number-average molecular weight of which is about 1000 or 2000.

In some embodiments, the polyethylene glycol derivative residue is selected from the following structures:

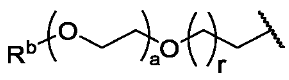 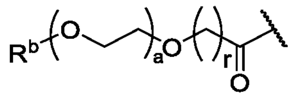

where a is an integer of 5 to 150, b is an integer of 5 to 150, and r is an integer of 0 to 8.

In some embodiments, the polyethylene glycol derivative residue is

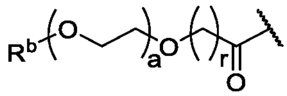,

a is an integer of 20 to 45, b is an integer of 5 to 10, and r is an integer of 0 to 3.

In some embodiments, the polyethylene glycol derivative residue is

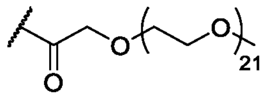 or 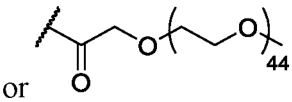.

In some embodiments, in the poly(ε-L-lysine) derivative-drug conjugate, the active functional groups of the pharmaceutically active agent D can be selected from one or more of primary amine, secondary amine, tertiary amine, hydroxyl, sulfhydryl, carboxyl, ester group, amide, boronic acid, borate, phosphoric acid, sulfonic acid, sulfoxide, aldehyde group and ketone group.

The pharmaceutically active agent is selected from one or more of anesthetics, antacids, anti-infective drugs, cardiovascular drugs, diuretics, blood tonics, immunosuppressants, GLP-1 receptor agonists, hormones and analogues, ophthalmic drugs, pain treatment agents, respiratory drugs, anti-arthritis drugs, anticonvulsants, antihistamines, anti-inflammatory drugs, anti-ulcer drugs, behavior modification drugs, antitumor drugs, anticancer antigens, central nervous system drugs, psychiatric disease drugs, contraceptives, diabetes drugs, growth promoters, hemostatic drugs, immunostimulants, immunomodulators, muscle relaxants, obesity therapeutics, osteoporosis drugs, sedatives, tranquilizers, urinary acidifiers, vitamins, polypeptide drugs, oligonucleotide drugs, mRNA drugs, antibody drugs, biological products, targeted protein degraders, PROTACs, oligosaccharide drugs or targeted drugs.

In particular, the pharmaceutically active agent residue D is an antitumor drug residue, the antitumor drug includes one or more of tumor targeted drugs, PROTACs, targeted protein degraders, tumor immunomodulators or chemotherapy drugs.

The antitumor drug can be selected from one or more of abemaciclib, abiraterone, abrocitinib, acalabrutinib, afatinib, aldesleukin, alectinib, alflutinib, almonertinib, altretamine, amcenestrant, aminoglutethimide, amsacrine, anastrozole, anlotinib, apalutamide, apatinib, arzoxifene, asciminib, asparaginase, avapritinib, avitinib, axitinib, azacitidine, baricitinib, belinostat, bendamustine, bexarotene, bicalutamide, bicyclol, binimetinib, bleomycin, boanmycin, bortezomib, bosutinib, brigatinib, buserelin, busulfan, cabazitaxel, cabozantinib, calaspargase, calicheamycin, capecitabine, capmatinib, carboplatin, carfilzomib, carmustine, carmofur, cedazuidine, ceritinib, cetrorelix, chidamide, chlorambucil, cisplatin, cladribine, clofarabine, cobimetinib, colchicine, copanlisib, crizotinib, cyclophosphamide, cytarabine, dabrafenib, dacarbazine, dacomitinib, dactinomycin, dalpiciclib, darolutamide, dasatinib, daunorubicin, decitabine, degarelix, delgociclib, denileukin, deruxtecan, docetaxel, donafenib, doxorubicin, duvelisib, enasidenib, encorafenib, ensartinib, entrectinib, enzalutamide, enzastaurin, elacestrant, epirubicin, erdafitinib, eribulin, erlotinib, estradiol, estramustine, etoposide, everolimus, exemestane, fasudil, fedatinib, filgotinib, floxuridine, fludarabine, flumatinib, fluorouracil, flutamide, fluzoparib, formestane, fostamatinib, fruquintinib, fulvestrant, gefitinib, gemcitabine, gilteritinib, giredestrant, glasdegib, goserelin, histrelin, hydroxyurea, ibrutinib, ibudilast, icaritin, icotinib, idarubicin, idelalisib, ifosfamide, imatinib, imiquimod, infigratinib, ingenol mebutate, interferon alfa-2b, irinotecan, ivosidenib, ixabepilone, ixazomib, lanreotide, lapatinib, larotrectinib, lenalidomide, lenvatinib, letrozole, leucovorin, leuprolide, lomustine, lonafarnib, lorlatinib, lurbinctedin, maytansine, mechlorethamine, medroxyprogesterone, megestrol, melphalan, melphlan flufenamide, mercaptopurine, methotrexate, methoxsalen, methylprednisolone, midostaurin, mitomycin, mitotane, mitoxantrone, mitozolomide, mobocertinib, monomethylauristatin E, monomethylauristatin F, nelarabine, nandrolone, neratinib, nearsudil, nilotinib, nilutamide, nintedanib, niraparib, octreotide, olaparib, olmutinib, omacetaxine, orelabrutinib, osimertinib, oxaliplatin, paclitaxel, pacritinib, palbociclib, pamidronate, pamiparib, panobinostat, pazopanib, peficitinib, pegaptanib, pegaspargase, peginteferon alfa-2b, pemigatinib, pemetrexed, pentetreotide, pentostatin, pexidartinib, phenoxybenzamine, pidotimod, plinabulin, plitidepsin, pomalidomide, ponatinib, porfimer, pralatrexate, pralsetinib, prednisolone, procarbazine, pyrotinib,

quizartinib, radotinib, raloxifene, raltitrexed, regorafenib, ribociclib, rintatolimod, ripretinib, romidepsin, rucaparib, ruxolitinib, savolitinib, selinexor, selpercatinib, selumetinib, sonidegib, sorafenib, sotorasib, streptozocin, sunitinib, surufatinib, talazoparib, tamoxifen, tazemetostat, tegafur, temozolomide, temsirolimus, teniposide, tepotinib, teprenone, thalidomide, thioguanine, thiotepa, thyrotropin alfa, tipiracil, tipifarnib, tirabrutinib, tirbanibulin, tivozanib, trametinib, tofacitinib, topotecan, toremifene, trabectedin, tretinoin, trifluride, trilaciclib, triptorelin, tucatinib, upadacitinib, umbralisib, utidelone, uroacitide, valrubicin, vandetanib, vemurafenib, venetoclax, vinblastine, vincristine, vindesine, vinflunine, vinorelbine, vismodegib, vorinostat, zanubrutinib, zoledronic acid, amatoxins, anthacyclines, anthracenes, anthramycins, auristatins, bryostatins, camptothecins, carmaphycins, combretastatins, cyclosporines, cryptomycins, ecteinascidins, ellipticenes, esperamicins, mustines, neothramycins, ozogamicins, phenoxazines, podophyllotoxins, pyrrolobenzodiazepines, sibiromycins, thailanstatins, tomamycns, tubulysins, taxanes, vinca alkaloids, 7-epipaclitaxel, 2'-acetylpaclitaxel, 10-deacetylpaclitaxel, 7-epi-10-deacetyltaxol, 7-xylosyltaxol, 10-deacetyl-7-glutarylpaclitaxel, 7-N,N-dimethylglycylpaclitaxel, 7-L-alanylacetaxel, larotaxel, camptothecin, 9-aminocamptothecin, 9-nitrocamptothecin, lurtotecan, gimatecan, belotecan, 10-hydroxycamptothecin, 10-hydroxy-7-ethyl-camptothecin (SN-38), exatecan, pirarubicin, aclacinomycin, sirolimus, tacrolimus, progesterone, estrogen, rapamycin, mithramycin, harringtonine or curcumin.

Furthermore, the chemotherapy drug is selected from one or more of amatoxins, anthacyclines, anthracenes, anthramycins, auristatins, bryostatins, camptothecins, carmaphycins, combretastatins, cyclosporines, cryptomycins, ecteinascidins, ellipticenes, esperamicins, maytansines, mustines, neothramycins, ozogamicins, phenoxazines, platinum complexes, podophyllotoxins, pyrrolobenzodiazepines, sibiromycins, thailanstatins, tomamycns, tubulysins, taxanes or vinca alkaloids.

In some specific embodiments, the chemotherapy drug is one or more of bleomycin, boanmycin, cabazitaxel, calicheamycin, carboplatin, cisplatin, dactinomycin, daunorubicin, deruxtecan, docetaxel, doxorubicin, epirubicin, eribulin, etoposide, idarubicin, irinotecan, ixabepilone, lurbinctedin, maytansinol, monomethylauristatin E, monomethylauristatin F, mitomycin, oxaliplatin, paclitaxel, streptozocin, teniposide, topotecan, trabectedin, valrubicin, vinblastine, vincristine, vindesine, vinflunine, vinorelbine, 7-epipaclitaxel, 2'-acetylpaclitaxel, 10-deacetylpaclitaxel, 7-epi-10-deacetyltaxol, 7-xylosyltaxol, 10-deacetyl-7-glutarylpaclitaxel, 7-N,N-dimethylglycylpaclitaxel, 7-L-alanylacetaxel, larotaxel, camptothecin, 9-aminocamptothecin, 9-nitrocamptothecin, lurtotecan, gimatecan, belotecan, 10-hydroxycamptothecin, 10-hydroxy-7-ethyl-camptothecin (SN-38), exatecan, pirarubicin, aclacinomycin or mithramycin.

In some embodiments, in the poly(ε-L-lysine) derivative-drug conjugate, the pharmaceutically active agent residue D is selected from the following structures:

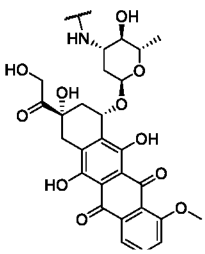
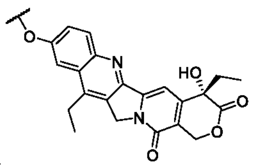
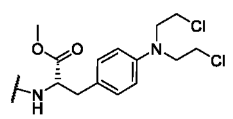
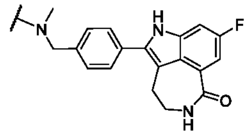

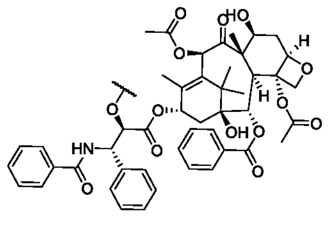
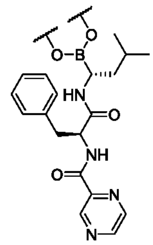
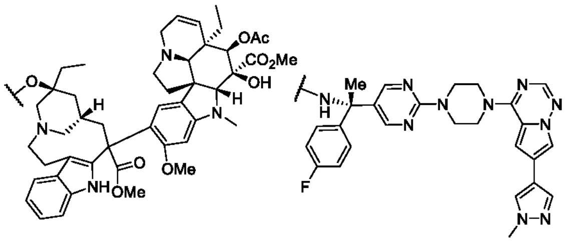

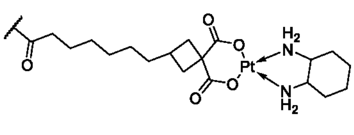
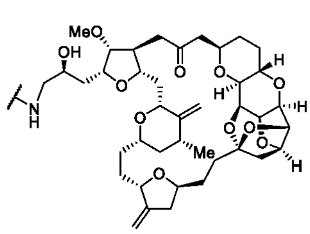
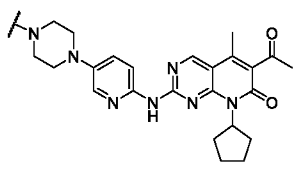

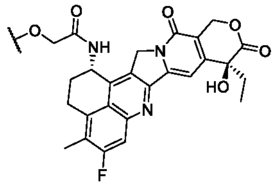

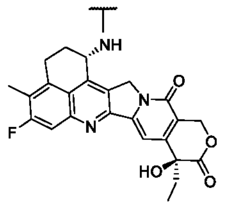

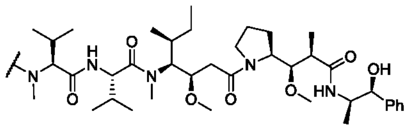

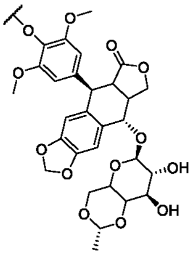

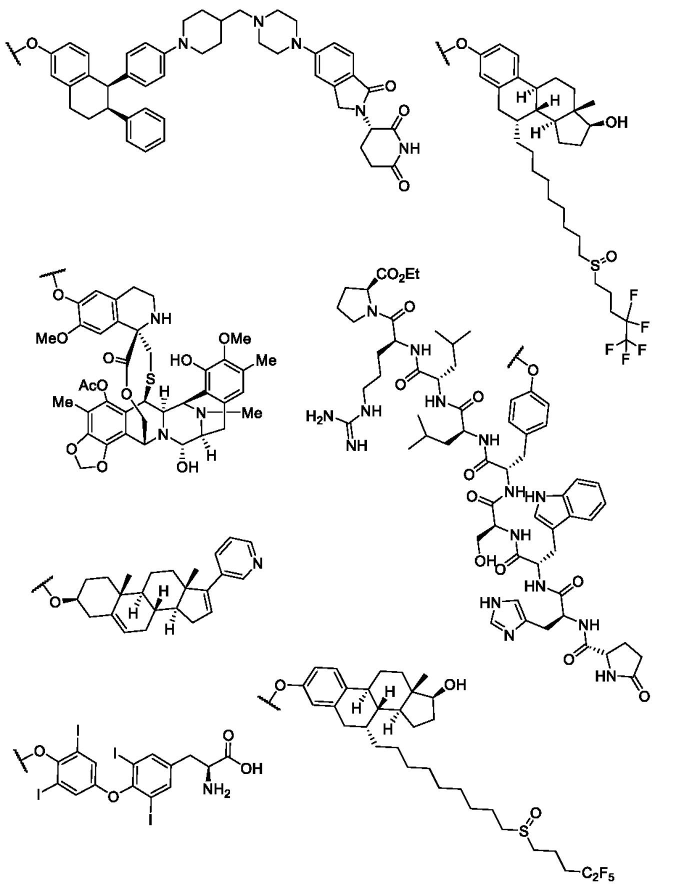

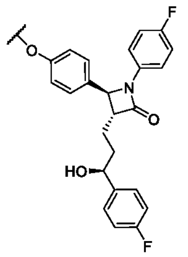

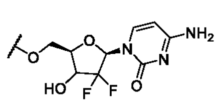

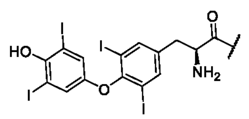

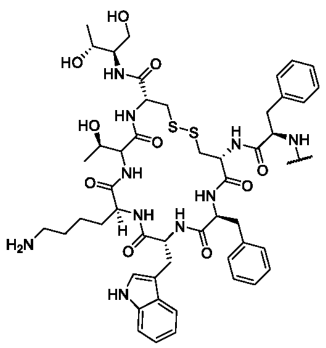

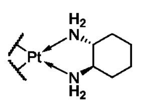

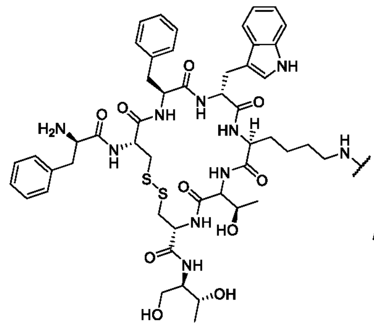

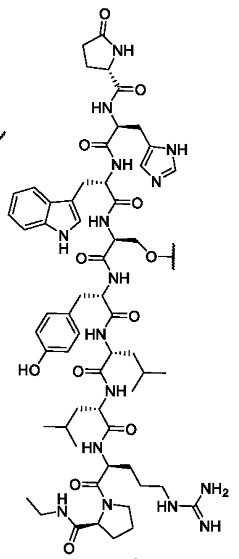

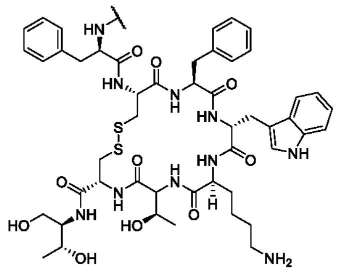

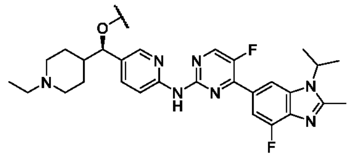

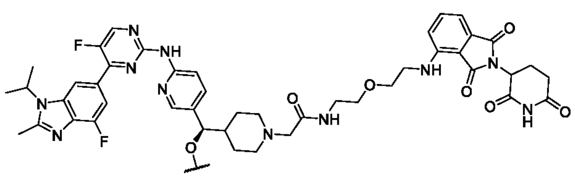

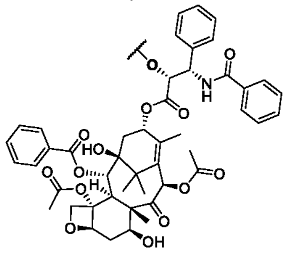

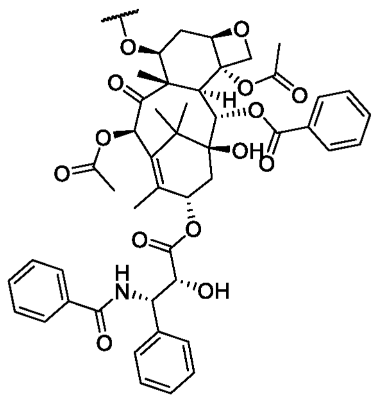

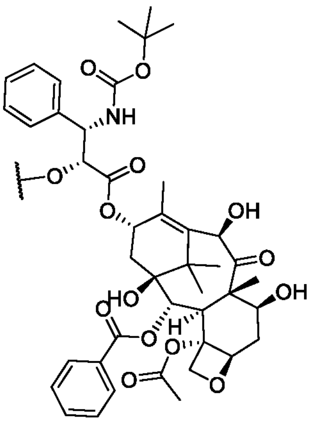

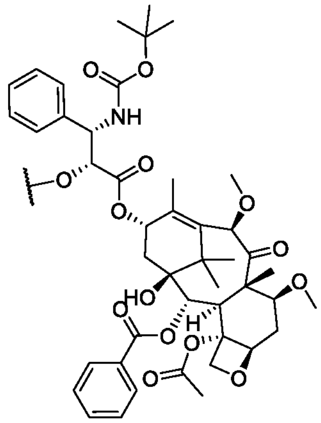

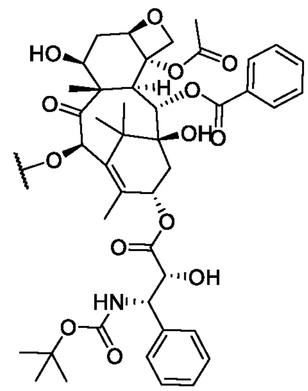

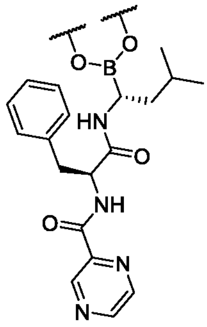

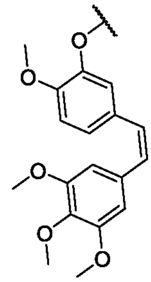

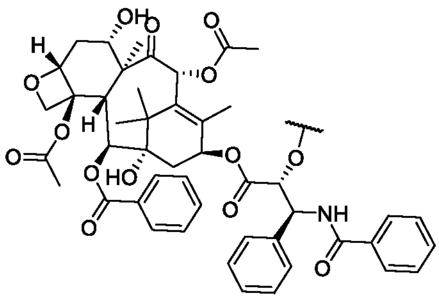

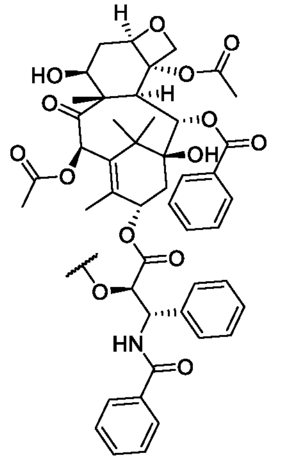

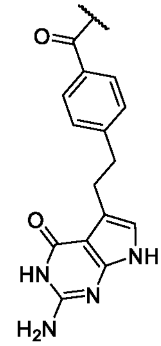

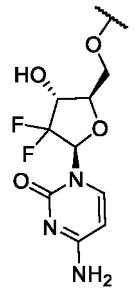

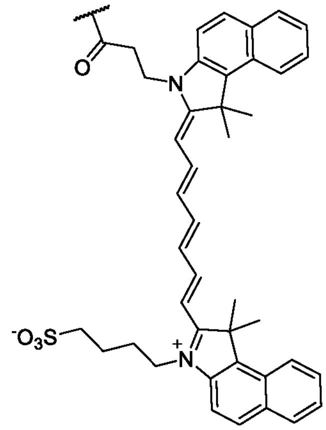

.

In some embodiments, the terminal group X is -OH,

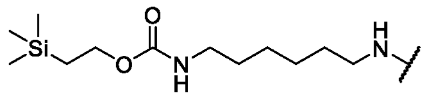

,

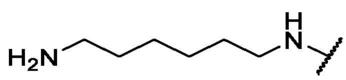

or $-L^{2b}-D$; the $-L^{2b}-D$ is preferably

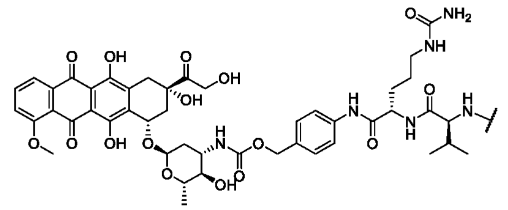

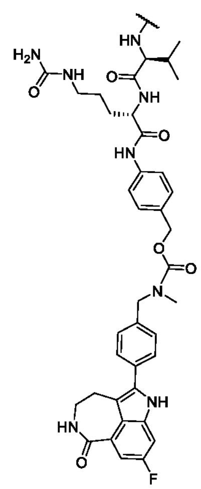

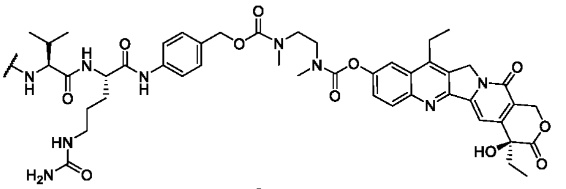

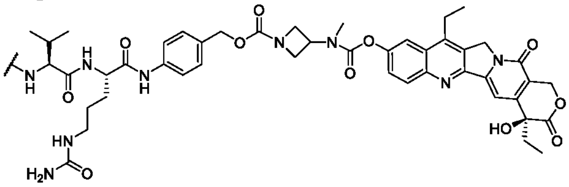

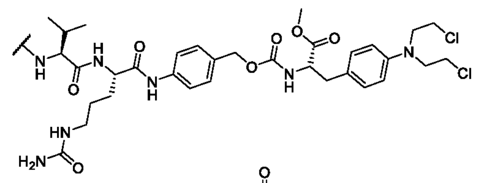

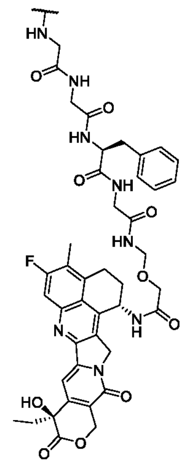

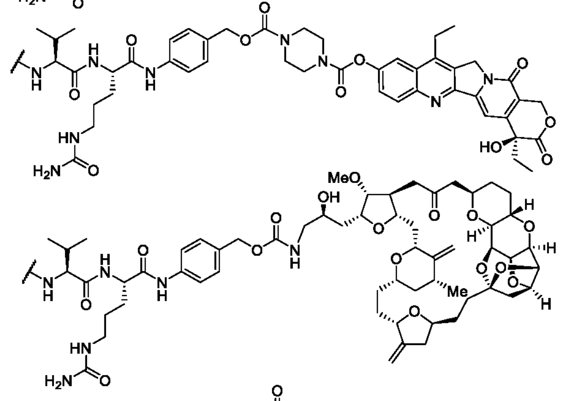

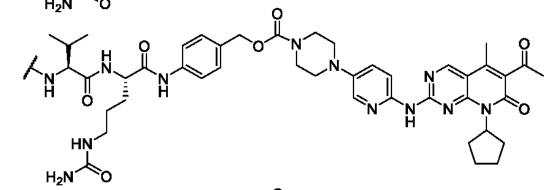

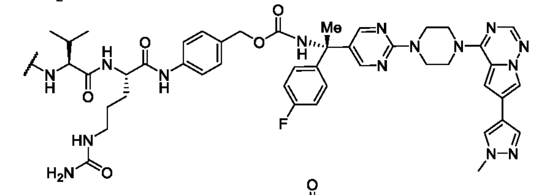

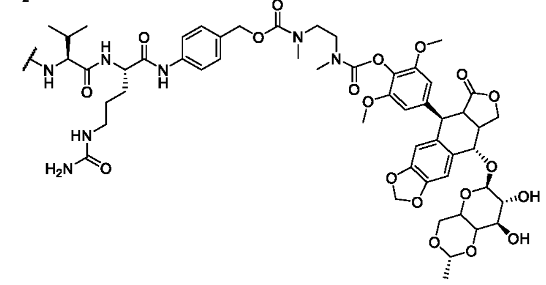

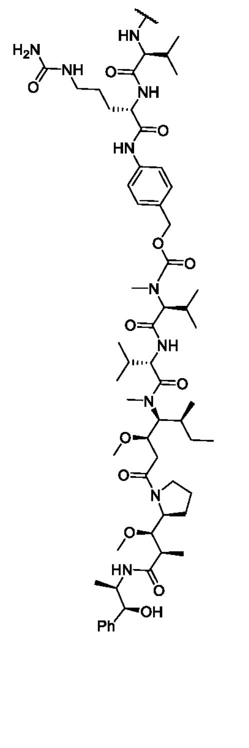

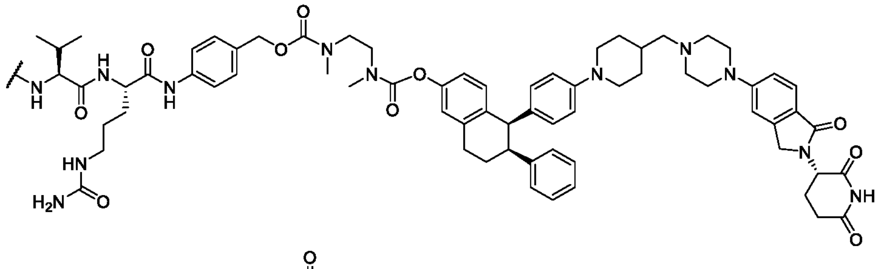

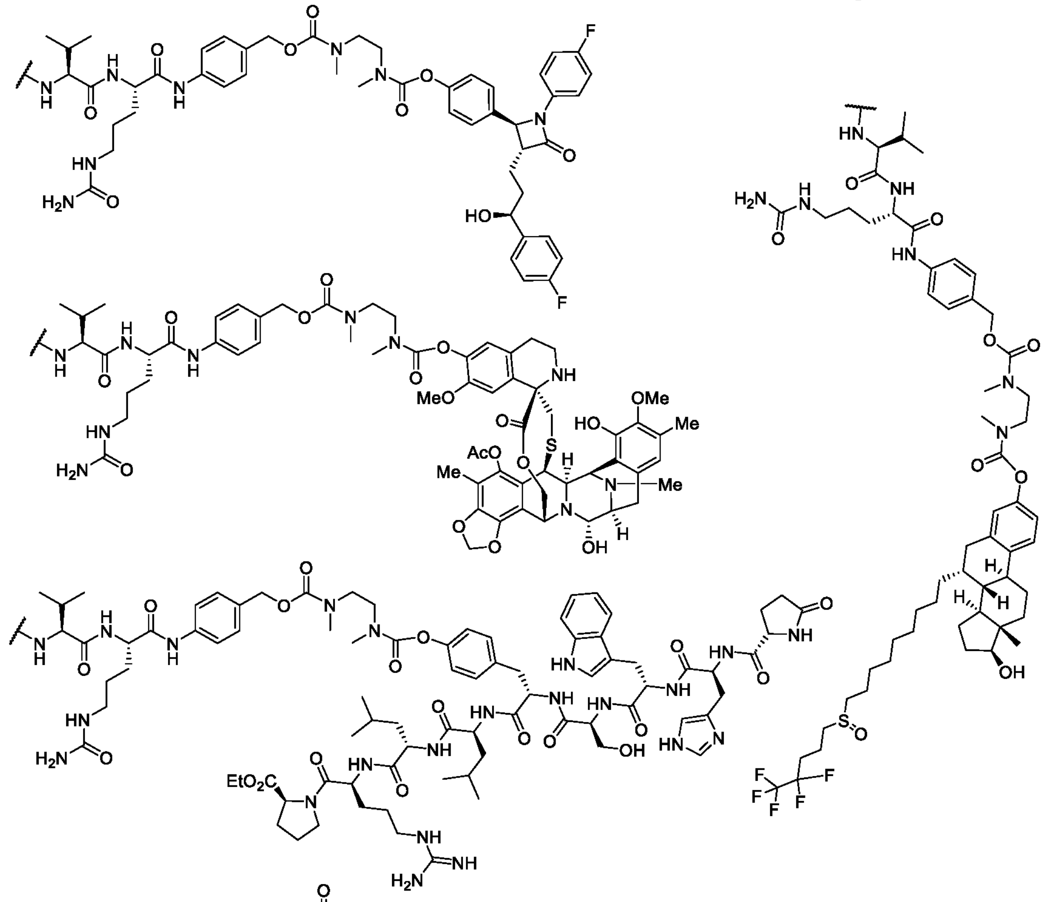

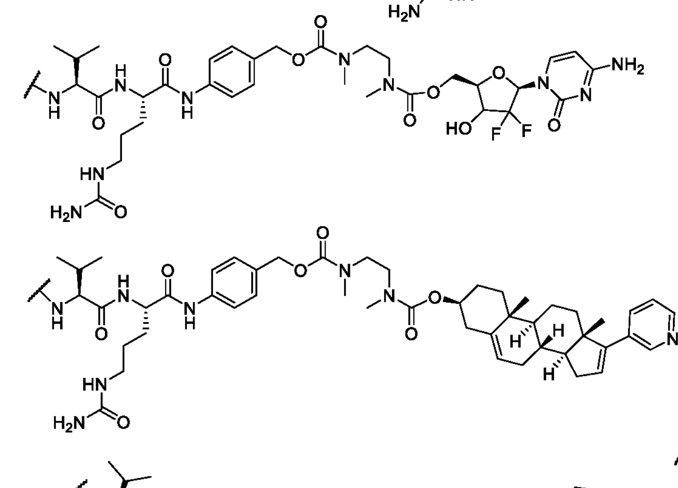

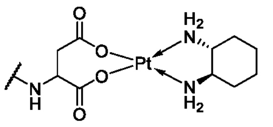

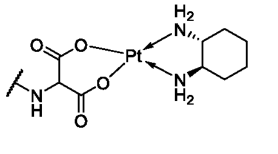

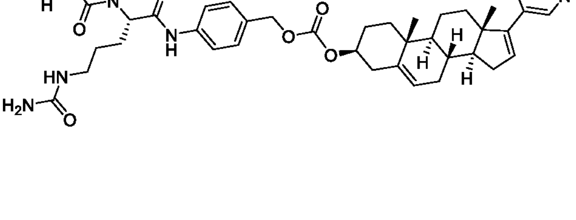

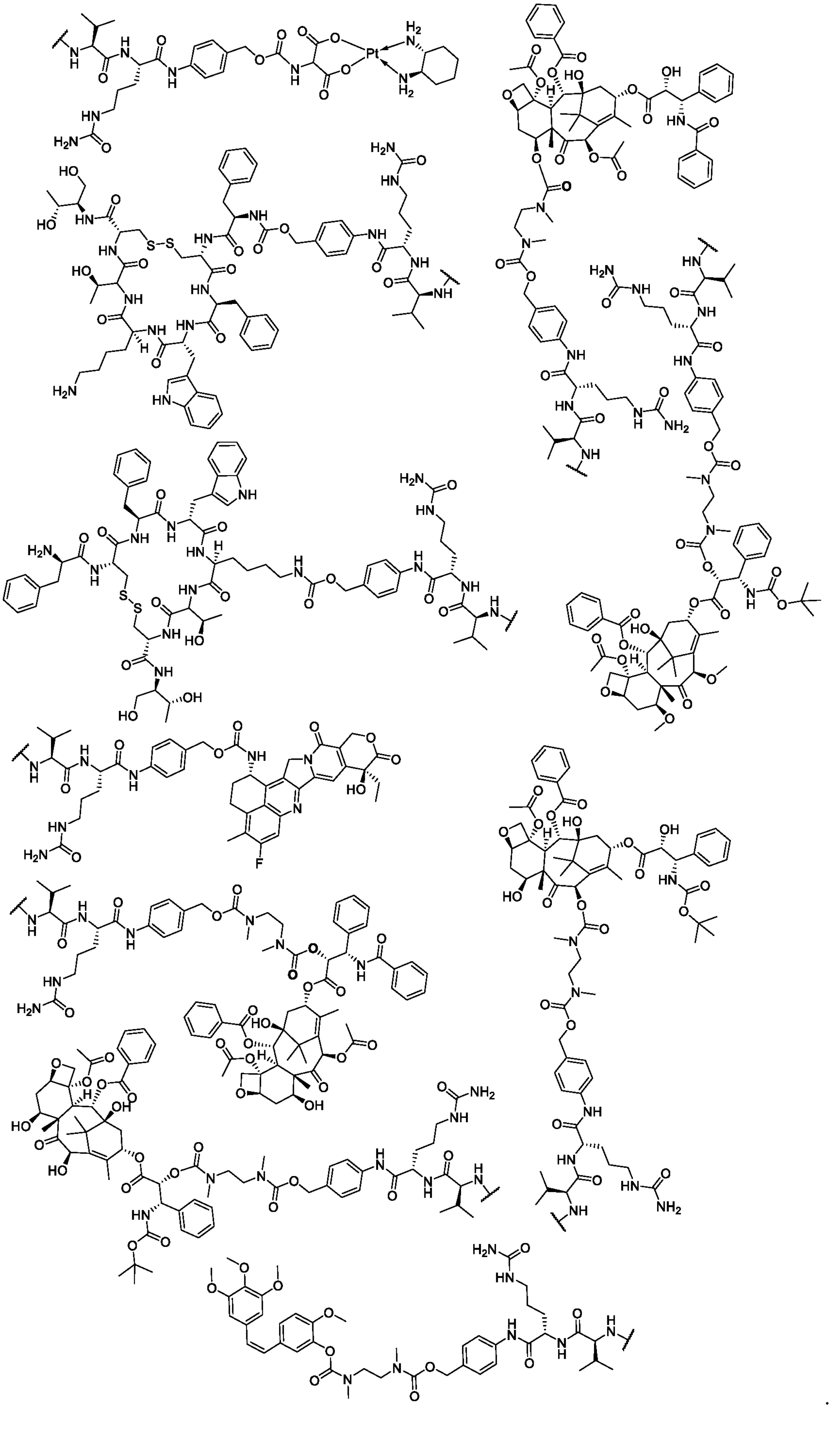

In some embodiments, $-L^2-D$ is any of the following structures:

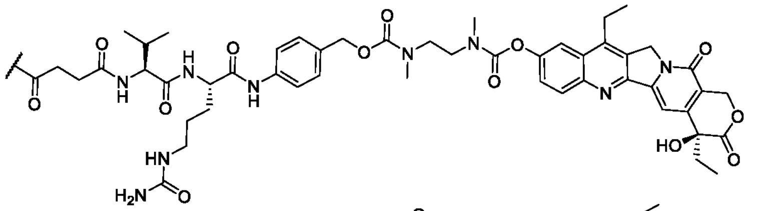

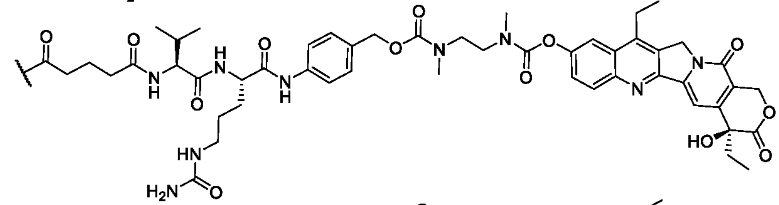

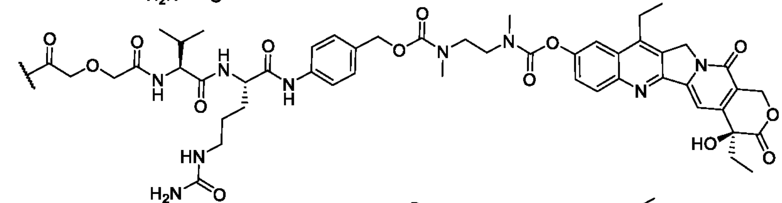

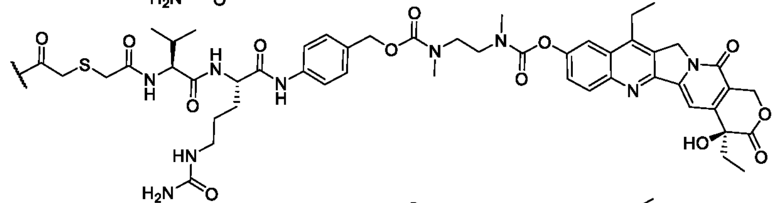

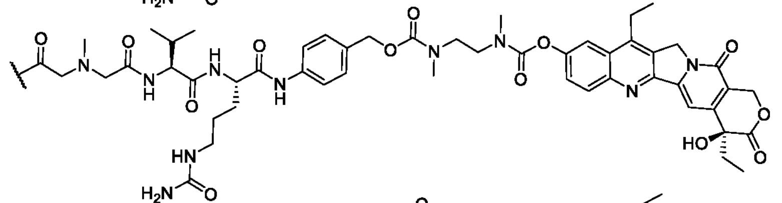

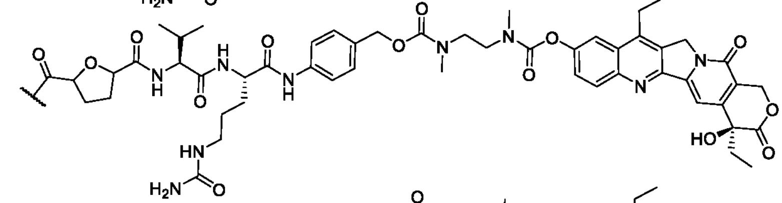

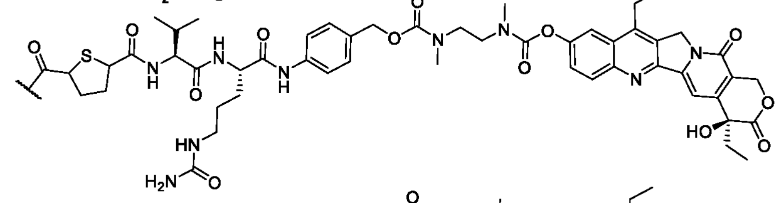

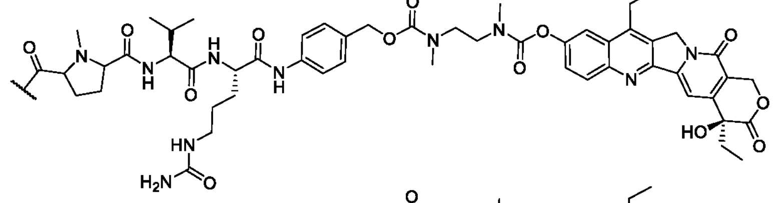

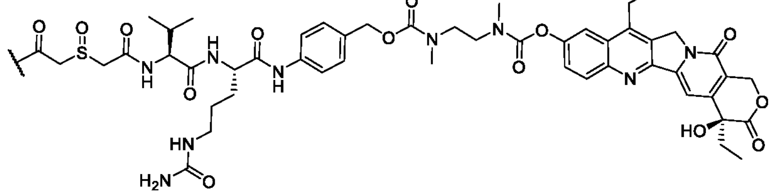

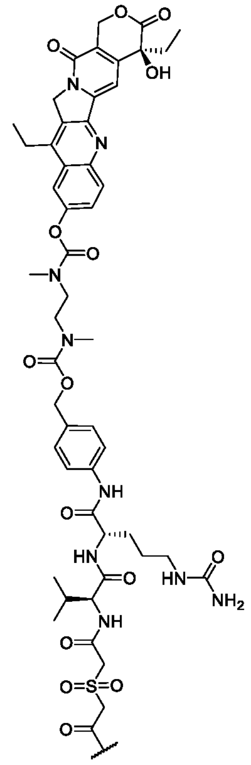

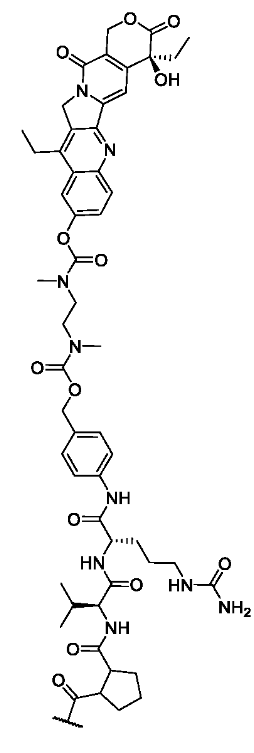

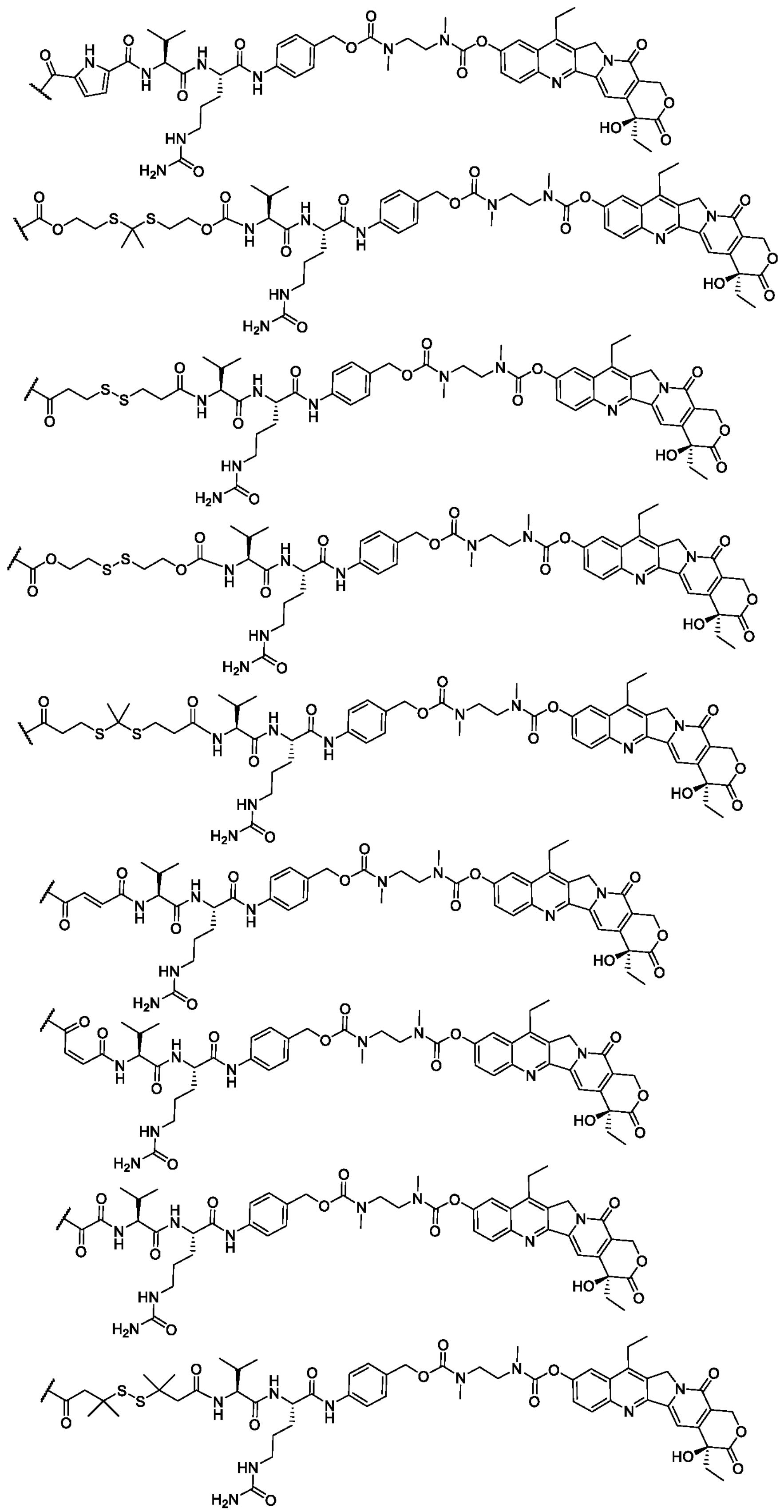

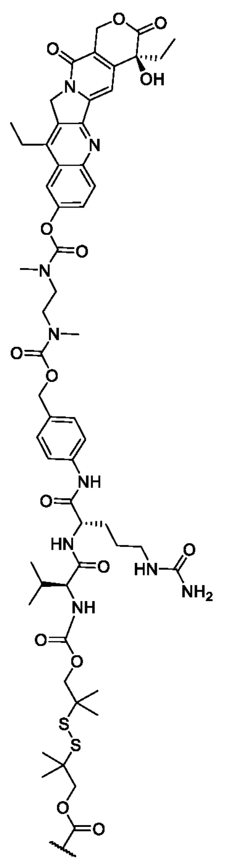

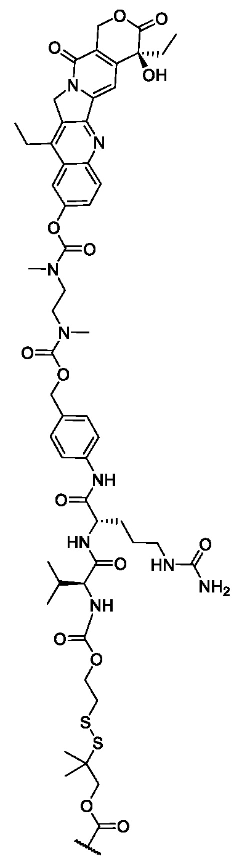

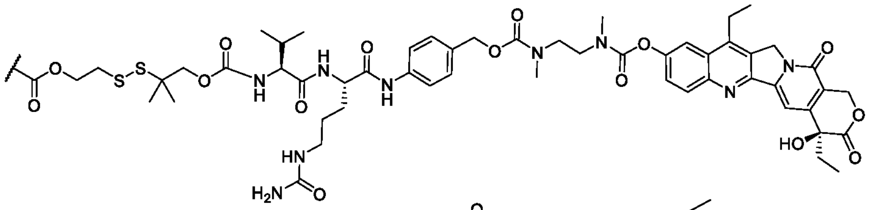

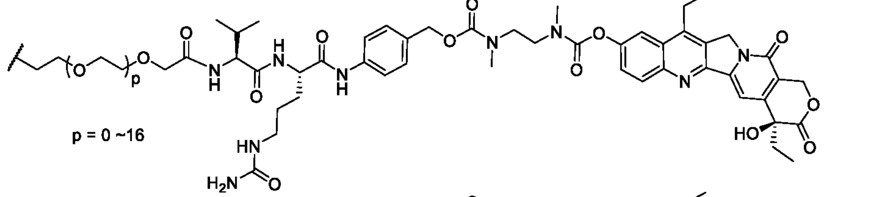

p = 0 ~16

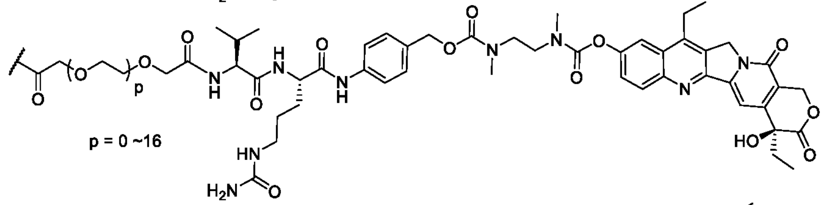

p = 0 ~16

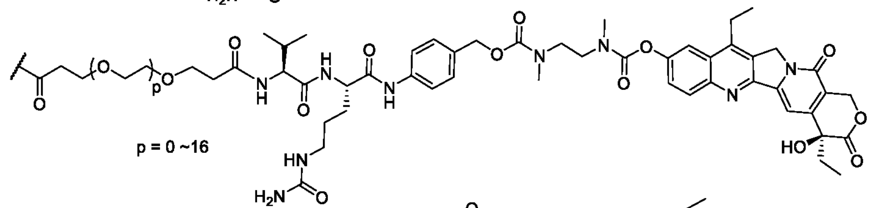

p = 0 ~16

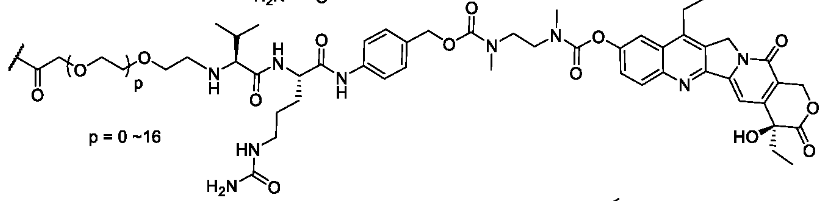

p = 0 ~16

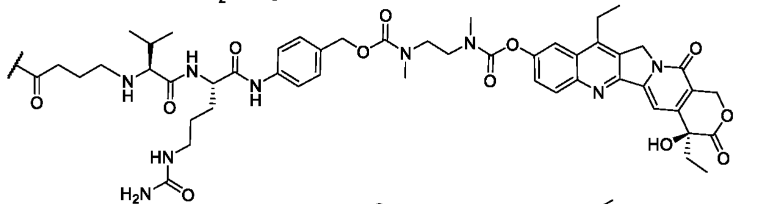

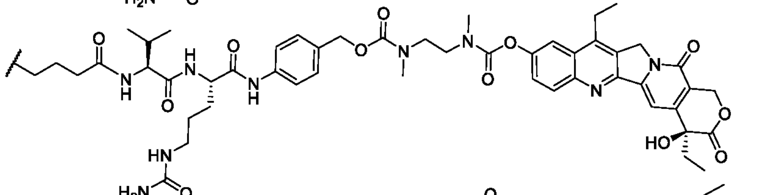

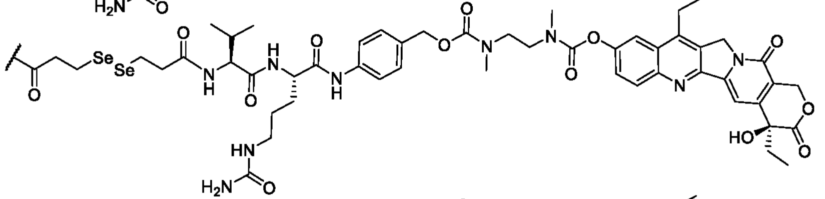

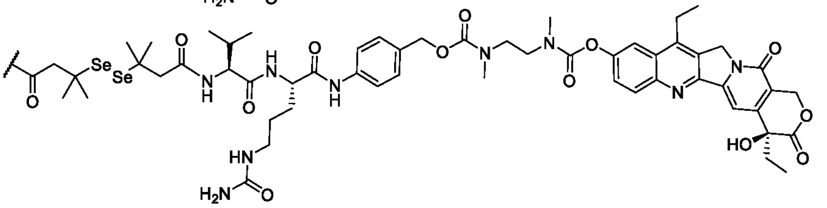

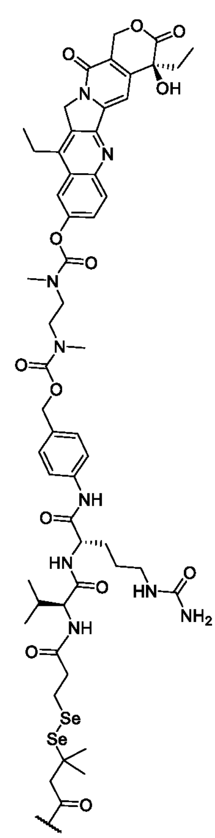

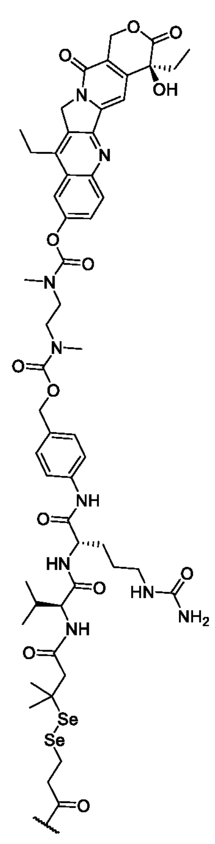

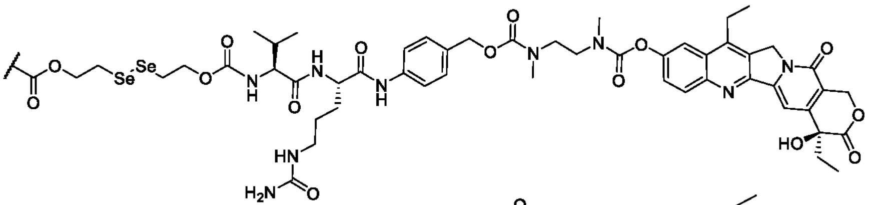

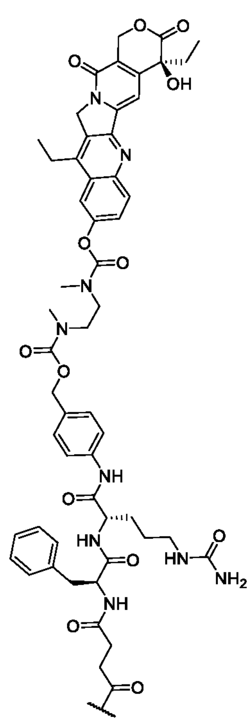

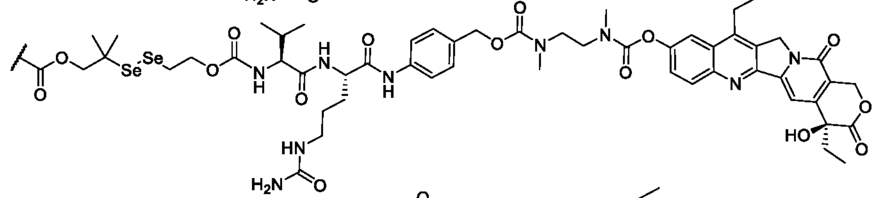

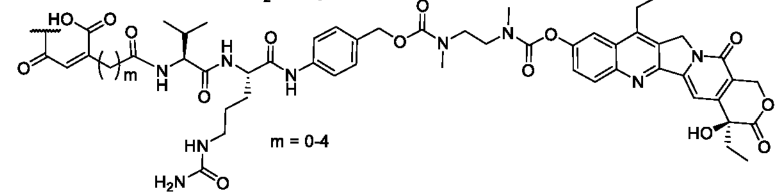

m = 0-4

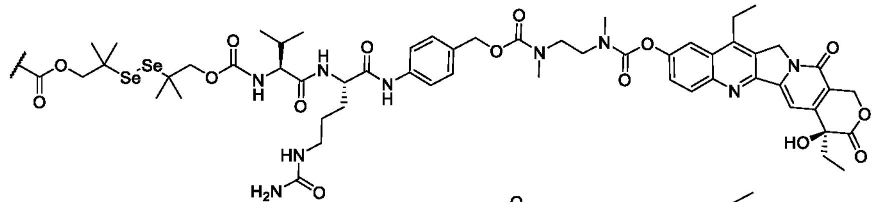

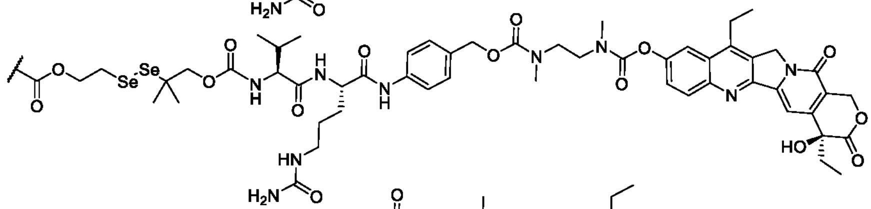

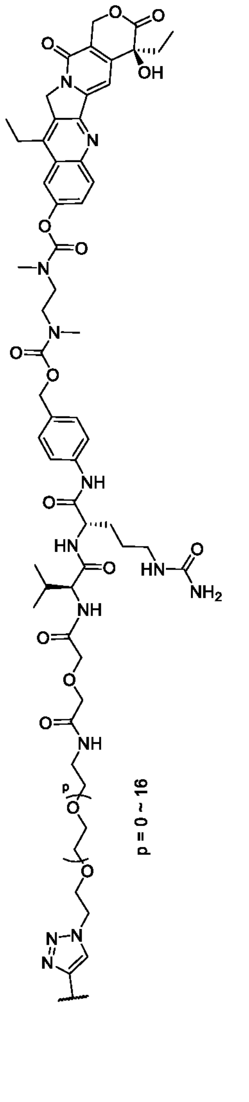

p = 0 ~ 16

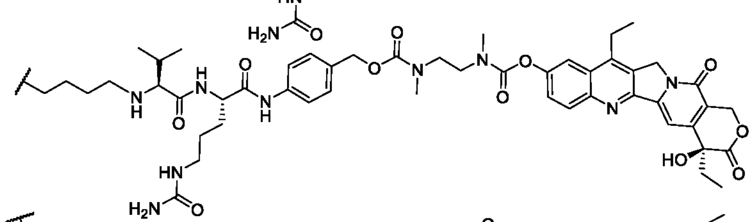

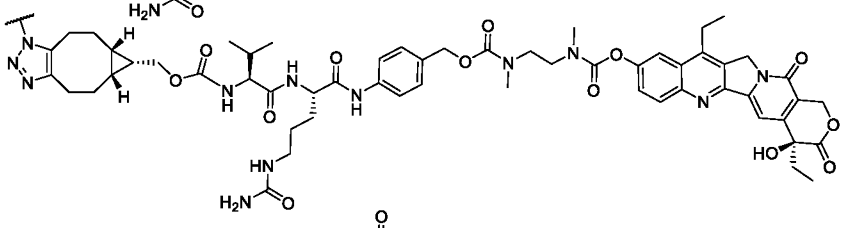

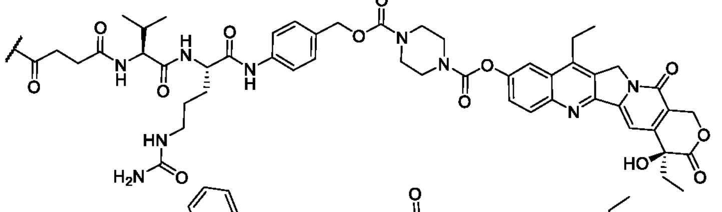

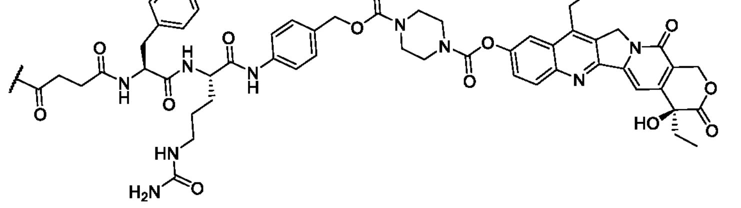

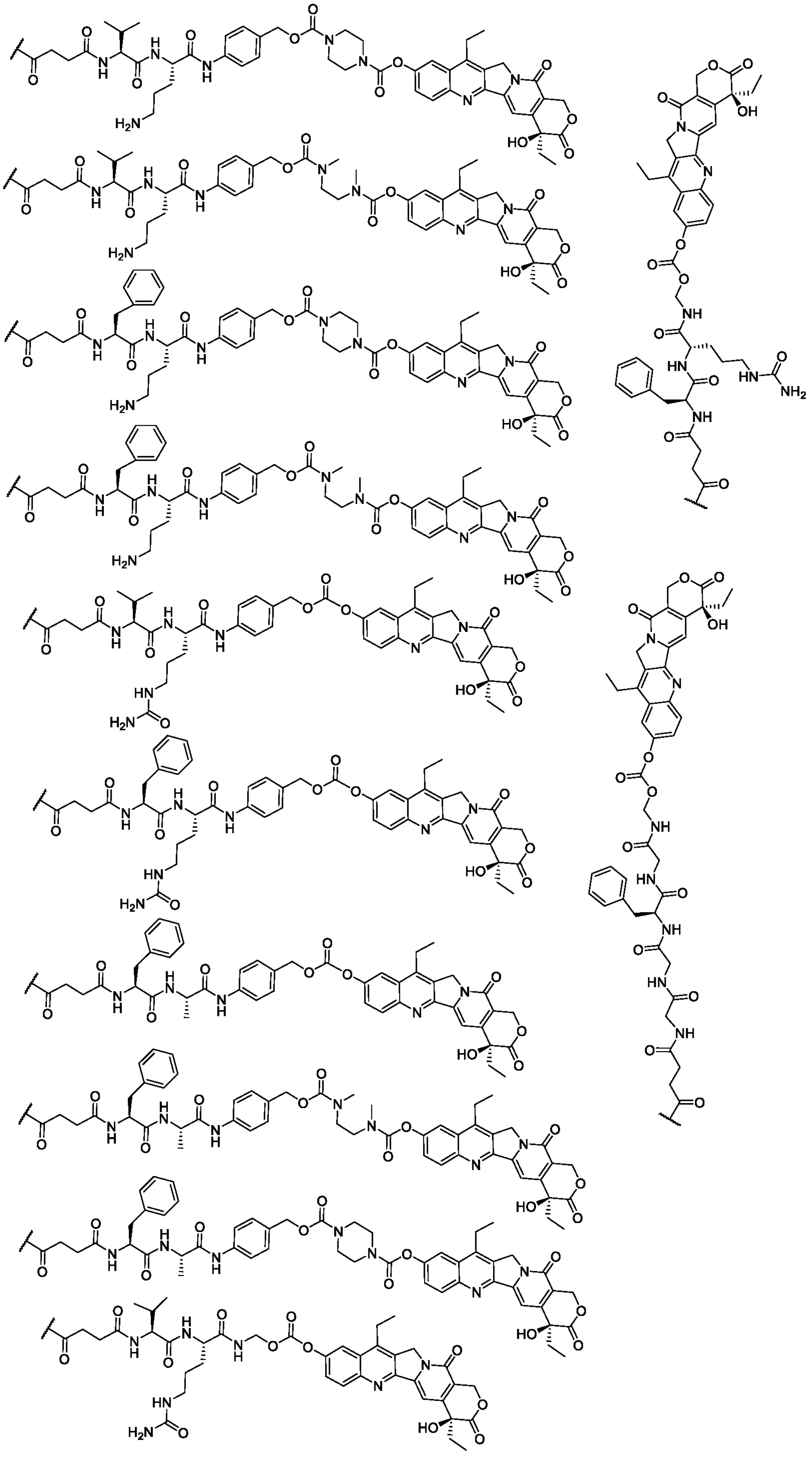

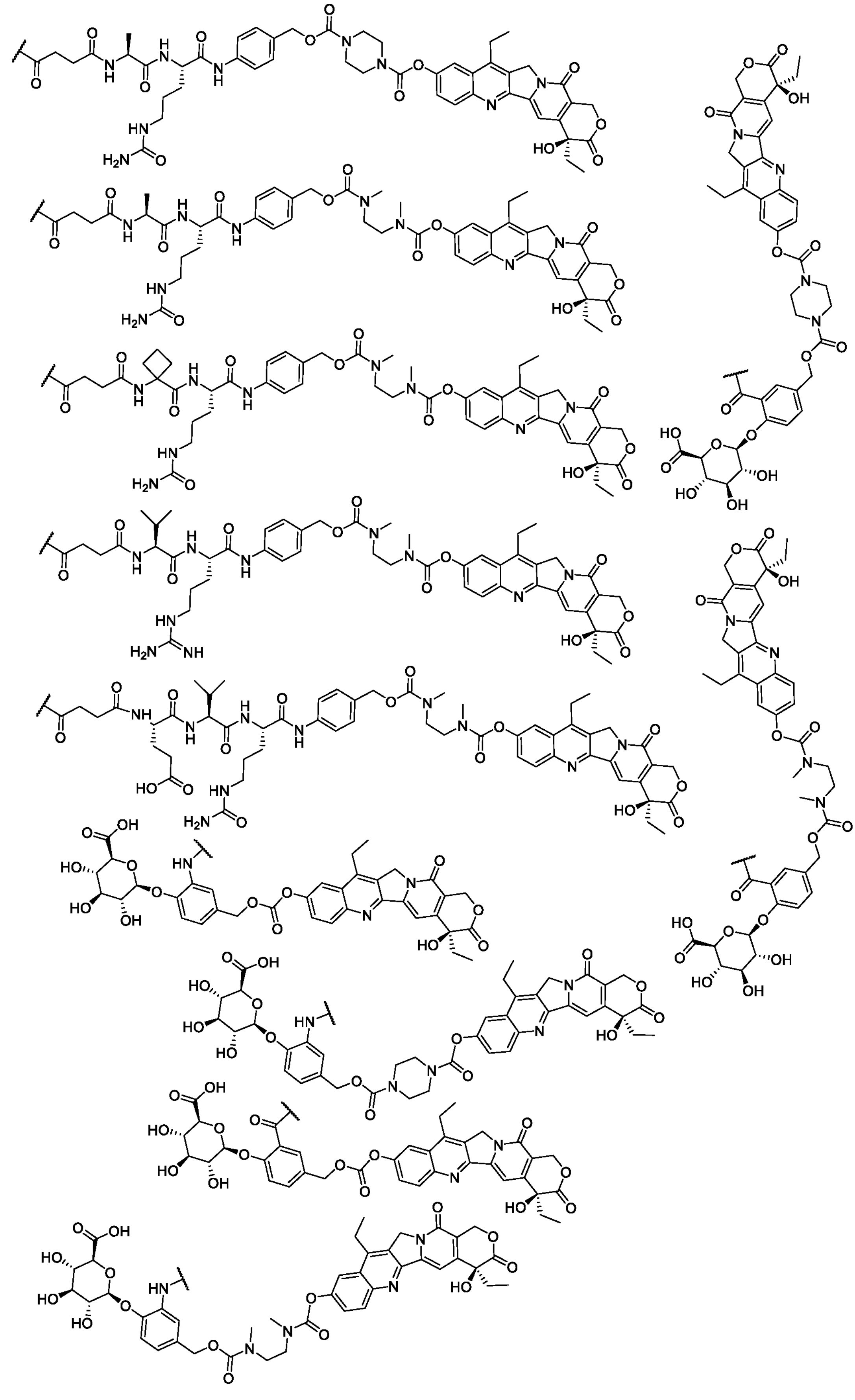

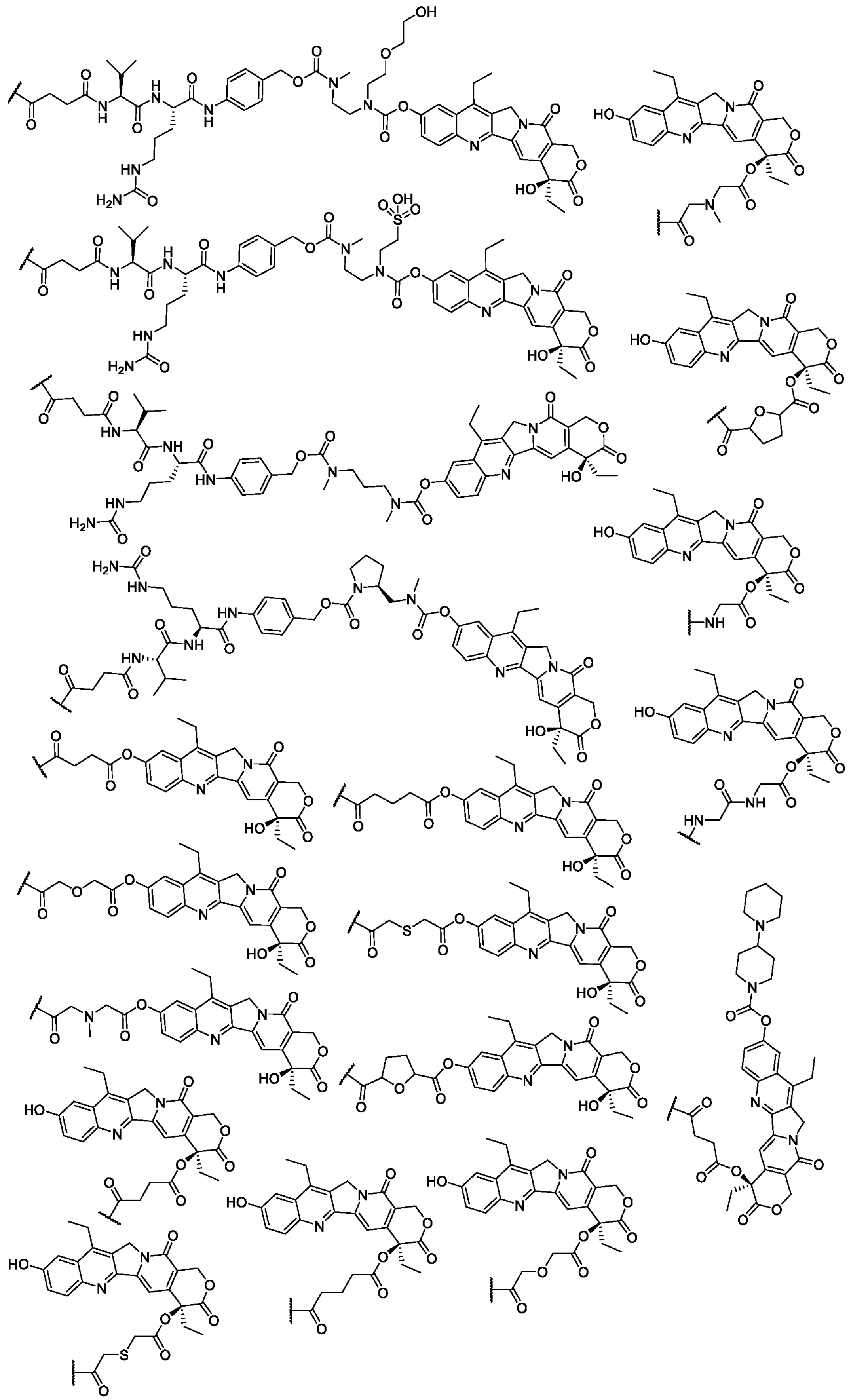

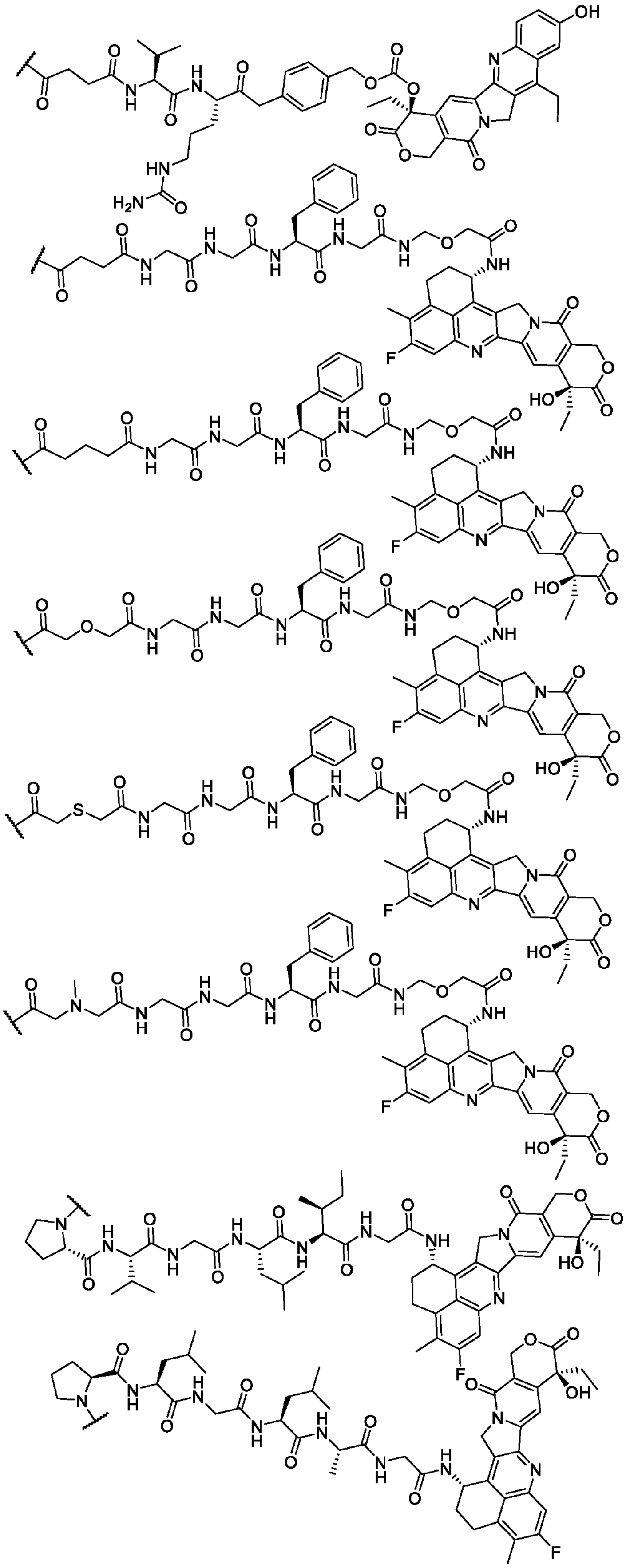

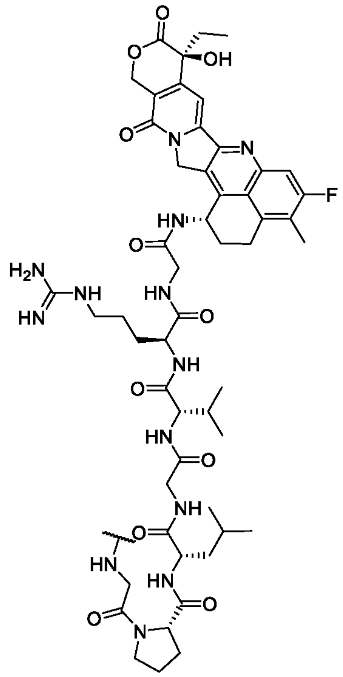

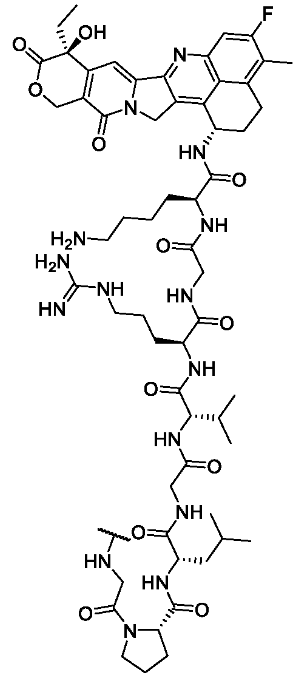

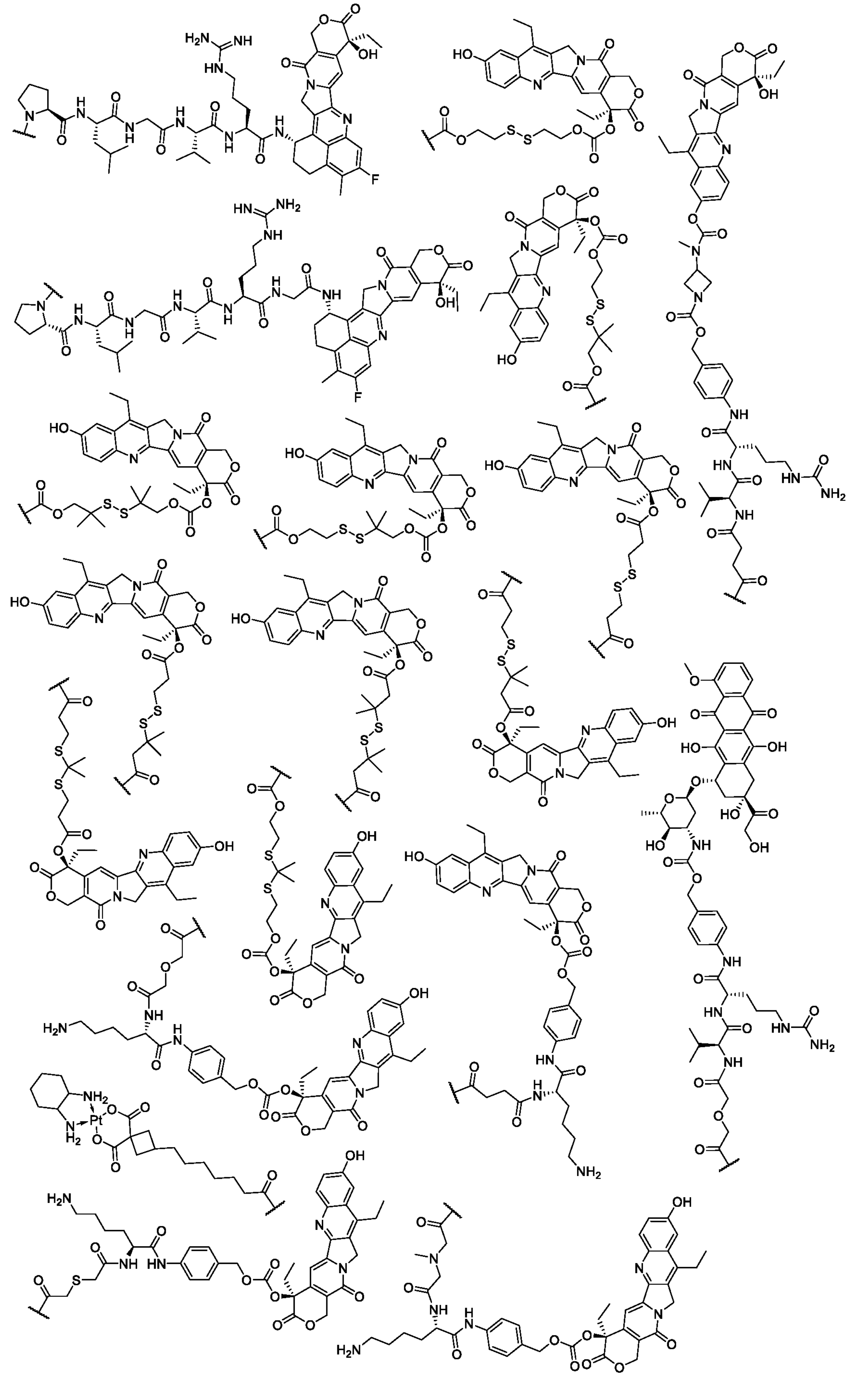

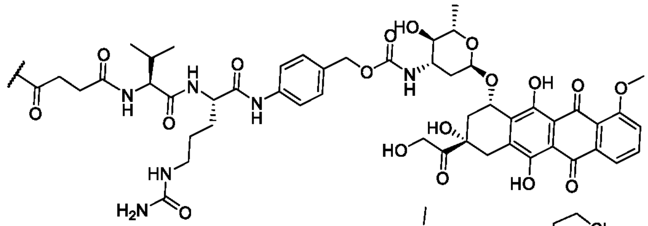

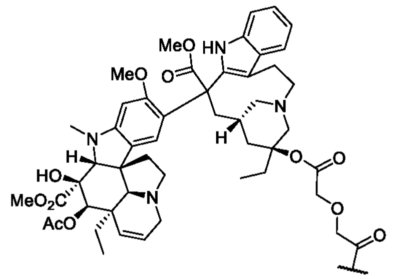

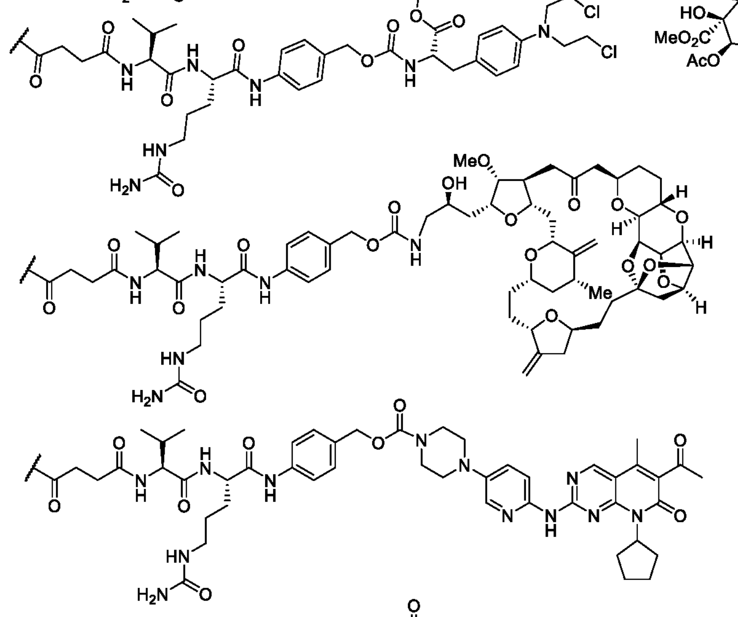

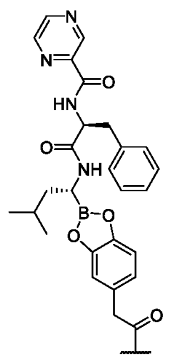

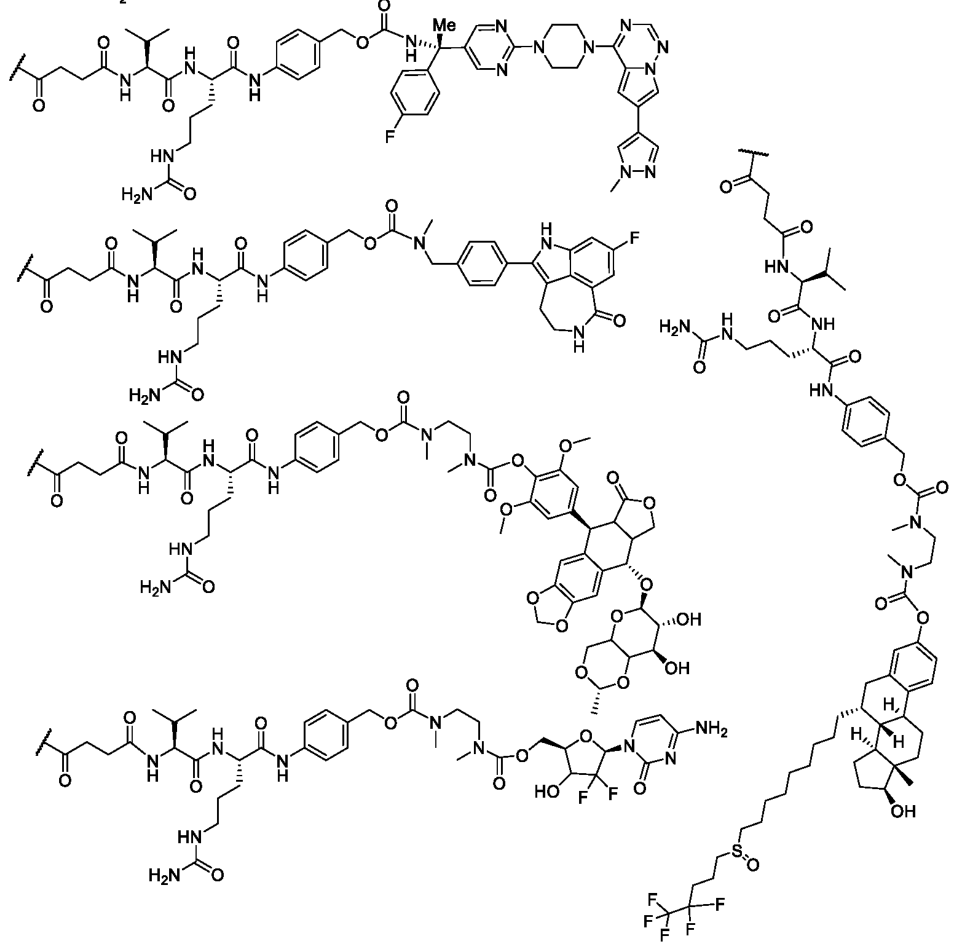

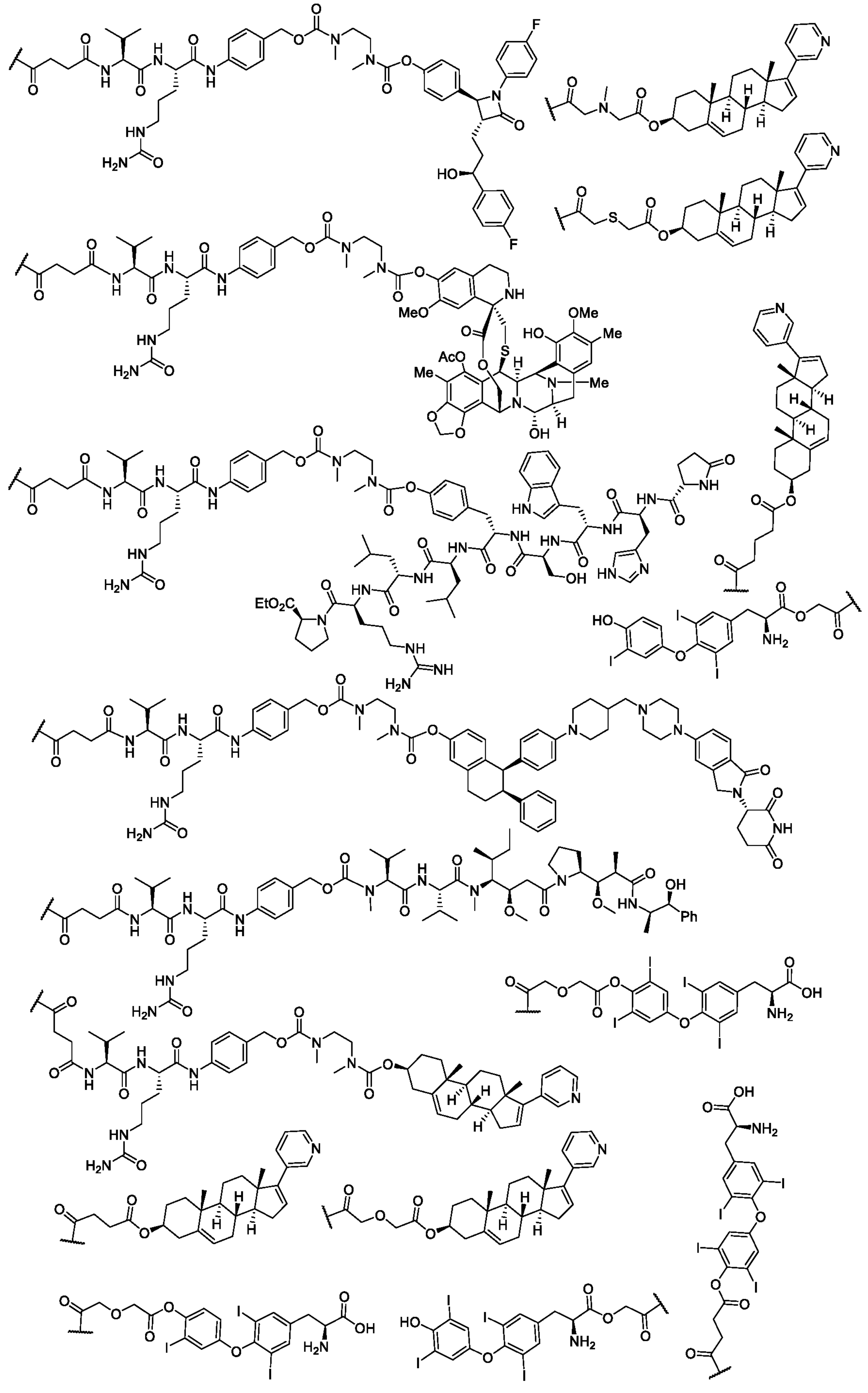

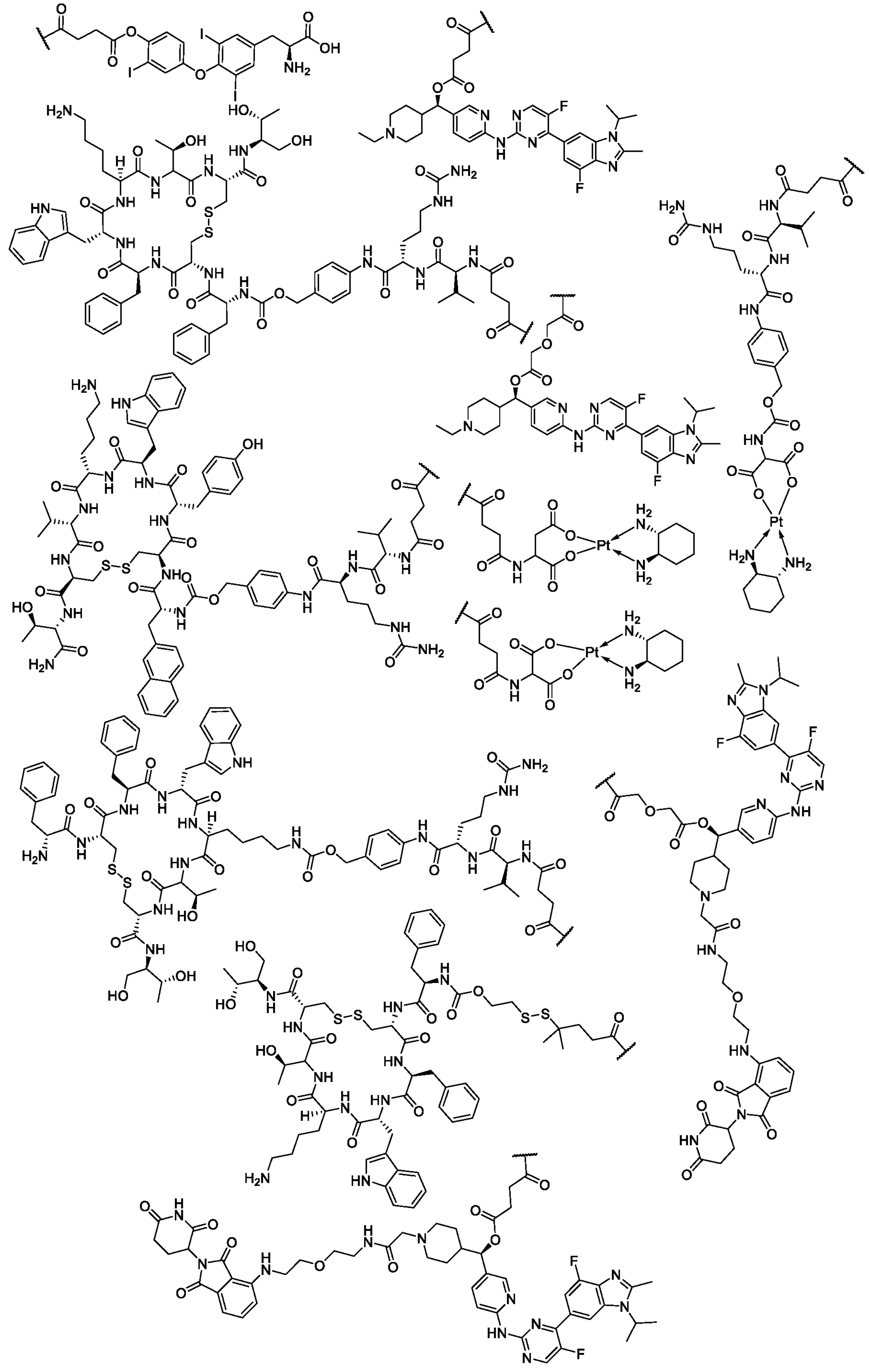

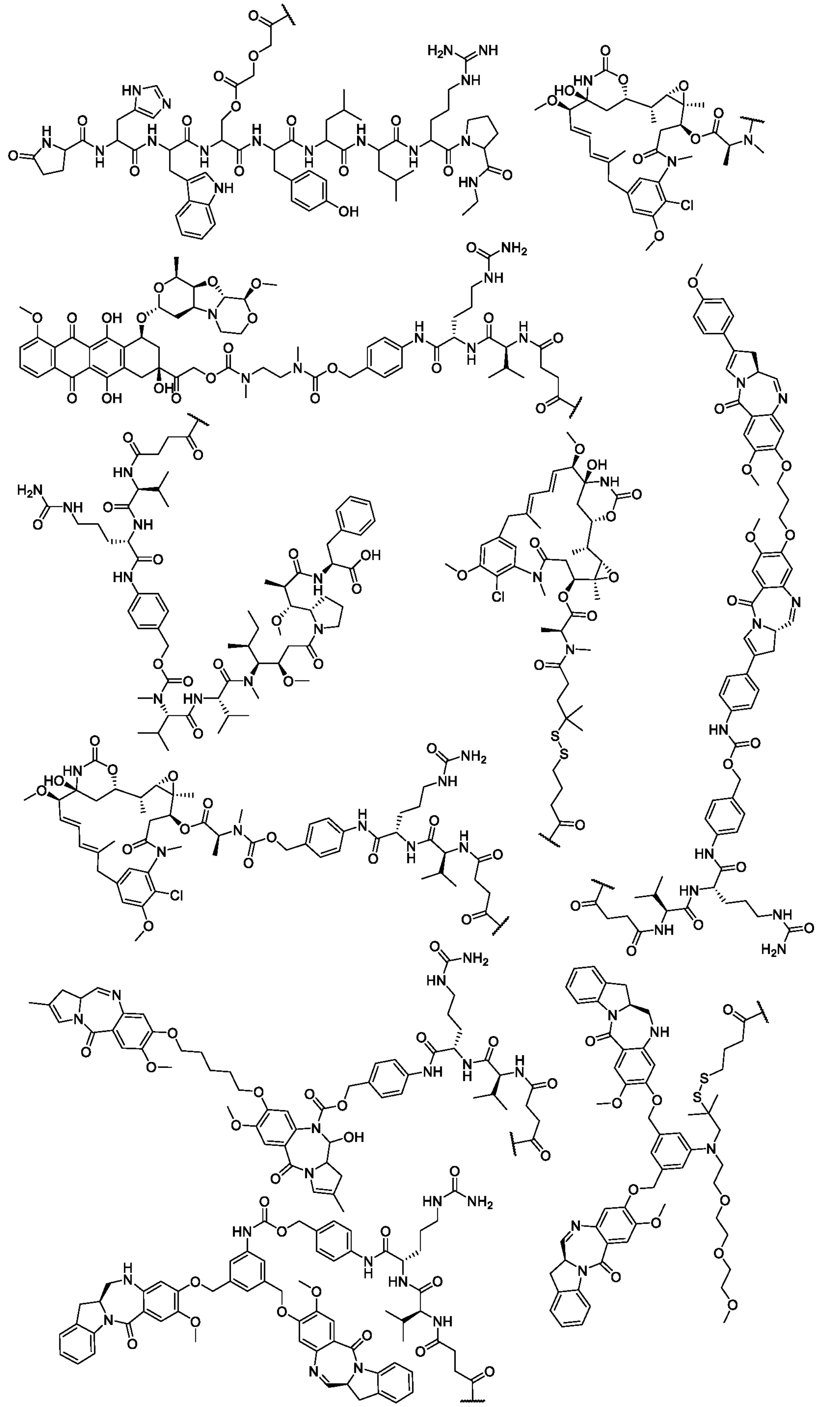

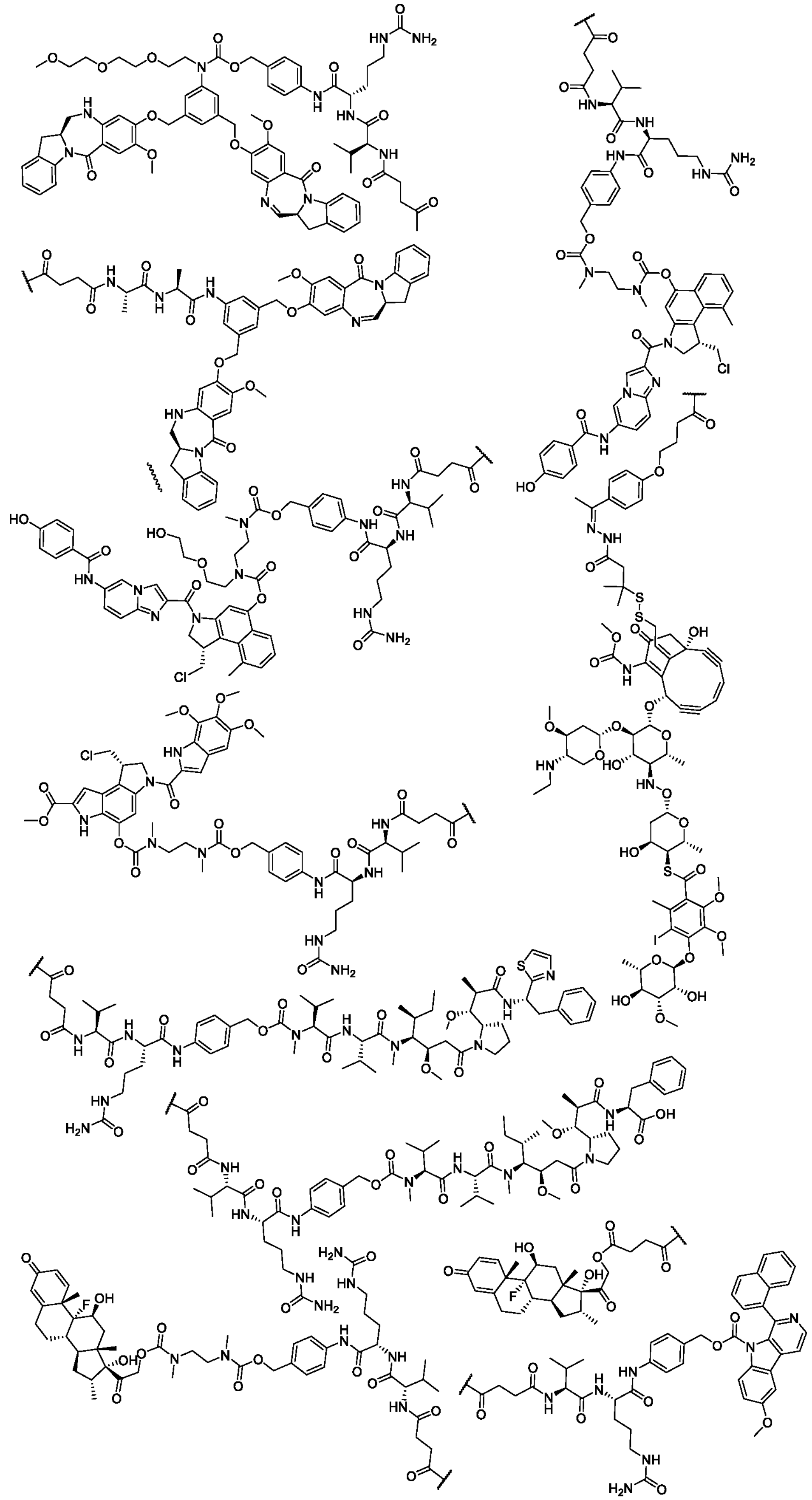

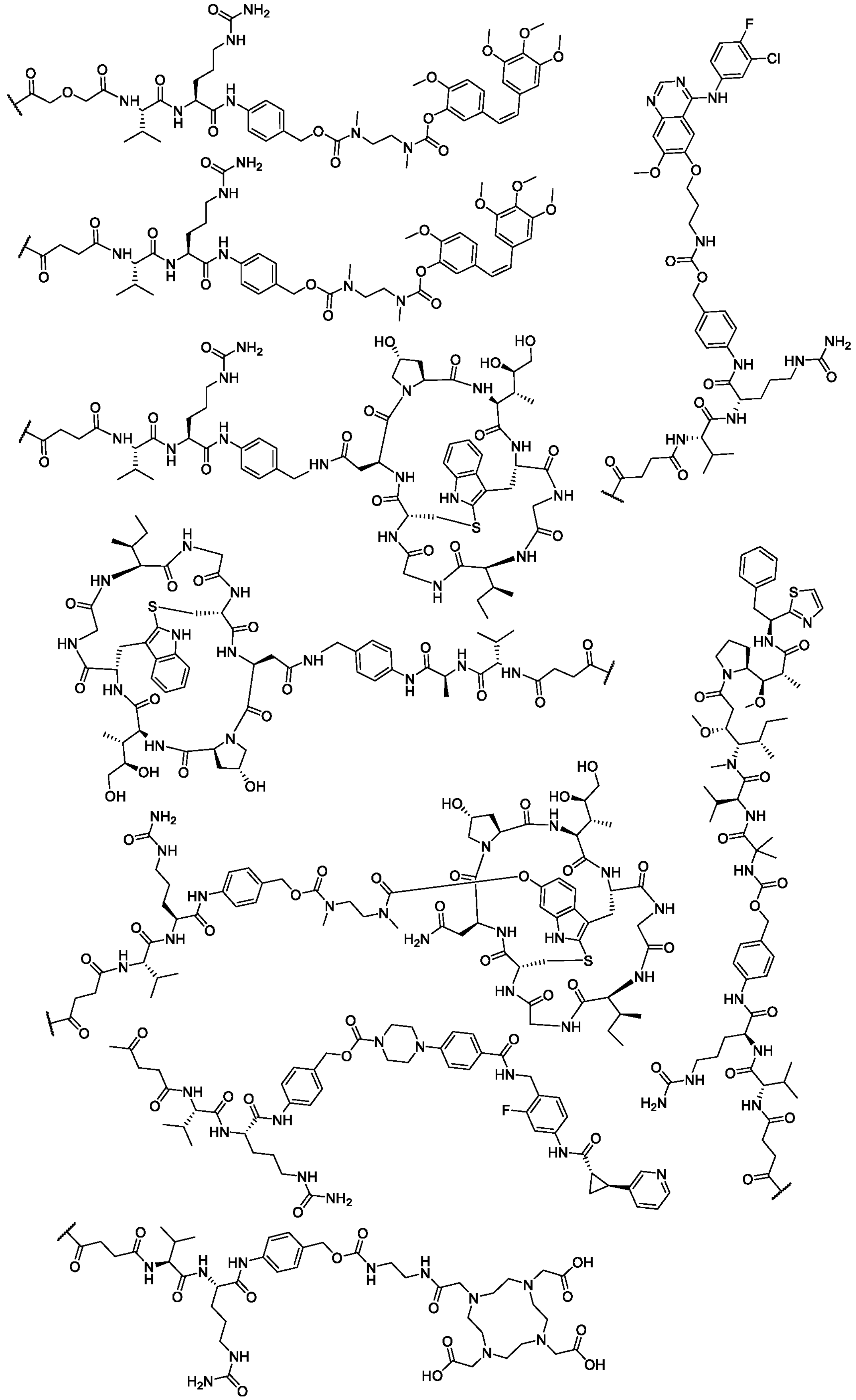

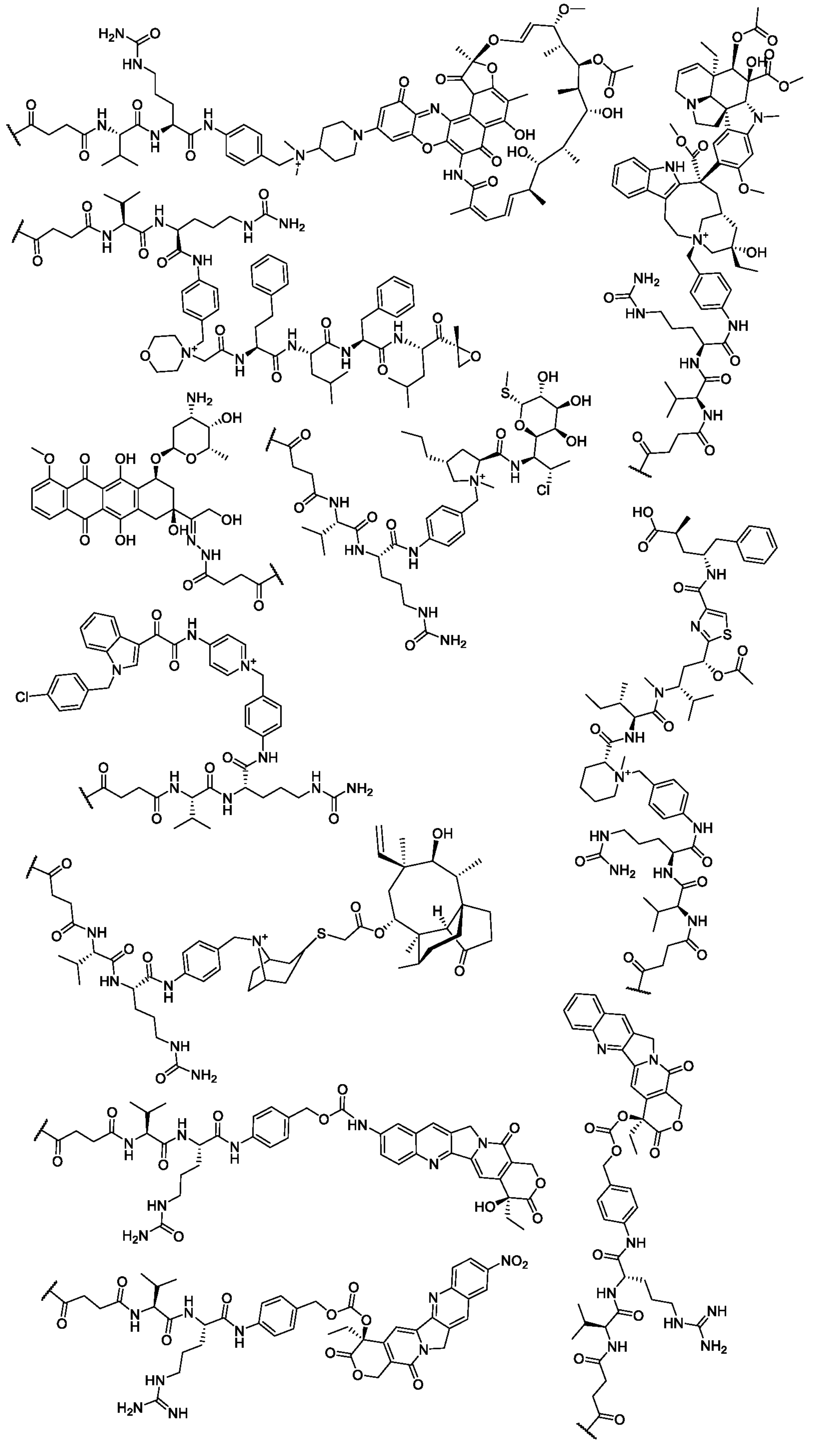

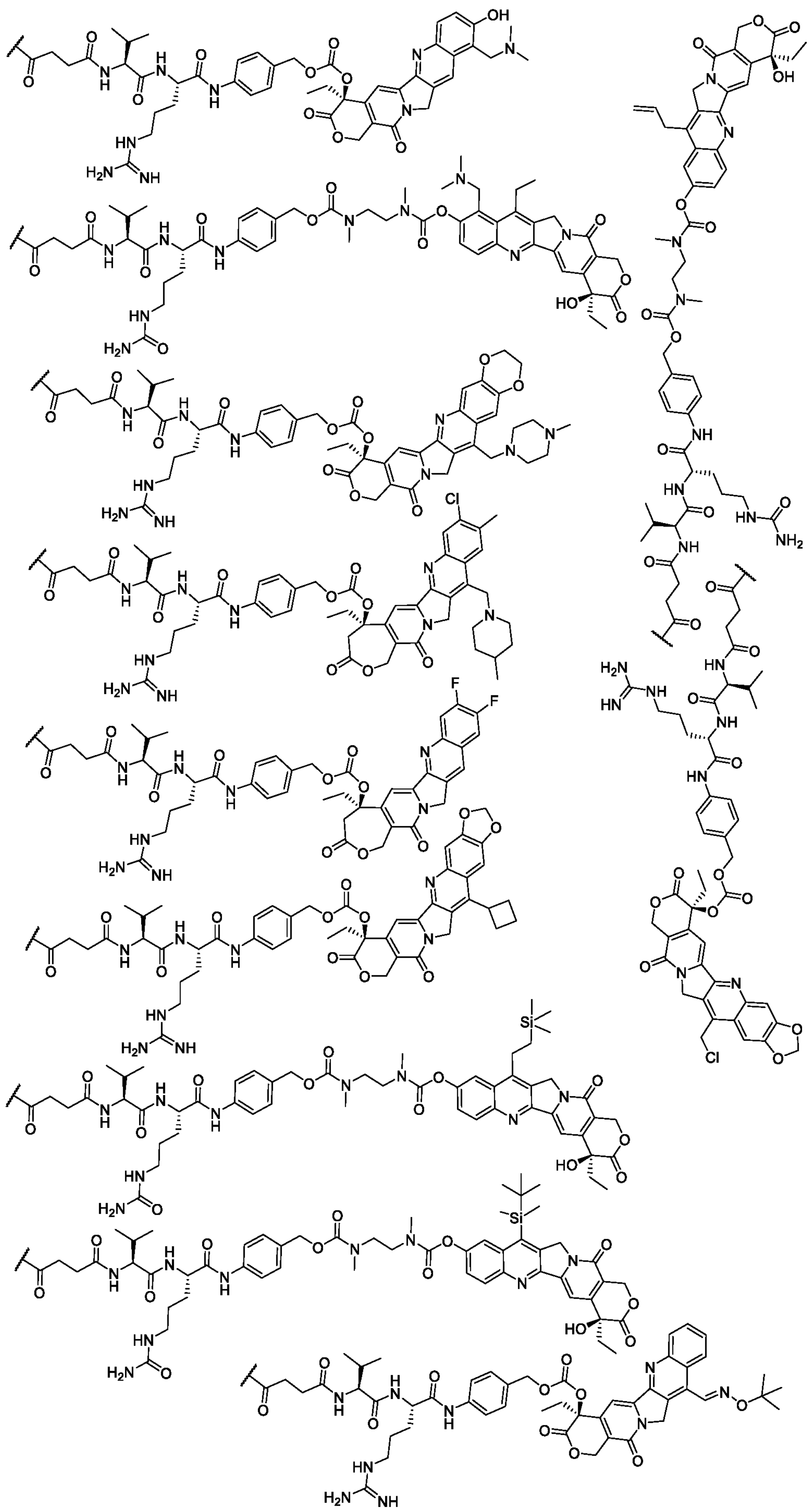

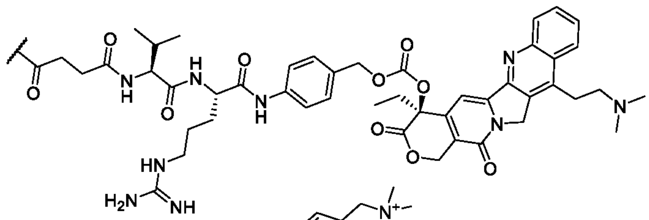

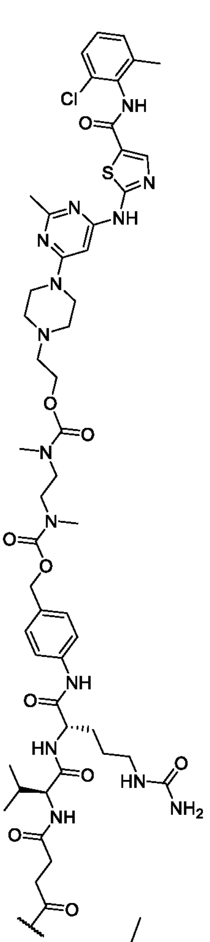

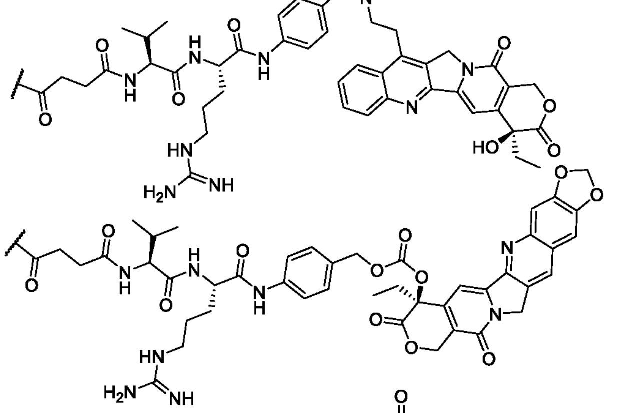

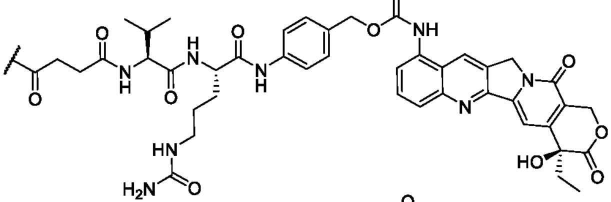

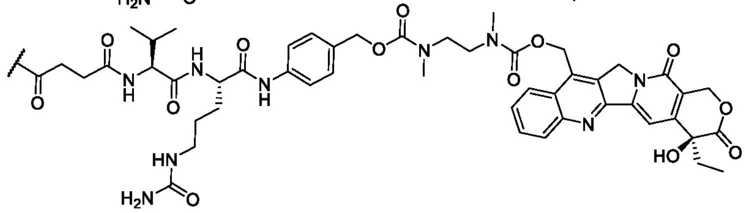

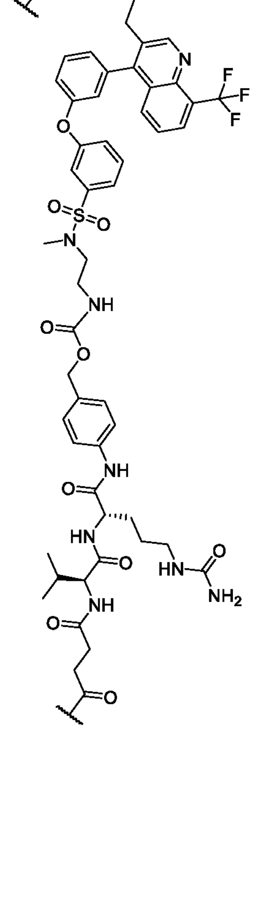

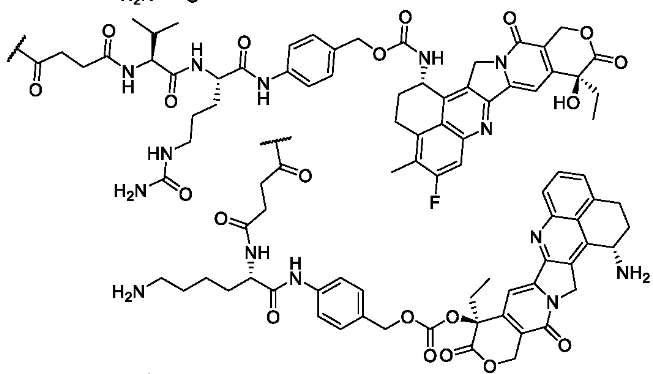

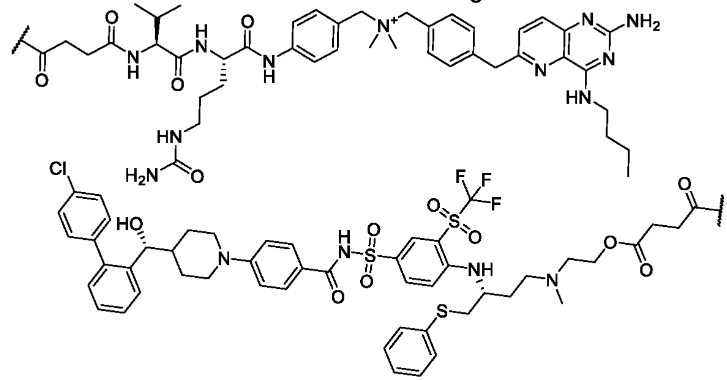

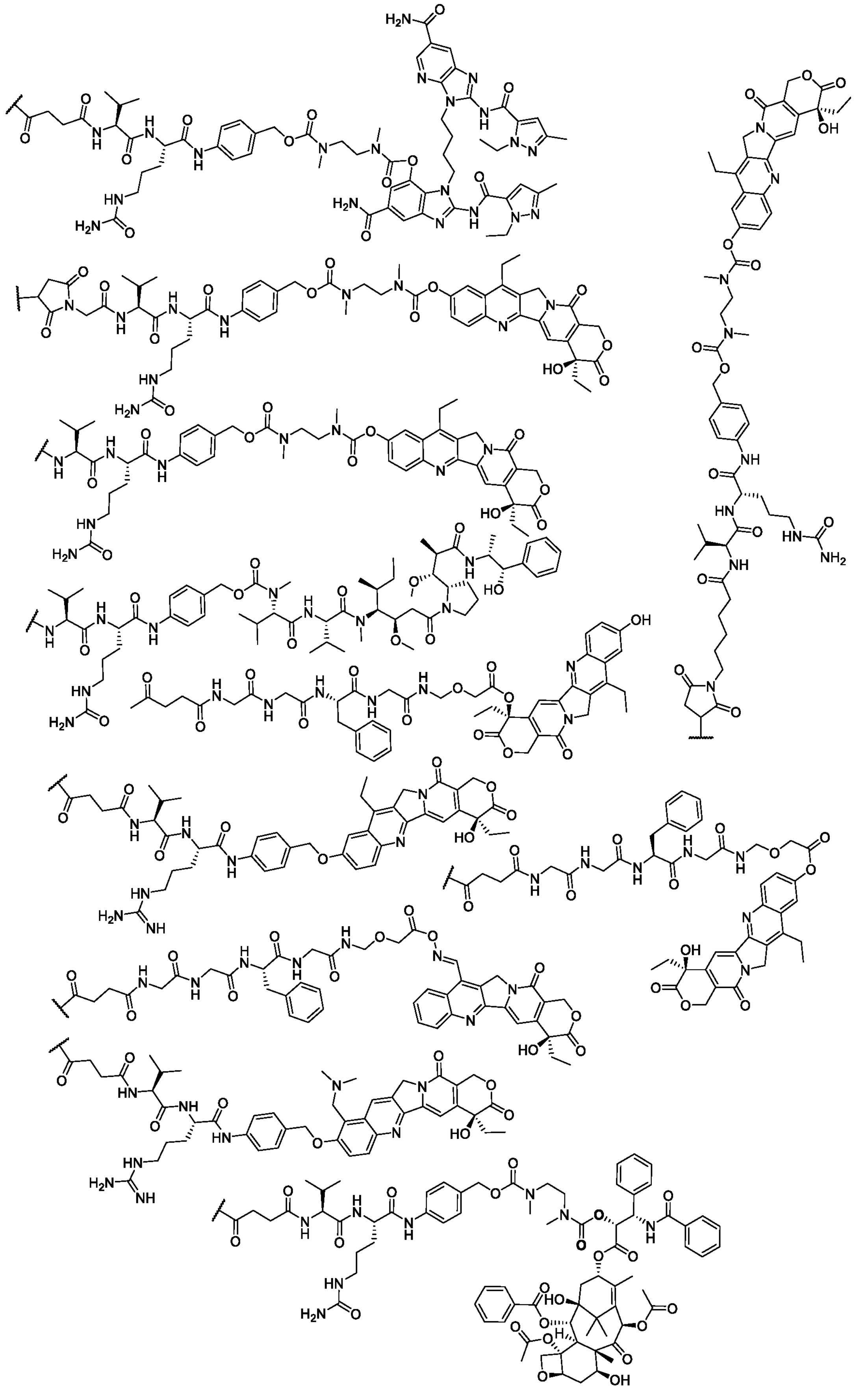

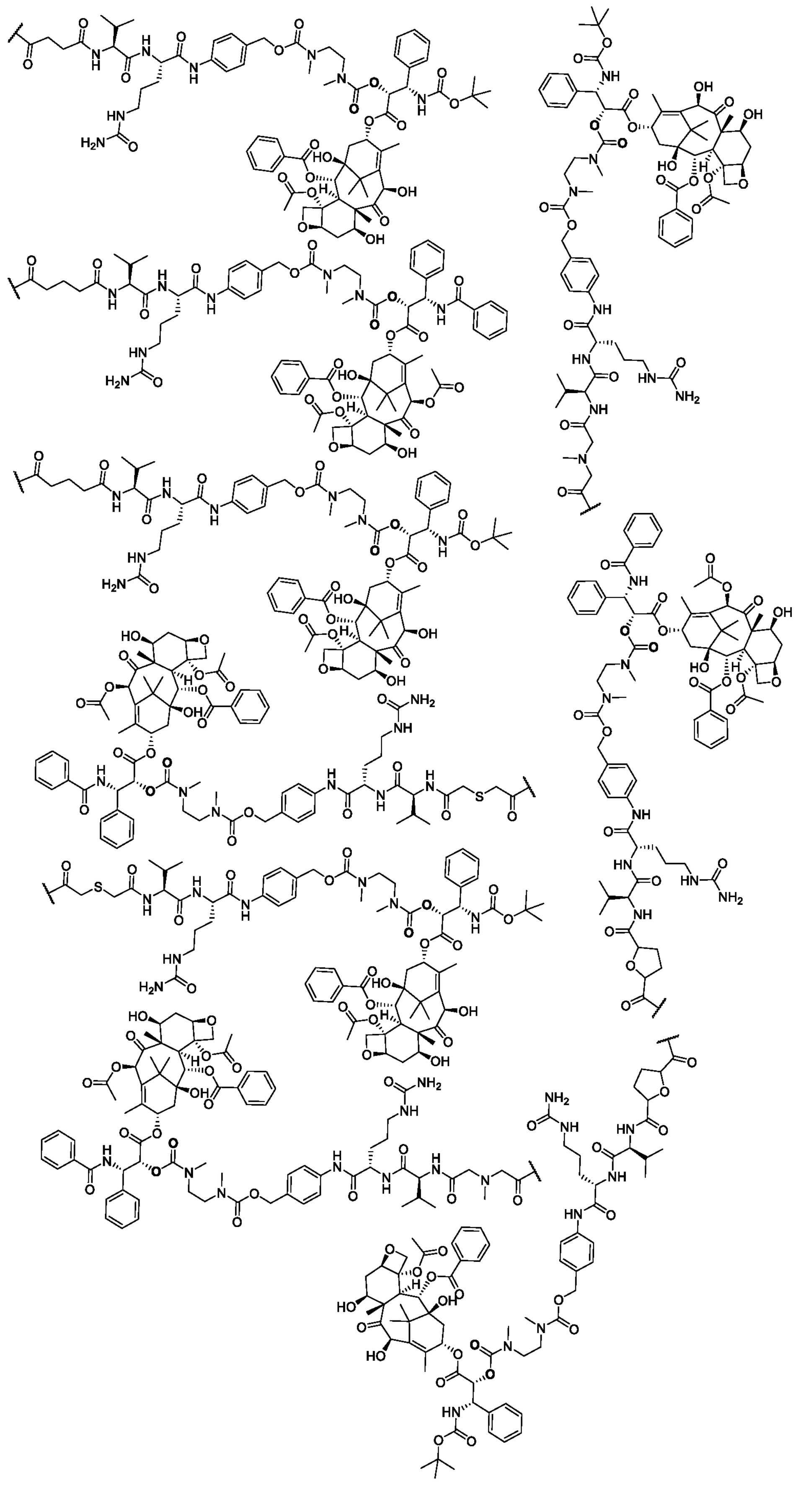

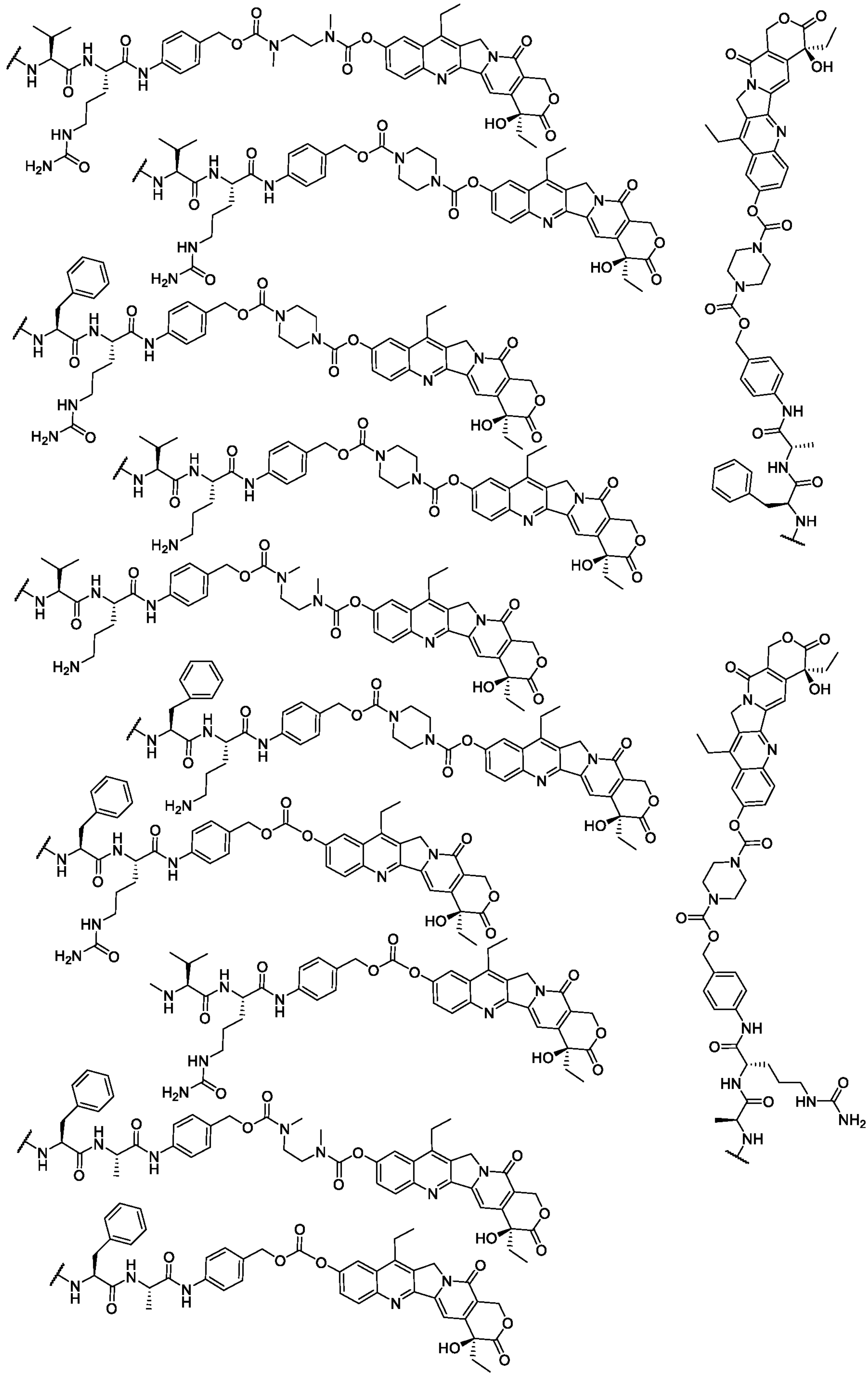

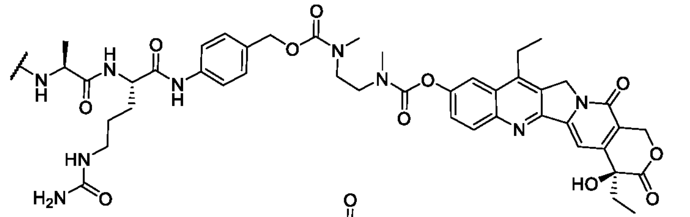

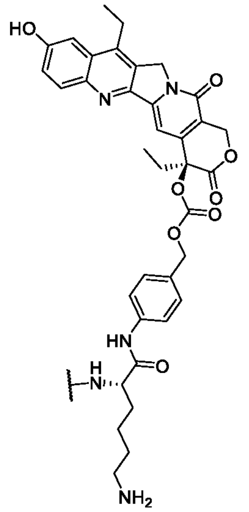

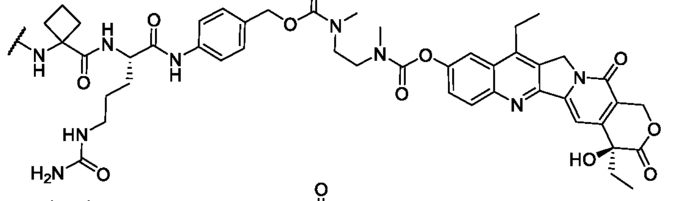

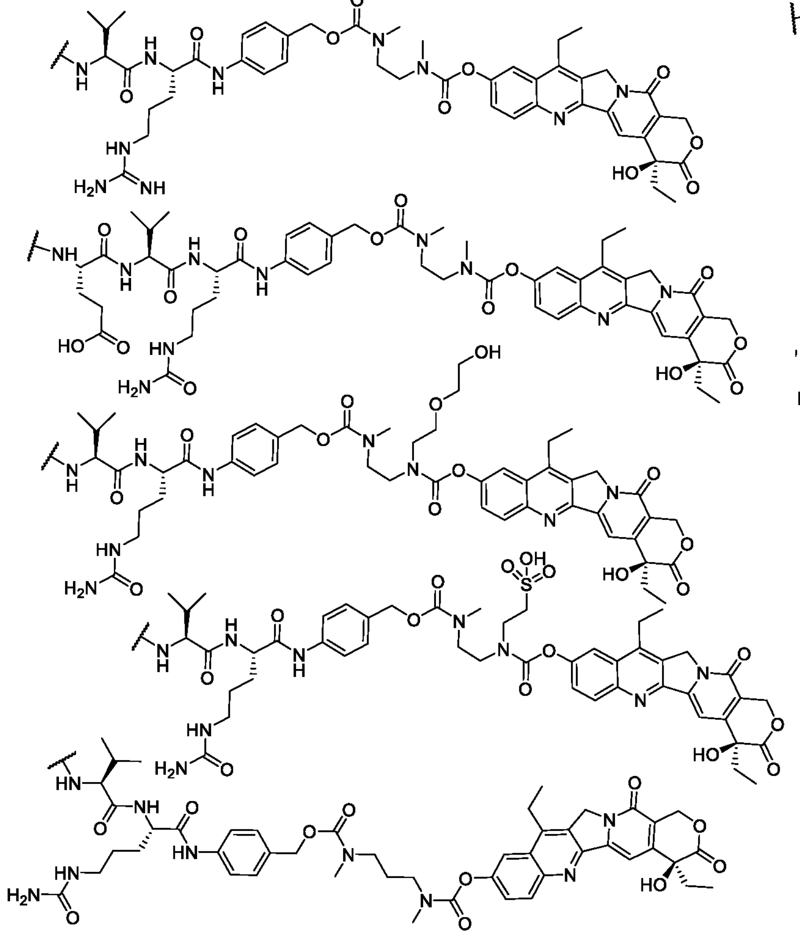

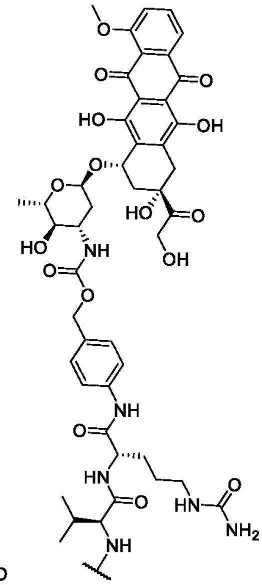

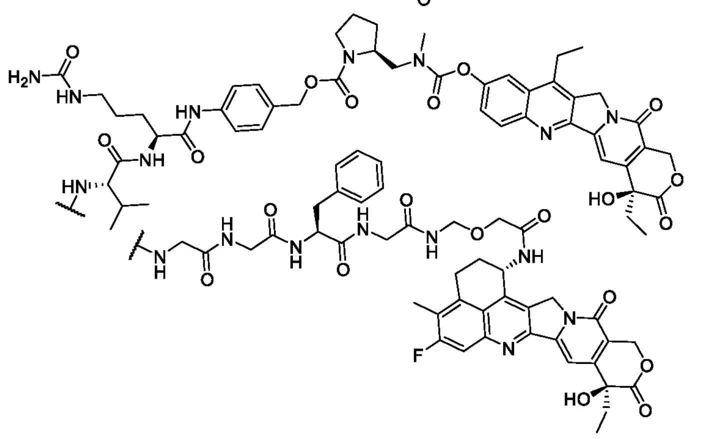

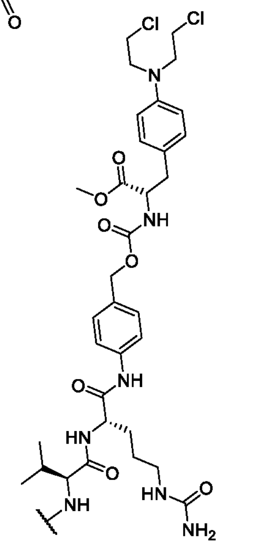

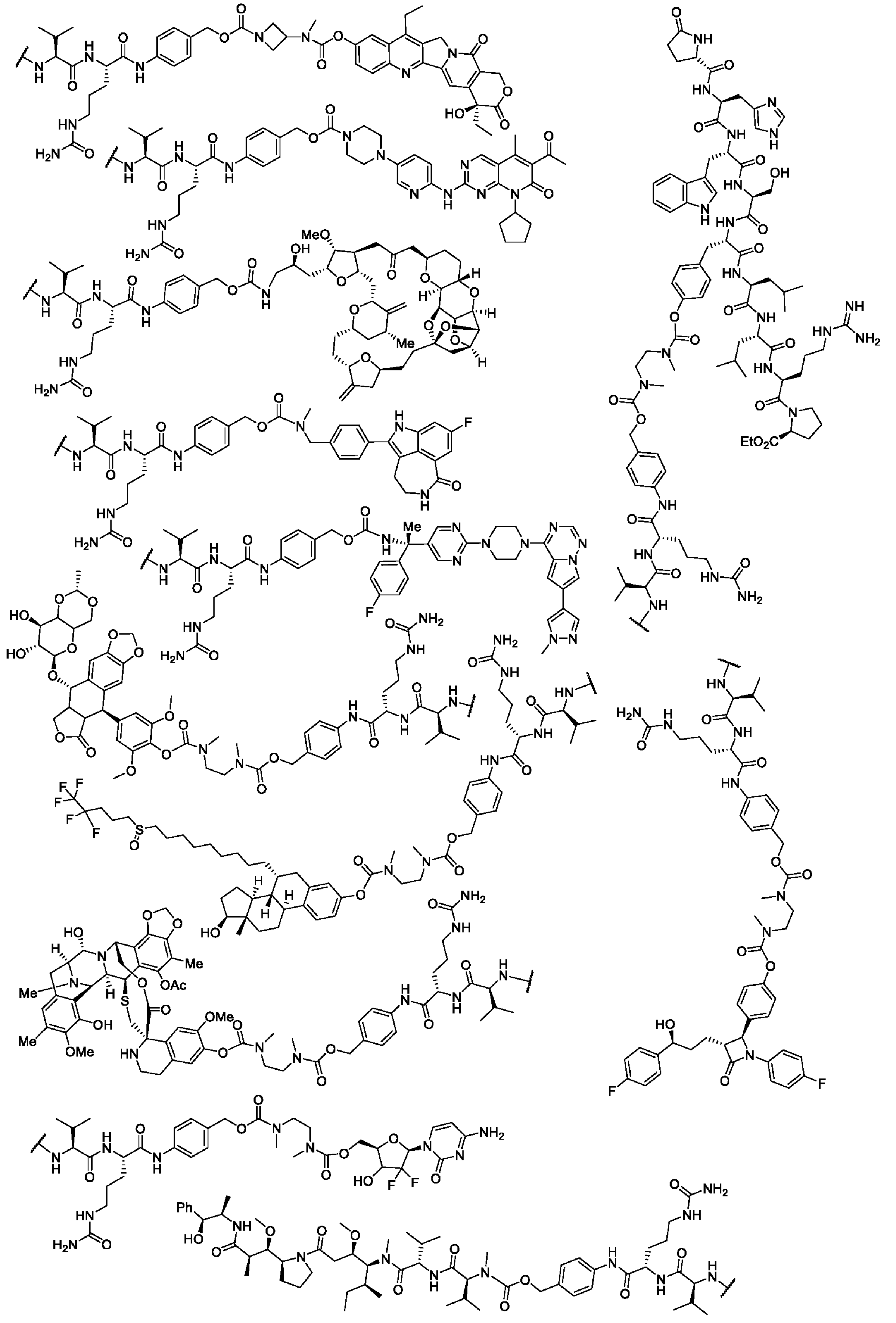

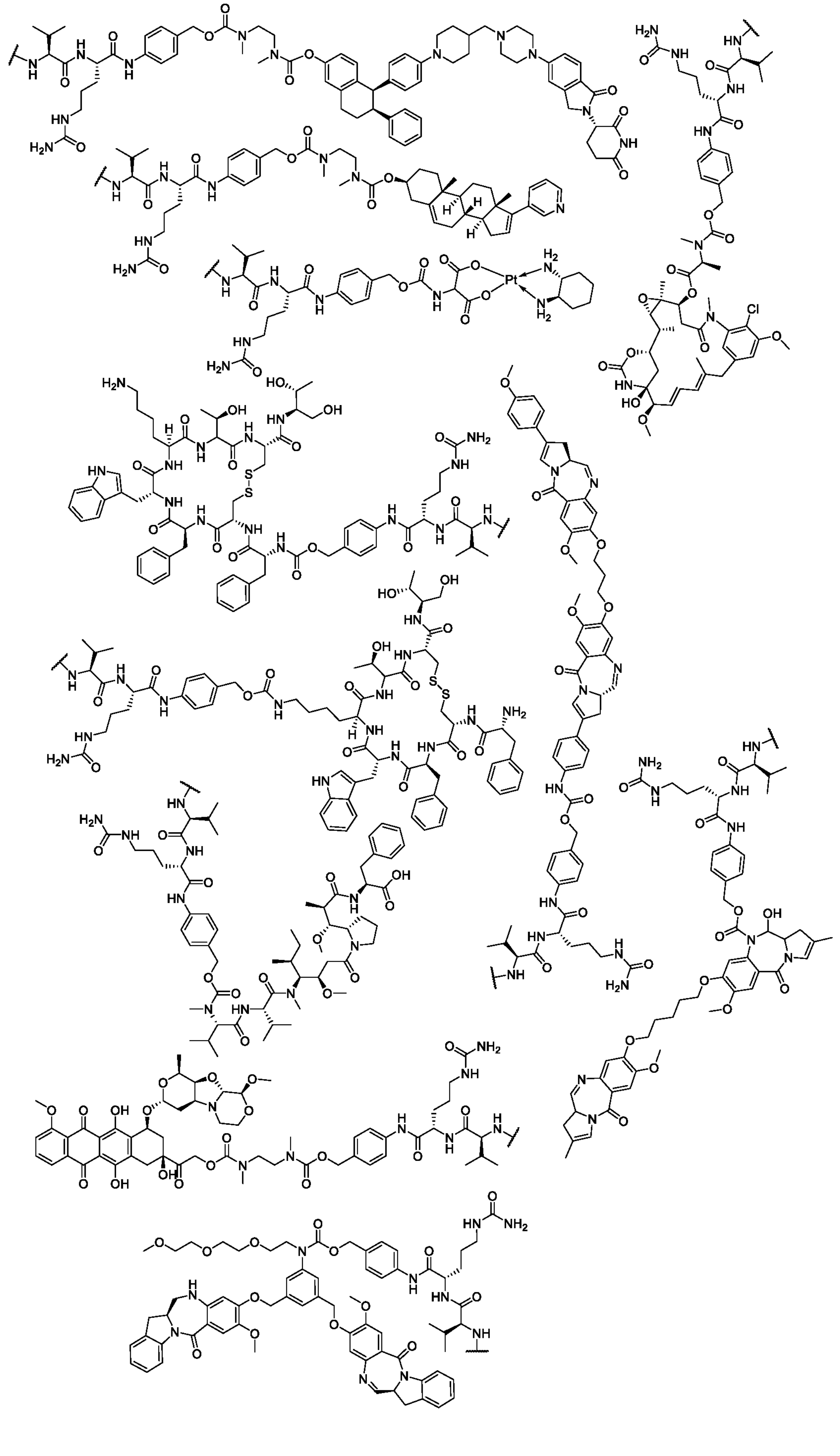

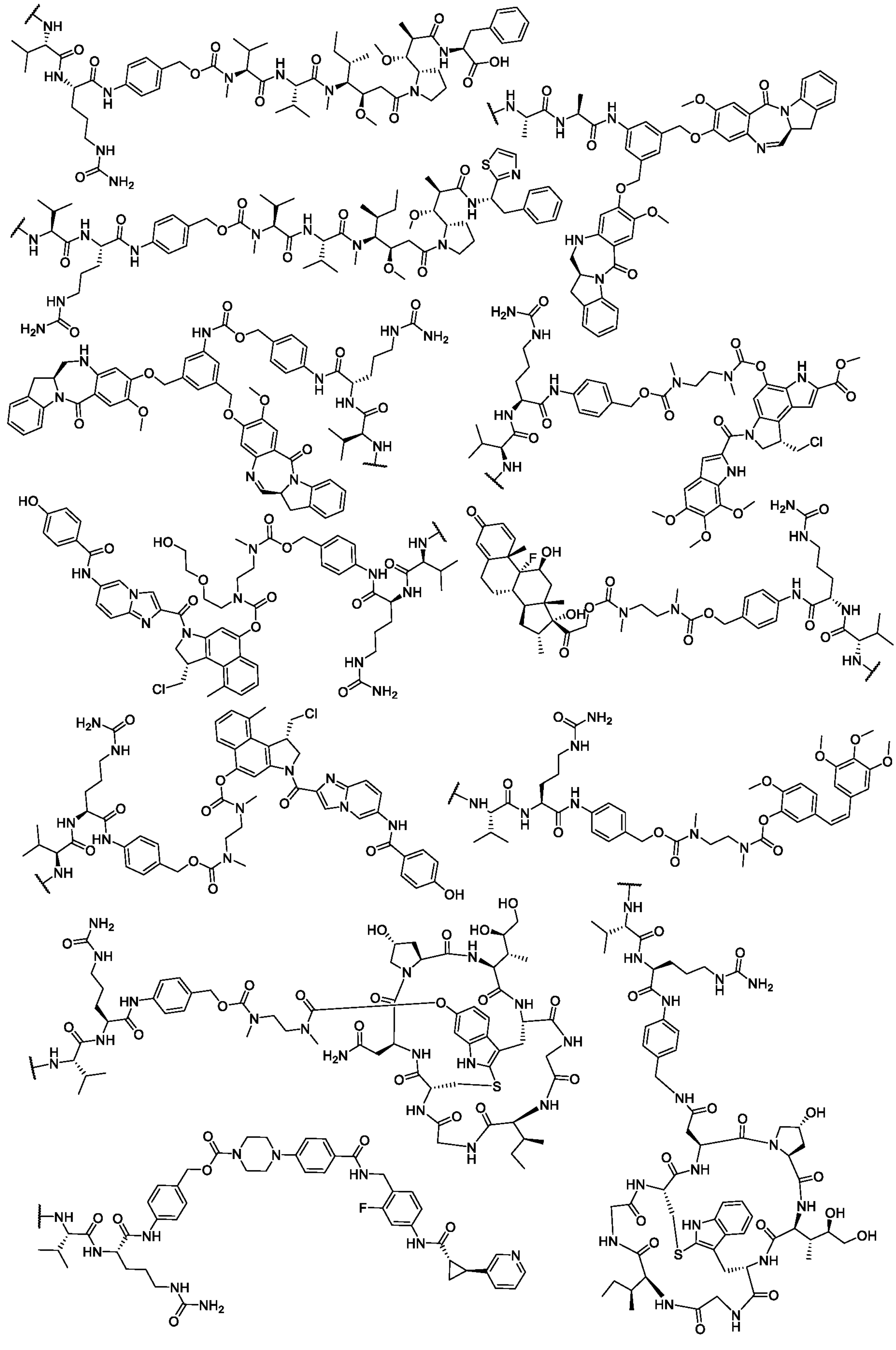

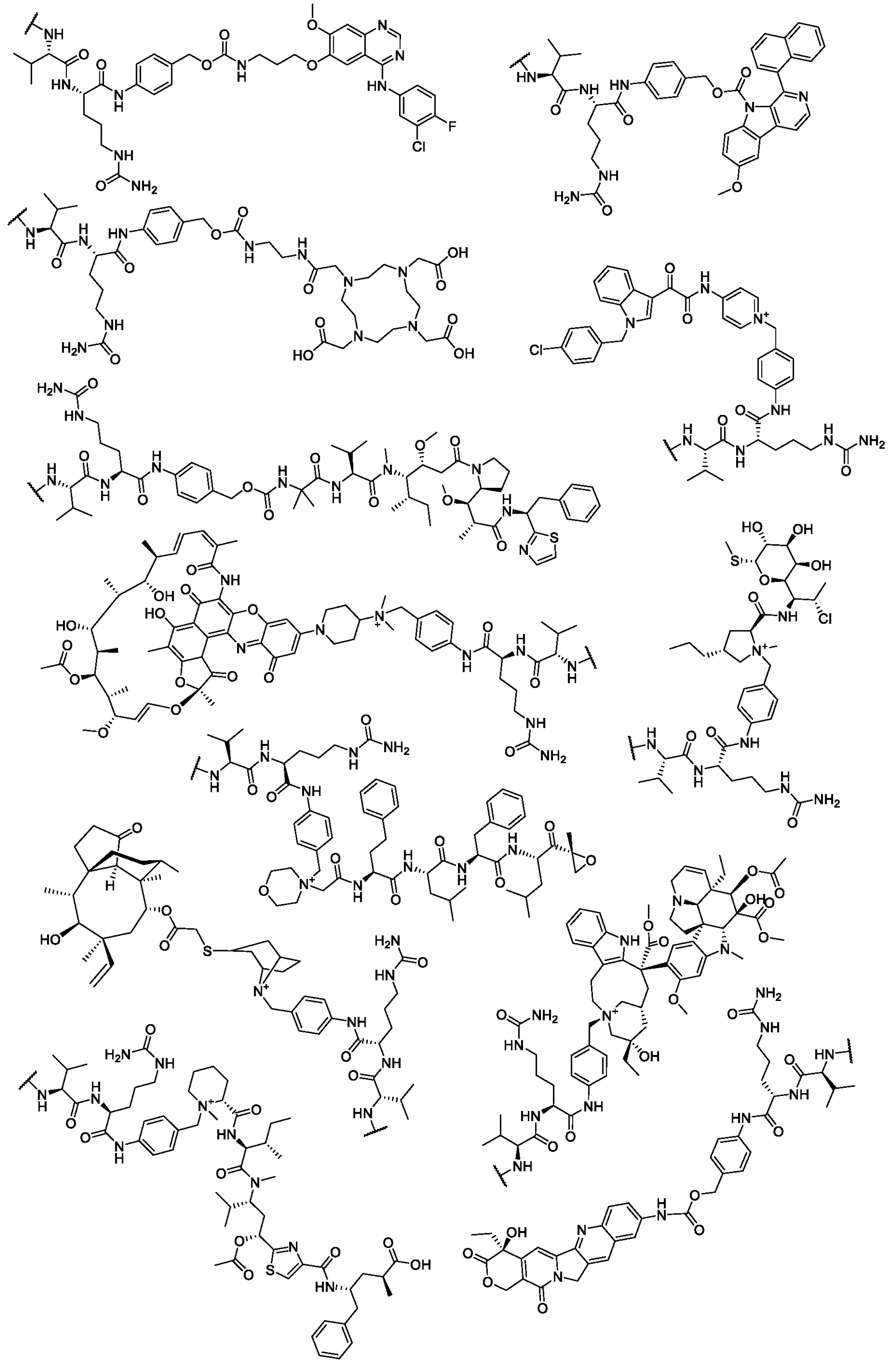

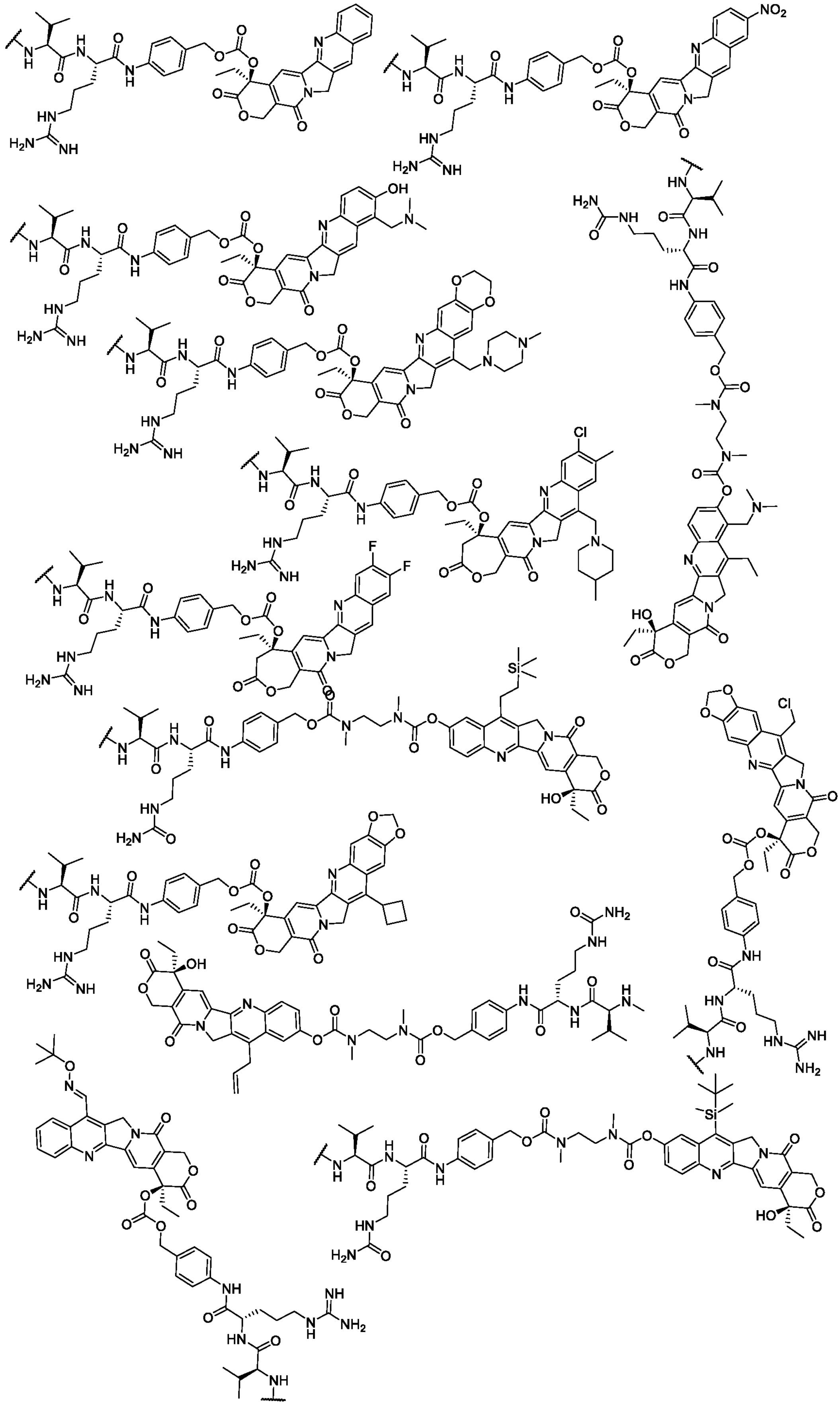

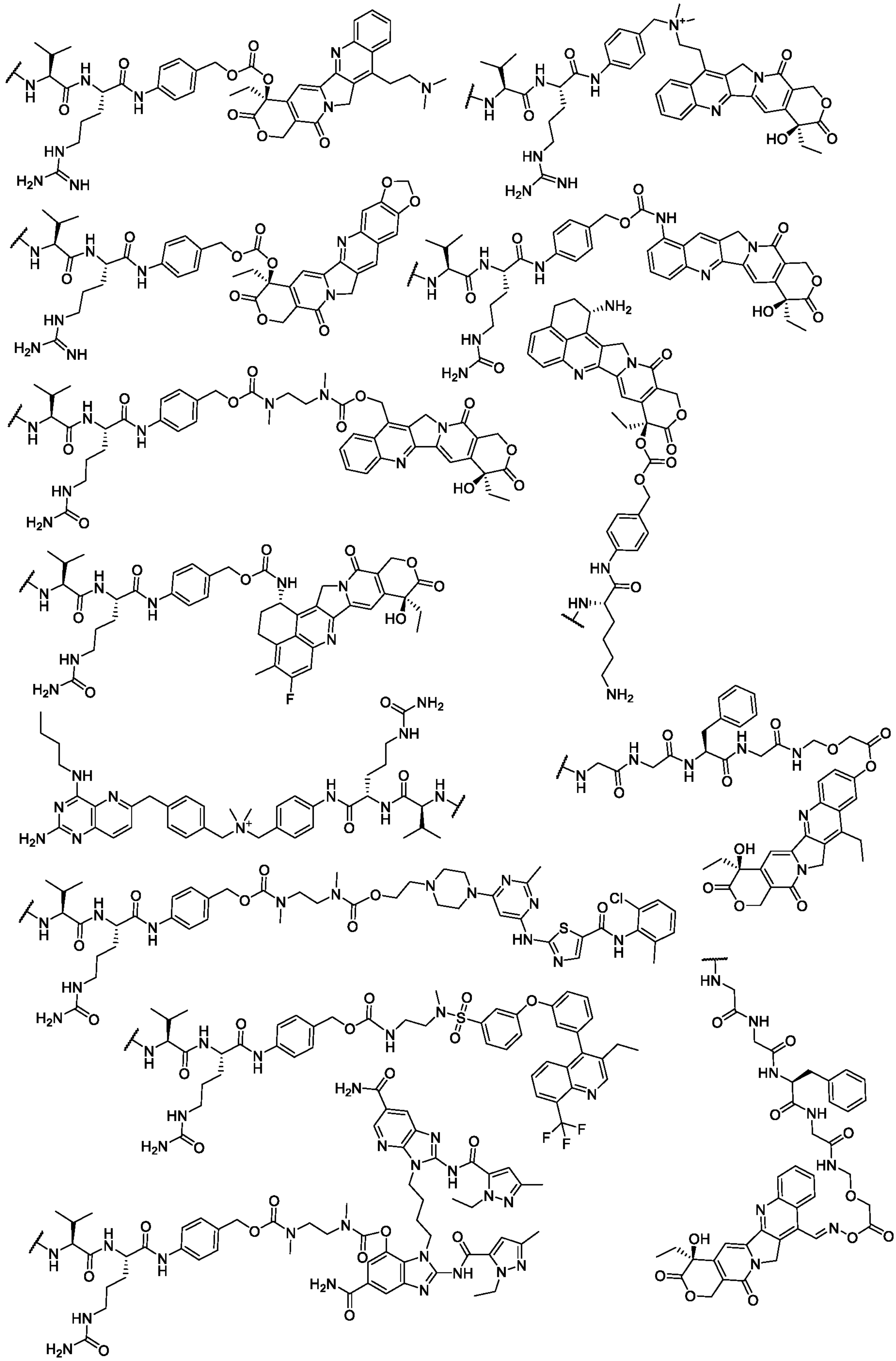

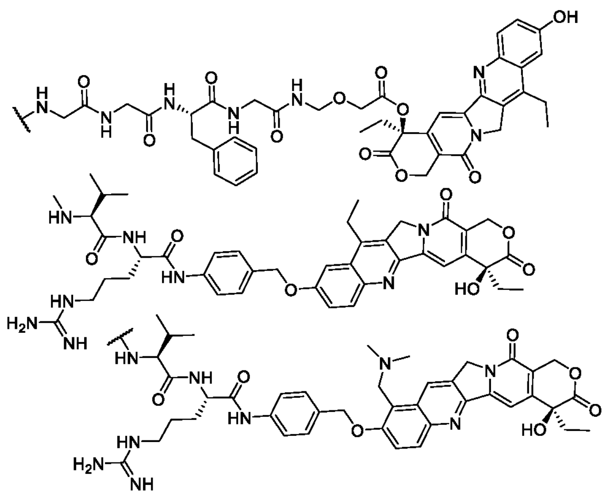

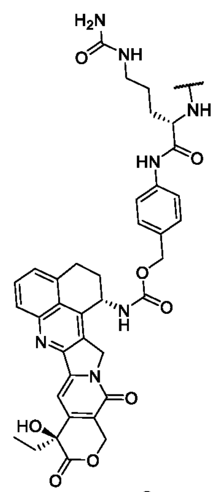

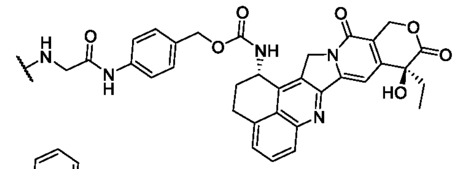

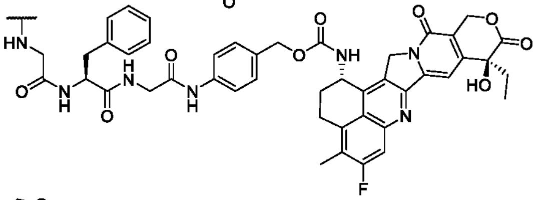

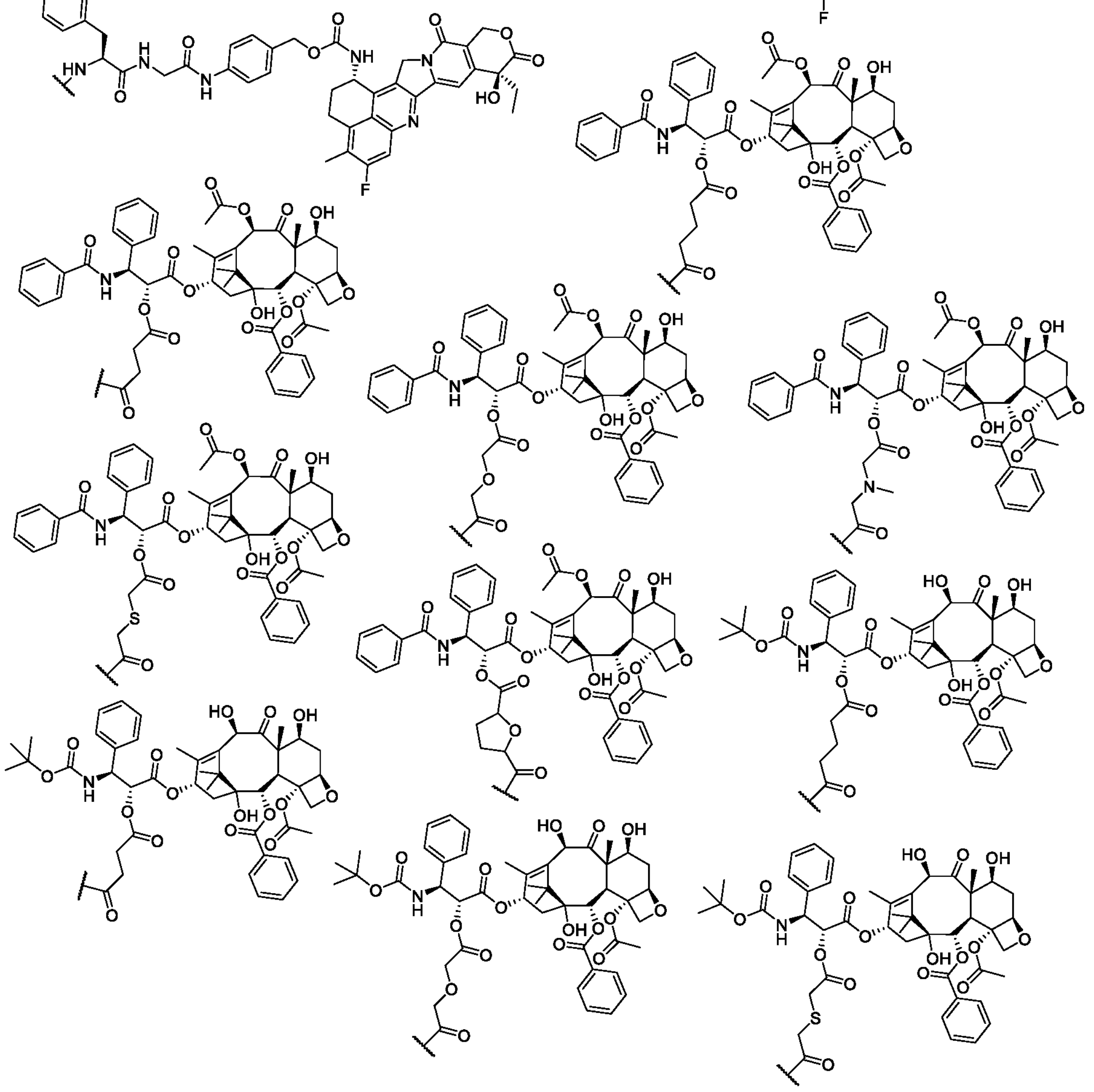

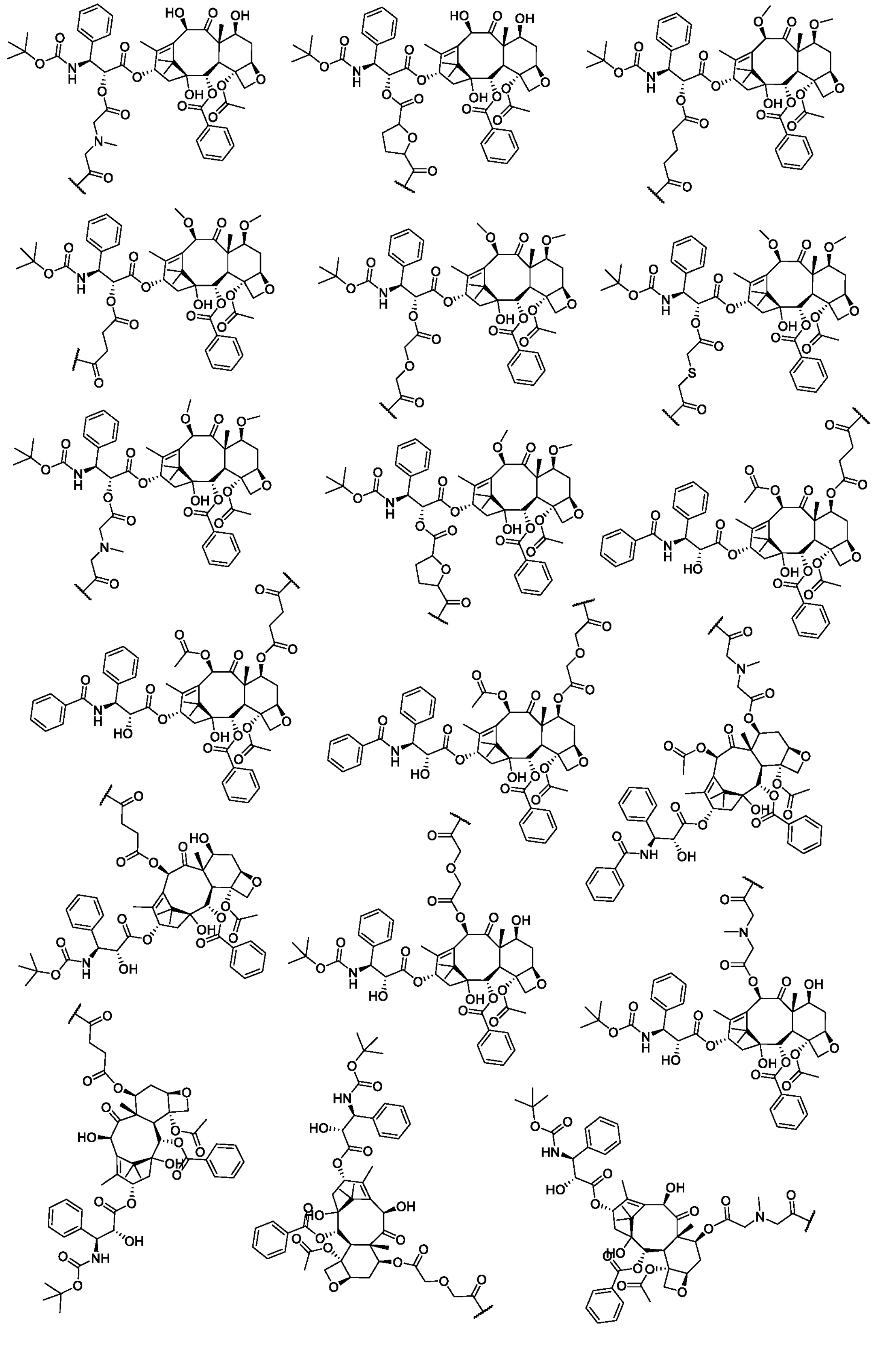

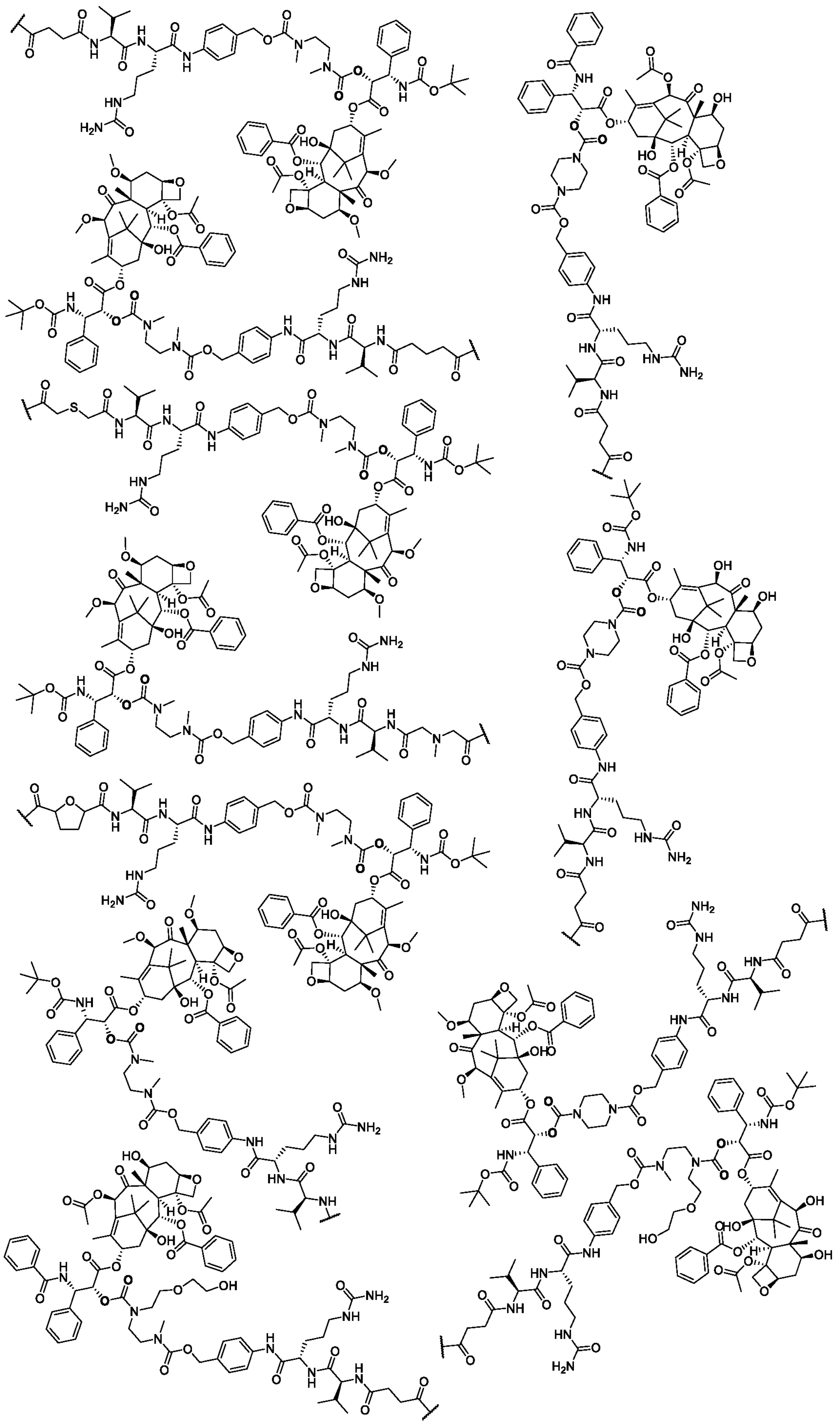

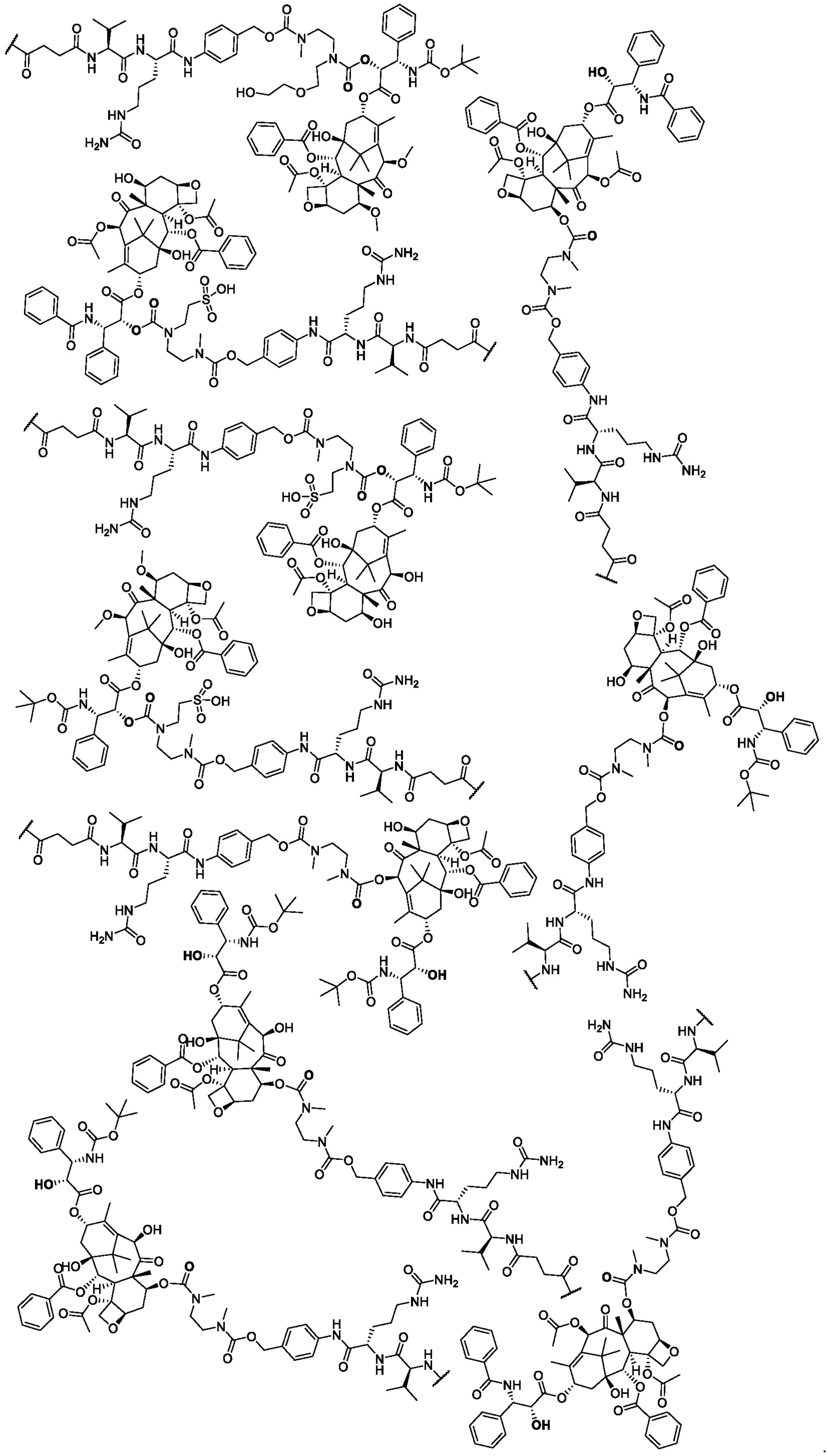

In some embodiments, $-L^2-D$ is preferably any of the following structures:

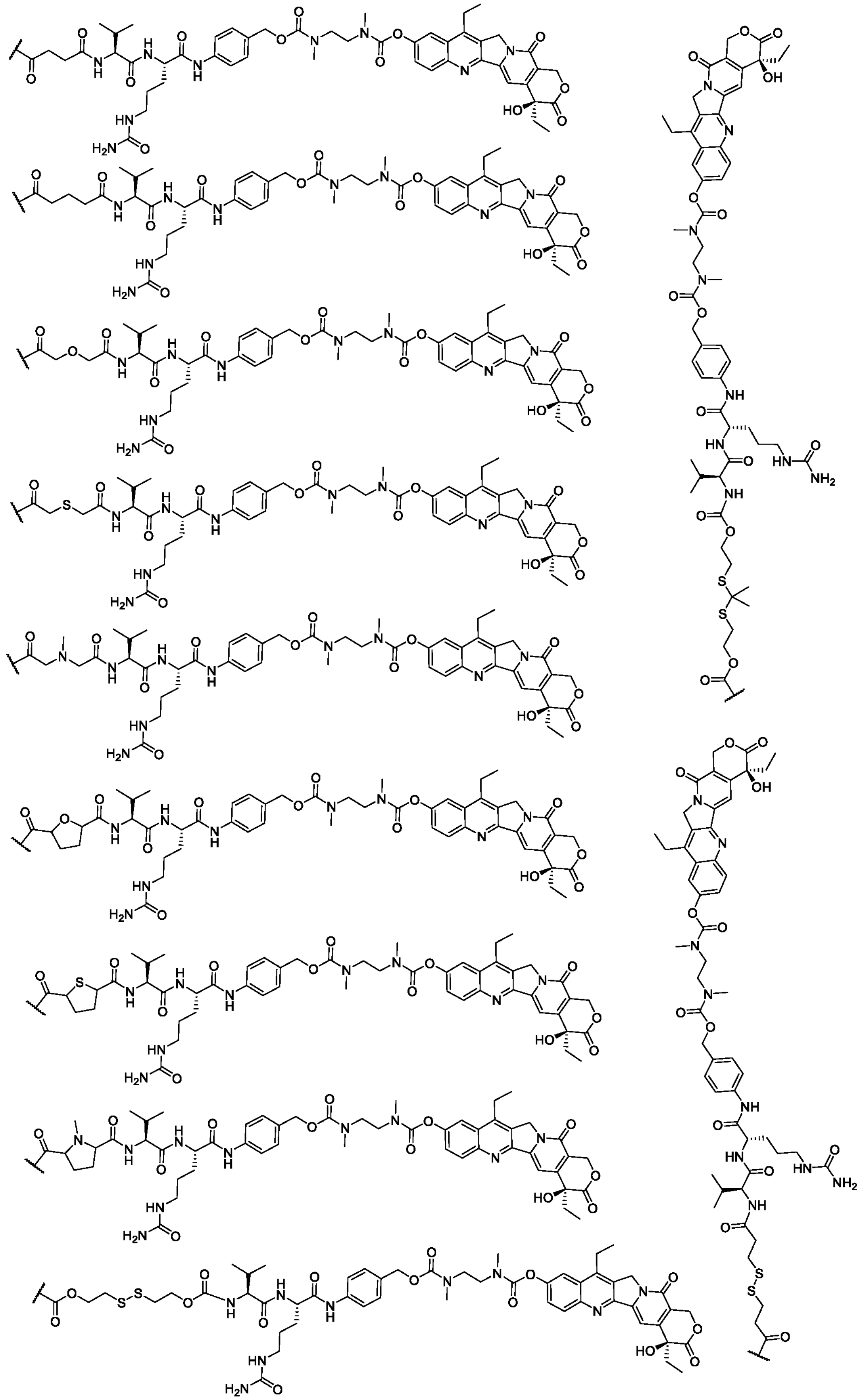

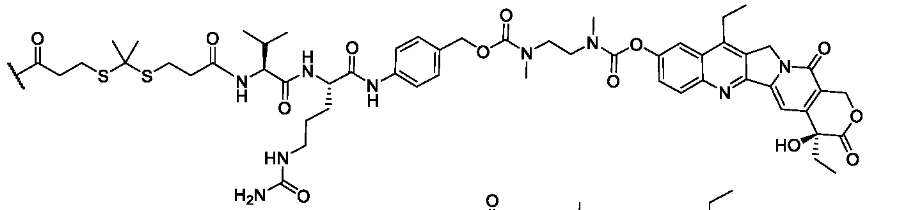

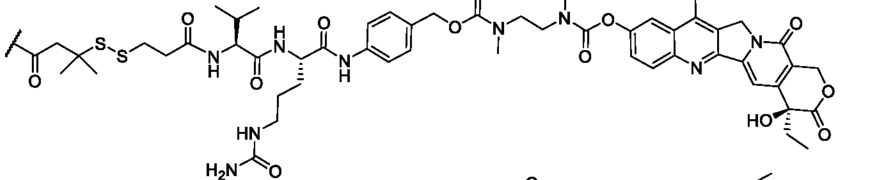

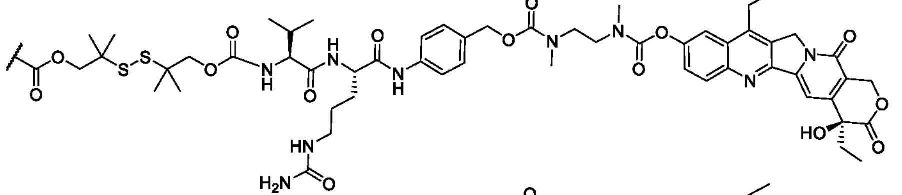

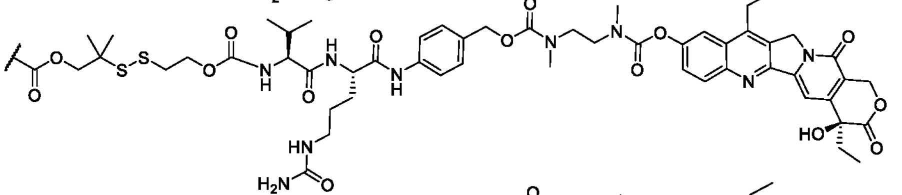

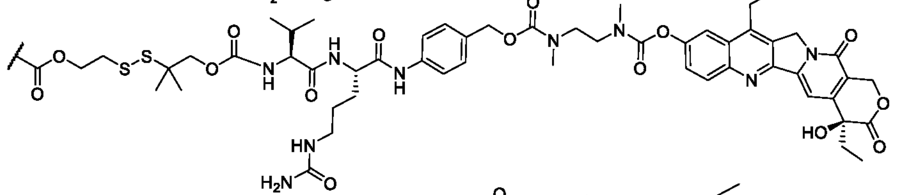

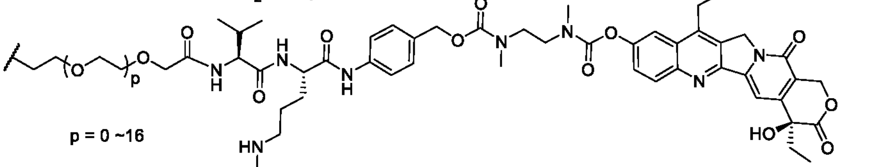

p = 0 ~16

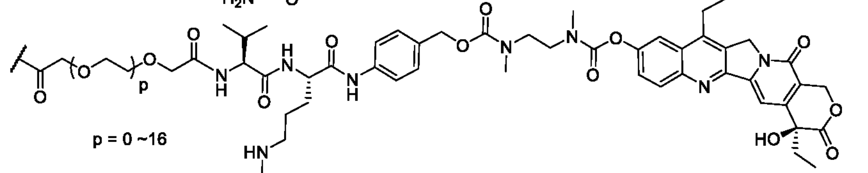

p = 0 ~16

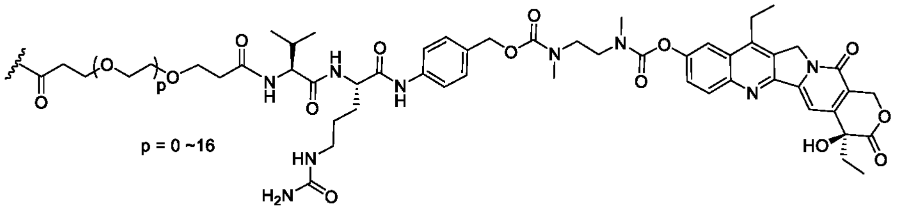

p = 0 ~16

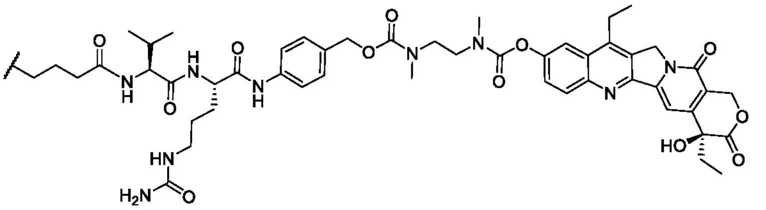

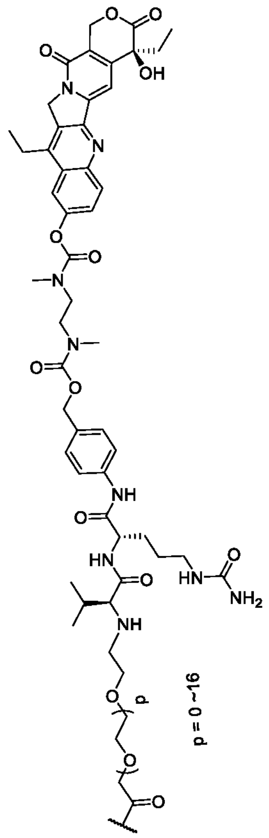

p = 0 ~16

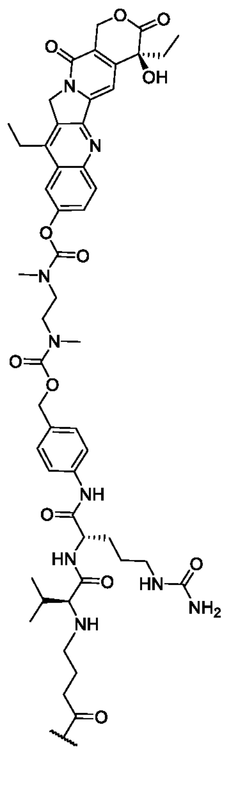

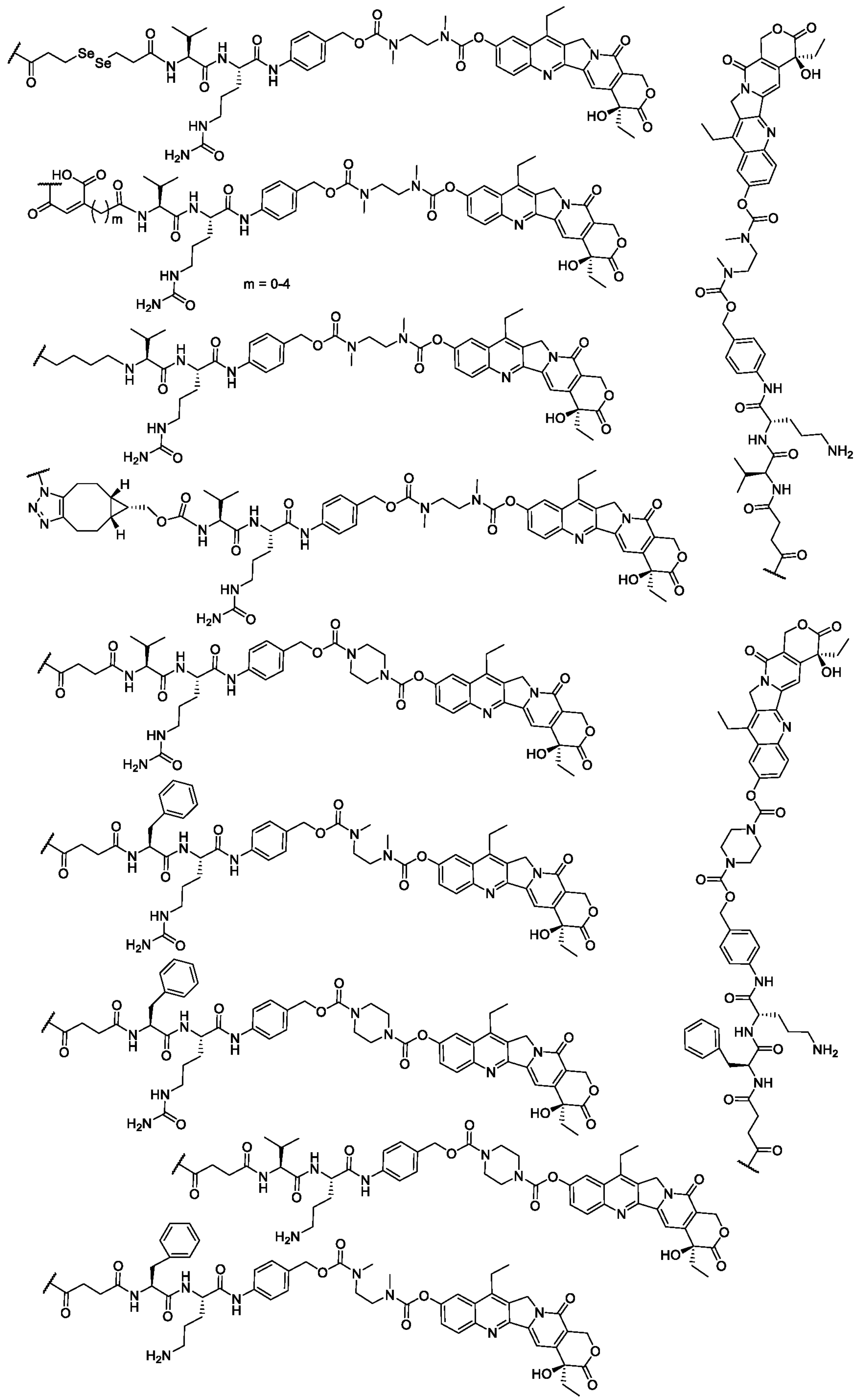

m = 0-4

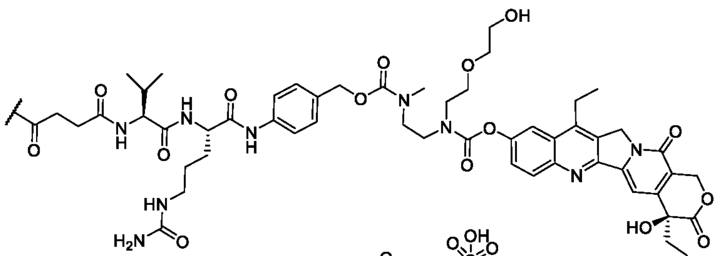
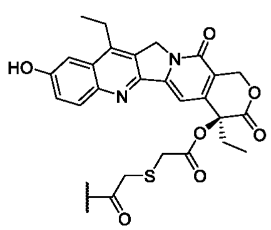
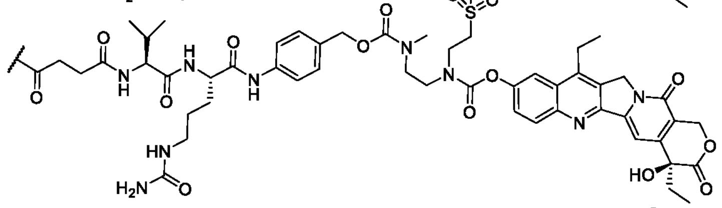
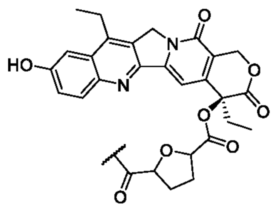
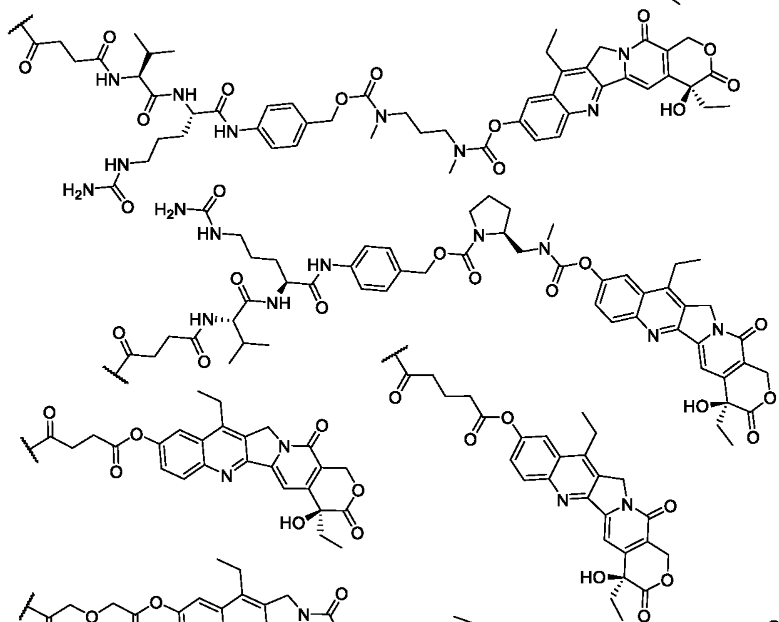
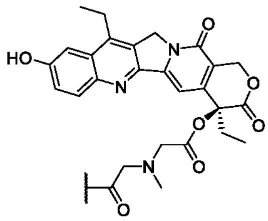
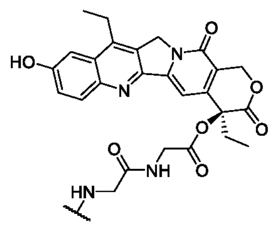
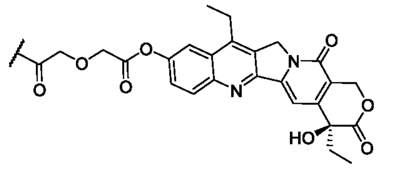
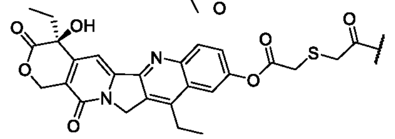
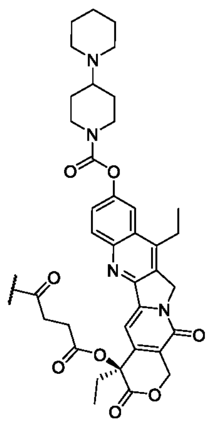
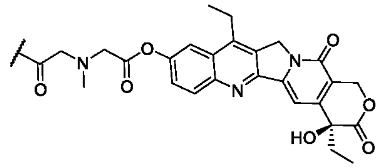
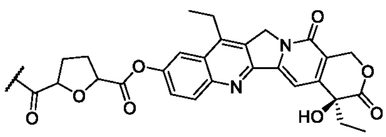
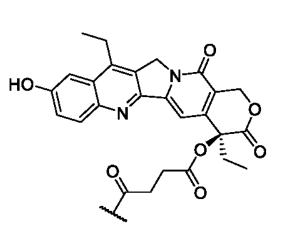
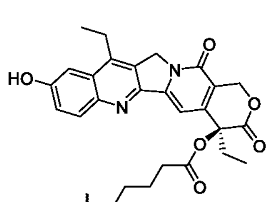
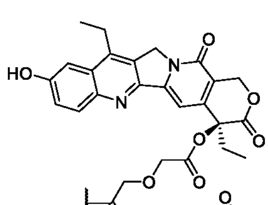
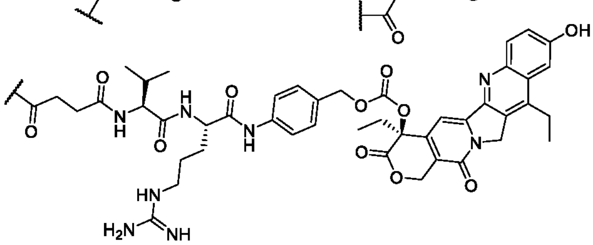
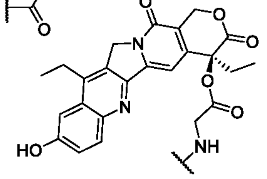

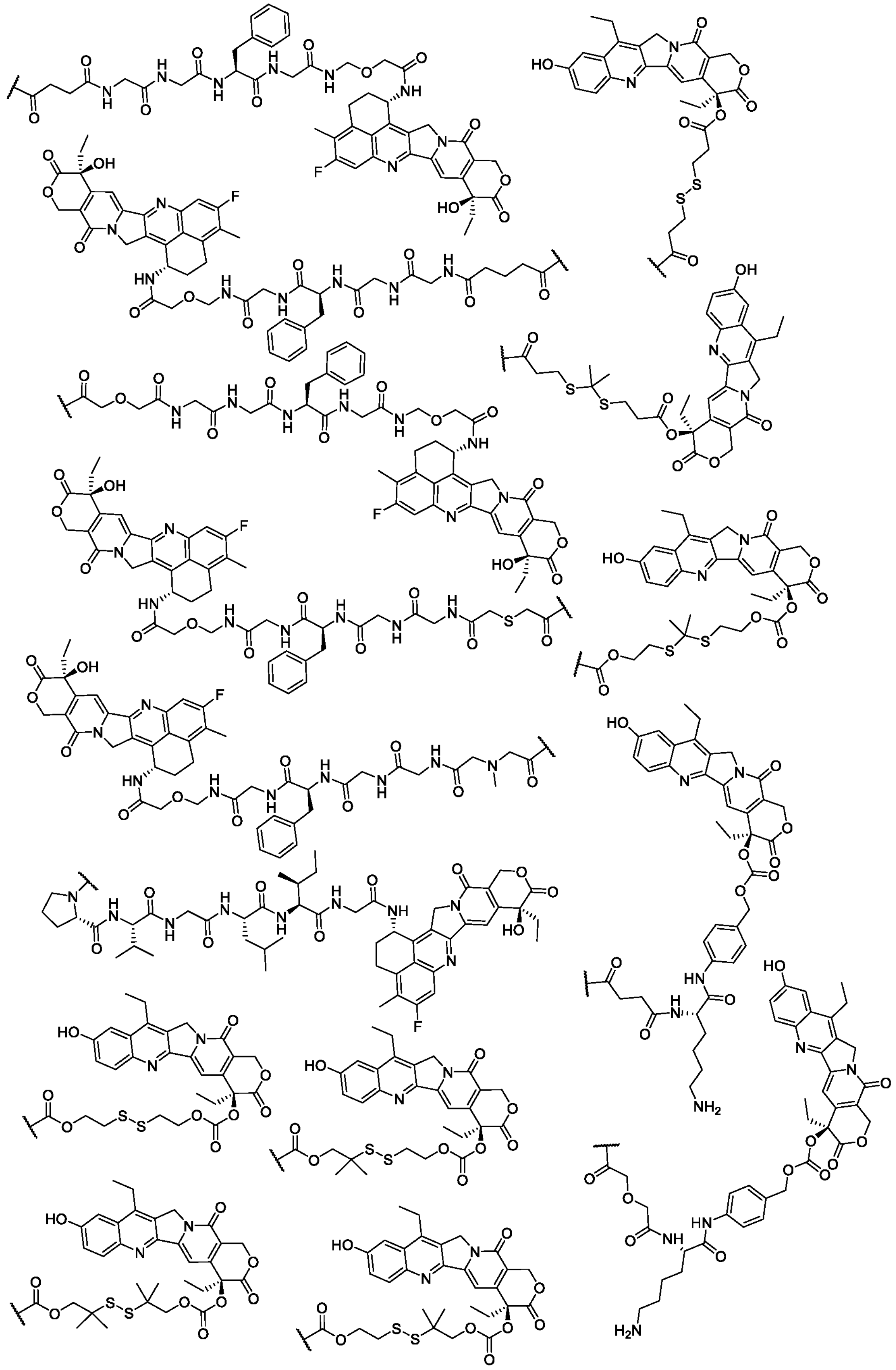

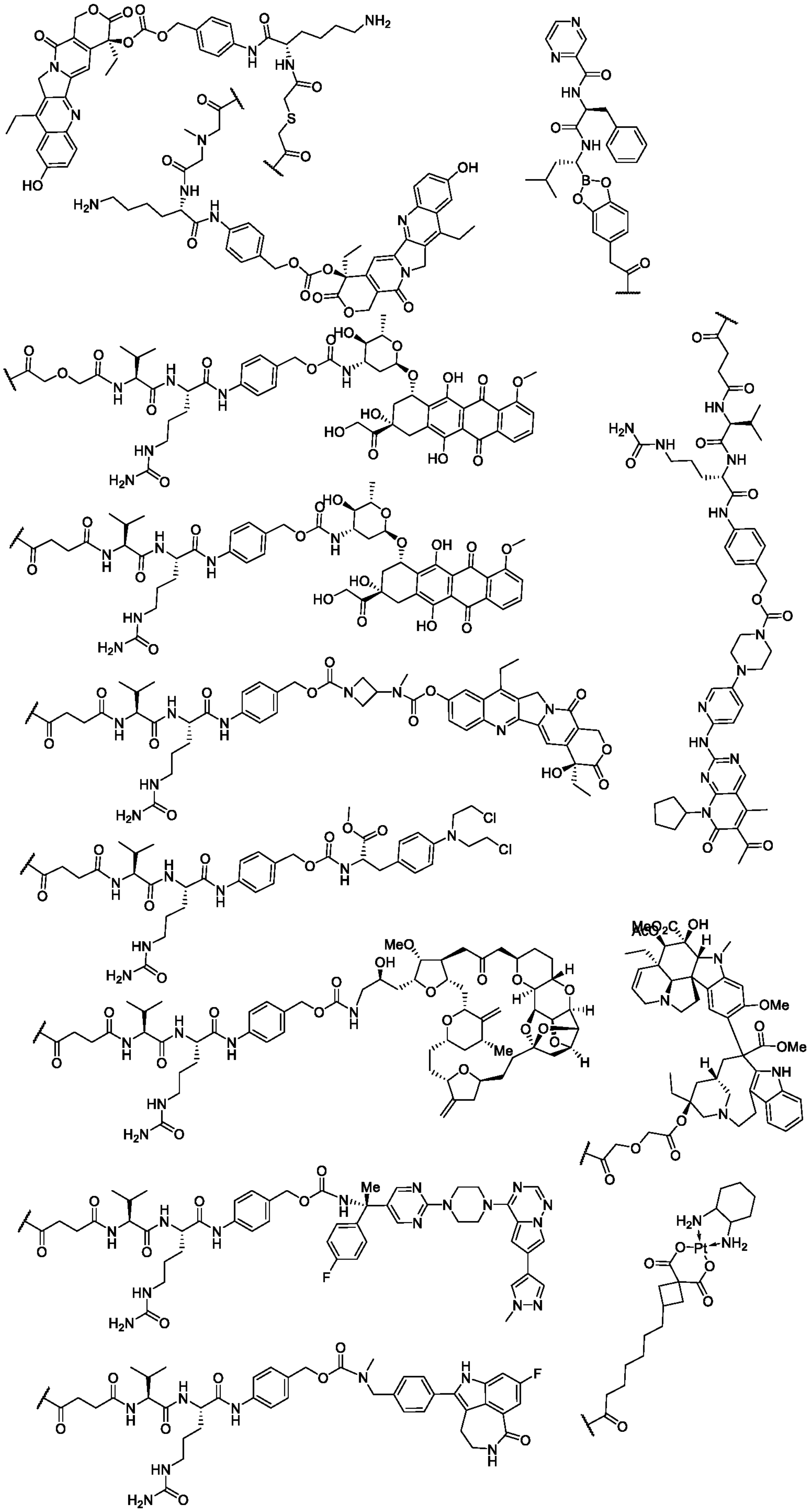

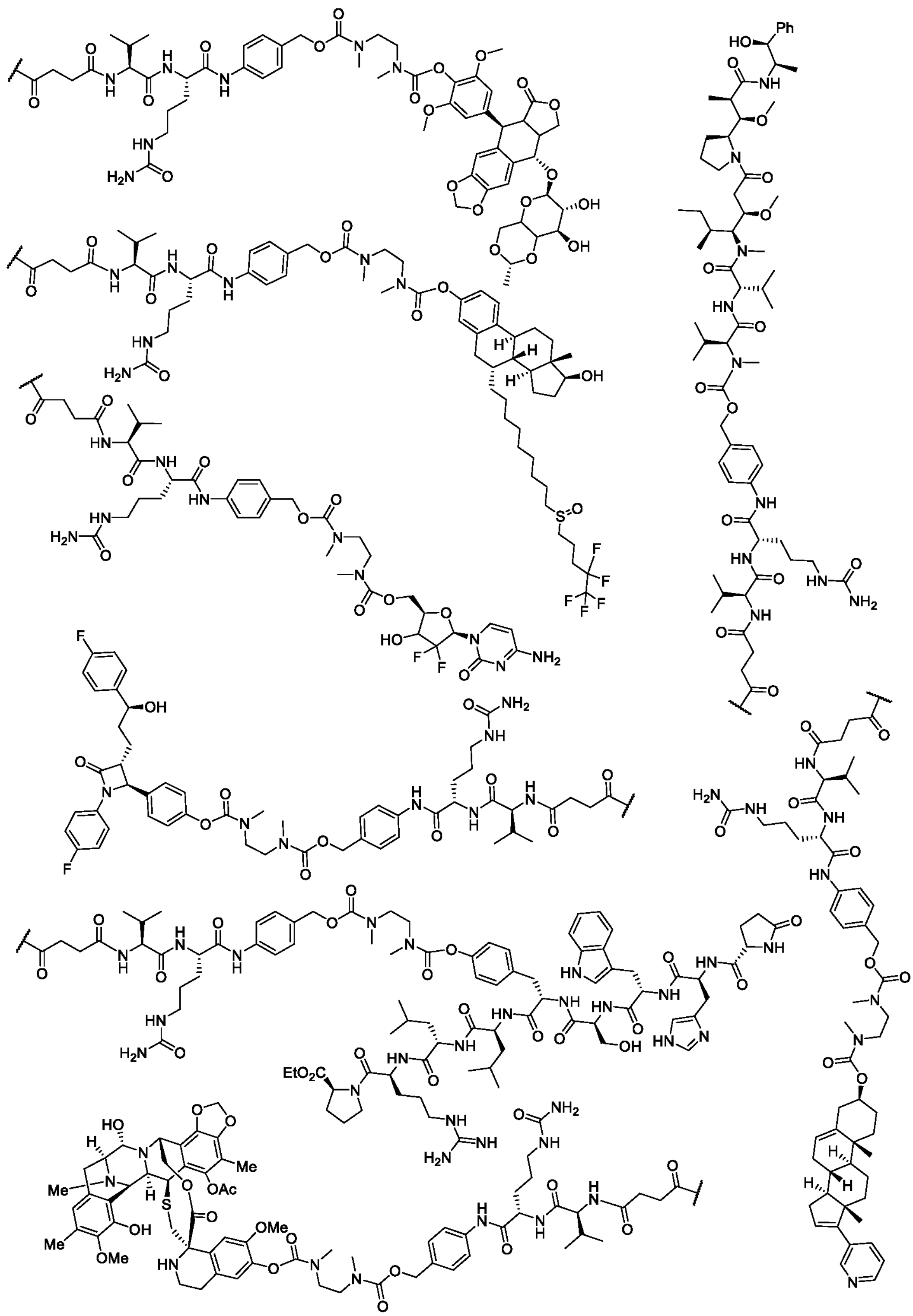

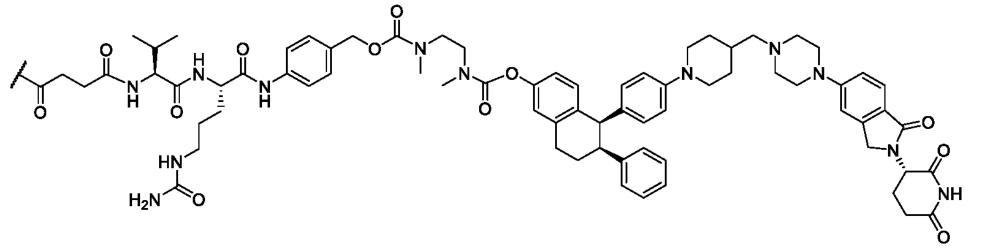

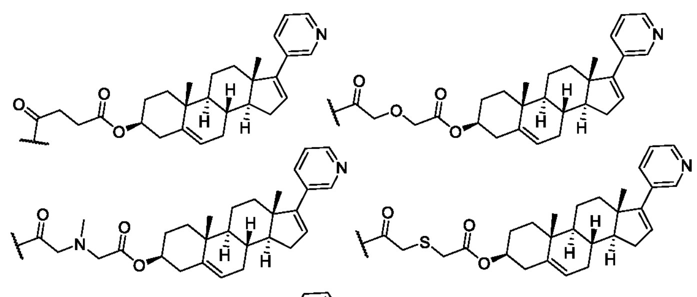

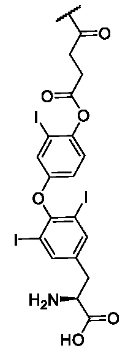

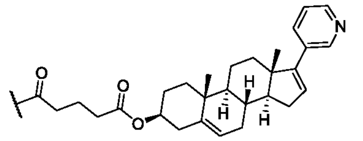

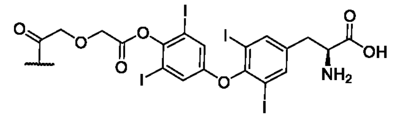

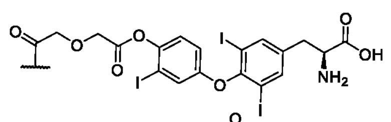

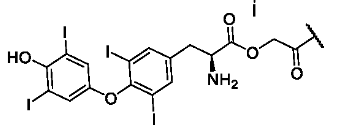

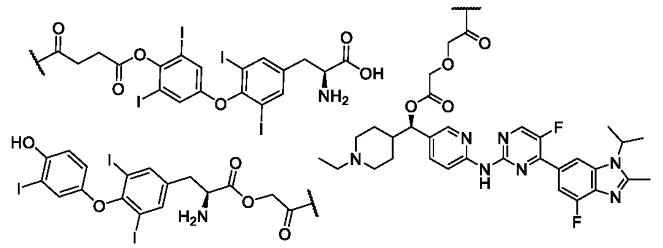

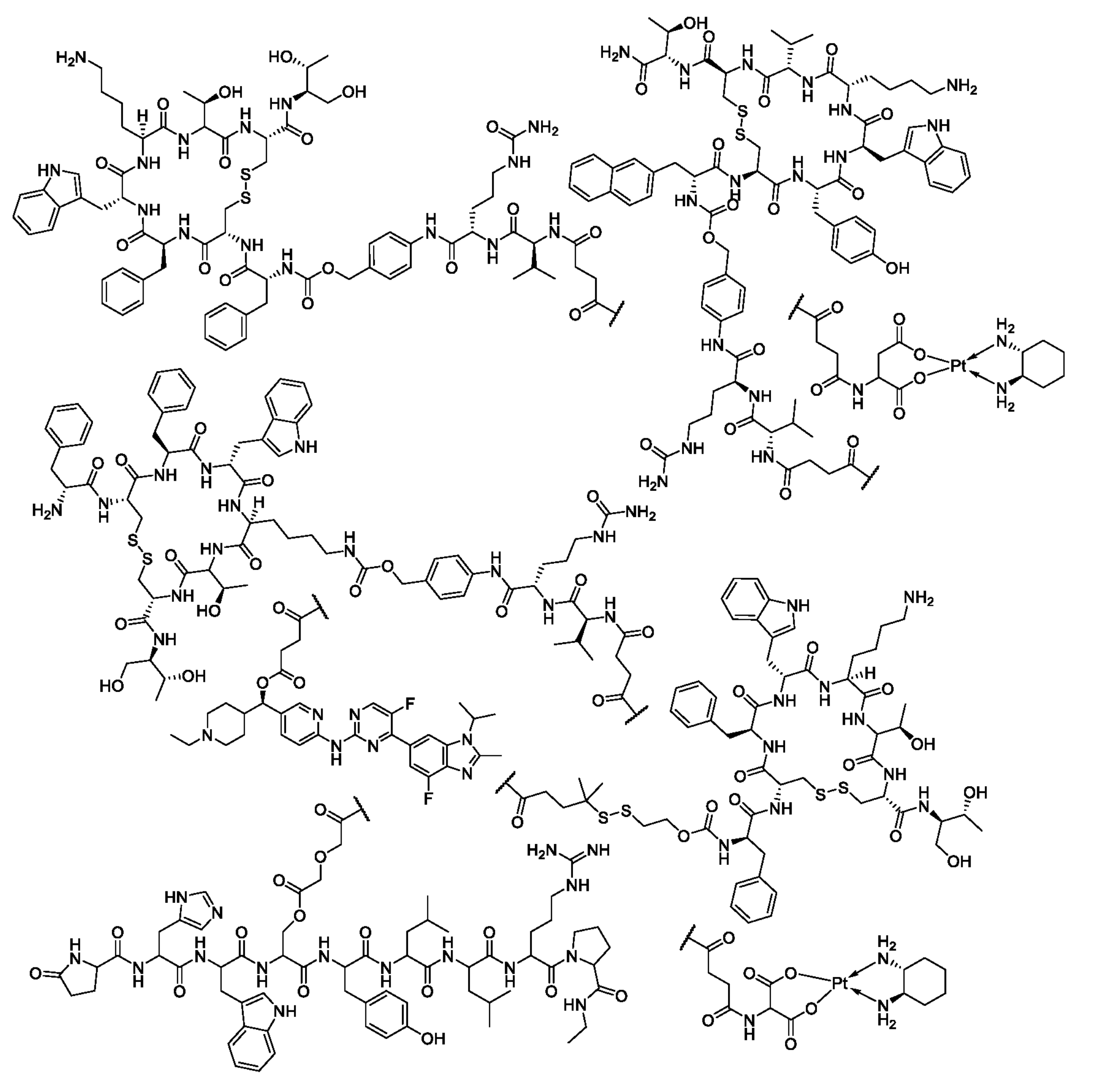

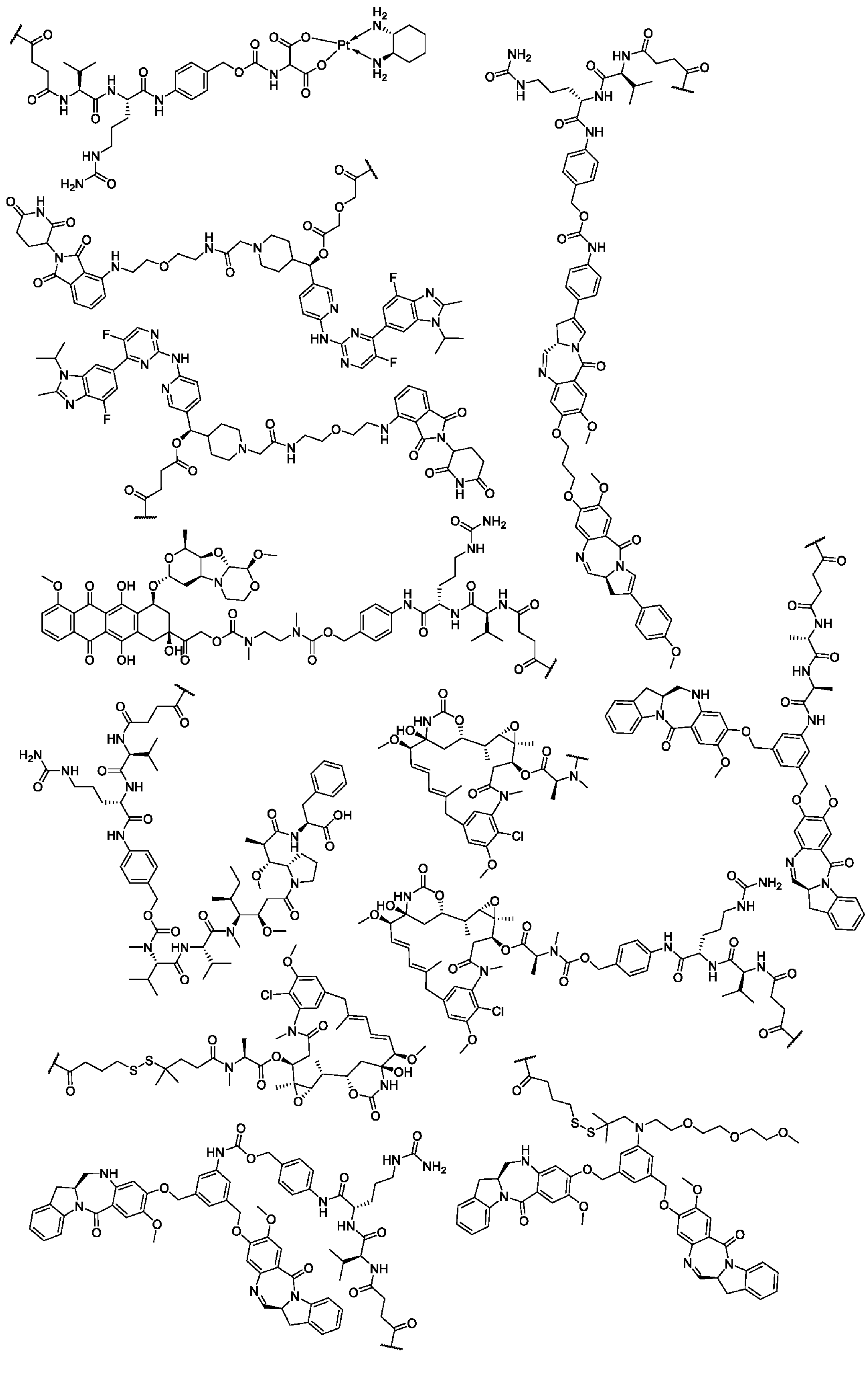

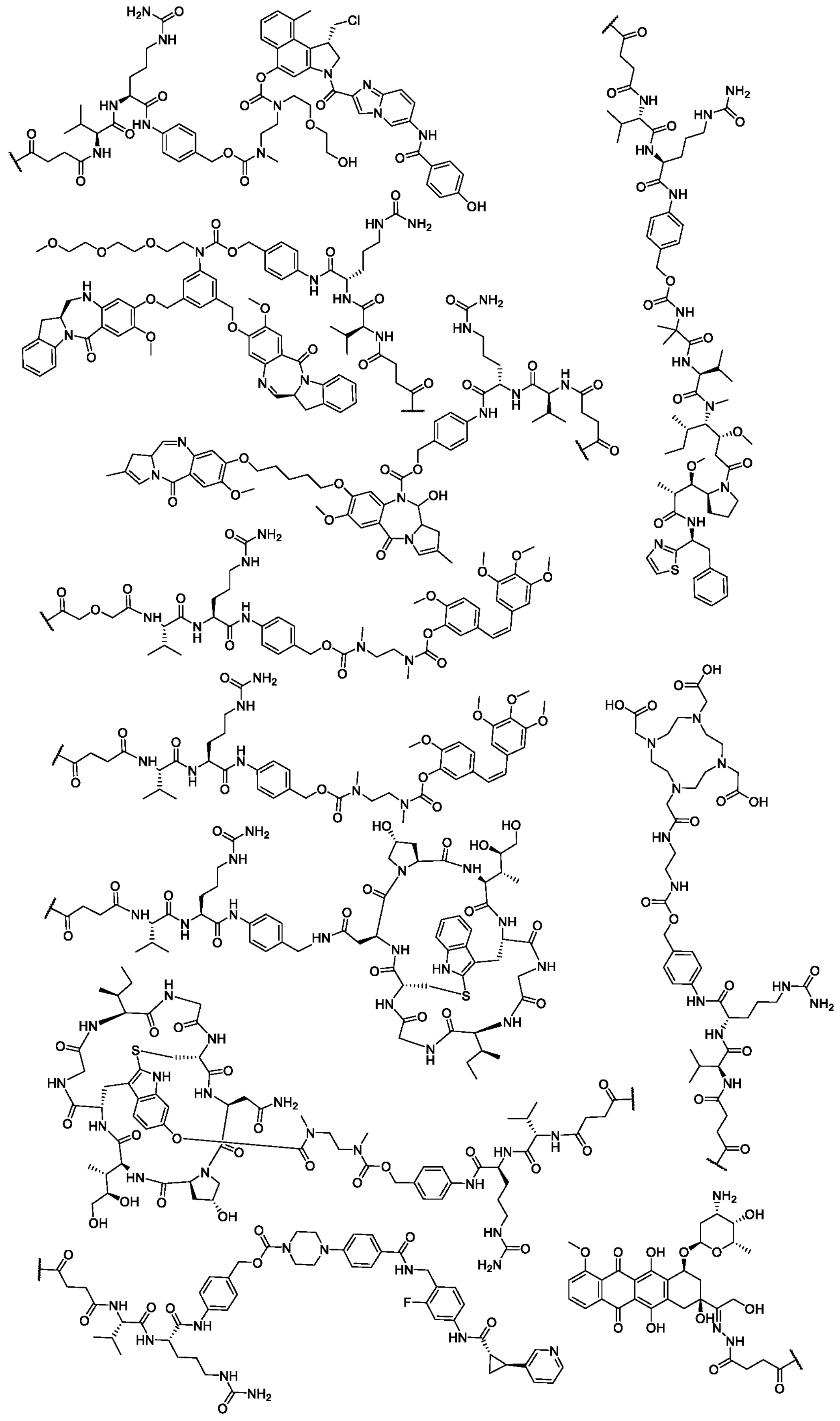

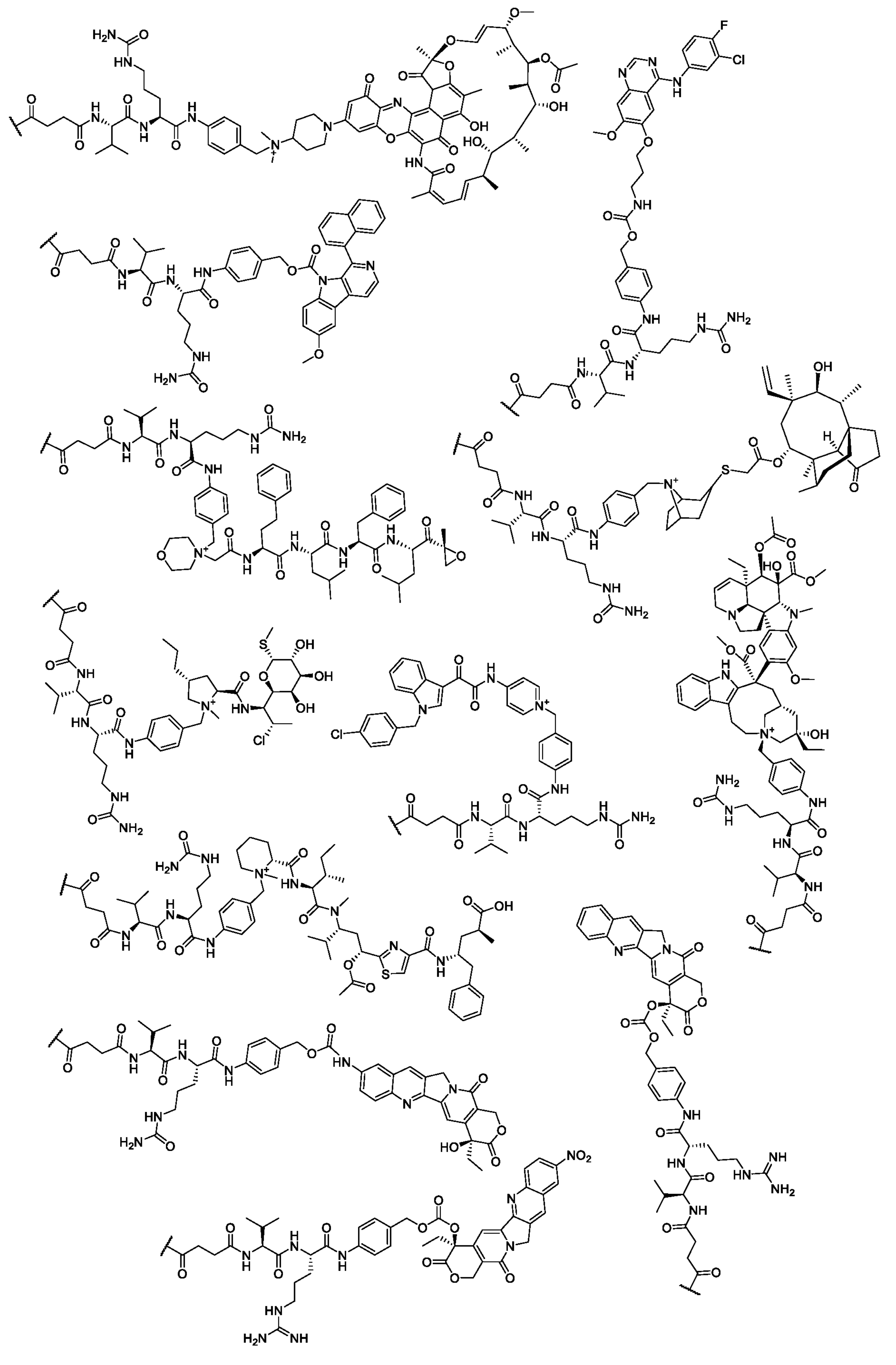

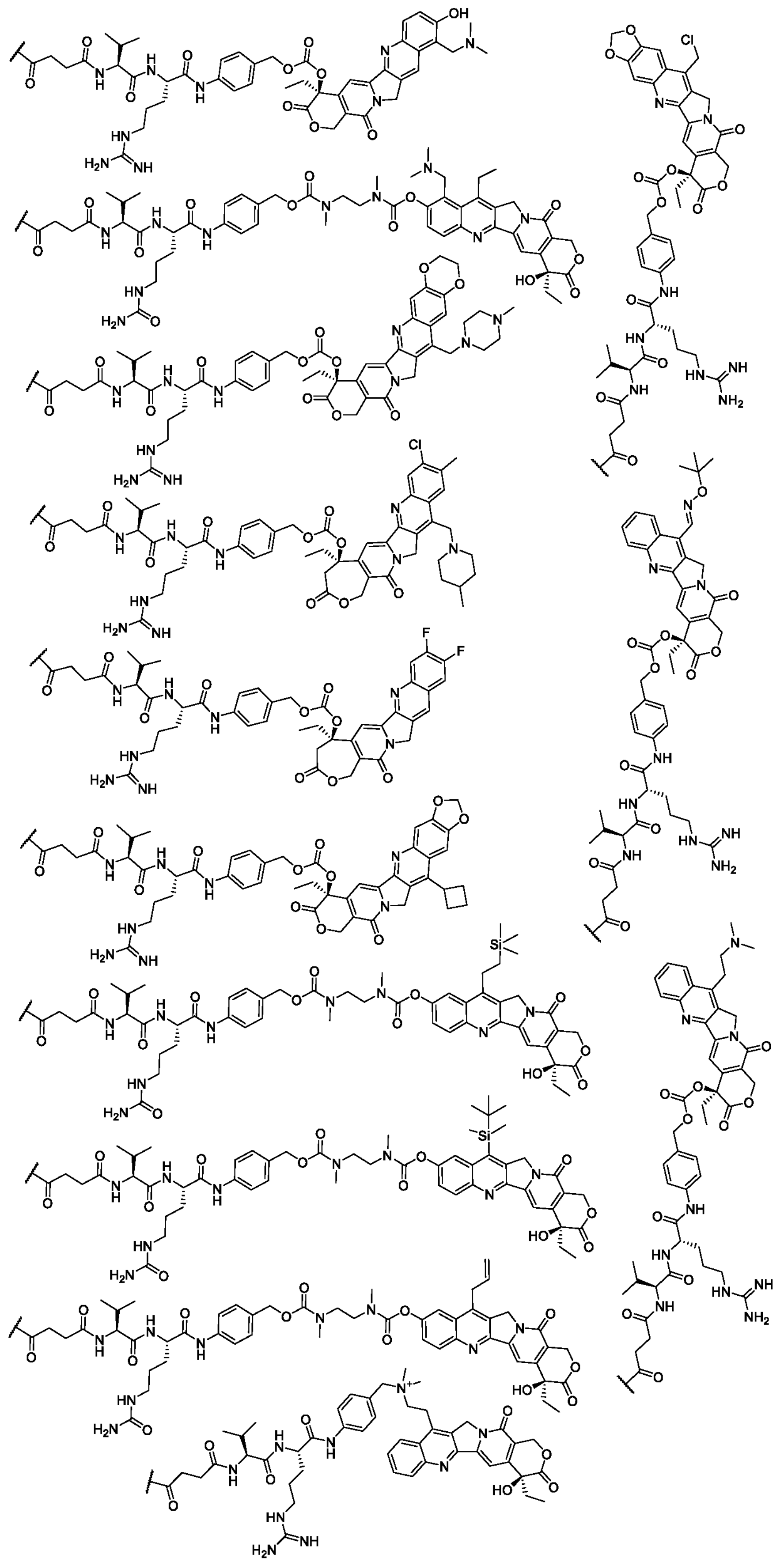

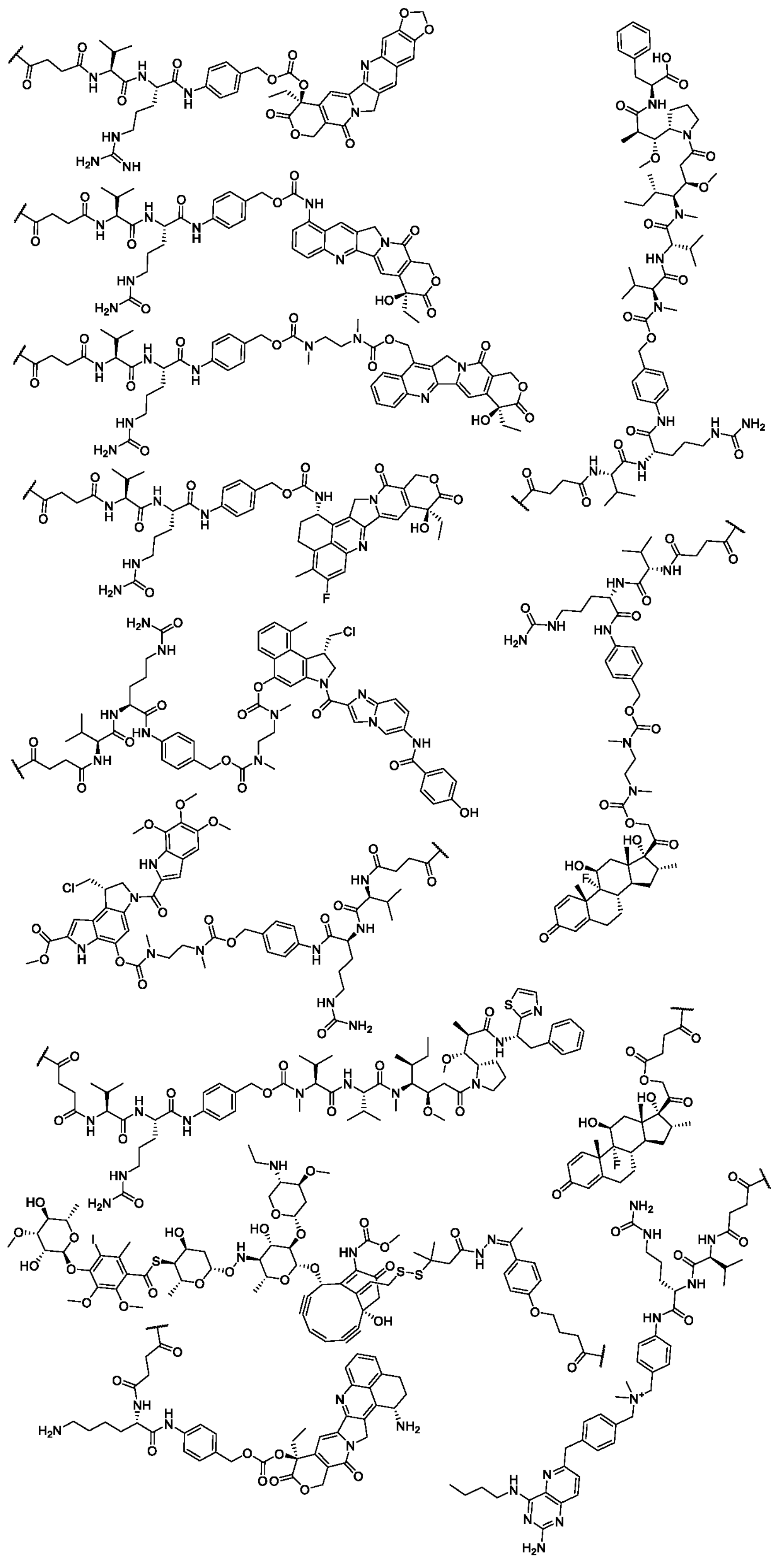

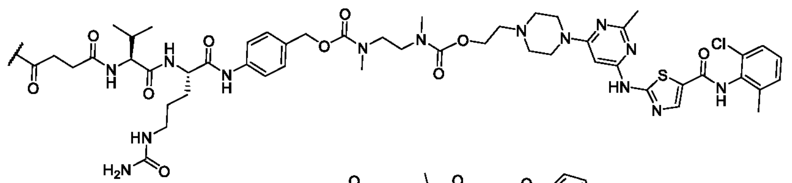

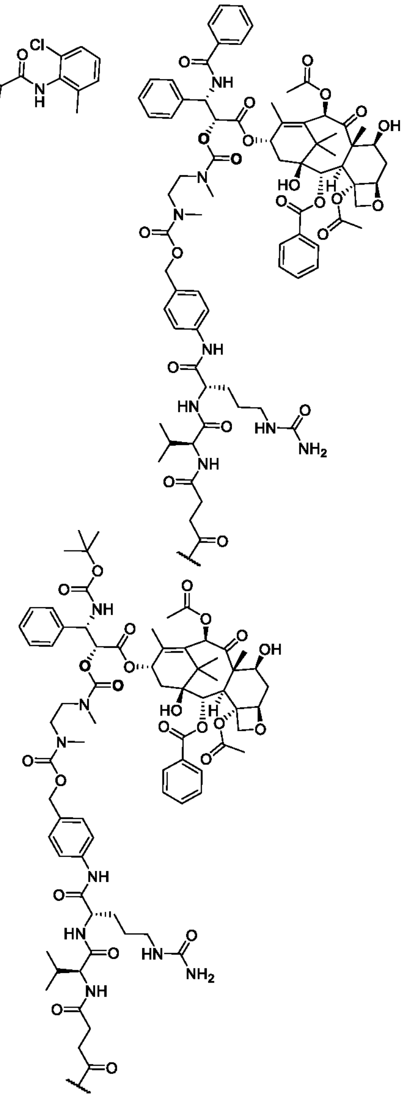

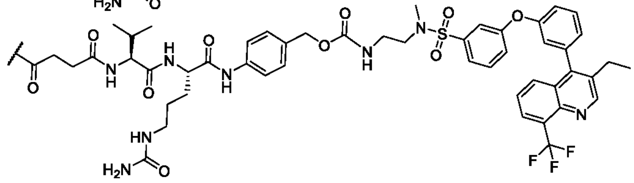

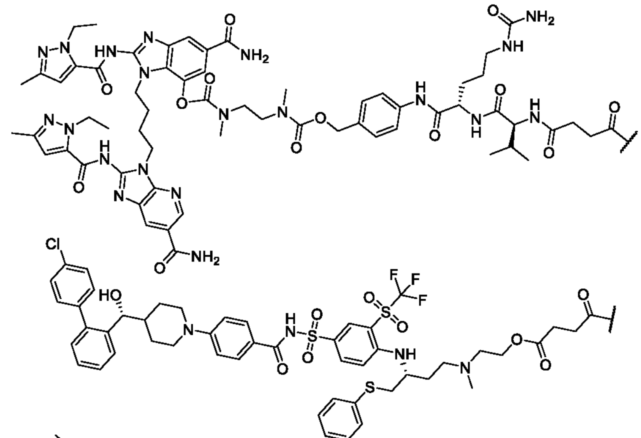

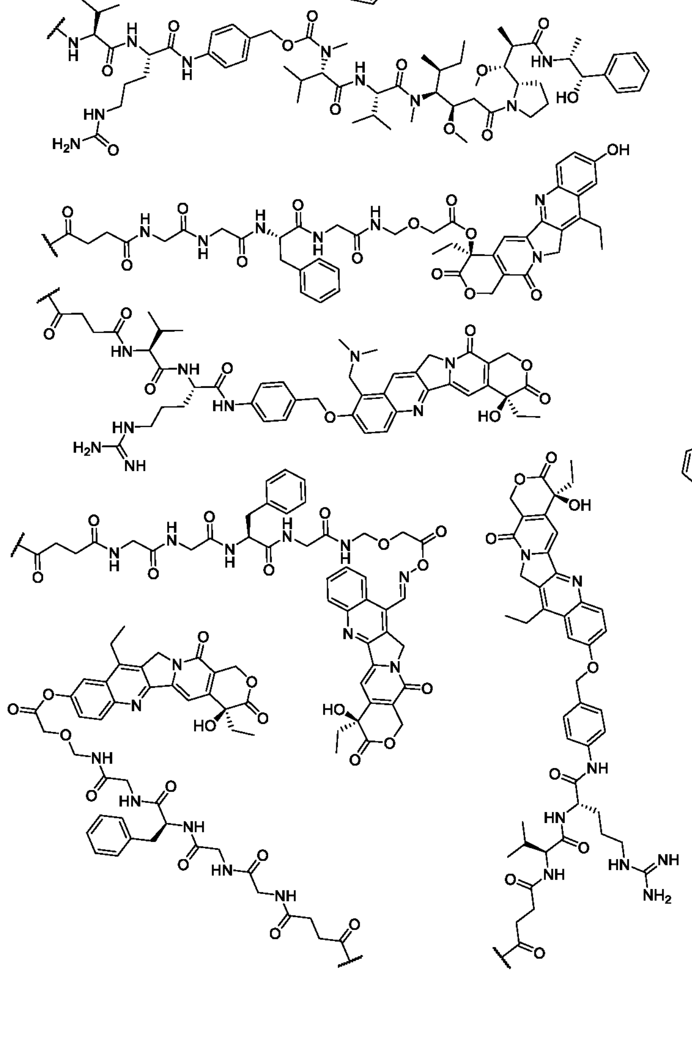

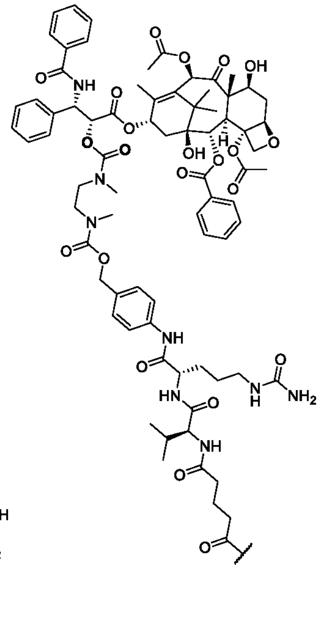

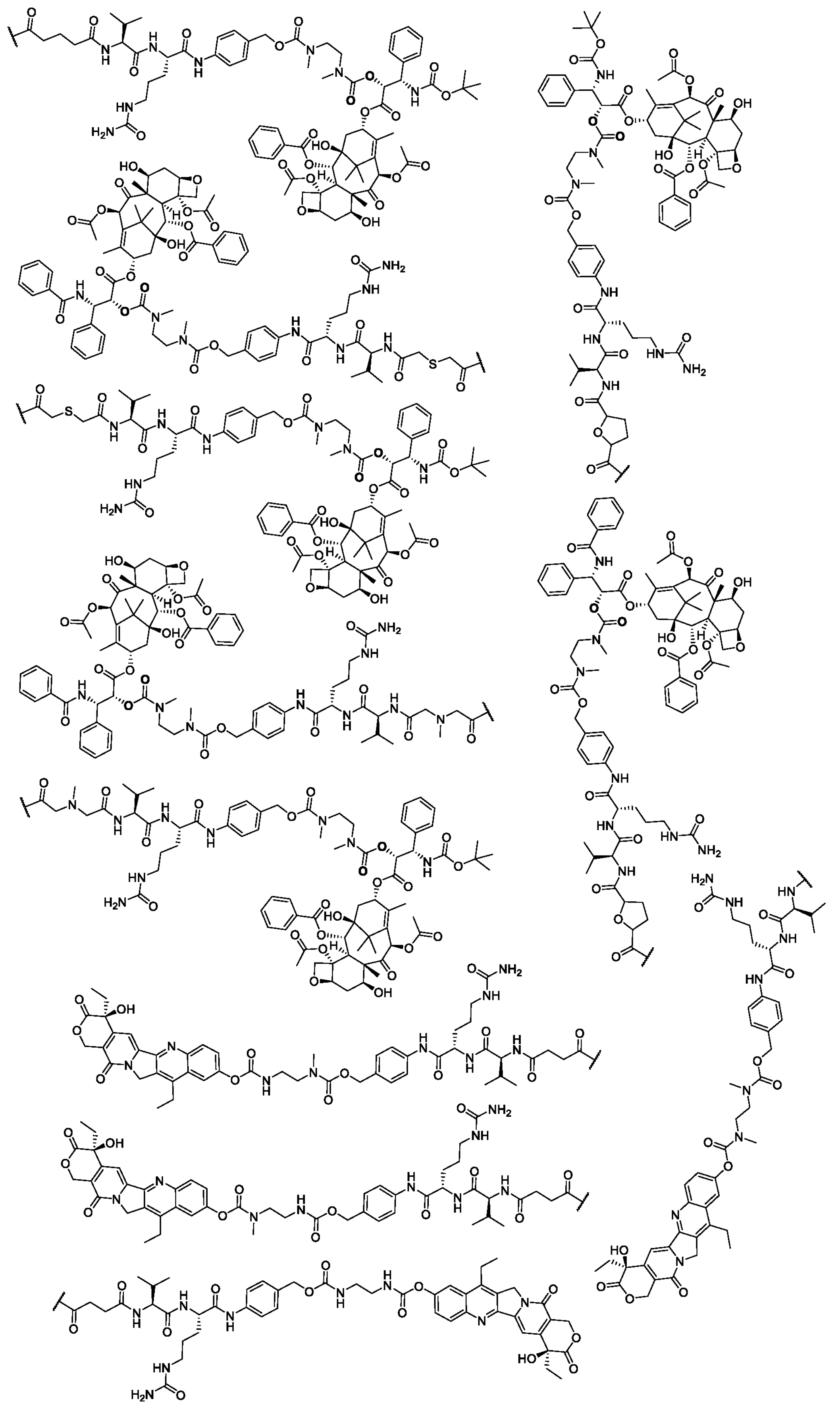

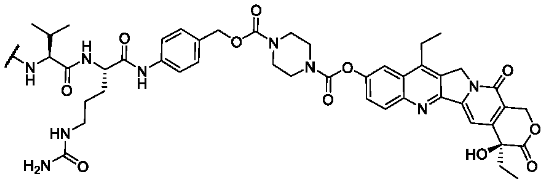

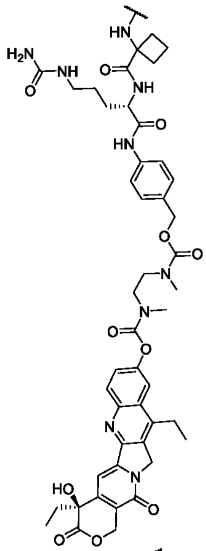

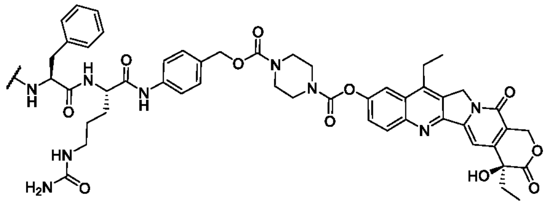

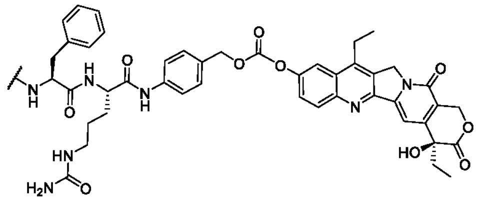

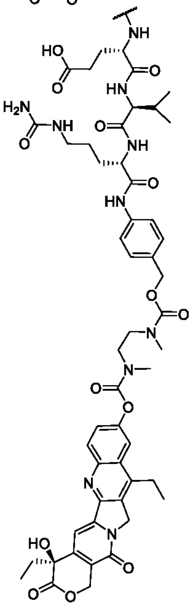

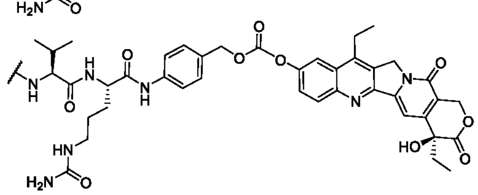

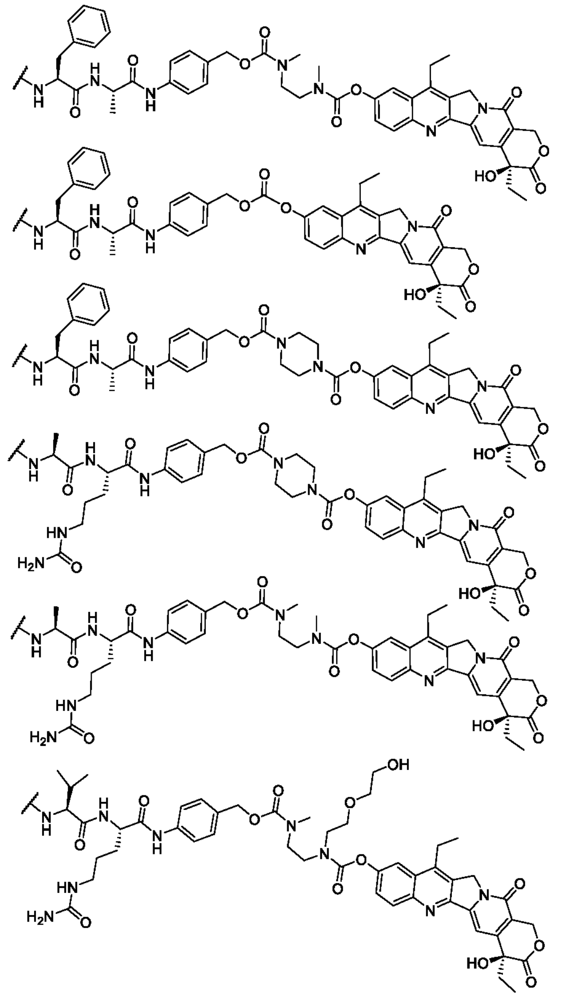

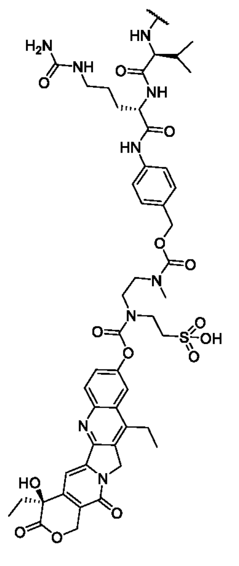

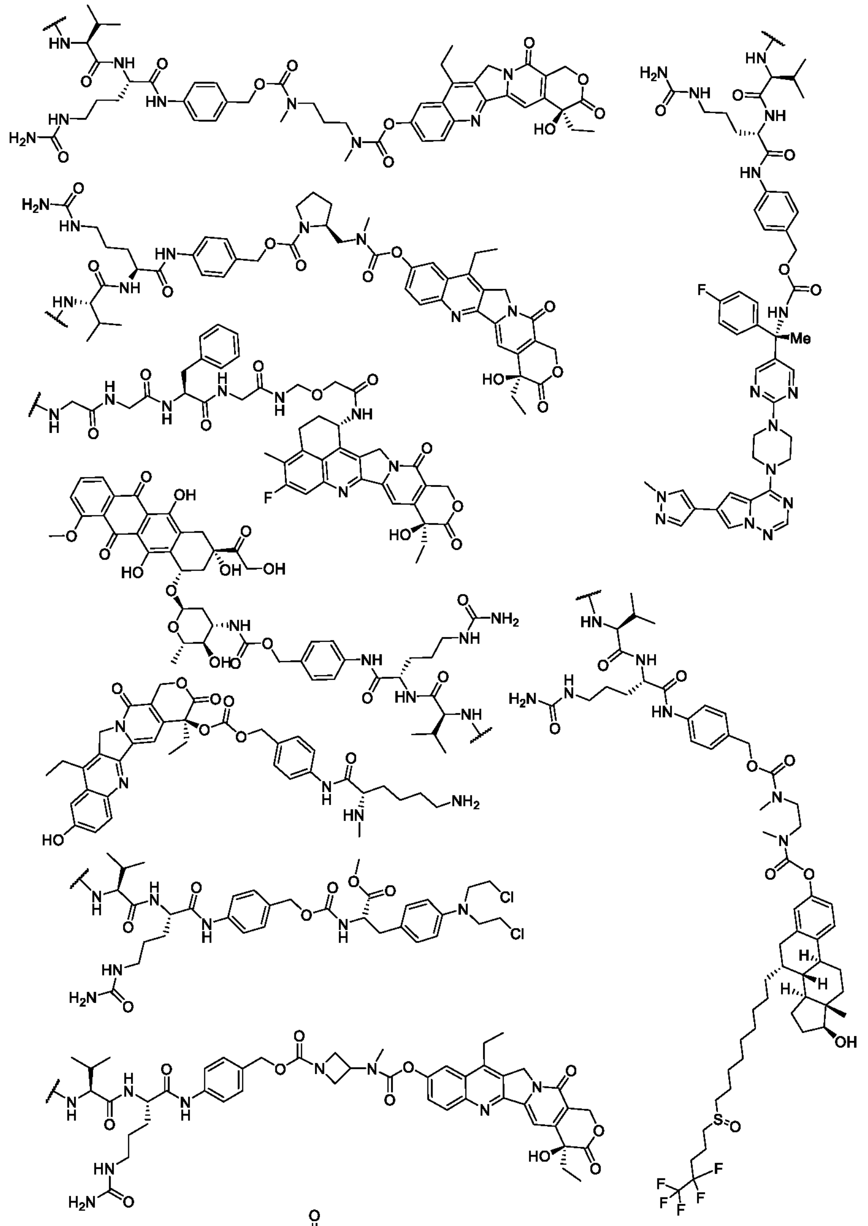

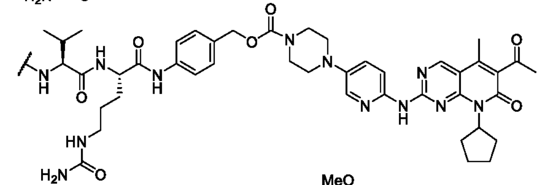

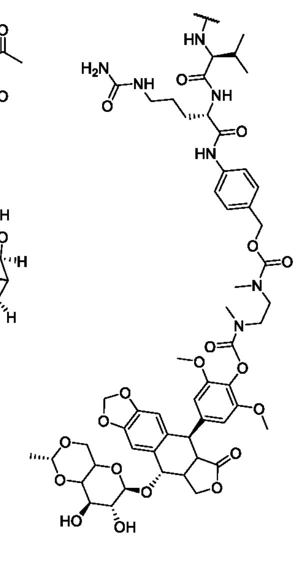

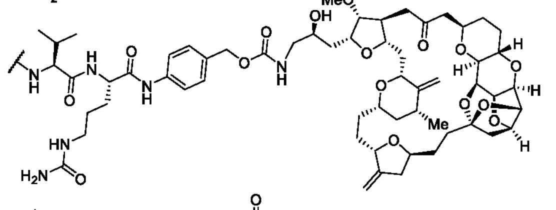

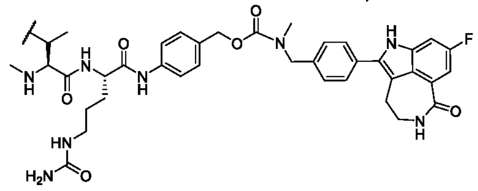

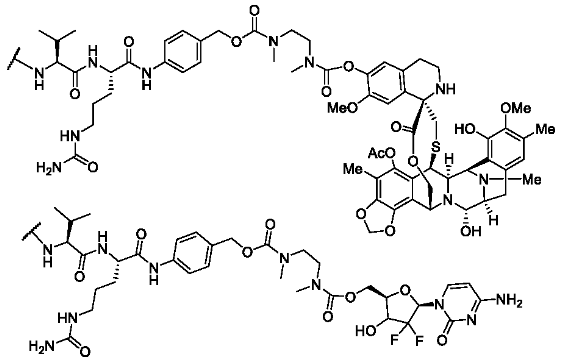

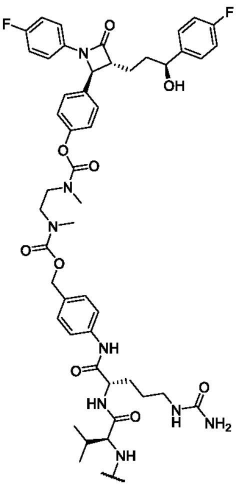

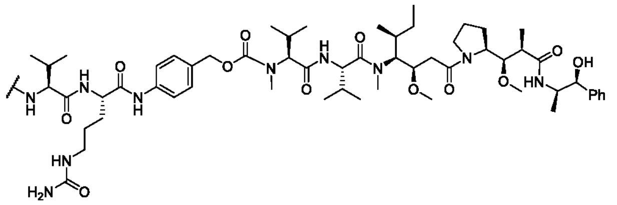

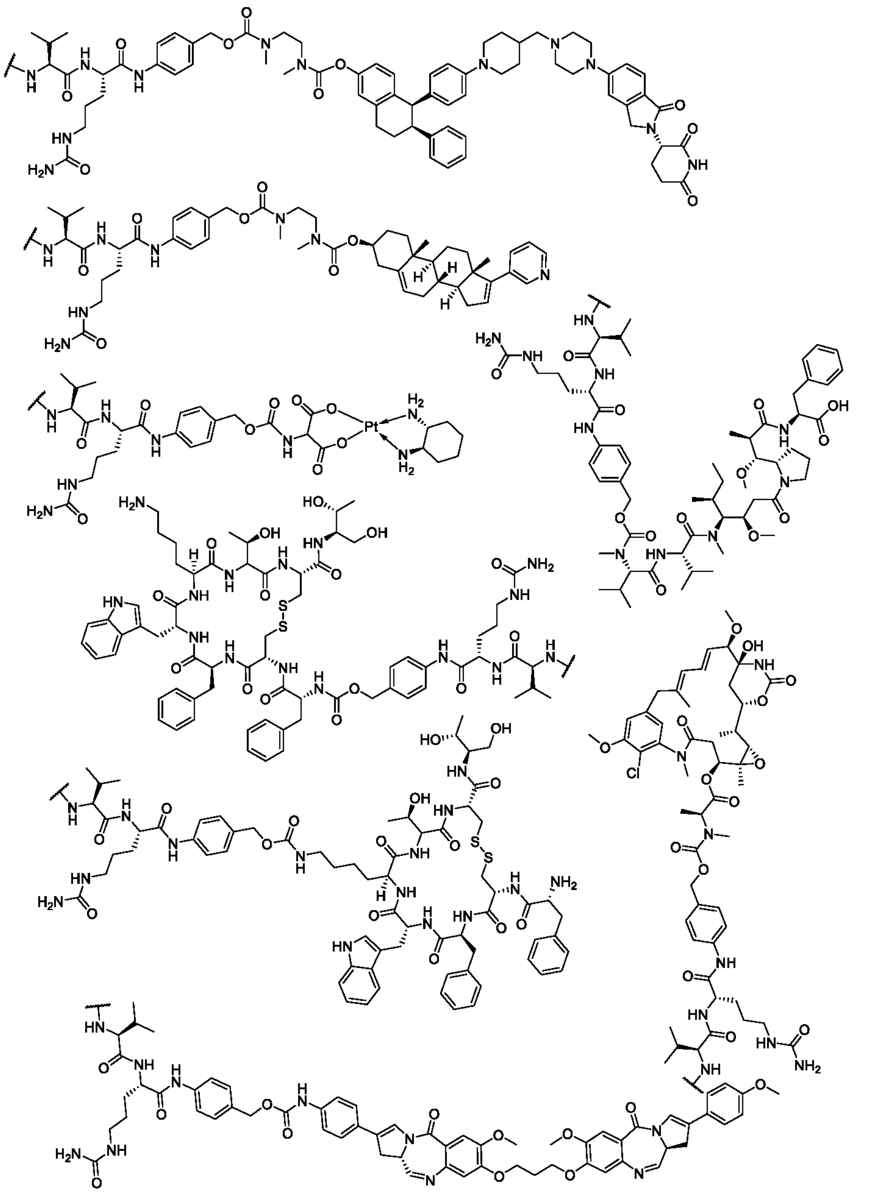

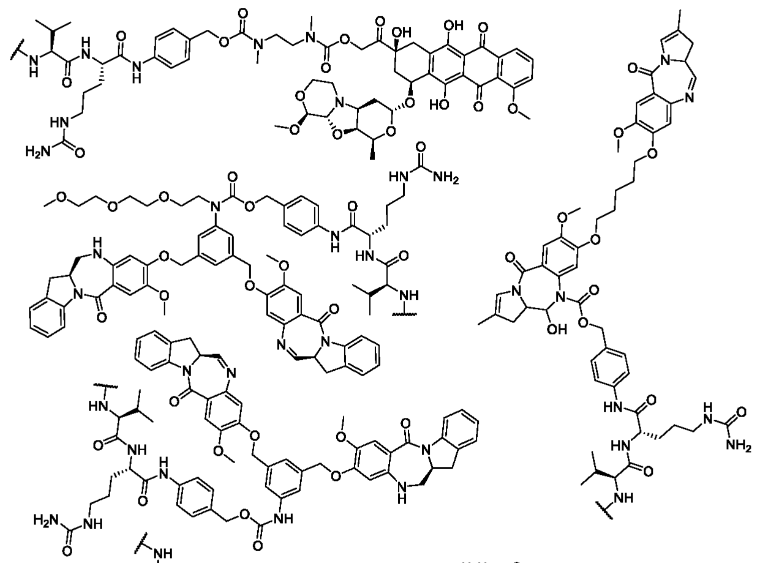

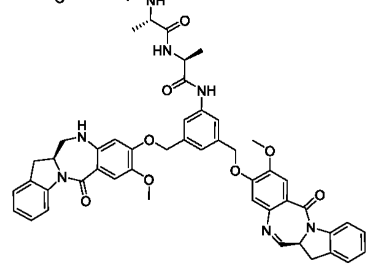

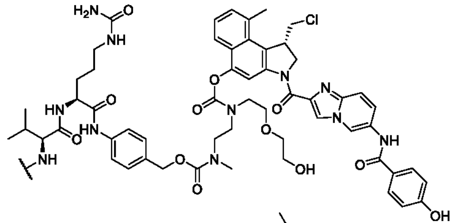

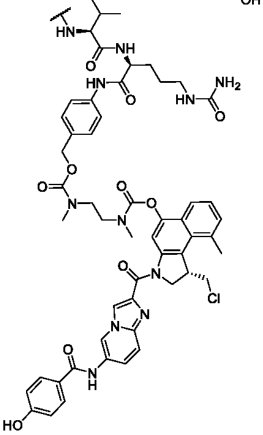

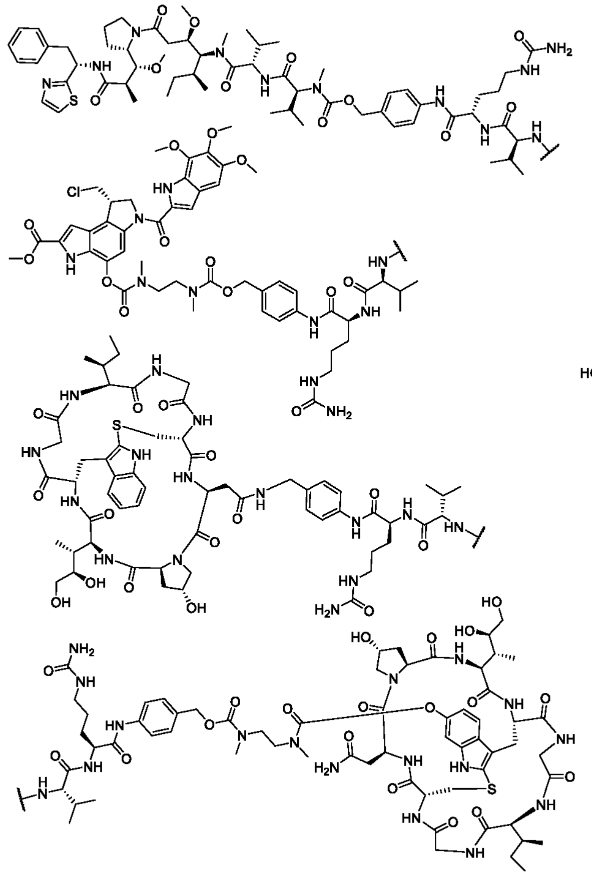

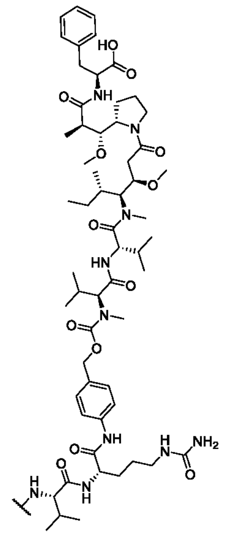

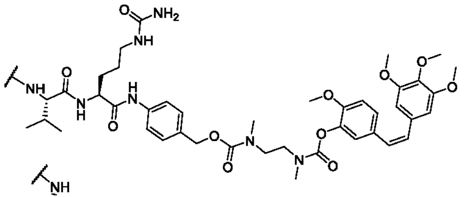

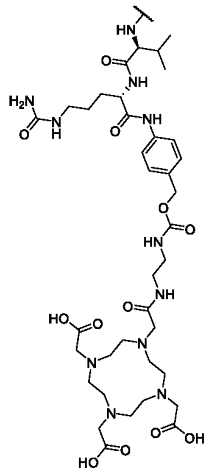

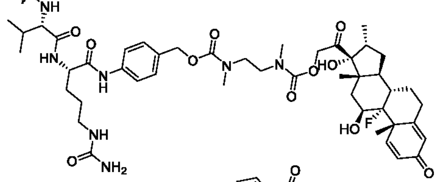

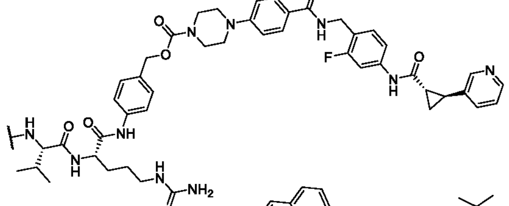

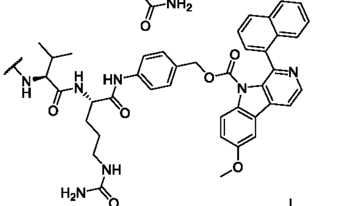

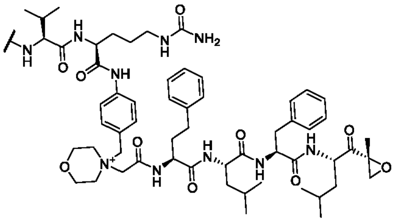

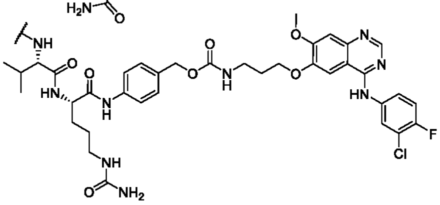

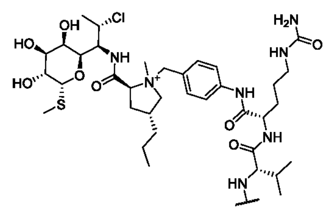

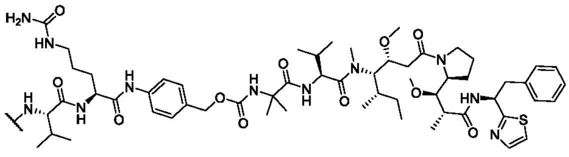

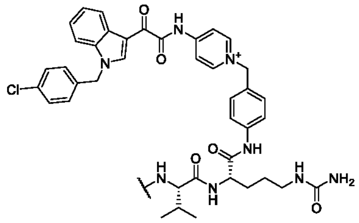

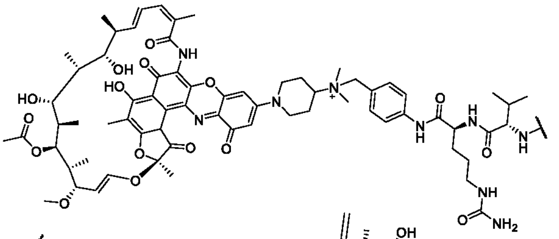

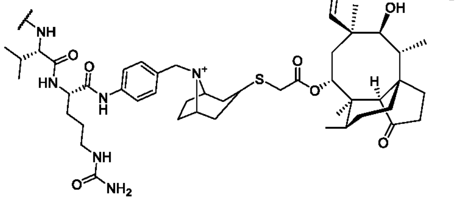

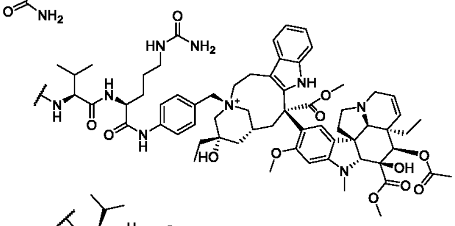

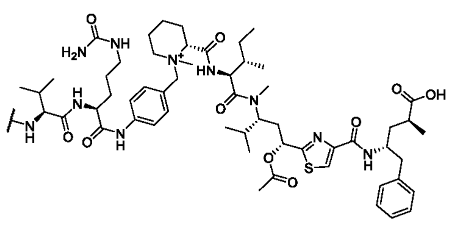

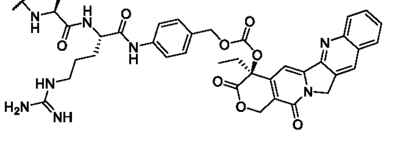

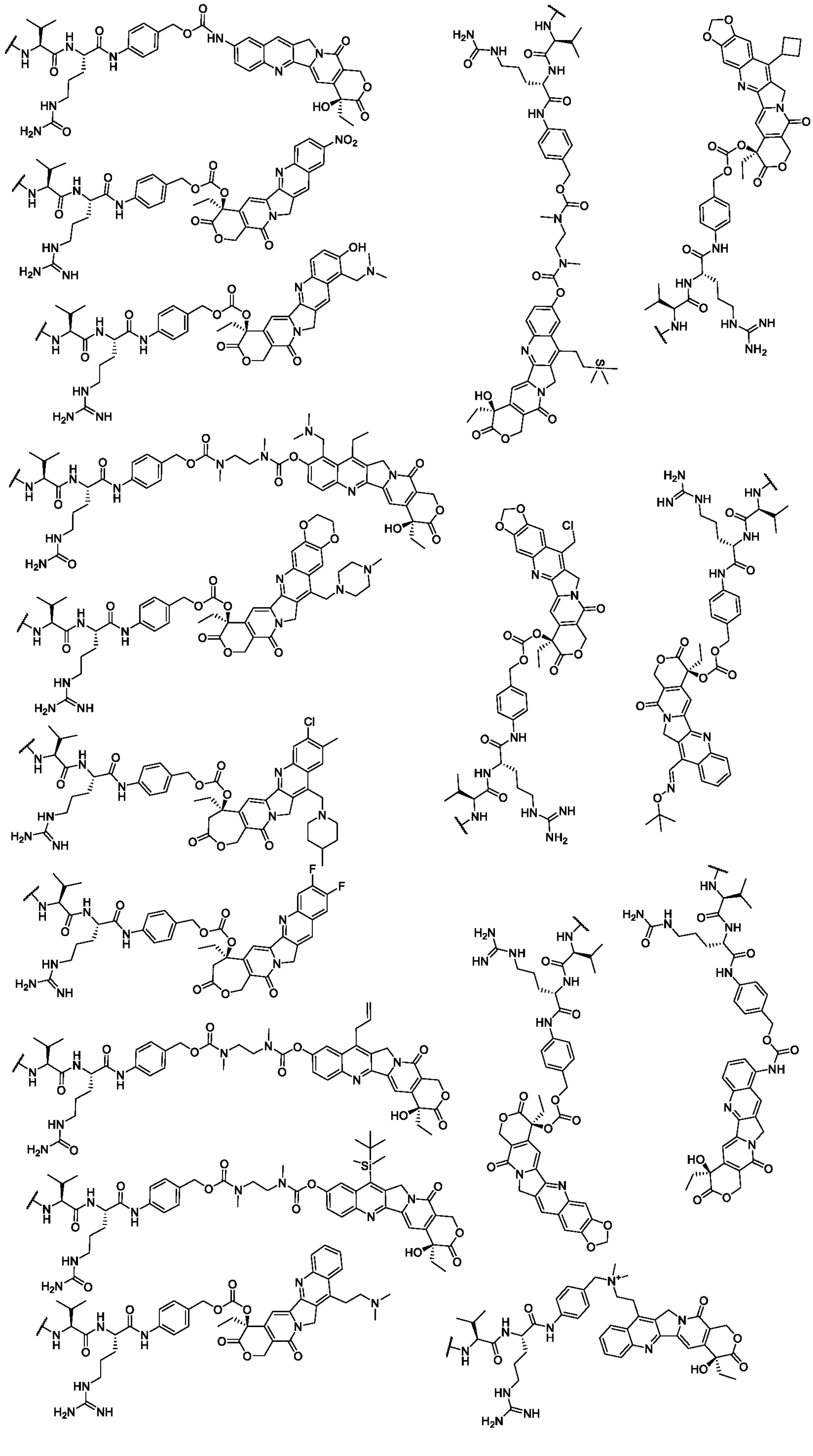

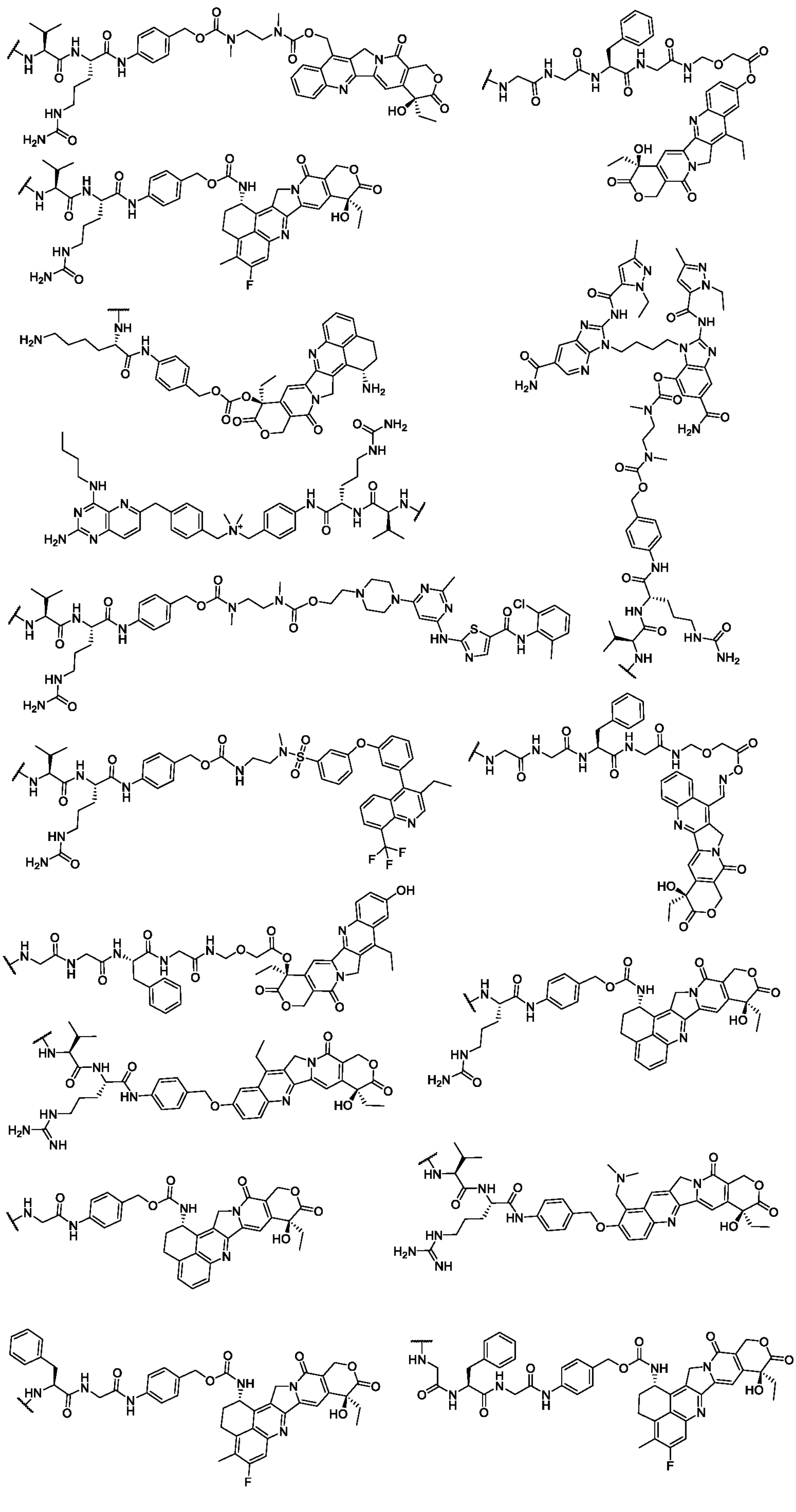

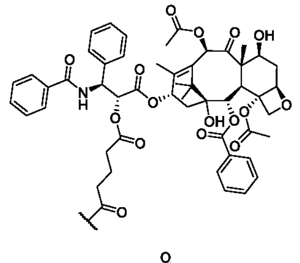
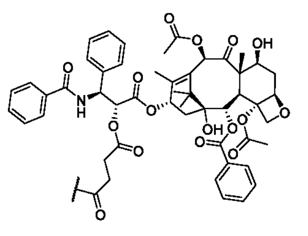
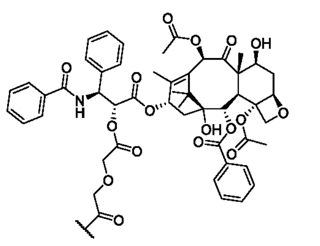
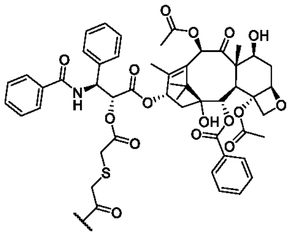
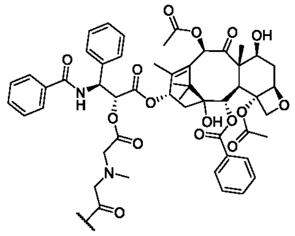
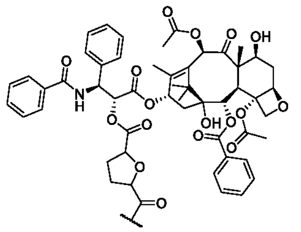
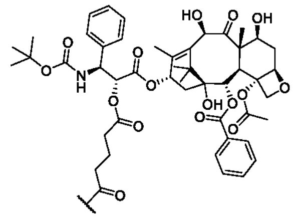
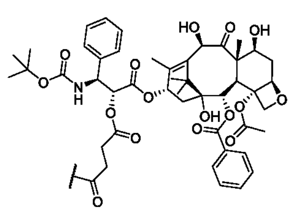
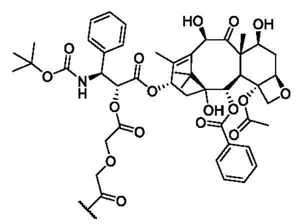
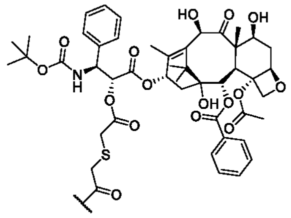
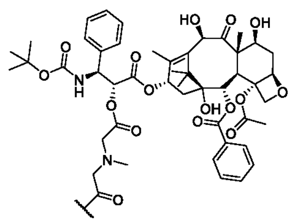
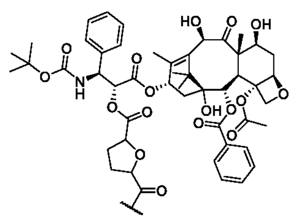
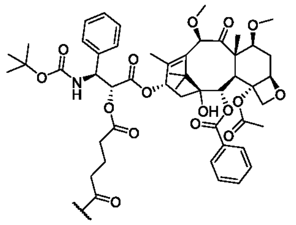
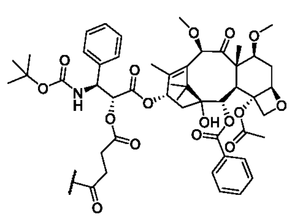
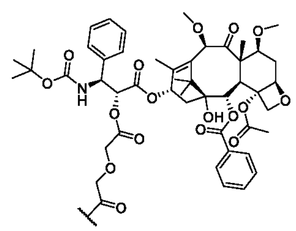
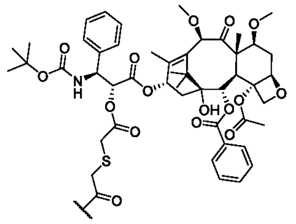
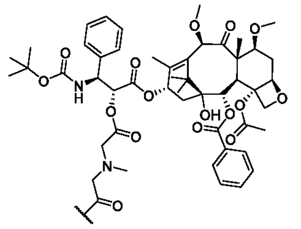
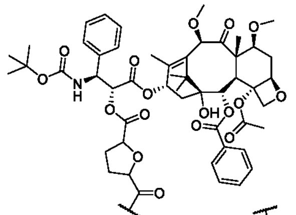
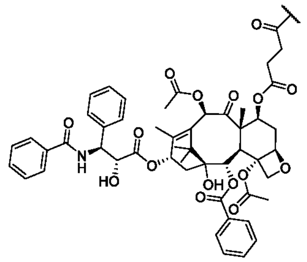
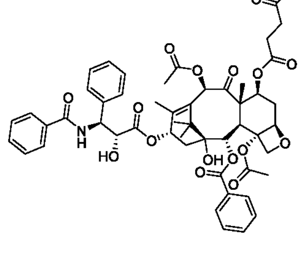
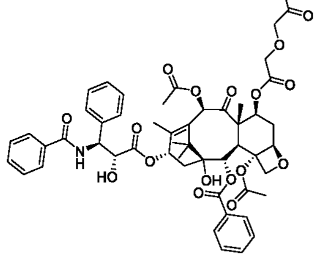

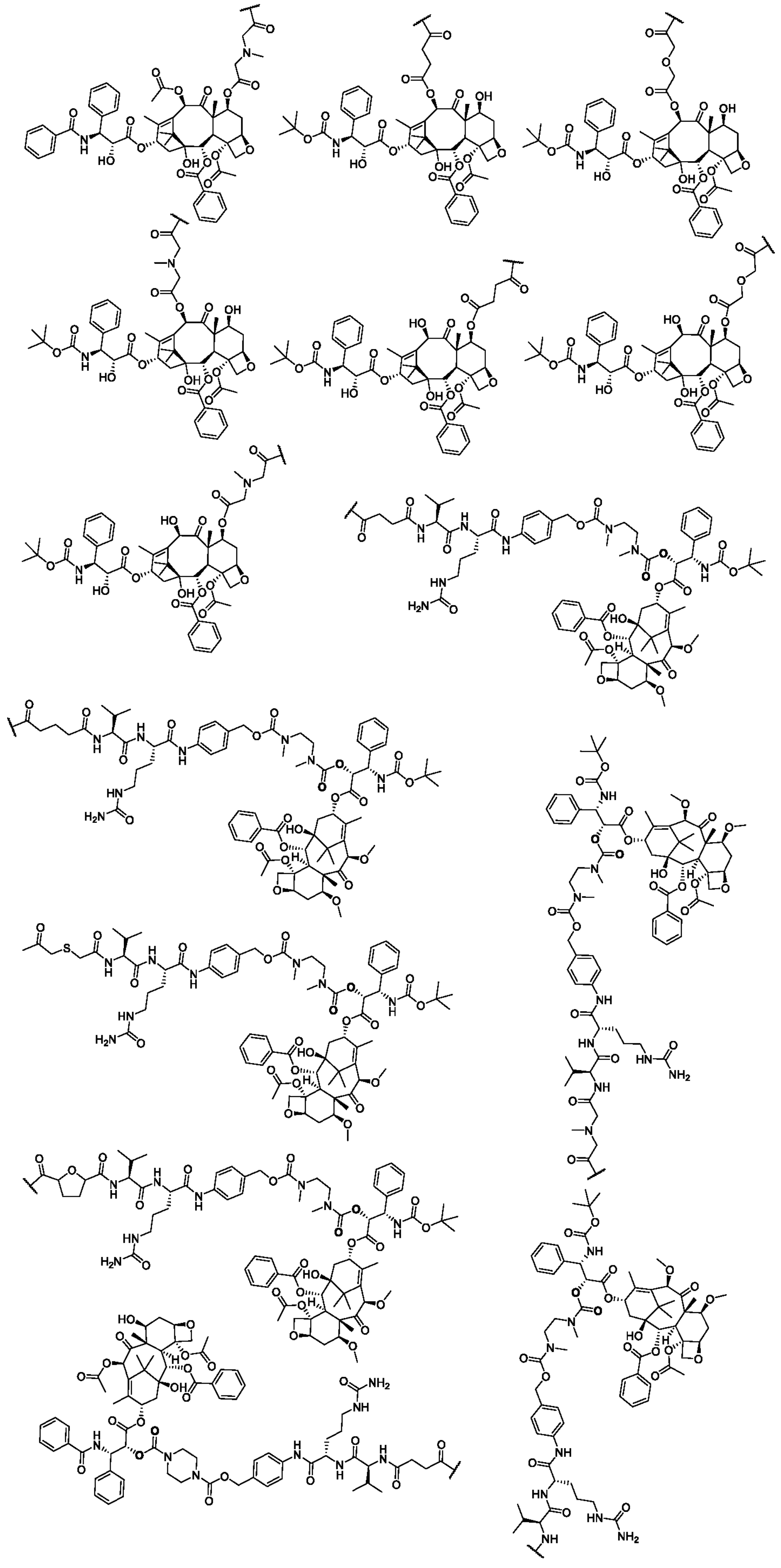

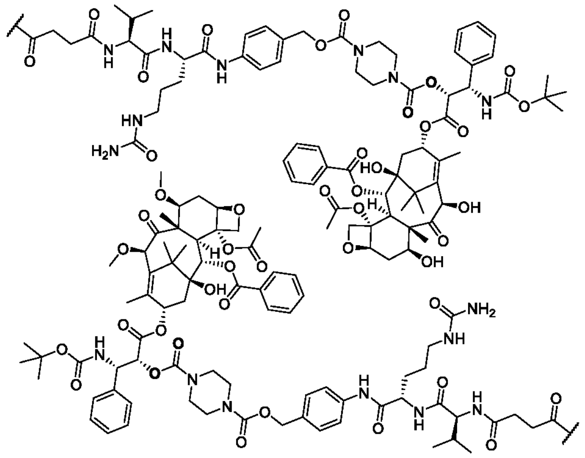

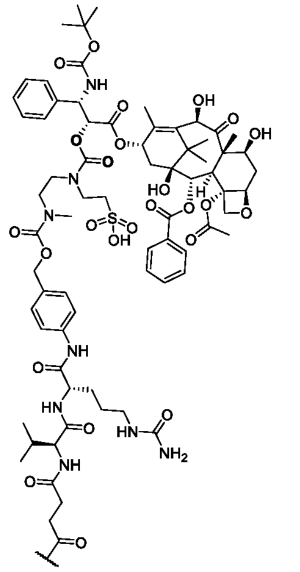

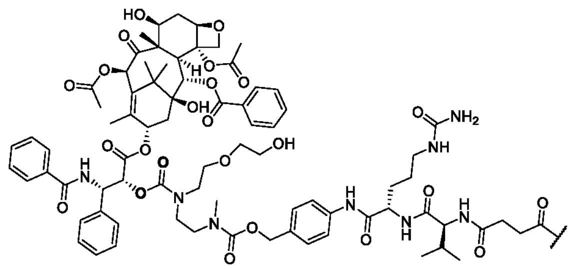

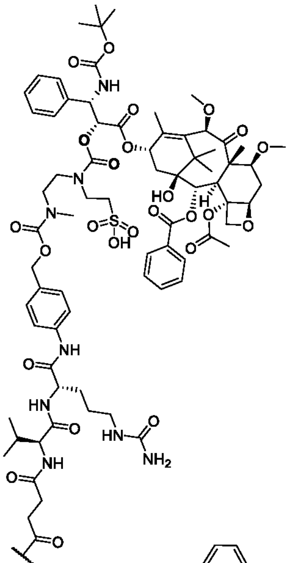

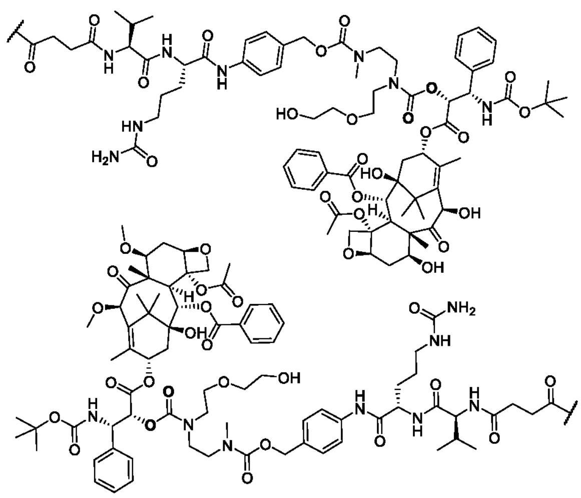

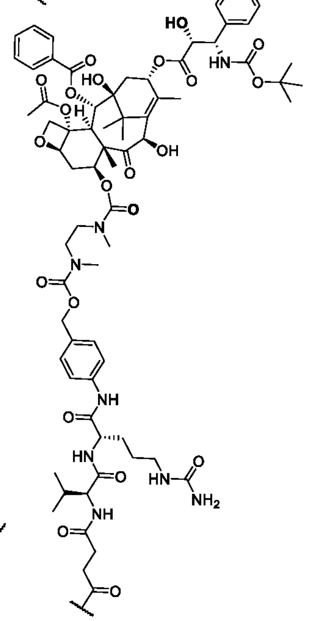

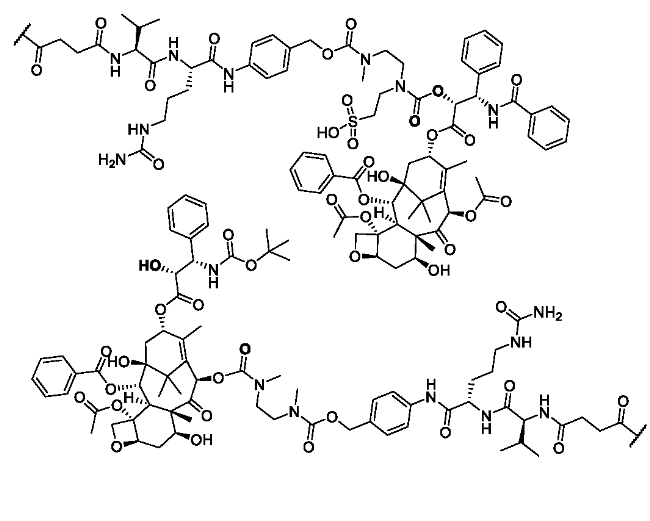

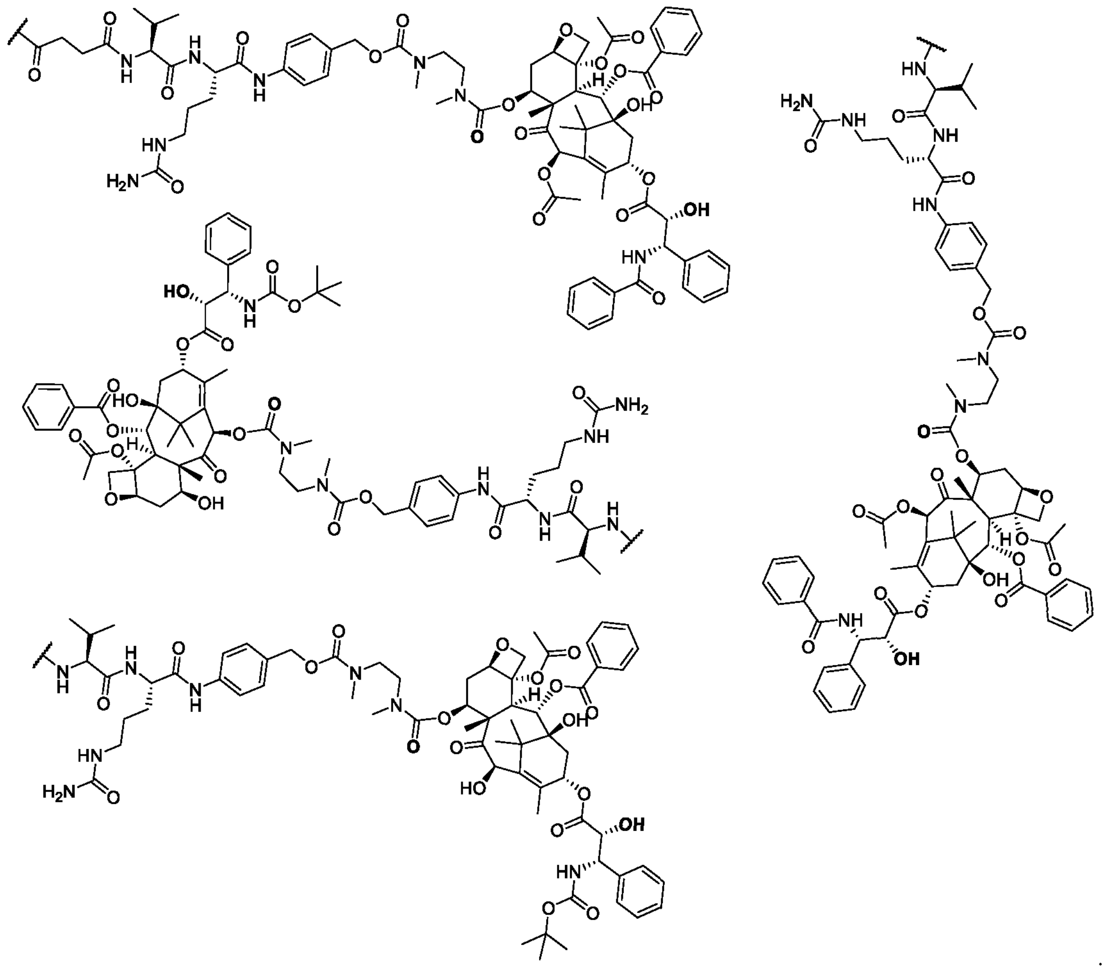

In some embodiments, the poly(ε-L-lysine) derivative-drug conjugate is selected from the following structures:

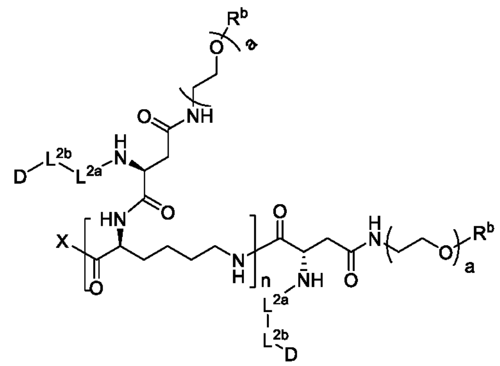

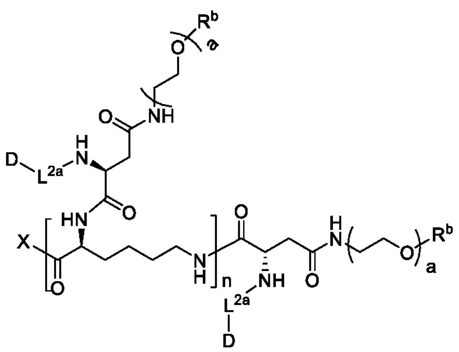

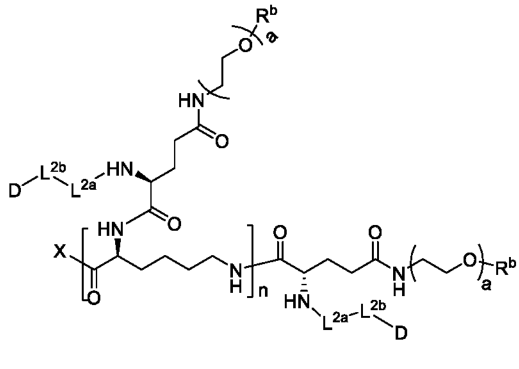

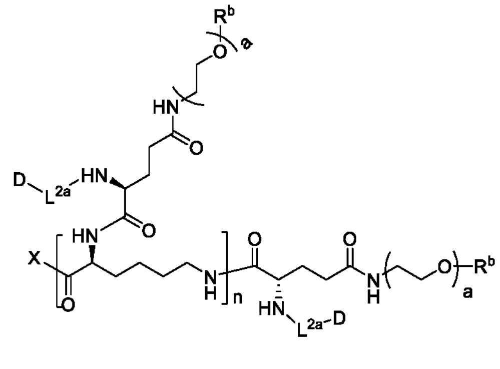

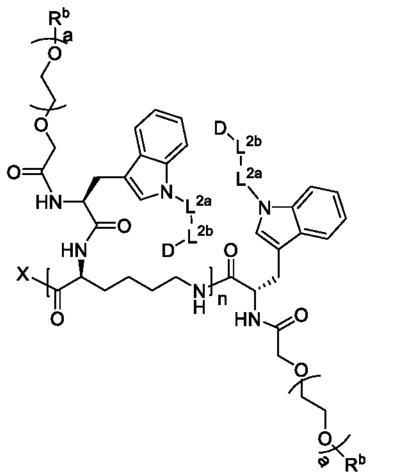

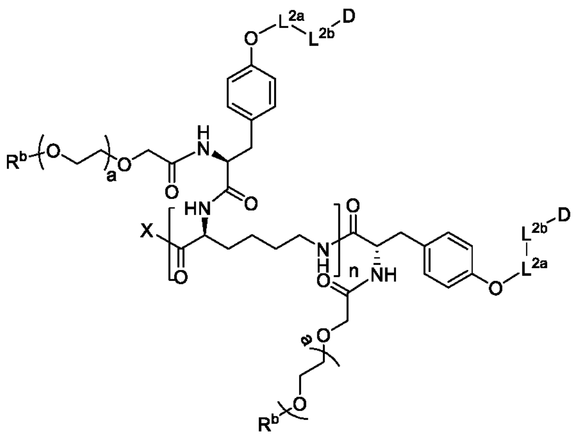

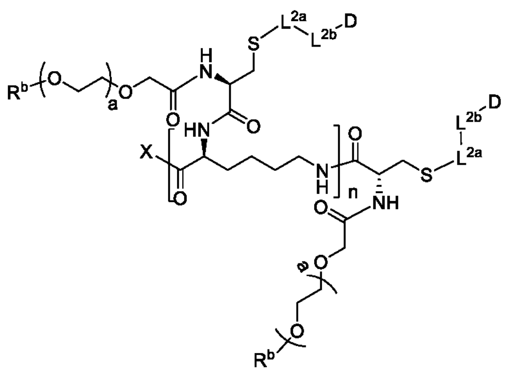

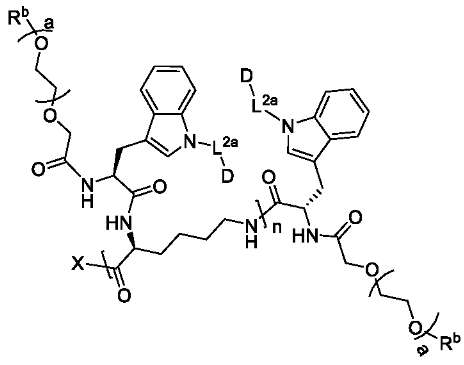

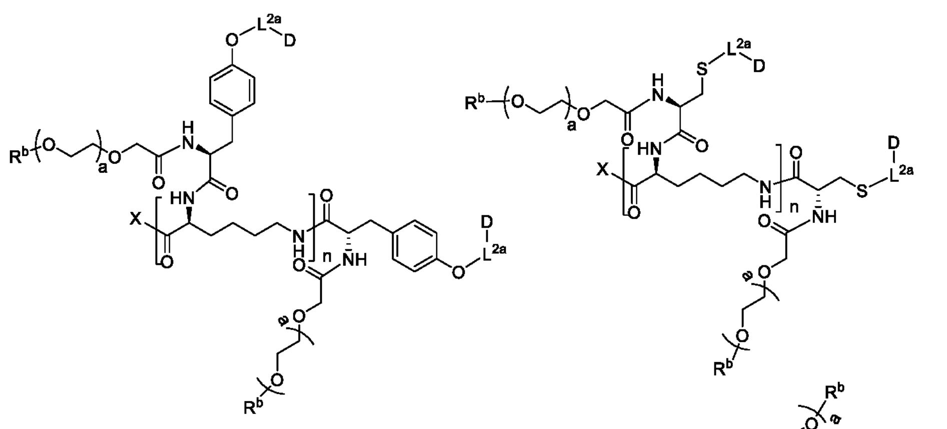

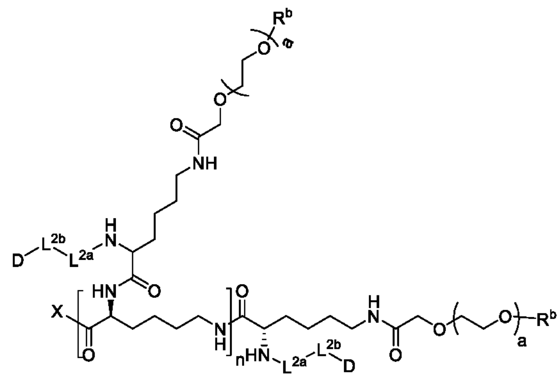

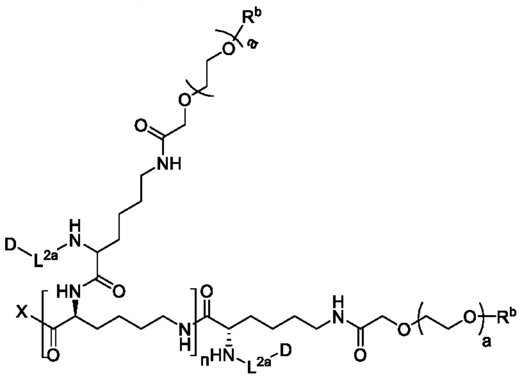

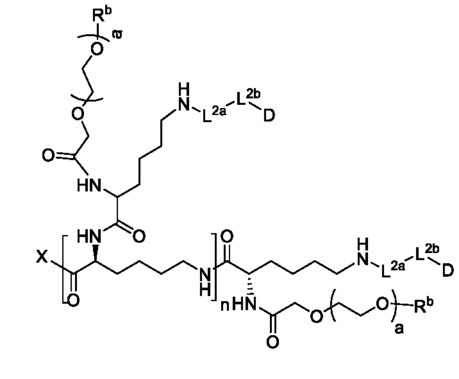

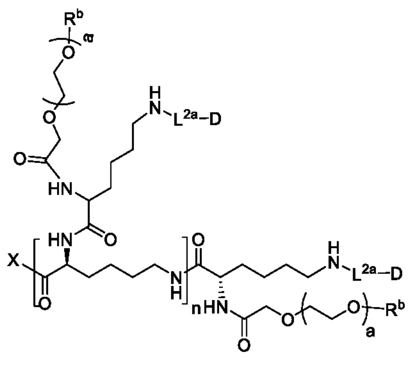

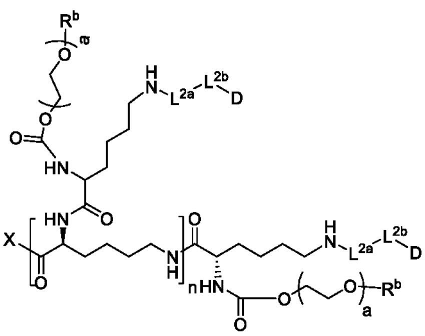

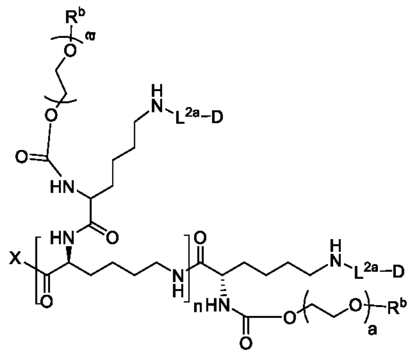

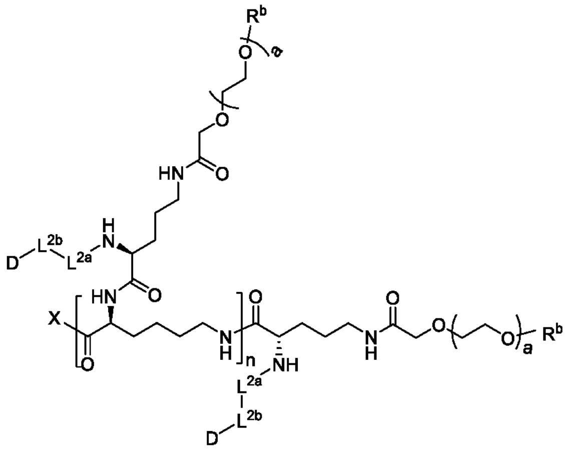

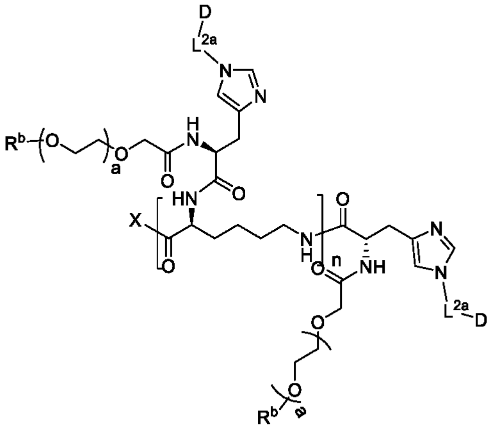

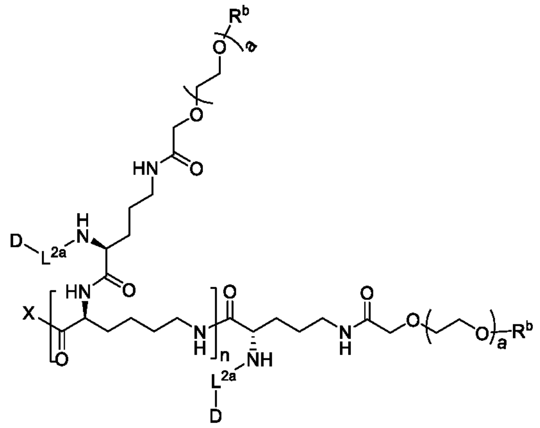

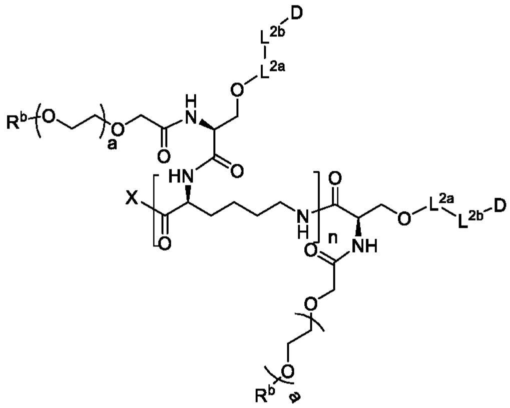

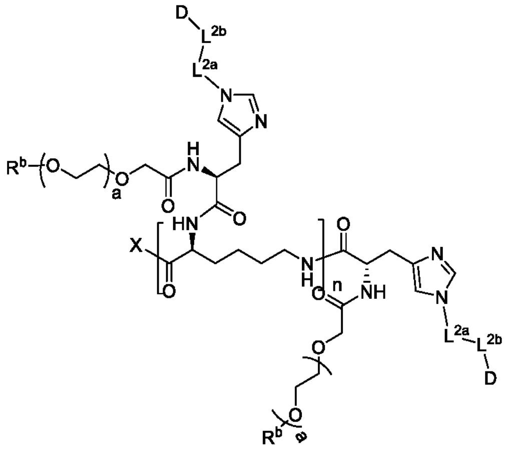

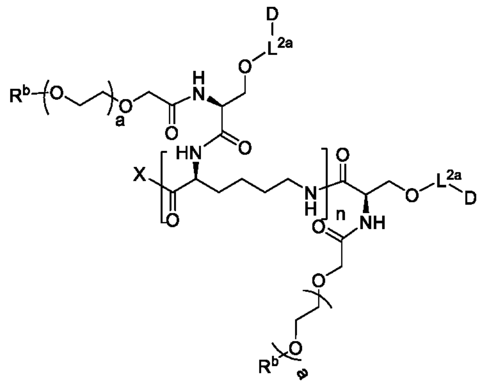

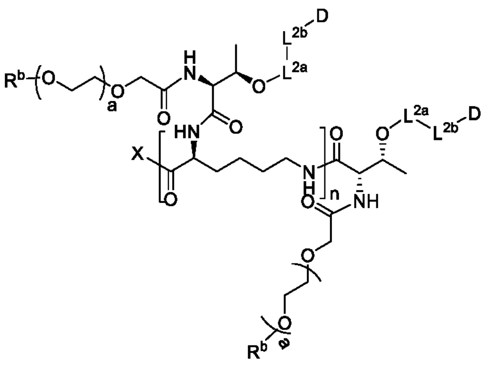

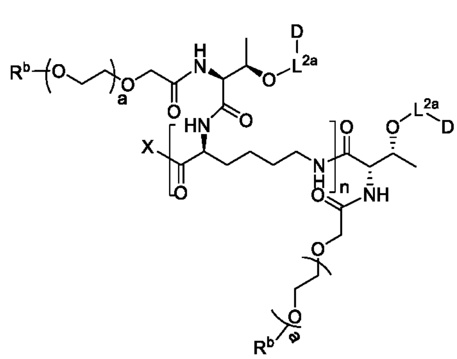

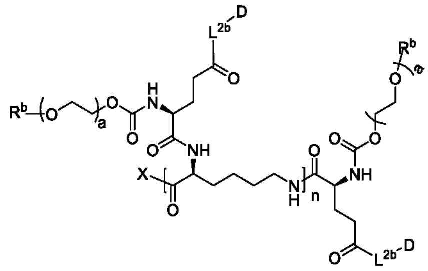

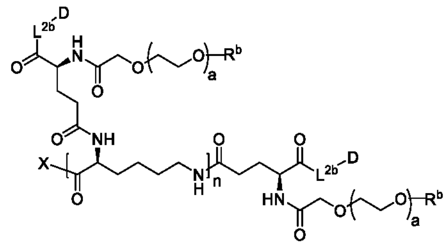

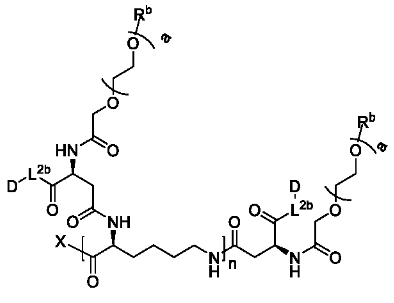

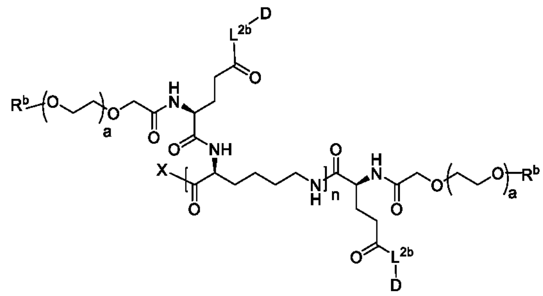

.

In some specific embodiments, $R^b$ is methyl.

In some specific embodiments, a=21 or 22.

In some specific embodiments, a=44 or 45.

In some specific embodiments, X is OH.

In some specific embodiments, X is $L^{2b}$-D.

In some specific embodiments, $L^{2b}$ is a linker containing an enzyme-responsive functional group.

In some specific embodiments, $L^{2b}$ is a linker containing a pH-responsive functional group.

In some specific embodiments, D is a residue of a targeted drug.

In some specific embodiments, D is a residue of a chemotherapy drug.

In some specific embodiments, the poly(ε-L-lysine) derivative-drug conjugate is selected from the following structures:

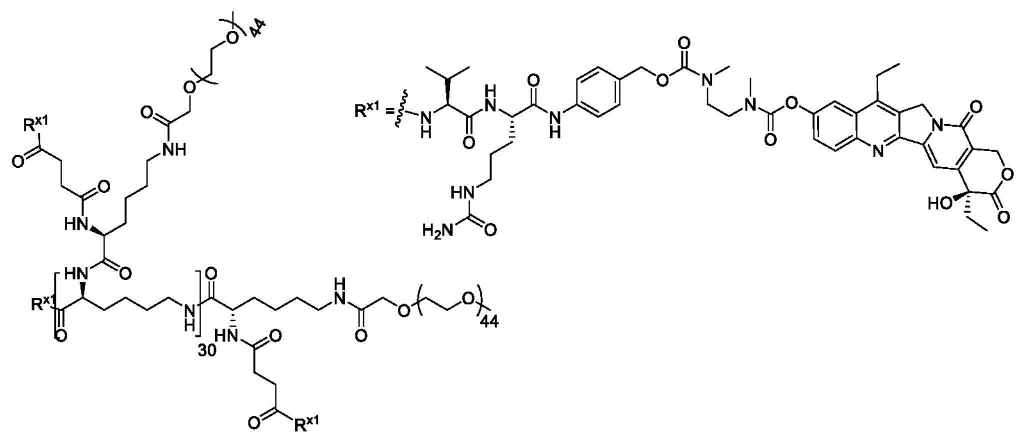

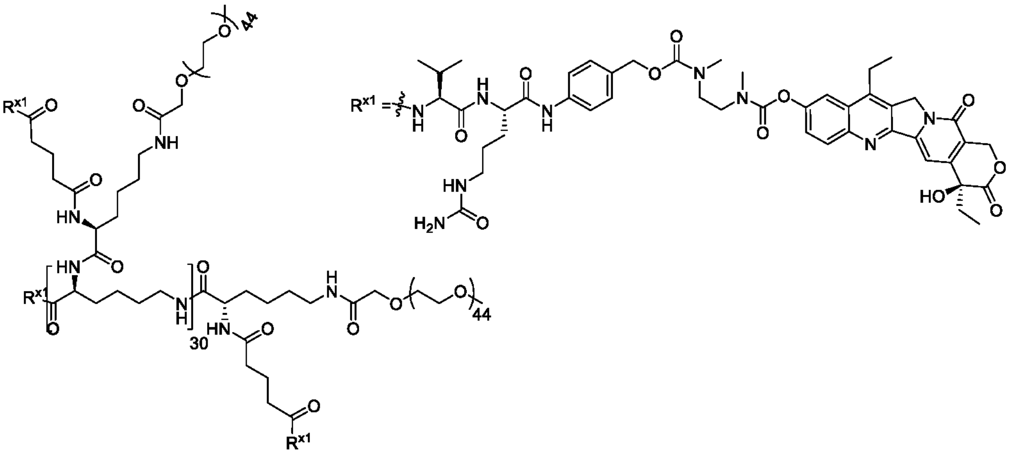

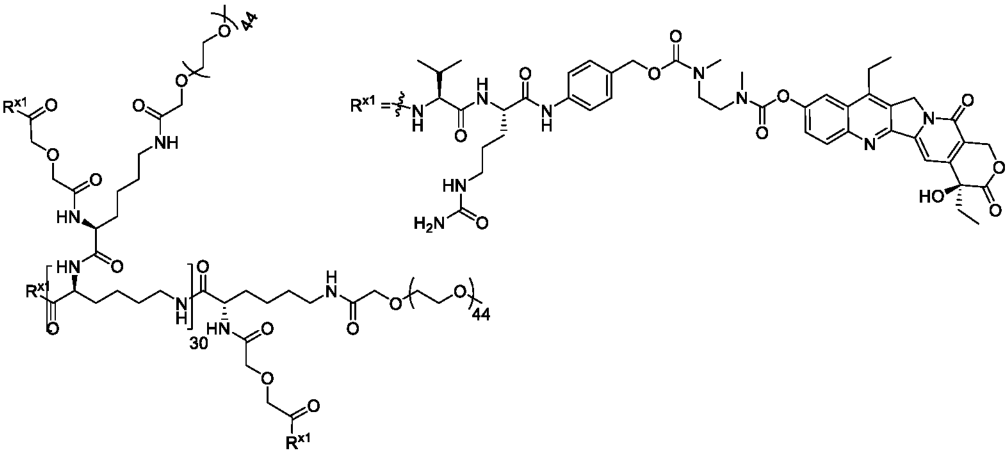

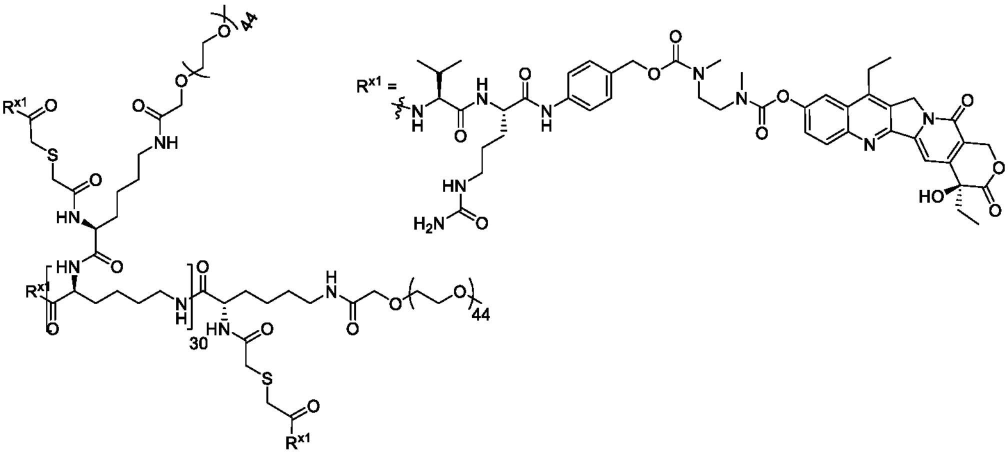

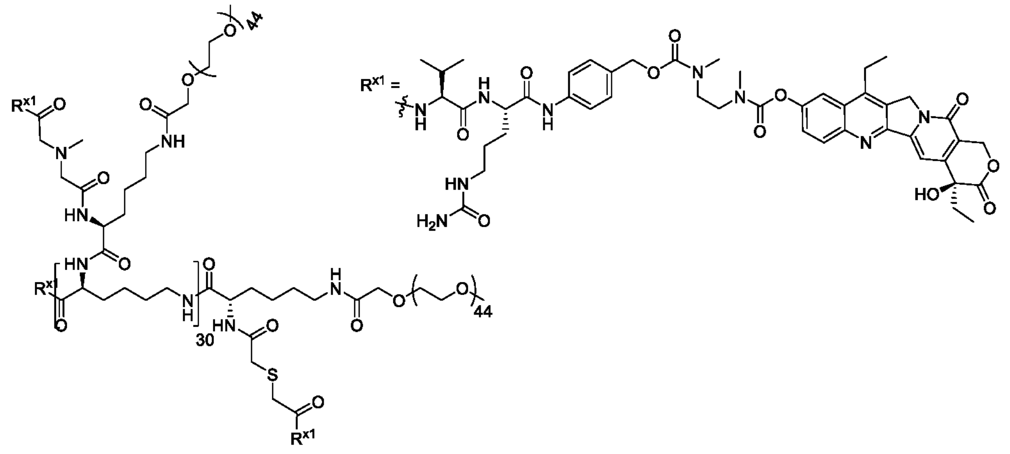

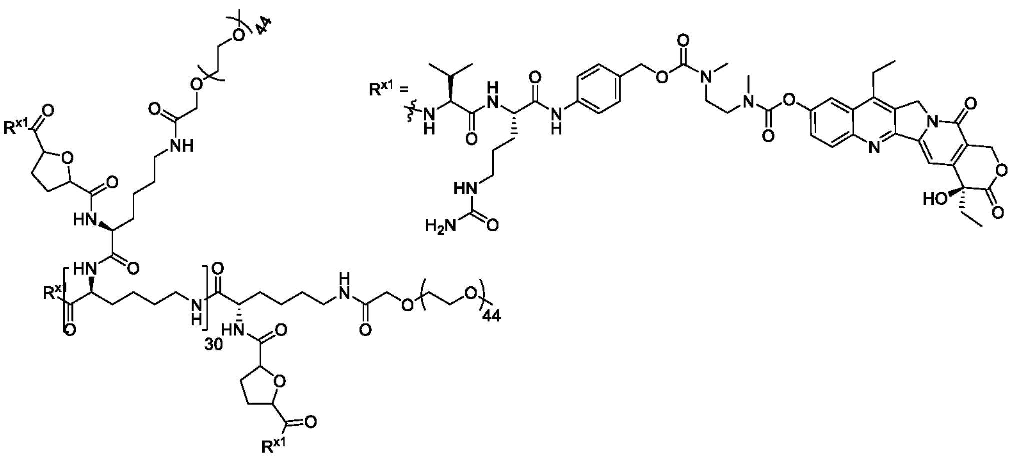

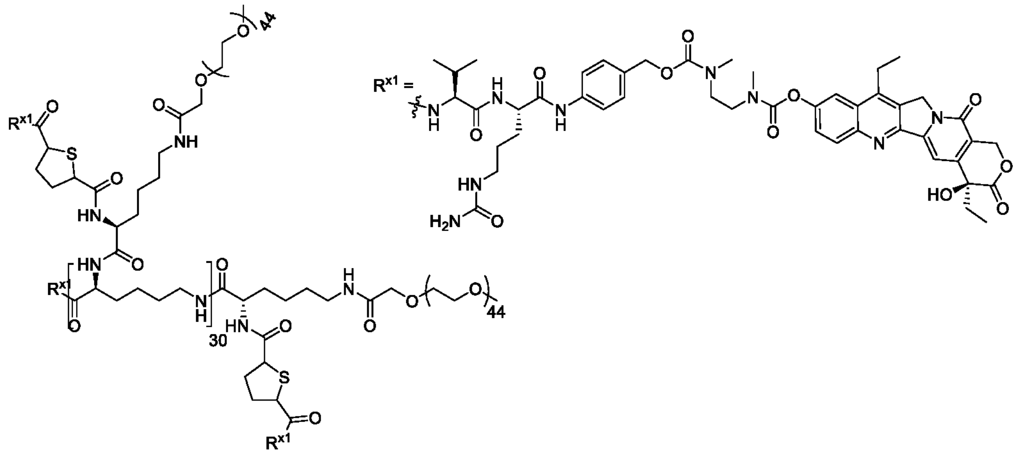

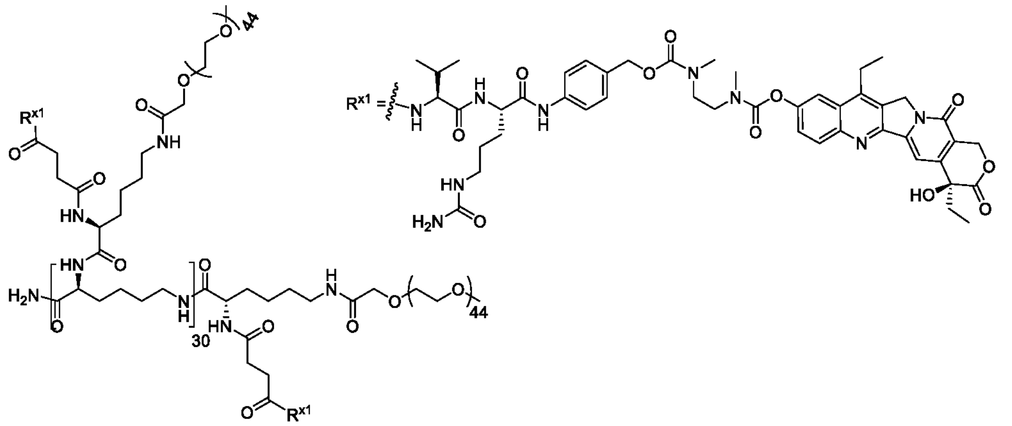

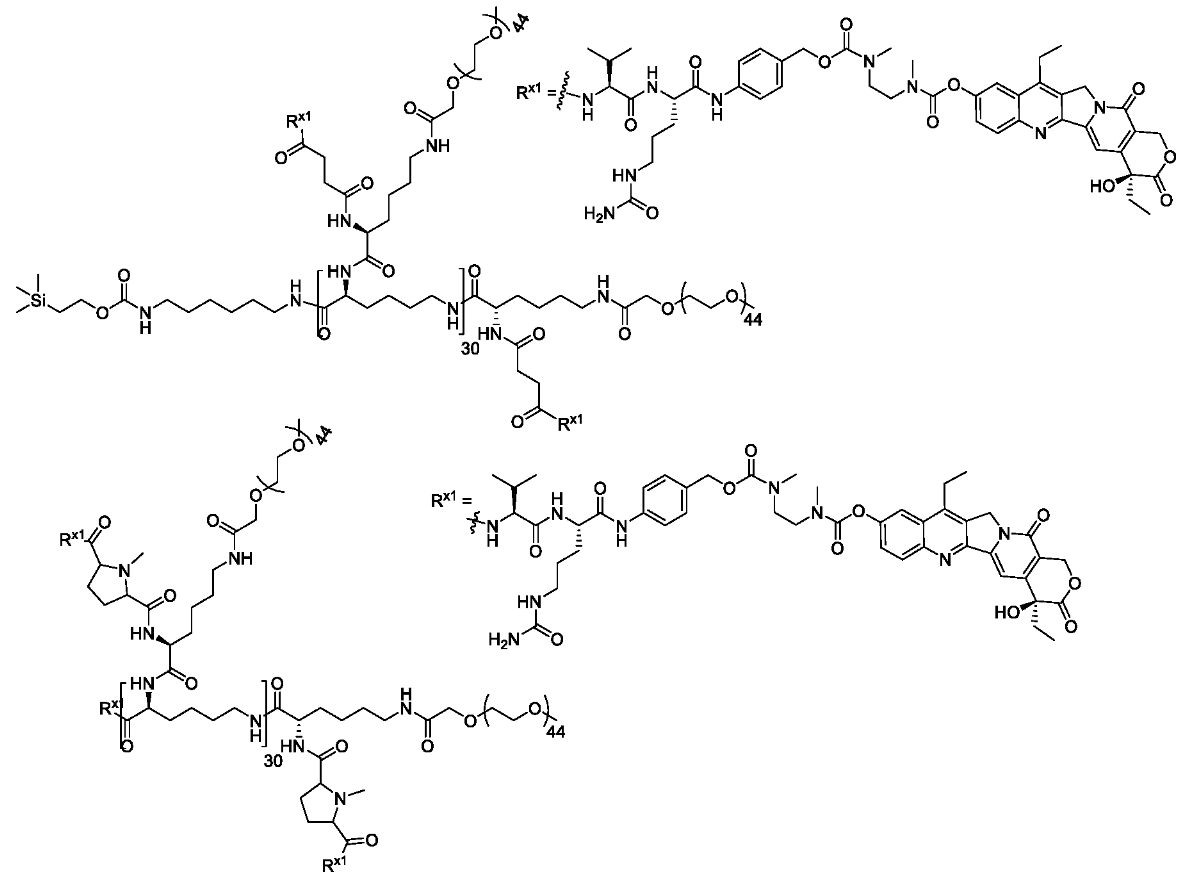

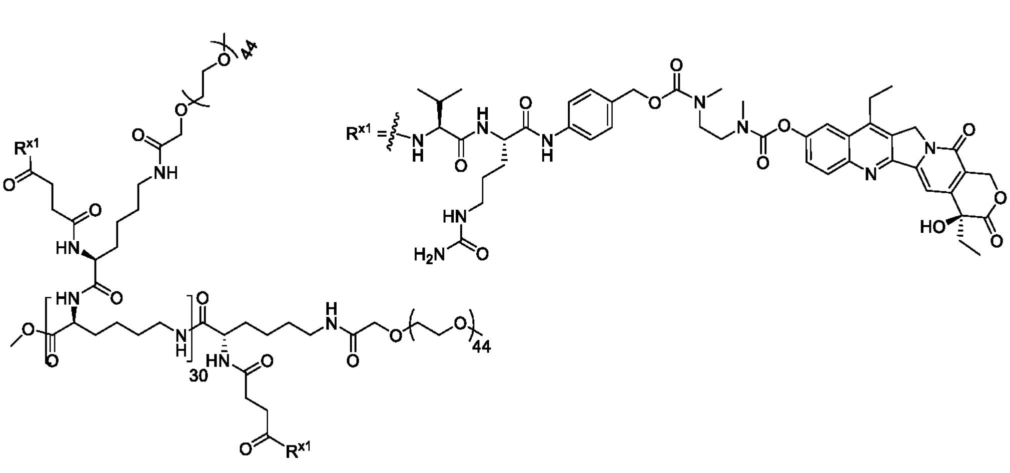

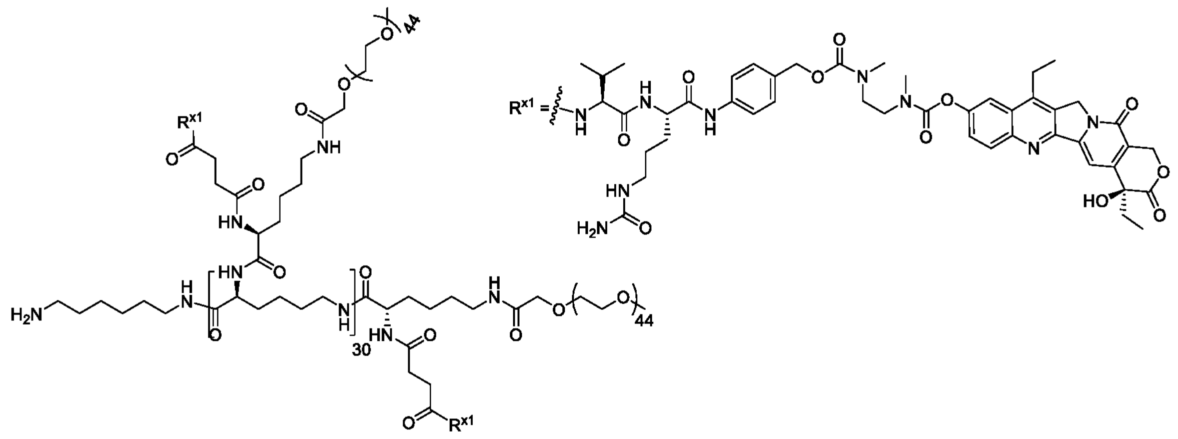

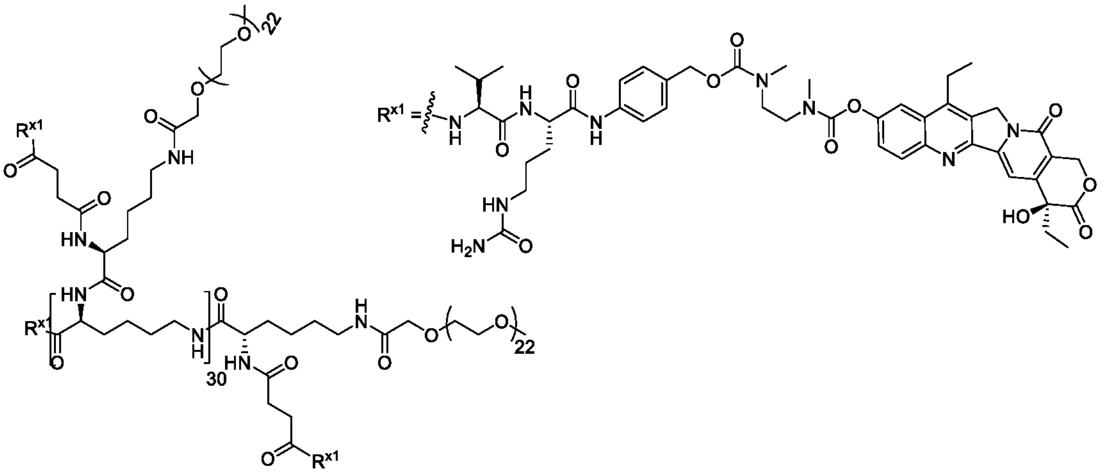

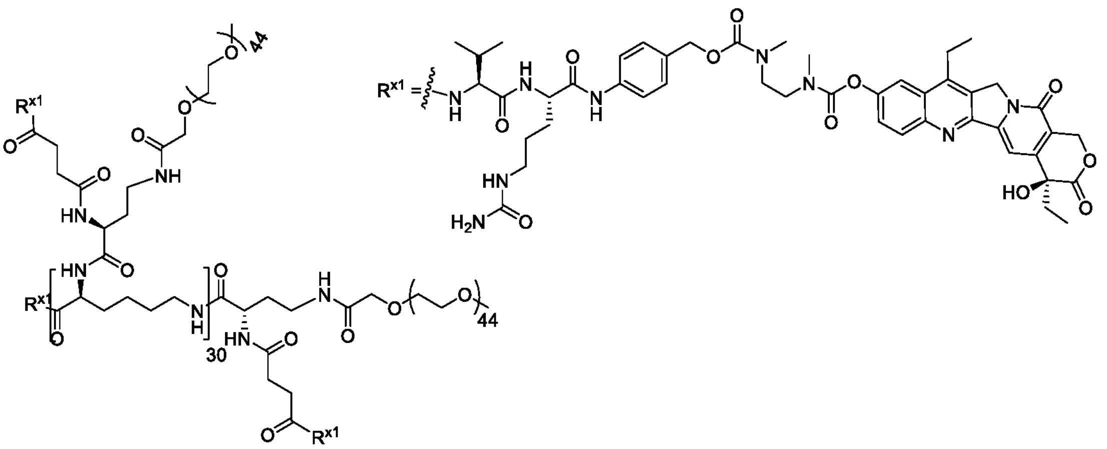

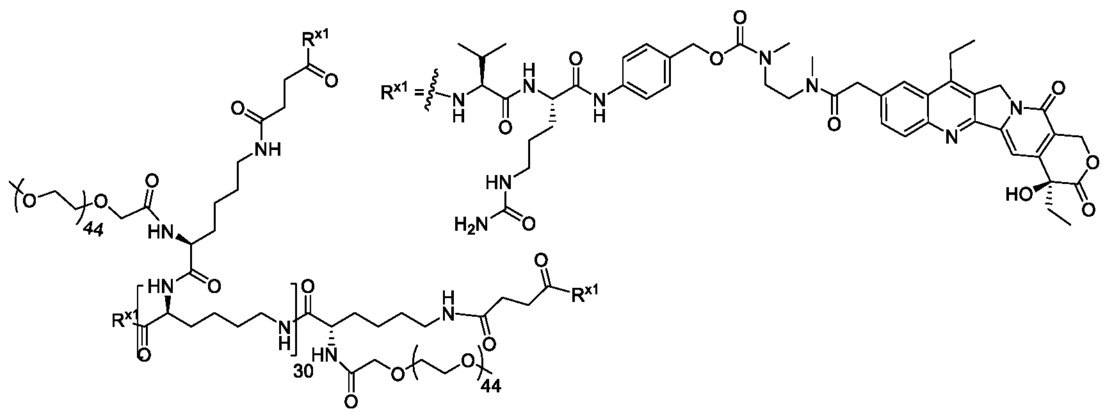

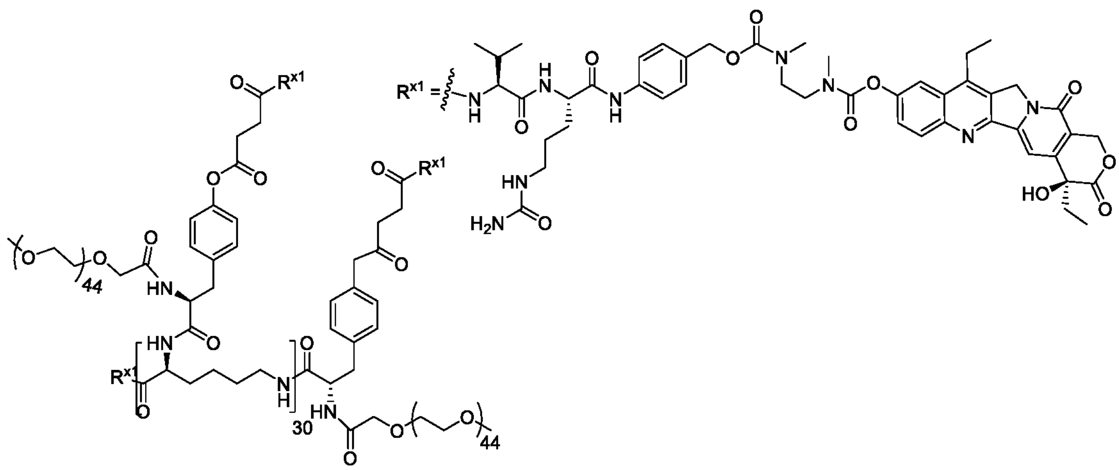

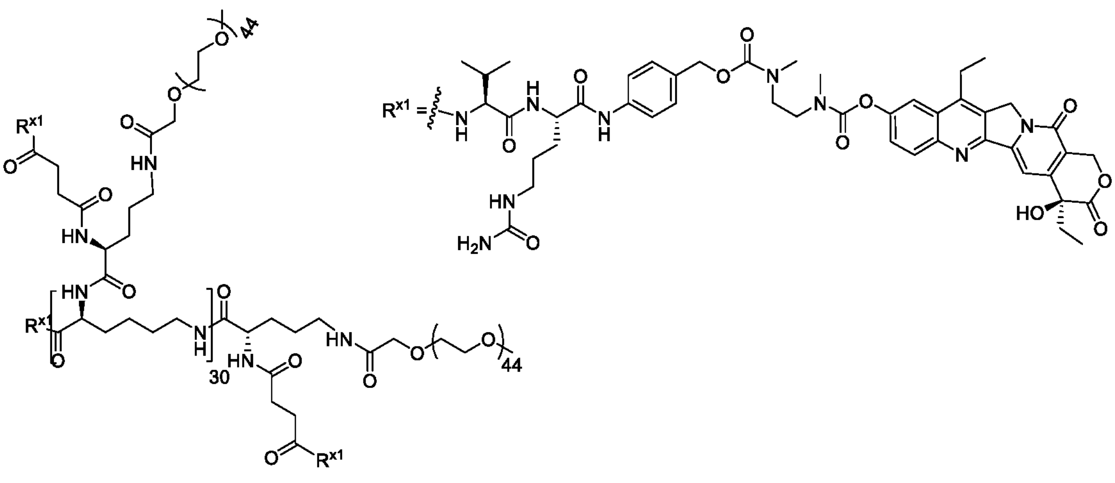

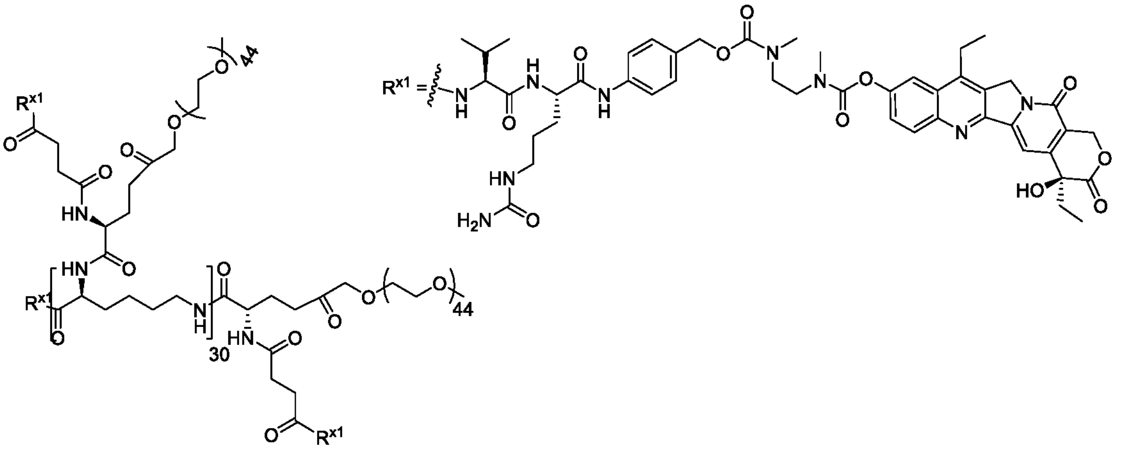

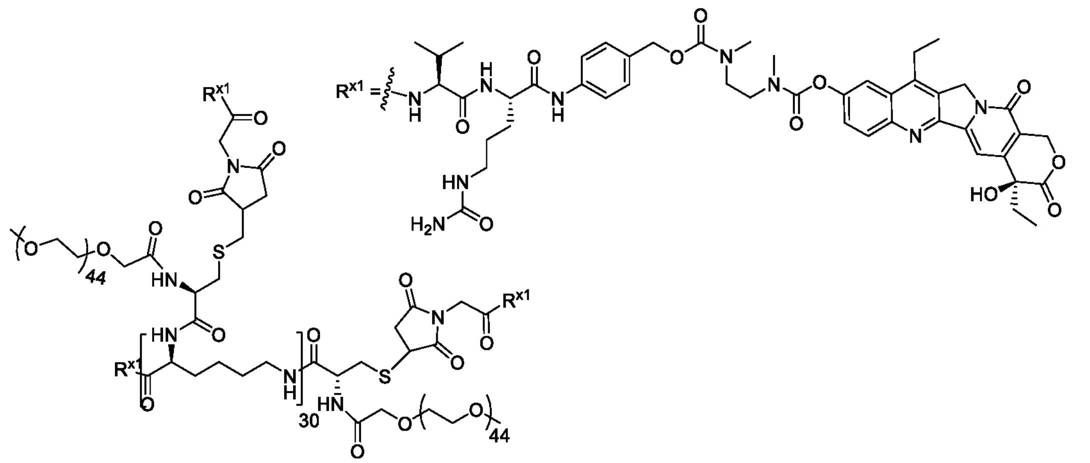

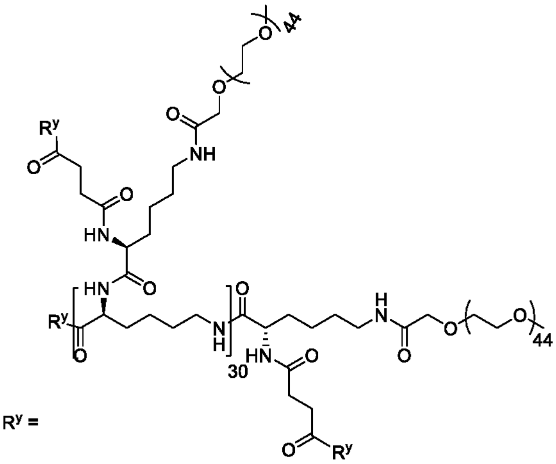

$R^y$ =

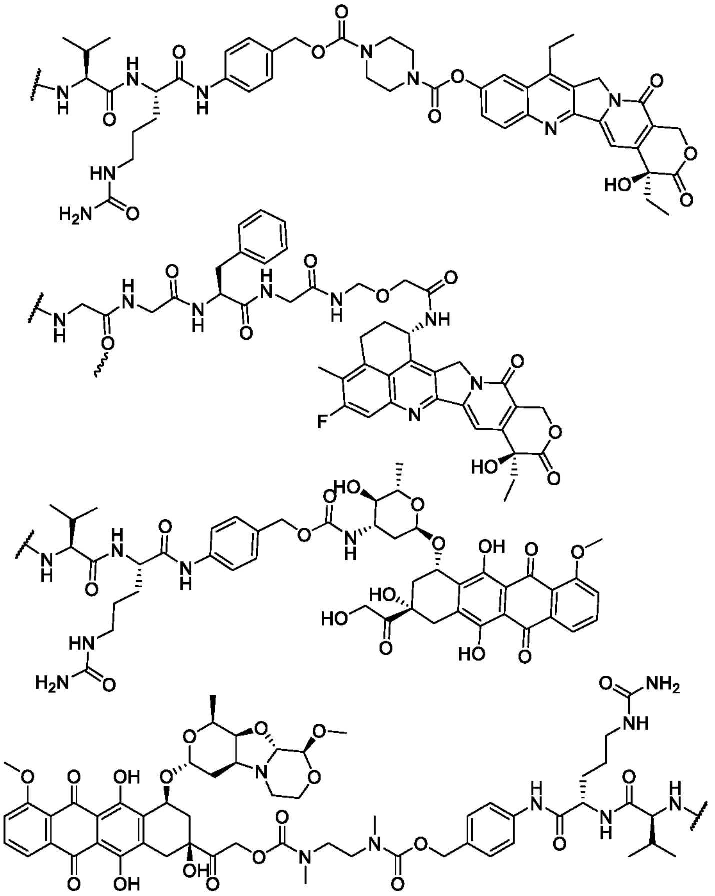

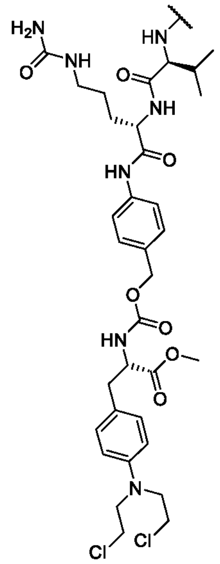

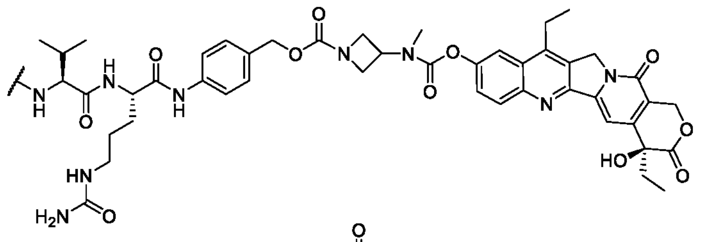

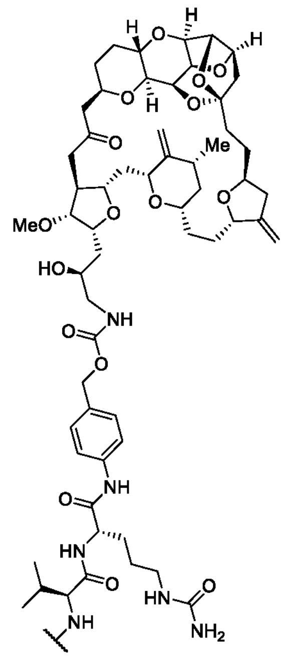

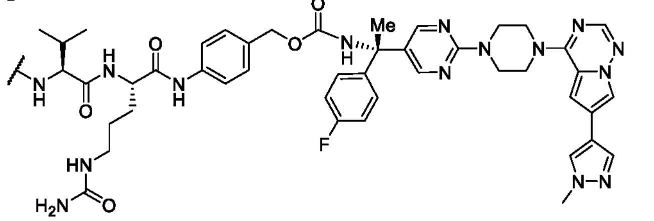

$R^y$ =

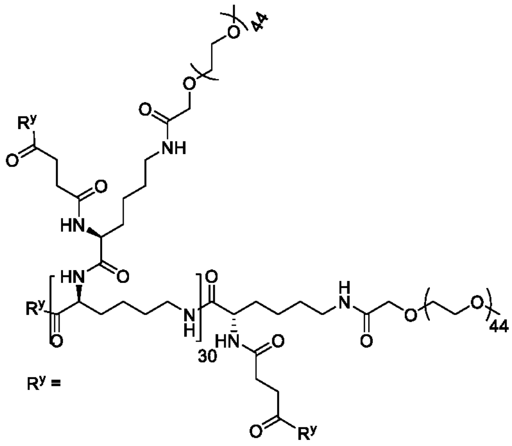

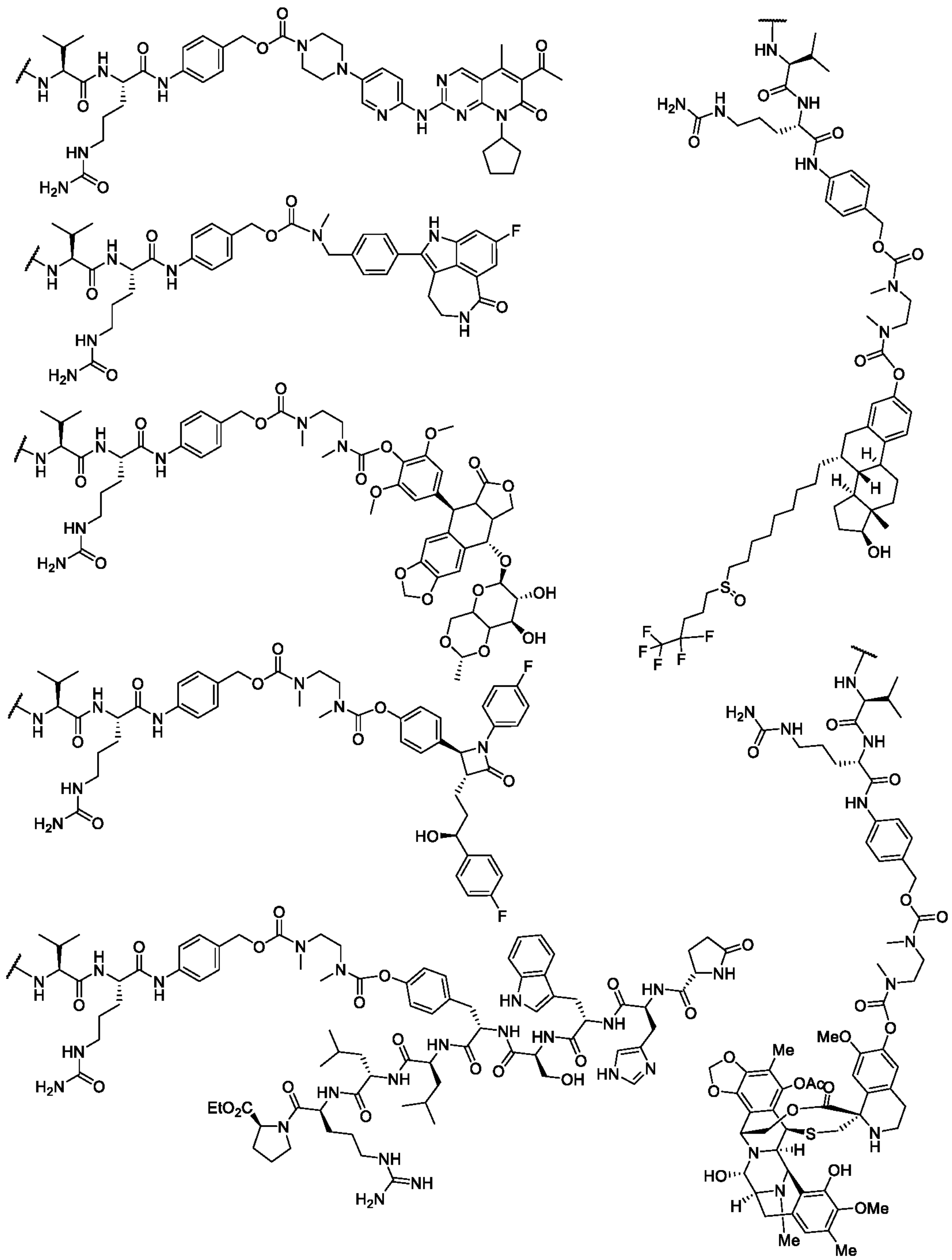

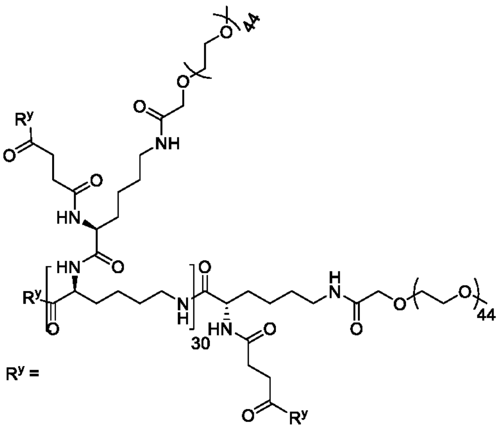

$R^y$ =

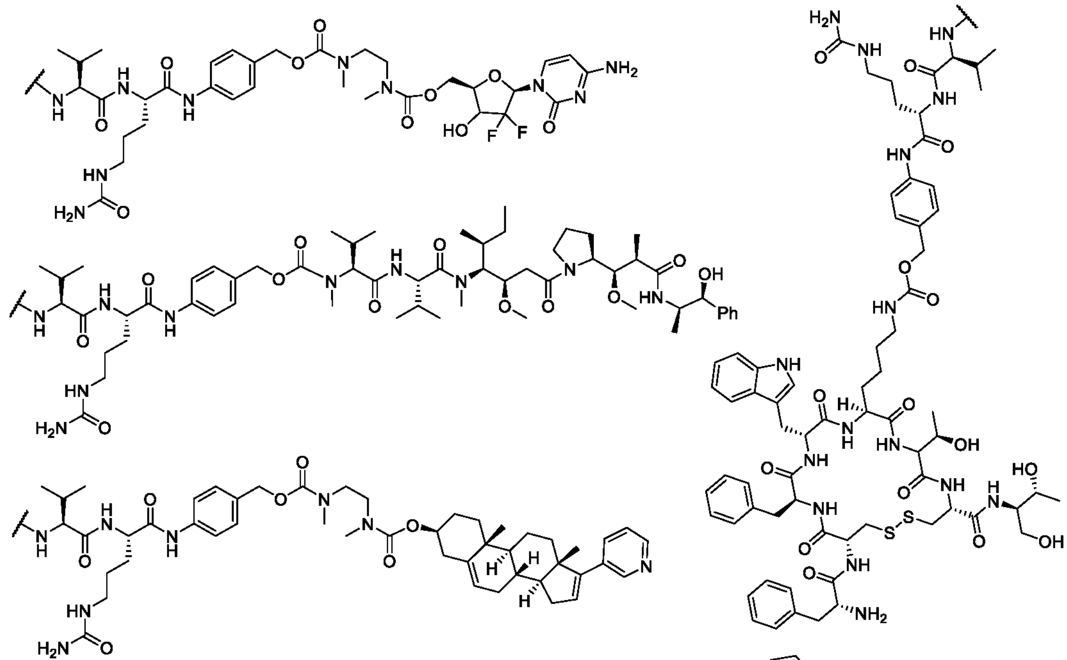

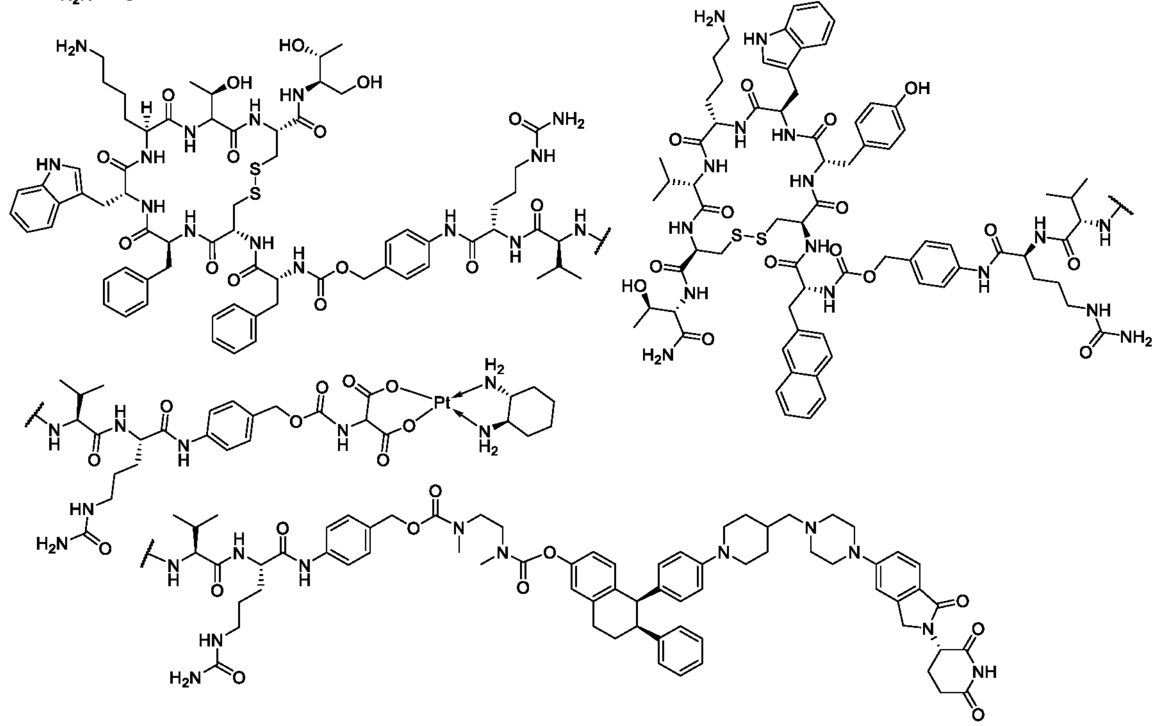

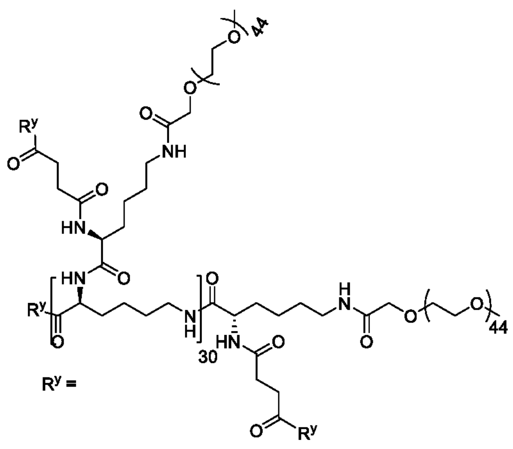

$R^y$ =

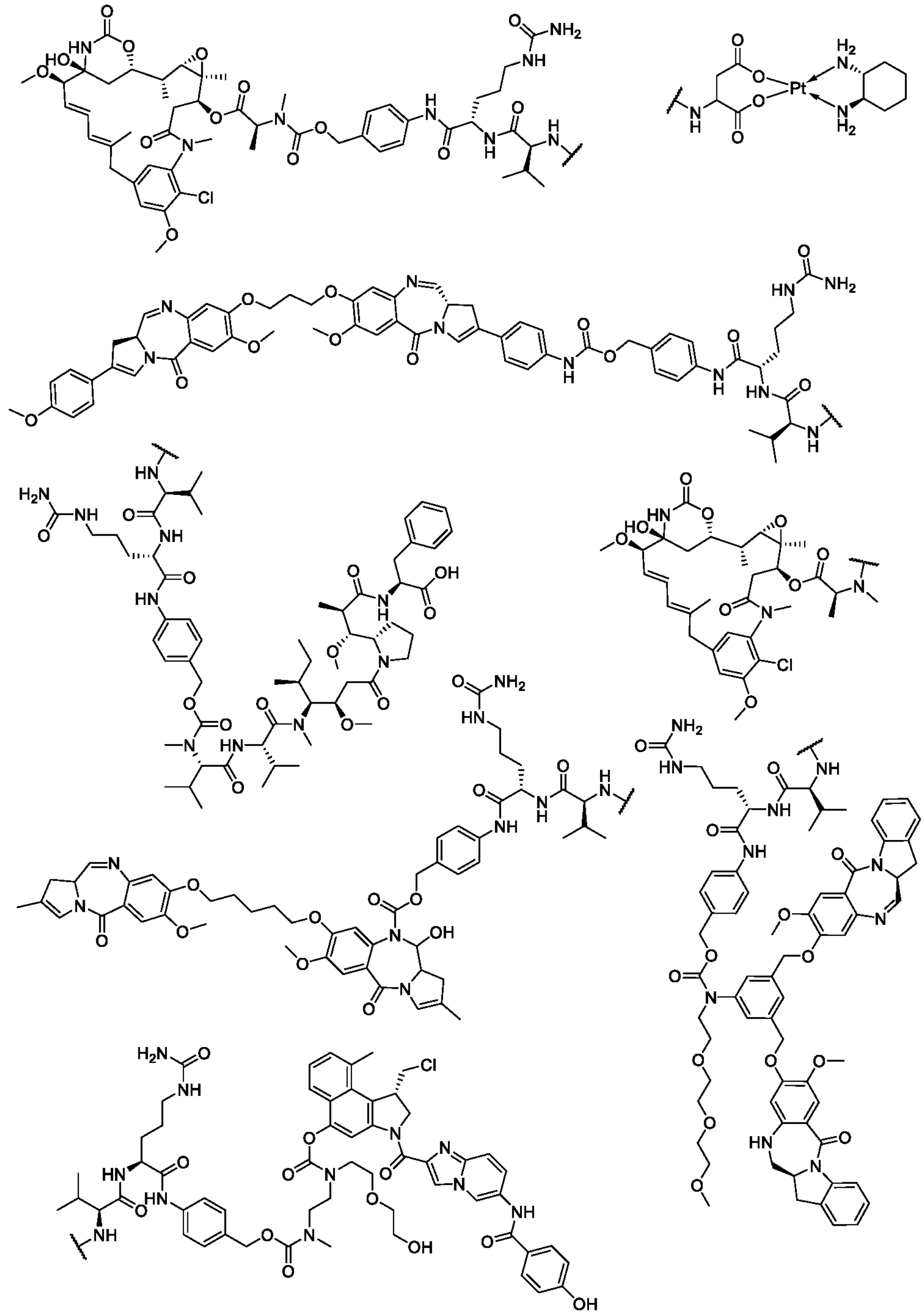

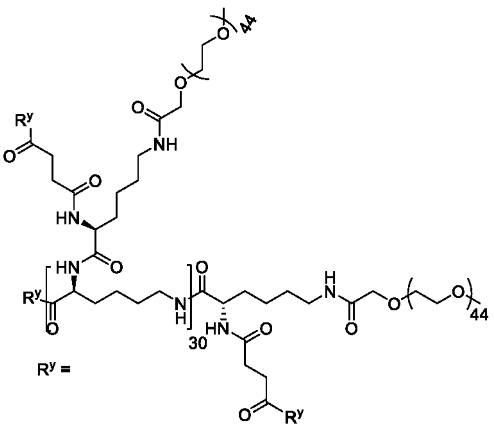

$R^y$ =

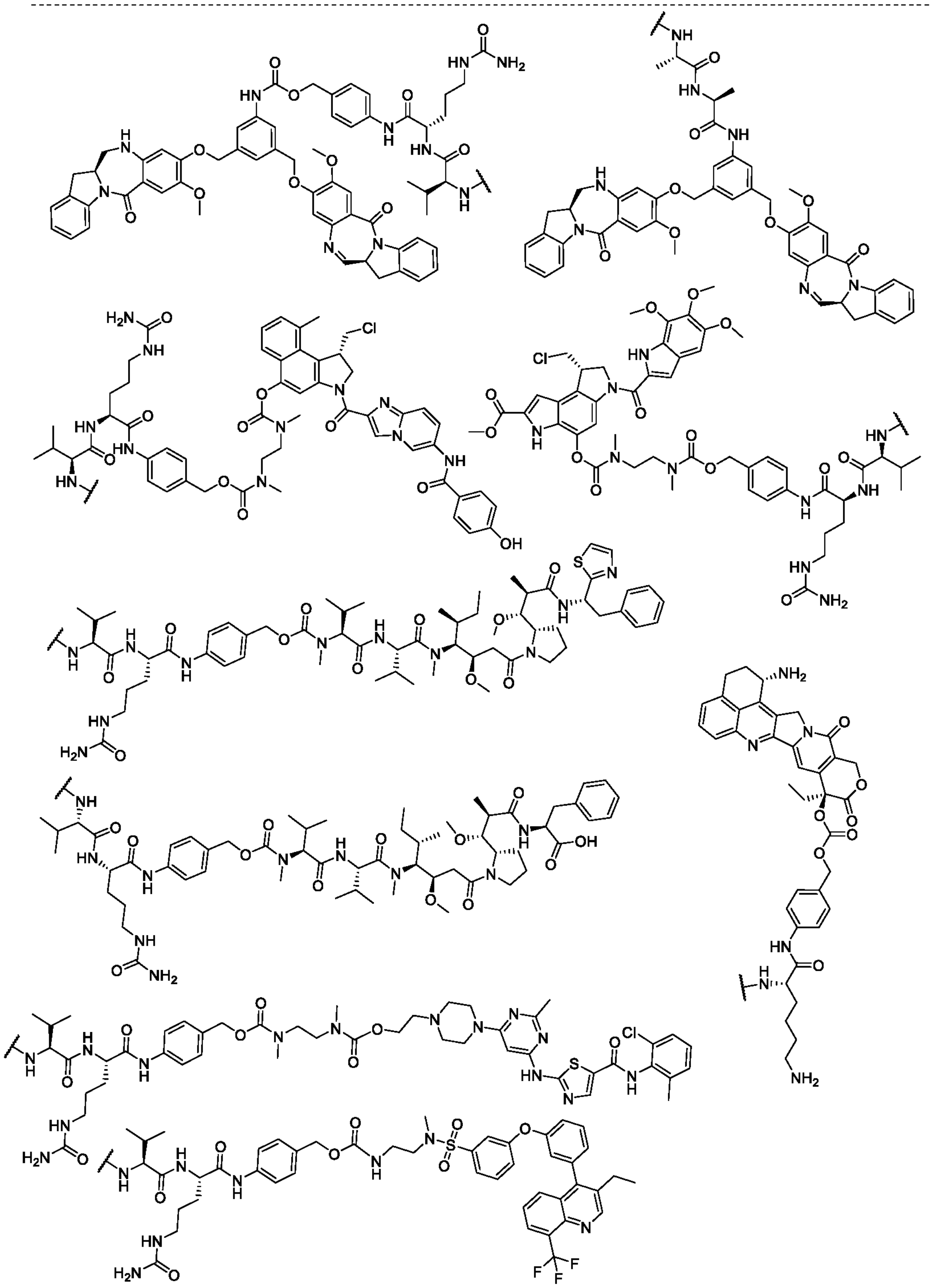

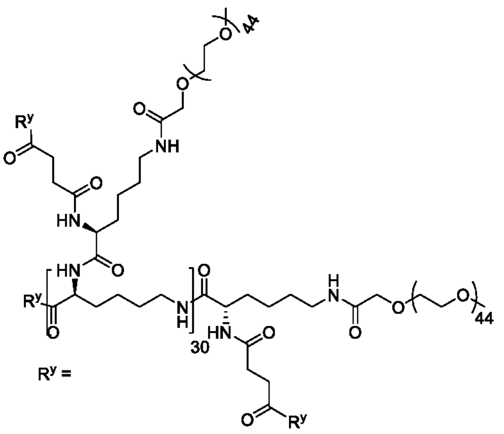

$R^y$ =

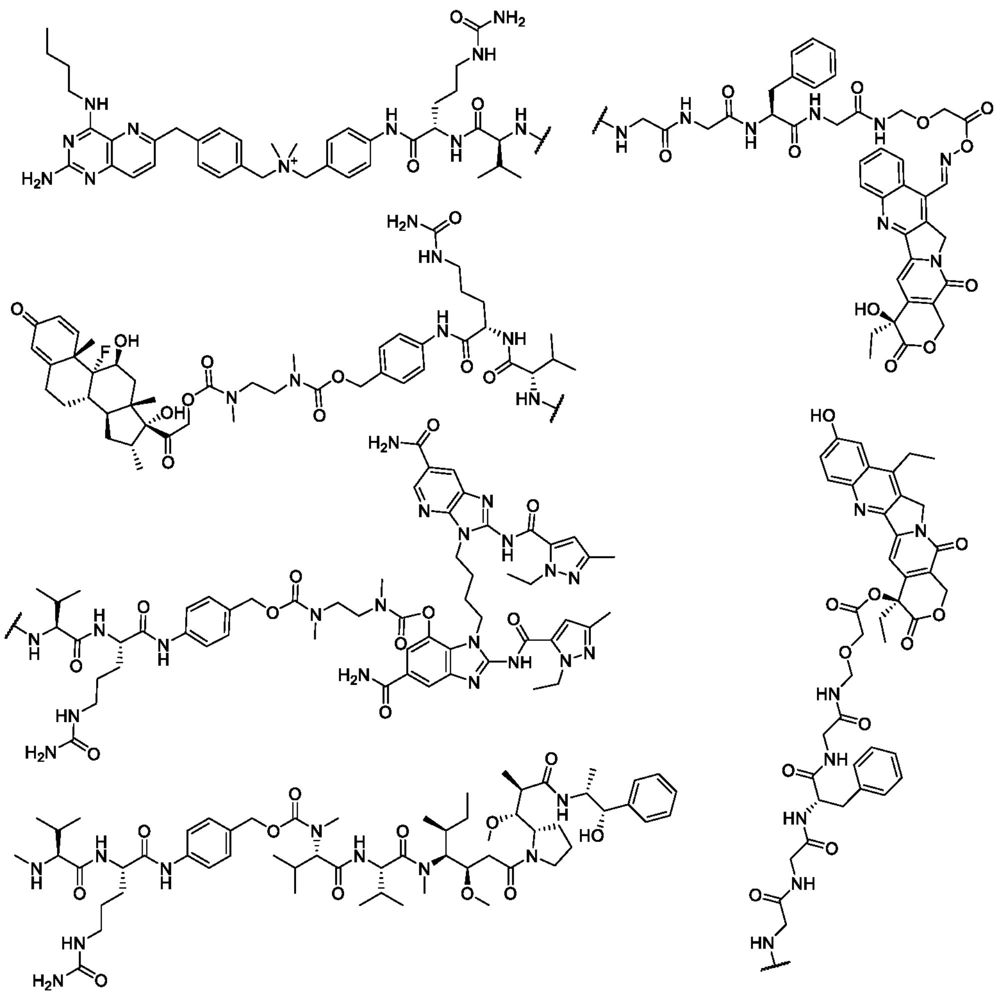

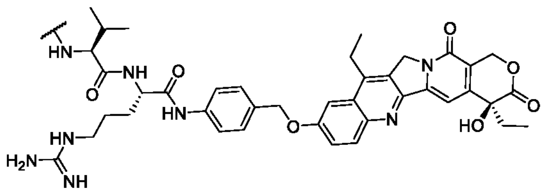

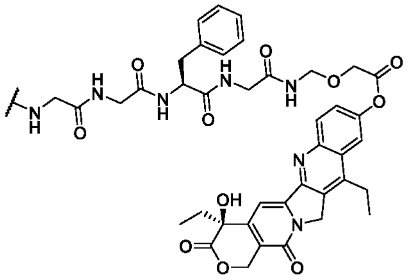

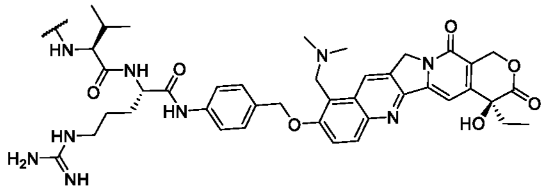

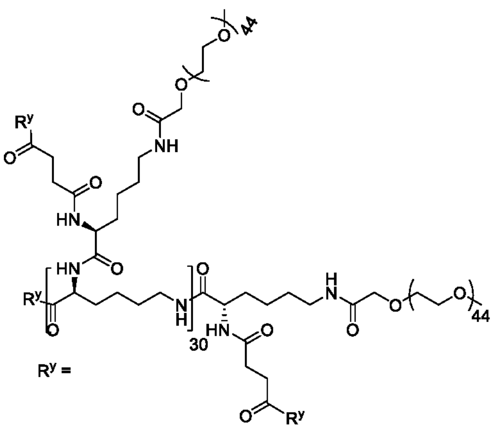

$R^y$ =

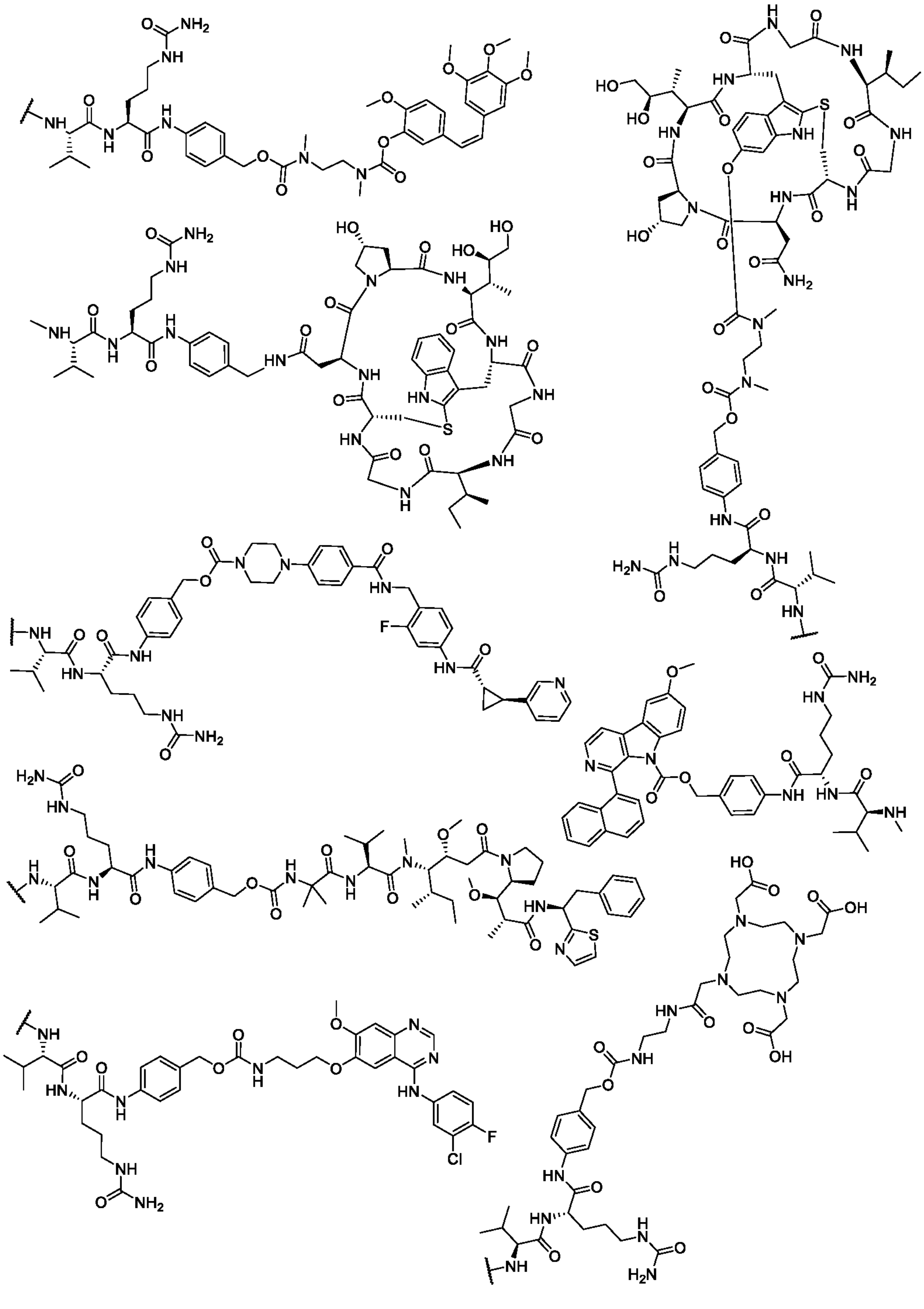

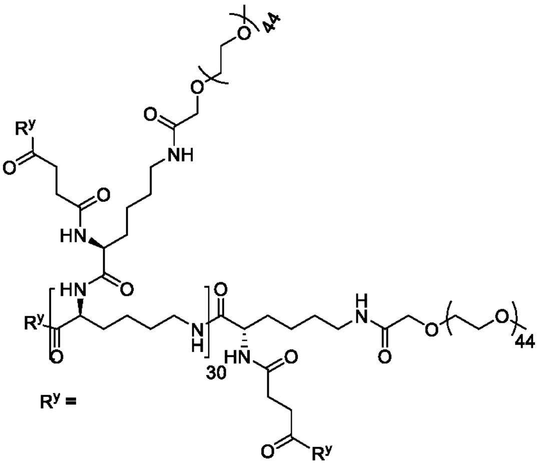

$R^y$ =

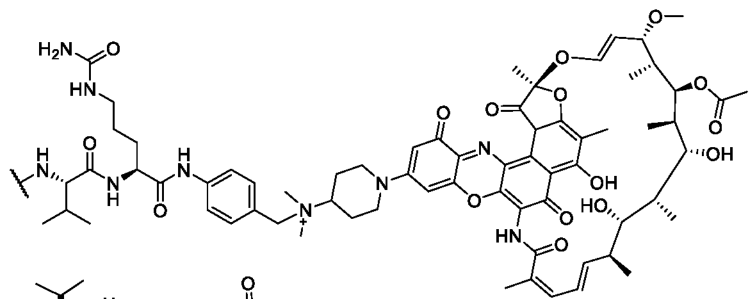

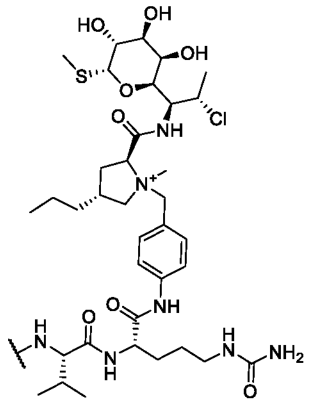

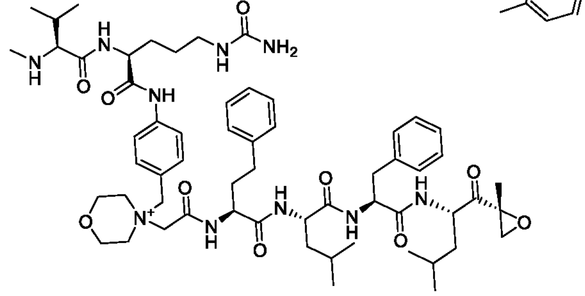

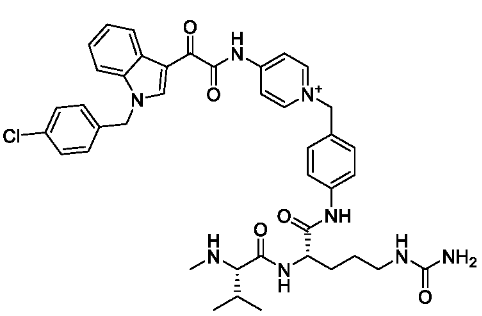

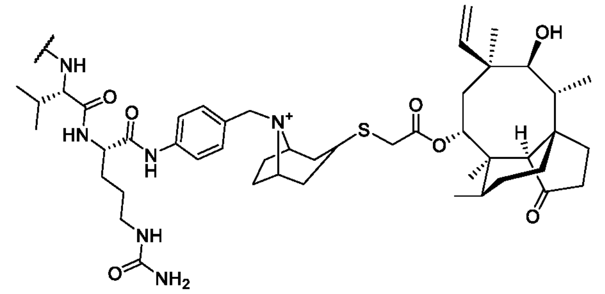

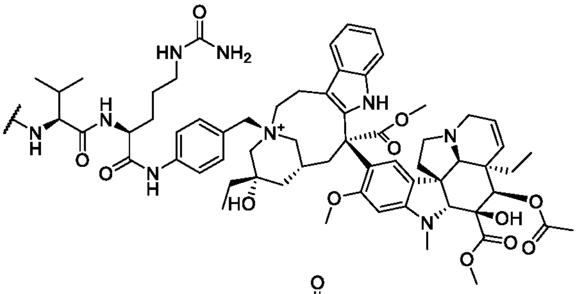

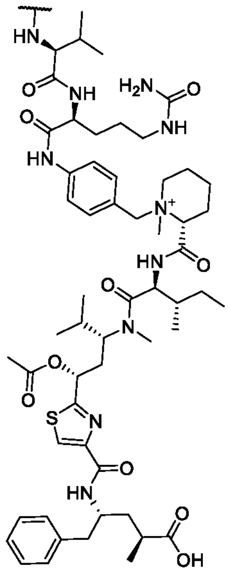

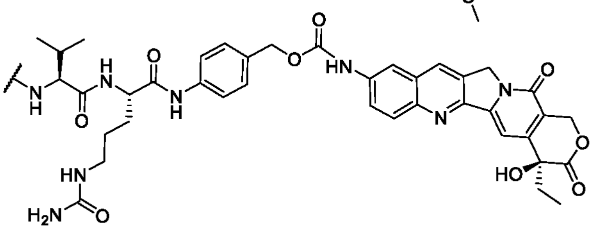

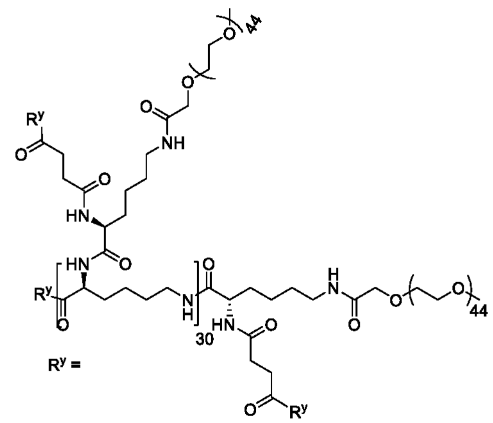

$R^y$ =

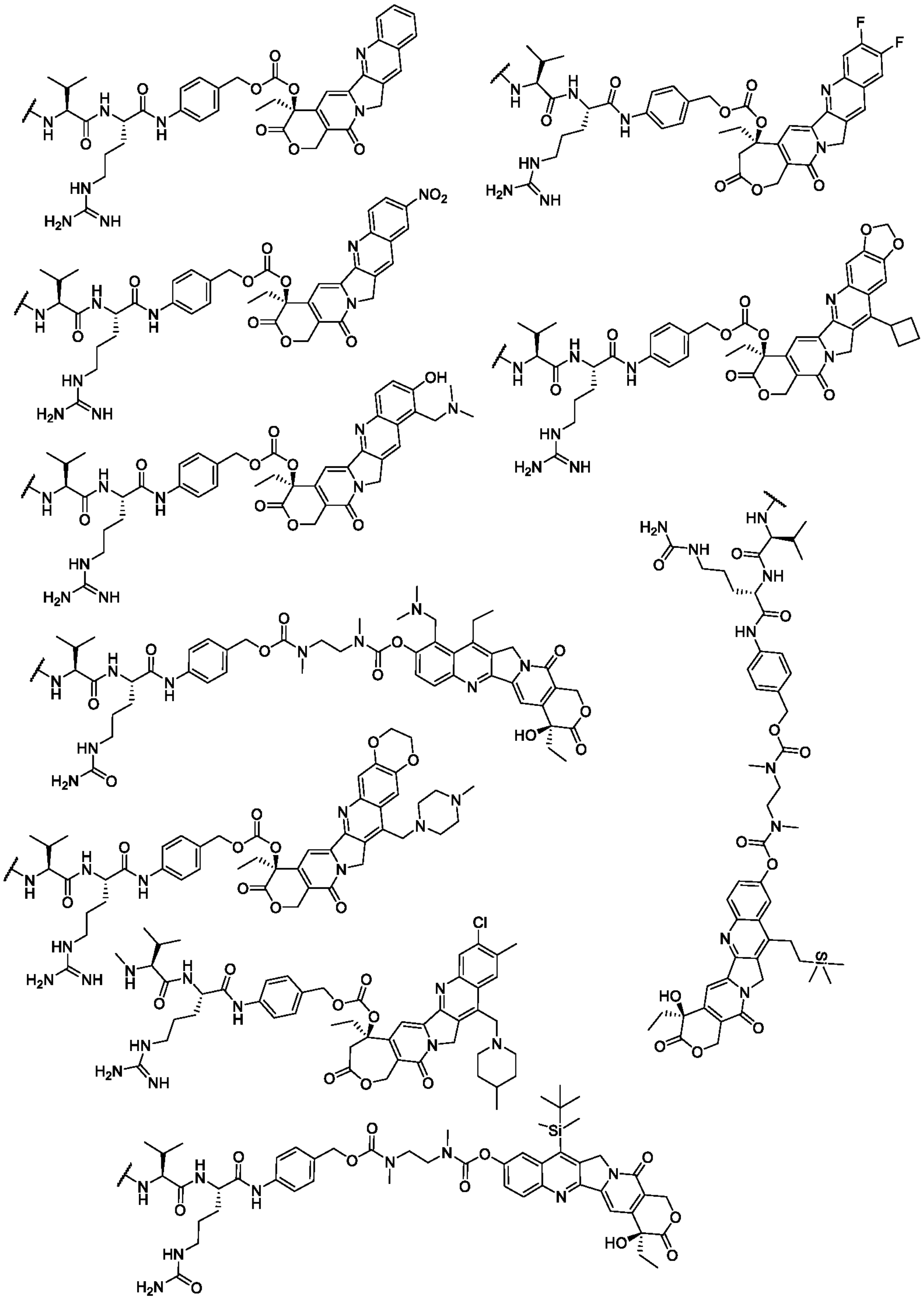

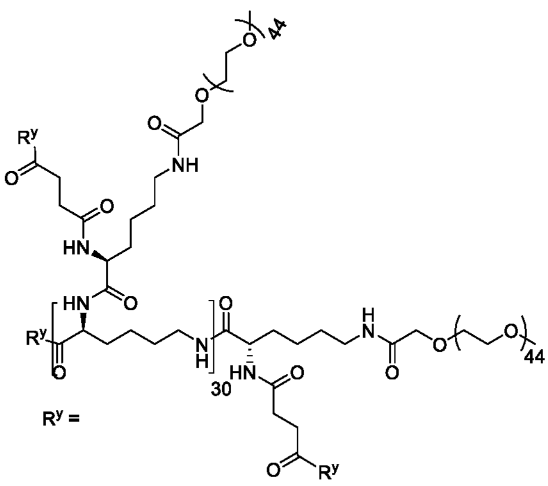

$R^y$ =

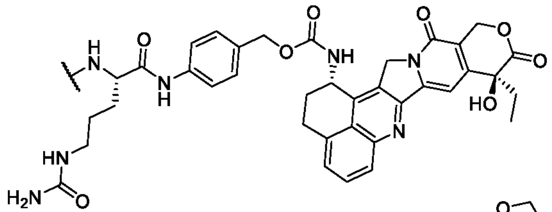

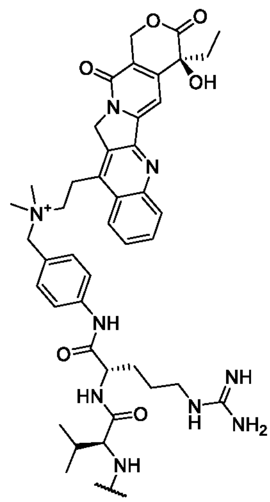

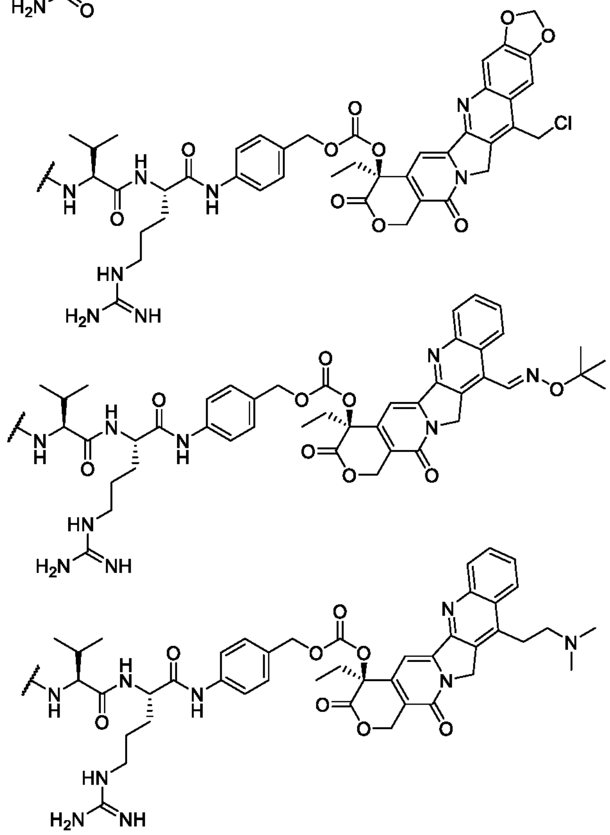

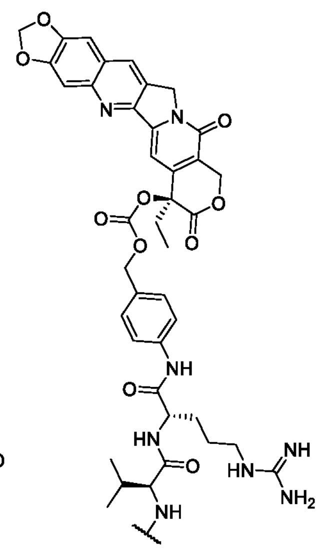

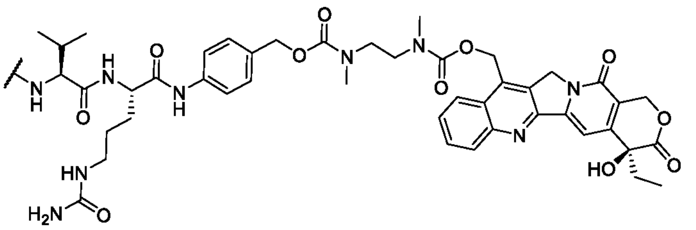

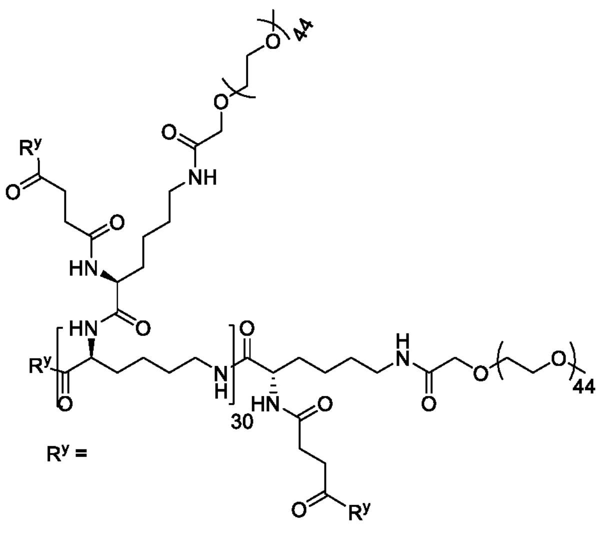

$R^y$ =

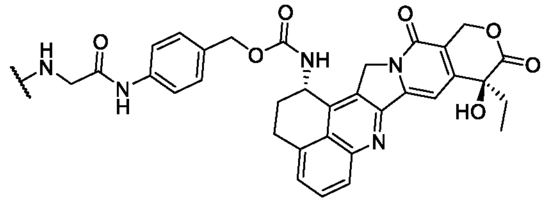

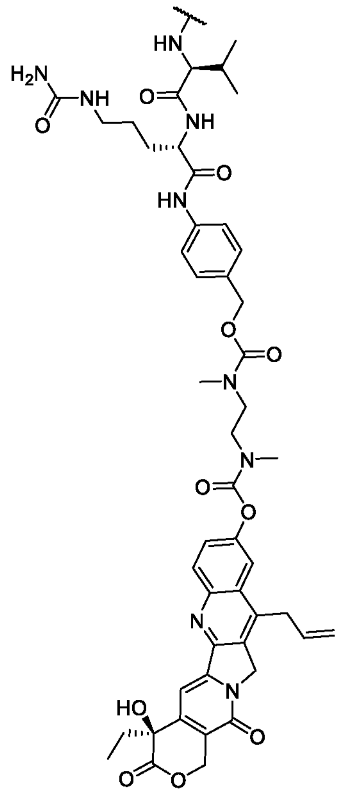

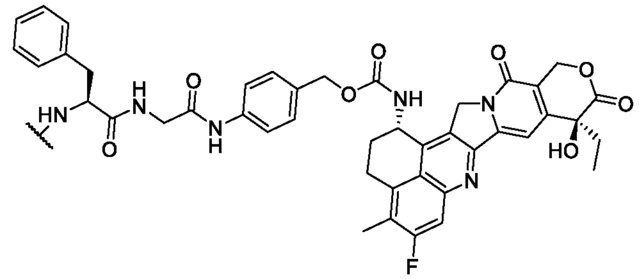

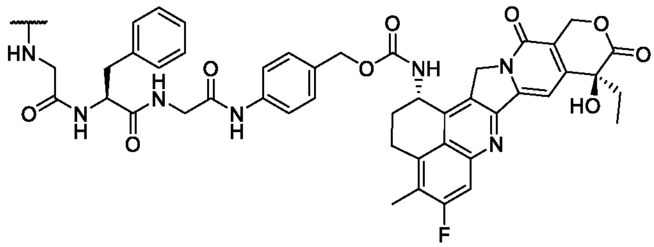

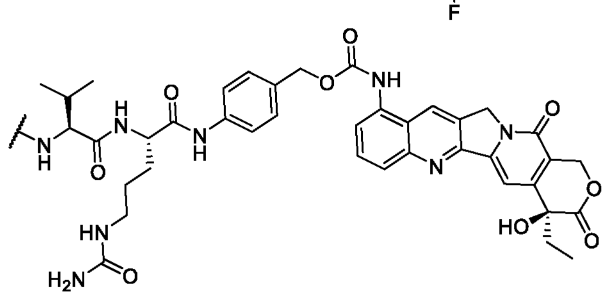

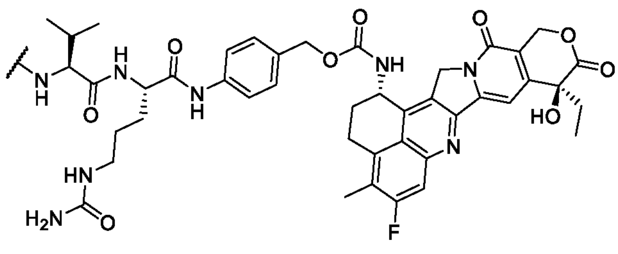

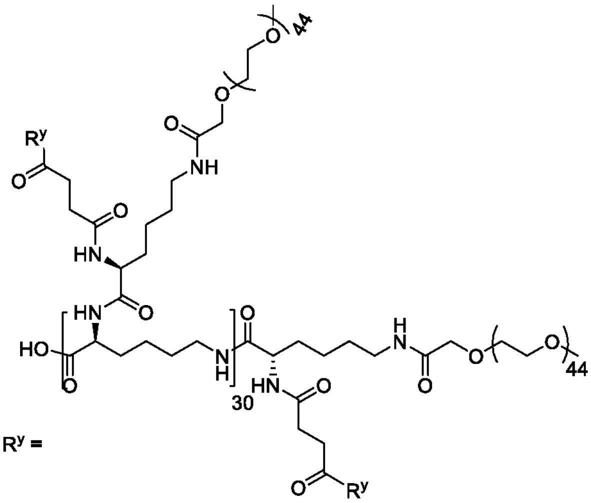

$R^y$ =

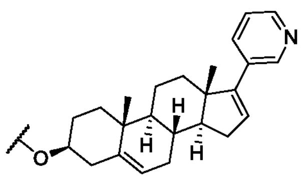

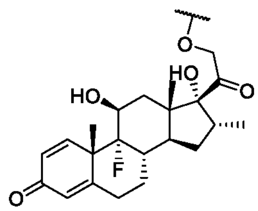

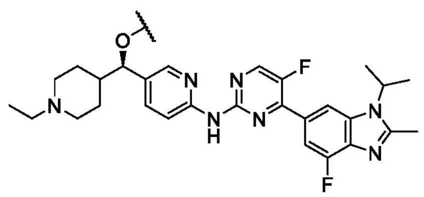

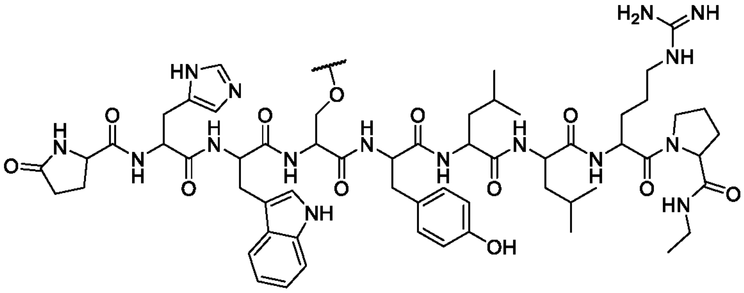

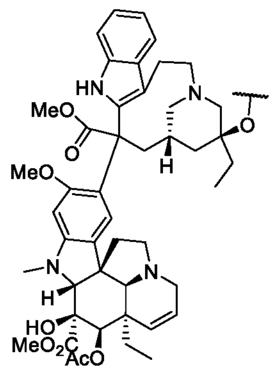

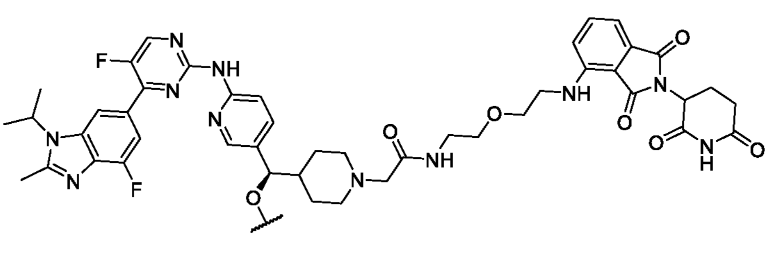

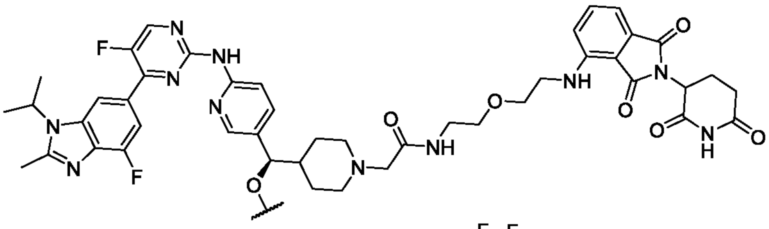

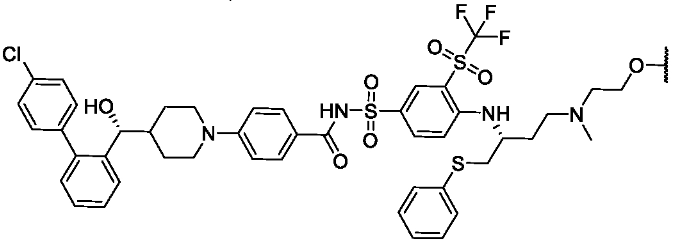

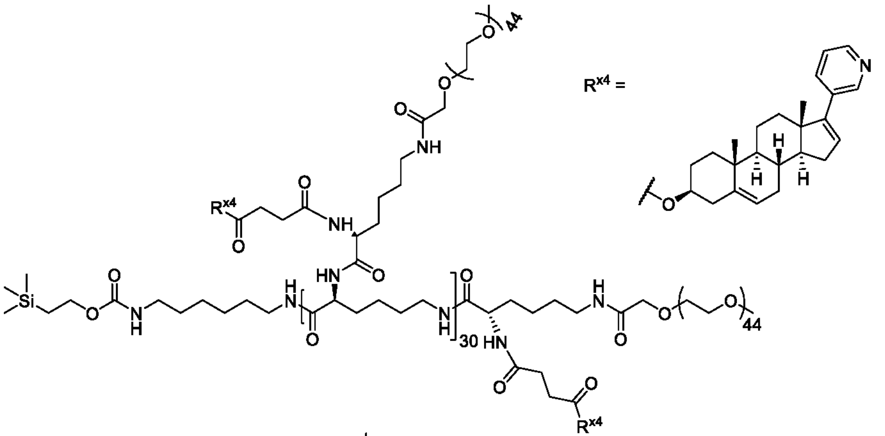

$R^{x4}$ =

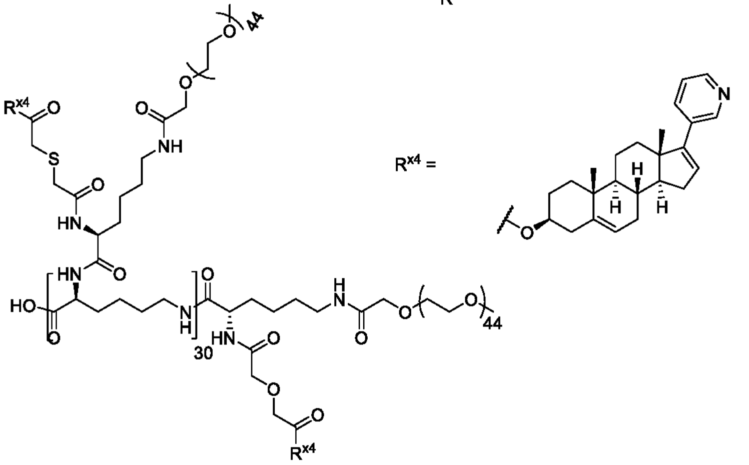

$R^{x4}$ =

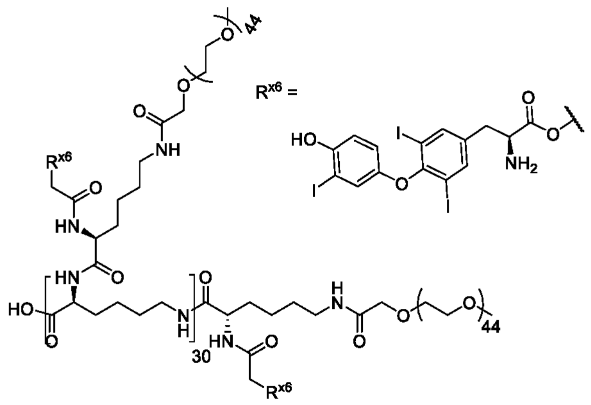

$R^{x6}$ =

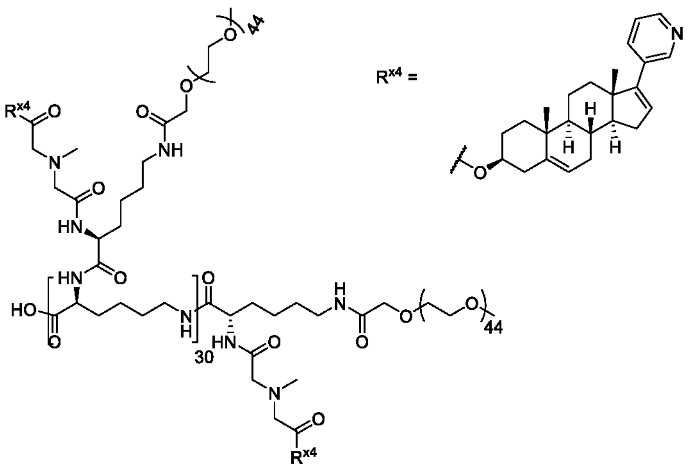

$R^{x4}$ =

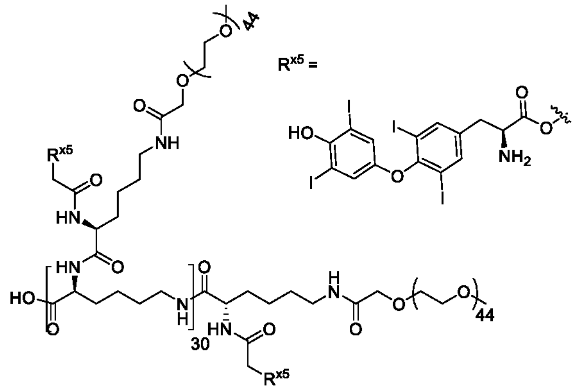

$R^{x5}$ =

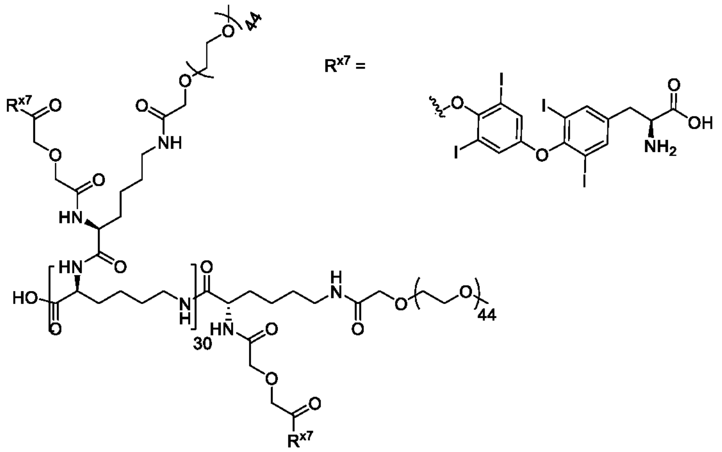

$R^{x7}$ =

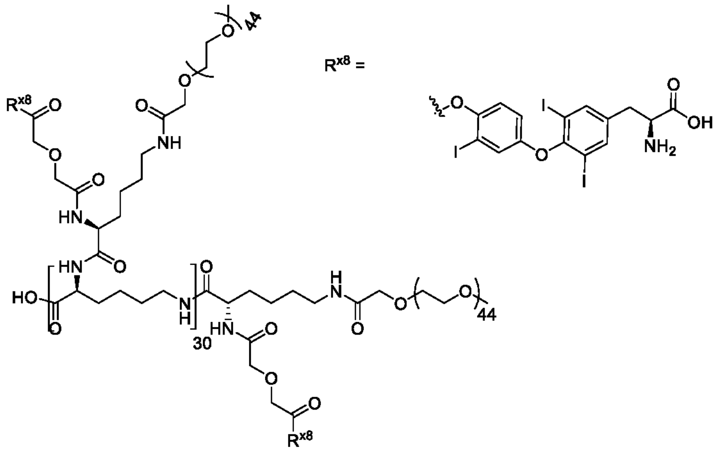

$R^{x8}$ =

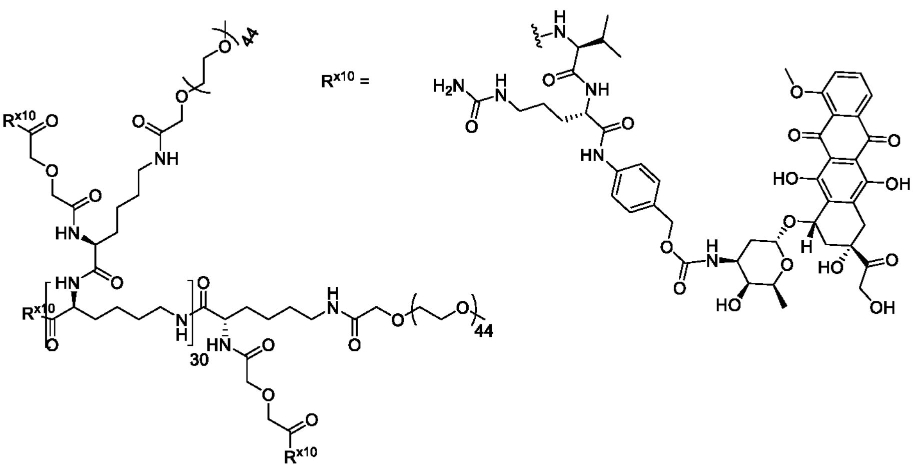

$R^{x10}$ =

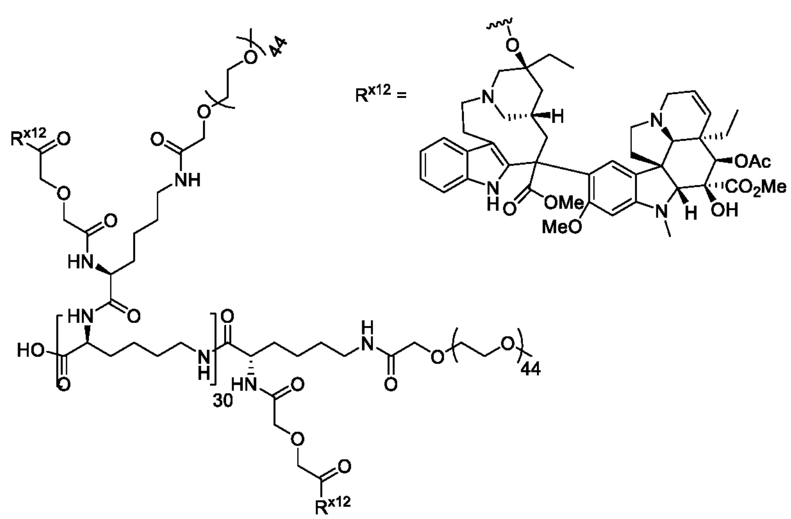

$R^{x12}$ =

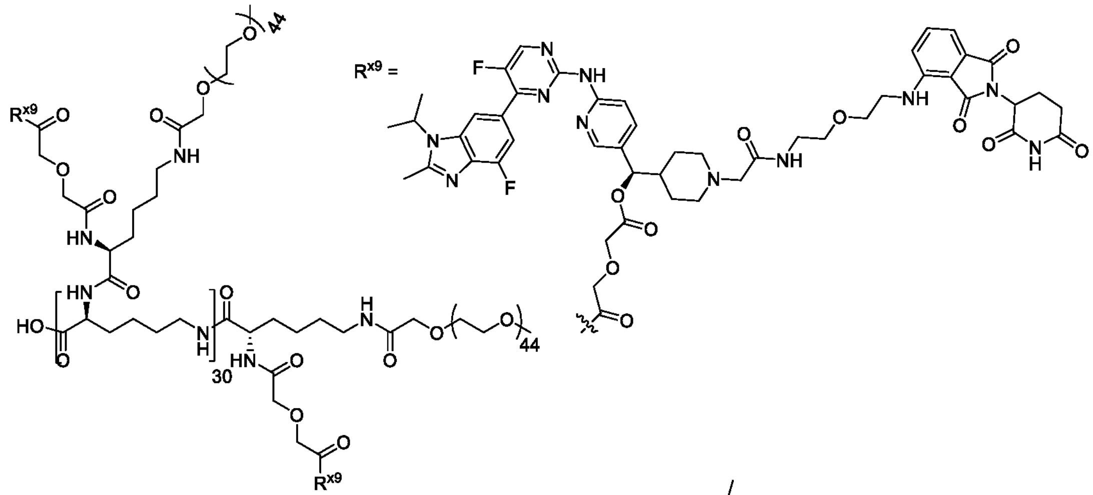

$R^{x9}$ =

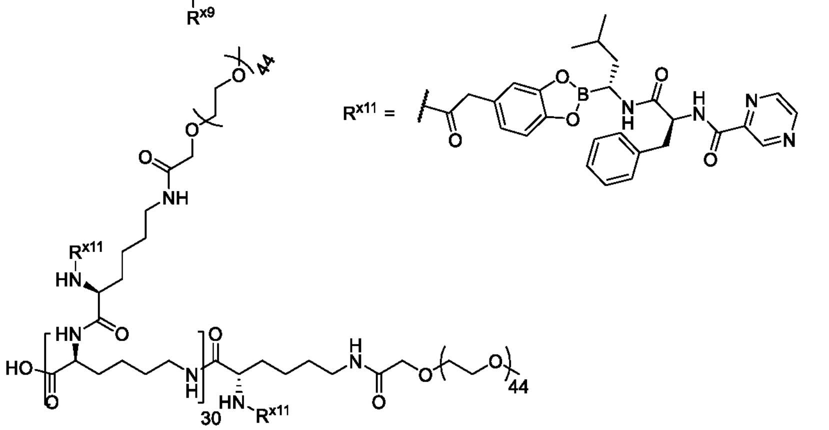

$R^{x11}$ =

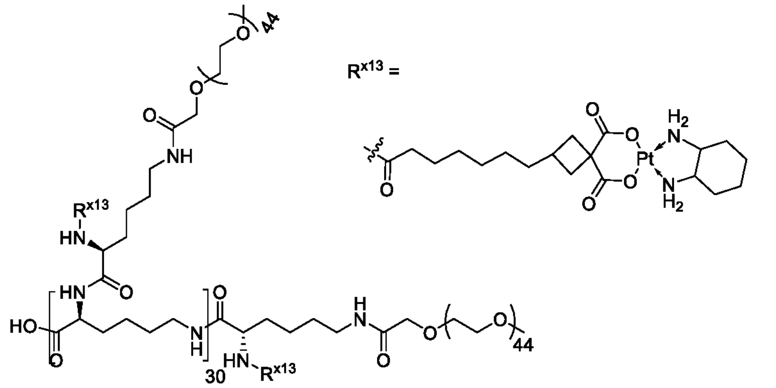

$R^{x13}$ =

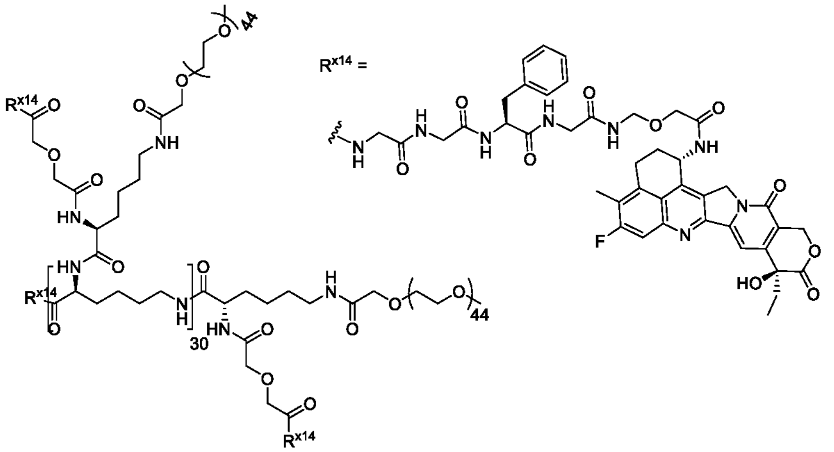

$R^{x14}$ =

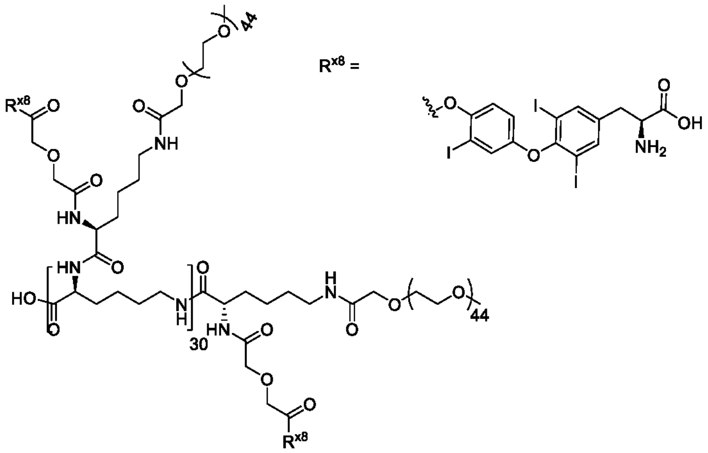

$R^{x8}$ =

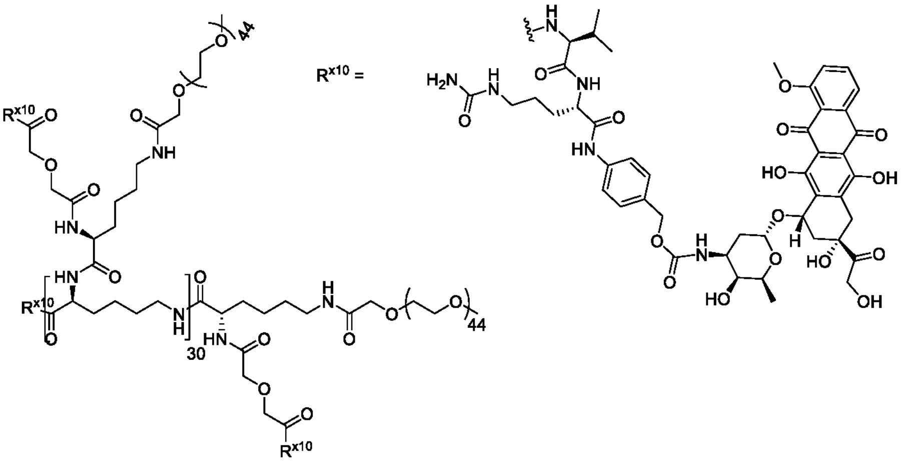

$R^{x10}$ =

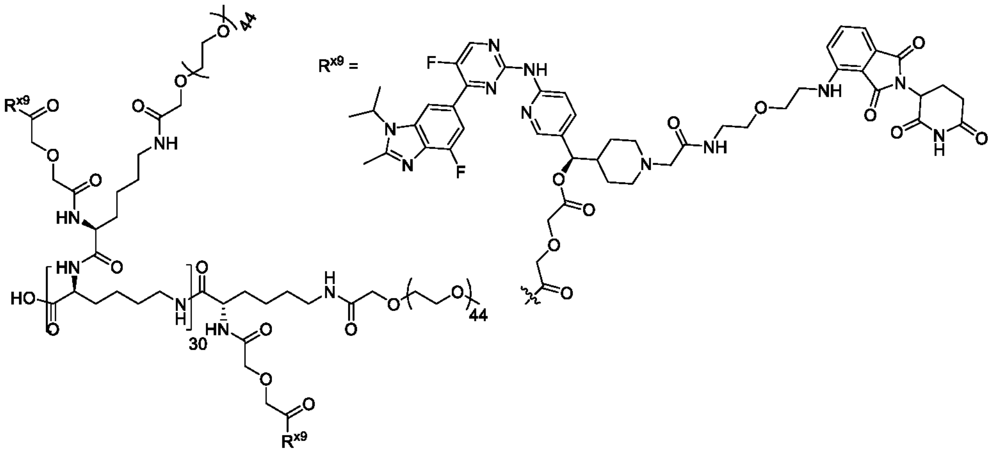

$R^{x9}$ =

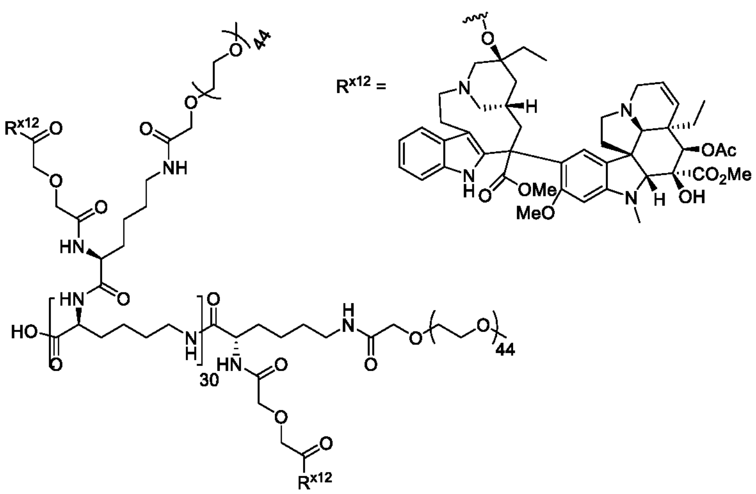

$R^{x12}$ =

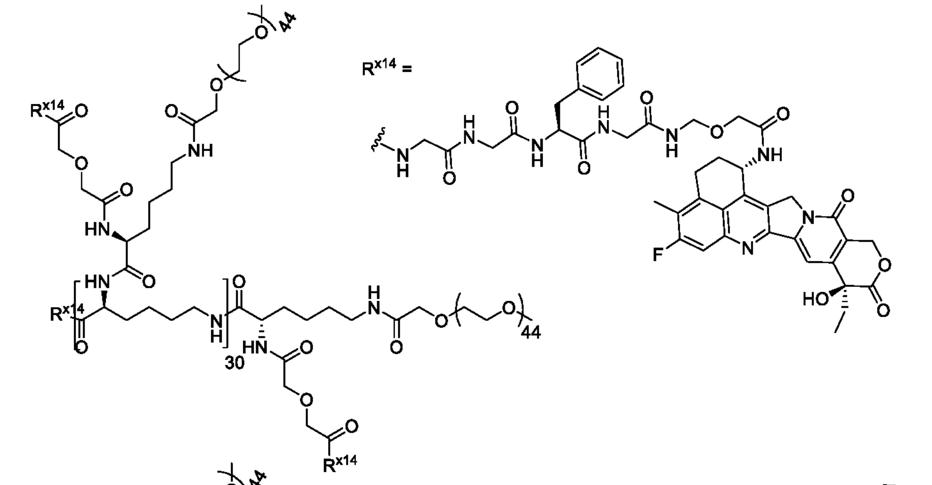

$R^{x14}$ =

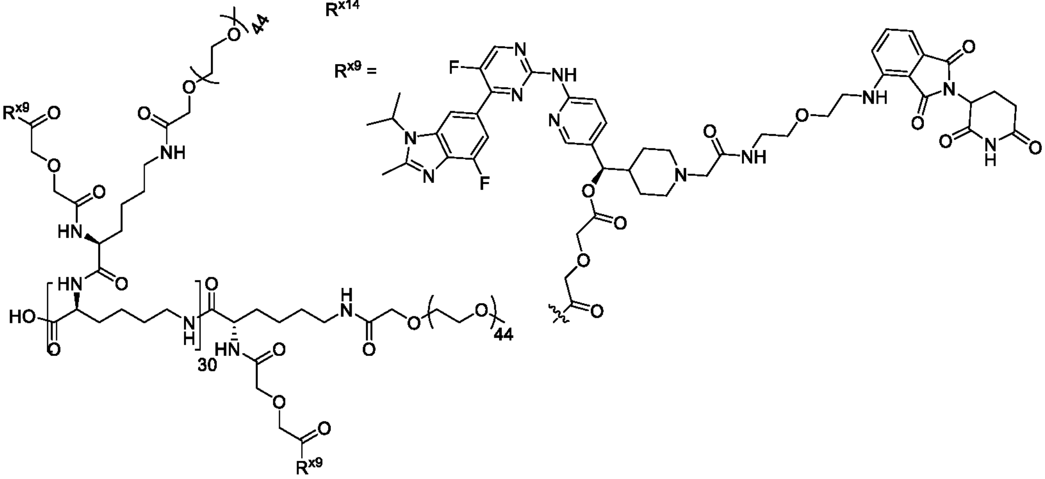

$R^{x9}$ =

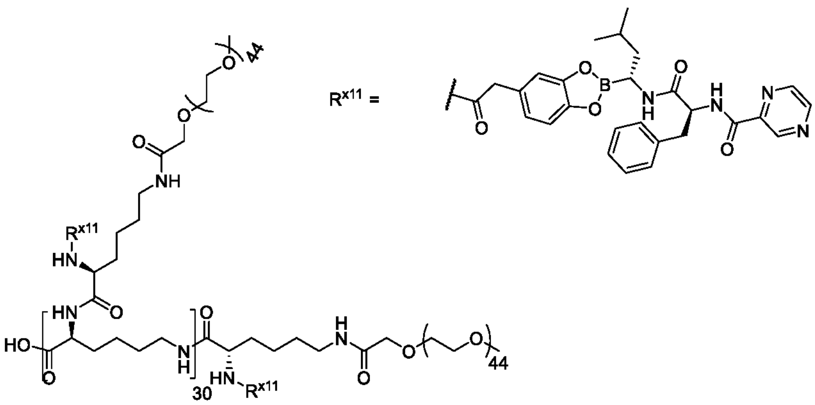

$R^{x11}$ =

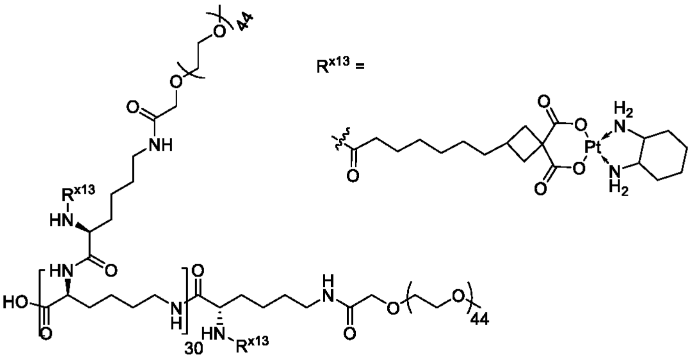

$R^{x13}$ =

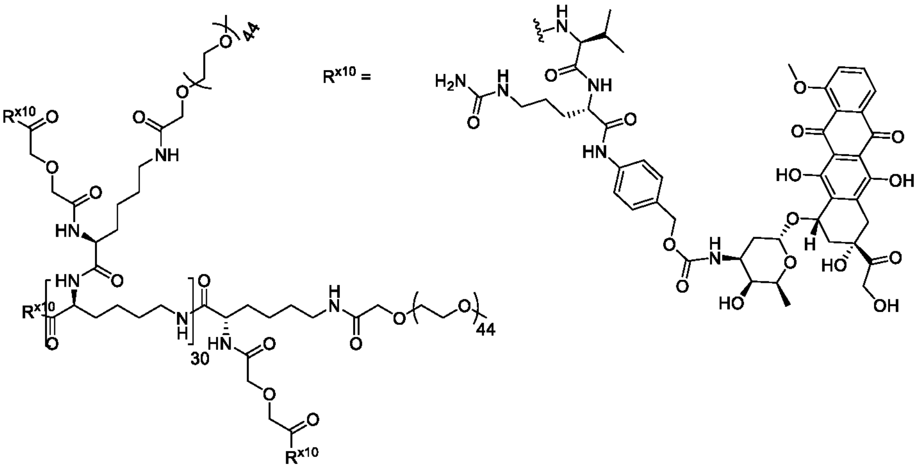

$R^{x10}$ =

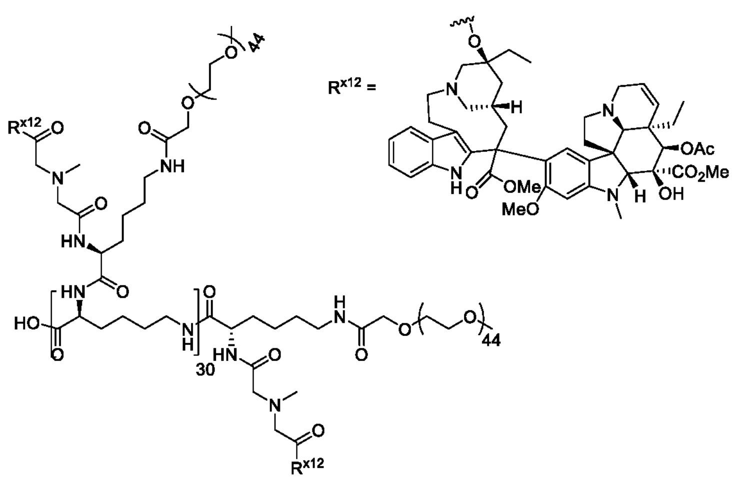

$R^{x12}$ =

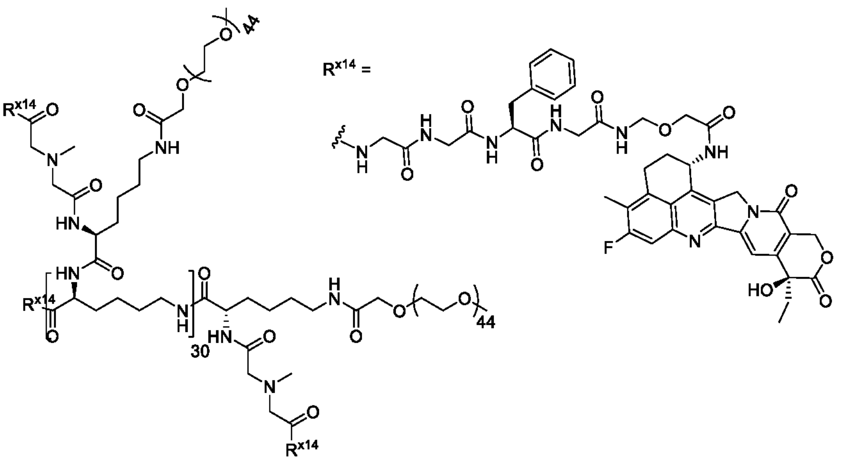

$R^{x14}$ =

$R^p$ =

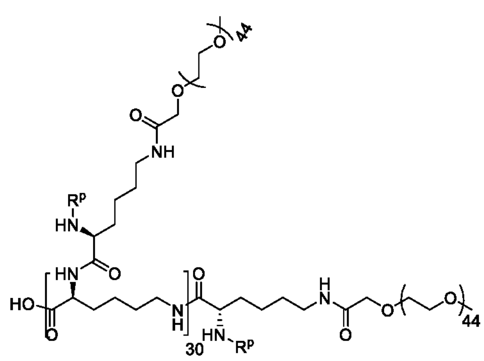

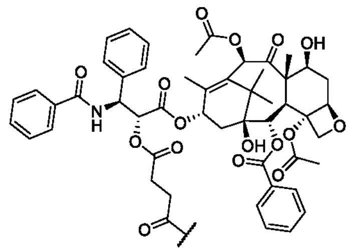

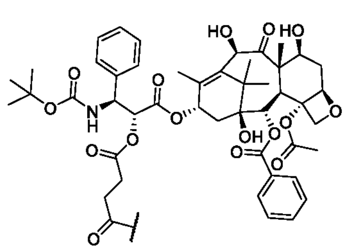

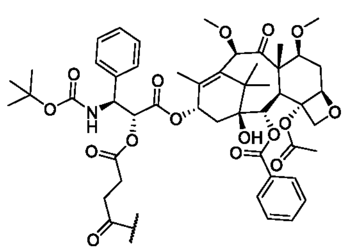

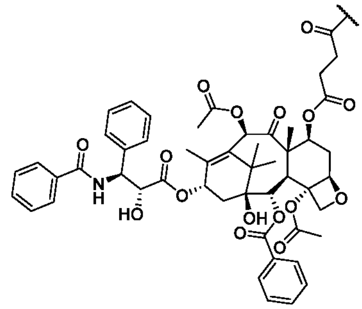

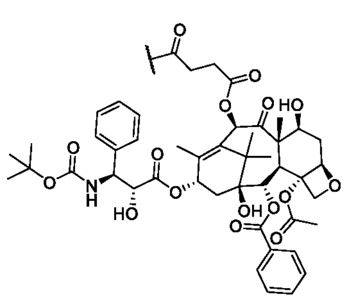

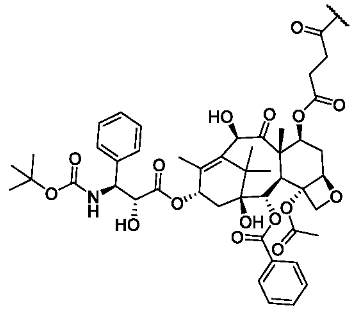

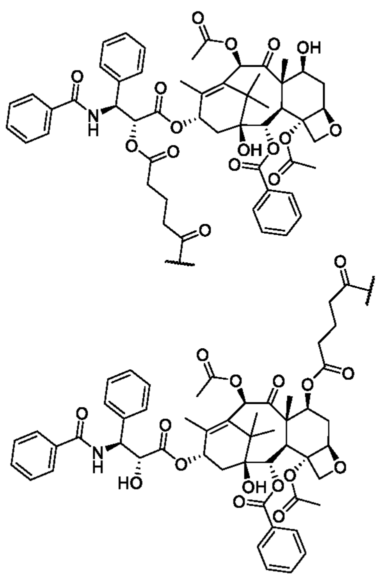

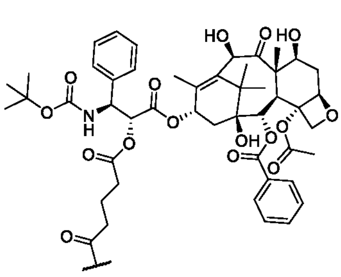

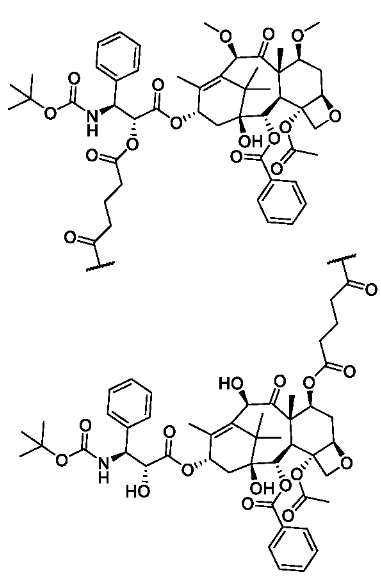

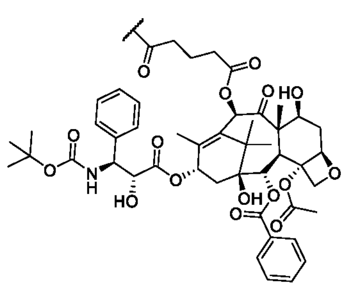

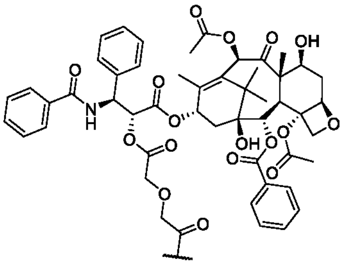
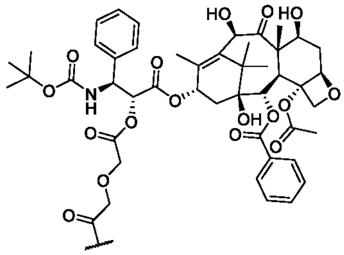
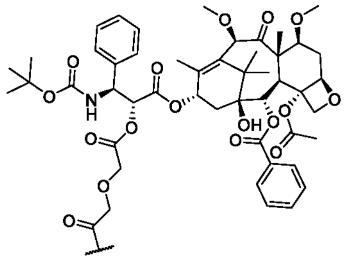

R^p =
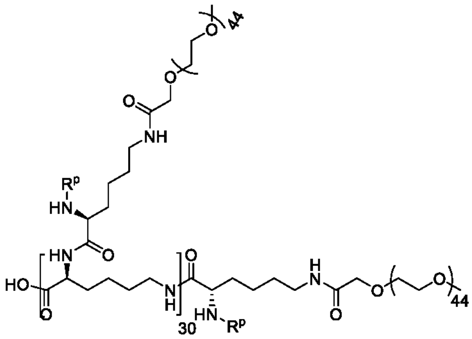

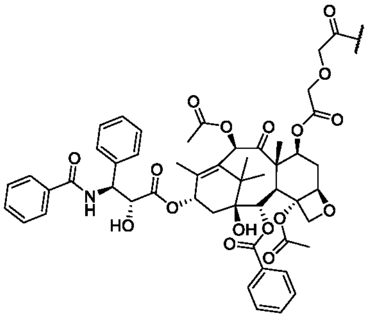
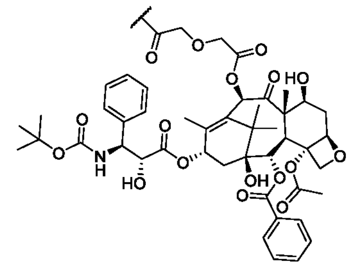
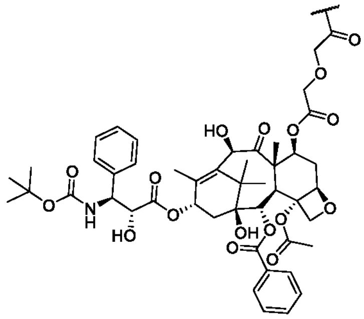

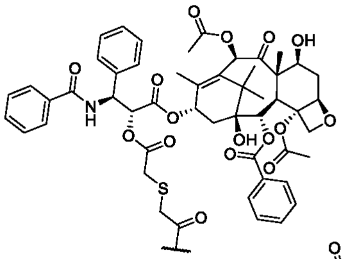
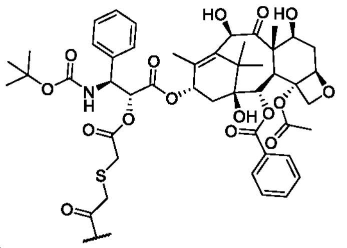
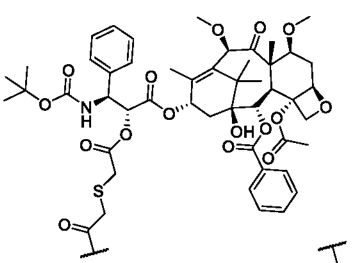

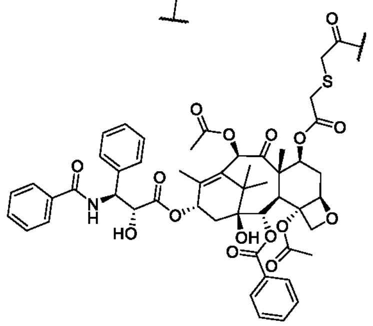
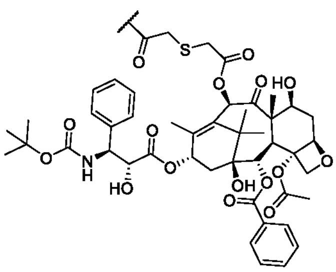
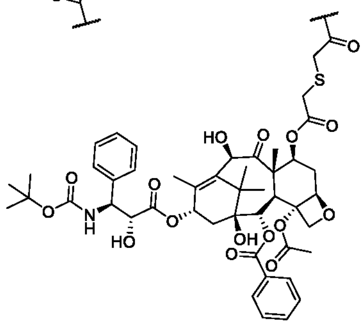

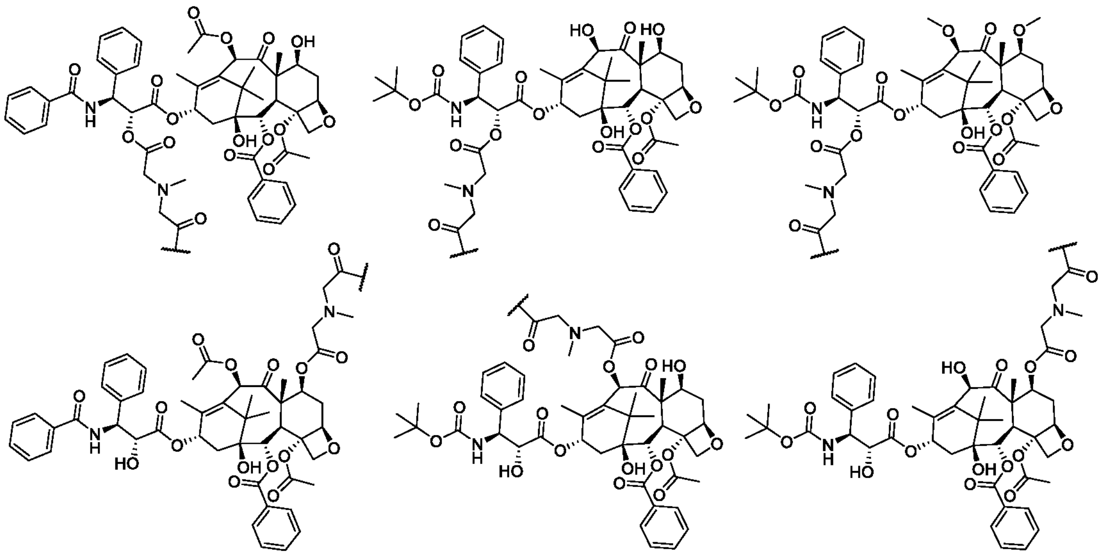

$R^p$ =

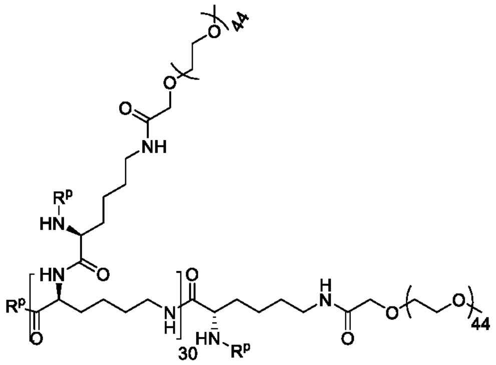

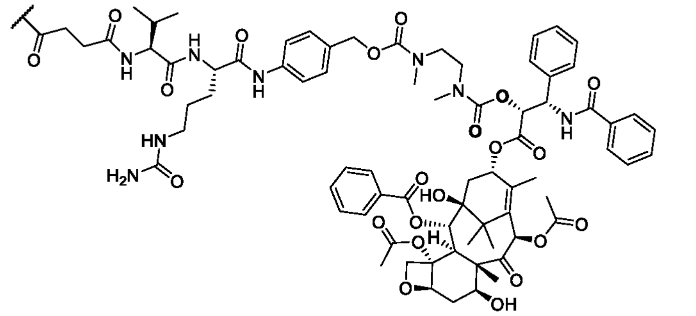

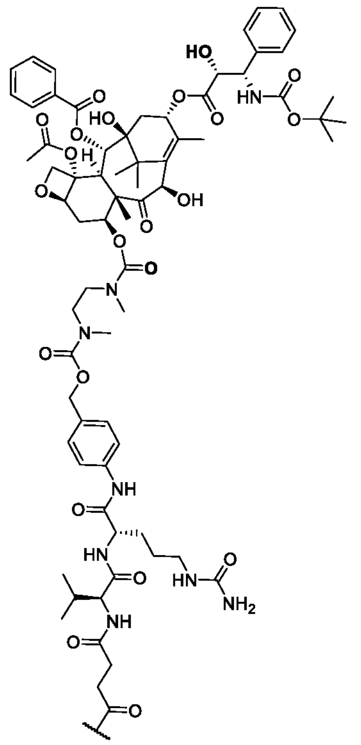

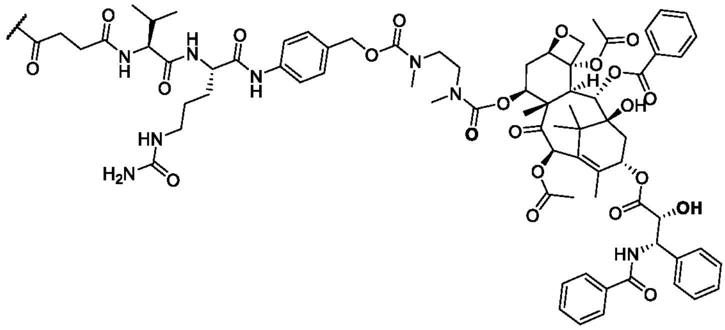

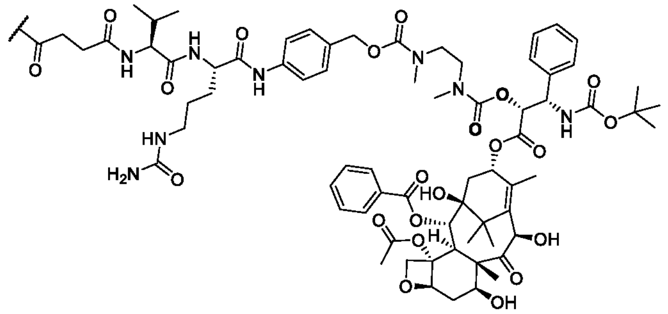

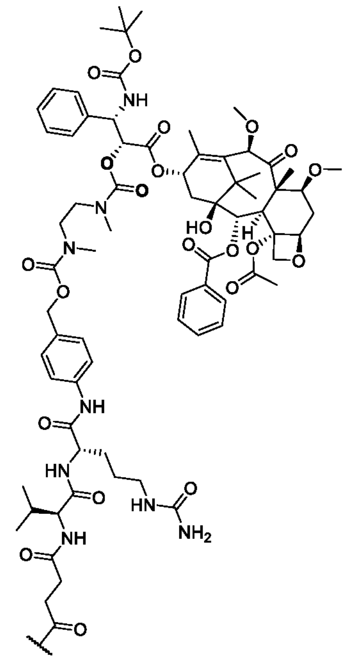

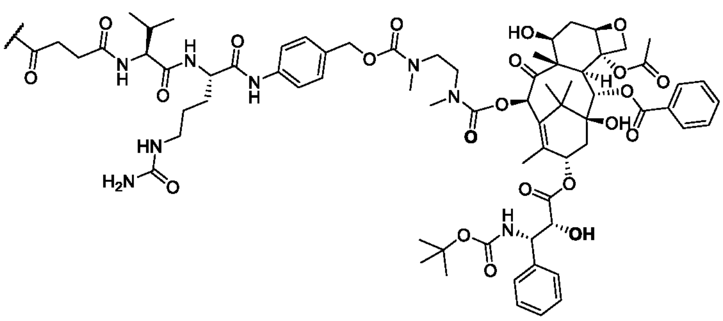

$R^p$ =

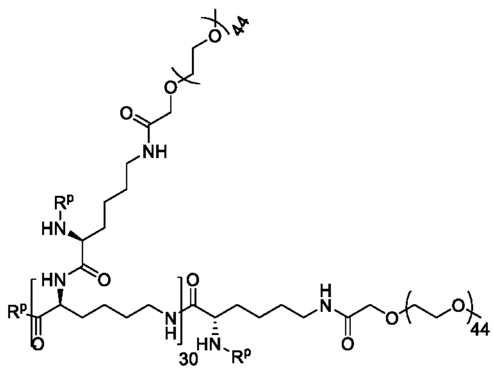

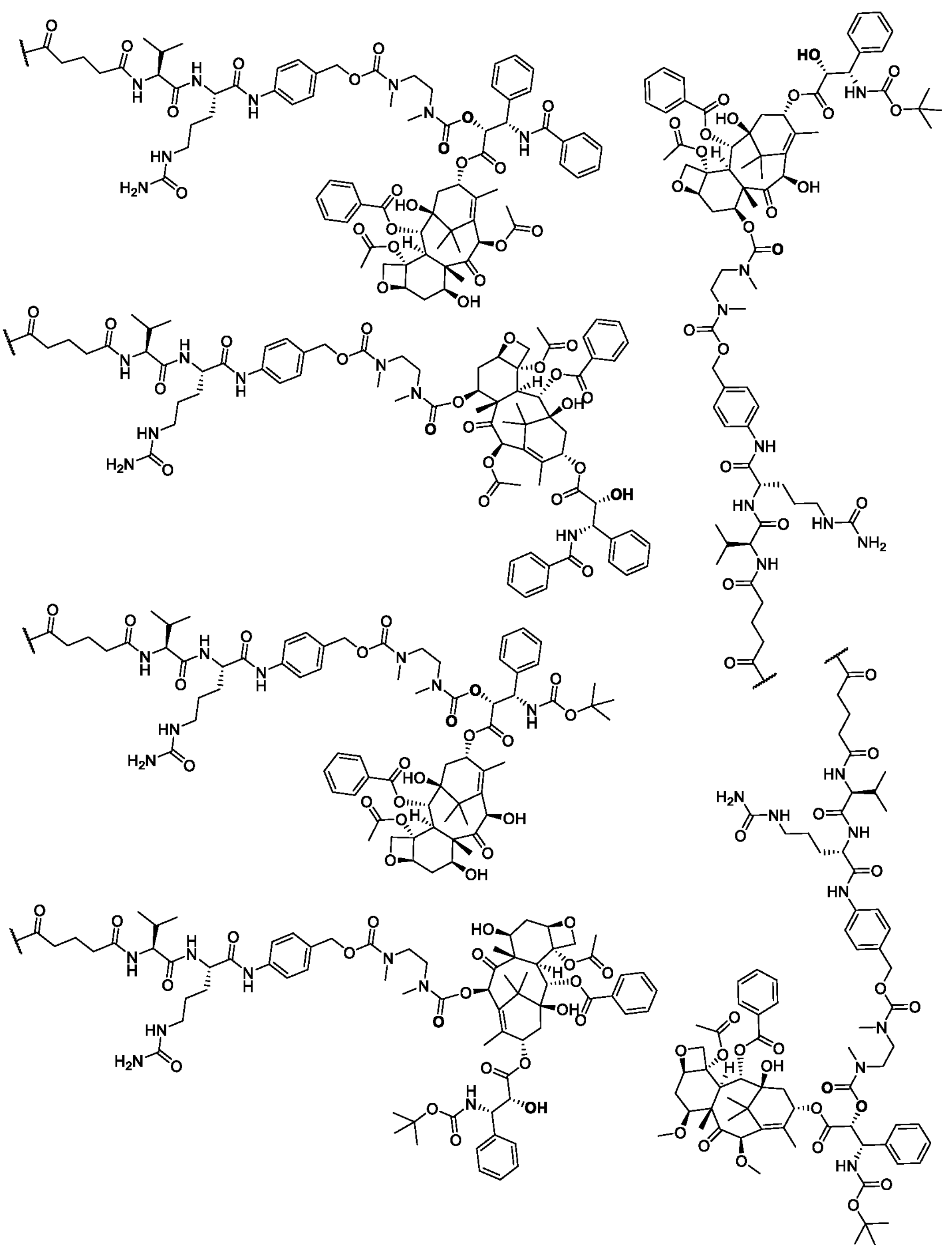

Rᵖ =

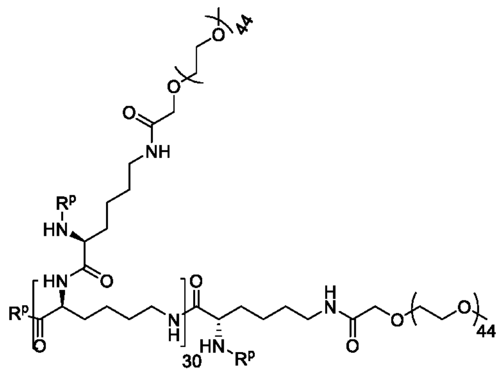

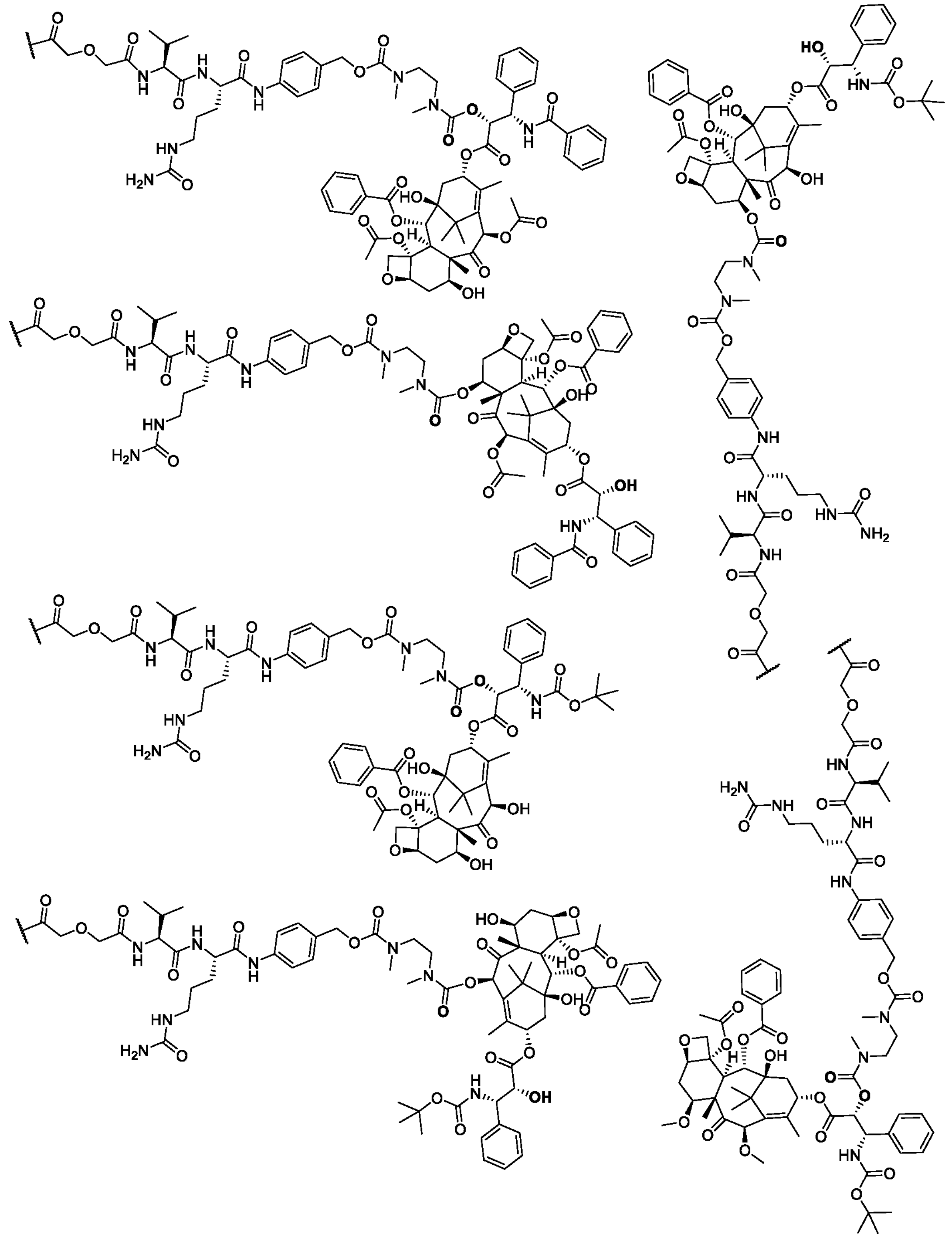

$R^p$ =

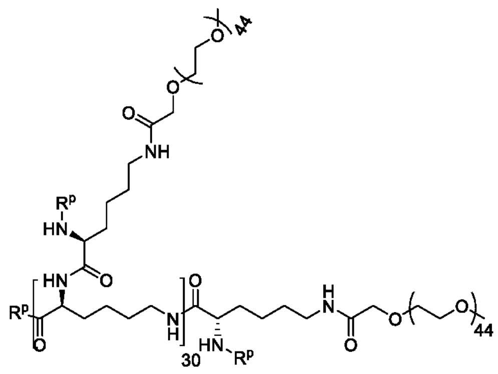

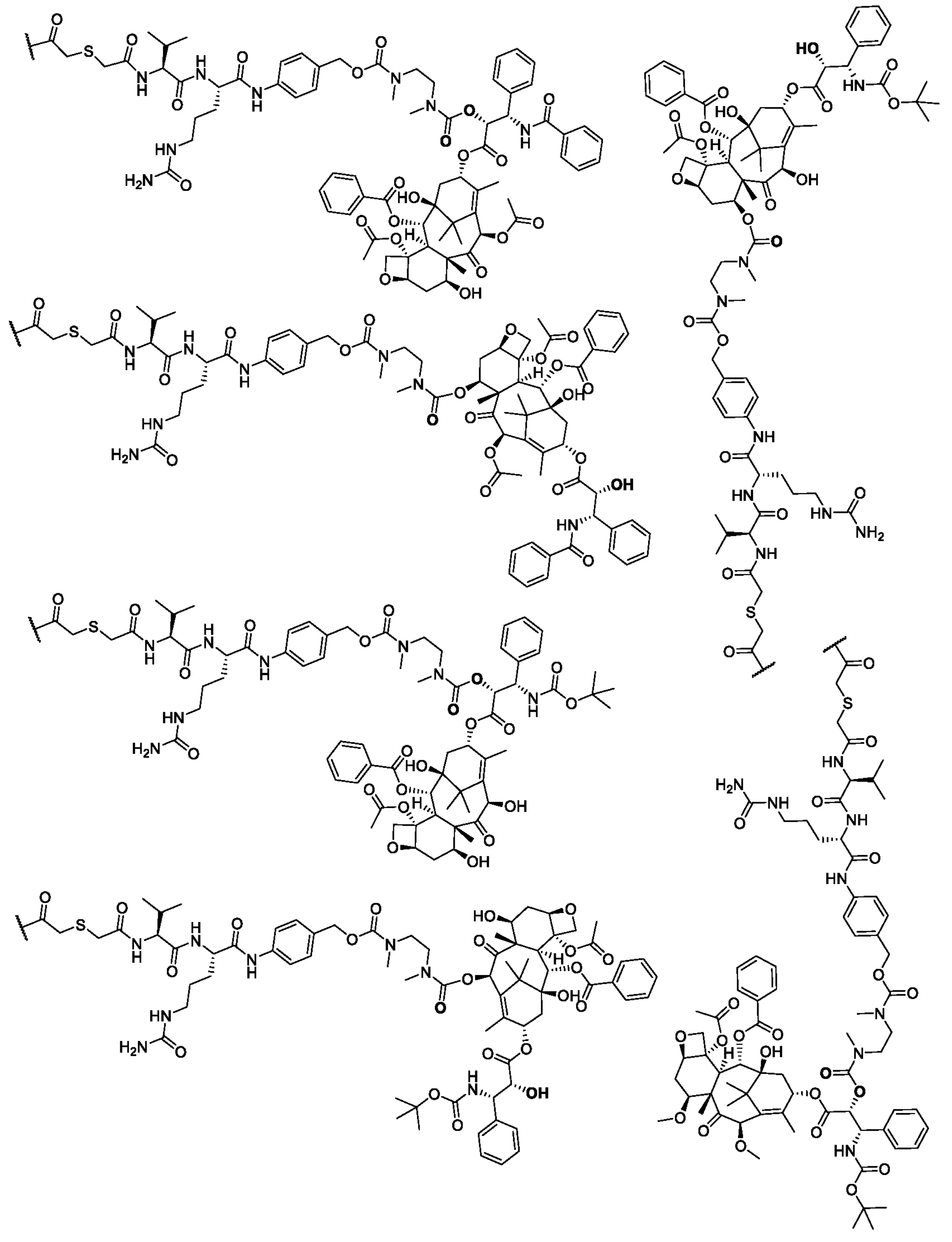

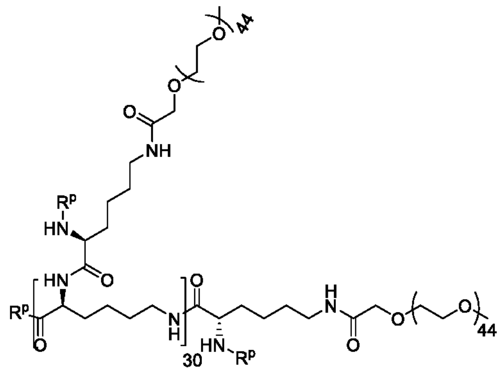

R^p =

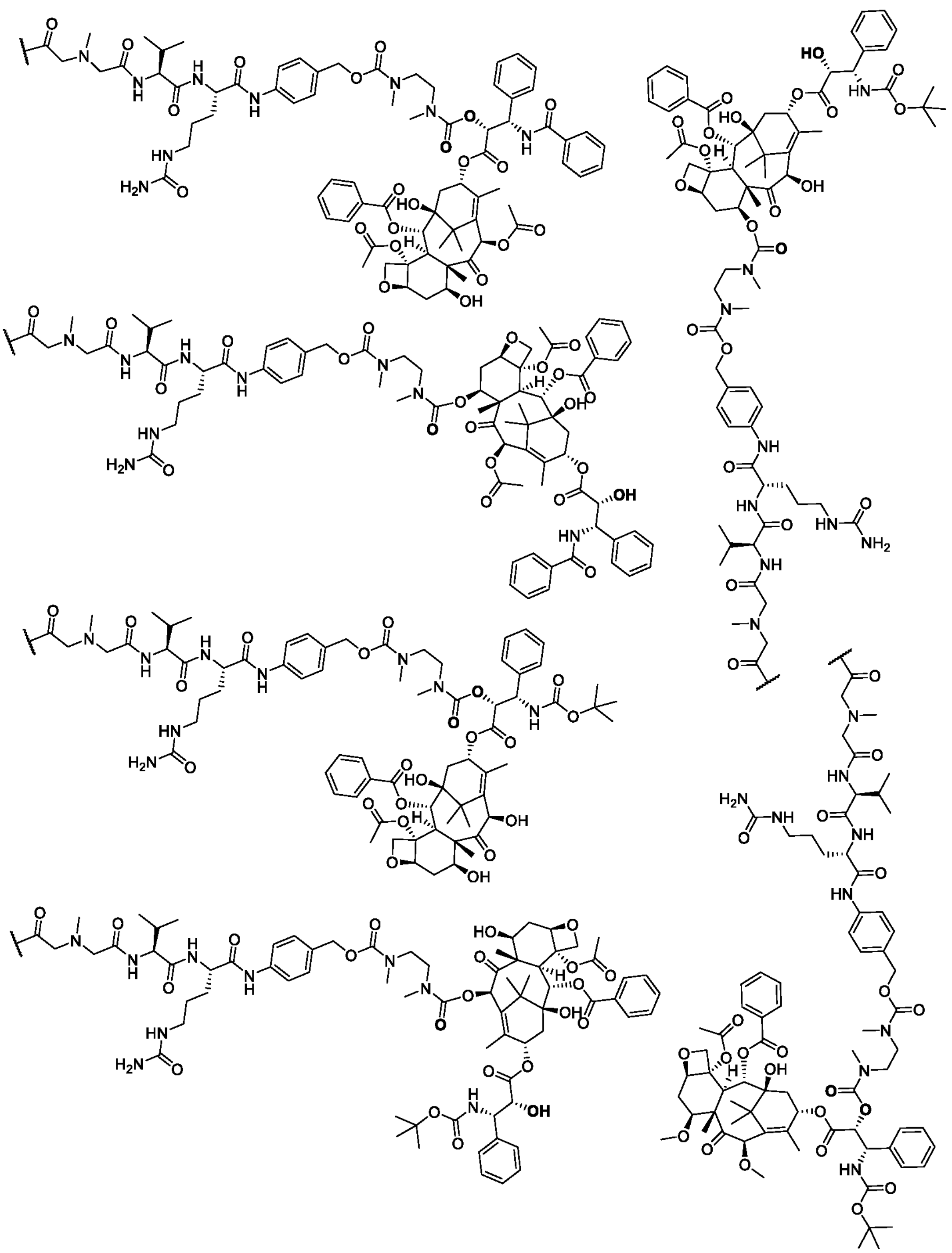

$R^p$ =

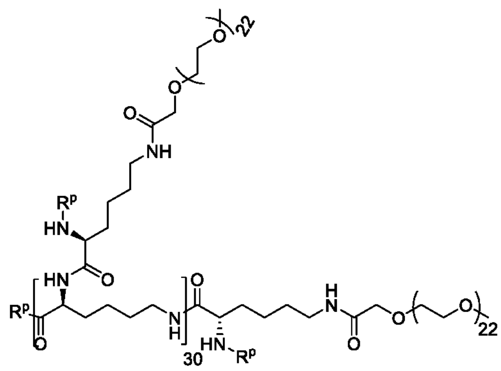

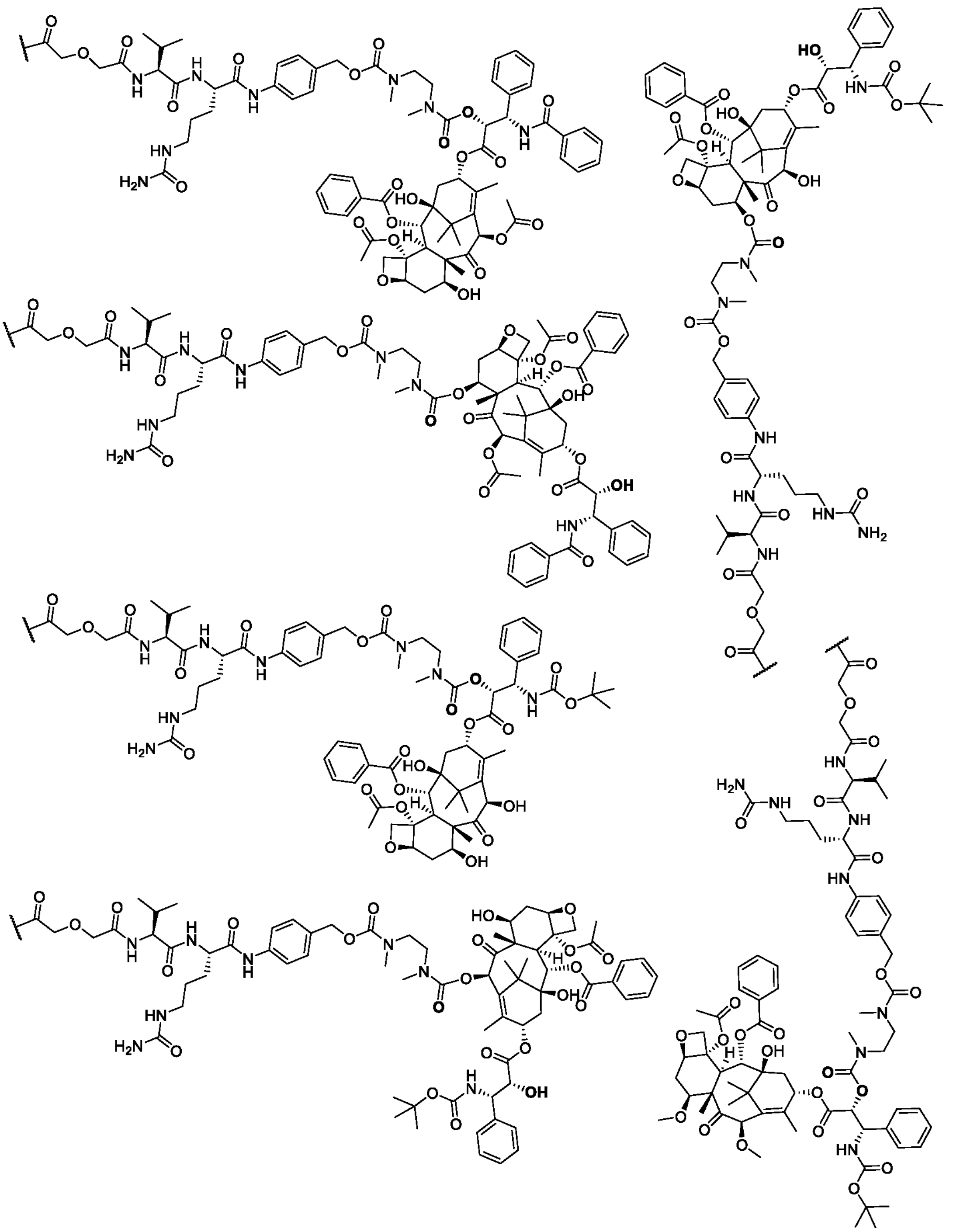

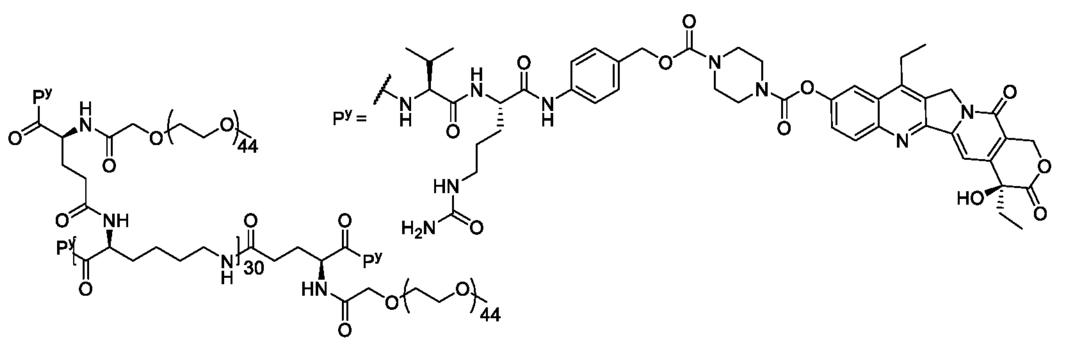

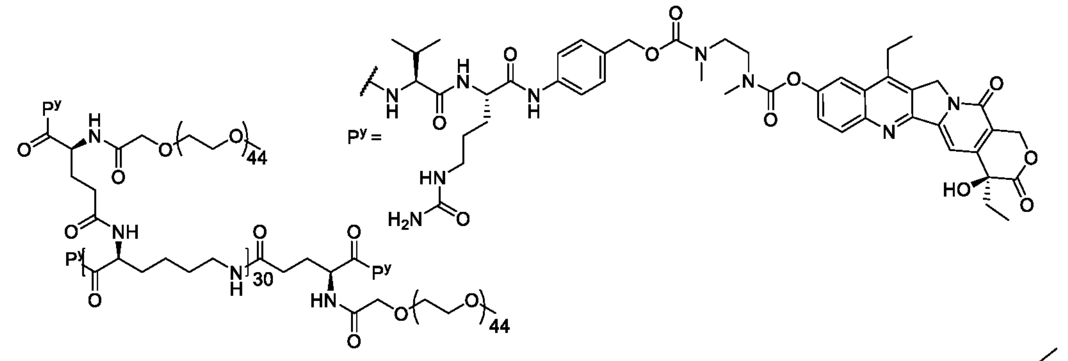

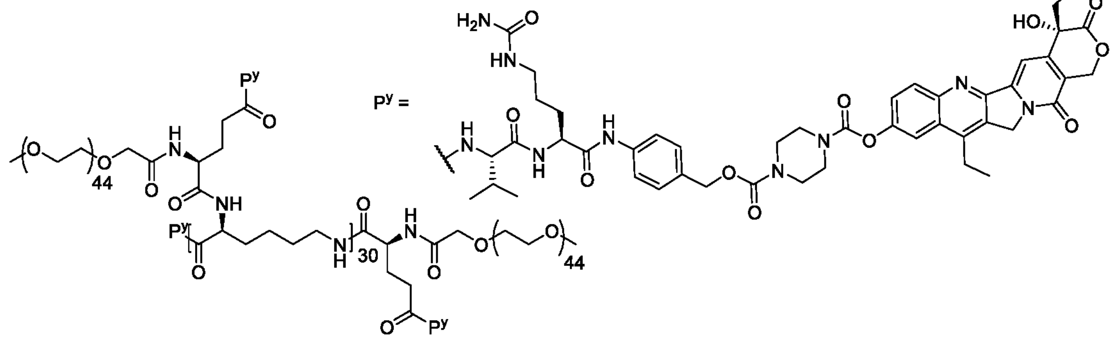

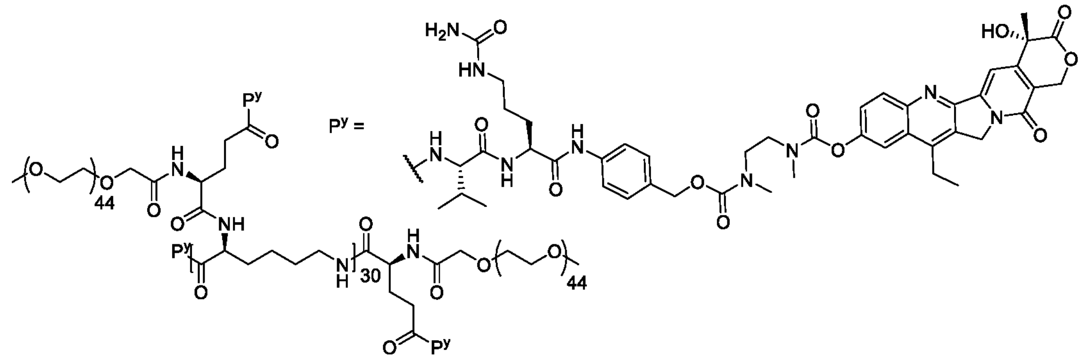

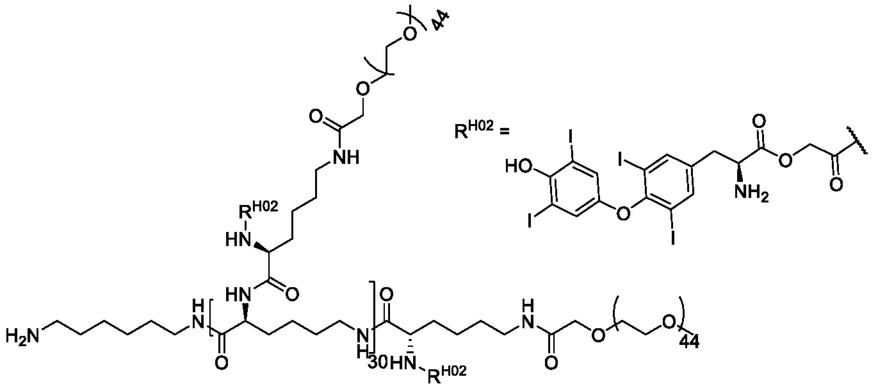

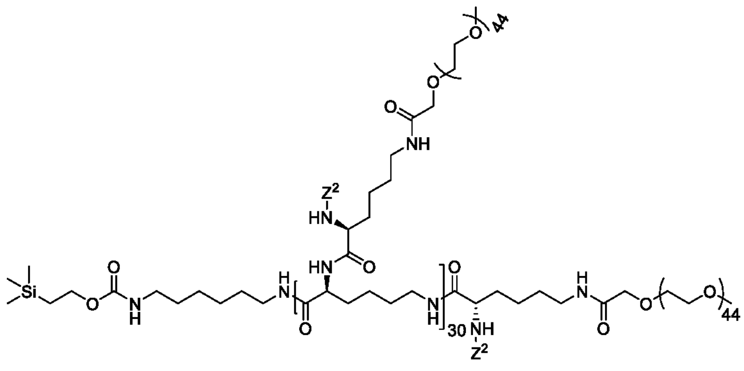

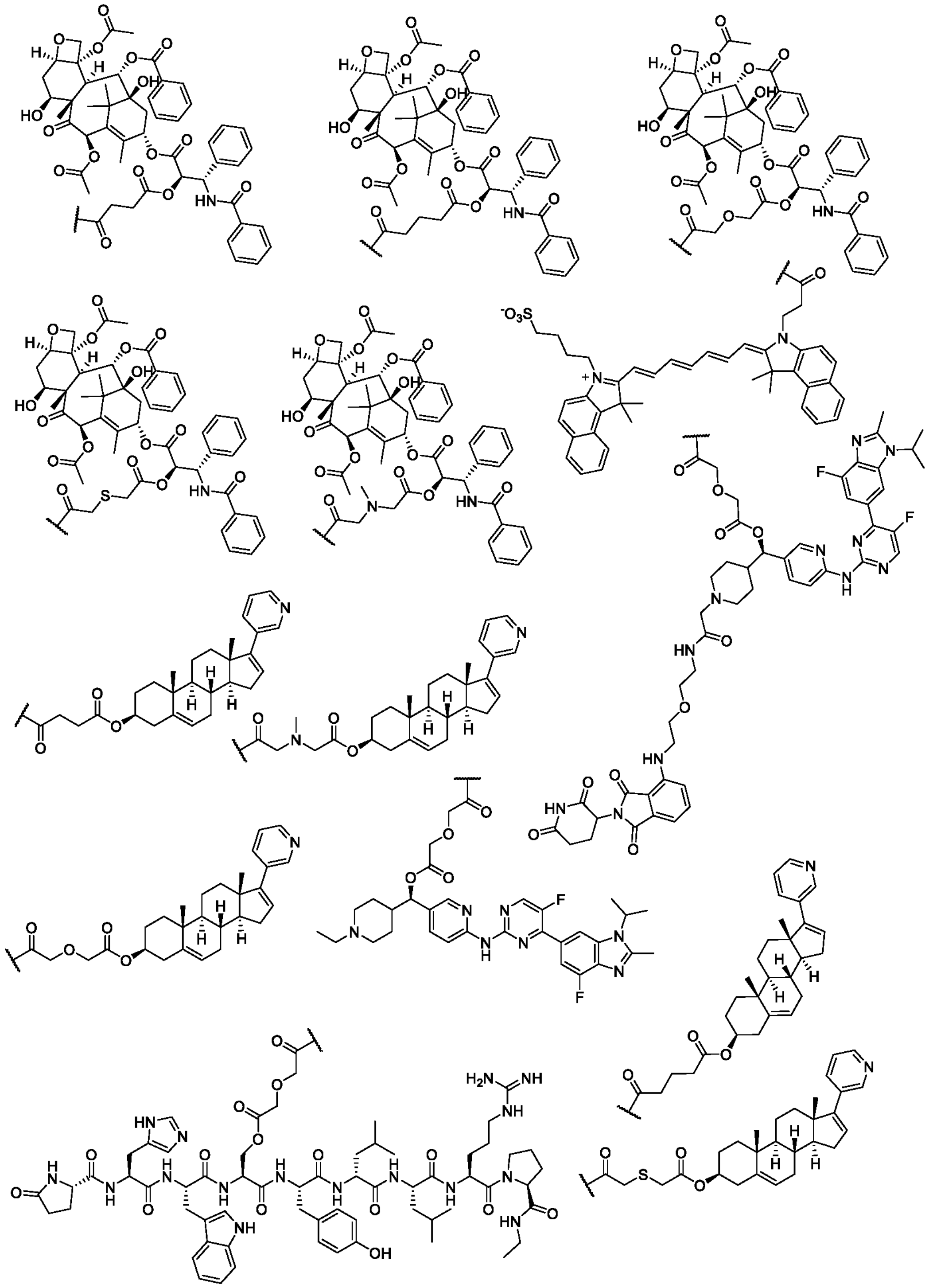

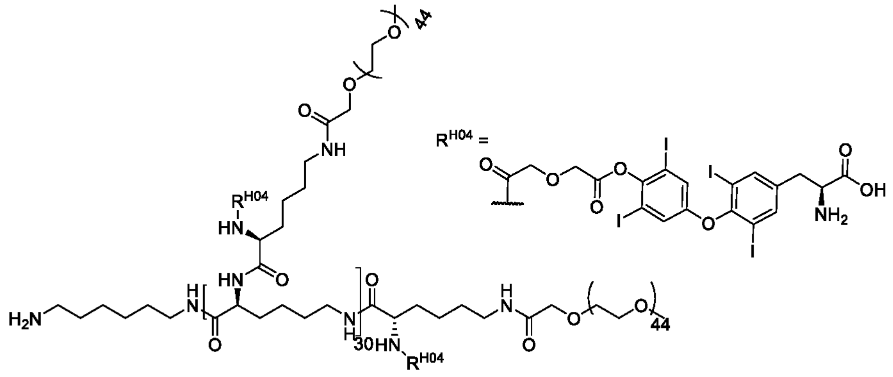

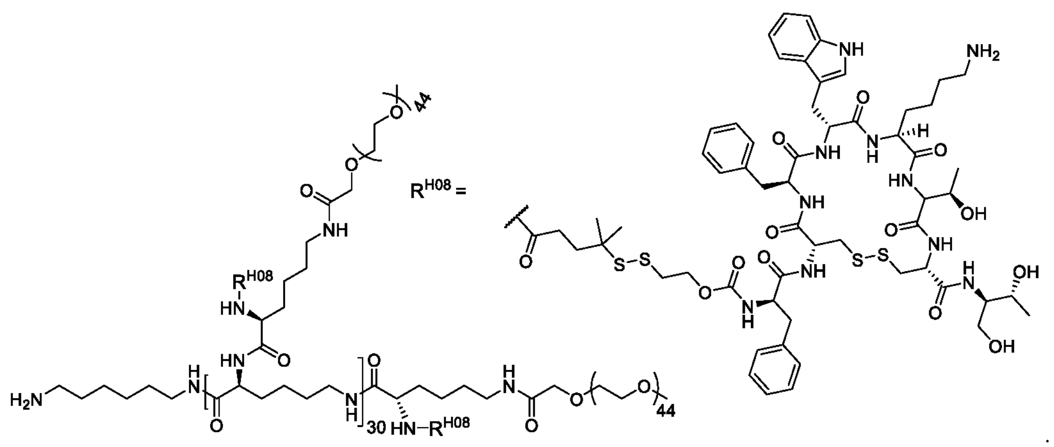

The present invention also provides compounds shown as: $R^{1'}$-$L^2$-D, where $R^{1'}$ is H, -OH or $C_1$~$C_3$ alkyl, $L^2$ and D are defined as described above, and the $C_1$~$C_3$ alkyl is preferably methyl.

The present invention also provides the following compounds:

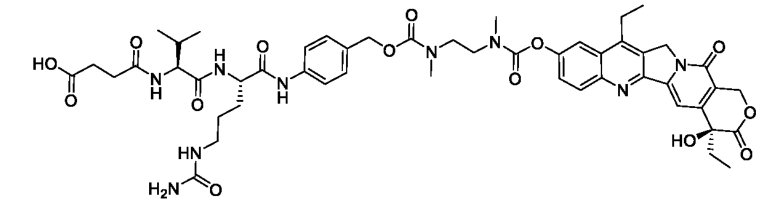

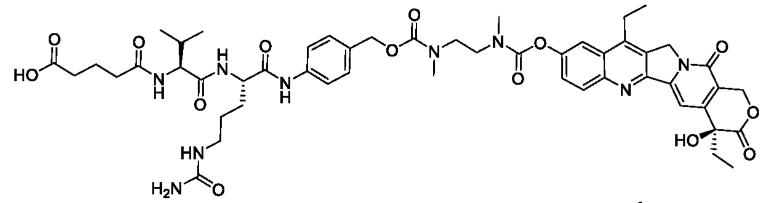

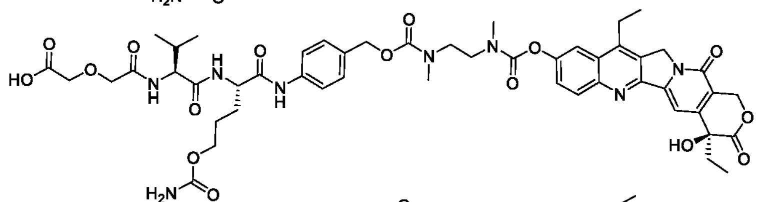

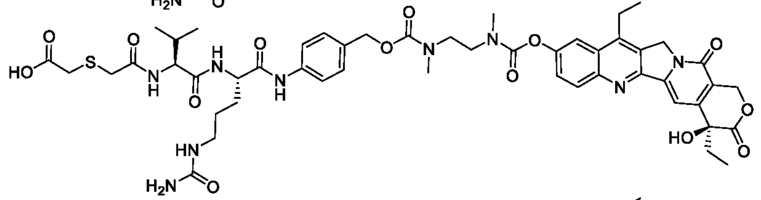

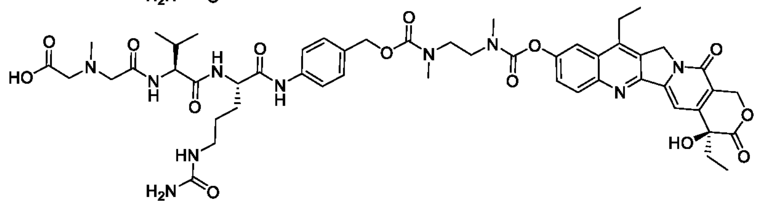

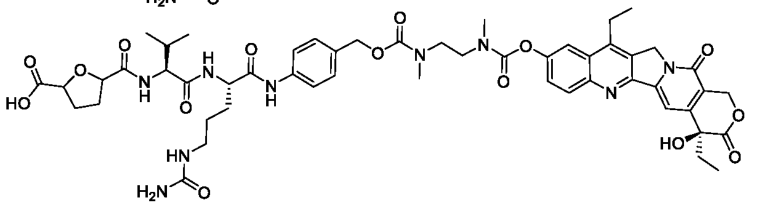

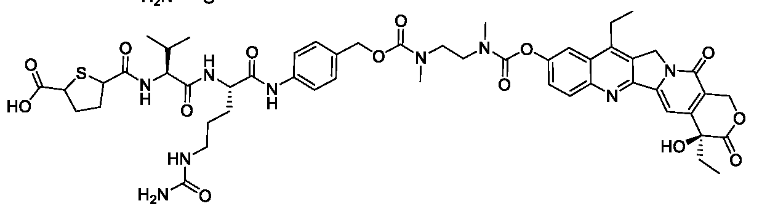

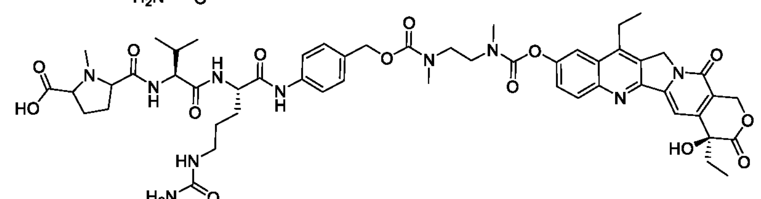

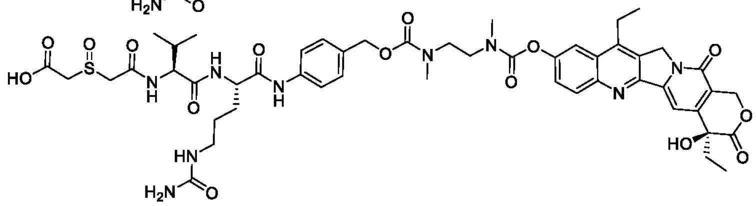

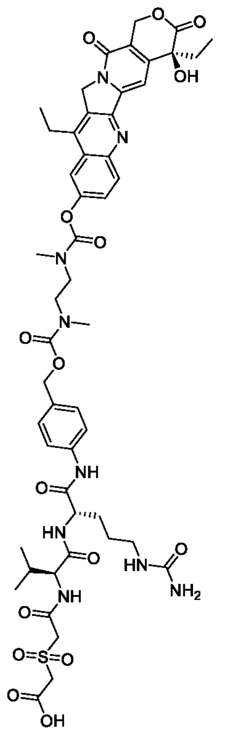

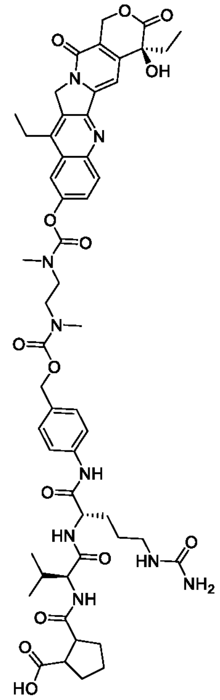

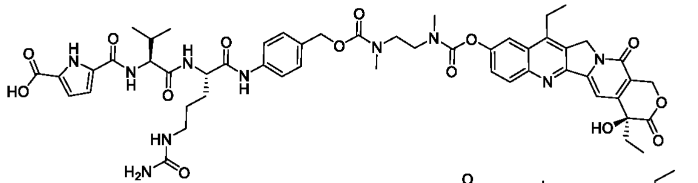

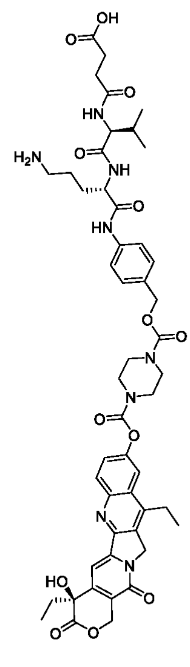

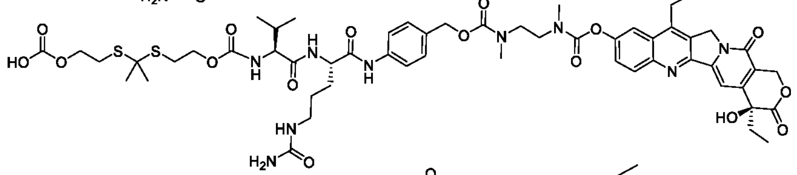

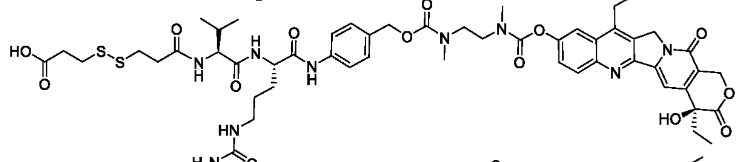

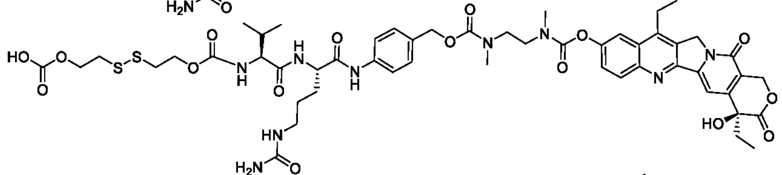

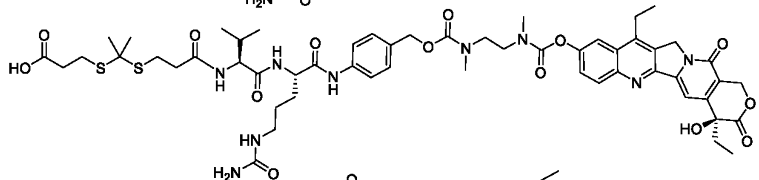

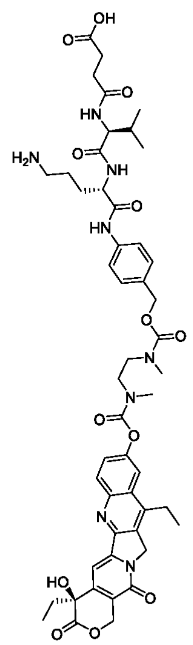

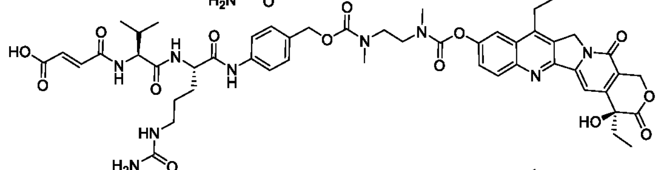

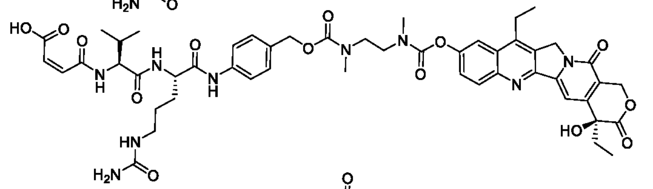

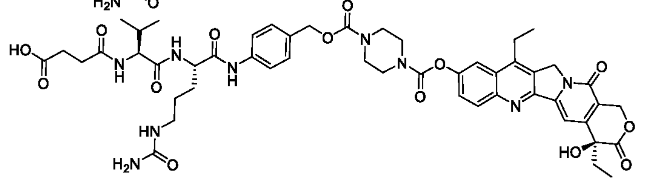

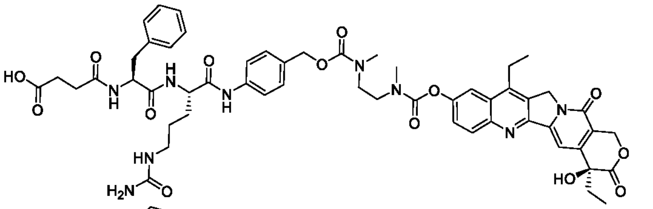

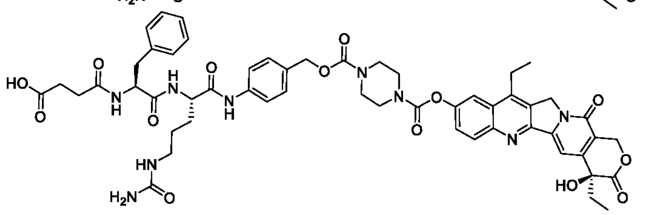

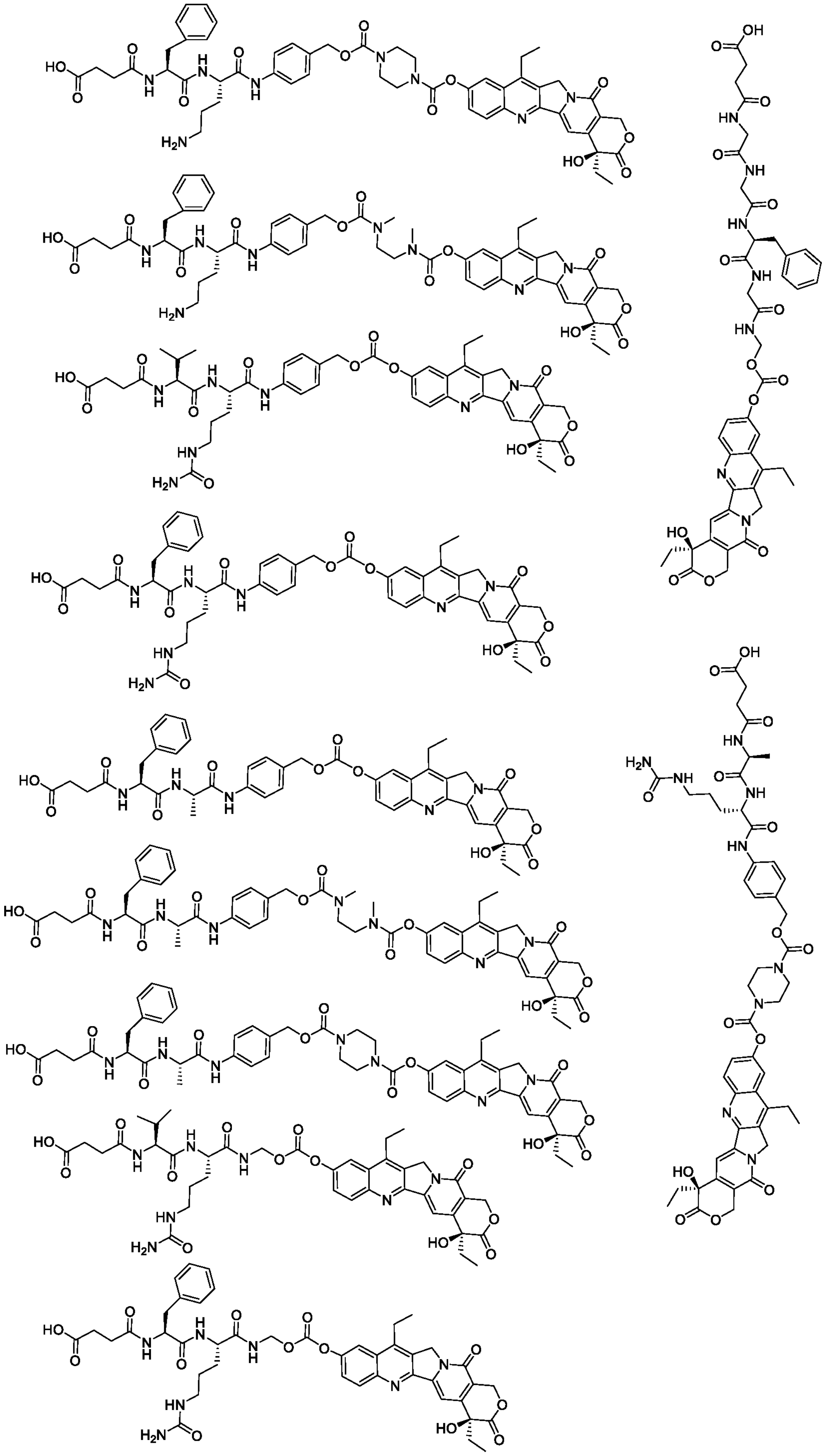

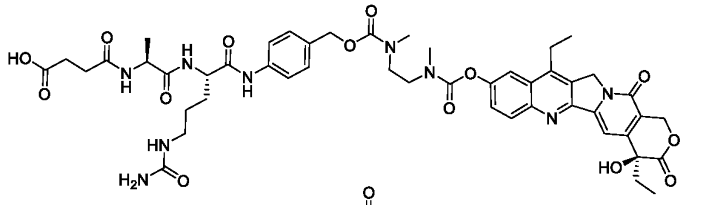

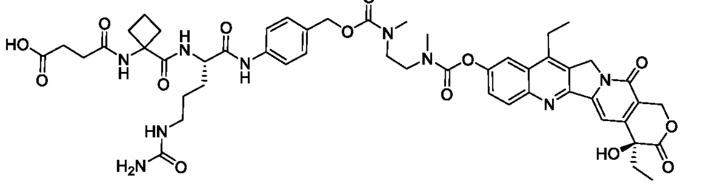

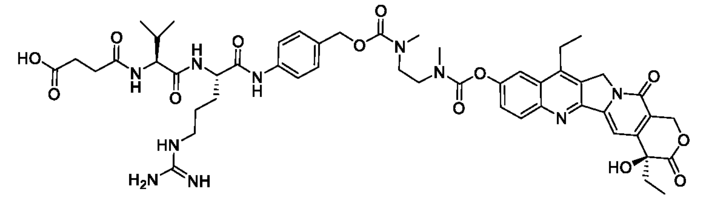

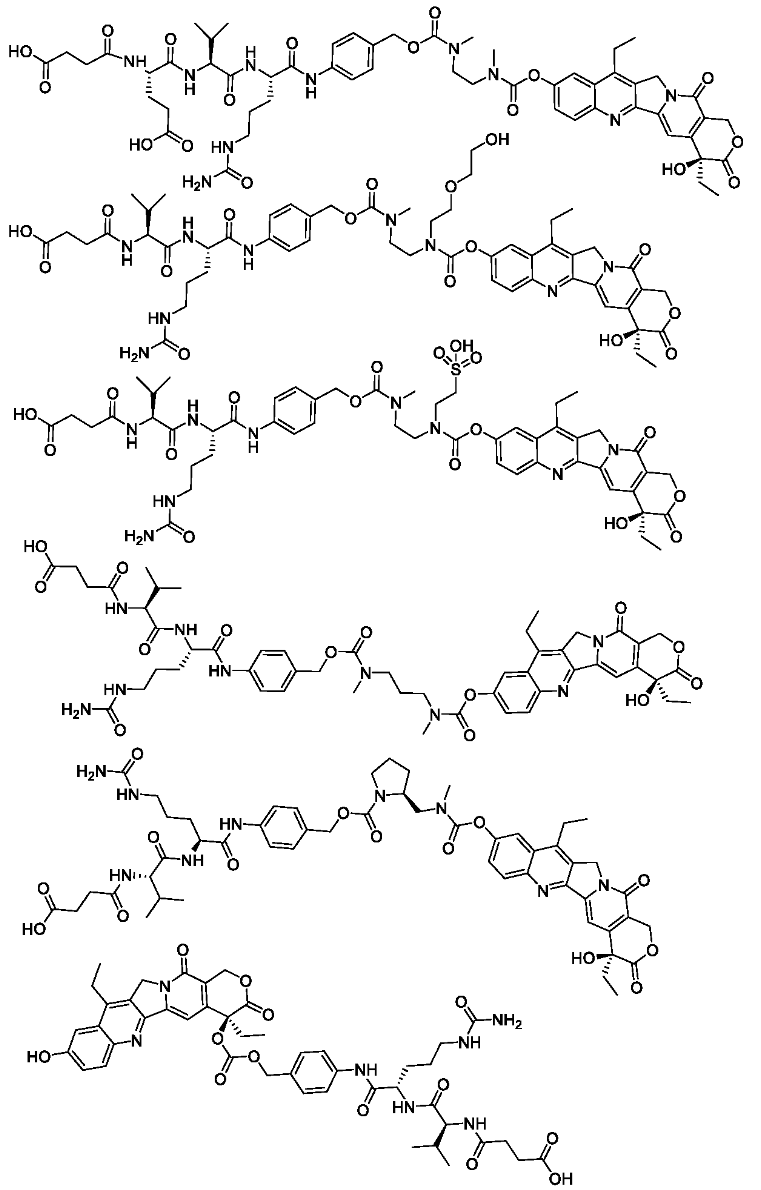

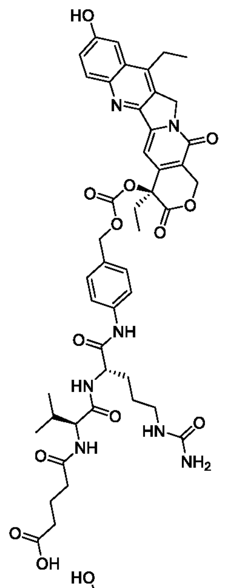

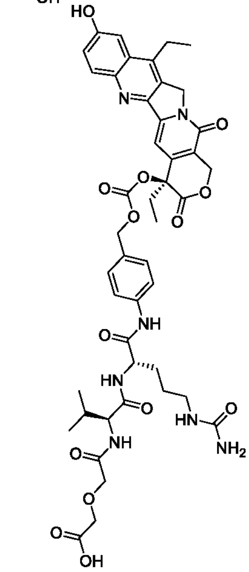

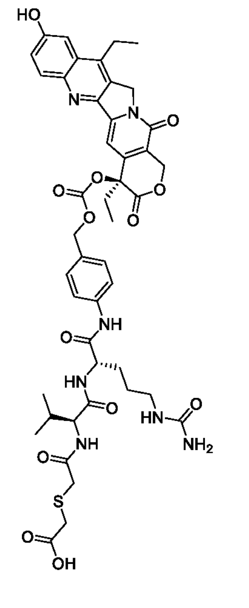

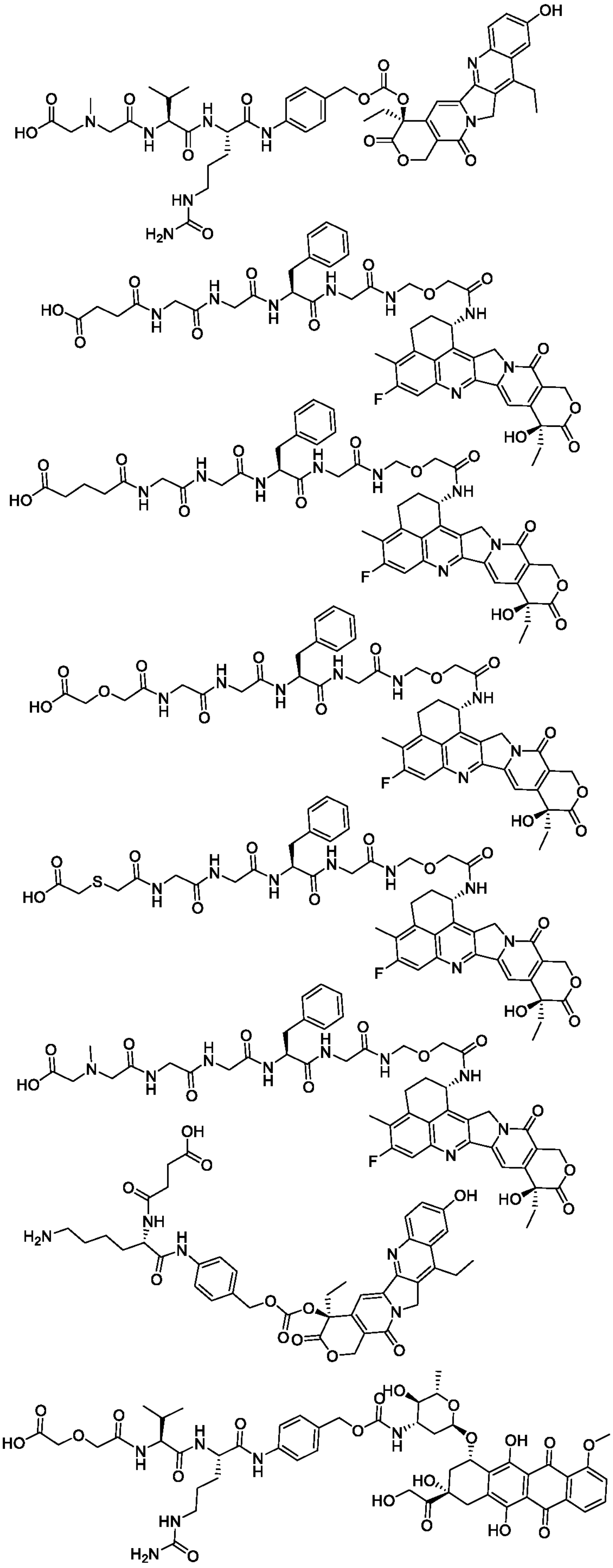

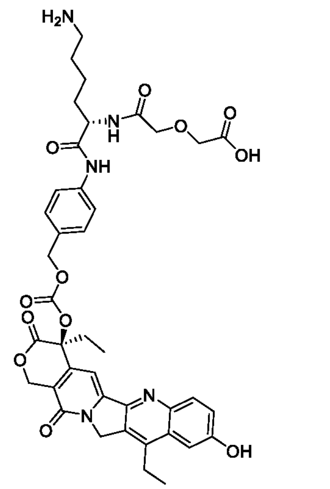

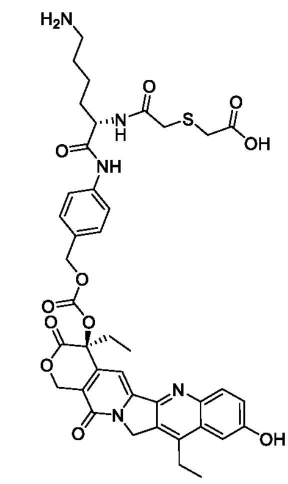

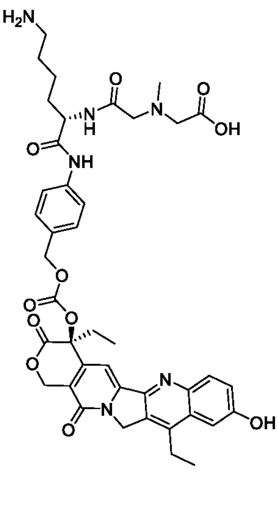

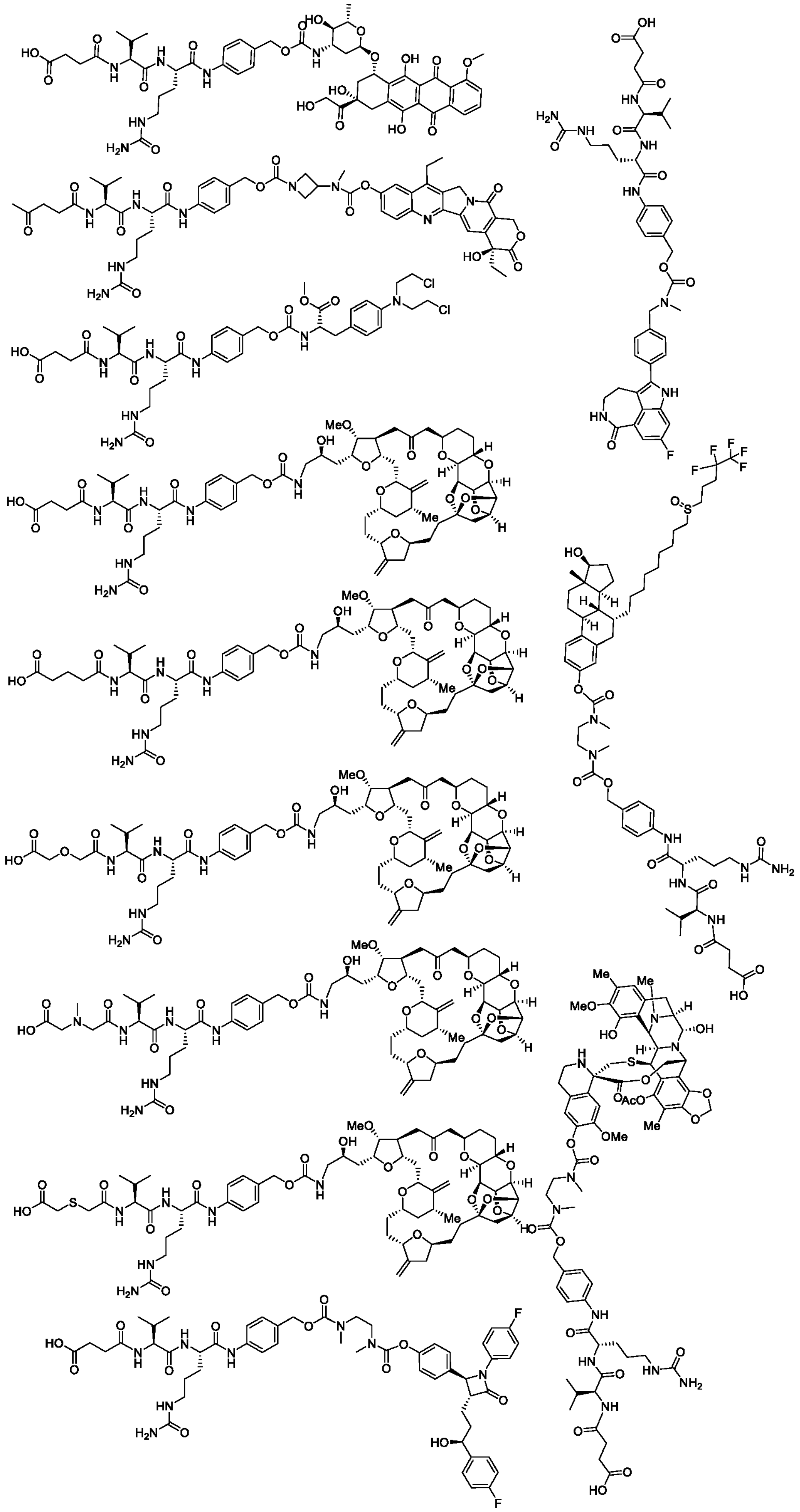

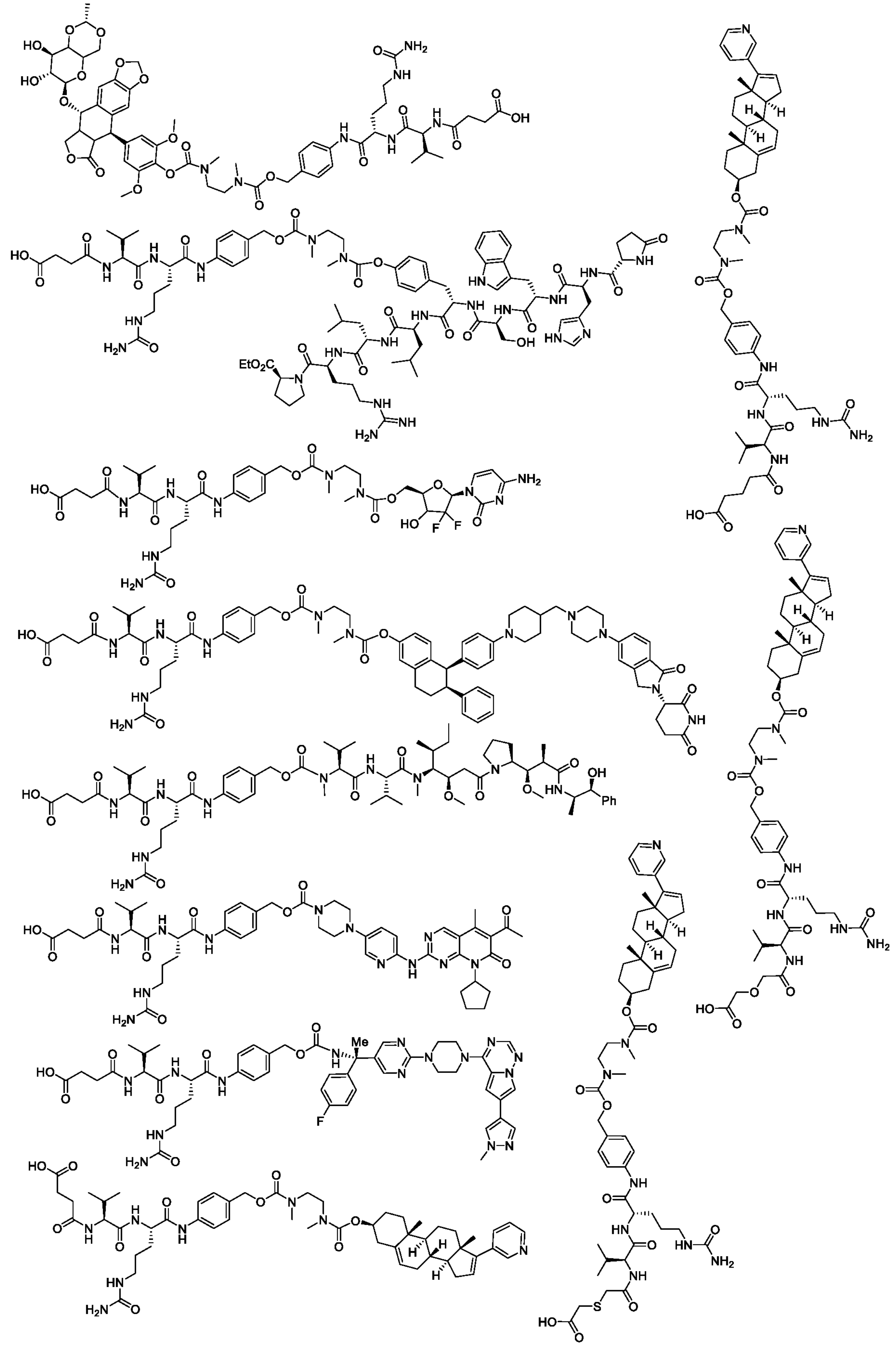

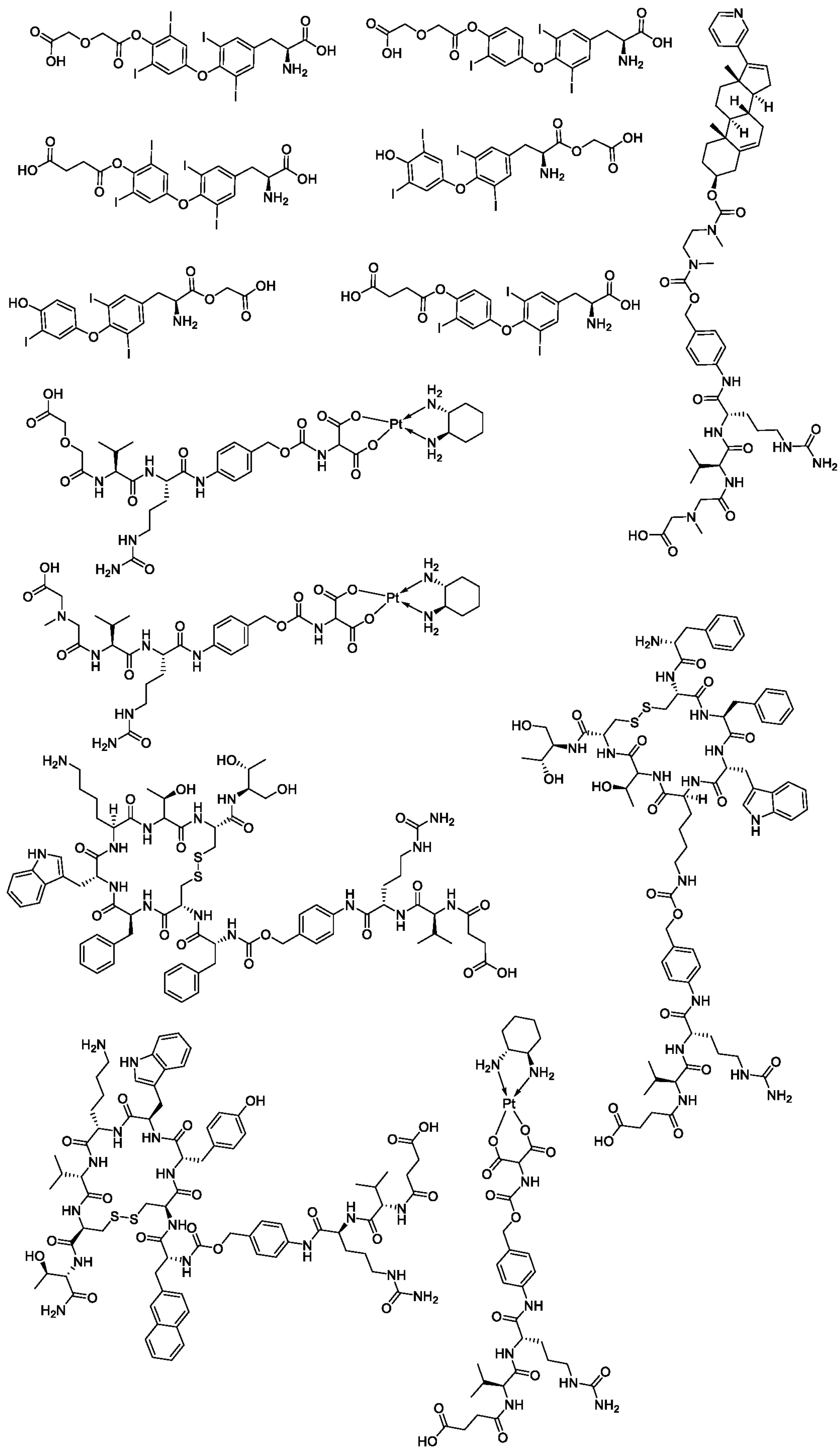

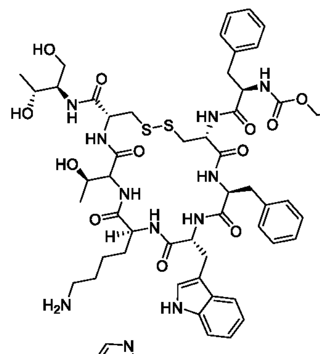

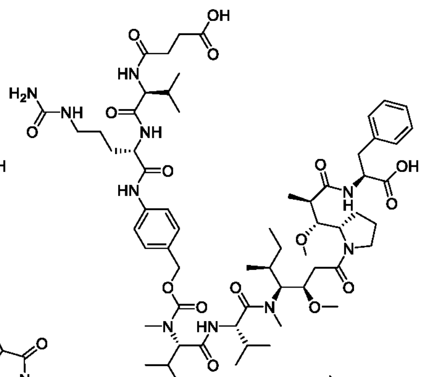

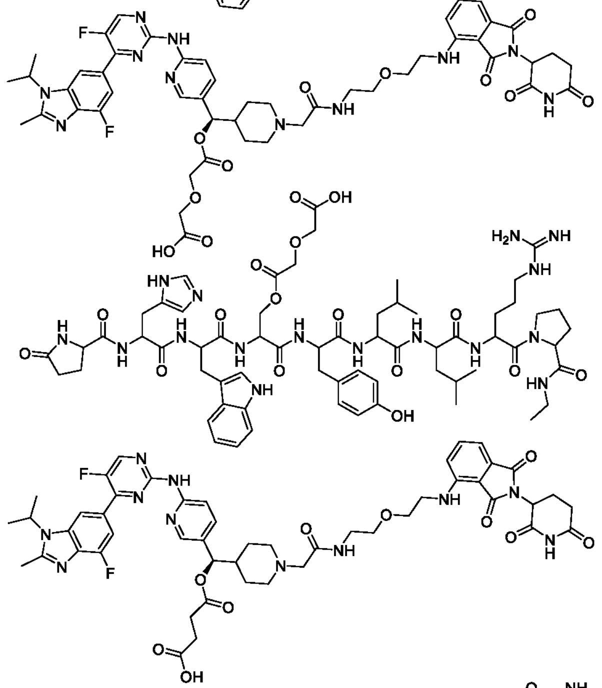

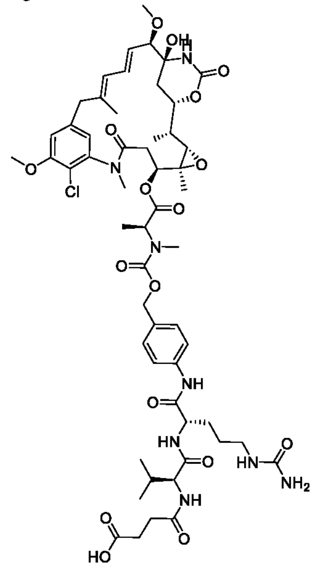

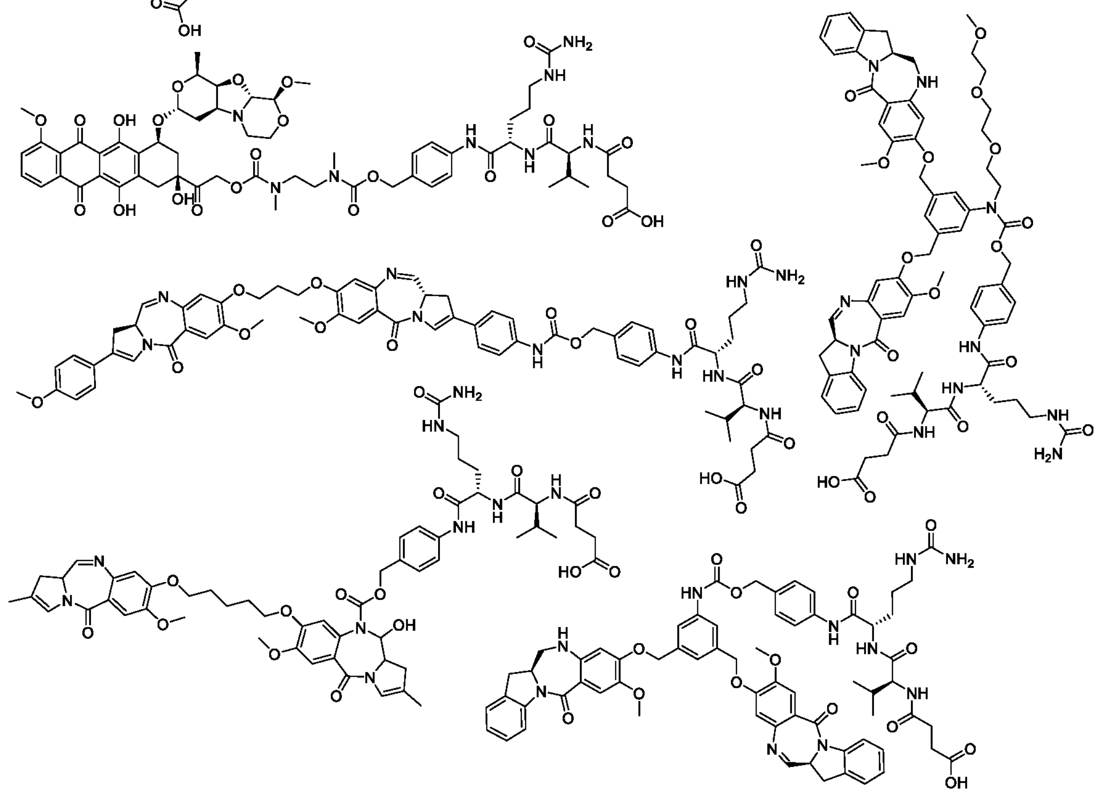

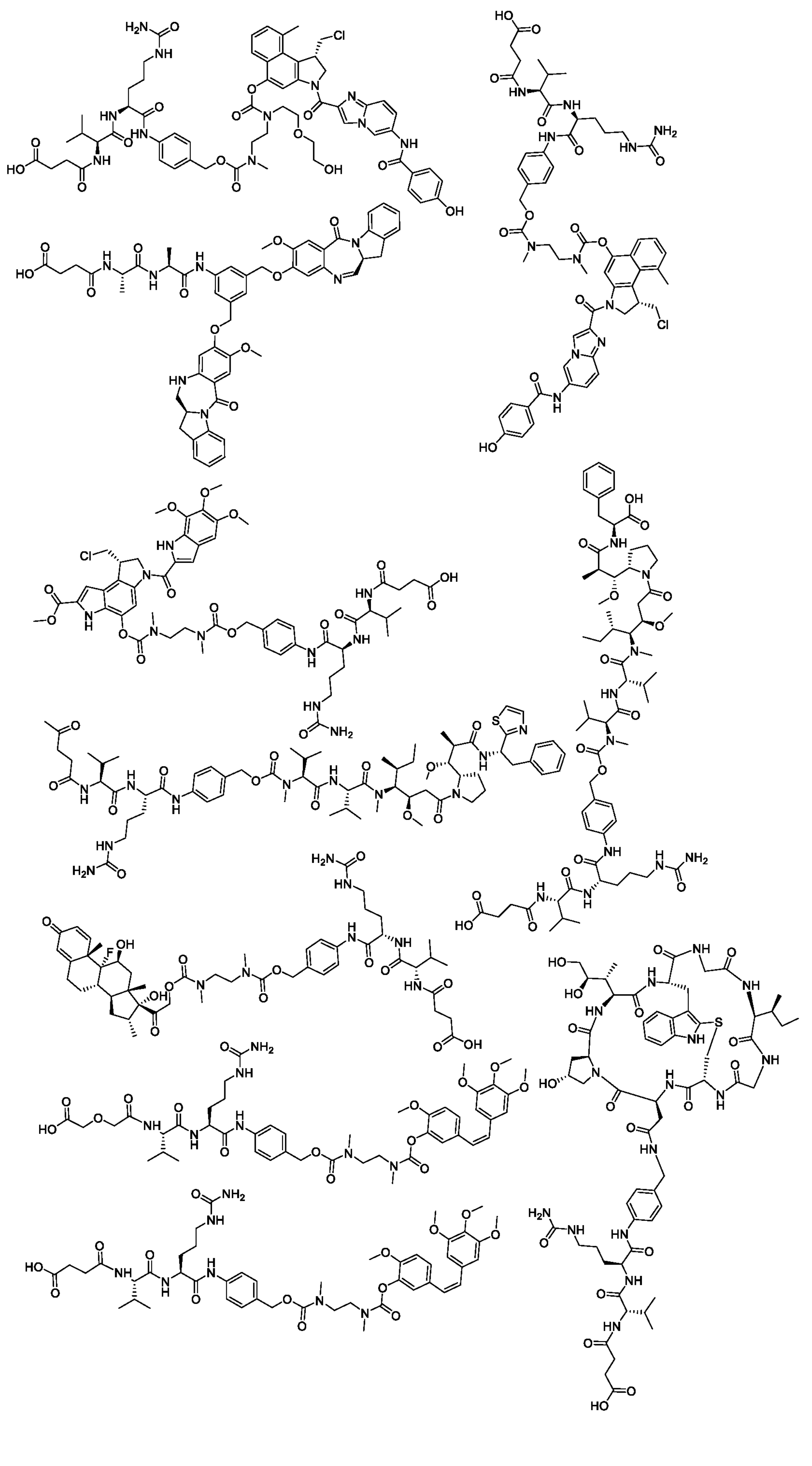

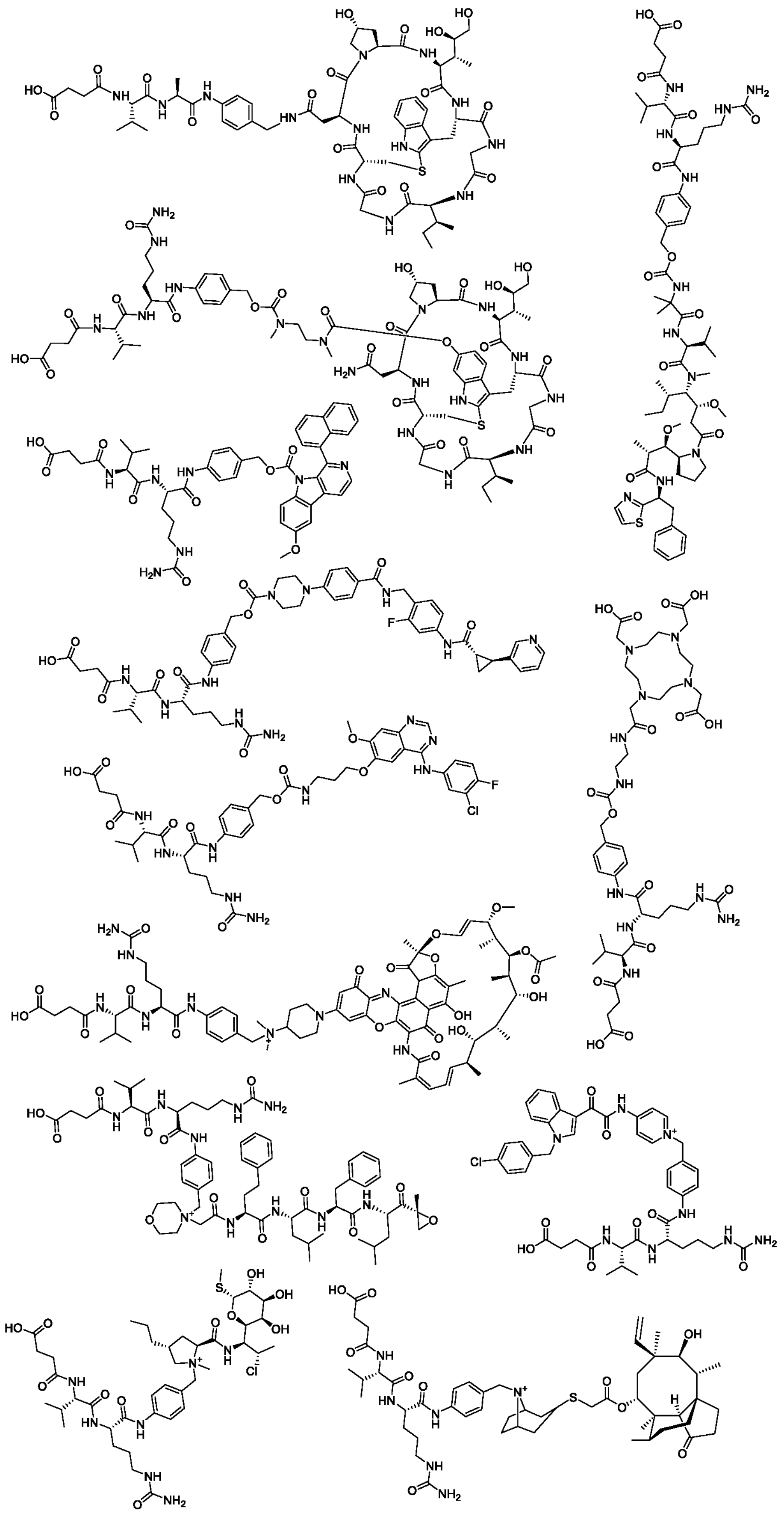

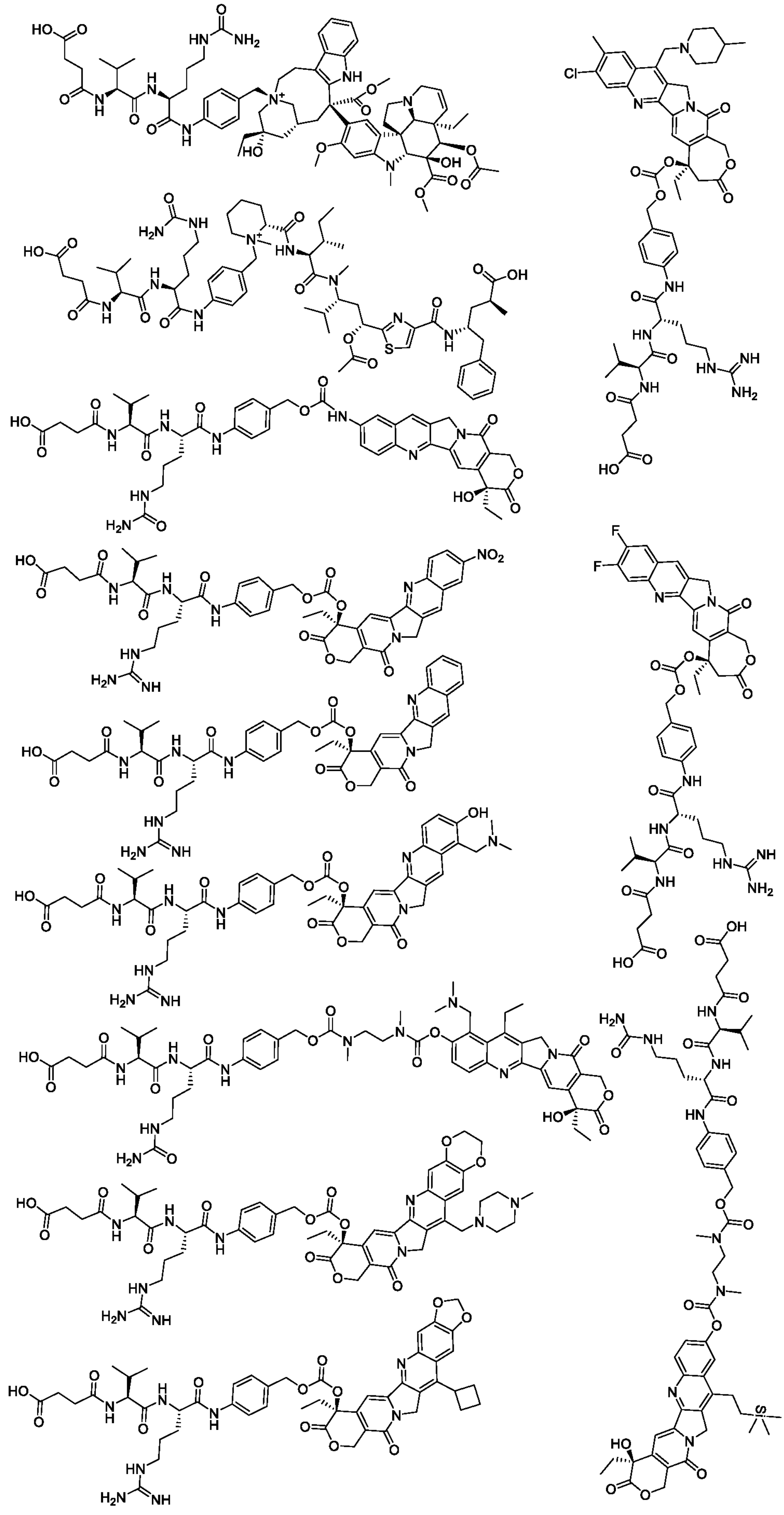

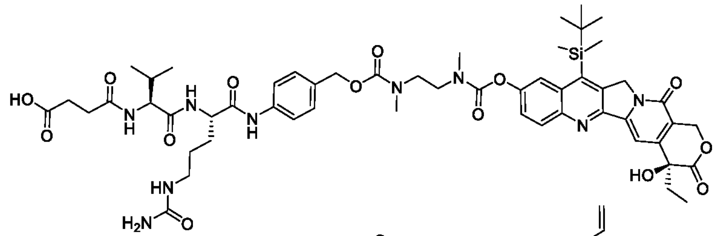

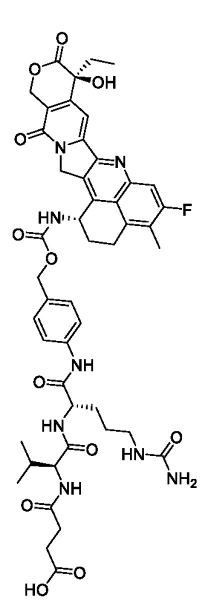

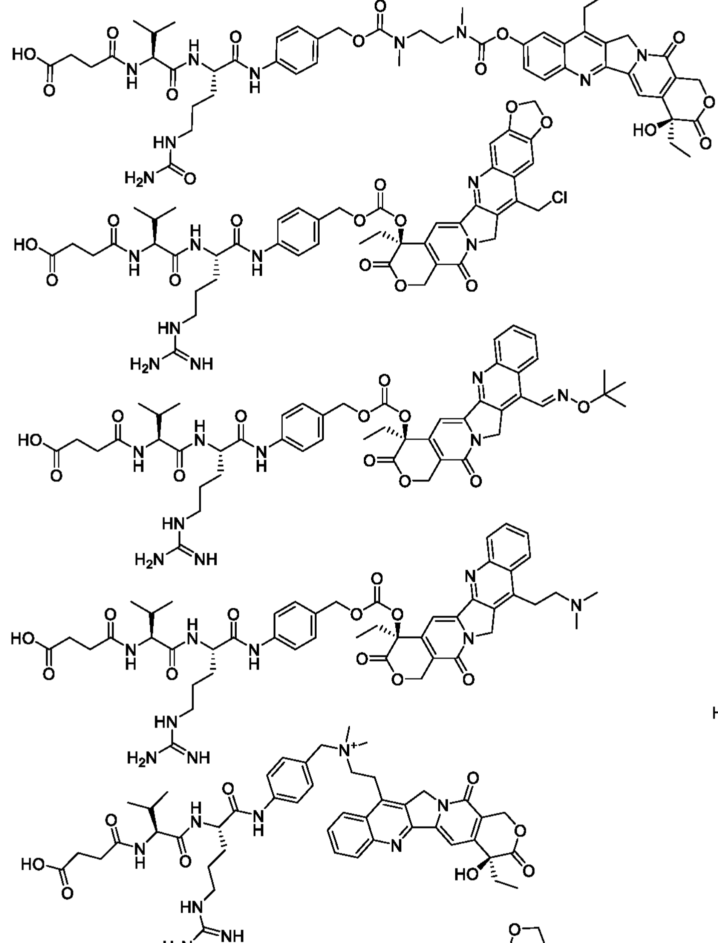

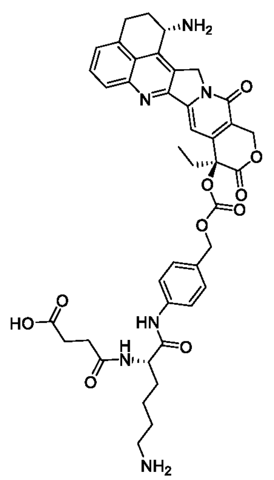

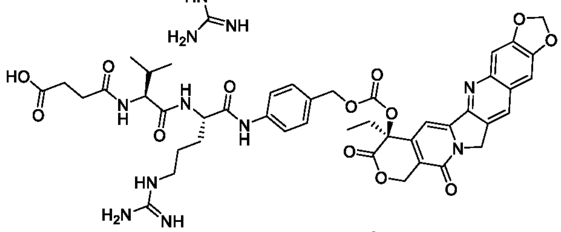

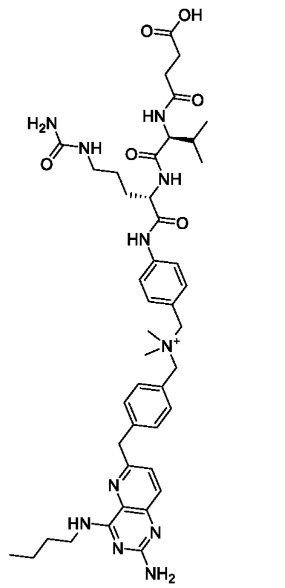

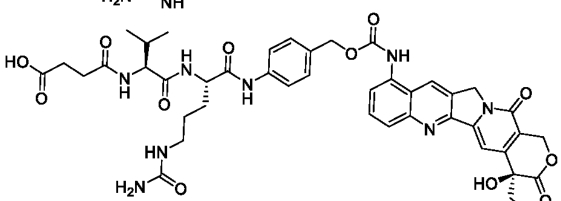

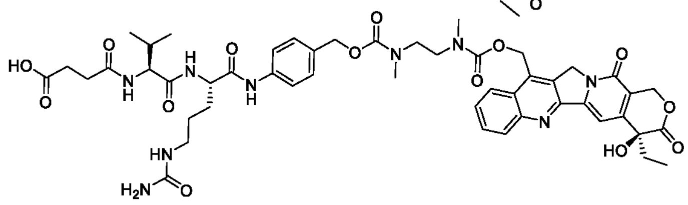

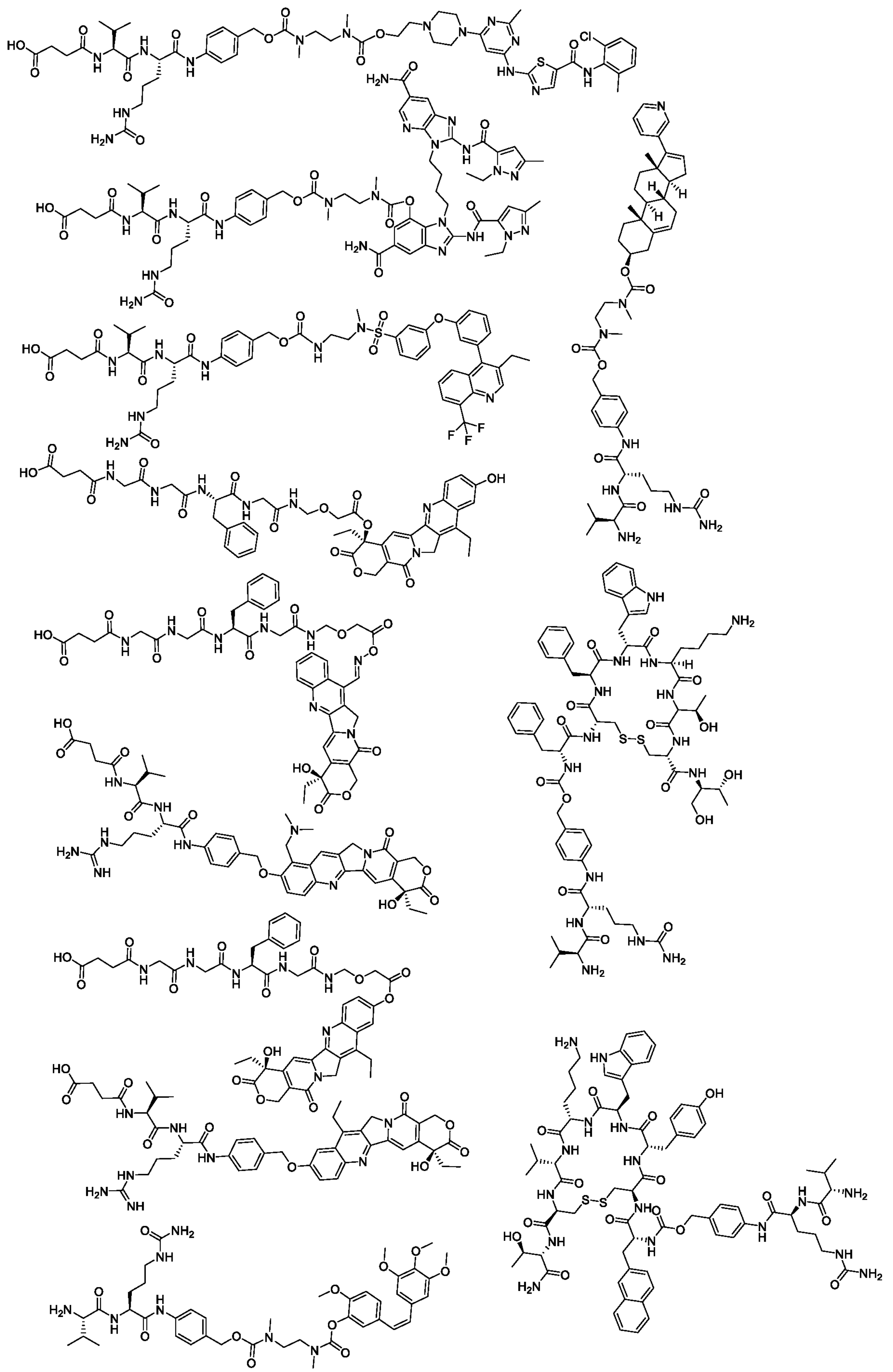

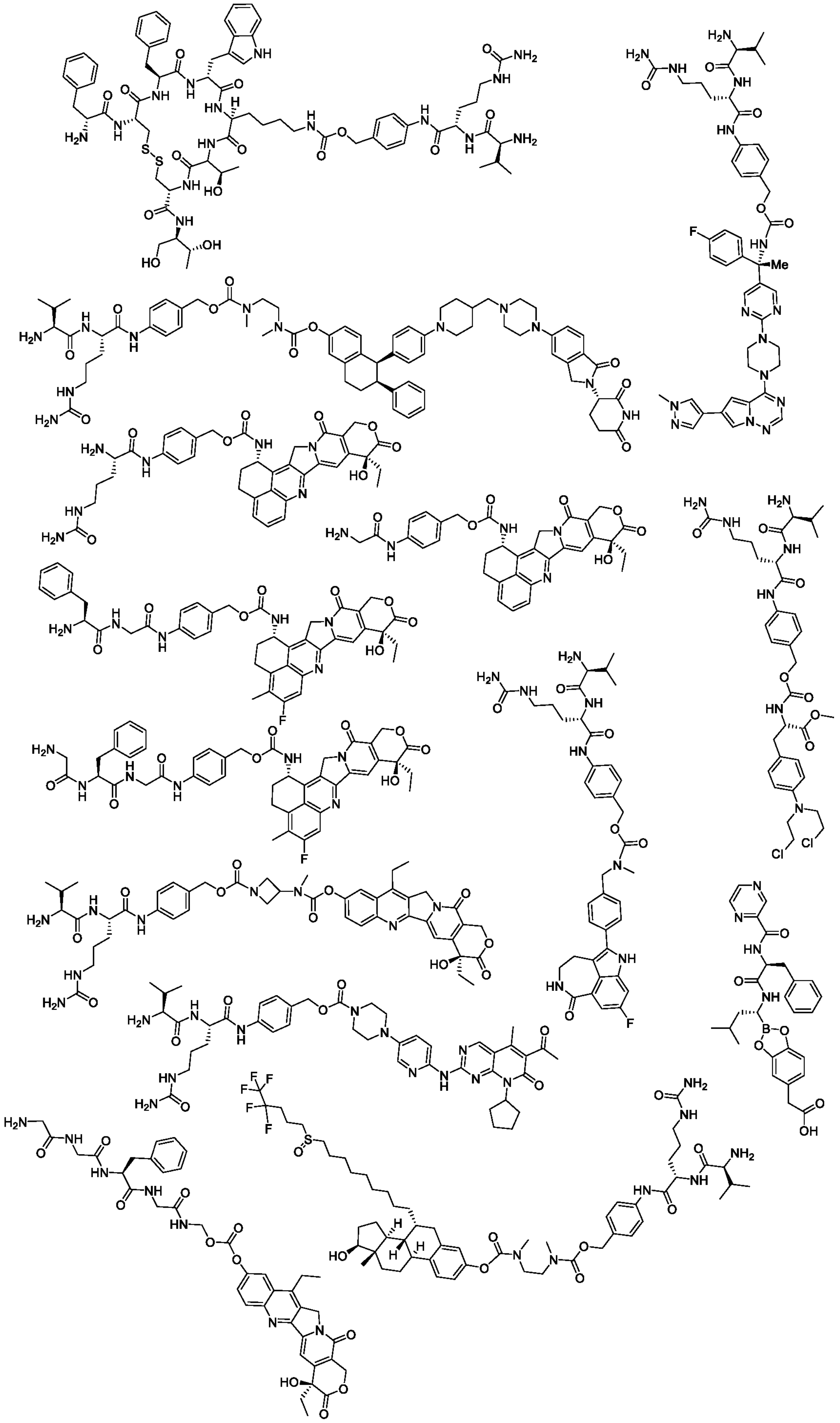

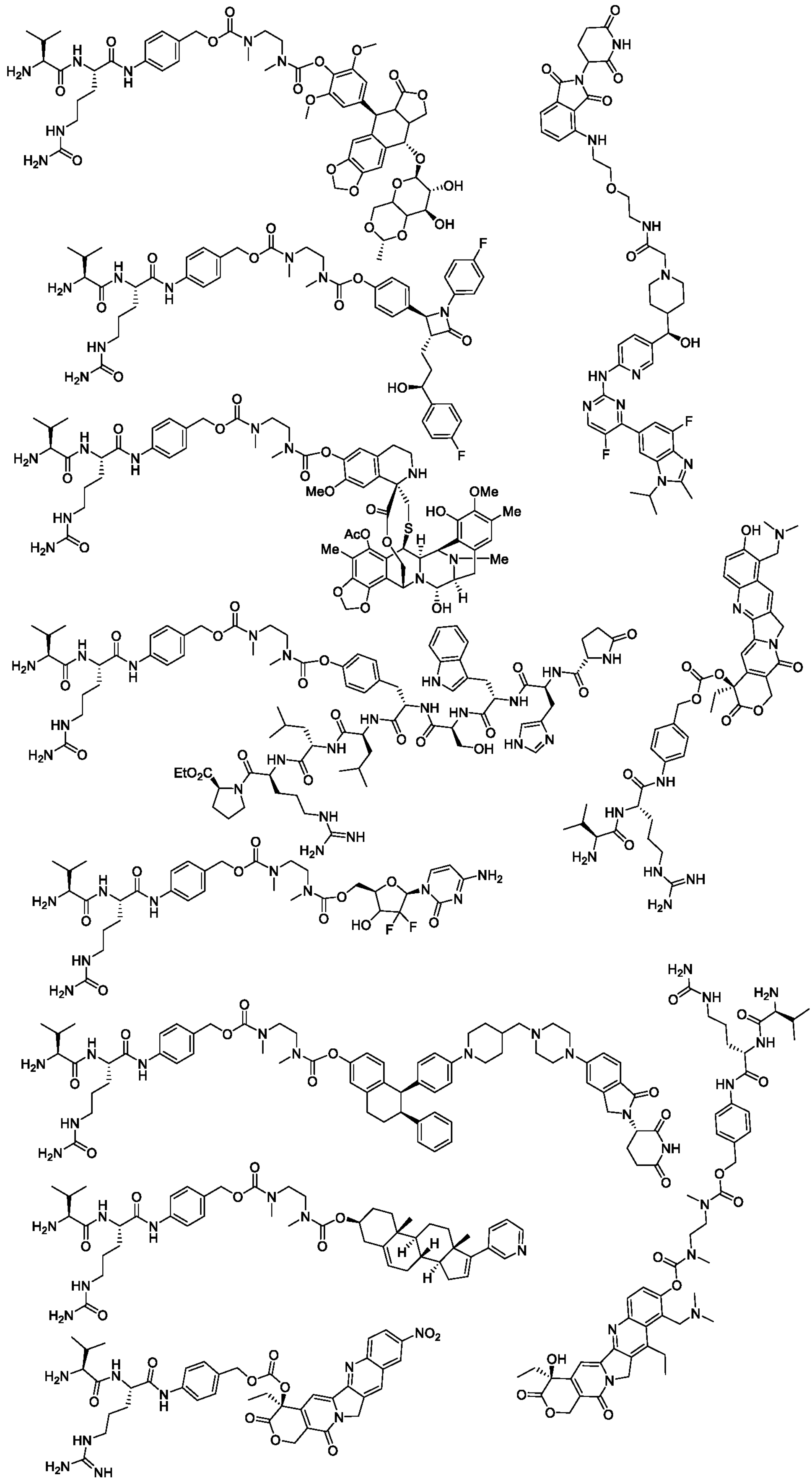

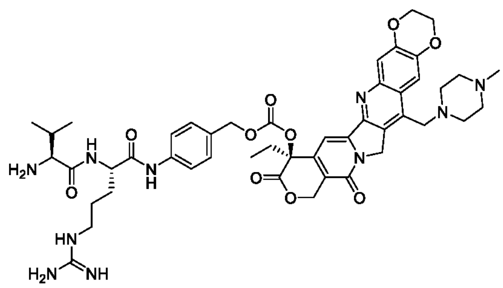

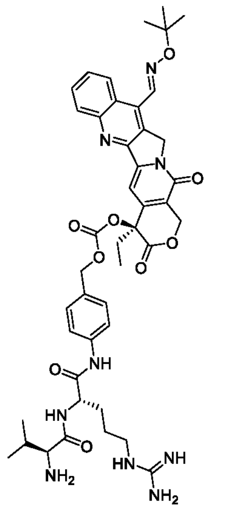

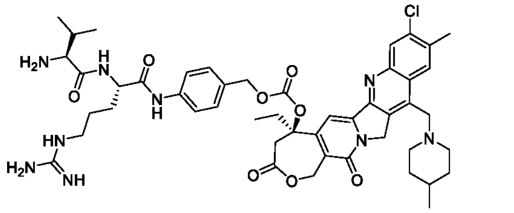

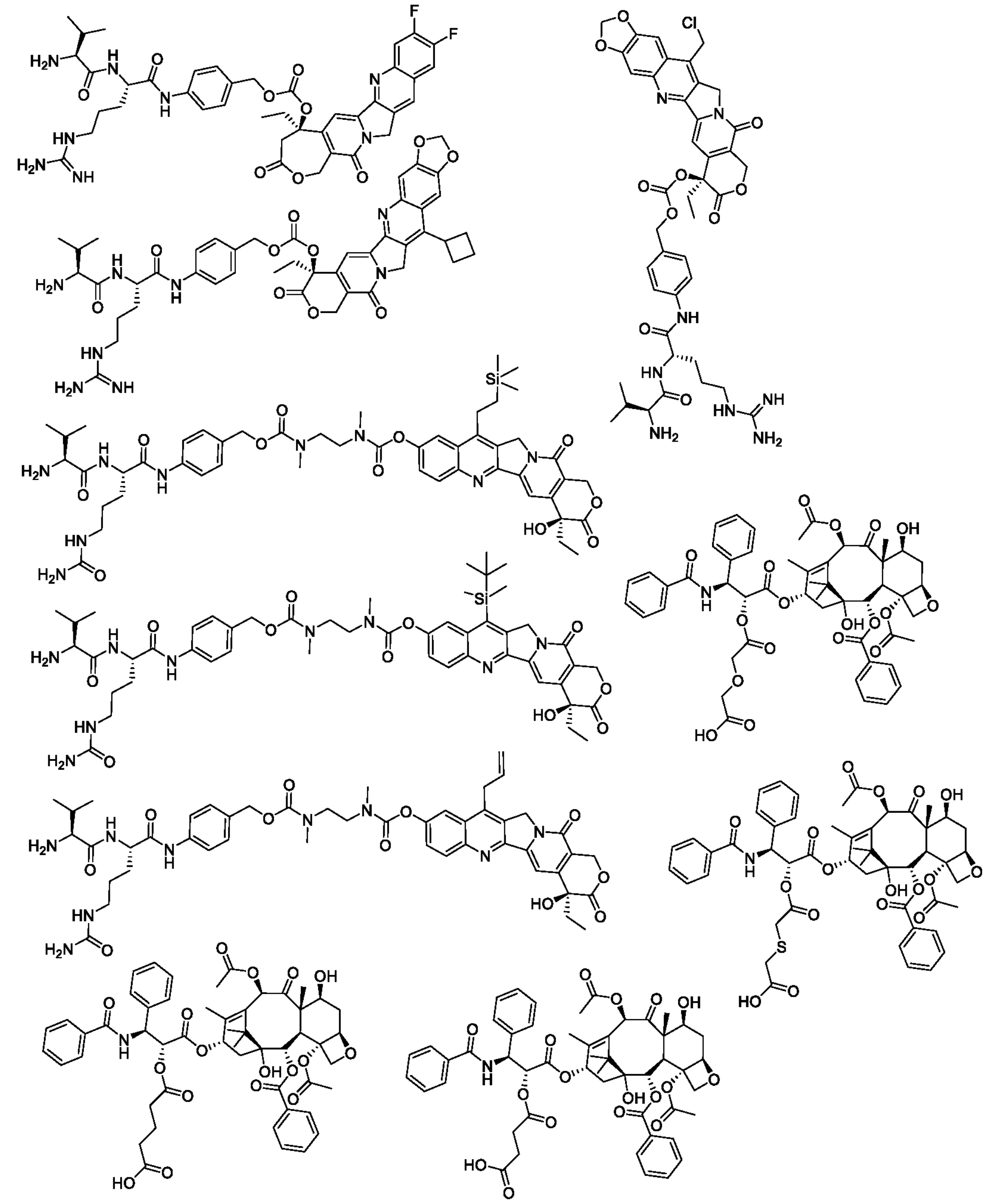

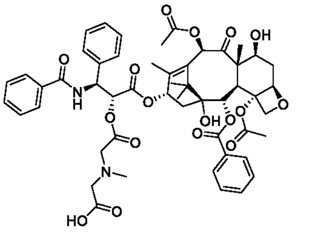
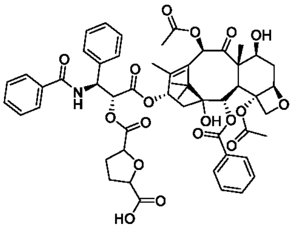
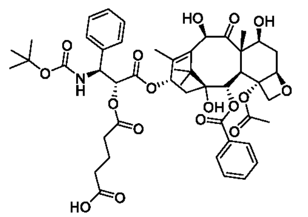
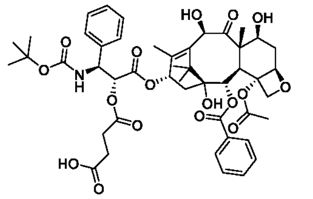
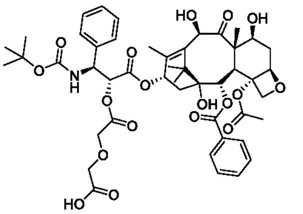
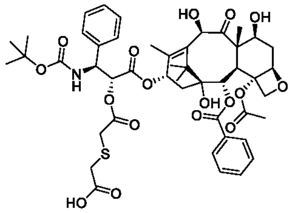
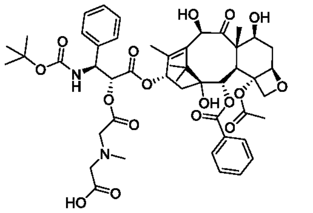
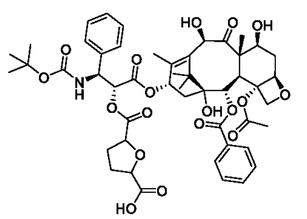
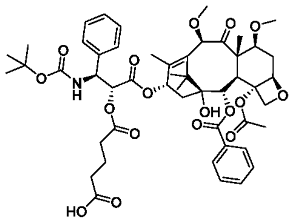
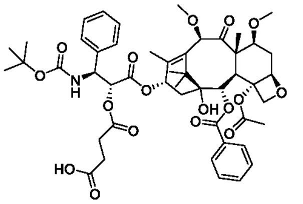
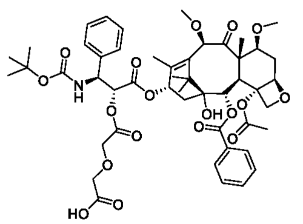
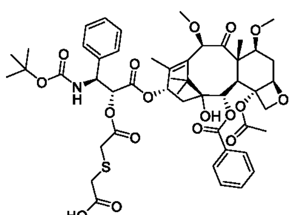
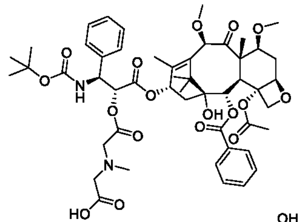
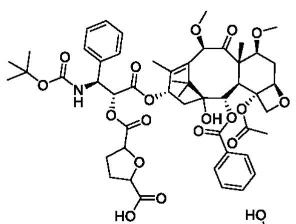
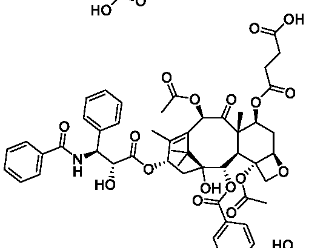
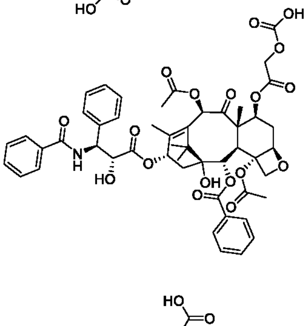
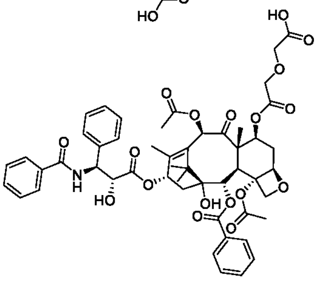
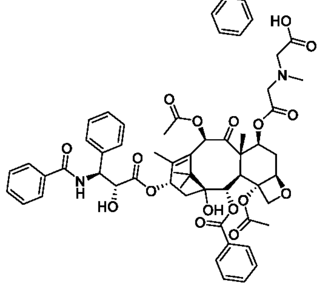
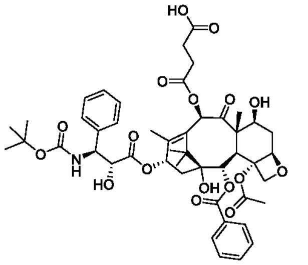
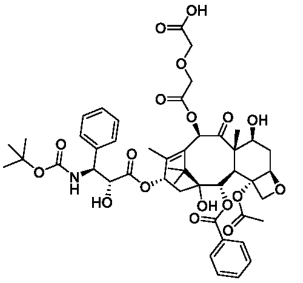
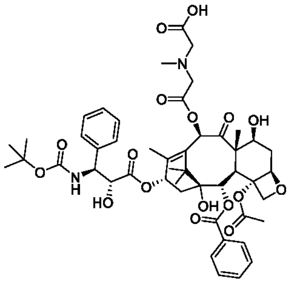

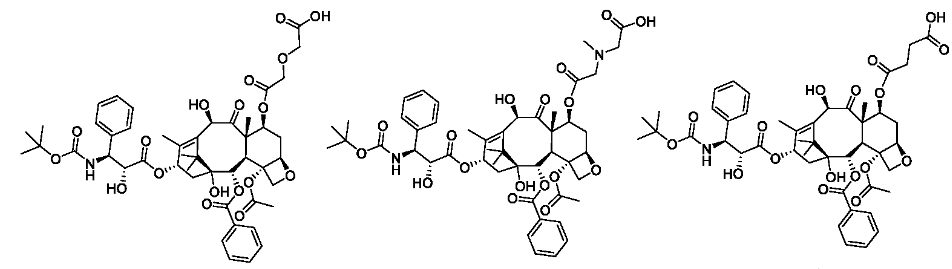

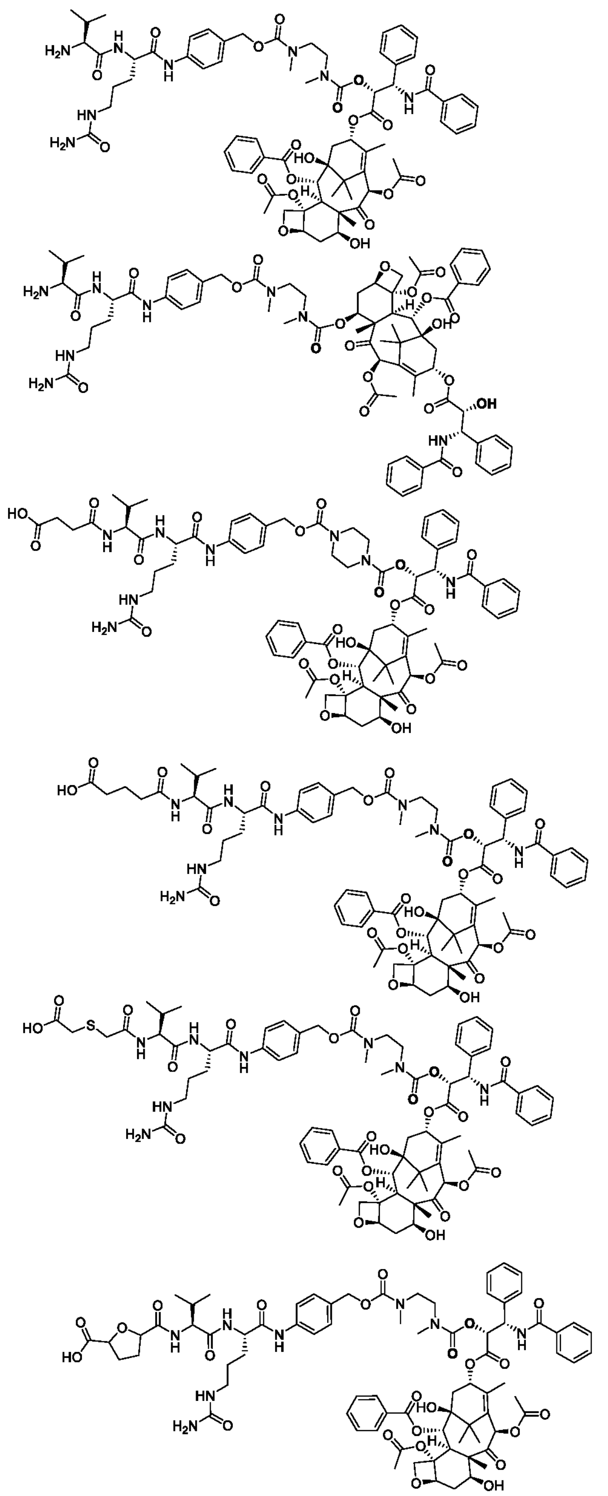

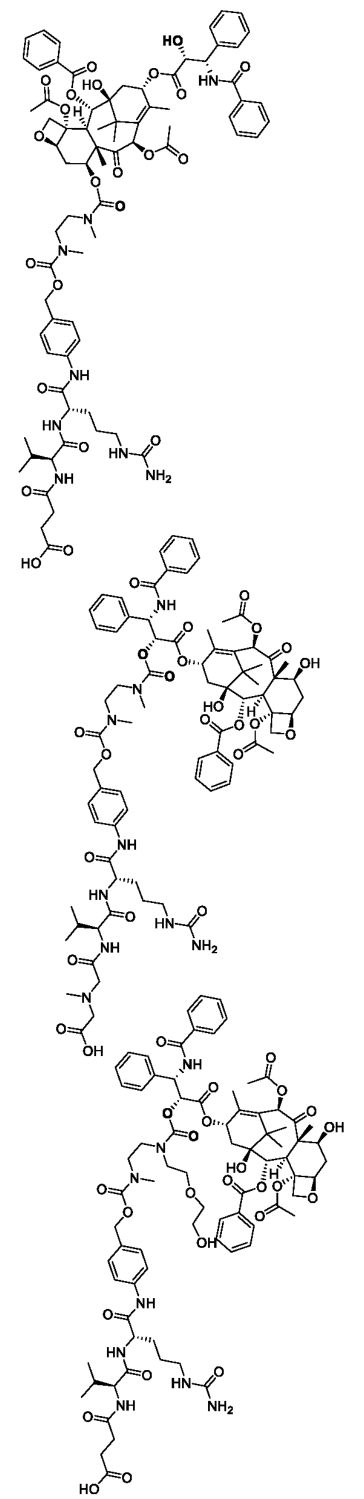

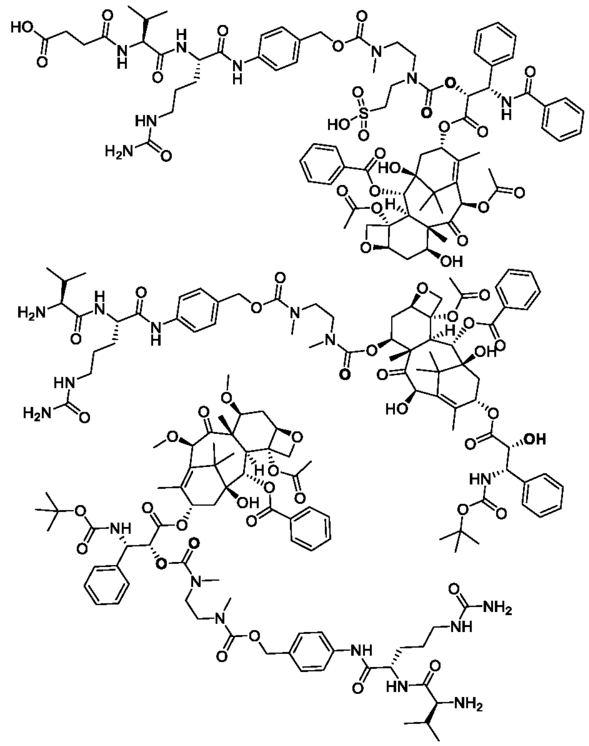

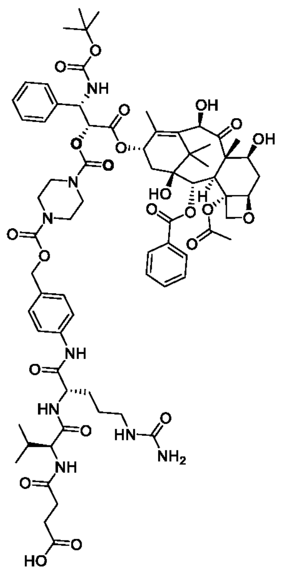

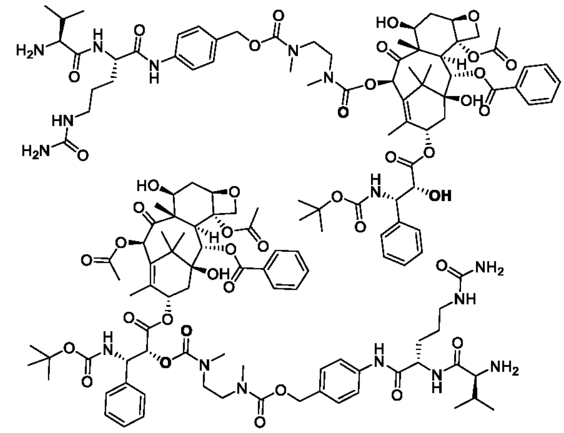

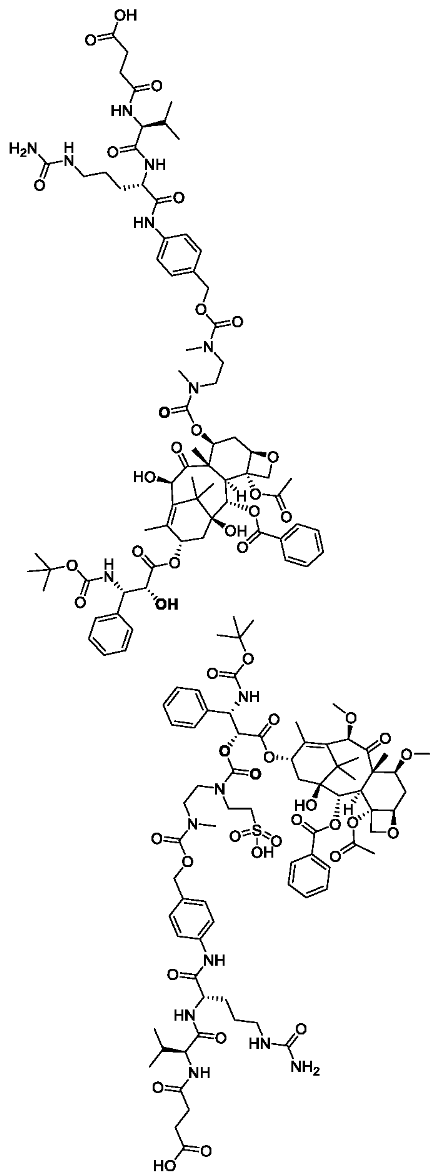

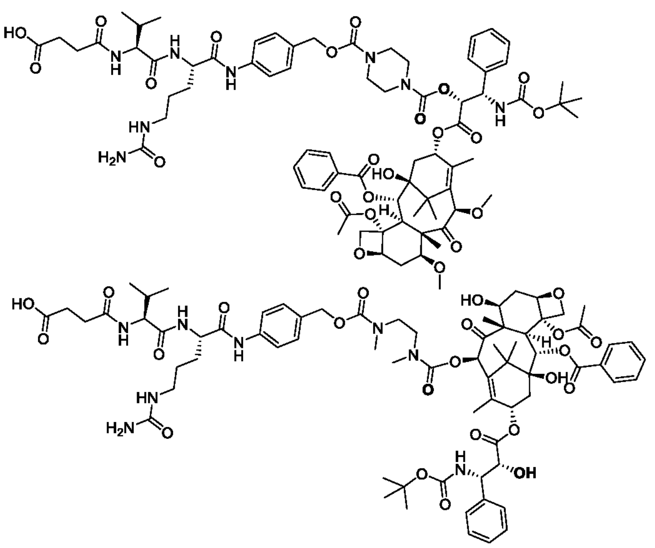

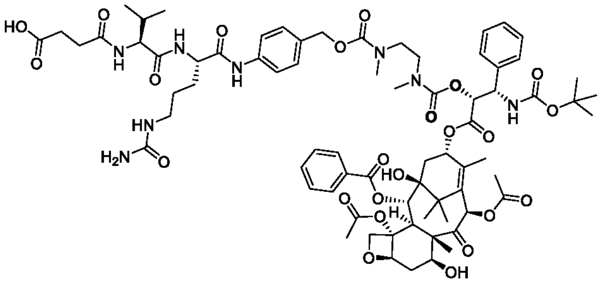

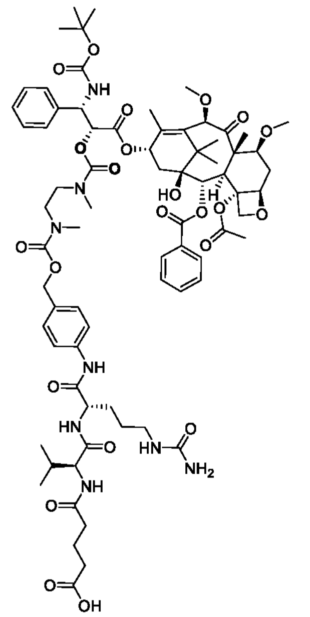

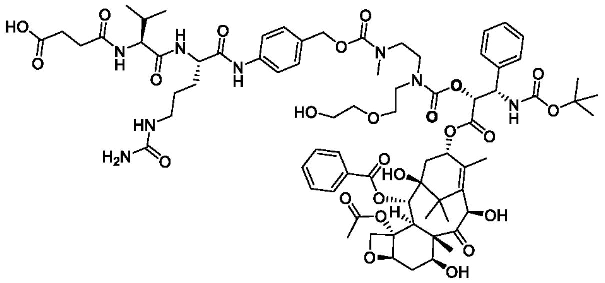

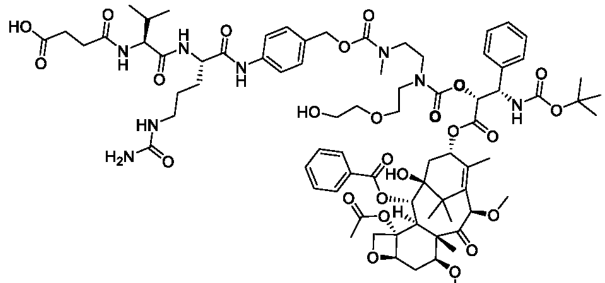

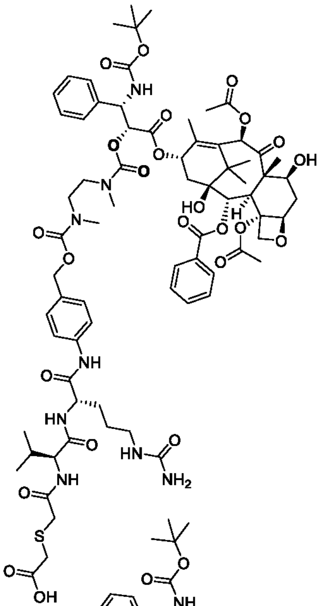

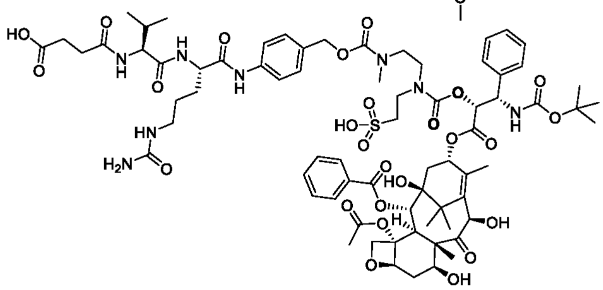

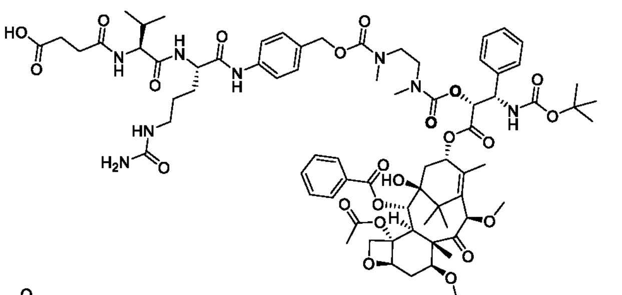

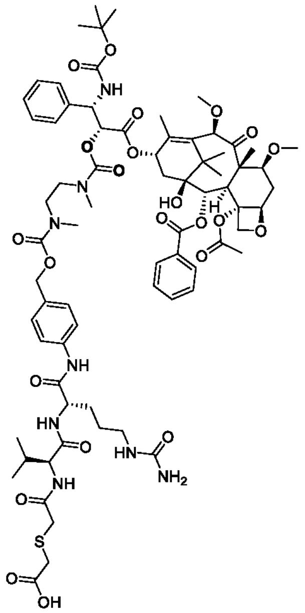

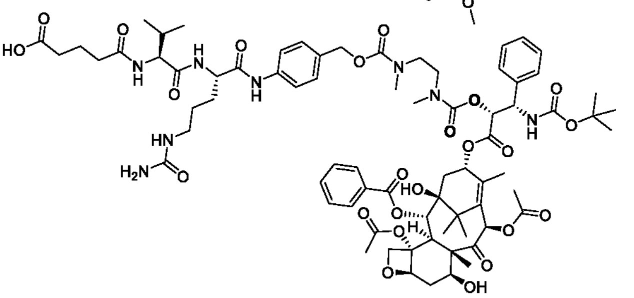

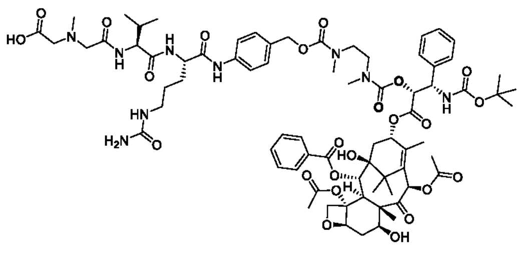

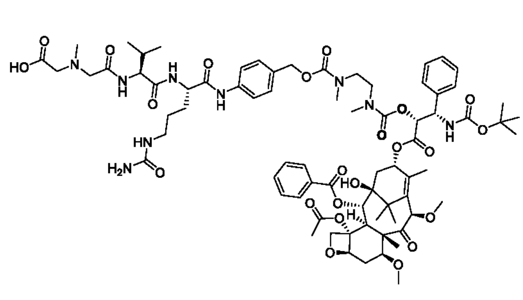

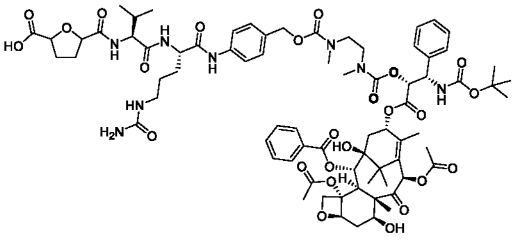

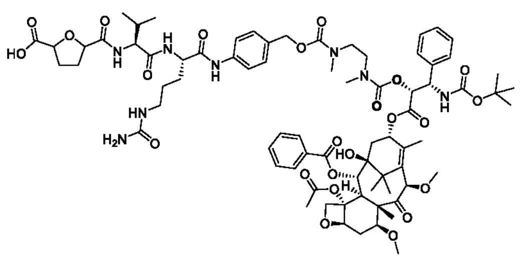

The present invention also provides compounds as shown in formulae VI-XV or salts thereof,

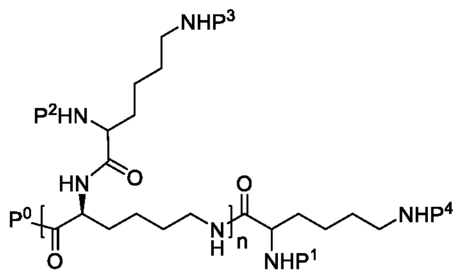

VI

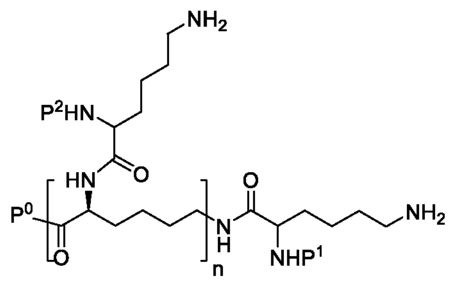

VII

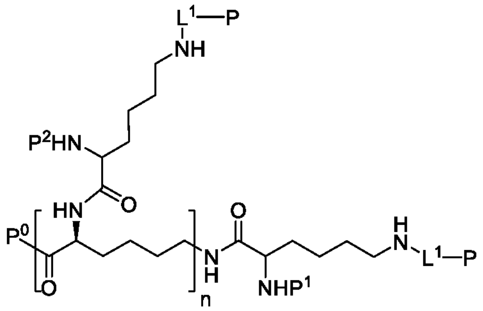

VIII

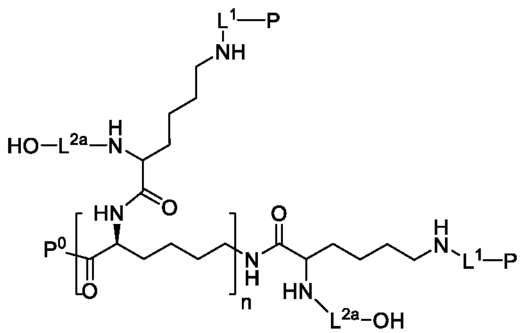

IX

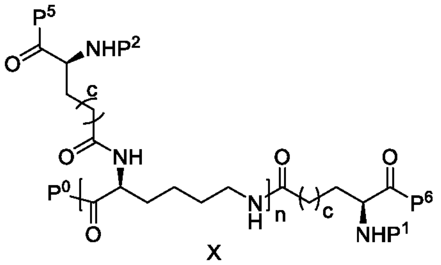

X

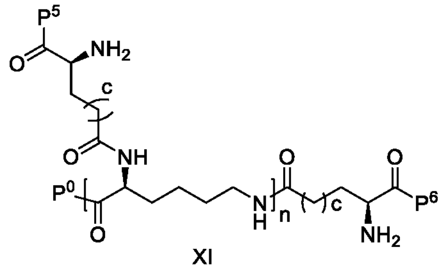

XI

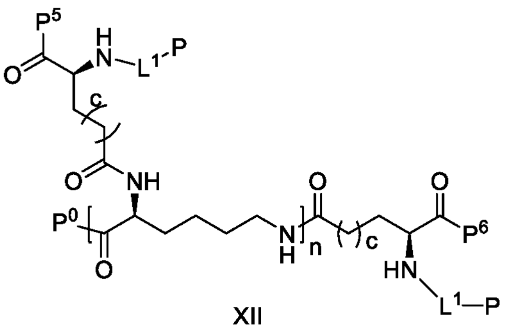

XII

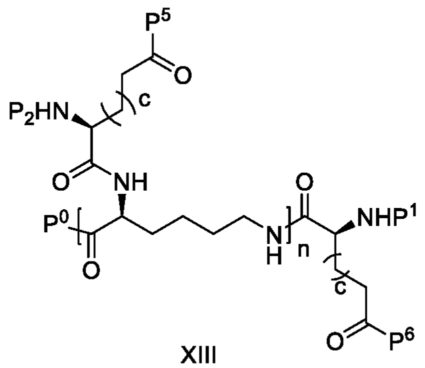

XIII

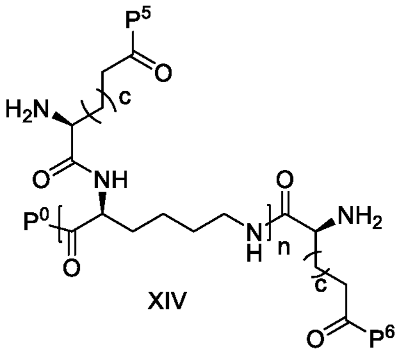

XIV

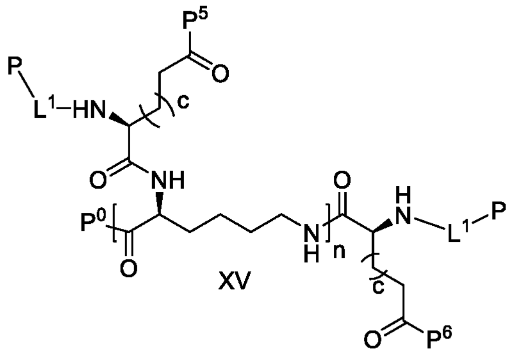

XV

where

$P^0$, $P^5$, and $P^6$ are each independently selected from OH or carboxyl protecting group; when $P^0$, $P^5$, and $P^6$ are carboxyl protection groups, they can be the same or different;

$P^1$, $P^2$, $P^3$ and $P^4$ are each independently selected from H or amine protecting group; when $P^1$, $P^2$, $P^3$ and $P^4$ are amine protecting groups, they can be the same or different.

In some specific examples, P is a methyl-terminated polyethylene glycol derivative residue, the number-average molecular weight of which is 500-5000.

In some specific examples, $L^1$ is selected from a covalent bond, C(O), or C(O)-O.

In some specific examples, $L^{2a}$ is C(O)-E-C(O), and the E is selected from a covalent bond, $CH_2$, $CH_2$-$CH_2$, $CH_2$-$CH_2$-$CH_2$, $CH_2$-O-$CH_2$, $CH_2$-S-$CH_2$, $CH_2$-NH-$CH_2$, $CH_2$-N(Me)-$CH_2$, or CH=CH.

In some specific examples, n is an integer of 10 to 70.

In some specific examples, the compounds shown in formulae VI-XV are selected from any of the following compounds:

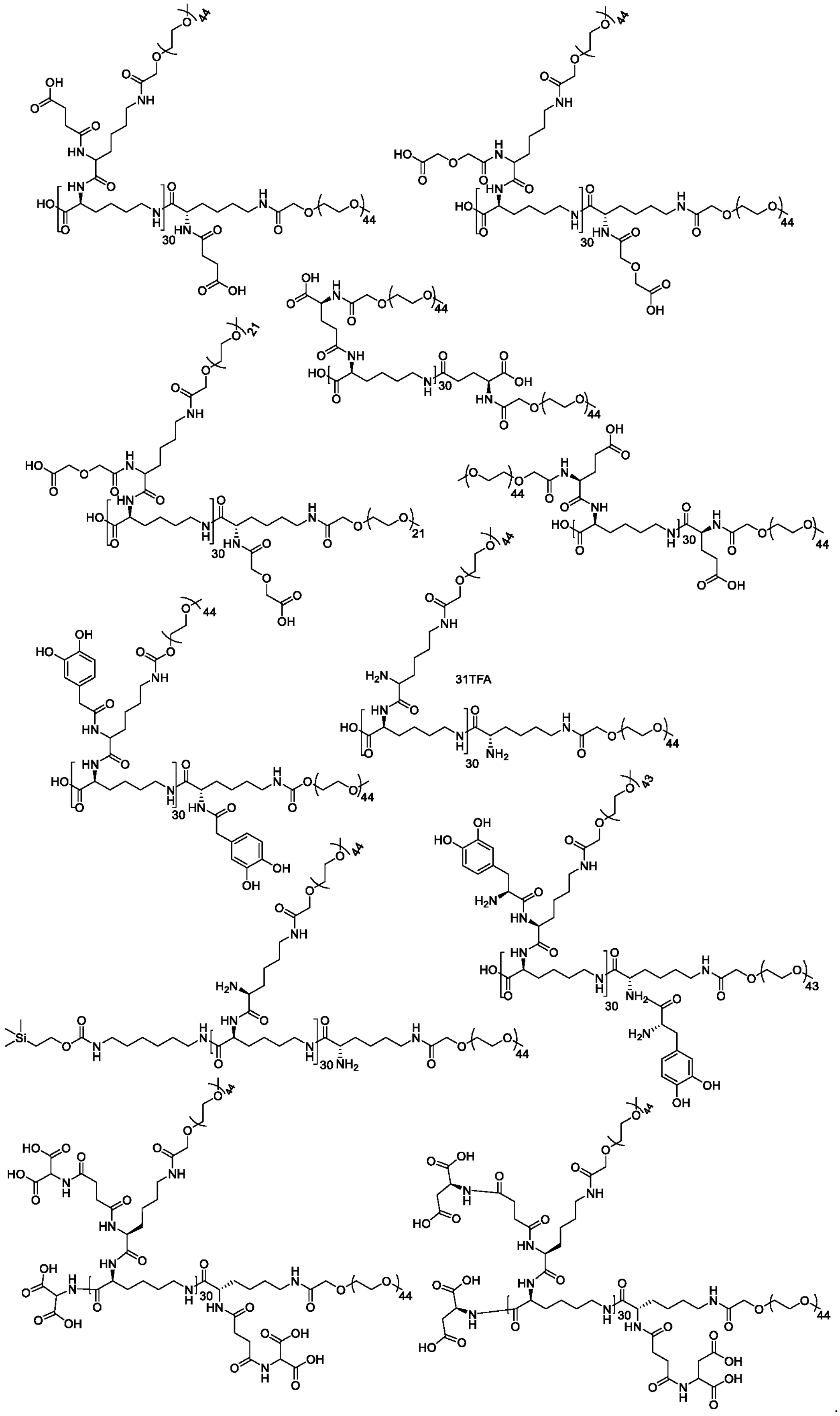

31TFA

In some embodiments, the trifunctional branched center Y and the poly(ε-L-lysine) residue are linked by the linker $L^0$, and the molar ratio of the trifunctional branched center Y to the poly(ε-L-lysine) structural unit is 0.5:1~1.5:1.

In some embodiments, the trifunctional branched center Y and the poly(ε-L-lysine) residue are linked by the linker $L^0$, and the molar ratio of the trifunctional branched center Y to the poly(ε-L-lysine) structural unit is 0.8:1~1.2:1.

In some embodiments, the pharmacokinetic regulator residue and the trifunctional branched center Y are linked by the linker $L^1$, and the molar ratio of the pharmacokinetic regulator residue to the poly(ε-L-lysine) structural unit is 0.5:1~1.5:1.

In some embodiments, the pharmacokinetic regulator residue and the trifunctional branched center Y are linked by the linker $L^1$, and the molar ratio of the pharmacokinetic regulator residue to the poly(ε-L-lysine) structural unit is 0.8:1~1.2:1.

In some embodiments, the pharmaceutically active agent residue D and the trifunctional branched center Y are linked by the linker $L^2$, and the molar ratio of the pharmaceutically active agent residue D to the poly(ε-L-lysine) structural unit is 0.5:1~1.5:1.

In some embodiments, the pharmaceutically active agent residue D and the trifunctional branched center Y are linked by the linker $L^2$, and the molar ratio of the pharmaceutically active agent residue D to the poly(ε-L-lysine) structural unit is 0.8:1~1.2:1.

The poly(ε-L-lysine) derivative-drug conjugate of the present invention is suitable for the prevention and treatment of tumors, inflammation, diabetes, central nervous system diseases, cardiovascular diseases, psychiatric diseases, respiratory diseases, ophthalmic diseases, pain, obesity and other diseases.

In some embodiments, the poly(ε-L-lysine) derivative-drug conjugate is used for the prevention and treatment of cancer, where the cancer includes breast cancer, ovarian cancer, prostate cancer, melanin cancer, brain cancer, nasopharyngeal cancer, esophageal cancer, stomach cancer, liver cancer, pancreatic cancer, colorectal cancer, lung cancer, renal cancer, skin cancer, glioblastoma, neuroblastoma, sarcoma, liposarcoma, osteochondroma, bone cancer, osteosarcoma, seminoma, testicular tumor, uterine tumor, head and neck tumor, multiple myeloma, malignant lymphoma, polycythemia vera, leukemia, thyroid tumor, ureteral tumor, bladder tumor, gallbladder cancer, cholangiocarcinoma, chorionic epithelioma or pediatric tumor.

In some embodiments, the average release rate of the poly(ε-L-lysine) derivative-drug conjugate 101B00A ($PEG_{1K}$) DOX of the present invention is 11.6 times the release rate of dendrimer-drug conjugate 101A1601 ($PEG_{1K}$) (time point: 4 h); the average release rate of poly(ε-L-lysine) derivative-drug conjugate 101B00B ($PEG_{2K}$) DOX of the present invention is 1.5 times the release rate of dendritic polymer 101A1602 ($PEG_{2K}$) (time point: 4 h). The results show that compared with the polylysine dendrimer-drug conjugate in the prior art, the drug conjugate with linear poly(ε-L-lysine) as a backbone according to the present invention has lower steric hindrance for surface functional groups, higher accessibility to enzymes, faster drug release rate and higher controllability. This is more conducive to quickly reaching effective therapeutic concentrations in the early stage of tumor treatment and effectively inhibiting tumor progression.

In some embodiments, the average release rate of the poly(ε-L-lysine) derivative-drug conjugate 101B00E SN-38 of the present invention is 5.6 times the release rate of dendrimer-drug conjugate 101A1603. This further indicates that the poly(ε-L-lysine) derivative-drug conjugate of the present invention has lower steric hindrance, which is more conducive to the effective treatment of tumors.

In some embodiments, the tumor/plasma ratio of released drug from the poly(ε-L-lysine) derivative-drug conjugate 101B00E of the present invention reaches the highest value of 21.2±6.5 at 24 h. These results show that the poly(ε-L-lysine) derivative-drug conjugate of the present invention can be highly enriched at the tumor site. The tumor/plasma ratio of released drug fomr irinotecan reaches the highest value of 4.4±1.2 at 8 h. Compared with the traditional chemotherapy drug irinotecan, the poly(ε-L-lysine) derivative-drug conjugate 101B00E of the present invention has better tissue-specific distribution and has a significant function of targeted delivery to the tumor site.

In some embodiments, for a HepG2 liver cancer CDX model, the poly(ε-L-lysine) derivative-drug conjugate 101B00A of the present invention has significantly better tumor inhibition rate than dendrimer-DOX conjugate 101A1601 and doxorubicin hydrochloride, while compared with doxorubicin hydrochloride, dendrimer-DOX conjugate 101A1601 has no statistical difference in tumor inhibition rate. These results show that compared with dendrimer-DOX conjugate 101A1601, which has a slower enzymatic release rate, the poly(ε-L-lysine) derivative-drug conjugate 101B00A has a faster enzymatic release rate and has better tumor inhibition effect on the HepG2 liver cancer CDX model.

In some embodiments, for the HepG2 liver cancer CDX model, on day 33, the poly(ε-L-lysine) derivative-SN-38 conjugates 101B00C and 101B00E of the present invention have tumor inhibition rates of 93% and 92%, respectively, while irinotecan as the positive drug group has a tumor inhibition rate of -49%. These results show that the poly(ε-L-lysine) derivative-SN-38 conjugates 101B00C and 101B00E both have excellent tumor inhibition effects.

In some embodiments, for the HepG2 liver cancer CDX model, on day 33, the poly(ε-L-lysine) derivative-SN-38 conjugates 101C00A and 101D00A of the present invention also show excellent tumor inhibition effects and have tumor inhibition rates of 90% and 91%, respectively, while irinotecan as the positive drug group has a tumor inhibition

rate of -49%. These results show that the poly(ε-L-lysine) derivative-drug conjugates 101C00A and 101D00A have significantly better tumor inhibition effects than the positive drug irinotecan.

In some embodiments, for a subcutaneous xenograft model of BxPC-3 pancreatic cancer, 101B00G 5 mg/kg (SN-38) can achieve the efficacy of nab-paclitaxel 60 mg/kg (MTD). Further increase of the amount of 101B00G to 20 mg/kg could further enhance the inhibition effect on BxPC-3 pancreatic cancer. Even 20 mg/kg 101B00G has less influence on mouse body weight than 60 mg/kg nab-paclitaxel. These results indicate that 101B00G has significantly better effect on BxPC-3 pancreatic cancer than the clinical first-line treatment nab-paclitaxel, thereby exhibiting its potential prospects in clinical application.

In some embodiments, for administration of 101B00G 15 mg/kg (SN-38) via rat tail vein, only within 0.083~2 h at the beginning of administration, the proportion of SN-38 released in plasma to total SN-38 is relatively high to be 0.16-0.03%. 4 h ~ 168 h after administration (0 h of day 22 is equivalent to 168 h after the third dose), the released SN-38 accounts for 0.012-0.015% of the total SN-38. These results show that 101B00G is extremely stable in rat plasma and does not release SN-38 non-specifically in plasma. Moreover, the half-life of total SN-38 is long, and the distribution half-life is up to 4 days, indicating that 101B00G has long circulation in plasma.

In some embodiments, for administration of topotecan at the MTD dose of 2 mg/kg (based as free base) in the literature once a day for five days, the inhibition effect for 1200 $mm^3$ small cell lung cancer is poor, and the tumor volume on day 22 is increased by 22% compared with the initial value. However, after a single dose of 101B00G, the tumor volume continues to decrease after a single dose, and the tumor volume on day 22 is decreased by 81% compared with the initial value. These results show that 101B00G is significantly better for large tumors of 1200 $mm^3$ than topotecan, the existing second-line clinical treatment for small cell lung cancer.

In some embodiments, for a subcutaneous large tumor model of HepG2 liver cancer of 600 $mm^3$, the tumor volume continues to decrease after 101B00G administration, indicating that 101B00G has potential prospects in clinical application.

In some embodiments, for the subcutaneous xenograft model of BxPC-3 pancreatic cancer, 21 days after administration, the nab-paclitaxel group (60 mg/kg) has a significant difference from the control group (T/C = 30%, p = 0.0007), and 101T00A-O has a significant difference from the control group (T/C = 7%, p = 0.00006). 101T00A-O has significantly better efficacy than the nab-paclitaxel group (60 mg/kg), thereby exhibiting potential prospects in clinical application.

Compared with the prior art, the drug conjugate with linear poly(ε-L-lysine) derivative as a backbone according to the present invention has the following advantages:

Compared with the PEG-drug conjugate, the polymer-drug conjugate provided by the present invention has longer half-life in vivo. When using poly(ε-L-lysine) with a polymerization degree of 25 to 35 as a skeleton, 25 to 35 PEG residues can be attached to the surface of the conjugate. The polymer-drug conjugate has lower renal clearance, lower hepatospleen clearance and longer plasma half-life.

Compared with the existing randomly grafted copolymer which has the problems of drug over-conjugation on a single polymer chain and uneven distribution of drug conjugation positions, the polymer-drug conjugate provided by the present invention has a more controllable ratio of pharmacokinetic regulation segments to drug conjugation positions. The polymer-drug conjugate provided by the present invention can achieve precise control of ratio and uniformity of drugs and pharmacokinetic regulation segments, and can better regulate and balance the relationship between drug clearance, drug retention and drug release, so as to achieve the best therapeutic effect.

Compared with the existing dendrimer-drug conjugate, the polymer-drug conjugate provided by the present invention has less steric hindrance on functional groups of the outermost layer, higher accessibility of enzymes to linkers, faster drug release rate, and accumulation of effective drug concentration in tumor within a short time. Compared with the existing dendrimer-drug conjugate, for the HepG2 liver cancer CDX tumor model, the polymer-drug conjugate provided by the present invention has better inhibition effect on the tumor.

Compared with the existing dendrimer-drug conjugate, the synthesis of the polymer-drug conjugate provided by the present invention is simpler. A grafted polymer with an average of 60 orthogonally protected functional groups can be obtained through only one step of conjugation. Further appropriate modification of the polymer could realize exceeding of the renal clearance threshold and achieve the characteristics of long circulation in the body. In the synthesis process, conjugate fragment intermediates can reach a high modification degree of >95% without excessive feeding. The process control is easier, the production process is shorter and suitable for industrial scale-up and production, with good prospects in industrial application. Moreover, the starting material poly(ε-L-lysine) can be produced on a large scale by microbial fermentation, and can be obtained in large quantities through a mature production process. In addition, the structure of the starting material poly(ε-L-lysine) is clear, and it is easy to establish quality test and quality control methods. As for the existing polylysine dendrimer, 11 steps are required for the synthesis of polylysine dendrimer macromolecules with 32 lysine residues on the surface. Reaction reagents and intermediates are required in large excess, with large solvent consumption, long synthetic cycle and high cost. It is also difficult to establish quality control standards.

Compared with the existing randomly grafted polymer conjugate, the drug conjugate with poly(ε-L-lysine) as a skeleton according to the present invention has a controllable number of conjugates and a controllable position of conjugation, and is suitable for the conjugation of drugs for various diseases, such as targeted drugs, immunosuppressants, immune agonists, immunomodulators, targeted protein degraders, chemotherapy drugs, small molecule drugs with responsive functional groups, polypeptide drugs, oligosaccharides, oligonucleotides, lipid drugs, vitamins, enzyme drugs, protein drugs, antibody drugs and so on.

Unless otherwise stated to the contrary, the terms used in the present invention have the following definitions: The term "number n of repeating units" can also be expressed as the degree of polymerization DP. Unless otherwise specified, the number n of repeating units in the present invention represents the number-average degree of polymerization, that is, the average number of repeating units contained on the polymer chain. When the poly(ε-L-lysine) is prepared by solid phase synthesis, the number n of repeating units is a single value. When the poly(ε-L-lysine) is synthesized by biological fermentation or other chemical polymerization methods, the number n of repeating units has a certain distribution, and its value is expressed as average number.

The amine protecting group, hydroxyl protecting group and carboxyl protecting group of the present invention are suitable groups for amine protection, hydroxyl protection and carboxyl protection, which are known in this field. For various protective groups, reference could be made to the literature ("Protective Groups in Organic Synthesis", 5Th Ed. T. W. Greene & P. G. M. Wuts).

The term **"alkyl"** refers to a saturated aliphatic hydrocarbon group, including a linear or branched group of 1 to 30 carbon atoms, preferably alkyl with 1 to 10 carbon atoms, preferably 1 to 8 carbon atoms. Non-limiting examples include, but are not limited to: methyl, ethyl, n-propyl, n-butyl, isobutyl, tert-butyl, sec-butyl, n-pentyl, 2-methylbutyl, 3-methylbutyl, 1,1-dimethylpropyl, 1,2-dimethylpropyl, 2,2-dimethylpropyl, 1-ethylpropyl, n-hexyl, 2-methylpentyl, 3-methylpentyl, 4-methylpentyl, 1,1-dimethylbutyl, 1,2-dimethylbutyl, 2,2-dimethylbutyl, 1,3-dimethylbutyl, 2,3-dimethylbutyl, 3,3-dimethylbutyl, 1,1,2-trimethylpropyl, 1-ethyl-2-methylpropyl, n-heptyl, 2-methylhexyl, 3-methylhexyl, 4-methylhexyl, 5-methylhexyl, 2,2-dimethylpentyl, 2,3-dimethylpentyl, 2,4-dimethylpentyl, 3,3-dimethylpentyl, 3,4-dimethylpentyl, 2-ethylpentyl, 3-ethylpentyl, 3-ethylpentyl, octyl, nonyl, decyl, undecyl, dodecyl and various isomers thereof.

The term **"cycloalkyl"** refers to a saturated or partially unsaturated monocyclic or polycyclic substituent, including 3 to 20 carbon atoms, preferably 3 to 12 carbon atoms, more preferably 3 to 10 carbon atoms, most preferably 3 to 6 carbon atoms. Non-limiting examples of monocycloalkyl include, but are not limited to: cyclopropyl, cyclobutyl, cyclopentyl, cyclopentenyl, cyclohexyl, cyclohexenyl, cyclohexadienyl, cycloheptyl, cyclooctyl, etc. Non-limiting examples of polycycloalkyl include, but are not limited to, cycloalkyl containing spiro rings, fused rings and bridged rings.

The term **"alkenyl"** refers to an alkyl group as defined in the present invention, composed of at least two carbon atoms and at least one carbon-carbon double bond, preferably $C_2$~$C_{10}$ alkenyl, more preferably $C_2$~$C_6$ alkenyl, such as ethenyl, propenyl, 1-propenyl, etc.

The term **"alkynyl"** refers to an alkyl group as defined in the present invention, composed of at least two carbon atoms and at least one carbon-carbon triple bond, preferably $C_2$~$C_{10}$ alkynyl, more preferably $C_2$~$C_6$ alkynyl, such as ethynyl, 1-propynyl, 2-propynyl, etc.

The term **"heterocycloalkyl"** refers to a saturated or partially unsaturated monocyclic or polycyclic hydrocarbon substituent, including 3 to 20 ring atoms, of which one or more ring atoms are heteroatoms selected from N, O, Si, B, $S(O)_m$ and $P(O)_m$ (where m is an integer of 0 to 2), excluding a ring moiety of -O-O, -O-S- or -S-S-, and the remaining ring atoms are carbon. Preferably included are 3 to 12 ring atoms which contain 1 to 4 heteroatoms. Non-limiting examples of monocyclic heterocycloalkyl include pyrrolyl, piperidinyl, piperazinyl, morpholinyl, tetrahydrofuranyl, pyranyl, etc. Polycyclic heterocycloalkyl includes heterocycloalkyl of spiro rings, fused rings and bridged rings.

The term **"alkoxy"** refers to -O-(alkyl) and -O-(cycloalkyl), where alkyl and cycloalkyl are defined as described above. Non-limiting examples include, but are not limited to, methoxy, ethoxy, propoxy, butoxy, cyclopropoxy, cyclobutoxy, cyclopentyloxy, cyclohexyloxy, etc.

The term **"alkylthio"** refers to -S-(alkyl) and -S-(cycloalkyl), where alkyl and cycloalkyl are defined as described above. Non-limiting examples include, but are not limited to, methylthio, ethylthio, propylthio, butylthio, cyclopropylthio, cyclobutylthio, cyclopentylthio, cyclohexylthio, etc.

The term "**substituted or unsubstituted amino**" refers to $NH_2$, monosubstituted $NH_2$ and disubstituted $NH_2$. When substituted, the monosubstituents or disubstituents are preferably independently selected from deuterium, alkyl, hydroxyl, sulfhydryl, alkenyl, alkynyl, alkoxy, alkylthio, alkylamino, cycloalkyl, heterocycloalkyl, aryl, heteroaryl, cycloalkoxy, heterocycloalkoxy, cycloalkylthio, heterocycloalkylthio, oxo, amino, haloalkyl, hydroxyalkyl, carboxyl or carboxylate, etc. The disubstituents together with nitrogen atoms linked thereto can form non-aromatic heterocyclic structures.

The term "**aryl**" refers to any stable conjugated cyclic hydrocarbon system of 6 to 18 carbon atoms, preferably 6 to 10 carbon atoms, which may be a monocyclic, bicyclic or tricyclic aromatic group or an aromatic group with more rings, such as phenyl, naphthyl, anthracenyl, etc. The aryl group may include an aryl group in which an aromatic ring is fused with a heterocycloalkyl group or a cycloalkyl group.

The term "**heteroaryl**" refers to an aromatic ring system in which at least one carbon atom on the ring is replaced with a heteroatom selected from N, O or S, preferably a 5 to 7-membered monocyclic structure or a 7 to 12-membered bicyclic structure, more preferably 5 to 6-membered heteroaryl, such as pyrrolyl, imidazolyl, pyridyl, pyrimidinyl, thiazolyl, thienyl, pyrazinyl, triazolyl, tetrazolyl, oxazolyl, indazolyl, etc. The heteroaryl may include heteroaryl in which a heteroaryl ring is fused with a heteroaryl, heterocycloalkyl or cycloalkyl ring.

The term **"sulfone"** refers to

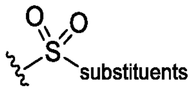

,

where the substituent is preferably alkyl, alkenyl, alkynyl, amino, alkoxy, alkylthio, alkylamino, cycloalkyl, haloalkyl, heterocycloalkyl, aryl, heteroaryl, cycloalkoxy, heterocycloalkoxy, cycloalkylthio or heterocycloalkylthio.

The term **"sulfoxide"** refers to

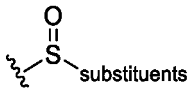

,

where the substituent is preferably alkyl, alkenyl, alkynyl, amino, alkoxy, alkylthio, alkylamino, cycloalkyl, haloalkyl, heterocycloalkyl, aryl, heteroaryl, cycloalkoxy, heterocycloalkoxy, cycloalkylthio or heterocycloalkylthio.

The term **"alkyl sulfoxide"** refers to

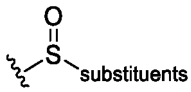

,

where the substituent is preferably alkyl as defined above.

The term **"hydroxyl"** refers to -OH.

The term **"halogen"** refers to fluorine, chlorine, bromine or iodine.

The term **"nitro"** refers to $-NO_2$.

The term **"amino"** refers to $-NH_2$.

The term **"cyano"** refers to -CN.

The term **"carboxylic acid"** refers to -C(O)OH.

The term **"sulfhydryl"** refers to -SH.

The term **"carboxylate"** refers to -C(O)O-alkyl, aryl or cycloalkyl, where alkyl, aryl and cycloalkyl are defined as described above.

The term **"thioester"** refers to -C(O)S-alkyl, aryl or cycloalkyl, where alkyl, aryl and cycloalkyl are defined as described above.

The term **"sulfate"** refers to $O-S(O)_2O$-alkyl, aryl or cycloalkyl, where alkyl, aryl and cycloalkyl are defined as described above.

The term **"sulfonate"** refers to $S(O)_2O$-alkyl, aryl or cycloalkyl, where alkyl, aryl and cycloalkyl are defined as described above.

The term **"boronic acid"** refers to $B(OH)_2$.

The term **"borate"** refers to $B(OR)_2$, R = alkyl, aryl or cycloalkyl, where alkyl, aryl and cycloalkyl are defined as described above. **"Substituted"** means that one or more hydrogen atoms in a group are independently replaced by a corresponding number of deuterium or substituents.

**"Pharmaceutically acceptable salt"** refers to a salt that can retain the biological effectiveness of free base without other toxic side effects. It can be an acidic group, a basic group or an amphoteric group. Non-limiting examples include but are not limited to: acidic salts, including hydrochloride, hydrobromide, sulfate, pyrosulfate, bisulfate, sulfite, bisulfite, phosphate, monodihydric phosphate, dihydric phosphate, metaphosphate, pyrophosphate, nitrate, acetate, propionate, decanoate, octanoate, formate, acrylate, isobutyrate, hexanoate, heptanoate, oxalate, malonate, succinate, suberate, benzoate, methylbenzoate, phthalate, maleate, methanesulfonate, p-toluenesulfonate, benzenesulfonate, (*D, L*)-tartaric acid, citrate, maleate, (*D, L*)-malate, fumarate, stearate, oleate, cinnamate, laurate, glutamate, aspartate, triflate, mandelate, ascorbate, salicylate, etc. When the compound of the present invention contains the acidic group,

its pharmaceutically acceptable salts may also include: alkali metal salts (such as sodium salt or potassium salt), alkaline earth metal salts (such as calcium salt or magnesium salt) and organic alkali salts (such as alkyl aryl amines, amino acids, etc.).

**"Pharmaceutical composition"** refers to a mixture containing one or more compounds described in the present invention, or their physiologically acceptable salts or prodrugs thereof, and other chemical components, together with other ingredients such as physiologically acceptable carriers and excipients. The purpose of the pharmaceutical composition is to facilitate administration to organisms and facilitate the absorption of active ingredients to exert biological activity.

Unless otherwise stated, the abbreviations of any protective groups, amino acids and other compounds used in the present invention are based on their commonly used and recognized abbreviations, or with reference to the IUPAC-IUBC Commission on Biochemical Nomenclature (see Biochem. 1972, 11, 942-944).

BRIEF DESCRIPTION OF THE DRAWINGS

Figure 1 shows a comparison of enzymatic drug release rates of 101A1601 and 101B00A in biological assessment example A.
Figure 2 shows a comparison of enzymatic drug release rates of 101A1602 and 101B00B in biological assessment example B.
Figure 3 shows a comparison of enzymatic drug release rates of 101B00E and 101A1603 in biological assessment example C.
Figure 4 shows a comparison of enzymatic drug release rates of 101C00A and 101D00A in biological assessment example D.
Figure 5 shows the SN-38 distribution in plasma/tumor after intravenous injection of irinotecan (10 mg/kg, based on SN-38) in mouse HepG2 liver cancer CDX model in biological assessment example E.
Figure 6 shows the piperazine-10-O-SN-38 (10112) distribution in plasma/tumor after intravenous injection of 101B00E (10 mg/kg, based on SN-38) in mouse HepG2 liver cancer CDX model in biological assessment example E.
Figure 7 shows the SN-38 ratio in tumor/plasma after intravenous injection of 101B00E and irinotecan (10 mg/kg, based on SN-38) in mouse HepG2 liver cancer CDX model in biological assessment example E (101B00E is measured on the basis of SN-38 converted from the released piperazine-10-O-SN-38 (10112)).
Figure 8 shows the inhibition of 101A1601 and 101B00A in HepG2 liver cancer CDX model in biological assessment example F.
Figure 9 shows the inhibition of 101B00C and 101B00E in HepG2 liver cancer CDX model in biological assessment example F.
Figure 10 shows the inhibition of 101C00A and 101D00A in HepG2 liver cancer CDX model in biological assessment example F.
Figure 11 shows a comparison of efficacy of 101B00G and nab-paclitaxel in subcutaneous xenograft model of pancreatic cancer in biological assessment example G.
Figure 12 shows the influence of 101B00G and nab-paclitaxel in subcutaneous xenograft model of pancreatic cancer on body weight of HepG2 liver cancer CDX model in biological assessment example G.
Figure 13 is a plasma concentration-time curve for total SN-38 and free SN-38 in rat plasma after the first injection of 101B00G in biological assessment example H.
Figure 14 is a plasma concentration-time curve for total SN-38 and free SN-38 in rat plasma after the fourth injection of 101B00G in biological assessment example H.
Figure 15 shows a comparison of efficacy of 101B00G and topotecan in subcutaneous xenograft model of H69 small cell lung cancer of 1200 $mm^3$ in biological assessment example I.
Figure 16 shows the efficacy of 101B00G in subcutaneous xenograft model of HepG2 liver cancer of 600 $mm^3$ in biological assessment example J.
Figure 17 shows a comparison of efficacy of 101T00A-O and nab-paclitaxel in subcutaneous xenograft model of pancreatic cancer in biological assessment example K.

The downward arrows in the figure indicate the time of dosing.

DETAILED DESCRIPTION OF THE PREFERRED EMBODIMENT

The present invention is further described below by means of examples, but the present invention is not limited to the scope of the described examples. Experimental methods that are not described in details in the following examples

should follow conventional methods and conditions, or product instructions.

The abbreviations used in the present invention are shown in the following table:

| Abbreviation | Full name |
|---|---|
| Cit, C | citrulline |
| Phe | phenylalanine |
| Lys | lysine |
| Arg | arginine |
| Val, V | valine |
| Ala | alanine |
| Ile | isoleucine |
| Trp | tryptophan |
| Asn | asparagine |
| Pro | proline |
| Nle | norleucine |
| Glu | glutamic acid |
| Gly | glycine |
| Leu | leucine |
| Asp | aspartic acid |
| BODIPY | 4,4-difluoro-4-bora-3a,4a-diaza-s-indacene |
| MMAE | methyl auristatin E |
| MMAF | methyl auristatin F |
| PBD | pyrrolobenzodiazepine |
| PEG | polyethylene glycol |
| mPEG | methoxypolyethylene glycol |
| mPEG-1K | methoxypolyethylene glycol (number average molecular weight = 1k) |
| mPEG-2K | methoxypolyethylene glycol (number average molecular weight = 2k) |
| DOX | doxorubicin |
| PAB | para-aminobenzyl alcohol |
| DMEDA | N,N'-dimethyl ethyl enediamine |
| SN-38 | 7-ethyl-10-hydroxycamptothecin |
| PTX | paclitaxel |
| CDX | xenograft model of cell line tumor |
| MTBE | methyl tert-butyl ether |
| DMF | N,N'-dimethylformamide |
| DCM | methylene chloride |
| DIPEA | N,N-diisopropylethylamine |
| DMSO | dimethyl sulfoxide |
| THF | tetrahydrofuran |
| EA | ethyl acetate |
| TFA | trifluoroacetate |

(continued)

| Abbreviation | Full name |
|---|---|
| DEA | diethylamine |
| DMAP | 4-dimethylaminopyridine |
| Boc | tert-butoxycarb onyl |
| Cbz | carbobenzoxyl |
| DCC | dicyclohexylcarbodiimide |
| NHS | N-hydroxysuccinimide |
| EDCI | 1-(3-dimethylaminopropyl)-3-ethylcarbodiimide hydrochloride |
| PyBop | benzotriazol-1-yl-oxytripyrrolidinylphosphonium hexafluorophosphate |
| TEMPO | tetramethylpiperidine oxide |
| EEDQ | 2-ethoxy-1 -ethoxycarbonyl-1,2-dihydroquinoline |
| Teoc | 2-(trimethylsilyl)ethoxycarbonyl |
| EtOAc | ethyl acetate |
| pNB | p-nitrophenol |
| DME | ethylene glycol dimethyl ether |
| Fmoc | fluorenylmethoxycarbonyl |
| DIPEA | N,N-diisopropylethylamine |
| TBSCl | tert-butyldimethylsilyl chloride |
| BHA | diphenylamine |
| ESI | electrospray ionization |
| MS | mass spectrometry |
| DGA | diglycolic acid |
| SA | succinic acid |
| VC | valine-citrulline |
| PBS | phosphate buffered saline |
| PB | phosphate buffer |
| Cys | cysteine |
| EDTA | disodium ethylenediaminetetraacetate |

Since the present invention has been described in terms of specific embodiments thereof, certain modifications and equivalent changes will be apparent to those skilled in the art and are included within the scope of the present invention.

Analytical methods:

**HPLC analytical method A:**

Instrument: Agilent 1260 HPLC
Column: Ultimate XB-C8 (3 μm 300 A 100 mm), column temperature 40°C
Phase A: 0.05% trifluoroacetic acid/water, phase B: 0.05% trifluoroacetic acid/acetonitrile, flow rate 0.4 mL/min, detection wavelength 210 nm, gradient 0.0-10 minutes 95-0% phase A, 10-13 min 0% phase A, 13-14 min 0-95%

phase A, 14-16 min 95% phase A.

**HPLC analytical method B:**

Instrument: Agilent 1260 HPLC
Column: Ultimate XB-C8 (3 μm 300 A 100 mm), column temperature 40°C
Phase A: 0.05% trifluoroacetic acid/water, phase B: 0.05% trifluoroacetic acid/acetonitrile, flow rate 0.4 mL/min, detection wavelength 210 nm, gradient 0.0-10 minutes 90-0% phase A, 10-15 min 0% phase A, 15-16 min 0-90% phase A, 16-18 min 90% phase A.

**HPLC analytical method C:**

Instrument: Agilent 1260 HPLC
Column: Ultimate XB-C8 (3 μm 300 A 100 mm), column temperature 40°C
Phase A: 0.05% trifluoroacetic acid/water, phase B: 0.05% trifluoroacetic acid/acetonitrile, flow rate 0.4 mL/min, detection wavelength 210 nm, gradient 0.0-10 minutes 90-0% phase A, 10-14 min 0% phase A, 14-15 min 0-90% phase A, 15-17 min 90% phase A.

**HPLC analytical method D:**

Instrument: Agilent 1260 HPLC
Column: Ultimate XB-C18 (4.6 μm 300 A 250 mm), column temperature 40°C
Phase A: 0.05% trifluoroacetic acid/water, phase B: 0.05% trifluoroacetic acid/acetonitrile, flow rate 1.0 mL/min, detection wavelength 210 nm, gradient 0.0-20 minutes 95-0% phase A, 20-25 min 0% phase A, 25-25.2 min 0-95% phase A, 25.2-28 min 95% phase A.

Example 1: synthesis of compound 10101

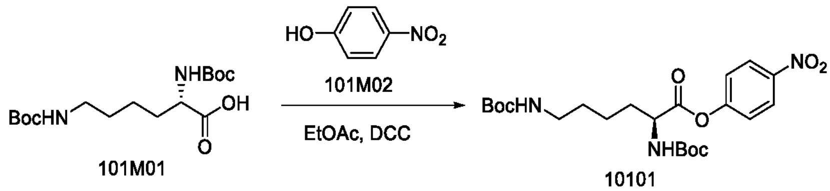


101M01

10101

Boc-Lys (Boc)-OH (0.42 kg) andp-nitrophenol (192 g) were dissolved in EtOAc (12 L) and cooled to 0°C with ice salt bath. DCC (284 g) was dissolved in EtOAc (6 L) and was added dropwisely to the above system. The reaction mixture was stirred at 0°C for 2 h. The reaction solution was warmed to room temperature and stirred for overnight. The reaction product was filtrated, the filter cake was washed with cold EtOAc, and the filtrate was concentrated and washed with petroleum ether/diethyl ether. The mixture was dissolved with EtOAc, precipitated with petroleum ether/diethyl ether, filtered and dried to give a product as white powder, Boc-Lys (Boc)-pNB (**10101**).

$^1$H NMR (400 MHz, DMSO-d6) δ 8.37 - 8.28 (m, 2H), 7.56 (d, J = 6.8 Hz, 1H), 7.44 - 7.35 (m, 2H), 6.82 (t, J = 5.7 Hz, 1H), 4.16 (dt, J = 12.0, 6.2 Hz, 1H), 2.93 (q, J = 6.1 Hz, 2H), 1.90 - 1.66 (m, 2H), 1.39 (d, J = 14.9 Hz, 22H).

Example 2: synthesis of compound 10102

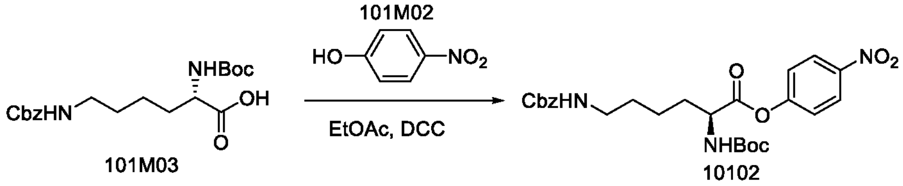

101M03 10102

Boc-Lys (Cbz)-OH (76 g) and *p*-nitrophenol (32 g) were dissolved in EtOAc (2 L), and cooled to 2°C with ice bath. DCC (47.4 g) was dissolved in EtOAc (1 L) and was added dropwisely to the above system. The reaction mixture was stirred at 2-3°C for 2 h. The reaction solution was warmed to room temperature and stirred for overnight. The reaction mixture was filtrated, concentrated, and precipitated with petroleum ether/methyl *tert*-butyl ether. The precipitate was vacuum-dried to give a product as white powder, Boc-Lys(Cbz)-pNB(**10102**).

$^1$H NMR (400 MHz, DMSO-d6) δ 8.41 - 8.24 (m, 2H), 7.59 (d, J = 6.8 Hz, 1H), 7.44 - 7.25 (m, 8H), 5.01 (s, 2H), 4.23 - 4.06 (m, 1H), 3.02 (q, J = 6.1 Hz, 2H), 1.91 - 1.68 (m, 2H), 1.41 (s, 13H).

Example 3: synthesis of compound 10103-1k

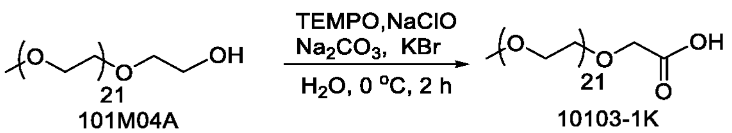

101M04A 10103-1K

mPEG$_{1k}$-OH (25.0 g), KBr (8.06 g), and $Na_2CO_3$ (18.55 g) were added to a 1 L flask, followed by 300 mL of water and TEMPO (0.78 g). The reaction mixture was cooled to 0 °C with ice bath. NaClO solution (74.4 g) was added slowly, and the reaction mixture was stirred for 2 h. Sodium bisulfite was added to quench the reaction. The reaction mixture was extracted with dichloromethane, dried over anhydrous sodium sulfate for 1 h, filtered, concentrated, and vacuum-dried to give a product, mPEG$_{1k}$-COOH (**10103-1K**) 24.00 g, 94.6% yield.

$^1$H NMR (400 MHz, $CDCl_3$) δ 4.15 (s, 2H), 3.65 (br, 85H), 3.38 (s, 3H).

Example 4: synthesis of compound 10103-2k

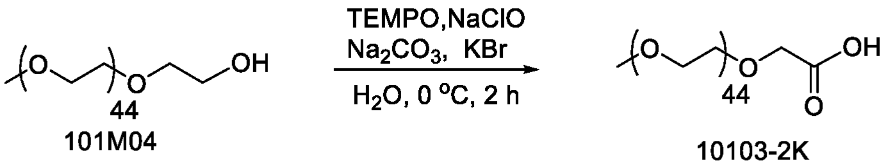

101M04 10103-2K

mPEG$_{2k}$-OH (50.0 g), KBr (2.083 g), and $Na_2CO_3$ (18.55 g) were added to a 1 L flask, followed by 300 mL of water and TEMPO (0.78 g). The reaction mixture was cooled to 0°C with ice bath. NaClO solution (74.4 g) was added slowly, and the reaction mixture was stirred for 2 h. Sodium bisulfite was added to quench the reaction. The reaction mixture was extracted with dichloromethane, and washed with saturated NaCl until the water phase was clear. The reaction mixture was dried over anhydrous sodium sulfate, and filtered. The filtrate was concentrated, and vacuum-dried to give a product as white solid, mPEG$_{2k}$-COOH (**10103-2K**) 48.81 g, 96.9% yield.

$^1$H NMR (400 MHz, $CDCl_3$) δ 4.15 (s, 2H), 3.65 (br, 175H), 3.38 (s, 3H).

Example 5: synthesis of compound 10104-1k

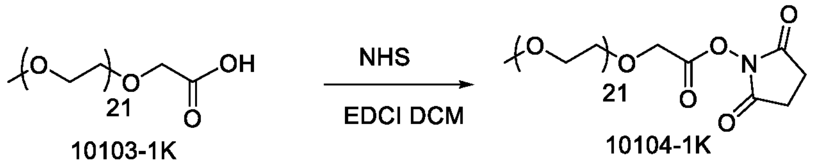


10103-1K 10104-1K

10103A (24.00 g) and NHS (5.44 g) were dissolved in dichloromethane and cooled to 0°C with ice bath. EDCI (9.06 g) was added, and the mixture was stirred overnight at room temperature until the precipitate was dissolved and the solution was clear, transparent and colorless. The reaction mixture was diluted with 120 mL of dichloromethane, washed with saturated NaCl solution, dried over anhydrous sodium sulfate, concentrated, and vacuum-dried to give a product, $mPEG_{1k}$-NHS (**10104-1K**) 23.46 g, 89.2% yield.

$^1H$ NMR (400 MHz, $CDCl_3$) δ 4.53 (s, 2H), 3.64 (br, 86H), 3.38 (s, 3H), 2.86 (s, 4H).

Example 6: synthesis of compound 10104-2k

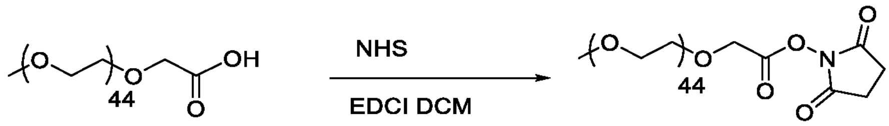


10103-2K 10104-2K

10103 (48.81 g) and NHS (5.54 g) were dissolved in dichloromethane and cooled to 0°C with ice bath. EDCI (9.23 g) was added, and the mixture was stirred overnight at room temperature. The reaction mixture was diluted with 250 mL of dichloromethane, washed with water and saturated NaCl, dried over anhydrous sodium sulfate, concentrated, and vacuum-dried to give a product, $mPEG_{2k}$-NHS (**10104-2K**) 48.34 g, 94.5% yield.

$^1H$ NMR (400 MHz, $CDCl_3$) δ 4.53 (s, 2H), 3.65 (br, 178H), 3.39 (s, 3H), 2.87 (s, 4H).

Example 7: synthesis of compound 10105

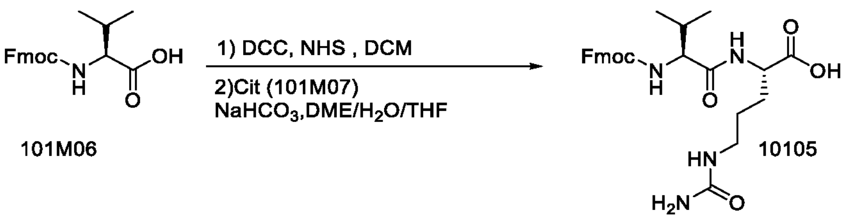


101M06 10105

Fmoc-Val-OH (10.17 g) and NHS (3.89 g) were dissolved in dichloromethane (200 mL), cooled with ice bath and stirred for 15 min, and DCC (6.59 g) was added in batches. The mixture was stirred at room temperature for ~5 h. The mixture was filtrated, and the filtrate was concentrated to obtain a transparent viscous mixture. The mixture was then dissolved with DME (80 mL). To **101M07** (5.78 g) in water (80 mL), $NaHCO_3$ (2.78 g) and THF (44 mL) were added. The resulting solution was added to the above DME solution, and the mixture was vigorously stirred for 12 h. 2 N hydrochloric acid was added to adjust pH to about 4 until a large amount of white precipitate appeared. The mixture was extracted with ethyl acetate, dried over anhydrous sodium sulfate, and concentrated to give a product, Fmoc-Val-Cit-OH (**10105**) (10.15 g), LCMS (m/z): $[M+H]^+$ = 496.9.

Example 8: synthesis of compound 10106

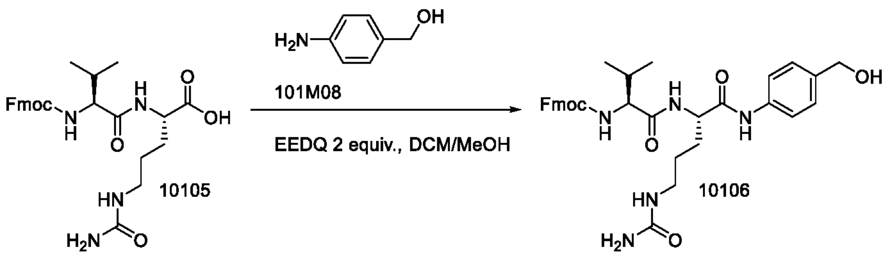


Fmoc-Val-Cit-OH (9.93 g) was dissolved in dichloromethane/MeOH (200/100 mL), and PABOH (4.93 g) and EEDQ (4.9 g) were added. The mixture was stirred at 30°C under nitrogen protection. After 1 h, EEDQ (5 g) was added, the mixture was stirred for 12 h. The mixture was concentrated until most of the solvent was removed, and 90 mL of ethyl acetate was added. The mixture was filtered and vacuum-dried to give a product Fmoc-Val-Cit-PABC (**10106**) 8.80 g (14.6 mmol, 73.1%), LCMS (m/z): $[M+H]^+$ = 601.9.

Example 9: synthesis of compound 10107

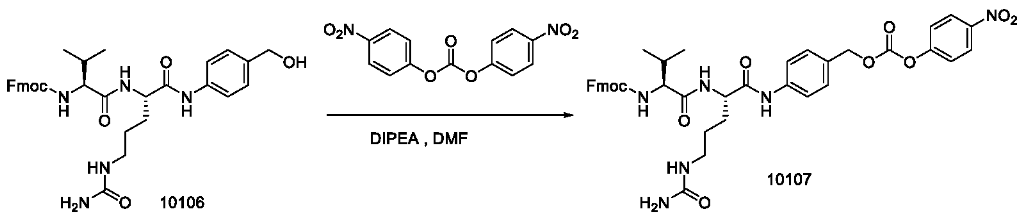


Fmoc-Val-Cit-PABC (8.80 g) was dissolved in DMF (170 mL), and cooled with ice bath. Bis(p-nitrobenzene) carbonate (13.32 g) and DIPEA (5.31 mL) were added. The mixture was warmed to room temperature and stirred for 1 h. DMF was removed via rotary evaporateion, and dichloromethane/EtOAc was added. The mixture was filtered and vacuum-dried to give a product as yellowish solid, Fmoc-Val-Cit-PABC-pNP (10107) 9.40 g (12.1 mmol, 82.9%).

LCMS (m/z): $[M+H]^+$ = 766.9.

Example 10: synthesis of compound 10108

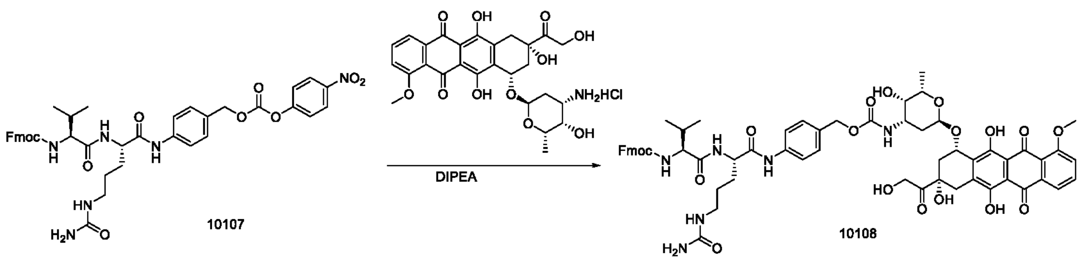


Fmoc-Val-Cit-PABC (1.23 g, 1 eq.) was dissolved in DMF (13 mL), and DOX·HCl (0.93 g) and DIPEA (0.836 mL) were added. The mixture was stirred at room temperature for 2 h. 120 mL of MTBE was added to obtain a reddish-brown precipitate. The precipitate was filtered, and the filter cake was washed with MTBE/MeOH and vacuum-dried to give a product, Fmoc-Val-Cit-PABC-DOX (**10108**) 1.85 g (98.9%).

LCMS: theoretical $[M+2H]^{2+}$=586.2, measured 584.2.

Example 11: synthesis of compound 10109

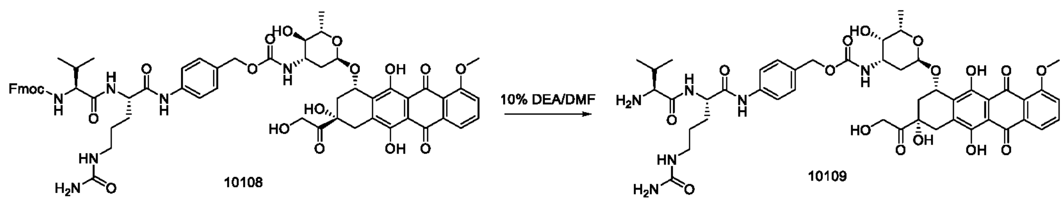


Fmoc-Val-Cit-PABC-DOX (1.85 g) was dissolved in DMF (18 mL). Diethylamine (2 mL) was added, and the solution turned purple-black. The mixture was stirred at room temperature for 15 min, and 280 mL of MTBE was added. The mixture was filtered, vacuum-dried to give dark red solid powder $NH_2$-Val-Cit-PABC-DOX (**10109**) crude 1.47 g. Purification with prep-HPLC gave red fluffy powder 1.12 g (74%).

$^1$H NMR (400 MHz, DMSO-d6) δ 14.03 (s, 1H), 13.27 (s, 1H), 10.17 (s, 1H), 8.67 (d, J = 7.6 Hz, 1H), 8.09 (d, J = 5.1 Hz, 3H), 7.91 (d, J = 4.9 Hz, 2H), 7.65 (p, J = 3.8 Hz, 1H), 7.54 (d, J = 8.1 Hz, 2H), 7.26 (d, J = 8.2 Hz, 2H), 6.83 (d, J = 8.1 Hz, 1H), 6.07 (t, J = 5.9 Hz, 1H), 5.47 (d, J = 7.9 Hz, 3H), 5.22 (d, J = 3.6 Hz, 1H), 4.92 (d, J = 17.6 Hz, 4H), 4.72 (s, 1H), 4.58 (s, 2H), 4.49 (td, J = 8.2, 5.1 Hz, 1H), 4.16 (q, J = 6.6 Hz, 1H), 3.99 (s, 3H), 3.80 - 3.60 (m, 2H), 3.11 - 2.87 (m, 5H), 2.26 - 1.97 (m, 4H), 1.84 (td, J = 13.0, 3.9 Hz, 1H), 1.71 (dq, J = 13.3, 7.2, 6.5 Hz, 1H), 1.61 (dtd, J = 13.5, 8.7, 4.6 Hz, 1H), 1.52 - 1.34 (m, 3H), 1.13 (d, *J* = 6.4 Hz, 3H), 0.93 (dd, *J* = 6.8, 2.4 Hz, 7H).

LC-MS: $[M+H]^+$=949.2

Example 12: synthesis of compound 10126

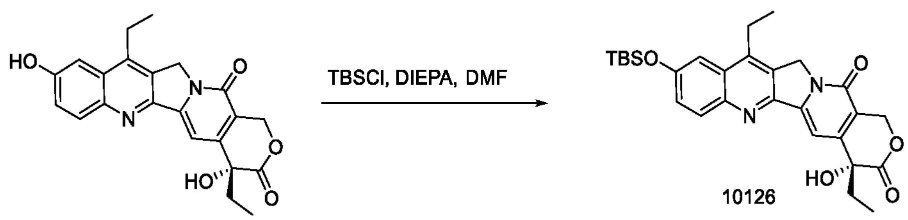


SN-38 (2.00 g) was dispersed in 80 mL of DMF. 3.18 mL of DIPEA was added to give a yellow suspension. TBSCl (2.69 g) was added, and the solution gradually turned clear. The mixture was stirred at room temperature for 1 h and cooled with ice bath. 80 mL of water was added dropwisely, and the precipitate was filtered. The filter cake was washed and vacuum-dried at 40°C overnight to give light yellow solid 10-O-TBS-SN-38 (10126), 2.42 g (93.9%). LCMS: $[M+H]^+$=507.2.

Example 13: synthesis of compound 10127

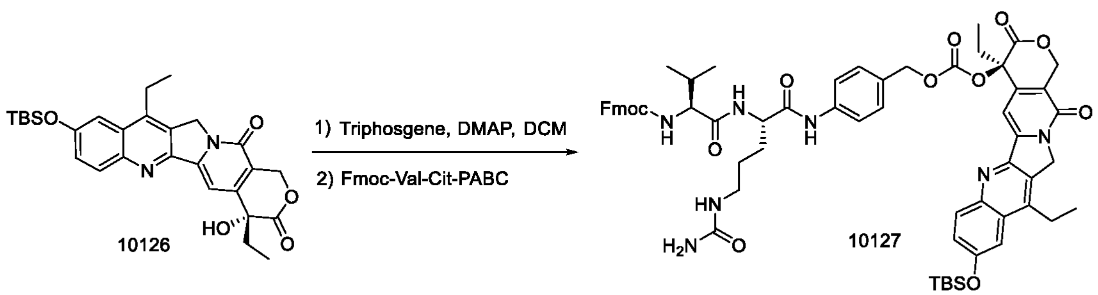


10-O-TBDMS-SN-38 (173 mg) and DMAP (136 mg) were suspended in dichloromethane (2 mL). Triphosgene (53 mg) in dichloromethane (3.5 mL) was added, and the solution became cloudy. The reaction mixture was stirred at 20°C for 10 min. Fmoc-Val-Cit-PABC (180 mg) was added, and the solution became clear. The reaction mixture was stirred at room temperature overnight. Purification with column chromatography using 5-20% MeOH/dichloromethane gave 20-0-(Fmoc-Val-Cit-PABC)-10-0-TBS-SN-38 (10127) 264 mg (46.5%).

LCMS: theoretical $[M+2H]^{2+}$=567.7, measured 565.2.

Example 14: synthesis of compound 10128

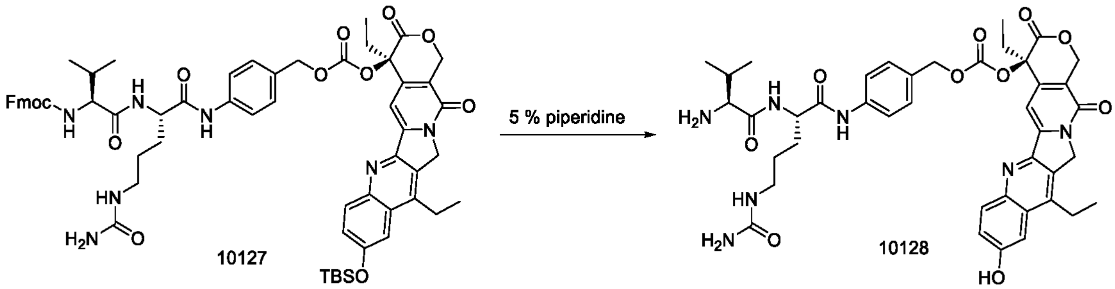


20-O-(Fmoc-Val-Cit-PABC)-10-O-TBS-SN-38 (10127) (200 mg) was dissolved in DMF (6 mL), and piperidine (300 μL) was added. The mixture was stirred at room temperature for 20 min, and methyl *tert*-butyl ether was added to form precipitate. The precipitate was vacuum-dried to give a product $NH_2$-Val-Cit-PABC-20-O-SN-38 (10128) 140 mg (99.0%).

LCMS: theoretical $[M+H]^+$=798.1.

Comparative example 1: synthesis of compound 101A1601

Synthesis of compound 101A01:

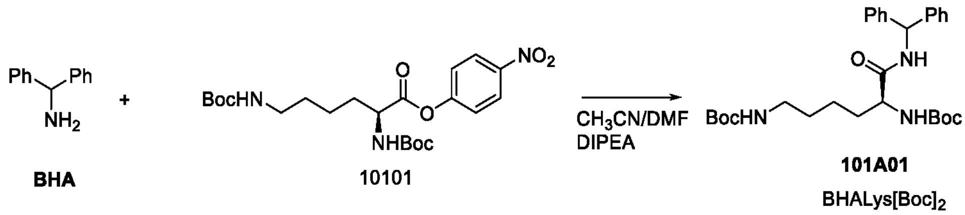


in ice-water bath at 5°C under nitrogen protection, DIPEA (2.53 g) was added to a solution of diphenylmethanamine (BHA, 2.50 g) in $CH_3CN$/DMF (20/10 mL), **10101** (7.02 g) was then added in batches, and the reaction mixture was stirred for 12 h. 0.5 N sodium hydroxide solution (100 mL) was added to the above reaction solution. The mixture was extracted with ethyl acetate, dried over anhydrous sodium sulfate, and concentrated to give a crude product BHA-Lys (α, ε-Boc) (**101A01**) (3.8 g, 55% yield). MS (ESI), m/z, 512.2 $[M+H]^+$.

Synthesis of compound 101A02:

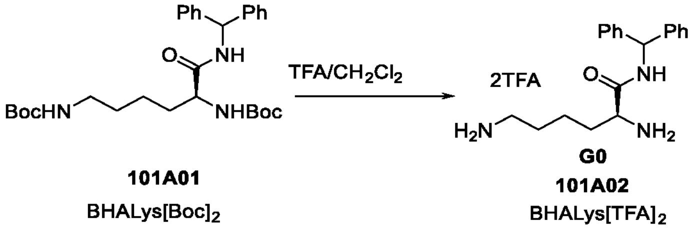


at room temperature (25°C) under nitrogen protection, TFA (2 mL) was added to a solution of **101A01** (3.80 g) in dichloromethane (10 mL). The reaction mixture was stirred for 12 h. The above reaction solution was concentrated, and

dichloromethane (10 mL) and MTBE (30 mL) were added. The mixture was concentrated to give a product **101A02** as foamy solid, BHA-Lys(α, ε-$NH_3$+$CF_3COO$-) (**101A02**) (3.0 g, 89% yield). MS (ESI), m/z, 312.1 $[M+H]^+$.

Synthesis of compound 101A03:

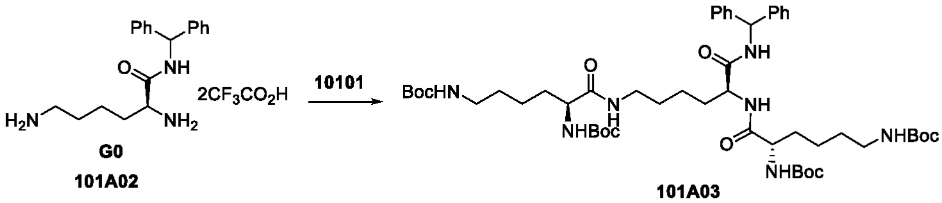


G0

101A02

BHALys[TFA]$_2$

101A03

BHALys[Lys]$_2$[Boc]$_4$

in ice-water bath at 5°C under nitrogen protection, DIPEA (1.70 g) was added to a solution of **101A02** (TFA salt, 1.00 g) in DMF (10 mL ), **10101** (1.91 g) was then added in batches, and the reaction solution was stirred for 12 h. 0.5 N sodium hydroxide solution (50 mL) was added to the above reaction solution. The reaction mixture was stirred for 1 h. The mixture was filtered, the filter cake was washed with 0.5 N sodium hydroxide solution, and the filter cake was vacuum-dried to give a product **101A03** as white solid (1.50 g, 84% yield). MS (ESI), m/z, 968.5 $[M+H]^+$.

Synthesis of compound 101A04:

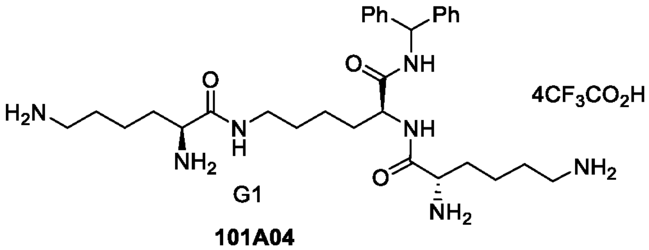


G1

**101A04**

BHALys[Lys]2[TFA]4

at room temperature (25°C) under nitrogen protection, TFA (2 mL) was added to a solution of **101A03** (1.5 g, 1.55 mmol) in dichloromethane (10 mL) at room temperature under nitrogen protection. The reaction mixture was stirred for 12 h. The above reaction solution was concentrated to give a product **101A04** as foamy solid (1.50 g, 95% yield).

$^1$H NMR (400 MHz, $D_2O$) δ 7.41 - 7.19 (m, 10H), 6.03 (s, 1H), 4.36 (t, *J* = 7.4 Hz, 1H), 3.94 (t, *J* = 6.5 Hz, 1H), 3.85 - 3.76 (m, 1H), 3.10 (t, *J* = 7.3 Hz, 2H), 2.90 (t, *J* = 7.9 Hz, 2H), 2.63 (t, *J* = 7.7 Hz, 2H), 2.03 - 0.99 (m, 18H).

MS (ESI), m/z, 568.3 $[M+H]^+$.

Synthesis of compound 101A05:

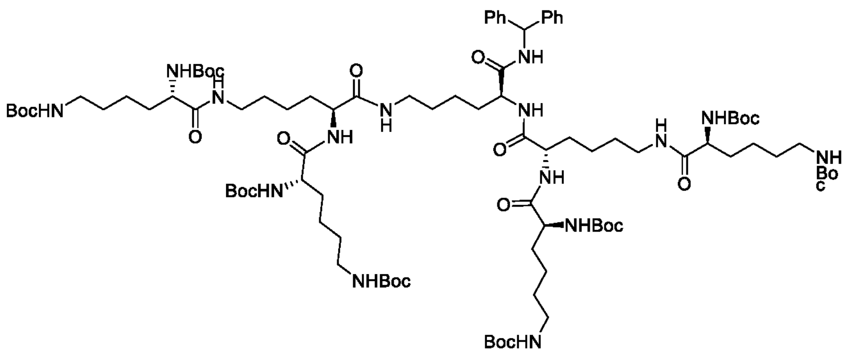

BHALys[Lys]$_4$[Boc]$_8$

**101A05**

in ice-water bath at 5°C under nitrogen protection, DIPEA (2.60 g) was added to a solution of **101A04** (TFA salt, 1.50 g) in DMF (30 mL), **10101** (prepared in example 1) (3.01 g) was then added, and the reaction mixture was stirred for 12 h. 0.5 N sodium hydroxide solution (100 mL) was added to the above reaction solution. The mixture was stirred for 1 h, and filtered. The solid was collected and vacuum-dried to give a product **101A05** as white solid (2.3 g, 83% yield).

MS (ESI), m/z, 941.0 $[M/2+H]^+$.

Synthesis of compound 101A06:

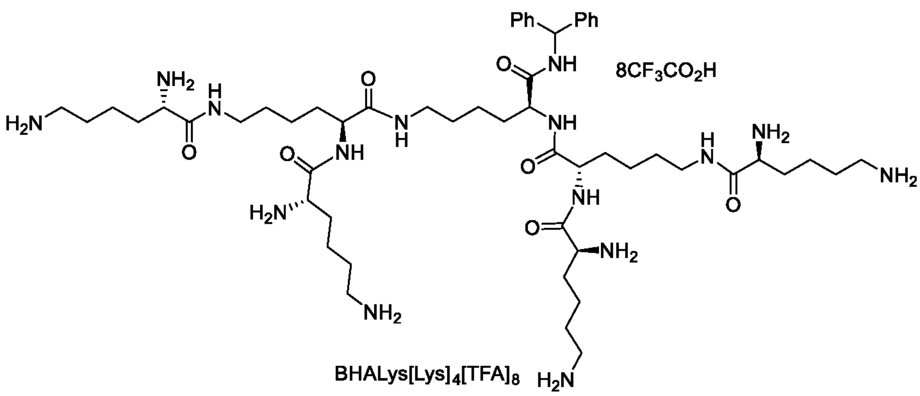

BHALys[Lys]$_4$[TFA]$_8$

**101A06**

at room temperature (25°C) under nitrogen protection, TFA (10 mL) was added to a solution of **101A05** (2.30 g) in dichloromethane (10 mL). The mixture was stirred for 4 h. The above reaction solution was concentrated and dissolved with dichloromethane/MeOH. MTBE was added, and the mixture was concentrated to give a product **101A06** as foamy solid (2.41 g, 98.5% yield). MS (ESI), m/z, 540.8 $[M/2+H]^+$.

$^1$H NMR (400 MHz, $D_2O$) δ 7.45 - 7.11 (m, 10H), 6.02 (s, 1H), 4.32 - 4.09 (m, 3H), 4.01 - 3.92 (m, 2H), 3.85 (t, *J* = 6.7 Hz, 1H), 3.78 (t, *J* = 6.7 Hz, 1H), 3.22 - 2.80 (m, 14H), 1.94 - 1.06 (m, 42H).

Synthesis of compound 101A07:

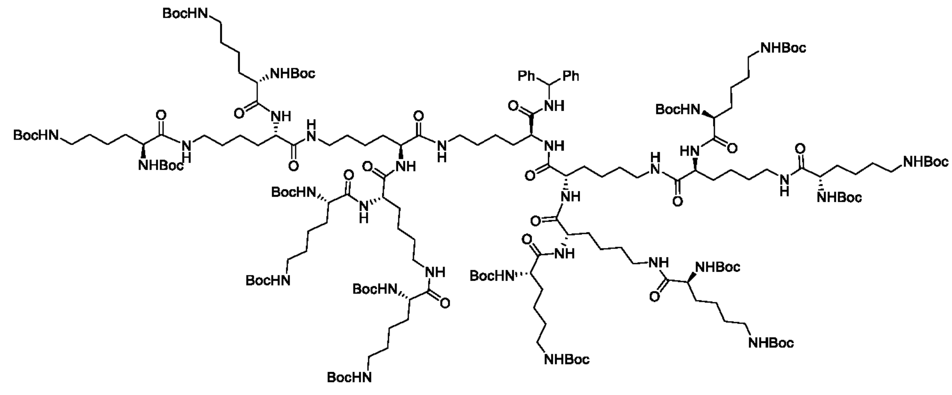

BHALys[Lys]$_8$[Boc]$_{16}$

**101A07**

in ice-water bath at 5°C under nitrogen protection, DIPEA (3.73 g) was added to a solution of **101A06** (TFA salt, 2.40 g) in DMF (20 mL), **10101** (prepared by Example 1) (5.41 g) was then added, and the reaction mixture was stirred for 12 h. 0.5 N sodium hydroxide solution (150 mL) was added to the above reaction solution. The reaction mixture was stirred for 1 h. The mixture was filtered, and the solid was collected and dissolved in acetonitrile. Water (1/1 volume) was added to the solution to form precipitate, and the mixture was stirred for 1 h and filtered. The solid was collected and vacuum-dried to give a product **101A07** as white solid (3.82 g, 85% yield).

Synthesis of compound 101A08:

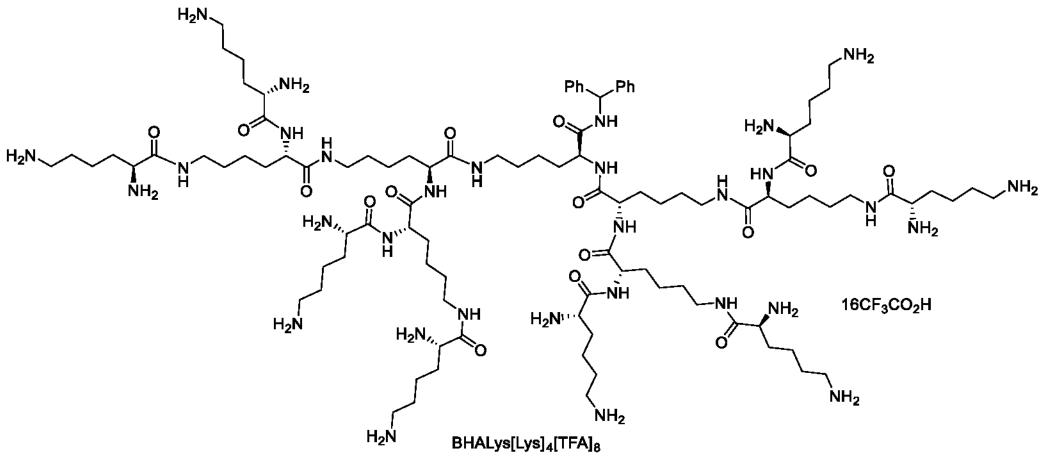

BHALys[Lys]$_4$[TFA]$_8$

**101A08**

at room temperature (25°C) under nitrogen protection, TFA (10 mL) was added to a solution of **101A07** (3.0 g) in dichloromethane (10 mL). The reaction mixture was stirred for 4 h. The above reaction solution was concentrated and dissolved with acetonitrile (30 mL). MTBE (150 mL) was added to form precipitate, and the mixtured was filtered. The soild was collected and vacuum-dried to give a product **101A08** as white solid, 2.9 g, 91% yield. MS (ESI), m/z, 702.7 $[M/3+H]^+$.

$^1H$ NMR (400 MHz, $D_2O$) δ 7.64 - 6.97 (m, 10H), 6.02 (s, 1H), 4.31 - 4.04 (m, 7H), 3.96 (t, *J* = 6.7 Hz, 4H), 3.90 - 3.79 (m, 5H), 3.30 - 2.77 (m, 30H), 1.93 - 0.96 (m, 90H).

Synthesis of compound 101A09:

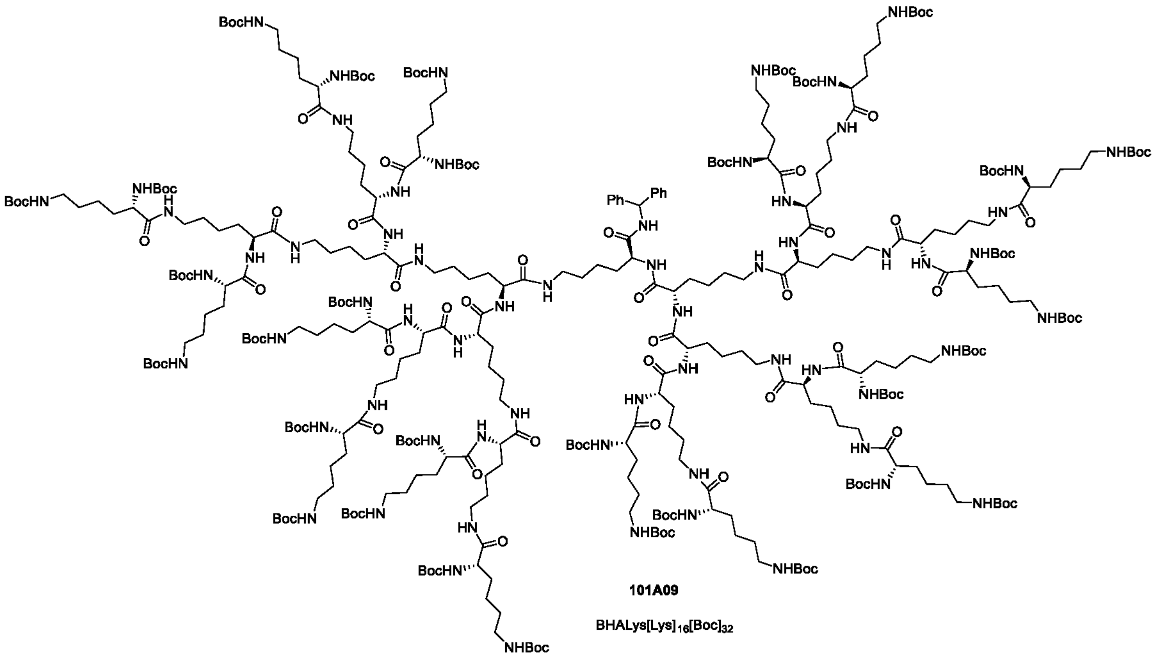

**101A09**

BHALys[Lys]$_{16}$[Boc]$_{32}$

in ice-water bath at 5°C under nitrogen protection, DIPEA (3.94 g) was added to a solution of **101A08** (TFA salt, 2.50 g) in DMF (20 mL), **10101** (5.94 g) was then added, and the reaction solution was stirred for 12 h. 0.5 N sodium hydroxide solution (100 mL) was added to the above reaction solution. The mixture was stirred for 1 h. The mixture was filtered, and the solid was collected and vacuum-dried to give a product **101A09** as white solid (3.7 g, 80% yield).

Synthesis of compound 101A10:

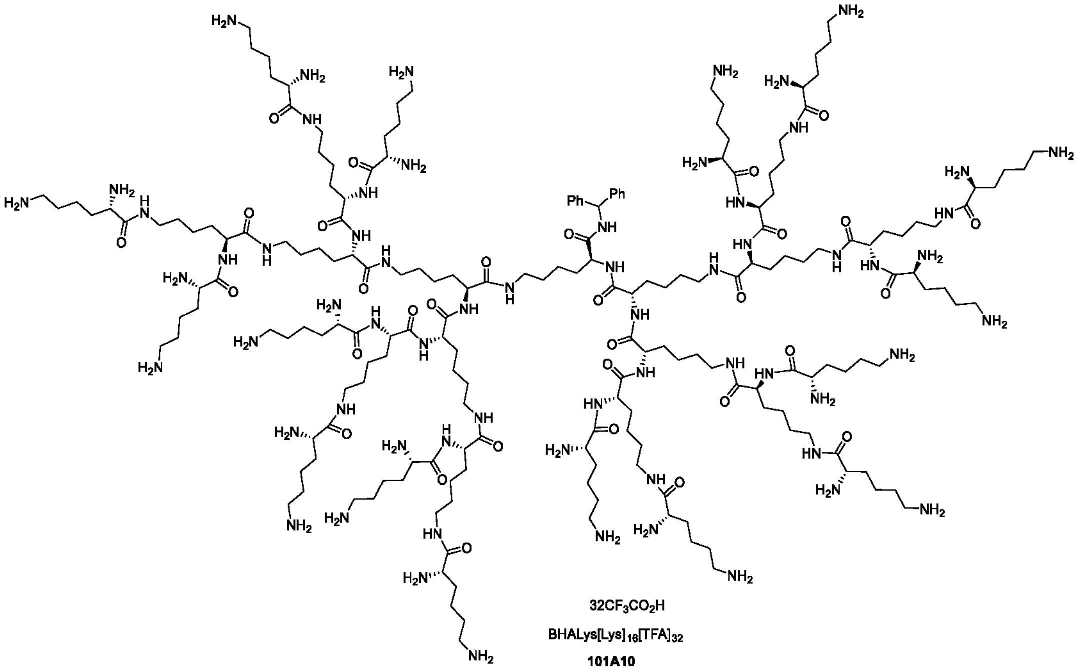

$32CF_3CO_2H$

BHALys[Lys]$_{16}$[TFA]$_{32}$

**101A10**

at 25°C under nitrogen protection, TFA (10 mL) was added to a solution of **101A09** (3.0 g) in dichloromethane (10 mL). The reaction mixture was stirred for 12 h. The above reaction solution was concentrated, and dissolved with acetonitrile (20 mL). MTBE (100 mL) was added to form precipitate, and the mixture was filtered. The solid was collected and vacuum-dried to give a product **101A10** as white solid (3.0 g, 94% yield). MS (ESI), m/z,832.1 [M/5+H]$^+$.

$^1H$ NMR (400 MHz, $D_2O$) δ 7.45 - 7.03 (m, 10H), 6.01 (s, 1H), 4.34 - 4.06 (m, 16H), 4.02 - 3.92 (m, 8H), 3.90 - 3.79 (m, 8H), 3.29 - 2.72 (m, 62H), 2.00 - 0.93 (m, 186H).

Synthesis of compound 101A11:

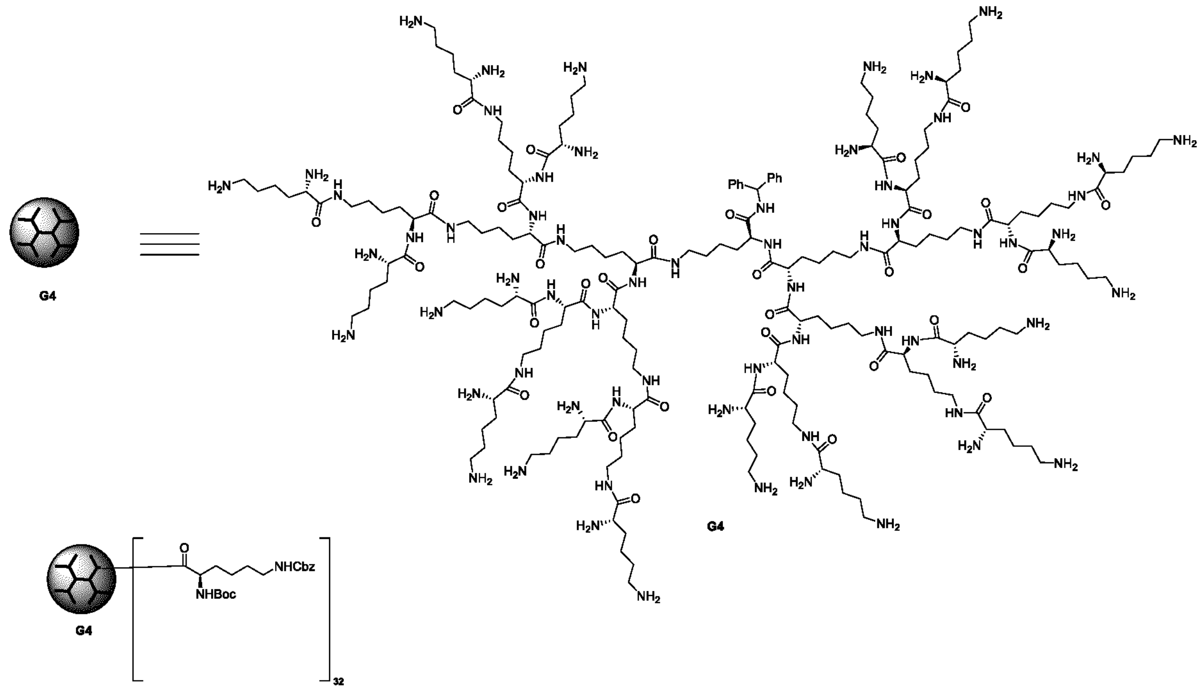

G4

BHALys[Lys]$_{32}$[α-Boc]$_{32}$[ε-Cbz]$_{32}$

101A11

in an ice-water bath at 5°C under nitrogen protection, DIPEA (3.3 g) was added to a solution of **101A10** (TFA salt, 2.0 g) in DMF (20 mL), **10102** (4.79 g) was then added, and the reaction solution was stirred for 6 h. 0.5 N sodium hydroxide solution (100 mL) was added to the above reaction solution. The mixture was stirred for 1 h. The mixture was filtered, and the solid was collected and suspended in $CH_3CN$ (50 mL). Aqueous solution (200 mL) was added, and the mixture was stirred for 1 h and filtered. These steps were repeated twice, and the solid was collected and vacuum-dried to give a product **101A11** as white solid (3.8 g, 94% yield).

Synthesis of compound 101A12:

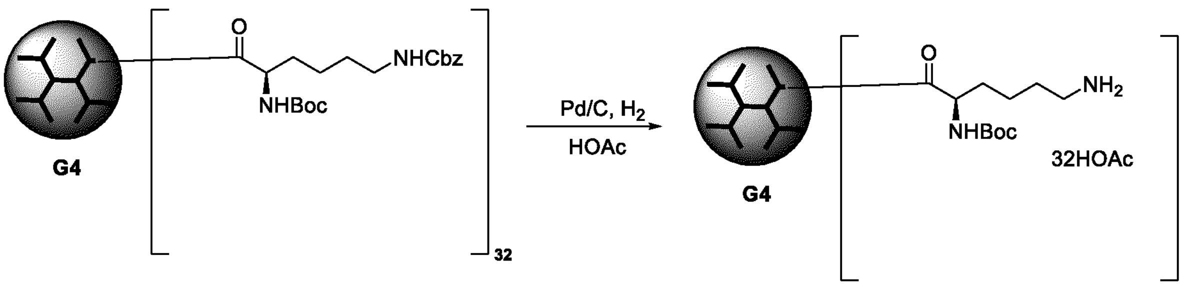


BHALys[Lys]$_{32}$[α-Boc]$_{32}$[ε-Cbz]$_{32}$

**101A11**

BHALys[Lys]$_{32}$[α-Boc]$_{32}$[HOAc]$_{32}$

**101A12**

at room temperature (25°C) under nitrogen protection, Pd/C or $Pd(OH)_2$ (10%, 1.0 g) was added to a solution of **101A11**

(2.50 g, 0.16 mmol) in acetic acid (50 mL, filtration through diatomaceous earth is required if the solution is not clear). The mixture was stirred in a hydrogen environment (hydrogen balloon) for 24 h at 30°C. The above reaction solution was concentrated, and dissolved with methanol (20 mL). MTBE (100 mL - 200 mL) was added to form precipitate. The mixture was filtered, and the solid was collected and vacuum-dried to give a product **101A12** as gray-white solid (1.7 g, 80% yield). MS (ESI), m/z,832.1 $[M/5+H]^+$.

$^1$HNMR (400 MHz, $D_2O$) δ 7.53 - 6.98 (m, 10H), 6.02 (s, 1H), 4.15 (s, 34H), 3.89 (d, *J* = 26.3 Hz, 38H), 3.38 - 2.71 (m, 126H), 1.86 (s, 118H), 2.21 - 0.70 (m, 669H).

Synthesis of compound 101A13-1K:

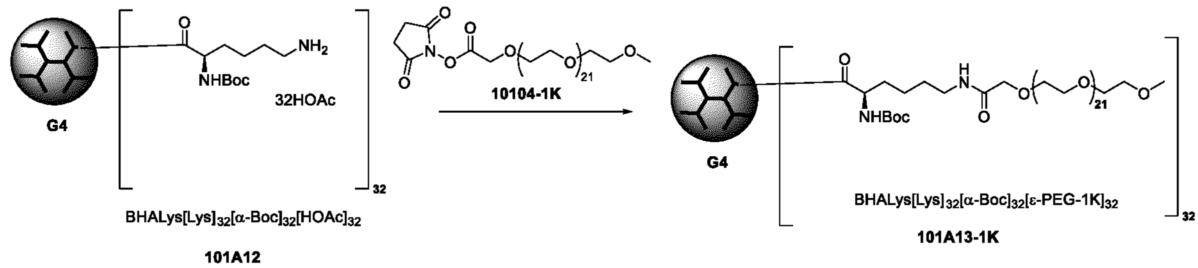


at 25°C under nitrogen protection, DIPEA (0.96 g) was added to a solution of **101A12** (1.0 g) in DMF (10 mL). **10104-1K** (3.6 g) was then added, and the reaction solution was stirred for 12 h. MTBE/MeOH was added, and the mixture was filtered. The filter cake was vacuum-dried to give a crude product **101A13-1K**, which was directly used for the next step.

Synthesis of compound 101A14-1K:

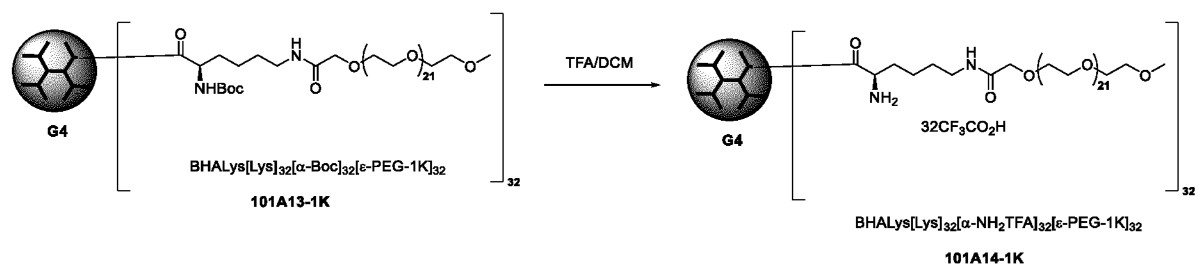


at 25°C under nitrogen protection, TFA (20 mL) was added to a solution of **101A13-1K** in dichloromethane (30 mL). The mixture was stirred for 12 h. The above reaction solution was concentrated, and MTBE (50 mL) was added. The mixture was stirred for 10 minutes and filtered, and the filter cake was vacuum-dried to give a product **101A14-1K** as colorless oil (2.5 g, 72% yield for two steps).

Synthesis of compound 101A15-DGA-1K:

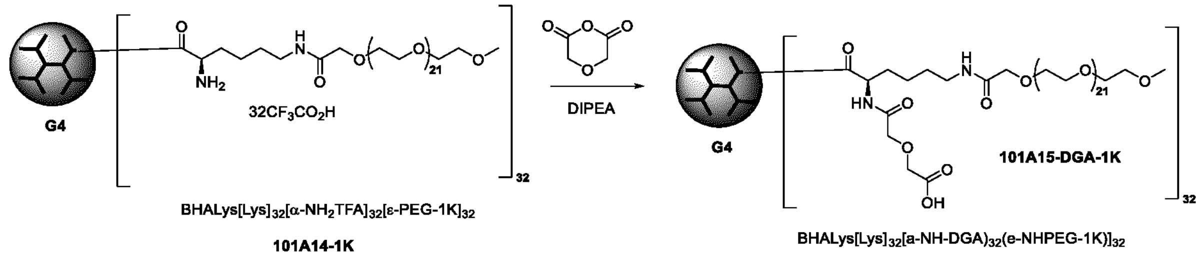

at 25°C under nitrogen protection, DIPEA (277 mg) was added to a solution of **101A14-1K** (1.0 g) in DMF (5 mL). Diethylene glycol anhydride (159 mg) was then added. The mixture was stirred for 12 h, and MTBE was added. The mixture was stirred for 10 minutes. The supernatant was poured out, and the residue was vacuum-dried to give a product **101A15-DGA-1K** as gray-white solid (0.8 g, 74% yield).

Synthesis of compound 101A1601:

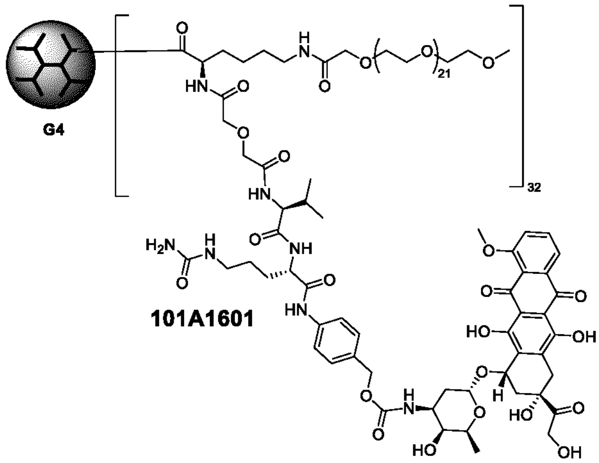

**101A1601**

in an ice-water bath at 5°C under nitrogen protection, DIPEA (97 mg, 0.76 mmol, 128.0 eq), **10109** (prepared in Example 11) (269 mg, 0.28 mmol, 48.0 eq) and PyBOP (246 mg, 0.47 mmol, 80.0 eq) were added to a solution of **101A15-DGA-1K** (300 mg, 0.006 mmol) in DMF (5 mL). The mixture was stirred for 12 h at room temperature. HPLC showed the reaction was complete. The residue was directly dissolved in DMSO/MeOH, purified by ultrafiltration and freeze-dried to give a final product (**101A1601**) 300 mg as dark purple solid, purity > 98%.

HPLC-based purity detection method: the compound was dissolved with methanol; instrument: Agilent 1260 HPLC; column: Ultimate XB-C8 (3 μm 300 A 100 mm); column temperature 40°C; flow rate 0.4 mL/min; detection wavelength UV 210 nm; area percentage calculation method; mobile phase A: 0.05% TFA in water, mobile phase B: 0.05% TFA in $CH_3CN$; gradient: run for 16 min, 10 min: 95:5 A/B to 0:100 A/B, 5 min: 0: 100 A/B, 1 min: 0: 100 A/B to 95:5 A/B.

Comparative example 2: synthesis of compound 101A1602:

Synthesis of compound 101A13-2K:

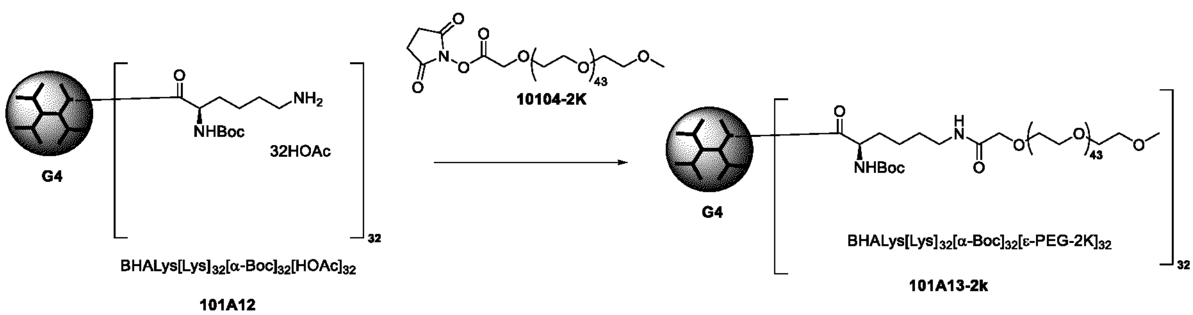


at 25°C under nitrogen protection, DIPEA (0.96 g) was added to a solution of **101A12** (1.0 g) in DMF (10 mL). **10104-2K** (6.48 g) was then added, and the reaction solution was stirred for 12 h. MTBE (100 mL) was added to the above reaction solution, and the mixture was filtered. The filter cake was vacuum-dried to give a product **101A13-2K** (4.0 g, 70% yield).

Synthesis of compound 101A14-2K:

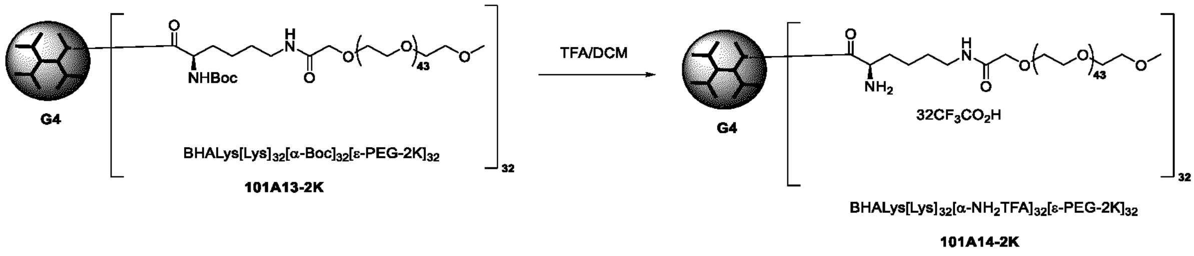


at 25°C under nitrogen protection, TFA (30 mL) was added to a solution of **101A13-2K** (4.0 g) in dichloromethane (50 mL). The mixture was stirred for 12 h. MTBE was added, and the mixture was cooled to -20°C to form precipitate. The mixture was filtered, and the solid was collected and vacuum-dried to give a product **101A14-2K** as gray-white solid (3.8 g, 95% yield).

Synthesis of compound 101A15-DGA-2K:

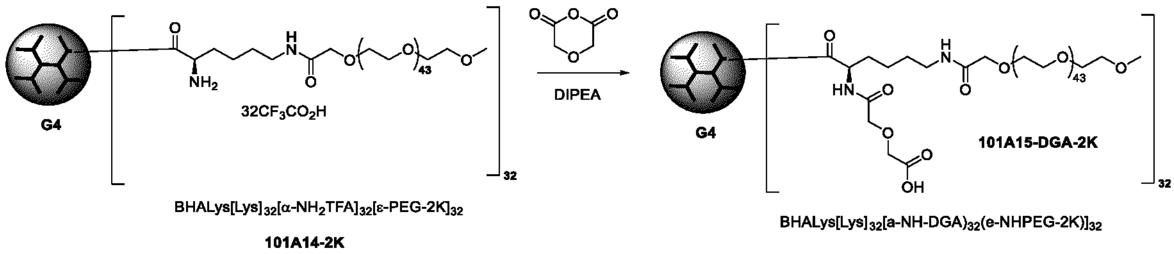


at 25°C under nitrogen protection, DIPEA (133 mg) was added to **101A14-2K** (0.8 g) in DMF (5 mL). Diethylene glycol anhydride (76 mg) was added, and the mixture was stirred for 12 h. $CH_3CN$/MTBE was added, and the mixture was stirred for 10 minutes. The supernatant was poured out, and the residue was vacuum-dried to give a product **101A15-DGA-2K** as gray-white solid (0.7 g, 83% yield).

Synthesis of compound 101A1602:

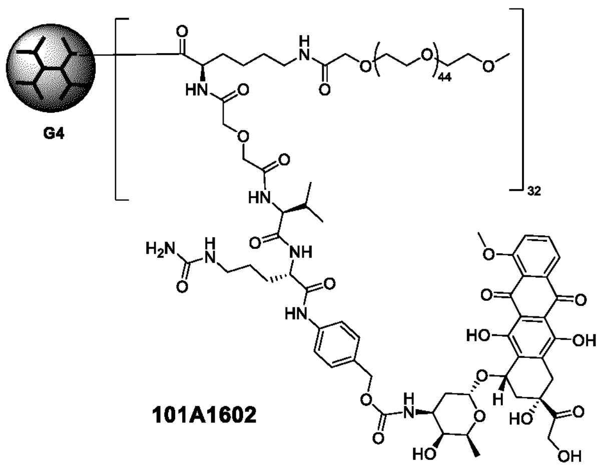

**101A1602**

in an ice-water bath at 5°C under nitrogen protection, DIPEA (101 mg, 0.78 mmol, 128.0 eq), 10109 (prepared in Example 11) (278 mg, 0.29 mmol, 48.0 eq) and PyBOP (254 mg, 0.49 mmol, 80.0 eq) were added to a solution of **101A15-DGA-2K** (500 mg, 0.006 mmol) in DMF (5 mL). The mixture was stirred for 12 h at room temperature. HPLC showed the reaction was complete. MTBE (50 mL) was added, and the mixture was stirred for 10 minutes. The supernatant was poured out, and the residue was dissolved in DMSO/MeOH, purified by ultrafiltration and freeze-dried to give a final product (101A1602) 500 mg as dark purple solid, purity > 98%.

HPLC-based purity detection method: the compound was dissolved with methanol; instrument: Agilent 1260 HPLC; column: Ultimate XB-C8 (3 μm 300 A 100 mm); column temperature 40°C; flow rate 0.4 mL/min; detection wavelength UV 210 nm; area percentage calculation method; mobile phase A: 0.05% TFA in water, mobile phase B: 0.05% TFA in $CH_3CN$; gradient: run for 16 min, 0-10 min: 5% B-100% B, 10-15 min: 100% B, 15-15.1 min: 100% B-5% B, 15.1-16 min 5% B.

Comparative example 3: synthesis and characterization of compound 101A1603

Synthesis of compound 101A15-SA-2K:

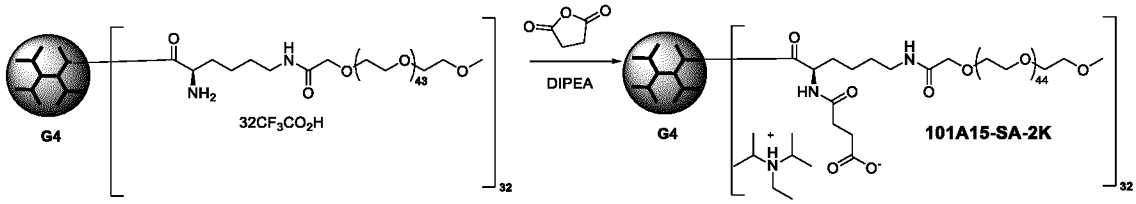


G4 32$CF_3CO_2H$ G4 101A15-SA-2K

BHALys[Lys]$_{32}$[α-$NH_2$TFA]$_{32}$[ε-PEG-2K]$_{32}$

101A14-2K

BHALys[Lys]$_{32}$[α-NH-SA)$_{32}$(ε-NHPEG-2K)]$_{32}$

at 25°C under nitrogen protection, DIPEA (166 mg, 1.29 mmol, 100.0 eq) was added to a solution of **101A14-2K** (1.0 g, 0.013 mmol) in DMF (5 mL). Succinic anhydride (82 mg, 0.82 mmol, 64.0 eq) was then added, and the mixture was stirred for 12 h. Upon completion, MTBE (50 mL) was added, and the mixture was stirred for 10 minutes. The supernatant was poured out. These steps were repeated 3 times, and the residue was vacuum-dried to give a product **101A15-SA-2K** (0.8 g, 76% yield).

Synthesis of compound 101A1603:

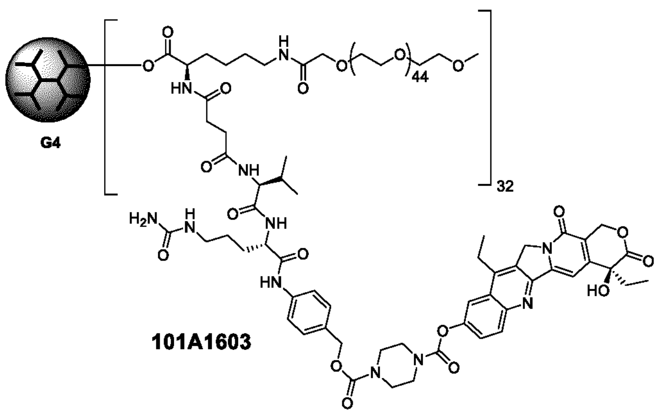

G4

**101A1603**

in an ice-water bath at 5°C under nitrogen protection, DIPEA (101 mg, 0.78 mmol, 128.0 eq), **10114** (269 mg, 0.30 mmol, 48.0 eq) and PyBOP (256 mg, 0.49 mmol, 80.0 eq) were added to a solution of **101A15-SA-2K** (500 mg, 0.006 mmol) in DMF (5 mL). The mixture was stirred for 12 h at room temperature. HPLC showed the reaction was complete. MTBE (50 mL) was added, and the mixture was stirred for 10 minutes. The supernatant was poured out, and the residue was dissolved in DMSO/MeOH, purified by ultrafiltration and freeze-dried to give a final product (**101A1603**), 592 mg, as light

yellow fluffy solid, HPLC purity> 90%.

Determination of SN-38 conjugation content in compound 101A1603

Papain was weighed and added to PBS to prepare 6.25 mg/mL PBS solution.

122.8 mg of freeze-dried product BHA-Lys32 (α-SA-VC-PABC-piperazine-10-O-SN-38, ε-mPEG2k)32 (**101A1603**) was weighed, and 4.910 mL of deionized water was added to obtain 25.0 mg/mL polymer solution.

0.200 mL of polymer solution and 1.800 mL of papain PBS solution (having a final concentration of polymer of 2.50 mg/mL) were taken, and were shaken in water bath at 37°C for 72 h. The solution was cooled to room temperature. 0.100 mL of solution was pipetted, and 0.900 mL of DMSO was added. The peak area of piperazine-10-O-SN-38 after enzymatic digestion was characterized by HPLC, and the concentration of TFA-piperazine-10-O-SN-38 was calculated to be 552.6 μg/mL from a standard curve of TFA-piperazine-10-O-SN-38, which was equivalent to a conclusion that the concentration of SN-38 was 267.1 μg/mL after the enzymatic digestion of 5.00 mg/mL of polymer, and therefore, the SN-38 conjugation content in BHA-Lys32 (α-SA-VC-PABC-piperazine-10-O-SN-38, ε-mPEG2k)32 was calculated to be 10.7 wt.%.

Example 15: synthesis of compound 101B00A

Synthesis of compound 101B01:

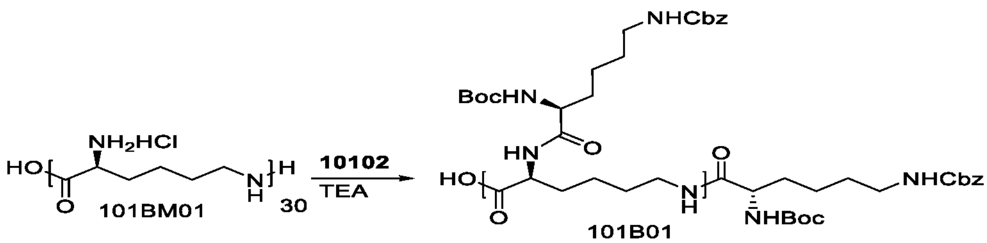


Poly(ε-L-Lysine) hydrochloride (4.792 g, 29.09 mmol, based on structural units) was suspended in 100 g DMSO. Triethylamine (8.89 g, 87.27 mmol) and α-Boc-ε-Cbz-L-lysine-NHS active ester (**10102**, prepared in Example 2) (20.83 g, 46.63 mmol) were added, and the mixture was stirred at 30°C under nitrogen protection for 13 h. Upon completion, the reaction solution was placed in a beaker, and 800 mL of ACN was added. The mixture was filtered, and the filter cake was sequentially washed with acetonitrile/water/acetonitrile and vacuum-dried to give a product ε-PolyLys$_{30}$-[Lys(α-Boc, ε-Cbz)]$_{30}$ (**101B01**) as white solid, 12.13 g (85%).

$^1$H NMR (400 MHz, DMSO-$d_6$) 7.74 (m, 62H), 7.27 (m, 179H), 6.90 (m, 28H), 5.16 - 4.80 (s, 60H), 4.31 - 4.01 (br, 30H), 4.00 - 3.68 (br, 30H), 3.12 - 2.82 (m, 120H), 1.91 - 0.58 (m, 642H).

The experiments were repeated three times to obtain 3 batches of which the conjugation contents were consistent.

| Batch No. | Production/g | Yield/% |
|---|---|---|
| Batch 1 | 12.13 | 85.0 |
| Batch 2 | 4.253 | 86.7 |
| Batch 3 | 4.371 | 89.1 |

| $^1$H NMR | Integr -ation range | Batch 1 | Batch 1 conjuga -tion content | Batch 2 | Batch 2 conjuga -tion content | Batch 3 | Batch 3 conjuga -tion content | Average conjugateon content |
|---|---|---|---|---|---|---|---|---|
| Cbz - CH2- | 5.16~ 4.80 | 60.2 | 29.1 | 60.4 | 29.2 | 61.2 | 29.6 | 29.3±0.3 |

(continued)

| $^1H$ NMR | Integr -ation range | Batch 1 | Batch 1 conjuga -tion content | Batch 2 | Batch 2 conjuga -tion content | Batch 3 | Batch 3 conjuga -tion content | Average conjugateon content |
|---|---|---|---|---|---|---|---|---|
| Lys-α-CH- | 4.31~ 3.68 | 59.9 | 29.5 | 60.9 | 29.9 | 61.4 | 30.2 | 29.9±0.4 |
| Lys-ε-CH2- | 3.12~ 2.82 | 120.3 | 29.6 | 120.2 | 29.6 | 121.3 | 29.8 | 29.7±0.2 |
| Lys-β, γ,δ -CH2- | 1.91~ 0.58 | 642.2 | 29.9 | 656.5 | 30.5 | 662.7 | 30.8 | 30.4±0.5 |
| Boc-C (CH3) 3 | | | | | | | | |

Synthesis of compound 101B02:

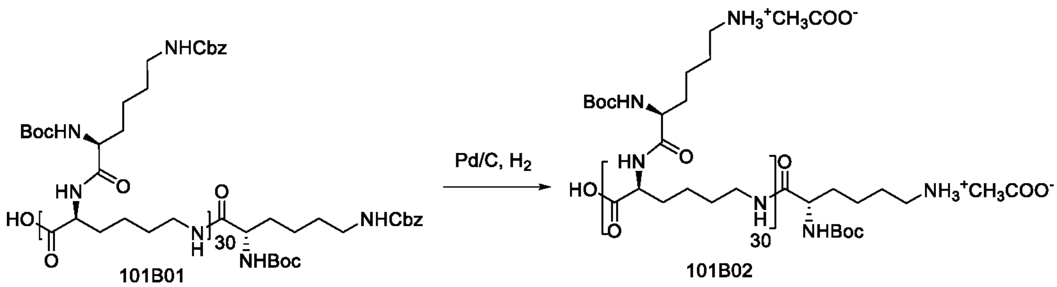


101B01 101B02

α-[Boc-Lys(Cbz)]$_{30}$-ε-PolyLys$_{30}$ (**101B01**) (2.92 g) was dissolved in 30 mL of acetic acid and 30 mL of methanol, and the mixture was heated to promote further dissolution. 591 mg palladium on carbon (10%) was added, and the mixture was stirred at 25°C under hydrogen atmosphere for 18 h. Celite was added, and the mixture was filtered. The filtrate was concentrated, precipitated with MTBE and vacuum-dried to give α-[Boc-Lys(NH3$^+$COO$^-$)]$_{30}$-ε-PolyLys$_{30}$ (**101B02**) as white powder.

$^1H$ NMR (400 MHz, Deuterium Oxide) δ 4.20 - 3.98 (m, 30H), 3.88 (t, $J$ = 7.0 Hz, 31H), 3.04 (s, 61H), 2.87 (t, $J$ = 7.6 Hz, 59H), 1.84 (s, 122H), 1.78 - 1.10 (m, 594H).

Synthesis of compound 101B03:

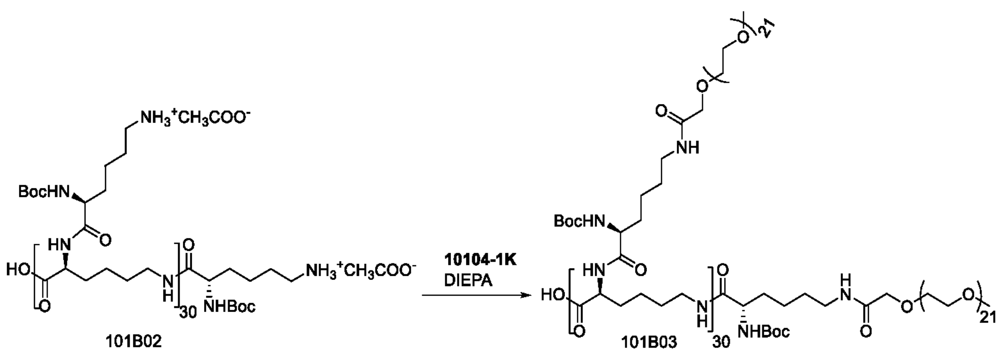


101B02 101B03

**101B02** (2.00 g) was dissolved in 9.12 mL of water, and **10104-1K** (3.42 g) was dissolved in 13.7 mL of acetonitrile. The two solutions were mixed, and 1.19 mL of DIPEA was added. The mixture was stirred overnight at room temperature. The mixture was concentrated, extracted with dichloromethane and dried over anhydrous sodium sulfate to give a product **101B03,** 8.69 g.

$^1H$ NMR (400 MHz, Deuterium Oxide) δ 4.25 (br, 30H), 4.09 (br, 96H), 3.93-3.45 (br, 2746H), 3.41 (br, 92H), 3.36 - 2.90 (m, 121H), 2.20-0.86 (m, 645H).

Synthesis of compound 101B04:

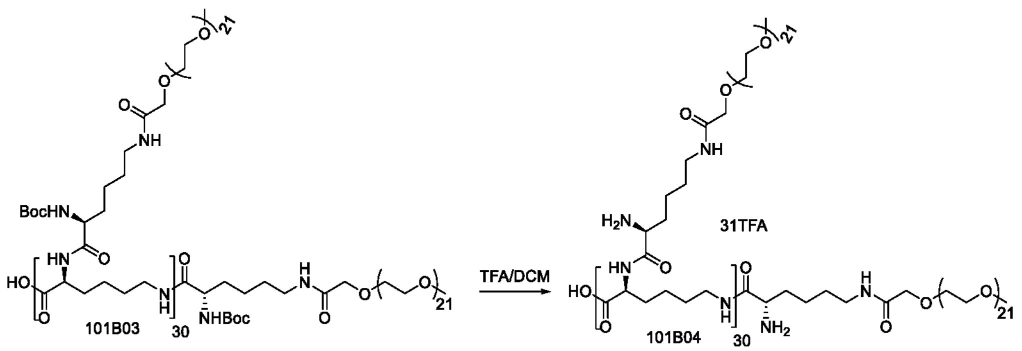


101B03 (8.69 g) was dissolved in 45 mL of dichloromethane, and 22 mL of TFA was added. The mixture was stirred overnight at room temperature under nitrogen protection. The solvent was removed to give transparent viscous **101B04** 6.178 g.

$^1H$ NMR (400 MHz, Deuterium Oxide) δ 4.09 (br, 32H), 3.91 (br, 91H), 3.57 (br, 2658H), 3.25 (br, 89H), 3.20 - 2.89 (m, 120H), 1.93 - 1.05 (m, 353H).

Synthesis of compound 101B05:

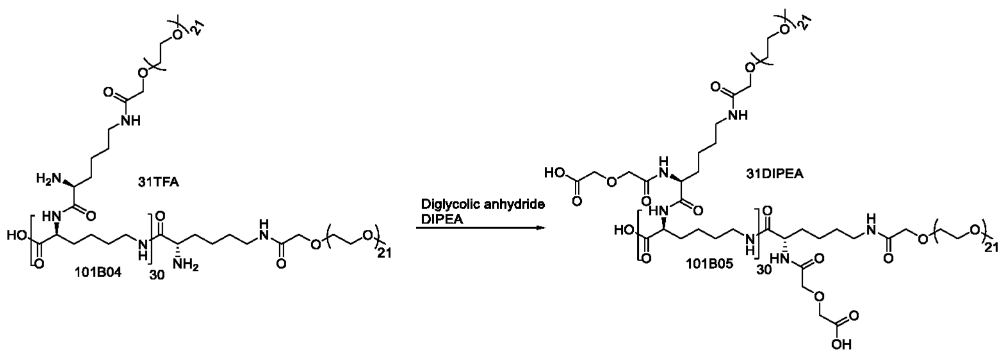


**101B04** (6.178 g) was dissolved in 21 mL of DMF, and DIPEA (3.01 mL) and diglycolic anhydride (1.35 g) were added. The mixture was stirred at room temperature. After 4 h, diglycolic anhydride (338.5 mg) and DIPEA (754 μL) were added, and the mixture was stirred overnight at room temperature. The mixture was precipitated with MTBE, and filtered. The filter cake was vacuum-dried to give a product **101B05.**

Synthesis of compound 101B00A:

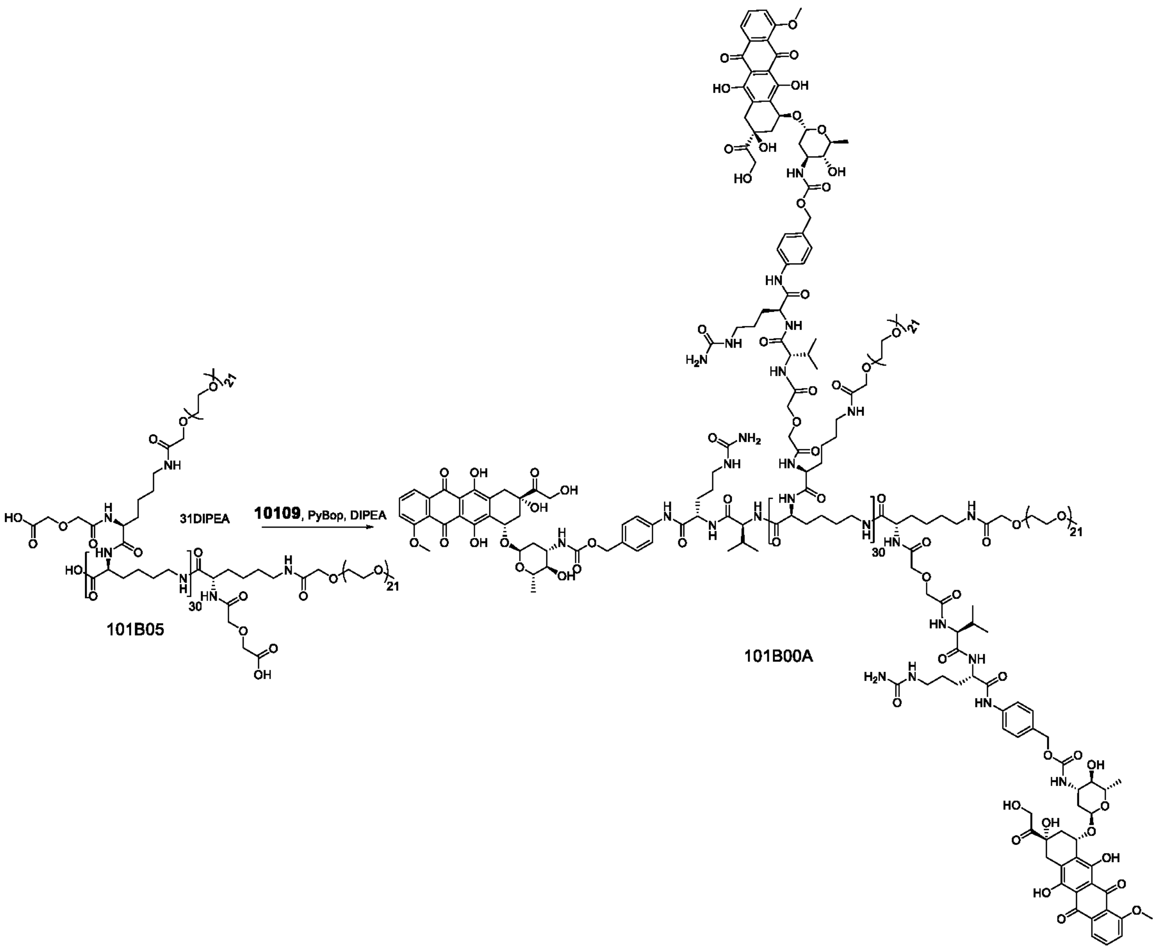


101B05

101B00A

**101B05** (0.230 g) was dissolved in 2 mL of DMF, and **10109** (0.208 g, prepared in example 11), PyBop (229 mg) and DIPEA (0.115 mL) were added. The mixture was stirred overnight at room temperature. The mixture was precipitated with MTBE. The solid was dissolved in 70 mL of methanol, and 30 mL of water was added. The mixture was purified by ultrafiltration, and freeze-dried to give a product (101B00A) 310 mg as red solid, HPLC purity: 98.8%.

Example 16: synthesis of compound 101B00B

Synthesis of compound 101B06:

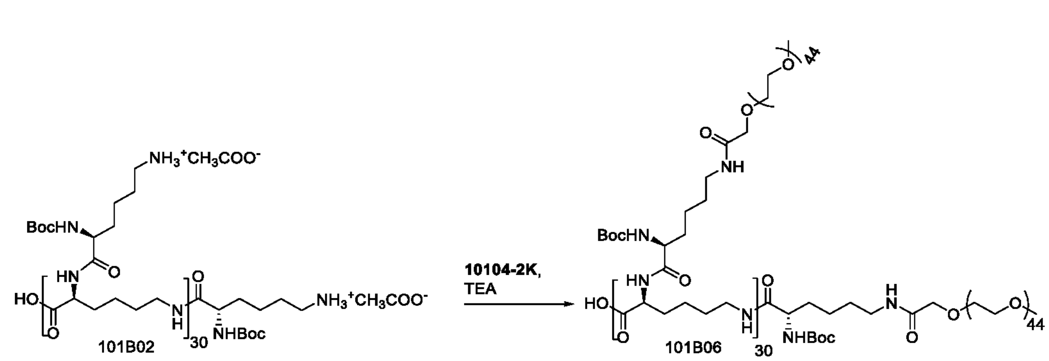


101B02

101B06

α-[Boc-Lys($NH3^+COO^-$)]$_{30}$-ε-PolyLys$_{30}$ (**101B02**) (prepared in example 46) (4.76 g) was dissolved in 20 mL of water, and 1014-2K (10.00 g) was dissolved in 20 mL of acetonitrile. The two solutions were mixed, and triethylamine (1.39 mL) was added. The mixture was stirred for 10 min, 1014-2K (20.00 g) in acetonitrile (40 mL) and triethylamine

(2.79 mL) were added. The mixture was stirred at 25°C for 4 h. Water was added, and the mixture was purified by ultrafiltration. The filtrate was concentrated and freeze-dried to give a product α-[Boc-Lys(mPEG$_{2k}$)]$_{30}$-ε-PolyLys$_{30}$ as white powder (**101B06**), 18.4 g.

$^1$H NMR (400 MHz, Deuterium Oxide) δ 4.25 (s, 32H), 4.09 (s, 95H), 3.93-3.45 (m, 5537H), 3.41 (s, 95H), 3.36 - 2.90 (m, 126H), 2.20-0.86 (m, 645H).

Synthesis of compound 101B07:

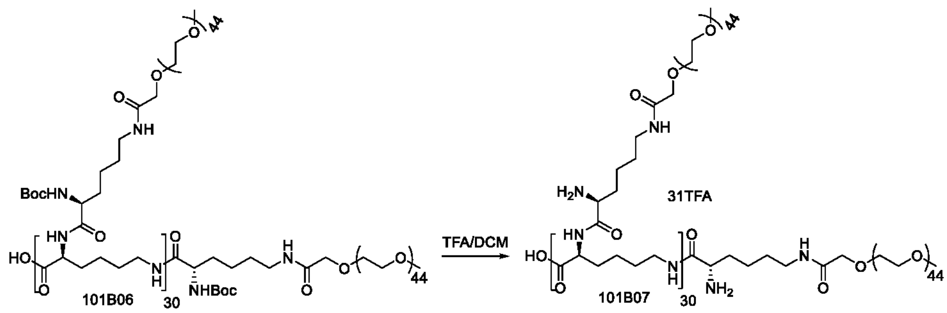


**101B06** (9.0 g) was dissolved in dichloromethane (90 mL), and TFA (30 mL) was added. The mixture was stirred at room temperature for 6 h. Removal of solvent and vaccum-drying gave 8.84 g **101B07** as white solid (97.6%).

$^1$H NMR (400 MHz, Deuterium Oxide) δ 4.23 (br, 35H), 4.04 (br, 94H), 3.70 (br, 5255H), 3.38 (br, 92H), 3.25 (m, 128H), 2.10 - 1.11 (m, 376H).

Synthesis of compound 101B08:

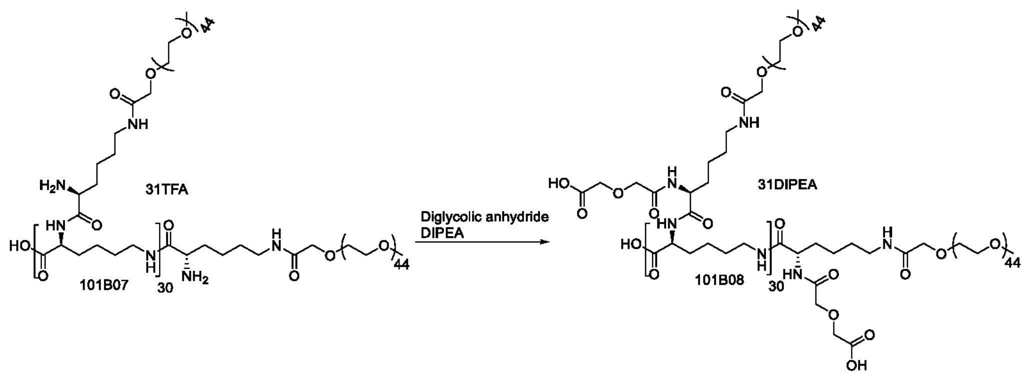


**101B07** (1.50 g) was dissolved in 6 mL of DMF, and DIPEA (0.66 mL) and diglycolic anhydride (0.25 g) were added. The mixture was stirred at room temperature. After 2 h, diglycolic anhydride (189 mg) and DIPEA (528 μL) were added, and the mixture was stirred overnight at room temperature. The mixture was precipitated with MTBE, and filtered. The filter cake was vacuum-dried to give **101B08** as white solid (DIPEA salt).

Synthesis of compound 101B00B:

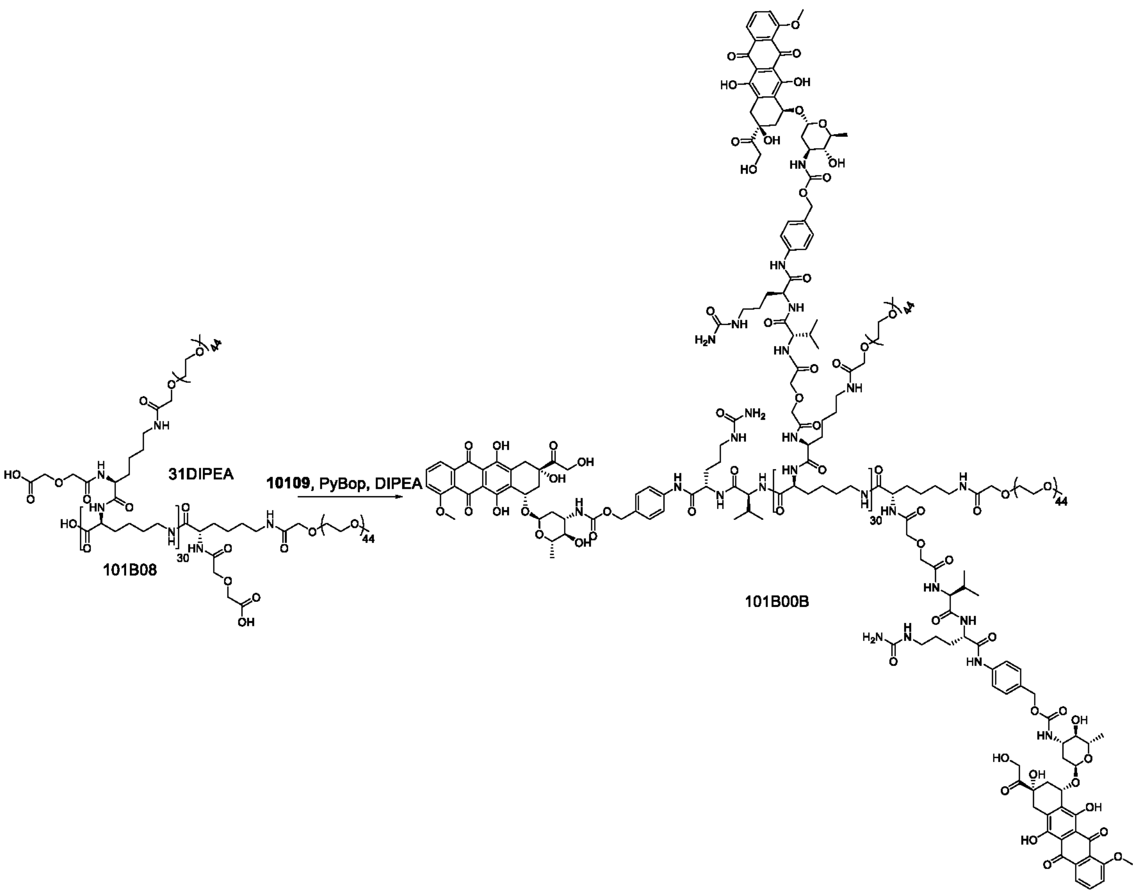


101B08

101B00B

**101B08** (0.43 g), **10109** (0.24 g) and PyBop (0.13 g) were dissolved in 4 mL of DMF, and DIPEA (0.13 mL) was added. The mixture was stirred overnight at room temperature. The reaction solution was precipitated with 30 mL of MTBE. The precipitate was vacuum-dried, and dissolved in 70 mL of methanol, and 30 mL of water was added. The mixture was purified by ultrafiltration, and freeze-dried to give a product (**101B00B**) as red solid, 480 mg, HPLC purity: 98.0%.

Example 17: determination of DOX conjugation content in polymers 101A1601, 101A1602, 101B00A and 101B00B

Papain was weighed and added to PBS to prepare 6.25 mg/mL PBS solution.

Preparation of polymer solutions

101A1601, 101A1602, 101B00A and 101B00B were weighed according to the table below and placed into a round bottom flask of known weights. 2 mL of methanol was added to dissolve the polymer. Water was added, and the methanol was removed. The weight of the remaning liquid was measured according to decrement method. More water was added to dilute the concentration of the polymer to 25 mg/mL.

| Compound No. | Weight/mg | Remaning liquid/g | Water added/mL | Concentration of polymer mg/g |
|---|---|---|---|---|
| 101B00A | 118.5 | 2.8620 | 1.8780 | 25 |
| 101B00B | 138.2 | 3.2321 | 2.2979 | 25 |
| 101A1601 | 108.4 | 2.3400 | 1.9950 | 25 |
| 101A1602 | 142.5 | 4.0101 | 1.6899 | 25 |

0.20 mL of polymer solution and 1.80 mL of papain PBS solution (having a final concentration of polymer of 2.50 mg/mL) were shaken in water bath at 37°C for 72 h. The solution was cooled to room temperature. 0.10 mL of

solution was pipetted, and 0.90 mL of DMSO was added. The peak area of DOX after enzymatic release was characterized by HPLC, and the DOX conjugation content (based on DOX·HCl) was calculated from a DOX·HCl standard curve.

Among them, 101B00B, 101A1601 and 101A1602 did not achieve full enzymatic release at 72 h, and the DOX conjugation contents of these three polymers were calculated with 101B00A as the standard sample.

| Compound No. | DOX·HCl wt.% |
|---|---|
| 101A1601 | 9.5 |
| 101B00A | 8.6 |
| 101A1602 | 7.1 |
| 101B00B | 5.9 |

Example 18: synthesis of compound 101B00C

Synthesis of compound 10110:

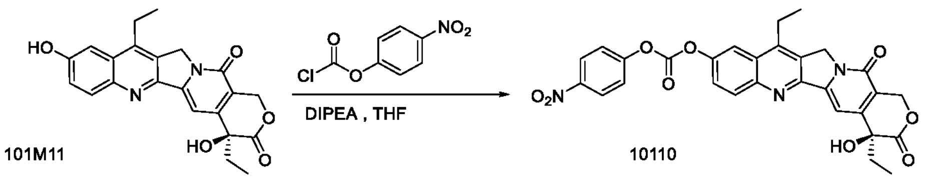


SN-38 (28.87 g) was added to a 2 L round bottom flask, 840 mL of THF was added to obtain a yellow suspension. The reaction mixture was cooled in ice bath for 10 min under nitrogen protection. *p*-Nitrophenyl chloroformate (18.09 g) was added, and DIPEA (20.12 mL) was added dropwisely. The ice bath was removed, and the reaction mixture was stirred at room temperature for 2 h. The THF was removed in vacuo, and 500 mL of ethyl acetate was added and slurred for 2 h. The solid was filtered and vacuum-dried to give light yellow powder pNP-10-O-SN-38 (10110) 42.35 g. LCMS (m/z): $[M+H]^+$ = 557.8.

Synthesis of compound 10111:

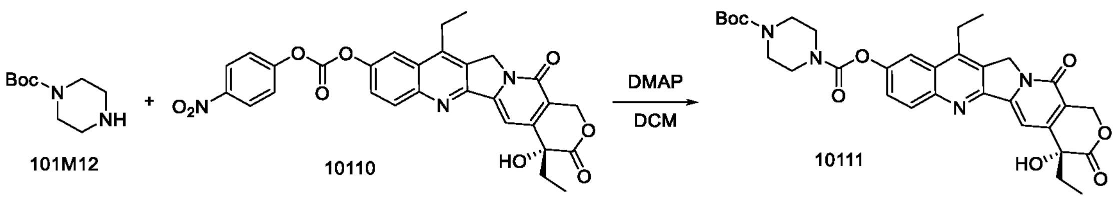


180 mL of dichloromethane was added to pNP-10-O-SN-38 (**10110**) (41.01 g) to obtain a yellow paste, and N-Boc-piperazine in dichloromethane (27.40 g/90 mL) was added. The reaction mixture was cooled in ice bath for 10 min. DMAP (8.99 g) was added in batches, and the mixture was stirred overnight at room temperature for 15 h. The dichloromethane was removed in vacuo, and 1100 mL of MTBE and 180 mL of methanol were added. The precipitate was filtered and vacuum-dried to give a product *N*-Boc-piperazine-10-O-SN-3 8 (**10111**) 33.89 g, 87.2% yield. LCMS (m/z): $[M+H]^+$ = 604.9.

Synthesis of compound 10112:

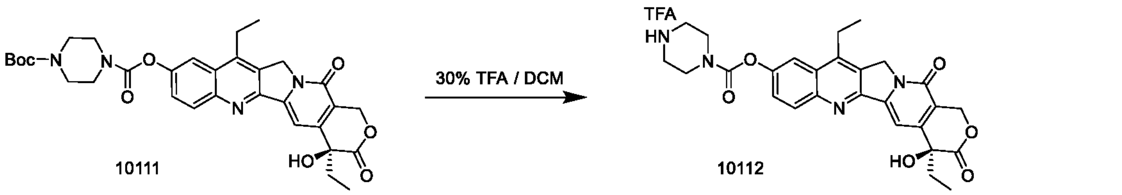


*N*-Boc-piperazine-10-O-SN-38 (**10111**) (38.79 g) was dissolved in 150 mL of dichloromethane, and 130 mL of TFA was added. The mixture was stirred at room temperature for 3 h and was concentrated in vacuo. 1.4 L of MTBE was added, and the precipitate was filtered and vacuum-dried to give a product TFA-piperazine-10-O-SN-38 (10112) 37.99 g, 95.7% yield. LCMS (m/z): $[M+H]^+$ = 504.9; $[M+2H]^+/2$ = 253.0.

Synthesis of compound 10113:

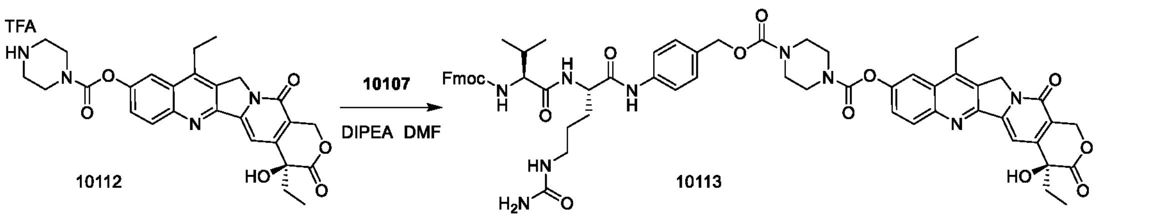


TFA-piperazine-10-O-SN-38 (**10112**) (2.71 g) and Fmoc-Val-Cit-PABC-pNP (10107) (3.36 g) were dissolved in DMF (34 mL). DIPEA (2.29 mL) was added, and the reaction mixture was stirred for 3 h at room temperature. 40 mL of MTBE was added, and the precipitate was filtered and vacuum-dried to give a product Fmoc-Val-Cit-PABC-piperazine-10-O-SN-38 (**10113**) 4.71 g, 95.0% yield. LCMS (m/z): $[M+H]^+$ = 1131.8; $[M+2H]^+/2$ =566.4.

Synthesis of compound 10114:

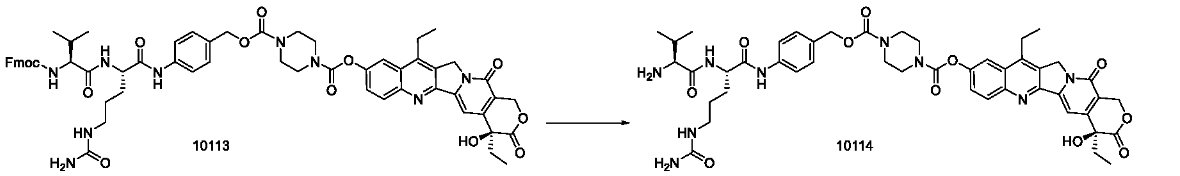


Fmoc-Val-Cit-PABC-piperazine-10-O-SN-38 (**10113**) (4.71 g) was dissolved in 10% piperidine/DMF (32 mL). The mixture was stirred at room temperature for 10 min. 210 mL of MTBE was added, and the precipitate was filtered to obtain light yellow filter cake. The filter cake was vacuum-dried to give a product **10114** 3.58 g, 94.6% yield. LCMS (m/z): $[M+H]^+$ =909.9; $[M+2H]^+/2$ =455.6.

Synthesis of compound 101B00C:

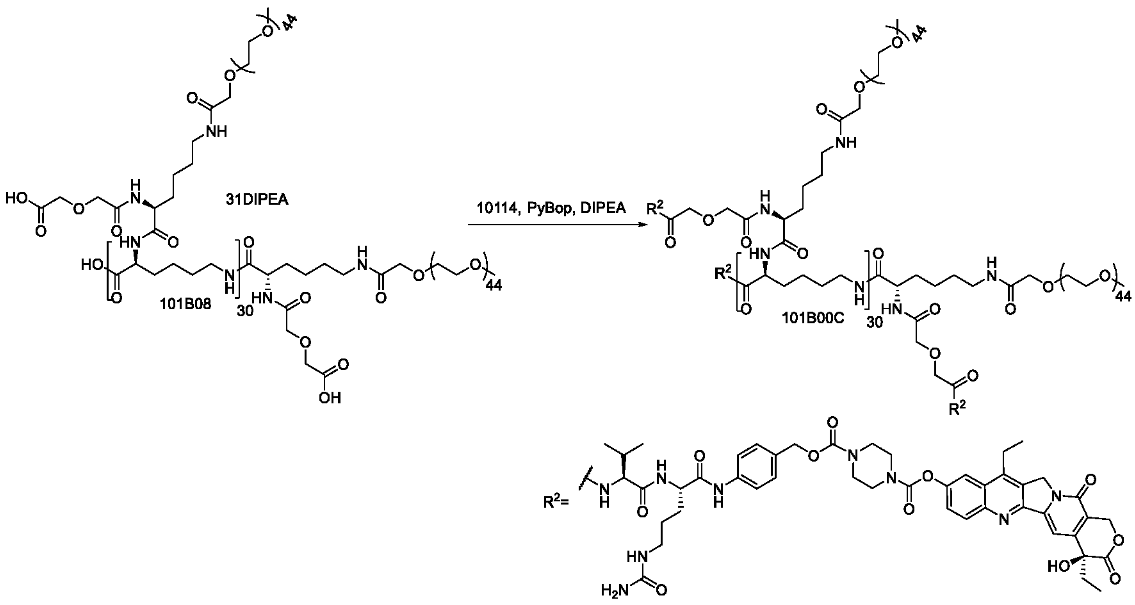


101B08 (0.423 g) and 10114 (0.231 g) were dissolved in 4 mL of DMF, and 0.176 g PyBop and 0.133 mL of DIPEA were added. The reaction mixture was stirred at room temperature for 6 h. 30 mL of MTBE was added, and the precipitate was centrifuged and dissolved in 3 mL of methanol/3 mL of DCM. The solution was precipitated with 30 mL of MTBE, and the precipitate was centrifuged and vacuum-dried to give a 101B00C crude product. The crude product was dissolved in 4 mL of DMSO. The solution was stirred for further dissolution, and diluted with 6 mL of methanol. The mixture was filtered through a 0.45 μm membrane. 10 mL of water was added, and the mixture was purified by ultrafiltration and diluted with 50% ACN/$H_2O$ each time. 392 mg solid product was obtained after freeze drying. HPLC purity: 95%.

Example 19: synthesis of compound 101B00D

Synthesis of compound 10117-2k:

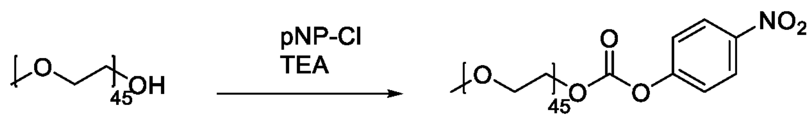


**101M04** **10117-2K**

To mPEG (50.0 g, number average molecular weight is approximately 2000) in 250 mL of DCM, *p*-nitrophenyl chloroformate (pNP-Cl, 15.12 g) and triethylamine (11.9 mL) were added. The mixture was stirred overnight under nitrogen atmosphere. The reaction solution was concentrated in vacuo, and 250 mL of MTBE was added, finally 300 mL. The precipitate was filtered and vacuum-dried to give a product (**10117-2k**), 46.89 g, gray-white, 86.6% yield.

$^1$H NMR (400 MHz, Chloroform-*d*) δ 8.28 (d, *J* = 9.2 Hz, 2H), 7.40 (d, *J* = 9.2 Hz, 2H), 4.43 (s, 2H), 3.64 (s, 172H), 3.38 (s, 3H).

Synthesis of compound 101B09:

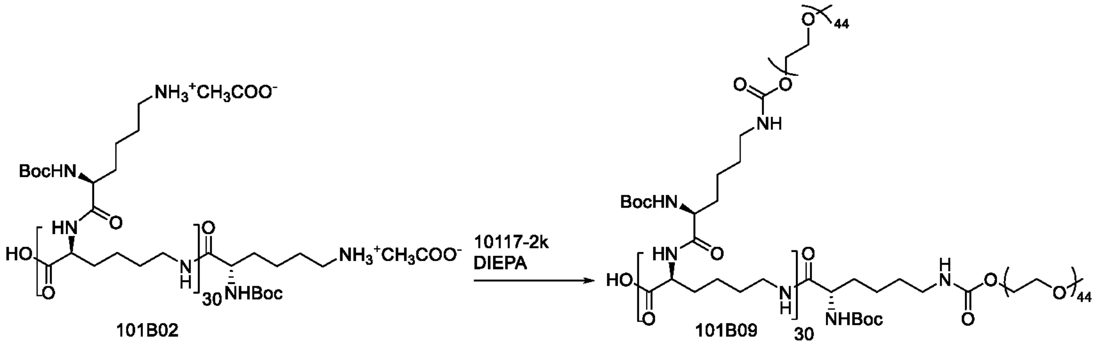


101B02 101B09

**101B02** (0.32 g) was dissolved in 1.44 mL of water, and 10117-2k (2.88 g) was dissolved in 4.15 mL of acetonitrile. After the two solutions were mixed, 0.50 mL of DIPEA was added, and the reaction was stirred overnight at room temperature. Water was added to the reaction solution, and the mixture was extracted with DCM, dried over anhydrous magnesium sulfate, and filerted. The filtrate was concentrated and precipitated with MTBE. The solid product was vacuum-dried to give 101B09 1.51 g, light yellow solid (87.5%).

Synthesis of compound 101B10:

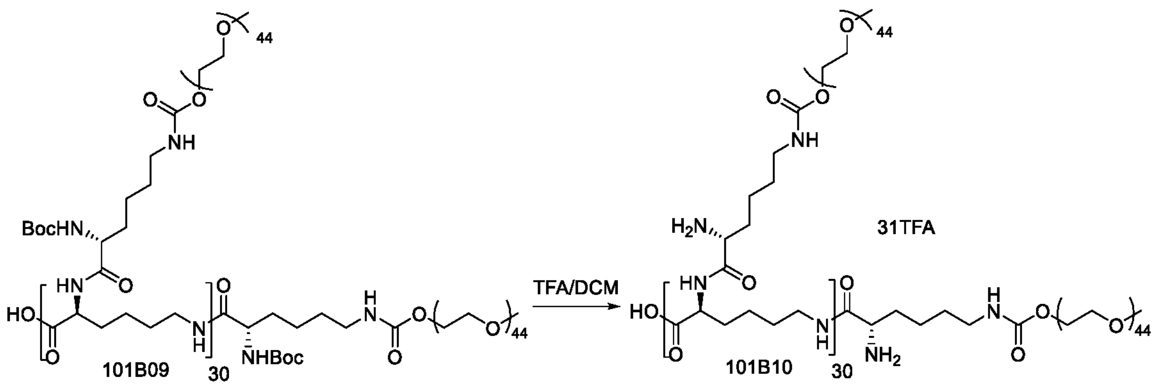


101B09 101B10

101B09 (1.51 g) was dissolved in 20 mL of DCM, and TFA (10 mL) was added. The mixture was stirred overnight at room temperature. The mixture was concentrated until most of the solvent was removed. MTBE was added, and the precipitate was vacuum-dried to give 101B10 which was directly used in the next step.

Synthesis of compound 101B12:

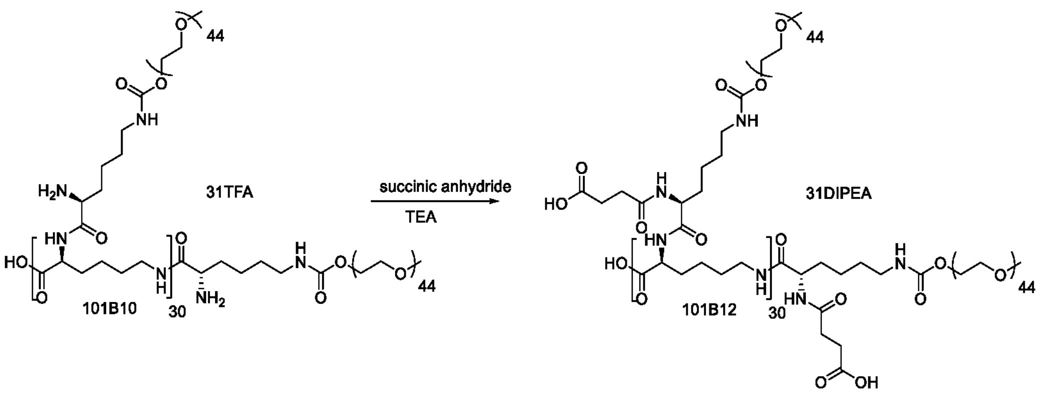


101B10 101B12

**101B10** (5.17 g, 2.13 mmol) was dissolved in 20 mL of DMF. Succinic anhydride (320 mg, 3.2 mmol) and triethylamine (0.89 mL, 6.4 mmol) were added. The reaction mixture was stirred at room temperature for 4 h, and MTBE was added. The precipitate was centrifuged and vacuum-dried to give **101B12** as white powder 5.01 g (94%).

Synthesis of compound 101B00D:

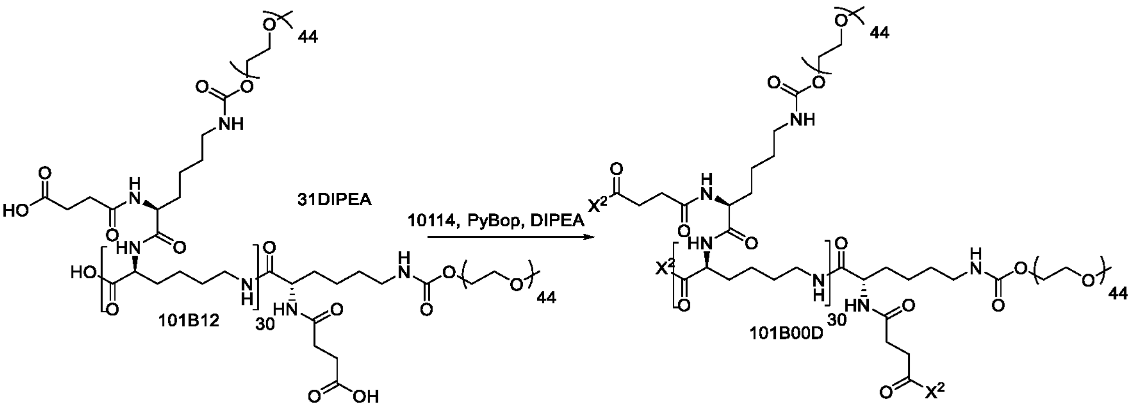


101B12 31DIPEA 101B00D

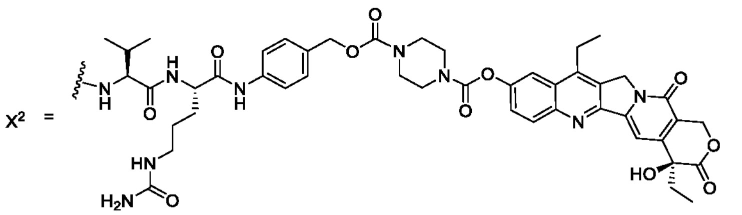

0.709 g of 101B12 and 0.386 g of 10114 were dissolved in 6 mL of DMF, and 0.220 g of PyBop and 0.221 mL of DIPEA were added. The reaction mixture was stirred overnight at room temperature. 40 mL of MTBE was added, and the precipitate was centrifuged. A small amount of precipitate was dissolved with methanol/water, and suspended in 3 mL of methanol/3 mL of DCM. The solution was further precipitated with MTBE, and the precipitate was centrifuged. A small amount of precipitate was dissolved with methanol. The precipitate was vacuum-dried to give a crude product.

The crude product was dissolved in acetonitrile/water (1:1) solution, filtered through a 0.45 μm membrane and ultrafiltered at 30 kDa, to obtain 101B00D with purity 98.3% increased from former 72.4% (210 nm).

Example 20: synthesis of compound 101B00E

Synthesis of compound 101B13:

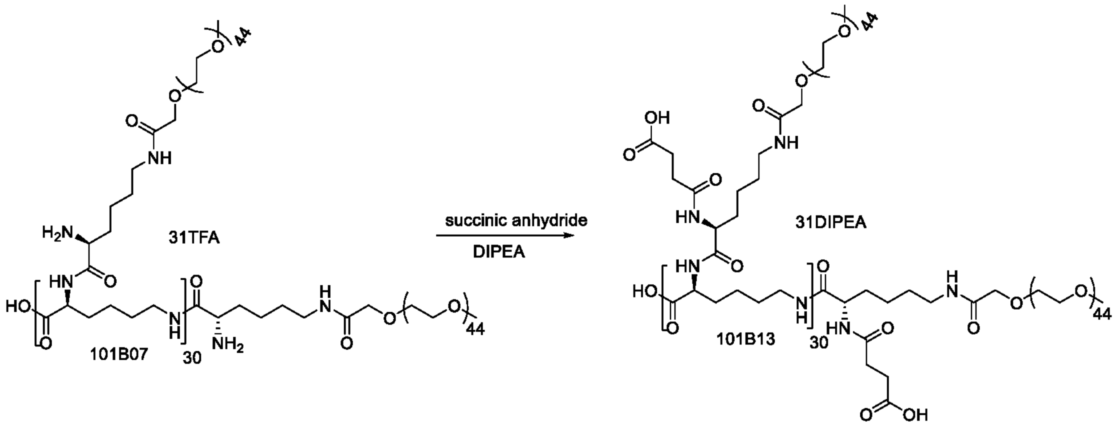


101B07 31TFA 101B13 31DIPEA

101B07 (27.37 g) was dissolved in 104 mL of DMF, succinic anhydride (2.373 g) and DIPEA (8.174 mL) were added. The reaction mixture was stirred at room temperature for 4 h. A small amount of solution was precipitated with MTBE, and ninhydrin test at 100°C for 3 minutes showed colorlessness. The mixture was precipitated with 300 mL of MTBE to obtain a viscous substance. The viscous substance was centrifuged, and GPC characterization indicated no product in the diluted supernatant. The viscous substance was dissolved in 100 mL of ACN. 350 mL of MTBE was slowly added to precipitate the product, and the solution was removed. The colloidal precipitate was dissolved in 100 mL of ACN, and 400 mL of MTBE was slowly added to precipitate the product. The precipitate was filtered and vacuum-dried to give a product 21.840 g (8.7 mmol, 76%). Succinic acid content was measured to be 218 ppm.

$^1H$ NMR (400 MHz, Deuterium Oxide) δ 4.16 (br, 61H), 4.00 (br, 63H), 3.64 (br, 5113H), 3.32 (br, 91H), 3.27 - 2.84 (m, 124H), 2.80 - 2.30 (br, 127H), 2.14 - 0.71 (br, 366H).

Synthesis of compound 101B00E:

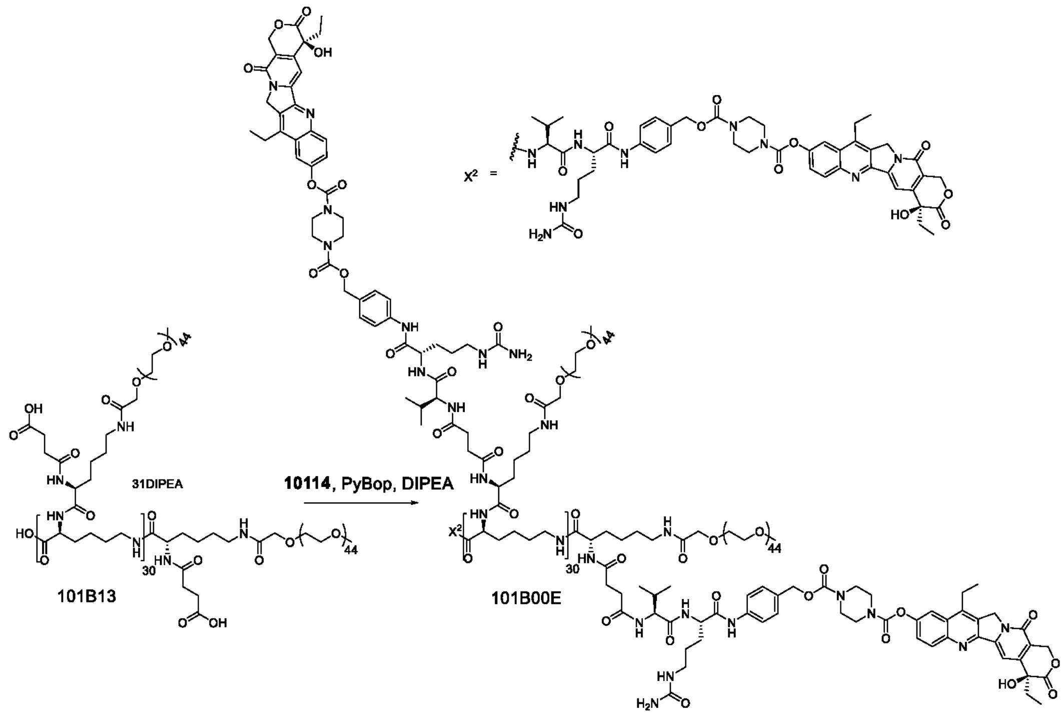


101B13

101B00E

**101B13** (4.873 g, 1.94 mmol) was dissolved in 40 mL of DMF, and 10114 (2.648 g, 2.91 mmol), PyBop (2.027 g, 3.88 mmol) and DIPEA (1.52 mL, 8.74 mmol) were added. The reaction was stirred at room temperature for 4.5 h. MTBE was added, and the precipitate was dissolved in MeOH/water (50/50, v/v) and purified by ultrafiltration. The concentrated solution was freeze-dried to give a final product as light yellow cotton-like solid, **101B00E**, 5.51 g.

**HPLC analytical method D:** 101B00E: RT=12.602 min

Determination of SN-38 conjugation content in compound 101B00E

Papain was weighed and added to PBS to prepare 10.0 mg/mL PBS solution.

38.39 mg of freeze-dried product 101B00E (ε-PolyLys$_{30}$-[Lys(α-SA-VC-PABC-piperazine-10-O-SN-38, ε-mPEG$_{2k}$)]$_{30}$) was weighed, and 3.839 mL of deionized water was added to obtain 10.0 mg/mL polymer solution.

0.500 mL of polymer solution and 0.500 mL of papain PBS solution (having a final concentration of polymer of 5.00 mg/mL) were shaken in water bath at 37°C for 2 h. The solution was cooled to room temperature. 0.200 mL of solution was pipetted, and 1.800 mL of DMSO was added. The peak area of piperazine-10-O-SN-38 after enzymatic release was characterized by HPLC, and the concentration of TFA-piperazine-10-O-SN-38 was calculated to be 841.1 μg/mL from a standard curve of TFA-piperazine-10-O-SN-38, which was equivalent to a conclusion that the concentration of SN-38 was 506.4 μg/mL after the enzymatic release of 5.00 mg/mL of polymer, and therefore, the SN-38 conjugation content in ε-PolyLys$_{30}$-[Lys(α-SA-VC-PABC-piperazine-10-O-SN-38, ε-mPEG$_{2k}$)]$_{30}$ was calculated to be 10.1 wt.%.

Example 21: synthesis of compound 101B00G

Synthesis of compound 10111A:

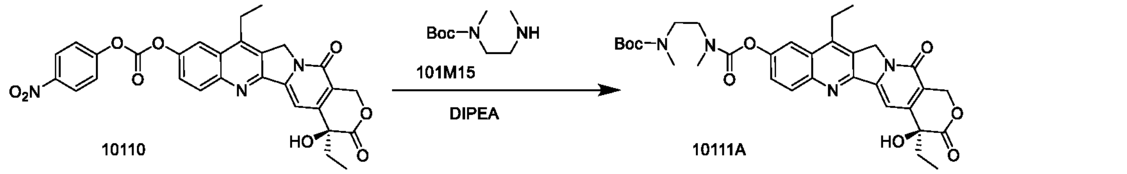


pNP-10-O-SN-38 (1.30 g) was added to dichloromethane (5 mL) to obtain a yellow paste, Boc-DMEDA (**101M15**) (0.87 g) and DIPEA (406 μL) were added. The mixture gradually dissolved and became light brown, and the solvent was removed after 3 h. Column purification gave a product Boc-DMEDA-10-O-SN-38 (**10111A**) 1.0749 g (76.0%). LCMS (m/z): $[M+H]^+$ = 607.2

$^1$HNMR (400 MHz, $CDCl_3$): δ=8.22 (d, 1H, J=8.8Hz), 7.90-7.84 (m, 1H), 7.64 (s, 1H), 7.61-7.59 (m, 1H), 5.75 (d, 1H, J=16.4Hz), 5.33-5.26 (m, 4H), 3.75 (s, 1H), 3.65-3.54 (m, 3H), 3.26-2.78 (m, 9H), 1.94-1.85 (m, 2H), 1.48-1.40 (m, 10H), 1.28-1.25 (m, 3H), 1.06-1.02 (m, 3H).

Synthesis of compound 10112A:

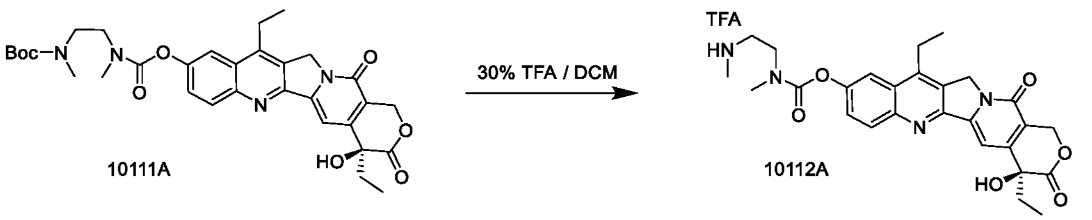


Boc-DMEDA-10-0-SN-38 (0.7429 g) was dissolved in 5 mL of dichloromethane, and 2 mL of TFA was added. The mixture was stirred at room temperature for 1 h. The mixture was concentrated, and crude product TFA-DMEDA-10-O-SN-38 (**10112A**) was directly used for the next reaction. LCMS (m/z): $[M+H]^+$ = 507.1

Synthesis of compound 10113A:

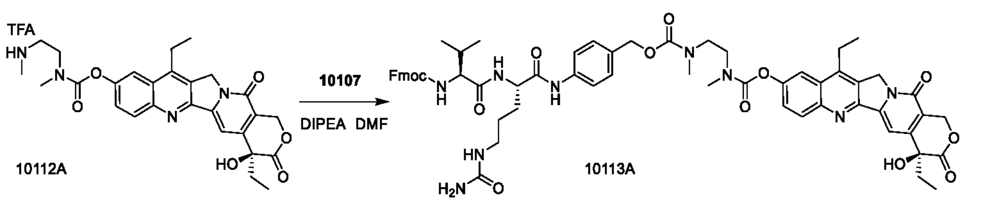


TFA-DMEDA-10-O-SN-38 (**10112A**) (732.5 mg) and Fmoc-Val-Cit-PABC-pNP (10107) (905.1 mg) were dissolved in DMF (9 mL), and DIPEA (620 μL) was added. The mixture was stirred at room temperature for 3 h. 90 mL of MTBE was added, the precipitate was centrifuged and vacuum-dried to give a product Fmoc-Val-Cit-PABC-DMEDA-10-O-SN-38 (**10113A**) 1.1213 g (83.8%). LCMS (m/z): $[M+2H]^+/2$ = 567.75

Synthesis of compound 10114A:

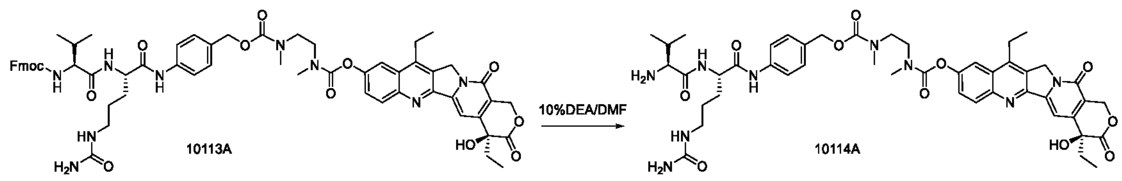


Fmoc-Val-Cit-PABC-DMEDA-10-O-SN-38 (**10113A**) (1.1213 g) was added to a 10 mL round bottom flask, and 5 mL of DMF was added for dissolution before 0.56 mL of DEA was added. The mixture was stirred for 20 min. 40 mL of MTBE was added, and the precipitate was centrifuged. The precipitate was dissolved with 4 mL of MeOH, and 40 mL of MTBE was added. The precipitate was centrifuged to give light yellow solid $NH_2$-Val-Cit-PABC-DMEDA-10-O-SN-38 (**10114A**) 0.7128 g, 79.1% yield. LCMS (m/z): $[M+H]^+ = 912$; $[M+2H]^+/2 = 456.7$.

Synthesis of compound 101B00G:

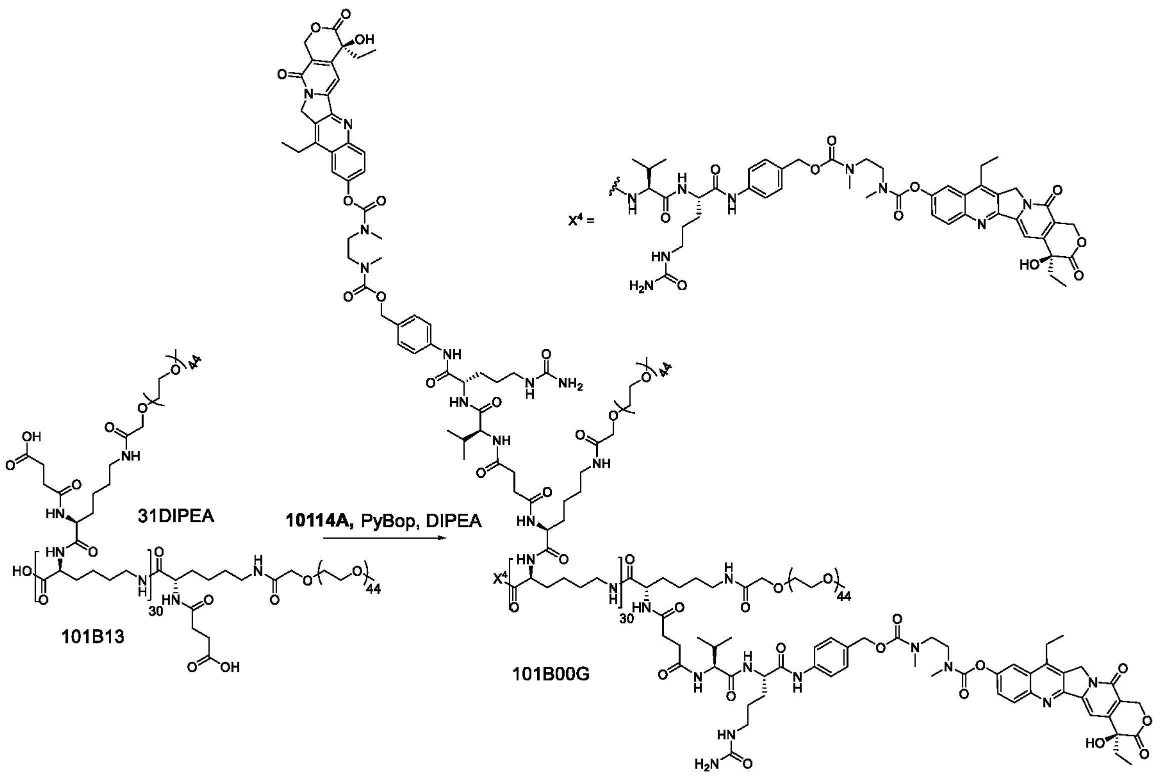


**101B13** (0.705 g), **10114A** (0.384 g) and PyBop (0.293 g) were dissolved in 6 mL of DMF, and DIPEA was added. The reaction mixture was stirred for 4 h at room temperature. MTBE was added, and the precipitate was dissolved in 60 mL of methanol. 70 mL of water was added. The precipitate was purified by ultrafiltration and freeze-dried to give a final product as light yellow cotton-like solid, 101B00G, 0.832 g.

HPLC analytical method D, RT=13.824 min.

$^1$H NMR (400 MHz, DMSO-$d_6$) δ 8.12 (br, 62H), 7.89 (br, 66H), 7.61 (br, 132H), 7.28 (br, 98H), 5.35 (br, 112H), 4.98 (br, 70H), 4.26 (br, 150H), 3.84 (br, 77H), 3.50 (br, 6237H), 3.23 (br, 90H), 3.18 - 2.74 (m, 374H), 1.34 (m, 899H).

Determination of SN-38 conjugation content in compound 101B00G

Papain was weighed and added to PBS to prepare 10.0 mg/mL PBS solution.

35.12 mg of freeze-dried product ε-$PolyLys_{30}$-[Lys(a-SA-VC-PABC-DMEDA-10-O-SN-38, ε-$mPEG_{2k}$)]$_{30}$ (101B00G) was weighed, and 3.510 mL of deionized water was added to obtain 10.0 mg/mL polymer solution.

0.50 mL of polymer solution and 0.50 mL of papain PBS solution (having a final concentration of polymer of 5.00 mg/mL) were mixed and shaken in water bath at 37°C for 2 h. The solution was cooled to room temperature. 0.20 mL of solution was pipetted, and 1.800 mL of DMSO was added. Based on HPLC characterization, the concentration of SN-38 after enzymatic release was calculated from a standard curve of SN-38. The calculated concentration of SN-38 after the enzymatic release of 5.00 mg/mL of polymer was 472.8 μg/mL. The SN-38 conjugation content in **101B00G** was calculated to be 9.5 wt.%.

Example 22: synthesis of compound 101B00H

Synthesis of compound 10111B:

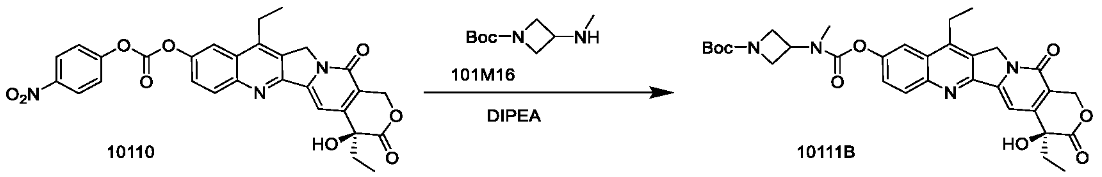


pNP-10-O-SN-38 (**10110**) (1.30 g) was dissolved in dichloromethane (5 mL). 1-BOC-3-methylaminoazetidine (**101M16**) (0.87 g) and DIPEA (406 μL) were added, and the reaction mixture was stirred at room temperature for 3.5 h. The mixture was concentrated, and 5 mL of MeOH and 80 mL of MTBE were added. The precipitate was centrifuged and vacuum-dried to give 1-Boc-Az-10-O-SN-38 (**10111B**) 1.24 g, 88.0% yield. LCMS (m/z): $[M+H]^+$ = 605.

Synthesis of compound 10112B:

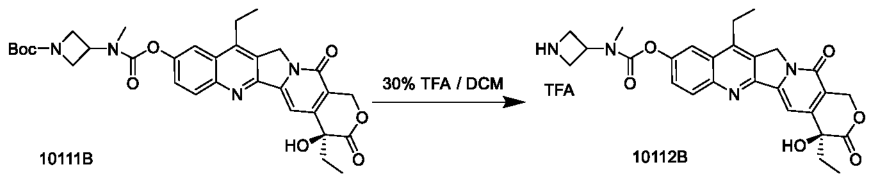


1- Boc-Az-10-O-SN-38 (**10111B**) (1.00 g) was dissolved in 10 mL of dichloromethane. 3 mL of TFA was added, and the reaction mixture was stirred for 1 h at room temperature. The mixture was concentrated, and 80 mL of MTBE was added. The precipitate was centrifuged and vacuum-dried to give Az-10-O-SN-38 (**10112B**) 0.97 g, 94.4% yield. LCMS (m/z): $[M+H]^+$ =505.1.

Synthesis of compound 10113B:

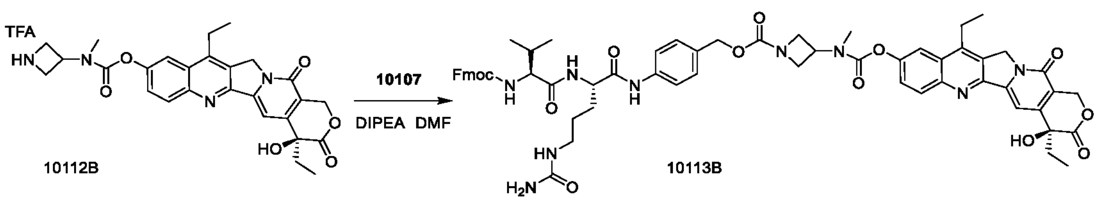


Az-10-O-SN-38 (**10112B**) (966.6 mg) and Fmoc-Val-Cit-PABC-pNP (**10107**) (1.1743) were dissolved in 12 mL of DMF, and DIPEA (816 μL) was added. The mixture was stirred at room temperature for 3 h. 80 mL of MTBE was added. The precipitate was centrifuged and vacuum-dried to give Fmoc-Val-Cit-PABC-Az-10-O-SN-38 (10113B) 1.60 g, 90.7% yield. LCMS (m/z): $[M+2H]^+/2$ =566.4.

Synthesis of compound 10114B:

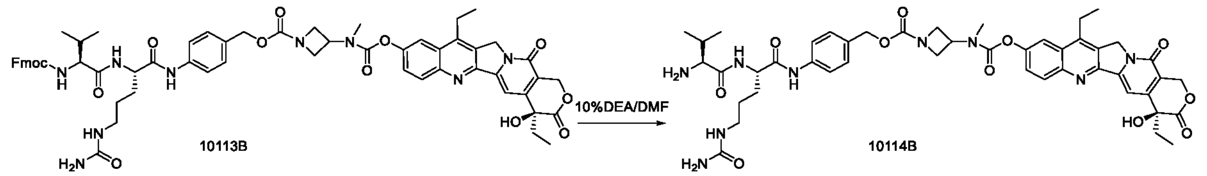


Fmoc-Val-Cit-PABC-Az-10-O-SN-38 (**10113B**) (1.6040 g) was dissolved in 10 mL of DMF before 1.2 mL of DEA was added. The mixture was stirred for 15 min. 80 mL of MTBE was added. The precipitate was centrifuged and vacuum-dried to give $NH_2$-Val-Cit-PABC-Az-10-O-SN-38 (**10114B**) 1.2630 g, 97.9% yield. LCMS (m/z): $[M+H]^+ = 910.2$; $[M+2H]^+/2 = 455.7$.

Synthesis of compound 101B00H:

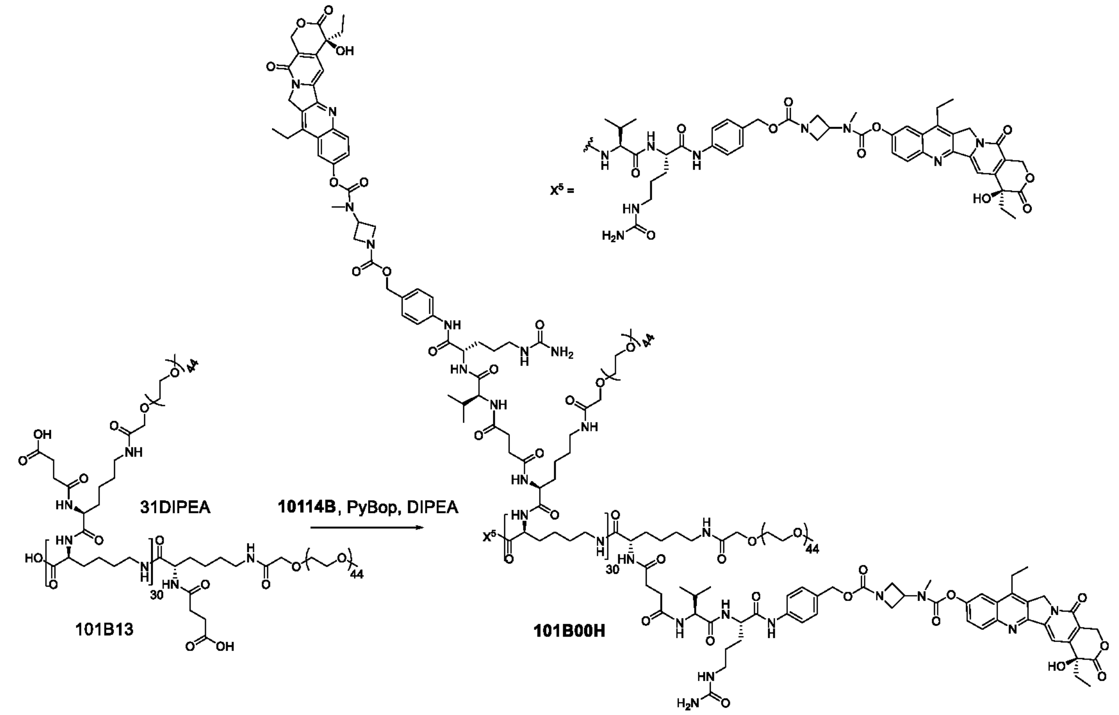


**101B13** (0.70 g), **10114B** (0.38 g) and PyBop (0.29 g) were dissolved in 6 mL of DMF, and DIPEA (0.22 mL) was added. The reaction mixture was stirred at room temperature for 3 h. HPLC showed the complete reaction of starting materials. The reaction solution was precipitated with MTBE. The crude product was dissolved in 5 mL of DMSO, 5 mL of methanol and 5 mL of water, purified by ultrafiltration, and freeze-dried to give a final product as transparent light brown solid, **101B00H**, 881 mg, HPLC purity 95.3%. HPLC analytical method D, RT=13.716 min.

Determination of SN-38 conjugation content in compound 101B00H

Papain was weighed and added to PBS to prepare 10.0 mg/mL PBS solution.

29.77 mg of freeze-dried product ε-PolyLys$_{30}$-[Lys(α-SA-VC-PABC-Az-10-O-SN-38, ε-mPEG$_{2k}$)]$_{30}$ (101B00H) was weighed, and 0.149 mL of DMSO was added. The mixture was heated to promote dissolution. 2.83 mL of deionized water was added to obtain 10.0 mg/mL polymer solution.

0.50 mL of polymer solution and 0.50 mL of papain PBS solution (having a final concentration of polymer of 5.00 mg/mL) were mixed, and were shaken in water bath at 37°C for 2 h. The solution was cooled to room temperature.

0.20 mL of solution was pipetted, and 1.80 mL of DMSO was added. Based on HPLC characterization, the calculated concentration of 10112B after enzymatic release from a standard curve of 10112B (Az-10-O-SN-38 trifluoroacetate) was 855.2 μg/mL, which was equivalent to a conclusion that the concentration of SN-38 was 514.8 μg/mL. The SN-38 conjugation content in ε-PolyLys$_{30}$-[Lys(α-SA-VC-PABC-Az-10-O-SN-38, ε-mPEG$_{2k}$)]$_{30}$ (101B00H) was calculated to be 10.3 wt.%.

Example 23: synthesis of compound 101B00I

Synthesis of compound 10121:

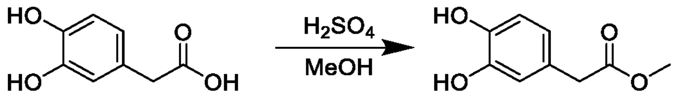


101M17 10121

101M17 (20.0 g) was dissolved in 200 mL of MeOH, and concentrated sulfuric acid (1.17 g) was added. The reaction mixture was refluxed at 75°C for 2 h under nitrogen protection. The reaction solution was cooled to room temperature, concentrated in vacuo. EA was added, and the mixture was washed with NaHCOs solution. The organic phase was dried over anhydrous sodium sulfate, filtered and concentrated to give a product as orange-red viscous oil, 10121, 18.48 g, 85.3% yield.

Synthesis of compound 10122:

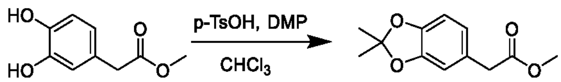


10121 10122

10121 was dissolved in 50 mL of chloroform, and acetone dimethyl acetal (22.04 mL) and *p*-TsOH·$H_2O$ (0.84 g) were added. The reaction mixture was refluxed at 70°C overnight. The chloroform was removed, and 50 mL of DCM was added. The reaction solution was neutralized with 100 mL of $NaHCO_3$ solution, and extracted with DCM. The organic phase was dried over anhydrous sodium sulfate and concentrated. The residue was purified by column chromatography to give product 10122 (2.21 g, 36.2% yield).

Synthesis of compound 10123:

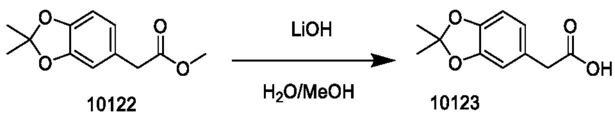


10122 10123

10122 (2.21 g) was dissolved in MeOH. Aqueous solution of LiOH was added, and the mixture was stirred overnight at room temperature. MeOH was removed in vacuo, and pH was adjusted to 5~6 with 1 M HCl. The mixture was extracted with DCM, dried over anhydrous sodium sulfate, concentrated and vacuum-dried to obtain a product as light yellow crystal, 10123, 1.76 g, 84.9% yield.

$^1H$ NMR (400 MHz, Chloroform-*d*) δ 6.70 (d, *J* = 5.7 Hz, 3H), 3.56 (s, 2H), 1.69 (s, 6H).

Synthesis of compound 10124:

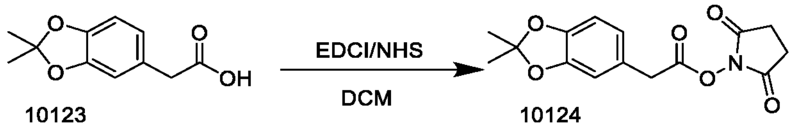


10123 10124

10123 (0.500 g), NHS (0.415 g) and EDCI (0.691 g) was added to 5 mL of DCM. The mixture was stirred for 2 h at room temperature. A small amount of mixture was washed with water and then diluted with acetonitrile. The organic phase was washed with water twice, dried over anhydrous sodium sulfate and concentrated to give a product 10124 (834 mg), which was directly used for the next step. ESI-MS: $[M+NH_4]^+$=323.

Synthesis of compound 101B14:

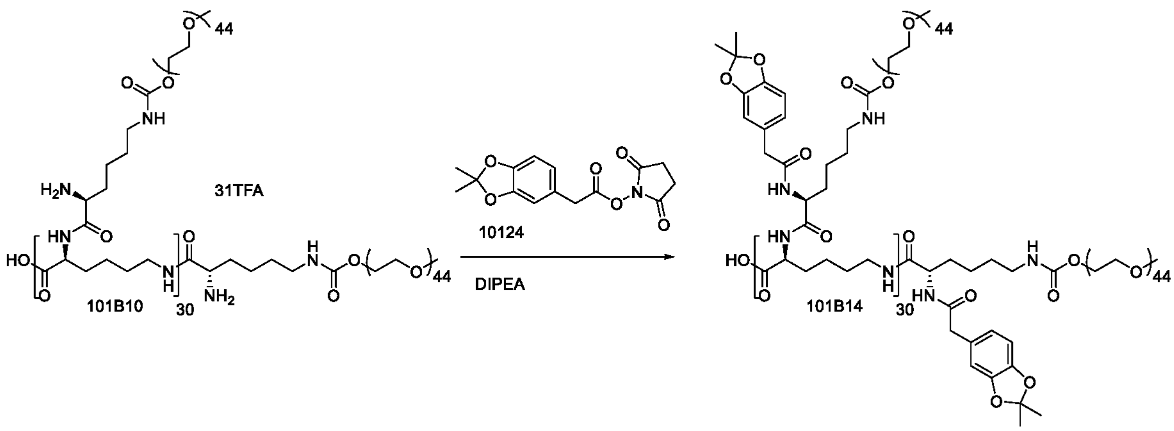


101B10 101B14

101B10 (3.70 g) was dissolved in 8 mL of DMF, and DIPEA (0.83 mL) and 10124 (0.72 g) in 5 mL of DMF were added to the above solution. The mixture was stirred overnight at room temperature. MTBE was added to the reaction solution, and the precipitate was vacuum-dried to give 101B14, 3.17 g, as light yellow solid (83.3%).

$^1$H NMR (400 MHz, DMSO-$d_6$) δ 6.69 (57H), 6.50 (9H), 4.03 (s, 126H), 3.51 (5240H), 3.24 (90H), 2.90 (160H), 1.59 (351H).

Synthesis of compound 101B15:

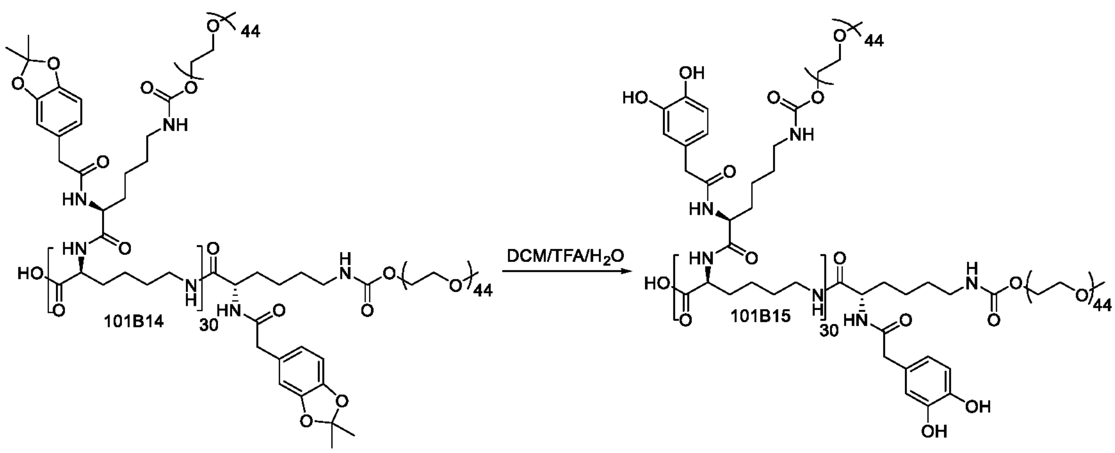


101B14 101B15

101B14 (1.00 g) was dissolved in 5 mL of chloroform, and 1.5 mL of TFA and 147 μL of water were added. The mixture was stirred at 50°C overnight under nitrogen atmosphere. MTBE was added to the reaction solution, and

the precipitate was vacuum-dried to give 101B105, 857 mg (87.0%).

Synthesis of compound 101B00I:

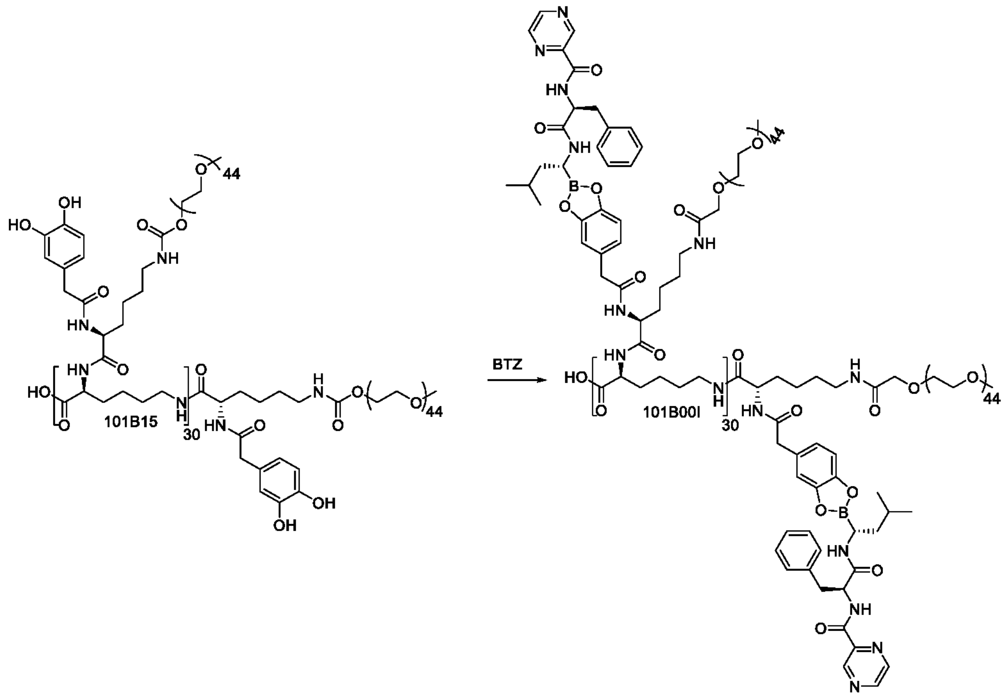


Bortezomib (13.54 mg) was dissolved in 271 μL DMSO. 101B15 (168.7 mg) was dissolved in 5 mL of phosphate buffer (0.01 M, pH=8.1), and the DMSO solution of bortezomib was added. The mixture was purified by ultrafiltration, and freeze-dried to give a final product. The conjugation content of bortezomib was determined to be 7.2 wt.%.

Example 24: synthesis of compound 101B00J-S

Synthesis of compound 101B00J-S:

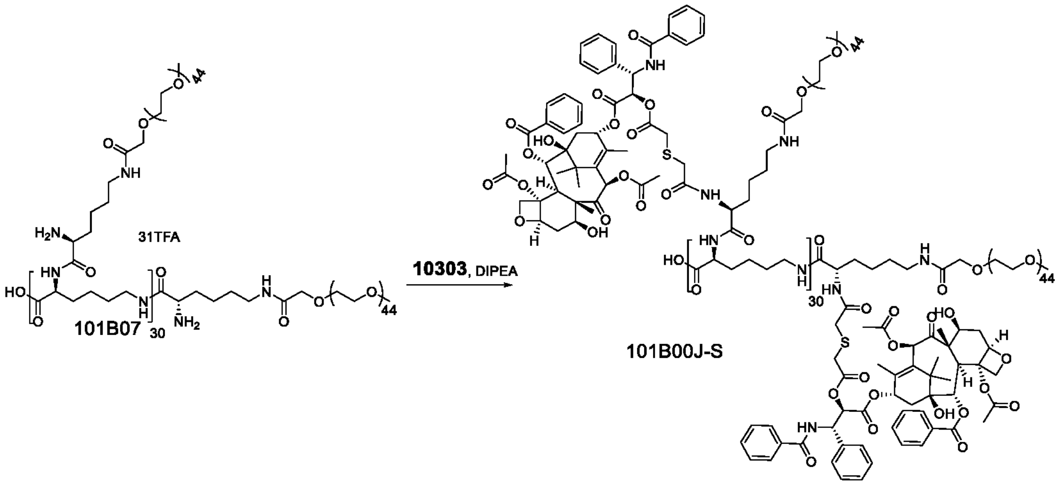


**101B07** (0.850 g) was dissolved in 5 mL of anhydrous DMF. **10303** (0.570 g) was dissolved in 5 mL of anhydrous

DMF. The two solutions were mixed, and DIPEA was added. The reaction mixture was stirred overnight at room temperature. MTBE was added to the reaction solution. The precipitate was dissolved in 100 mL of methanol/water, purified by ultrafiltration and freeze-dried to obtain a final product as white solid, **101B00J-S**, 681 mg.

HPLC analytical method A, RT=8.870 min.

Determination of PTX conjugation content in 100B00J-S

10302 was used as the standard to determine PTX conjugation content.

101B00J-S: 0.807 mg/mL, Area=4009.3, corresponding to 10302 of 0.195 mg/mL, 10302 conjugation content: 24.2%.

10302 molecular weight: 986.05, PTX molecular weight: 853.92, PTX conjugation content: 20.9%.

101B00J-S concentration of 39.98 mg/mL, corresponding to PTX concentration of 8.36 mg/mL

516.2 mg 101B00J-S was dissolved in 10.20 g water, and the concentration of polymer was 50.57 mg/mL. 100 μL of solution was taken, and 7.90 mL of water was added. The concentration of polymer was 0.632 mg/mL. After filtration, HPLC showed Area=3399.2, corresponding to 10302 of 0.166 mg/mL.

Standard curve of 10302: y=20421x+10.234

The corresponding PTX concentration in the stock solution was 11.5 mg/mL.

Example 25: synthesis of compound 101B00J-V

Synthesis of compound 10401:

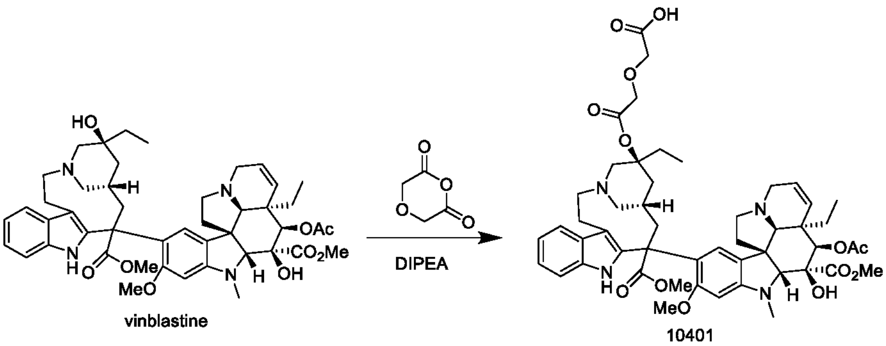


vinblastine 10401

Vinblastine (1.00 g) and 1,4-dioxane-2,6-dione (0.28 g) were added to a flask. A mixed solution of DIPEA (1.00 mL) and 50 mL of anhydrous dichloromethane was added. The reaction mixture was stirred at room temperature for 5 h under nitrogen atmosphere. TLC showed that the starting materials were almost fully consumed. The mixture was washed with 0.5 M hydrochloric acid, washed with water, dried over anhydrous sodium sulfate, filered, concentrated and purified by column chromatography to give product **10401** as solid, 0.82 g.

Synthesis of compound 10402:

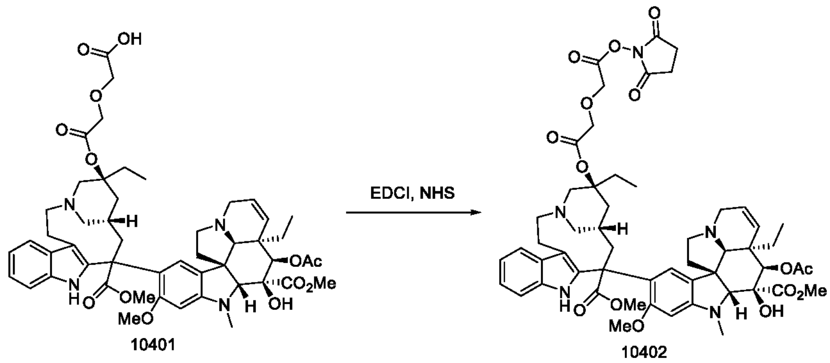


10401 10402

**10401** (0.50 g), NHS (0.095 g) and EDCI (0.15 g) were added to a flask, and 10 mL of dichloromethane was added. The reaction mixture was stirred overnight at room temperature. The organic phase was washed with water twice, dried over anhydrous sodium sulfate, filered, concentrated and vacuum-dried to give product **10402** as solid, 0.55 g, which was directly used for the next reaction.

Synthesis of compound 101B00J-V:

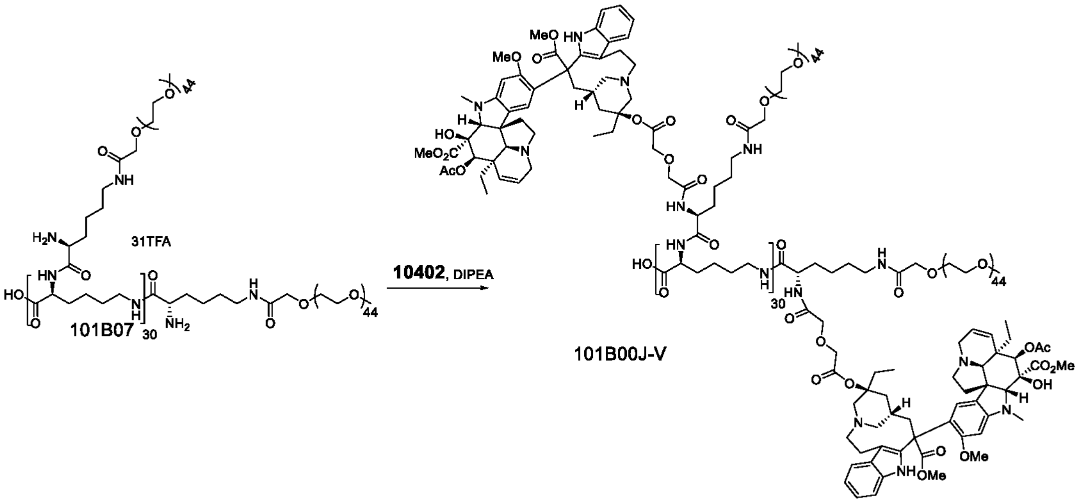


101B07 101B00J-V

**101B07** (0.85 g) was dissolved in 5 mL of anhydrous DMF. **10402** (0.54 g) was dissolved in 5 mL of anhydrous DMF. The two solutions were mixed, and DIPEA was added. The reaction mixture was stirred overnight at room temperature. MTBE was added to the reaction solution. The precipitate was dissolved in 100 mL of methanol/water, purified by ultrafiltration and freeze-dried to obtain a final product as white solid, **101B00J-V**, 721 mg.

Example 26: synthesis of compound 101B00J-Pt

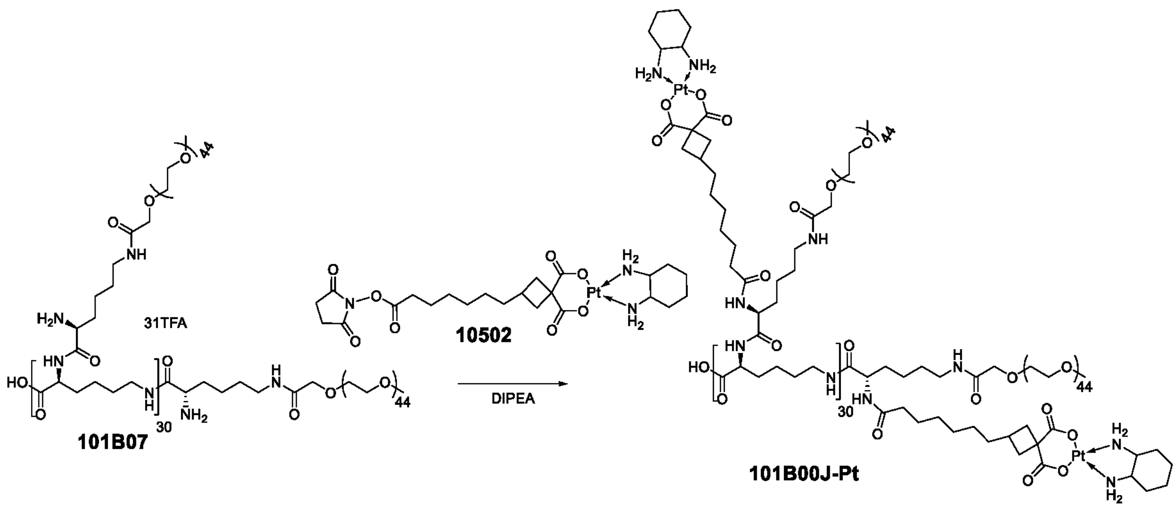


101B07 10502 101B00J-Pt

**101B07** (0.5 g) was dissolved in 5 mL of anhydrous DMF. **10502** (0.44 g, synthesized according to similar methods in the literature, Can. J. Chem. 1993, 71(6)896) was dissolved in 5 mL of anhydrous DMF. The two solutions were mixed, and DIPEA was added. The reaction mixture was stirred overnight at room temperature. MTBE was added to the reaction solution. The precipitate was dissolved in 100 mL of methanol/water, purified by ultrafiltration and freeze-dried to give a final product **101B00J-Pt**, 389 mg.

Example 27: synthesis of compound 101B00F

Synthesis of compound 10118:

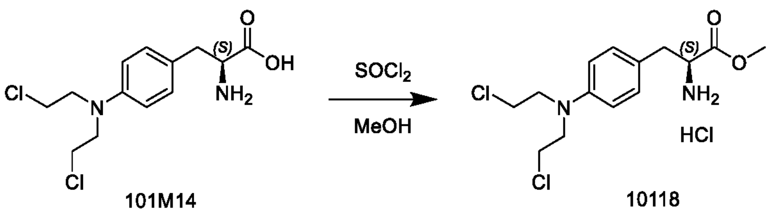


101M14 10118

Melphalan (**101M14**) (500.0 mg) was dispersed in 16 mL of methanol. 0.48 mL of $SOCl_2$ was added dropwisely in ice bath. The mixture was refluxed at 65°C for 18 h. The solvent was removed, and the mixture was vacuum-dried to give a crude product Melphalan-OMe (**10118**), 523 mg, which was used directly in the next reaction. LCMS (m/z): $[M+H]^+$ = 319.0.

Synthesis of compound 10119:

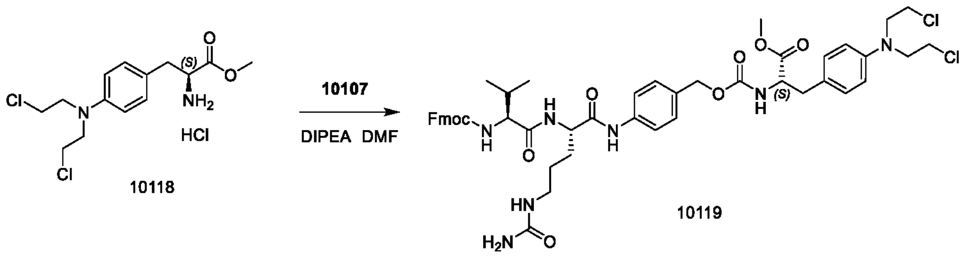


10118 10119

Melphalan-OMe·HCl (**10118**) (381.3 mg) and Fmoc-Val-Cit-PABC-pNP (**10107**) (805.6 mg) were dissolved in

5 mL of DMF. DIPEA (560 μL) was then added, and the mixture was stirred at room temperature for 4 h. 80 mL of MTBE was added. The precipitate was centrifuged and vacuum-dried to give a product Fmoc-Val-Cit-PABC-Melphalan-OMe (**10119**) 0.95 g, 95.9% yield. LCMS (m/z): $[M+H]^+$ = 946.2.

Synthesis of compound 10120:

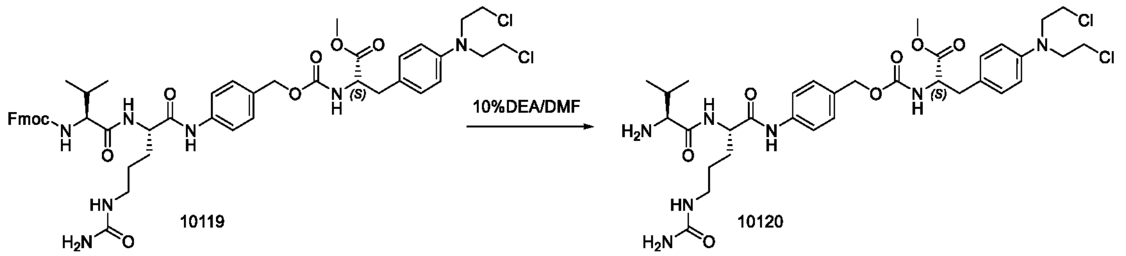


Fmoc-Val-Cit-PABC-Melphalan-OMe (**10119**) (1.39 g) was dissolved in 5 mL of DMF, and 0.7 mL of DEA was added. The mixture was stirred at room temperature for 15 min. 100 mL of MTBE was added. The precipitate was centrifuged and vacuum-dried to give a product $NH_2$-Val-Cit-PABC-Melphalan-OMe (**10120**), 1.05 g, 98.8% yield. LCMS (m/z): $[M+H]^+$ =724; $[M+2H]^+/2$ =362.6.

Synthesis of compound 101B00F:

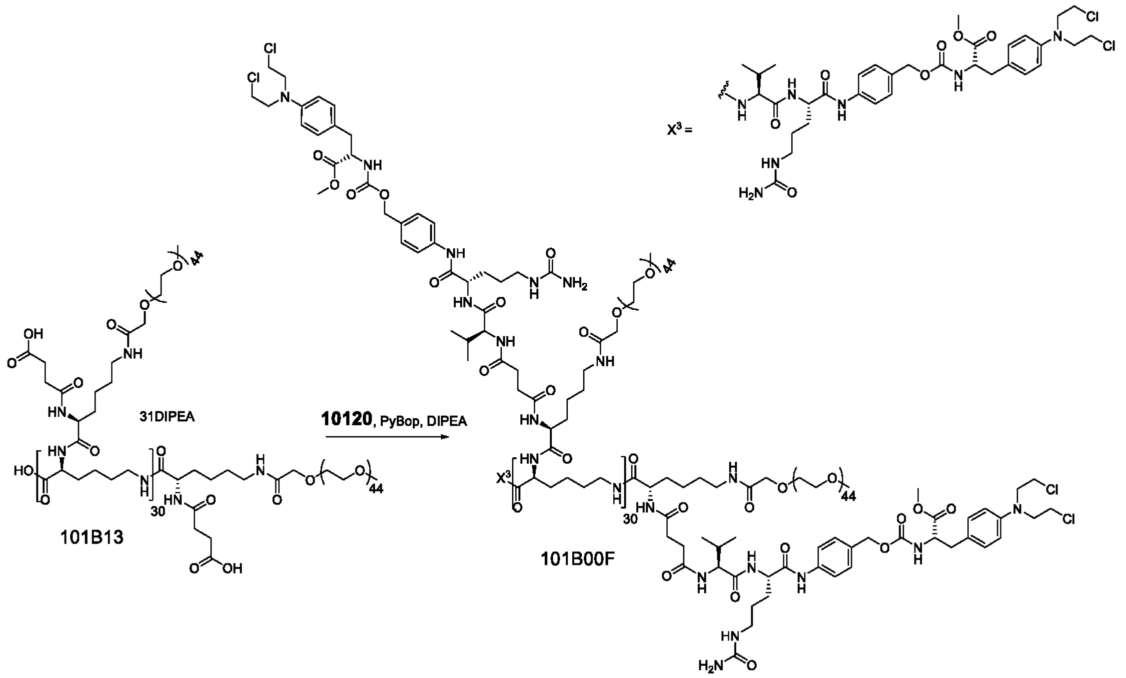


**101B13** (0.840 g), **10120** (0.364 g) and PyBop (0.348 g) were dissolved in 9 mL of DMF. The mixture was stirred at room temperature for 3 h. MTBE was added. The precipitate was dissolved in 50% $MeOH/H_2O$, purified by ultrafiltration, and freeze-dried to obtain a final product (**101B00F**) as brown solid, 955.5 mg.

HPLC analytical method C, RT=9.093 min.

Example 28: synthesis of compound 101B00K

101B00K was synthesized according to similar methods in examples 15-17 and example 70:

$X^7$ =

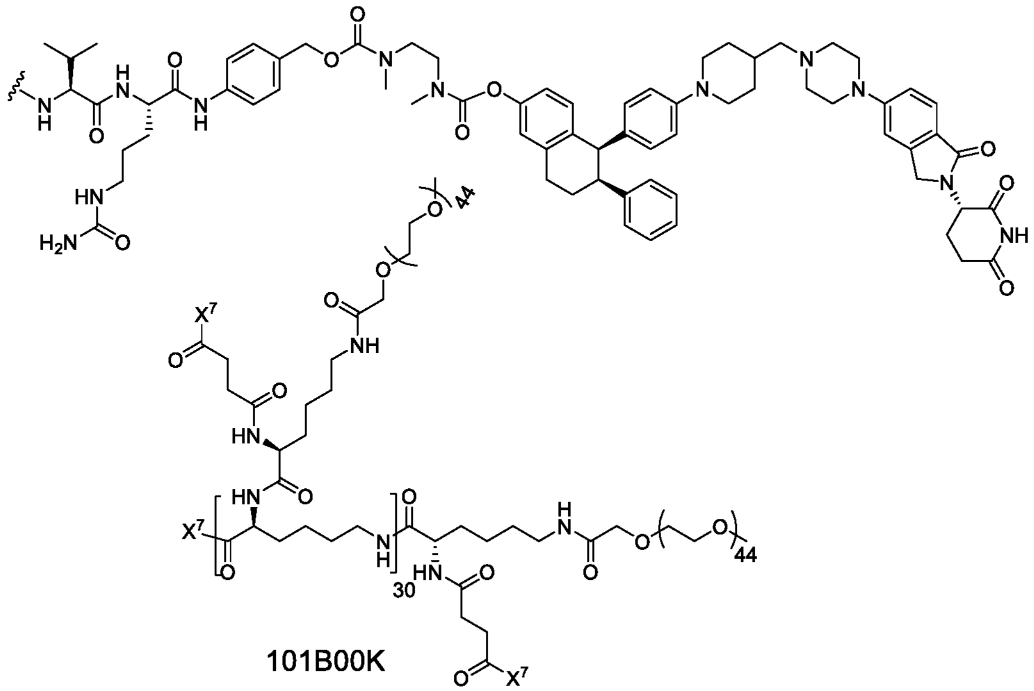

101B00K

Example 29: synthesis of compound 101B00L

101B00L was synthesized according to similar methods in examples 15-17 and example 70:

$X^8$ =

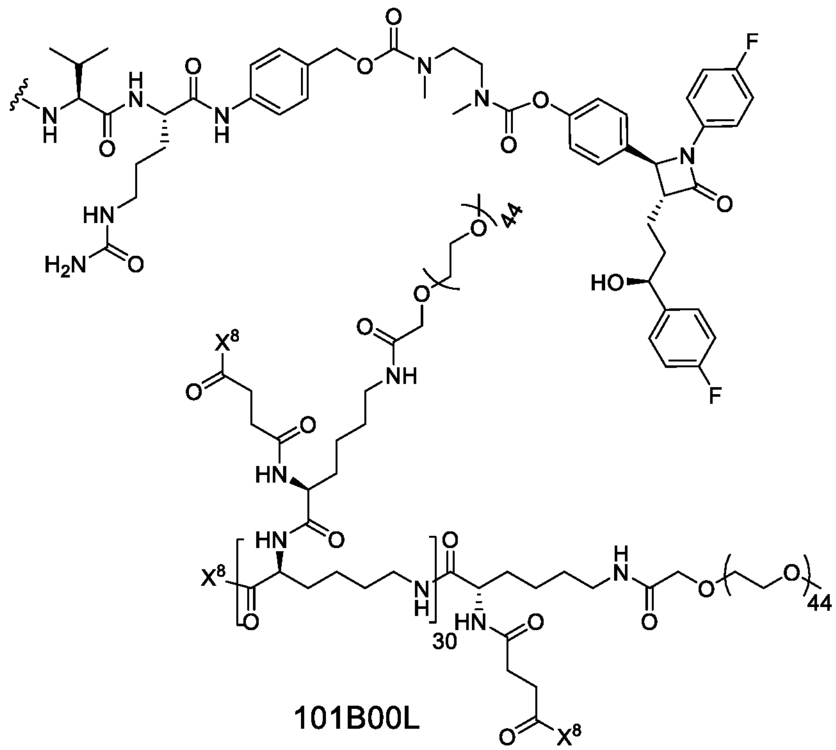

101B00L

Example 30: synthesis of compound 101B00M

101B00M was synthesized according to similar methods in examples 15-17 and example 70:

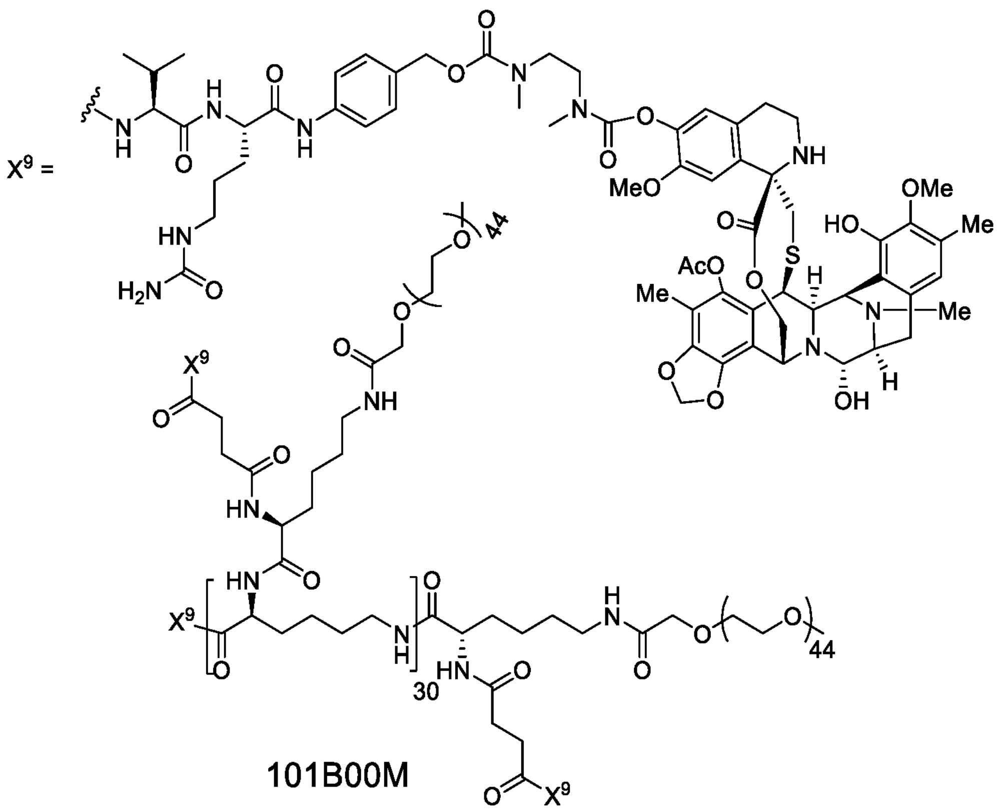

$X^9$ =

101B00M

Example 30: synthesis of compound 101B00N

101B00N was synthesized according to similar methods in examples 15-17 and example 70:

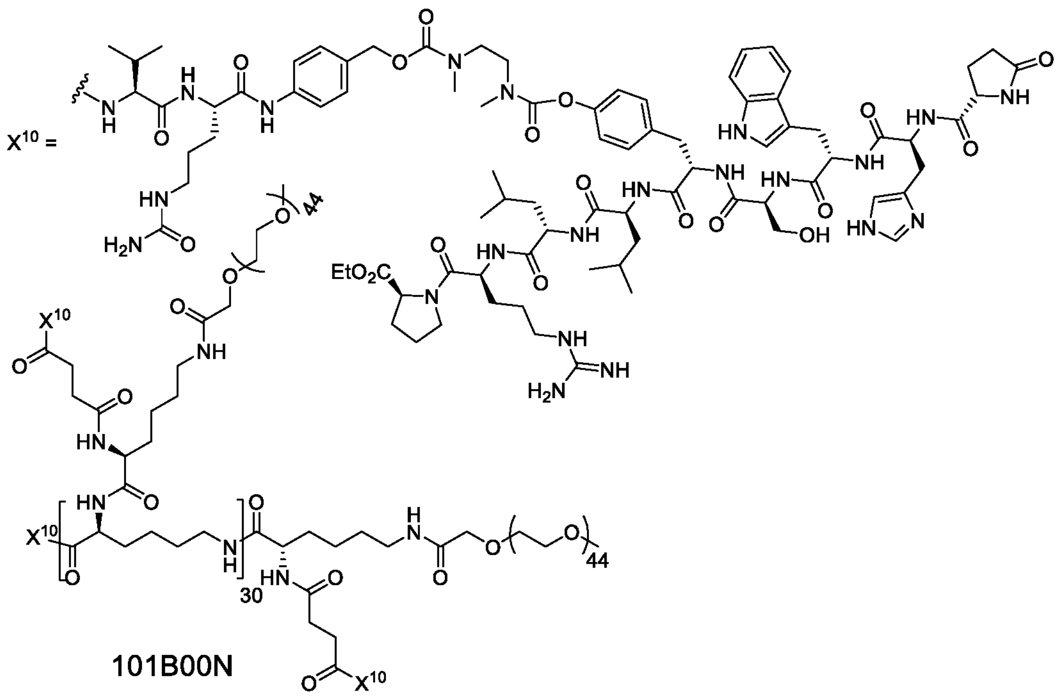

$X^{10}$ =

101B00N

Example 31: synthesis of compound 101B00O

101B00O was synthesized according to similar methods in examples 15-17 and example 70:

$X^{11}$ =

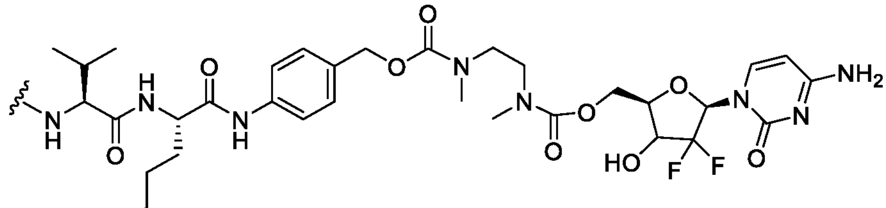

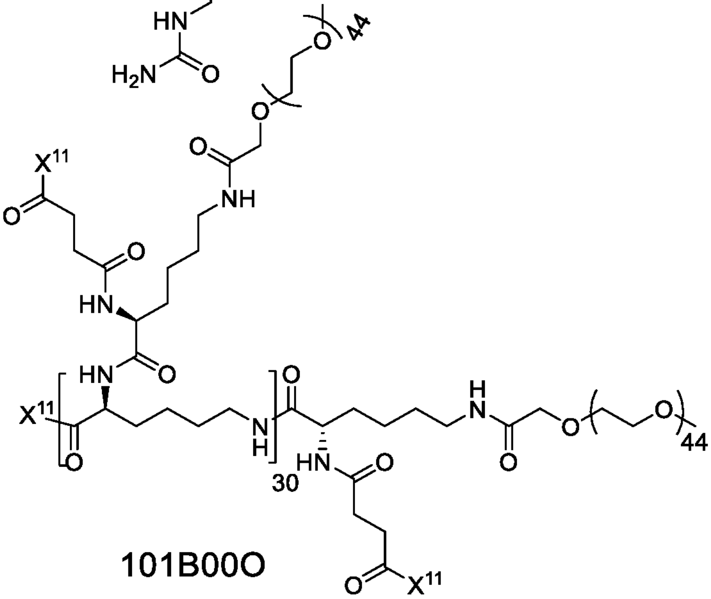

101B00O

Example 32: synthesis of compound 101B00P

21209 was synthesized with reference to synthesis steps of compound 3c in 113355, European Journal of Medicinal Chemistry, 2021, 216.

Synthesis of compound 101B00P:

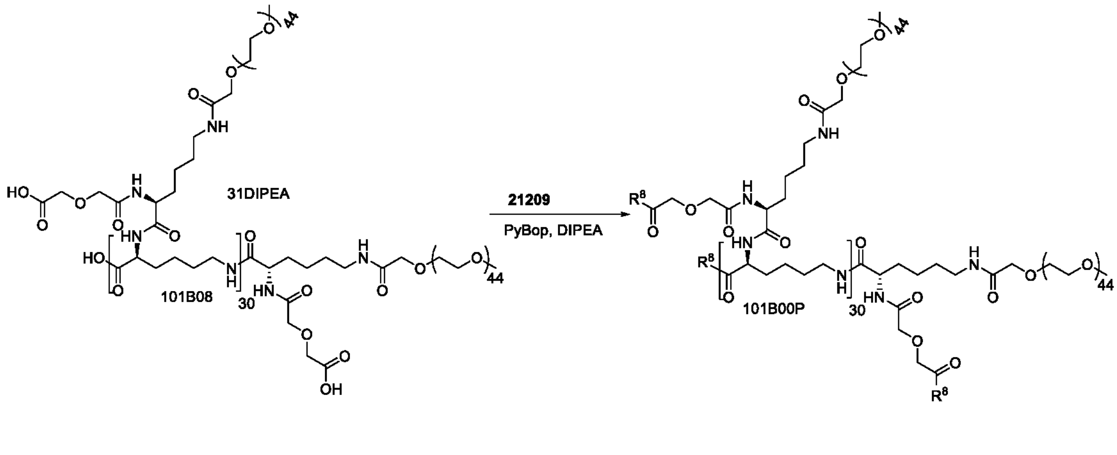


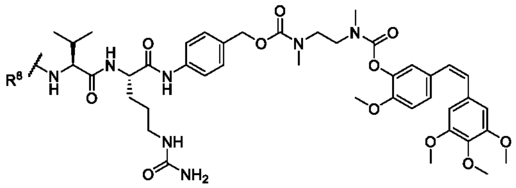

101B08 (1.500 g) and 21209 (0.753 g) were dissolved in 7.5 mL of DMF. 0.625 g of PyBop and 0.471 mL of DIPEA were added. The mixture was stirred for 24 h at room temperature.

30 mL of MTBE was added and centrifuged. The precipitate was dissolved in 3 mL of methanol/3 mL of DCM. The solution was further precipitated with 30 mL of MTBE and centrifuged. A small amount of precipitate was dissolved with methanol. The precipitate was vacuum-dried to obtain a crude product.

The crude product was dissolved in 20 mL of methanol. The solution was added to 150 mL of 50% MeOH, and purified by ultrafiltration. The purified solution was concentrated to about 26 mL and freeze-dried in portions to give a product as white solid, 1.707 g, molecular weight of structural units: 3242, 0.533 mmol, 88.8 %, HPLC puriety 99.6%.

$^1$H NMR (400 MHz, DMSO-d6) δ 7.73 - 7.41 (m, 61H), 7.25 (s, 65H), 7.11 (d, J = 8.5 Hz, 33H), 6.96 (d, J = 12.2 Hz, 67H), 6.64 - 6.35 (m, 128H), 4.97 (s, 65H), 4.27 (d, J = 92.8 Hz, 285H), 3.97 (s, 63H), 3.71 (s, 5400H), 3.35 (s, 84H), 3.27 - 2.75 (m, 391H), 2.28 - 1.14 (m, 549H), 0.95 (t, J = 8.6 Hz, 203H).

Determination of CA4 conjugation content in 101B00P

The freeze-dried product 101B00P was directly weighed 15~20 mg (recording weight as $m_A$) on an analytical balance with a readability of 0.01 mg, and placed into a well-sealed glass sample vial ( plastic centrifuge tubes are prohibited to avoid concentration deviation due to leakage). The vial was sealed after adding 1.5~2.0 mL of water. 101B00P was titrated to a final concentration of 10 mg/mL. The weight of the added water was recorded as $m_{AW}$. The vial was shaken in a 37°C shaker for 20 min for dissolution until a colorless clear solution was obtained.

Preparation of buffer solution

Sodium dihydrogen phosphate: 352 mM
Disodium hydrogen phosphate: 48 mM
EDTA 2Na: 4.0 mM
L-cysteine hydrochloride: 8.0 mM
Brij 35: 0.1% (v/v)
1 M HCl or 1 M NaOH: pH adjusted to 6.0

Preparation of enzyme solution

The above buffer solution contained 10 mg/mL papain B
Enzymatic release of the samples

| | | Polymer solution | Enzyme solution |
|---|---|---|---|
| No. | From | μL | μL |
| 1 | A | 200 | 200 |
| 2 | A | 200 | 200 |

200 μL of 101B00P aqueous solution (weight $m_{A1}$) was pipetted via a 200 μL pipette gun into a 1.5 mL vial (tared) arranged on an analytical balance with a readability of 0.01 mg, and 200 μL of papain B solution was added. The vial was sealed with a cover, and the solution was uniformly vortexed at 1000 rpm for 30 s.

The vial was placed in a 37°C shaker for enzymatic release for more than 20 h.

The vial was taken from the shaker and cooled to room temperature. All of the solution was transferred to a 10 mL volumetric flask using a 200 μL pipette gun. The tip of the pipette gun and the vial were respectively washed with 400 μL of 1% DIPEA/methanol solution three times, and the solutions were transferred into the volumetric flask. 0.5% DIPEA/methanol was added, q.s. to the scale (when being close to the scale, it is required to use the 200 μL pipette gun) (NOTE: the pH of the final solution is greater than 8).

After filteration, the CA4 concentration $[CA4]_A$ (295 nm) was determined by HPLC, and the concentration of the CA4 standard sample was 80-100 μg/mL (DMSO).

The CA4 conjugation content was calculated by:

$$CA4 \text{ conjugation content\% of sample A} = \frac{[\text{CA4}]_A \times 10}{m_A/(m_A+m_{AW}) \times m_{A1}} \times 100\%$$

Based on HPLC, the CA4 conjugation content was determined to be 9.73%, which was consistent with the theoretical value.

Example 33: synthesis of compound 101B00Q

101B00Q was synthesized according to similar methods in examples 15-17 and example 70:

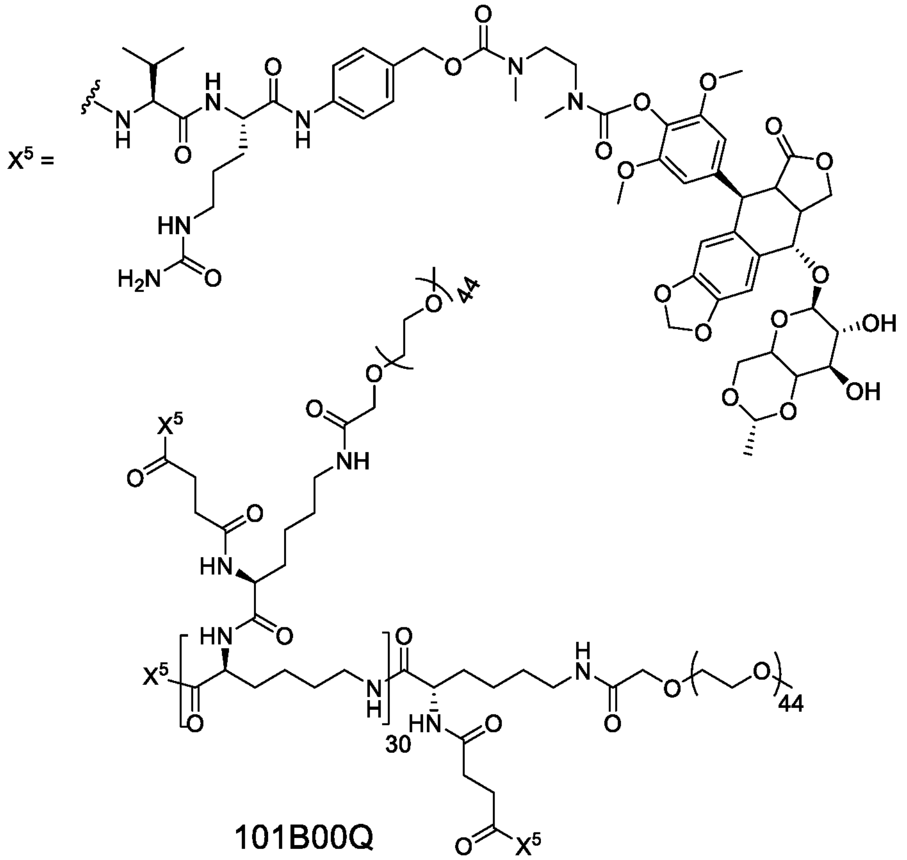

101B00Q

Example 34: synthesis of compound 101C00A

Synthesis of compound 101C04:

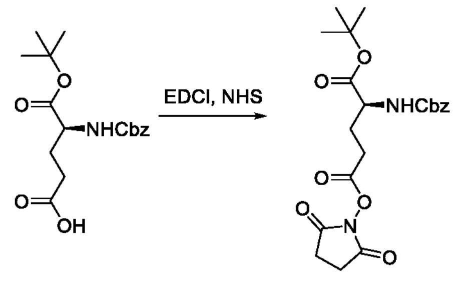


101C04

*N*-carbobenzoxyl-*L*-glutamic acid-1-*tert*-butyl ester was dissolved in 20 mL of DCM. 1.02 g NHS and 1.70 g of EDCI were added. The mixture was stirred for 4 h at room temperature. The mixture was washed three times, dried over anhydrous sodium sulfate, and concentrated to give a transparent foaming product, 2.849 g (Mw=434.45, 6.56 mmol).

Synthesis of compound 101C05:

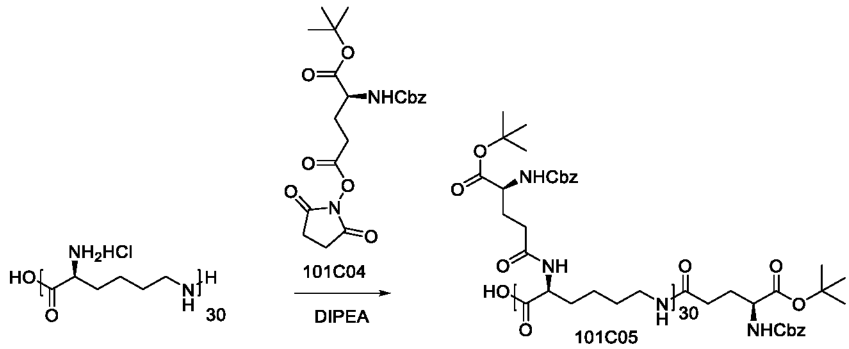


polylysine hydrochloride (0.650 g) was dissolved in 0.878 mL of water and 3 mL of DMSO, and **101C04** (2.573 g) was dissolved in 5.85 mL of DMSO. The two solutions were mixed, and DIPEA (2.06 mL) was added. The mixture was stirred at room temperature for 6 h. 1 M NaOH and water were added, and the mimture was filtered and vacuum-dried to give a white powdery product **101C05**, 1.709 g (96.7%).

$^1$H NMR (400 MHz, DMSO-d6) δ 8.46 - 7.51 (m, 85H), 7.34 (s, 153H), 5.02 (m, 60H), 4.14 (m, 30H), 3.87 (m, 33H), 2.97 (s, 66H), 2.21 (s, 69H), 1.37 (m, 528H).

Synthesis of compound 101C06:

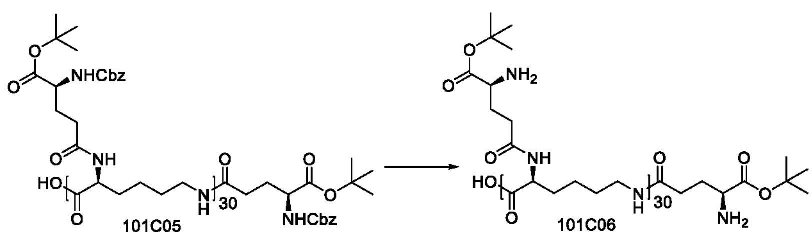


**101C05** (1.68 g) was added to 17 mL of acetic acid in a 50 mL flask, and dissolved at 40°C. 840 mg of 10% palladium on carbon was added, and the mixture was stirred overnight at 30°C under hydrogen atmosphere. The mixture was filtered through celite and the filtrate was concentrated to remove the solvent, and vacuum-dried to give **101C06** as white solid, 1.64 g.

$^1$H NMR (400 MHz, Deuterium Oxide) 4.22 - 3.99 (m, 30H), 3.92 (m, 29H), 3.10 (m, 104H), 2.43 (m, 60H), 2.09 (m, 55H), 1.26 (m, 566H).

Synthesis of compound 101C08:

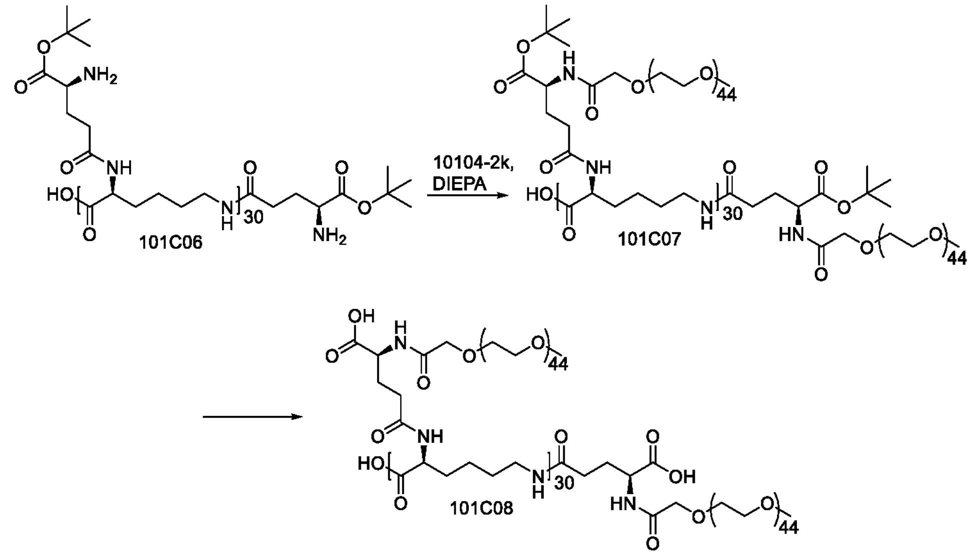


101C06 101C07 101C08

**101C06** (0.500 g) was dissolved in 2.68 mL of water, and **10104-2k** (3.724 g) was dissolved in 7 mL of acetonitrile. After mixing the two solutions, DIPEA was added. The mixture was stirred overnight at room temperature. The mixture was concentrated to remove the acetonitrile, and water was added. The mixture was extracted with dichloromethane, dried over anhydrous sodium sulfate, concentrated and vacuum-dried to give **101C07** as white solid, 2.892 g. **101C07** (2.00 g) was dissolved in 10 mL of dichloromethane. 5 mL of TFA was added. The mixture was stirred overnight at room temperature. The solvent was removed in vacuo. MTBE was added, and the precipitate was vacuum-dried to give **101C08** as white solid, 1.80 g.

Synthesis of compound 101C00A:

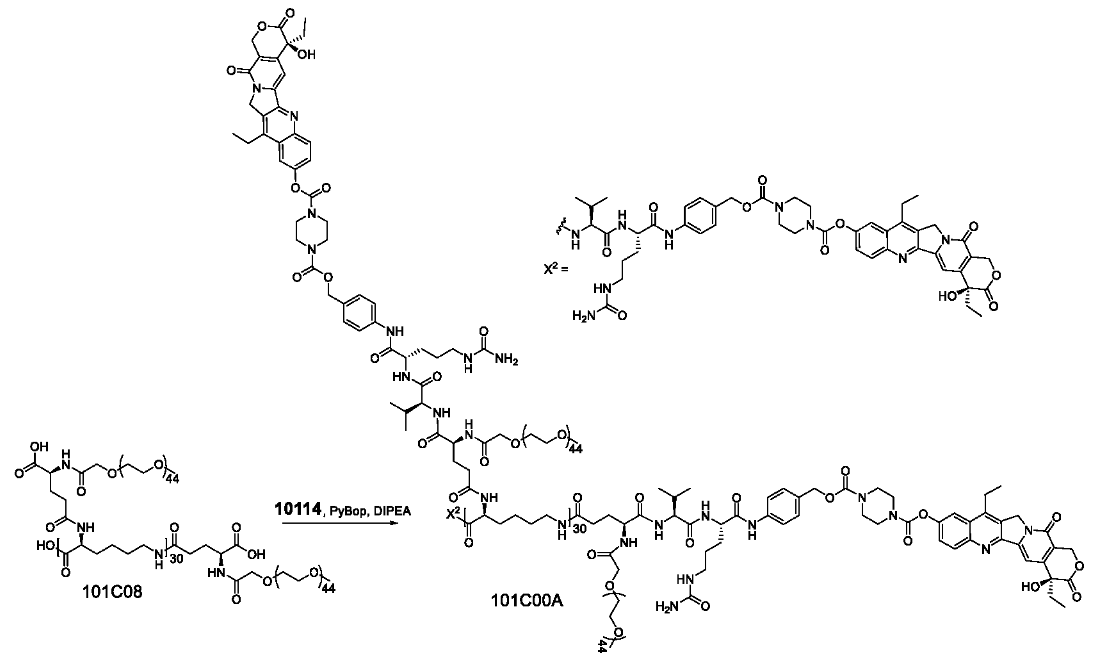


101C08 101C00A

**101C08** (0.66 g), **10114** (0.39 g) and PyBop (0.443 g) were dissolved in 6 mL of DMF, and DIPEA (0.22 mL)

was added. The mixture was stirred overnight at room temperature. 50 mL of MTBE was added. The precipitate was dissolved in 4 mL of DMSO and 10 mL of methanol, purified by ultrafiltration, and freeze-dried to give **101C00A** (422 mg).

HPLC analytical method A, RT=8.121 min.

Determination of SN-38 conjugation content in compound 101C00A

Papain was weighed and added to PBS to prepare 10.0 mg/mL PBS solution.

30.12 mg of freeze-dried product ε-PolyLys$_{30}$-[γ-Glu-(α-CO-VC-PABC-piperazine-10-O-SN-3 8, α-NH-CO-mPEG$_{2k}$)]$_{30}$ was weighed, and 3.012 mL of deionized water was added to obtain 10.0 mg/mL polymer solution.

0.500 mL of polymer solution and 0.500 mL of papain PBS solution (having a final concentration of polymer of 5.00 mg/mL) were mixed and were shaken in water bath at 37°C for 2 h. The solution was cooled to room temperature. 0.200 mL of solution was pipetted, and 1.800 mL of DMSO was added. The peak area of TFA-piperazine-10-O-SN-38 after enzymatic release was characterized by HPLC, and the concentration of TFA-piperazine-10-O-SN-38 after the full enzymatic release of polymer was 876.31 μg/mL, which was equivalent to a conclusion that the concentration of SN-38 was 527.6 μg/mL after the enzymatic release of 5.00 mg/mL of polymer, and therefore, the SN-38 conjugation content in ε-PolyLys$_{30}$-[γ-Glu-(α-CO-VC-PABC-piperazine-10-O-SN-38, α-NH-CO-mPEG$_{2k}$)]$_{30}$ was calculated to be 10.6 wt.%.

Example 35: synthesis of compound 101D00A

Synthesis of compound 101D07:

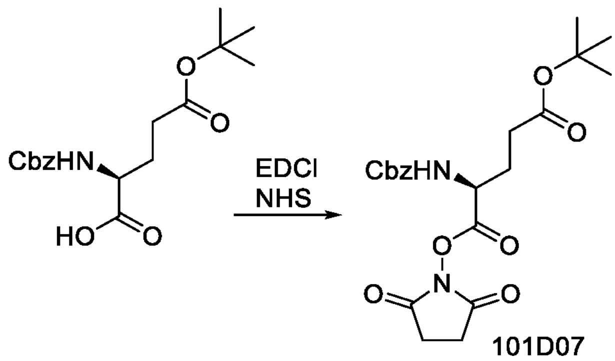


101D07

*N*-carbobenzoxyl-*L*-glutamic acid-5-*tert*-butyl ester 4.944 g was dissolved in 50 mL of DCM. 2.53 g NHS and 4.214 g EDCI were added in ice bath. The mixture was stirred overnight at room temperature. The mixture was washed with water three times, dried over anhydrous sodium sulfate, concentrated and vacuum-dried to give a product 6.77 g (15.58 mmol, 106%).

Synthesis of compound 101D08:

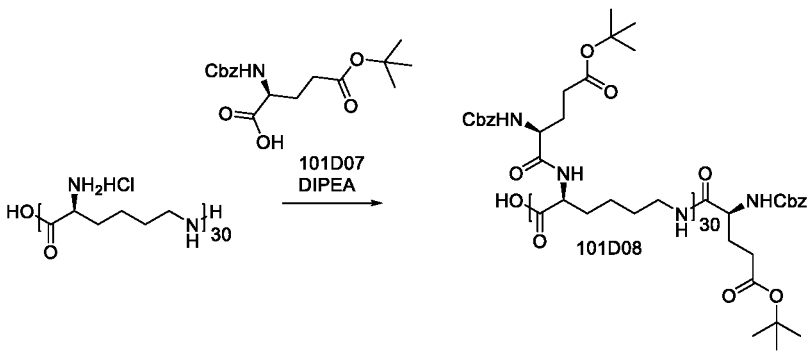

polylysine hydrochloride (1.70 g) was dissolved in 2.29 mL of water. 101D07 (6.73 g) was dissolved in 30 mL of DMSO. The two solutions were mixed, and DIPEA was added. The mixture was stirred for 6 h at room temperature. 1 M NaOH was added, and the precipitate was filtered. The filter cake was vacuum-dried to give a product 101D08 as white powder, 4.12 g, (89.2%).

$^1$H NMR (400 MHz, DMSO-$d_6$) δ 8.14 - 7.57 (m, 64H), 7.33 (m, 174H), 5.02 (m, 58H), 4.17 (m, 30H), 4.02 (m, 33H), 2.97 (s, 70H), 2.22 (m, 66H), 1.36 (s, 522H), 1.00 (d, *J*= 6.6 Hz, 38H).

Synthesis of compound 101D09:

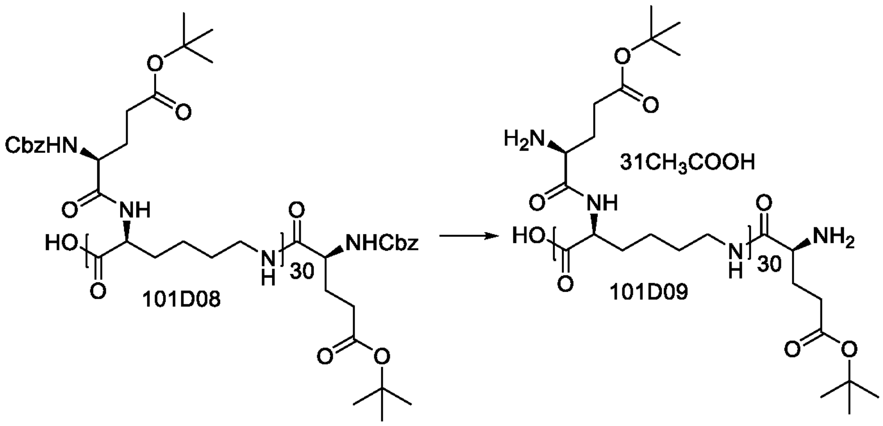


**101D09** (3.92 g) was dissolved in 39 mL of acetic acid in a 50 mL flask. 10% palladium on carbon (0.58 g) was added, and the mixture was stirred overnight at room temperature under hydrogen atmosphere. Celite was added, and the mixture was filtered. The filtrate was concentrated until most of the solvent was removed. The mixture was precipitated with MTBE and filtered. The filter cake was vacuum-dried to give **101D09** (2.578 g).

Synthesis of compound 101D10:

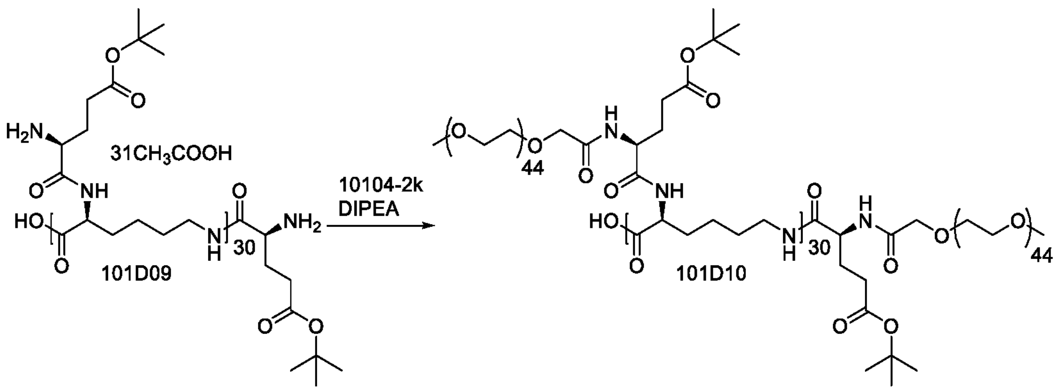


**101D09** (1.00 g) was dissolved in 10 mL of DMSO. **10104-2k** (8.53 g), 5 mL of DMSO, 10 mL of DMF and DIPEA (1.40 mL) were added. The mixture was stirred overnight at room temperature. MTBE was added. The precipitate was dissolved in acetonitrile. The mixture was precipitated with MTBE. The precipitate was filtered, and vacuum-dried to give a product **101D10** as white powder (8.15 g). The 2.165-2.388 ppm $^1$H integration value was 62, and the 0.865-2.130 ppm $^1$H integration value was 514.

Synthesis of compound 101D11:

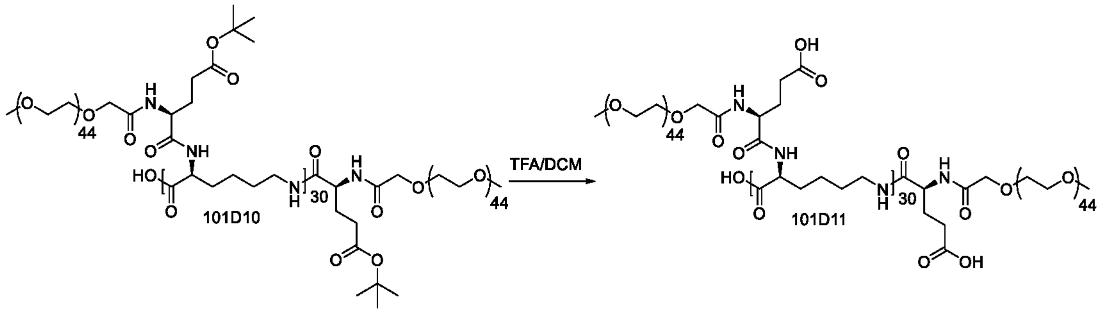


101D10 (4.00 g) was dissolved in 24 mL of dichloromethane. 12 mL of TFAwas added. The mixture was stirred overnight at room temperature. The mixture was concentrated. MTBE was added. The precipitate was dissolved in ACN. The mixture was precipitated with MTBE and vacuum-dried to give a product **101D11** as white solid, 1.80 g, (60.6%). The 2.165-2.388 ppm $^1$H integration value was 62, and the 0.865-2.130 ppm $^1$H integration value was 197, indicating complete removal of tert-butyl ester.

Synthesis of compound 101D00A:

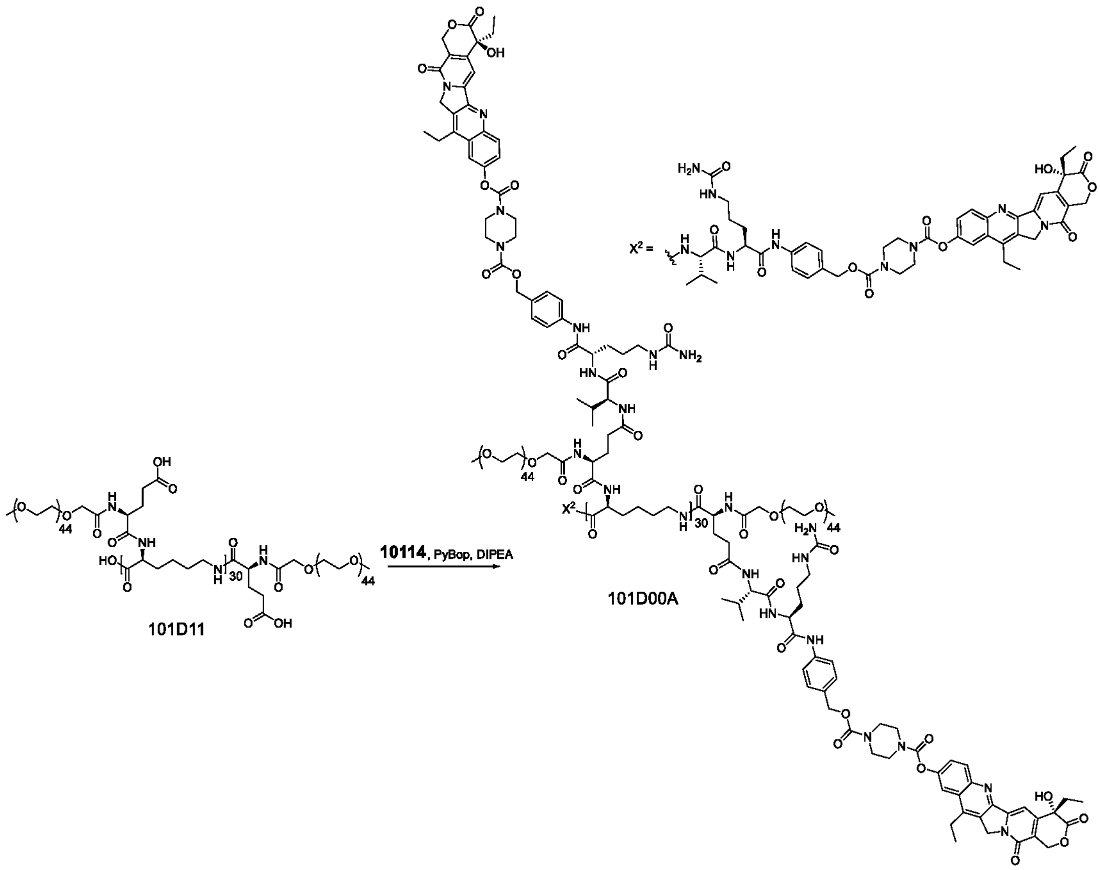


**101D11** (0.53 g), **10114** (0.32 g) and PyBop (0.36 g) were dissolved in 5 mL of DME DIPEA (0.18 mL) was added, and the mixture was stirred overnight at room temperature. MTBE was added. The precipitate was dissolved in 4 mL of DMSO and 50 mL 50% MeO/$H_2O$. The mixture was purified by ultrafiltration, and freeze-dried to give a final product **101D00A** as light yellow solid, 450 mg.

HPLC analytical method B, RT=7.442 min.

Determination of SN-38 conjugation content in compound 101D00A

Papain was weighed and added to PBS to prepare 10.0 mg/mL PBS solution.

28.35 mg of freeze-dried product ε-PolyLys$_{30}$-[α-Glu-(γ-CO-VC-PABC-piperazine-10-O-SN-38, α-NH-CO-mPEG$_{2k}$)]$_{30}$ was weighed, 0.142 mL of DMSO was added, and the mixture was heated to dissolve. 2.690 mL of deionized water was added under ultrasonic conditions to obtain 10.0 mg/mL polymer solution.

0.500 mL of polymer solution and 0.500 mL of papain PBS solution (having a final concentration of polymer of 5.00 mg/mL) were mixed and were shaken in water bath at 37°C for 2 h. The solution was cooled to room temperature. 0.200 mL of solution was pipetted, and 1.800 mL of DMSO was added. The peak area of TFA-piperazine-10-O-SN-38 after enzymatic release was characterized by HPLC, and the concentration of TFA-piperazine-10-O-SN-38 after the full enzymatic release of polymer was 871.7 μg/mL, which was equivalent to a conclusion that the concentration of SN-38 was 524.8 μg/mL after the enzymatic release of 5.00 mg/mL of polymer, and therefore, the SN-38 conjugation content in ε-PolyLys$_{30}$-[a-Glu-(y-CO-VC-PABC-piperazine-10-O-SN-38, NH-CO-mPEG$_{2k}$)]$_{30}$ was calculated to be 10.5 wt.%.

Example 36: synthesis of compound 101E00A

Synthesis of compound 10108-E:

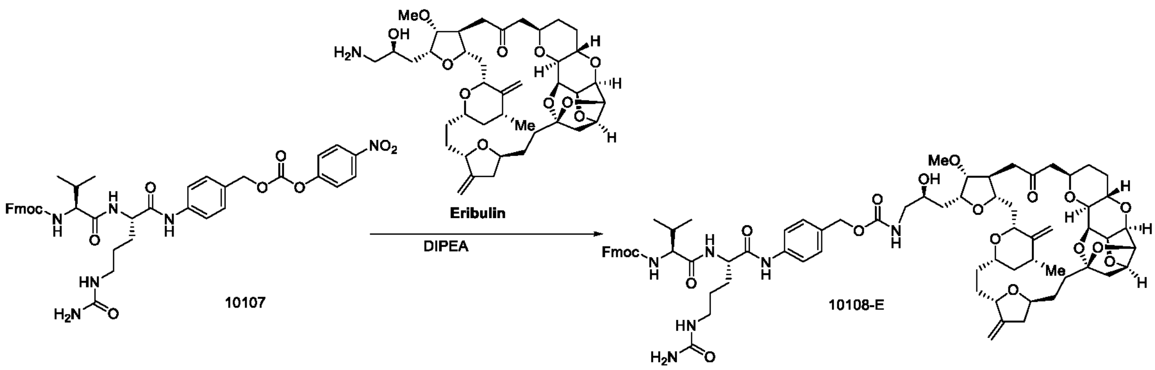


Fmoc-Val-Cit-PABC10107 (prepared in example 9) (0.30 g) was dissolved in DMF (5 mL). Eribulin (0.21 g) and DIPEA (0.32 mL) were added. The mixture was stirred at room temperature for 2 h. 30 mL of MTBE was added. The precipitate was filtered and vacuum-dried to give a product Fmoc-Val-Cit-PABC-ERB (**10108-E**) 0.41 g.

Synthesis of compound 10109-E:

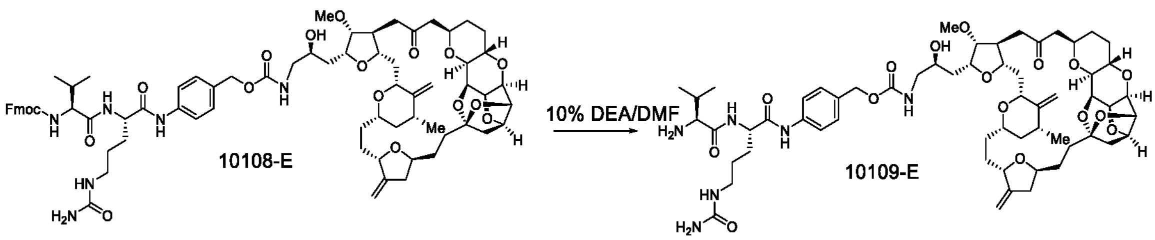


Fmoc-Val-Cit-PABC-ERB (0.41 g) was dissolved in DMF (10 mL), and DEA (1 mL) was added. The solution turned purple-black and was stirred at room temperature for 15 min. 100 mL of MTBE was added. The precipitate was filtered and vacuum-dried to give white solid powder $NH_2$-Val-Cit-PABC-ERB (**10109-E**) 0.25 g.

Synthesis of compound 101E00A:

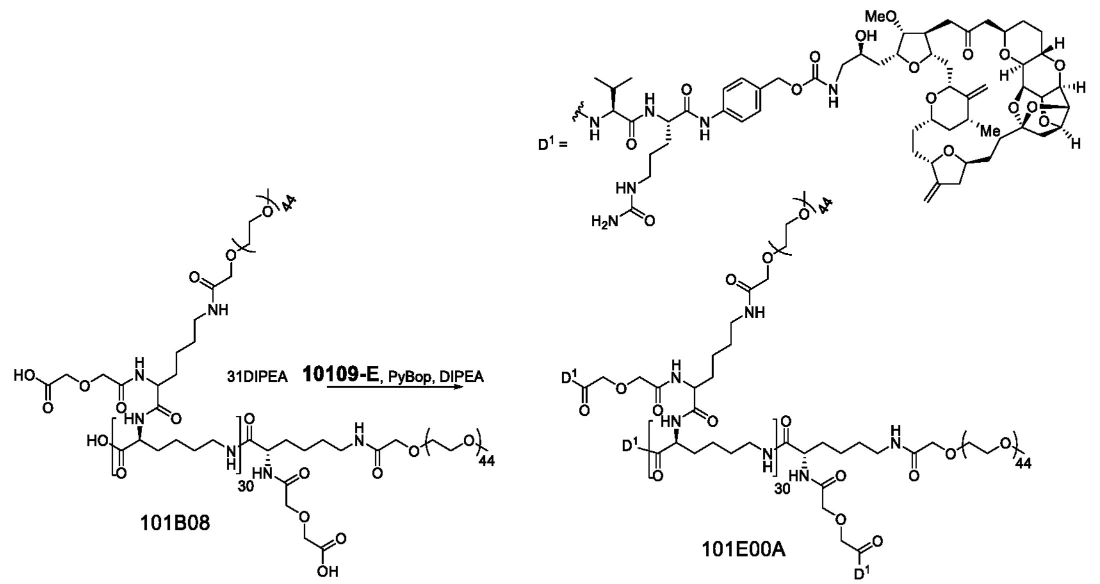

101B08
101E00A

**101B08** (0.10 g) was dissolved in 2 mL of DMF. **10109-E**, PyBop (229 mg) and DIPEA (0.10 mL) were added. The mixture was stirred overnight at room temperature. MTBE was added. The precipitate was dissolved in 30 mL of methanol. 10 mL of water was added. The mixture was purified by ultrafiltration and freeze-dried to give a final product as white solid (**101E00A**) 110 mg, HPLC purity: 98.0%.

Example 37: synthesis of compound 101P00A

Synthesis of compound 10108-P:

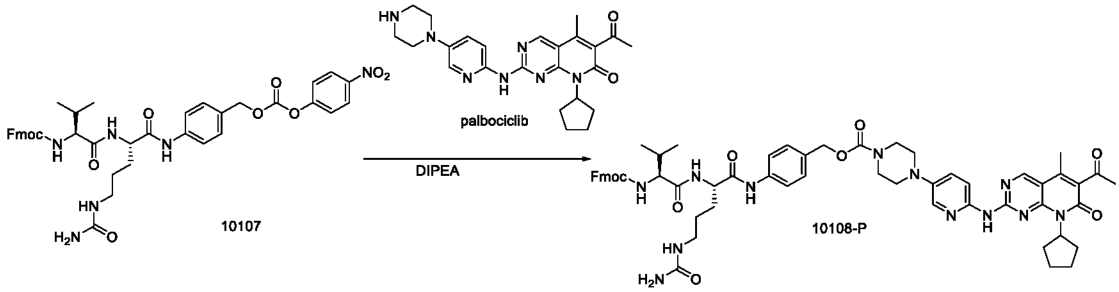

10107
10108-P

Fmoc-Val-Cit-PABC (0.30 g) was dissolved in DMF (5 mL). Palbociclib (0.15 g) and DIPEA (0.32 mL) were added. The mixture was stirred at room temperature for 2 h. 30 mL of MTBE was added. The precipitate was filtered and vacuum-dried to give a product Fmoc-Val-Cit-PABC-PAB (**10108-P**) 0.35 g.

Synthesis of compound 10109-P:

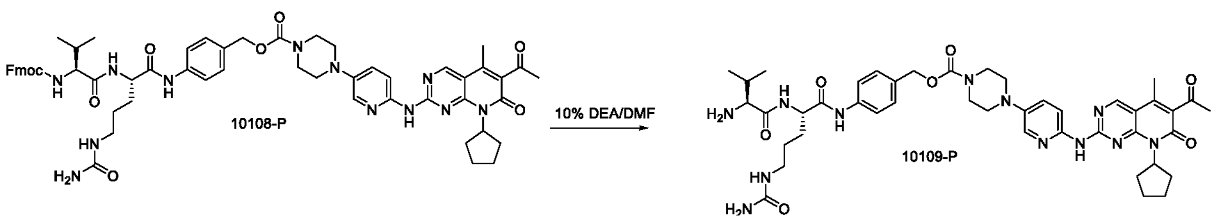

10108-P
10109-P

Fmoc-Val-Cit-PABC-PAB (0.35 g) was dissolved in DMF (10 mL), and DEA (1 mL) was added. The solution turned purple-black and was stirred at room temperature for 15 min. 50 mL of MTBE was added. The precipitate was filtered and vacuum-dried to give white solid powder $NH_2$-Val-Cit-PABC-PAB (**10109-P**) 0.22 g.

Synthesis of compound 101P00A:

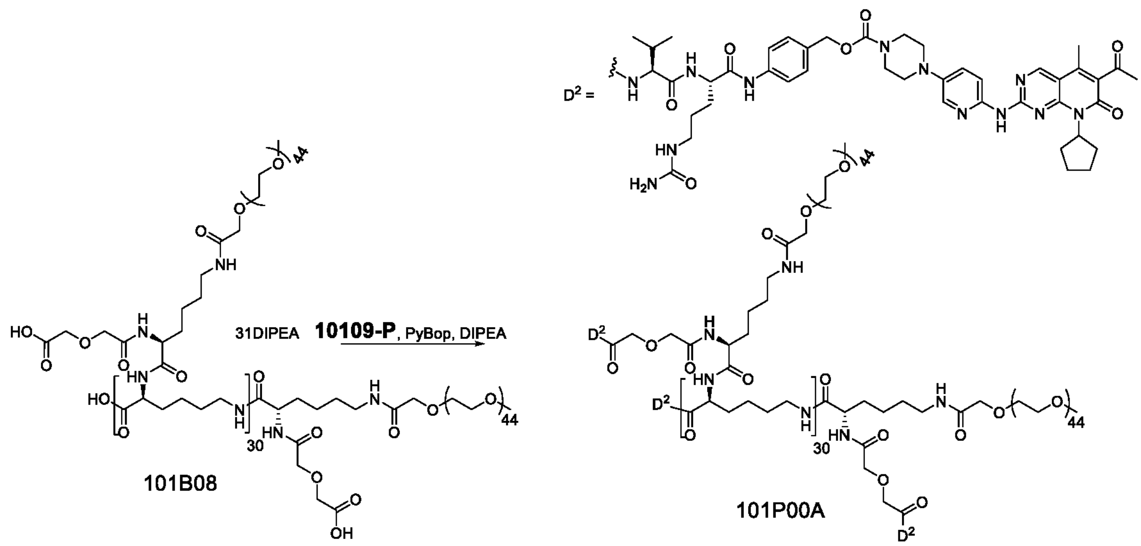


101B08

101P00A

**101B08** (0.10 g) was dissolved in 2 mL of DMF. **10109-P**, PyBop (200 mg) and DIPEA (0.10 mL) were added. The mixture was stirred overnight at room temperature. MTBE was added. The precipitate was dissolved in 30 mL of methanol. 10 mL of water was added. The mixture was purified by ultrafiltration and freeze-dried to give a final product as white solid (**101P00A**) 135 mg, HPLC purity: 98.5%.

Example 38: synthesis of compound 101P00B

101P00B was synthesized according to similar methods in examples 105-107:

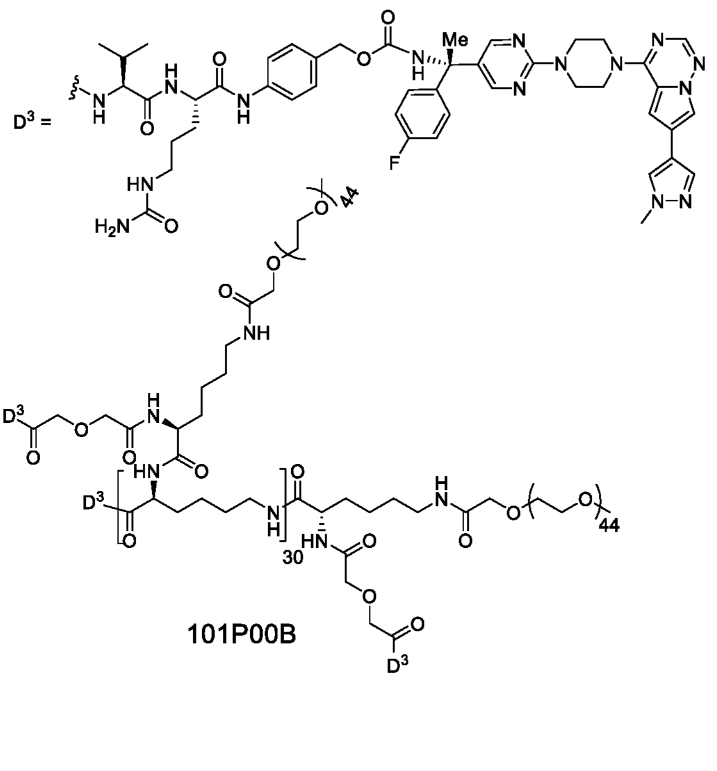

101P00B

Example 40: synthesis of compound 101P00C

101P00C was synthesized according to similar methods in examples 105-107:

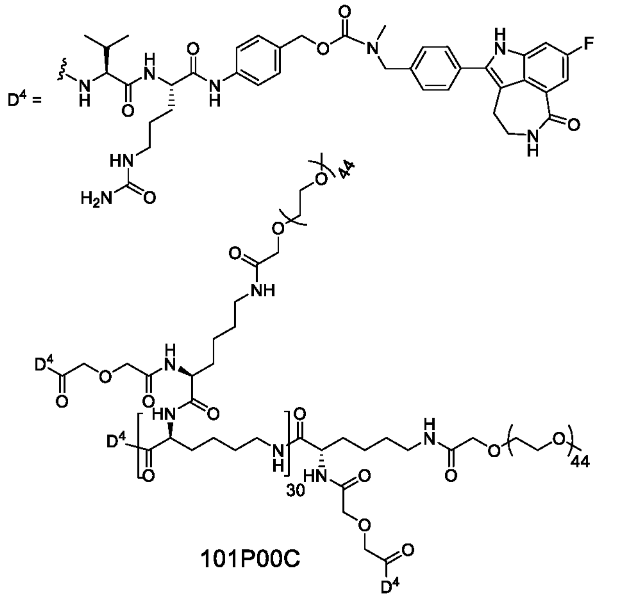

101P00C

Example 41: synthesis of compound 101P00D

101P00D was synthesized according to similar methods in examples 105-107:

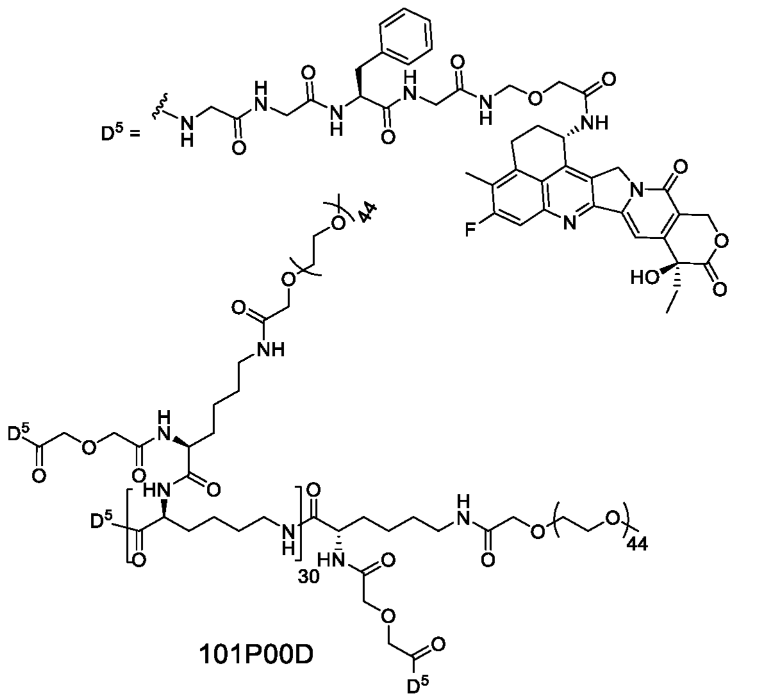

101P00D

Example 39: synthesis of compound 101P00E

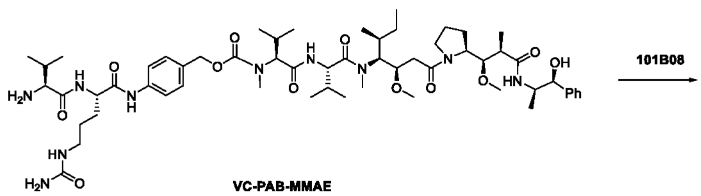


VC-PAB-MMAE

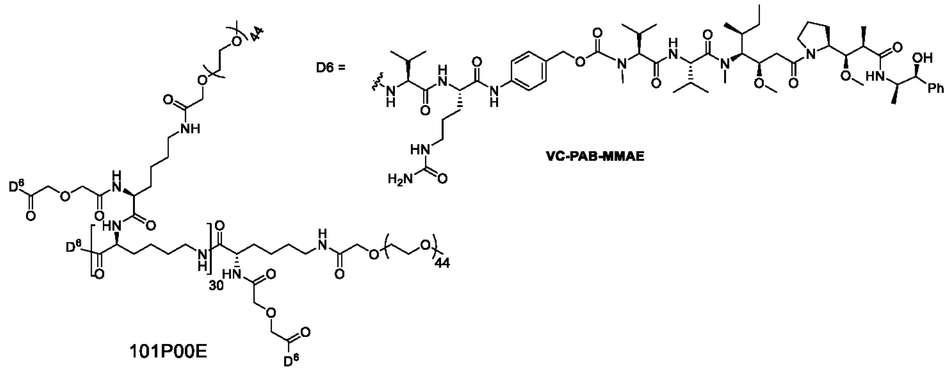


VC-PAB-MMAE

101P00E

at room temperature under nitrogen atmosphere, **VC-PAB-MMAE** (CAS: 644981-35-1) (0.54 g), PyBop (0.33 g) and DIPEA (0.18 g) were added to compound **101B08** (0.7 g) in DMF (6 mL). The mixture was stirred for 12 h at room temperature. MTBE was added. The precipitate was dissolved in 60 mL of methanol, and 70 mL of water was added. The mixture was purified by ultrafiltration and freeze-dried to give a final product 101P00E as light yellow solid (0.8 g).

HPLC analytical method D, RT=15.457 min.

Example 40: synthesis of compound 101T19

Synthesis of compound 101T02:

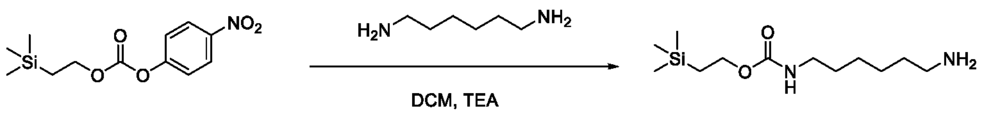


101T01 MW:283.36

101T02 MW:260.45

32 g of hexanediamine was dissolved in 120 mL of DCM. To the resultant solution, a solution of 15.60 g of 101T01 and 15 mL of triethylamine in DCM was added slowly with a syringe. The mixture gradually turned yellow, and was stirred for 15 h. TLC showed that the reaction was complete. The precipitated solid was dissolved by adding of DCM, and the organic phase was washed with water, saturated NaCl, saturated $NaHCO_3$, and 2M NaOH in succession, dehydrated and concentrated to get a light yellow liquid.

$^1H$ NMR (400 MHz, DMSO-$d_6$) δ 6.94 (t, *J* = 5.5 Hz, 1H), 4.16 - 3.84 (m, 2H), 2.92 (td, *J* = 7.0, 5.8 Hz, 2H), 2.48 (t, *J* = 6.7 Hz, 2H), 1.68 - 1.05 (m, 10H), 0.88 (s, 9H).

Synthesis of compound 101T03:

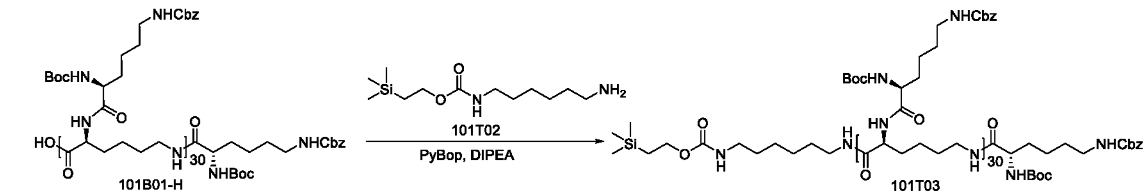


101B01 (8.541 g) was dissolved in 20 mL of DMSO/10 mL of DMF. To the resultant solution, 1.692 g of 101T02, 3.020 g of PyBop and 1.516 mL of DIPEA were added. The mixture was stirred at room temperature for 16 h under nitrogen atmosphere. 6 mL of 0.5 M NaOH and sufficient amount of ACN were added. The resultant precipitate was crushed, filtered, washed and vacuum dried to get 8.5 g of white powder.

$^1$H NMR (400 MHz, DMSO-$d_6$) δ 7.32 (d, *J* = 2.2 Hz, 144H), 4.99 (s, 61H), 4.16 (br, 29H), 4.00 (t, *J* = 8.3 Hz, 2H), 3.85 (br, 30H), 3.22 - 2.70 (m, 123H), 1.35 (s, 627H), 0.00 (s, 9H).

Synthesis of compound 101T04:

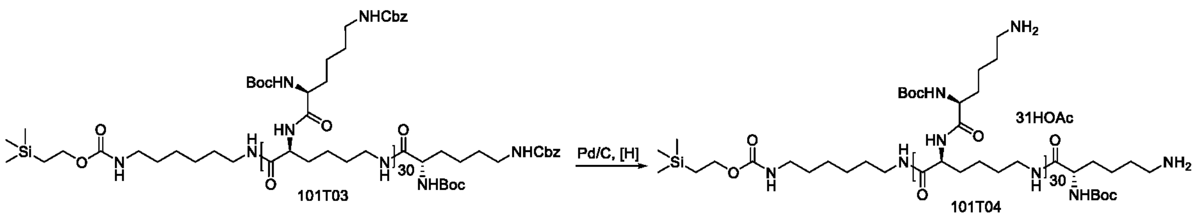


8.5 g of 101T03 was dissolved in 85 mL of acetic acid. To the resultant solution, 28 mL of methanol and 10% Pd/C 1.7 g of were added, and the mixture was purified by 24 h of hydrogenation under normal pressure, filtered, concentrated and dried in vacuo to obtain 8.57 g of light yellow solid. NMR spectrum contains 1.86 eq. HOAc (amount of acetic acid: 32.2 mmol in total, 14.85 mmol in excess).

$^1$H NMR (400 MHz, Deuterium Oxide) δ 4.37 - 4.09 (m, 31H), 3.97 (dd, *J* = 9.1, 5.3 Hz, 31H), 3.14 (s, 57H), 2.96 (t, *J* = 7.6 Hz, 55H), 1.98 (s, 168H), 1.86 - 1.13 (m, 635H), 0.00 (s, 9H).

Synthesis of compound 101T18:

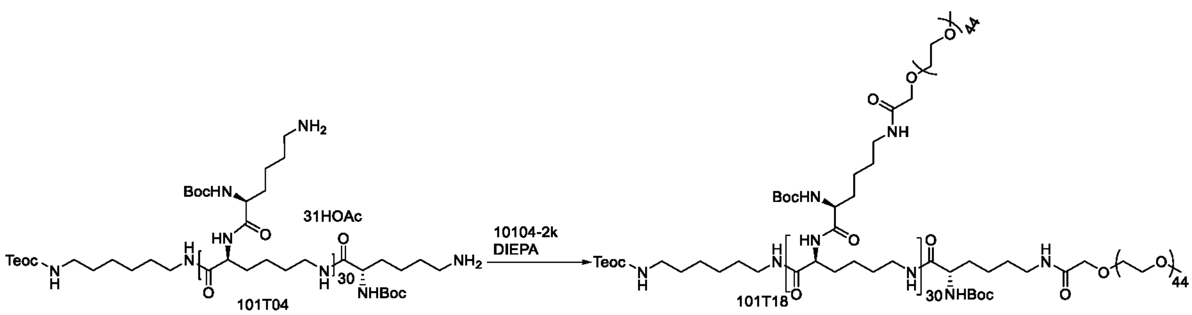


101T04 (3.79 g, content 84.4%, 7.68 mmol) was dissolved in 15.4 mL of methanol (0.5 M).

21.20 g of 10104-2k was dissolved in 20 mL of acetonitrile (0.5 M). Half of it was added to 101T04 methanol solution and stirred for 10 min at room temperature. The remaining 10104-2k solution and 2.676 mL of DIPEA were added, and the mixture was stirred for 4 h at room temperature. 175 mL of MTBE was gradually added to the reaction solution, the mixture was cooled down to 4°C for 15 min. The upper liquid was discarded, and 10 mL of acetonitrile was added to the lower oil. 150 mL of MTBE was added, and the mixture was cooled down to -20°C. The precipitate was vacuum dried, purified by ultrafiltration and freeze-dried to obtain 18.7 g of white powder (5.5 mmol, 98.9%, structural unit 2364).

$^1$H NMR (400 MHz, Deuterium Oxide) 4.17 (s, 37H), 4.02 (s, 98H), 3.66 (s, 5315H), 3.34 (s, 91H), 3.21 (s,

122H), 1.39 (s, 613H), 0.00 (s, 9H).

Synthesis of compound 101T19:

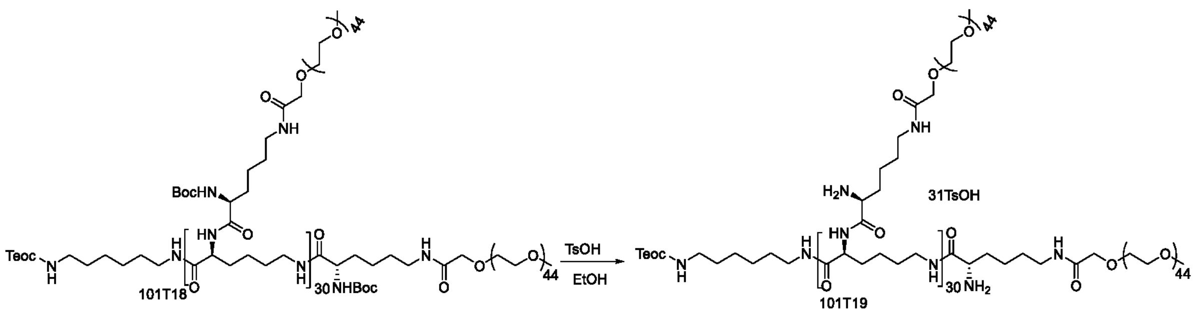


101T18 101T19

18.15 g of 101T18 was dissolved in 80 mL of ethanol; to the resultant solution, 1.679 g of *p*-TsOH·$H_2O$ was added and sonicated until dissolve. The reaction mixture was heated at 60°C for 2 h to remove the solvent with a rotovap. The mixture was concentrated and dried in vacuo to obtain the product.

$^1$H NMR (400 MHz, Deuterium Oxide) δ 7.65 (d, *J* = 8.0 Hz, 59H), 7.33 (d, *J* = 7.9 Hz, 64H), 4.20 (d, *J* = 8.2 Hz, 43H), 4.02 (s, 87H), 3.67 (s, 5160H), 3.35 (s, 94H), 3.19 (t, *J* = 6.9 Hz, 134H), 2.36 (s, 96H), 2.03 - 1.09 (m, 366H), 0.00 (s, 9H).

Example 41: synthesis of compound 101H00A

Synthesis of compound 101H07:

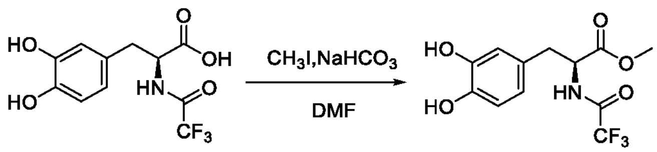


101H06 MW:293.20 101H07 MW:307.23

To a pre-heated dry 300 mL single-necked flask, 30 g of 101H06, 21.69 g of $Na_2CO_3$, and 300 mL of DMF were added; after three times exchanges of nitrogen, 29 g of $CH_3I$ was added with a syringe, and the mixture was stirred at room temperature overnight. LCMS showed the reaction was complete. Methanol was removed with a rotovap. The mixture was washed with 1 N HCl and extracted with EtOAc; the organic phase was washed with 1 N HCl and $H_2O$, dried with anhydrous sodium sulfate and concentrated to obtain the product.

Synthesis of compound 101H08:

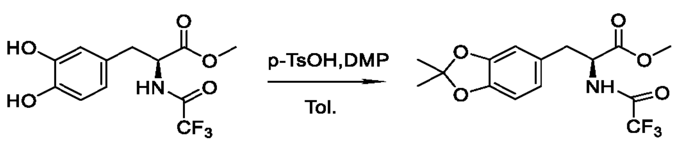


101H07 MW:307.23 101H08 MW:347.29

A 1 L three-neck flask was pre-heated and dried in vacuo and allowed to cooled down to room temperature under nitrogen atmosphere; to the flask, 40 g of 101H07, 64.04 mL of DMP, 3.71 g of *p*-TsOH·$H_2O$, and 500 mL of toluene were added; 40 g of 101H07, 64.04 mL of DMP, 3.71 g of *p*-TsOH·$H_2O$ and 500 mL of toluene were added.

After three times exchanges of nitrogen, the reaction was heated up to reflux at 130°C for overnight. LCMS showed the reaction was complete, and the toluene was removed with a rotovap. The mixture was purified by column chromatography (PE:EA=100:1~10:1), recrystallized with DCM/petroleum ether to give the product. Purity by HPLC: 98.7%.

$^1$H NMR (400 MHz, DMSO-$d_6$) δ 9.86 (d, *J* = 7.1 Hz, 1H), 6.85 - 6.69 (m, 2H), 6.63 (dd, *J* = 7.9, 1.7 Hz, 1H), 4.54 (ddd, *J* = 11.4, 7.0, 4.7 Hz, 1H), 3.67 (s, 3H), 3.09 (dd, *J* = 13.8, 4.9 Hz, 1H), 2.90 (dd, *J* = 13.9, 10.6 Hz, 1H), 1.60 (d, *J* = 5.8 Hz, 6H).

Synthesis of compound 101H09:

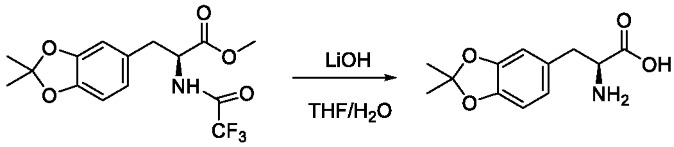


101H08 MW:347.29 101H09 MW:237.26

15 g of 101H08 was dissolved in 60 mL of THF. When the solution became clear, 65 mL of 2M LiOH aqueous solution was added. The mixture was stirred for 2 h of reaction at room temperature. THF was removed with a rotovap, and 6N HCl was added to adjust pH=5-6. The mixture was freeze-dried, dissolved with EtOAc, filtered and the filtrate was concentrated with a rotovap to get the product.

Synthesis of compound 101H17:

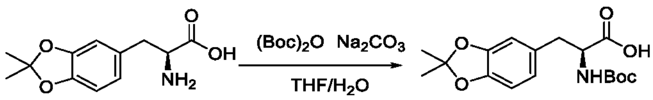


101H09 MW:237.26 101H17 MW:337.37

6 g of 101H09 was dissolved in 50 mL of THF, and 25 mL of 2 M $Na_2CO_3$ aqueous solution was added into the resultant solution. Under ice bath, 6.62 g of Boc anhydride was added and stirred for reaction at room temperature overnight.

TLC and LCMS showed the reaction was complete. THF was removed with a rotovap. The residue was washed with 1N HCl and extracted by EA. The organic phase was washed with 1N HCl and saturated NaCl in succession, dehydrated and concentrated. The excessive Boc anhydride was removed by chromatography with DCM column, and the eluate was concentrated with a rotovap to get the product.

Synthesis of compound 101H18:

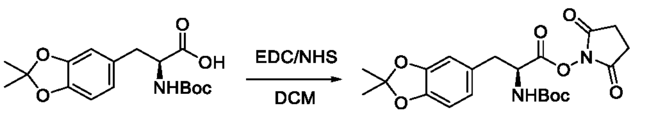


101H17 MW:337.37 101H18 MW:434.45

3 g of 101H17, 1.54 g of NHS and 2.56 g of EDCI were added to 20 mL of DCM at 0°C. The mixture was stirred for 4 h at room temperature. TLC and LCMS showed the reaction was complete. The reaction solution was washed with 1N HCl and saturated NaCl, dried with anhydrous sodium sulfate, purified by column chromatography (PE/EA, 100:1-10:1), and the eluate was concentrated to dry with a rotovap to get white solid.

Synthesis of compound 101H19:

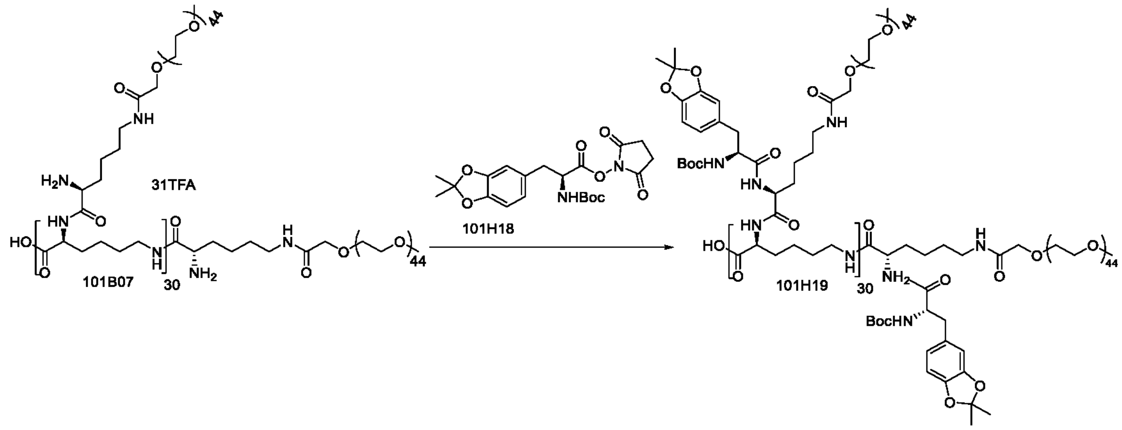


101B07 (3.0 g, 1.26 mmol) was dissolved in 16 mL ofDMF. To the resultant solution, 101H18 (1.729 g, 2.03 eq.) and DIPEA (1.344 mL) were added. The reaction was stirred for 18 h at room temperature. The reaction solution was added about 75 mL of MTBE. The precipitate was centrifuged, washed and dissolved in 3 mL of ACN. 40 mL of MTBE was added and the precipitate was dried under reduced pressure to obtain 3.4 g of white solid.

$^{1}$H NMR (400 MHz, Deuterium Oxide) $\delta$ 7.01 - 6.48 (m, 93H), 4.32 (s, 33H), 4.20 (d, *J* = 7.4 Hz, 67H), 4.03 (s, 55H), 3.71 (s, 4631H), 3.39 (s, 90H), 3.24 (s, 415H), 1.23 (s, 1098H).

Synthesis of compound 101H20:

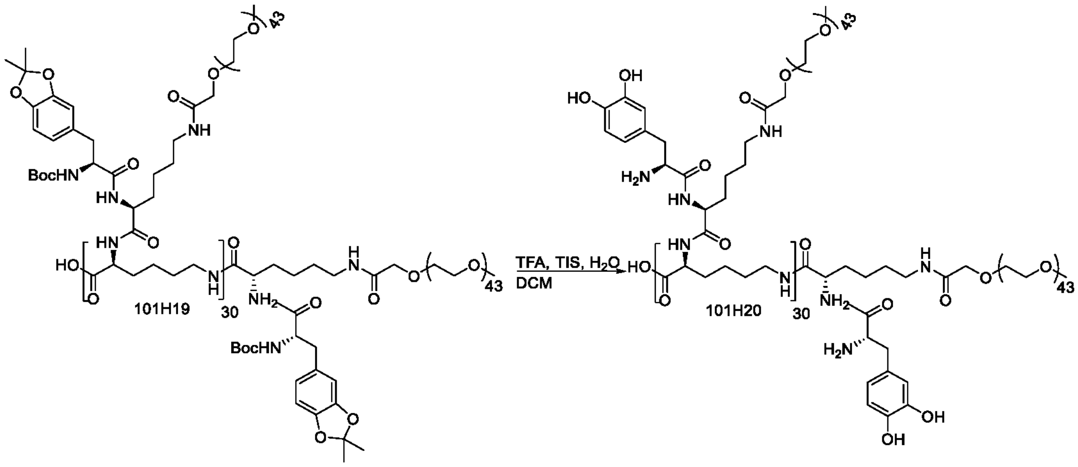


3.0 g of 101H19 was dissolved with 16 mL of DCM. To the resultant solution, 2.5% TIS, 0.6% water and 25% TFA were added. The mixture was stirred for 16 h at room temperature. The mixture was concentrated with a rotovap. The residue was added MTBE. The precipitate was dissolved in ACN and MTBE was added to the resultant solution. The precipitate was vacuum dried to obtain 2.463 g of 101H20 (83% yield), levodopa coupling% = 112.48/3/31 = 120%.

$^{1}$H NMR (400 MHz, Deuterium Oxide) $\delta$ 6.94 - 6.45 (m, 112H), 4.23 (s, 35H), 4.10 (d, *J* = 19.4 Hz, 62H), 3.94 (s, 53H), 3.62 (s, 4028H), 3.30 (s, 90H), 3.09 (s, 213H), 1.41 (t, *J* = 81.2 Hz, 351H).

Synthesis of compound 101H00A:

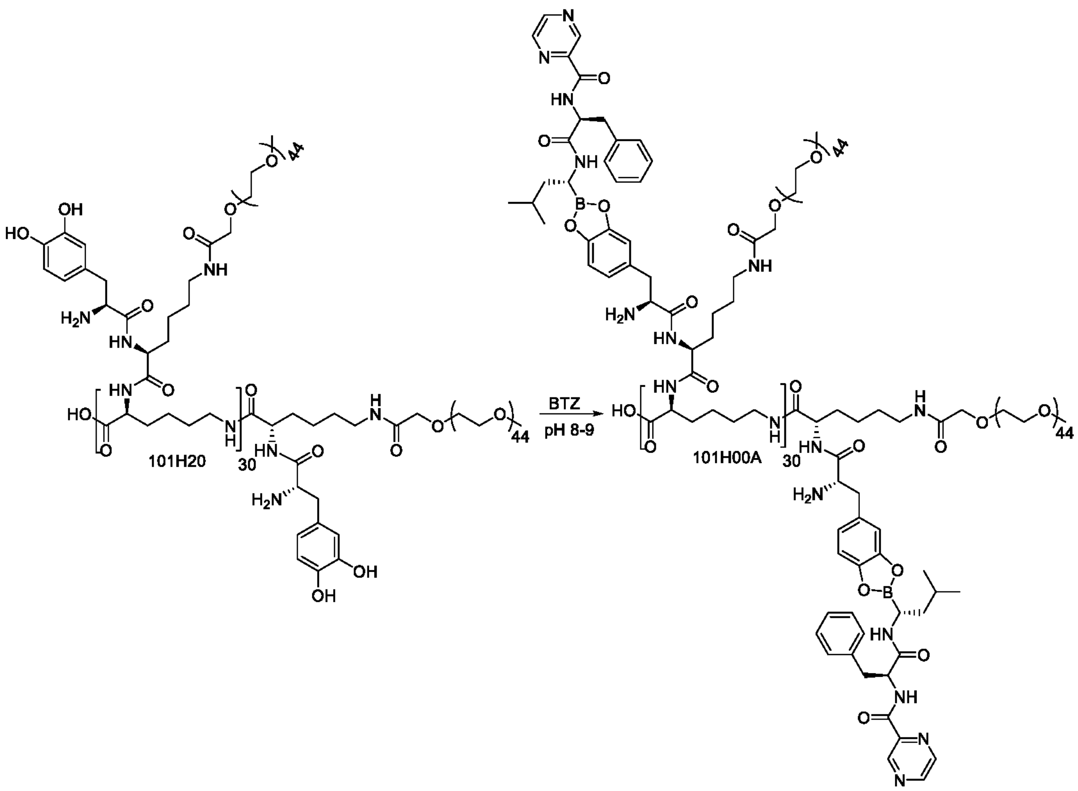


101H20

101H00A

201 mg of 101H20 and 30.0 mg of bortezomib were dissolved in 2 mL of acetonitrile in a 10 mL eggplant flask. The residue after the removal of solvent with a rotovap was added 3 mL of 20 mM pH 8.0 phosphate buffer and sonicated. The resultant solution was freeze-dried to obtain 101H00A as a white solid.

Example 42: synthesis of compound 101T00A-C4

Synthesis of compound 10137C:

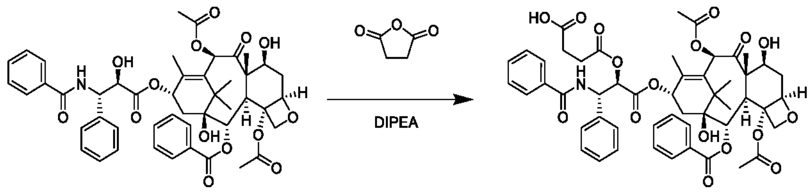


10137C

2 g of PTX and 0.4688 g of succinic anhydride were added 20 mL of absolute DCM and 1.632 mL of DIPEA. The mixture was stirred at 25°C for 4 h under nitrogen atmosphere. TLC (DCM/MeOH 15: 1) showed the reaction was complete. 20 mL DCM was added, and the mixture was washed with 0.5 M HCl (20 mL×2) and concentrated with a rotovap to obtain a white solid. The solid was re-slurred in 40 mL of water for 20 min, filtered, washed and freeze-dried to obtain 2.0909 g of product (Yield: 93.6%).

Synthesis of compound 101T00A-C4:

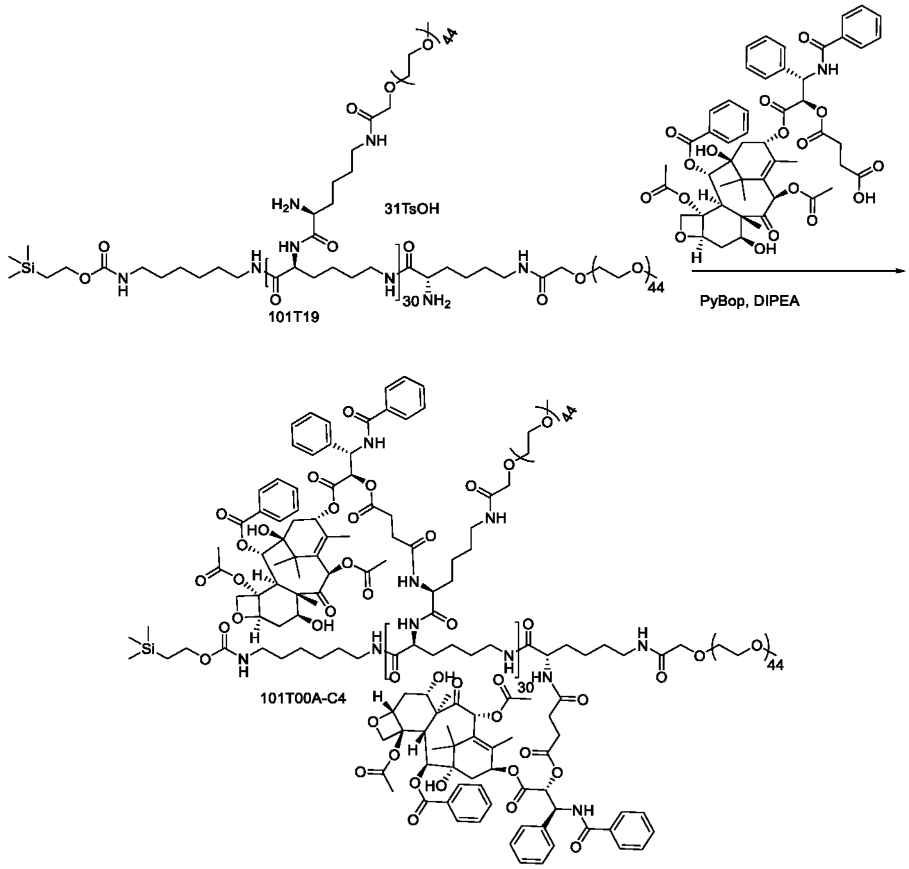


101T19

101T00A-C4

To 2.800 g of 101T19 was added to 15 mL of DMF. 1.644 g of 10137C (purity 86.8%, 1.89 g) was added at 0°C. 0.897 g of PyBop and 0.801 mL of DIPEA were added. The mixture was stirred for 16 h under nitrogen atmosphere. 25 mL of MTBE was added, and the precipitate was washed, centrifuged and dissolved in EtOAc. 25 mL of MTBE was added, and the precipitate was dissolved in 50 mL of methanol, purified by ultrafiltration with $MeOH/H_2O$, concentrated and freeze-dried to obtain 2.784 g of solid (structural unit 3156, 0.88 mmol, 77%), theoretical coupling content 853.9/3156=27 wt.%.

$^1H$ NMR (400 MHz, Methanol-$d_4$) $\delta$ 8.27 - 8.00 (61H), 8.00 - 7.10 (363H), 6.59 - 6.37 (28H), 6.21 - 5.99 (34H), 5.94 - 5.78 (31H), 5.77 - 5.37 (70H), 4.57 - 4.06 (155H), 4.05 - 3.90 (78H), 3.86 - 3.49 (5160H), 3.38 - 3.33 (91H), 3.27 - 3.08 (79H), 2.96 - 2.30 (255H), 2.28 - 0.80 (857H).

Example 43: synthesis of compound 101T00A-C5

Synthesis of compound 10137:

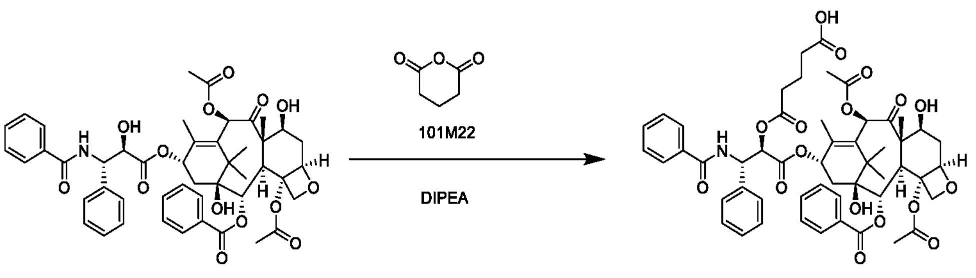


101M21

10137

1.5 g of PTX and 0.3007 g of 101M22 were added 15 mL of absolute DCM and 0.918 mL of DIPEA. The mixture was stirred at room temperature for 5 h under nitrogen atmosphere. TLC showed complete reaction. 10 mL of DCM was

added, and the mixture was washed with 1M HCl (20 mL×2), filtered, dehydrated and concentrated with a rotovap to obtain white solid. The solid was combined with the above filter cake, re-slurred in 50 mL of water for 30 min. The precipitate was filtered, washed and vacuum dried to obtain 1.6542 g of white solid powder(Yield: 97.3%; Purity by HPLC: 99.61%).

Synthesis of compound 101T00A-C5:

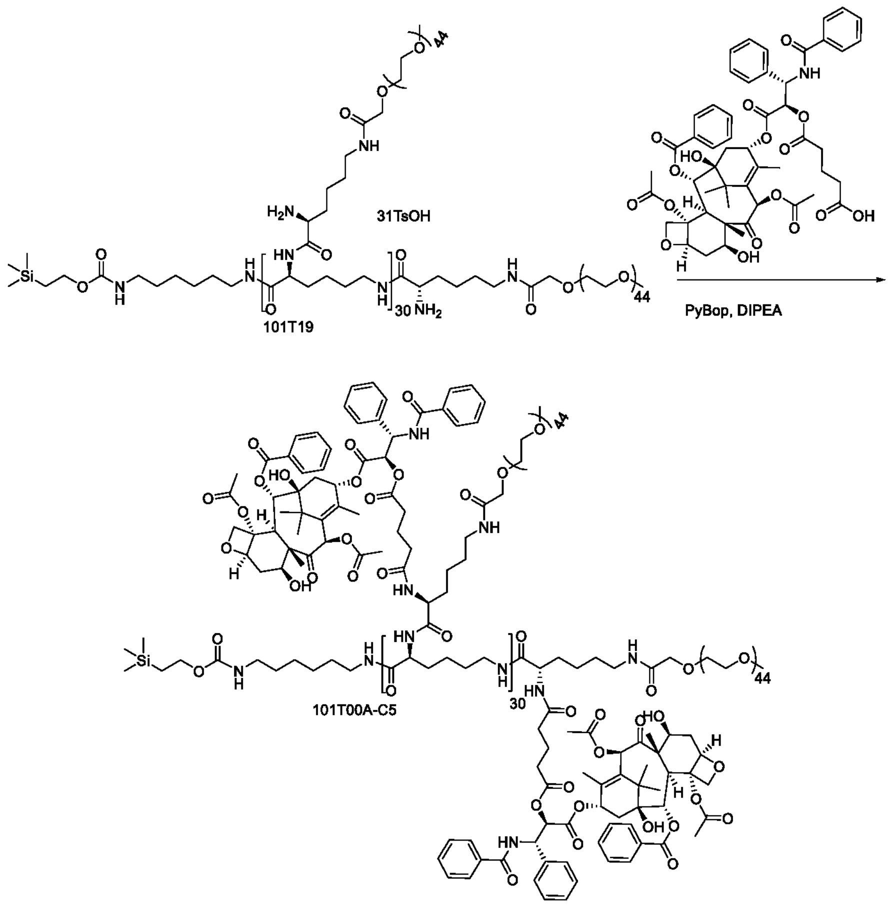


1.80 g of 101T19 was dissolved in 9 mL of DMF, and 10137 (1.076 g, 99.6% purity) was added at 0°C. 0.577 g of PyBop and 0.515 mL of DIPEA were added. The mixture was stirred for 15 h under nitrogen atmosphere. 25 mL of MTBE was added, and the resultant precipitate was washed, centrifuged and dissolved in EtOAc. 25 mL of MTBE was added, and the resultant precipitate was dissolved in 25 mL of methanol, purified by ultrafiltration with $MeOH/H_2O$, concentrated and freeze-dried to obtain 1.7794 g of white solid (structural unit 3170, 0.56 mmol, 76%). Theoretical coupling content: 853.9/3170=26.9 wt.%.

$^1H$ NMR (400 MHz, Methanol-$d_4$) $\delta$ 8.27 - 8.00 (65H), 8.00 - 7.10 (395H), 6.60 - 6.34 (34H), 6.20 - 5.99 (34H), 5.95 - 5.76 (34H), 5.74 - 5.56 (33H), 5.56 - 5.34 (37H), 5.13 - 4.92 (38H), 4.53 - 4.04 (158H), 4.04 - 3.87 (68H), 3.86 - 3.49 (5160H), 3.38 - 3.33 (83H), 3.27 - 2.93 (127H), 2.67 - 0.84 (1206H).

Example 44: synthesis of compound 101T00A-O

Synthesis of compound 10137O:

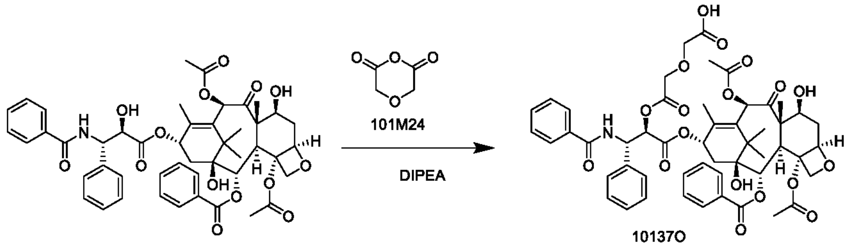


1.5 g of PTX and 0.3058 g of 101M24 were transferred into a 100 mL flask and, after three times exchanges of nitrogen, added 15 mL of absolute DCM and 0.918 mL of DIPEA. The mixture was stirred at 10°C for 6 h under nitrogen atmosphere. TLC showed complete reaction. 10 mL of DCM was added, and the mixture was washed with 1 M HCl (20 mL×2), filtered, dehydrated and concentrated with a rotovap to obtain white solid. The solid was combined with the above filter cake, cooled down to -20°C, and re-slurred in 50 mL of water for 30 min. The precipitate was filtered, washed and vacuum dried to obtain 1.6386 g of white solid powder (Yield: 96.2%).

Synthesis of compound 101T00A-O:

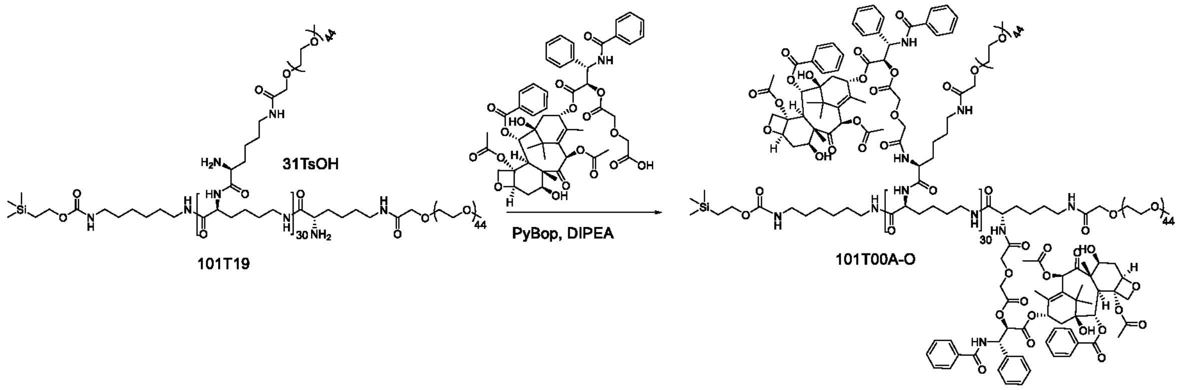


1.80 g of 101T19 was dissolved in 9 mL of DMF; the resultant solution was added 101370 (1.156 g; Purity: 92.6%), placed in ice bath, then added 0.577 g of PyBop and 0.515 mL of DIPEA. The mixture was stirred for 16 h under nitrogen atmosphere.

25 mL of MTBE was added, and the resultant precipitate was centrifuged, and the viscous liquid at the bottom layer was washed with 20 mL of 1:3 EtOAc/MTBE centrifuged and dissolved in 10 mL of EtOAc. 25 mL of MTBE was added, and the resultant precipitate was centrifuged and dried under reduced pressure; the crude product was dissolved in 25 mL of methanol, filtered through 0.45 $\mu$m membrane; the filtrate was added 25 mL of methanol and 50 mL of water, concentrated to 70 mL by ultrafiltration; the filter was washed with 700 mL of 50% methanol and the filtrate was pooled, concentrated and freeze-dried to obtain 1.7743 g of white solid (structural unit 3174, 0.56 mmol, 75.5%). Theoretical coupling content: 853.9/3174=26.9 wt.%.

$^1$H NMR (400 MHz, Methanol-$d_4$) $\delta$ 8.27 - 8.00 (61H), 8.00 - 7.10 (403H), 6.62 - 6.35 (27H), 6.25 - 6.00 (32H), 5.98 - 5.81 (31H), 5.77 - 5.42 (65H), 5.13 - 4.93 (34H), 4.67 - 4.00 (268H), 4.00 - 3.87 (69H), 3.86 - 3.50 (5160H), 3.38 - 3.33 (82H), 3.27 - 2.89 (117H), 2.71 - 0.79 (1032H).

Example 45: synthesis of compound 101T00A-S

Synthesis of compound 10137S:

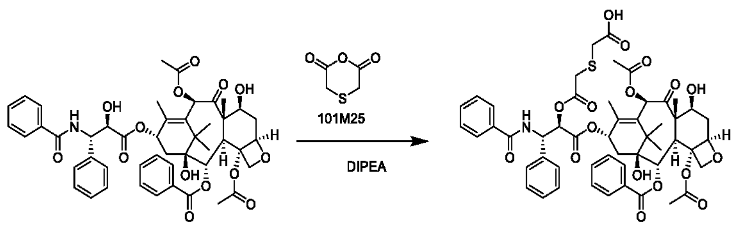


10137S

1.500 g of PTX and 0.6963 g of 101M25 were transferred in a 100 mL flask and, after three times nitrogen flushing operations, added 15 mL of absolute ACN and 1.836 mL of DIPEA. The mixture was stirred at 41°C for 5 h. TLC (DCM/MeOH 10:1) showed complete reaction. ACN was removed with a rotovap; the residue was dissolved with 25 mL of DCM, and washed with 1 M HCl (20 mL×2); the organic phase was filtered, dehydrated and concentrated with a rotovap to obtain solid. The solid was re-slurred in 50 mL of water for 30 min. The precipitate was filtered, washed and vacuum dried to obtain 1.58 g of product.

Synthesis of compound 101T00A-S:

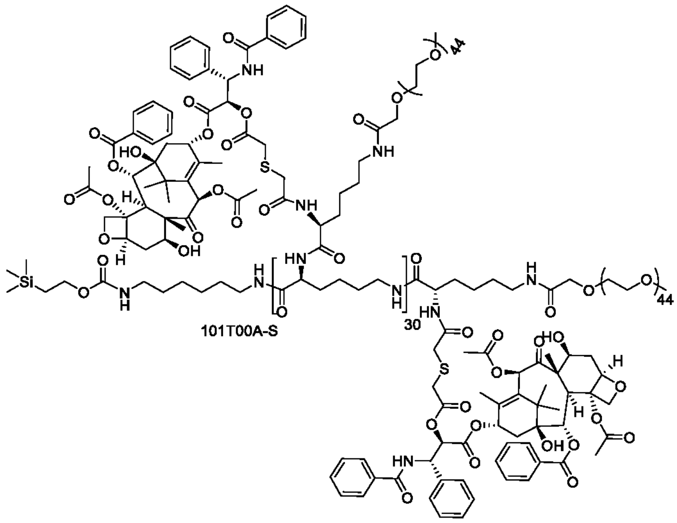

101T00A-S

1.800 g of 101T19 was dissolved in 9 mL of DMF; the resultant solution was added 10137S (1.365 g, 80% purity) and placed in ice bath; after the addition of 0.577 g of PyBop and 0.515 mL of DIPEA, the mixture was stirred for 16 h under nitrogen atmosphere. 25 mL of MTBE was added, and the precipitate was washed with 20 mL of 1:3 EtOAc/MTBE, centrifuged and dissolved in 10 mL of EtOAc. 25 mL of MTBE was added to the solution, and the resultant precipitate was centrifuged, dried under reduced presssure to get crude 101T00AS.

The crude 101T00A-S was heated up and dissolved in 25 mL of methanol, filter through 0.45 μm membrane; the filtrate was added 25 mL of MeOH and 50 mL of water to get an opalescent solution; 50 mL of MeOH was added to the solution, which was concentrated to 70 mL by ultrafiltration. The filter was washed with 700 mL of 67% MeOH and the filtrate was concentrated and freeze-dried to obtain 1.8641 g of light brown solid (structural unit 3206, 0.581 mmol, 78.6%). Theoretical coupling content: 853.9/3174=26.6 wt.%.

$^1$H NMR (400 MHz, Methanol-$d_4$) δ 8.27 - 8.00 (56H), 8.00 - 7.10 (362H), 6.57 - 6.33 (22H), 6.30 - 6.00 (37H), 6.00 - 5.79 (30H), 5.79 - 5.41 (65H), 5.10 - 4.94 (28H), 4.56 - 4.03 (138H), 4.02 - 3.86 (67H), 3.86 - 3.49 (5160H), 3.38 - 3.33 (103H), 3.26 - 2.93 (139H), 2.68 - 0.79 (1013H).

Example 46: synthesis of compound 101T00A-NMe

Synthesis of compound 10137N:

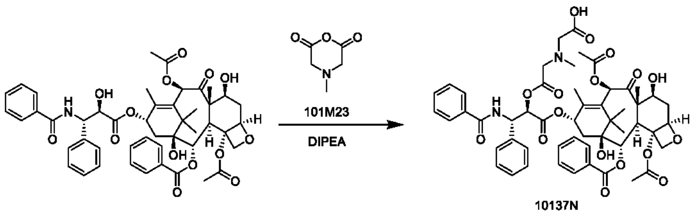


2.0087 g of PTX and 0.6075 g of 101M23 were added 20 mL of absolute ACN and 1.639 mL of DIPEA. The mixture was stirred at 25°C for 5 h. TLC showed complete reaction. The mixture was washed with 0.5 M HCl, filtered and vacuum dried to obtain 2.27 g of product.

Synthesis of compound 101T00A-NMe:

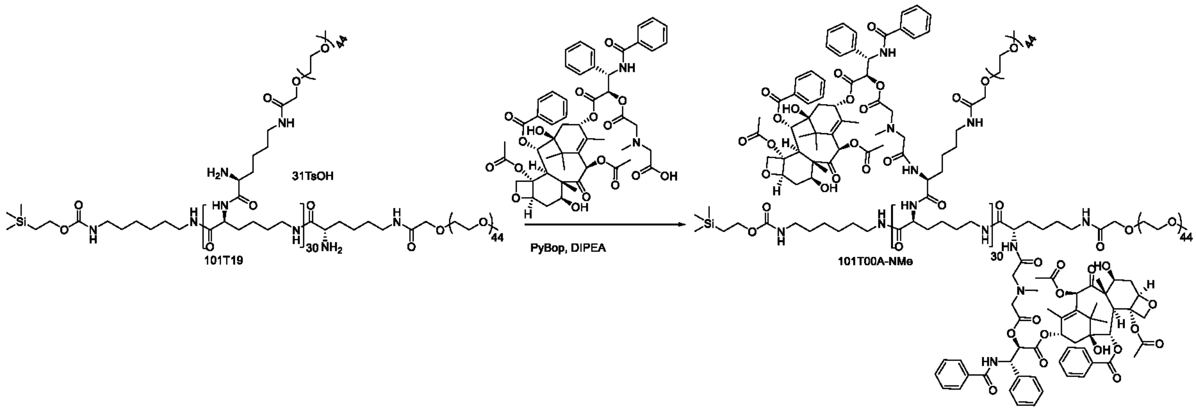


1.400 g of 101T19 was dissolved in 7.5 mL of DMF, and 10137N (0.911 g, 93% purity) was added at 0°C. 0.448 g of PyBop and 0.400 mL of DIPEA were added. The mixture was stirred for 16 h at room temperature under nitrogen atmosphere.

25 mL of MTBE was added, and the precipitate was washed with EtOAc/MTBE, centrifuged and dissolved in EtOAc. 25 mL of MTBE was added, and the precipitate was dissolved in 25 mL of methanol. The mixture was purified by ultrafiltration with MeOH/$H_2O$, concentrated and freeze-dried to obtain 1.4407 g of white solid (structural unit 3187, 0.452 mmol, 79.3%). Theoretical coupling content: 853.9/3187=26.9 wt.%.

$^1H$ NMR (400 MHz, Methanol-$d_4$) $\delta$ 8.27 - 8.00 (60H), 7.99 - 7.10 (379H), 6.60 - 6.35 (30H), 6.25 - 5.99 (33H), 5.99 - 5.81 (31H), 5.76 - 5.52 (56H), 5.19 - 4.93 (36H), 4.56 - 4.04 (154H), 4.04 - 3.87 (71H), 3.86 - 3.49 (5160H), 3.38 - 3.33 (82H), 3.27 - 2.94 (155H), 2.64 - 0.82 (1073H).

Example 50: determination of PTX coupling content

Preparation of polymer solution:

101T00A-C4, 101T00A-C5, 101T00A-O, 101T00A-S, 101T00A-NMe were weighed in sufficient amount into volumetric flasks, topped up to the volume of 10.00 mL with ACN. The ACN was weighed. The average ACN density calculated from the weight data of 6 bottles was 0.7765 g/$cm^3$, and the ACN volume added was calculated by the solution mass data.

| No. | mg | ACN/ g | Vol/m L | mg/m L | From No. | stock/ g | Total/ g | Sampl e | μg/ mL |
|---|---|---|---|---|---|---|---|---|---|
| 101T00A-C4 | 14.8 6 | 7.793 9 | 10.04 | 1.480 | C4 | 0.684 5 | 2.905 3 | 1 | 348. 8 |
| 101T00A-C5 | 16.1 6 | 7.633 6 | 9.831 | 1.644 | C5 | 0.698 5 | 3.048 3 | 2 | 376. 7 |
| 101T00A-O | 17.8 2 | 7.802 0 | 10.05 | 1.774 | O | 0.648 6 | 2.771 0 | 3 | 415. 1 |

(continued)

| No. | mg | ACN/ g | Vol/m L | mg/m L | From No. | stock/ g | Total/ g | Sampl e | μg/ mL |
|---|---|---|---|---|---|---|---|---|---|
| 101T00A-S | 15.9 8 | 7.798 5 | 10.04 | 1.591 | S | 0.640 1 | 2.849 1 | 4 | 357. 5 |
| 101T00A-NMe | 15.7 8 | 7.779 5 | 10.02 | 1.575 | N | 0.661 4 | 2.923 1 | 5 | 356. 4 |

Preparation of PTX solution:

| | mg | ACN/g | Vol/m L | mg/mL | From No. | stock/g | Total/g | Sam -pie | μg/mL |
|---|---|---|---|---|---|---|---|---|---|
| PTX | 17.45 | 7.7826 | 10.02 | 1.741 | 6 | 1.0484 | 4.3199 | 7 | 422.5 |
| | | | | | 7 | 1.1377 | 2.2308 | 8 | 215.5 |
| | | | | | 7 | 1.1842 | 4.4530 | 9 | 112.4 |
| | | | | | 9 | 1.0736 | 2.1826 | 10 | 55.27 |

HPLC standard curve of PTX: y=8.776x+485.47, $R^2$=0.9984

Calculation of PTX conjugate content by HPLC:

| No. | polymer | Area | PTX | PTX (HPLC) | PTX |
|---|---|---|---|---|---|
| | μg/mL | mAU | μg/mL | measured value wt.% | theoratical value wt.% |
| 1 | 348.8 | 1150.8 | 75.81 | 21.7 | 27.0 |
| 2 | 376.7 | 1289.7 | 91.63 | 24.3 | 26.9 |
| 3 | 415.1 | 1404.4 | 104.7 | 25.2 | 26.9 |
| 4 | 357.5 | 1209.5 | 82.50 | 23.1 | 26.6 |
| 5 | 356.4 | 1252.1 | 87.36 | 24.5 | 26.8 |

10-15 mg of polymer was dissolved in $CD_3OD$. PEG was calculated as 44 repetitive units. Since the same batch of starting material 101T19 was used in the synthesis, the integration value of PEG in 101T19 was fixed at 5159.96. The integration range of repetitive units $-CH_2CH_2O-$ in PEG was fixed at 3.49-3.86, and the coupling number of PEG chains in polymers was: PEG 3.49-3.86 integration value/(44*4)=29.

The number of hydrogens on the PTX aromatic ring was 15, and the integration value of hydrogen in 7.10-8.00 range should be 13. The coupling number of PTX: PTX 7.10-8.00 integration value/13.

$$\text{wt.\%} = \frac{\text{coupling number of PTX} \times (\text{Mw of PTX} - 1)}{\text{Mw of repeating unit of polymer} \times 31} \times 100\%$$

coupling content of PTX

Calculation of PTX conjugate content by NMR:

| No. | PEG integration value | Integration range ppm | PEG/po -lymer | PTX integration value | Integration range ppm | PTX/po -lymer | PTX wt.% |
|---|---|---|---|---|---|---|---|
| 101T00 A-C4 | 5159.96 | 3.49-3.86 | 29 | 362.7 | 7.10-8.00 | 27.9 | 24.3 |
| 101T00 A-C5 | 5159.96 | 3.49-3.86 | 29 | 394.8 | 7.10-8.00 | 30.4 | 26.5 |
| 101T00 A-O | 5159.96 | 3.49-3.86 | 29 | 402.9 | 7.10-8.00 | 31.0 | 27.0 |

(continued)

| No. | PEG integration value | Integration range ppm | PEG/po -lymer | PTX integration value | Integration range ppm | PTX/po -lymer | PTX wt.% |
|---|---|---|---|---|---|---|---|
| 101T00 A-S | 5159.96 | 3.49-3.86 | 29 | 362.1 | 7.10-8.00 | 27.9 | 24.3 |
| 101T00 A-NMe | 5159.96 | 3.49-3.86 | 29 | 379.3 | 7.10-8.00 | 29.2 | 25.4 |

Example 51: synthesis of compound 101N19

Synthesis of compound 101N01:

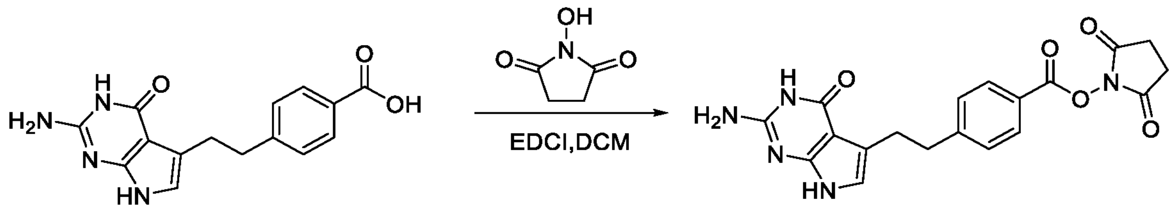


101N-SM 101N01

101N-SM (3.2 g, 0.01 mol), NHS (1.36 g, 0.011 mol), DCC (2.43 g, 0.011 mmol) and DMF (60 mL) were added into a 100 mL single-neck flask, and the mixture was stirred at room temperature for 16 h. LCMS showed the reaction was complete. The precipitate was filtered, washed with dichloromethane, dried under reduced pressure to obtain a crude product. The crude product was re-slurred with 20% isopropanol/ethyl acetate for 2 h, filtered, washed and dried to obtain 3.89 g of white solid (Yield: 92.6%; Purity: 95.6%).

Synthesis of compound 101N02:

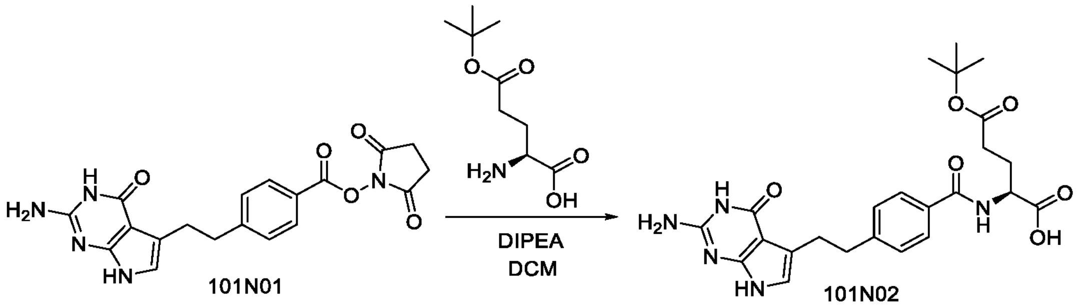


101N01 101N02

101N01 (1 g, 2.53 mmol), Z-glutamic acid-5-tert-butyl ester (565 mg, 2.78 mmol), DIPEA (0.98 g, 7.58 mmol) and DMF (45 mL) were added into a 100 mL single-neck flask, and the mixture was stirred at room temperature for 16 h under nitrogen atmosphere. LCMS showed the reaction was complete. The reaction mixture was directly concentrated to obtain 3 g of crude product. The crude product was re-slurred in 1N hydrochloric acid (50 mL) for 30 min, filtered, washed and dried to obtain 1.07 g of white solid (Yield: 87.7%; LCMS chromatogram was correct; Purity by HPLC: 91.2%).

$^1$H NMR (400 MHz, DMSO-$d_6$) δ 11.25 (s, 1H), 8.52 (d, *J* = 7.7 Hz, 1H), 7.80 (d, *J* = 8.1 Hz, 1H), 7.29 (d, *J* = 8.1 Hz, 1H), 6.46 (d, *J* = 2.2 Hz, 1H), 4.39 (ddd, *J* = 9.9, 7.7, 4.8 Hz, 2H), 3.12 - 2.83 (m, 2H), 2.33 (t, *J* = 7.6 Hz, 1H), 2.17 - 1.85 (m, 1H), 1.39 (s, 3H).

Synthesis of compound 101N03:

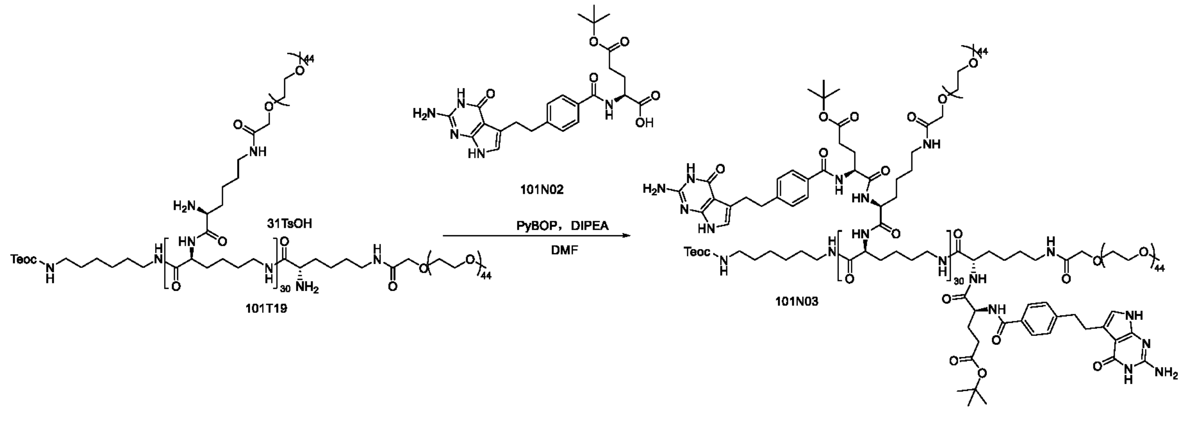


To a 10 mL single-neck flask was added 101T19 (1 g, 0.41 mmol), 101N02 (298 mg, 0.62 mmol) and DMAC (15 mL), dissolve by ultrasonic, cooled to 0°C with ice bath. And then PyBOP (321 mg, 0.62 mmol) and DIPEA (213 mg, 1.64 mmol) was added. The mixture was stirred at 0°C for 10 min, then at room temperature and for 16 h under nitrogen atmosphere. HPLC showed the reaction was complete. The reaction mixture was precipitated in -68°C MTBE (80 mL), centrifuged and dried to obtain 1.21 g of yellow solid. The solid was dissolved in 50 mL of methanol and purified by ultrafiltration with methanol: water =1:1. The filtrate was freeze-dried to obtain 954 mg of light yellow solid (Purity: 99.32%).

$^1H$ NMR (400 MHz, Methanol-$d_4$) $\delta$ 7.71 (d, $J$ = 26.5 Hz, 60H), 7.24 (s, 62H), 6.30 (s, 27H), 4.68 - 4.05 (m, 158H), 4.05 - 3.85 (m, 92H), 3.84 - 3.37 (m, 7128H), 3.37 - 3.25 (m, 334H), 3.23 - 2.59 (m, 226H), 2.57 - 2.27 (m, 73H), 2.25 - 0.90 (m, 872H), 0.00 (s, 9H).

Synthesis of compound 101N19:

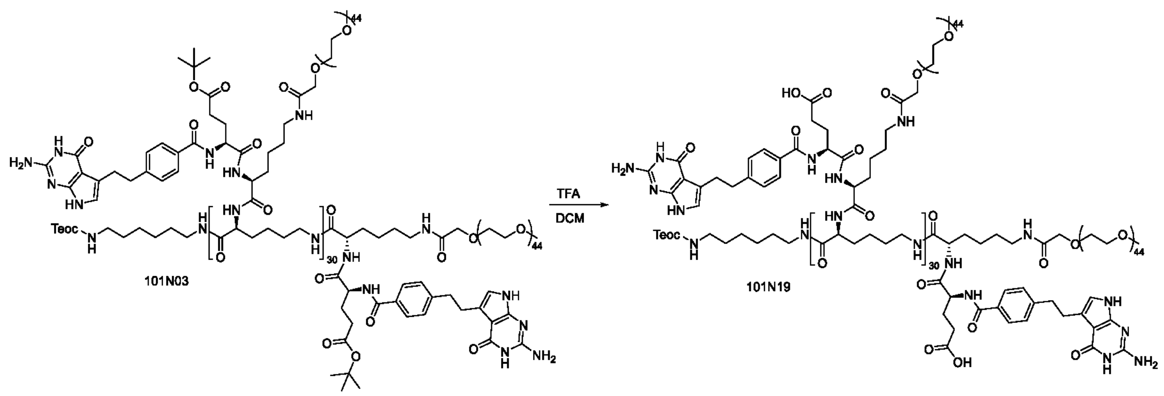


101N03 (954 mg, 0.011 mmol), trifluoroacetic acid (3 mL) and DCM (12 mL) were charged into a 50 mL single-neck flask, and the mixture was stirred at room temperature for 2 h. HPLC showed the reaction was complete. The reaction mixture was directly concentrated and dried to obtain a crude product. The crude product was added MTBE (10 mL), sonicated, and centrifuged; the resultant supernatant was discarded, and the solid was vacuum dried to obtain 901 mg of brown solid (Purity: 98.55%).

$^1H$ NMR (400 MHz, Methanol-$d_4$) $\delta$ 8.04 - 7.56 (m, 60H), 7.49 - 7.05 (m, 61H), 6.69 - 6.26 (m, 23H), 4.67 - 4.12 (m, 111H), 4.11 - 3.87 (m, 66H), 3.86 - 3.41 (m, 4963H), 3.40 - 3.29 (m, 492H), 3.27 - 3.13 (m, 318H), 3.10 - 2.83 (m, 119H), 2.66 - 2.40 (m, 72H), 2.35 - 2.07 (m, 69H), 1.96 - 1.26 (m, 344H).

Example 47: synthesis of compound 101N20

Synthesis of compound 101N04:

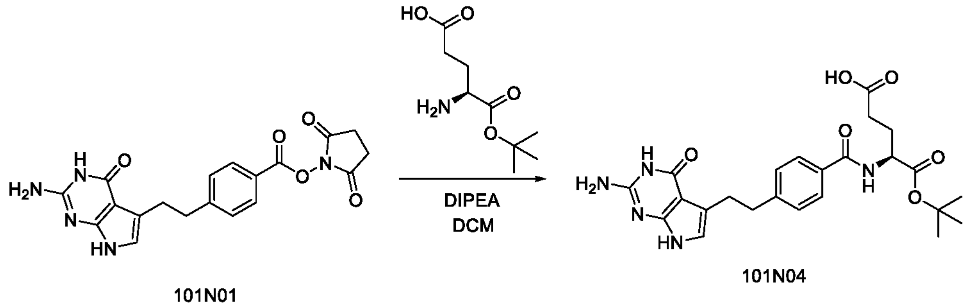


101N01

101N04

101N01 (760 mg, 1.9 mmol), 1-tert-butyl-L-glutamic acid (586 mg, 2.88 mmol), DIPEA (745 mg, 5.7 mmol) and DMF (40 mL) were charged into a 50 mL single-neck flask, and the mixture was stirred at room temperature for 16 h under nitrogen atmosphere. LCMS showed the reaction was complete. 1N hydrochloric acid was added to adjust pH to 4. The mixture was washed with dichloromethane for three times, and the combined organic phase was washed with saturated sodium chloride solution; the water phase and organic phase were tested by LCMS; it was found that the product was in the water phase, which was then concentrated with a rotovap to obtain 1.11 g of a crude product.

$^1$H NMR (400 MHz, DMSO-$d_6$) δ 12.18 (s, 1H), 10.61 (d, J= 2.2 Hz, 1H), 10.15 (s, 1H), 8.54 (d, *J* = 7.5 Hz, 1H), 7.78 (d, *J* = 8.1 Hz, 2H), 7.29 (d, *J* = 8.0 Hz, 2H), 6.31 (d, *J* = 2.1 Hz,1H), 6.00 (s, 2H), 4.32 (ddd, *J* = 9.6, 7.4, 5.1 Hz, 1H), 2.98 (dd, *J* = 9.4, 6.1 Hz, 2H), 2.86 (dd, *J* = 9.4, 6.1 Hz, 2H), 2.36 (t, *J* = 7.5 Hz, 2H), 2.12 - 1.88 (m, 2H), 1.41 (s, 9H).

Synthesis of compound 101N05:

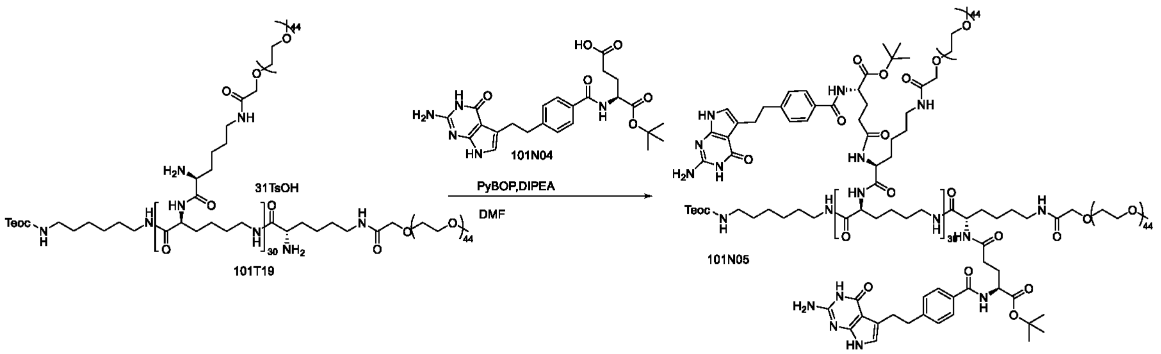


101T19 (1 g, 0.41 mmol) and 101N04 (298 mg, 0.62 mmol) were added into and sonicated to dissolve in DMAC (15 mL) in a 10 mL single-neck flask; the reaction system was cooled down to 0°C and PyBOP (321 mg, 0.62 mmol), DIPEA (213 mg, 1.64 mmol) were added. The mixture was stirred at 0°C for 10 min and at room temperature for 16 h under nitrogen atmosphere. HPLC showed the reaction was complete. The reaction mixture was precipitated with -68°C MTBE (80 mL). The precipitate was centrifuged and dried to obtain 1.21 g of gray solid. The solid was dissolved in 50 mL of methanol and purified by ultrafiltration with methanol: water =1:1. The filtrate was freeze-dried to obtain 951 mg of light yellow solid (Purity: 99.86%).

$^1$H NMR (400 MHz, Methanol-$d_4$) δ 7.72 (s, 60H), 7.24 (s, 62H), 6.32 (s, 36H), 4.65 - 4.13 (m, 153H), 3.90 (s, 64H), 3.77 (t, *J* = 4.9 Hz, 48H), 3.59 (s, 5210H), 3.37 - 3.23 (m, 345H), 3.12 (d, *J* = 16.7 Hz, 89H), 2.96 (s, 131H), 2.41 (s, 63H), 2.01 (s, 41H), 2.17 (dd, *J* = 17.4, 10.0 Hz, 43H),1.64 (d, *J* = 58.7 Hz, 128H), 1.41 (s, 540H), 0.00 (s,9H).

Synthesis of compound 101N20:

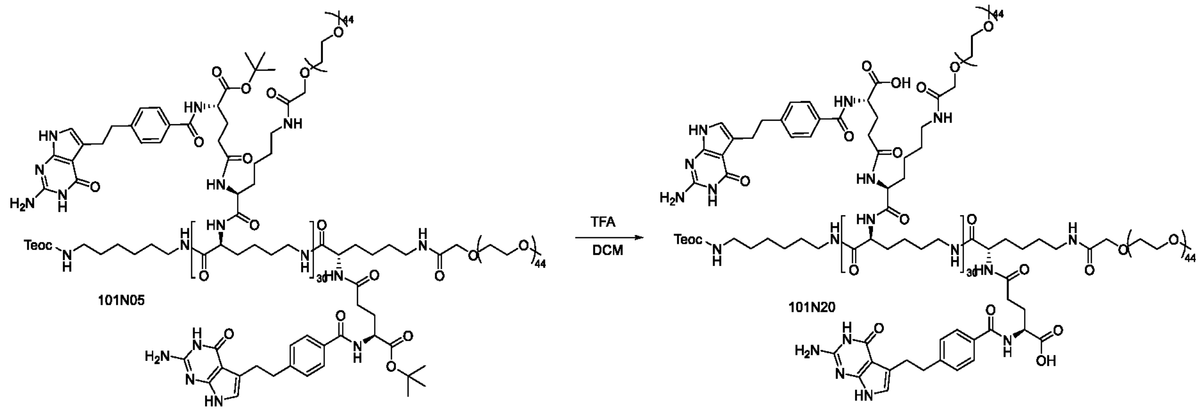


101N05 101N20

101N05 (951 mg, 0.011 mmol) was added into trifluoroacetic acid (3 mL) and DCM (12 mL) in a 50 mL single-neck flask, and the mixture was stirred at room temperature for 2 h. HPLC showed the reaction was complete. The reaction mixture was directly concentrated and dried to obtain a crude product. The crude product was added MTBE (10 mL), sonicated, and centrifuged; the supernatant was discarded and the solid was vacuum dried to obtain 820 mg of brown solid (Purity: 99%).

$^1$H NMR (400 MHz, Methanol-$d_4$) δ 7.80 (s, 60H), 7.30 (s, 60H), 6.46 (s, 27H), 4.62 (s, 32H), 4.33 (d, *J* = 26.9 Hz, 53H), 3.97 (s, 57H), 3.64 (d, *J* = 4.7 Hz, 4032H), 3.37 (s, 62H), 3.33 (p, *J* = 1.7 Hz, 298H), 3.27 - 2.83 (m, 185H), 2.50 (s, 58H), 2.27 (d, *J*= 44.3 Hz, 73H), 1.95 - 1.01 (m, 336H).

Example 48: synthesis of compound 101J00A-O

Synthesis of compound 10133:

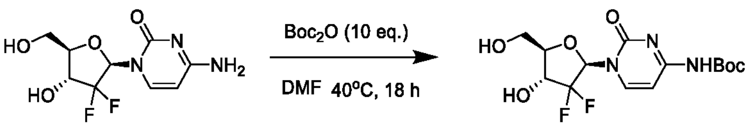


101M20 10133

41.46 g of $(Boc)_2O$ was added to a solution of 5.00 g of 101M20 in DMF, and the mixture was stirred at 37°C for 18 h. MS peak [M-tBu+H]=364. 400 mL of water and 200 mL of PE/EA (1:1) was added to the mixture. The white precipitate was filtered, washed and vacuum dried to obtain 6.3160 g of white solid powder (Yield: 91.5%).

$^1$H NMR (400 MHz, DMSO) δ 10.50 (s, 1H), 8.18 (d, *J* = 7.6 Hz, 1H), 7.07 (d, *J* = 7.6 Hz, 1H), 6.35 - 6.24 (m, 1H), 6.16 (t, *J* = 7.5 Hz, 1H), 5.29 (t, *J* = 5.5 Hz, 1H), 4.18 (tt, *J* = 12.8, 6.9 Hz, 1H), 3.88 (dt, *J*= 8.5, 3.0 Hz, 1H), 3.85 - 3.60 (m, 2H), 1.46 (s, 9H).

Synthesis of compound 10134:

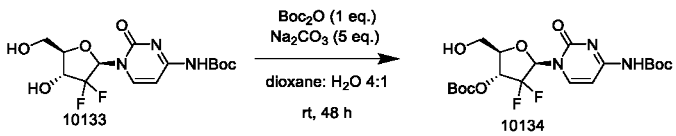


10133 10134

9.077 g of sodium carbonate was dissolved in 62 mL of water in a 500 mL flask; to the solution, 6.2227 g of compound 10133 was added. A solution of 3.738 g of $(Boc)_2O$ in 249 mL of dioxane was added to the above mixture to form white precipitate. The mixture was stirred at 30°C for 48 h. 250 mL of water was added, the mixture was extracted with ethyl acetate (250 mL, 150 mL×2). The organic phase was washed with saturated NaCl (100 mL), dried over anhydrous sodium sulfate, concentrated and vacuum dried. The mixture was purified by silica gel column chromatography

(PE: EA=2:1~1:1, then DCM: MeOH=20:1). The product was dried in vacuo to obtain 6.250 g of white solid powder (Yield: 78.7%).

$^1H$ NMR (400 MHz, DMSO) δ 10.57 (s, 1H), 8.11 (d, *J* = 7.7 Hz, 1H), 7.10 (d, *J* = 7.7 Hz, 1H), 6.28 (t, *J* = 8.7 Hz, 1H), 5.32 (dd, *J* = 6.4, 5.2 Hz, 1H), 5.28 - 5.13 (m, 1H), 4.24 (dt, *J* = 7.0, 3.4 Hz, 1H), 3.79 (ddd, *J* = 12.7, 5.3, 3.2 Hz, 1H), 3.68 (ddd, *J* = 12.7, 6.5, 3.9 Hz, 1H), 1.47 (d, *J* = 2.9 Hz, 18H).

Synthesis of compound 10135O:

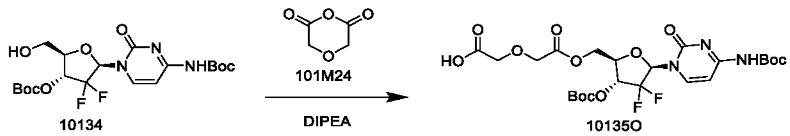


1.100 g of 10134 and 0.5510 g of 101M24 were charged into a 50 mL flask and 11 mL of absolute DCM and 1.654 mL of DIPEA were added into the flask under nitrogen atmosphere. The mixture was stirred at 25°C for 5 h. Afterwards, the mixture was washed with 0.5 M HCl for 3 times, then washed with water and vacuum dried to obtain 1.3342 g of white powder (Yield: 97.0%).

Synthesis of compound 101J11O:

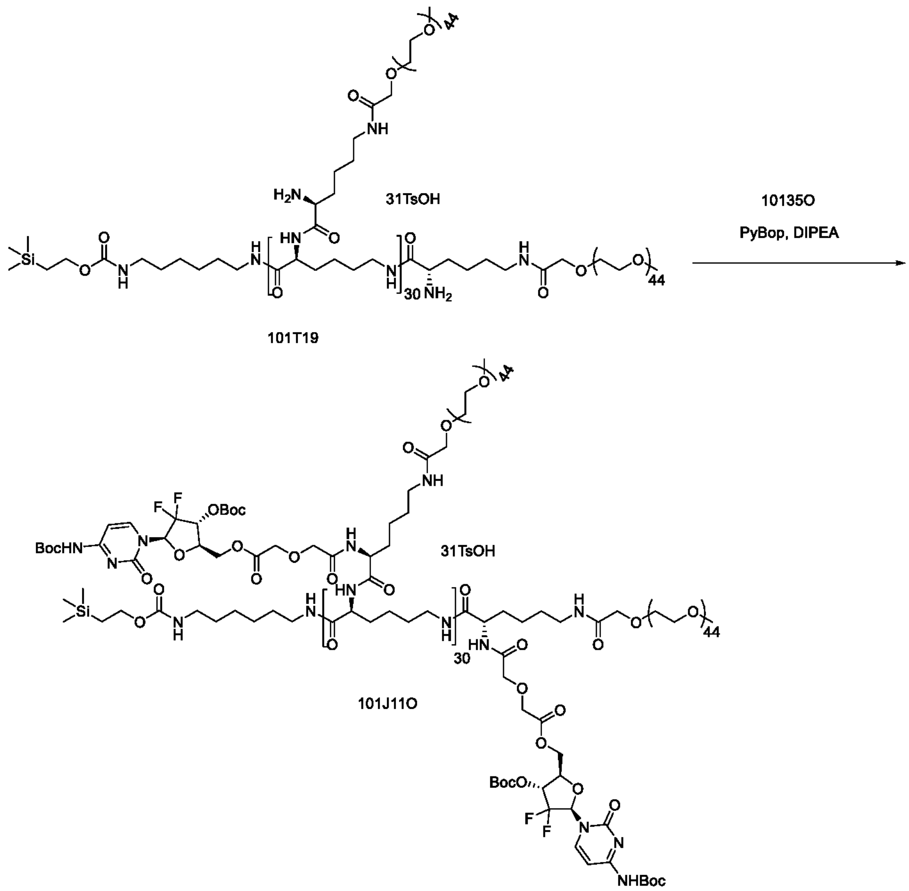


To a solution of 150 mg of 101T19 in DMF, 53.5 mg of compound 101350 was added, and the reaction was placed in ice bath; 20 min later, 48 mg of PyBop and 43 μL of DIPEA were added. The mixture was stirred at room temperature for 18 h under nitrogen atmosphere. MTBE was added, the resultant precipitate was centrifuged and vacuum

dried to give a crude product. The crude product was dissolved in 5 mL of methanol, the solution was filtered through 0.45 μm membrane and the filtrate was added 5 mL of water. The product was purified by ultrafiltration and freeze-dried to obtain 138 mg of white solid.

Synthesis of compound 101J00A-O:

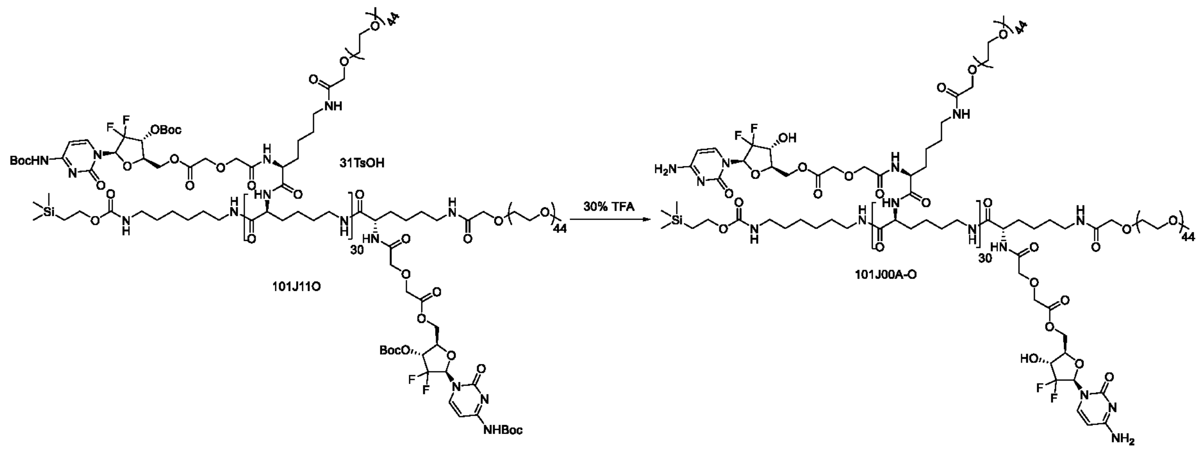


101J11O (138 mg), trifluoroacetic acid (3 mL) and DCM (7 mL) were mixed and allowed to react at room temperature for 8 h under stirring. Afterwards, the reaction mixture was concentrated and added MTBE; the precipitate was centrifuged and vacuum dried to obtain 101J00A-O as a white solid.

Example 49: synthesis of compound 101T28

Synthesis of compound 101L09:

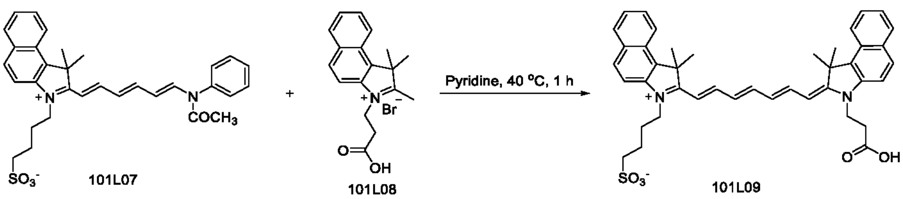


0.470 g of compound 101L07 and 0.2988 g of compound 101L08 were dissolved in 4.7 mL of pyridine. The mixture was stirred at 40°C under nitrogen atmosphere. The solution gradually turned green. After 1 h, MTBE was added, the precipitate was re-slurred in acetone and vacuum dried to obtain 0.661 g of a dark green powder product (Yield: 99.4% (Mw=768, pyridinium salt); Purity by HPLC: 87.47%).

Synthesis of compound 101L10:

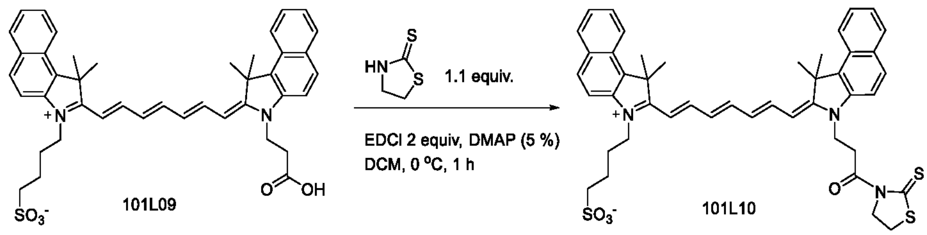


0.662 g of 101L09 was suspended in 12 mL of DCM; to the suspension, 0.246 g of 2-mercaptothiazoline and 5.9 mg of DMAP were added. The reaction system was placed in ice batch, and 686 mg of EDCI was added. Then the reaction system was removed from the ice batch and allowed to react at room temperature under stirring for 2 h. The reaction was complete. MTBE was added, the precipitate was dissolved in 6 mL of DCM. To the solution, 90 mL of acetone was added, the precipitate was centrifuged and re-slurred in 5 mL of acetone for 40 min. The precipitate was centrifuged and vacuum dried to obtain 0.7200 g of product (Yield: 95%).

Synthesis of compound 101T28:

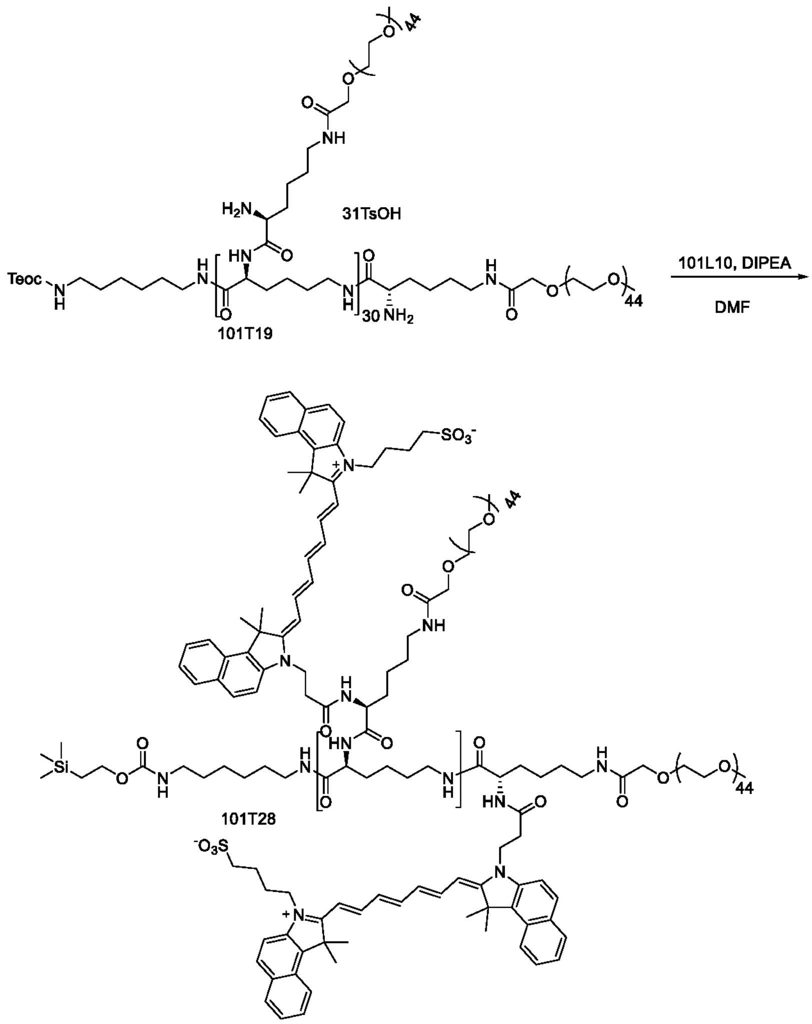


200.0 mg of 101T19 was dissolved in 1 mL of DMF; to the solution, 149 mg of compound 101L10 and 58 $\mu$L of DIPEA were added. The mixture was stirred at room temperature under nitrogen atmosphere and allowed to react in the dark for 2 h. Compound 101L10 was fully consumed.

The product was precipitated into 20 mL of MTBE and centrifuged. The lower layer of dark green oil was

dissolved in 1 mL of MeOH. To the solution, 15 mL of MTBE was added, the precipitate was centrifuged and vacuum dried to obtain 190 mg of dark green solid powder (Yield: 95%; HPLC retention time: 9.698 min). Maximum absorption in the wavelength range of 300-900 nm was observed at 786 nm.

Example 50: synthesis of compound 216A13

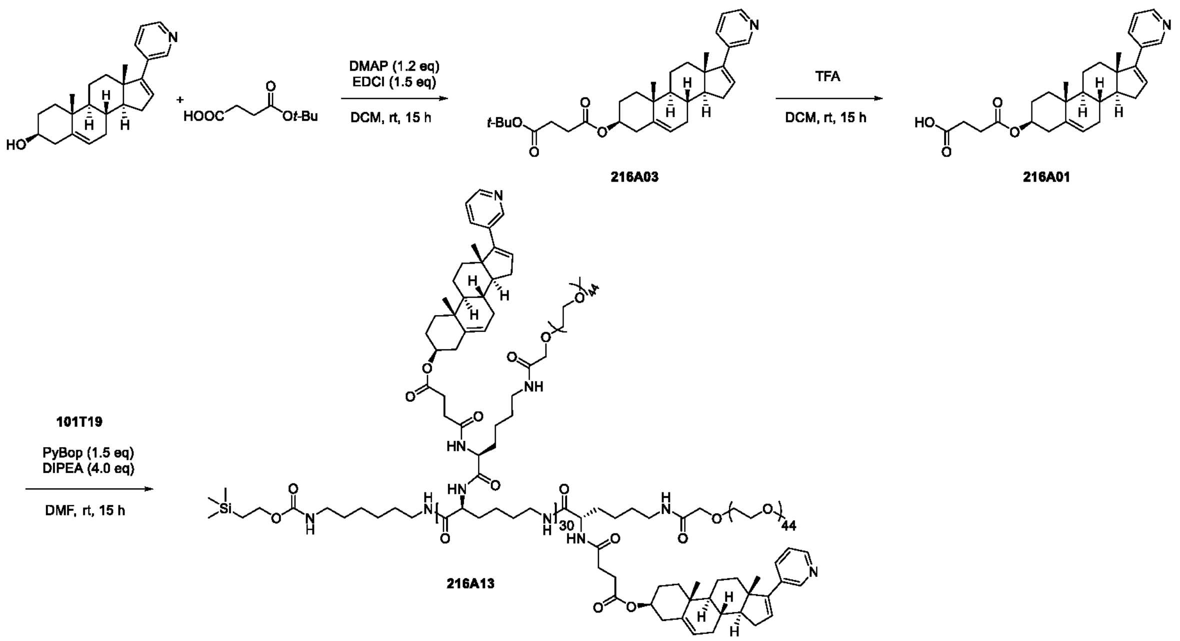


216A03

216A01

216A13

Synthesis of compound **216A03:**

Abiraterone (399 mg, 2.0 mmol, 1.0 eq), tert-butyl carboxylate (383 mg, 2.2 mmol, 1.1 eq), DMAP (293 mg, 2.4 mmol, 1.2 eq), EDC HCl (575 mg, 3.0 mmol, 1.5 eq) and absolute DCM (40 mL) were charged into a flask and the system was allow to react for 15 h at room temperature under stirring and nitrogen atmosphere. TLC showed complete reaction. The solvent was removed with a rotovap. After purification by silica gel column chromatography (33-50% EtOAc in PE), 1.1 g of the compound **216A03** was obtained as a white solid (Yield: 99%).

$^1$H NMR (400 MHz, $CDCl_3$) $\delta$ 8.61 (d, $J$ = 2.2 Hz, 1H), 8.45 (d, $J$ = 4.6 Hz, 1H), 7.68 - 7.58 (m, 1H), 7.21 (dd, $J$ = 8.0, 4.8 Hz, 1H), 5.99 (s, 1H), 5.40 (d, $J$ = 5.1 Hz, 1H), 4.72 - 4.56 (m, 1H), 2.64 - 2.48 (m, 4H), 2.43 - 2.19 (m, 3H), 2.09 - 2.02 (m, 3H), 1.91 - 1.82 (m, 2H), 1.75 - 1.57 (m, 5H), 1.54 - 1.42 (m, 10H), 1.27 - 1.23 (m, 1H), 1.20 - 1.06 (m, 5H), 1.04 (s, 3H).

Synthesis of compound **216A01:**

TFA (5 mL) was added to a solution (25 mL) of **216A03** (1.144 g, 2.26 mmol, 1.0 eq) in DCM. The system was stirred for 15 h of reaction at room temperature. At the end of the reaction, the solvent was removed with a rotovap. After purification by silica gel column chromatography (DCM/MeOH = 10/1), 1.0 g of the compound **216A01** was obtained as white solid(Yield: 99%).

$^1$H NMR (400 MHz, $CDCl_3$) $\delta$ 8.88 (s, 1H), 8.68 (d, $J$ = 5.5 Hz, 1H), 8.24 (d, $J$ = 8.2 Hz, 1H), 7.74 (dd, $J$ = 8.1, 5.5 Hz, 1H), 6.31 (s, 1H), 5.41 (d, $J$ = 5.0 Hz, 1H), 4.71 - 4.57 (m, 1H), 2.74 - 2.57 (m, 4H), 2.41 - 2.23 (m, 3H), 2.17 - 2.01 (m, 3H), 1.92-1.43 (m, 10H), 1.15 - 1.07 (m, 7H).

Synthesis of compound **216A13:**

**101T19** (200 mg, 0.082 mmol, 1.0 eq), **216A01** (55.4 mg, 0.12 mmol, 1.5 eq), PyBOP (64.0 mg, 0.12 mmol, 1.5 eq), DIPEA (0.09 mL, 0.49 mmol, 4.0 eq) and DMF (1.6 mL) were charged and allowed to react for 15 h at room temperature under stirring and nitrogen atmosphere. HPLC showed complete reaction. MTBE (10 mL) was added to the DMF solution; the resultant precipitate was washed with MTBE, dissolved in MeOH, purified by ultrafiltration with

MeOH/aqueous solution and freeze-dried to obtain 197 mg of compound **216A13** as white solid (Purity: 99%).

Example 51: synthesis of compound 216A14

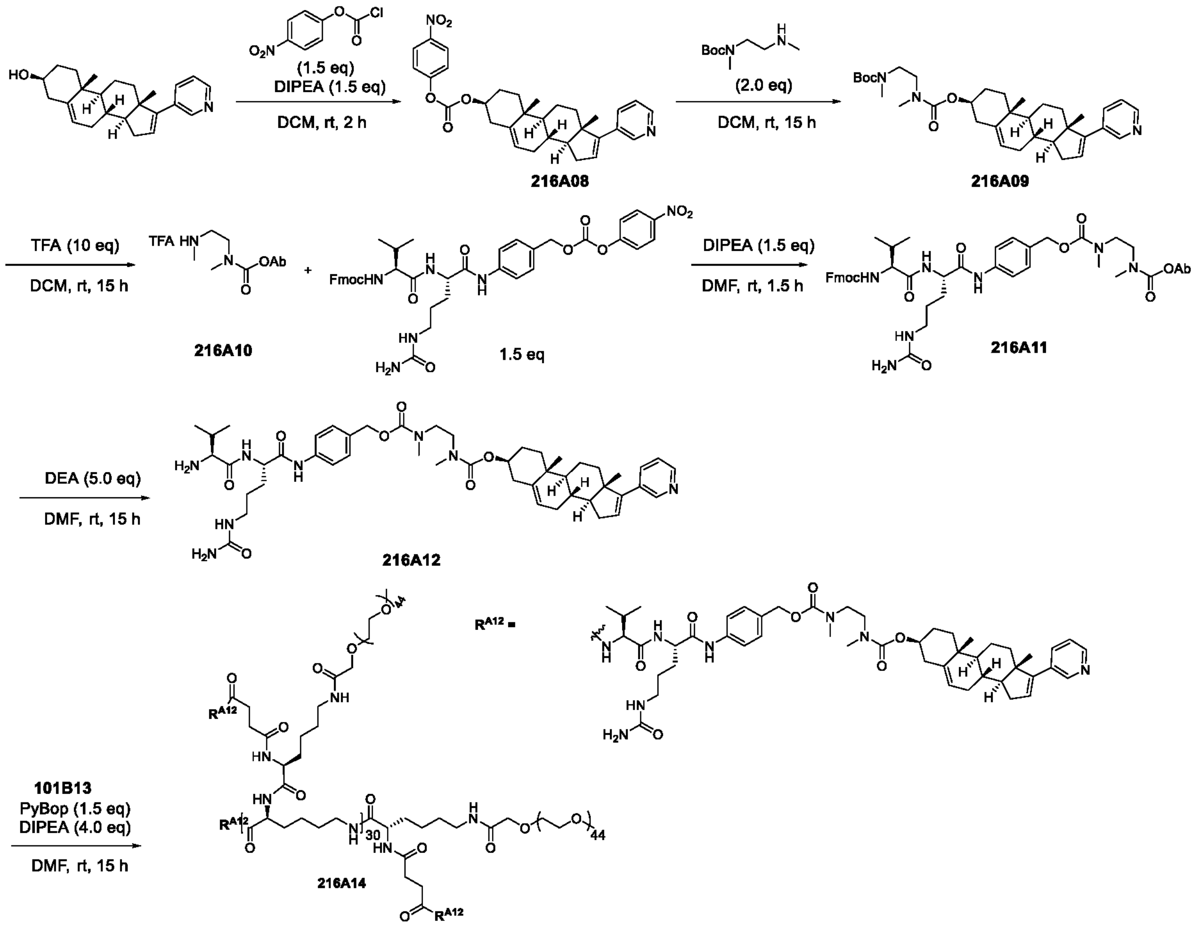


Synthesis of compound **216A08:**

Abiraterone (2.0 g, 5.7 mmol, 1.0 eq), acid chloride (1.73 g, 8.6 mmol, 1.5 eq), DIPEA (1.42 mL, 8.6 mmol, 1.5 eq) and absolute DCM (40 mL) were charged and allowed to react for 2 h at room temperature under stirring and nitrogen atmosphere. HPLC showed complete reaction. The reaction solution was used directly in the next step.

Synthesis of compound **216A09:**

Boc-amine (2.14 mL, 11.4 mmol, 2.0 eq) were added to the above reaction solution and stirred for 15 h at room temperature. HPLC showed complete reaction. The solvent was removed and, after purification by silica gel column chromatography (PE/EA= 3/1) , 2.83 g of the compound **216A09** was obtained as white solid (Yield: 88% for the two steps).

$^1$H NMR (400 MHz, $CDCl_3$) $\delta$ 8.61 (d, $J$ = 2.2 Hz, 1H), 8.45 (dd, $J$ = 4.8, 1.6 Hz, 1H), 7.64 (dt, $J$ = 7.9, 2.0 Hz, 1H), 7.21 (dd, $J$ = 7.9, 4.8 Hz, 1H), 6.04 - 5.92 (m, 1H), 5.41 (d, $J$ = 4.7 Hz, 1H), 4.60 - 4.40 (m, 1H), 3.46 - 3.23 (m, 4H), 2.98 - 2.82 (m, 6H), 2.45 - 2.21 (m, 3H), 2.11 - 1.97 (m, 3H), 1.88 - 1.55 (m, 9H), 1.45 (s, 9H), 1.20 - 1.01 (m, 8H).

Synthesis of compound **216A10:**

TFA(5 mL) was added to a solution (50 mL) of **216A09** (2.83 g, 5.0 mmol, 1.0 eq) in DCM at 25 °C. The reaction was stirred for 15 h at room temperature. HPLC showed complete reaction. Solvent was removed with a rotovap to obtain a white solid.

Synthesis of compound **216A11:**

**10107** (4.6 g, 5.0 mmol, 1.5 eq) and DIPEA (1.8 mL, 10.0 mmol, 2.0 eq) was added to a solution (50 mL) of compound **216A10** in DMF at 25 °C under nitrogen atmosphere. The system was stirred for 15 h at room temperature. HPLC showed complete reaction. The reaction solution was used directly in the next step.

Synthesis of compound **216A12:**

Diethylamine (2.6 mL, 25.0 mmol, 5.0 eq) was added to the above DMF solution and stirred for 15 h at room temperature under nitrogen atmosphere. HPLC showed complete reaction. The solvent was removed and purified by silica gel column chromatography (5%-25% MeOH in DCM) to give 2.43 g of the compound **216A12** as yellow solid (Yield: 56% for the three steps).

$^1$H NMR (400 MHz, $CDCl_3$) δ 9.82 - 9.58 (br, 1H), 8.59 (d, *J* = 2.2 Hz, 1H), 8.49 - 8.32 (m, 2H), 7.63 (d, *J* = 8.0 Hz, 1H), 7.59 - 7.46 (m, 2H), 7.24 - 7.16 (m, 3H), 6.12 - 5.87 (m, 2H), 5.41 - 5.29 (m, 2H), 5.01 (br, 2H), 4.71 (br, 1H), 4.45 (br, 1H), 3.35 (br, 6H), 2.94 - 2.70 (m, 7H), 2.48 - 2.15 (m, 3H), 2.15 - 1.92 (m, 4H), 1.88 - 1.43 (m, 12H), 1.15 - 0.96 (m, 8H), 0.85 (dd, *J* = 21.5, 6.6 Hz, 7H).

Synthesis of compound **216A14:**

The mixture of **101B13** (100 mg, 0.04 mmol, 1.0 eq), **216A12** (52.1 mg, 0.06 mmol, 1.5 eq), PyBOP (31.2 mg, 0.06 mmol, 1.5 eq), DIPEA (0.03 mL, 0.16 mmol, 4.0 eq) and DMF (0.8 mL) were stirred for 15 h at room temperature under nitrogen atmosphere. HPLC showed complete reaction. MTBE (10 mL) was added to the DMF solution; the resultant precipitate was washed with MTBE, dissolved in MeOH, purified by ultrafiltration with MeOH/aqueous solution and freeze-dried to obtain 117 mg of the compound **216A14** as white solid (Purity: 97.55%).

Example 52: synthesis of compound 216A15

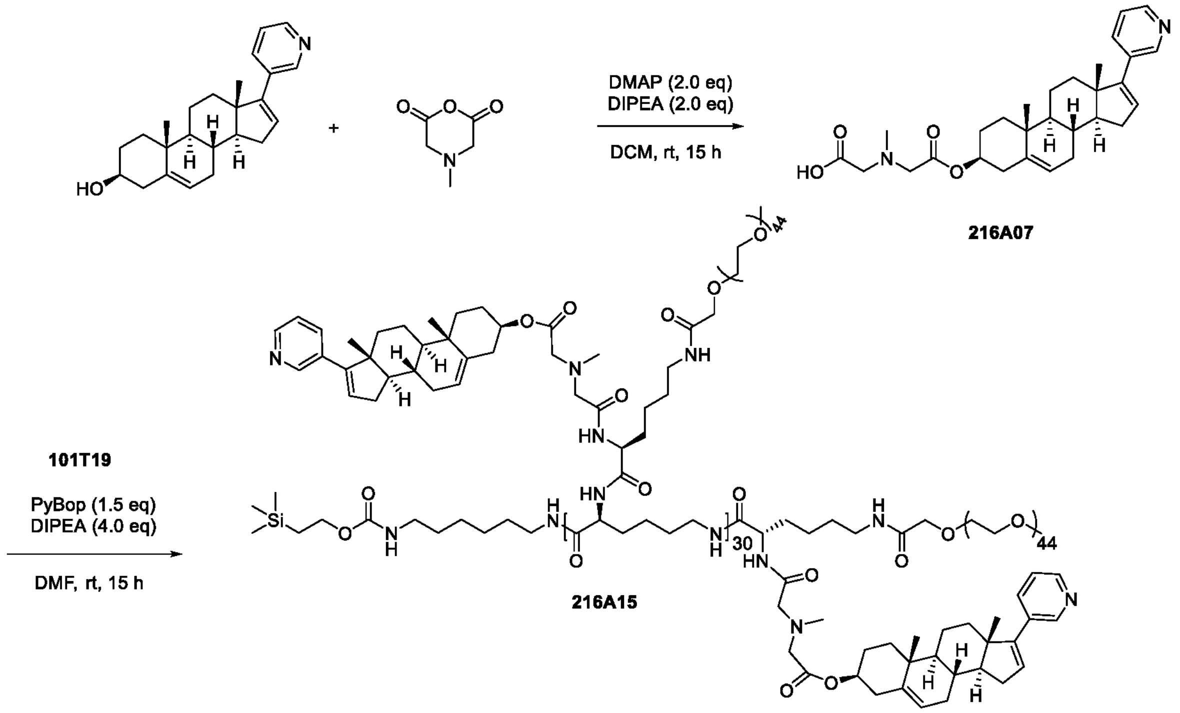


Synthesis of compound **216A07:**

The mixture of Abiraterone (1.0 g, 2.86 mmol, 1.0 eq), anhydride (1.11 g, 8.6 mmol, 3.0 eq), DMAP (697 mg, 5.7 mmol, 2.0 eq), DIPEA (1.0 mL, 5.7 mmol, 2.0 eq) and absolute DCM (10 mL) were stirred for 15 h at room temperature under nitrogen atmosphere. TLC showed complete reaction. The solvent was removed with a rotovap; after purification

by silica gel column chromatography (10-30% MeOH in DCM), 1.08 g of the compound **216A07** was obtained as light yellow solid (Yield: 79%).

$^1$H NMR (400 MHz, $CDCl_3$) $\delta$ 8.61 (d, *J* = 2.2 Hz, 1H), 8.45 (dd, *J* = 4.8, 1.6 Hz, 1H), 7.69 - 7.61 (m, 1H), 7.23 (dd, *J* = 7.9, 4.8 Hz, 1H), 6.02 - 5.96 (m, 1H), 5.42 (d, *J* = 4.9 Hz, 1H), 4.76 - 4.63 (m, 1H), 3.57 - 3.34 (m, 4H), 2.57 (s, 3H), 2.39 - 2.20 (m, 3H), 2.11 - 1.99 (m, 3H), 1.93 - 1.81 (m, 2H), 1.74 - 1.56 (m, 5H), 1.50 - 1.45 (m, 3H), 1.16 - 1.06 (m, 4H), 1.04 (s, 3H).

Synthesis of compound **216A15:**

The mixture of **101T19** (100 mg, 0.041 mmol, 1.0 eq), **216A07** (29.2 mg, 0.061 mmol, 1.5 eq), PyBOP (32.0 mg, 0.061 mmol, 1.5 eq), DIPEA (0.03 mL, 0.16 mmol, 4.0 eq) and DMF (0.8 mL) were stirred for 15 h at room temperature under nitrogen atmosphere. HPLC showed complete reaction. MTBE (10 mL) was added to the DMF solution; the resultant precipitate was washed with MTBE, dissolved in MeOH, purified by ultrafiltration with MeOH/aqueous solution and freeze-dried to obtain 92 mg of the compound **216A15** as white solid (Purity: 99.13%).

Example 53: synthesis of compound 216A16

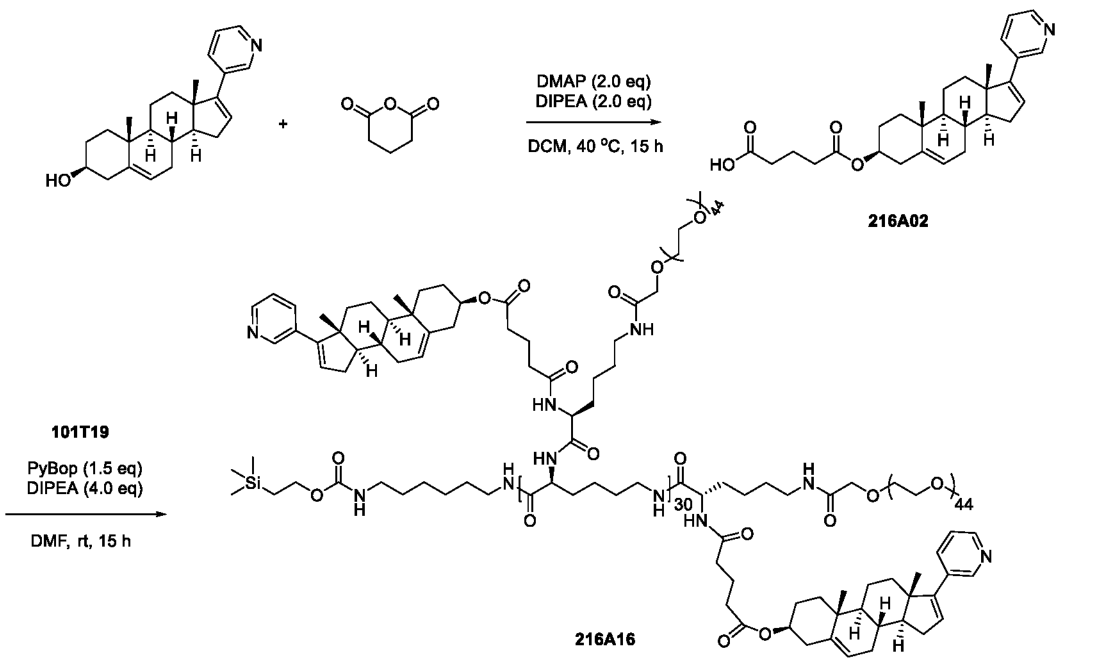


Synthesis of compound **216A02:**

The mixture of Abiraterone (350 mg, 1.0 mmol, 1.0 eq), anhydride (342 mg, 3.0 mmol, 3.0 eq), DMAP (244 mg, 2.0 mmol, 2.0 eq), DIPEA (0.35 mL, 2.0 mmol, 2.0 eq) and absolute DCM (50 mL) were stirred for 15 h at 40°C under nitrogen atmosphere. TLC showed complete reaction. The solvent was removed with a rotovap; after purification by silica gel column chromatography (5-20% MeOH in DCM), 382 mg of the compound **216A02** was obtained as white solid (Yield: 82%).

$^1$H NMR (400 MHz, $CDCl_3$) $\delta$ 8.62 (d, *J* = 2.2 Hz, 1H), 8.46 (d, *J* = 4.7 Hz, 1H), 7.68 (d, *J* = 7.9 Hz, 1H), 7.26 - 7.23 (m, 1H), 6.13 - 5.93 (m, 1H), 5.41 (d, *J* = 5.1 Hz, 1H), 4.70 - 4.53 (m, 1H), 2.46 - 2.26 (m, 10H), 2.08 - 1.95 (m, 5H), 1.90 - 1.83 (m, 2H), 1.71 - 1.52 (m, 5H), 1.17 - 1.07 (m, 4H), 1.04 (s, 3H).

Synthesis of compound **216A16:**

The mixture of **101T19** (200 mg, 0.082 mmol, 1.0 eq), **216A02** (57.0 mg, 0.12 mmol, 1.5 eq), PyBOP (64.0 mg, 0.12 mmol, 1.5 eq), DIPEA (0.09 mL, 0.49 mmol, 4.0 eq) and DMF (1.6 mL) were stirred for 15 h at room temperature under nitrogen atmosphere. HPLC showed complete reaction. MTBE (10 mL) was added to the DMF solution; the

resultant precipitate was washed with MTBE, dissolved in MeOH, purified by ultrafiltration with MeOH/aqueous solution and freeze-dried to obtain 236 mg of the compound **216A16** as white solid (Purity: 99.60%).

Example 54: synthesis of compound 216A17

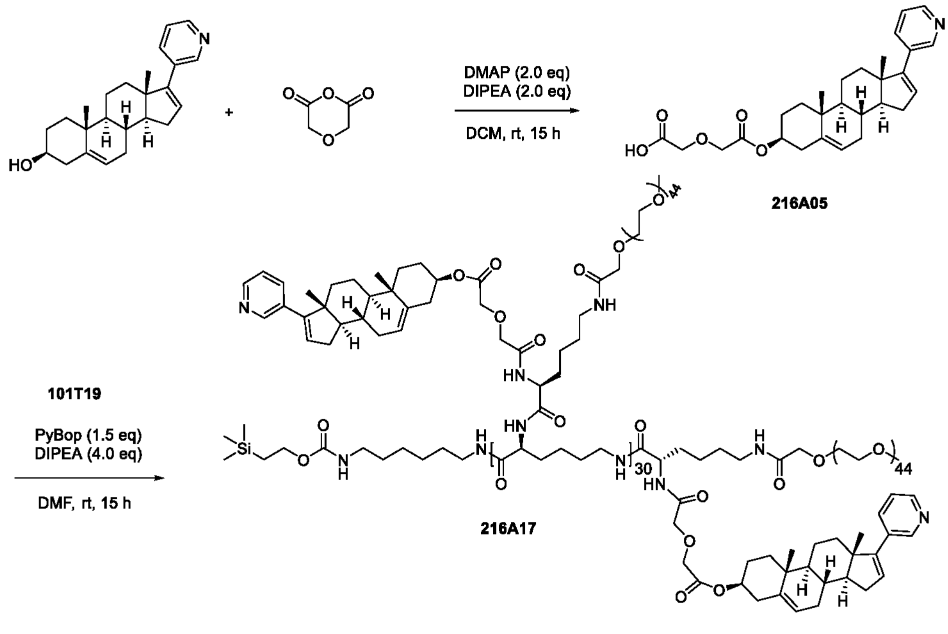


Synthesis of compound **216A05:**

The mixture of Abiraterone (1.0 g, 2.86 mmol, 1.0 eq), anhydride (1.0 g, 8.6 mmol, 3.0 eq), DMAP (697 mg, 5.7 mmol, 2.0 eq), DIPEA (1.0 mL, 5.7 mmol, 2.0 eq) and absolute DCM (10 mL) were stirred for 15 h at room temperature under nitrogen atmosphere. TLC showed complete reaction. The solvent was removed with a rotovap; after silica gel column chromatography (5-20% MeOH in DCM), 934 mg of the compound **216A05** was obtained as white solid (Yield: 70%).

$^1$H NMR (400 MHz, $CDCl_3$) δ 8.65 (d, J= 2.3 Hz, 1H), 8.47 (d, *J* = 4.9 Hz, 1H), 7.75 (dt, *J* = 7.9, 1.9 Hz, 1H), 7.32 (dd, *J* = 8.0, 4.9 Hz, 1H), 6.10 - 5.99 (m, 1H), 5.42 (d, *J* = 5.0 Hz, 1H), 4.79 - 4.68 (m, 1H), 4.25 (s, 4H), 2.44 - 2.23 (m, 3H), 2.14 - 1.99 (m, 3H), 1.96 - 1.83 (m, 2H), 1.79 - 1.43 (m, 8H), 1.16 - 1.06 (m, 4H), 1.04 (s, 3H).

Synthesis of compound **216A17:**

The mixture of **101T19** (200 mg, 0.082 mmol, 1.0 eq), **216A05** (57.3 mg, 0.12 mmol, 1.5 eq), PyBOP (64.0 mg, 0.12 mmol, 1.5 eq), DIPEA (0.09 mL, 0.49 mmol, 4.0 eq) and DMF (1.6 mL) were stirred for 15 h at room temperature under nitrogen atmosphere. HPLC showed complete reaction. MTBE (10 mL) was added to the DMF solution; the resultant precipitate was washed with MTBE, dissolved in MeOH, purified by ultrafiltration with MeOH/aqueous solution and freeze-dried to obtain 175 mg of the compound **216A17** as white solid (Purity: 99.13%).

Example 60: synthesis of compound 216A18

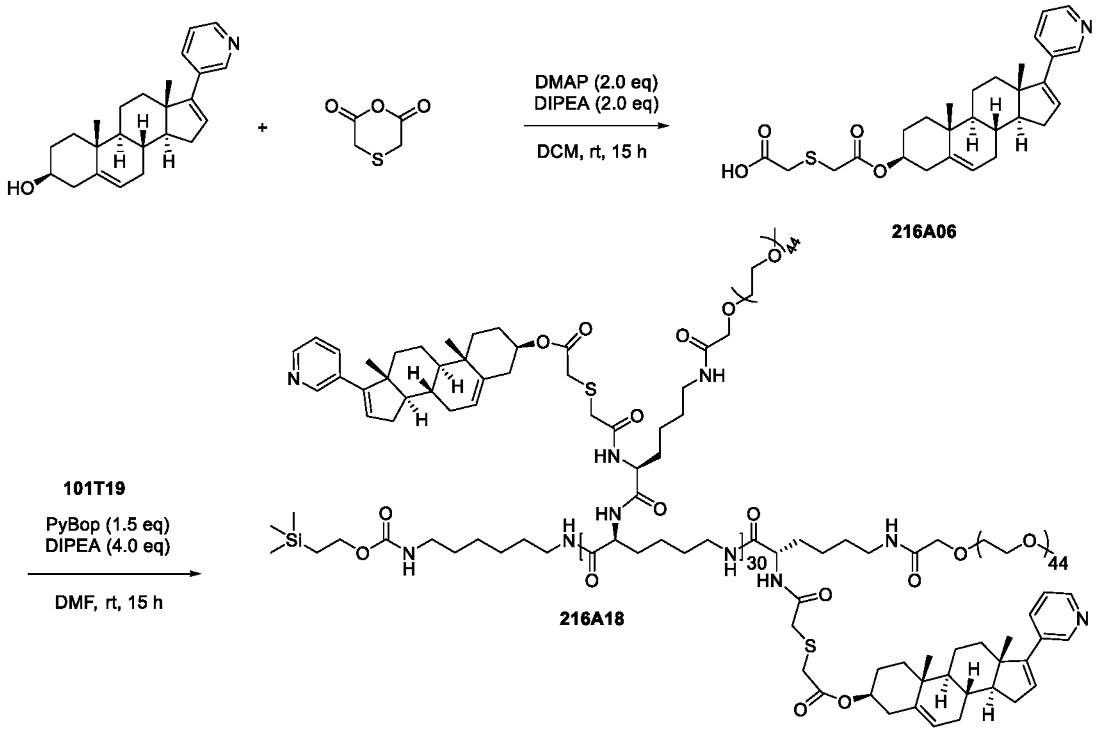


Synthesis of compound **216A06:**

The mixture of Abiraterone (1.0 g, 2.86 mmol, 1.0 eq), anhydride (1.14 g, 8.6 mmol, 3.0 eq), DMAP (697 mg, 5.7 mmol, 2.0 eq), DIPEA (1.0 mL, 5.7 mmol, 2.0 eq) and absolute DCM (10 mL) were stirred for 15 h at room temperature under nitrogen atmosphere. TLC showed complete reaction. The solvent was removed with a rotovap, and the mixture was purified by silica gel column chromatography (5-20% MeOH in DCM) to obtain 0.95 g of the compound **216A06** as white solid (Yield: 69%).

$^1$H NMR (400 MHz, $CDCl_3$) $\delta$ 8.66 (s, 1H), 8.47 (d, J= 4.2 Hz, 1H), 7.74 (dt, *J*= 8.0, 1.9 Hz, 1H), 7.31 (dd, *J* = 8.0, 4.9 Hz, 1H), 6.09 - 6.03 (m, 1H), 5.38 (d, *J* = 5.1 Hz, 1H), 4.73 -4.60 (m, 1H), 3.43 (s, 2H), 3.40 (s, 2H), 2.41 - 2.20 (m, 3H), 2.11 - 2.00 (m, 3H), 1.93 - 1.42 (m, 9H), 1.15 - 0.99 (m, 8H).

Synthesis of compound **216A18:**

The mixture of **101T19** (200 mg, 0.082 mmol, 1.0 eq), **216A06** (59.2 mg, 0.12 mmol, 1.5 eq), PyBOP (64.0 mg, 0.12 mmol, 1.5 eq), DIPEA (0.09 mL, 0.49 mmol, 4.0 eq) and DMF (1.6 mL) were stirred for 15 h at room temperature under nitrogen atmosphere. HPLC showed complete reaction. MTBE (10 mL) was added to the DMF solution; the resultant precipitate was washed with MTBE, dissolved in MeOH, purified by ultrafiltration with MeOH/aqueous solution and freeze-dried to obtain 184 mg of the compound **216A18** as white solid (Purity: 99.74%).

Example 61: synthesis of compound 216A26

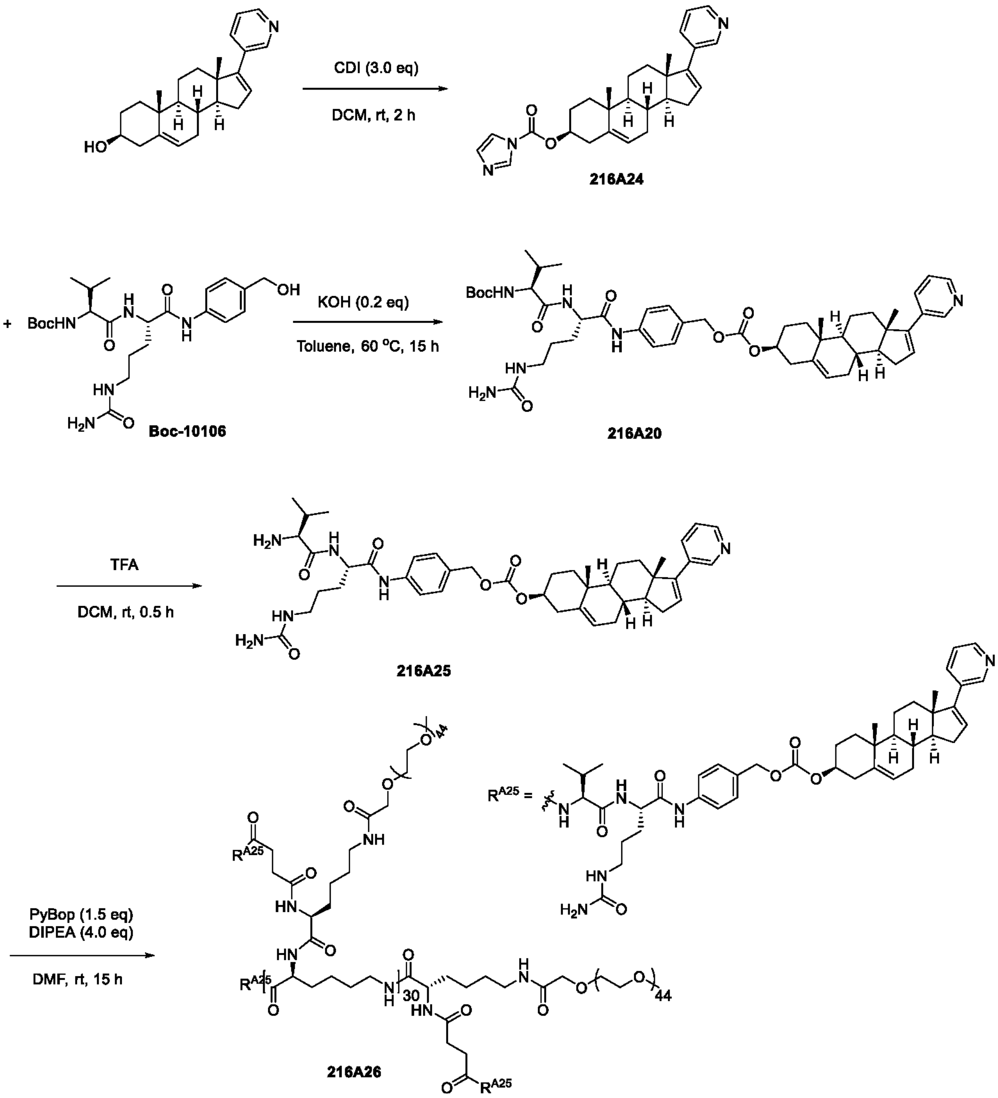


216A24

Boc-10106

216A20

216A25

216A26

Synthesis of compound **216A24:**

The mixture of Abiraterone (1.05 g, 3.0 mmol, 1.0 eq), CDI (1.46 g, 9.0 mmol, 3.0 eq) and absolute DCM (15 mL) were stirred for 2 h at room temperature under nitrogen atmosphere. LCMS showed complete reaction. MTBE (50 mL) was added, and the precipitate was filtered, washed and dried to obtain 1.15 g of the compound **216A24** as white solid (Yield: 86%).

$^1$H NMR (400 MHz, $CDCl_3$) δ 8.62 (d, *J* = 2.2 Hz, 1H), 8.46 (dd, *J* = 4.8, 1.6 Hz, 1H), 8.14 (s, 1H), 7.65 (dt, *J* = 7.9, 1.9 Hz, 1H), 7.43 (t, J= 1.5 Hz, 1H), 7.23 (dd, *J* = 7.9, 4.8 Hz, 1H), 7.07 (s, 1H), 6.04 - 5.96 (m, 1H), 5.49 (d, *J* = 4.8 Hz, 1H), 4.92 - 4.78 (m, 1H), 2.58 - 2.49 (m, 2H), 2.33 - 2.24 (m, 1H), 2.14 - 2.03 (m, 4H), 1.99 - 1.92 (m, 1H), 1.81 - 1.46 (m, 8H), 1.23 - 1.12 (m, 4H), 1.06 (s, 3H).

Synthesis of compound **216A20:**

The mixture of **Boc-10106** (0.96 g, 2.0 mmol, 1.0 eq), **216A24** (1.07 g, 2.4 mmol, 1.2 eq), KOH (22 mg, 0.4 mmol, 0.2 eq) and Toluene (50 mL) were stirred for 15 h at 60°C under nitrogen atmosphere. LCMS showed that there was still a large amount of unreacted starting materials. The solvent was removed with a rotovap, and the mixture was purified by silica gel column chromatography (5%-25% MeOH in DCM) to obtain 165 mg of the compound **216A20** as white solid (Yield: 10%).

$^1$H NMR (400 MHz, $CDCl_3$) δ 9.20 (s, 1H), 8.61 (d, *J* = 2.3 Hz, 1H), 8.45 (dd, *J* = 4.8, 1.7 Hz, 1H), 7.65 (d, *J* = 8.0 Hz, 1H), 7.60 (d, *J* = 8.3 Hz, 2H), 7.32 (d, *J* = 8.4 Hz, 2H), 7.22 (dd, *J* = 8.0, 4.8 Hz, 1H), 7.14 (d, *J* = 8.0 Hz, 1H), 6.03 - 5.95 (m, 1H), 5.43 (d, *J* = 5.1 Hz, 1H), 5.23 (t, *J*= 6.2 Hz, 1H), 5.18 (d, *J*= 8.1 Hz, 1H), 5.09 (s, 2H), 4.80 - 4.70 (m, 1H), 4.63 (s, 2H), 4.56 - 4.42 (m, 1H), 4.03 - 3.93 (m, 1H), 3.49 - 3.37 (m, 1H), 3.28 - 3.15 (m, 1H), 2.45 - 1.84 (m,

13H), 1.43 (s, 10H), 1.05 (d, *J* = 9.2 Hz, 6H), 0.98 (d, *J* = 6.7 Hz, 3H), 0.94 (d, *J* = 6.8 Hz, 3H).

Synthesis of compound **216A25:**

To a solution of the compound **216A20** (165 mg, 0.19 mmol, 1.0 eq) in DCM (2 mL) was added TFA (0.2 mL) and stirred for 0.5 h at room temperature. LCMS showed the content of remaining starting materials was <10%. DCM (20 mL) and saturated sodium bicarbonate (20 mL) were added to the mixture. The water phase and the organic phase were separated and the organic phase was collected; the water phase was washed with DCM solution for 3 times; the combined organic phase was washed with saturated NaCl solution, then concentrated with a rotovap; the residue was purified by silica gel column chromatography (5%-30% MeOH in DCM) to obtain 84 mg of the compound **216A25** as white solid (Yield: 59%).

$^1$H NMR (400 MHz, $CD_3OD$) $\delta$ 8.55 (d, *J* = 2.3 Hz, 1H), 8.40 (dd, *J* = 4.9, 1.6 Hz, 1H), 7.89 - 7.82 (m, 1H), 7.65 - 7.56 (m, 2H), 7.42 - 7.33 (m, 3H), 6.13 - 6.07 (m, 1H), 5.48 (d, *J* = 5.1 Hz, 1H), 5.41 - 5.32 (m, 1H), 5.11 (s, 2H), 4.61 - 4.53 (m, 4H), 4.50 - 4.38 (m, 1H), 3.27 - 3.10 (m, 3H), 2.47 - 2.28 (m, 3H), 2.22 - 1.51 (m, 20H), 1.21 - 1.08 (m, 8H), 1.01 (d, *J* = 6.9 Hz, 3H), 0.95 (d, *J* = 6.8 Hz, 3H).

Synthesis of compound **216A26:**

The mixture of **101T13** (90 mg, 0.036 mmol, 1.0 eq), **216A25** (40.5 mg, 0.054 mmol, 1.5 eq), PyBOP (27.9 mg, 0.054 mmol, 1.5 eq), DIPEA (0.025 mL, 0.14 mmol, 4.0 eq), and DMF (0.8 mL) were stirred for 15 h at room temperature under nitrogen atmosphere. HPLC showed complete reaction. MTBE (10 mL) was added to the DMF solution; the resultant precipitate was washed with MTBE, dissolved in MeOH, purified by ultrafiltration with MeOH/aqueous solution and freeze-dried to obtain 120 mg of the compound **216A26** as white solid (Purity: 97.13%).

Example 55: synthesis of compound 216B06

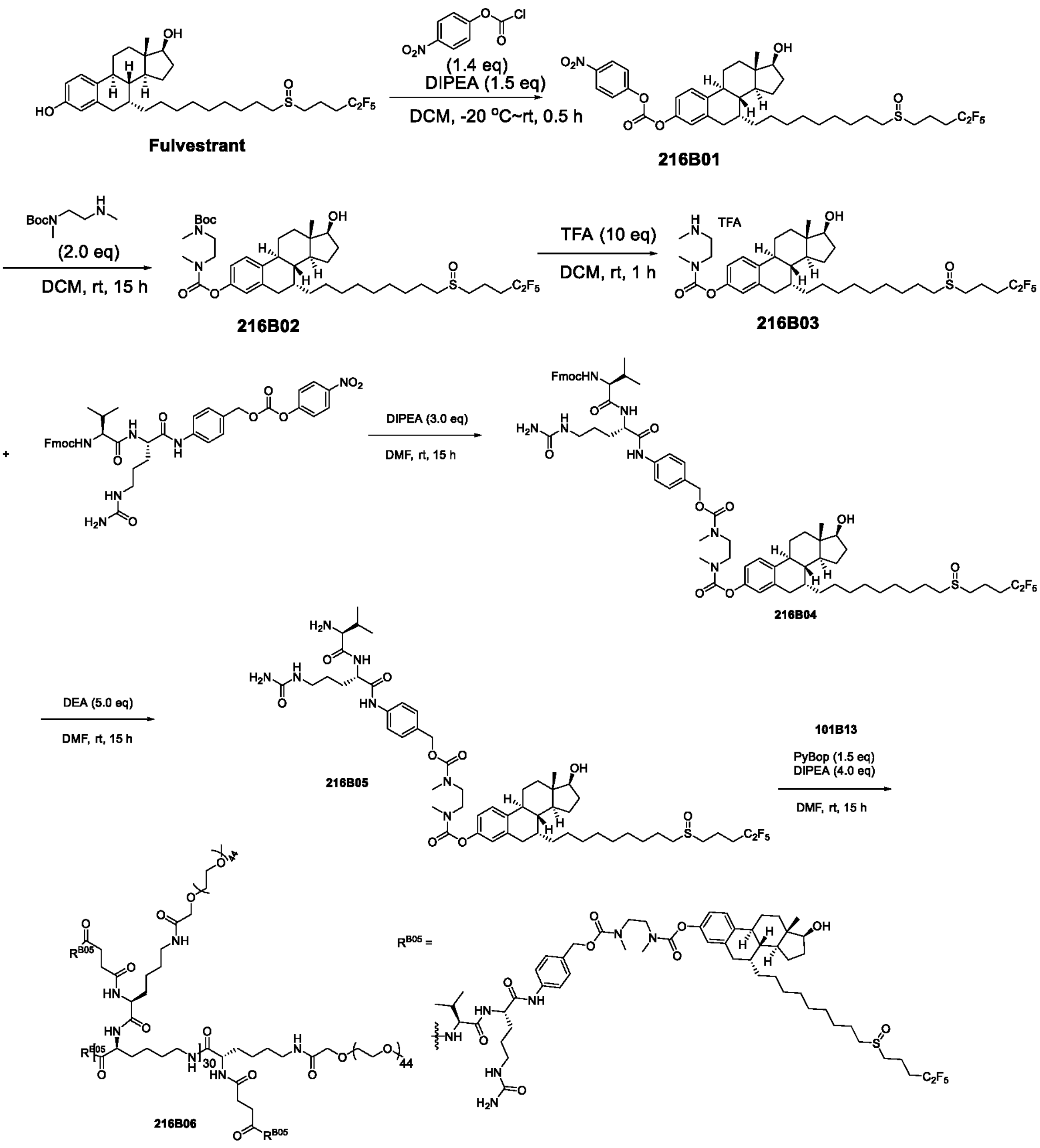


Synthesis of compound **216B01:**

Fulvestrant (930 mg, 1.53 mmol, 1.0 eq), DIPEA (0.41 mL, 2.3 mmol, 1.5 eq) and absolute DCM (15 mL) were charged at -20°C under nitrogen atmosphere; after a short period of stirring, acid chloride (433 mg, 2.15 mmol, 1.4 eq) was added. The reaction mixture was stirred at room temperature for 2 h under nitrogen atmosphere. LCMS showed complete reaction. The reaction solution was directly used in the next step.

Synthesis of compound **216B02:**

Boc-amine (584 mg, 3.1 mmol, 2.0 eq) was added to the above reaction solution. The reaction mixture was stirred at room temperature for 15 h under nitrogen atmosphere. LCMS showed complete reaction. The solvent was removed with a rotovap, and the residue was purified by silica gel column chromatography (0%-10% MeOH in DCM) to get 1.24 g of the compound **216B02** as white solid (Yield: 99% for the 2 steps).

$^1$H NMR (400 MHz, $CDCl_3$) δ 7.26 (d, *J* = 7.4 Hz, 1H), 6.91 - 6.83 (m, 1H), 6.80 (s, 1H), 3.74 (t, *J* = 8.5 Hz, 1H), 3.63 - 3.39 (m, 4H), 3.13 - 2.99 (m, 3H), 2.95 - 2.85 (m, 4H), 2.80 - 2.69 (m, 4H), 2.69 - 2.58 (m, 1H), 2.38 - 2.09 (m,

7H), 1.97 - 1.86 (m, 1H), 1.80 - 1.58 (m, 9H), 1.52 - 1.16 (m, 32H), 0.77 (s, 3H).

Synthesis of compound **216B03:**

TFA (1 mL) was added to a solution (5 mL) of the compound **216B02** (500 mg, 0.61 mmol, 1.0 eq) in DCM at 0°C. The reaction was stirred for 1 h at room temperature. LCMS showed complete reaction. The solvent was removed with a rotovap to obtain a white solid. The solid was used directly in the next step without purification.

Synthesis of compound **216B04:**

Compound **10107** (702 mg, 0.92 mmol, 1.5 eq) and DIPEA (0.33 mL, 1.83 mmol, 3.0 eq) were added to the DMF solution (5 mL) of the compound **216B03.** The reaction was stirred for 15 h at room temperature under nitrogen atmosphere. DIPEA (0.2 mL) and **10107** (500 mg) were added and the mixture was stirred overnight. LCMS showed complete reaction. The solvent was removed with a rotovap. The residue was purified by silica gel column chromatography (0%-15% MeOH in DCM) to give 0.56 g of the compound **216B04** as white solid (Yield: 68% for the two steps).

Synthesis of compound **216B05:**

Diethylamine (0.3 mL, 3.1 mmol, 5.0 eq) was added to a solution of **216B04** (809 mg, 0.61 mmol, 1.0 eq) in DMF. The reaction was stirred for 15 h at room temperature under nitrogen atmosphere. LCMS showed complete reaction. The solvent was removed with a rotovap. The residue was subjected to silica gel column chromatography (5%-30% MeOH in DCM) to obtain 427 mg of the compound **216B05** as white solid (Yield: 62%).

$^1$H NMR (400 MHz, $CDCl_3$) δ 9.66 (s, 1H), 8.28 (s, 1H), 7.75 - 7.45 (m, 2H), 7.25 - 7.03 (m, 2H), 6.76 (d, *J* = 10.3 Hz, 2H), 5.88 (s, 1H), 5.29 - 4.92 (m, 4H), 4.77 (s, 1H), 3.71 (s, 1H), 3.65 - 2.56 (m, 24H), 2.58 - 1.99 (m, 22H), 1.95 - 1.09 (m, 38H), 1.04 - 0.71 (m, 13H).

Synthesis of compound **216B06:**

The mixture of compound **101B13** (150 mg, 0.06 mmol, 1.0 eq, calculated by molar amount of polymerization unit), **216B05** (101.4 mg, 0.09 mmol, 1.5 eq), PyBOP (46.8 mg, 0.09 mmol, 1.5 eq), DIPEA (0.04 mL, 0.24 mmol, 4.0 eq), and DMF (0.6 mL) were stirred for 15 h at room temperature under nitrogen atmosphere. HPLC showed complete reaction. MTBE (10 mL) was added to the DMF solution; the resultant precipitate was washed with MTBE, dissolved in MeOH, purified by ultrafiltration with MeOH/aqueous solution and freeze-dried to obtain 200 mg of the compound **216B06** as white solid (Purity: 98.76%).

Example 56: synthesis of compound 216D06

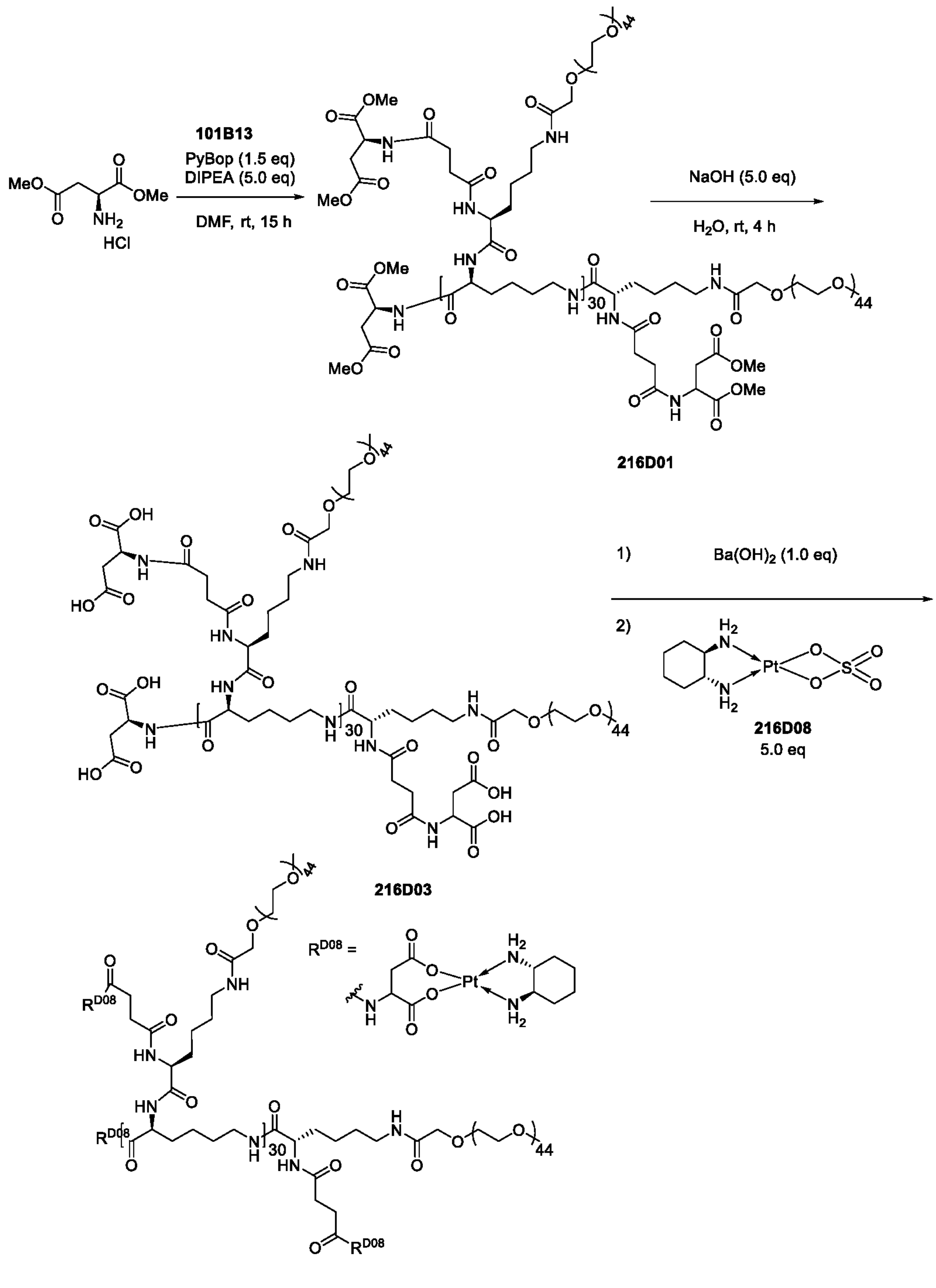


216D01

216D03

216D06

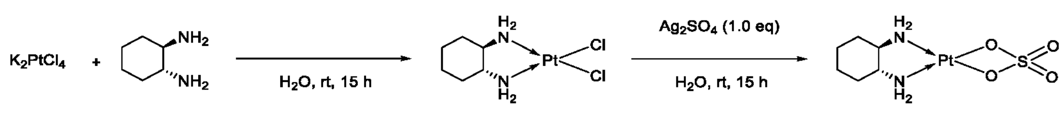


216D04

216D08

Synthesis of compound **216D01:**

The mixture of compound **101B13** (500 mg, 0.21 mmol, 1.0 eq), amine hydrochloride (62.6 mg, 0.32 mmol, 1.5 eq), PyBOP (165.0 mg, 0.32 mmol, 1.5 eq), DIPEA (0.19 mL, 1.06 mmol, 5.0 eq) and DMF (4.0 mL) were stirred for 15 h at room temperature under nitrogen atmosphere. HPLC showed complete reaction. MTBE (40 mL) was added to

the DMF solution; the resultant precipitate was washed with MTBE, dissolved in MeOH, purified by ultrafiltration with MeOH/aqueous solution and freeze-dried to obtain 455 mg of the compound **216D01** as white solid.

Synthesis of compound **216D03:**

Compound **216D01** (277 mg, 0.11 mmol, 1.0 eq) was added to NaOH solution (0.1 M, 5.6 mL, 0.56 mmol, 5.0 eq). The reaction was stirred for 4 h at room temperature. HPLC showed complete reaction. The reaction liquid was purified by ultrafiltration with water and freeze-dried to obtain 244 mg of the compound **216D03** as white solid.

Synthesis of compound **216D04:**

1,2-cyclohexanediamine (550 mg, 4.82 mmol, 1.0 eq) in water (10 mL) was added dropwisely to $K_2PtCl_4$ (2.0 g, 4.82 mmol, 1.0 eq) in water (12 mL). The reaction was stirred for 15 h at room temperature. The solution was filtered, washed with water and methanol and dried in vacuo to obtain product **216D04,** 1.6 g, earthy yellow solid.

Synthesis of compound **216D08:**

**216D04** (226.9 mg, 0.6 mmol, 1.0 eq), silver sulfate (187.1 mg, 0.6 mmol, 1.0 eq) and water (3.0 mL) were added to a brown bottle. The reaction was stirred for 24 h at room temperature. The solution was filtered to obtain a clear **216D08** solution (0.2 M).

Synthesis of compound **216D06:**

$Ba(OH)_2$ solution (0.1 M, 0.22 mL, 0.022 mmol, 1.0 eq) was added to the aqueous solution (0.5 mL) of **216D03** (55 mg, 0.022 mmol, 1.0 eq). The mixture was stirred for 10 min at room temperature. **216D08** aqueous solution (0.2 M, 0.5 mL, 0.11 mmol, 5.0 eq) was then added to this solution. The reaction was stirred for 15 h at room temperature. HPLC showed complete reaction. The mixture was filtered, purified by ultrafiltration with water and freeze-dried to obtain the product **216D06,** 51 mg, white solid, purity 98.5%.

Example 57: synthesis of compound 216D13

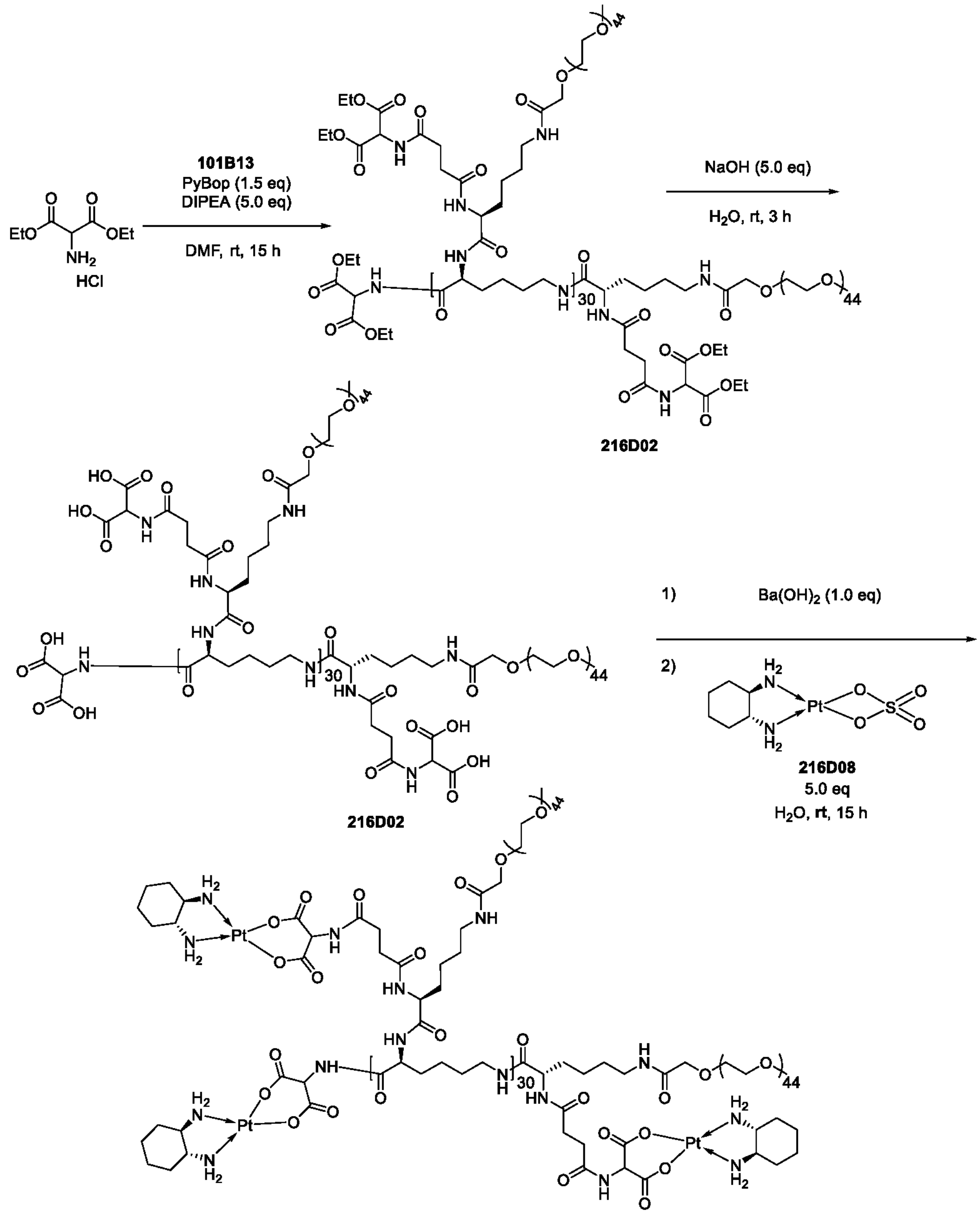


216D02

216D02

216D13

Synthesis of compound **216D02:**

The mixture of **101B13** (500 mg, 0.21 mmol, 1.0 eq), amine hydrochloride (67.1 mg, 0.32 mmol, 1.5 eq), PyBOP (165.0 mg, 0.32 mmol, 1.5 eq), DIPEA (0.19 mL, 1.06 mmol, 5.0 eq) and DMF (4.0 mL) were stirred for 15 h at room temperature under nitrogen atmosphere. HPLC showed complete reaction. MTBE (40 mL) was added to the DMF solution; the resultant precipitate was washed with MTBE, dissolved in MeOH, purified by ultrafiltration with MeOH/aqueous solution and freeze-dried to obtain compound **216D02,** 529 mg, as white solid.

Synthesis of compound **216D07:**

**216D02** (250 mg, 0.1 mmol, 1.0 eq) was added to NaOH solution (0.1 M, 5.0 mL, 0.5 mmol, 5.0 eq). The reaction system was stirred for 3 h at room temperature. HPLC showed complete reaction. The solution was purified by ultrafiltration with water and freeze-dried to obtain the product **216D07,** 209 mg, white solid.

Synthesis of compound **216D13:**

$Ba(OH)_2$ solution (0.1 M, 0.3 mL, 0.03 mmol, 1.0 eq) was added to the aqueous solution (0.5 mL) of **216D07** (75 mg, 0.03 mmol, 1.0 eq). The mixture was stirred for 10 min at room temperature. **216D08** aqueous solution (0.2 M, 0.75 mL, 0.15 mmol, 5.0 eq) was then added to this solution. The reaction system was stirred for 15 h at room temperature. HPLC showed complete reaction. The mixture was filtered, purified by ultrafiltration with water and freeze-dried to obtain the product **216D13,** 57 mg, white solid, purity 99%.

Example 58: synthesis of compound 216D36

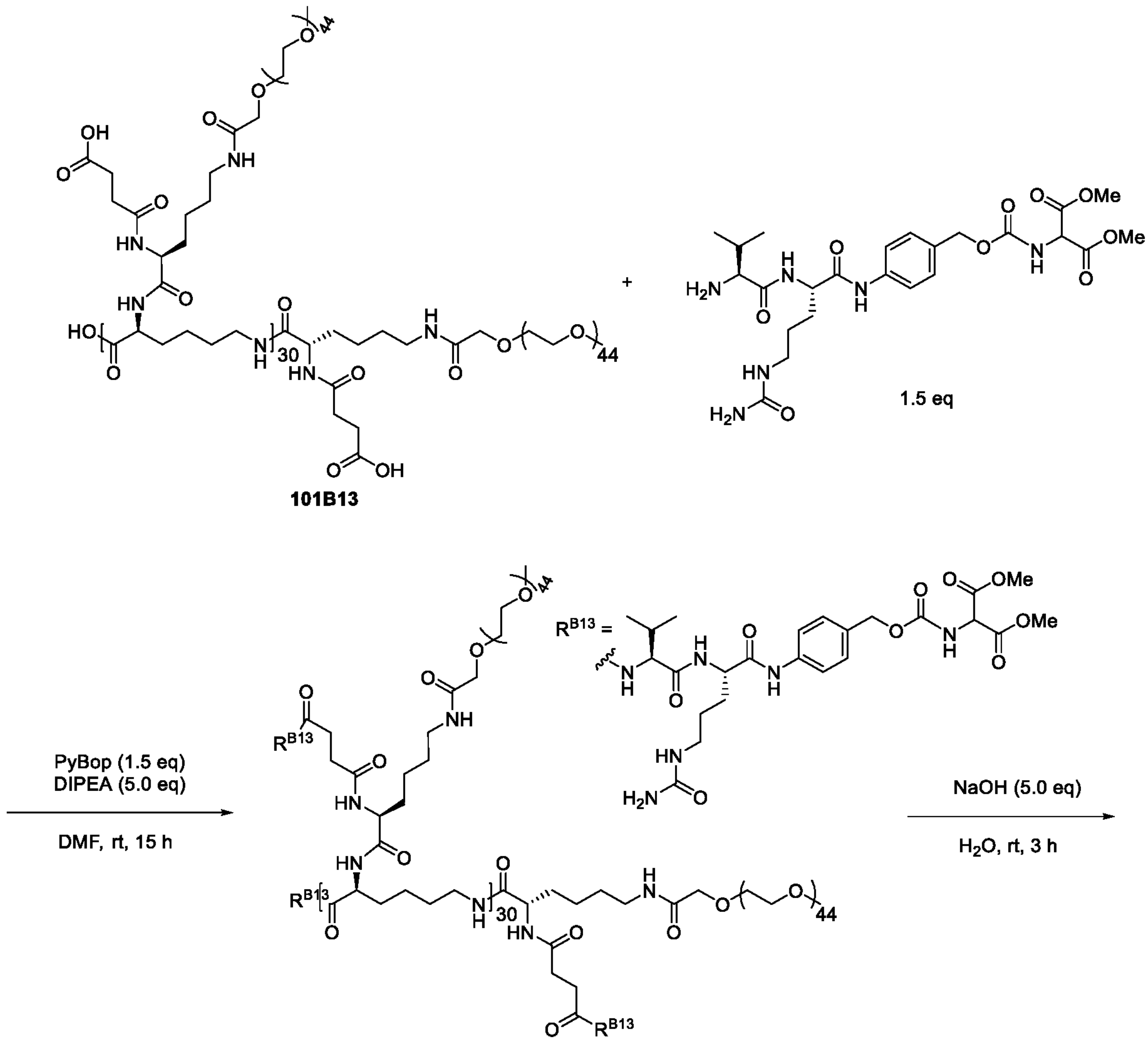


**101B13**

**216D34**

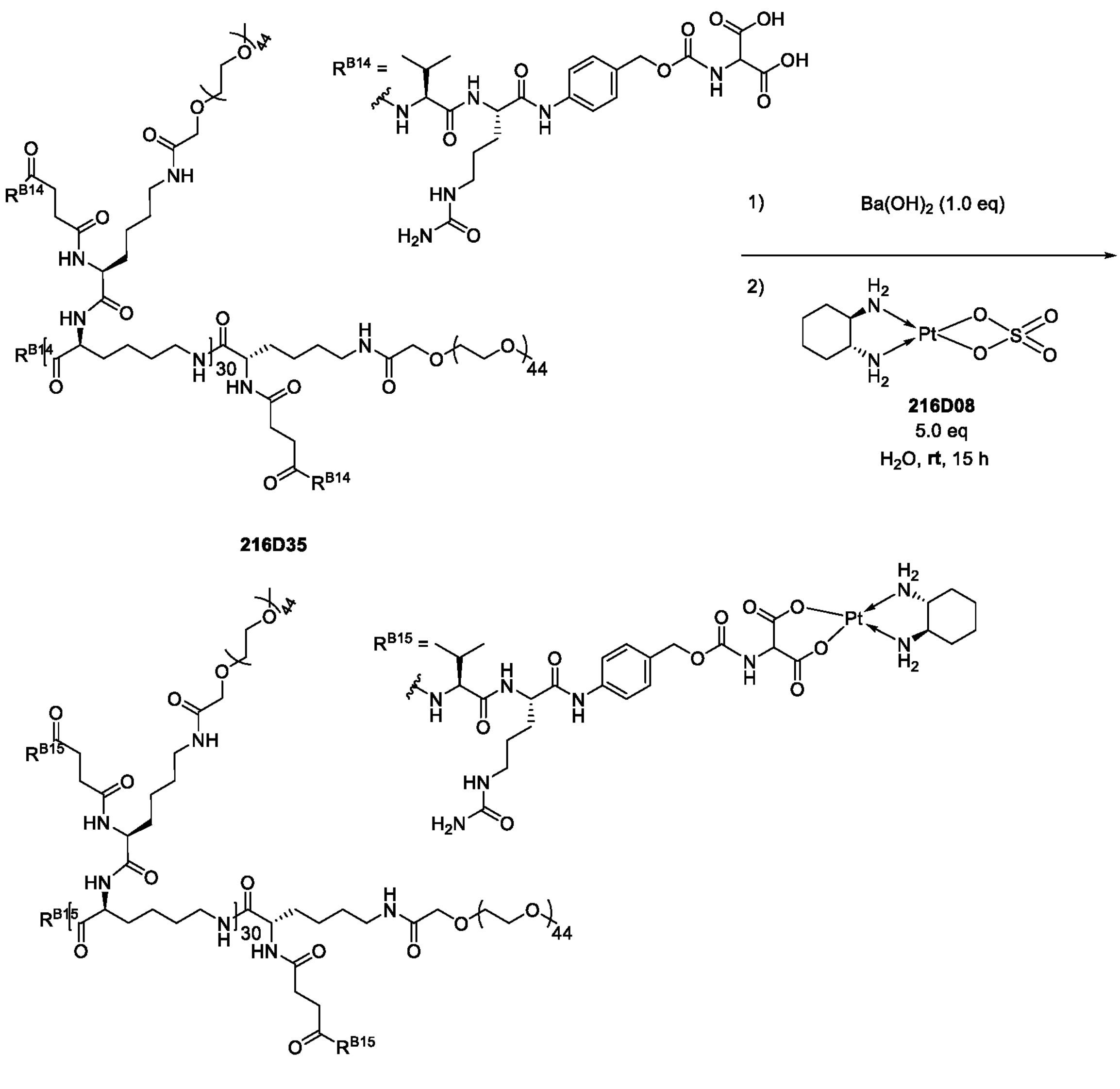


216D35

216D36

Synthesis of compound **216D34:**

The mixture of **101B13** (500 mg, 0.21 mmol, 1.0 eq), amine (176.8 mg, 0.32 mmol, 1.5 eq), PyBOP (165.0 mg, 0.32 mmol, 1.5 eq), DIPEA(0.19 mL, 1.06 mmol, 5.0 eq) and DMF (4.0 mL) were stirred for 15 h at room temperature under nitrogen atmosphere. HPLC showed complete reaction. MTBE (40 mL) was added to the DMF solution; the resultant precipitate was washed with MTBE, dissolved in MeOH, purified by ultrafiltration with MeOH/aqueous solution and freeze-dried to obtain compound **216D34,** 514 mg, as white solid.

Synthesis of compound **216D35:**

**216D34** (290 mg, 0.1 mmol, 1.0 eq) was added to NaOH solution (0.1 M, 5.0 mL, 0.5 mmol, 5.0 eq). The reaction was stirred for 3 h at room temperature. HPLC showed complete reaction. The solution was purified by ultrafiltration with water and freeze-dried to obtain the product **216D35,** 245 mg, white solid.

Synthesis of compound **216D36:**

$Ba(OH)_2$ solution (0.1 M, 0.3 mL, 0.03 mmol, 1.0 eq) was added to the aqueous solution (0.5 mL) of **216D35** (87 mg, 0.03 mmol, 1.0 eq). The mixture was stirred for 10 min at room temperature. 216D08 aqueous solution (0.2 M, 0.75 mL, 0.15 mmol, 5.0 eq) was then added to this solution. The reaction system was stirred for 15 h at room temperature. HPLC showed complete reaction. The mixture was filtered, purified by ultrafiltration with water and freeze-dried to obtain

the product **216D36**, 67 mg, white solid, purity 98%.

Example 59: synthesis of compound 21401

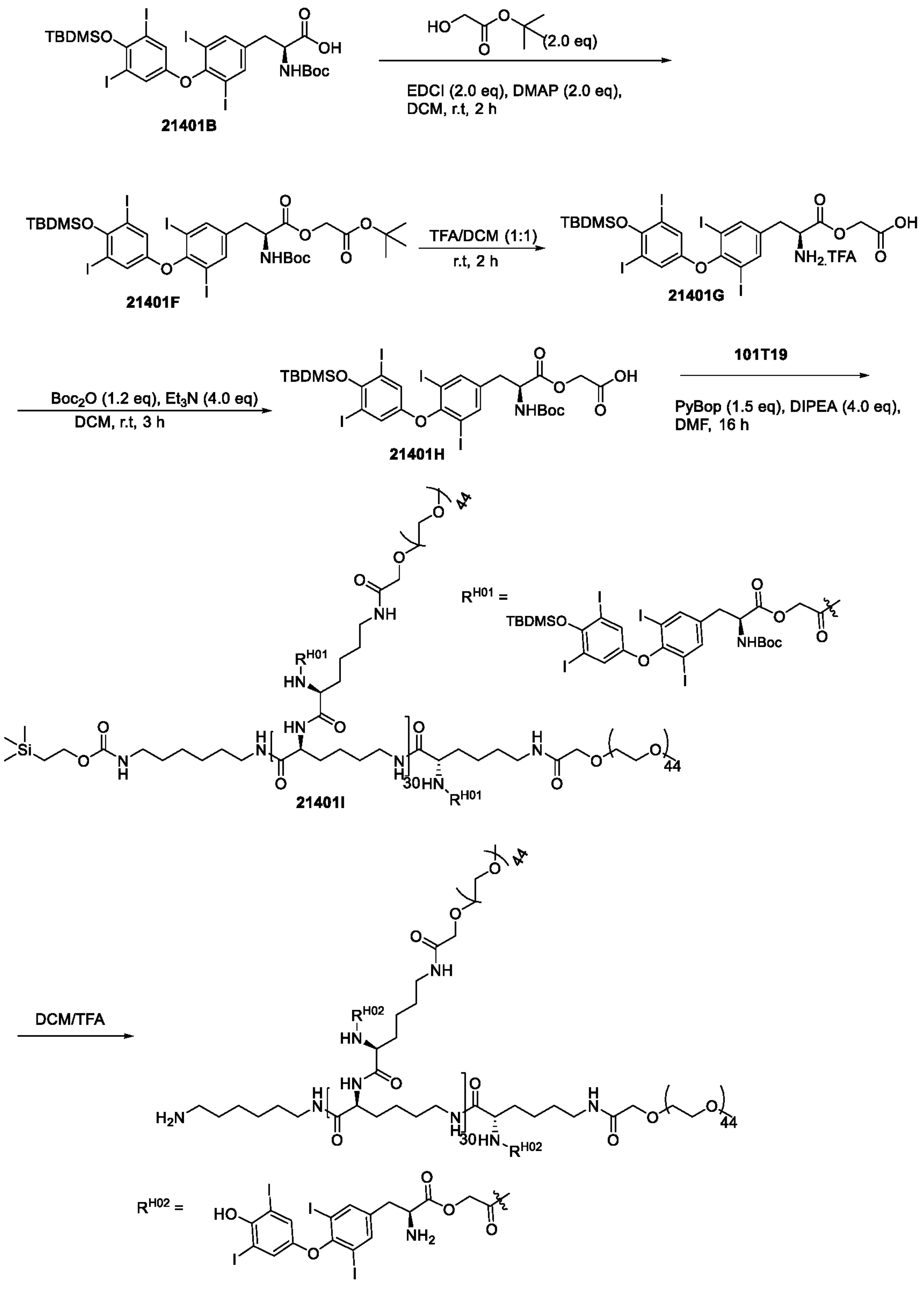


**21401B**

**21401F**

**21401G**

**21401H**

**21401I**

**21401**

Synthesis of compound **21401F:**

*tert*-Butyl glycolate (667 mg, 5.05 mmol, 2.0 eq), EDCI (965 mg, 5.05 mmol, 2.0 eq) and DMAP (615 mg, 5.05 mmol, 2.0 eq) were added to the DCM solution (40 mL) of **21401B** (2.5 g, 2.52 mmol, 1.0 eq) at 0°C. The mixture was stirred at room temperature for 16 h. LCMS showed high conversion and product generation. The solvent was removed with a rotovap. The residue was subjected to silica gel column chromatography (PE/EA=9:1) to obtain 1.64 g of white solid. MS (ESI), m/z, 1126.9 $[M+Na]^+$.

Synthesis of compound **21401G:**

TFA (10 mL) was added dropwisely to a solution (10 mL) of **21401F** (1.52 g, 1.38 mmol, 1.0 eq) in DCM at 0°C. The mixture was stirred at room temperature for 2 h. The solvent was removed with a rotovap. Acetonitrile (200 mL) was added and the residual TFA was removed with a rotovap to obtain 1.6 g of yellow solid.

MS (ESI), m/z, 949.0 $[M+H]^+$; m/z, 990.0 $[M+H+CH_3CN]^+$.

Synthesis of compound **21401H:**

$Boc_2O$ (361 mg, 1.65 mmol, 1.2 eq) and $Et_3N$ (557 mg, 5.52 mmol, 4.0 eq) were added to a solution (15 mL) of the compound **21401G** (1.52 g, 1.38 mmol, 1.0 eq) in DCM. The mixture was stirred at room temperature for 3 h. The solution was dried with a rotovap and the product was purified by chromatography with C18 column ($CH_3CN:H_2O$ = 5:95). The crude product partially decomposed during the purification. 230 mg of product was obtained. MS (ESI), m/z, 1070.9 $[M+Na]^+$.

Synthesis of compound **21401J:**

TBAF (1M in THF, 2.0 mL, 2.0 mmol, 2.9 eq) and AcOH (228 mg, 3.79 mmol, 5.5 eq) were added to a solution of the compound **21401H** (720 mg, 0.686 mmol, 1.0 eq) in THF (20 mL). The mixture was stirred at room temperature for 3 h. The solution was dried with a rotovap and the product was purified by chromatography with silica gel column (PE/EA=2:1~0:1~DCM/MeOH=10:1) and then with C18 column ($CH_3CN:H_2O$ = 5-70%). 230 mg of product was obtained. MS (ESI), m/z, 957.0 $[M+Na]^+$.

Synthesis of compound **214011:**

The mixture of compound **101T19** (78 mg, 0.032 mmol, 1.0 eq), **21401H** (50 mg, 0.048 mmol, 1.5 eq), PyBop (25 mg, 0.048 mmol, 1.5 eq), DIPEA (17 mg, 0.128 mmol, 4.0 eq) and DMF (6.0 mL) were stirred for 15 h at room temperature under nitrogen atmosphere. HPLC showed complete reaction. The mixture was filtered and the filtrate was purified by ultrafiltration with MeOH/aqueous solution and freeze-dried to obtain 60 mg of compound **214011** as white solid (Purity: 98.6%).

Synthesis of compound **21401:**

TFA (2 mL) was added dropwisely to a solution (2 mL) of compound **214011** (60 mg) in $CH_2Cl_2$. The mixture was stirred for 2 h at room temperature. The solution was dried with a rotovap. Saturated $NaHCO_3$ solution was added to adjust pH to 7. The crude product was dissolved, purified by ultrafiltration with MeOH/aqueous solution and freeze-dried to obtain 45 mg of light yellow solid.

Example 60: synthesis of compound 21404

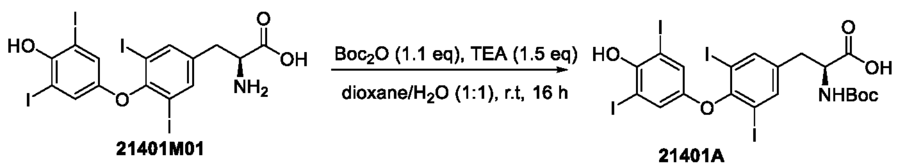


21401M01 21401A

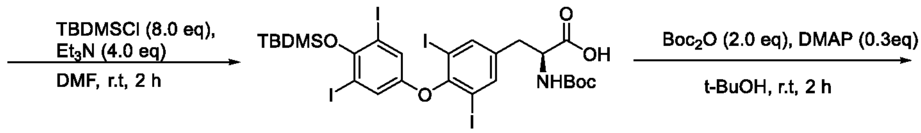


21401B

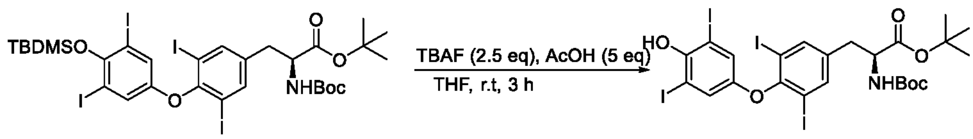


21404C 21401D

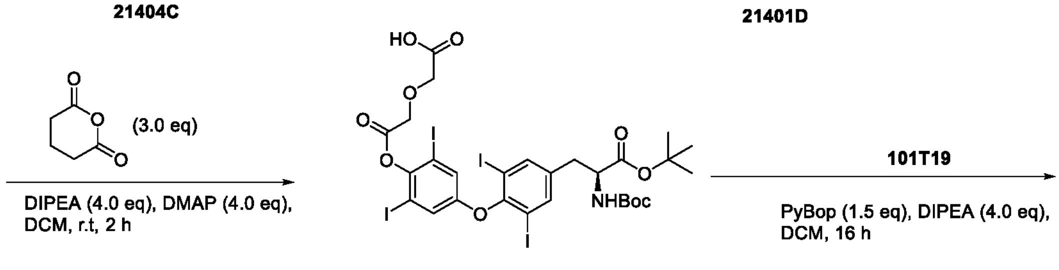


21404E

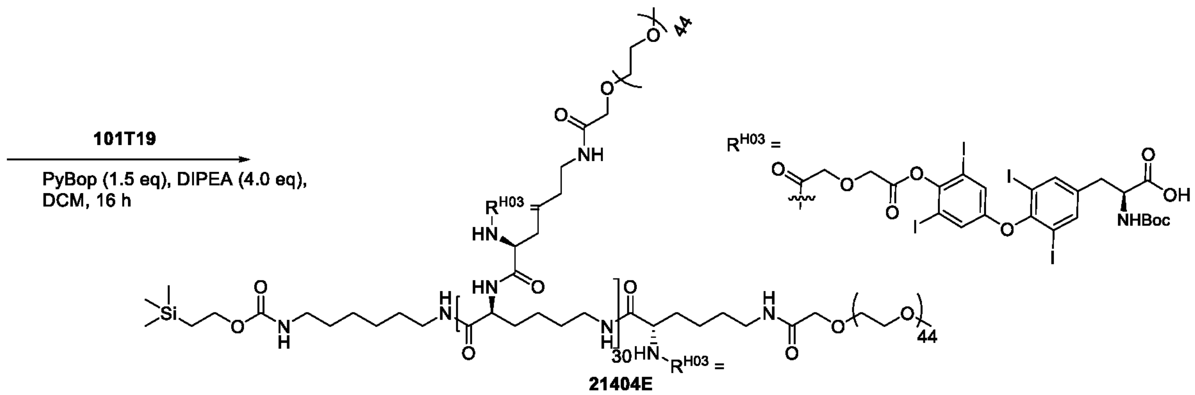


21404E

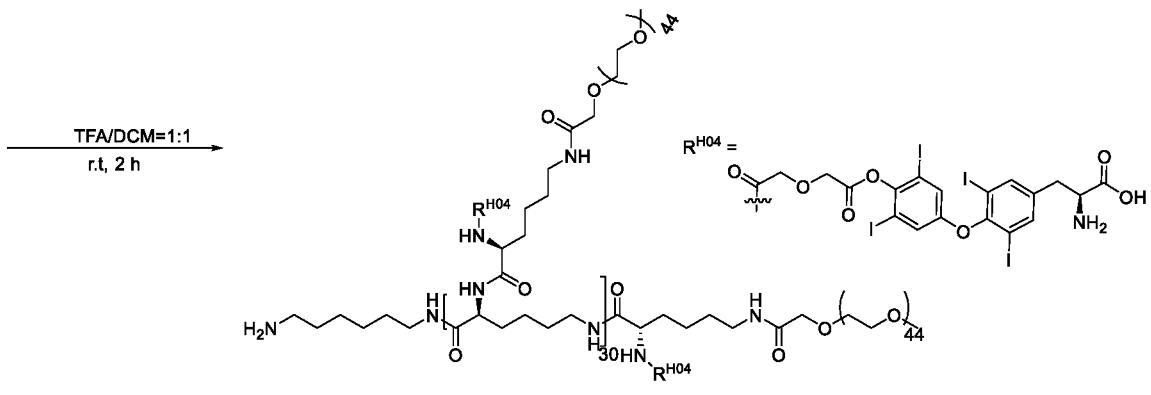


21404

Synthesis of compound **21401A:**

$Boc_2O$ (1.55 g, 7.10 mol, 1.1 eq) was added to a solution of compound **214M01** (5.0 g, 6.45 mmol, 1.0 eq) in dioxane/$H_2O$ (100 mL/100 mL) at 0°C. TEA was added dropwisely (980 mg, 9.70 mmol, 1.5 eq). The mixture was stirred at room temperature for 16 h. The reaction solution was poured into saturated NaCl (100 mL), the water phase was extracted with EA (100 mL × 3). The organic phase was combined, washed (500 mL × 2), dried over anhydrous $Na_2SO_4$ and concentrated to about 10-20 mL. The residual mixture was re-slurred in acetonitrile (200 mL) at room temperature. The precipitate was filtered and dried to obtain 5.1 g of light gray solid (Yield: 96.5%). MS (ESI), m/z, 821.10 $[M+H-C_4H_8]^+$; m/z, 777.10 $[M+H-Boc]^+$.

Synthesis of compound **21401B:**

TBDMSCl (3.44 g, 22.8 mmol, 8.0 eq) and TEA (1.15 g, 11.41 mmol, 4.0 eq) were added dropwisely to 21401A (2.5 g, 2.85 mmol, 1.0 eq) in DMF (20 mL) at 0°C. The mixture was stirred at room temperature for 2 h. The reaction solution was poured into saturated $NaHCO_3$ (aq) (200 mL), the water phase was extracted with DCM (150 mL × 2). The organic phase was combined, washed, dried over anhydrous $Na_2SO_4$ and concentrated. The crude residue was subjected to C18 column ($CH_3CN$:$H_2O$ = 5-95%) to obtain 2.5 g of white solid. MS (ESI), m/z, 936.20 $[M+H-C_4H_8]^+$.

Synthesis of compound **21401C:**

$Boc_2O$ (593 mg, 2.72 mmol, 2.0 eq) and DMAP (183 mg, 1.50 mmol, 1.1 eq) were added to a solution (20 mL) of compound **21401B** (1.5 g, 1.36 mmol, 1.0 eq) in tert-butanol. The mixture was stirred at room temperature for 2 h. The solution was dried with a rotovap and the residue was purified by silica gel column chromatography (PE/EA=20:1) to obtain 1.21 g of white solid. MS (ESI), m/z, 991.10 $[M+H-C_4H_8]^+$.

Synthesis of compound **21401D:**

TBAF (1 M, 2.9 mL, 2.9 mmol, 2.5 eq) and AcOH (345 mg, 5.75 mmol, 5.0 eq) were added dropwisely to a solution (25 mL) of compound **21404C** (1.2 g, 1.15 mmol, 1.0 eq) in THF. The mixture was stirred for 3 h at room temperature. The solution was dried with a rotovap and the resultant product was purified by chromatography with silica gel column (PE/EA=4:1), and then with C18 column ($CH_3CN$:$H_2O$ = 5-70%) to obtain 1.05 g of light yellow solid. MS (ESI), m/z, 876.30 $[M+H-C_4H_8]^+$.

Synthesis of compound **21404E:**

Glutaric anhydride (386 mg, 3.39 mmol, 3.0 eq), DIPEA (583 mg, 4.52 mmol, 4.0 eq) and DMAP (551 mg, 4.52 mmol, 4.0 eq) were added to the DCM solution (1.05 g, 1.13 mmol, 1.0 eq) of **21401D.** The mixture was stirred at room temperature for 2 h. The reaction solution was poured into aqueous saturated citric acid solution (50 mL), the water phase was extracted with DCM (100 mL × 3); the organic phase was combined, washed, dried over anhydrous $Na_2SO_4$ and concentrated. The residue was purified by silica gel column chromatography (PE/EA=1:1~ DCM/MeOH=10:1) to obtain 700 mg of white solid. MS (ESI), m/z, 935.00 $[M+H-C_4H_8-C_4H_8]^+$.

$^1$H NMR (400 MHz, Chloroform-*d*) δ 7.71 (s, 2H), 7.21 (s, 2H), 5.20 (d, *J* = 7.6 Hz, 1H), 4.44 (d, *J* = 6.9 Hz, 1H), 3.15 - 2.91 (m, 2H), 2.80 (t, *J* = 7.2 Hz, 2H), 2.63 (t, *J* = 7.3 Hz, 2H), 2.19 (p, *J* = 7.3 Hz, 2H), 1.49 (s, 9H), 1.47 (s, 9H).

Synthesis of compound **21404F:**

Compound 21404E (250 mg, 0.238 mmol, 1.49 eq), PyBop (125 mg, 0.240 mmol, 1.50 eq) and DIPEA were added to **101T19** (390 mg, 0.16 mmol, 1.0 eq) in DCM (5 mL). The mixture was stirred at room temperature overnight. The mixture was re-slurred in MTBE (50 mL) for 10 min. The residual solids were vacuum dried to get 610 mg of crude product.

Synthesis of compound **21404:**

TFA (3 mL) was added dropwisely to compound **21404E** (610 mg, 0.185 mmol, 1.0 eq) in DCM (3 mL). The mixture was stirred for 2 h at room temperature. The solvent was removed with a rotovap. Saturated $NaHCO_3$ (aq) was added to adjust pH to 7. The crude product was dissolved, purified by ultrafiltration with MeOH/aqueous solution and freeze-dried to obtain 250 mg of light yellow solid.

Example 61: synthesis of compound 21701

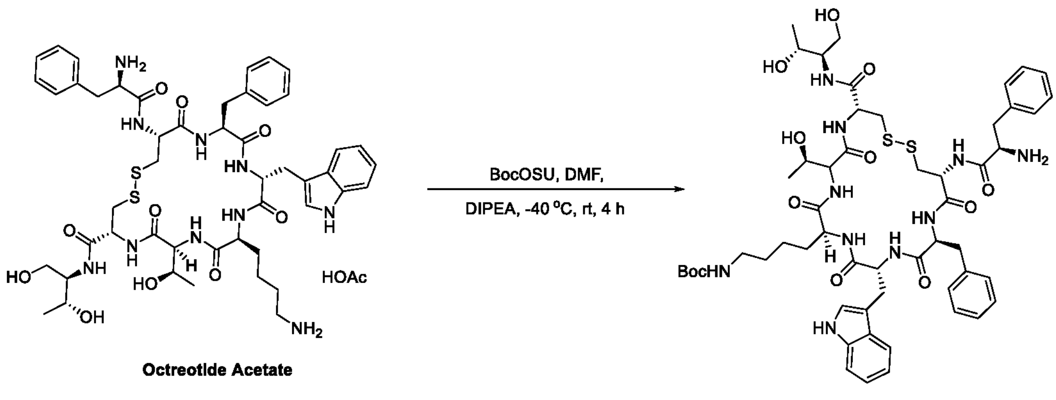


Octreotide Acetate

21701-1

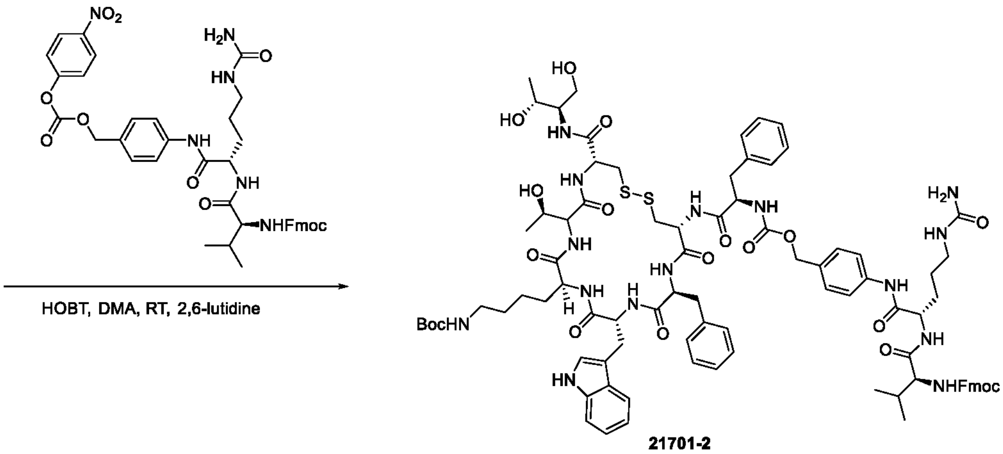


21701-2

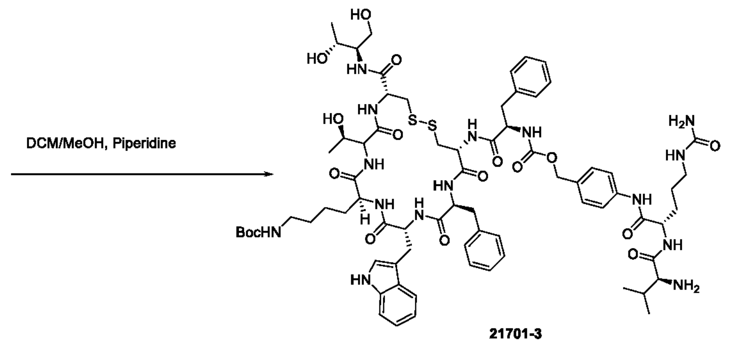


21701-3

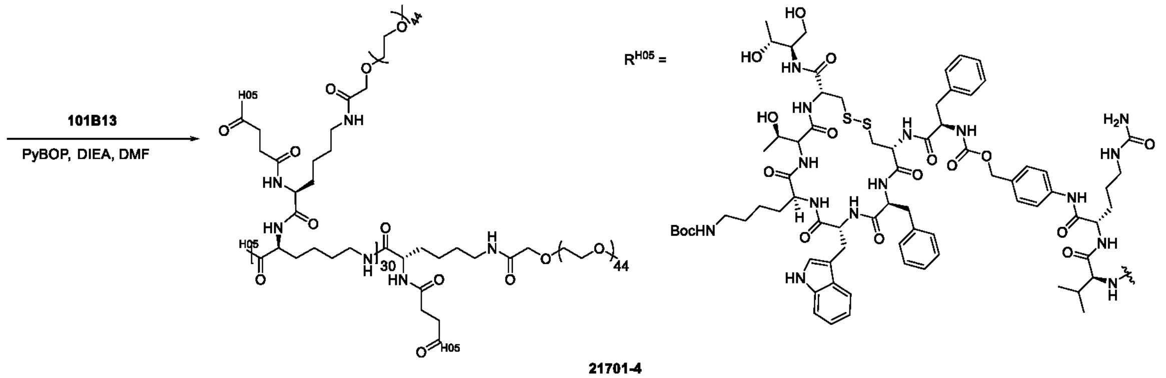


21701-4

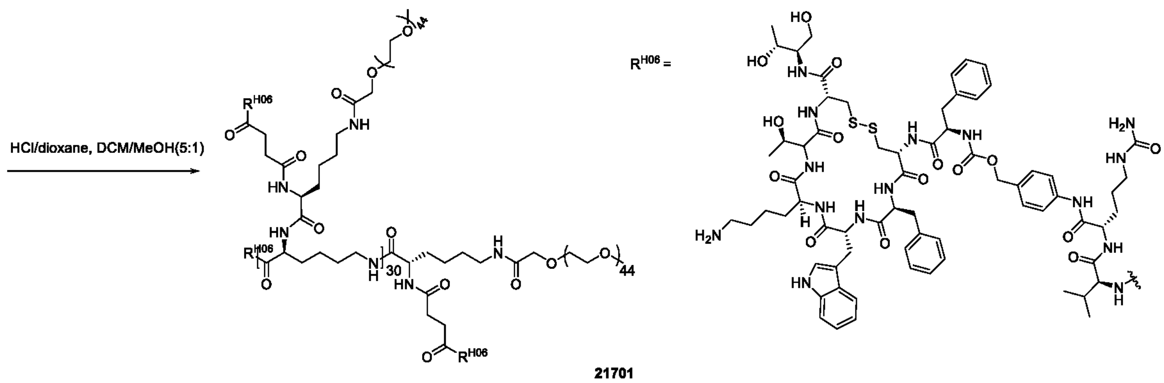


21701

Synthesis of compound **21701-1:**

DIPEA (1.14 g, 8.82 mmol, 3.0 eq) was added to a solution of Octreotide acetate (3.0 g, 2.94 mmol, 1.0 eq) in DMF (60 mL). N-succinimide carbonate tert-butyl carbonate (633 mg, 2.94 mmol, 1.0 eq) was added slowly at -40°C. The reaction mixture was stirred at -40°C for 0.5 h, and then at room temperature for 2 h under nitrogen atmosphere. LCMS showed complete reaction. The mixture was purified by silica gel column chromatography to obtain 2.8 mg of the product as a white solid (Yield: 85%). MS (ESI), m/z, 1118.40[M+H]$^+$.

Synthesis of compound **21701-2:**

**21701-1** (150 mg, 0.13 mmol, 1.0 eq), HOBT (35 mg, 0.26 mmol, 2.0 eq) and 2,6-dimethylpyridine (43 mg, 0.40 mmol, 3.0 eq) were added to a solution (10 mL) of **SM-1** (102 mg, 0.13 mmol, 1.0 eq) in DMF. The reaction mixture was stirred at room temperature overnight under nitrogen atmosphere. 50 mL of water was added. The mixture was filtered and the filter cake was dried to obtain 220 mg of white solid (Yield: 96.9%). MS (ESI), m/z, 1746.2 [M+H]$^+$.

Synthesis of compound **21701-3:**

Piperidine (20 mg, 0.23 mmol, 1.5 eq) was added to compound **21701-2** (270 mg, 0.15 mmol, 1.0 eq) in DCM/methanol (5:1, 1.0 mL). The mixture was stirred overnight at room temperature and under nitrogen atmosphere.

The mixture was directly concentrated and purified by column chromatography to obtain 210 mg of white solid(Yield: 89%). MS (ESI), m/z, 1524.2 $[M+H]^+$.

Synthesis of compound **21701-4:**

Compound **101B13** (0.09 g, 0.036 mmol), DIEA (0.2 mL) and PyBOP (0.037 g, 0.072 mmol) were added to **21701-3** (0.1 g, 0.065 mmol, 1.8 eq) in DMF (10 mL). The mixture was stirred overnight at room temperature and under nitrogen atmosphere. HPLC showed complete reaction. The mixture was directly concentrated to get 150 mg of crude product. The crude product was directly used in the next step.

Synthesis of compound **21701:**

**HCl/Dioxane** (4 M, 0.5 mL) was added to compound **21701-4** (0.15 g) in DCM/MeOH (5:1,10 mL) at 0°C. After HPLC showed complete reaction, the reaction liquid was purified by ultrafiltration with methanol and water and the filtrate was freeze-dried to obtain 90 mg of product (Purity: 98.79%).

Example 62: synthesis of compound 21702

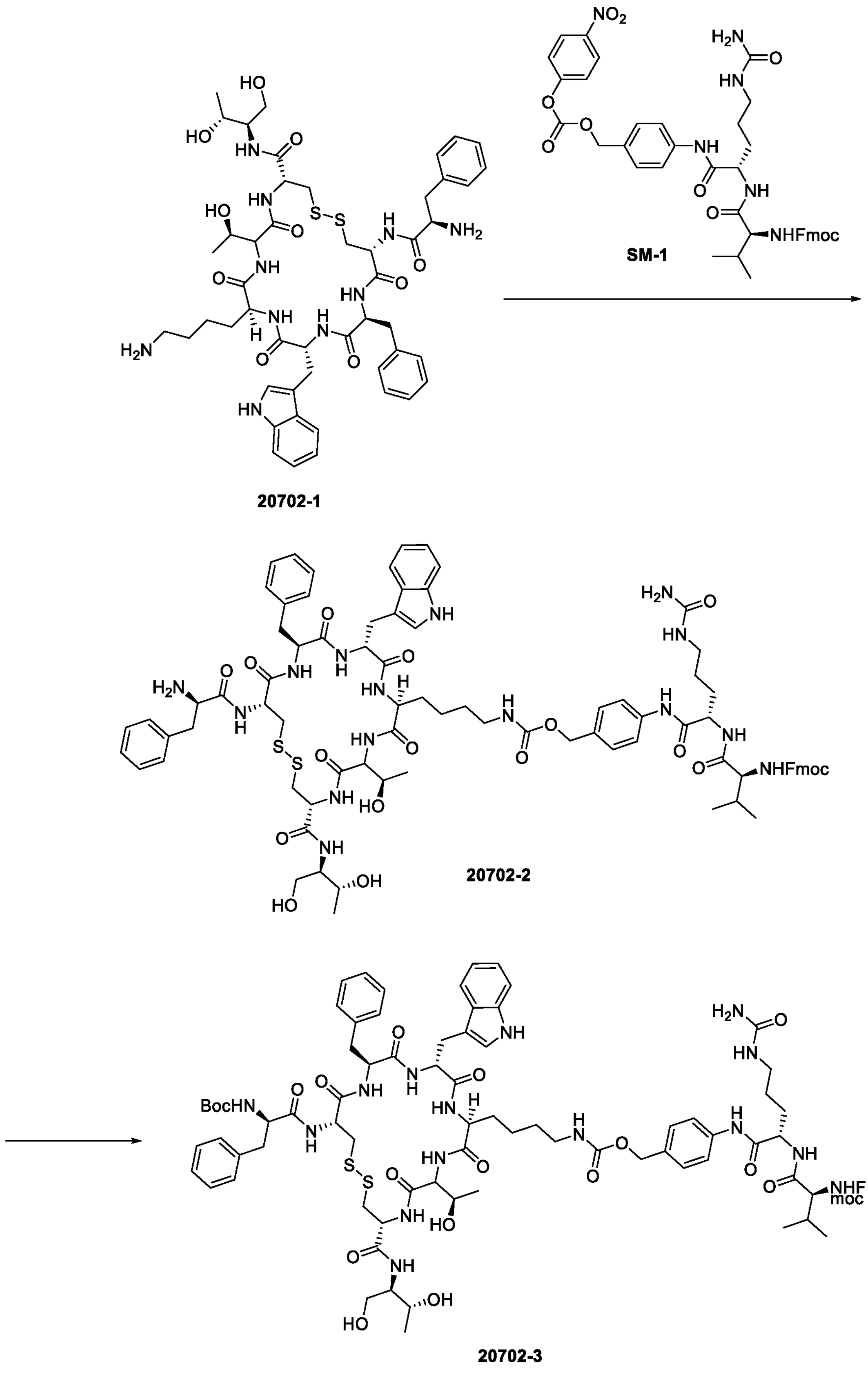

SM-1

20702-1

20702-2

20702-3

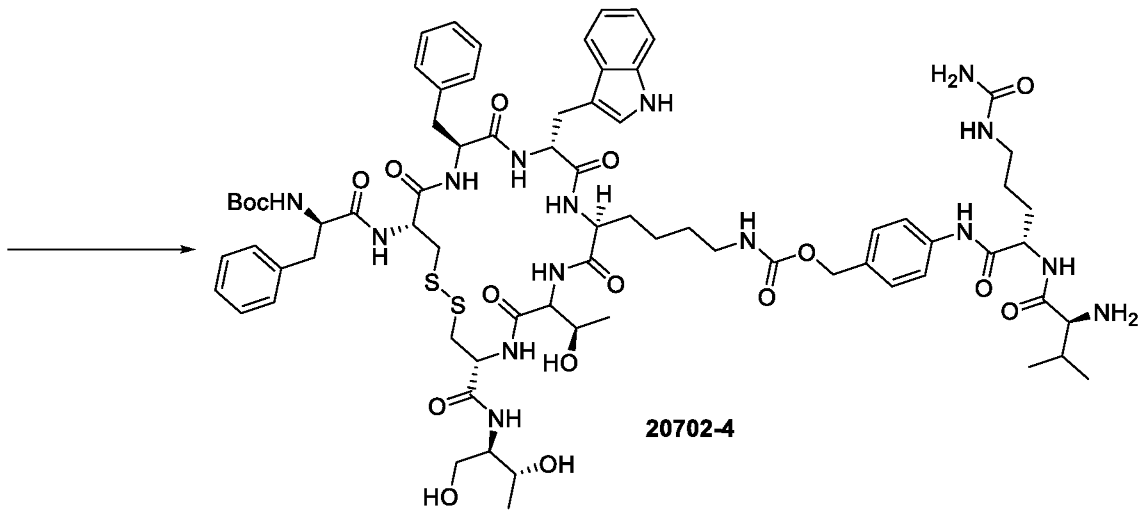

20702-4

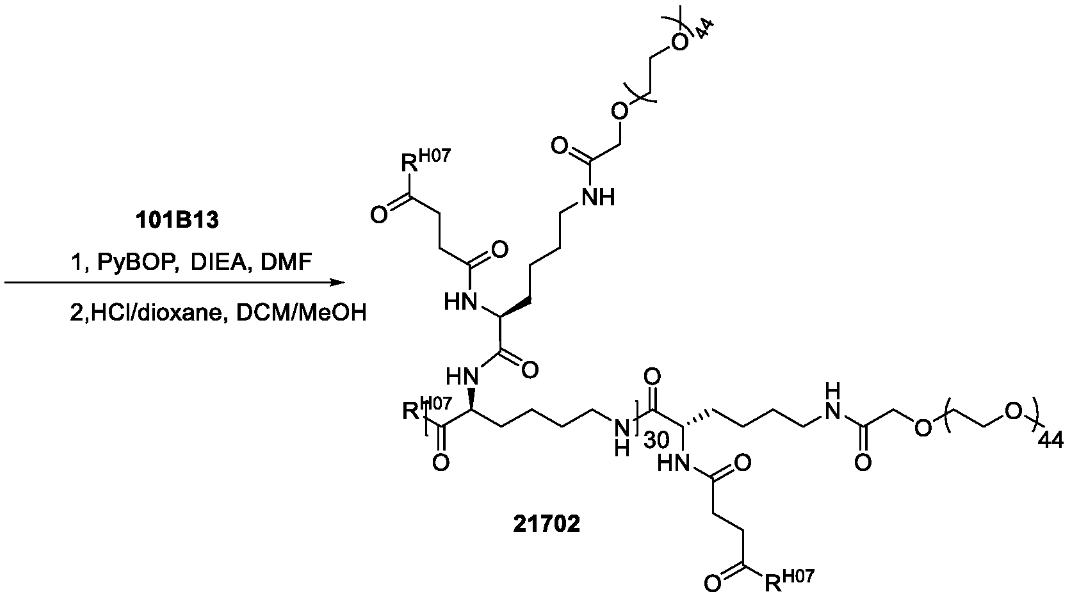


21702

$R^{H07}$ =

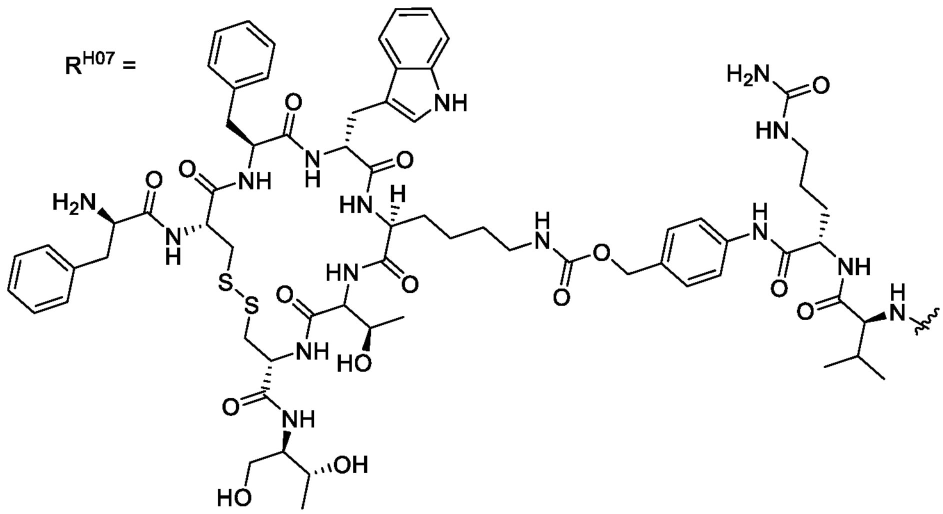

Synthesis of compound **21702-2:**

Compound **21701-1** (300 mg, 0.29 mmol, 1.0 eq), HOBT (80 mg, 0.58 mmol, 2.0 eq), and 2,6-dimethylpyridine

(95 mg, 0.87 mmol, 3.0 eq) were added to a solution of **SM-1** (225 mg, 0.29 mmol, 1.0 eq) in DMF (10 mL). The mixture was stirred overnight at room temperature under nitrogen atmosphere. 50 mL of water was added. The mixture was filtered and dried to obtain 310 mg of white solid (Yield: 63.9%). MS (ESI), m/z, 1646.2 $[M+H]^+$.

Synthesis of compound **21702-3:**

$Boc_2O$ (50 mg, 0.23 mmol, 1.2 eq) and catalytic amount of DMAP were added to compound **21702-2** (310 mg, 0.188 mmol, 1.0 eq) in DCM (20 mL). The mixture was stirred overnight at room temperature under nitrogen atmosphere. The mixture was directly concentrated and purified by column chromatography to obtain 250 mg of white solid (Yield: 76%). MS (ESI), m/z, 1746.2 $[M+H]^+$.

Synthesis of compound **21702-4:**

Piperidine (20 mg, 0.23 mmol, 1.5 eq) was added to compound **21701-3** (250 mg, 0.14 mmol, 1.0 eq) in DCM/methanol (5:1, 10 mL). The mixture was stirred overnight at room temperature under nitrogen atmosphere. The mixture was directly concentrated and purified by prep HPLC to obtain 130 mg of white solid (Yield: 59.6%). MS (ESI), m/z, 1524.2 $[M+H]^+$.

Synthesis of compound **21702-5:**

Compound **101B13** (0.119 g, 0.047 mmol), DIEA (0.2 mL) and PyBOP (0.037 g, 0.072 mmol) were added to a solution (10 mL) of compound **21702-4** (0.13 g, 0.085 mmol, 1.8 eq) in DMF. The mixture was stirred overnight at room temperature under nitrogen atmosphere. HPLC showed complete reaction. The mixture was directly concentrated to get 160 mg of product, which was used directly for the next step.

Synthesis of compound **21702:**

HCl/Dioxane (4M, 0.5 mL) was added to compound **21702-5** (0.16 g) in DCM/MeOH (5:1, 10 mL) at 0°C under nitrogen atmosphere. The reaction was monitored by HPLC. After HPLC showed complete reaction, the mixture was purified by ultrafiltration with methanol and water and the filtrate was freeze-dried to obtain 65 mg of the product (Purity: 98%).

Example 70: synthesis of compound 21703

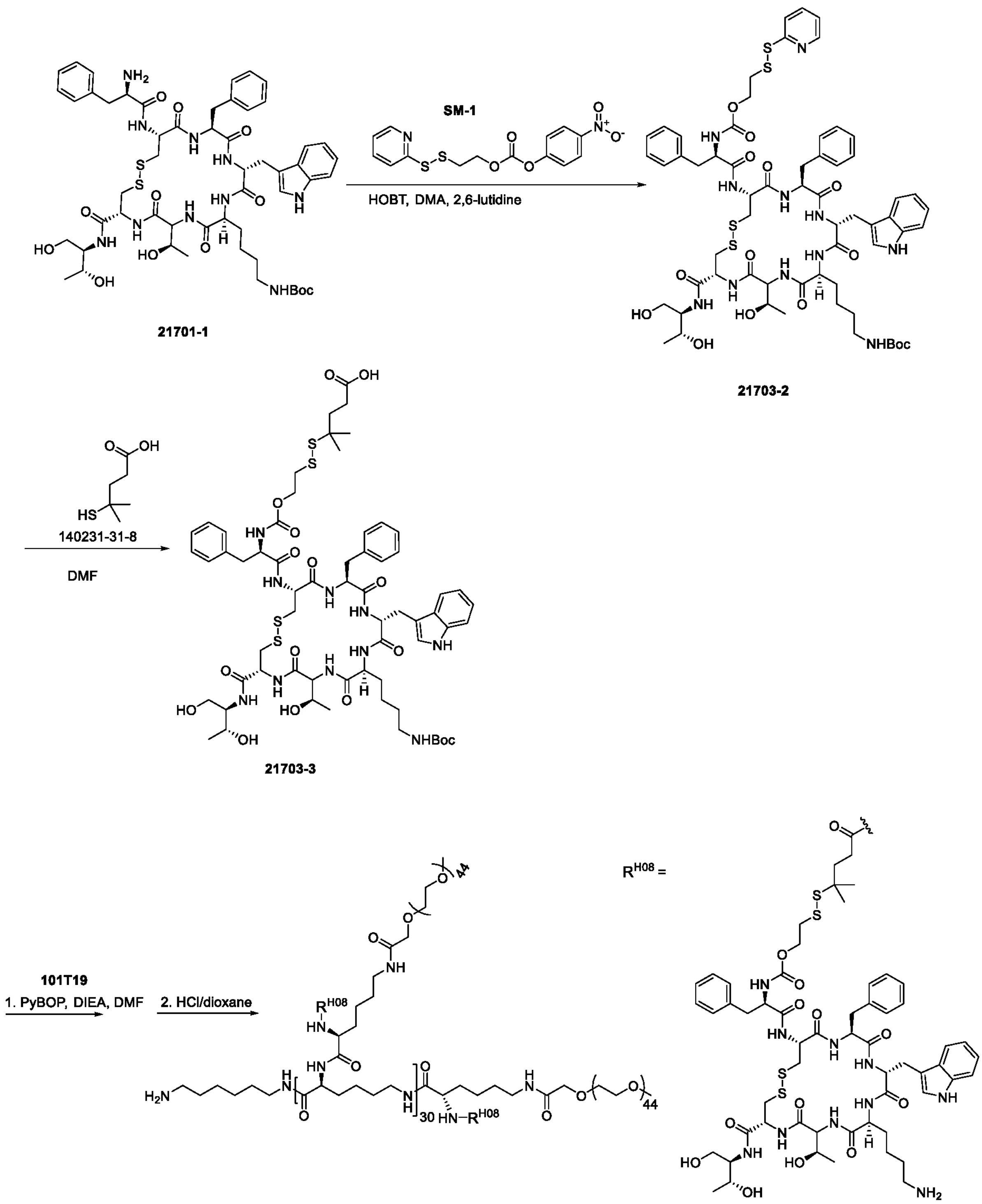


21701-1

21703-2

21703-3

21703

Synthesis of compound **21703-2:**

Compound **21701-1** (160 mg, 0.143 mmol, 1.0 eq), HOBT (40 mg, 0.29 mmol, 2.0 eq) and 2,6-dimethylpyridine (48 mg, 0.42 mmol, 3.0 eq) were added to a solution (10 mL) of **SM-1** (50 mg, 0.143 mmol, 1.0 eq) in DMF. The mixture was stirred overnight at room temperature under nitrogen atmosphere. 50 mL of water was added. The mixture was filtered and the filter cake was dried to obtain 130 mg of white solid (Yield: 68%). MS (ESI), m/z, 1332.2 $[M+H]^+$.

Synthesis of compound **21703-3:**

**SM-2** (20 mg, 0.108 mmol, 1.2 eq) and catalytic amount of acetic acid were added to compound **21702-2** (120 mg, 0.09 mmol, 1.0 eq) in DMF (10 mL). The mixture was stirred overnight at room temperature under nitrogen atmosphere. The mixture was directly concentrated and purified by prep HPLC to obtain 60 mg of white solid (Yield: 46.5%). MS (ESI), m/z, 1369.2 $[M+H]^+$.

Synthesis of compound **21703:**

Compound 101T19 (69 mg, 0.029 mmol), DIEA (0.2 mL) and PyBOP (0.037 g, 0.072 mmol) were added to compound **21703-3** (60 mg, 0.043 mmol, 1.5 eq) in DMF (10 mL) at room temperature 25°C under nitrogen atmosphere. The reaction was stirred overnight at room temperature under nitrogen atmosphere. The mixture was directly concentrated to obtain 80 mg of crude product. The crude product was dissolved in DCM/MeOH (5:1, 10 mL) and HCl/Dioxane (4M, 0.5 mL) was added. HPLC showed complete reaction. The mixture was purified by ultrafiltration and the filtrate was freeze-dried to obtain 60 mg of product (Purity: 99.5%).

Example 71: synthesis of compound 21704

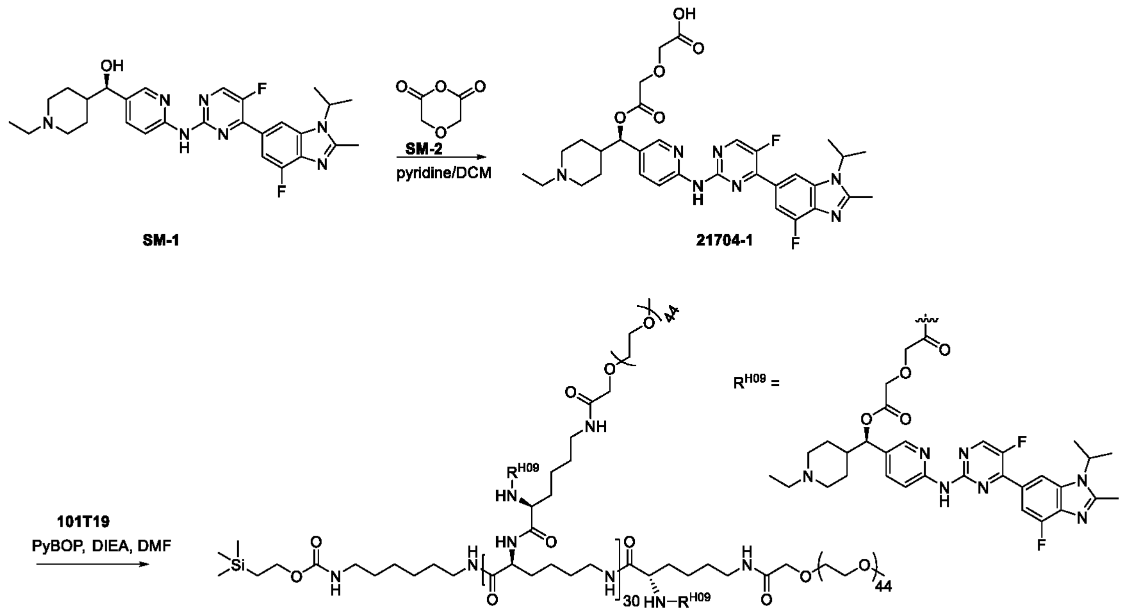


SM-1

21704-1

21704

Synthesis of compound **21704-1:**

**SM-2** (133 mg, 1.15 mmol, 1.2 eq) and pyridine (230 mg, 2.88 mmol, 3.0 eq) were added to **SM-1** (500 mg, 0.96 mmol, 1.0 eq) in DCM (20 mL) at room temperature under nitrogen atmosphere. The mixture was stirred overnight at room temperature under nitrogen atmosphere. The mixture was directly concentrated and the residue was purfied by prep HPLC to obtain 400 mg of white solid (Yield: 65.4%). MS (ESI), m/z, 638.2 $[M+H]^+$.

Synthesis of compound **21704:**

Compound **101T19** (0.992 g, 0.418 mmol), DIEA (0.5 mL) and PyBOP (0.435 g, 0.836 mmol) were added to compound **21704-1** (0.4 g, 0.627 mmol, 1.5 eq) in DMF (100 mL). The mixture was stirred overnight at room temperature under nitrogen atmosphere. The mixture was purified by ultrafiltration and the filtrate was freeze-dried to obtain 920 mg of product (Purity: 98%).

Example 63: synthesis of compound 21705

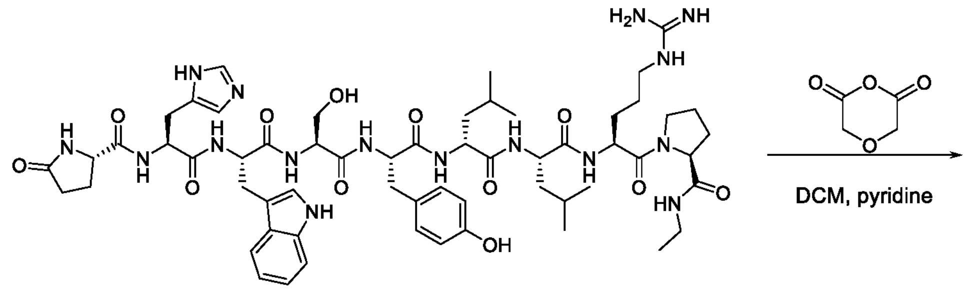


Leuprorelin

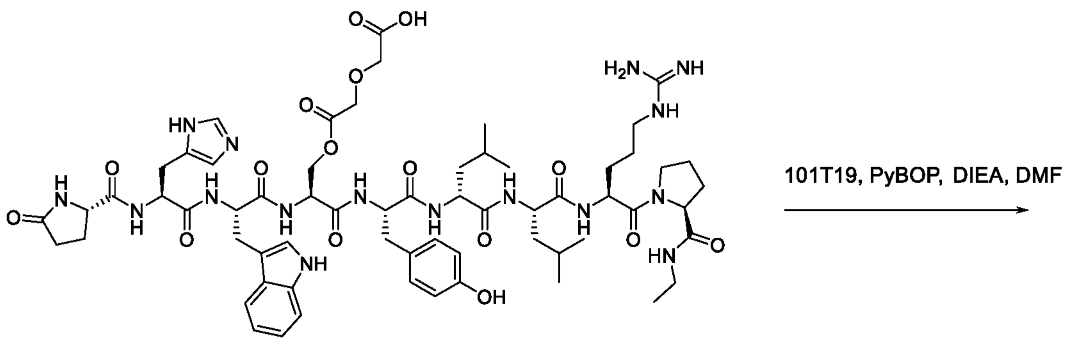


**21705-1**

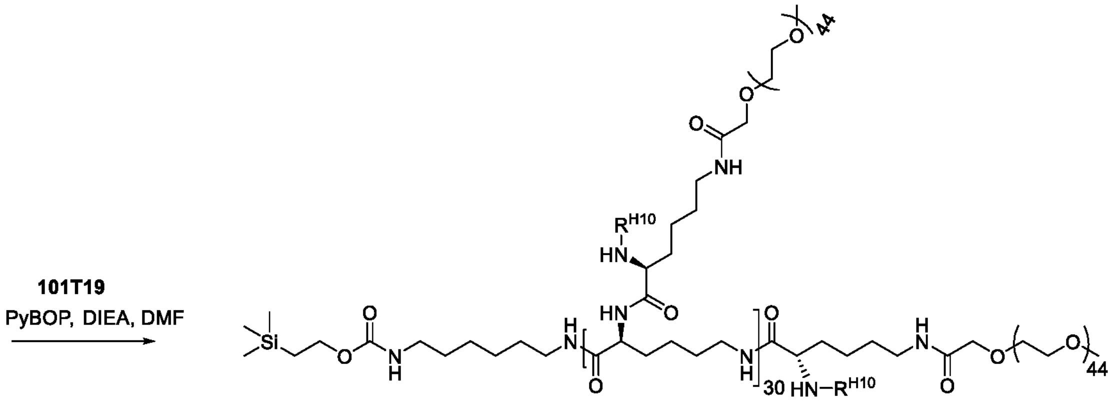


**21705**

$R^{H10}$ =

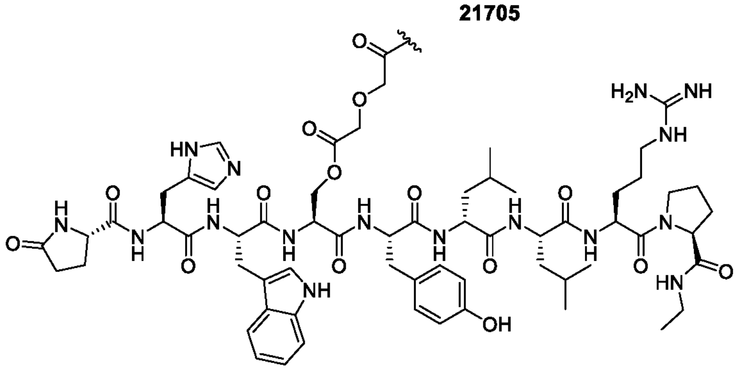

Synthesis of compound **21705-1:**

Leuprolide (750 mg, 0.62 mmol, 1.0 eq) and pyridine (147 mg, 01.86 mmol, 3.0 eq) were added to a solution (10 mL) of compound **SM-1** (94 mg, 0.807 mmol, 1.3 eq) in DMF. The mixture was stirred overnight at room temperature under nitrogen atmosphere. 50 mL of water was added. The mixture was filtered and the filter cake was dried to obtain 260 mg of white solid (Yield: 31.7%). MS (ESI), m/z, 1325.2 $[M+H]^+$.

Synthesis of compound **21705:**

Compound **101T19** (119 mg, 0.05 mmol), DIEA (0.2 mL) and PyBOP (0.052 g, 0.1 mmol) were added to a solution (10 mL) of compound **21705-1** (100 mg, 0.075 mmol, 1.5 eq) in DMF. The mixture was stirred overnight at room temperature under nitrogen atmosphere. The mixture was purified by ultrafiltration and the filtrate was freeze-dried to obtain 95 mg of product (Purity: 99%).

Example 64: synthesis of compound 21706

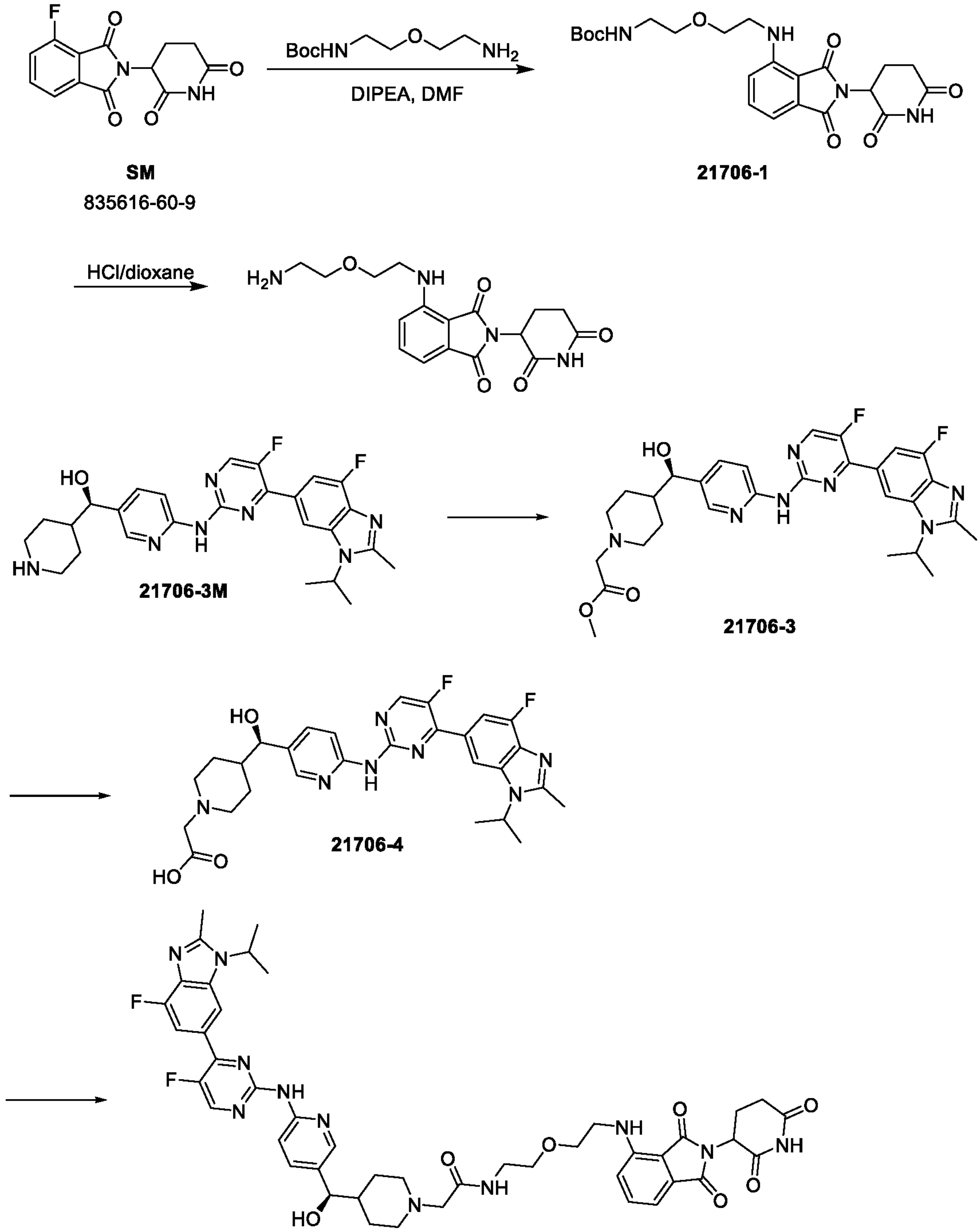


**SM**
835616-60-9

**21706-1**

**21706-3M**

**21706-3**

**21706-4**

**21706-5**

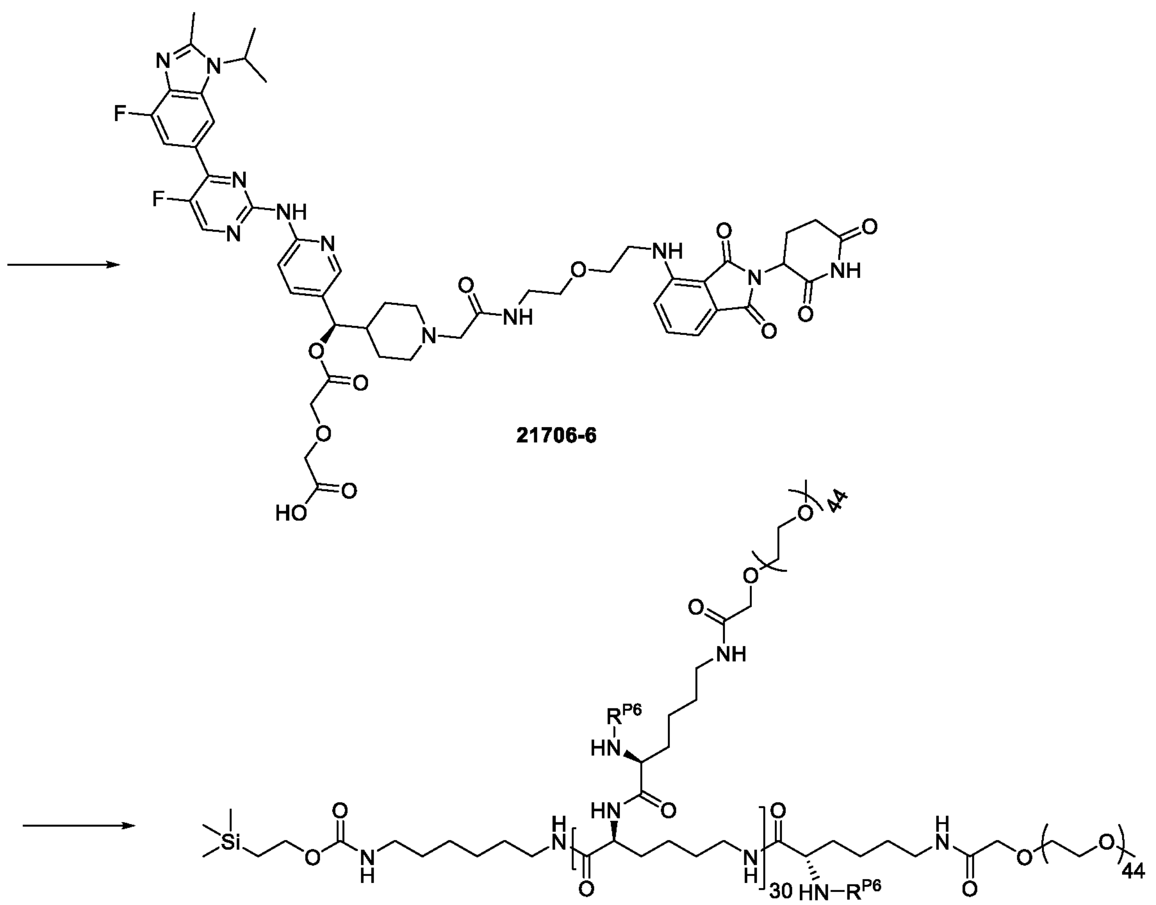

21706-6

21706

$R^{P6}$ =

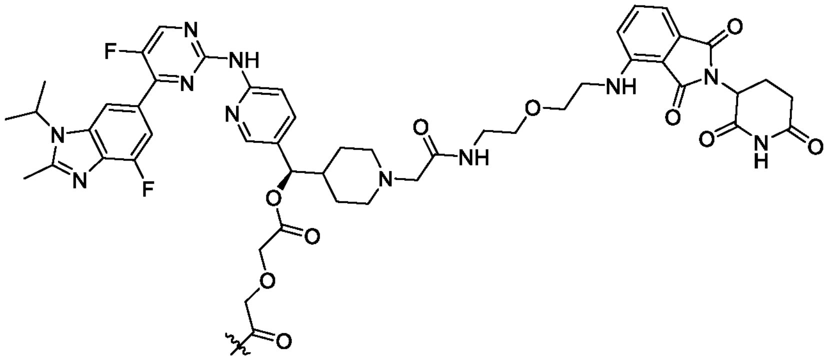

Synthesis of compound **21706-1:**

2-(2,6-dioxo-piperidin-3-yl)-4-fluoro-isoindole-1, 3-dione (1.2 g, 6.1 mol, 1.2 eq) and DIPEA (1.3 h, 10.1 mmol, 2.0 eq) were added to a solution (20 mL) of compound **SM-1** (1.4 g, 5.1 mol, 1.0 eq) in DMF. The mixture was stirred at 90°C for 3 h. The reaction was quenched with water and the reaction was extracted with DCM (100 mL × 4); the organic phase was pooled, dehydrated and concentrated and the residue was purified by column chromatography to obtain 800 mg of compound **21706-1** as light yellow solid. LCMS (ESI) M-100 = 361.1; M-56 = 405.0.

Synthesis of compound **21706-2:**

Hydrochloride in dioxane (4.0 M, 10 mL) was added to compound **21706-1** (0.8 g, 1.74 mmol) in DCM (10 mL) at 0°C. The mixture was stirred for 1 h under nitrogen atmosphere. The reaction mixture was concentrated and used directly in the next step. MS (ESI), m/z, 361.1 $[M+1]^+$.

Synthesis of compound **21706-3:**

Methyl bromoacetate (552 mg, 3.6 mmol, 1.2 eq) and potassium carbonate (500 mg, 3.6 mmol, 1.2 eq, 4.0 M, 10 mL) were added to **21706-3M** (prepared according to Example 40 of the patent CN108794452B) in DMF. The mixture was stirred for 1 h at 50°C under nitrogen atmosphere. The reaction was quenched with water, and the reaction was extracted with DCM (100 mL × 4); the organic phase was pooled, washed, dehydrated and concentrated and the residue was subj ected to column chromatography to obtain 1.4 g of compound **21706-3** as light yellow solid. MS (ESI), m/z, 566.2 $[M+1]^+$.

Synthesis of compound **21706-4:**

Lithium hydroxide (600 mg, 25 mmol) was added to a solution of compound **21706-3** (1.4 g, 2.48 mmol) in THF (20 mL) and water (5 mL). The mixture was stirred for 2 h at 0°C under nitrogen atmosphere. The reaction was quenched with water and the reaction was extracted with DCM (100 mL × 4); the organic phase was pooled, washed, dehydrated, and concentrated and the residue was purified by column chromatography to obtain 1.3 g of compound **21706-4** as light yellow solid. MS (ESI), m/z, 552.1 $[M+1]^+$.

Synthesis of compound **21706-5:**

HATU (1.3 g, 3.3 mmol, 2.0 eq) was added to compound **21706-2** (600 mg, 1.7 mmol, 1.0 eq) and compound **21706-4** (920 mg, 1.7 mmol, 1.0 eq) in DCM (20 mL). The mixture was stirred for 2 h at 0°C under nitrogen atmosphere. The reaction was quenched with water and the reaction liquid was extracted with DCM (100 mL × 4); the organic phase was pooled, washed, dehydrated, and concentrated and the residue was purified by column chromatography to obtain 1.1 g of compound **21706-5.** MS (ESI), m/z, 894.2 $[M+1]^+$.

$^1H$ NMR (400 MHz, DMSO-$d_6$) δ 11.12 (s, 1H), 11.00 (s, 1H), 9.52 (s, 1H), 8.80 (d, *J* = 3.6 Hz, 1H), 8.62 (t, *J* = 5.5 Hz, 1H), 8.35 - 8.24 (m, 2H), 8.03 (d, *J* = 8.7 Hz, 1H), 7.92 (dd, *J* = 8.9, 2.2 Hz, 1H), 7.73 (d, *J* = 11.9 Hz, 1H), 7.58 (dd, *J* = 8.6, 7.1 Hz, 1H), 7.13 (d, *J* = 8.6 Hz, 1H), 7.05 (d, *J* = 7.0 Hz, 1H), 6.58 (d, *J* = 5.8 Hz, 1H), 5.05 (dd, *J* = 12.7, 5.4 Hz, 1H), 4.86 (p, *J* = 6.9 Hz, 1H), 4.45 (d, *J* = 6.1 Hz, 1H), 3.85 - 3.80 (m, 2H), 3.61 (t, *J* = 5.4 Hz, 2H), 3.47 (dq, *J* = 13.4, 8.6, 7.1 Hz, 6H), 3.32 (t, *J* = 5.8 Hz, 2H), 3.02 - 2.82 (m, 3H), 2.67 (s, 3H), 2.63 - 2.56 (m, 1H), 2.53 (d, *J* = 4.4 Hz, 1H), 2.46 (d, *J* = 4.3 Hz, 1H), 2.06 - 1.98 (m, 1H), 1.90 (d, *J* = 13.2 Hz, 1H), 1.78 (s, 1H), 1.63 (d, *J* = 6.9 Hz, 6H), 1.60 - 1.50 (m, 2H).

Synthesis of compound **21706-6:**

Diglycolic anhydride (78 mg, 0.68 mmol, 1.2 eq) and pyridine (132 mg, 1.68 mmol, 3.0 eq) were added to compound **21706-5** (500 mg, 0.56 mmol, 1.0 eq) in DCM (10 mL). The mixture was stirred for 12 h at room temperature under nitrogen atmosphere. The mixture was directly concentrated and the residue was purified by prep HPLC to obtain 400 mg of white solid. MS (ESI), m/z, 1010.3 $[M+H]^+$.

Synthesis of compound **21706:**

Compound **21706-6** (0.38 g, 0.38 mmol, 1.5 eq), PyBOP (262 mg, 0.5 mmol, 2.0 eq) and DIPEA (147 mg, 1.1 mmol, 4.5 eq) were added to a solution (10 mL) of compound **101T19** (600 mg, 0.25 mmol, 1.0 eq) in DMF. The mixture was stirred overnight at room temperature under nitrogen atmosphere. The mixture was purified by ultrafiltration and the filtrate was freeze-dried to obtained 700 mg of product.

Example 65: synthesis of compound 20323C

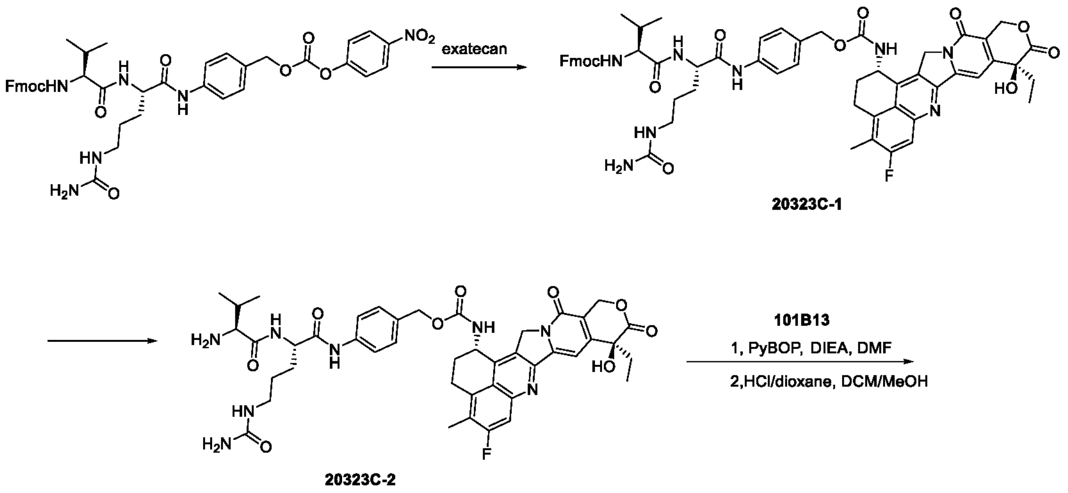


20323C-1

20323C-2

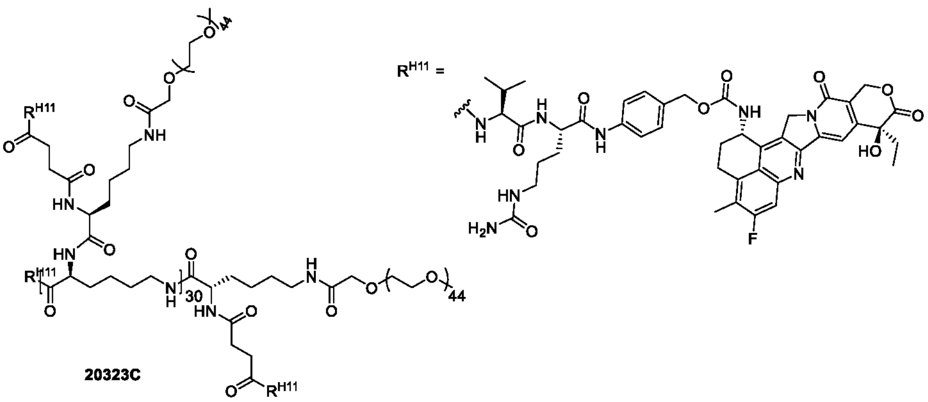

20323C

Synthesis of compound **20323C-1:**

Fmoc-Val-Cit-PAB-PNP (CAS No. 863971-53-3, 0.79 g, 1.0 mmol, 1.1 eq) and DIPEA (0.36 g, 2.82 mmol, 3.0 eq) were added to a suspension of Exatecan mesylate (0.5 g, 0.94 mmol, 1.0 eq) in DMF (10 mL). The mixture was stirred overnight at room temperature under nitrogen atmosphere. The mixture was directly concentrated and the residue was purified by column chromatography to obtain 0.85 g of compound **20323C-1** (Yield: 85%).

Synthesis of compound **20323C-2:**

Piperidine (1 mL) was added to a suspension of compound **20323C-1** (0.8 g, 0.75 mmol, 1.0 eq) in DMF (5 mL) at 0°C. The mixture was stirred for 1 h. The mixture was directly concentrated and the residue was purified by column chromatography to obtain 0.55 g of compound **20323C-2** (Yield: 87%).

Synthesis of compound **20323C:**

Compound **101B13** (800 mg, 0.01 mmol, 1.0 eq), compound **20323C-2** (420 mg, 0.50 mmol, 46.0 eq), PyBOP (351 mg, 0.68 mmol, 62.0 eq), DIPEA (108 mg, 0.84 mmol, 77.0 eq) and DMF (5 mL) were charged and the reaction was stirred for 15 h at room temperature under nitrogen atmosphere. HPLC showed complete reaction. MTBE (20 mL) was added to the DMF solution and the mixture was stirred for 0.5 h. The residue was dissolved, purified by ultrafiltration with MeOH/$H_2O$ and the filtrate was freeze-dried to obtain 880 mg of compound 20323C as white solid (Purity: 98.1%).

Example 66: synthesis of compound 22101

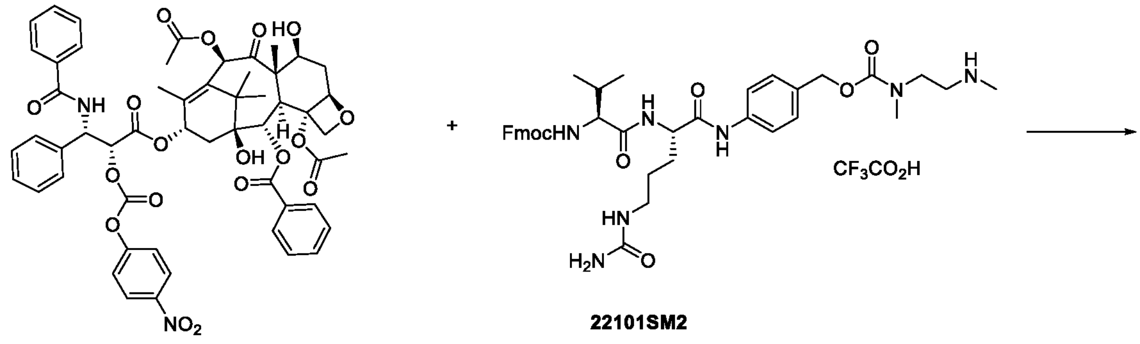


22101SM1

22101SM2

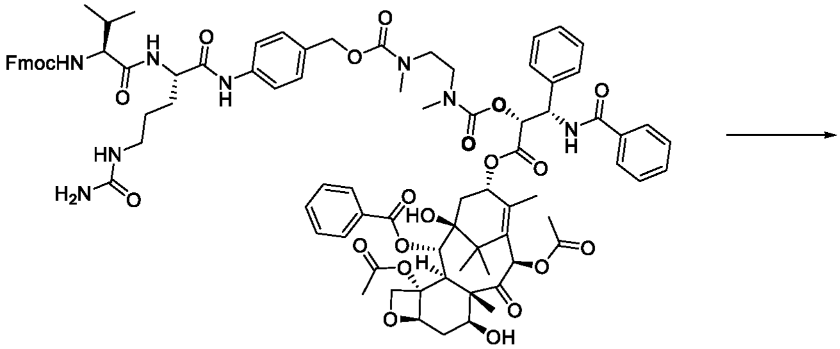

22101A

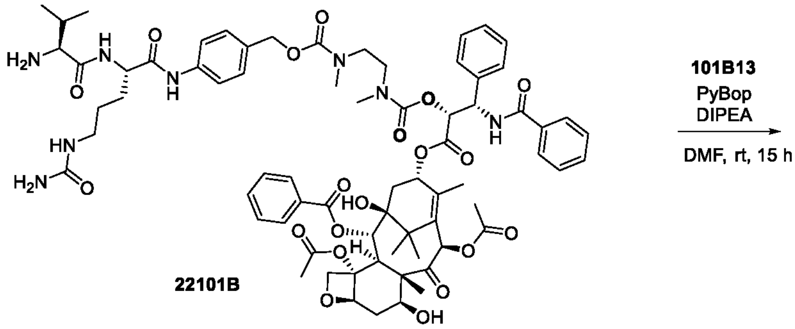


22101B

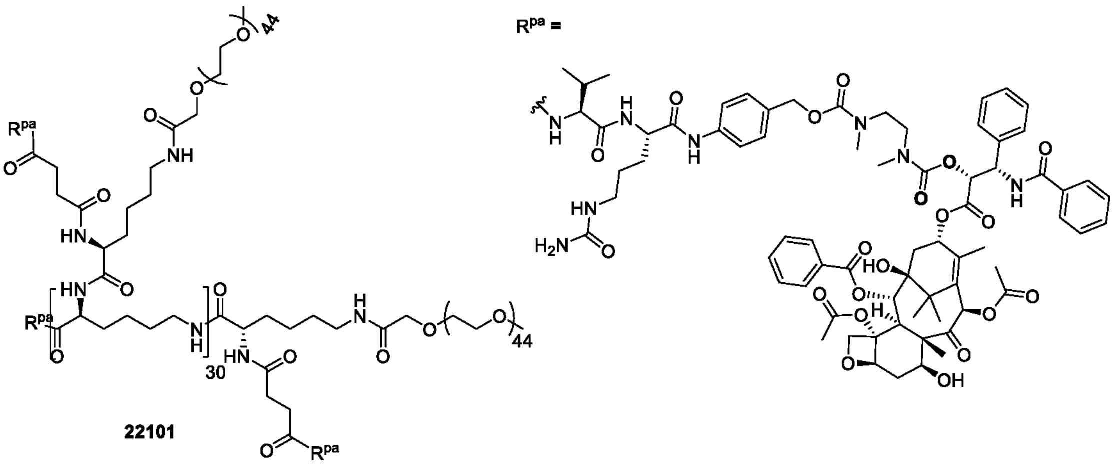

22101

Synthesis of compound **22101A:**

**22101SM1** (Reference: J. Med. Chem. 2000, 43, 3093-3102 compound 7, 0.5 g, 0.49 mmol, 1.0 eq) in DMF (5 mL) was added **22101SM2** (TFA salt, reference: patent WO2016151432A1 Example 96, 0.41 g, 0.49 mmol, 1.0 eq) and DIPEA (0.32 g, 2.46 mmol, 5.0 eq) at 0°C. The mixture was stirred overnight at room temperature under nitrogen atmosphere. The mixture was concentrated and directly used for the next step. MS (ESI), m/z, 1595.2 $[M+H]^+$.

Synthesis of compound **22101B:**

Piperidine (1 mL) was added to a solution of the above crude product **22101A** in DMF (5 mL) and the mixture was stirred for 1 h. The mixture was concentrated and purified by prep HPLC to obtain 450 mg of compound **22101B.** MS (ESI), m/z, 1373.6 $[M+H]^+$.

Synthesis of compound **22101:**

Compound **101B13** (500 mg, 0.007 mmol, 1.0 eq), compound **22101B** (430 mg, 0.31 mmol, 46.0 eq), PyBOP (220 mg, 0.42 mmol, 62.0 eq), DIPEA (68 mg, 0.52 mmol, 77.0 eq), and DMF (5 mL) were charged and the reaction was stirred for 15 h at room temperature under nitrogen atmosphere. HPLC showed complete reaction. MTBE (20 mL) was added to the DMF solution and the mixture was stirred for 0.5 h. The residue was dissolved, purified by ultrafiltration with $MeOH/H_2O$ and the filtrate was freeze-dried to obtain 750 mg of compound **22101** as white solid (Purity: 98.8%).

Example 67: synthesis of compound 22102

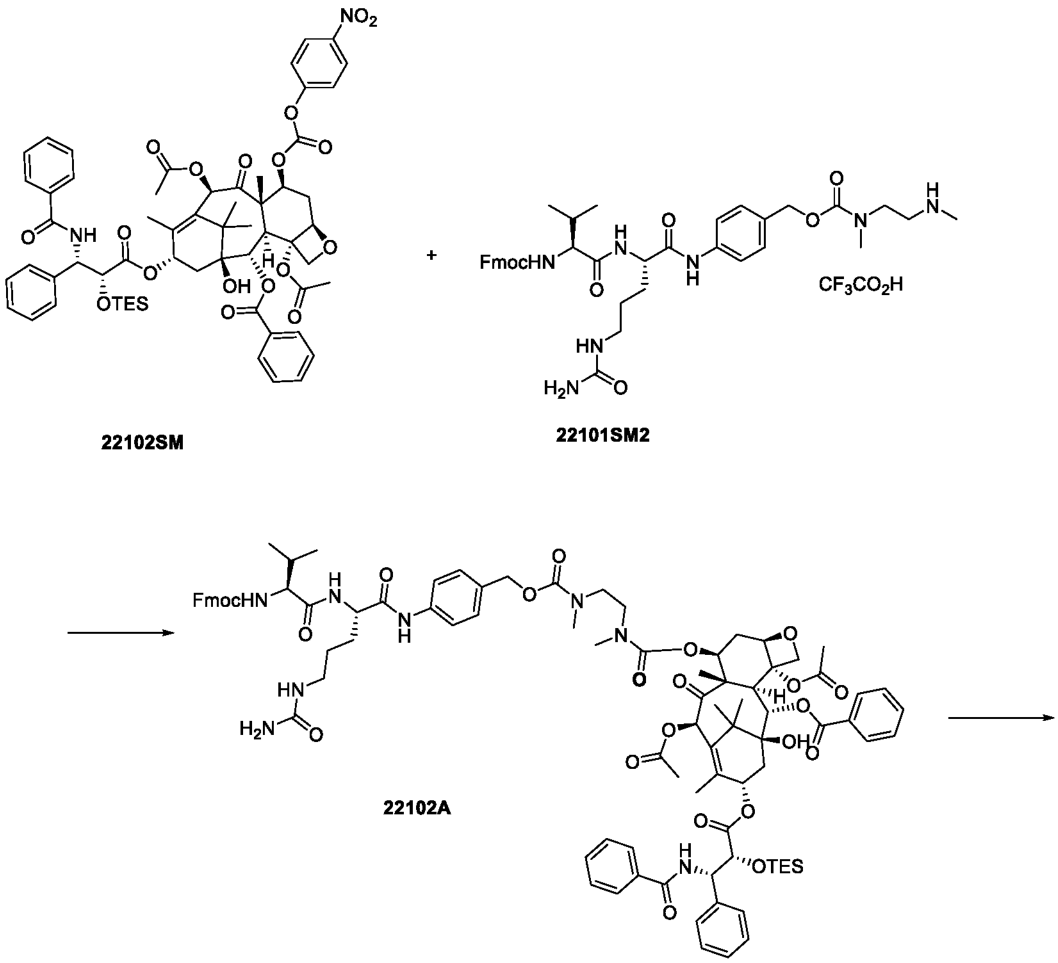

22102SM

22101SM2

22102A

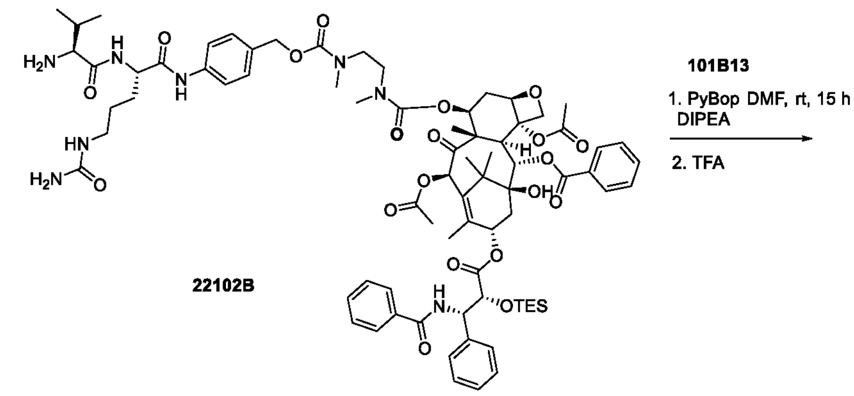


22102B

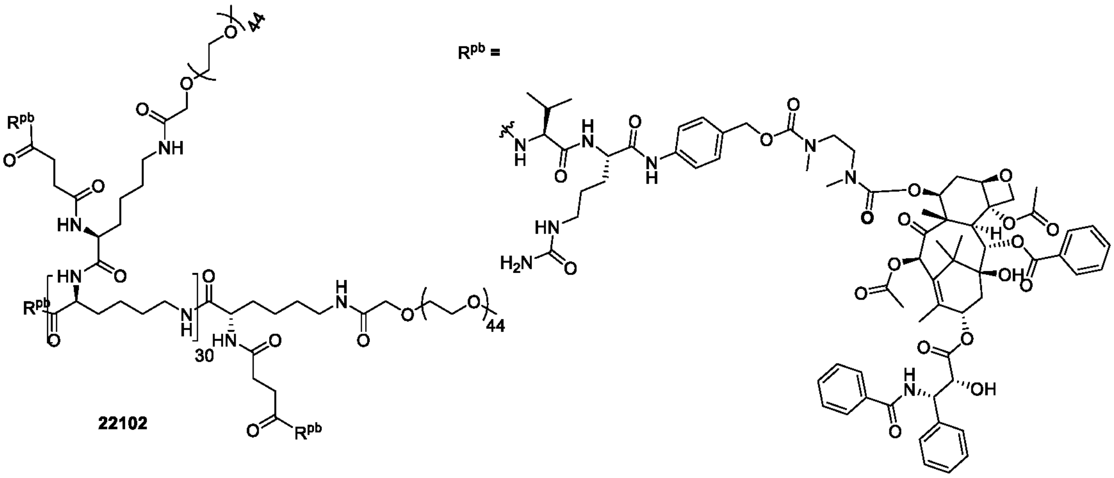

22102

$R^{pb}$ =

Synthesis of compound **22102A:**

To a solution of **22102SM** (References: ChemPlusChem 2013, 78, 222 - 226. In the synthesis of compound **14,** 0.5 g, 0.44 mmol, 1.0 eq) in DMF (5 mL) at 0°C under nitrogen atmosphere, **22101SM2** (0.37 g, 0.44 mmol, 1.0 eq) and DIPEA (0.28 g, 2.20 mmol, 5.0 eq) were added. The mixture was stirred overnight at room temperature under nitrogen atmosphere. The mixture was concentrated and directly used for the next step. MS (ESI), m/z, 1709.2 $[M+H]^+$.

Synthesis of compound **22102B:**

Piperidine (1 mL) was added to a solution of the above crude product **22102A** in DMF (5 mL) and stirred for 1 h. The mixture was concentrated and the residue was purified by prep HPLC to obtain 485 mg of compound **22102B.** MS (ESI), m/z, 1487.6 $[M+H]^+$.

Synthesis of compound **22102:**

Compound **101B13** (500 mg, 0.00 mmol, 1.0 eq), compound **22102B** (467 mg, 0.31 mmol, 46.0 eq), PyBOP (220 mg, 0.42 mmol, 62.0 eq), DIPEA (68 mg, 0.52 mmol, 77.0 eq) and DMF (5 mL) were charged and the reaction was stirred overnight at room temperature under nitrogen atmosphere. HPLC showed complete reaction. MTBE (20 mL) was added and the mixture was stirred for 0.5 h and then allowed to settle. After removal of the upper layer liquid, the residue was dissolved in 5 mL of DCM, cooled in an ice water bath, and 1 mL of trifluoroacetic acid was added. The mixture was stirred for 1 h. HPLC showed complete reaction. The mixture was concentrated, the resultant residue was purified by ultrafiltration, and the filtrate was concentrated to obtain 720 mg of compound **22102** as white solid (Purity: 99.1%).

Example 68: synthesis of compound 22103

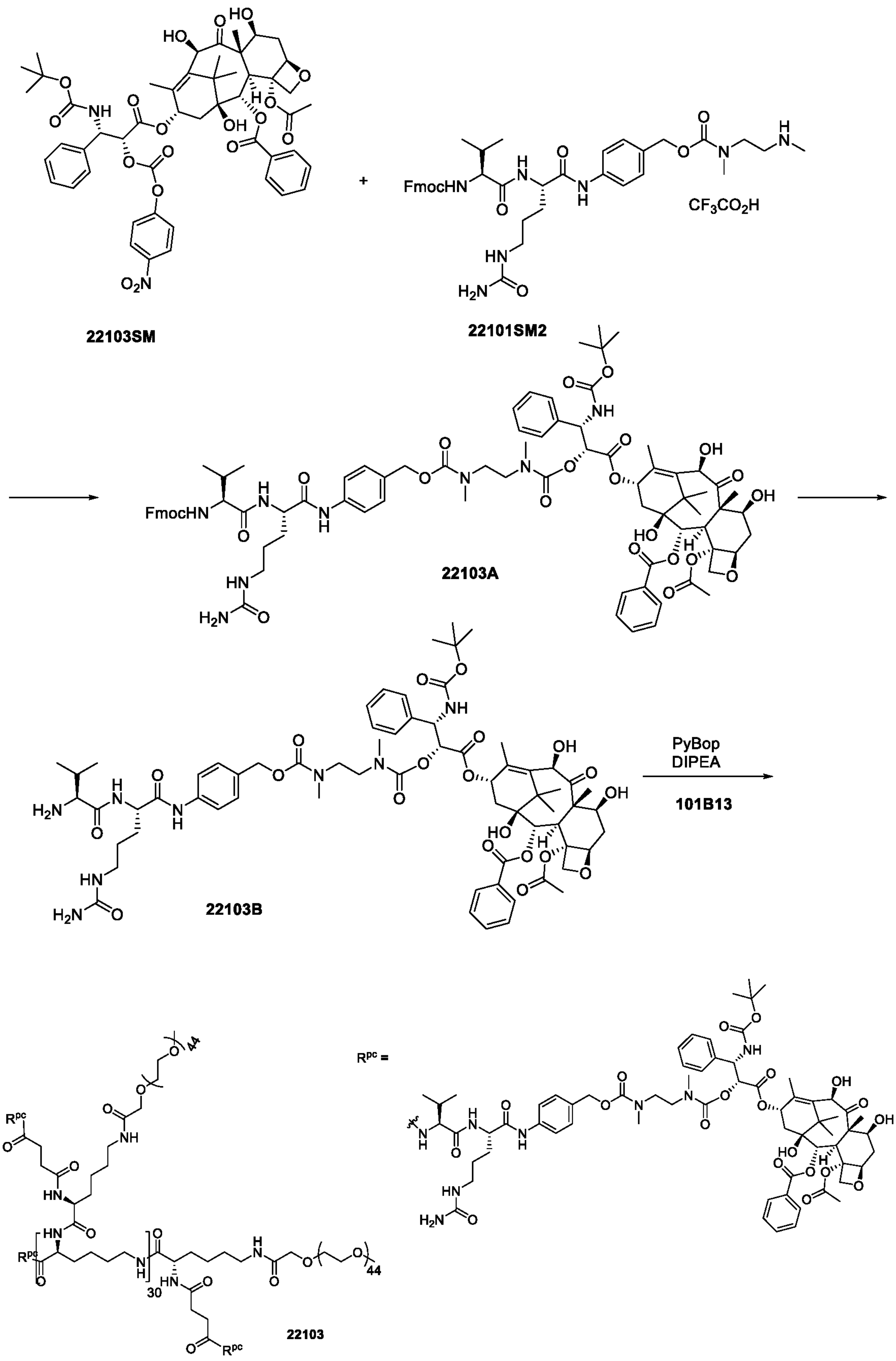


22103SM

22101SM2

22103A

22103B

22103

Synthesis of compound **22103A:**

Compound **22101SM2** (0.43 g, 0.51 mmol, 1.0 eq) and DIPEA (0.33 g, 2.57 mmol, 5.0 eq) were added to a solution of **22103SM** (0.43 g, 0.51 mmol, 1.0 eq, reference patent WO2015038649A1, compound 72) in DMF (5 mL) at 0°C. The mixture was stirred overnight at room temperature under nitrogen atmosphere. The mixture was concentrated and directly used for the next step. MS (ESI), m/z, 1549.2 $[M+H]^+$.

Synthesis of compound **22103B:**

Piperidine (1 mL) was added to a solution of the above crude product **22103A** in DMF (5 mL) and the reaction was stirred for 1 h. The mixture was concentrated and the residue was purified by prep HPLC to obtain 550 mg of compound **22103B.** MS (ESI), m/z, 1327.7 $[M+H]^+$.

Synthesis of compound **22103:**

Compound **101B13** (500 mg, 0.00 mmol, 1.0 eq), compound **22103B** (416 mg, 0.31 mmol, 46.0 eq), PyBOP (220 mg, 0.42 mmol, 62.0 eq), DIPEA (68 mg, 0.52 mmol, 77.0 eq) and DMF (5 mL) were charged and the reaction was stirred overnight at room temperature under nitrogen atmosphere. HPLC showed complete reaction. MTBE (20 mL) was added and the mixture was stirred for 0.5 h. The precipitate was dissolved and purified by ultrafiltration and the filtrate was concentrated to obtain 650 mg of compound 22103 as white solid (Purity: 97.9%).

Example 69: synthesis of compound 22104

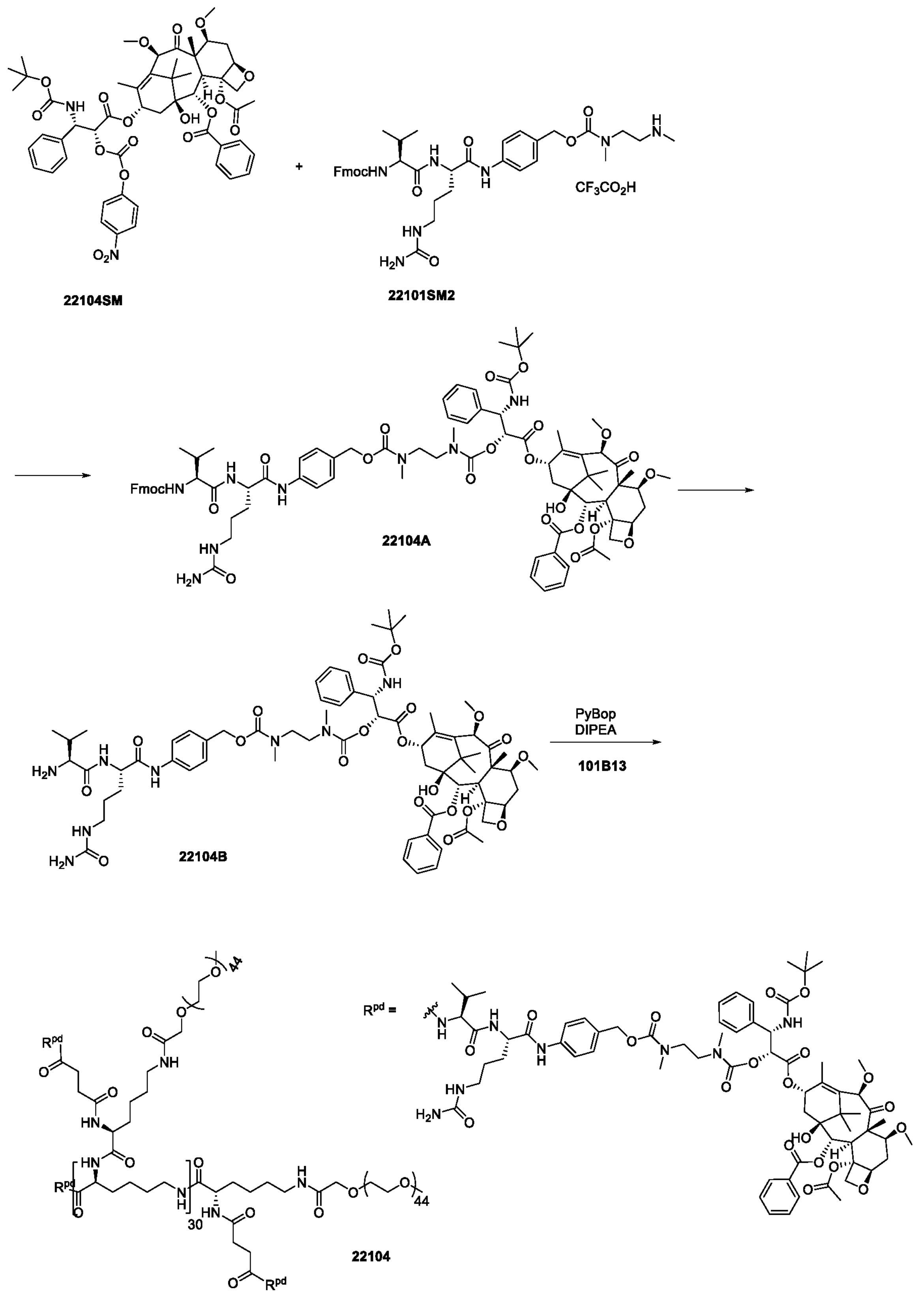


22104SM 22101SM2

22104A

22104B

22104

Synthesis of compound **22104A:**

Compound **22101SM2** (0.42 g, 0.50 mmol, 1.0 eq) and DIPEA (0.32 g, 2.50 mmol, 5.0 eq) were added to a solution of **22104SM** (0.5 g, 0.50 mmol, 1.0 eq, reference to patent WO2017180834A1 compound 20') in DMF (5 mL)

at 0°C. The mixture was stirred overnight at room temperature under nitrogen atmosphere. The mixture was concentrated and directly used for the next step. MS (ESI), m/z, 1577.2 [M+H]$^+$.

Synthesis of compound **22104B:**

Piperidine (1 mL) was added to a solution of the above crude product **22104A** in DMF (5 mL) and the reaction was stirred for 1 h. The mixture was concentrated and the residue was purified by prep HPLC to obtain 570 mg of compound **22104B.** MS (ESI), m/z, 1355.7 [M+H]$^+$.

Synthesis of compound **22104:**

Compound **101B13** (500 mg, 0.00 mmol, 1.0 eq), compound **22104B** (424 mg, 0.31 mmol, 46.0 eq), PyBOP (220 mg, 0.42 mmol, 62.0 eq), DIPEA (68 mg, 0.52 mmol, 77.0 eq) and DMF (5 mL) were charged and the reaction was stirred overnight at room temperature under nitrogen atmosphere. HPLC showed complete reaction. MTBE (20 mL) was added and the mixture was stirred for 0.5 h. The precipitate was dissolved for ultrafiltration and the filtrate was concentrated to obtain 580 mg of compound **22104** as white solid (Purity: 99.0%).

Example 70: synthesis of compound 22105

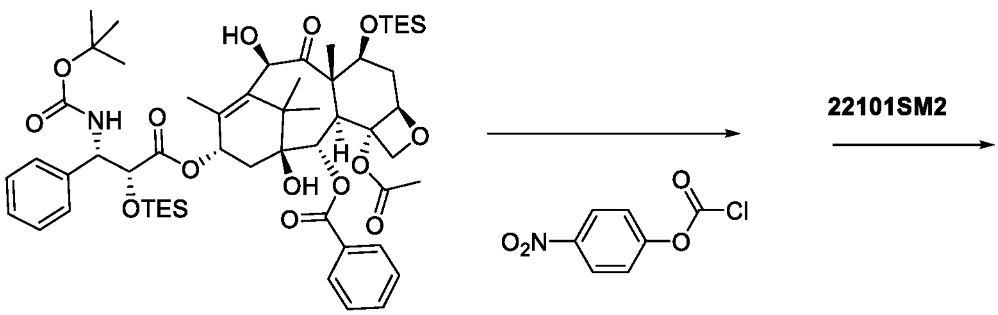


**22105SM**

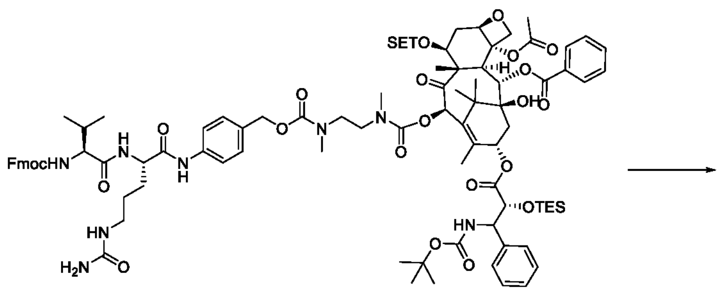

22105A

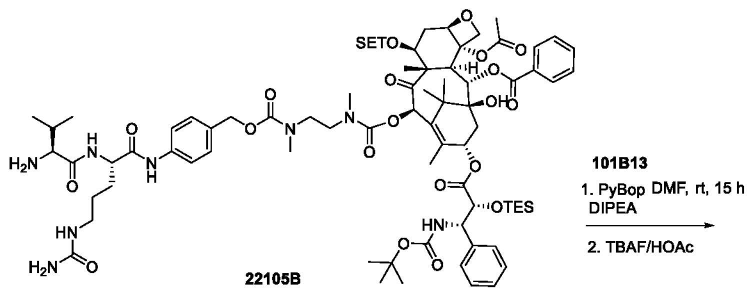


22105B

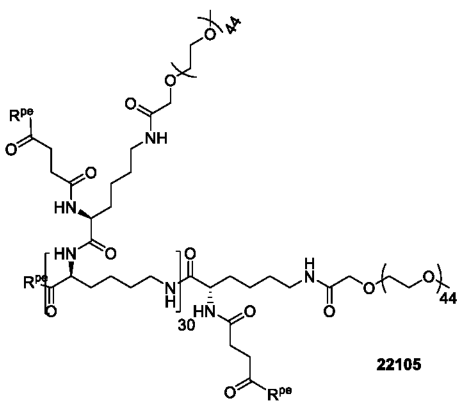

22105

R^pe =

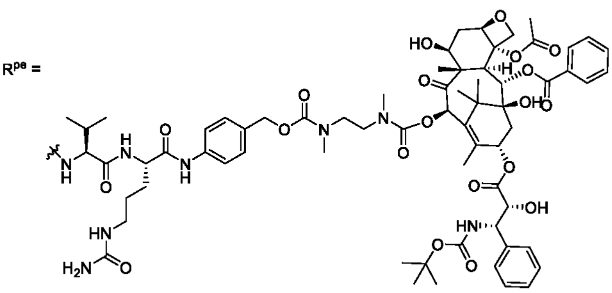

Synthesis of compound **22105A:**

Pyridine (0.5 mL) and p-nitrophenyl chloroformate (0.48 g, 2.41 mmol, 5.0 eq) were added to a solution of **22105SM** (0.5 g, 0.48 mmol, 1.0 eq, references: Molecules 2011, 16, 6769-6777) in anhydrous DCM (10 mL) at -40°C. The mixture was stirred for 1 h under nitrogen atmosphere. The reaction solution was directly concentrated and DMF (5 mL), compound **22101SM2** (0.48 g, 0.58 mmol, 1.2 eq) and DIPEA (0.31 g, 2.41 mmol, 5.0 eq) were added. The mixture was stirred overnight at room temperature. The mixture was concentrated and the resultant residue was purified by column chromatography to obtain 450 mg of compound **22105A.** MS (ESI), m/z, 1781.1 $[M+H]^+$.

Synthesis of compound **22105B:**

Piperidine (1 mL) was added to a solution of **22105A** (450 mg) in DMF (5 mL) and the reaction was stirred for 1 h. The mixture was concentrated and the residue was purified by prep HPLC to obtain 350 mg of compound **22105B.** MS (ESI), m/z, 1559.7 $[M+H]^+$.

Synthesis of compound **22105:**

Compound **101B13** (300 mg, 0.0041 mmol, 1.0 eq), compound **22105B** (293 mg, 0.19 mmol, 46.0 eq), PyBOP (132 mg, 0.25 mmol, 62.0 eq), DIPEA (41 mg, 0.32 mmol, 77.0 eq), and DMF (5 mL) were stirred overnight at room temperature under nitrogen atmosphere. HPLC showed complete reaction. MTBE (20 mL) was added and the mixture was stirred for 0.5 h. The precipitate was dissolved in THF (10 mL), and TBAF (1.0 M, 1 mL) was added to the resultant solution at 0°C. Acetic acid was added to adjust pH to 7. The mixture was stirred for 12 h. The mixture was purified by

ultrafiltration and the filtrate was concentrated to obtain 380 mg of compound **22105** as white solid (Purity: 98.2%).

Biological assessment example A: comparison of enzymatic drug release rates of 101A1601 and 101B00ADOX

Preparation of papain phosphate buffer:

| Name | Concentration | |
|---|---|---|
| PBS, pH=6.5 | 20 | mM |
| EDTA | 1.1 | mM |
| Cys | 5.5 | mM |
| Papain | 0.2 | mg/mL |

Preparation of drug release solution:

| No. | Concentration of polymer | Volumn of polymer solution | Volumn of papain solution | Concentration of DOX·HCl |
|---|---|---|---|---|
| | mg/mL | mL | mL | μg/mL |
| 101A1601* | 25 | 0.200 | 9.800 | 47.7 |
| 101B00A* | 25 | 0.200 | 9.800 | 42.9 |
| *: The average number molecular weight of PEG is 1000. | | | | |

The polymer solution was added to papain buffer and placed in a 37°C water bath with shaking. 0.500 mL of solution was taken over time, transferred to test tubes and diluted with 0.500 mL of DMSO. The concentration of released doxorubicin was determined by HPLC, and the released percentage of doxorubicin was calculated.

Cumulative released doxorubicin (calculated as doxorubicin hydrochloride):

| Time | 101A1601 | 101B00A | 101A1601 | 101B00A |
|---|---|---|---|---|
| h | mAU | mAU | % | % |
| 0.67 | 17.04 | 186.90 | 2.5 | 30.2 |
| 2.0 | 22.45 | 250.80 | 3.3 | 40.5 |
| 4.0 | 27.46 | 285.90 | 4.0 | 46.2 |
| 7.0 | 33.64 | 326.90 | 4.9 | 52.8 |
| 23.5 | 70.59 | 447.79 | 10.2 | 72.4 |
| 31.0 | 82.64 | 474.26 | 12.0 | 76.7 |
| 46.0 | 115.73 | 524.60 | 16.8 | 84.8 |
| 75.0 | 181.30 | 516.90 | 26.3 | 83.5 |

The average release rate of poly(ε-L-lysine) derived drug conjugate 101B00A was 11.6 times of that of dendrimer 101A1601($PEG_{1K}$) in 4 h (Figure 1), indicating that the poly(ε-L-lysine) derived drug conjugate has lower steric hindrance and faster drug release rate.

Biological assessment example B: comparison of enzymatic drug release rates of 101A1602 and 101B00B DOX

Preparation of papain phosphate buffer:

| Name | Concentration | |
|---|---|---|
| PBS, pH=6.5 | 20 | mM |
| EDTA | 1.1 | mM |
| Cys | 5.5 | mM |
| Papain | 0.2 | mg/mL |

Preparation of drug release solution:

| No. | Concentration of polymer | Volumn of polymer solution | Volumn of papain solution | Concentration of DOX·HCl |
|---|---|---|---|---|
| | mg/mL | mL | mL | μg/mL |
| 101A1602* | 25 | 0.2 | 9.8 | 35.3 |
| 101B00B* | 25 | 0.2 | 9.8 | 29.7 |
| *: The average number molecular weight of PEG is 2000. | | | | |

The polymer solution was added to papain buffer and placed in a 37°C water bath with shaking. 0.500 mL of solution was taken over time, transferred to test tubes and diluted with 0.500 mL of DMSO. The concentration of released doxorubicin was determined by HPLC, and the released percentage of doxorubicin was calculated.

Cumulative released doxorubicin (calculated as doxorubicin hydrochloride):

| Time | 101A1602 | 101B00B | 101A1602 | 101B00B |
|---|---|---|---|---|
| h | mAU | mAU | % | % |
| 0.67 | 16.58 | 21.42 | 3.3 | 5.0 |
| 2.0 | 20.63 | 26.80 | 4.0 | 6.3 |
| 4.0 | 24.47 | 31.19 | 4.8 | 7.3 |
| 7.0 | 30.04 | 37.42 | 5.9 | 8.7 |
| 23.5 | 54.51 | 68.06 | 10.7 | 15.9 |
| 31.0 | 64.36 | 83.56 | 12.6 | 19.5 |
| 46.0 | 79.63 | 102.93 | 15.6 | 24.0 |
| 75.0 | 119.80 | 144.30 | 23.5 | 33.7 |

The average release rate of poly(ε-L-lysine) derived drug conjugate 101B00B was 1.5 times of that of dendrimer 101A1602($PEG_{2K}$) in 4 h (Figure 2), indicating that the poly(ε-L-lysine) derived drug conjugate still has lower steric hindrance, faster drug release rate and better efficacy even with $PEG_{2k}$.

Biological assessment example C: comparison of enzymatic drug release rates of 101A1603 and 101B00E SN-38

Preparation of papain phosphate buffer:

| Name | Concentration | |
|---|---|---|
| PBS, pH=6.5 | 20 | mM |
| EDTA | 1.1 | mM |
| Cys | 5.5 | mM |
| Papain | 0.2 | mg/mL |

Preparation of drug release solution:

| No. | Concentration of polymer | Volumn of polymer solution | Volumn of papain solution | Concentration of drug |
|---|---|---|---|---|
| | mg/mL | mL | mL | μg/mL |
| 101A1603 | 25 | 0.200 | 9.800 | 53.4 |
| 101B00E | 25 | 0.200 | 9.800 | 53.2 |
| *: The average number molecular weight of PEG is 2000. | | | | |

The polymer solution was added to papain buffer and placed in a 37°C water bath with shaking. 0.500 mL of solution was taken over time, transferred to test tubes and diluted with 0.500 mL of DMSO. The concentration of released SN-38 was determined by HPLC, and the released percentage of SN-38 was calculated.

Cumulative released SN-38:

| Time | 101A1603 | 101B00E | 101A1603 | 101B00E |
|---|---|---|---|---|
| h | mAU | mAU | % | % |
| 0.67 | 6.0 | 7.6 | 1.1 | 1.4 |
| 2.0 | 9.0 | 15.2 | 1.6 | 2.7 |
| 4.0 | 11.1 | 25.2 | 2.0 | 4.5 |
| 7.0 | 14.2 | 41.3 | 2.5 | 7.4 |
| 23.5 | 25.9 | 126.7 | 4.6 | 22.8 |
| 31.0 | 31.3 | 159.8 | 5.6 | 28.7 |
| 46.0 | 39.1 | 219.0 | 7.0 | 39.4 |
| 75.0 | 55.3 | 311.5 | 9.9 | 56.0 |

The average release rate of poly(ε-L-lysine) derived drug conjugate 101B00E was 5.6 times of that of dendrimer 101A1603 in 75 h (Figure 3). Likewise, this indicated that the poly(ε-L-lysine) derived drug conjugate has lower steric hindrance, which was conducive to release of active ingredients and treatment of diseases.

Biological assessment example D: comparison of enzymatic drug release rates of 101C00A and 101D00A SN-38

Preparation of papain phosphate buffer:

| Name | Concentration | |
|---|---|---|
| PBS, pH=6.5 | 20 | mM |
| EDTA | 1.1 | mM |
| Cys | 5.5 | mM |
| Papain | 0.2 | mg/mL |

Preparation of drug release solution:

| No. | Concentration of polymer | Volumn of polymer solution | Volumn of papain solution | Concentration of drug |
|---|---|---|---|---|
| | mg/mL | mL | mL | μg/mL |
| 101C00A* | 25 | 0.200 | 9.800 | 53.0 |
| 101D00A* | 25 | 0.200 | 9.800 | 52.6 |
| *: The average number molecular weight of PEG is 2000. | | | | |

The polymer solution was added to papain buffer and placed in a 37°C water bath with shaking. 0.500 mL of solution was taken over time, transferred to test tubes and diluted with 0.500 mL of DMSO. The concentration of released SN-38 was determined by HPLC, and the released percentage of SN-38 was calculated.

Cumulative released SN-38:

| Time | 101D00A | 101C00A | 101D00A | 101C00A |
|---|---|---|---|---|
| h | mAU | mAU | % | % |
| 0.67 | 12.8 | 5.9 | 2.3 | 1.1 |
| 2.0 | 18.8 | 11.4 | 3.4 | 2.1 |
| 4.0 | 22.9 | 18.3 | 4.2 | 3.3 |
| 7.0 | 27.7 | 27.5 | 5.0 | 5.0 |
| 23.5 | 39.7 | 67.3 | 7.2 | 12.2 |
| 31.0 | 43.2 | 78.3 | 7.9 | 14.1 |
| 46.0 | 48.1 | 99.7 | 8.7 | 18.0 |
| 75.0 | 58.5 | 136.5 | 10.6 | 24.7 |

The average release rate of poly(ε-L-lysine) derived drug conjugate 101C00A was 2.33 times of that of dendrimer 101D00A in 75 h (Figure 4).

Biological assessment example E: comparison of drug distribution in plasma/tumor after intravenous injection of irinotecan and 101B00E (10 mg/kg, calculated as SN-38) in HepG2 xenograft CDX mouse model

4-5 week-old, 20-25 g BALB/C nude mice were fed in the experimental environment for one week after arrival. HepG2 tumors were subcutaneously inoculated on the right back of nude mice. When the tumor volume reached 200-400 $mm^3$, dosing in the group was started, on three tumor-bearing nude mice per group/time point. The tumor volume is calculated as: $V = 0.5a \times b^2$, a and b represent the length and width of the tumor, respectively.

Both irinotecan and 101B00E were administered (10 mg/kg, calculated as SN-38) via tail vein. Mice were killed

at set time points. Plasma was taken after blood centrifugation. Tumor tissues were isolated, temporarily stored in dry ice and then transferred to -80°C freezer for later use.

Tumor tissues were thawed, weighed and then added a certain amount of 50/50 methanol and water. The mixture was homogenized on the handheld homogenizer with ice bath, centrifuged at 4°C 6000×g for 10 min, and the supernatant was collected.

30 μL of plasma or homogenized tumor supernatant were taken and added 200 μL of acetonitrile with internal standard (tolbutamide, 50 ng/mL). The mixture was vortexed for 5 min and centrifuged at 6000 ×g for 10 min. 75 μL of the supernatant was transferred to a 96-well plate pre-added with 75 μL of water and shaked at 500 rpm for 5 min. 2 μL of sample was injected for LC MS/MS analysis.

Column: ACQUITYUPLC BEH C18 (2.1×50 mm, 1.7 μm)
Phase A: 0.1% formic acid/water, Phase B: 0.1% formic acid/acetonitrile
Gradient: 0.3 min 90% Phase A, 1.5-2.3 min 10% Phase A, 2.30-2.31 min 10-90% Phase A, 2.31-2.8 min 90% Phase A.

Among them, the 101B00E group used 10112 as standard. The 10112 released in plasma and tumor was detected and converted into SN-38. The irinotecan group detected the released SN-38.

Results of irinotecan:

| Plasma | | | | | Tumor | | | | |
|---|---|---|---|---|---|---|---|---|---|
| **Time (h)** | **Average value** | | **SD** | **CV (%)** | **Time (h)** | **Average value** | | **SD** | **CV (%)** |
| 0.083 | 865 | ± | 168.8 | 19.5 | 0.083 | 261.8 | ± | 44.8 | 17.1 |
| 0.50 | 385 | ± | 14.7 | 3.8 | 0.50 | 100.7 | ± | 37.3 | 37.0 |
| 1.00 | 407 | ± | 118.1 | 29.05 | 1.00 | 88 | ± | 4.9 | 5.6 |
| 3.00 | 172.6 | ± | 44.62 | 25.86 | 3.00 | 131 | ± | 26.7 | 20.4 |
| 8.00 | 27.7 | ± | 13.77 | 49.7 | 8.00 | 109 | ± | 23.8 | 21.8 |
| 24.0 | 31.9 | ± | 33.44 | 104.7 | 24.0 | 39.2 | ± | 13.12 | 33.4 |
| 48.0 | - | - | - | - | 48.0 | - | - | - | - |
| 72.0 | - | - | - | - | 72.0 | - | - | - | - |
| **PK paramet -ers** | **Average value** | | **SD** | **CV (%)** | **PK paramet** -ers | **Average value** | | **SD** | **CV (%)** |
| T1/2 h | 5.7 | ± | 2.0 | 34.6 | T1/2 h | 13 | ± | 5 | 39.96 |
| Tmax h | 0.08 | ± | -- | -- | Tmax h | ND | ± | -- | -- |
| Cmax ng/mL | 865 | ± | 169 | 19.5 | Cmax ng/mL | 416 | ± | 242 | 58.31 |
| AUC(0-t) ng/mL*h | 2094 | ± | 391 | 18.68 | AUC(0-t) ng/mL*h | 2404 | ± | 339 | 14.1 |
| AUC(0-∞) ng/mL*h | 2412 | ± | 750 | 31.09 | AUC(0-∞) ng/mL*h | 3195 | ± | 204 | 6.4 |
| Vz mL/kg | 35910 | ± | 8842 | 24.62 | Vz mL/kg | 56903 | ± | 19290 | 33.9 |
| CL mL/hr/kg | 4571 | ± | 990 | 21.65 | CL mL/hr/kg | 3123 | ± | 193 | 6.2 |
| MRT(0-t) h | 5.0 | ± | 2.1 | 42.72 | MRT(0-t) h | 7.9 | ± | 2.0 | 25.4 |

The tumor/plasma concentration-time curve of SN-38 produced by irinotecan metabolism is shown in Figure 5.

Results of 101B00E:

| Plasma | | | | | Tumor | | | | |
|---|---|---|---|---|---|---|---|---|---|
| **Time (h)** | **Average value** | | **SD** | **CV (%)** | **Time (h)** | **Average value** | | **SD** | **CV (%)** |
| 0.083 | 32 | ± | 6.2 | 19.2 | 0.083 | 0.7 | ± | 1.3 | 173.2 |
| 0.50 | 30 | ± | 15.2 | 51.4 | 0.50 | 1.7 | ± | 1.5 | 89.9 |
| 1.00 | 17 | ± | 7.2 | 42.02 | 1.00 | 41.6 | ± | 21.3 | 51.2 |
| 3.00 | 17.4 | ± | 4.69 | 26.89 | 3.00 | 28.5 | ± | 3.3 | 11.4 |
| 8.00 | 20.1 | ± | 3.31 | 16.51 | 8.00 | 61.1 | ± | 10.6 | 17.4 |
| 24.0 | 17.8 | ± | 5.73 | 32.24 | 24.00 | 347.5 | ± | 54.5 | 15.7 |
| 48.0 | 9.3 | ± | 2.50 | 27.0 | 48.00 | 110.3 | ± | 16.8 | 15.2 |
| 72.0 | 14.7 | ± | 17.68 | 120.2 | 72.0 | 53.3 | ± | 12.3 | 23.1 |
| **PK parameters** | **Average value** | | **SD** | **CV (%)** | **PK parameters** | **Average value** | | **SD** | **CV (%)** |
| T1/2 h | 28.3 | ± | 4.7 | 16.7 | T1/2 h | 18 | ± | 4 | 21.86 |
| Tmax h | ND | ± | -- | -- | Tmax h | 24.0 | ± | -- | -- |
| Cmax ng/mL | 37 | ± | 8 | 22.2 | Cmax ng/mL | 347 | ± | 55 | 15.69 |
| AUC(0-t) ng/mL*h | 1070 | ± | 383 | 35.76 | AUC(0-t) ng/mL*h | 11029 | ± | 1467 | 13.3 |
| AUC(0-∞) ng/mL*h | 1046 | ± | 227 | 21.72 | AUC(0-∞) ng/mL*h | 12473 | ± | 942 | 7.6 |
| Vz mL/kg | 393484 | ± | 17066 | 4.34 | Vz mL/kg | 21530 | ± | 6173 | 28.7 |
| CL mL/hr/kg | 9799 | ± | 2053 | 20.95 | CL mL/hr/kg | 813 | ± | 66 | 8.1 |
| MRT(0-t) h | 29.8 | ± | 7.6 | 25.49 | MRT(0-t) h | 31.4 | ± | 0.9 | 2.8 |

The tumor/plasma concentration-time curve of 10112 (piperazine-10-O-SN-38) produced by 101B00E metabolism is shown in Figure 6.

Tumor/plasma ratio of 10112 released by 101B00E and SN-38 released by irinotecan:

| | 101B00E | | Irinotecan | |
|---|---|---|---|---|
| Time (h) | Tumor/plasma | SD | Tumor/plasma | SD |
| 0.083 | 0.02 | 0.04 | 0.31 | 0.08 |
| 0.50 | 0.08 | 0.08 | 0.26 | 0.08 |
| 1.00 | 2.36 | 0.34 | 0.16 | 0.12 |
| 3.00 | 1.77 | 0.61 | 0.80 | 0.22 |
| 8.00 | 3.07 | 0.35 | 4.38 | 1.25 |
| 24.00 | 21.25 | 6.53 | 3.15 | 2.73 |
| 48.00 | 12.63 | 3.42 | - | - |
| 72.0 | 7.86 | 4.75 | - | - |

As shown in the table above, the tumor/plasma ratio of piperazine-10-O-SN38 (10112) released from poly(ε-L-Lysine) derivative-drug conjugate 101B00E reached its maximum value of 21.2±6.5 at 24 h (Figure 7), while the tumor/plasma ratio of SN-38 released by irinotecan reached its maximum value of 4.4±1.2 at 8 h. Compared with the conventional chemotherapy drug irinotecan, poly(ε-L-Lysine) derivative-drug conjugate 101B00E has more tissue-specific distribution and can be used for targeted delivery of chemotherapy drugs.

Biological assessment example F: comparison of in vivo efficacy 1

4-5 week-old, 20-25 g BALB/C nude mice were fed in the experimental environment for one week after arrival. HepG2 tumors were subcutaneously inoculated on the right back of nude mice. When the tumor volume reached 160 $mm^3$, dosing in the group was started, on 6-7 mice per group. Body weight and tumor size were measured twice a week to see if tumor growth was suppressed, delayed, or cured. Tumor diameter was measured with vernier calipers twice a week. The tumor volume was calculated as: $V = 0.5a \times b^2$, a and b represent the length and width of the tumor, respectively.

The tumor growth inhibition effect of the compound was evaluated by tumor growth inhibition T/C ratio (T/C, %). The evaluation criteria are as follows: T/C (%) >40% for "ineffective"; T/C(%) ≤40% with $P < 0.05$ for "effective".

Tumor growth inhibition T/C ratio: $T/C\% = T_{RTV}/C_{RTV} \times 100\%$ ($T_{RTV}$: mean RTV in the treatment group; $C_{RTV}$: mean RTV in the negative control group). According to the results of tumor measurement, the relative tumor volume (RTV) is calculated, and the calculation formula is $RTV = Vt / V_0$, where $V_0$ is the tumor volume measured when the group is administered (i.e. $d_0$); Vt is the tumor volume measured at a certain time, and $T_{RTV}$ and $C_{RTV}$ data are from the same day.

$$TGI\ (\%) = 100\%\text{-}T/C\ (\%)$$

(1) Comparison of in vivo efficacy between poly(ε-L-Lysine) derivative-drug conjugate 101B00A and dendrimer-drug conjugate 101A1601:
As shown in Figure 8, in HepG2 xenograft CDX mouse model, sterically less hindered 101B00A showed significantly better inhibition effect on tumors than 101A1601 and doxorubicin hydrochloride, and there was no statistical difference between 101A1601 and doxorubicin hydrochloride. These results showed that the poly(ε-L-Lysine) derivative-drug conjugate 101B00A, which had faster enzymatic release rate in vitro, had better tumor suppression effect in vivo than dendritic polylysine 101A1601.
(2) Comparison of in vivo efficacy between poly(ε-L-Lysine) derivative-drug conjugates 101B00C and 101B00E:
As shown in Figure 9, in HepG2 xenograft CDX mouse model, on Day 33, the TGI (%) of 101B00C and 101B00E were 93% and 92%, respectively, while the TGI (%) of irinotecan in the positive drug group was -49%. These results indicated that the poly(ε-L-Lysine) derivative-drug conjugate 101B00C and 101B00E had excellent tumor inhibition ability compared with the positive drug irinotecan.
(3) Comparison of in vivo efficacy between poly(ε-L-Lysine) derivative-drug conjugates 101C00A and 101D00A:
As shown in Figure 10, in HepG2 xenograft CDX mouse model, on Day 33, the TGI (%) of 101C00A and 101D00A were 90% and 91%, respectively, while the TGI (%) of irinotecan in the positive drug group was -49%. These results indicated that the poly(ε-L-Lysine) derivative-drug conjugate 101C00A and 101D00A had excellent tumor inhibition ability compared with the positive drug irinotecan.

Biological assessment example G: comparison of efficacy of 101B00G and nanoparticle albumin paclitaxel on pancreatic cancer xenograft model 2

4-5 week-old, 20-25 g BALB/C nude mice were fed in the experimental environment for one week after arrival. BxPC-3 tumors were subcutaneously inoculated on the right back of nude mice. When the tumor volume reached 157 $mm^3$, dosing in the group was started, on 6 mice per group. Body weight and tumor size were measured twice a week to see if tumor growth was suppressed, delayed, or cured. Tumor diameter was measured with vernier calipers twice a week. The tumor volume was calculated as: $V = 0.5a \times b^2$, a and b represent the length and width of the tumor, respectively.

The tumor growth inhibition effect of the compound is evaluated by tumor growth inhibition T/C ratio (T/C, %). The evaluation criteria are as follows: T/C (%) >40% for "ineffective"; T/C (%) ≤40% with $P < 0.05$ for "effective".

Tumor growth inhibition T/C ratio: $T/C\% = T_{RTV}/C_{RTV} \times 100\%$ ($T_{RTV}$: mean RTV in the treatment group; $C_{RTV}$: mean RTV in the negative control group). According to the results of tumor measurement, the relative tumor volume (RTV) is calculated, and the calculation formula is $RTV = Vt / V_0$, where $V_0$ is the tumor volume measured when the group is administered (i.e., $d_0$); Vt is the tumor volume measured at a certain time, and $T_{RTV}$ and $C_{RTV}$ data are from the same day.

$$TGI\ (\%) = 100\%\text{-}T/C\ (\%)$$

Comparison of poly(ε-L-Lysine) derivative-SN-38 conjugate 101B00G and nanoparticle albumin paclitaxel:

As shown in Figure 11, 21 days after administration, the tumor volume of mice in the control group reached 959±113 $mm^3$. There were significant differences between the nab-paclitaxel group and the control group (T/C = 30%, *p* = 0.0006). The poly(ε-L-Lysine) derivative-SN-38 conjugate 101B00G (20 mg/kg) group was significantly different from the control group (T/C = 5%, *p* = 0.00006); 101B00G (10 mg/kg) group was significantly different from the control group (T/C = 9%, *p* = 0.0001); 101B00G (5 mg/kg) group was significantly different from the control group (T/C = 27%, *p* = 0.0016), indicating that the efficacy of nab-paclitaxel 60 mg/kg (MTD) could be achieved with 5 mg/kg of 101B00G. When the amount of 101B00G was increased to 20 mg/kg, the inhibitory efficacy of BxPC-3 pancreatic cancer was further enhanced. As shown in Figure 12, even 20 mg/kg of 101B00G has less influence on mouse body weight than 60 mg/kg of nab-paclitaxel, indicating that animals have good tolerance of 101B00G. 101B00G has significantly better efficacy on BxPC-3 pancreatic cancer than nab-paclitaxel, and has great potential for clinical application.

**Biological assessment example H: pharmacokinetics of 101B00G in rats**

Male SD rats (body weight: 220-250 g) were fed in the experimental environment for one week after arrival. Blood was collected alternately for 6 rats of a single sex at a single dose (before administration, 0.25 h, 1 h, 4 h, 24 h were the blood collection time points of 3 rats; 0.083 h, 0.5 h, 2 h, 8 h, 48 h, 72 h were the blood collection time points of the other 3 rats).

101B00G was dissolved in 5% glucose solution and administered via tail vein with the dose of 15 mg/kg (calculated as SN-38). 101B00G was administered once a week, for a total of four times. About 0.2 mL of whole blood was drawn from the jugular vein at set time points before and after the first and fourth doses in an EDTA-2K anticoagulant tube, centrifuged at 6000 ×g for 10 min. Then the upper plasma was taken to determine the concentration of released SN-38 and the concentration of SN-38 coupled to the polymer.

Determination of concentration of released SN-38:

30 μL of plasma was taken and added 200 μL of acetonitrile/methanol mixture with internal standard (10-hydroxycamptothecin, 50 ng/mL). The mixture was vortexed for 5 min and centrifuged at 6000 ×g for 10 min. 75 μL of the supernatant was transferred to a 96-well plate pre-added with 75 μL of water and shaked at 500 rpm for 5 min. 2 μL of sample was injected for LC MS/MS analysis of concentration of released SN-38.

Determination of concentration of total SN-38:

40 μL of plasma was taken and added 10 μL of 6 M HCl. The vial was closed and the solution was mixed well by vortexing at 1000 rpm for 30 s, and then hydrolyzed in a water bath at 80°C for 4 h. After 4 h, the mixture was cooled down to room temperature, added 10 μL of 6.5 M NaOH, vortexed at 1000 rpm for 30 s, and reacted at room temperature for 15 min. 480 μL of methanol/acetonitrile (1:1) was added. The mixture was vortexed at 1000 rpm for 5 min, and centrifuged at 4°C, 4700 g for 10 min. 130 μL of supernatant was transferred to the 96-well plate according to the plate map, added 2 μL of 50% trifluoroacetic acid in water, and mixed well. The plate was wrapped tightly with aluminum foil and transferred to an autosampler to determine the SN-38 concentration.

The concentration of released SN-38 and total SN-38 in plasma after the first administration are shown in Figure 13. The concentration of released SN-38 and total SN-38 in plasma after the fourth administration are shown in Figure 14. The calculation results of the ratio of released SN-38 concentration to total SN-38 concentration in plasma over time are shown in the following two tables:

| **SN-38 release rate (‰) Day 1** | | | | | | | |
|---|---|---|---|---|---|---|---|
| **G3: 101B00G (15 mg/kg, calculated as SN-38, QW*4) ♂** | | | | | | | |
| **Intravenous injection** | | | | | | | |
| **IV time (h)** | **301** | **302** | **303** | **Average** | **SD** | | **CV (%)** |
| 0.0833 | 1.460 | 0.918 | 1.152 | 1.18 | ± | 0.272 | 23.1 |
| 0.250 | 1.546 | 1.414 | 0.774 | 1.24 | ± | 0.413 | 33.2 |

(continued)

| SN-38 release rate (‰) Day 1 | | | | | | | |
|---|---|---|---|---|---|---|---|
| G3: 101B00G (15 mg/kg, calculated as SN-38, QW*4) ♂ | | | | | | | |
| Intravenous injection | | | | | | | |
| **IV time (h)** | **301** | **302** | **303** | **Average** | **SD** | | **CV (%)** |
| 0.500 | 0.995 | 1.199 | 0.840 | 1.01 | ± | 0.180 | 17.8 |
| 1.00 | 0.689 | 0.740 | 0.468 | 0.632 | ± | 0.145 | 22.9 |
| 2.00 | 0.262 | 0.269 | 0.260 | 0.264 | ± | 0.00458 | 1.74 |
| 4.00 | 0.144 | 0.158 | 0.170 | 0.157 | ± | 0.0129 | 8.23 |
| 8.00 | 0.132 | 0.128 | 0.151 | 0.137 | ± | 0.0126 | 9.19 |
| 24.0 | 0.165 | 0.189 | 0.169 | 0.174 | ± | 0.0131 | 7.51 |
| 48.0 | 0.207 | 0.749 | 0.208 | 0.388 | ± | 0.312 | 80.5 |
| 72.0 | 0.175 | 0.122 | 0.188 | 0.162 | ± | 0.0348 | 21.5 |

| SN-38 release rate (‰) Day 22 | | | | | | | |
|---|---|---|---|---|---|---|---|
| G3: 101B00G (15 mg/kg, calculated as SN-38, QW*4) ♂ | | | | | | | |
| Intravenous injection | | | | | | | |
| **IV time (h)** | **301** | **302** | **303** | **Average** | | **SD** | **CV (%)** |
| 0 | 0.153 | 0.112 | 0.136 | 0.134 | ± | 0.0205 | **15.3** |
| 0.0833 | 1.823 | 1.660 | 1.340 | 1.608 | ± | 0.246 | **15.3** |
| 0.250 | 0.244 | 1.088 | 1.221 | 0.851 | ± | 0.530 | **62.3** |
| 0.500 | 0.935 | 0.983 | 0.906 | 0.941 | ± | 0.0388 | **4.12** |
| 1.00 | 0.645 | 0.661 | 0.628 | 0.645 | ± | 0.0164 | **2.55** |
| 2.00 | 0.268 | 0.388 | 0.290 | 0.315 | ± | 0.0641 | **20.3** |
| 4.00 | 0.134 | 0.148 | 0.123 | 0.135 | ± | 0.0128 | **9.47** |
| 8.00 | 0.144 | 0.122 | 0.125 | 0.131 | ± | 0.0121 | **9.27** |
| 24.0 | 0.169 | 0.157 | 0.142 | 0.156 | ± | 0.0137 | **8.74** |
| 48.0 | 0.142 | 0.153 | 0.151 | 0.149 | ± | 0.00609 | **4.09** |
| 72.0 | 0.134 | 0.134 | 0.139 | 0.136 | ± | 0.00306 | **2.26** |

As shown in the above two tables, only within 0.083 h~2 h at the beginning of administration, the ratio of released SN-38 to total SN-38 is high (0.16-0.03%). 4 h~168 h after administration (0 h on day 22 is equivalent to 168 h after the third dose), the released SN-38 accounts for 0.012-0.015% of the total SN-38, indicating that 101B00G is extremely stable in rat plasma and will not release SN-38 non-specifically in plasma. From Figures 13 and 14, it can be concluded that SN-38 conjugated to polymers has a long half-life, indicating that 101B00G has a long circulation time in plasma.

**Biological assessment example I: comparison of efficacy of 101B00G and topotecan on subcutaneous transplanted 1200 mm$^3$ H69 small cell lung cancer model**

4-5 week-old, 20-25 g BALB/C nude mice were fed in the experimental environment for one week after arrival. H69 small cell lung cancer blocks were subcutaneously inoculated on the right back of nude mice. When the tumor volume reached -1200 mm$^3$, dosing in the group was started. Body weight and tumor size were measured twice a week to see if tumor growth was suppressed, delayed, or cured. Tumor diameter was measured with vernier calipers twice a

week. The tumor volume was calculated as: $V = 0.5a \times b^2$, a and b represent the length and width of the tumor, respectively.

As shown in Figure 15, topotecan administration with the MTD dose of 2 mg/kg (calculated as free base) once a day for five days, according to literatures, showed a poor inhibition effect on 1200 $mm^3$ small cell lung cancer. The tumor volume on Day 22 was increased by 22% compared with the initial value. However, the tumor volume of 101B00G group continued to decrease after a single dose, and the tumor volume on Day 22 decreased by 81% compared with the initial value. These results show that 101B00G is significantly better than the existing second-line clinical drugs for small cell lung cancer, and has great potential for clinical application.

**Biological assessment example J: efficacy of 101B00G on 600 $mm^3$ large subcutaneous transplanted HepG2 liver cancer model**

4-5 week-old, 20-25 g BALB/C nude mice were fed in the experimental environment for one week after arrival. HepG2 tumors were subcutaneously inoculated on the right back of nude mice. When the tumor volume reached ~600 $mm^3$, dosing in the group was started. Body weight and tumor size were measured twice a week to see if tumor growth was suppressed, delayed, or cured. Tumor diameter was measured with vernier calipers twice a week. The tumor volume was calculated as: $V = 0.5a \times b^2$, a and b represent the length and width of the tumor, respectively.

As shown in Figure 16, even for 600 $mm^3$ large subcutaneous HepG2 liver cancer model, the tumor volume could continue to decrease after 101B00G administration, indicating that 101B00G has great potential for clinical application.

**Biological assessment example K: comparison of efficacy of 101T00A-O and nab-paclitaxel on pancreatic cancer xenograft model**

4-5 week-old, 20-25 g BALB/C nude mice were fed in the experimental environment for one week after arrival. BxPC-3 tumors were subcutaneously inoculated on the right back of nude mice. When the tumor volume reached 157 $mm^3$, dosing in the group was started. Body weight and tumor size were measured twice a week to see if tumor growth was suppressed, delayed, or cured. Tumor diameter was measured with vernier calipers twice a week. The tumor volume was calculated as: $V = 0.5a \times b^2$, a and b represent the length and width of the tumor, respectively.

The tumor growth inhibition effect of the compound was evaluated by tumor growth inhibition T/C ratio (T/C, %). The evaluation criteria are as follows: T/C (%) >40% for "ineffective"; T/C (%) $\leq$40% with $P < 0.05$ for "effective".

Tumor growth inhibition T/C ratio (T/C, %) T/C (%): $T/C\% = T_{RTV}/C_{RTV} \times 100\%$ ($T_{RTV}$: mean RTV in the treatment group; $C_{RTV}$: mean RTV in the negative control group). According to the results of tumor measurement, the relative tumor volume (RTV) is calculated, and the calculation formula is $RTV = Vt / V_0$, where $V_0$ is the tumor volume measured when the group is administered (i.e., $d_0$); Vt is the tumor volume measured at a certain time, and $T_{RTV}$ and $C_{RTV}$ data are from the same day.

$$TGI\ (\%) = 100\%\text{-}T/C\ (\%)$$

As shown in Figure 17, 21 days after administration, the tumor volume of mice in the control group reached $959 \pm 113$ $mm^3$. There were significant differences between the nab-paclitaxel group (60 mg/kg) and the control group (T/C = 30%, $p = 0.0007$). The 101T00A-O group was significantly different from the control group (T/C = 7%, $p = 0.00006$). On Day 21, the efficacy of 101T00A-O was significantly better than nab-paclitaxel group (60 mg/kg), and has great potential for clinical application.

Although the above describes specific embodiments of the present invention, those skilled in the art should understand that these are only illustrative examples. Without deviating from the principle and essence of the present invention, these embodiments may be modified in different ways. Therefore, the scope of protection of the present invention is as defined by the claims.

**Claims**

1. A poly(ε-L-lysine) derivative-drug conjugate which has a controllable number of conjugates and a controllable position of conjugation, as shown in formula I:

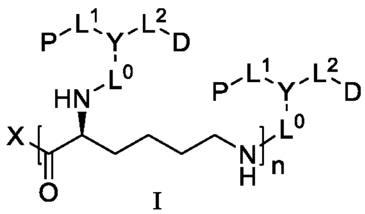

I

the poly(ε-L-lysine) derivative-drug conjugate comprises:

(1) poly(ε-L-lysine) residue, as shown in formula II, the number of repeating units of the poly(ε-L-lysine) being n,

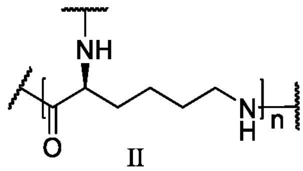

II

wherein the poly(ε-L-lysine) is obtained from biosynthesis or chemical synthesis;
n is an integer of 4 to 100;

(2) Y, which is a branched center with at least three functionalities;
(3) P, which is a pharmacokinetic regulator residue;
(4) D, which is a pharmaceutically active agent residue;
(5) X, which is a terminal group;
(6) $L^0$, $L^1$ and $L^2$, which are each independently covalent bonds or $C_1$-$C_{40}$ linkers with or without heteroatoms, wherein the heteroatoms are O, S, Se, N, P, Si or B, and the number of the heteroatoms is one or more; when the number is more than one, the heteroatoms could be the same or different; the linkers contain unsaturated groups or contain no unsaturated groups; the $L^0$ links poly(ε-L-lysine) residue and Y, the $L^1$ links P and Y, and the $L^2$ links D and Y

**2.** The poly(ε-L-lysine) derivative-drug conjugate according to claim 1, wherein the poly(ε-L-lysine) derivative-drug conjugate fulfills one or more of the following:

(1) the number n of the poly(ε-L-lysine) repeating units is an integer of 10 to 70, preferably an integer of 20 to 40, such as 30;
(2) Y is a branched center comprising the following structures, or a multifunctional branched center composed of two or more of the following structures:

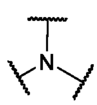 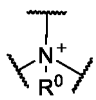 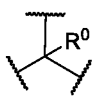 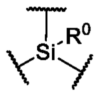 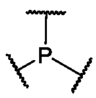 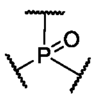 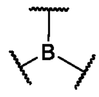

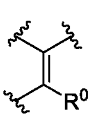 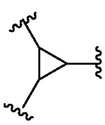 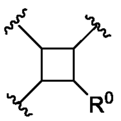 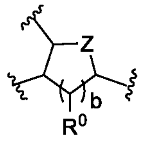 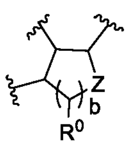 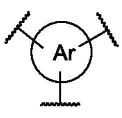

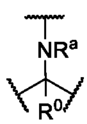 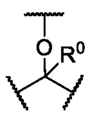 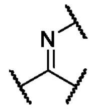 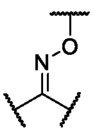 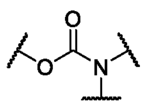 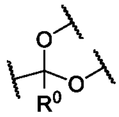 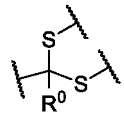

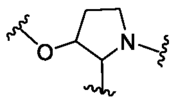 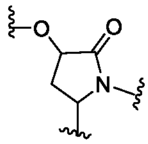 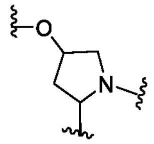

wherein Z is O, S, S(O), $S(O)_2$, $NR^a$, $CHR^0$;

$R^0$ is selected from H, D, halogen, nitro, cyano, $C_1$~$C_{10}$ alkyl, $C_1$~$C_{10}$ alkoxy, $C_3$~$C_{10}$ alkenyl, $C_3$~$C_{10}$ alkynyl, $C_3$~$C_8$ cycloalkyl, $C_2$~$C_8$ heterocycloalkyl, $C_6$~$C_{10}$ aryl, $C_5$~$C_{10}$ heteroaryl or functional groups containing primary amine, secondary amine, tertiary amine, hydroxyl, sulfhydryl, carboxyl, ester group, amide, boronic acid, borate, phosphoric acid, sulfonic acid, sulfoxide, aldehyde or ketone group; the heteroatoms in the $C_2$~$C_8$ heterocycloalkyl are O, S or N, and the number of the heteroatoms is one or more; when the number is more than one, the heteroatoms could be the same or different; the heteroatoms in the $C_5$~$C_{10}$ heteroaryl are O, S or N, and the number of the heteroatoms is one or more; when the number is more than one, the heteroatoms could be the same or different;

$R^a$ is selected from H, $C_1$~$C_{10}$ alkyl, $C_1$~$C_{10}$ alkoxy, $C_3$~$C_{10}$ alkenyl, $C_3$~$C_{10}$ alkynyl, $C_3$~$C_8$ cycloalkyl, $C_2$~$C_8$ heterocycloalkyl, $C_6$~$C_{10}$ aryl or $C_5$~$C_{10}$ heteroaryl; the heteroatoms in the $C_2$~$C_8$ heterocycloalkyl are O, S or N, and the number of the heteroatoms is one or more; when the number is more than one, the heteroatoms could be the same or different; the heteroatoms in the $C_5$~$C_{10}$ heteroaryl are O, S or N, and the number of the heteroatoms is one or more; when the number is more than one, the heteroatoms could be the same or different;

Ar is $C_6$~$C_{20}$ aryl or $C_5$~$C_{20}$ heteroaryl; the heteroatoms in the $C_5$~$C_{20}$ heteroaryl are O, S or N, and the number of the heteroatoms is one or more; when the number is more than one, the heteroatoms could be the same or different;

b is an integer of 0 to 3.

**3.** The poly(ε-L-lysine) derivative-drug conjugate according to claim 1 or 2, wherein the branched center Y is a substituted or non-substituted amino acid with at least 3 functionalities, the amino acid is a natural amino acid or an unnatural amino acid, and the configuration of the amino acid is D type or L type or D/L mixture; when the amino acid is a mixture of D/L configuration, the L configuration proportion is greater than 0% and less than 100%; the branched center Y is preferably an amino acid with at least 3 functionalities, the amino acid is one or more of aspartic acid, glutamic acid, lysine, ornithine, arginine, citrulline, histidine, serine, threonine, tryptophan, tyrosine, hydroxyproline, cystine, cysteine or selenocysteine, and the configuration of the amino acid is D type or L type or D/L mixture; when the amino acid is a mixture of D/L configuration, the L configuration proportion is greater than 0% and less than 100%.

**4.** The poly(ε-L-lysine) derivative-drug conjugate according to claim 1, wherein the linkers $L^0$ and $L^1$ are each independently covalent bonds, environmentally responsive linkers or environmentally non-responsive linkers; $L^2$ is an environmentally responsive linker, its structure is $L^{2a}$-$L^{2b}$, and $L^{2a}$ or $L^{2b}$ exists alone or both exist together; $L^{2a}$ and $L^{2b}$ are each independently covalent bonds, environmentally responsive linkers or environmentally non-responsive linkers; the structure of the poly(ε-L-lysine) derivative-drug conjugate is shown in formula III:

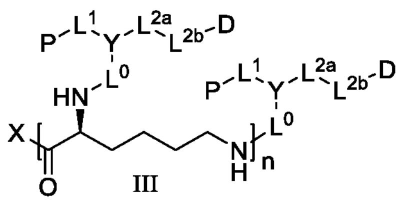

III

wherein X, Y, P, D, $L^0$ and $L^1$ are each defined as described in claim 1.

**5.** The poly(ε-L-lysine) derivative-drug conjugate according to claim 4, wherein the linkers $L^0$ and $L^1$ are covalent bonds, $L^2$ is an environmentally responsive linker, and its structure is $L^{2a}$-$L^{2b}$, wherein $L^{2a}$ is linked to Y, $L^{2b}$ is linked to D, and $L^{2a}$ or $L^{2b}$ exists alone or the both exist together; $L^{2a}$ and $L^{2b}$ are each independently covalent bonds environmentally responsive linkers or environmentally non-responsive linkers; the structure of the poly(ε-L-lysine) derivative-drug conjugate is shown in formula IV:

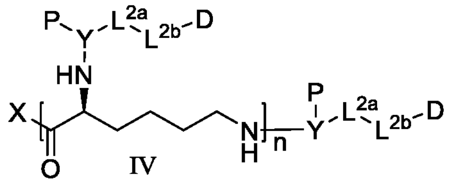

IV

wherein X, Y, P and D are each defined as described in claim 1.

**6.** The poly(ε-L-lysine) derivative-drug conjugate according to claim 1 or 4, when an electrophilic group in Y is linked to $L^2$, $L^{2a}$ does not exist; at this time, $L^2 = L^{2b}$, and the linking of the trifunctional branched center Y to the linker $L^0$, $L^1$ and $L^{2b}$ is selected from any of the following structures:

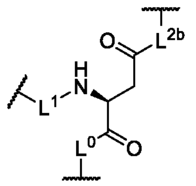 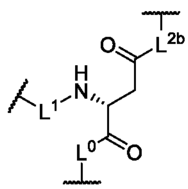 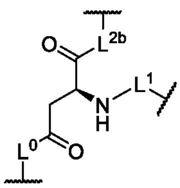 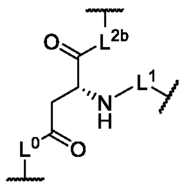

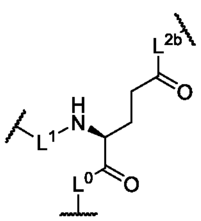 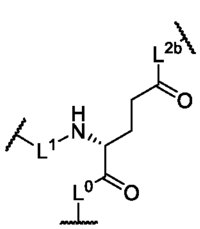 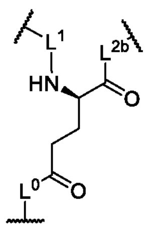 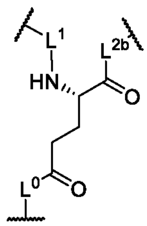 ;

preferably

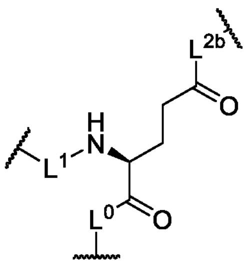 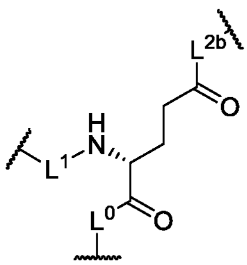 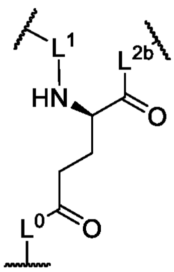 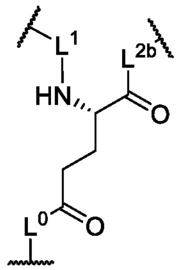 ,

more preferably

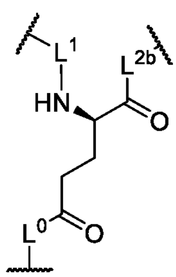 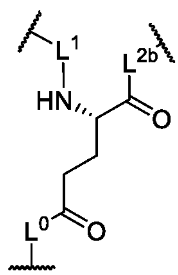 ;

alternatively, when a nucleophilic group in Y is linked to $L^2$, $L^{2a}$ exists alone or $L^{2a}$ and $L^{2b}$ exist together; at this time, $L^2 = L^{2a}$ or $L^2 = L^{2a}$-$L^{2b}$, and the linking of the trifunctional branched center Y to the linker $L^0$, $L^1$, $L^{2a}$ and $L^{2b}$

is selected from any of the following structures:

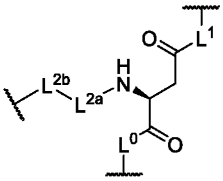
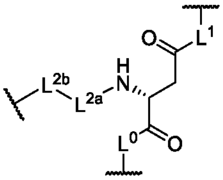
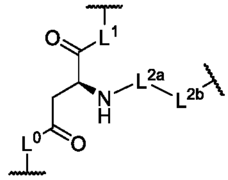
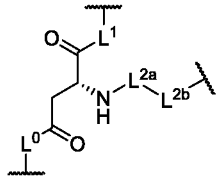

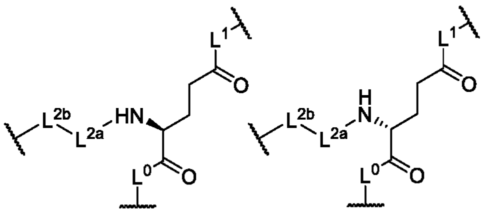
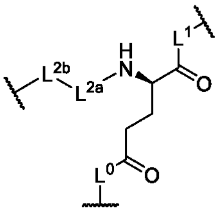
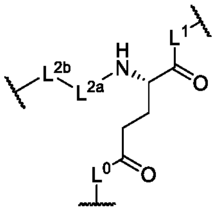

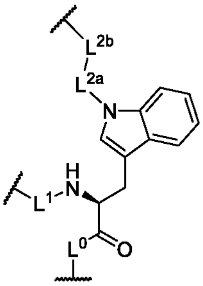
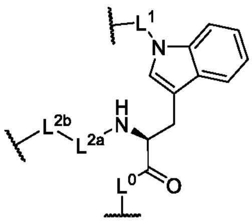
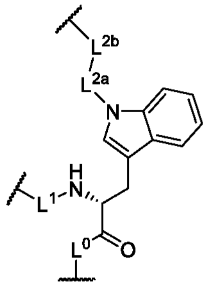
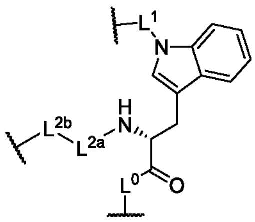

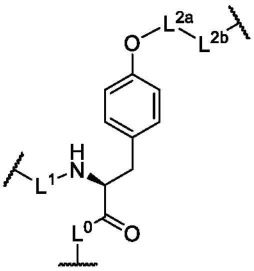
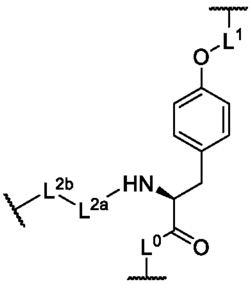
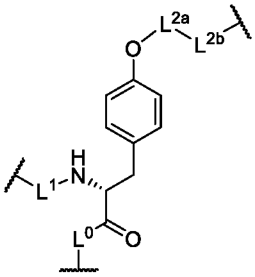
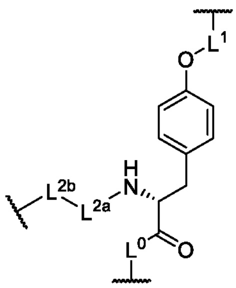

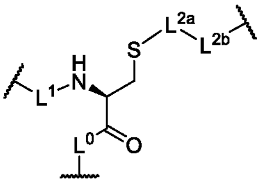
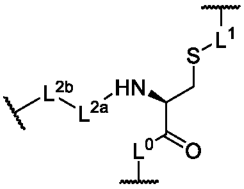
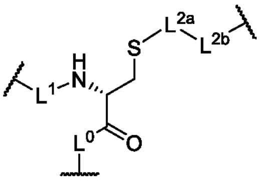
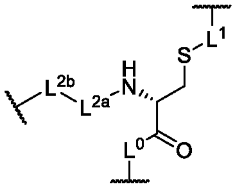

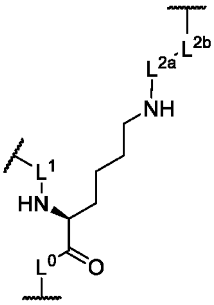
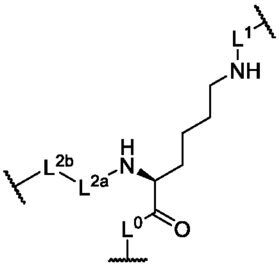
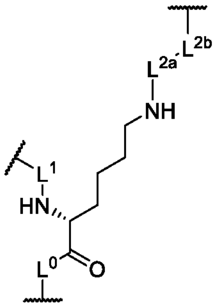
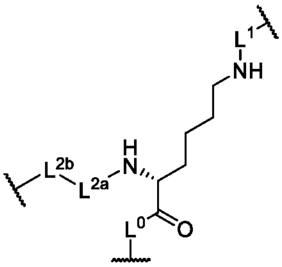

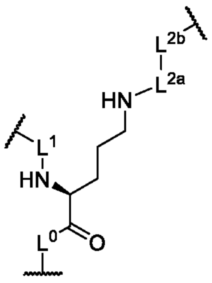
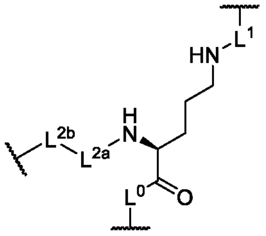
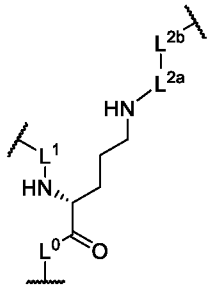
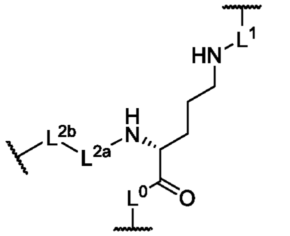

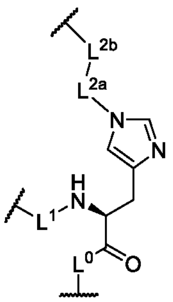
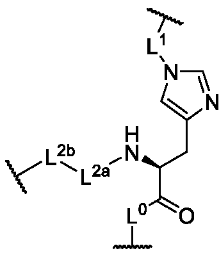
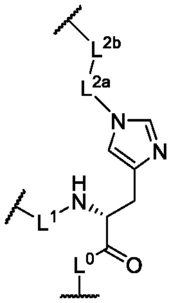
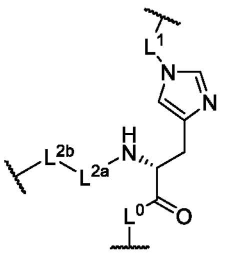

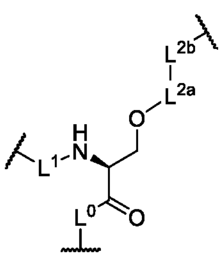
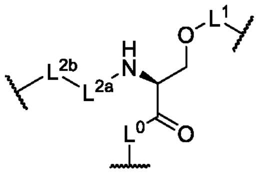
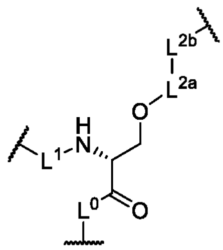
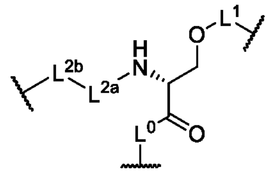

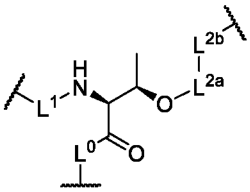
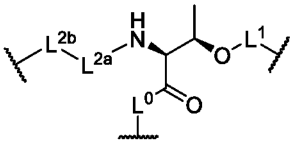
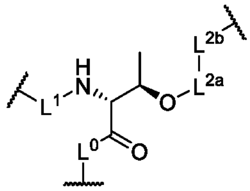
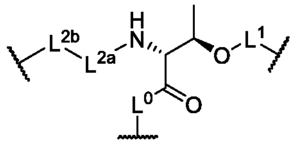

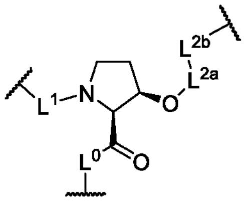
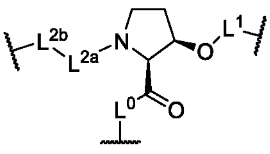
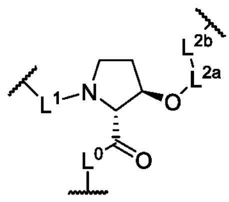
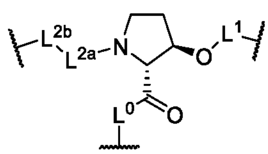

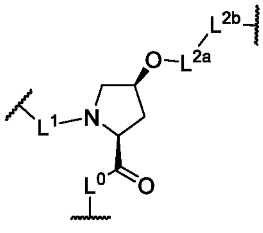
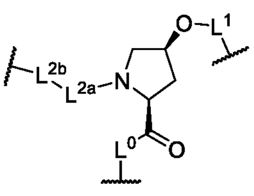
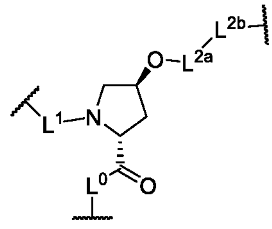
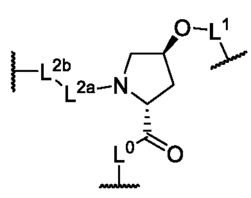

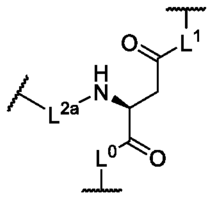
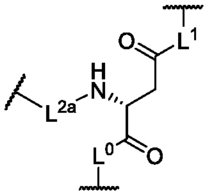
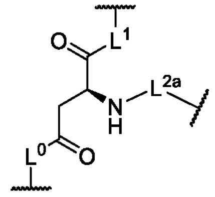
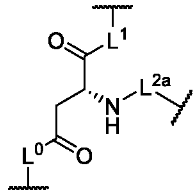

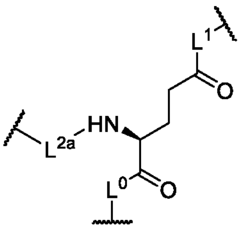
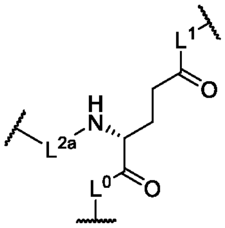
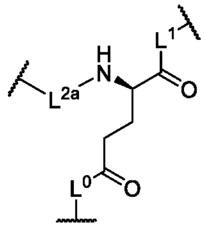
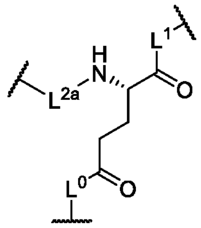

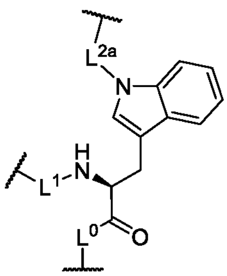
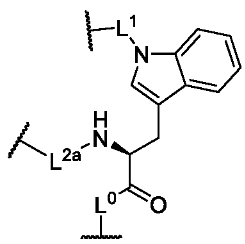
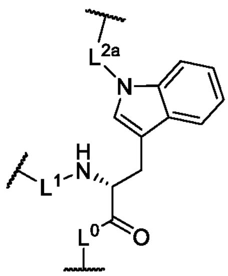
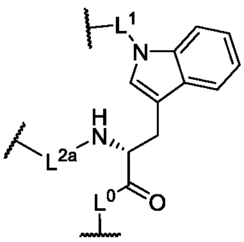

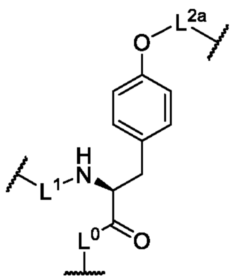
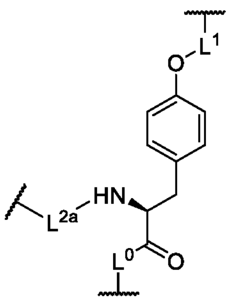
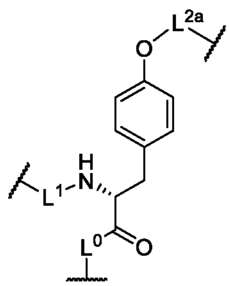
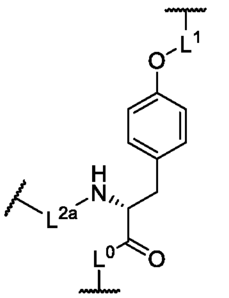

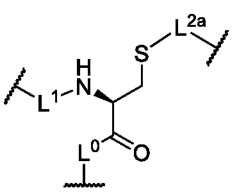
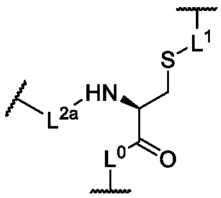
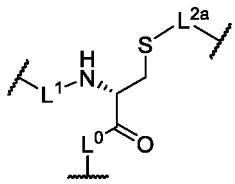
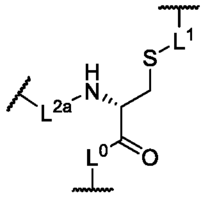

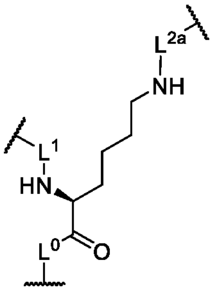
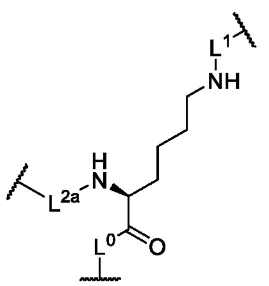
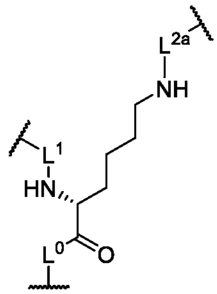
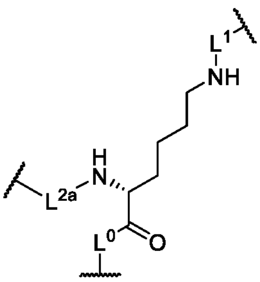

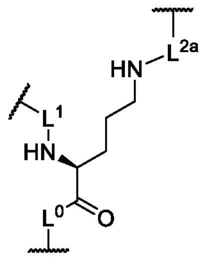 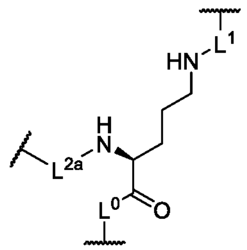 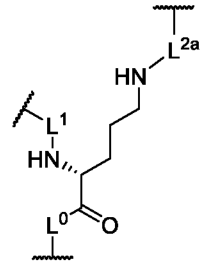 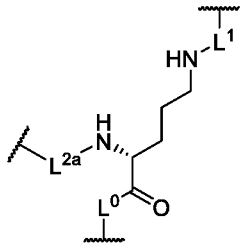

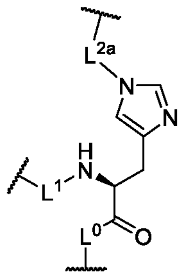 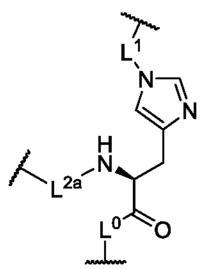 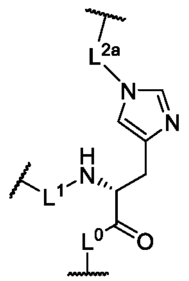 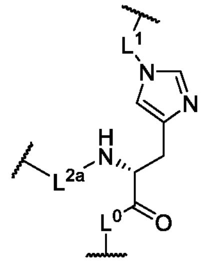

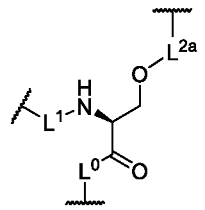 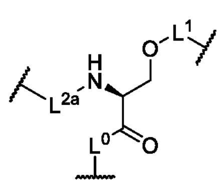 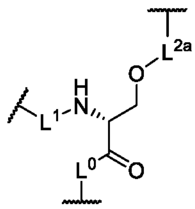 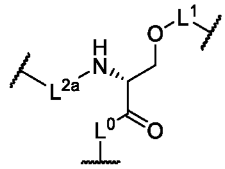

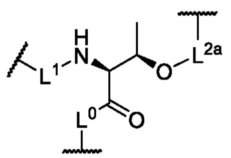 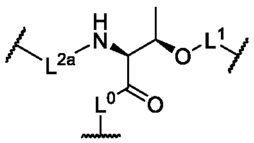 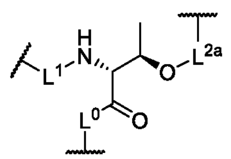 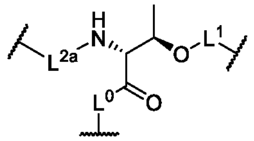

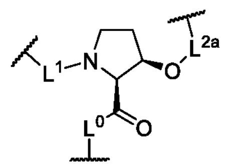 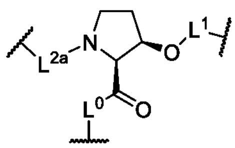 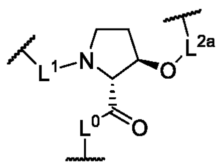 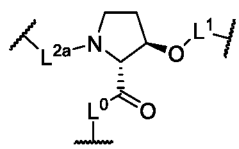

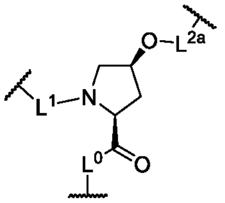 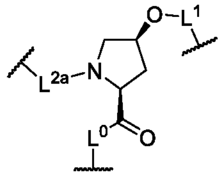 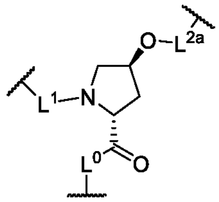 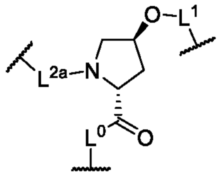 ;

preferably

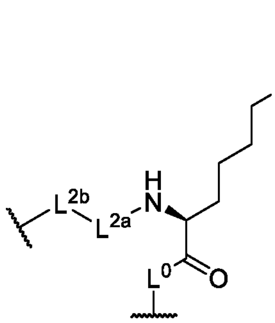

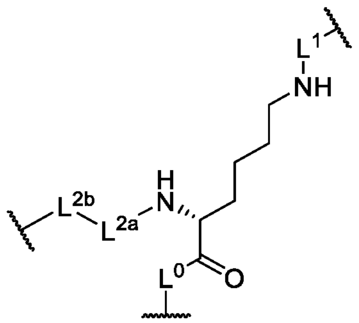

,

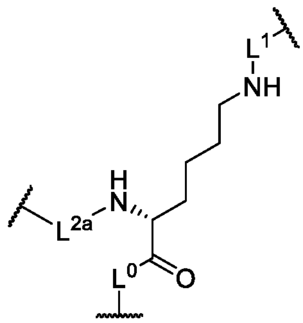

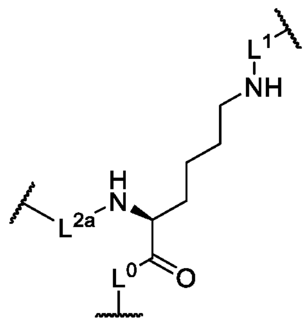

.

7. The poly(ε-L-lysine) derivative-drug conjugate according to any of claims 1, 4 and 5, wherein the terminal group X is selected from OR, SR, $NR^1R^2$, carboxyl protecting group or $L^{2b}$-D, and the R, $R^1$ and $R^2$ are independently selected from H, $C_1$~$C_{30}$ alkyl, $R^{1-1}$-substituted $C_1$~$C_{10}$ alkyl, $C_1$~$C_{30}$ alkoxy, $C_3$~$C_{30}$ alkenyl, $C_3$~$C_{30}$ alkynyl, $C_3$~$C_8$ cycloalkyl, $C_2$~$C_8$ heterocycloalkyl, $C_6$~$C_{20}$ aryl or $C_5$~$C_{20}$ heteroaryl; or $R^1$, $R^2$ and N atoms linked thereto form $C_2$~$C_8$ heterocycloalkyl; the heteroatoms in the $C_2$~$C_8$ heterocycloalkyl are O, S or N, and the number of the heteroatoms is one or more; when the number is more than one, the heteroatoms could be the same or different; the heteroatoms in the $C_5$~$C_{20}$ heteroaryl are O, S or N, and the number of the heteroatoms is one or more; when the number of the heteroatoms is more than one, the heteroatoms are the same or different; the terminal group X is preferably selected from OR, SR, $NR^1R^2$, carboxyl protection group or $L^{2b}$-D, and the R, $R^1$ and $R^2$ are independently selected from H, $C_1$~$C_{10}$ alkyl, or $R^{1-1}$-substituted $C_1$~$C_{10}$ alkyl; the $R^{1-1}$ is $-NH_2$ or

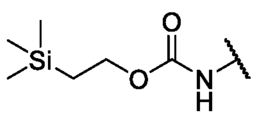

.

8. The poly(ε-L-lysine) derivative-drug conjugate according to any of claims 1 and 4-6, wherein the environmentally responsive linker is one or more of enzyme-responsive linker, pH-responsive linker, photo-responsive linker and redox-responsive linker.

9. The poly(ε-L-lysine) derivative-drug conjugate according to claim 8, wherein the poly(ε-L-lysine) derivative-drug conjugate fulfills one or more of the following:

(1) the enzyme-responsive linker is cleavable by one or more of the following enzymes: secretory phospholipase A2, acid phosphatase, serum alkaline phosphatase, cytochrome P450, sulfatase, prostate-specific antigen, phospholipase A1, phospholipase A2, phospholipase B, phospholipase C, phospholipase D, neutrophil elastase, cysteine protease-3, cathepsin, matrix metalloproteinase, β-glucuronidase, β-galactosidase, DTP, nitroreductase, NADPH, aminopeptidase N, carboxylesterase, diaphorase, histone deacetylase, asparaginyl endopeptidase, urokinase-type plasminogen activator, urokinase-type plasminogen activator receptor and collagenase; the enzyme-responsive linker is preferably cleaved by one or more of the following enzymes: cysteine protease-3, cathepsin, matrix metalloproteinase and β-glucuronidase;
(2) the pH-responsive linker comprises one or more of the following structures: hydrazone, imine, oxime, carboxylate, thioester, sulfate, sulfonate, orthoester, carbonate, carbamate, substituted carbamate, ketal, acetal, silyl ether, phosphate, borate, phosphoramidate or cis-aconitic acid groups;

(3) the photo-responsive linker comprises one or more of the following structures: o-nitrobenzene, coumarin, benzoin, BODIPY or cyanine groups;

(4) the redox-responsive linker comprises one or more of the following structures: thioketal, phenylborate, phenylboronic acid, oxalate, vinyl ether, thioether, amino acrylate, disulfide, diselenide, 2,4-dinitrobenzenesulfonate, 2-azidomethylbenzoate, 4-azidobenzyl, unsaturated acid ester or azobenzene groups.

**10.** The poly(ε-L-lysine) derivative-drug conjugate according to claim 8, wherein the enzyme-responsive linker comprises one or more of the following amino acid sequences: Cit-Phe, Lys-Lys, Phe-Lys, Arg-Arg, Val-Cit, Val-Ala, Val-Lys, Ala-Lys, Phe-Cit, Leu-Cit, Ile-Cit, Trp-Cit, Phe-Arg, Asn-Pro-Val, Gly-Pro-Nle, Glu-Val-Cit, Glu-Val-Ala, Gly-Phe-Gly, Gly-Phe-Phe, Gly-Leu-Gly, Gly-Val-Ala, Gly-Phe-Leu-Gly, Gly-Phe-Phe-Leu, Gly-Leu-Leu-Gly, Gly-Phe-Tyr-Ala, Gly-Phe-Gly-Phe, Gly-Gly-Phe-Gly, Ala-Gly-Val-Phe, Gly-Phe-Phe-Gly, Asp-Glu-Val-Asp, Gly-Phe-Leu-Gly-Phe, Gly-Phe-Ala-Gly-Leu-Phe, Gly-Leu-Ala-Ala-Val-Ala, Gly-Gly-Phe-Leu-Gly-Phe or Gln-Ser-Phe-Arg-Phe-Lys.

**11.** The poly(ε-L-lysine) derivative-drug conjugate according to claim 8, wherein the poly(ε-L-lysine) derivative-drug conjugate fulfills one or more of the following:

(1) the enzyme-responsive linker comprises the following structures:

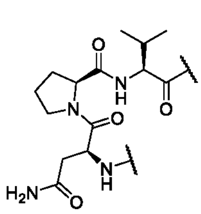
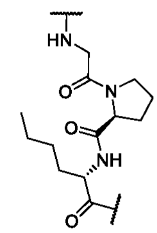
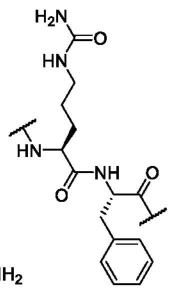
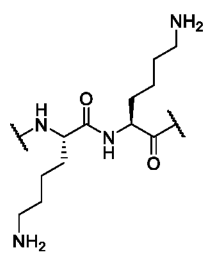

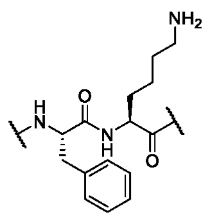
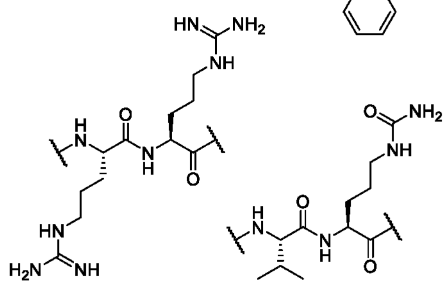
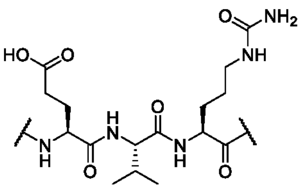

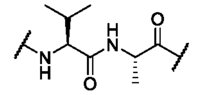
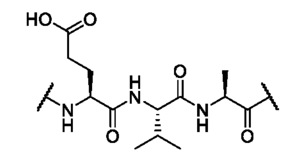
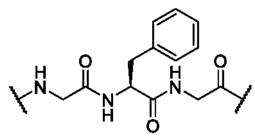
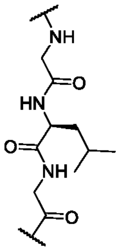

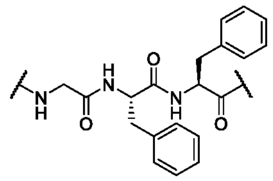
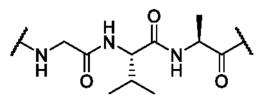
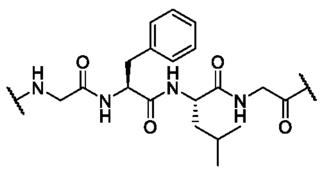

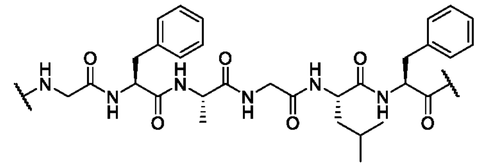
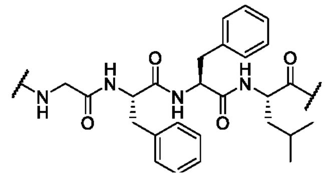

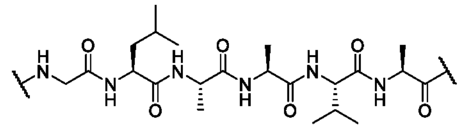

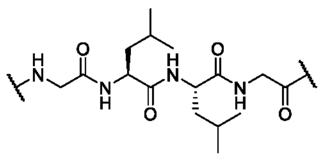

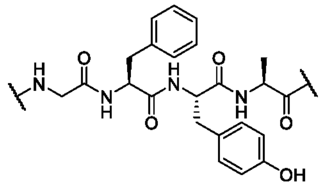

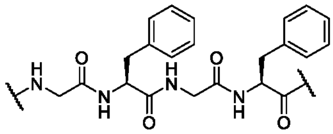

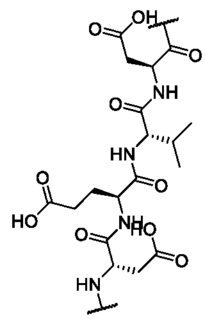

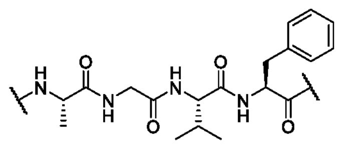

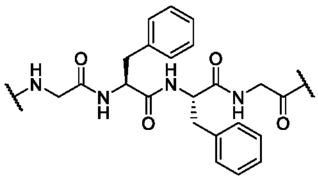

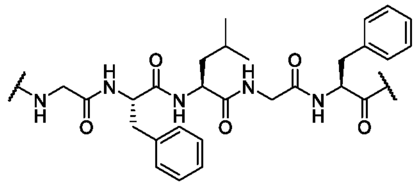

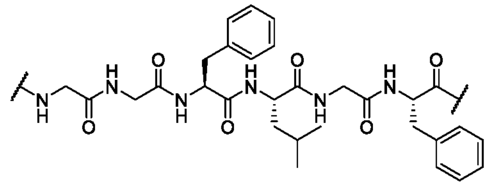

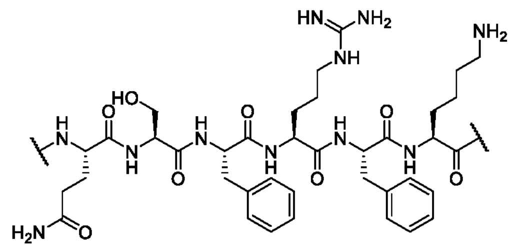

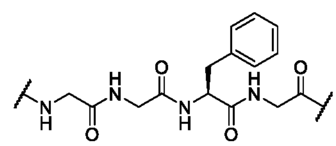

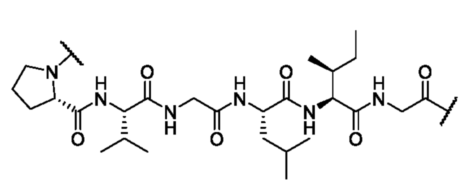

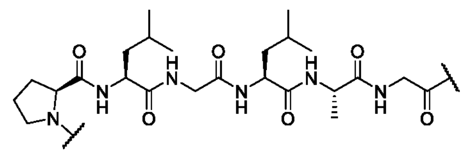

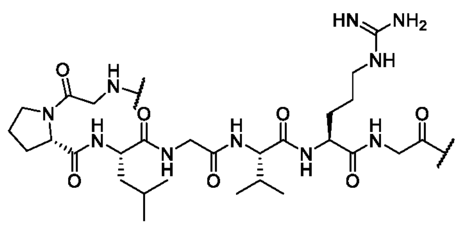

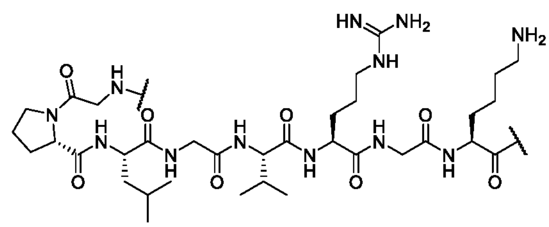

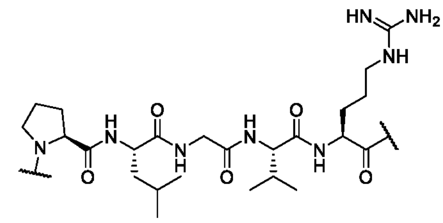
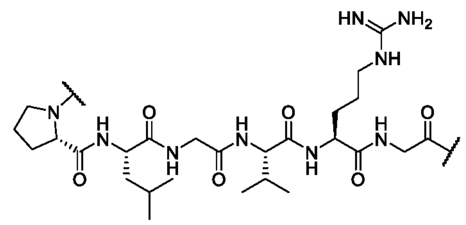
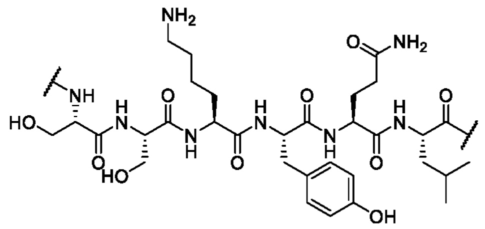
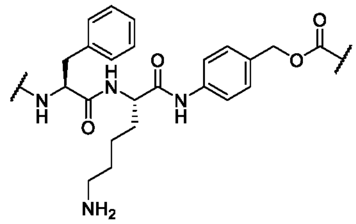
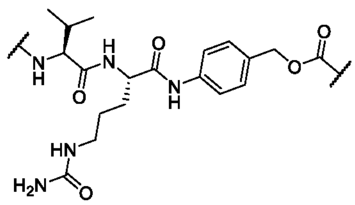
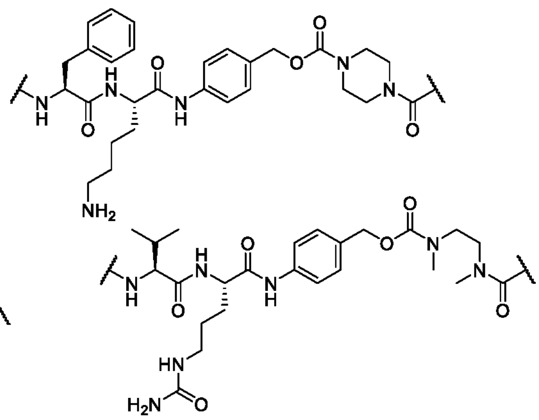
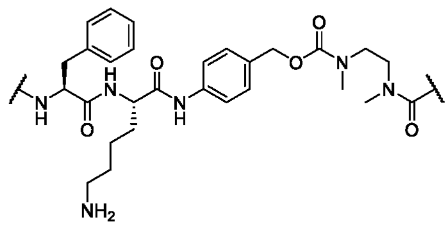

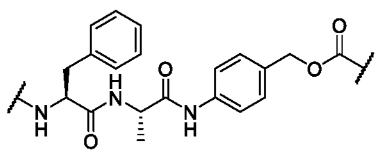
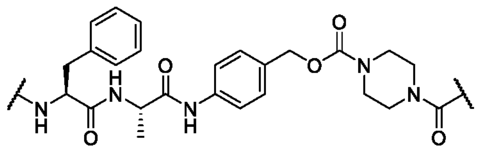
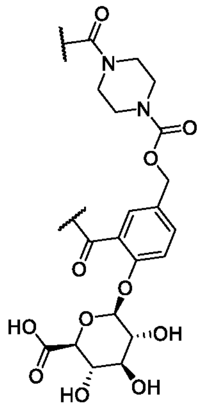
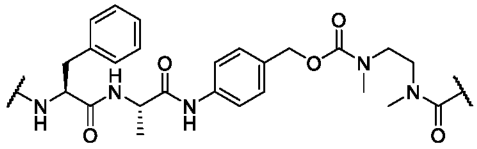
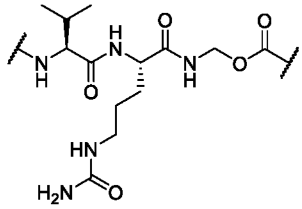
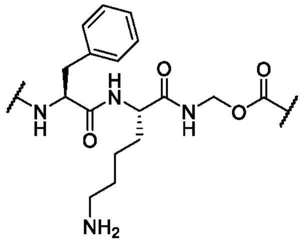
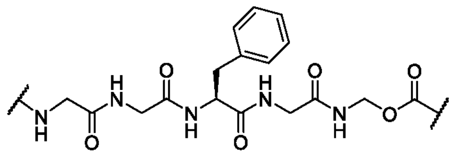
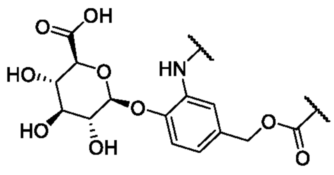
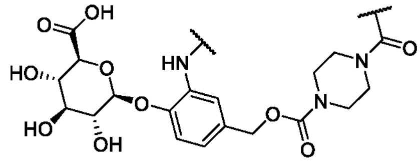
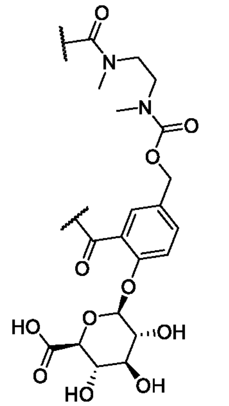
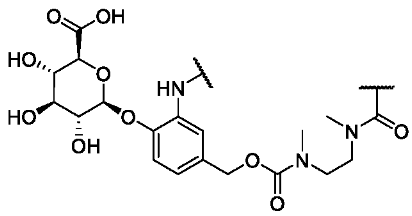
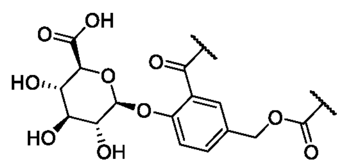

;

(2) the pH-responsive linker comprises the following structures:

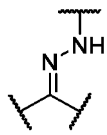
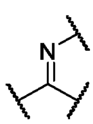
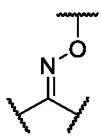
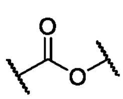
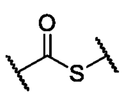
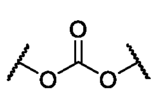
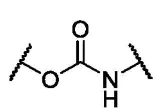
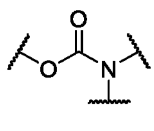
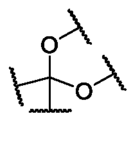
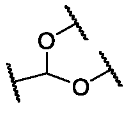
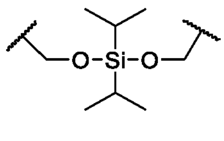
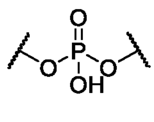
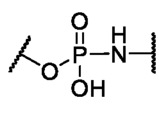
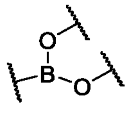
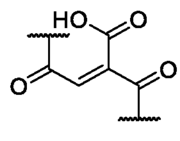
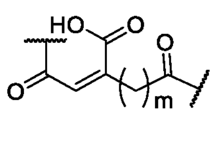

,

wherein m is 0~4;

(3) the photo-responsive linker comprises the following structures:

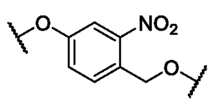
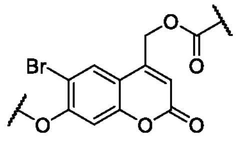
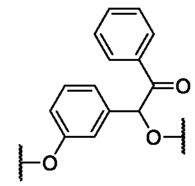
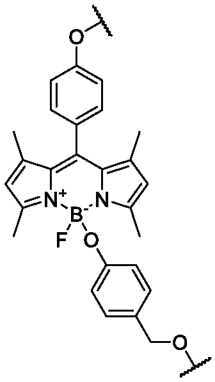
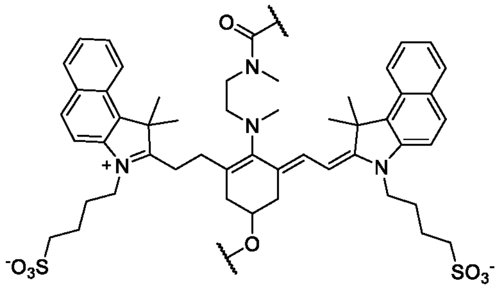

;

(4) the redox-responsive linker comprises the following structures:

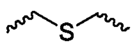
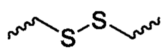
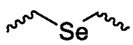
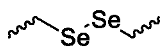
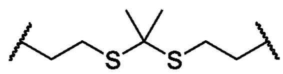
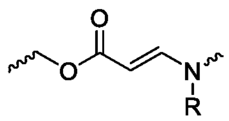
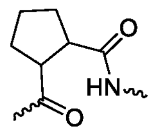
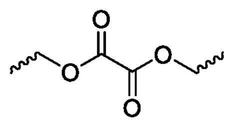
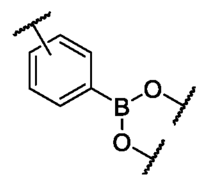
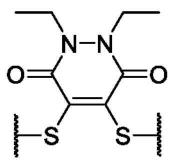
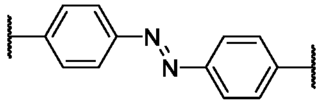

**12.** The poly(ε-L-lysine) derivative-drug conjugate according to any of claims 4-5 and 8, wherein the linker $L^{2a}$ is a covalent bond or has a structure shown in formula V:

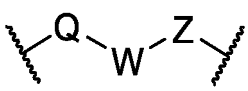

V

Q is selected from one of

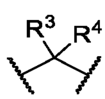
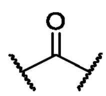
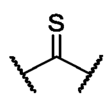

;

wherein $R^3$ and $R^4$ are independently selected from H, D, $C_1$~$C_6$ alkyl, $C_1$~$C_6$ alkoxy, $C_3$~$C_6$ alkenyl, $C_3$~$C_6$ alkynyl, $C_3$~$C_8$ cycloalkyl, $C_2$~$C_8$ heterocycloalkyl, $C_6$~$C_{10}$ aryl or $C_5$~$C_{10}$ heteroaryl; or $R^3$, $R^4$ and C atoms linked thereto form $C_3$~$C_8$ alkyl or heterocycloalkyl; the heteroatoms in the $C_2$~$C_8$ heterocycloalkyl are O, S or N, and the number of the heteroatoms is one or more; when the number is more than one, the heteroatoms could be the same or different; the heteroatoms in the $C_5$~$C_{10}$ heteroaryl are O, S or N, and the number of the heteroatoms is one or more; when the number is more than one, the heteroatoms could be the same or different; W is a covalent bond or a $C_0$~$C_{20}$ fragment with or without heteroatoms, wherein the heteroatoms are O, S, Se, N, P, Si or B, and the number of the heteroatoms is one or more; when the number is more than one, the heteroatoms could be the same or different; the W fragment contains unsaturated bonds or contains no unsaturated bonds;

Z is selected from one of

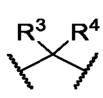 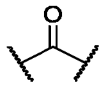 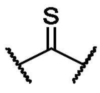 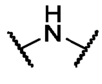 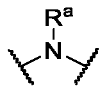 ,

wherein $R^a$ is defined as described in claim 2.

**13.** The poly(ε-L-lysine) derivative-drug conjugate according to claim 12, wherein the linker $L^{2a}$ is selected from the following structures or a covalent bond:

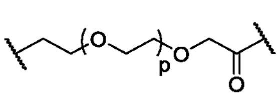 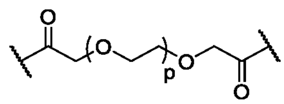 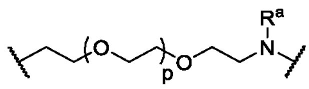

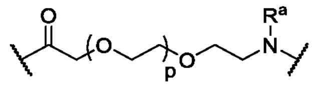

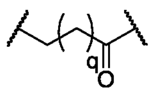 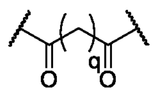 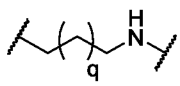 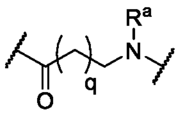

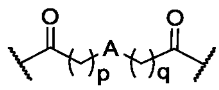 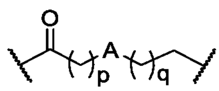 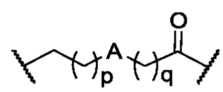

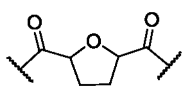 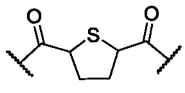 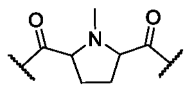 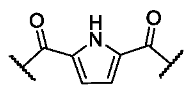 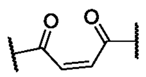

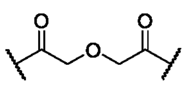 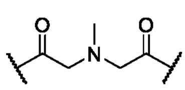 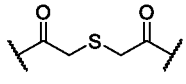 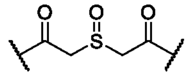 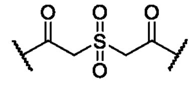

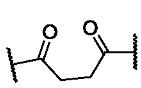 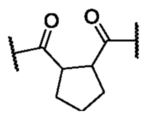 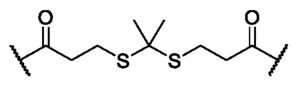 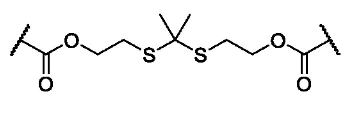

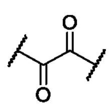 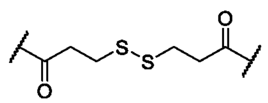 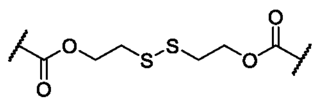 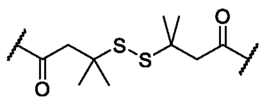

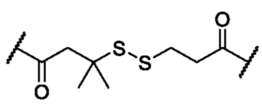 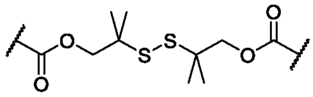 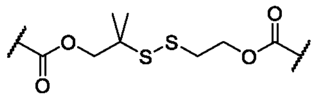

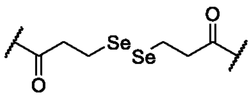 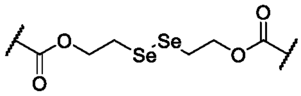 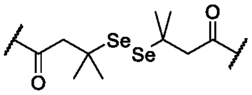

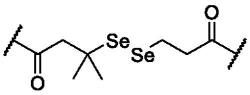 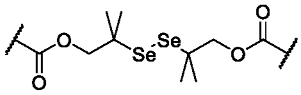 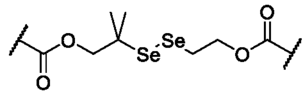

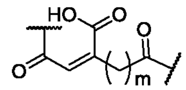

wherein,

A is O, S, S(O), $S(O)_2$, $NR^a$ or $C(R^3R^4)$;
p is an integer of 0 to 16;
q is an integer of 0 to 16;
m is an integer of 0 to 4;
$R^a$ is defined as described in claim 2;
$R^3$ and $R^4$ are defined as described in claim 12.

**14.** The poly(ε-L-lysine) derivative-drug conjugate according to claim 1, wherein the linker $L^{2a}$ is selected from the following structures:

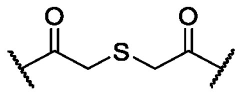, 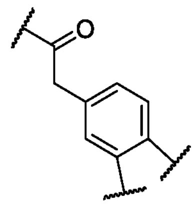,

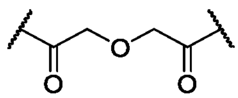, 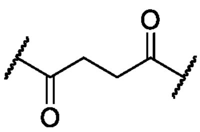, 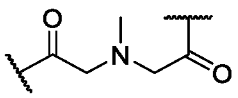, 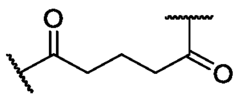,

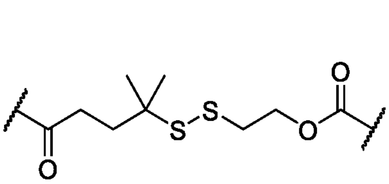, 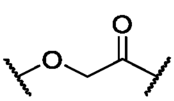, 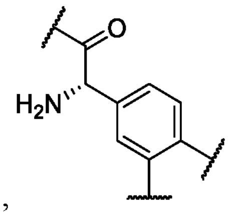, 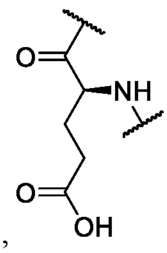 or 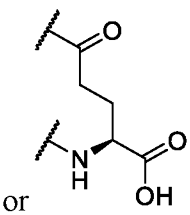.

**15.** The poly(ε-L-lysine) derivative-drug conjugate according to any of claims 4-6, wherein the linker $L^{2b}$ is selected from the following structures:

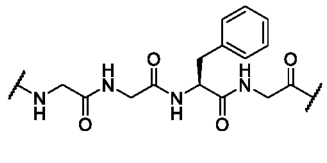

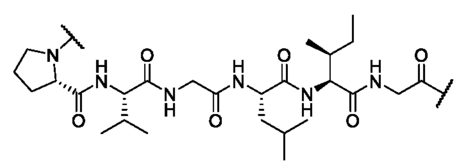

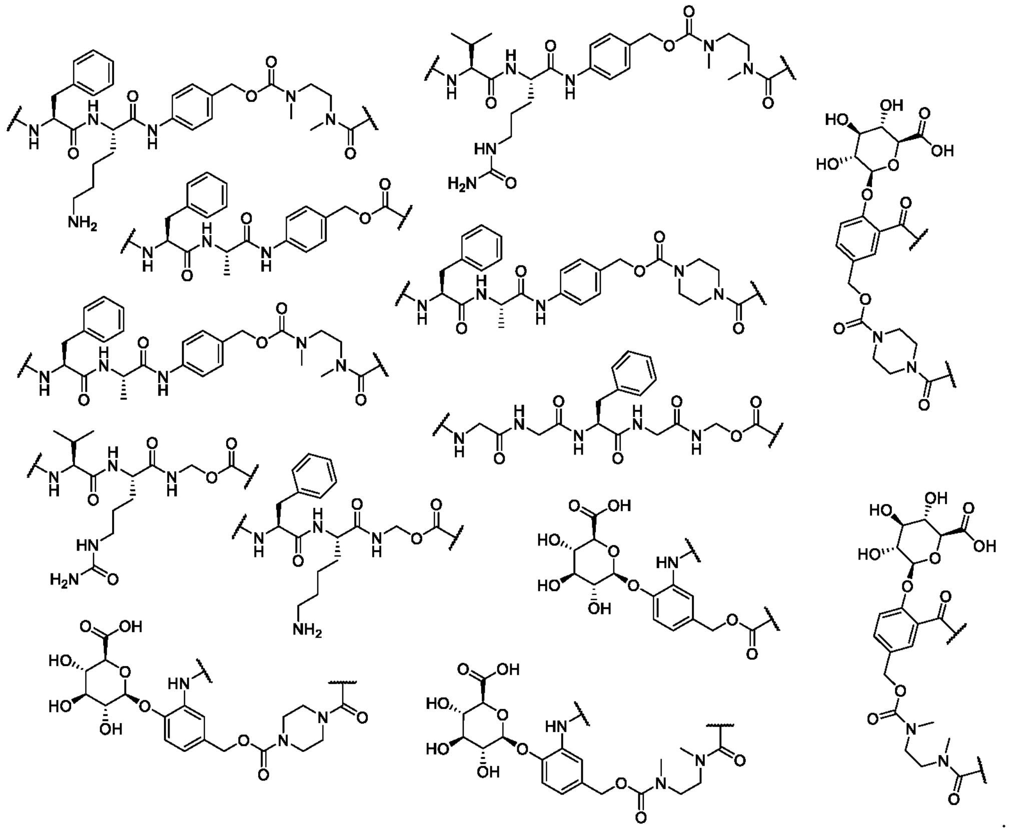

16. The poly(ε-L-lysine) derivative-drug conjugate according to any of claims 4-6, wherein the linker $L^{2b}$ is selected from the following structures:

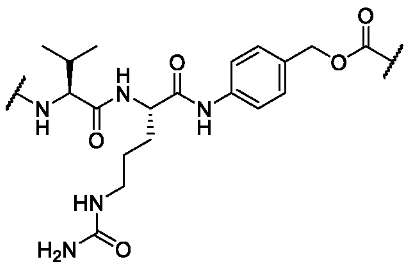

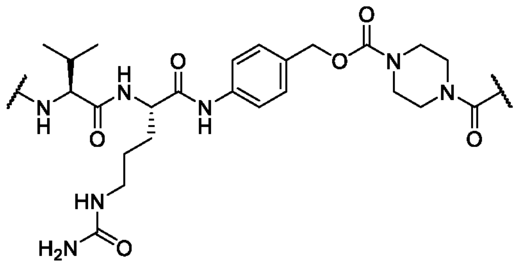

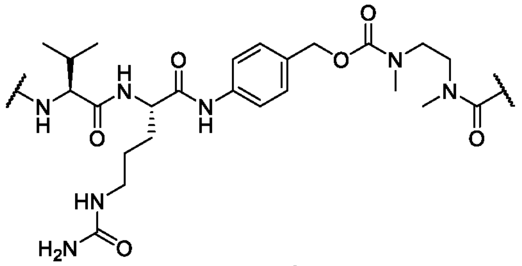

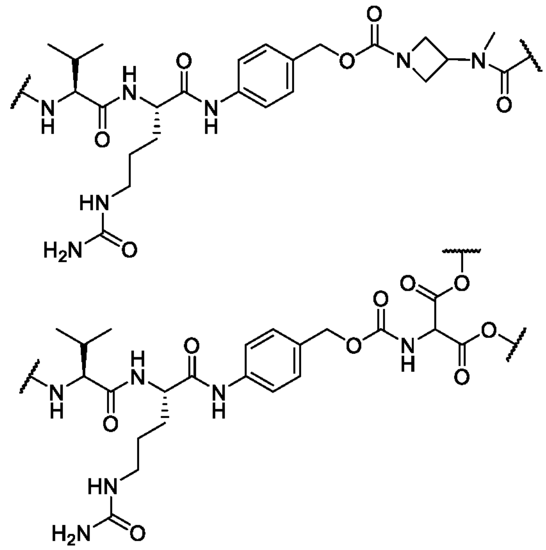

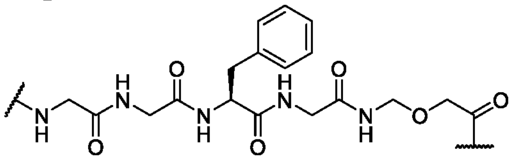

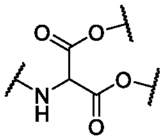

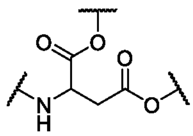

.

**17.** The poly(ε-L-lysine) derivative-drug conjugate according to any of claims 1 and 4-5, wherein the pharmacokinetic regulator residue P is selected from a polyethylene glycol derivative residue with the number of repeating units being a and a terminal group being $R^b$, a hyaluronic acid derivative residue with the number of repeating units being b, a polyphosphate residue with the number of repeating units being c, a polysarcosine residue with the number of repeating units being d or a polyoxazoline residue with the number of repeating units being f;

wherein a is an integer of 5 to 250, b is an integer of 5 to 250, c is an integer of 5 to 250, d is an integer of 5 to 250, e is an integer of 5 to 250, and f is an integer of 5 to 250; $R^b$ is H, $C_1$~$C_{10}$ alkyl, $C_1$~$C_{10}$ heteroalkyl, $C_3$~$C_{10}$ cycloalkyl, $C_3$~$C_{10}$ alkenyl, $C_3$~$C_{10}$ alkynyl or hydroxyl protecting group;
preferably, the pharmacokinetic regulator residue P fulfills one or more of the following:

(1) when the pharmacokinetic regulator residue P is the polyethylene glycol derivative residue with the number of repeating units being a and a terminal group being $R^b$, a is an integer of 5 to 150, preferably an integer of 10 to 60, more preferably an integer of 15 to 50, such as 21, 43 or 44;
(2) when the pharmacokinetic regulator residue P is the polyethylene glycol derivative residue with the number of repeating units being a and a terminal group being $R^b$, $R^b$ is $C_1$~$C_{10}$ alkyl, preferably Ci~Ce alkyl, more preferably $C_1$~$C_3$ alkyl, such as methyl, ethyl, n-propyl or i-propyl.

**18.** The poly(ε-L-lysine) derivative-drug conjugate according to claim 17, wherein the pharmacokinetic regulator residue P is a polyethylene glycol derivative residue with the number of repeating units being a and a terminal group being $R^b$, or a hyaluronic acid derivative residue with the number of repeating units being b; the polyethylene glycol derivative residue is selected from the following structures:

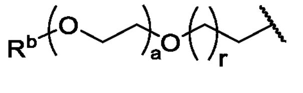 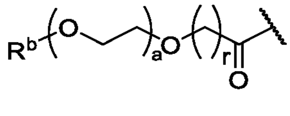

wherein a is an integer of 5 to 150, b is an integer of 5 to 150, r is an integer of 0 to 8; the polyethylene glycol derivative residue is preferably

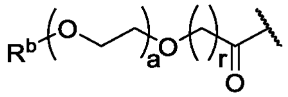
,

a is an integer of 20 to 45, b is an integer of 5 to 10, and r is an integer of 0 to 3.

**19.** The poly(ε-L-lysine) derivative-drug conjugate according to claim 18, wherein the pharmacokinetic regulator residue P is a methyl-terminated polyethylene glycol derivative residue with the number of repeating units being a; the methyl-terminated polyethylene glycol derivative residue is selected from the following structures:

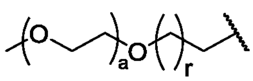 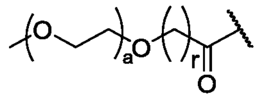

wherein a is an integer of 5 to 150, and r is an integer of 0 to 8;
the methyl-terminated polyethylene glycol derivative residue is preferably

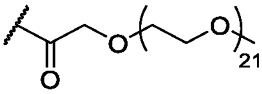 or 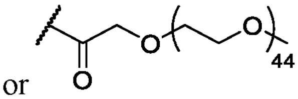.

**20.** The poly(ε-L-lysine) derivative-drug conjugate according to claim 1, 4 or 5, wherein the pharmaceutically active agent D contains active functional groups; the active functional groups are selected from one or more of primary amine, secondary amine, tertiary amine, hydroxyl, sulfhydryl, carboxyl, ester, amide, boronic acid, borate, phosphoric acid, sulfonic acid, sulfoxide, aldehyde and ketone group.

**21.** The poly(ε-L-lysine) derivative-drug conjugate according to claim 1, 4 or 5, wherein the pharmaceutically active agent is selected from one or more of anesthetics, antacids, anti-infective drugs, cardiovascular drugs, diuretics, blood tonics, immunosuppressants, GLP-1 receptor agonists, hormones and analogues, ophthalmic drugs, pain treatment agents, respiratory drugs, anti-arthritis drugs, anticonvulsants, antihistamines, anti-inflammatory drugs, anti-ulcer drugs, behavior modification drugs, antitumor drugs, anticancer antigens, central nervous system drugs, psychiatric disease drugs, contraceptives, diabetes drugs, growth promoters, hemostatic drugs, immunostimulants, immunomodulators, muscle relaxants, obesity therapeutics, osteoporosis drugs, sedatives, tranquilizers, urinary acidifiers, vitamins, polypeptide drugs, oligonucleotide drugs, mRNA drugs, antibody drugs, biological products, targeted protein degraders, PROTACs, oligosaccharide drugs or targeted drugs.

**22.** The poly(ε-L-lysine) derivative-drug conjugate according to any of claims 1, 4-5 and 20-21, wherein the pharmaceutically active agent residue D is an antitumor drug residue, the antitumor drug is selected from one or more of targeted therapy drugs for tumor, PROTACs, targeted protein degraders, tumor immunomodulators or chemotherapy drugs.

**23.** The poly(ε-L-lysine) derivative-drug conjugate according to claim 22, wherein the antitumor drug is selected from one or more of abemaciclib, abiraterone, abrocitinib, acalabrutinib, afatinib, aldesleukin, alectinib, alflutinib, almonertinib, altretamine, amcenestrant, aminoglutethimide, amsacrine, anastrozole, anlotinib, apalutamide, apatinib, arzoxifene, asciminib, asparaginase, avapritinib, avitinib, axitinib, azacitidine, baricitinib, belinostat, bendamustine, bexarotene, bicalutamide, bicyclol, binimetinib, bleomycin, boanmycin, bortezomib, bosutinib, brigatinib, buserelin, busulfan, cabazitaxel, cabozantinib, calaspargase, calicheamycin, capecitabine, capmatinib, carboplatin, carfilzomib, carmustine, carmofur, cedazuidine, ceritinib, cetrorelix, chidamide, chlorambucil, cisplatin, cladribine, clofarabine, cobimetinib, colchicine, copanlisib, crizotinib, cyclophosphamide, cytarabine, dabrafenib, dacarbazine, dacomitinib, dactinomycin, dalpiciclib, darolutamide, dasatinib, daunorubicin, decitabine, degarelix, delgociclib, denileukin, deruxtecan, docetaxel, donafenib, doxorubicin, duvelisib, enasidenib, encorafenib, ensartinib, entrectinib, enzalutamide, enzastaurin, elacestrant, epirubicin, erdafitinib, eribulin, erlotinib, estradiol, estramustine, etoposide, everolimus, exemestane, fasudil, fedatinib, filgotinib, floxuridine, fludarabine, flumatinib, fluorouracil, flutamide, fluzoparib, formestane, fostamatinib, fruquintinib, fulvestrant, gefitinib, gemcitabine, gilteritinib, giredestrant, glasdegib, goserelin, histrelin, hydroxyurea, ibrutinib, ibudilast, icaritin, icotinib, idarubicin, idelalisib, ifosfamide, imatinib, imiquimod, infigratinib, ingenol mebutate, interferon alfa-2b, irinotecan, ivosidenib, ixabepilone, ixazomib, lanreotide, lapatinib, larotrectinib, lenalidomide, lenvatinib, letrozole, leucovorin, leuprolide, lomustine, lonafarnib, lorlatinib, lurbinctedin, maytansine, mechlorethamine, medroxyprogesterone, megestrol, melphalan, melphlan flufenamide, mercaptopurine, methotrexate, methoxsalen, methylprednisolone, midostaurin, mitomycin, mitotane, mitoxantrone, mitozolomide, mobocertinib, monomethylauristatin E, monomethylauristatin F, nelarabine, nandrolone, neratinib,

nearsudil, nilotinib, nilutamide, nintedanib, niraparib, octreotide, olaparib, olmutinib, omacetaxine, orelabrutinib, osimertinib, oxaliplatin, paclitaxel, pacritinib, palbociclib, pamidronate, pamiparib, panobinostat, pazopanib, peficitinib, pegaptanib, pegaspargase, peginteferon alfa-2b, pemigatinib, pemetrexed, pentetreotide, pentostatin, pexidartinib, phenoxybenzamine, pidotimod, plinabulin, plitidepsin, pomalidomide, ponatinib, porfimer, pralatrexate, pralsetinib, prednisolone, procarbazine, pyrotinib, quizartinib, radotinib, raloxifene, raltitrexed, regorafenib, ribociclib, rintatolimod, ripretinib, romidepsin, rucaparib, ruxolitinib, savolitinib, selinexor, selpercatinib, selumetinib, sonidegib, sorafenib, sotorasib, streptozocin, sunitinib, surufatinib, talazoparib, tamoxifen, tazemetostat, tegafur, temozolomide, temsirolimus, teniposide, tepotinib, teprenone, thalidomide, thioguanine, thiotepa, thyrotropin alfa, tipiracil, tipifarnib, tirabrutinib, tirbanibulin, tivozanib, trametinib, tofacitinib, topotecan, toremifene, trabectedin, tretinoin, trifluride, trilaciclib, triptorelin, tucatinib, upadacitinib, umbralisib, utidelone, uroacitide, valrubicin, vandetanib, vemurafenib, venetoclax, vinblastine, vincristine, vindesine, vinflunine, vinorelbine, vismodegib, vorinostat, zanubrutinib, zoledronic acid, amatoxins, anthacyclines, anthracenes, anthramycins, auristatins, bryostatins, camptothecins, carmaphycins, combretastatins, cyclosporines, cryptomycins, ecteinascidins, ellipticenes, esperamicins, mustines, neothramycins, ozogamicins, phenoxazines, podophyllotoxins, pyrrolobenzodiazepines, sibiromycins, thailanstatins, tomamycns, tubulysins, taxanes, vinca alkaloids, 7-epipaclitaxel, 2'-acetylpaclitaxel, 10-deacetylpaclitaxel, 7-epi-10-deacetyltaxol, 7-xylosyltaxol, 10-deacetyl-7-glutarylpaclitaxel, 7-N,N-dimethylglycylpaclitaxel, 7-L-alanylacetaxel, larotaxel, camptothecin, 9-aminocamptothecin, 9-nitrocamptothecin, lurtotecan, gimatecan, belotecan, 10-hydroxycamptothecin, 10-hydroxy-7-ethyl-camptothecin (SN-38), exatecan, pirarubicin, aclacinomycin, sirolimus, tacrolimus, progesterone, estrogen, rapamycin, mithramycin, harringtonine or curcumin.

**24.** The poly(ε-L-lysine) derivative-drug conjugate according to claim 22 or 23, wherein the poly(ε-L-lysine) derivative-drug conjugate fulfills one or more of the following:

(1) the antitumor drug is a targeted therapy drugs for tumor, and is selected from one or more of aldesleukin, abemaciclib, abiraterone, abrocitinib, acalabrutinib, afatinib, alectinib, alflutinib, almonertinib, amcenestrant, anastrozole, anlotinib, apalutamide, apatinib, arzoxifene, asciminib, avapritinib, avitinib, axitinib, baricitinib, belinostat, bexarotene, bicalutamide, binimetinib, bleomycin, boanmycin, bortezomib, bosutinib, brigatinib, buserelin, cabozantinib, capmatinib, carfilzomib, carmustine, ceritinib, cetrorelix, chidamide, cobimetinib, copanlisib, crizotinib, dabrafenib, dacomitinib, dalpiciclib, darolutamide, dasatinib, degarelix, delgociclib, donafenib, duvelisib, enasidenib, encorafenib, ensartinib, entrectinib, enzalutamide, enzastaurin, elacestrant, erdafitinib, erlotinib, everolimus, fedatinib, filgotinib, flumatinib, fluzoparib, formestane, fostamatinib, fruquintinib, fulvestrant, gefitinib, gilteritinib, giredestrant, glasdegib, goserelin, histrelin, ibrutinib, ibudilast, icotinib, idarubicin, idelalisib, imatinib, imiquimod, infigratinib, ivosidenib, ixazomib, lanreotide, lapatinib, larotrectinib, lenalidomide, lenvatinib, letrozole, leucovorin, leuprolide, lonafarnib, lorlatinib, medroxyprogesterone, megestrol, methylprednisolone, midostaurin, mobocertinib, nandrolone, neratinib, nilotinib, nilutamide, nintedanib, niraparib, olaparib, olmutinib, orelabrutinib, osimertinib, pacritinib, palbociclib, pamidronate, pamiparib, panobinostat, pazopanib, peficitinib, pegaptanib, pemigatinib, pexidartinib, pidotimod, pomalidomide, ponatinib, pralsetinib, pyrotinib, quizartinib, radotinib, raloxifene, regorafenib, ribociclib, rintatolimod, ripretinib, rucaparib, ruxolitinib, savolitinib, selinexor, selpercatinib, selumetinib, sonidegib, sorafenib, sotorasib, sunitinib, surufatinib, talazoparib, tamoxifen, tazemetostat, temsirolimus, tepotinib, thalidomide, tipifarnib, tirabrutinib, tivozanib, trametinib, tofacitinib, toremifene, tretinoin, trilaciclib, triptorelin, tucatinib, upadacitinib, umbralisib, vandetanib, vemurafenib, venetoclax, vismodegib, vorinostat, zanubrutinib or zoledronic acid;
(2) the antitumor drug is a chemotherapy drug, and is selected from one or more of altretamine, aminoglutethimide, amsacrine, asparaginase, azacitidine, bendamustine, bexarotene, bicyclol, bleomycin, boanmycin, buserelin, busulfan, cabazitaxel, calaspargase, calicheamycin, capecitabine, carboplatin, carmustine, carmofur, cedazuidine, chlorambucil, cisplatin, cladribine, clofarabine, colchicine, cyclophosphamide, cytarabine, dacarbazine, dactinomycin, daunorubicin, decitabine, denileukin, deruxtecan, docetaxel, doxorubicin, epirubicin, eribulin, estradiol, estramustine, etoposide, exemestane, fasudil, floxuridine, fludarabine, fluorouracil, flutamide, formestane, gemcitabine, hydroxyurea, icaritin, idarubicin, ifosfamide, ingenol mebutate, irinotecan, ixabepilone, leucovorin, lomustine, lurbinctedin, maytansine, mechlorethamine, medroxyprogesterone, megestrol, melphalan, melphlan flufenamide, mercaptopurine, methotrexate, methoxsalen, methylprednisolone, mitomycin, mitotane, mitoxantrone, mitozolomide, monomethylauristatin E, monomethylauristatin F, nelarabine, nandrolone, nearsudil, octreotide, omacetaxine, oxaliplatin, paclitaxel, pamidronate, pemetrexed, pentetreotide, pentostatin, phenoxybenzamine, plinabulin, plitidepsin, porfimer, pralatrexate, prednisolone, procarbazine, procarbazine, raltitrexed, romidepsin, streptozocin, tegafur, temozolomide, teniposide, teprenone, thioguanine, thiotepa, thyrotropin alfa, tipiracil, tirbanibulin, topotecan, trabectedin, trifluride, utidelone, uroacitide, valrubicin, vinblastine, vincristine, vindesine, vinflunine, vinorelbine, amatoxins, anthacyclines, anthracenes, anthramycins, auristatins, bryostatins, camptothecins, carmaphycins, combretastatins, cyclosporines, cryptomycins, ecteinascidins, ellipticenes, esperamicins, mustines, neothramycins, ozogamicins, phenoxazines, podophyllotoxins, pyrrolobenzodiazepines, sibiromycins, thailanstatins, tomamycns, tubulysins, taxanes, vinca alkaloids, 7-epipaclitaxel, 2'-acetylpaclitaxel, 10-deacetylpaclitaxel, 7-epi-10-deacetyltaxol, 7-xylosyltaxol, 10-deacetyl-7-glutarylpaclitaxel, 7-*N, N*-dimethylglycylpaclitaxel, 7-L-alanylacetaxel, larotaxel, camptothecin, 9-aminocamptothecin, 9-nitrocamptothecin, lurtotecan, gimatecan, belotecan, 10-hydroxycamptothecin, 10-hydroxy-7-ethyl-camptothecin (SN-38), exatecan, pirarubicin, aclacinomycin, sirolimus, tacrolimus, progesterone, estrogen, rapamycin, mithramycin, harringtonine or curcumin.

**25.** The poly(ε-L-lysine) derivative-drug conjugate according to claim 1, 4 or 5, wherein the pharmaceutically active agent residue D is selected from the following structures:

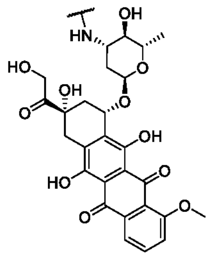
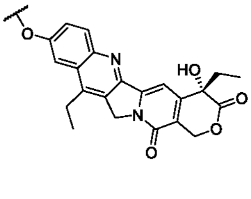
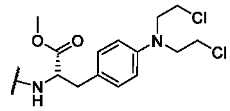
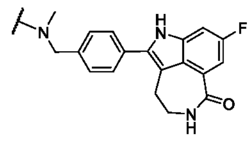
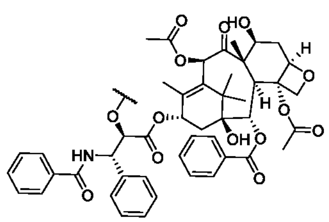
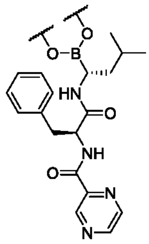
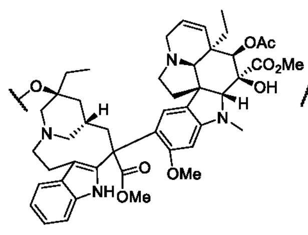
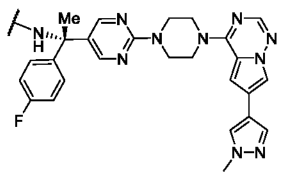
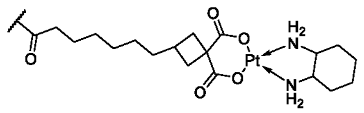
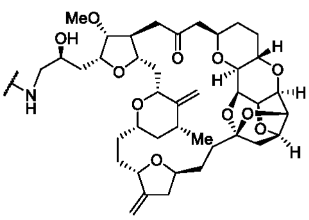
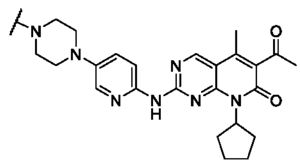
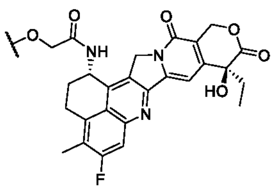
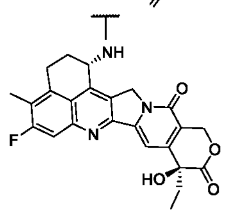
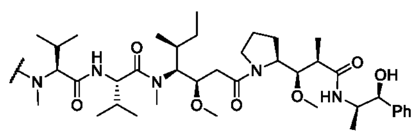

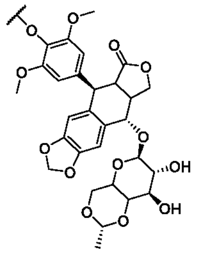

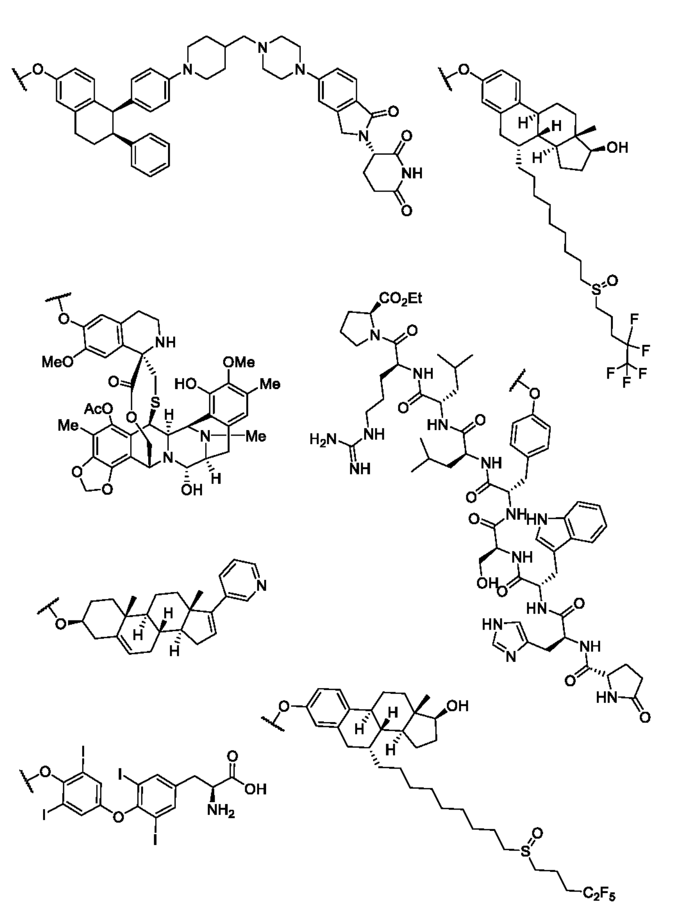

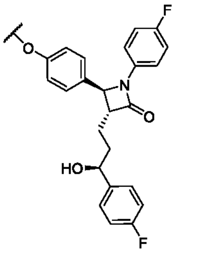

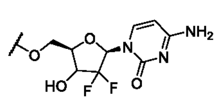

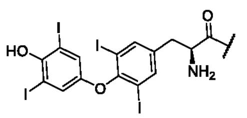

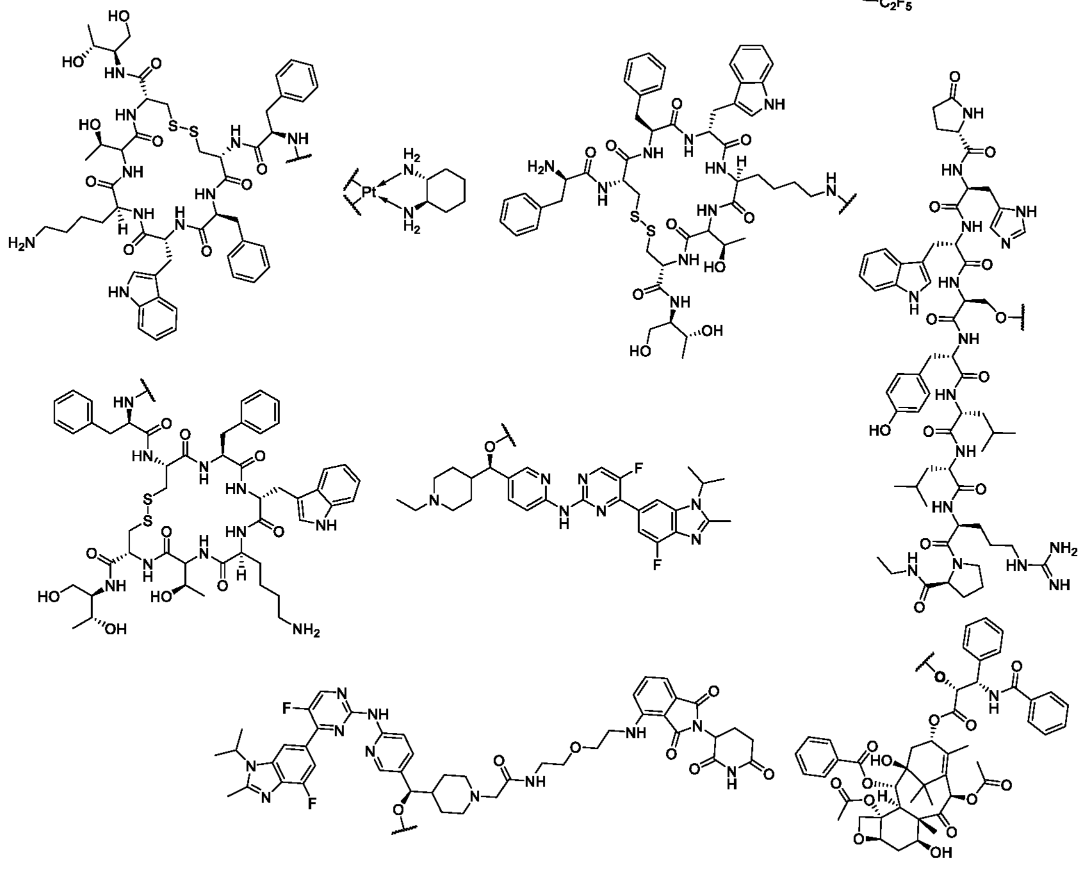

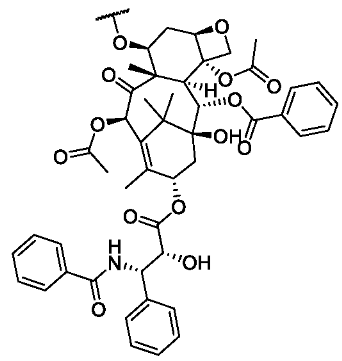
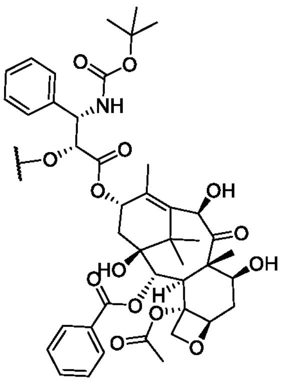
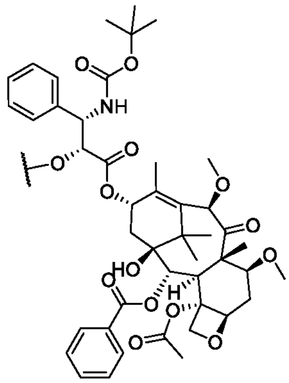

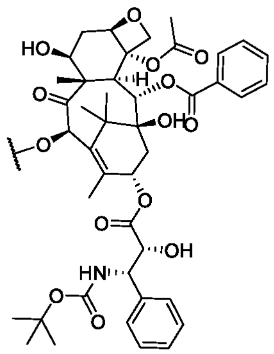
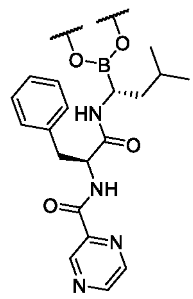
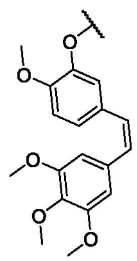
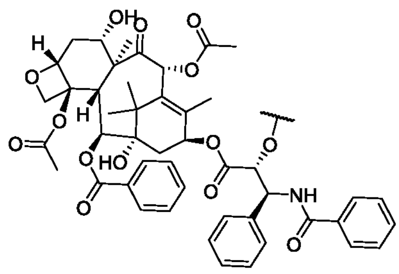

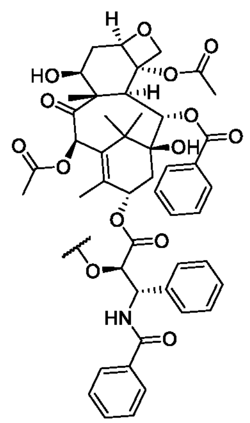
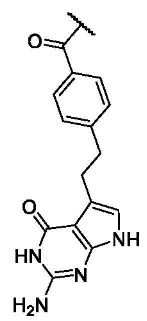
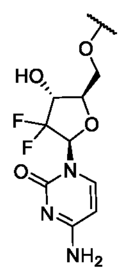
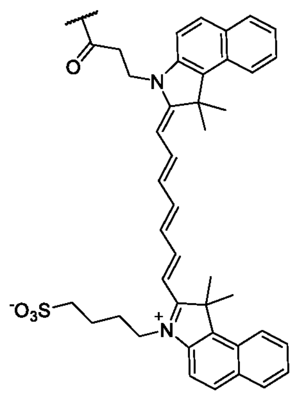

**26.** The poly(ε-L-lysine) derivative-drug conjugate according to claim 1, 4 or 5, wherein the poly(ε-L-lysine) derivative-drug conjugate is selected from the following structures:

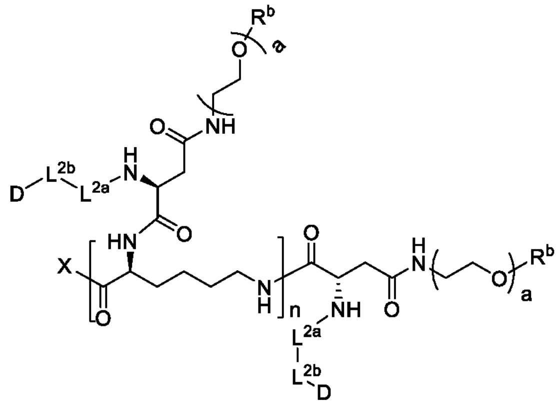

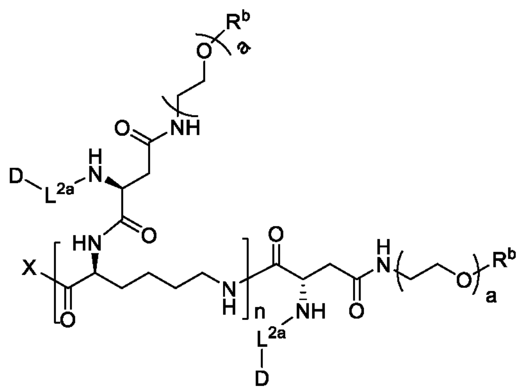

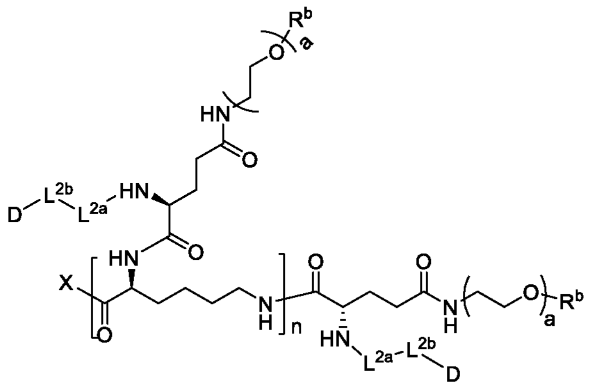

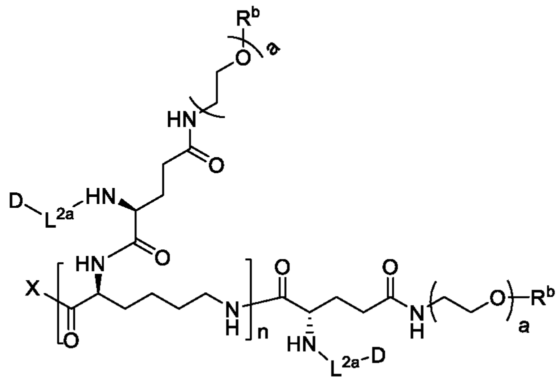

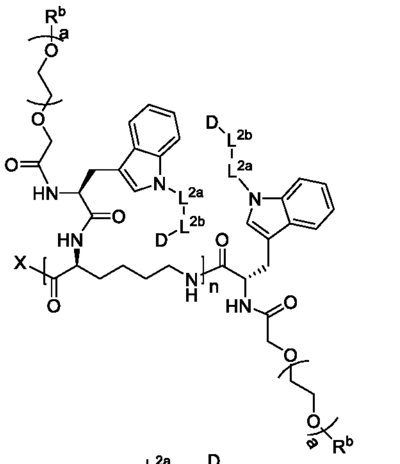

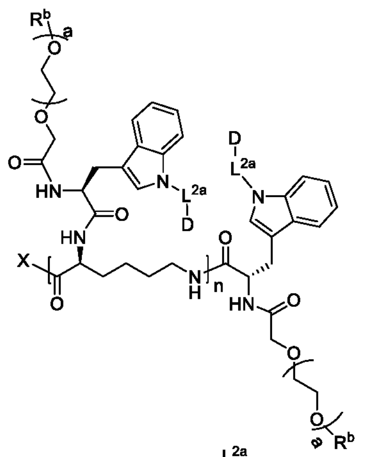

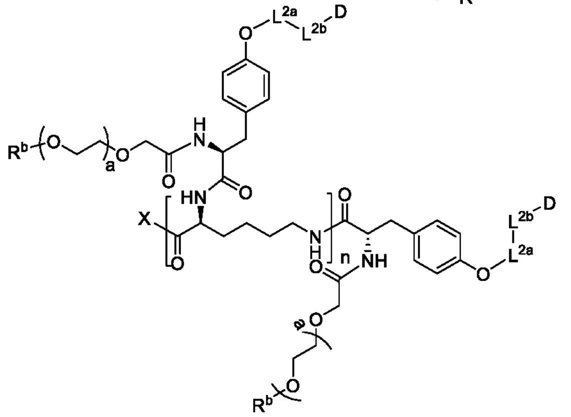

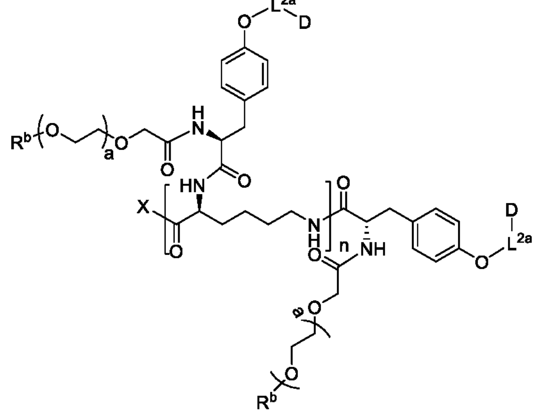

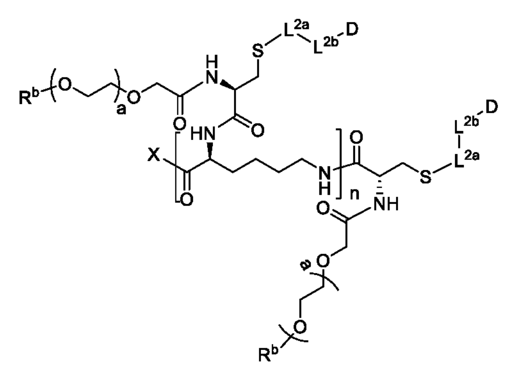

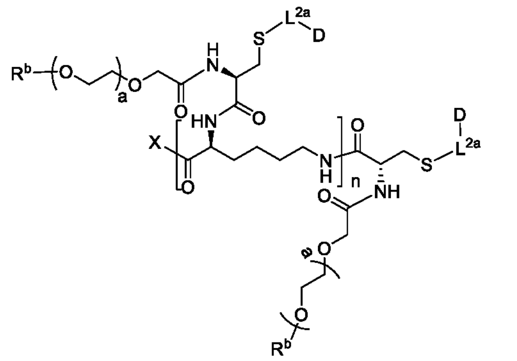

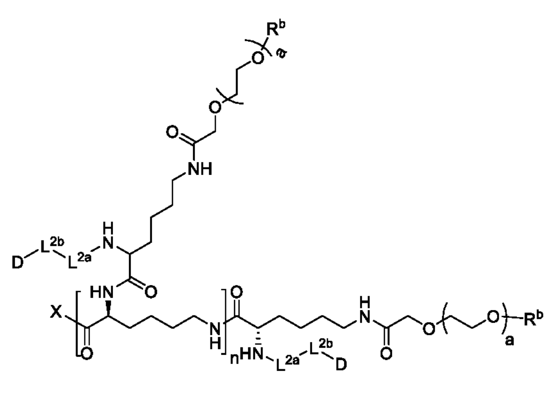

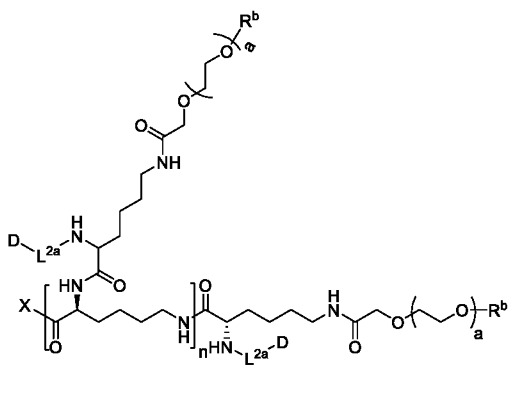

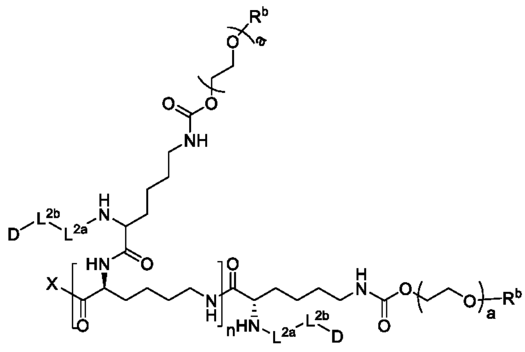

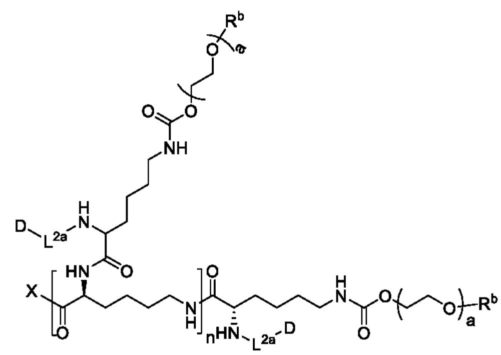

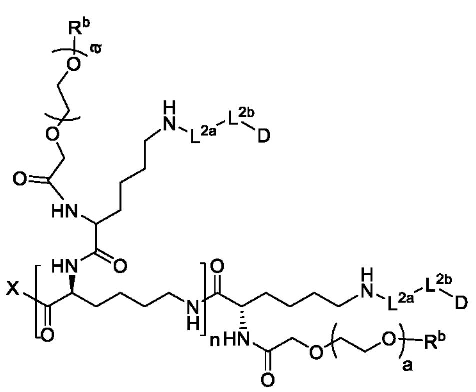

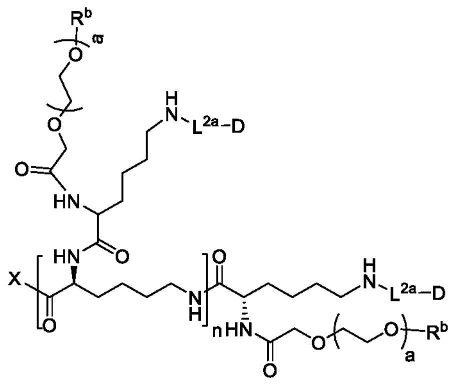

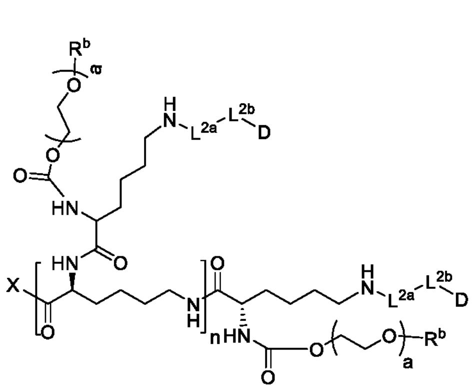

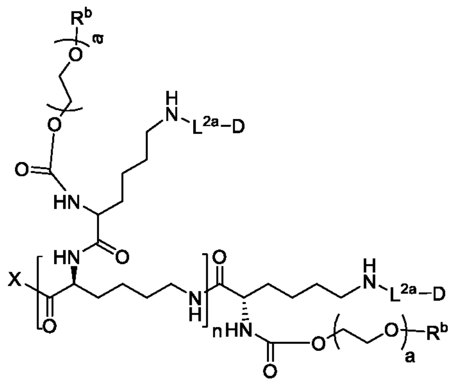

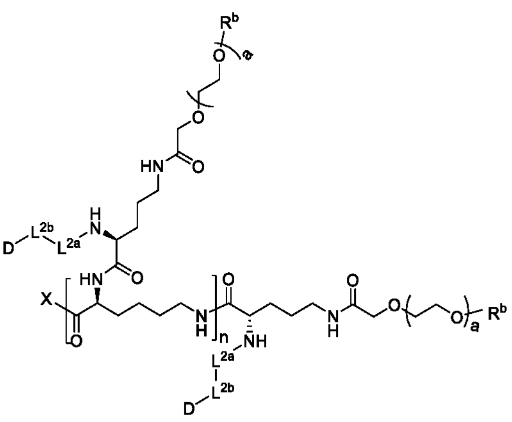

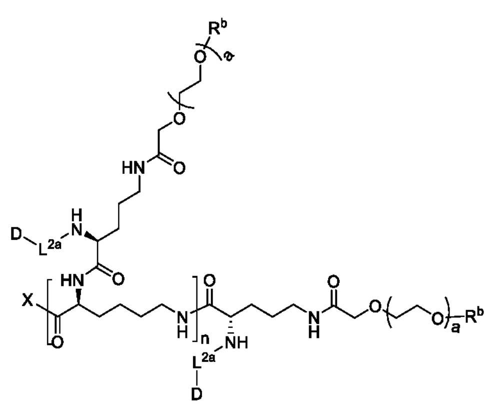

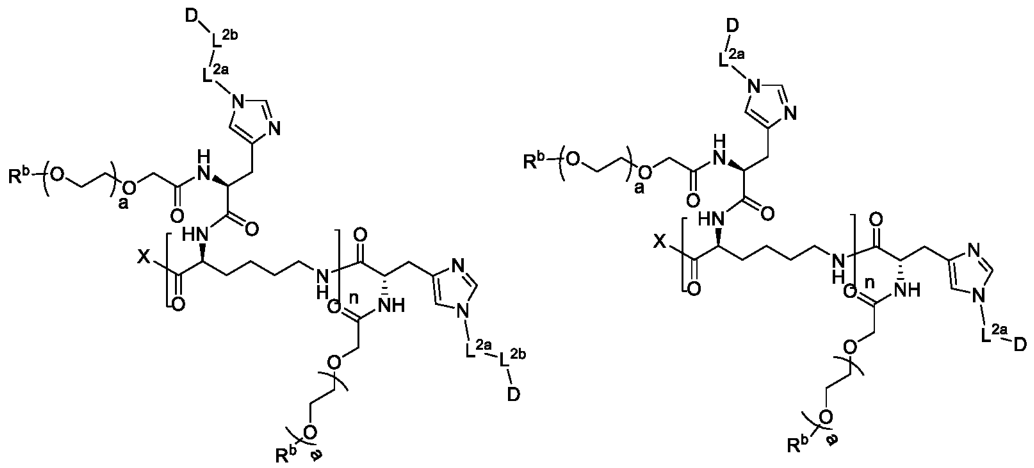

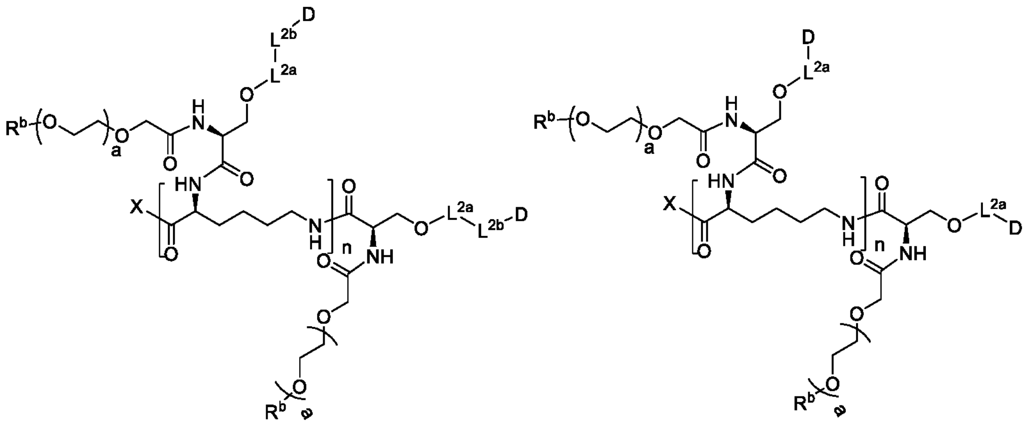

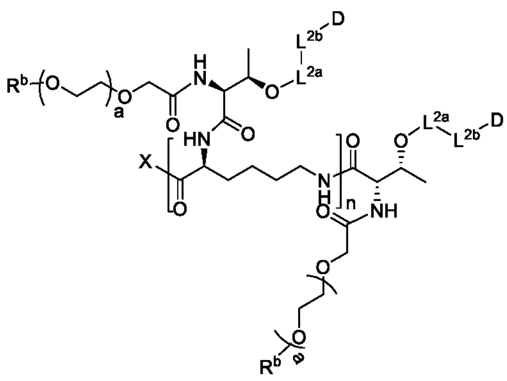

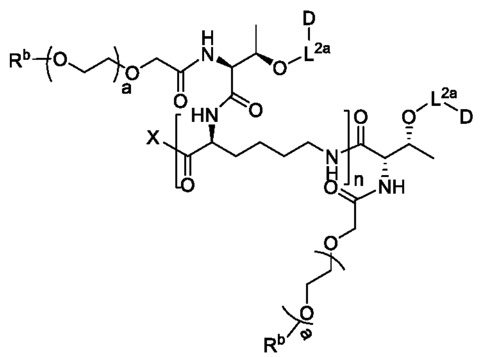

wherein $L^{2a}$ and $L^{2b}$ are each defined as described in any of claims 1 and 4-16;
D is defined as described in any of claims 1 and 20-25;
X is defined as described in any of claims 1 and 7;
$R^b$ is defined as described in claim 17;
n is an integer of 10 to 70;
a is an integer of 5 to 150.

**27.** The poly(ε-L-lysine) derivative-drug conjugate according to any of claims 1 and 4-5, wherein the poly(ε-L-lysine) derivative-drug conjugate is selected from the following structures:

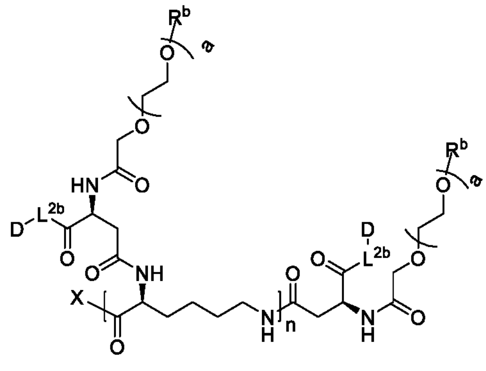

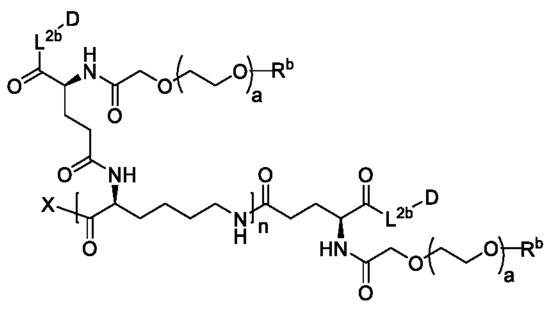

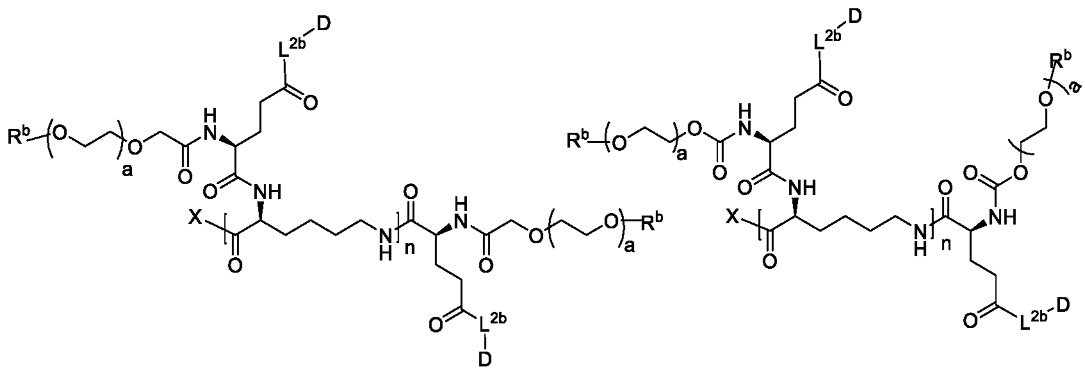

wherein,

$L^{2a}$ and $L^{2b}$ are each defined as described in any of claims 1 and 4-16;
D is defined as described in any of claims 1 and 20-24;
X is defined as described in any of claims 1 and 7;
$R^b$ is defined as described in claim 17;
n is an integer of 10 to 70;
a is an integer of 5 to 150.

**28.** The poly(ε-L-lysine) derivative-drug conjugate according to claim 26 or 27, wherein $R^b$ is methyl, the number-average molecular weight of the PEG residue is 550, 1000, 2000, 3000, 4000 or 5000.

**29.** The poly(ε-L-lysine) derivative-drug conjugate according to claim 26 or 27, wherein the X is -OH,

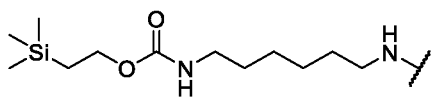,
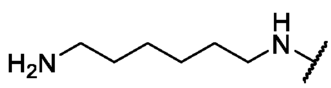

or -$L^{2b}$-D; the - $L^{2b}$-D is selected from:

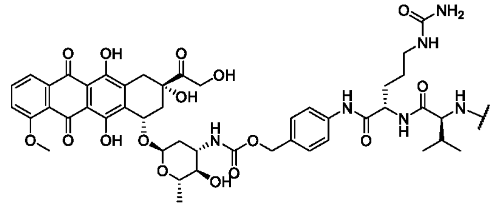

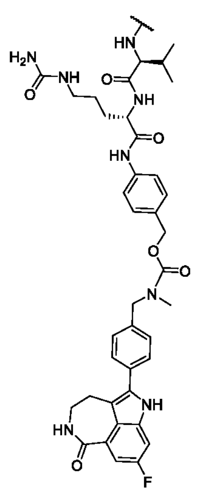

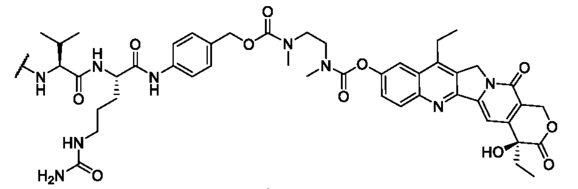

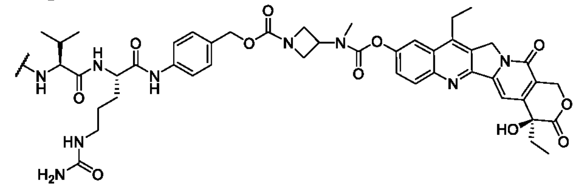

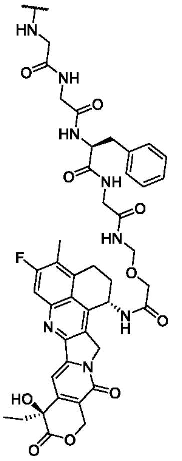

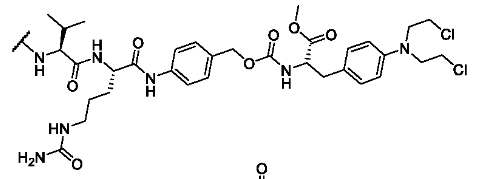

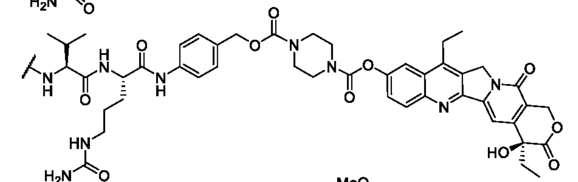

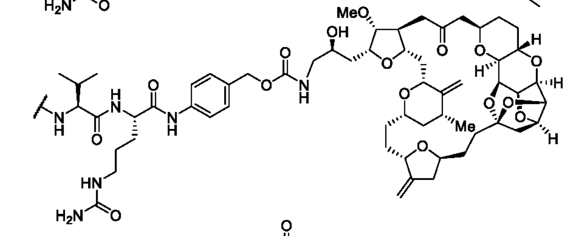

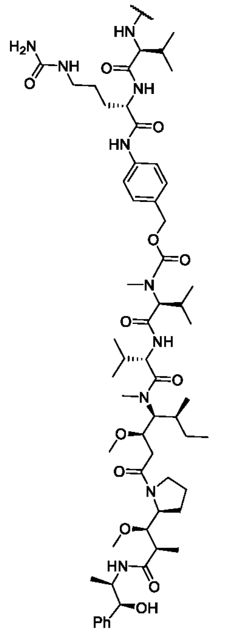

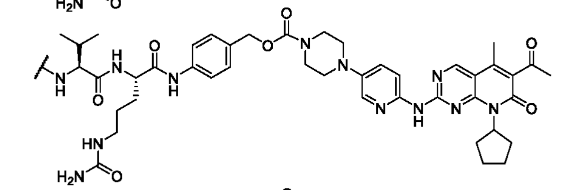

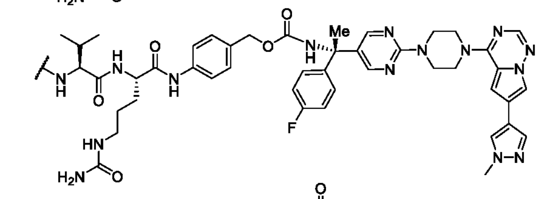

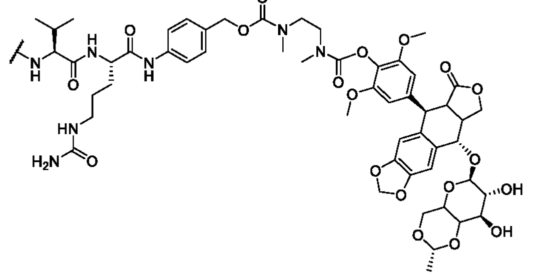

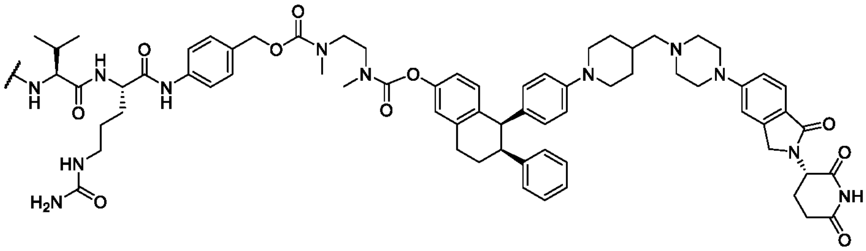

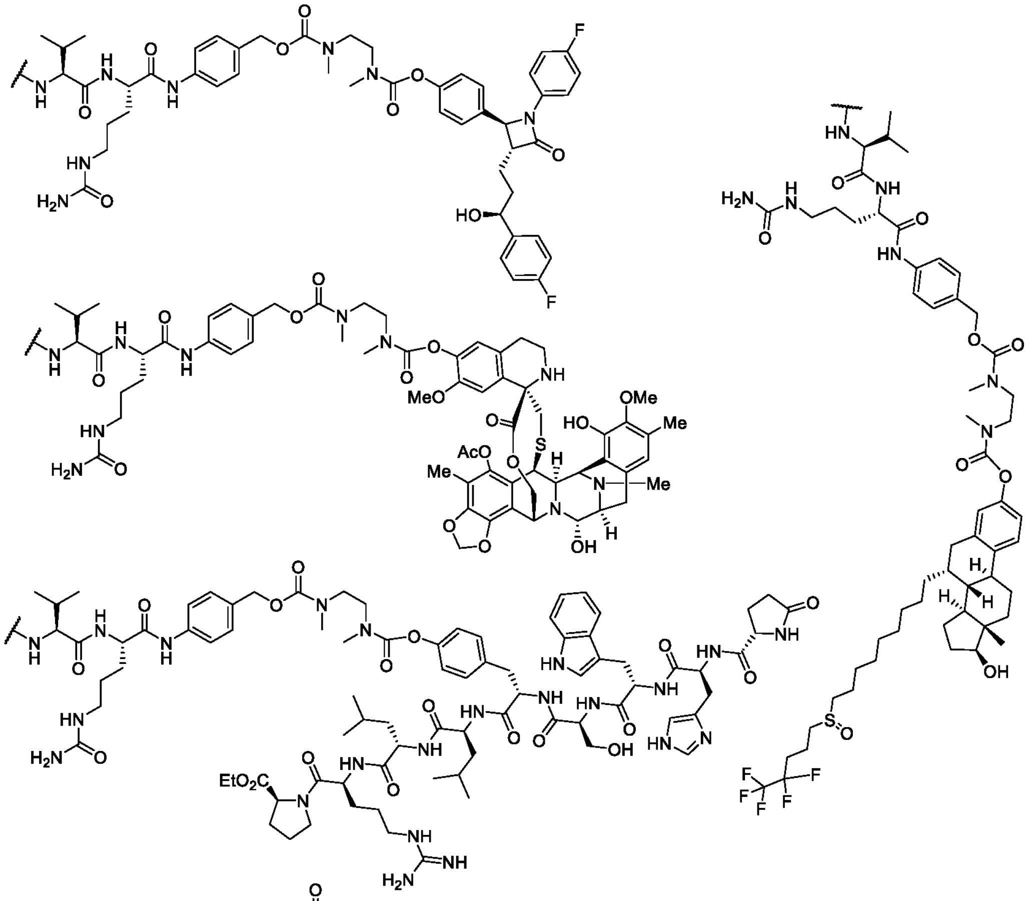

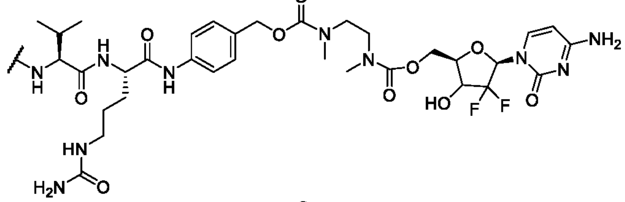

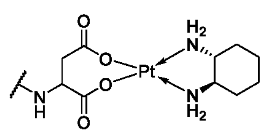

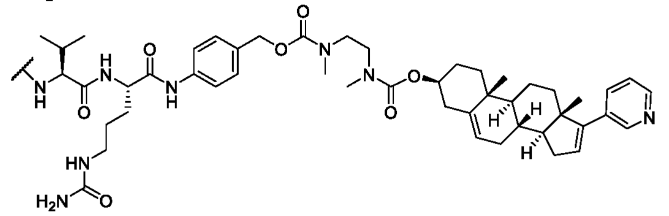

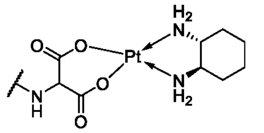

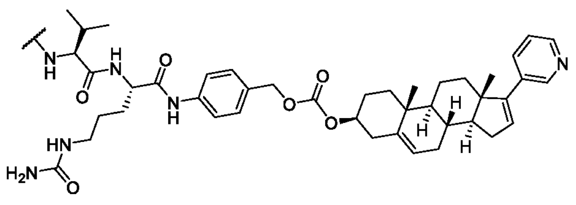

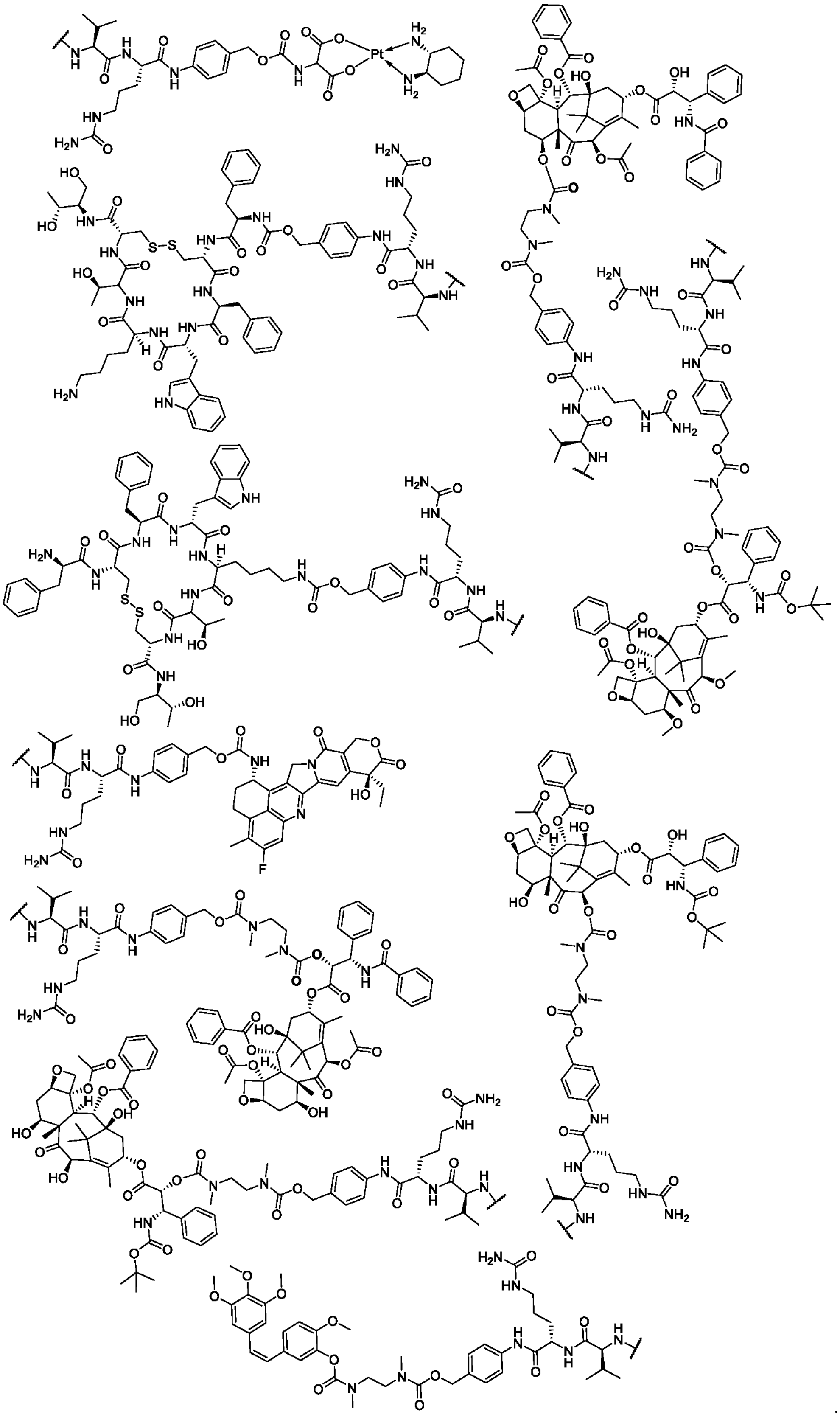

**30.** The poly(ε-L-lysine) derivative-drug conjugate according to claim 26 or 27, wherein $L^{2b}$ is an enzyme-responsive linker or a pH-responsive linker.

**31.** The poly(ε-L-lysine) derivative-drug conjugate according to claim 26 or 27, wherein D is a chemotherapy drug residue, and the chemotherapy drug is selected from one or more of amatoxins, anthacyclines, anthracenes, anthramycins, auristatins, bryostatins, camptothecins, carmaphycins, combretastatins, cyclosporines, cryptomycins, ecteinascidins, ellipticenes, esperamicins, maytansines, mustines, neothramycins, ozogamicins, phenoxazines, platinum complexes, podophyllotoxins, pyrrolobenzodiazepines, sibiromycins, thailanstatins, tomamycns, tubulysins, taxanes or vinca alkaloids.

**32.** The poly(ε-L-lysine) derivative-drug conjugate according to claim 31, wherein the chemotherapy drug is selected from one or more of bleomycin, boanmycin, cabazitaxel, calicheamycin, carboplatin, cisplatin, dactinomycin, daunorubicin, deruxtecan, docetaxel, doxorubicin, epirubicin, eribulin, etoposide, idarubicin, irinotecan, ixabepilone, lurbinctedin, maytansinol, monomethylauristatin E, monomethylauristatin F, mitomycin, oxaliplatin, paclitaxel, streptozocin, teniposide, topotecan, trabectedin, valrubicin, vinblastine, vincristine, vindesine, vinflunine, vinorelbine, 7-epipaclitaxel, 2'-acetylpaclitaxel, 10-deacetylpaclitaxel, 7-epi-10-deacetyltaxol, 7-xylosyltaxol, 10-deacetyl-7-glutarylpaclitaxel, *7-N, N*-dimethylglycylpaclitaxel, 7-L-alanylacetaxel, larotaxel, camptothecin, 9-aminocamptothecin, 9-nitrocamptothecin, lurtotecan, gimatecan, belotecan, 10-hydroxycamptothecin, 10-hydroxy-7-ethyl-camptothecin (SN-38), exatecan, pirarubicin, aclacinomycin or mithramycin.

**33.** The poly(ε-L-lysine) derivative-drug conjugate according to any of claims 1, 4-5 and 26-27, wherein the poly(ε-L-lysine) derivative-drug conjugate fulfills one or more of the following:

(1) the branched center Y and the poly(ε-L-lysine) residue are linked by the linker $L^0$, and the molar ratio of the branched center Y to the poly(ε-L-lysine) structural unit is 0.5:1-1.5:1;
(2) the pharmacokinetic regulator residue P and the branched center Y are linked by the linker $L^1$, and the molar ratio of the pharmacokinetic regulator residue P to the poly(ε-L-lysine) structural unit is 0.5:1~1.5:1;
(3) the pharmaceutically active agent residue D and the trifunctional branched center Y are linked by the linker $L^2$, and the molar ratio of the pharmaceutically active agent residue D to the poly(ε-L-lysine) structural unit is 0.5:1-1.5:1.

**34.** The poly(ε-L-lysine) derivative-drug conjugate according to claim 33, wherein the poly(ε-L-lysine) derivative-drug conjugate fulfills one or more of the following:

(1) the branched center Y and the poly(ε-L-lysine) residue are linked by the linker $L^0$, and the molar ratio of the branched center Y to the poly(ε-L-lysine) structural unit is 0.8:1-1.2:1;
(2) the pharmacokinetic regulator residue P and the trifunctional branched center Y are linked by the linker $L^1$, and the molar ratio of the pharmacokinetic regulator residue P to the poly(ε-L-lysine) structural unit is 0.8:1-1.2:1;
(3) the pharmaceutically active agent residue D and the trifunctional branched center Y are linked by the linker $L^2$, and the molar ratio of the pharmaceutically active agent residue D to the poly(ε-L-lysine) structural unit is 0.8:1~1.2:1.

**35.** The poly(ε-L-lysine) derivative-drug conjugate according to any of claims 1, 4-5 and 26-27, wherein the poly(ε-L-lysine) derivative-drug conjugate is used for the prevention and treatment of tumors, inflammation, diabetes, central nervous system diseases, cardiovascular diseases, psychiatric diseases, respiratory diseases, ophthalmic diseases, pain, obesity and other diseases.

**36.** The poly(ε-L-lysine) derivative-drug conjugate according to claim 35, wherein the poly(ε-L-lysine) derivative-drug conjugate is used for the prevention and treatment of cancer; the cancer comprises breast cancer, ovarian cancer, prostate cancer, melanin cancer, brain cancer, nasopharyngeal cancer, esophageal cancer, stomach cancer, liver cancer, pancreatic cancer, colorectal cancer, lung cancer, renal cancer, skin cancer, glioblastoma, neuroblastoma, sarcoma, liposarcoma, osteochondroma, bone cancer, osteosarcoma, seminoma, testicular tumor, uterine tumor, head and neck tumor, multiple myeloma, malignant lymphoma, polycythemia vera, leukemia, thyroid tumor, ureteral tumor, bladder tumor, gallbladder cancer, cholangiocarcinoma, chorionic epithelial or pediatric tumor.

**37.** The poly(ε-L-lysine) derivative-drug conjugate according to claim 1, wherein

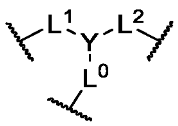

is

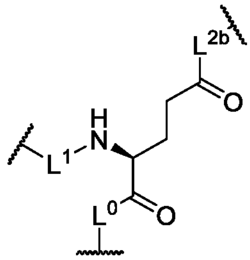

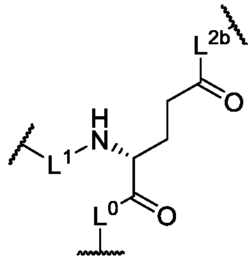

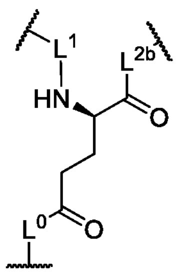

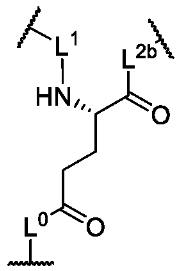

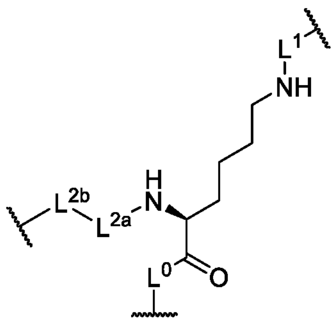

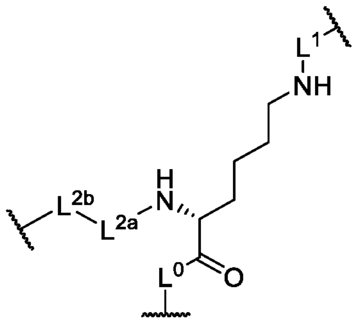

,

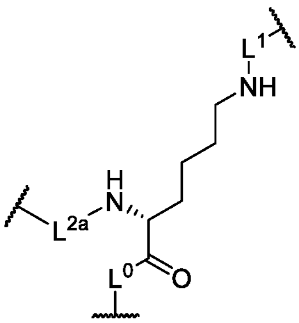

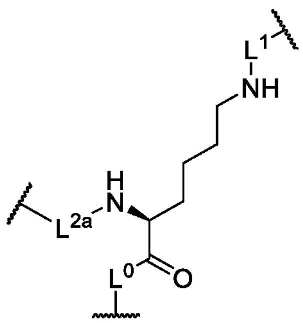

,

such as

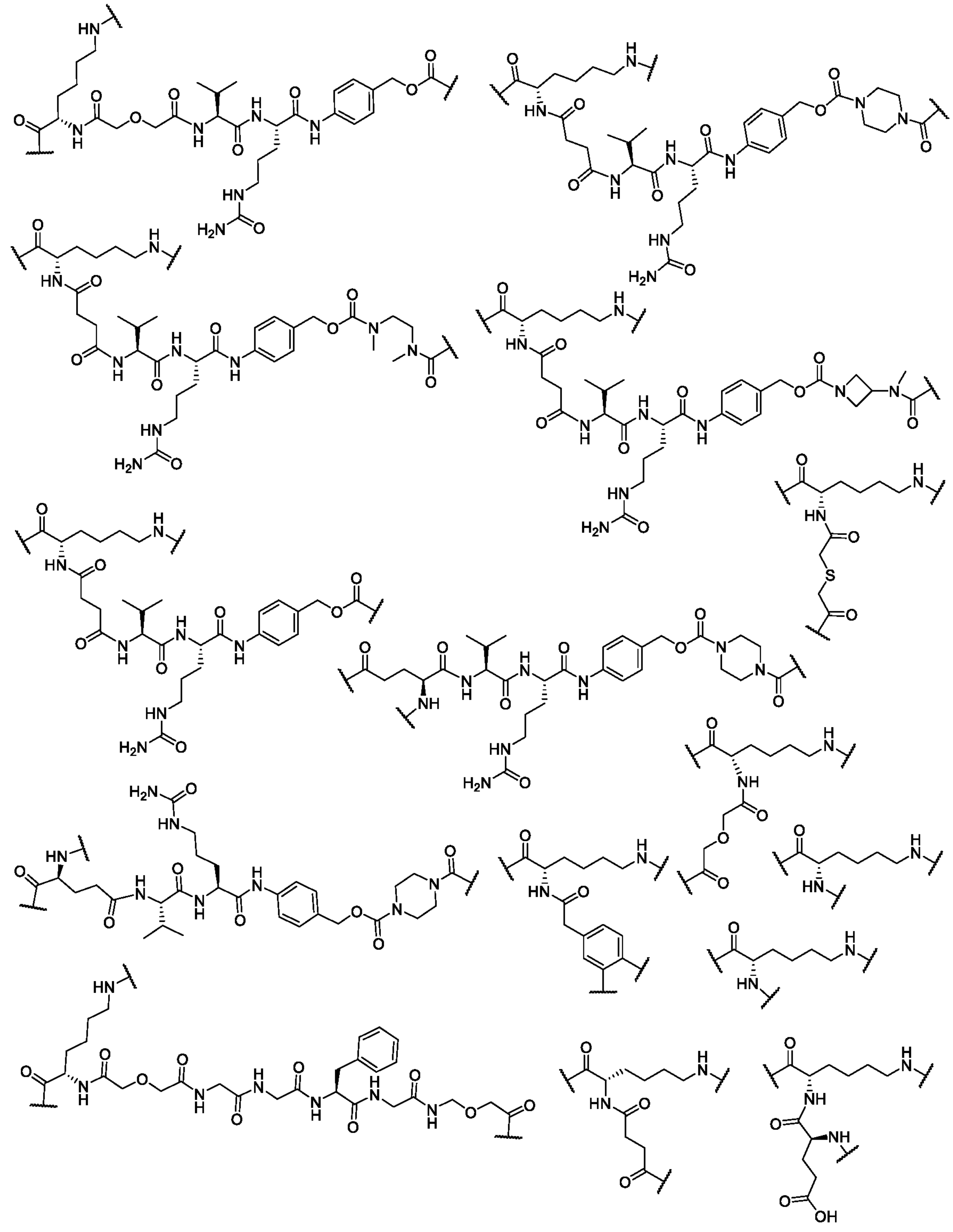

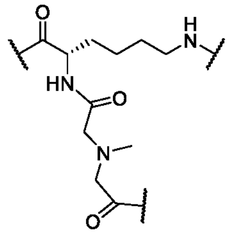

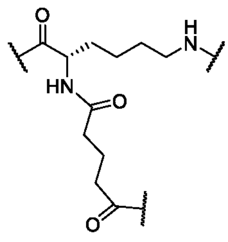

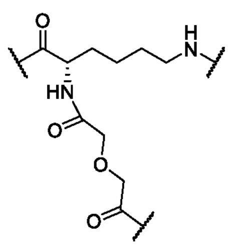

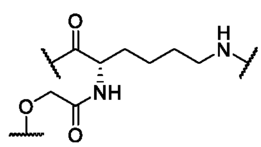

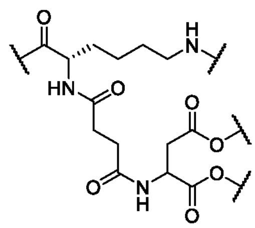
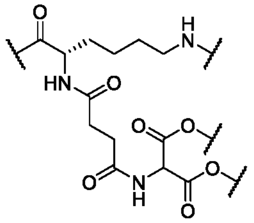
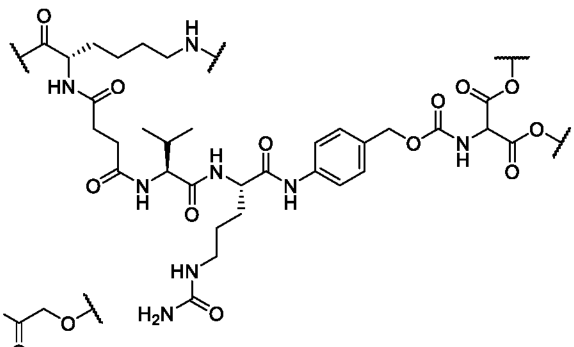
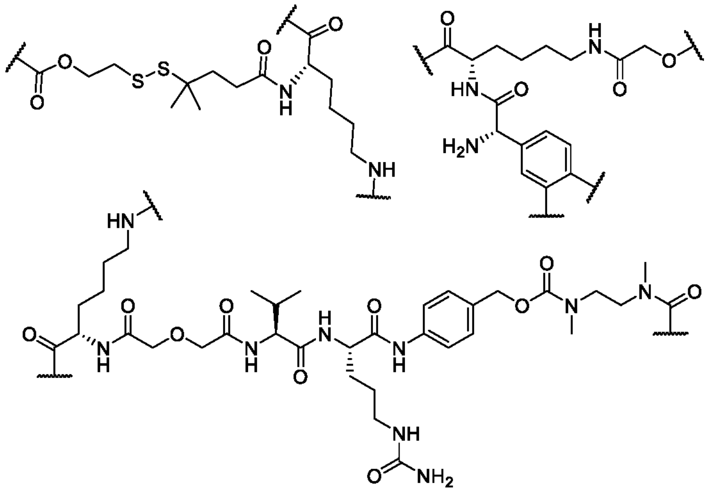
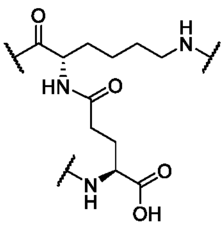

.

**38.** The poly(ε-L-lysine) derivative-drug conjugate according to any of claims 1-27, wherein $-L^2-D$ is any of the following structures:

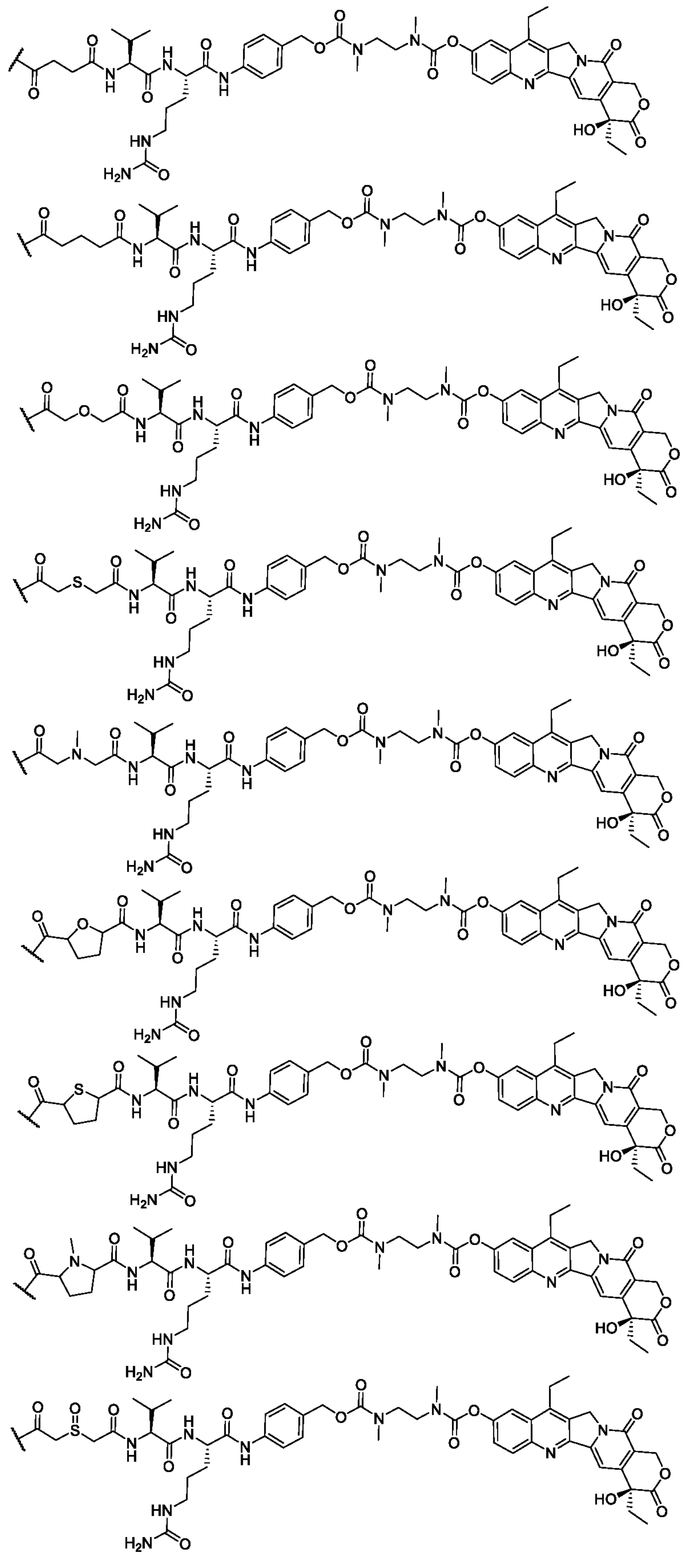

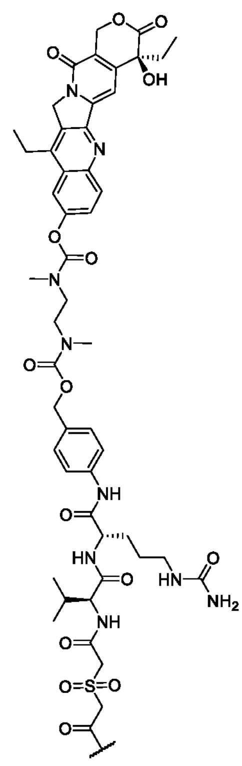

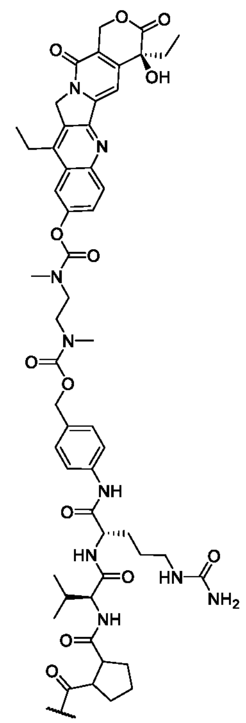

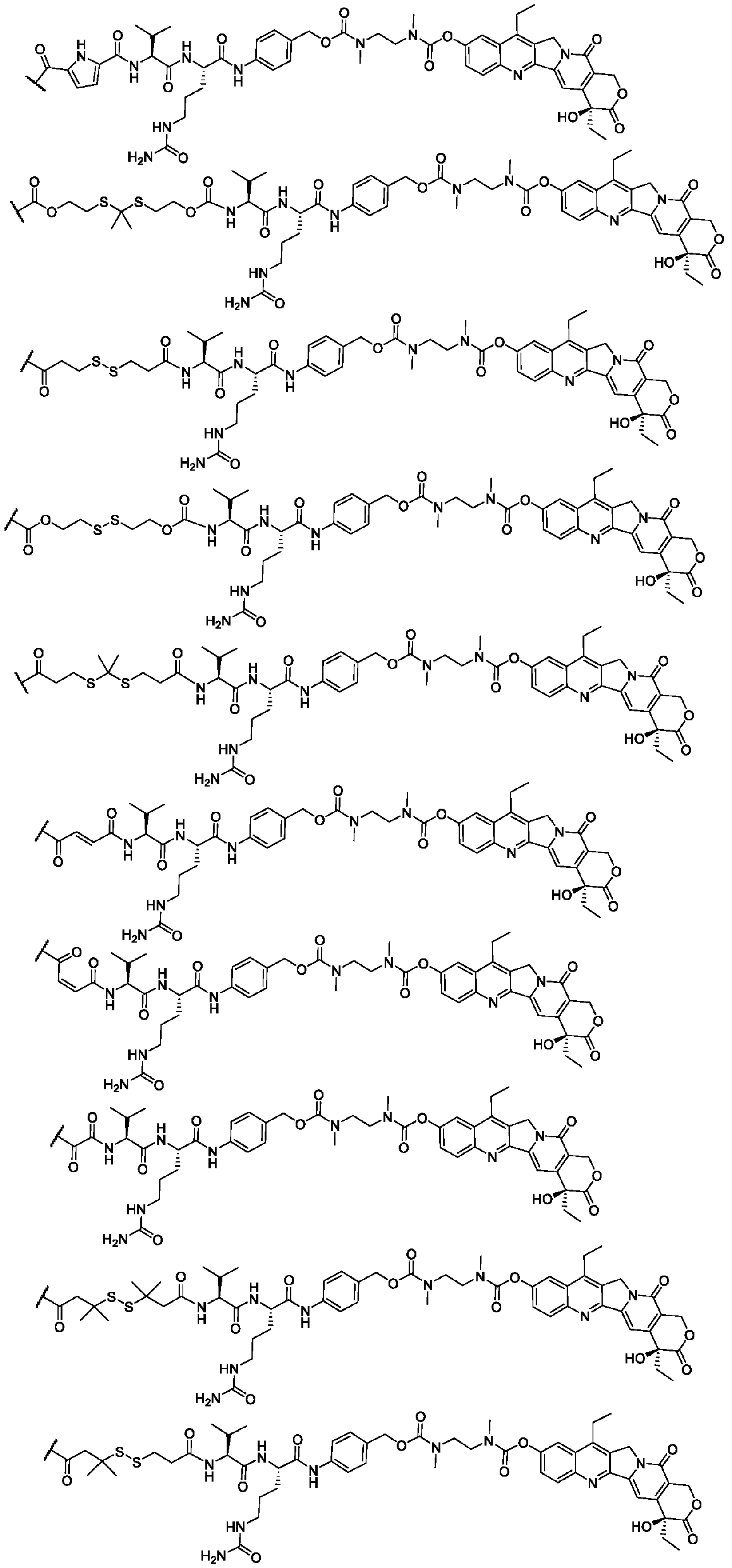

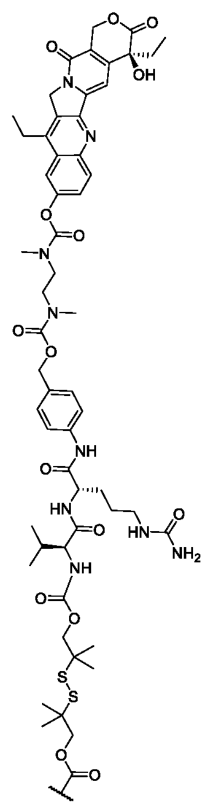

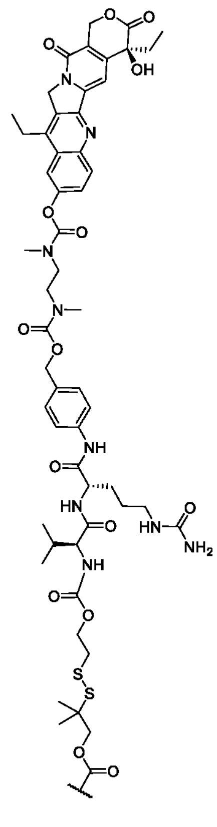

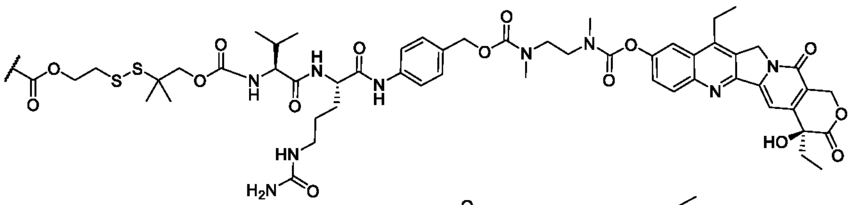

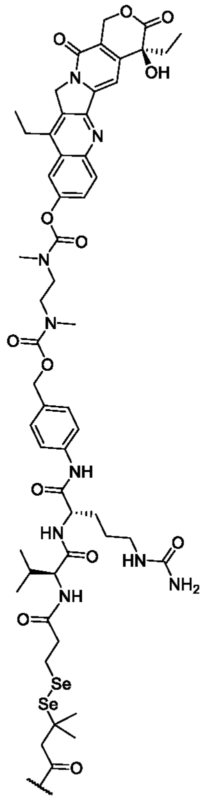

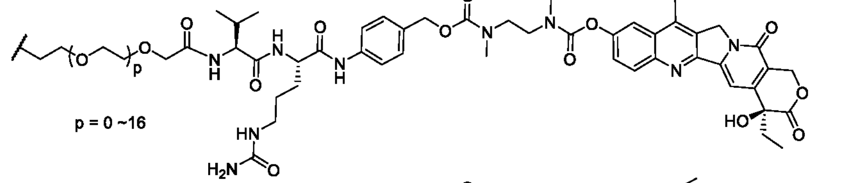

p = 0 ~16

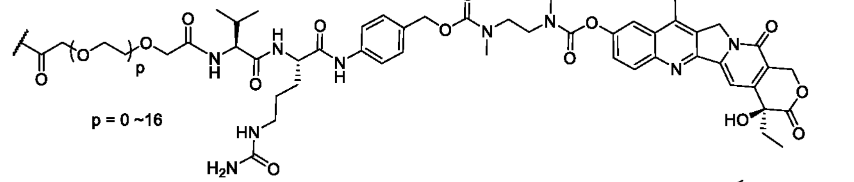

p = 0 ~16

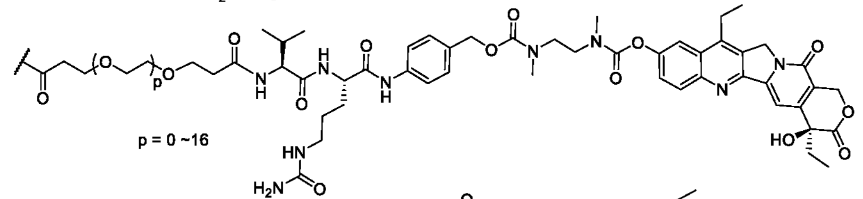

p = 0 ~16

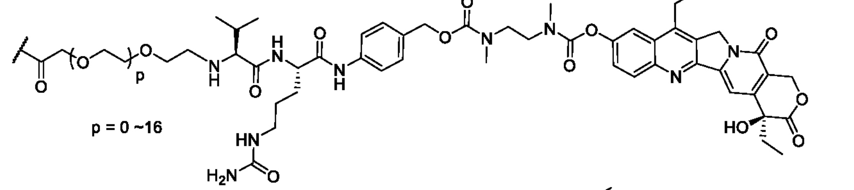

p = 0 ~16

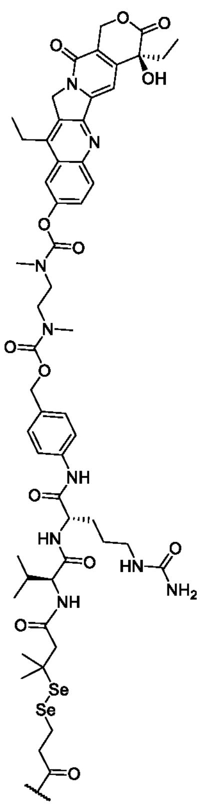

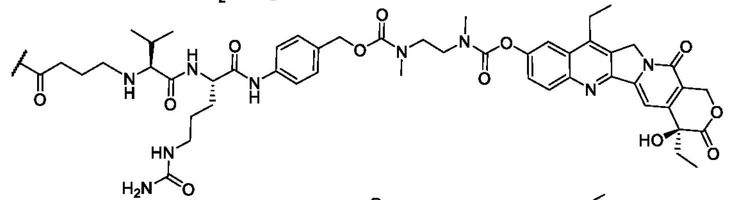

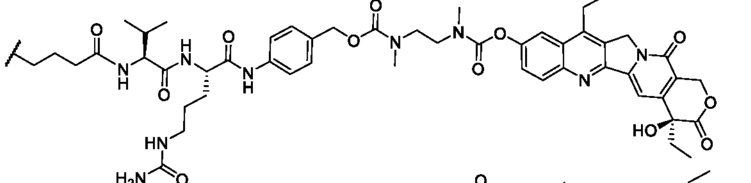

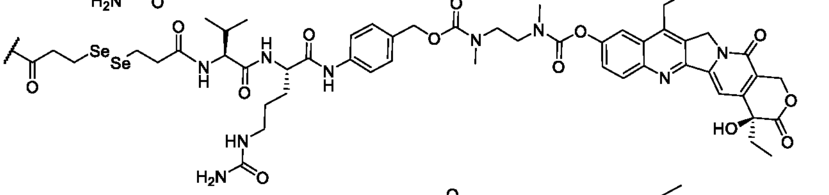

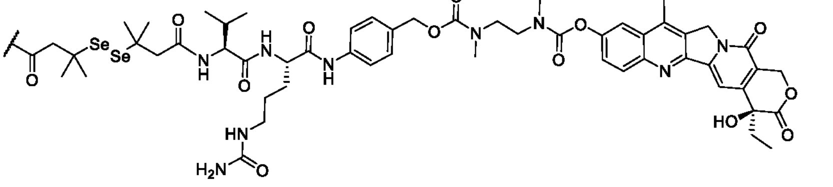

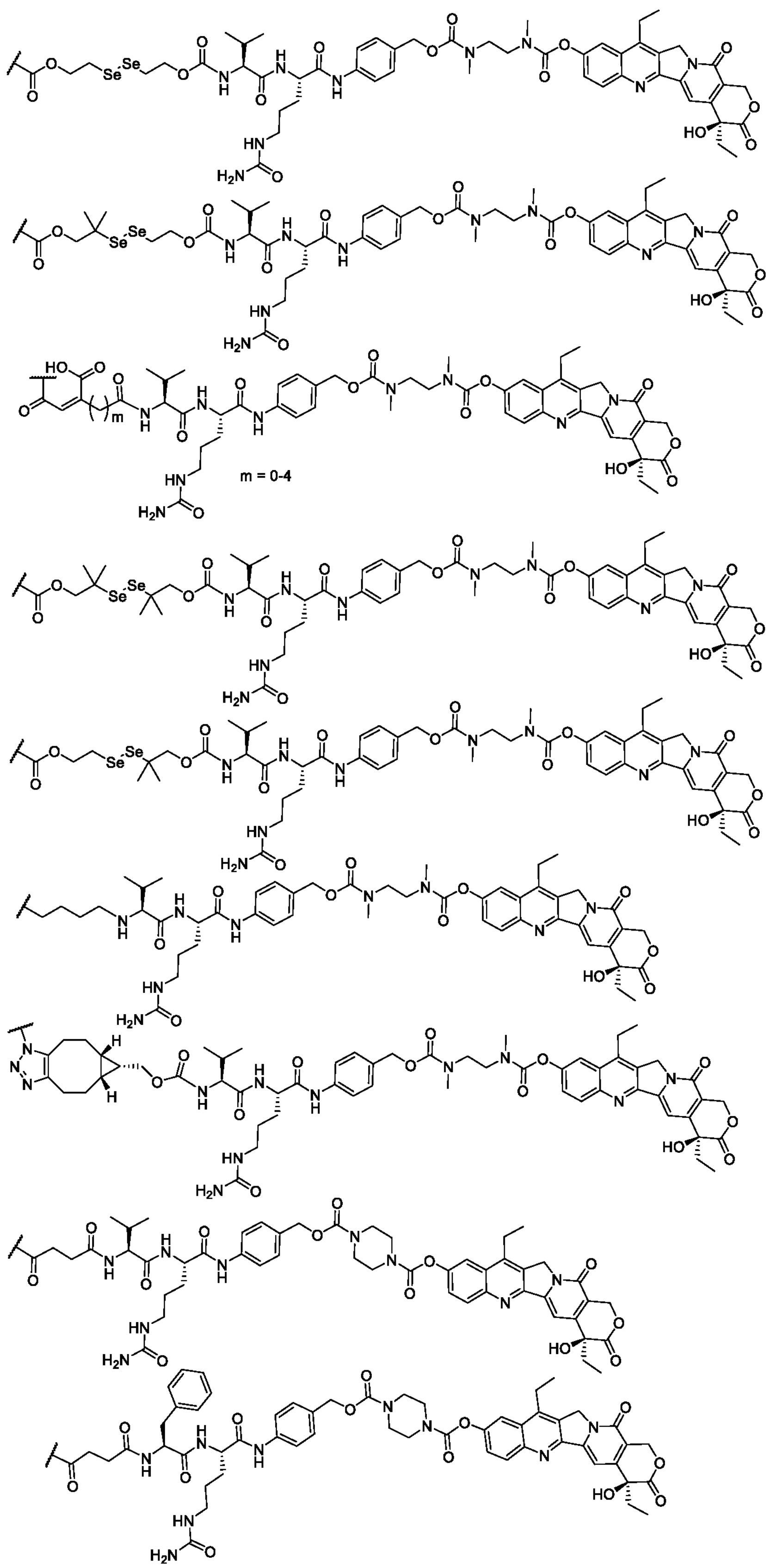

m = 0-4

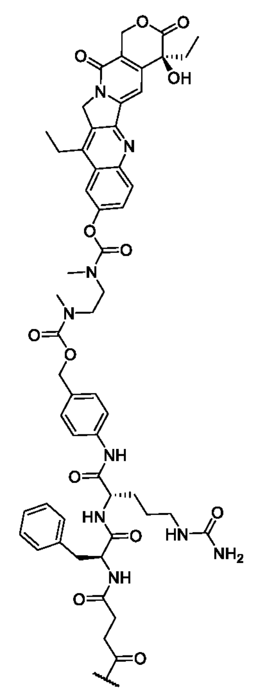

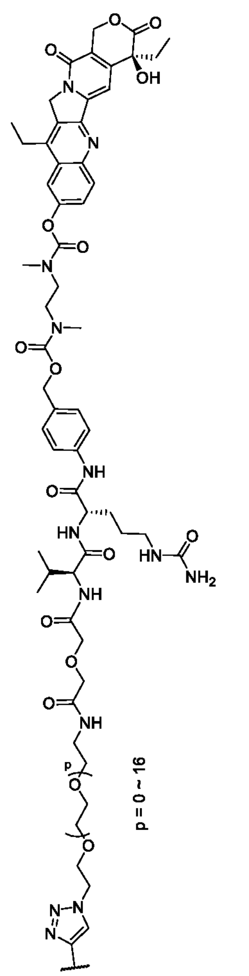

p = 0 ~ 16

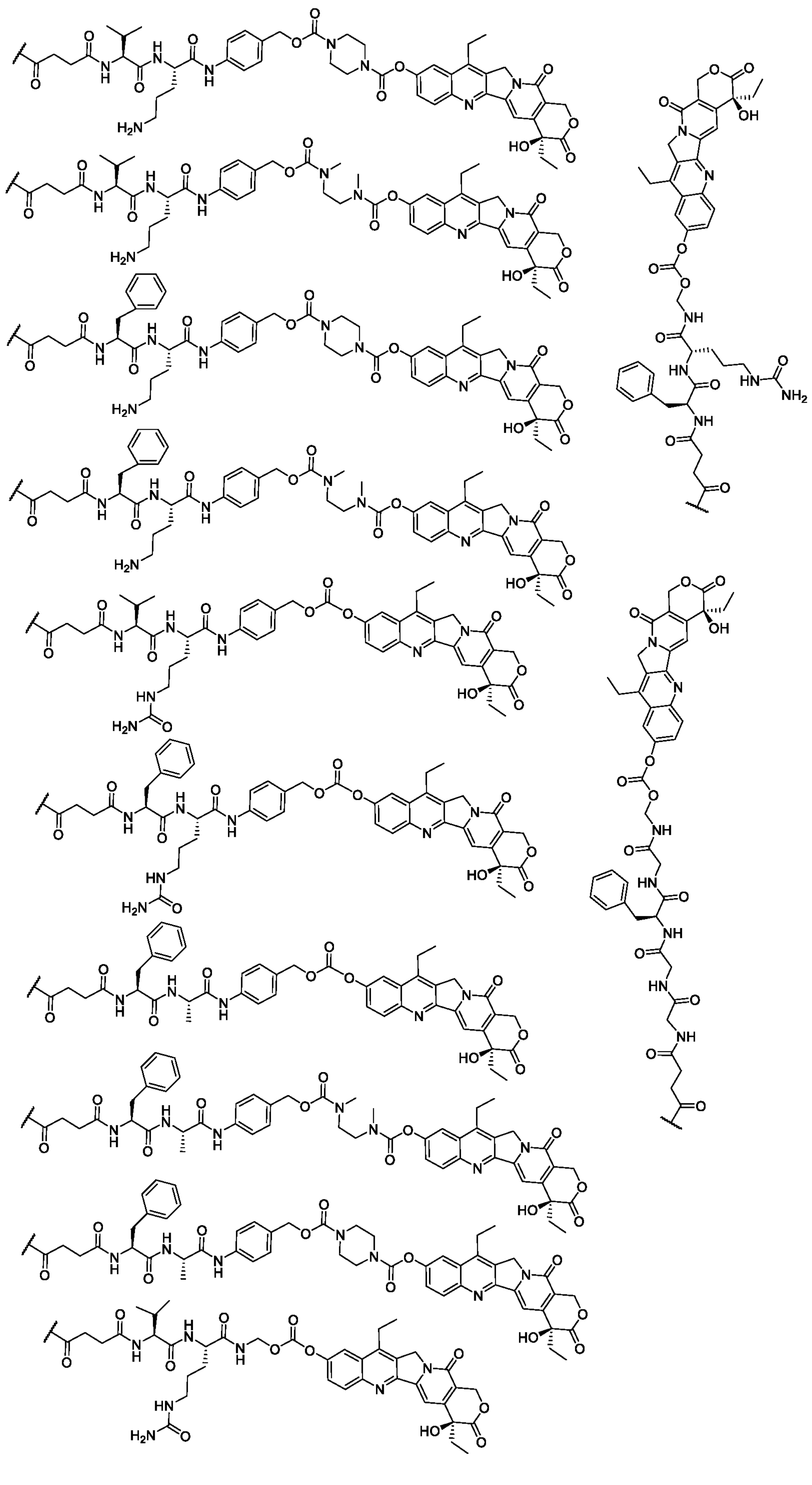

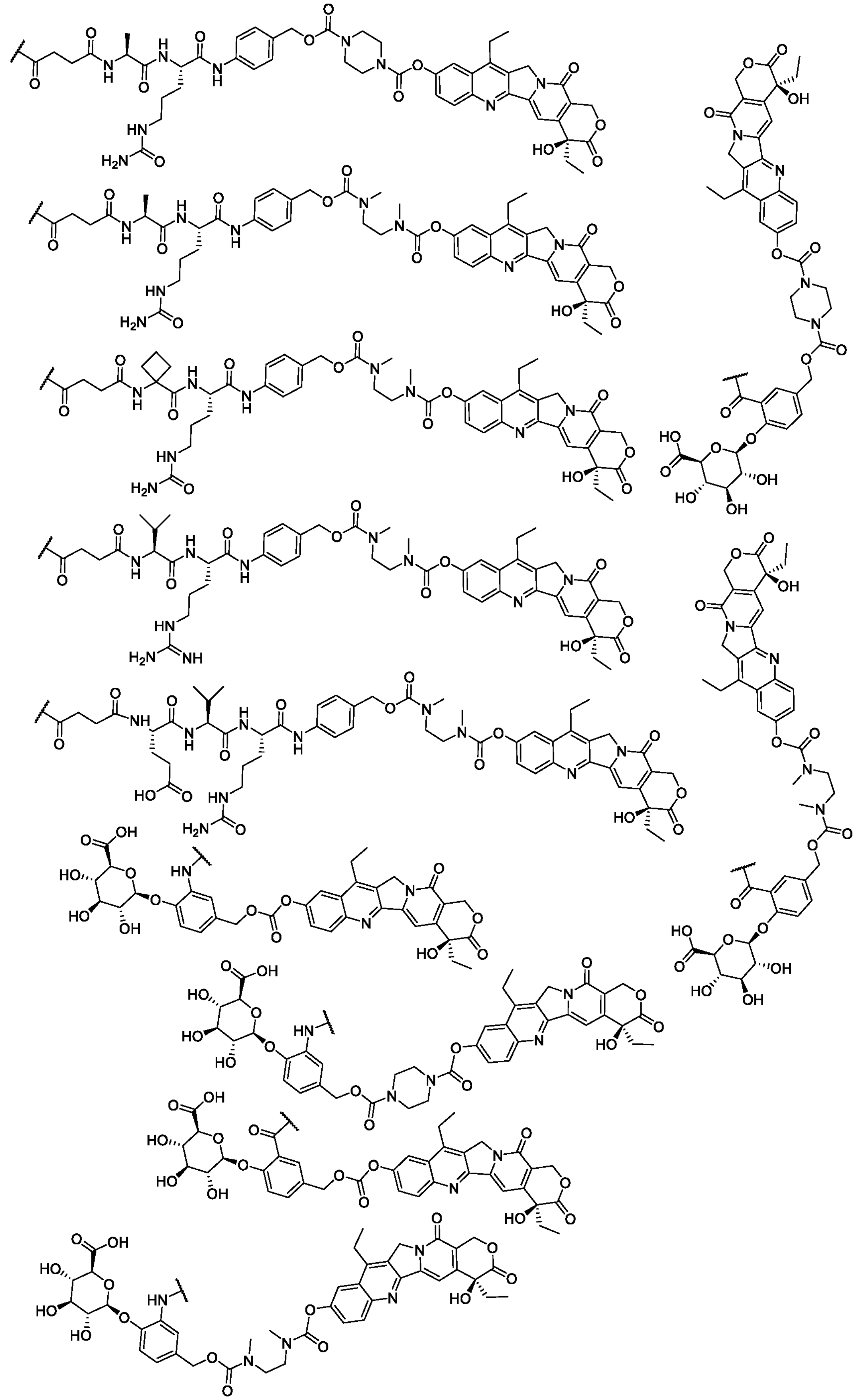

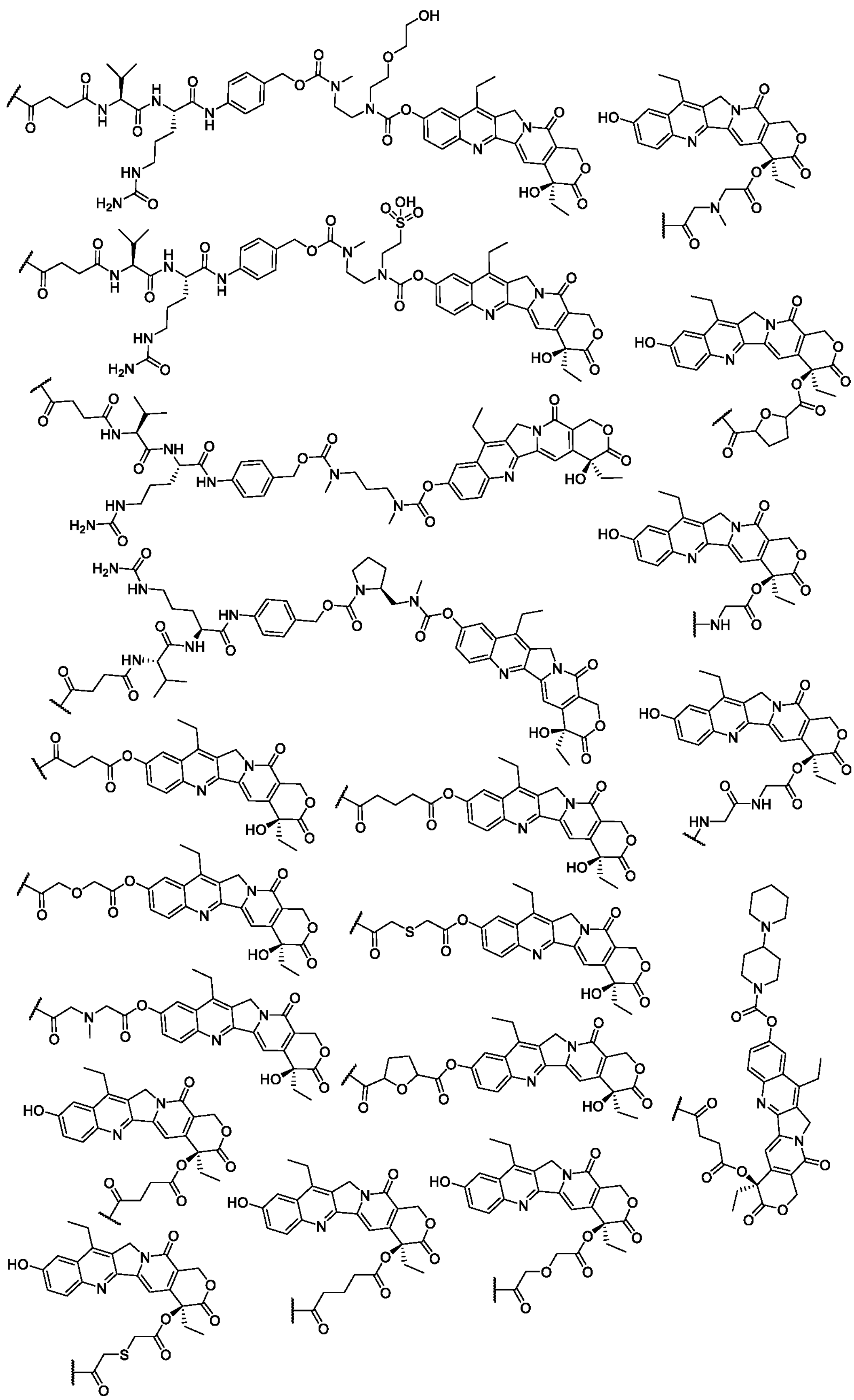

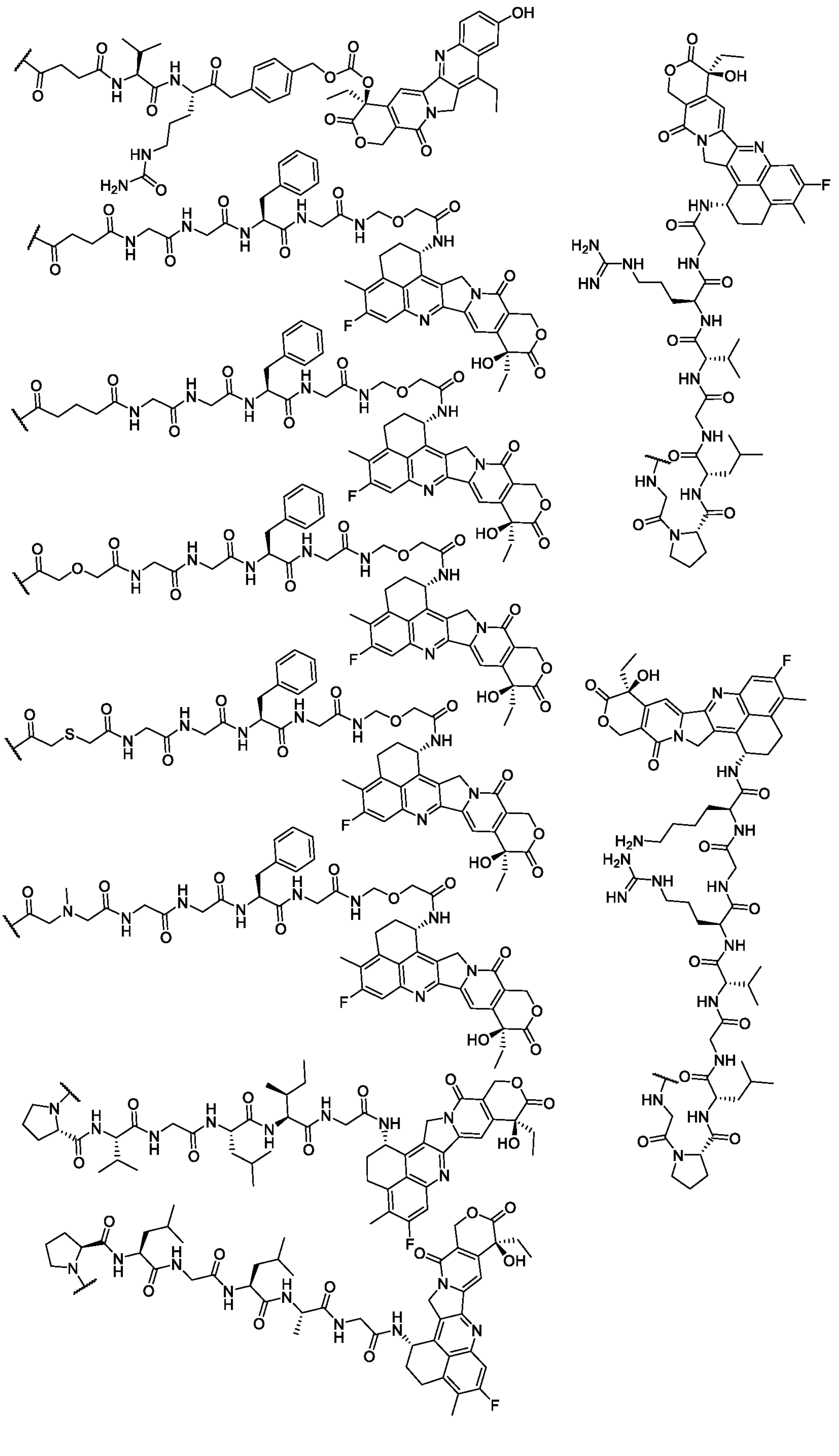

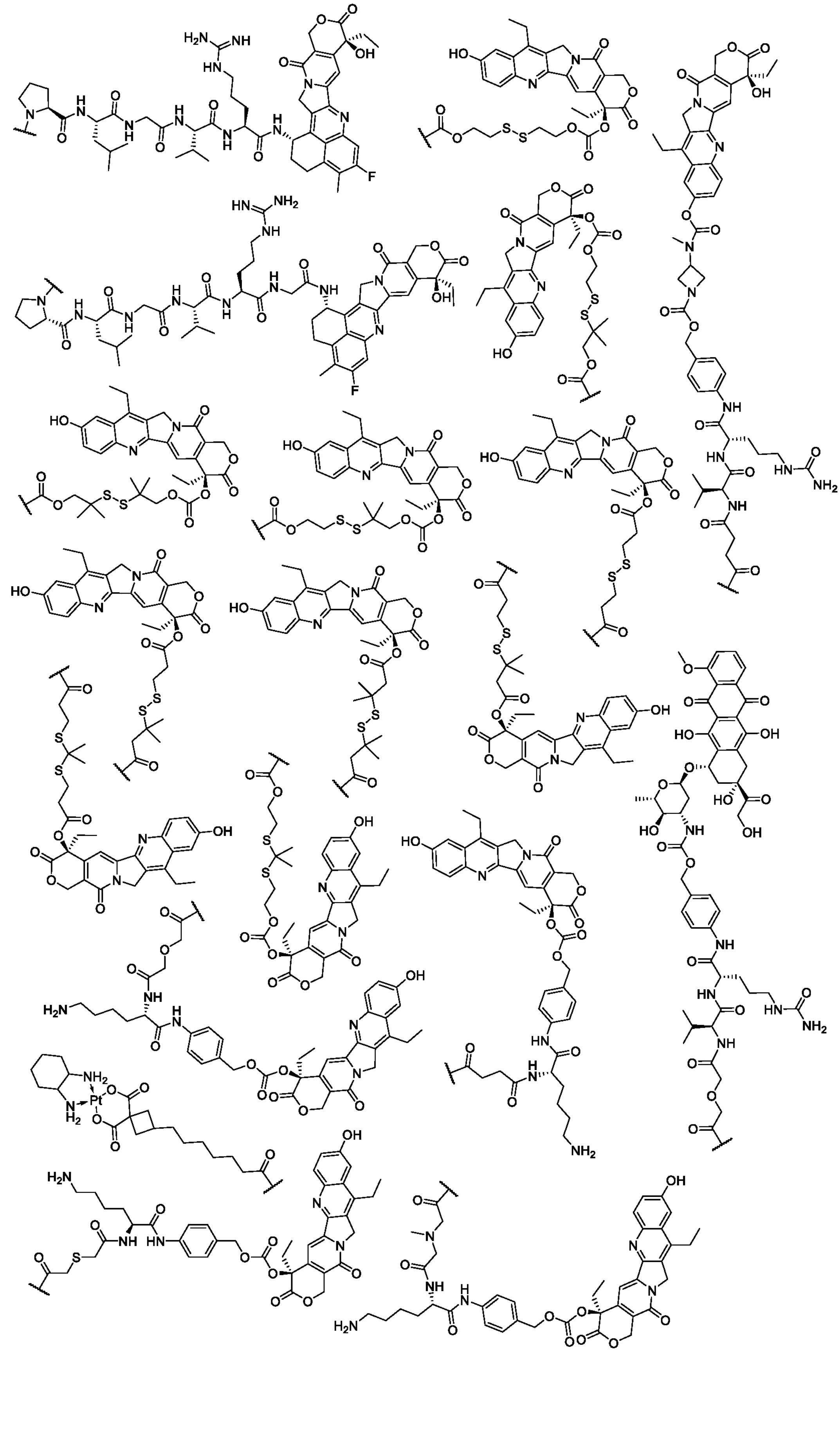

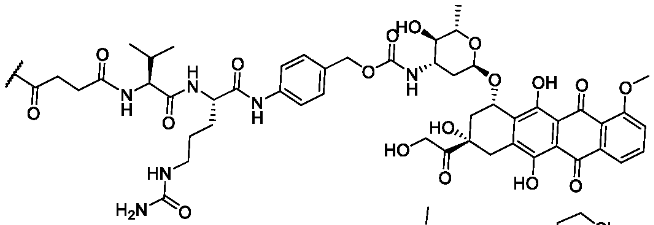

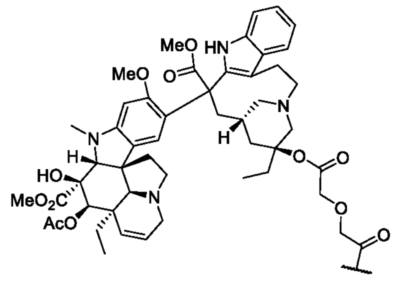

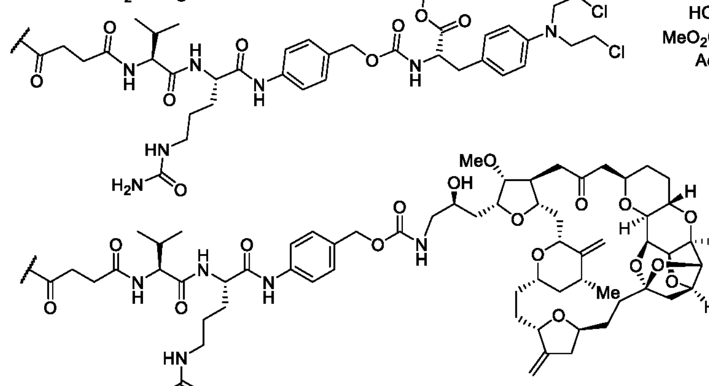

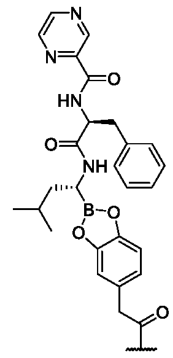

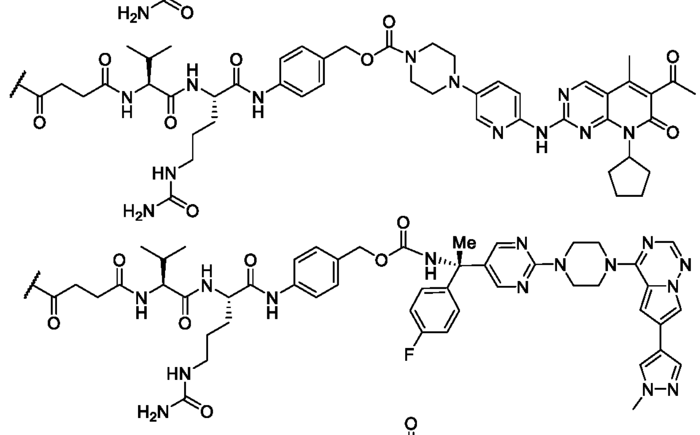

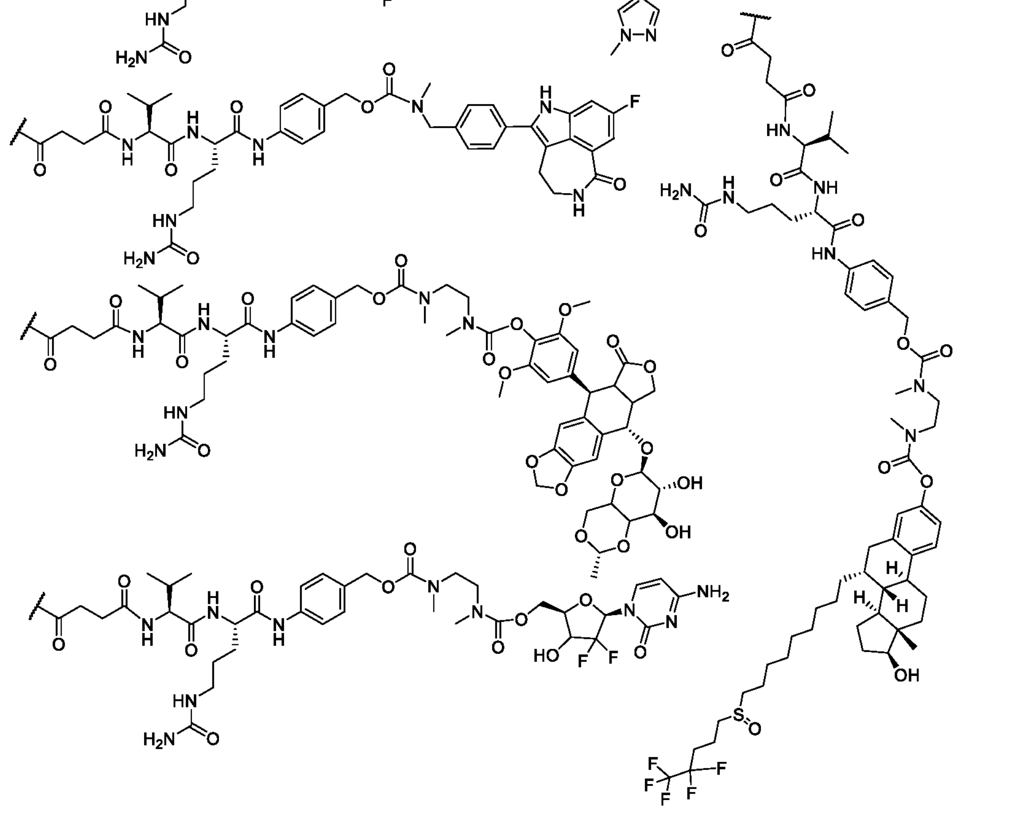

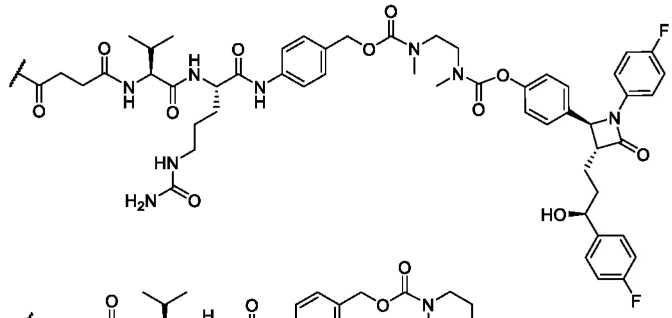

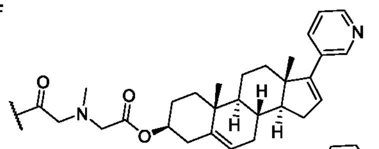

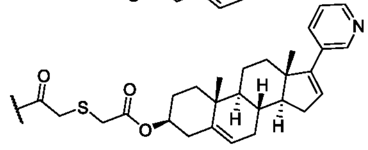

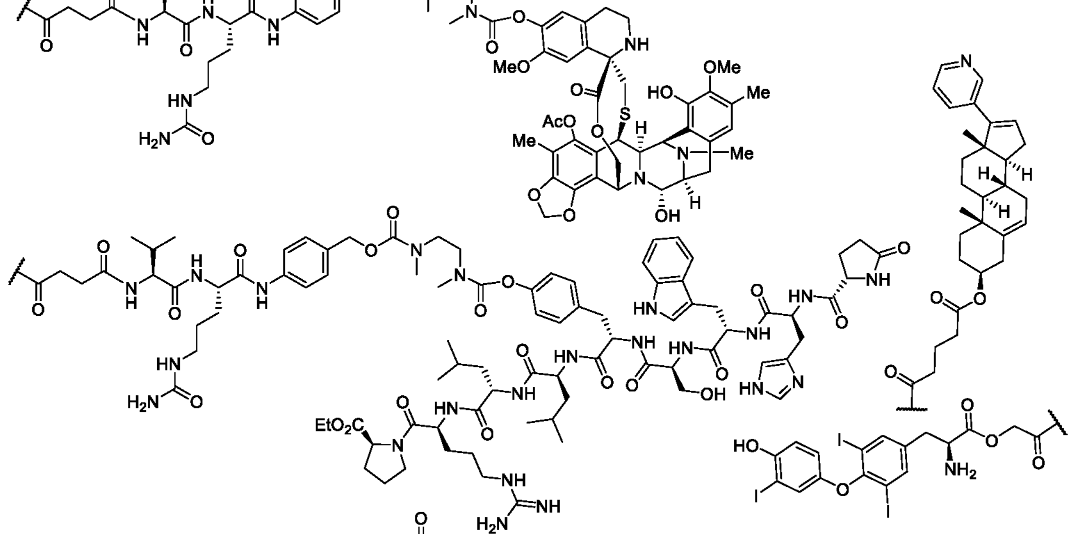

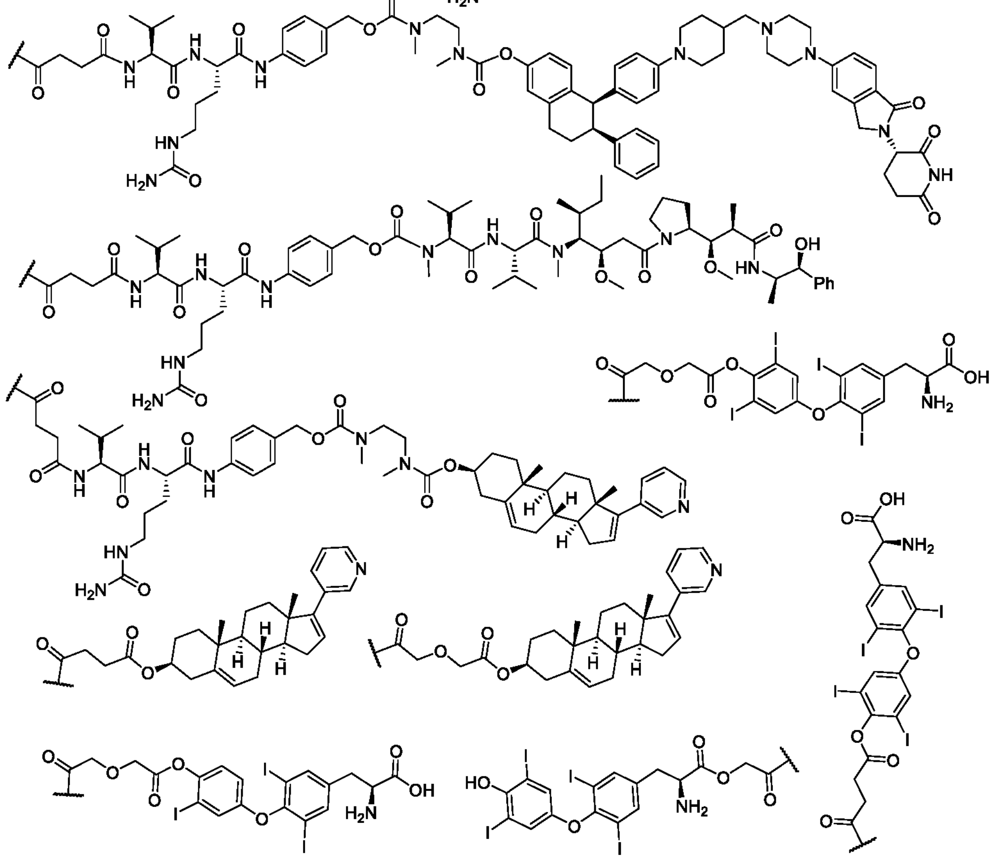

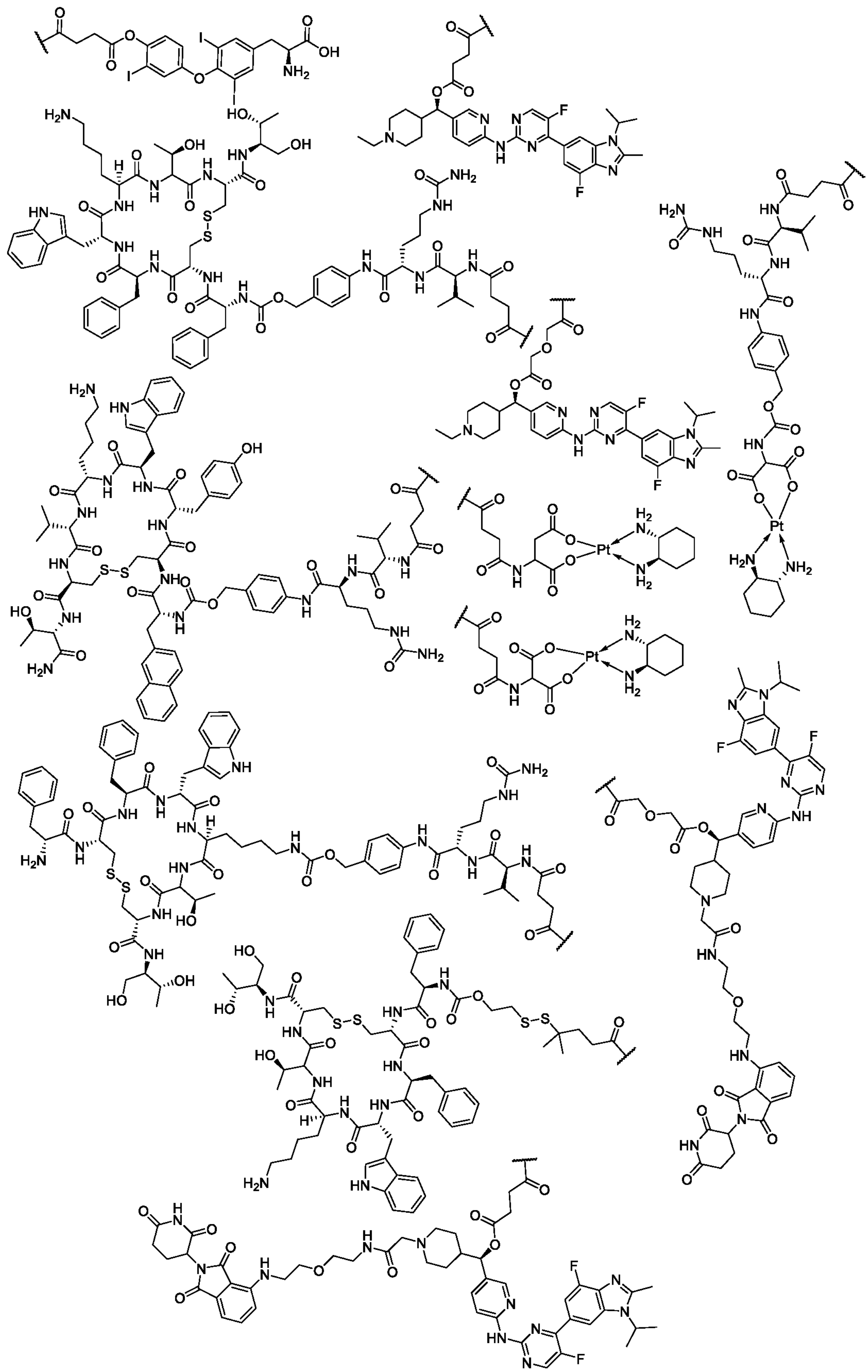

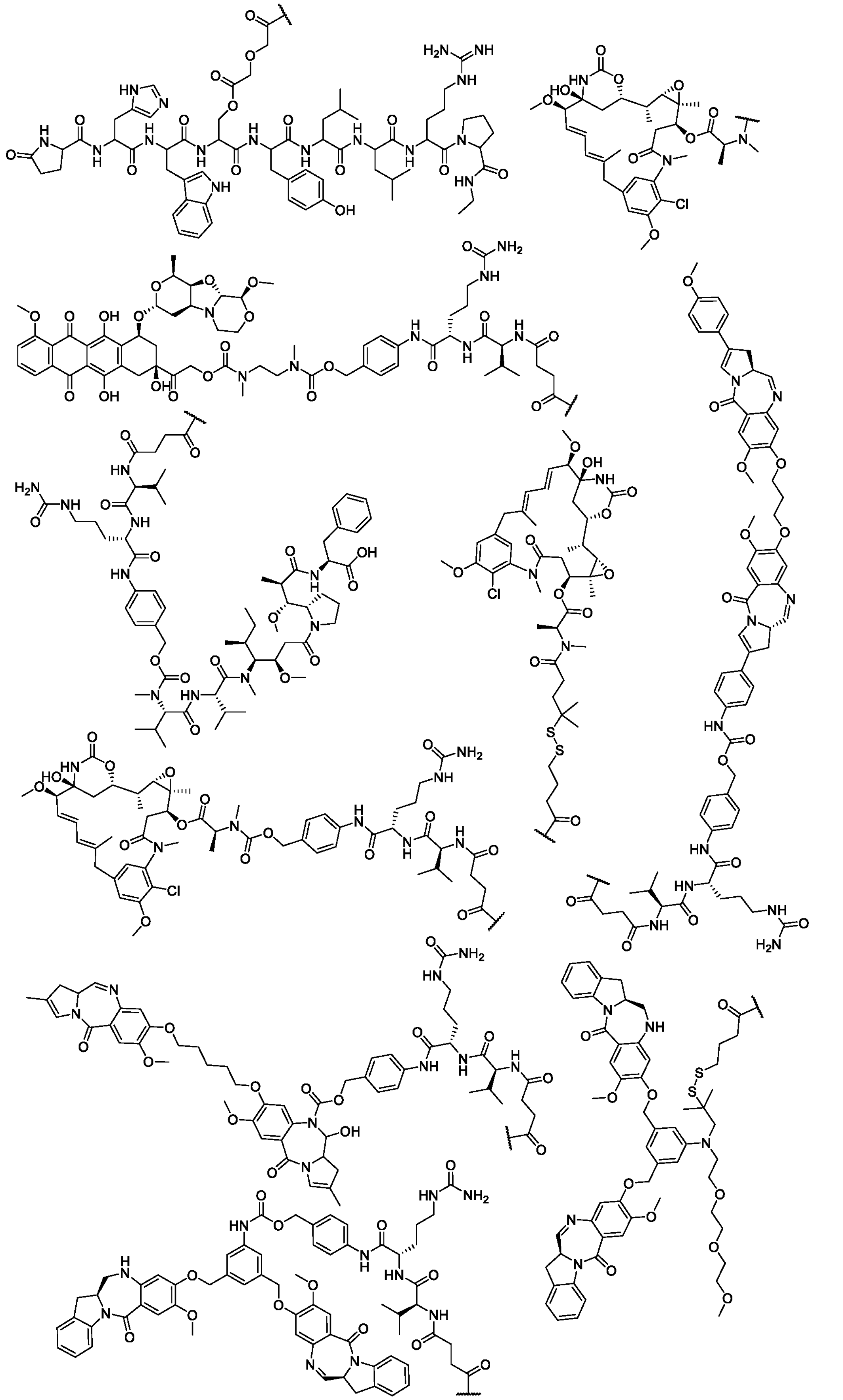

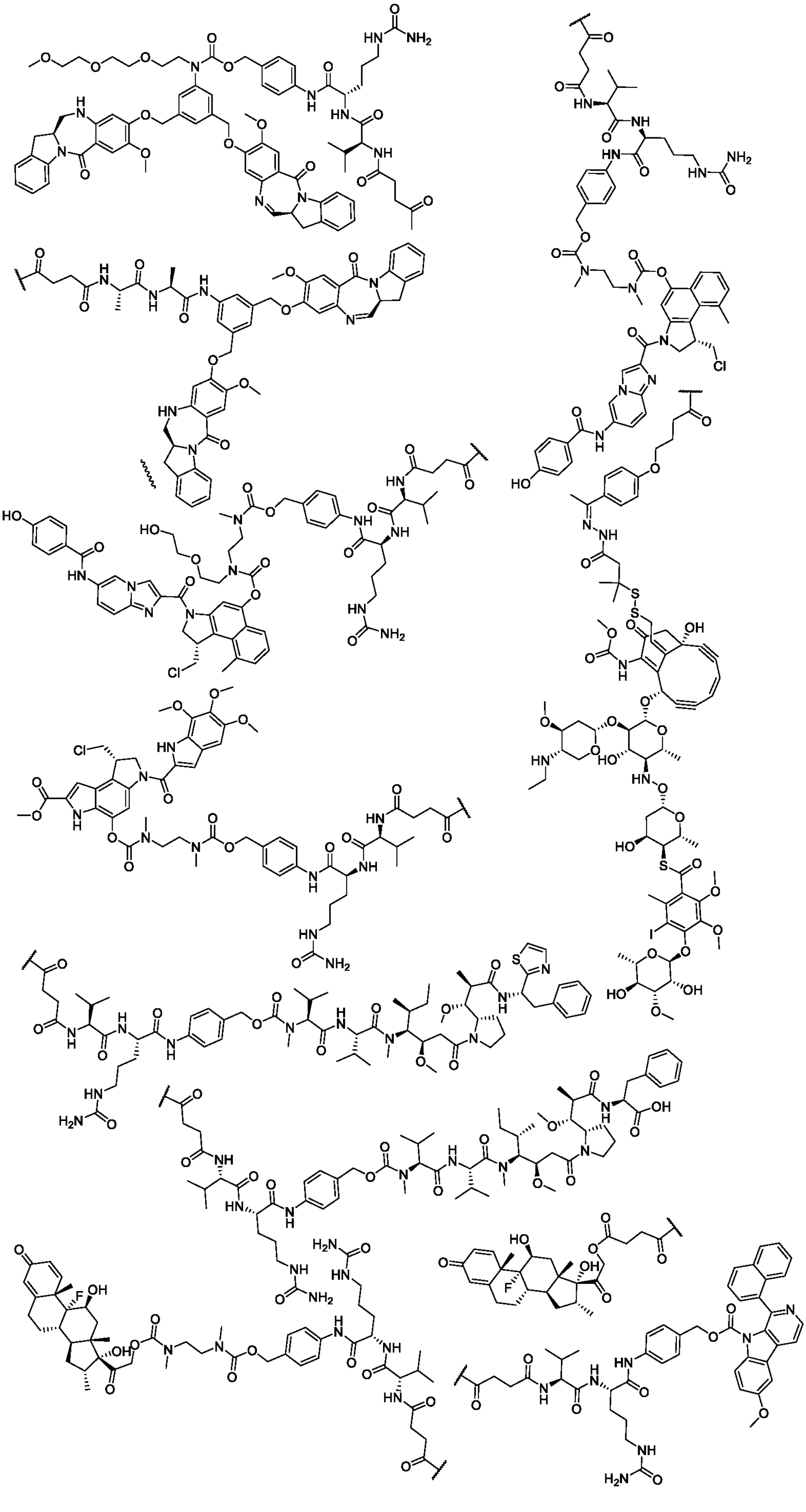

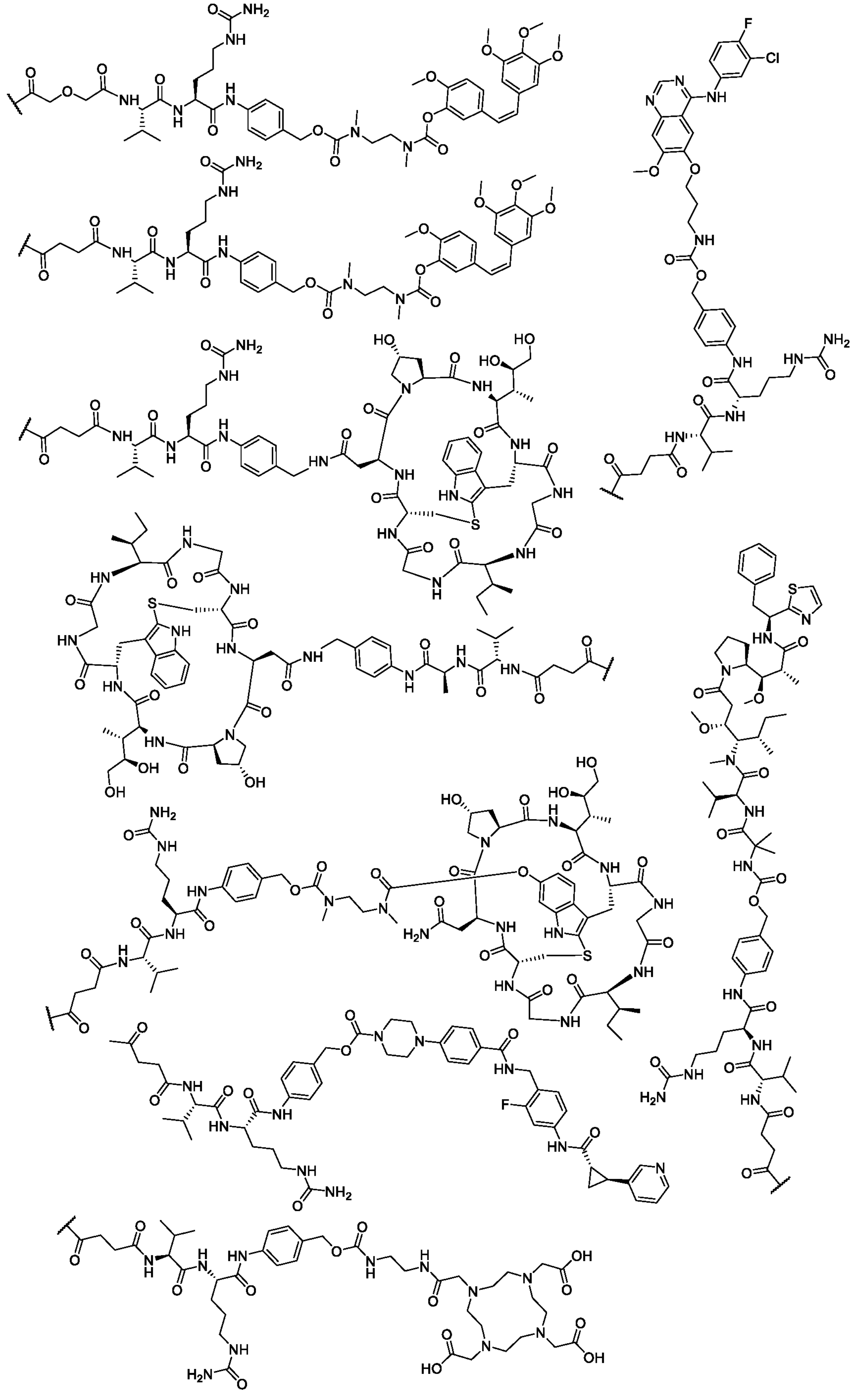

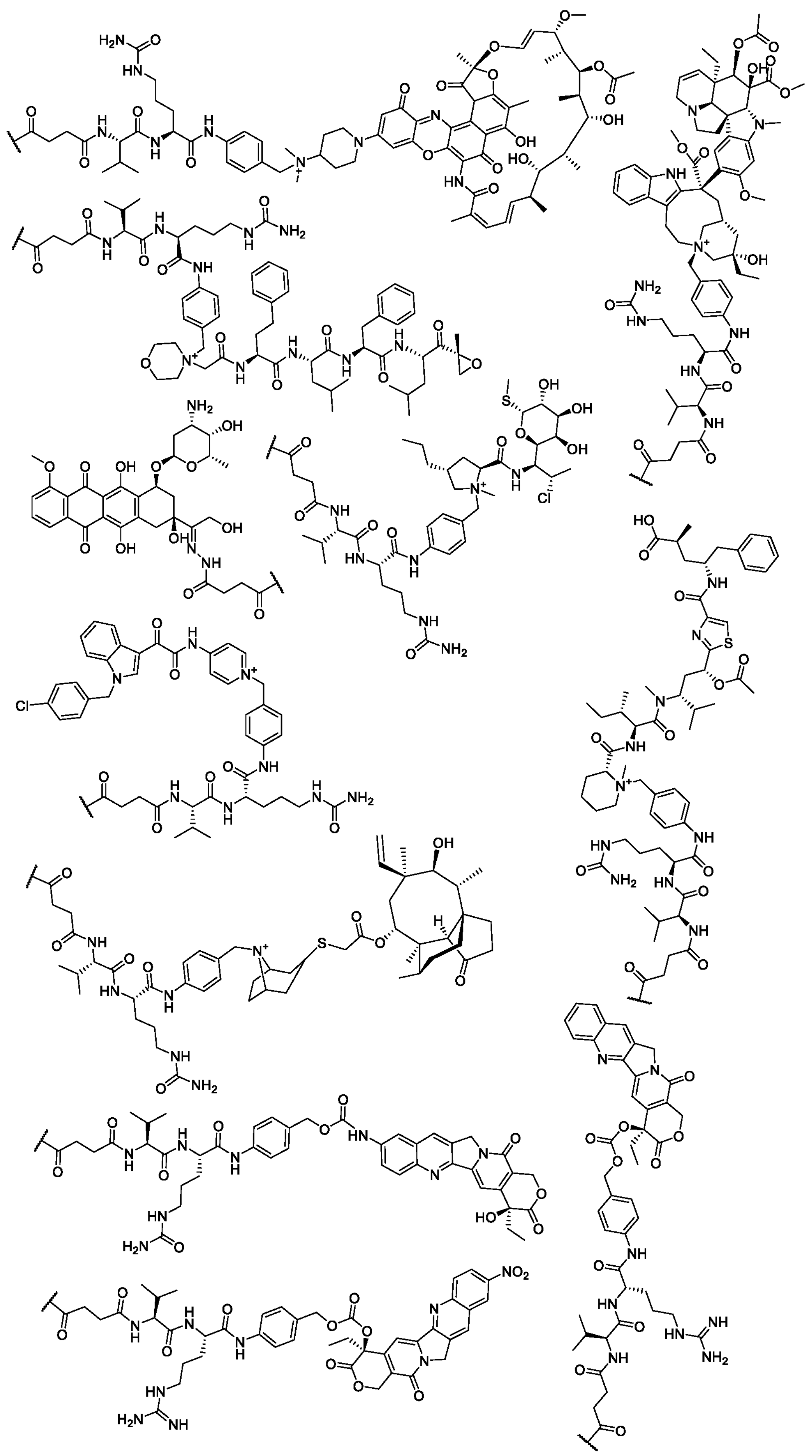

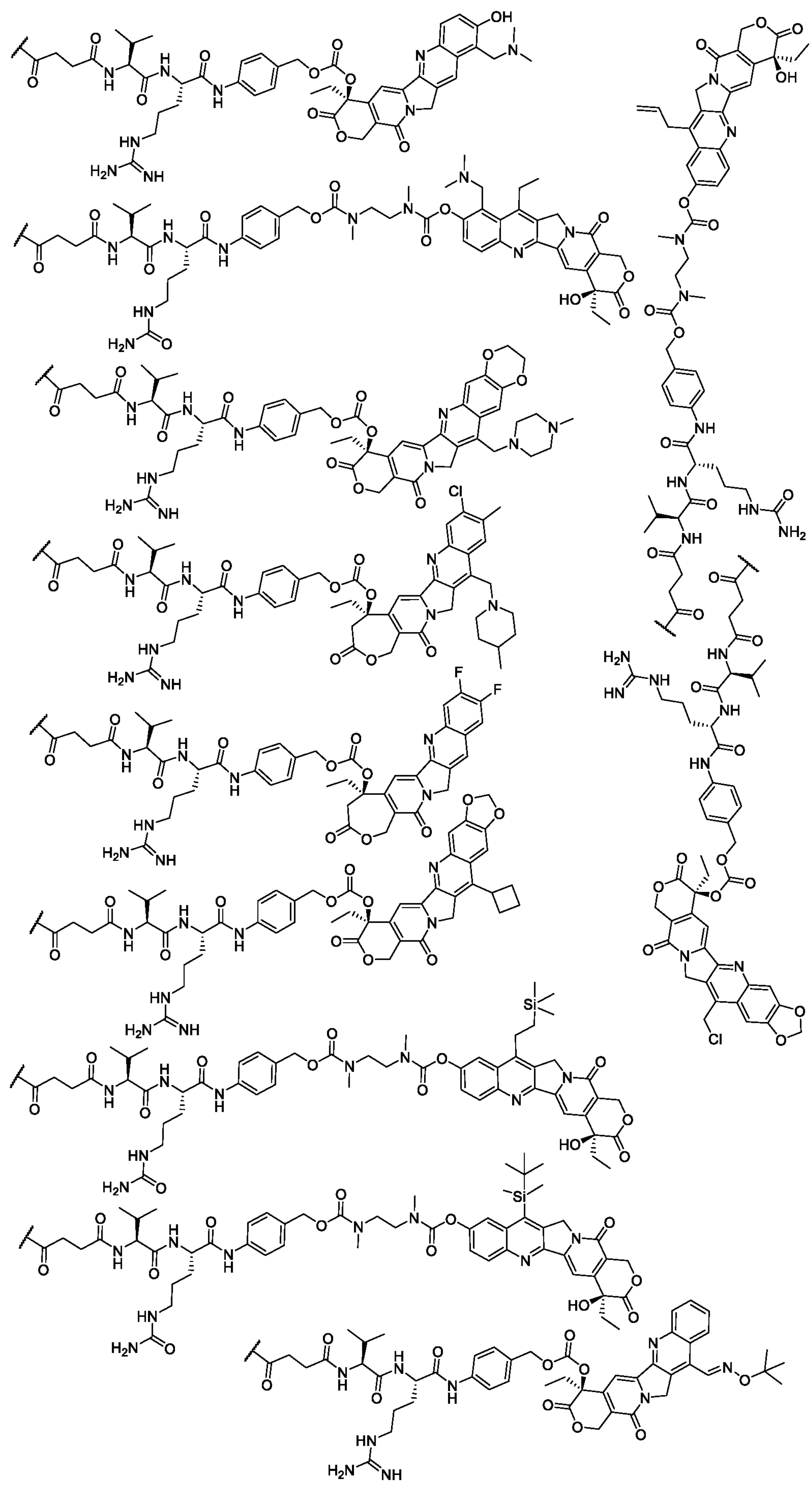

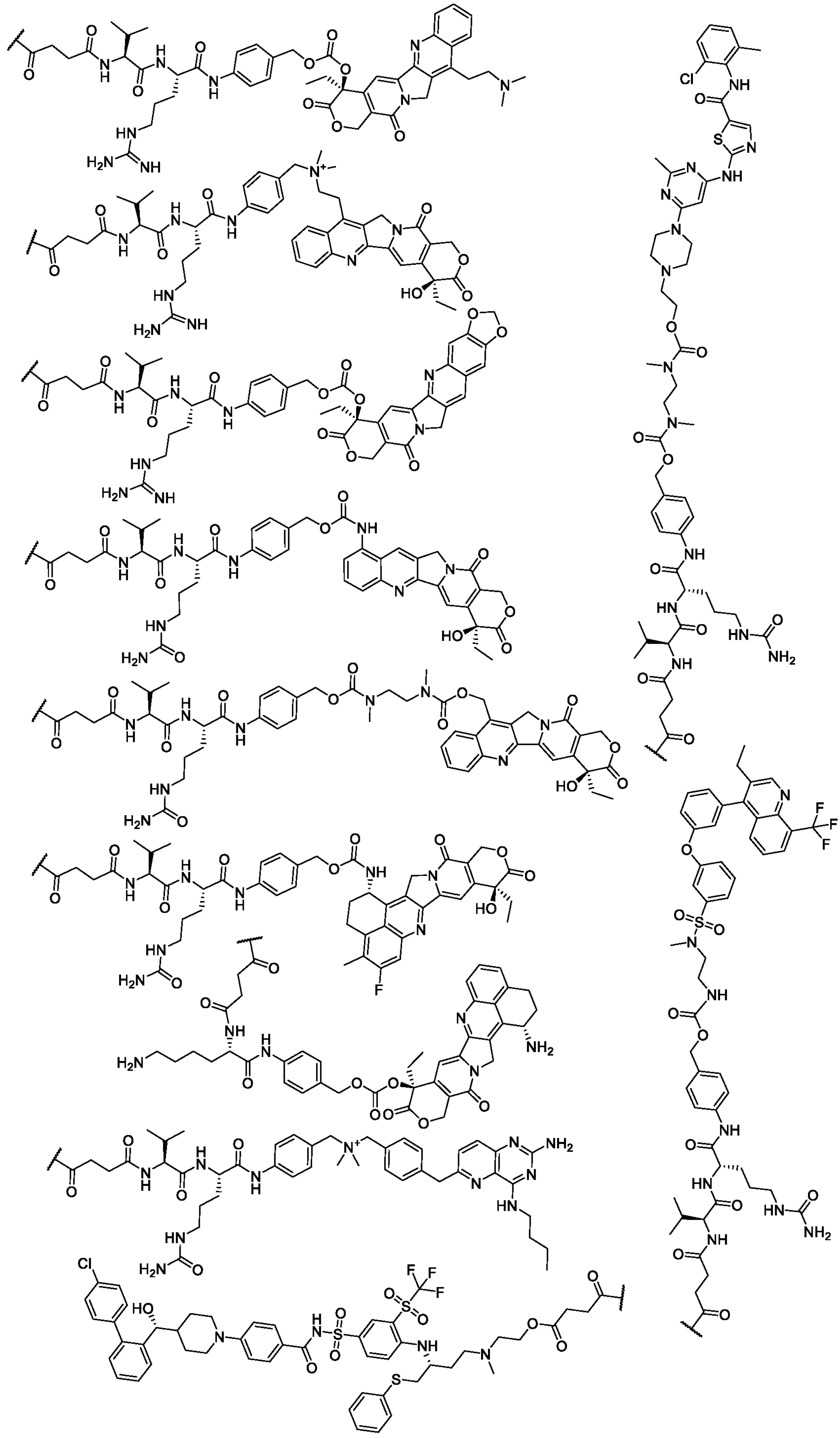

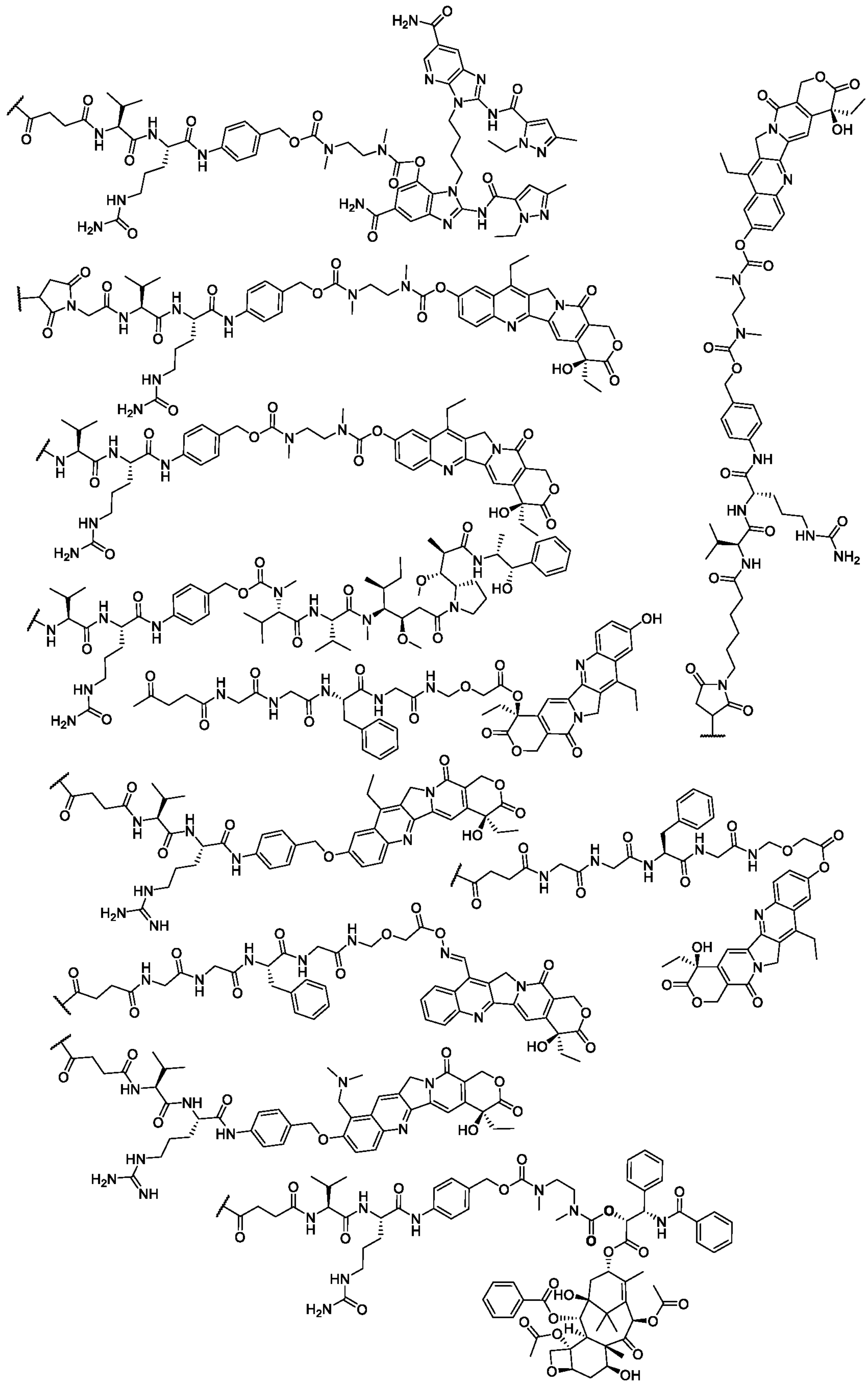

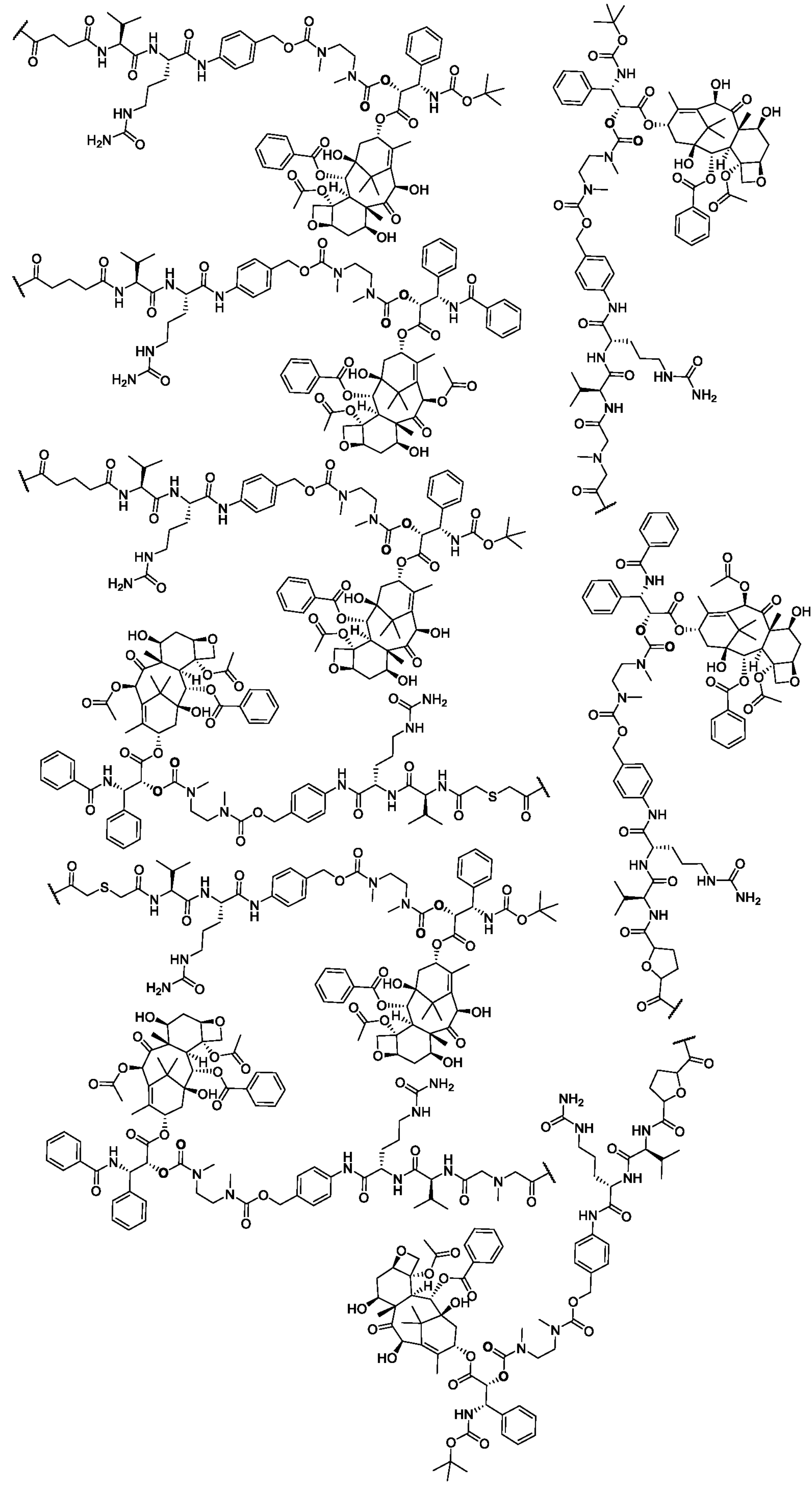

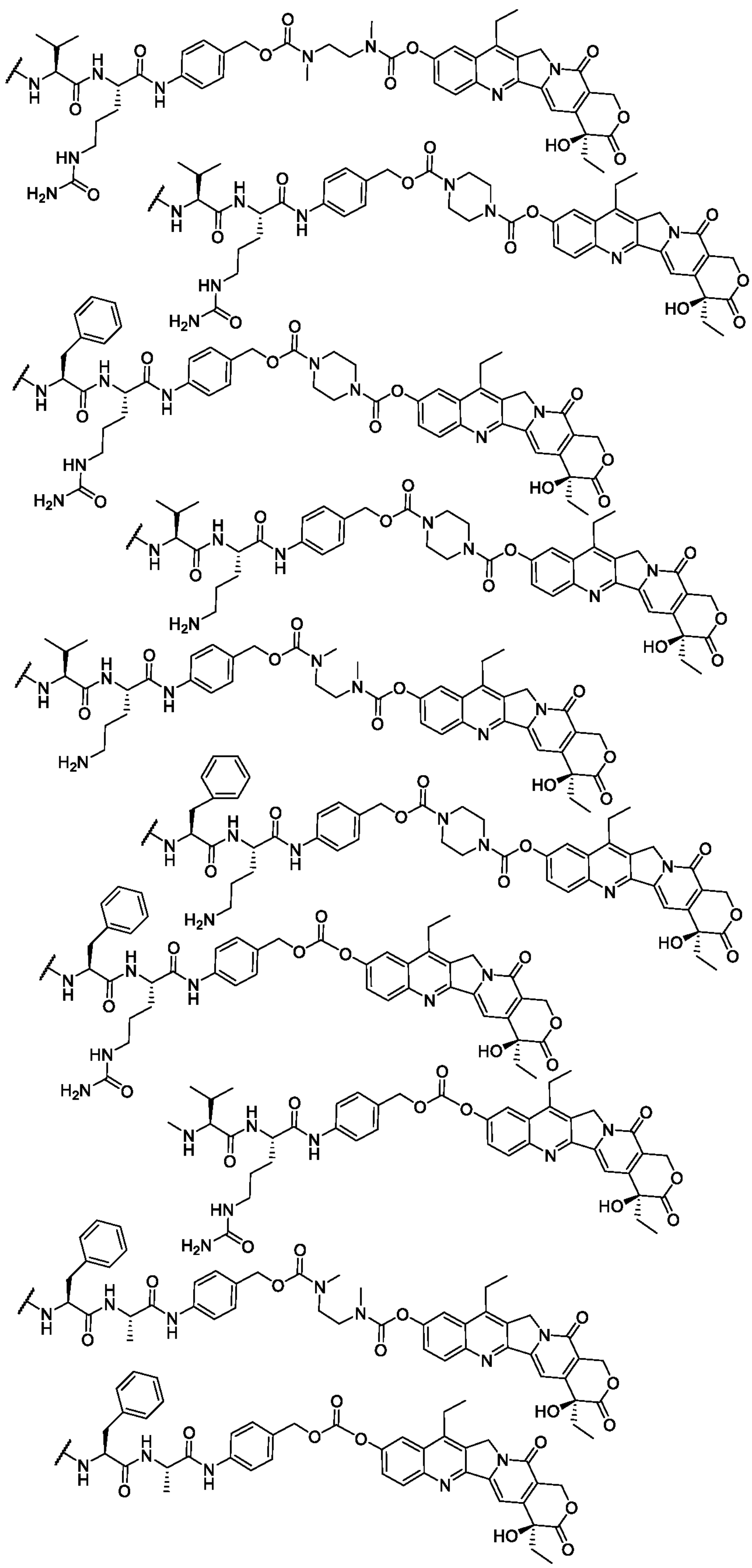

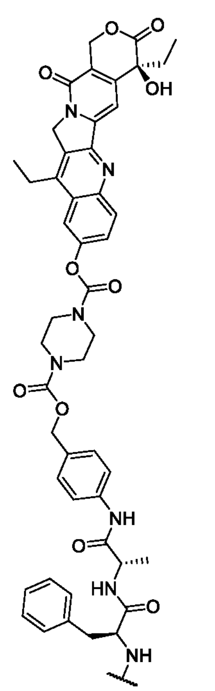

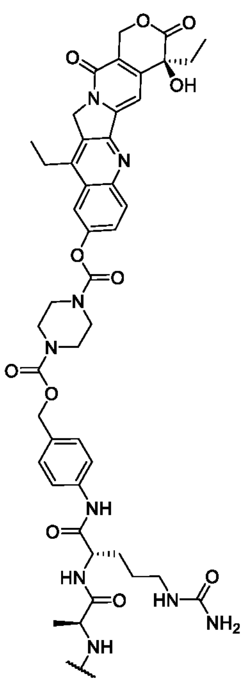

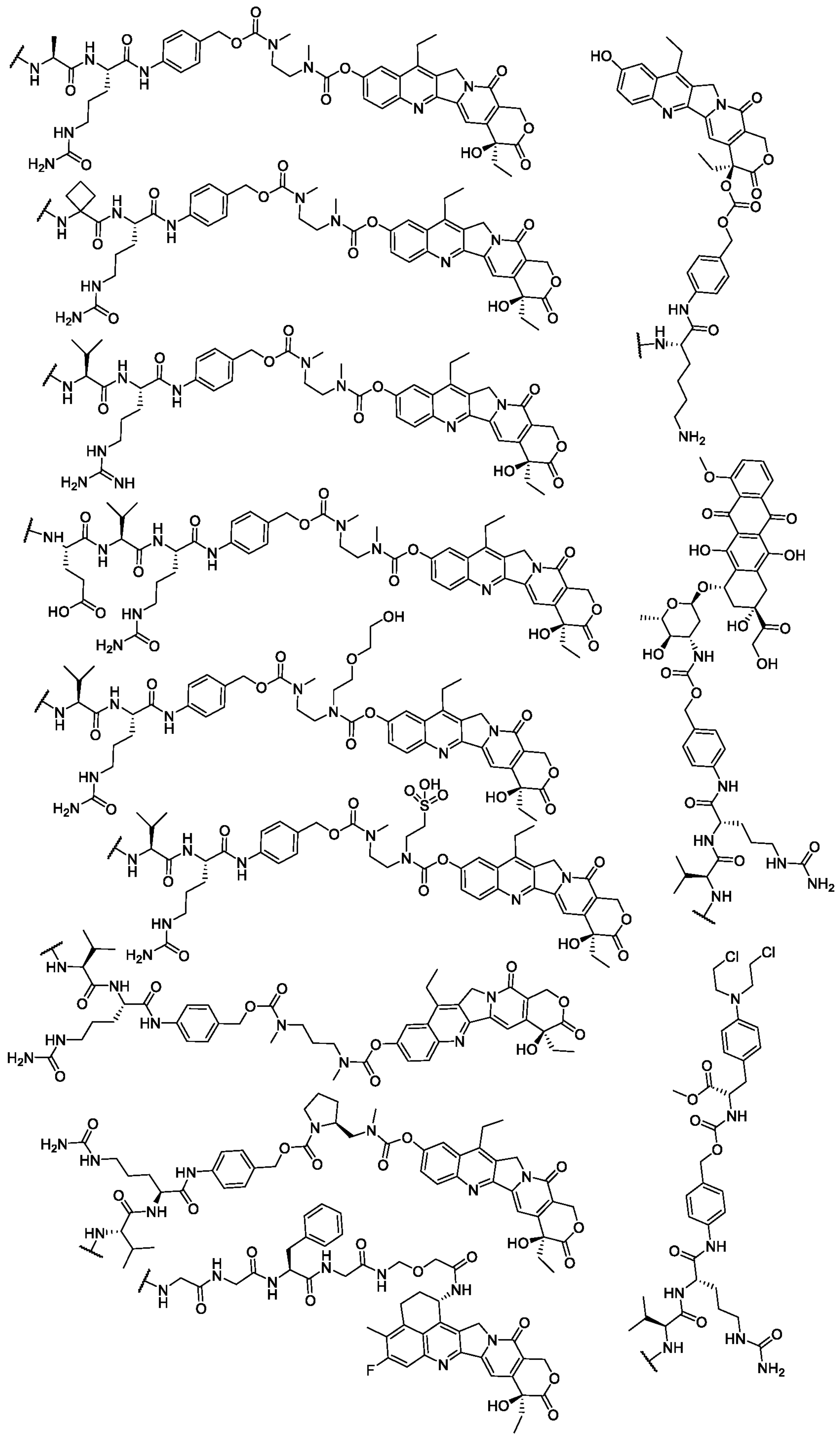

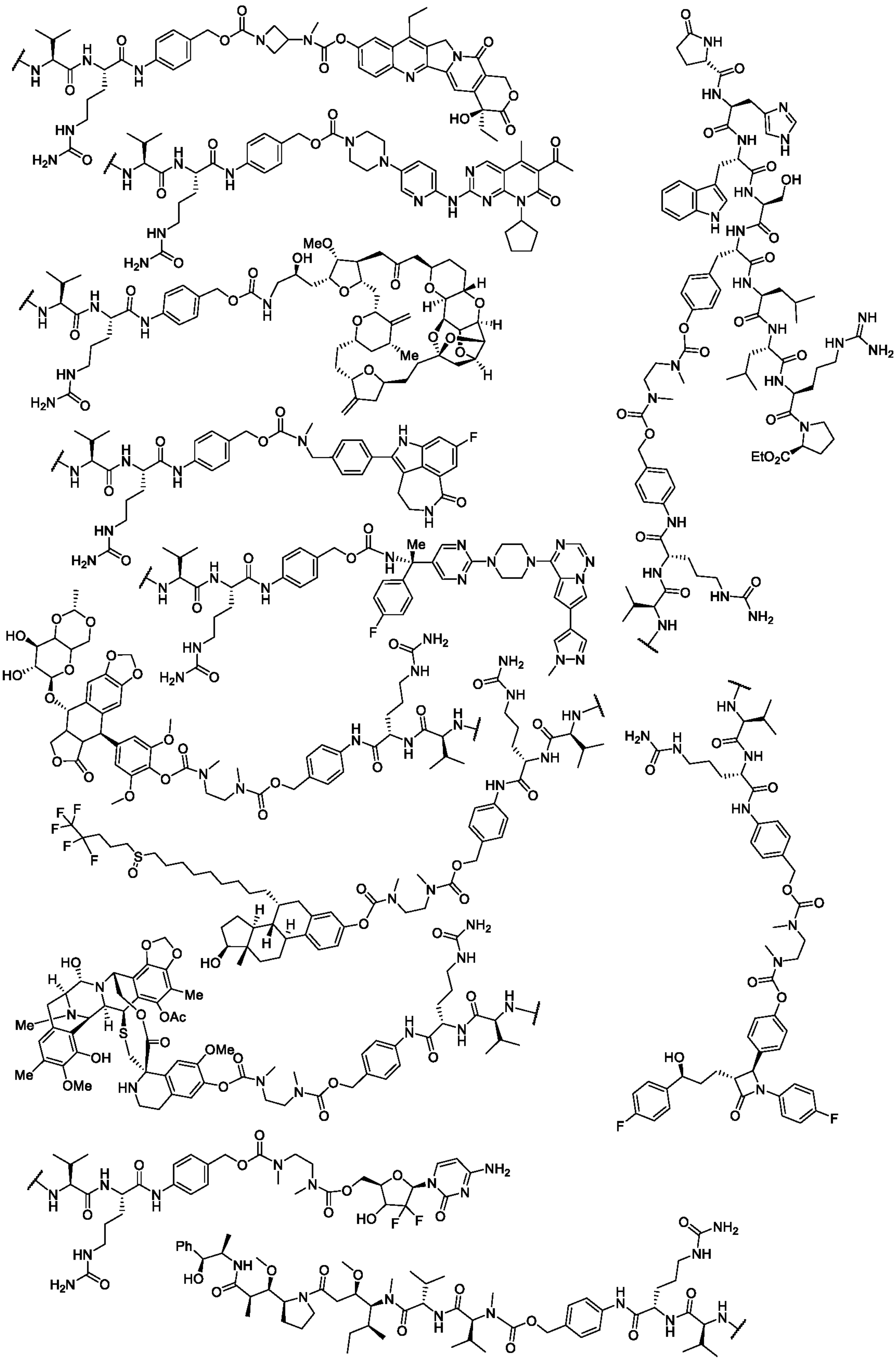

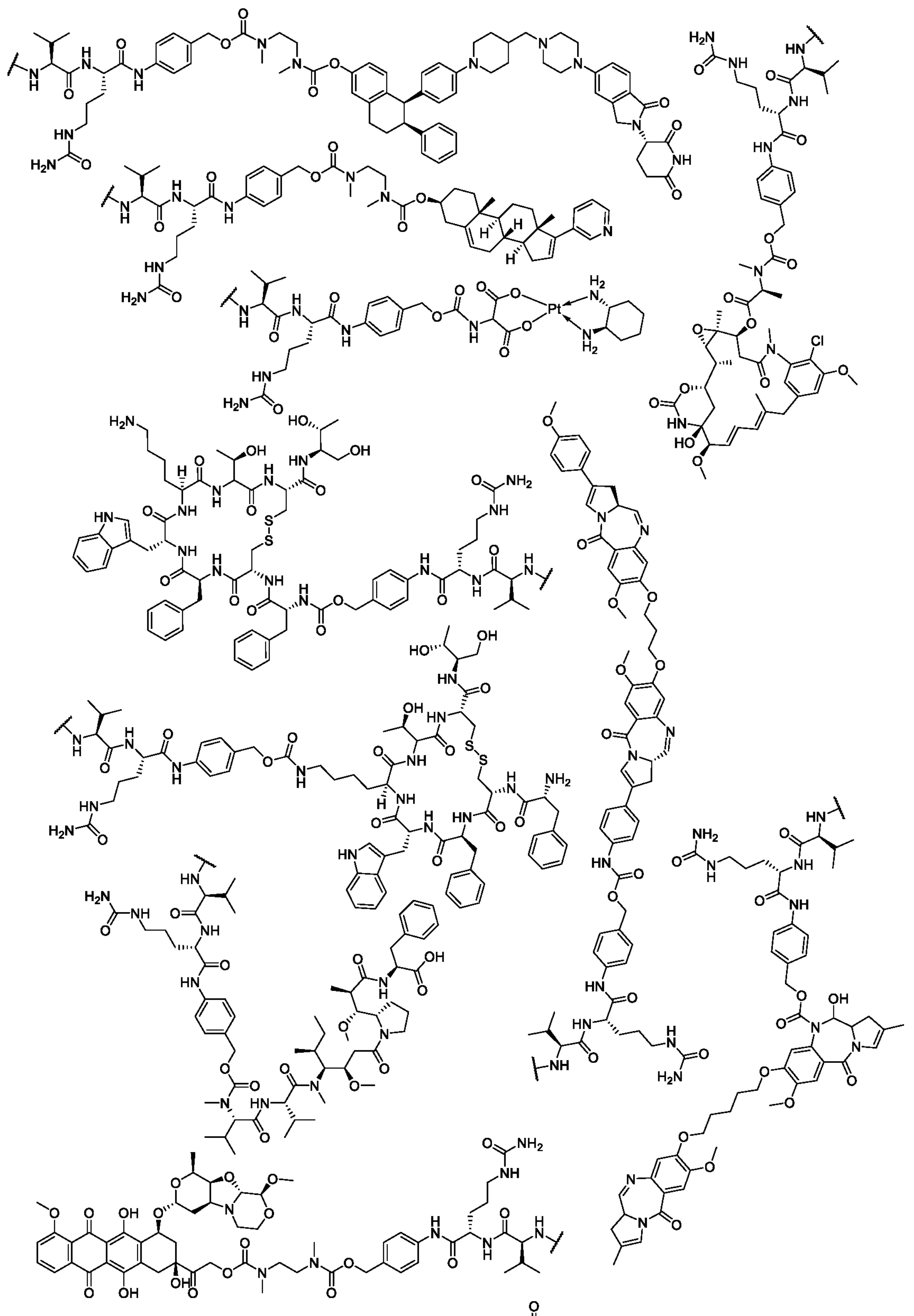

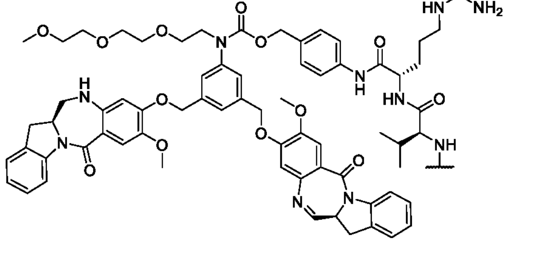

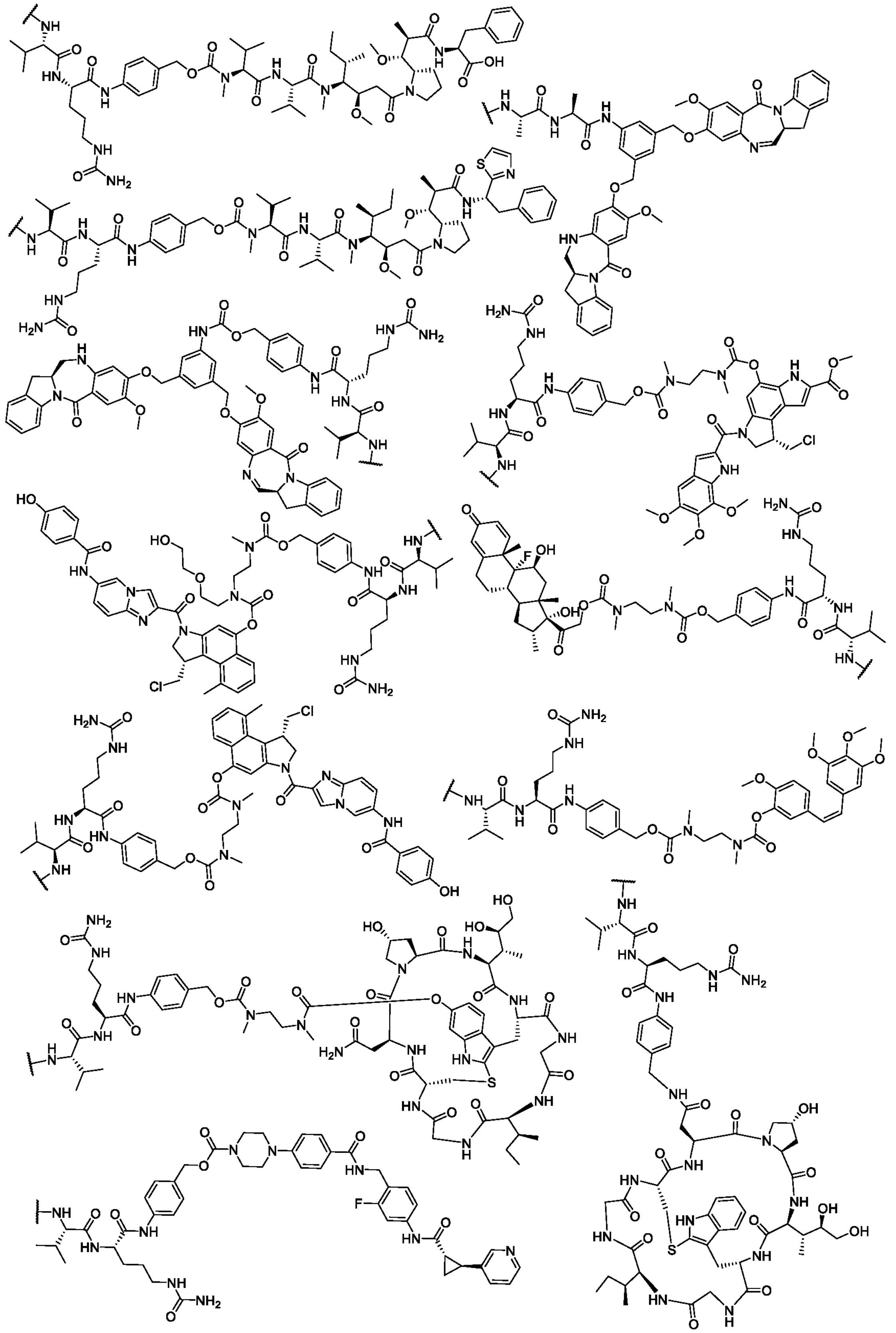

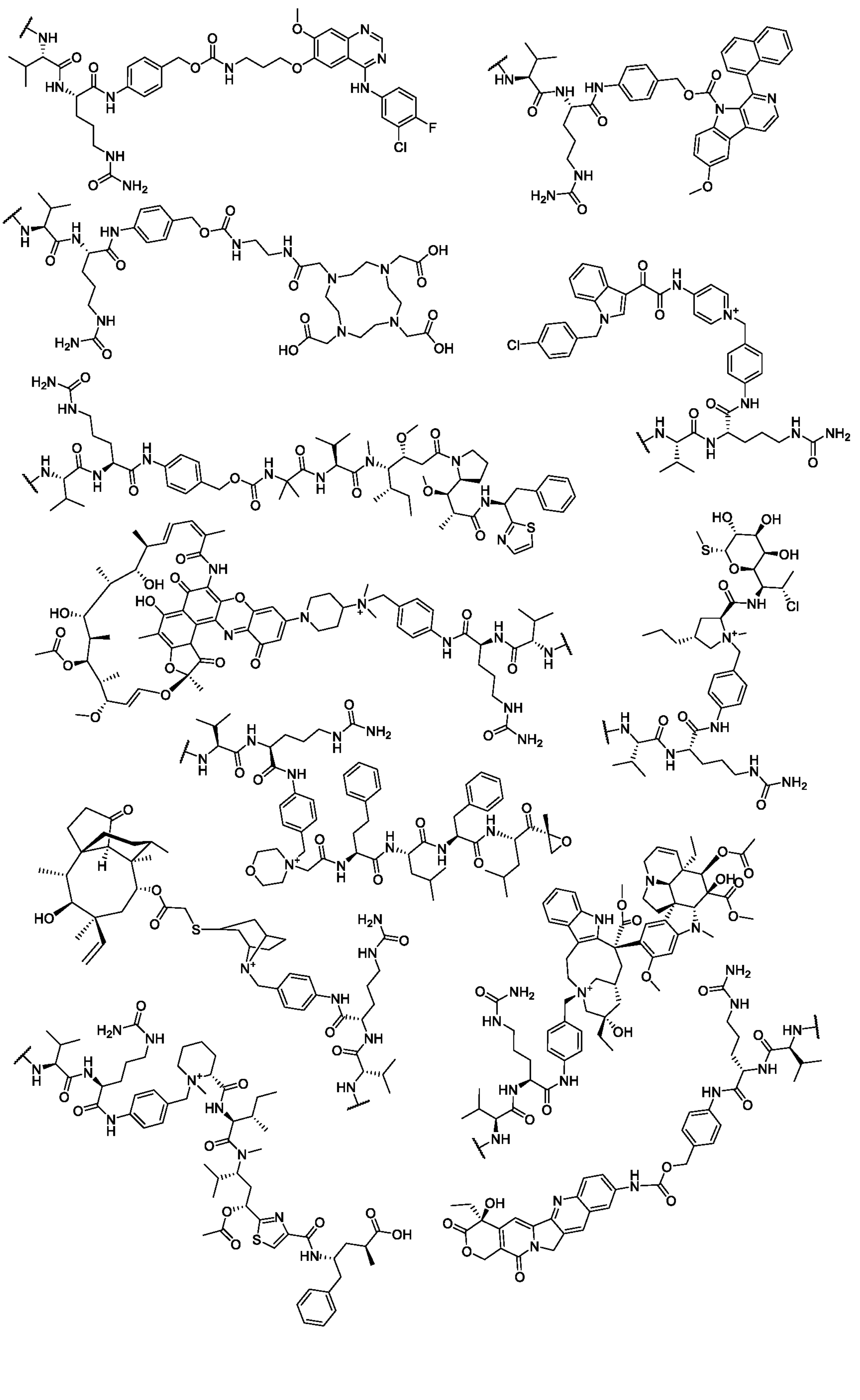

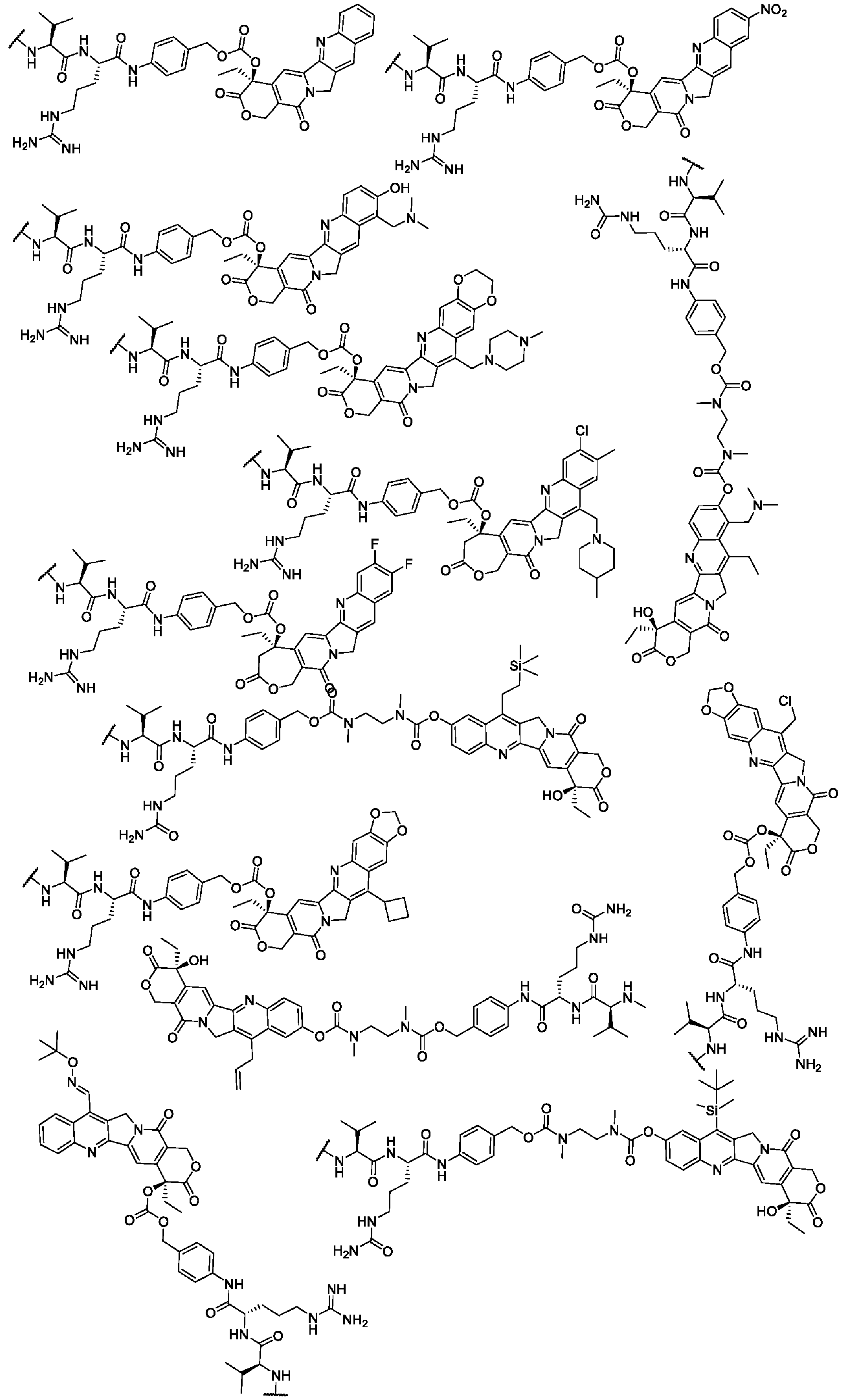

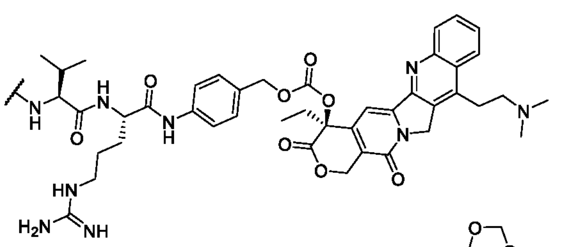

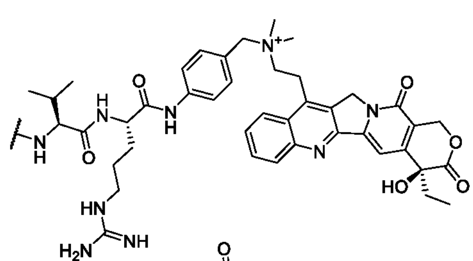

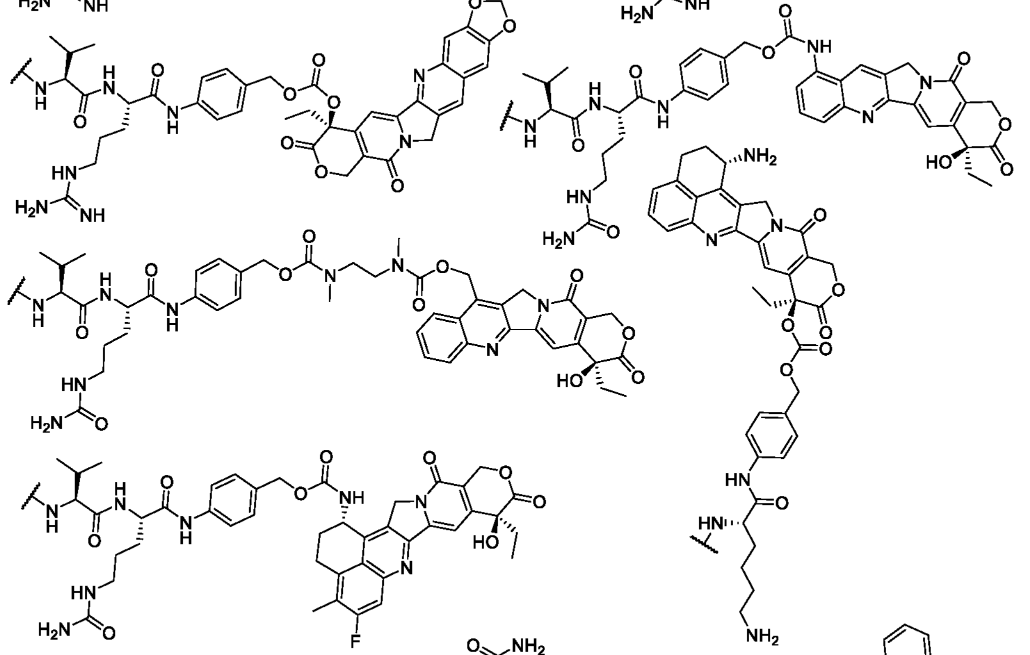

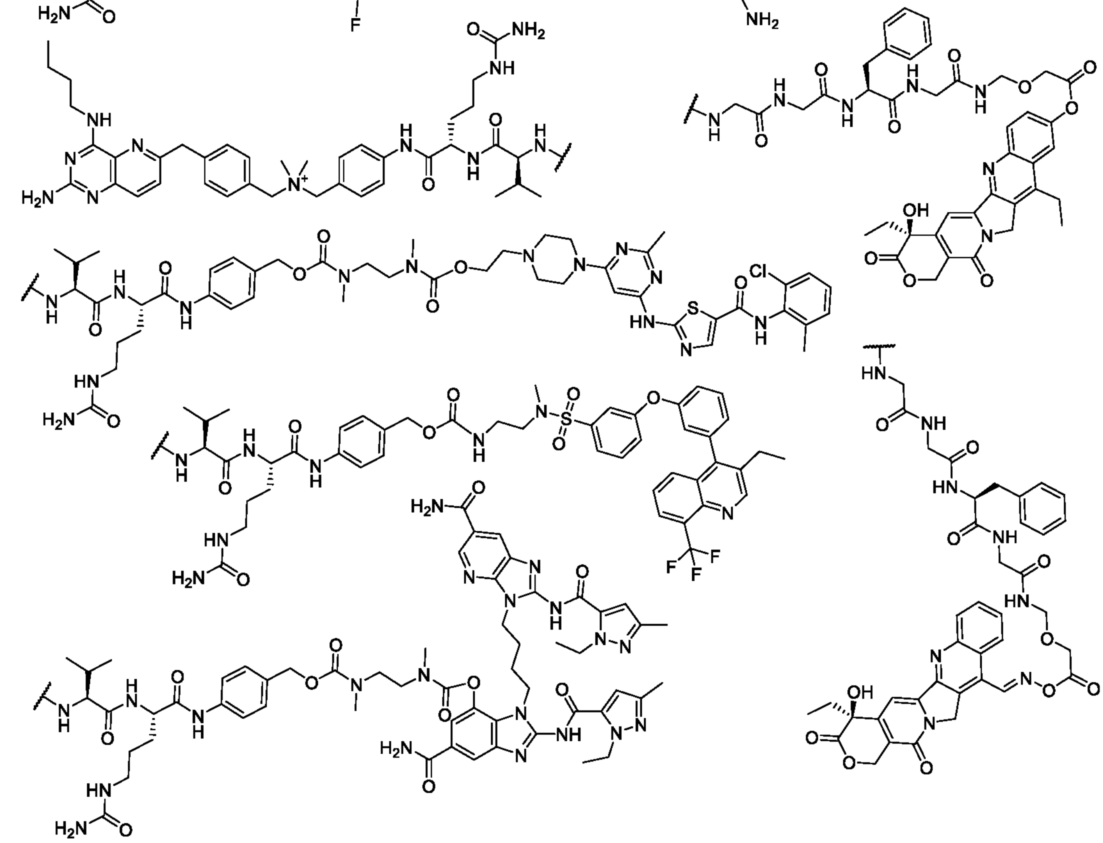

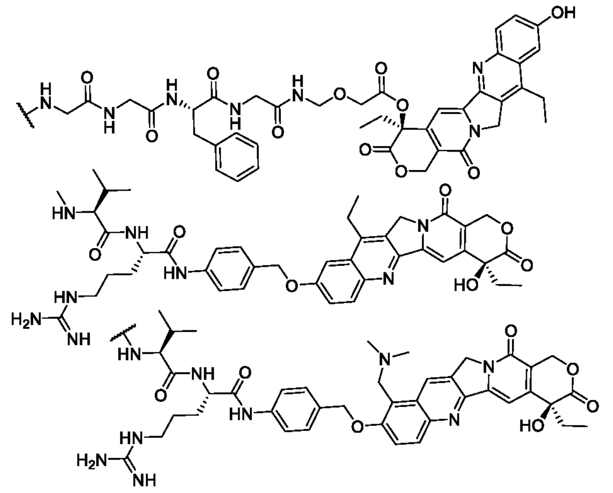

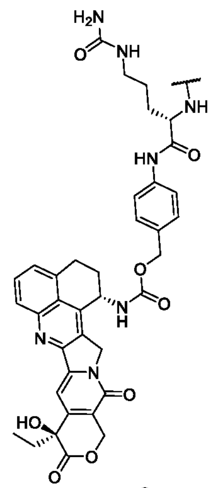

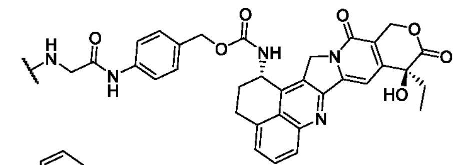

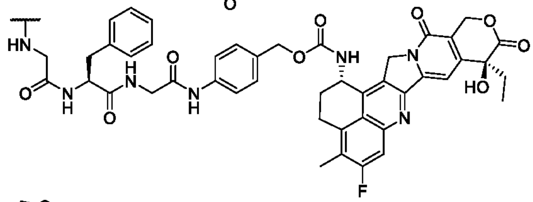

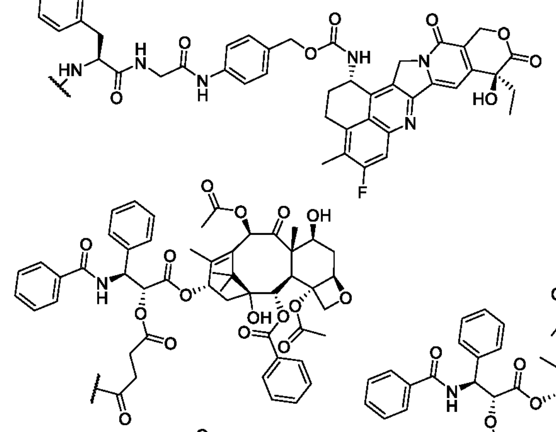

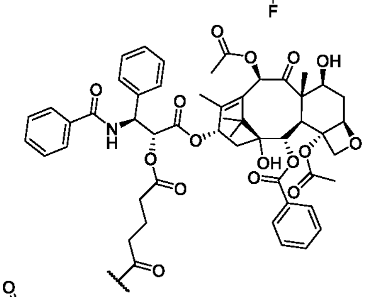

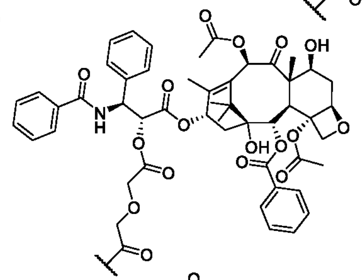

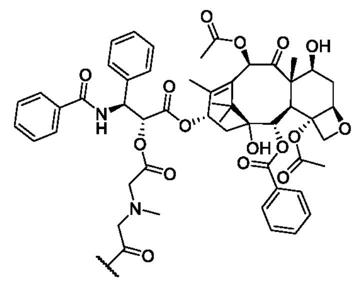

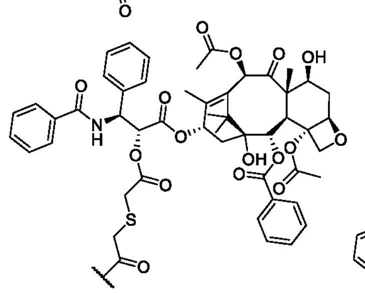

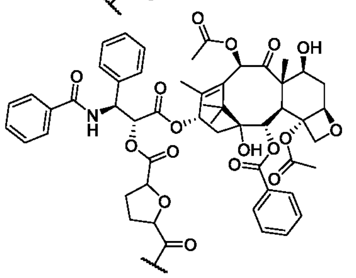

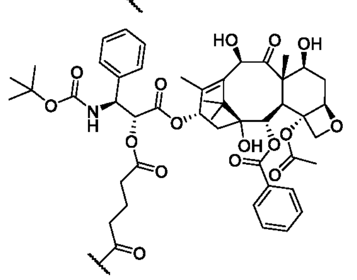

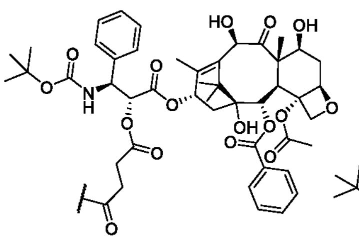

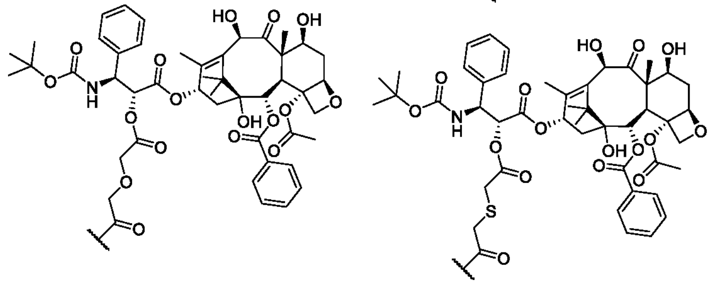

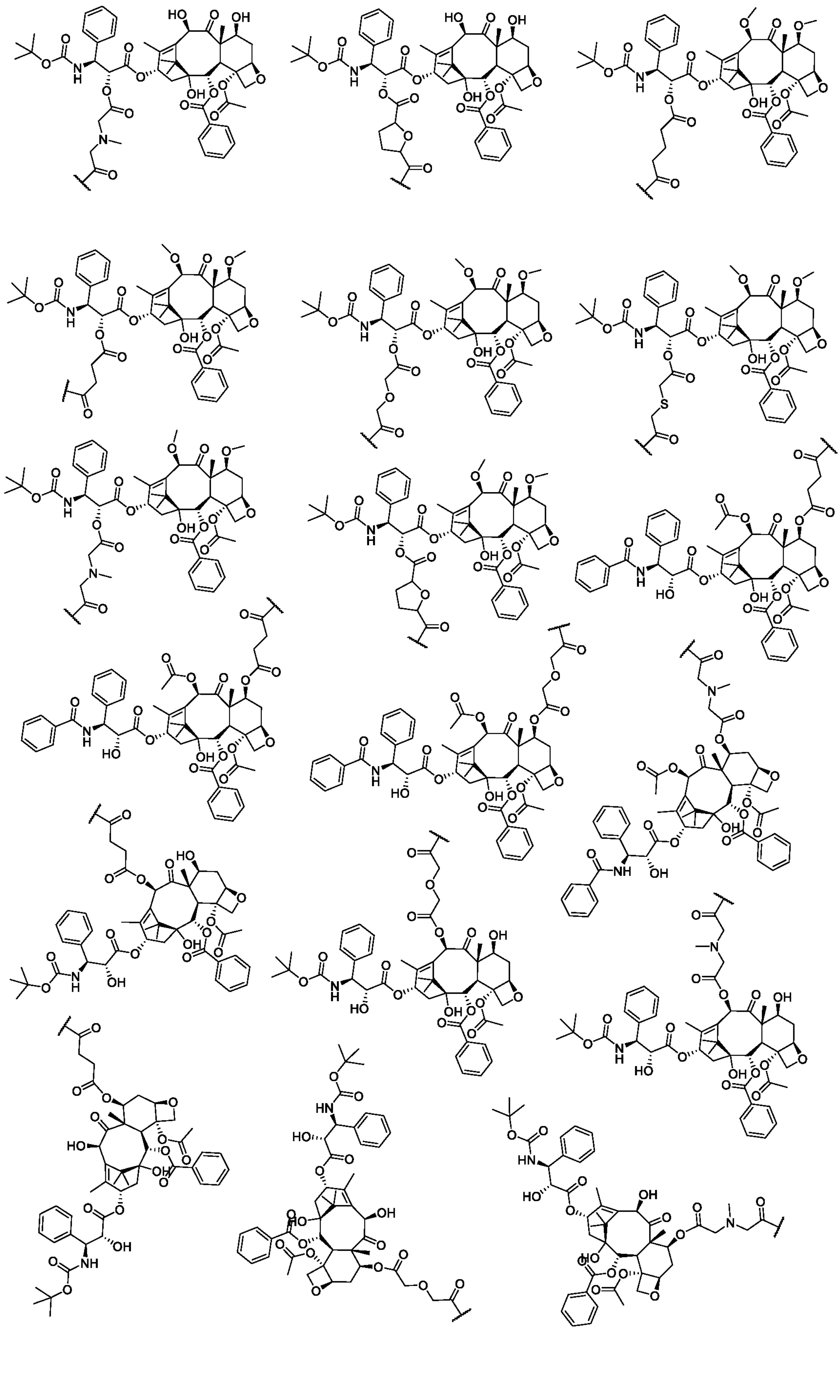

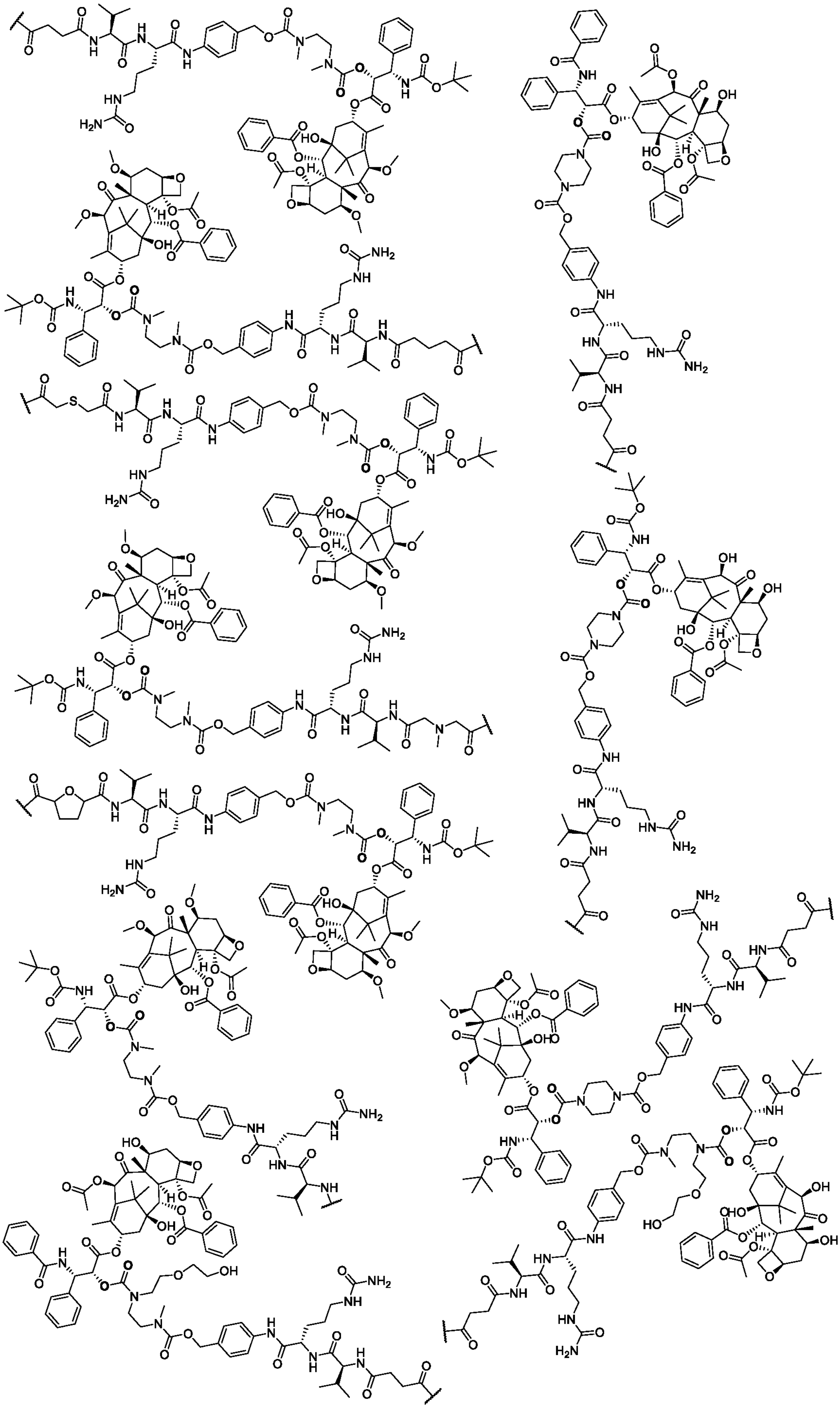

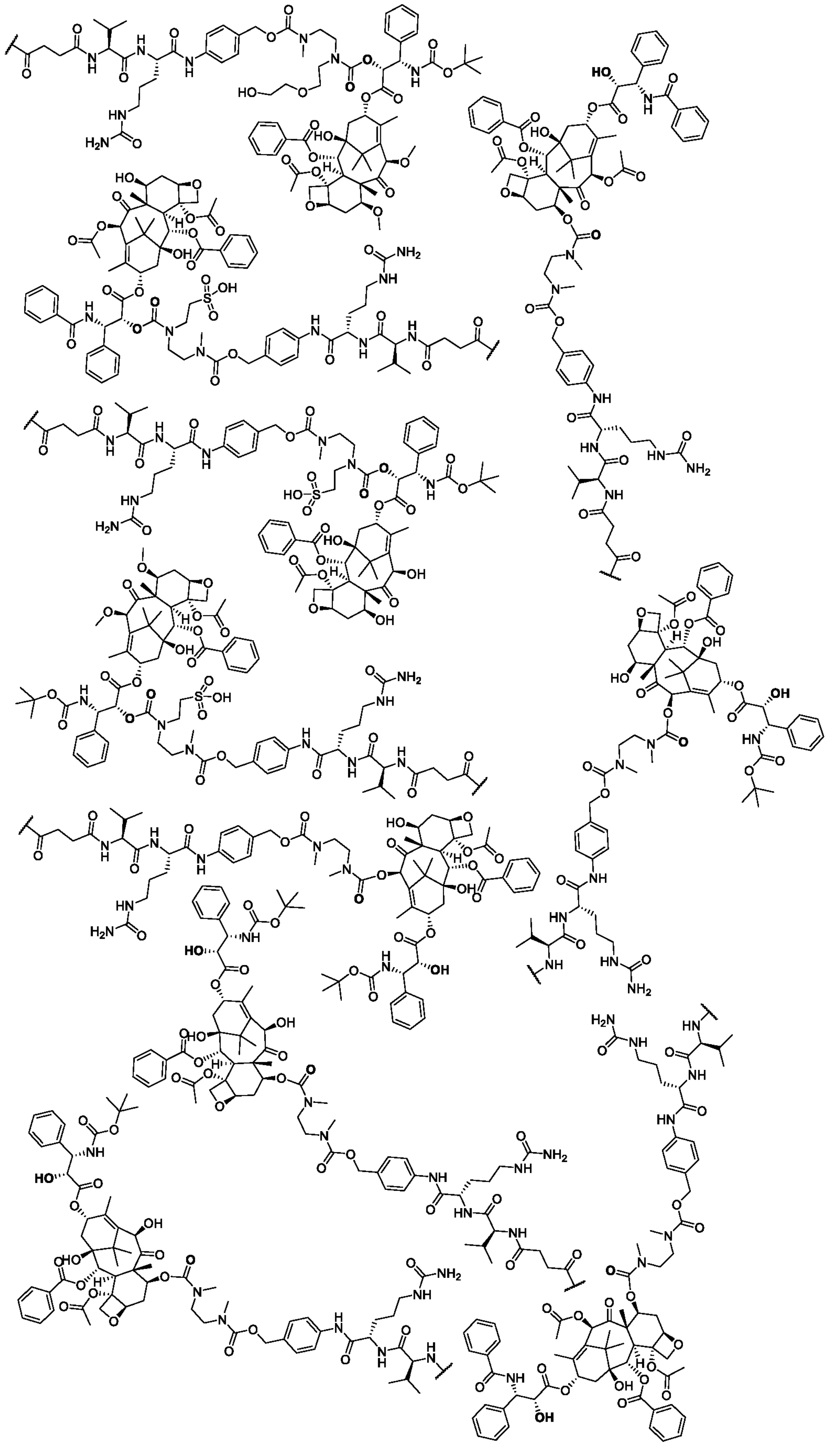

;

preferably

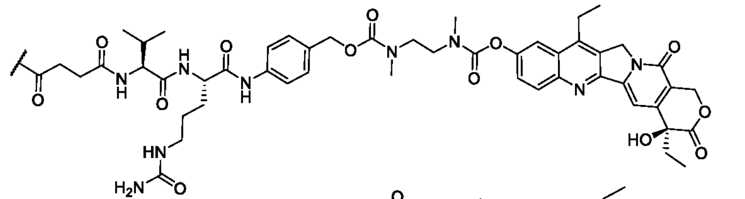

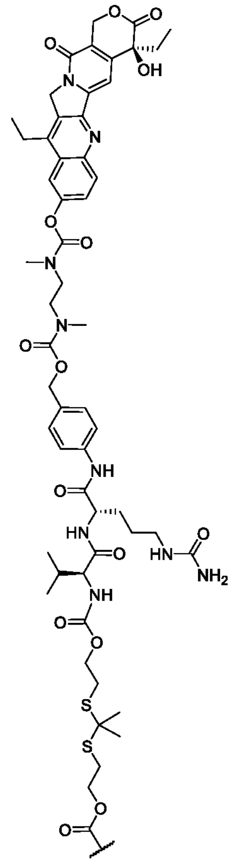

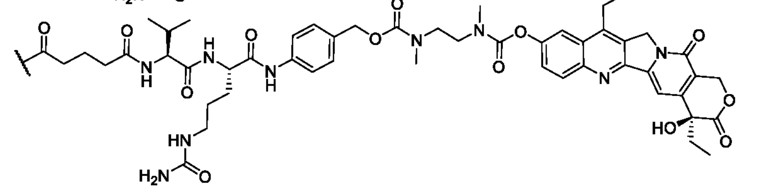

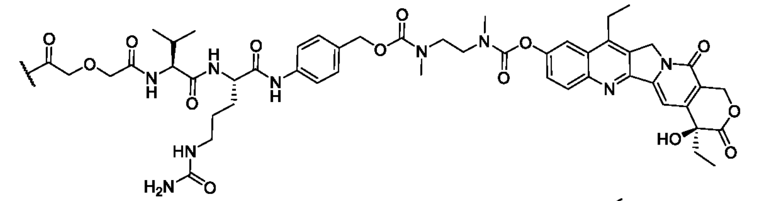

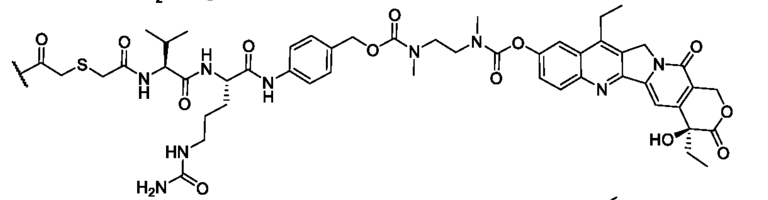

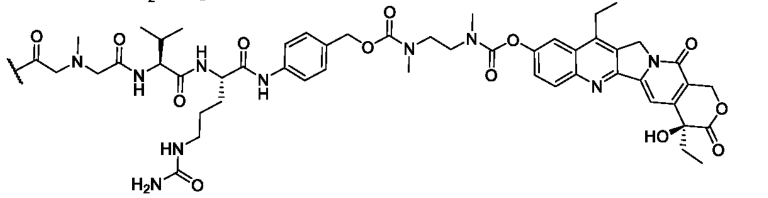

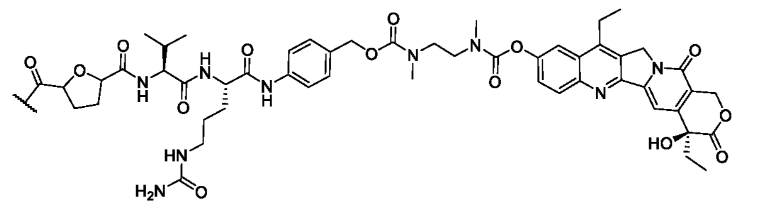

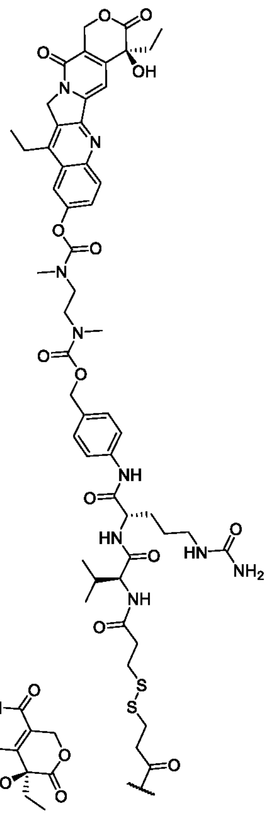

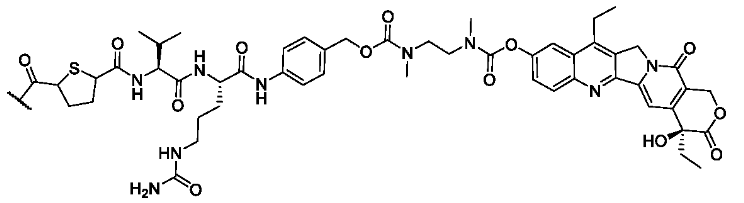

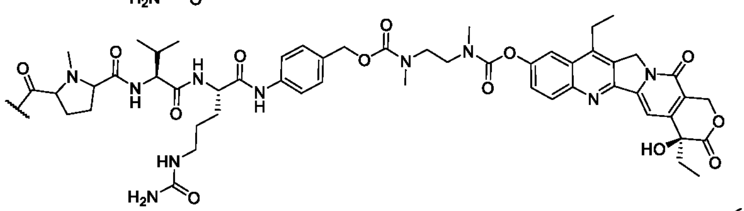

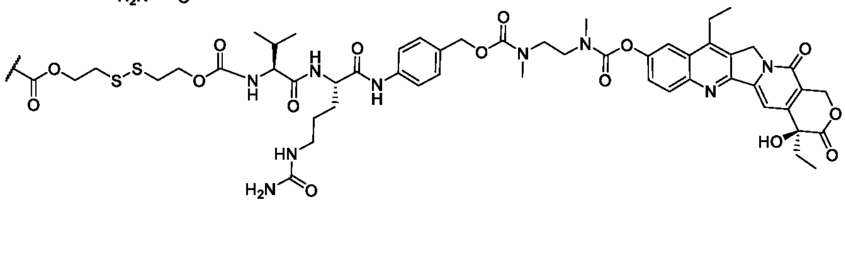

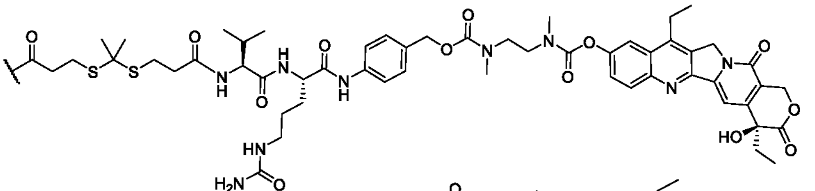

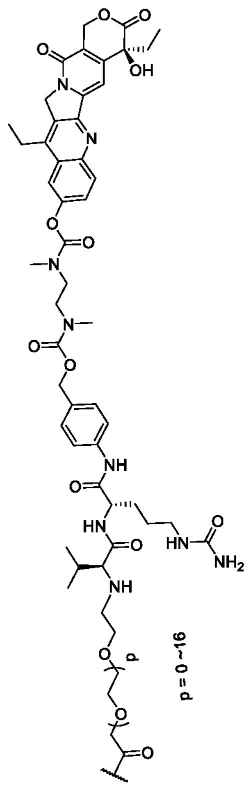

p = 0 ~16

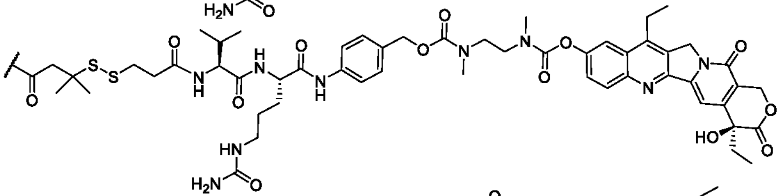

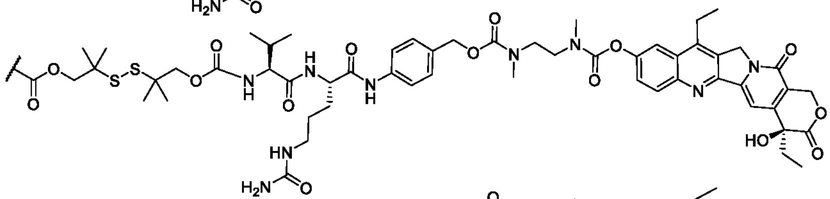

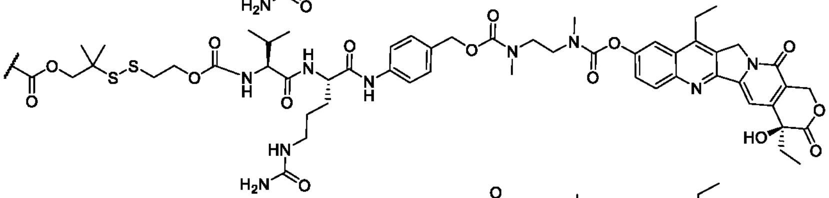

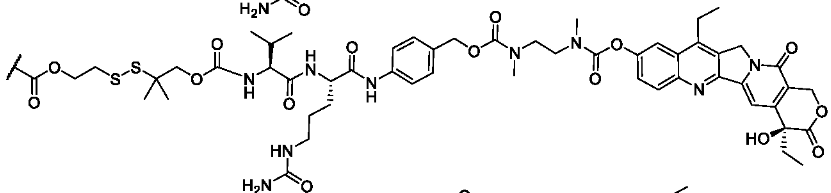

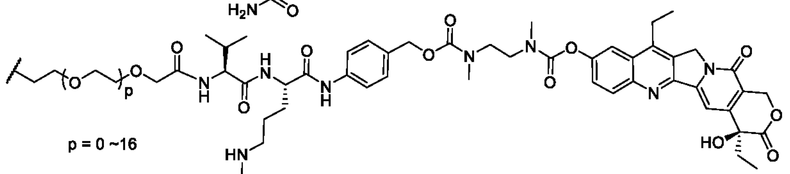

p = 0 ~16

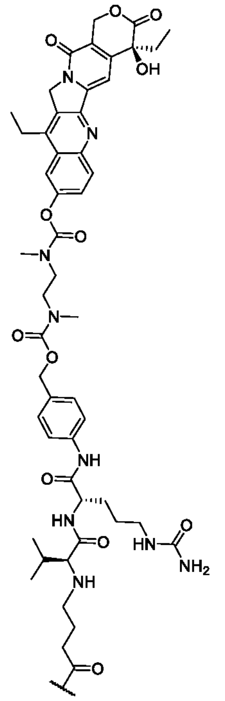

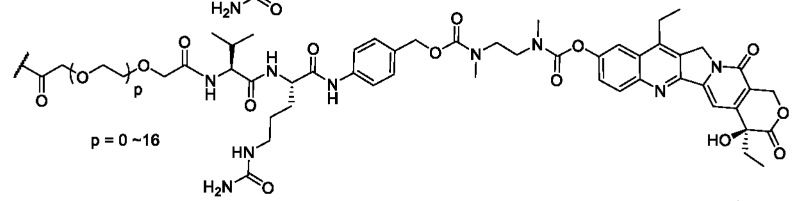

p = 0 ~16

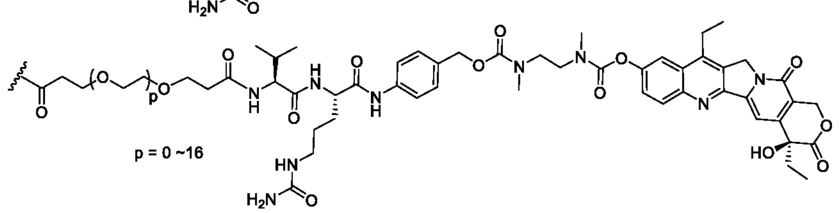

p = 0 ~16

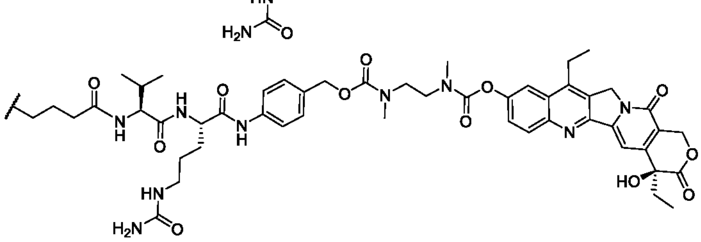

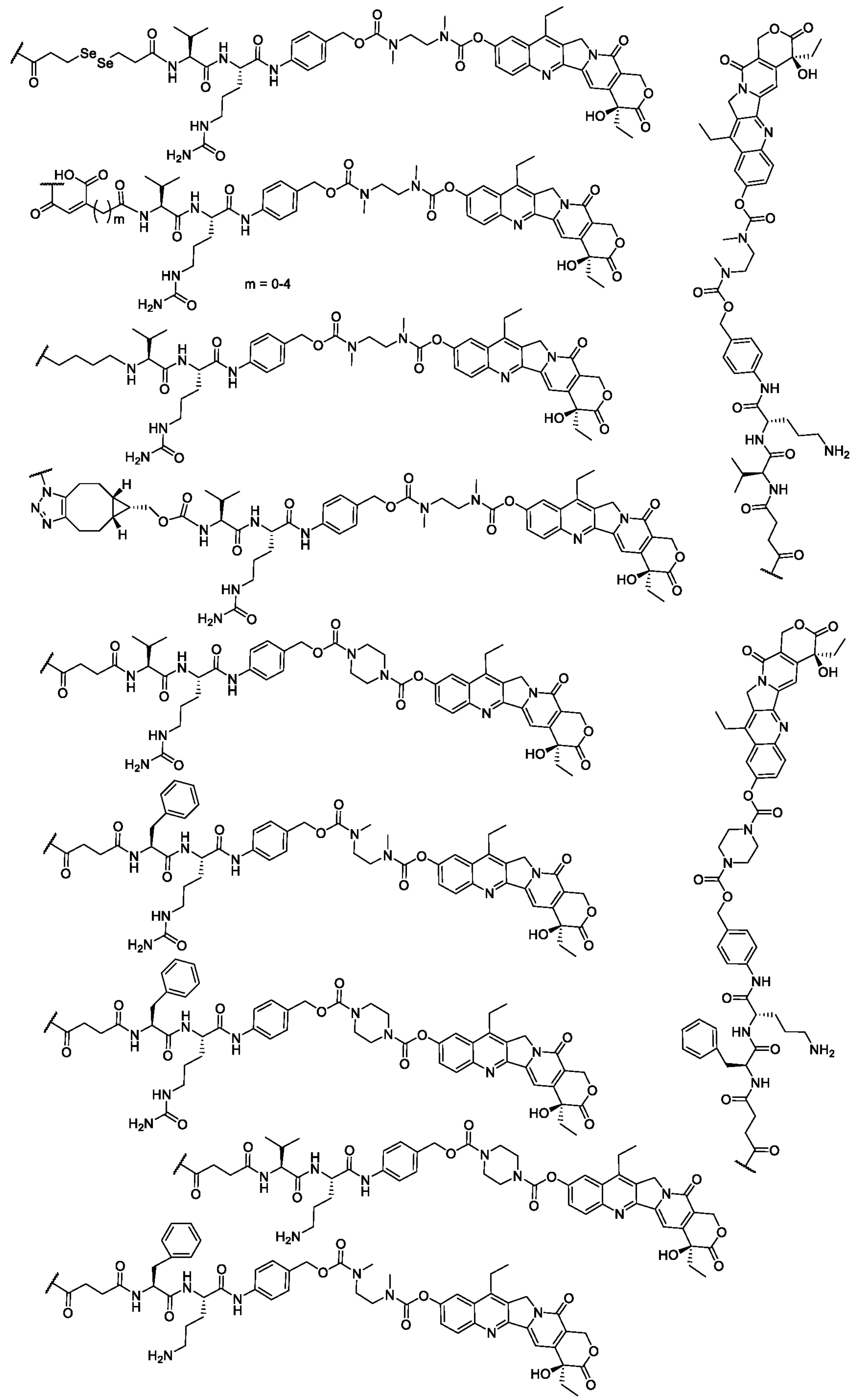

m = 0-4

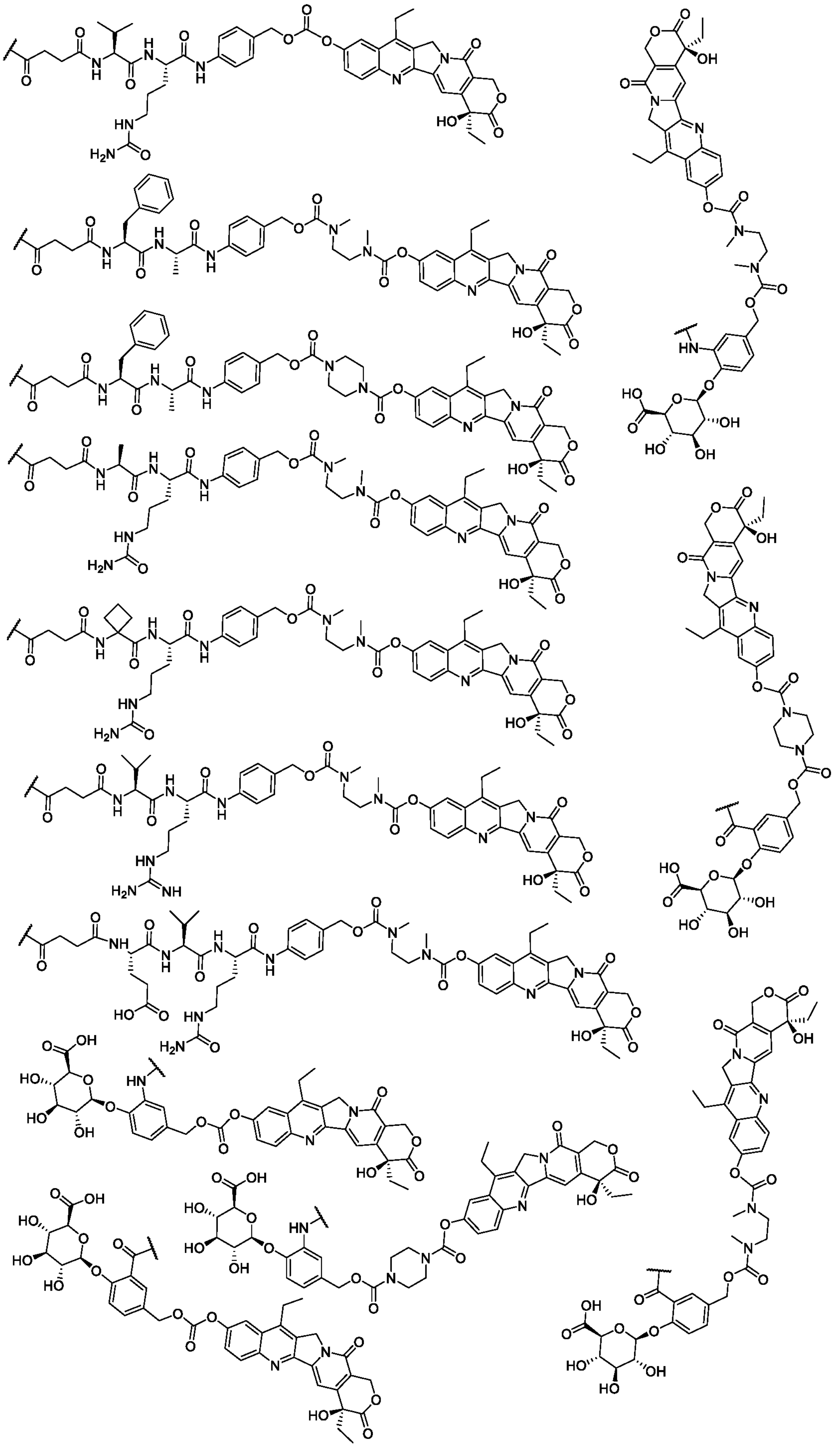

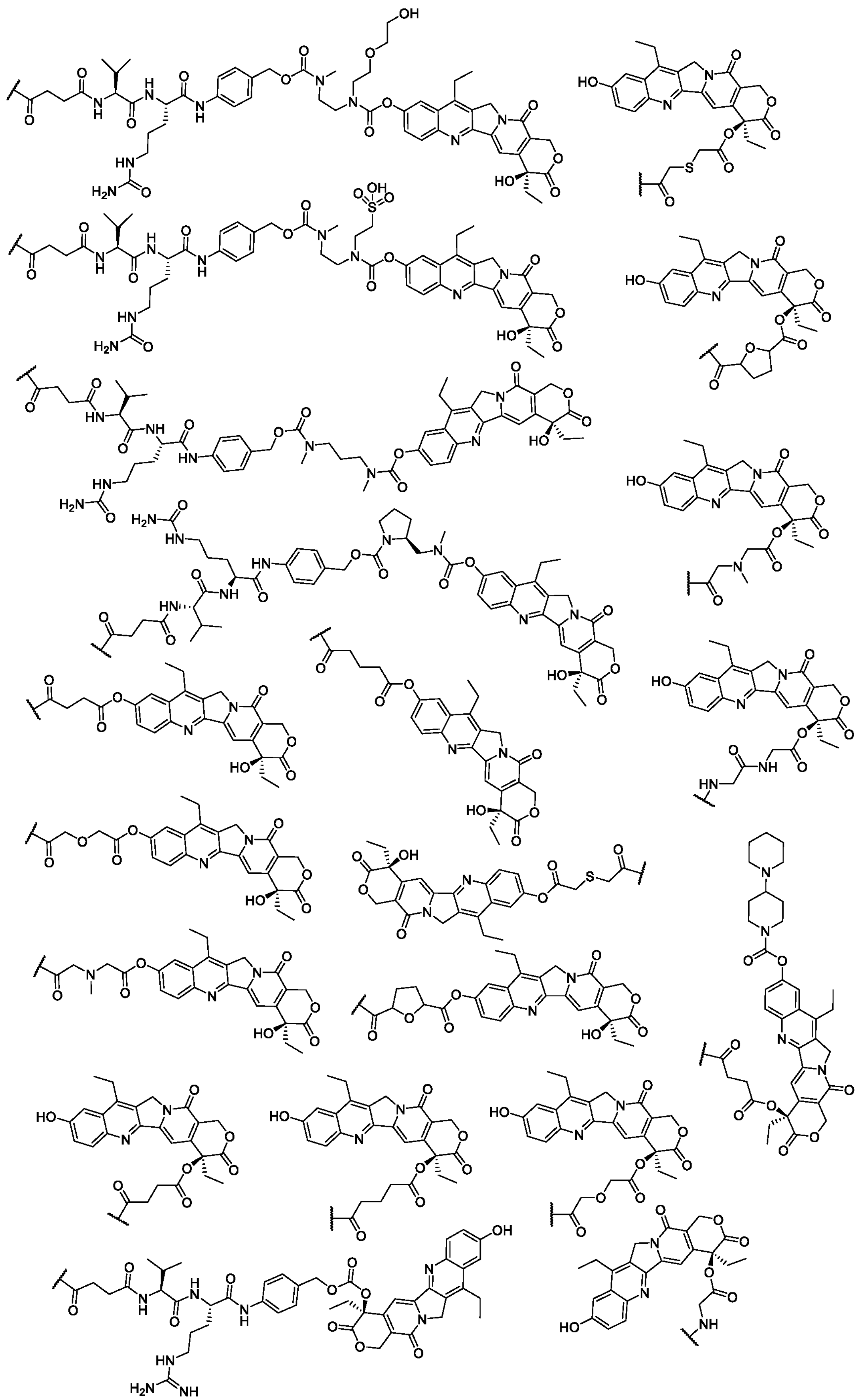

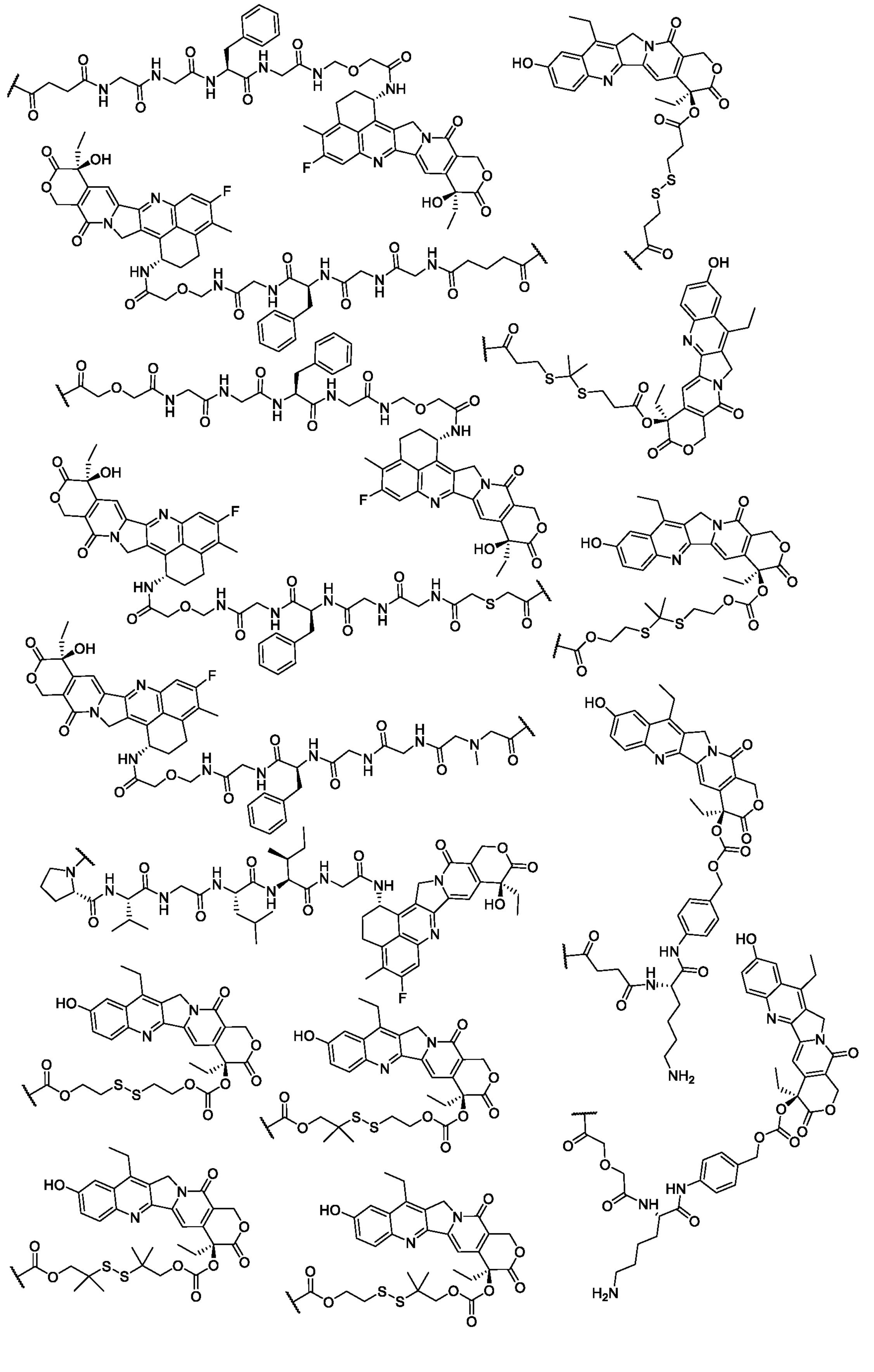

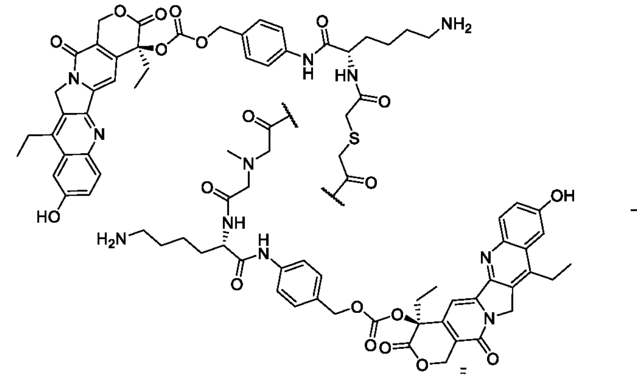
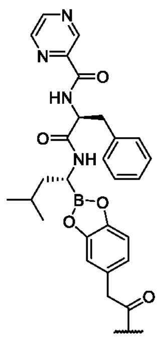
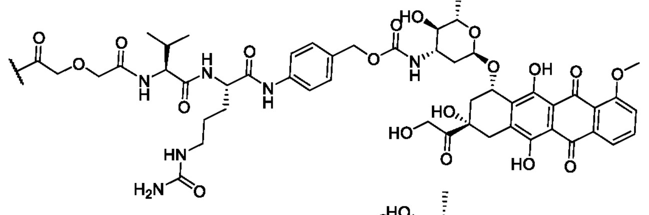
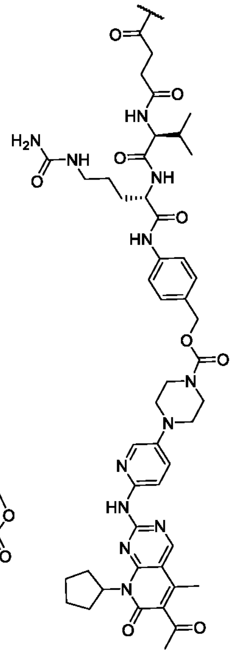
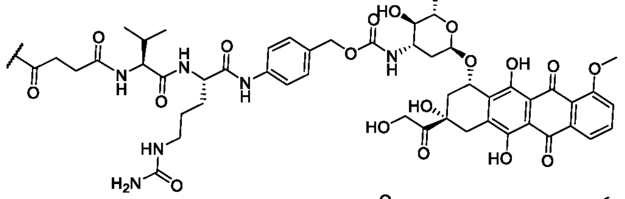
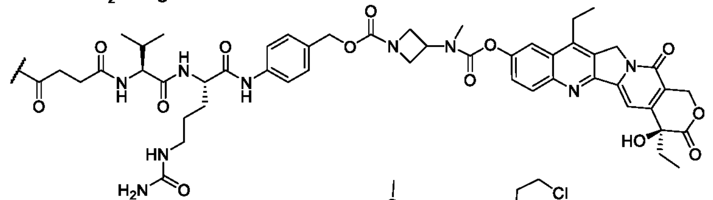
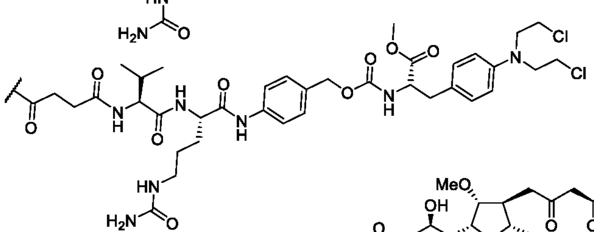
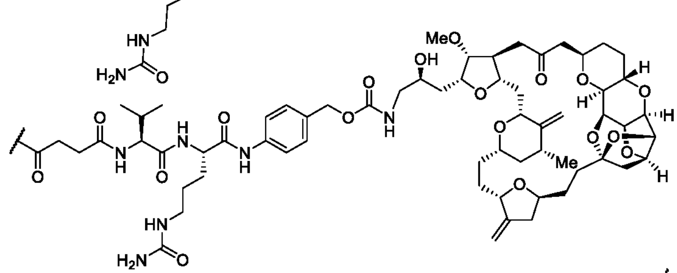
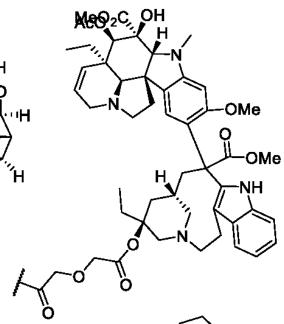
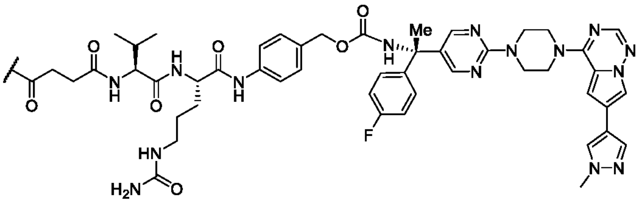
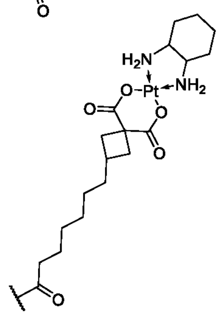
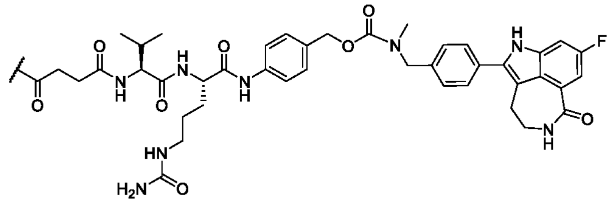

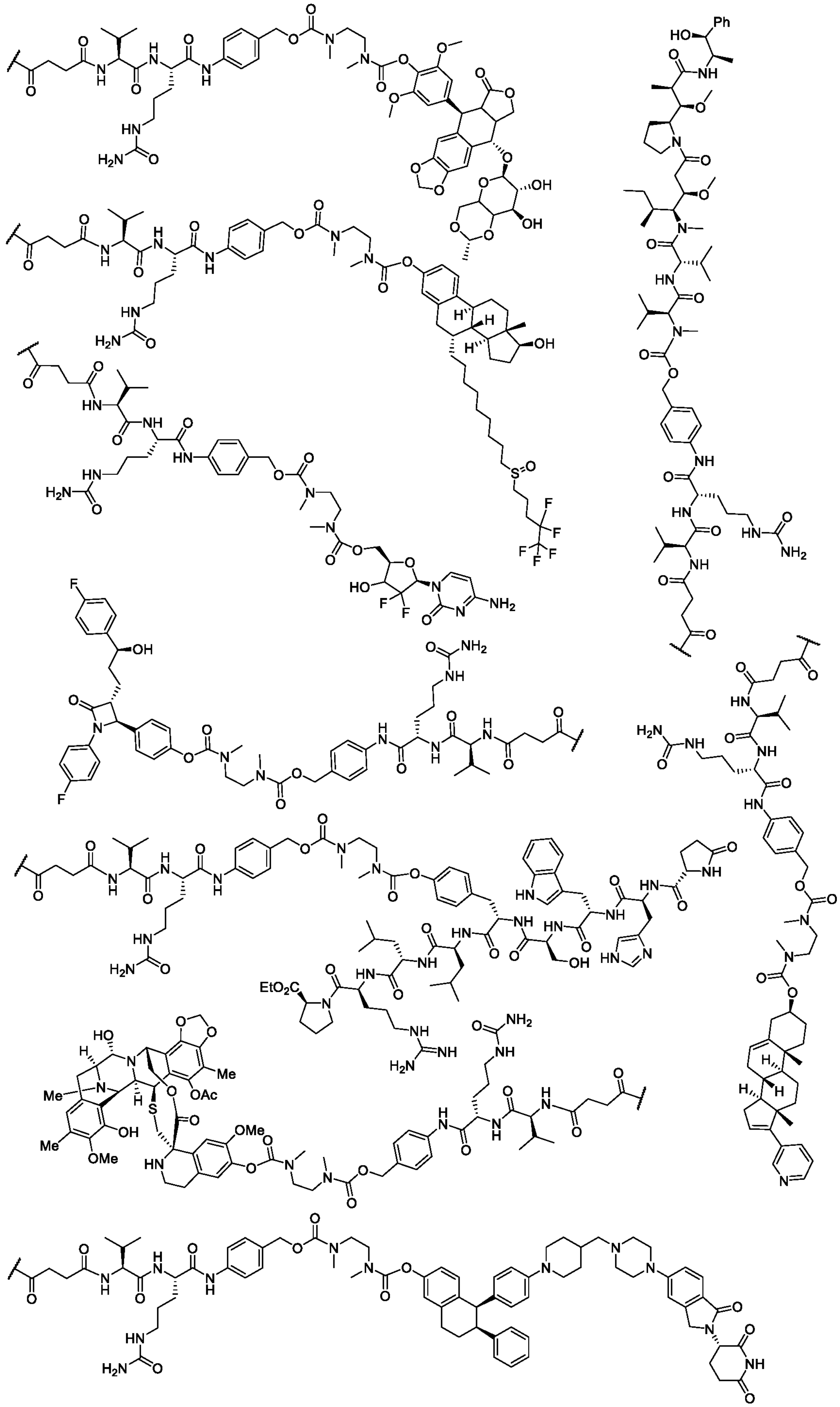

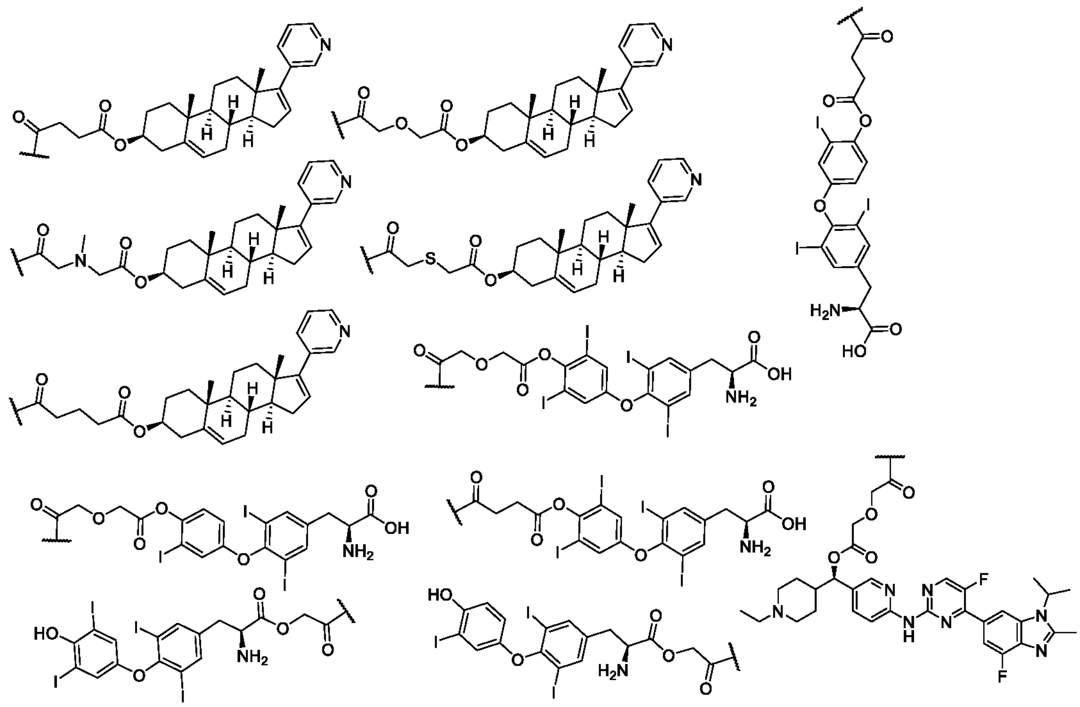

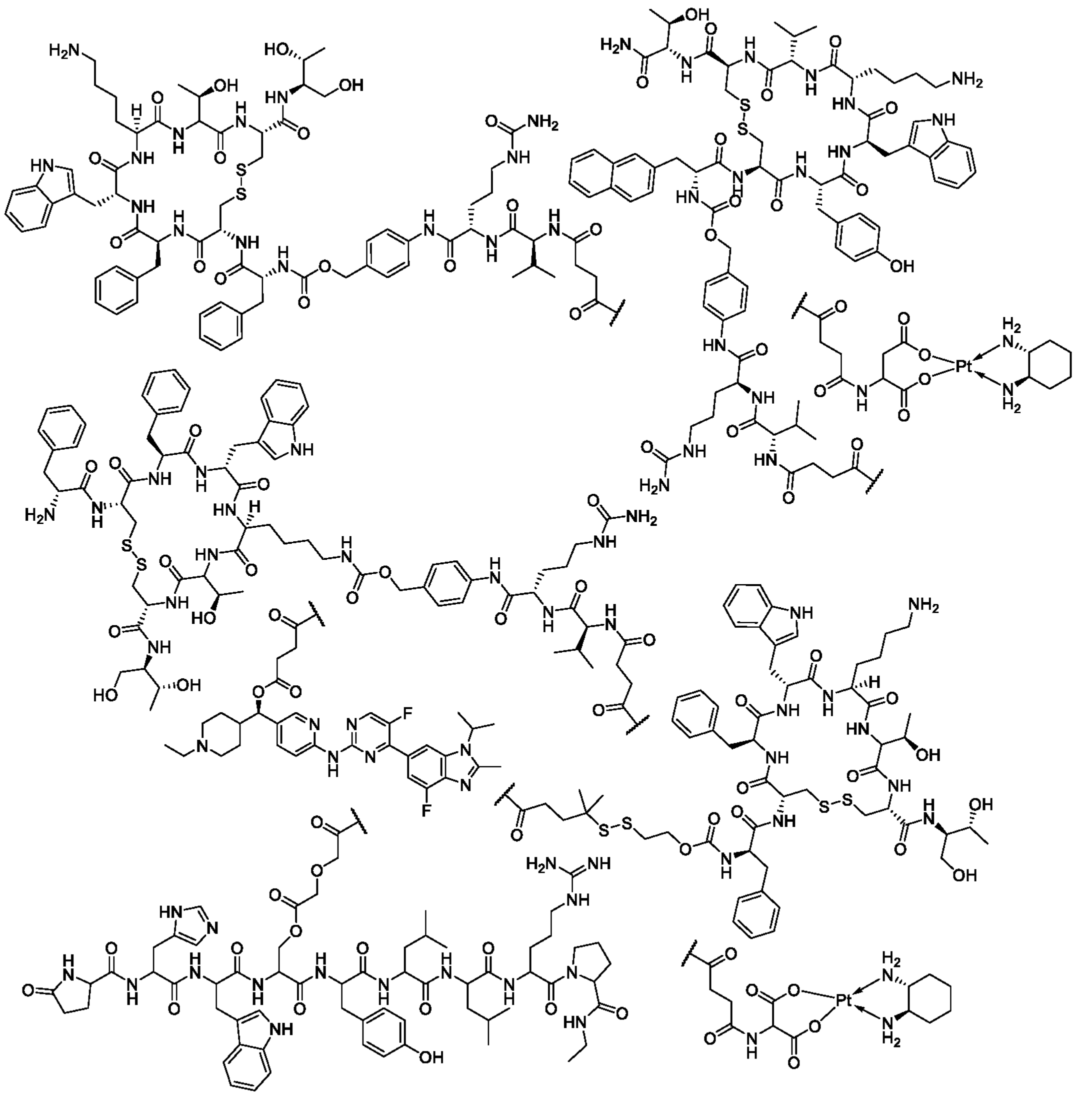

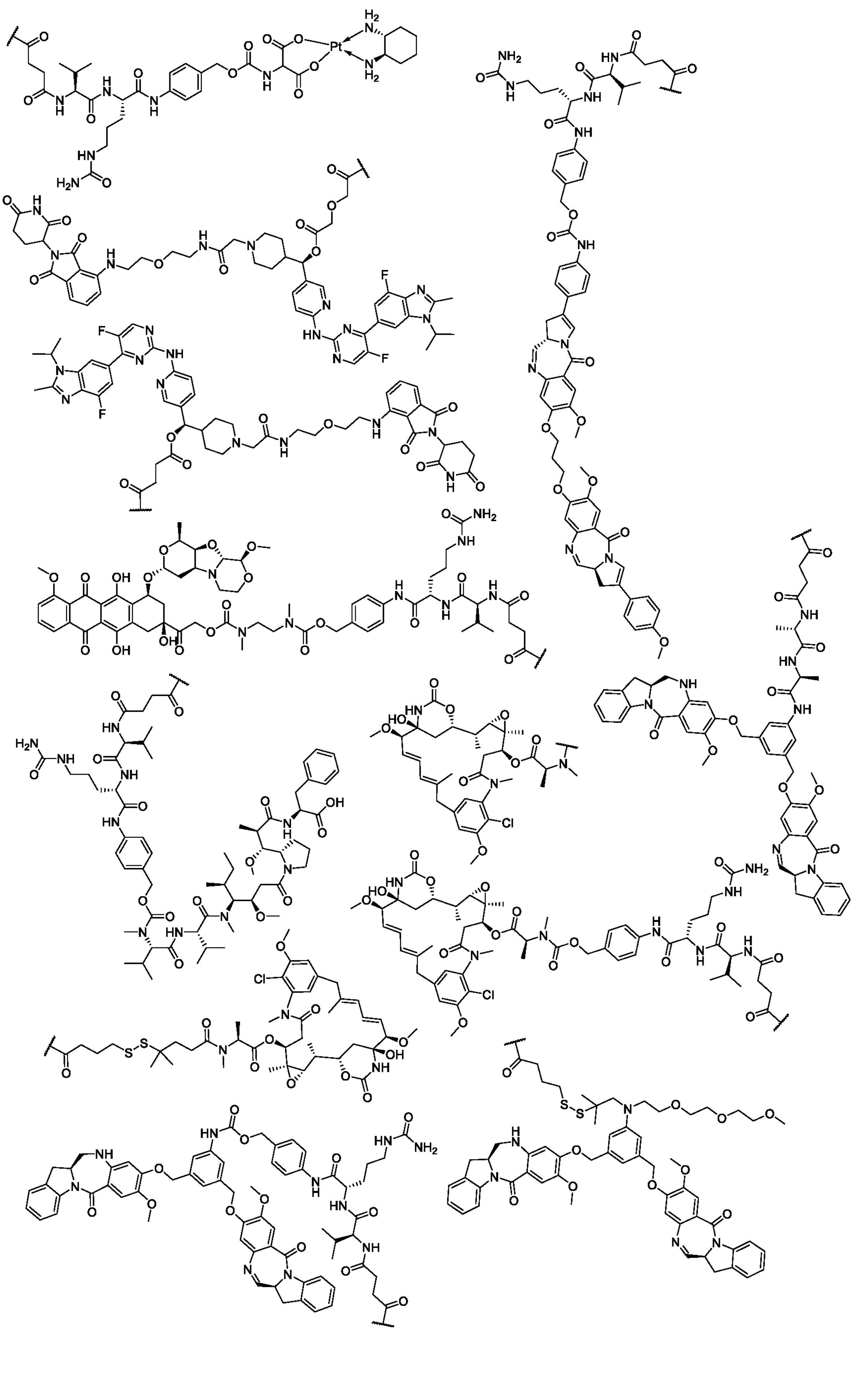

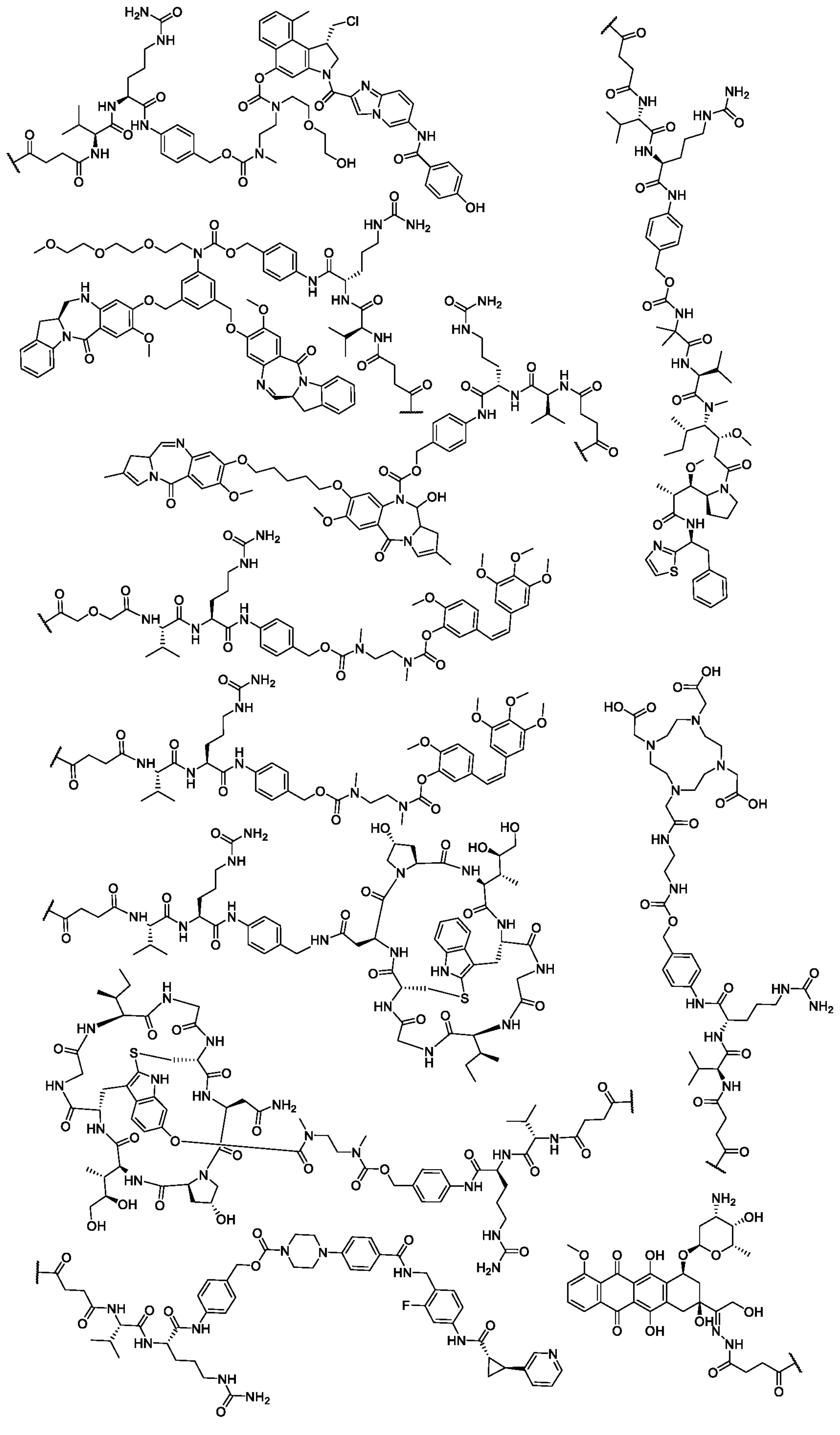

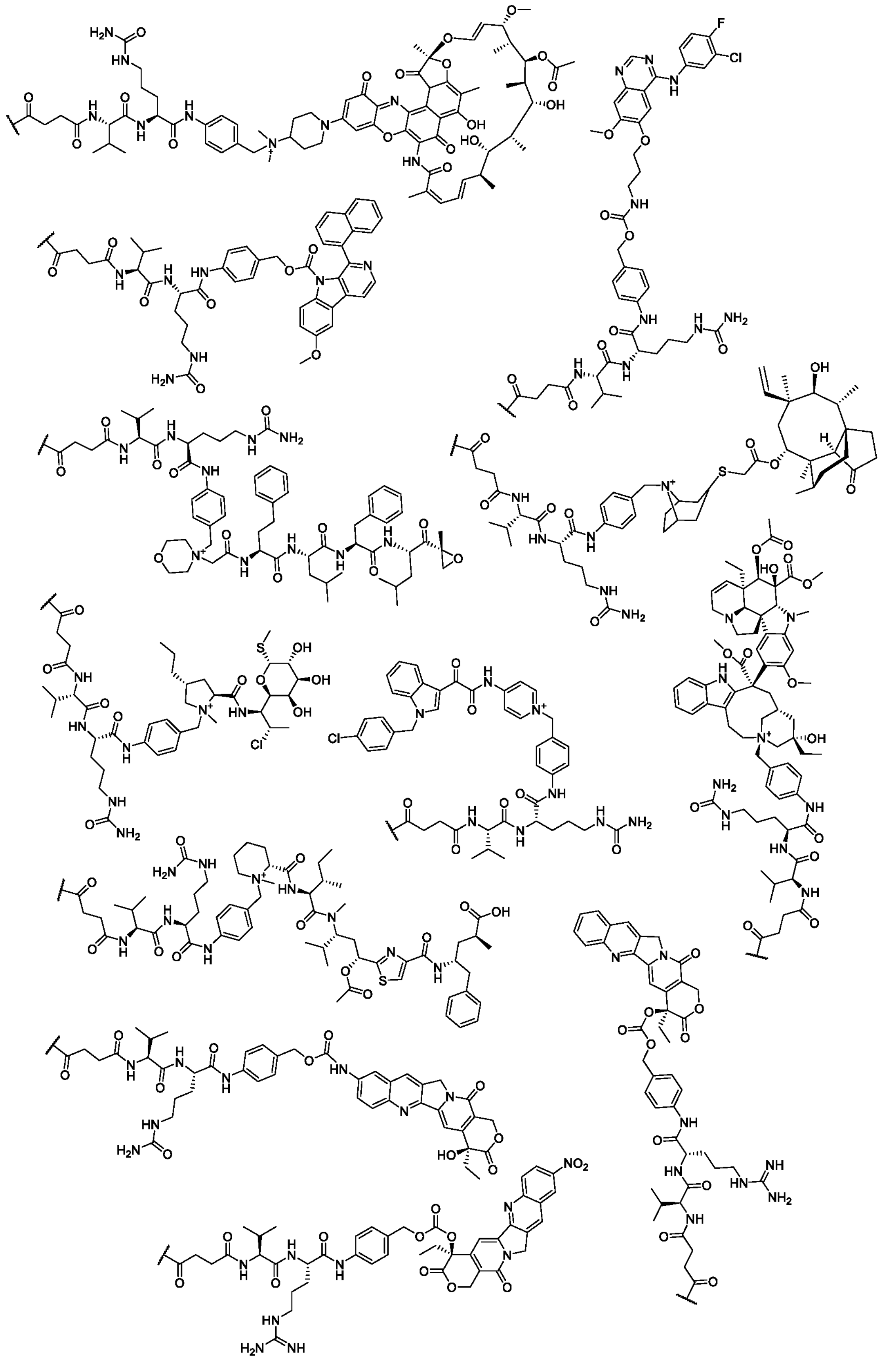

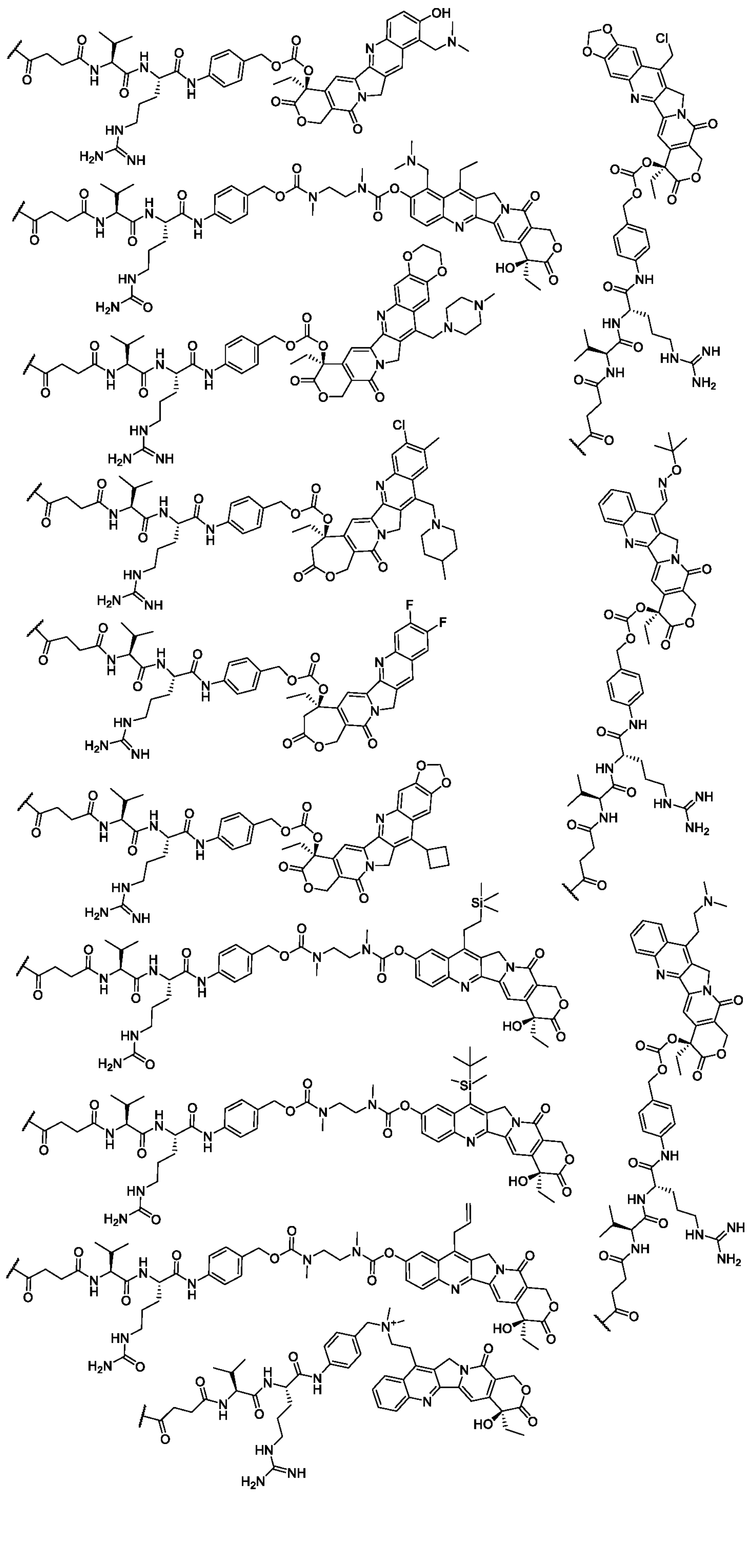

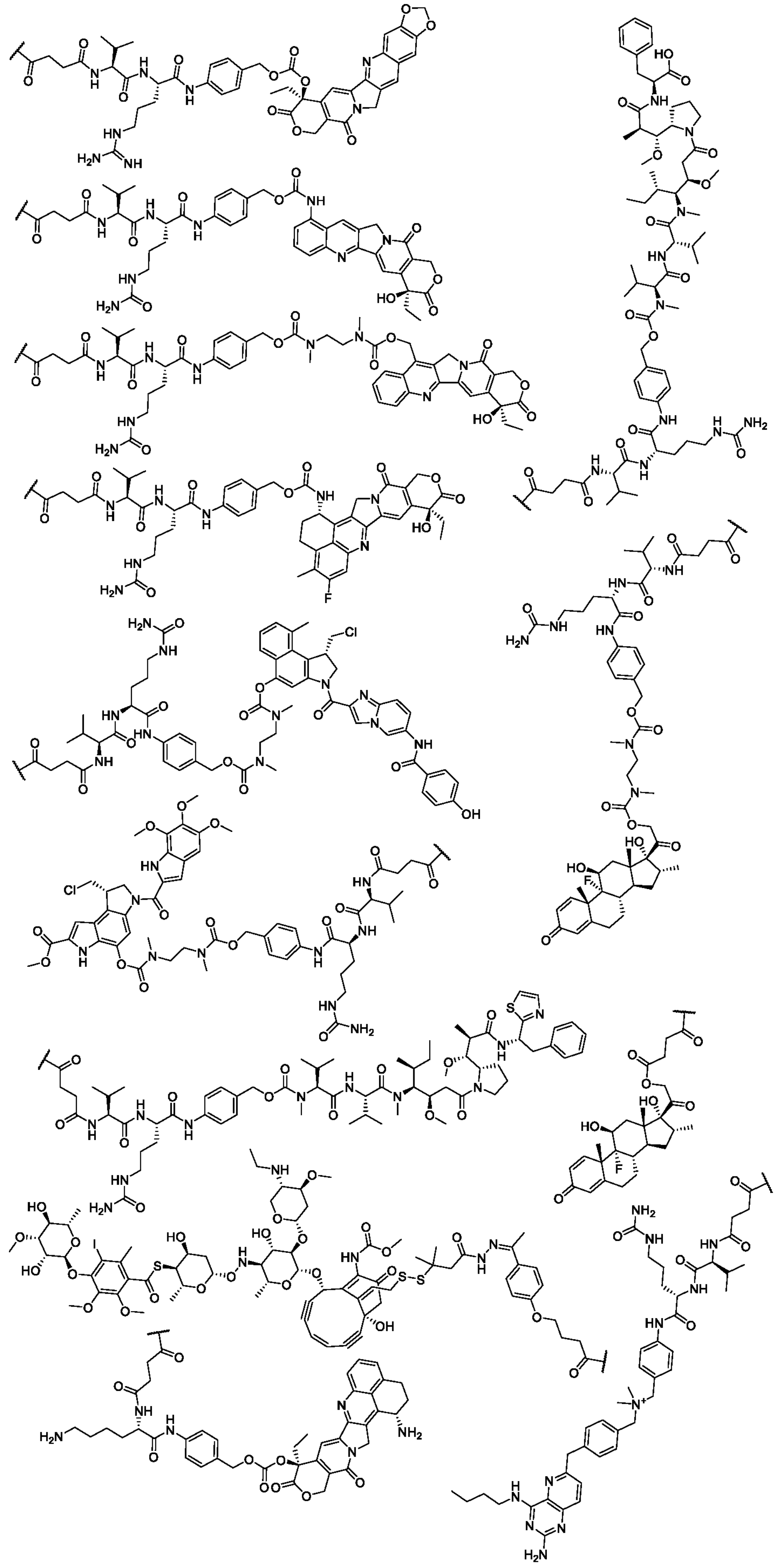

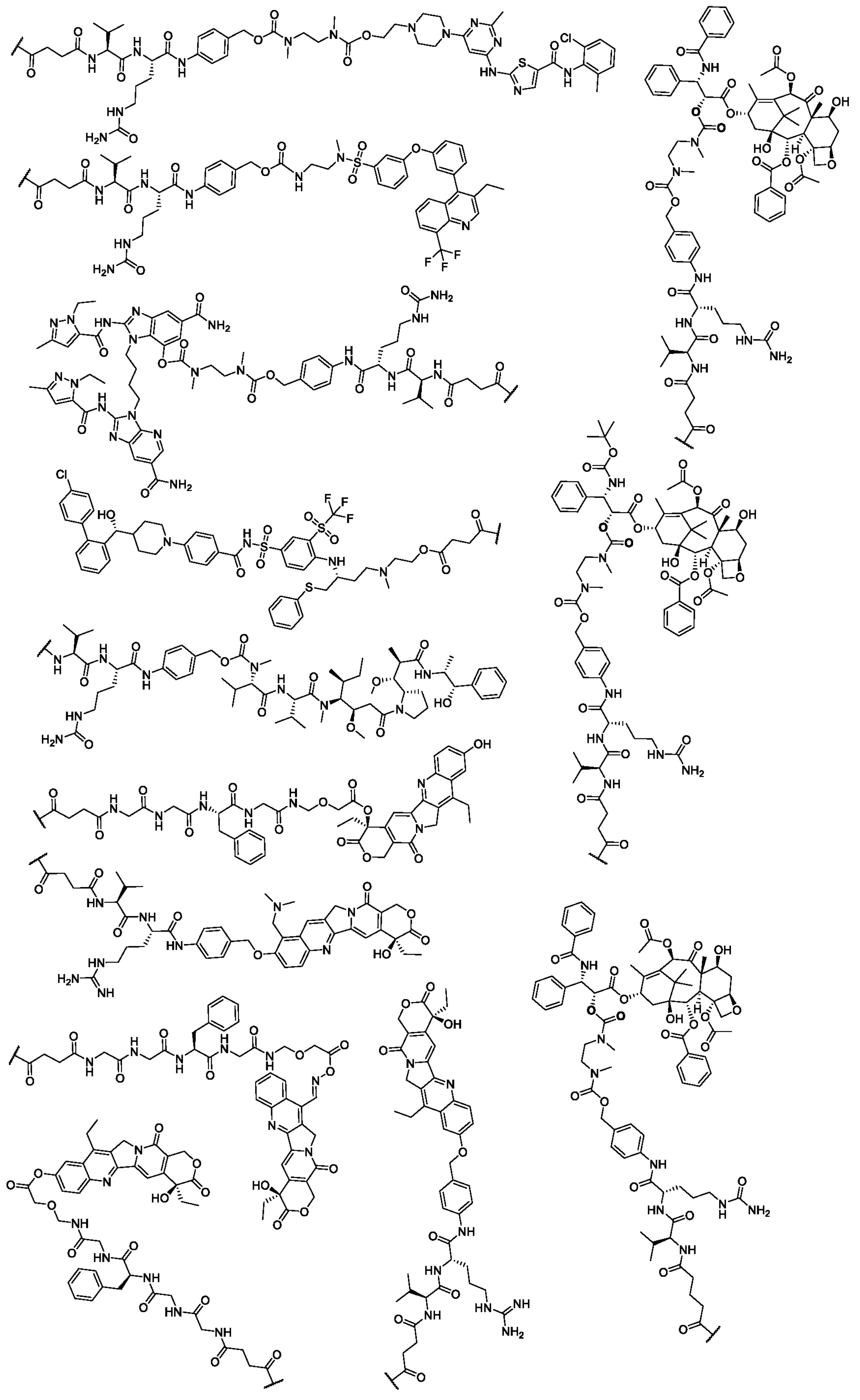

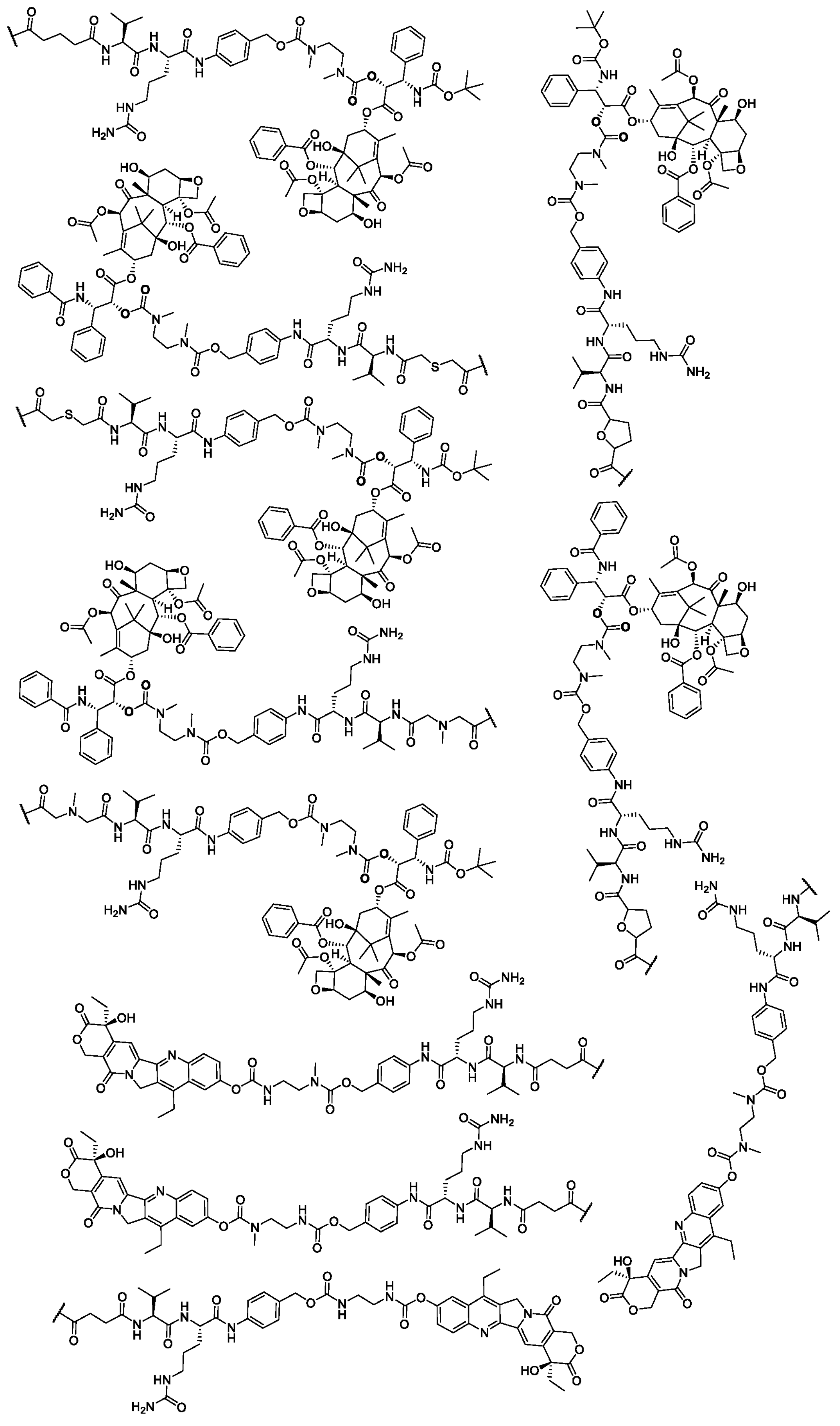

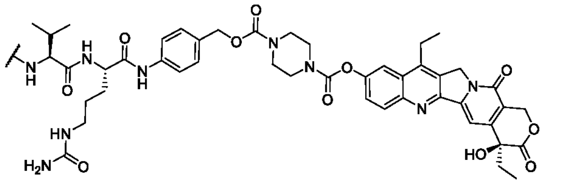

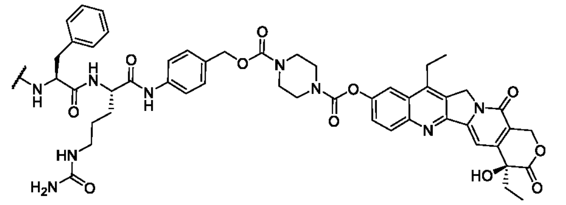

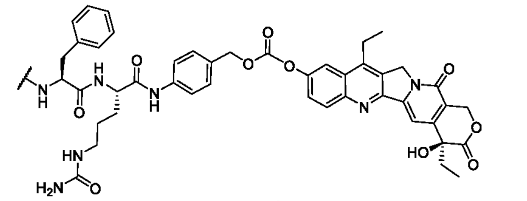

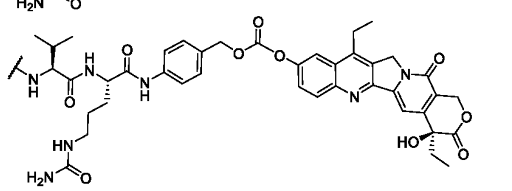

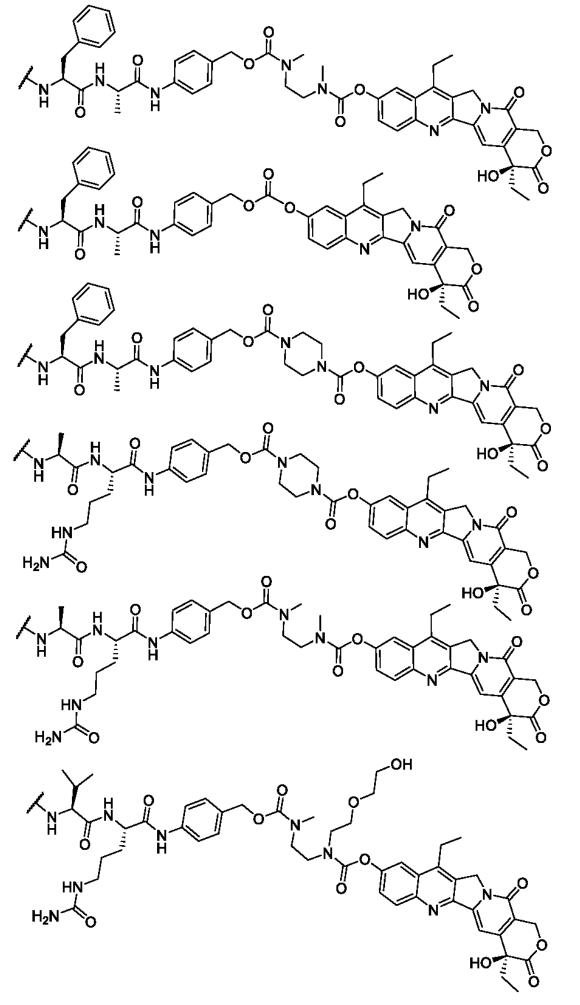

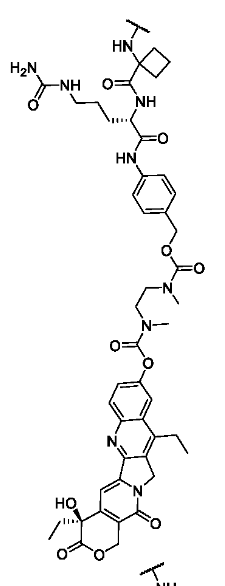

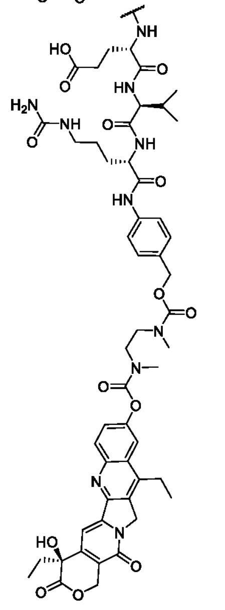

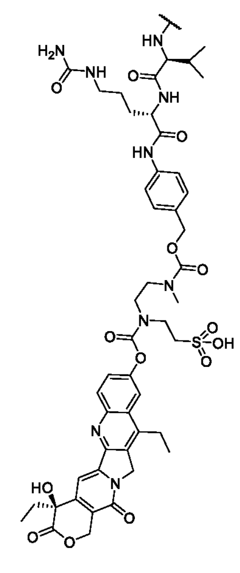

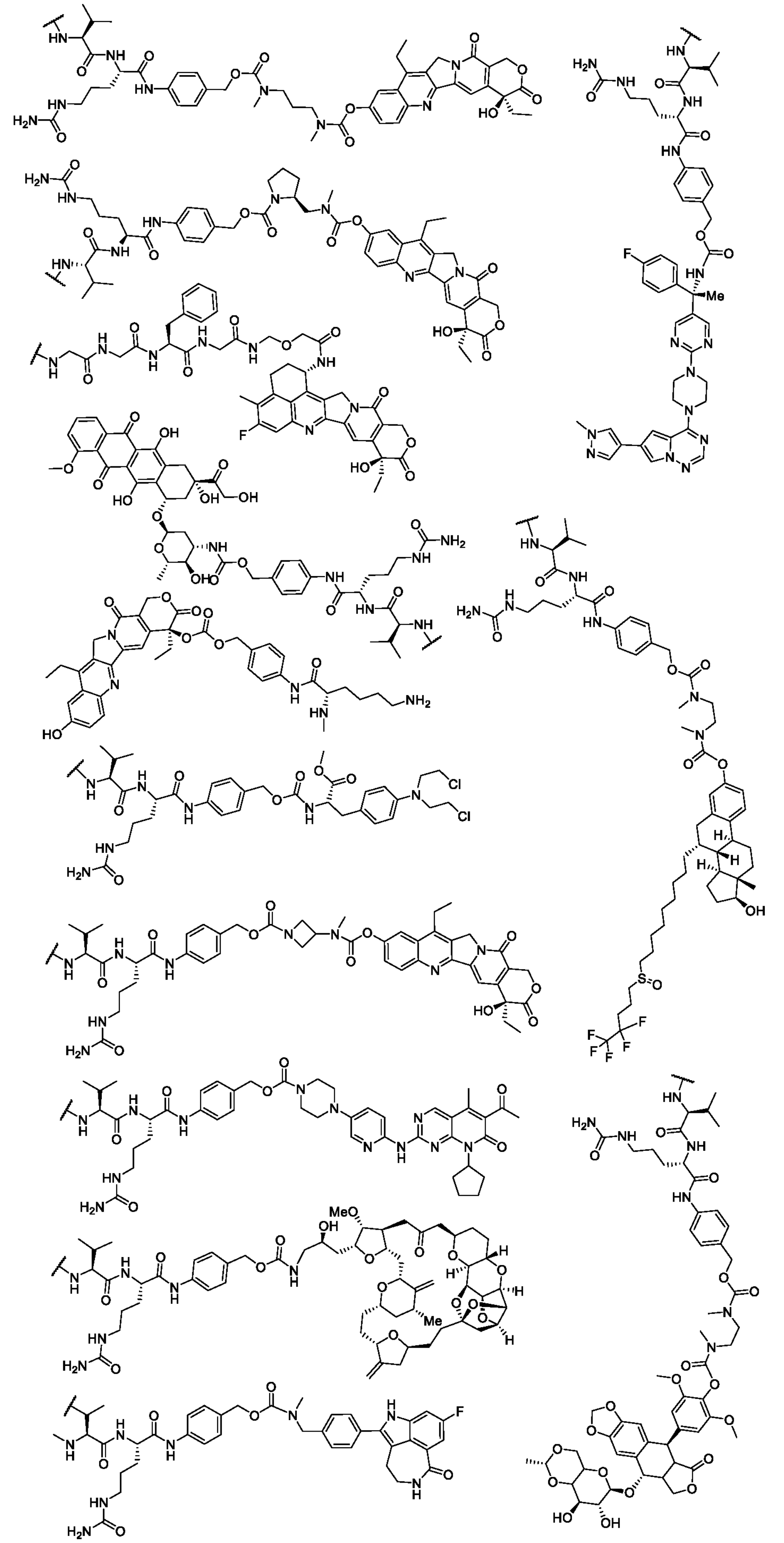

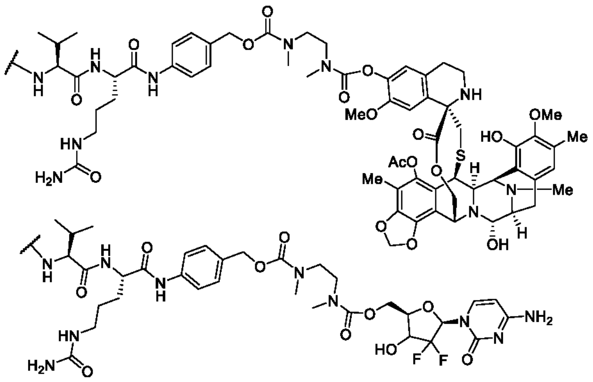

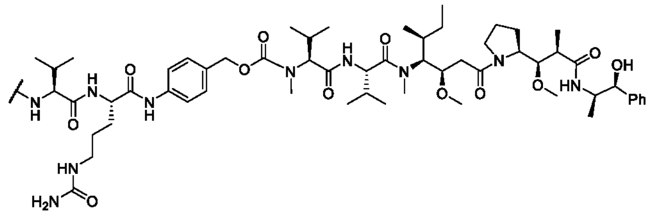

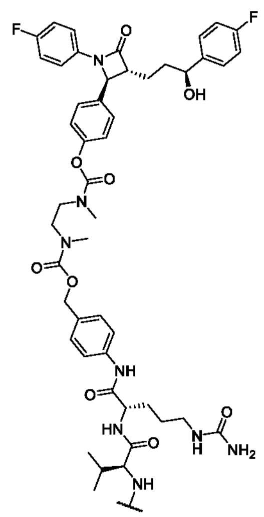

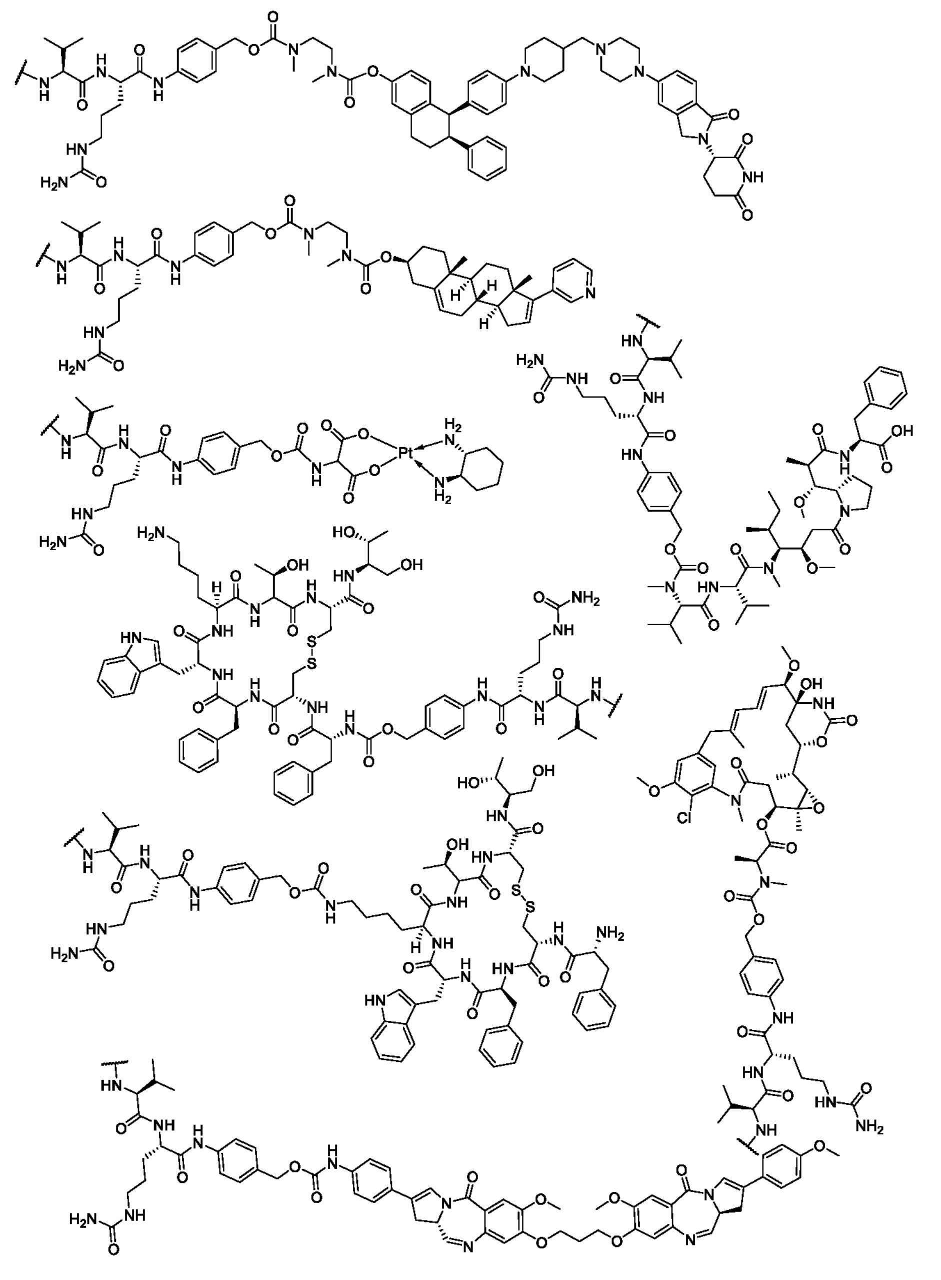

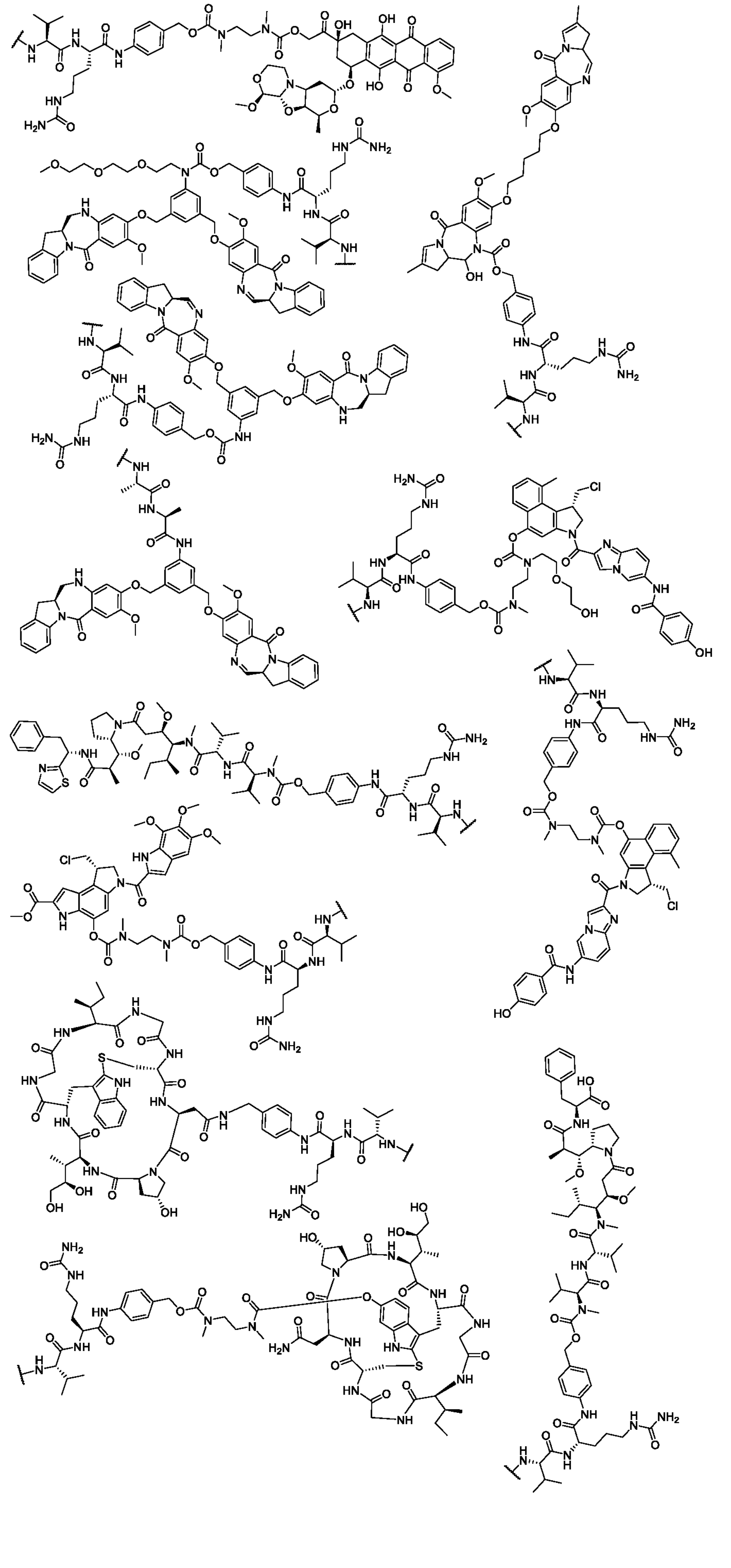

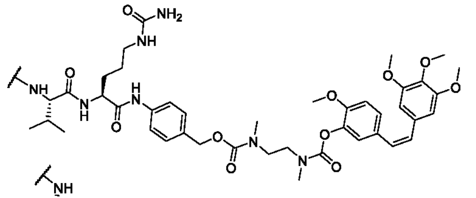

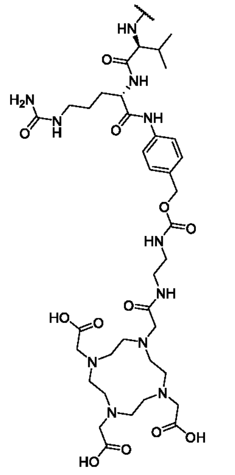

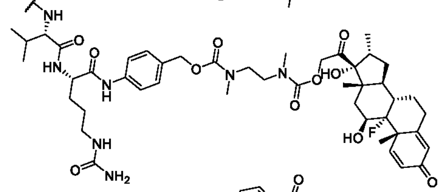

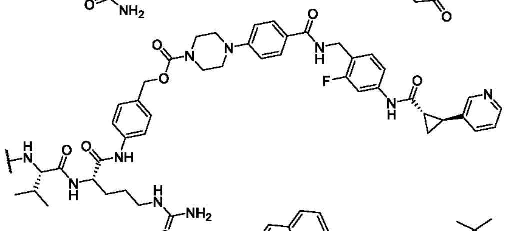

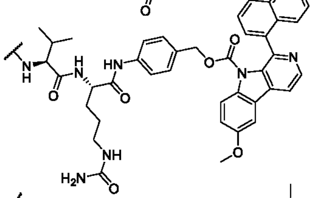

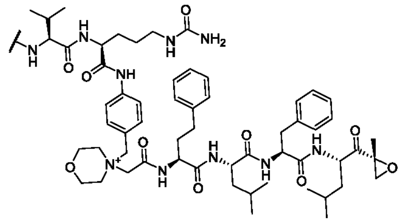

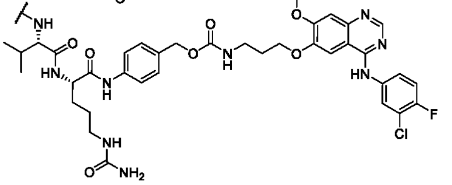

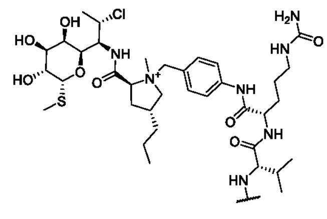

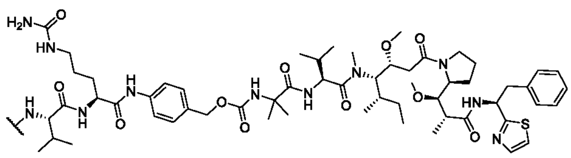

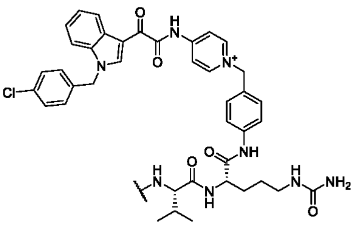

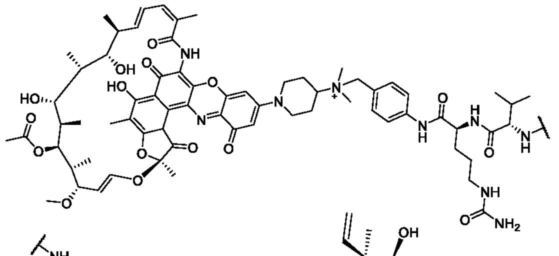

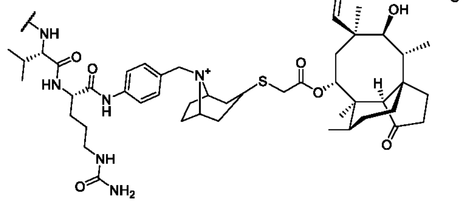

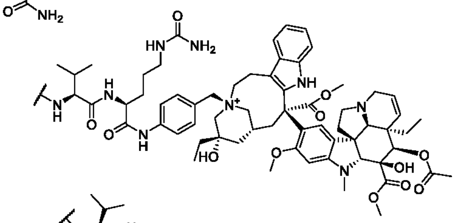

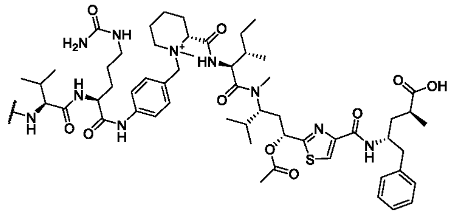

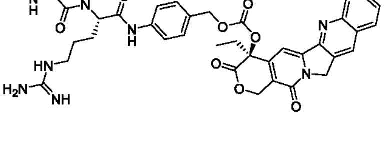

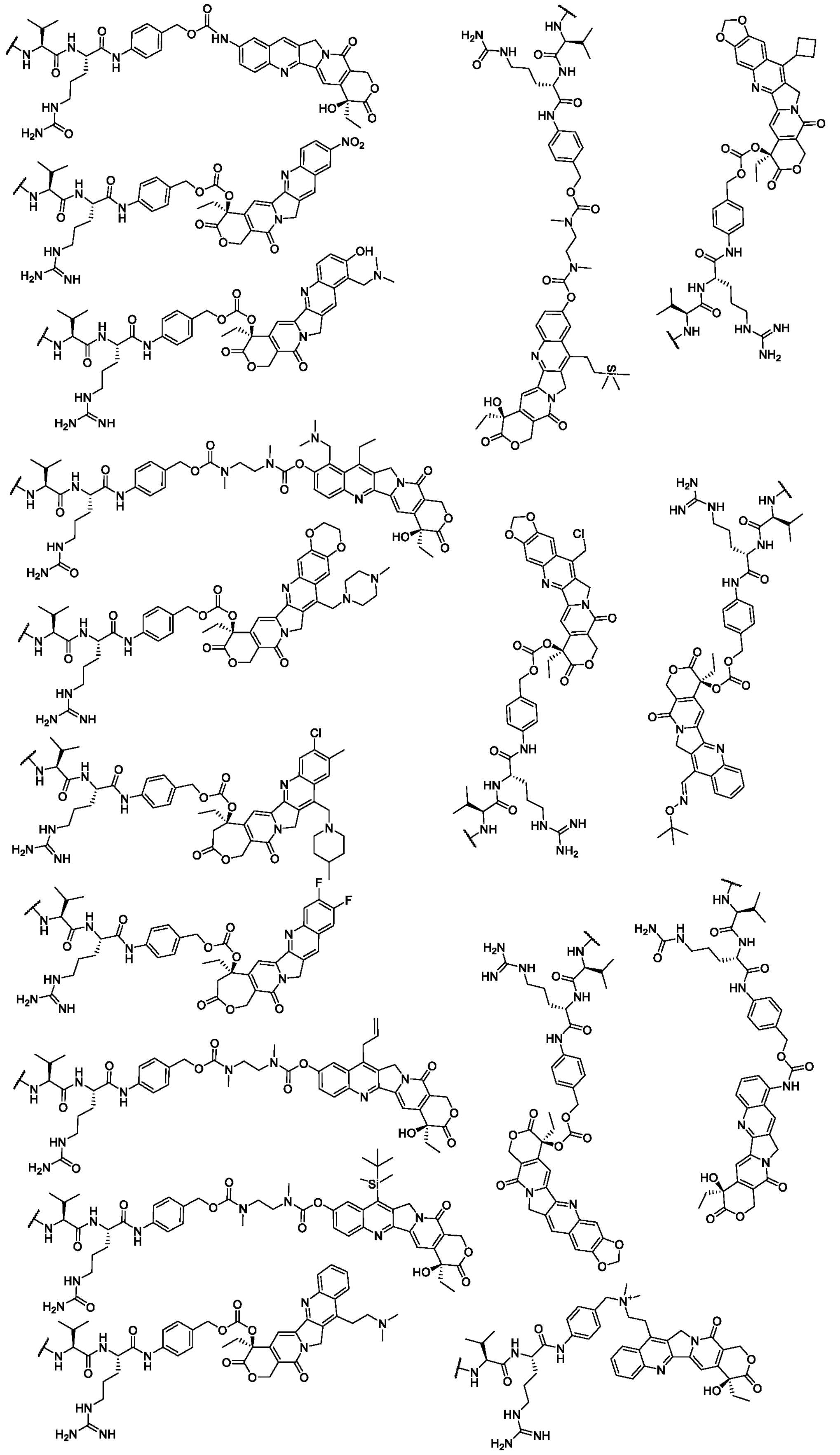

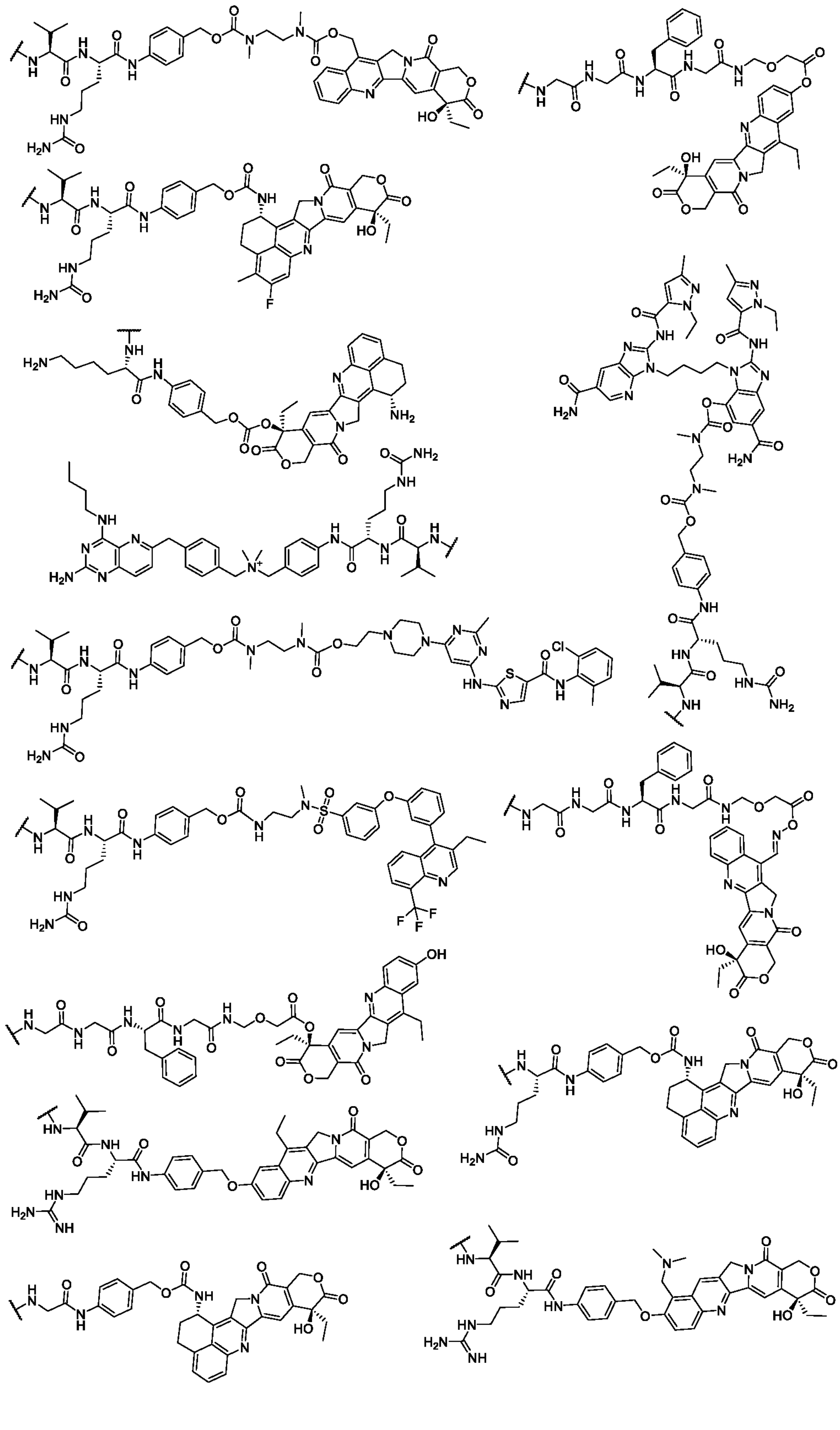

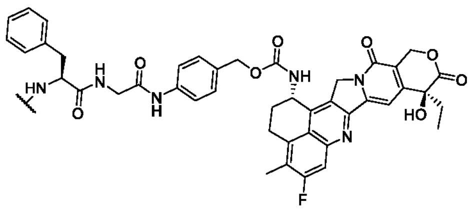

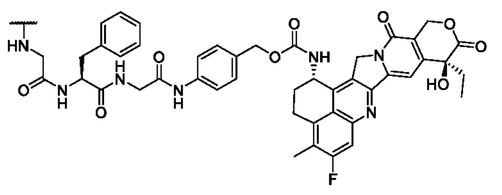

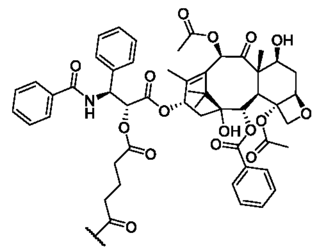

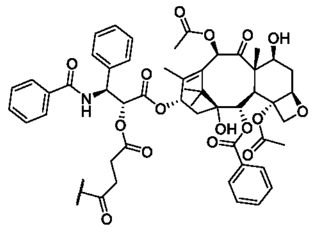

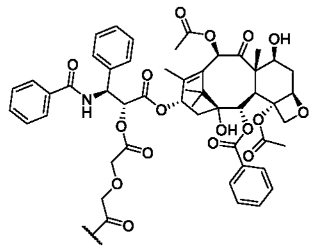

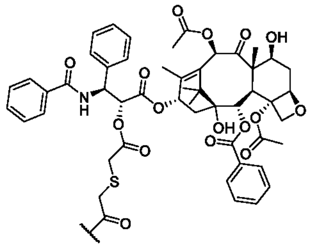

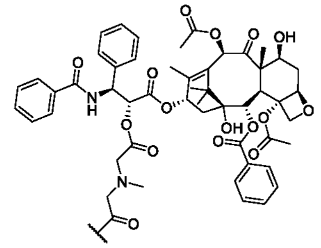

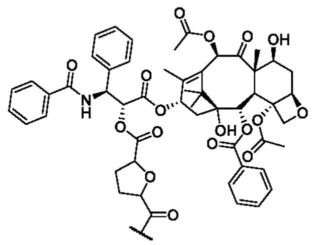

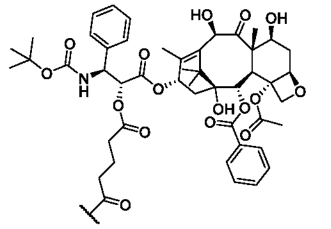

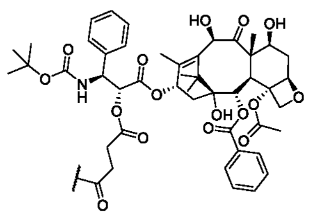

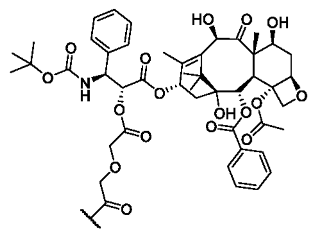

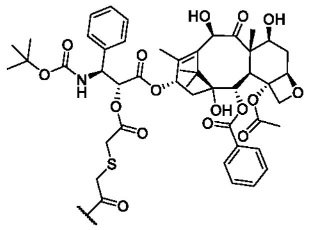

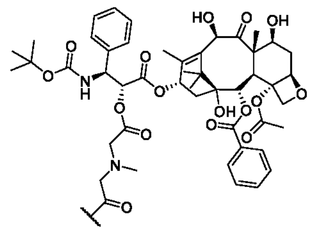

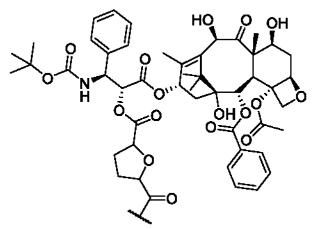

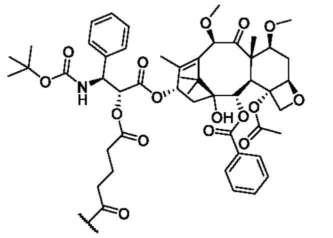

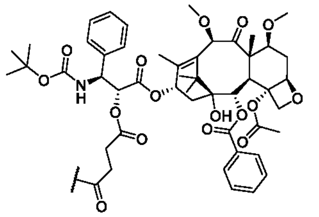

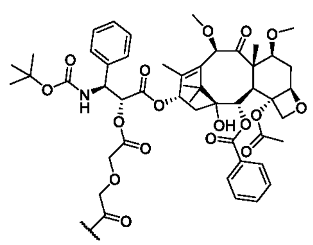

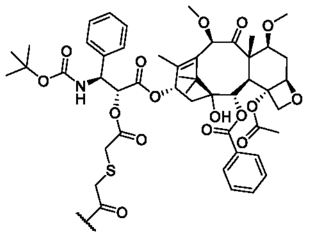
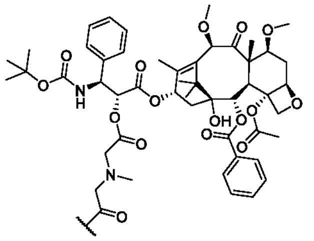
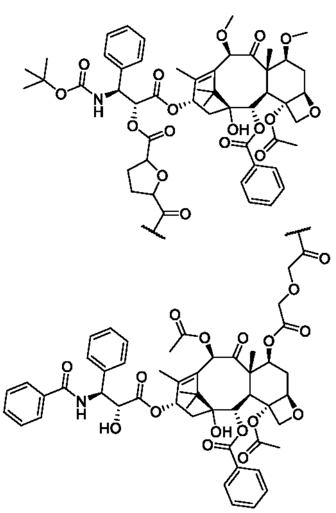
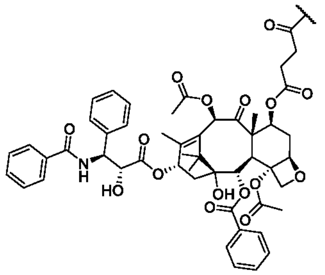
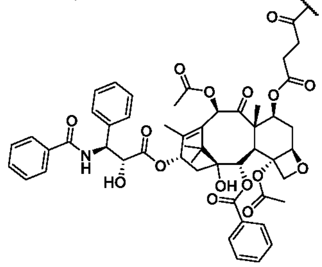

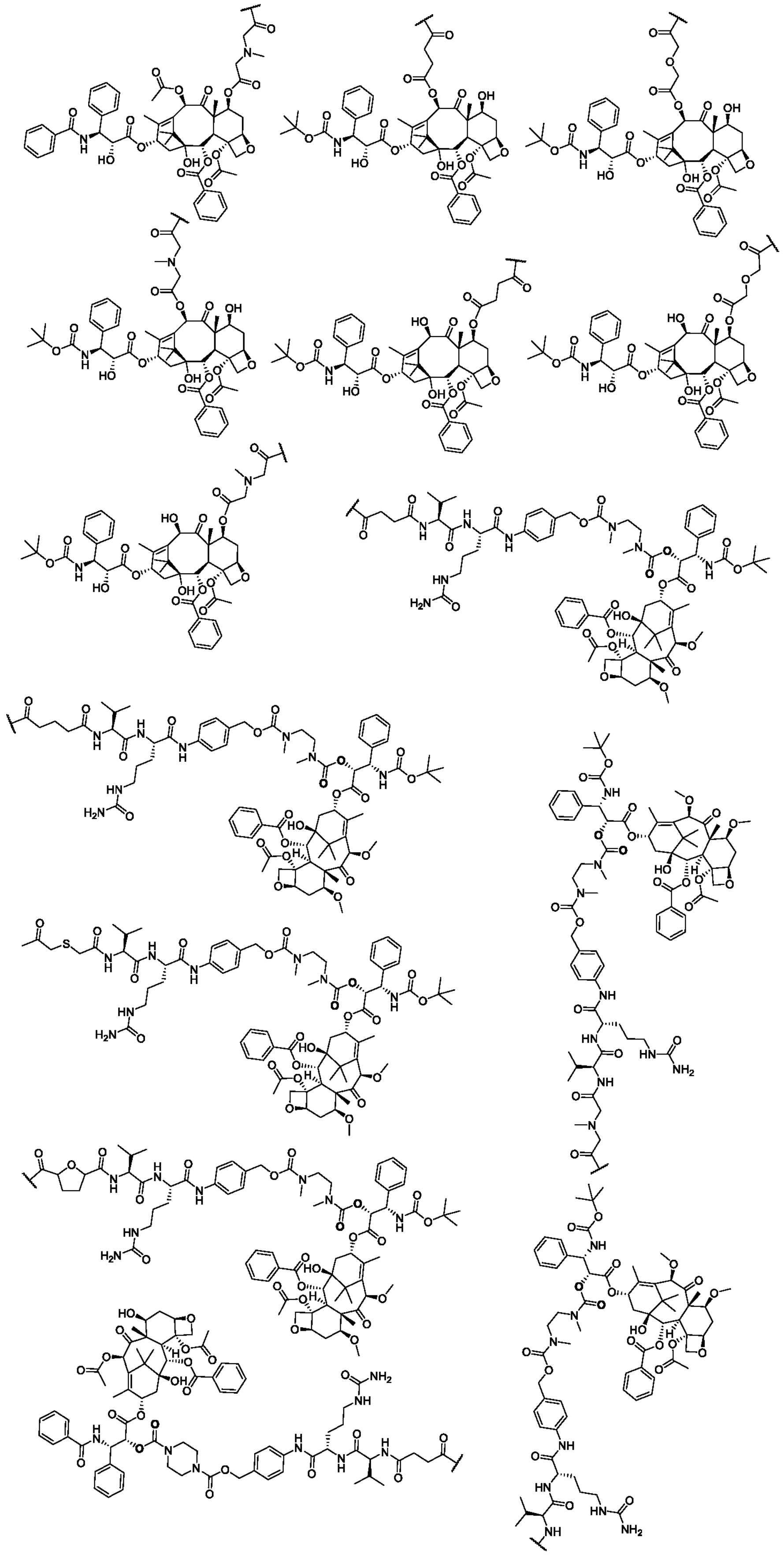

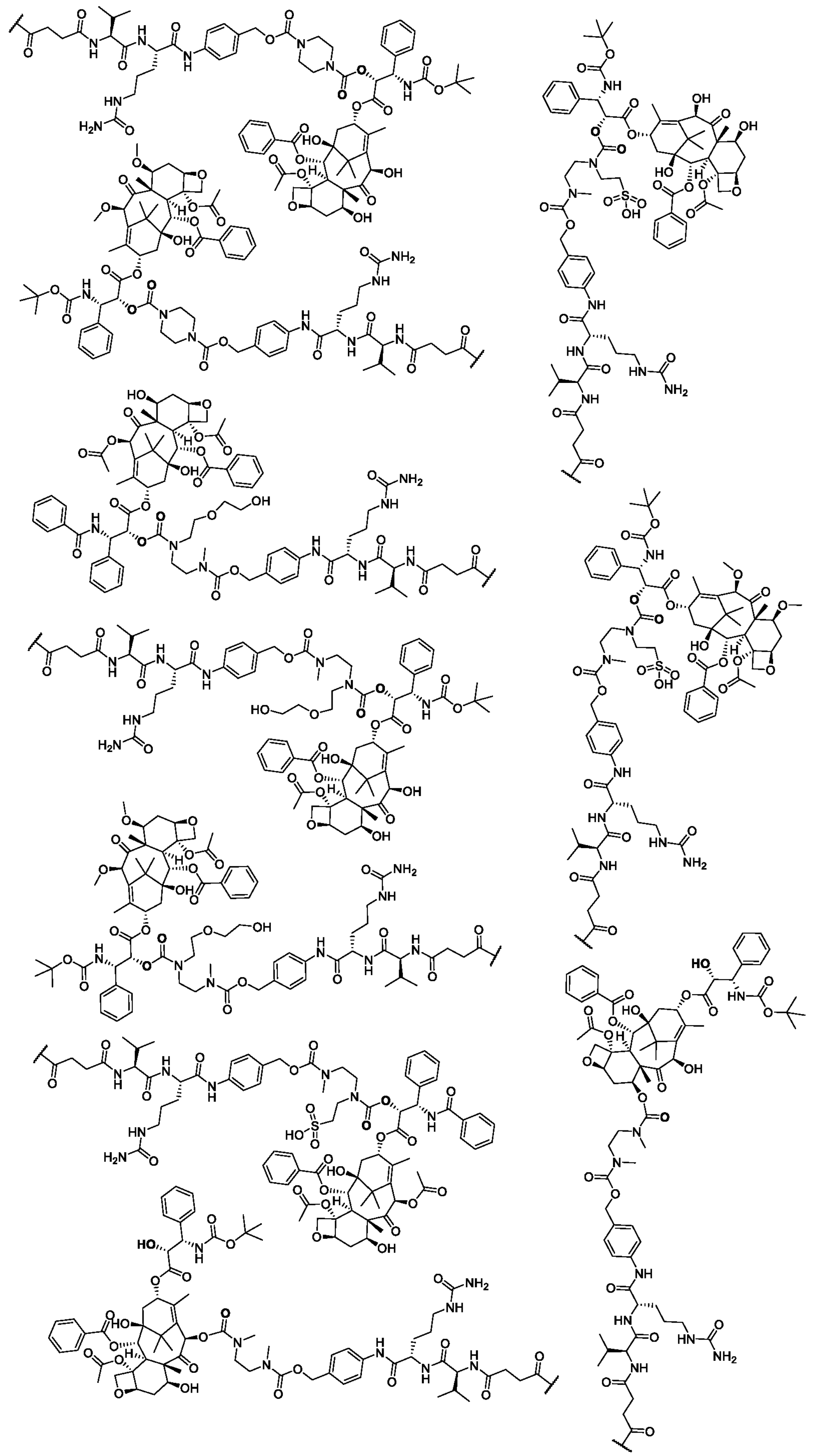

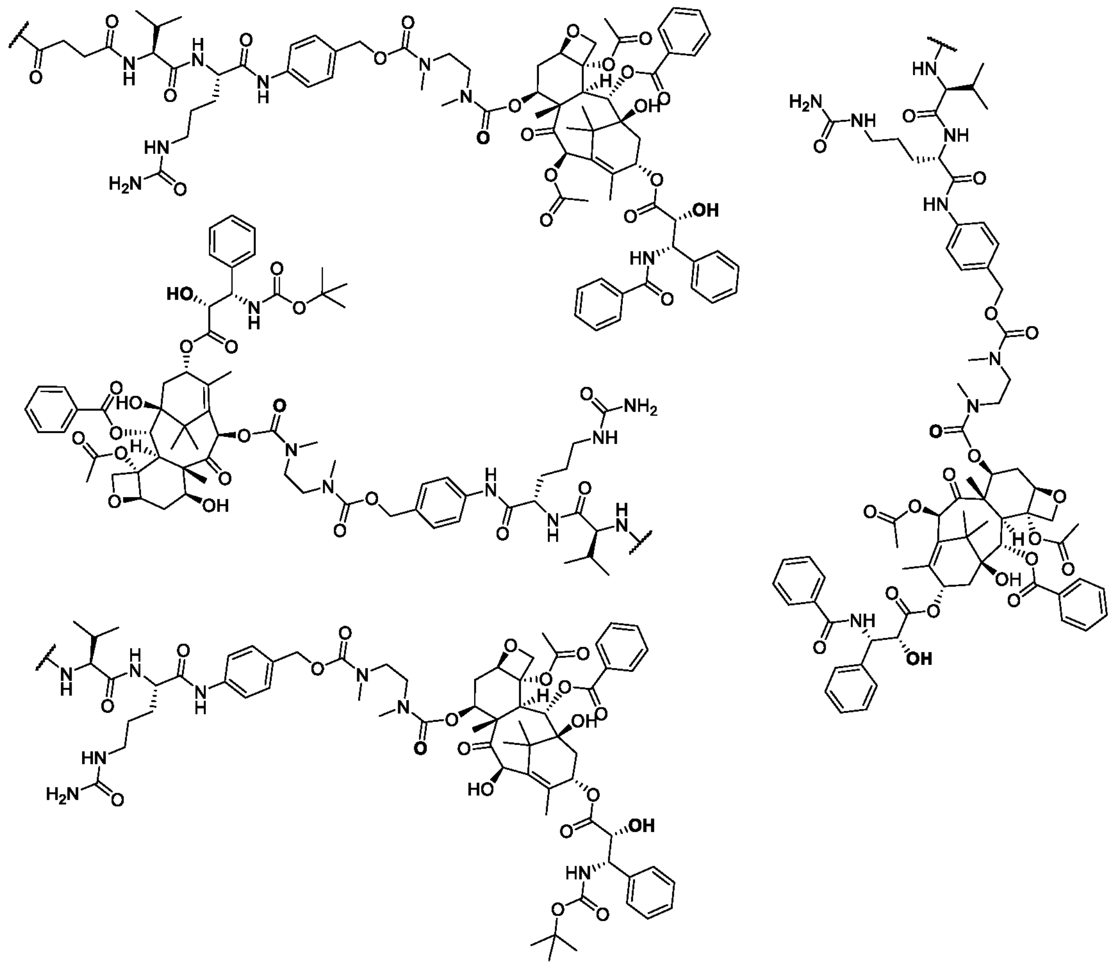

**39.** The poly(ε-L-lysine) derivative-drug conjugate according to claim 1, wherein the poly(ε-L-lysine) derivative-drug conjugate is selected from the following structures:

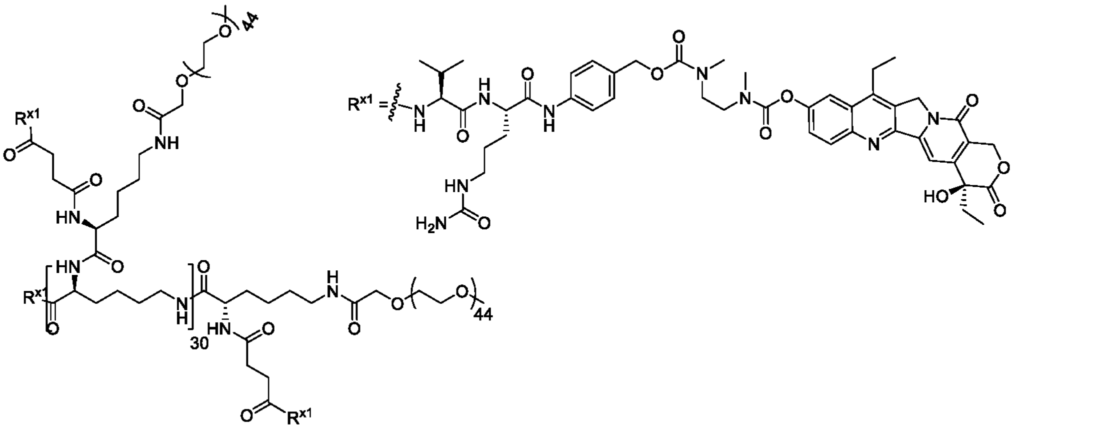

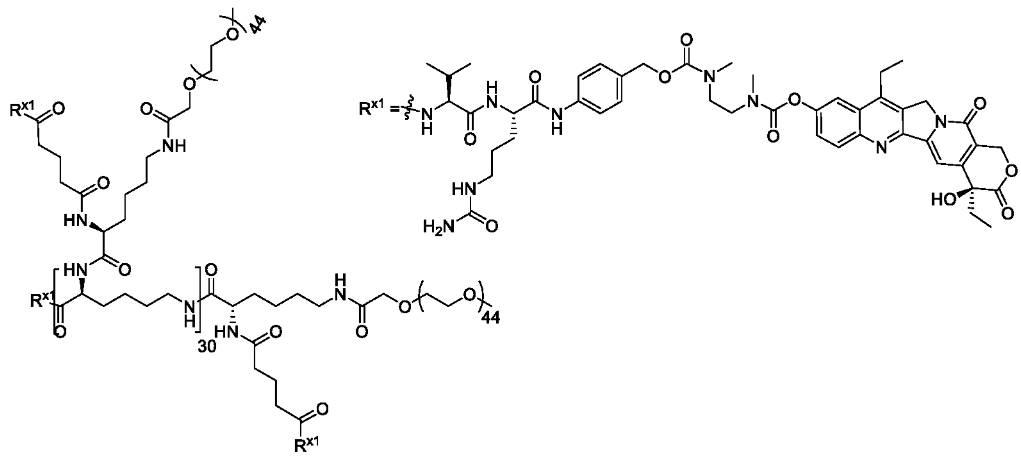

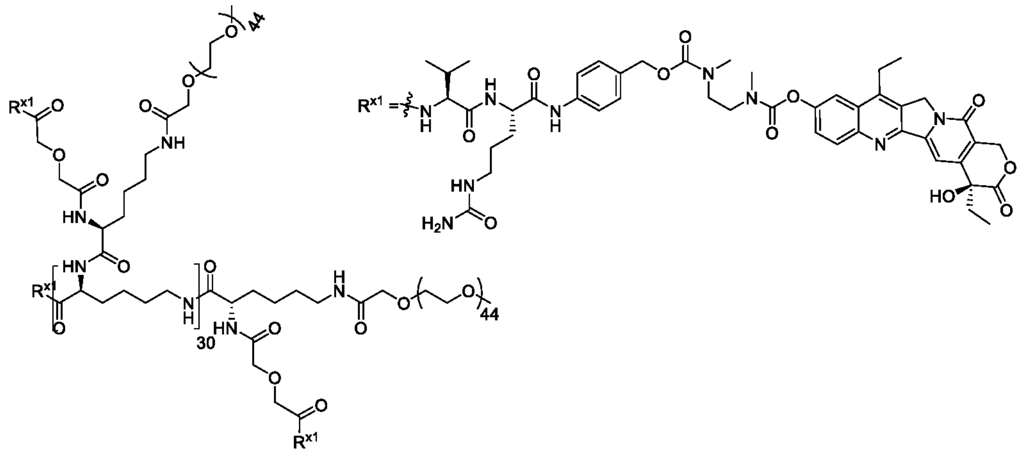

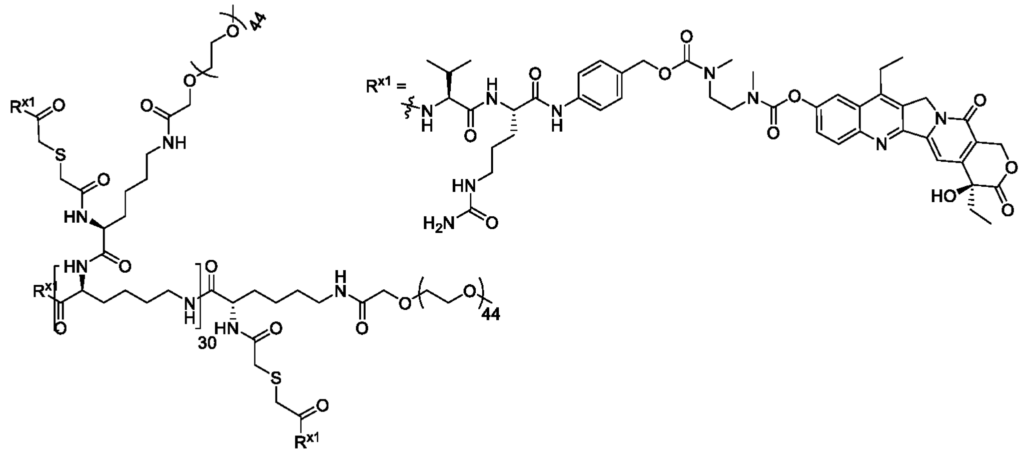

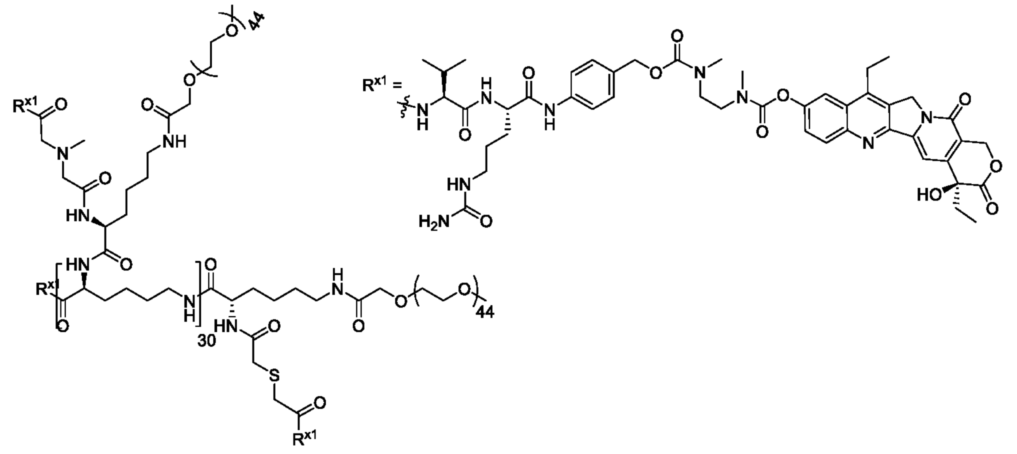

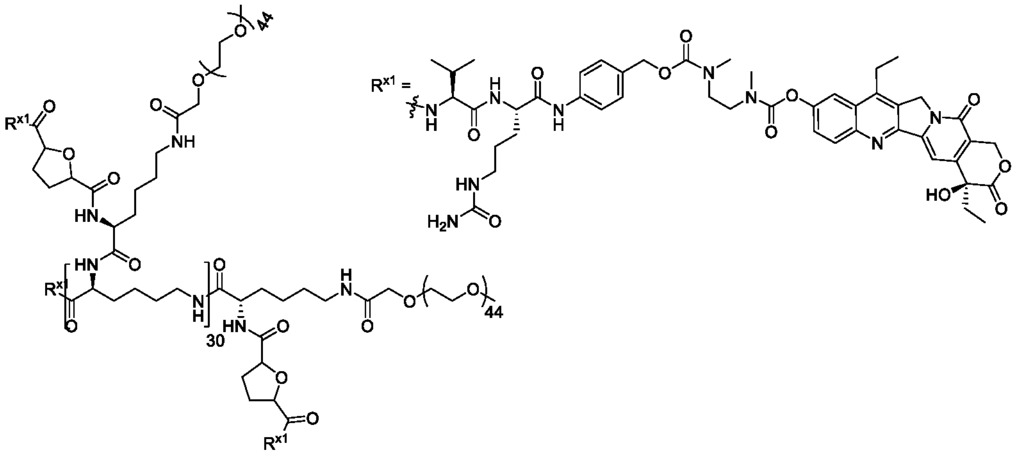

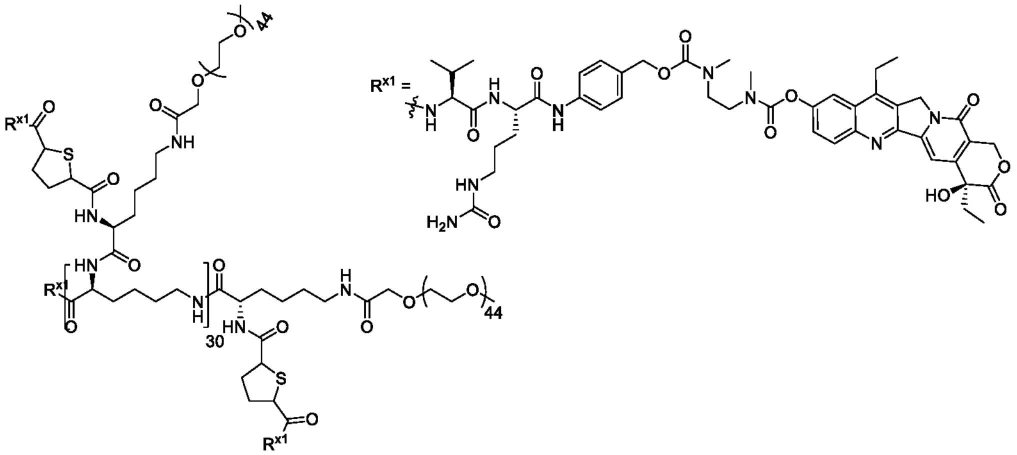

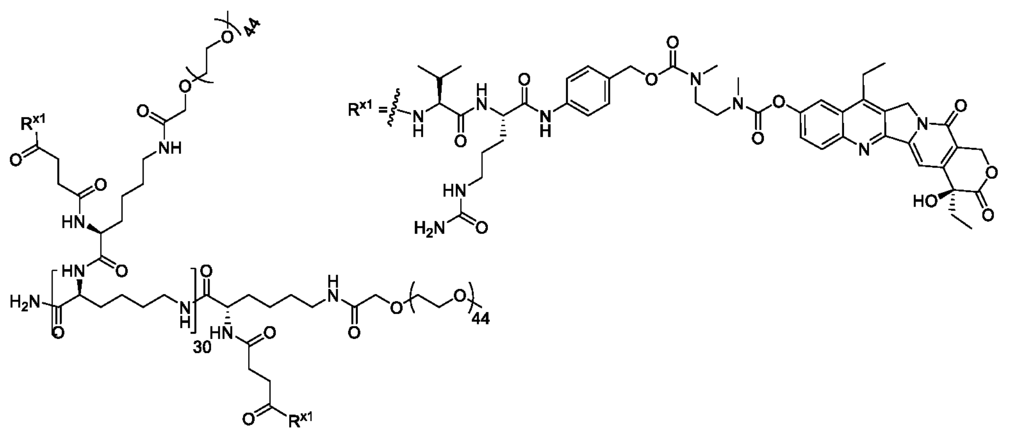

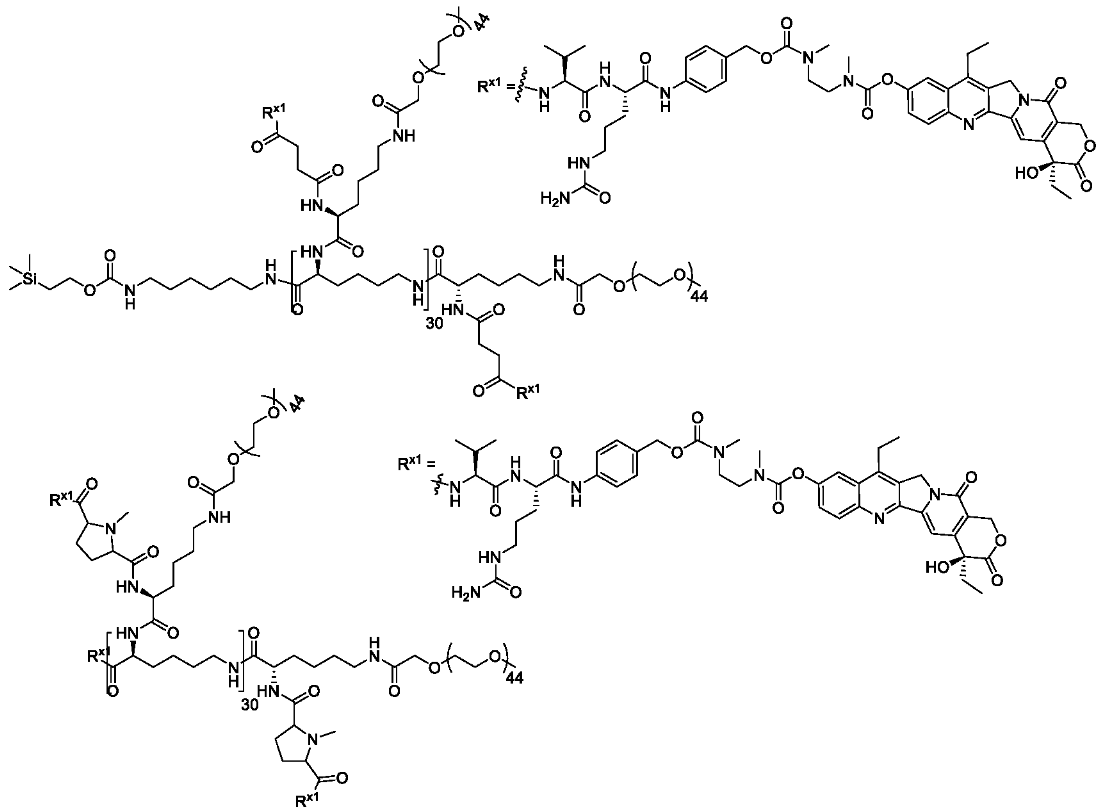

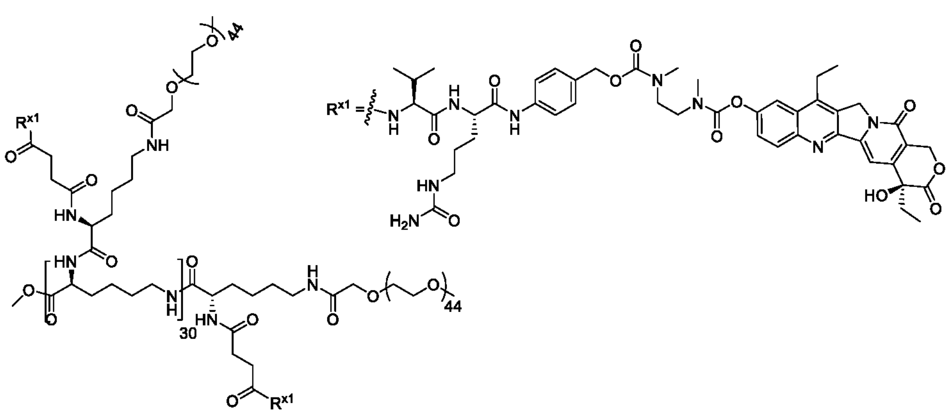

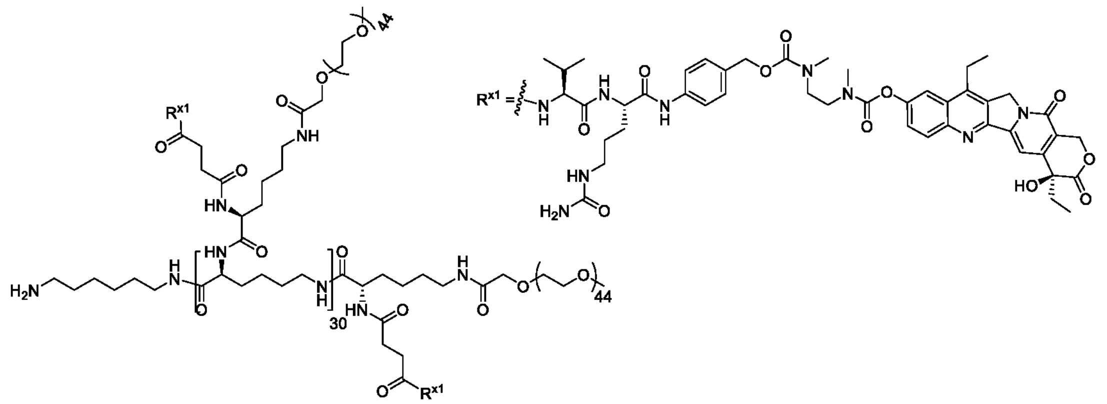

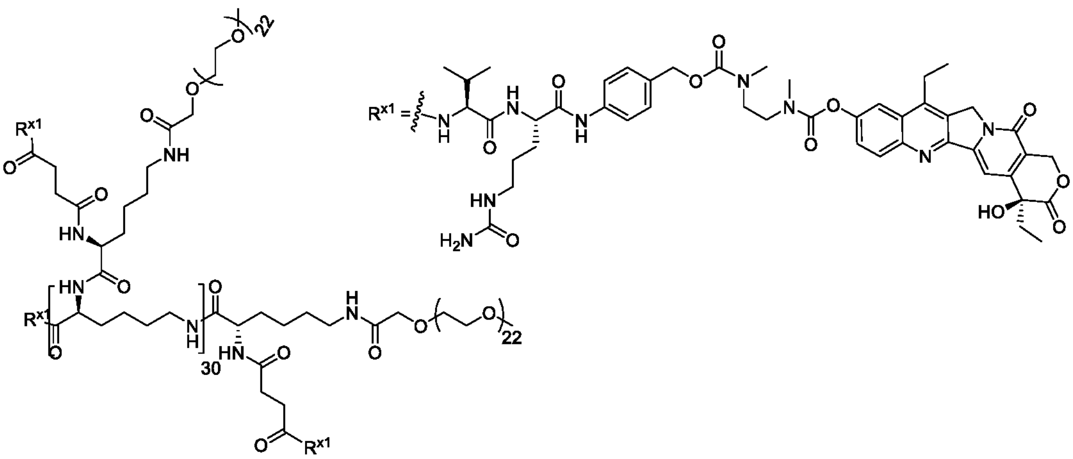

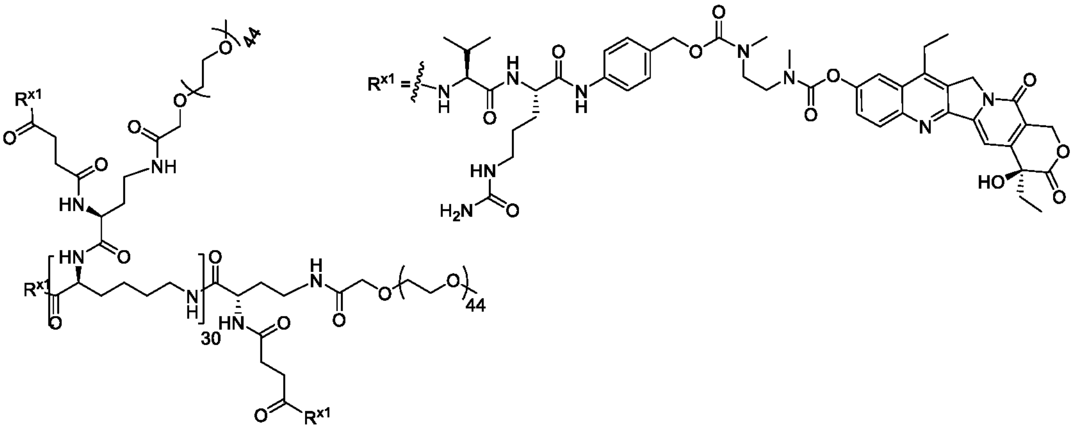

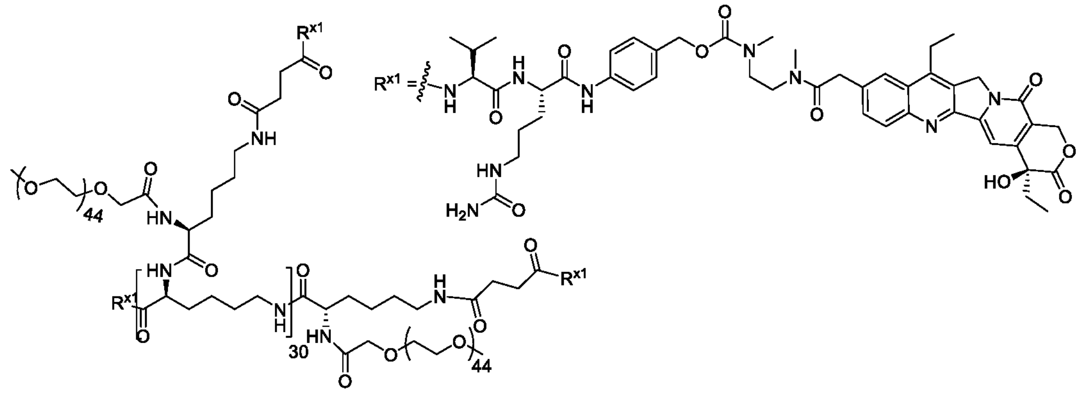

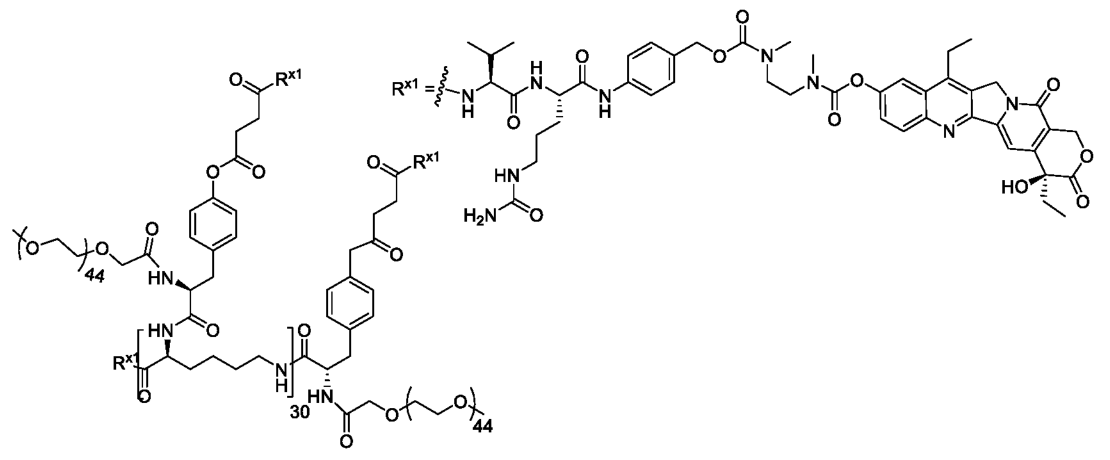

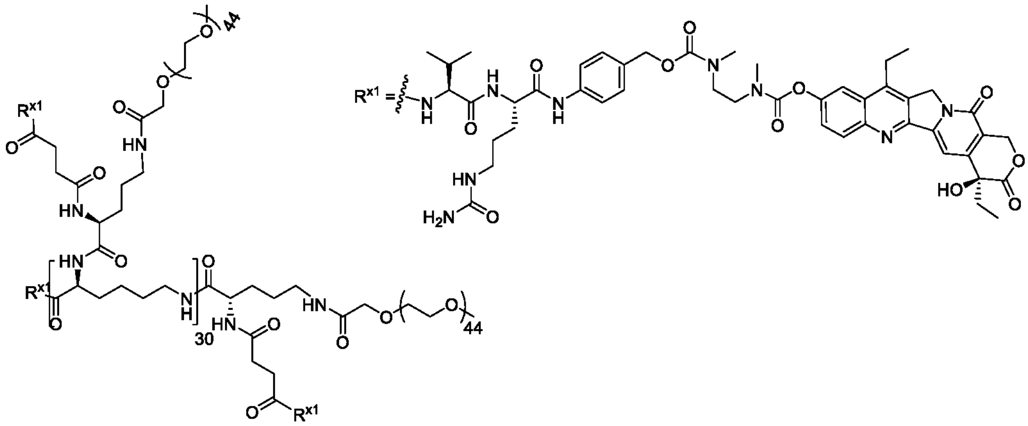

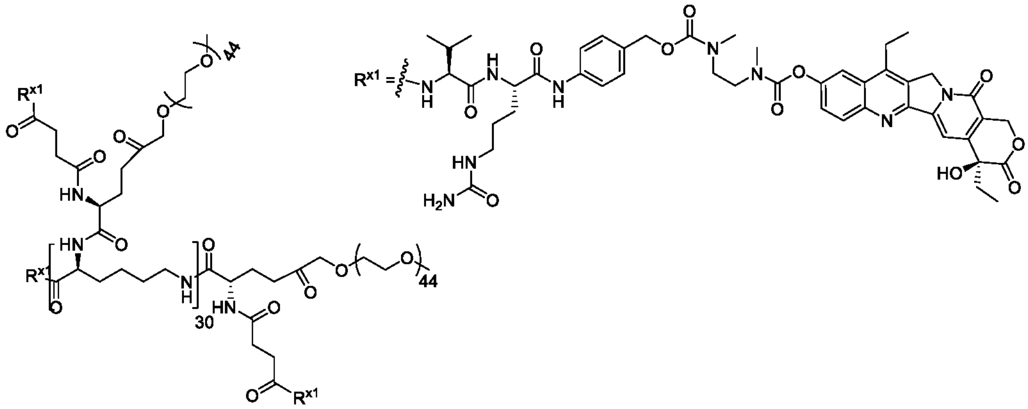

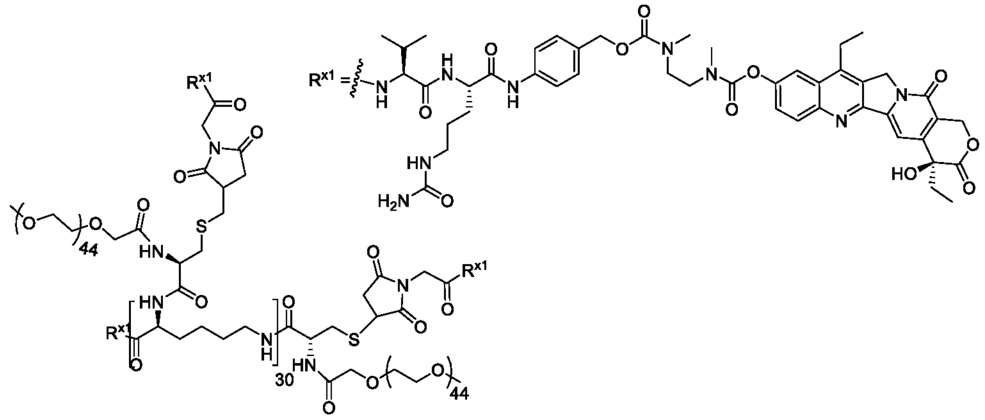

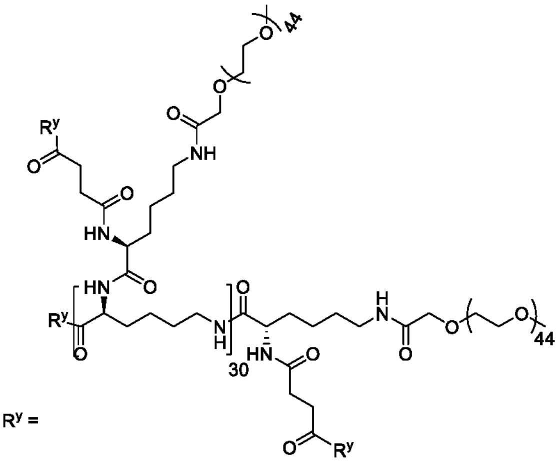

$R^y$ =

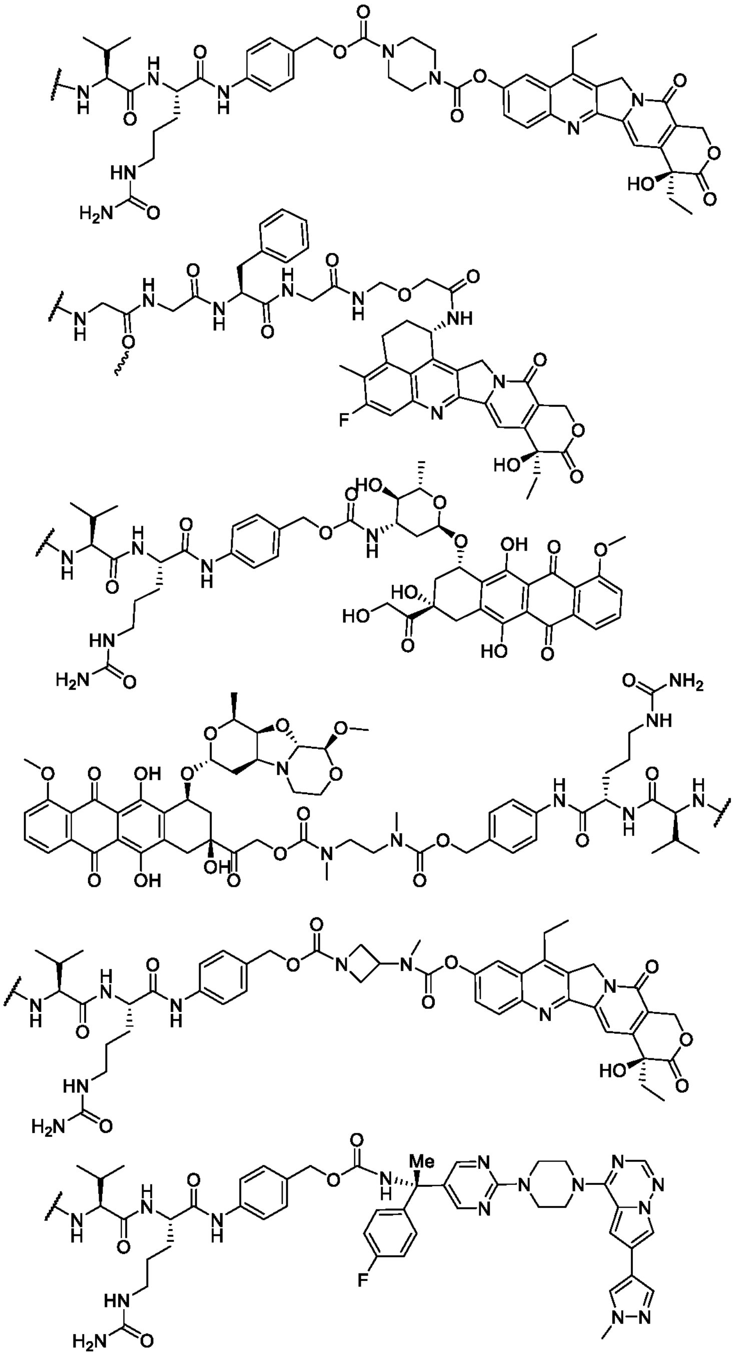

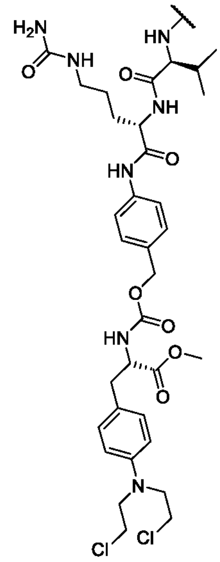

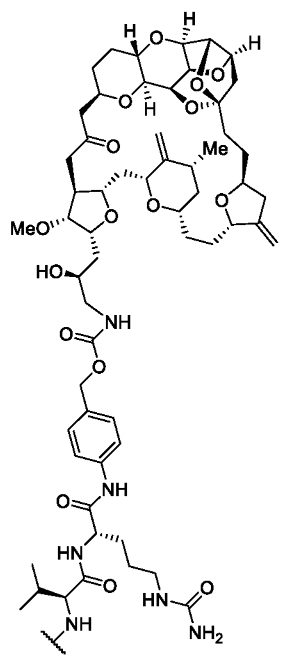

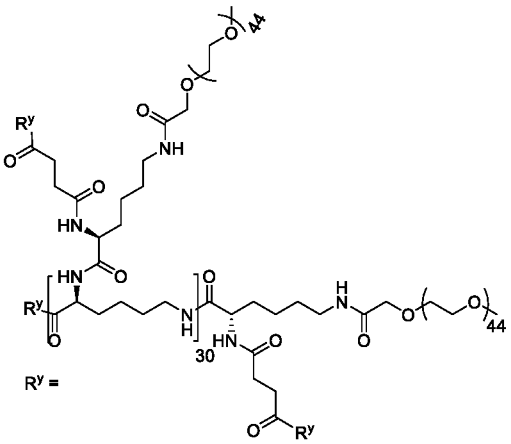

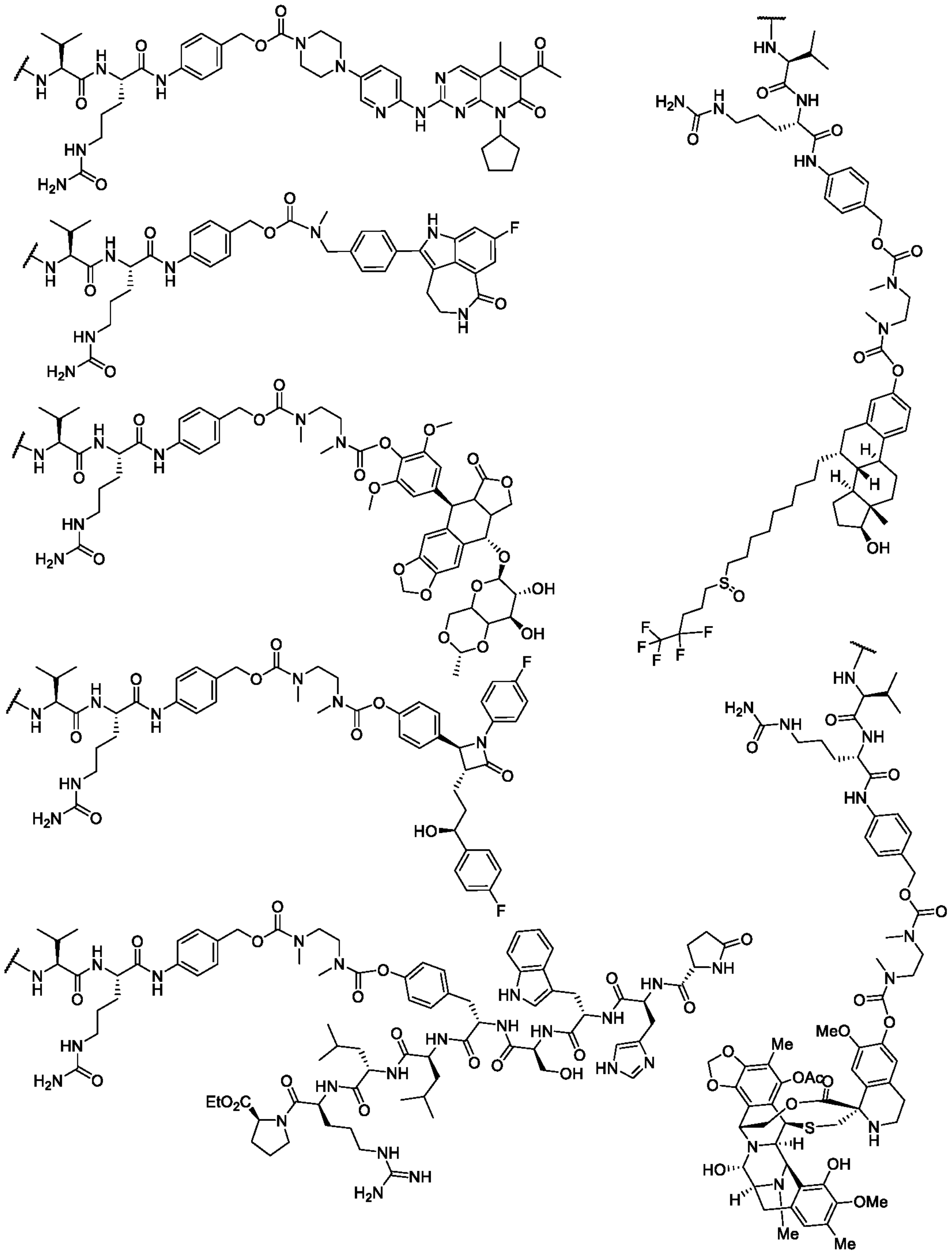

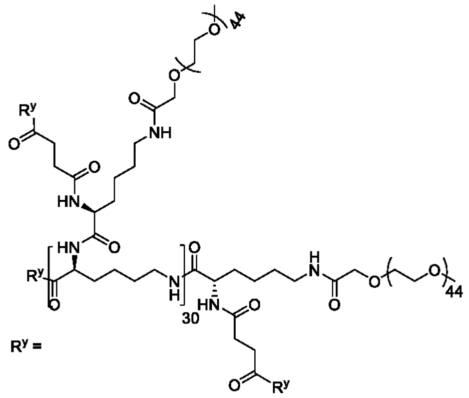

$R^y$ =

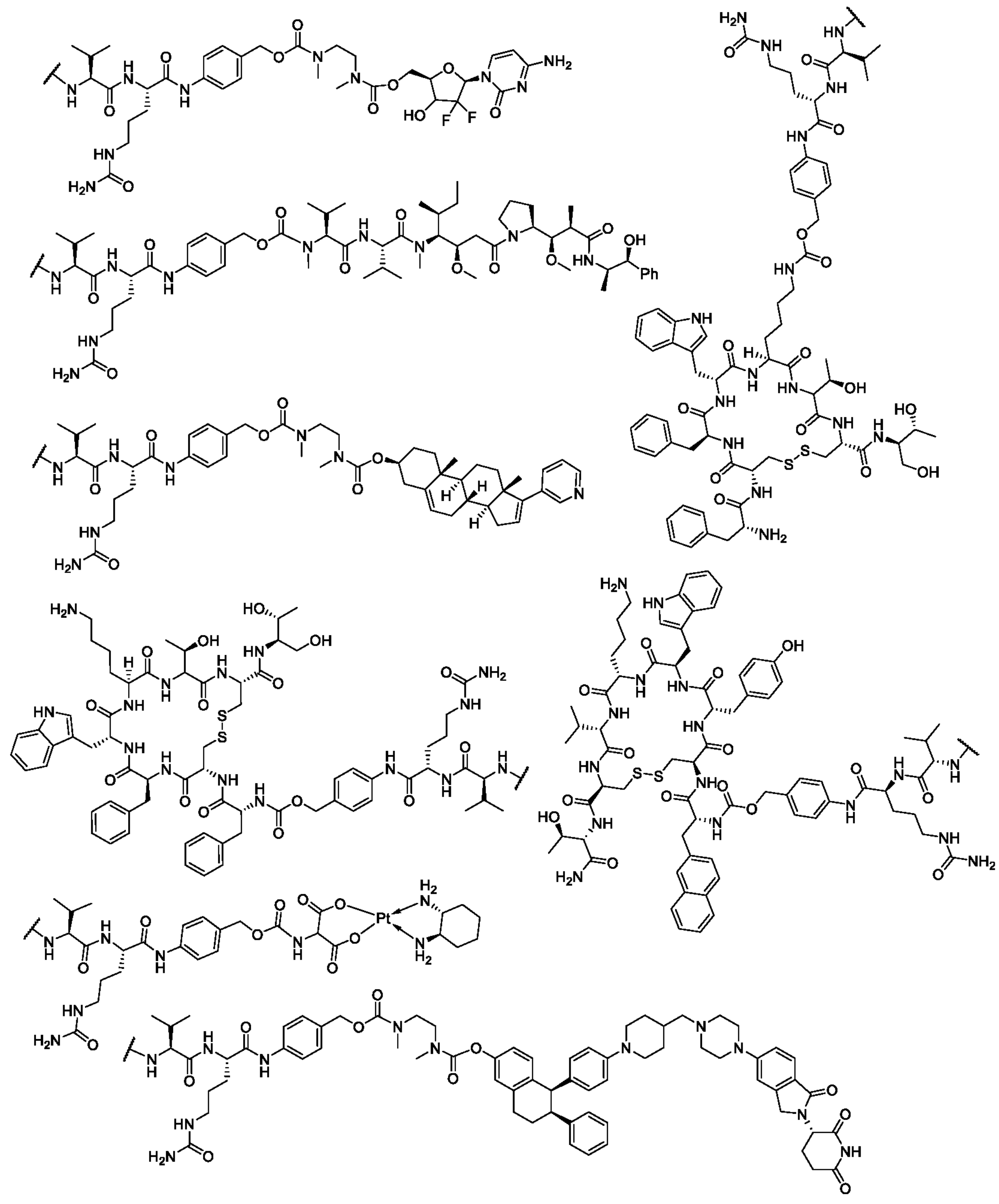

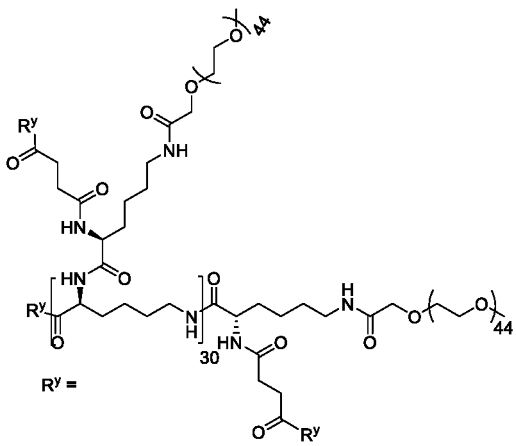

$R^y$ =

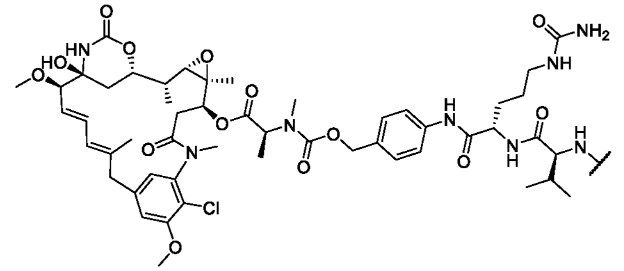

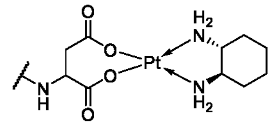

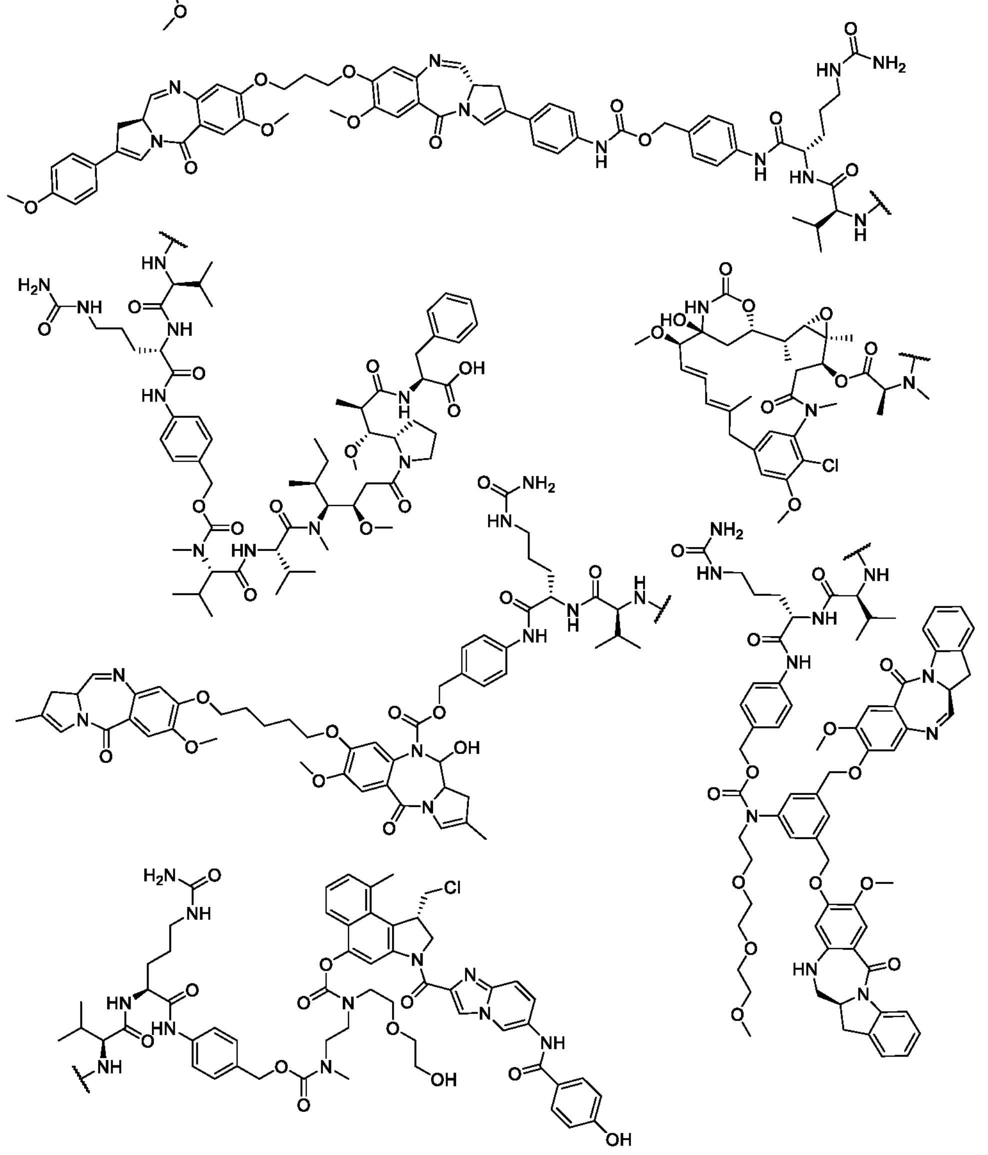

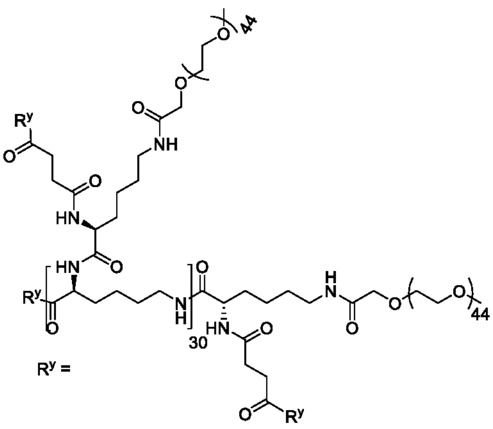

$R^y$ =

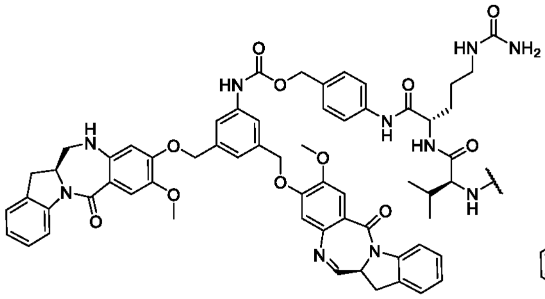

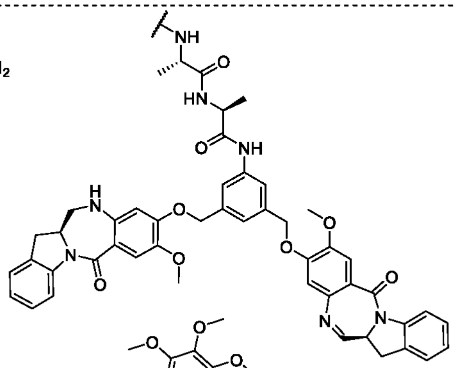

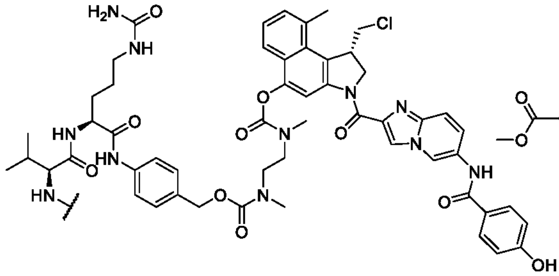

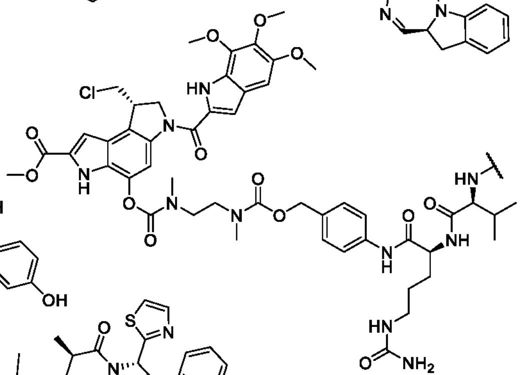

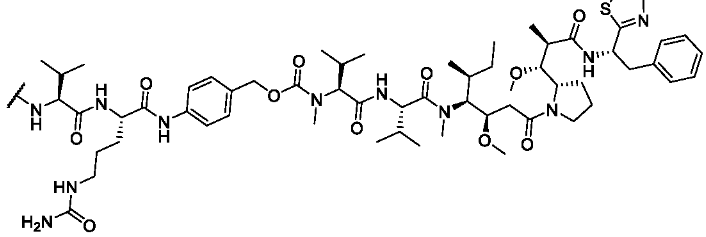

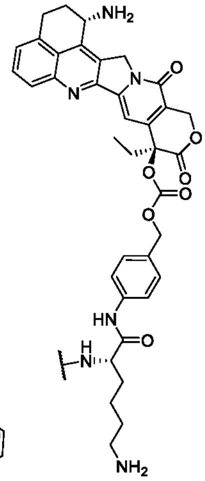

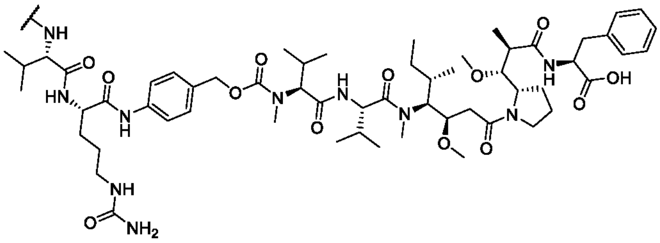

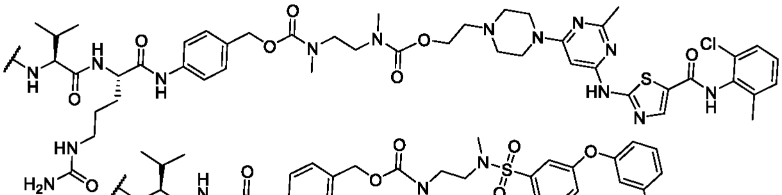

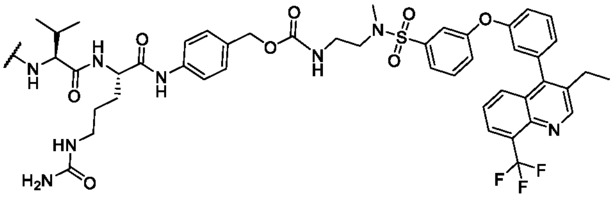

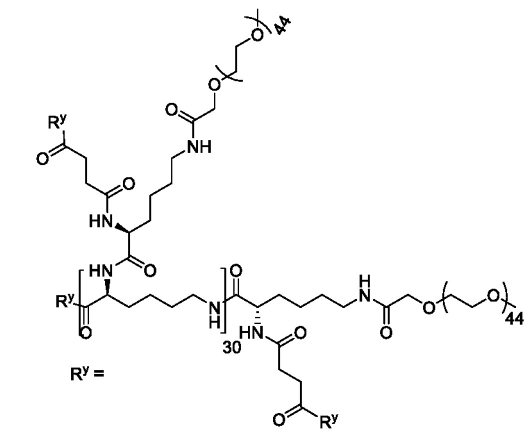

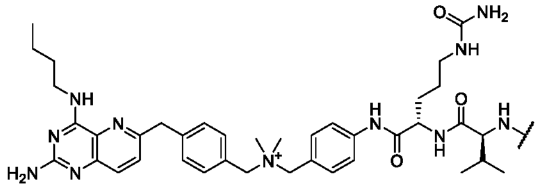

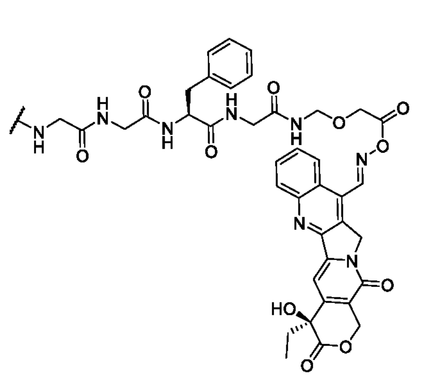

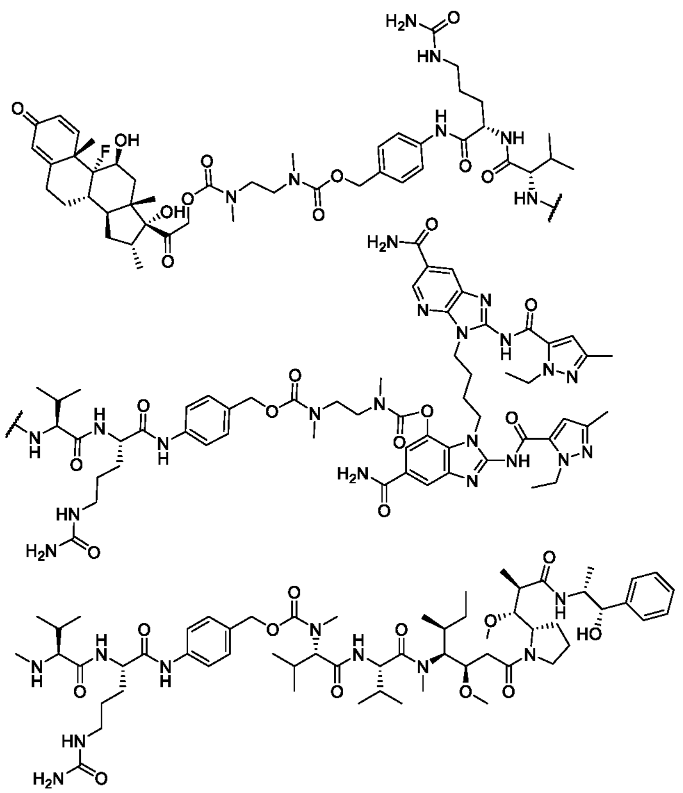

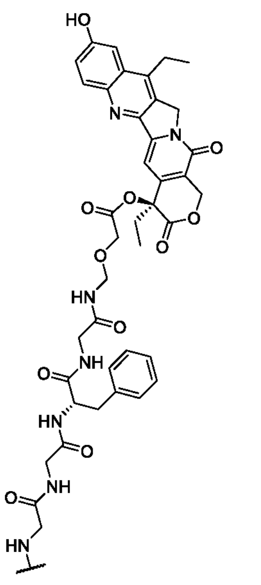

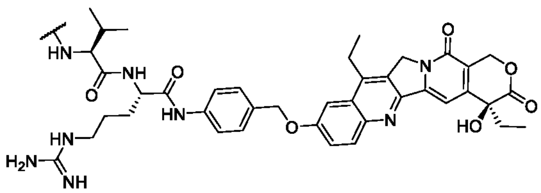

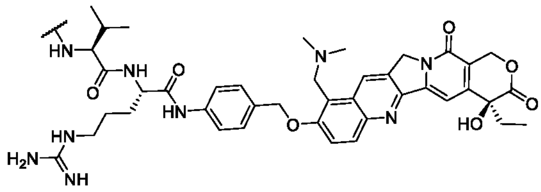

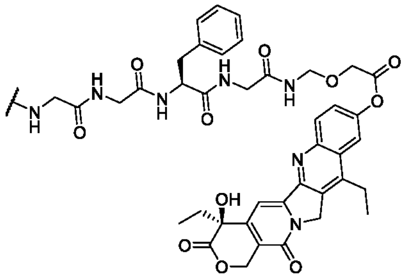

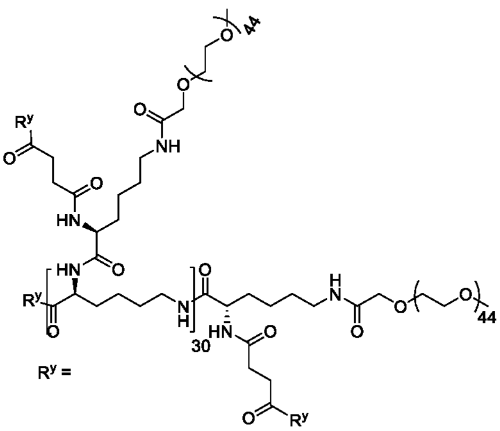

$R^y$ =

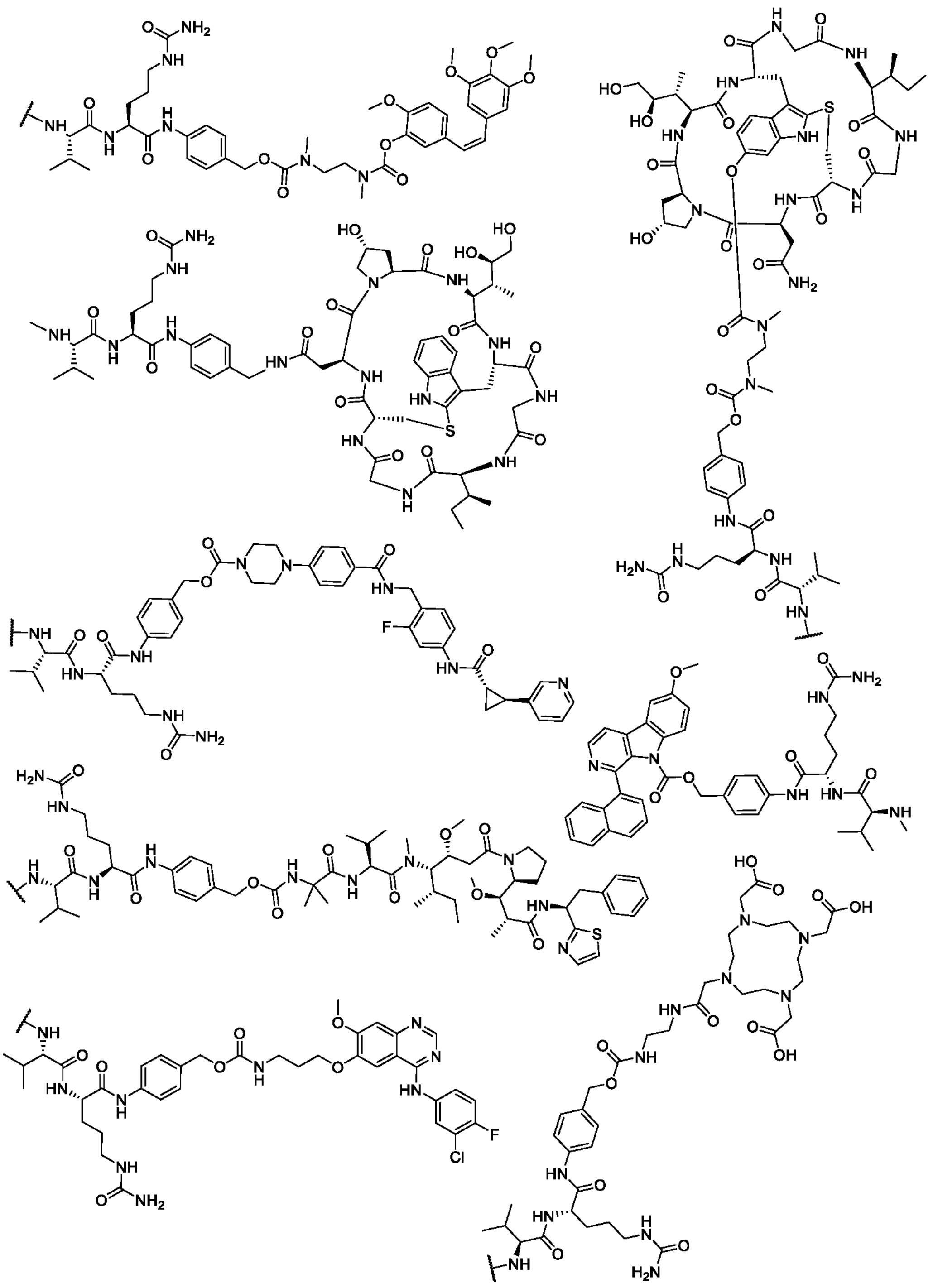

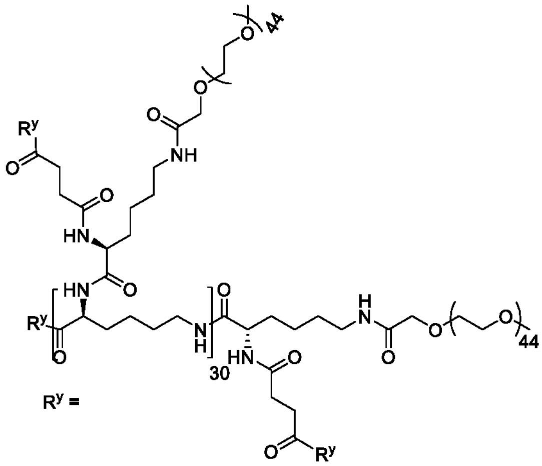

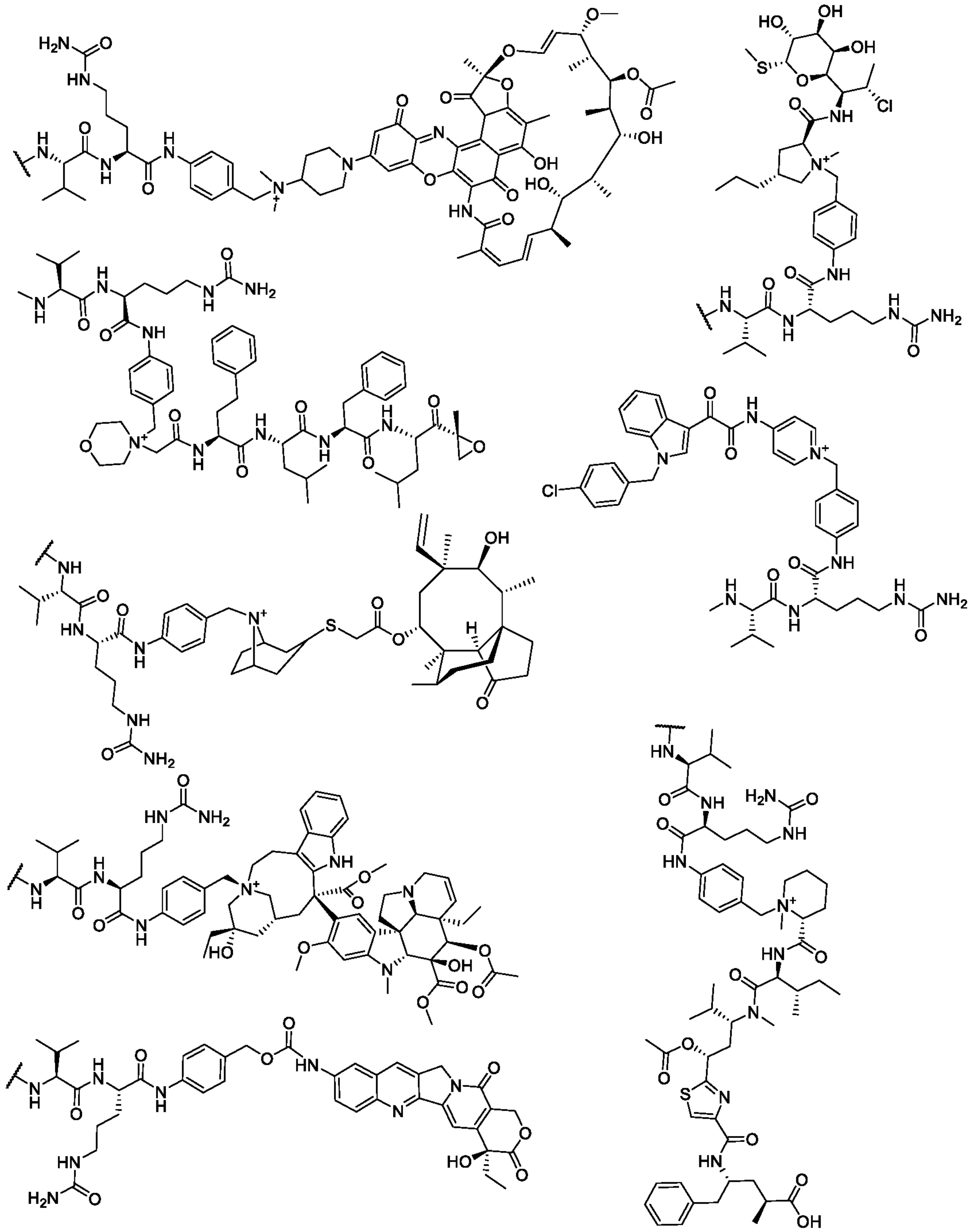

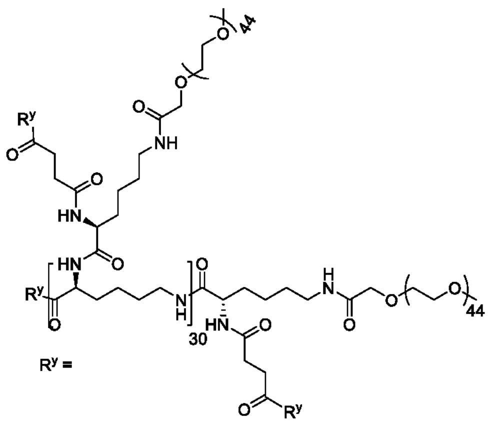

$R^y$ =

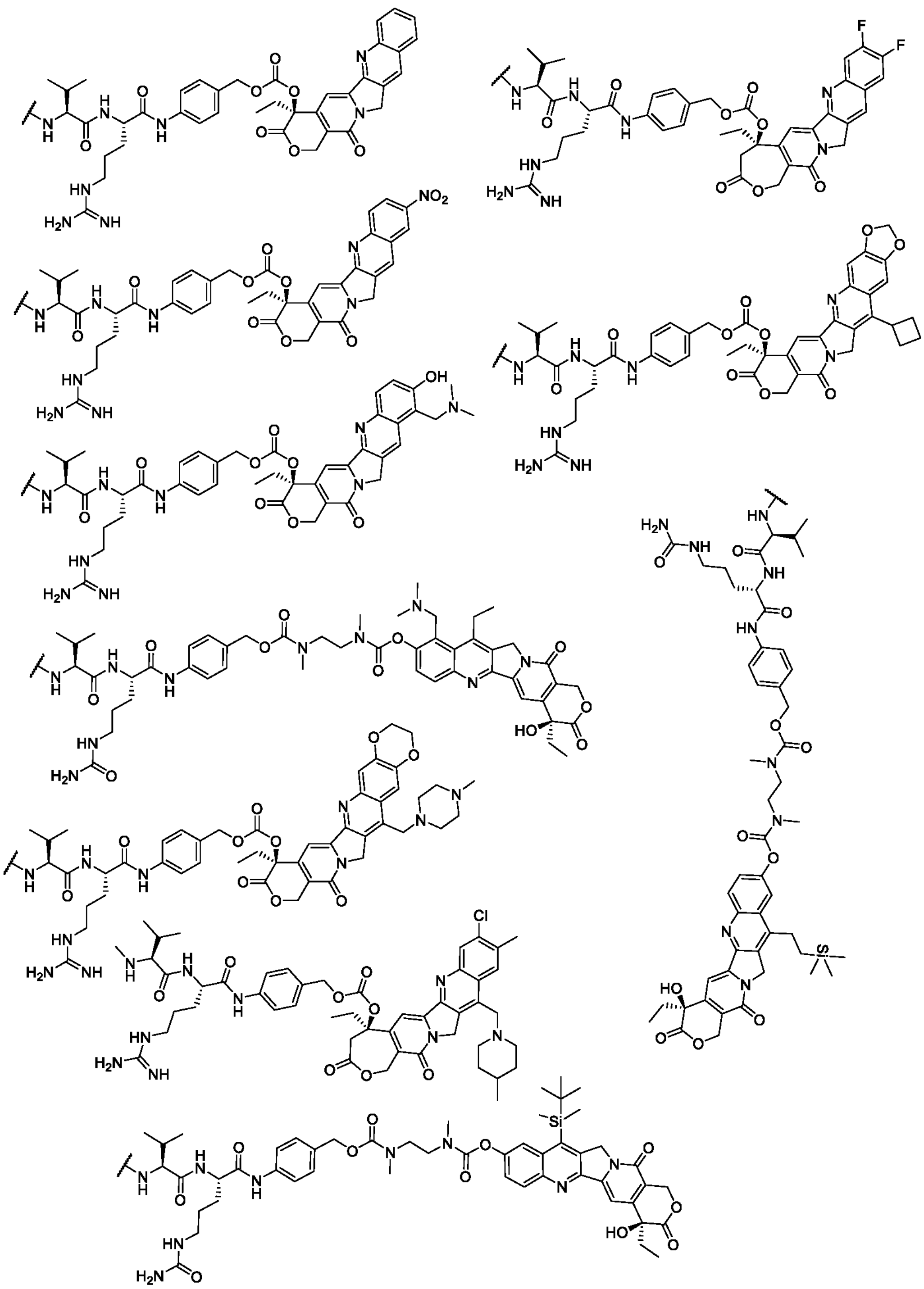

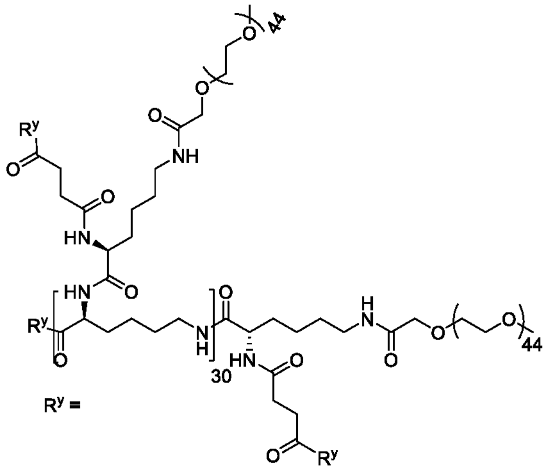

Ry =

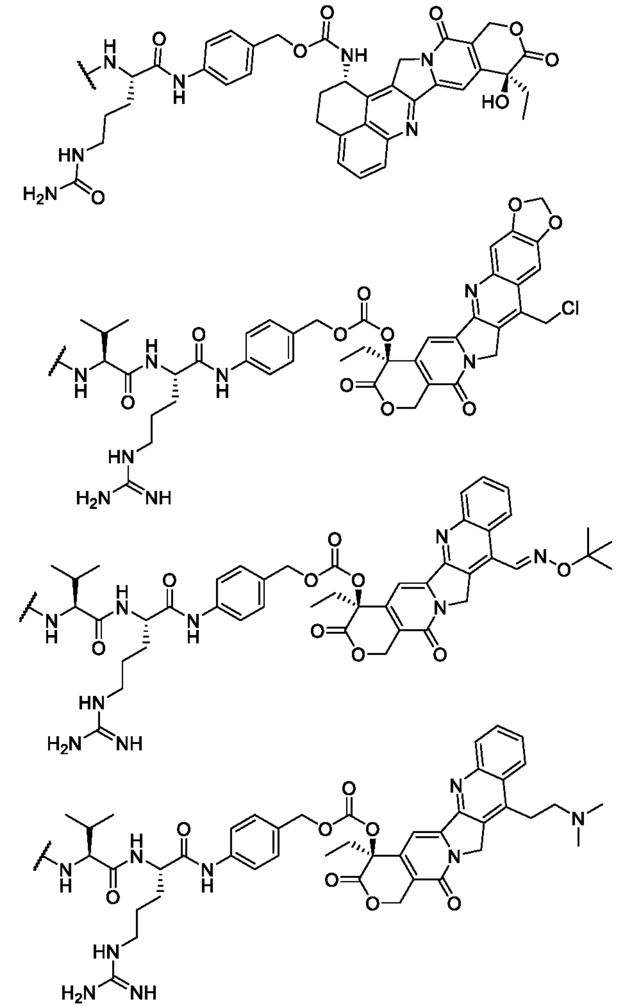

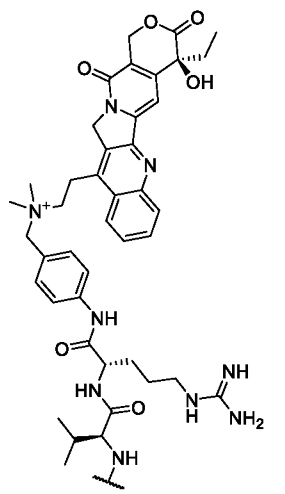

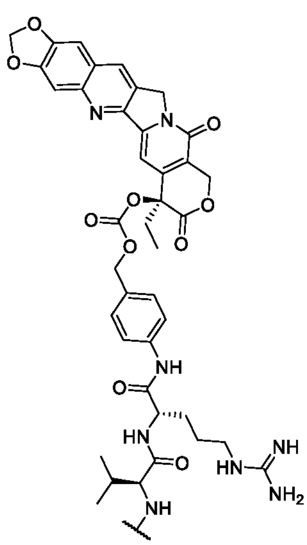

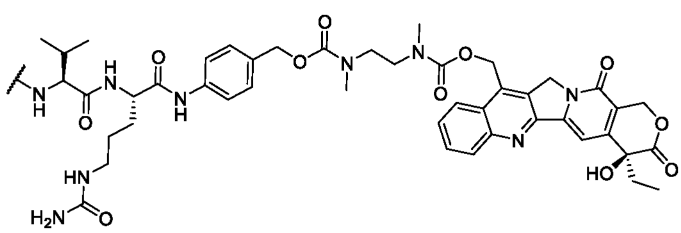

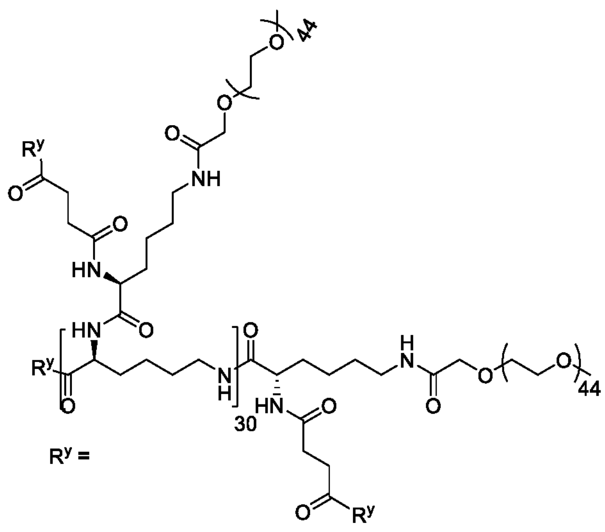

$R^y$ =

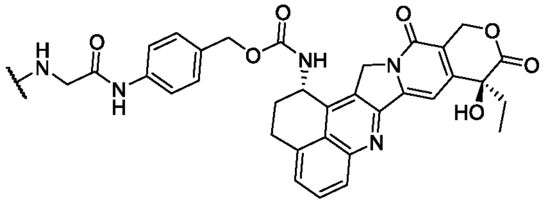

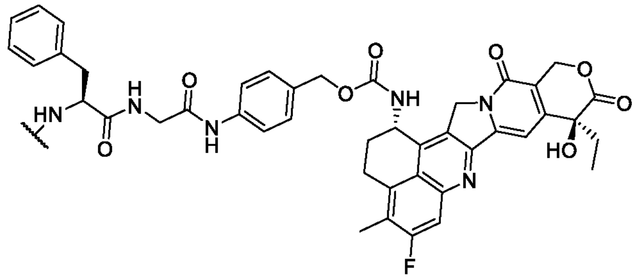

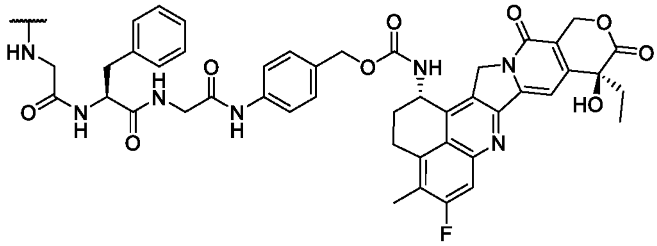

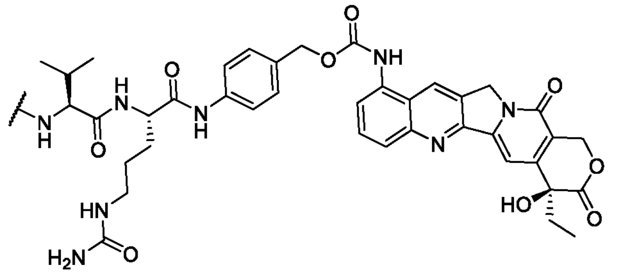

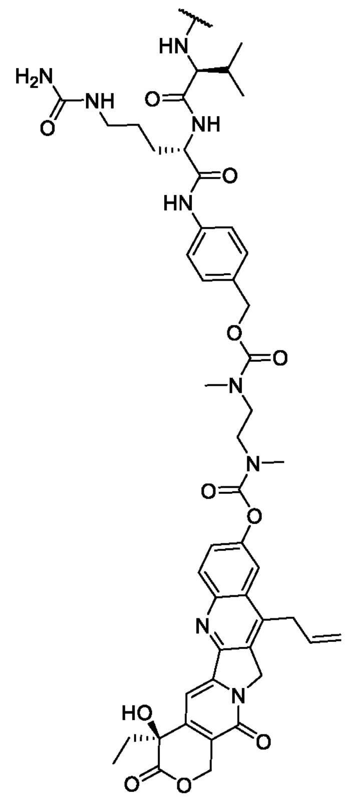

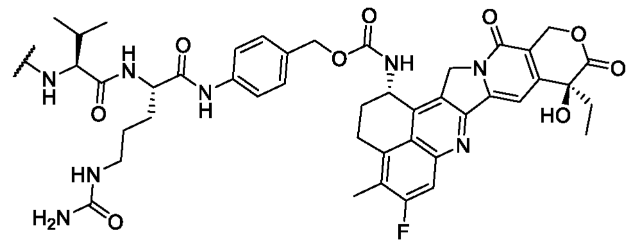

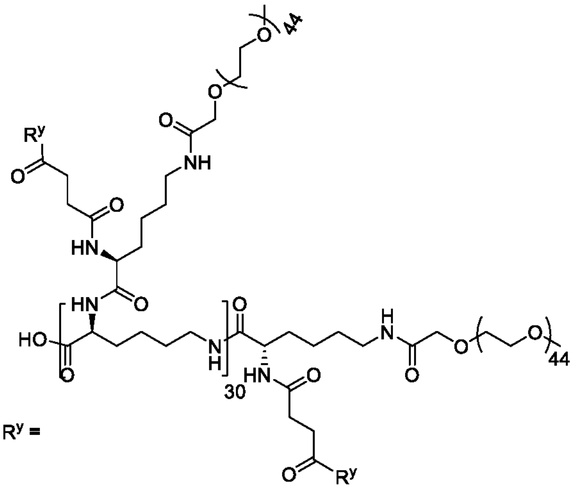

Rʸ =

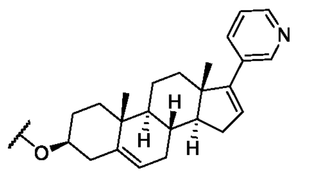

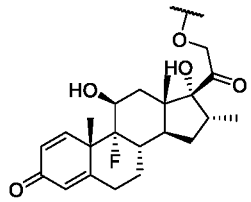

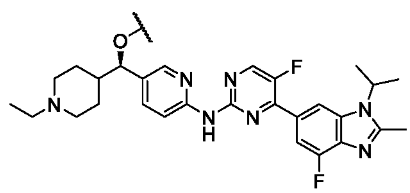

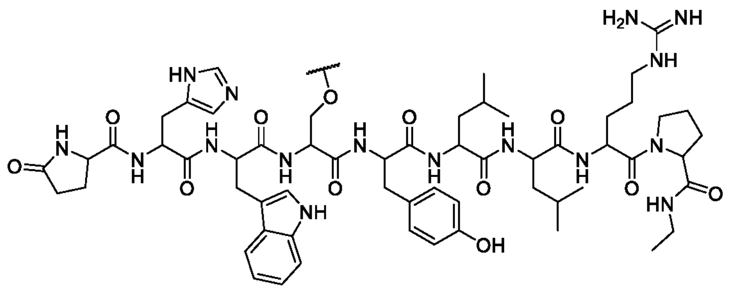

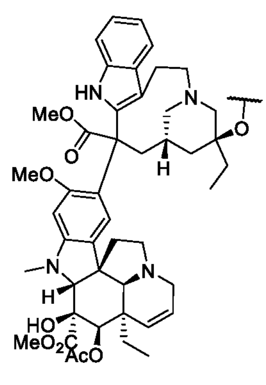

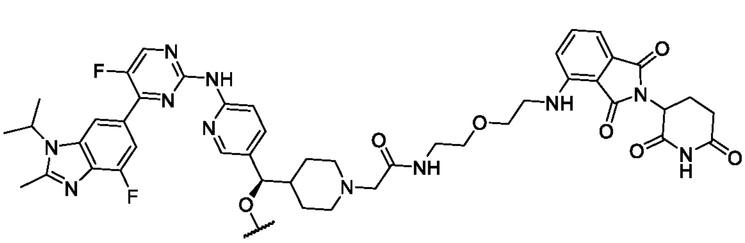

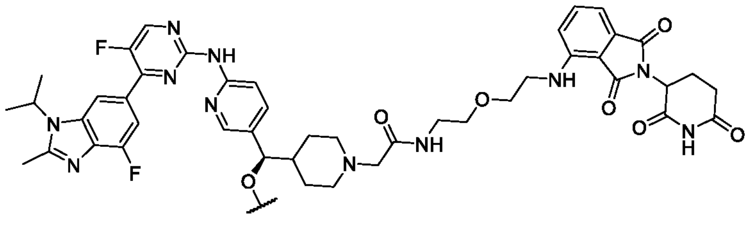

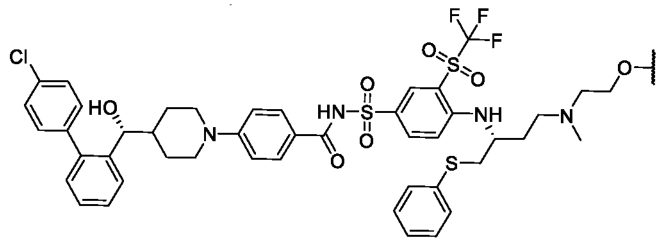

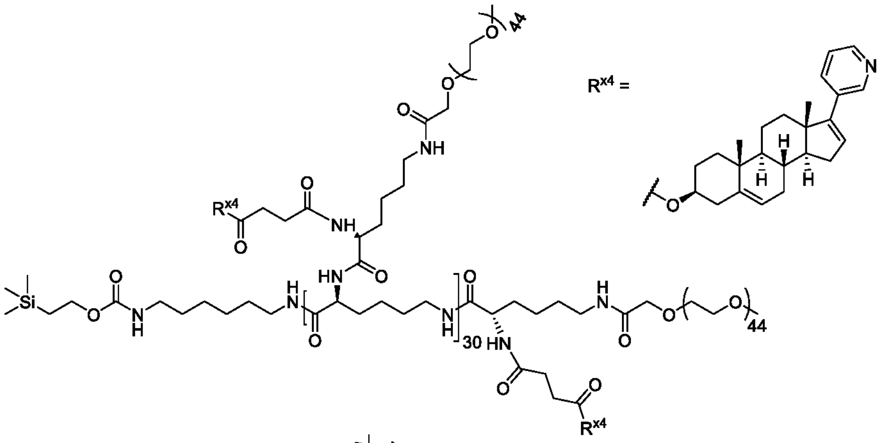

$R^{x4}$ =

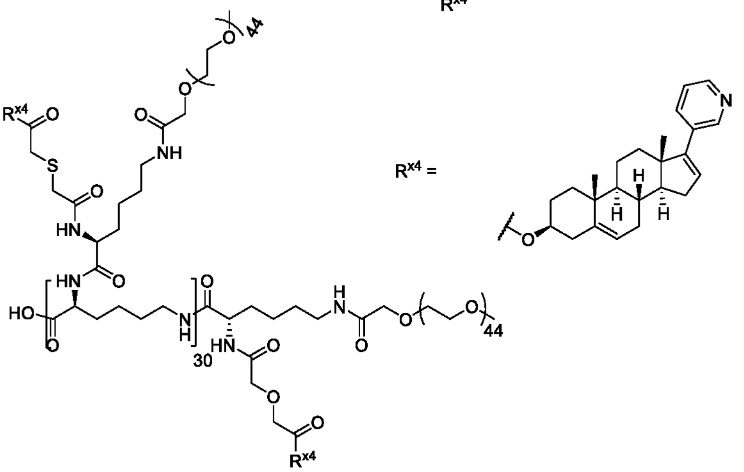

$R^{x4}$ =

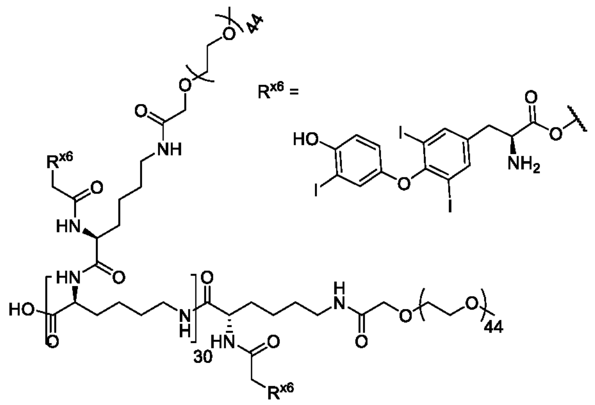

$R^{x6}$ =

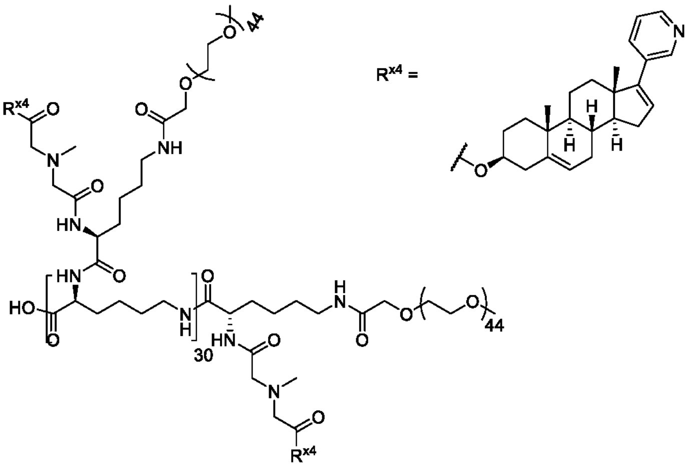

$R^{x4}$ =

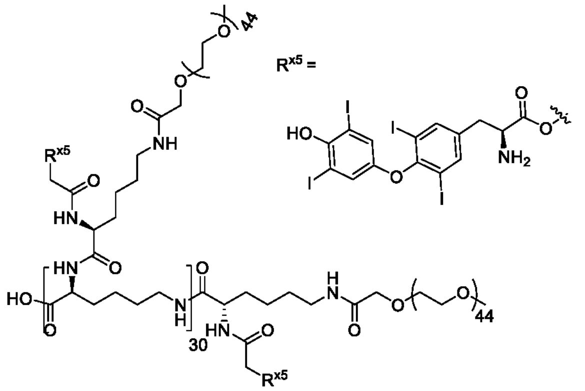

$R^{x5}$ =

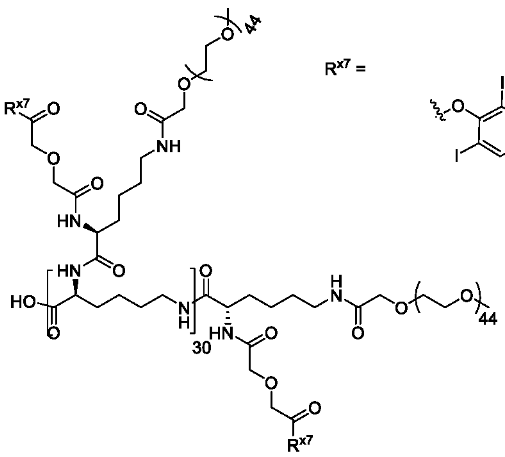

R$^{x7}$ =

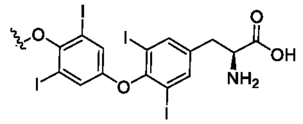

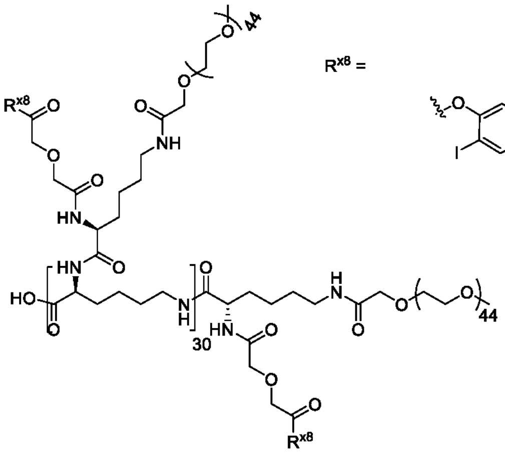

R$^{x8}$ =

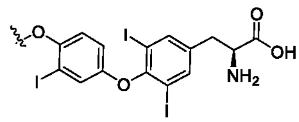

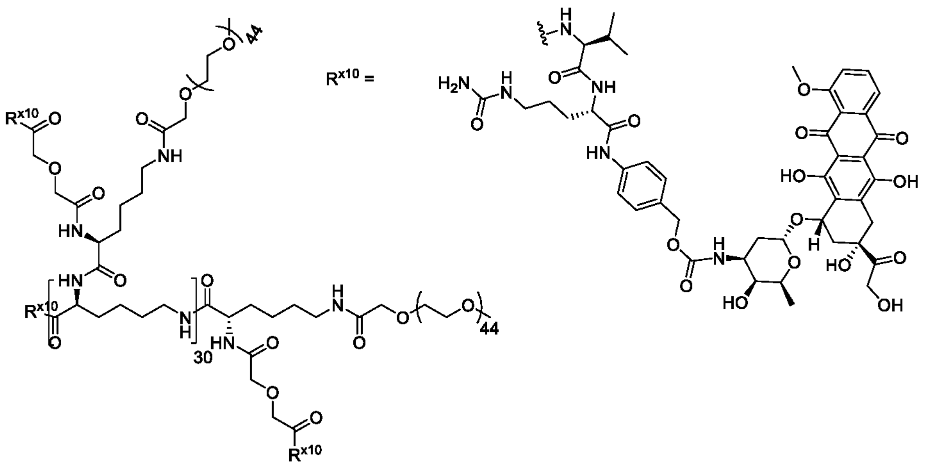

R$^{x10}$ =

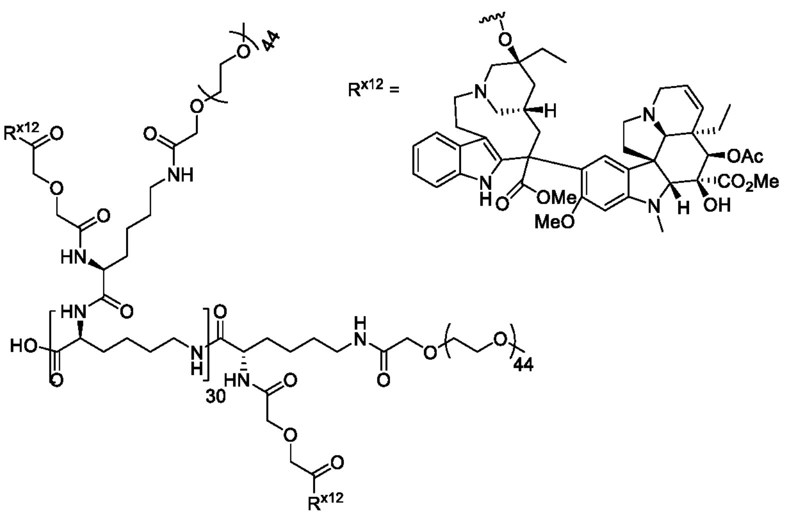

$R^{x12}$ =

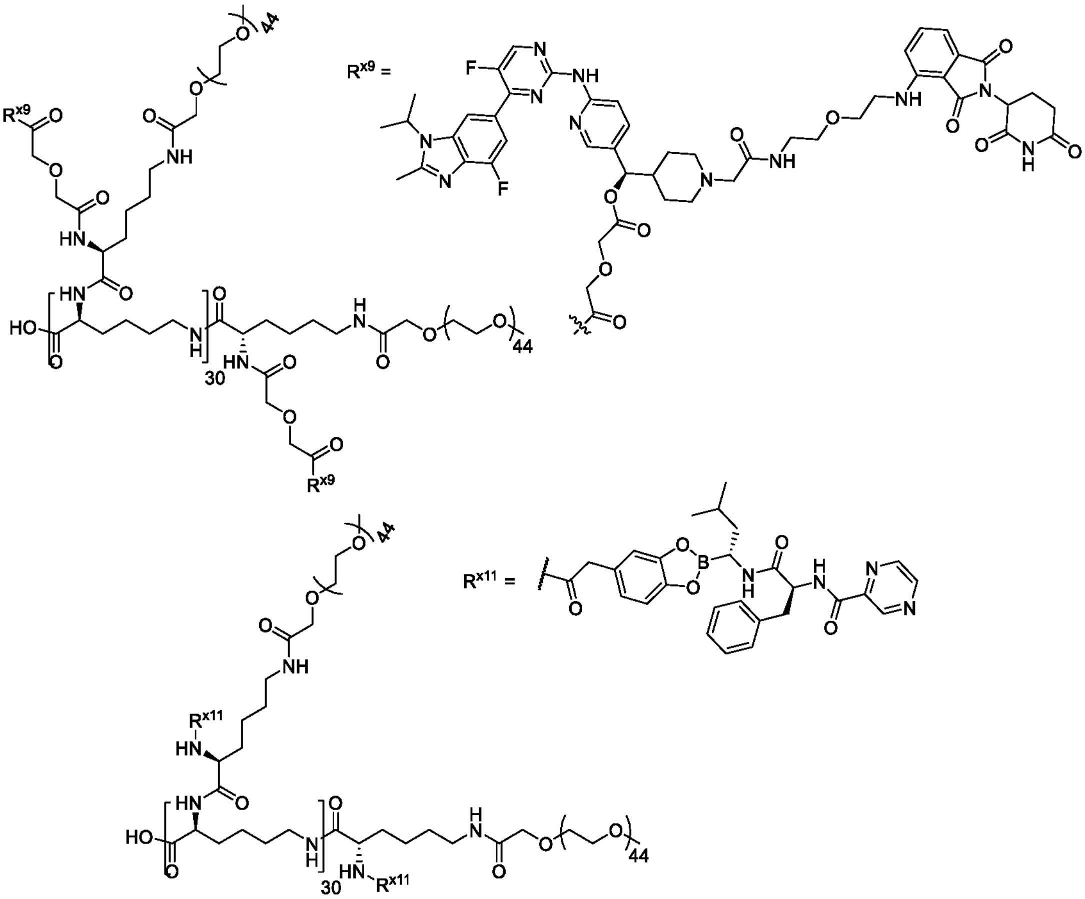

$R^{x9}$ =

$R^{x11}$ =

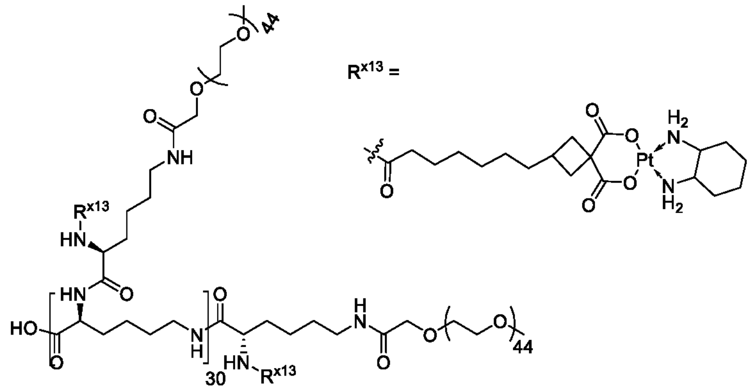

$R^{x13}$ =

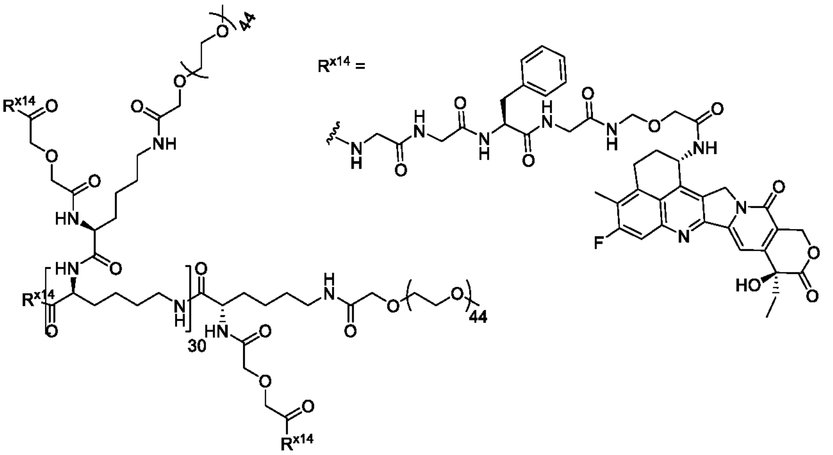

$R^{x14}$ =

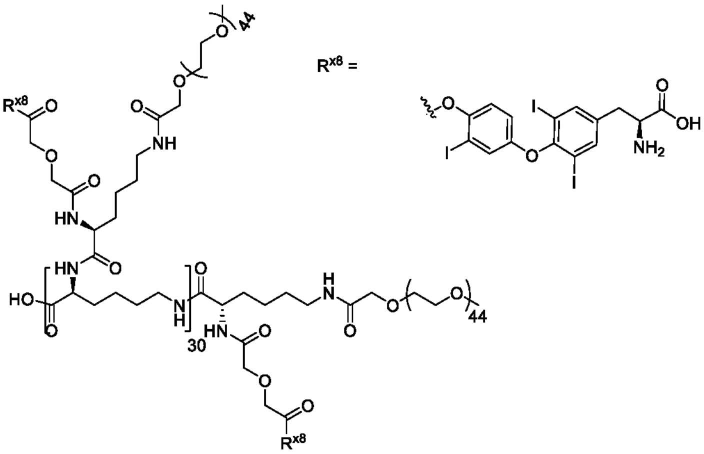

$R^{x8}$ =

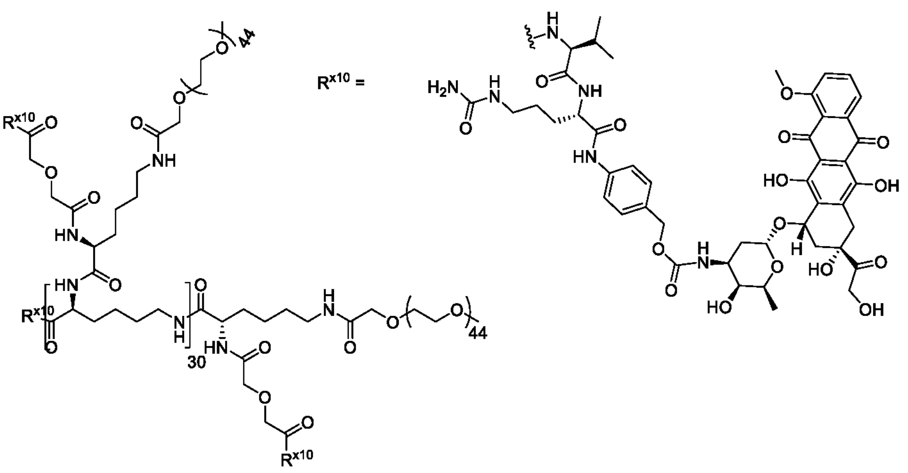
R^x10 =

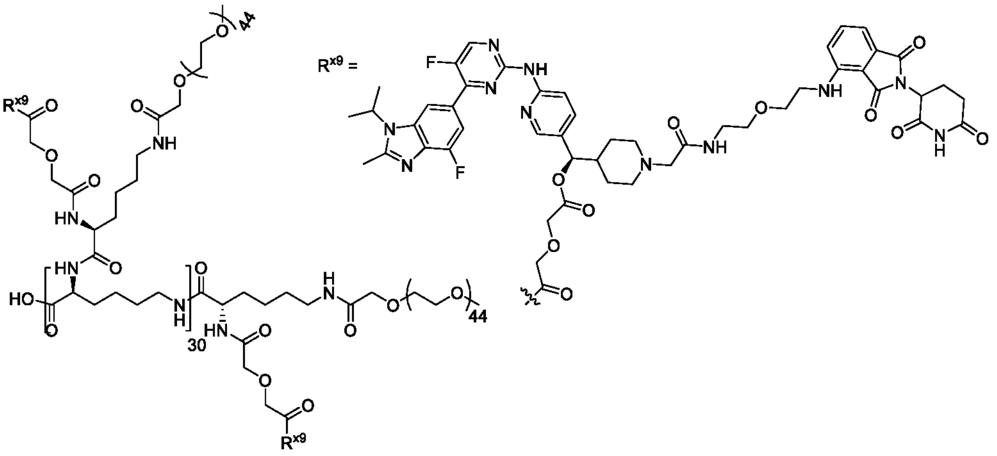
R^x9 =

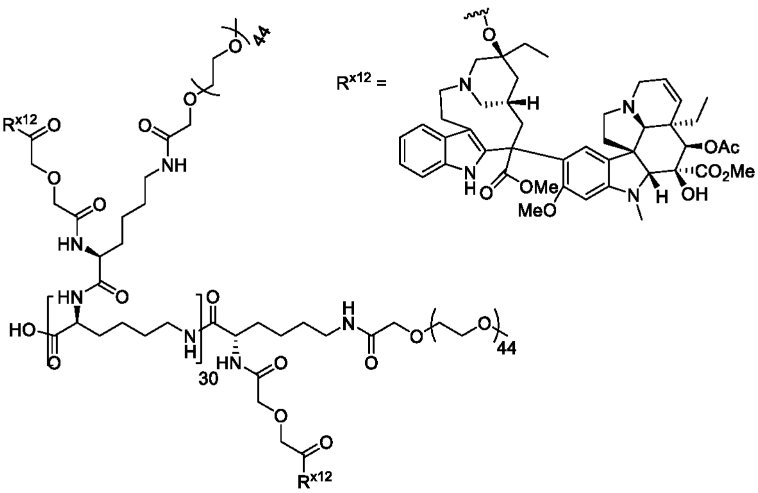
R^x12 =

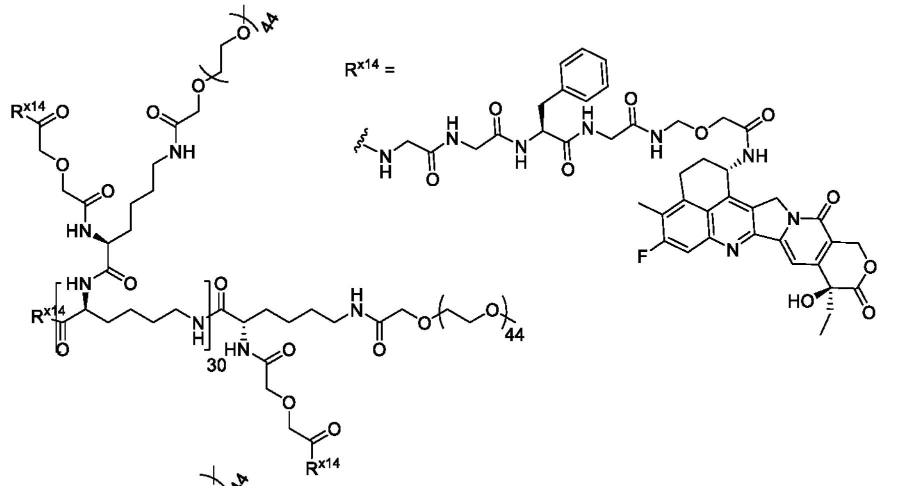

$R^{x14}$ =

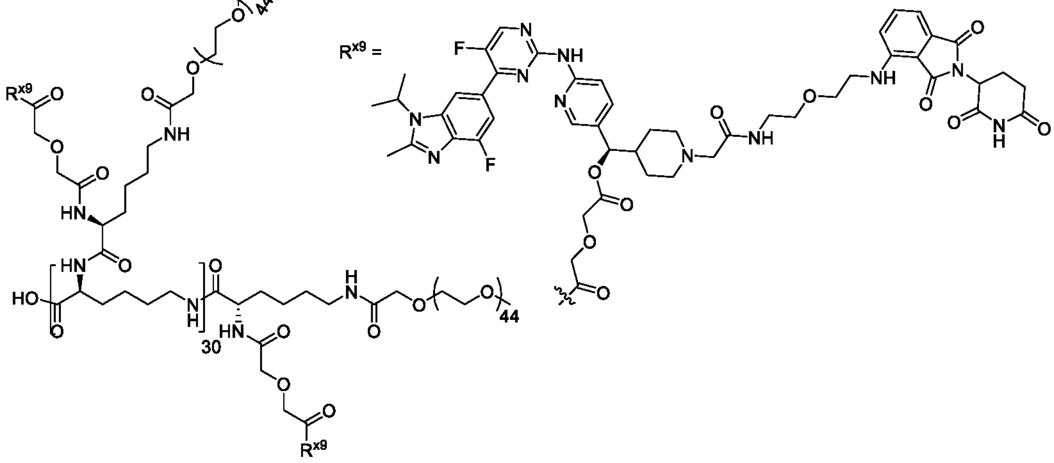

$R^{x9}$ =

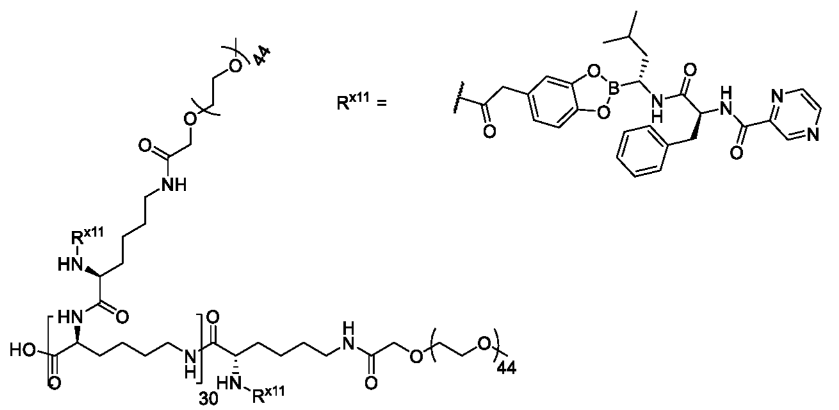

$R^{x11}$ =

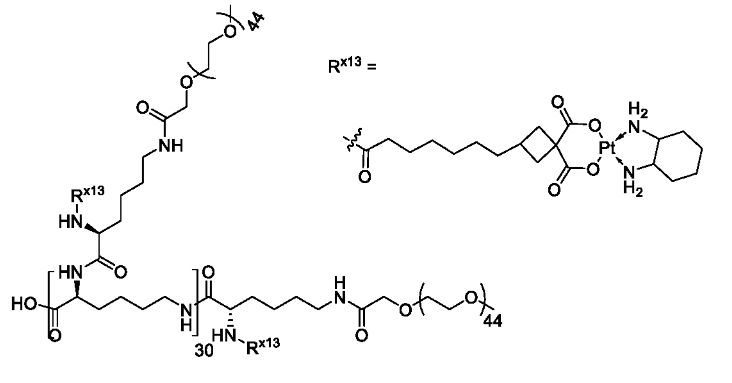

$R^{x13}$ =

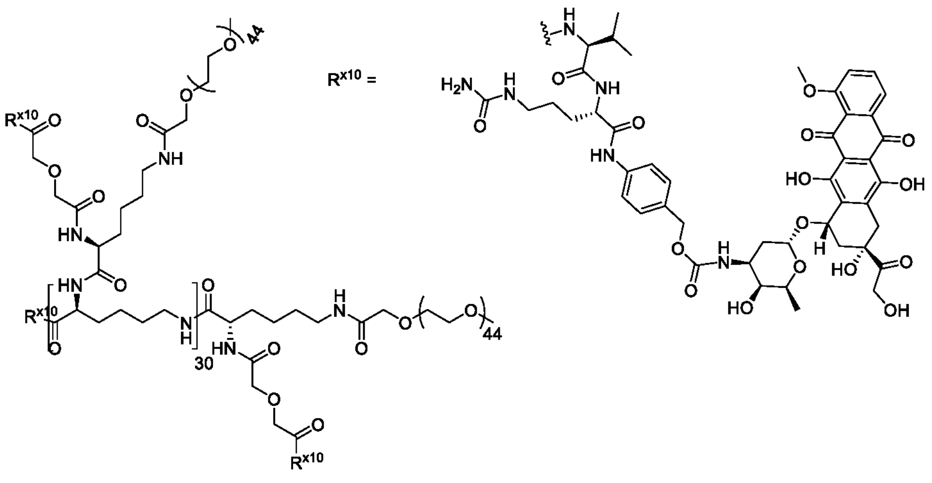

$R^{x10}$ =

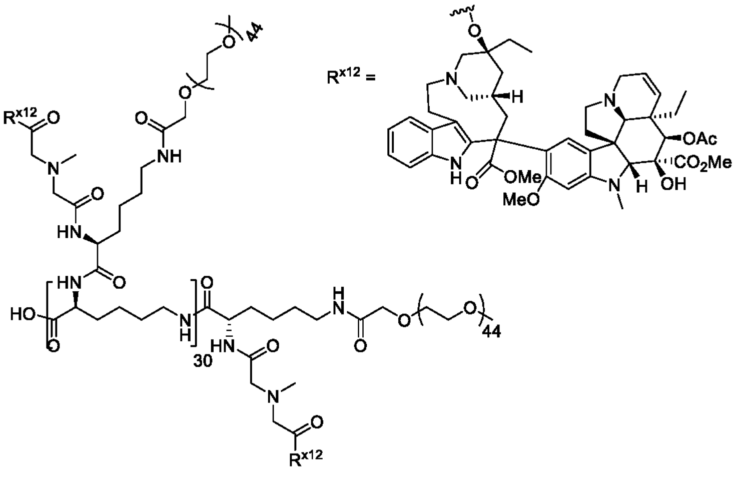

$R^{x12}$ =

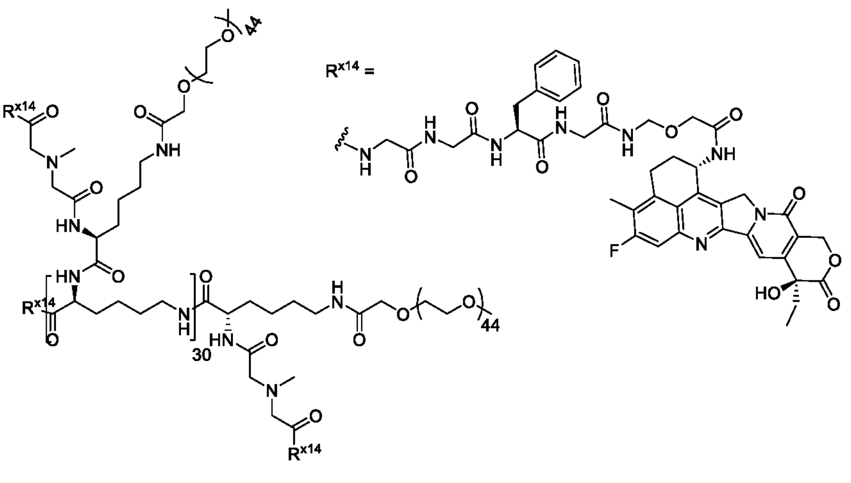

$R^{x14}$ =

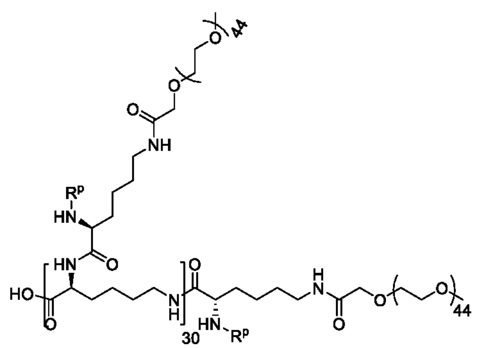

$R^p$ =

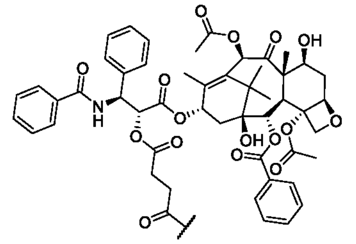
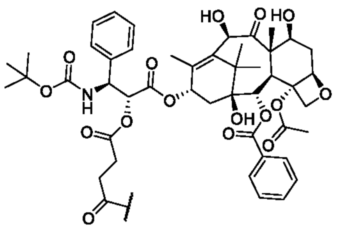
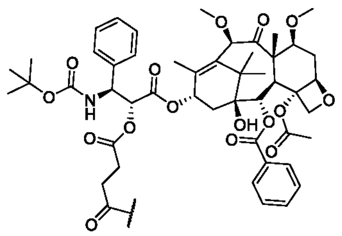
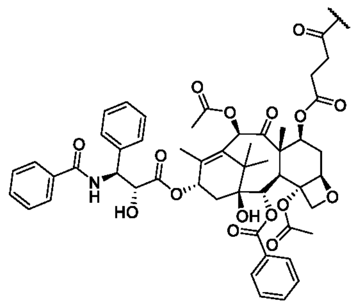
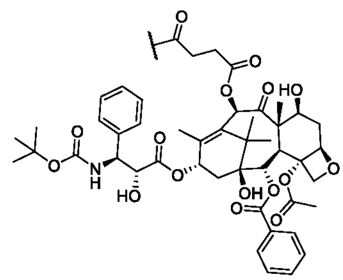
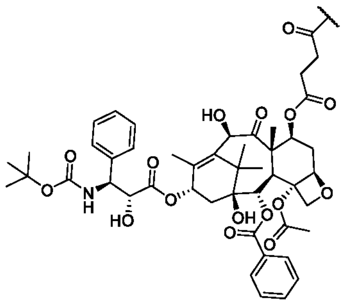
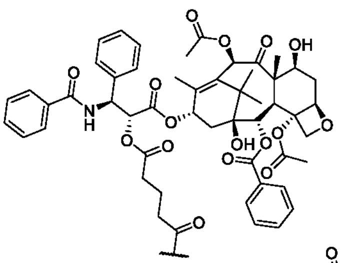
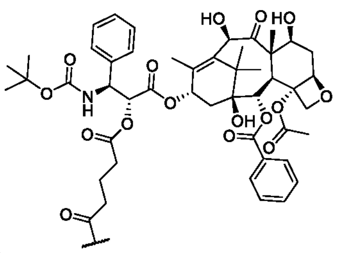
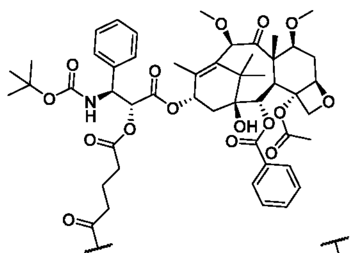
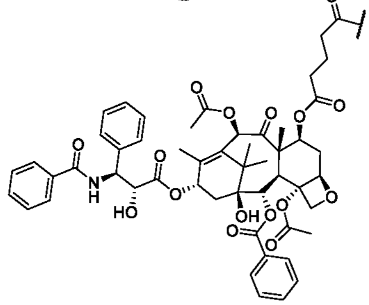
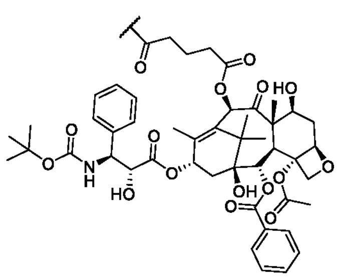
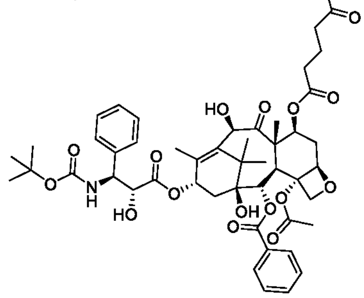

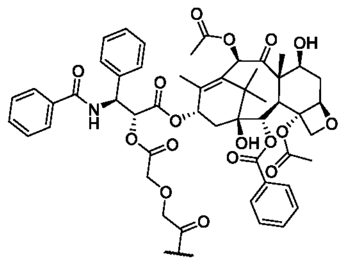
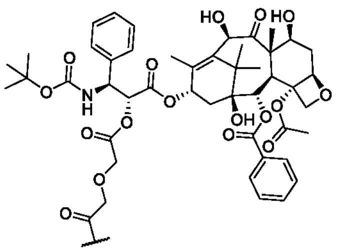
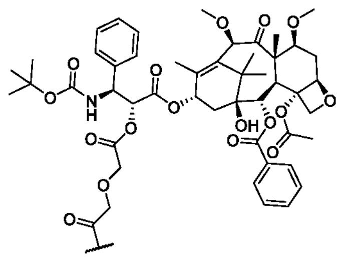

$R^p$ =

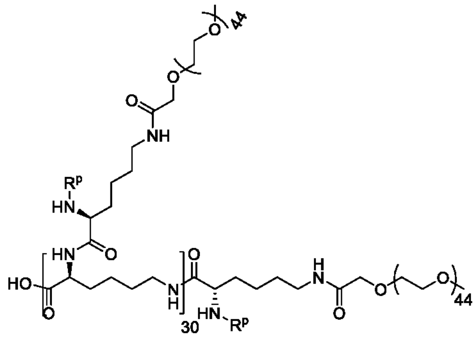

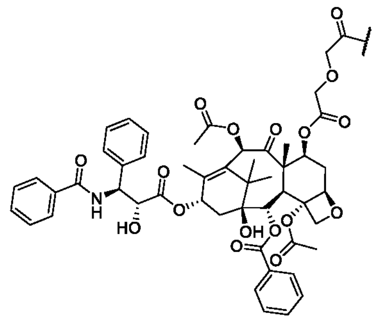
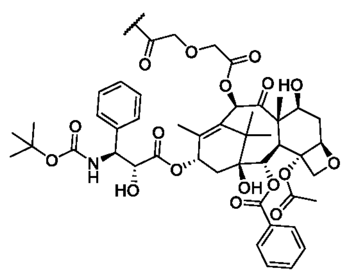
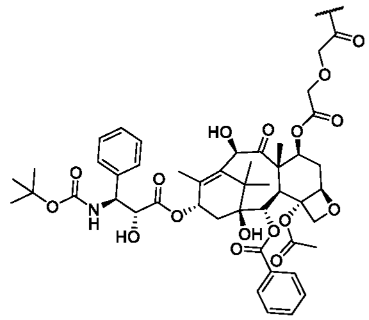

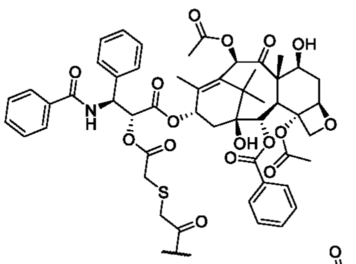
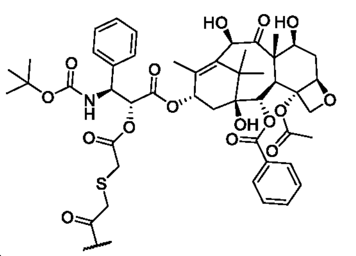
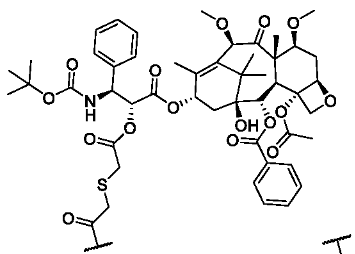

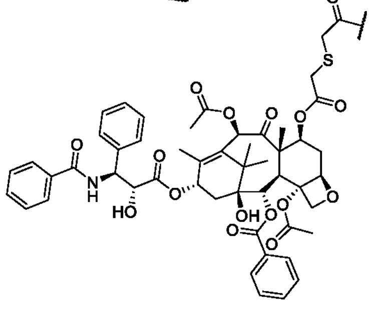
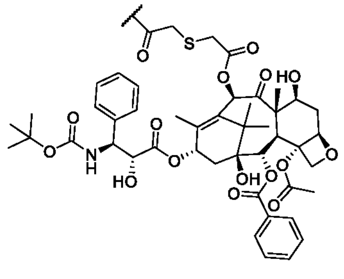
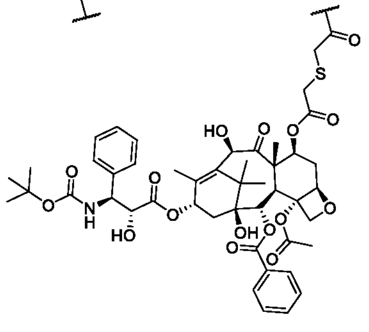

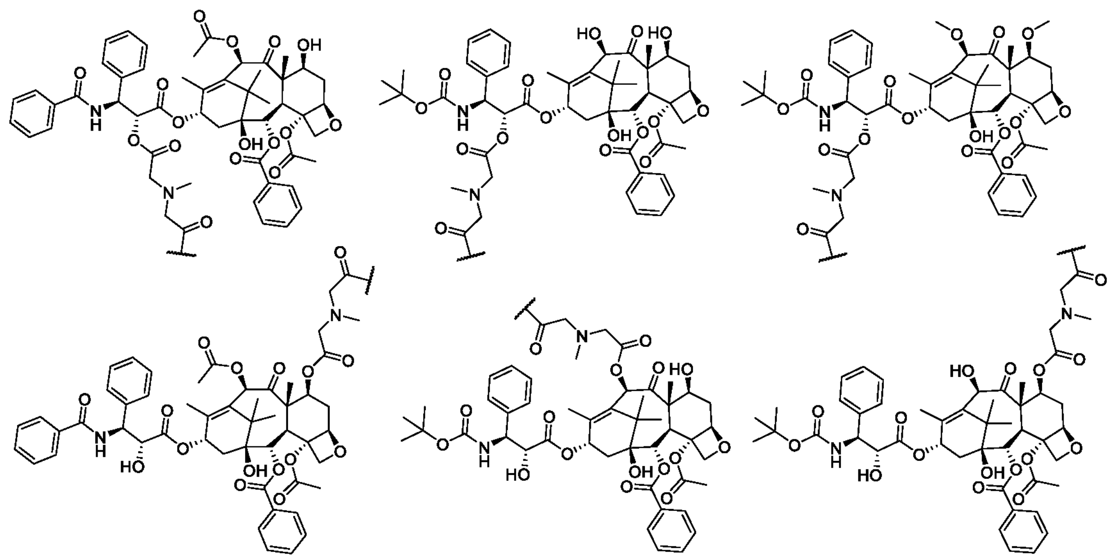

$R^p$ =

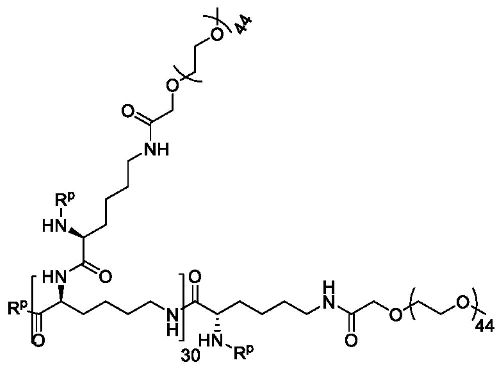

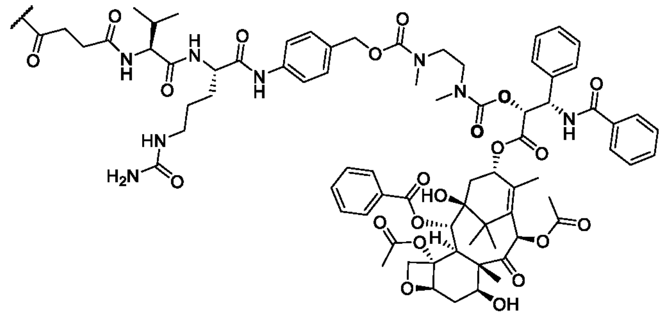

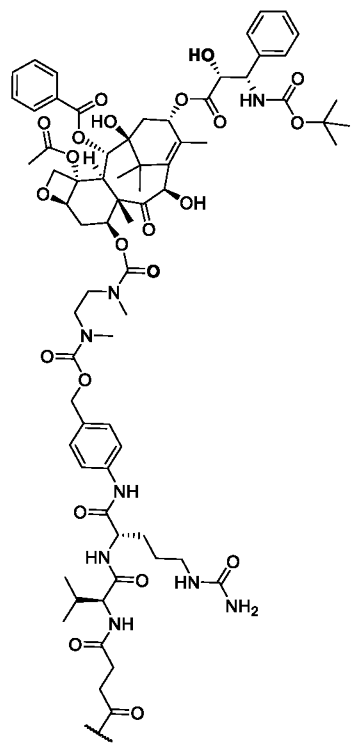

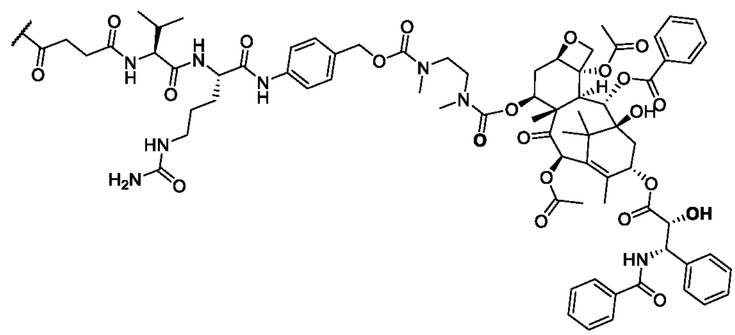

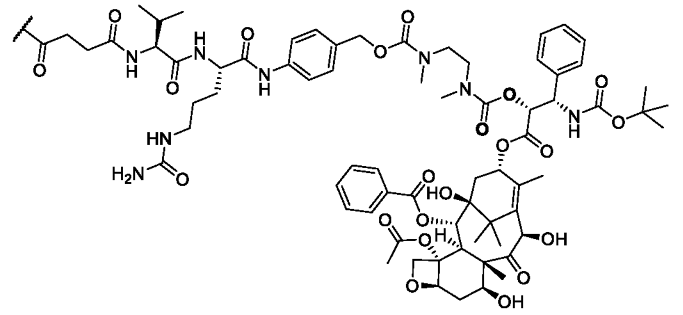

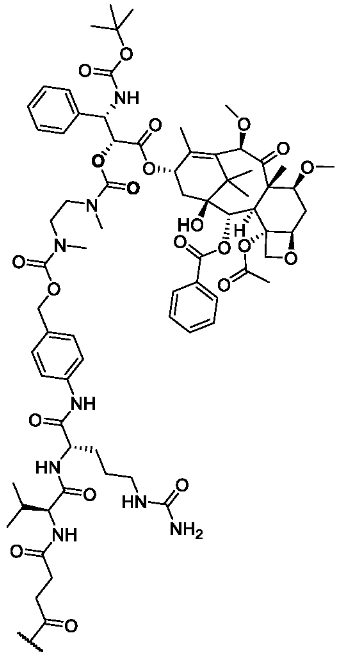

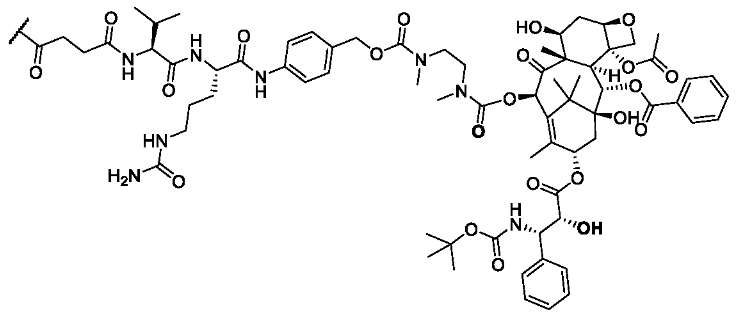

$R^p$ =

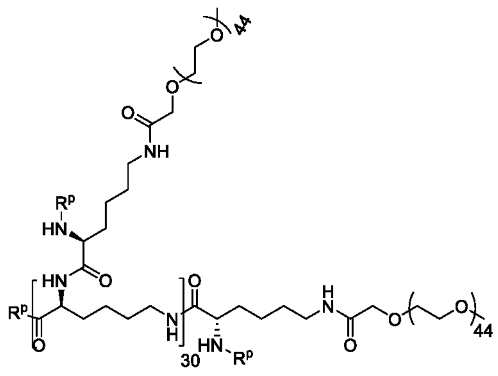

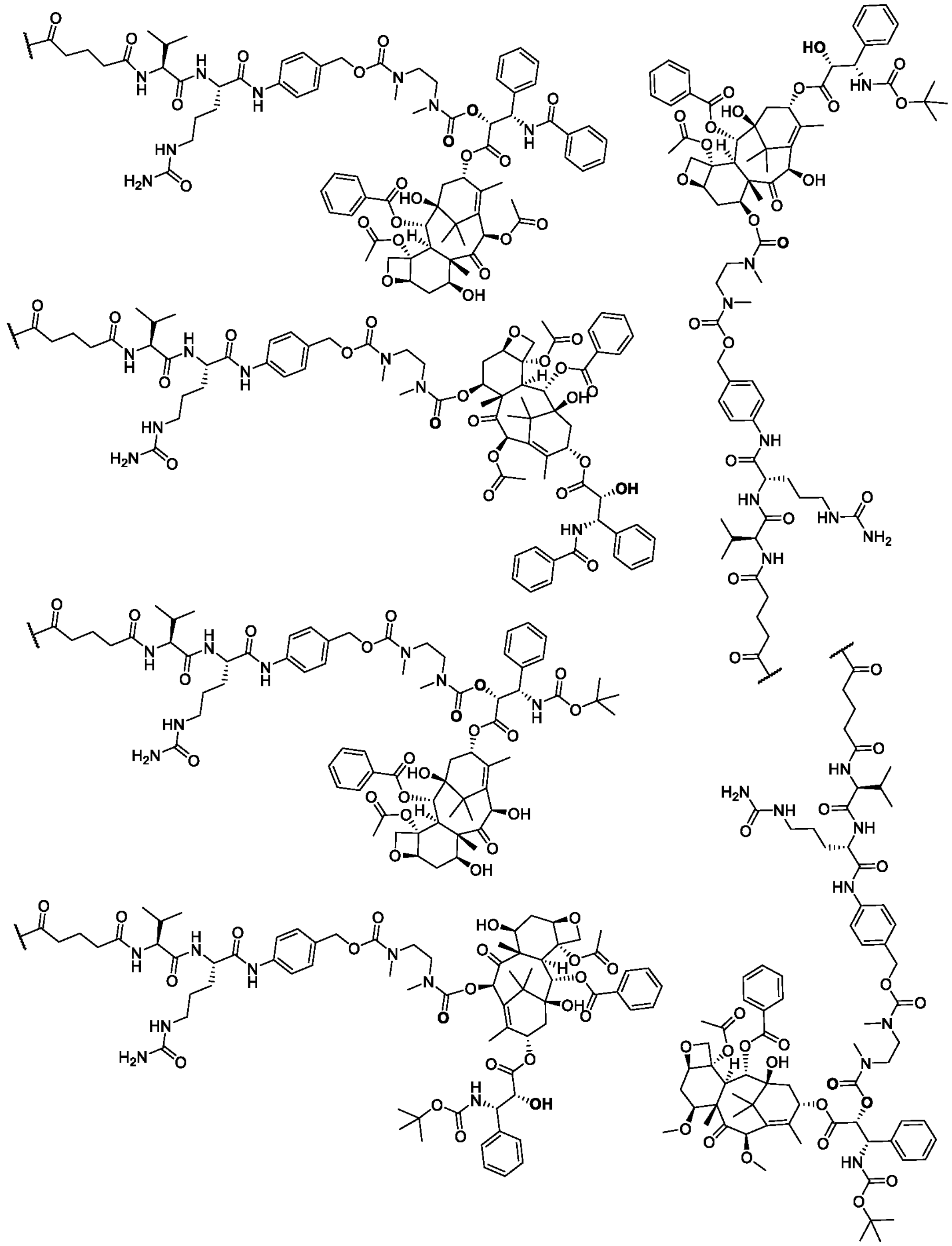

$R^p$ =

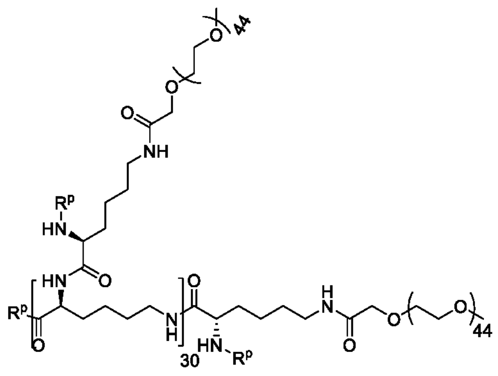

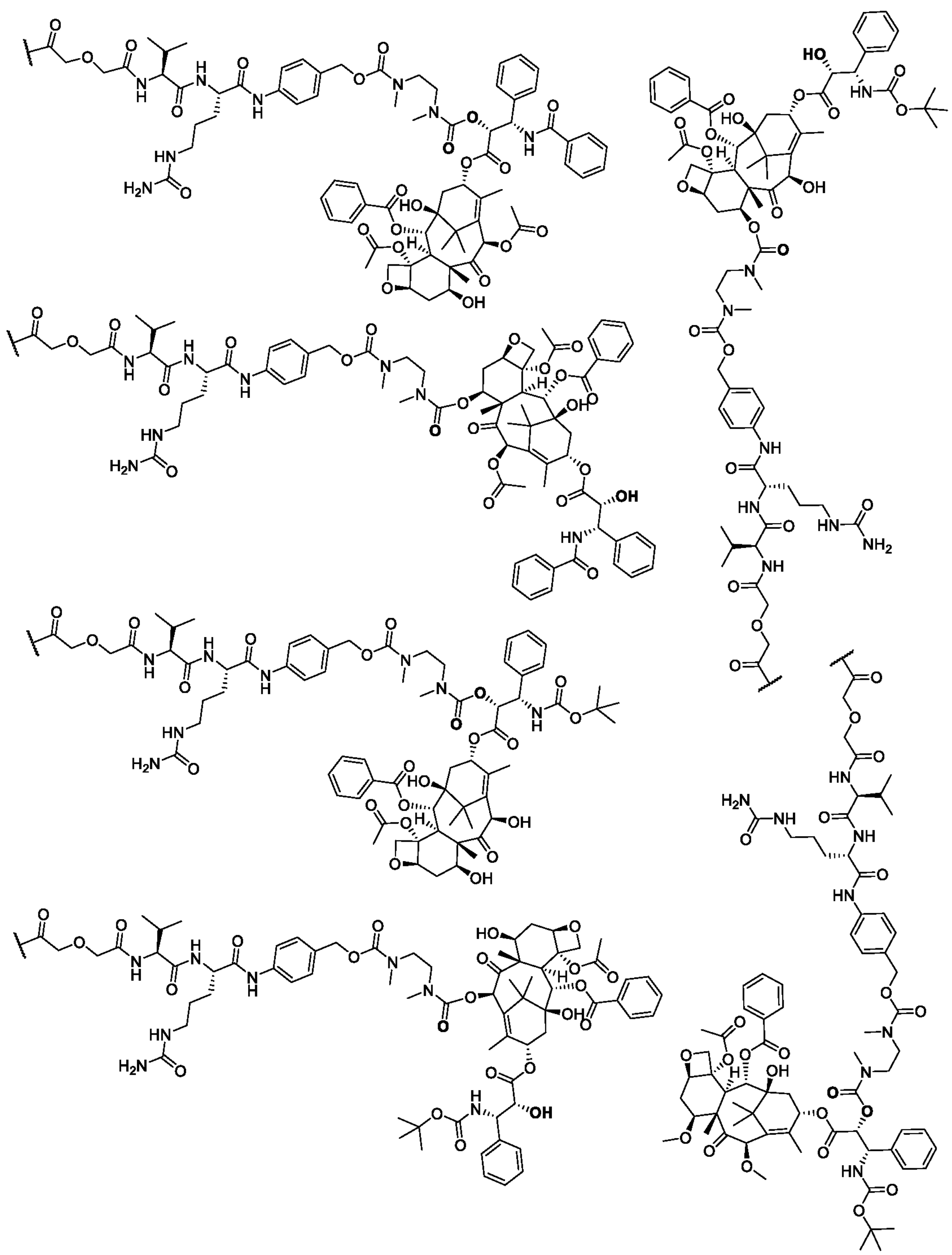

$R^p$ =

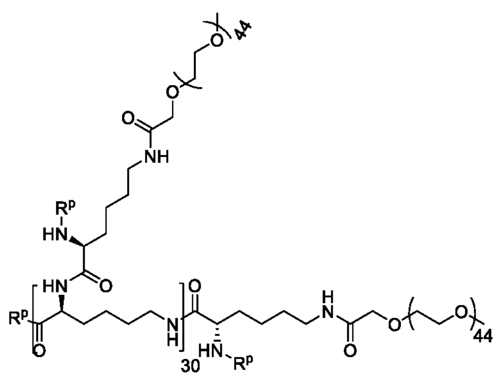

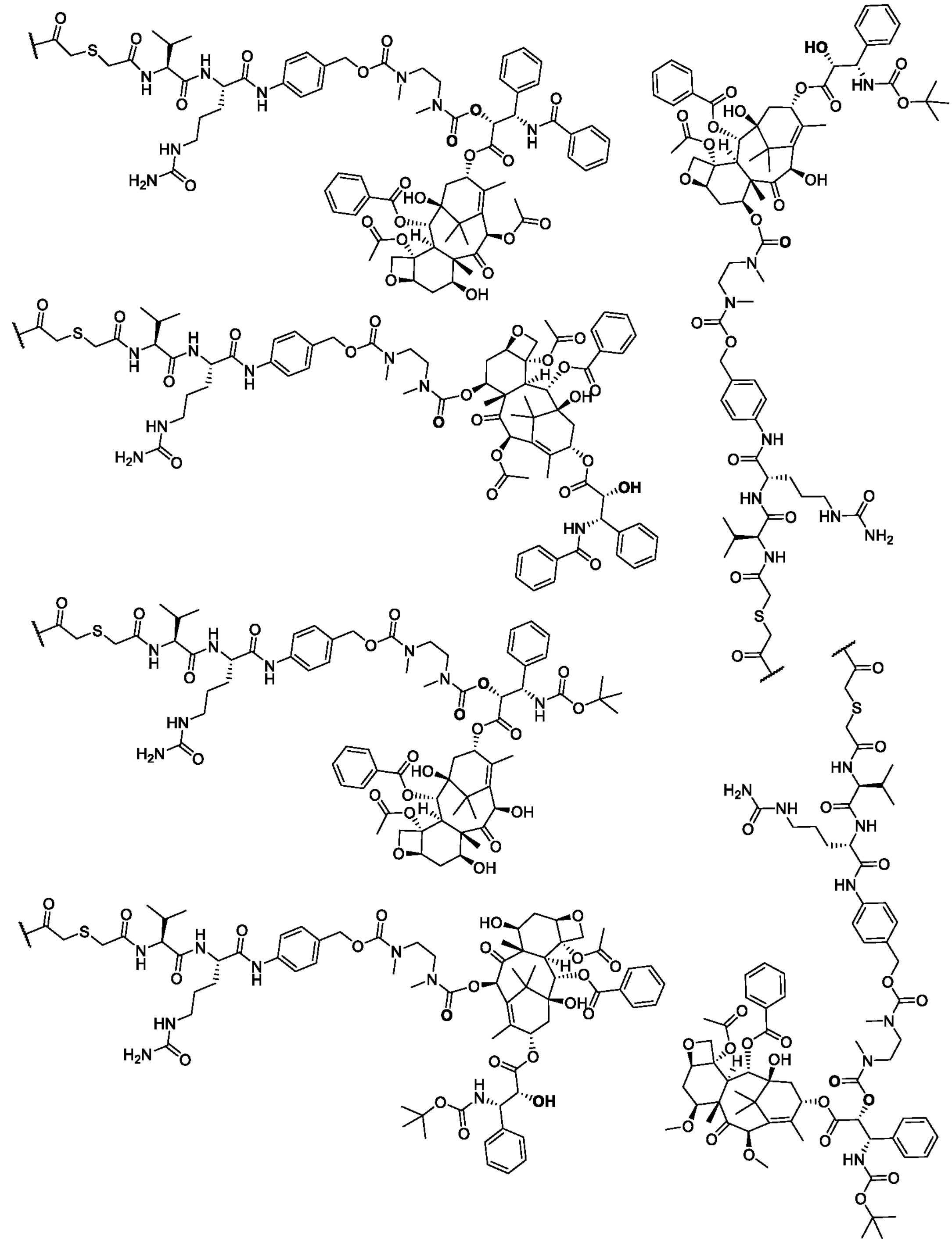

$R^p$ =

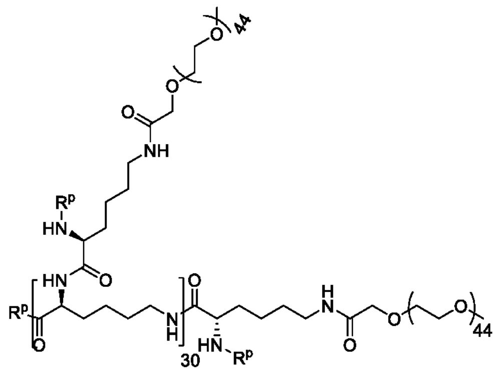

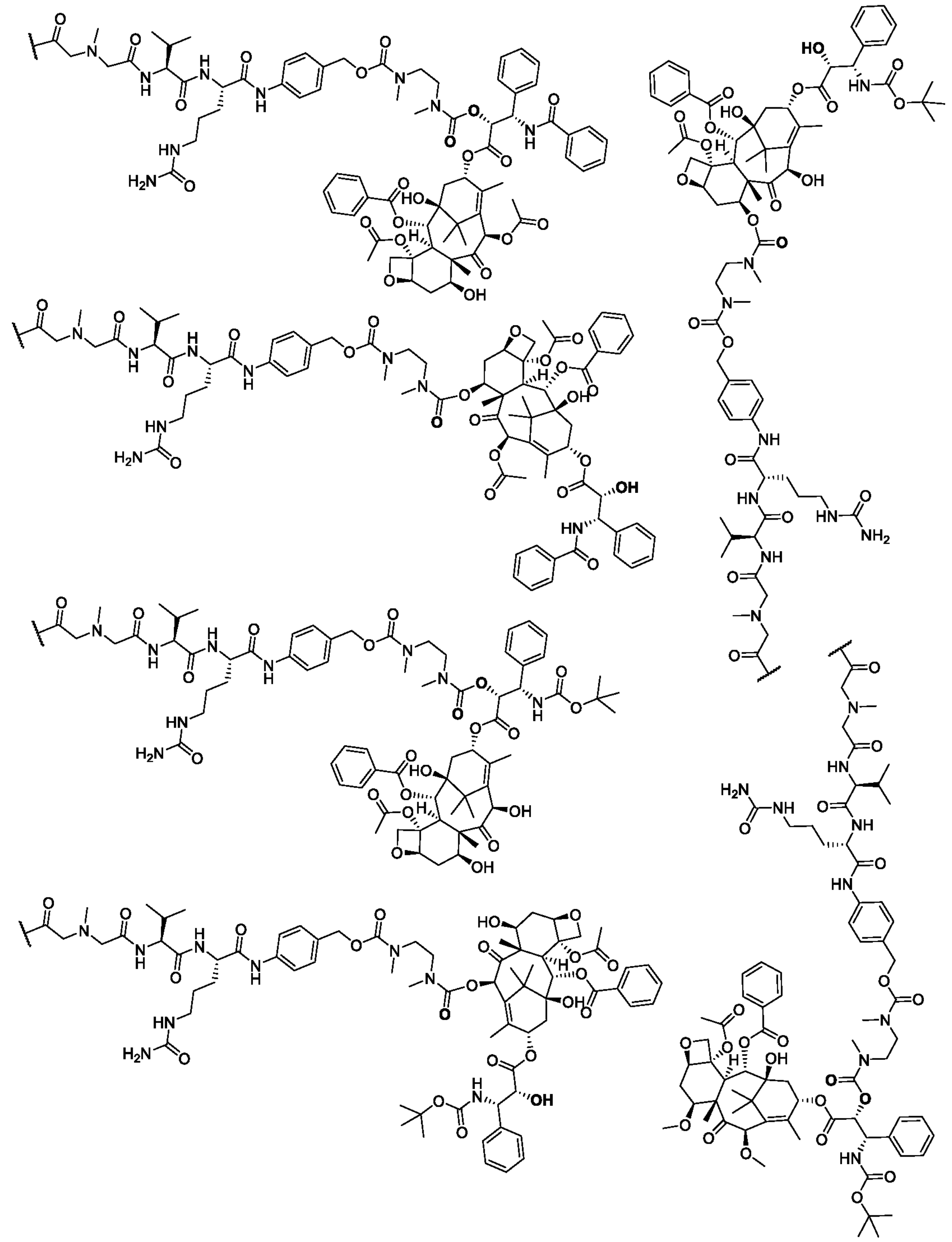

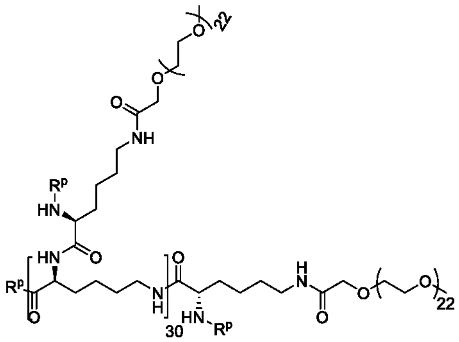

$R^p$ =

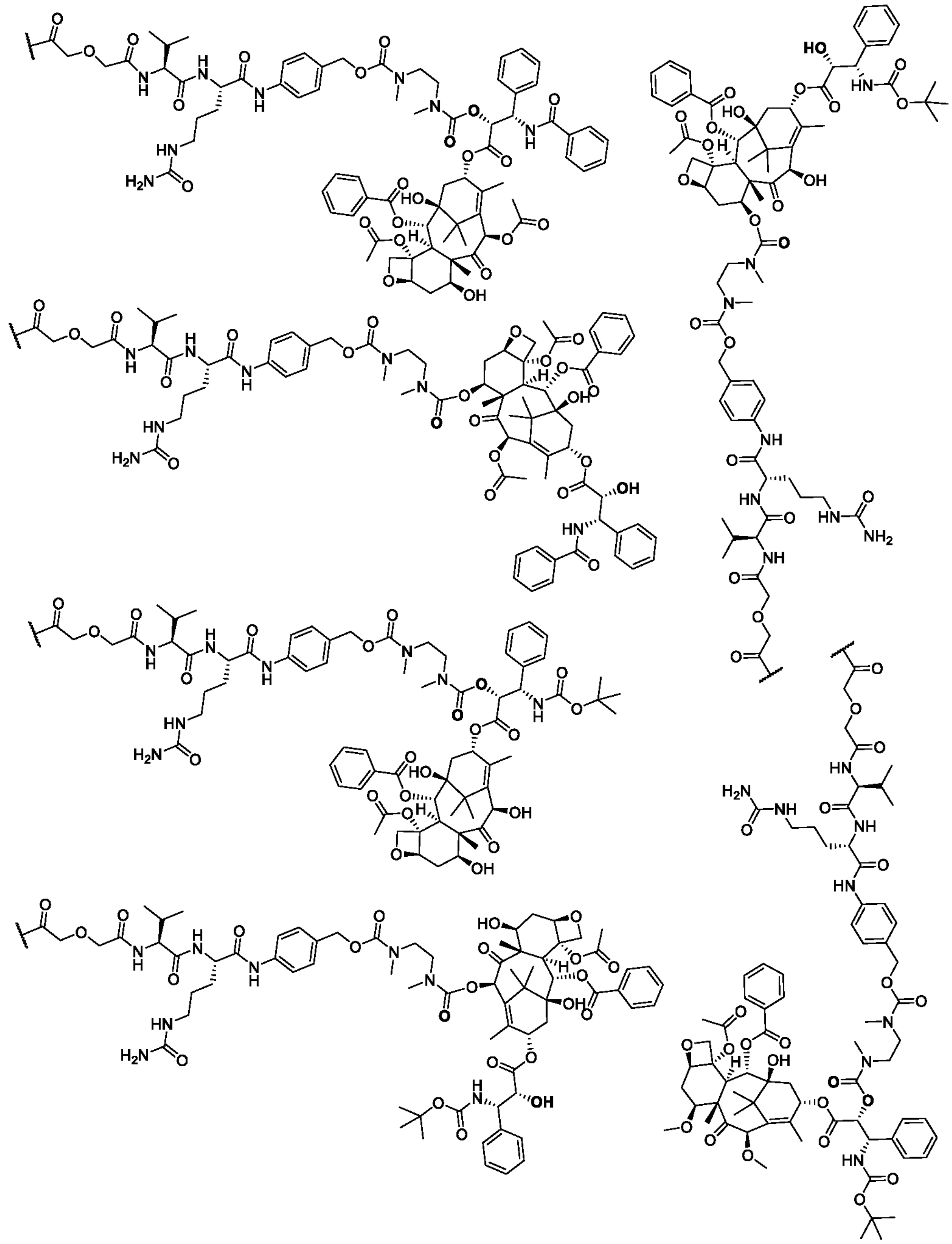

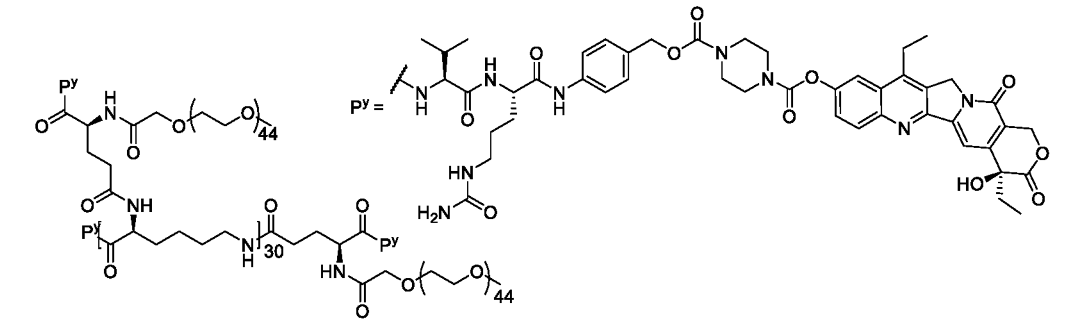

$P^y$ =

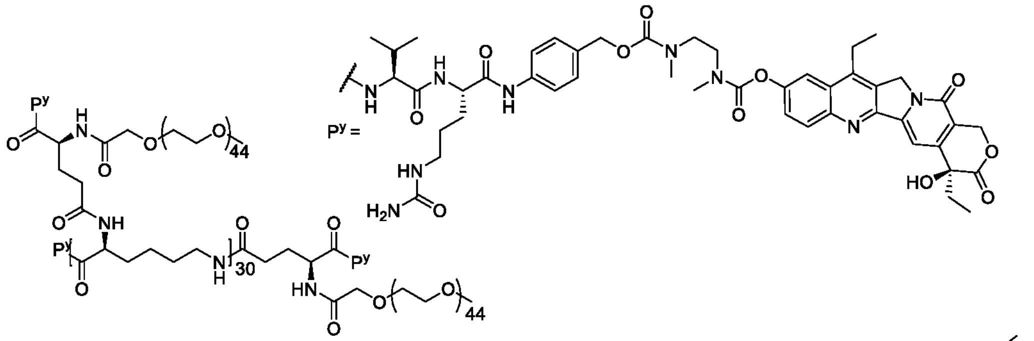

$P^y$ =

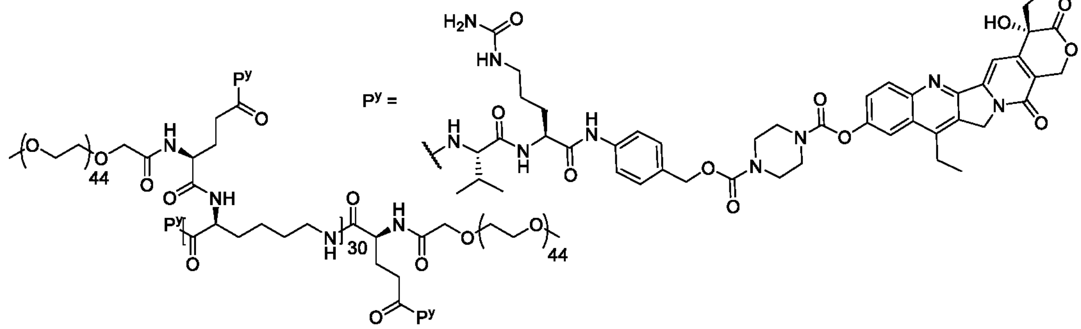

$P^y$ =

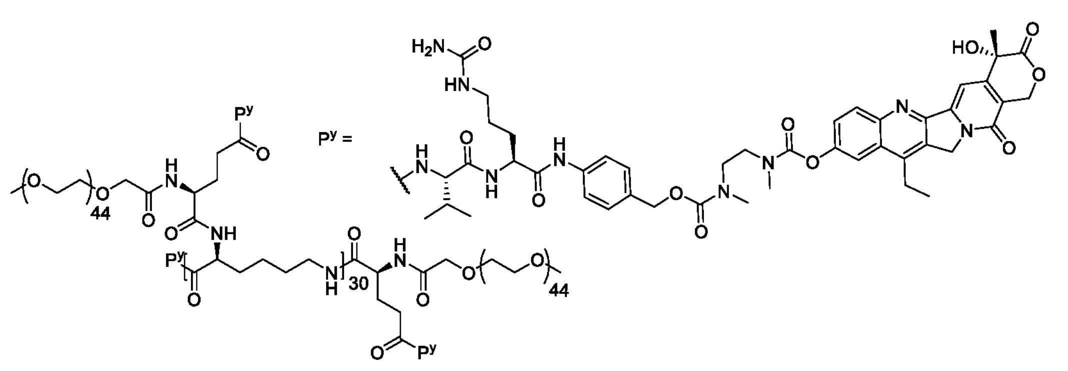

$P^y$ =

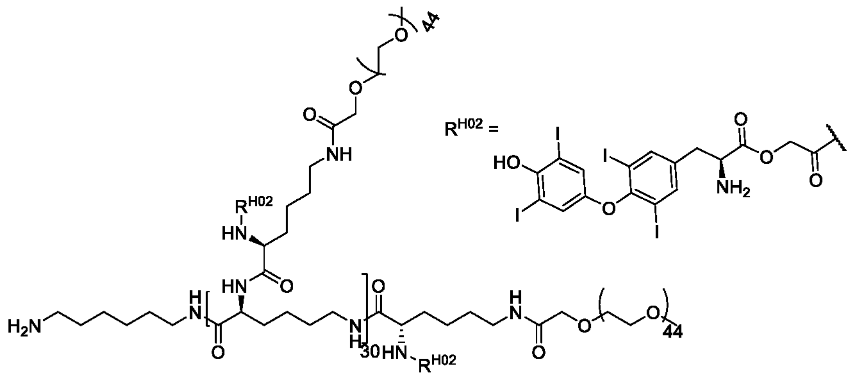

$R^{H02}$ =

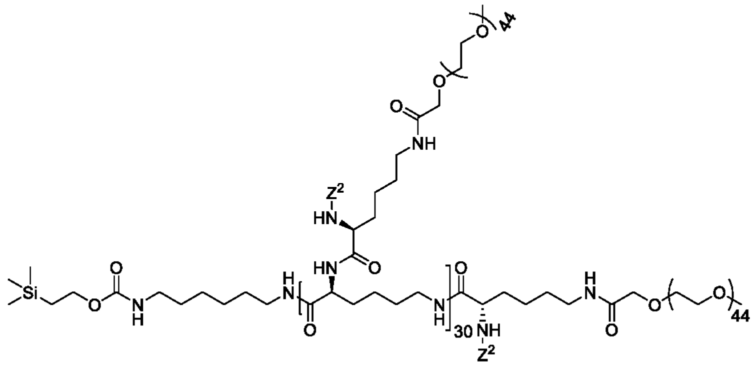

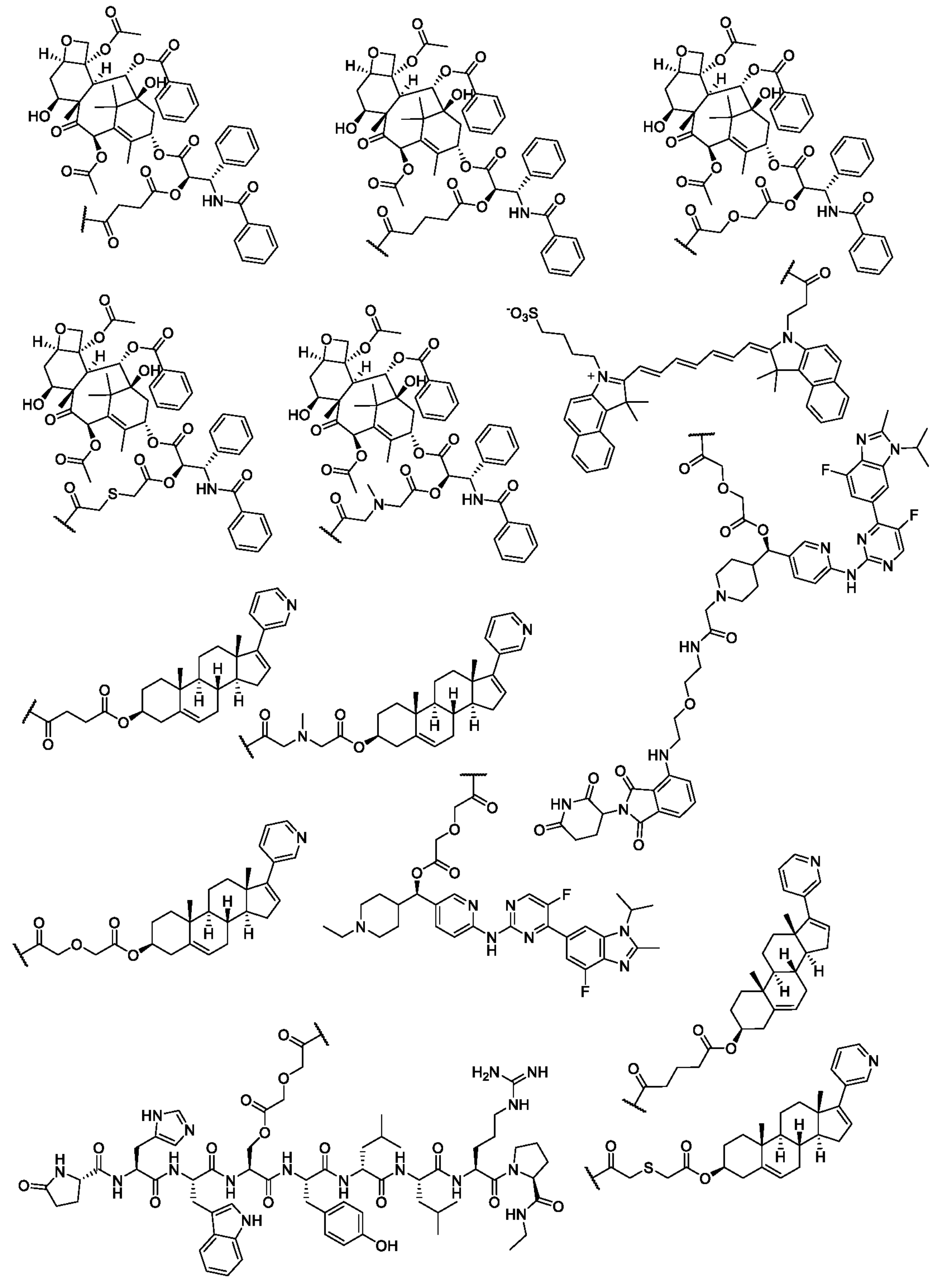

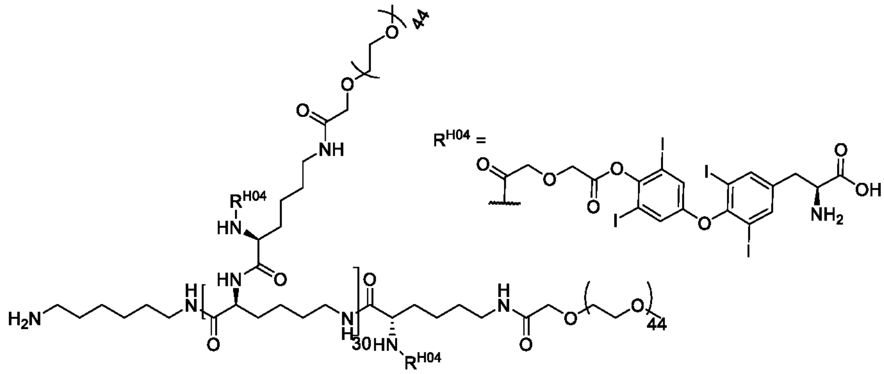

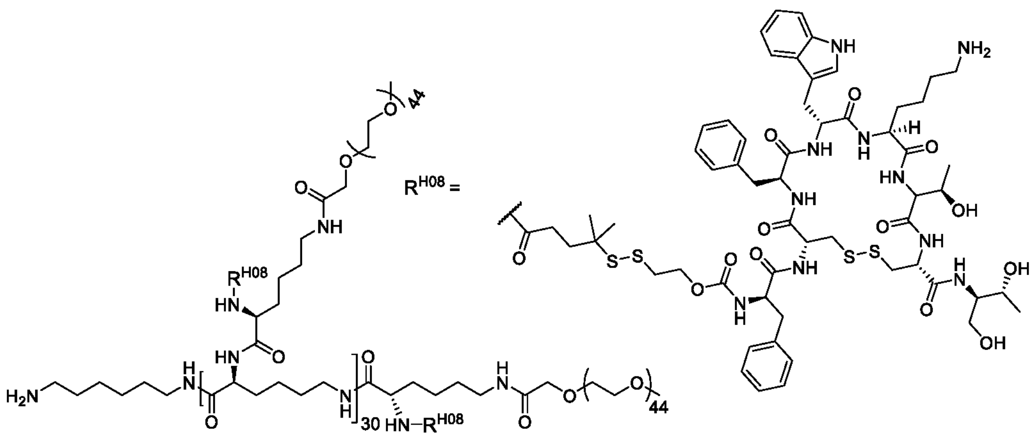

**40.** Compounds as shown in formulae VI-XV:

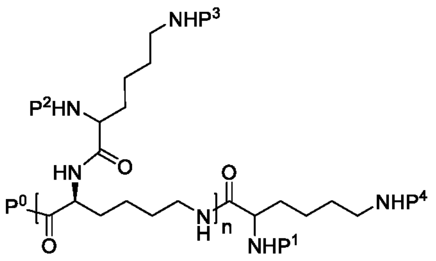

VI

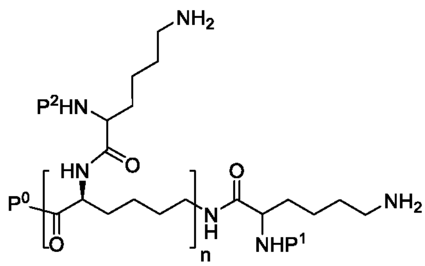

VII

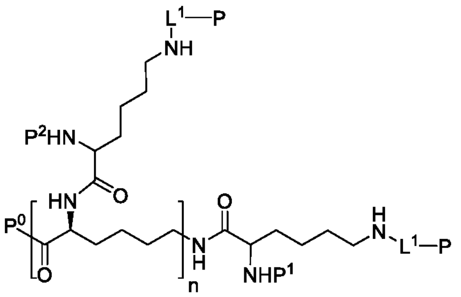

VIII

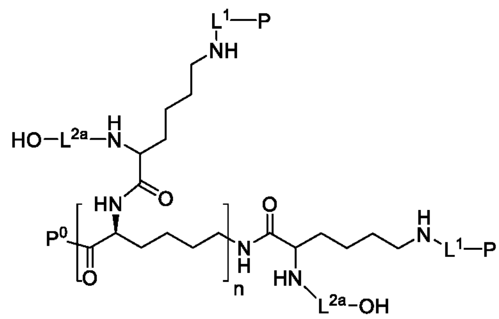

IX

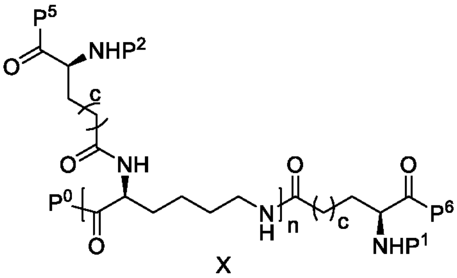

X

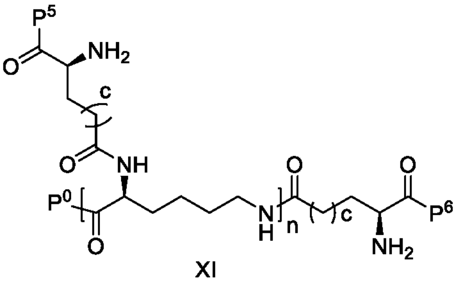

XI

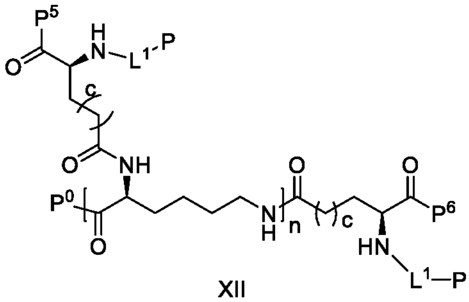

XII

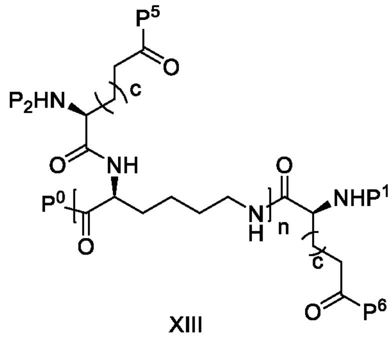

XIII

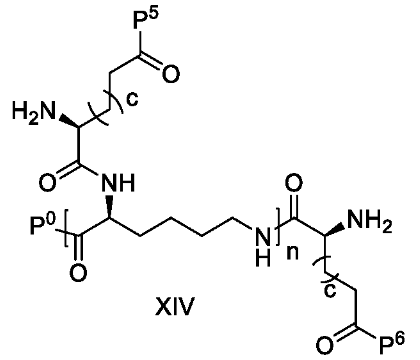

XIV

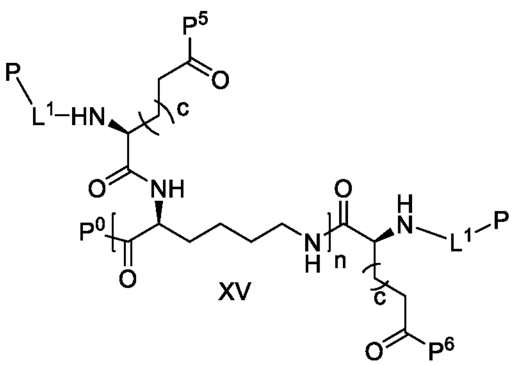

XV

wherein,

$P^0$, $P^5$, and $P^6$ are each independently selected from OH or carboxyl protecting groups; when $P^0$, $P^5$, and $P^6$ are carboxyl protection groups, they could be the same or different;

$P^1$, $P^2$, $P^3$ and $P^4$ are each independently selected from H or amine protecting groups; when $P^1$, $P^2$, $P^3$ and $P^4$ are amine protecting groups, they could be the same or different;

$L^1$ is defined as described in any of claims 1 and 4-5;

$L^{2a}$ is defined as described in claim 1 or 13;

P is defined as described in any of claims 1 and 17-19;

n is an integer of 4 to 100;

c is an integer of 0 to 3.

**41.** The compounds as shown in formulae VI-XV according to claim 40, wherein,

n is an integer of 10 to 70;

$L^1$ is selected from a covalent bond, C(O), or C(O)-O;

P is a methyl-terminated polyethylene glycol derivative residue, the number-average molecular weight of which is 500-5000;

$L^{2a}$ is C(O)-E-C(O), and the E is selected from a covalent bond, $CH_2$, $CH_2$-$CH_2$, $CH_2$-$CH_2$-$CH_2$, $CH_2$-O-$CH_2$, $CH_2$-S-$CH_2$, $CH_2$-NH-$CH_2$, $CH_2$-N(Me)-$CH_2$ or CH=CH.

**42.** The compounds as shown in formulae VI-XV according to claim 41, wherein the compounds as shown in formulae

VI-XV are selected from any of the following compounds:

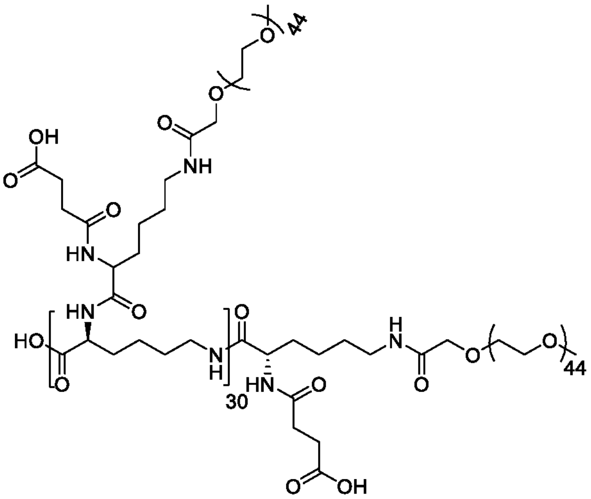

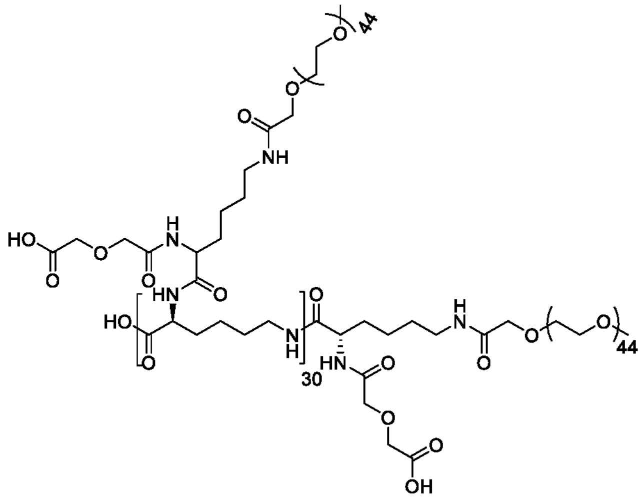

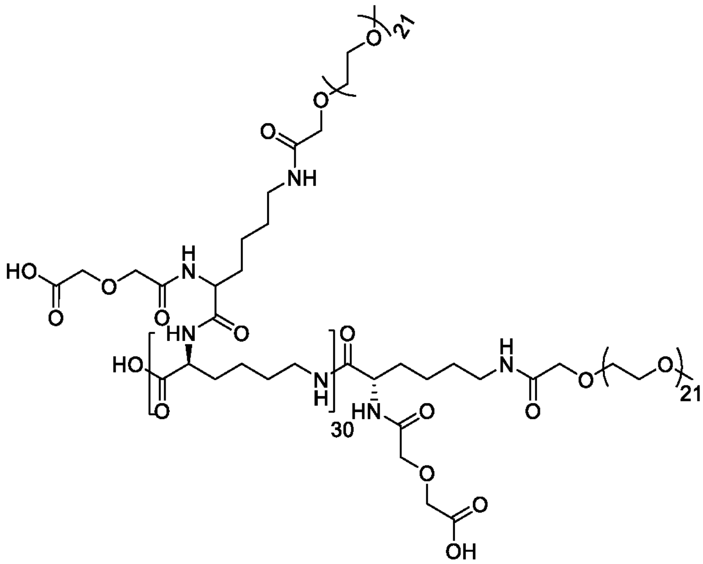

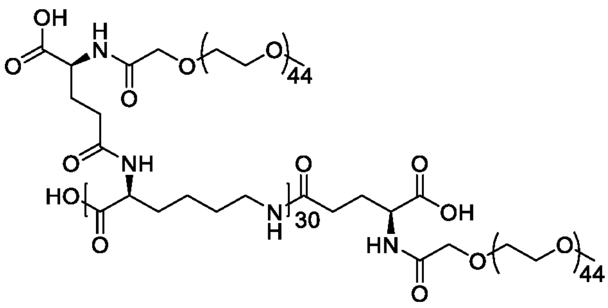

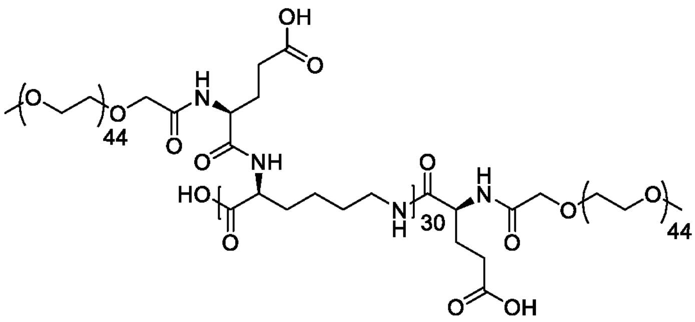

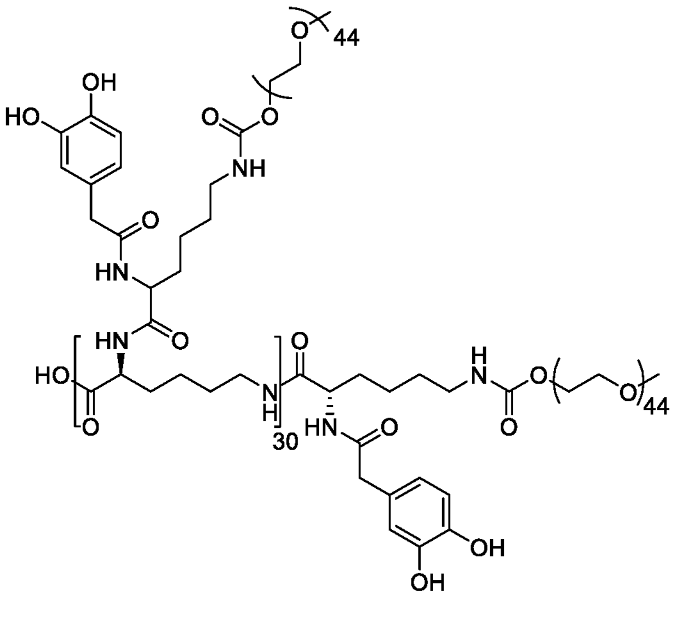

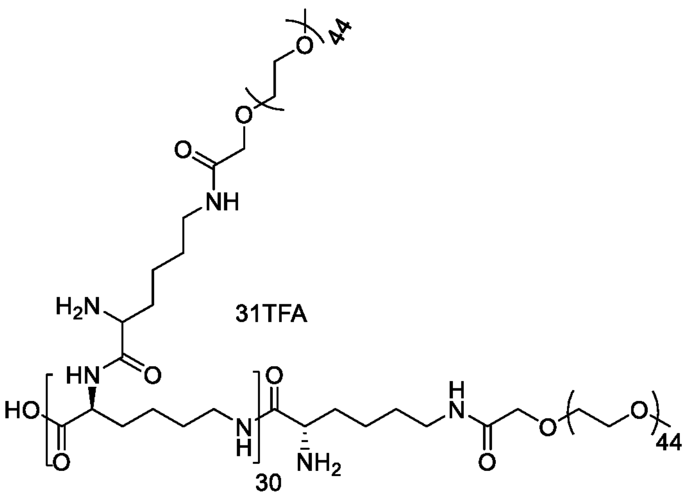

31TFA

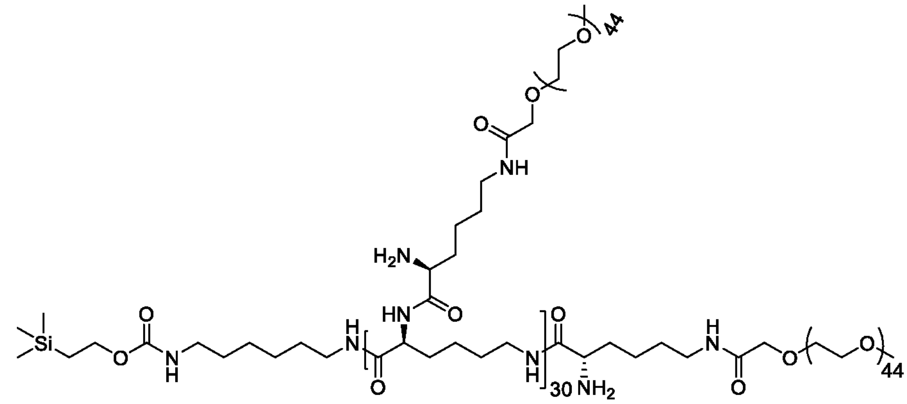

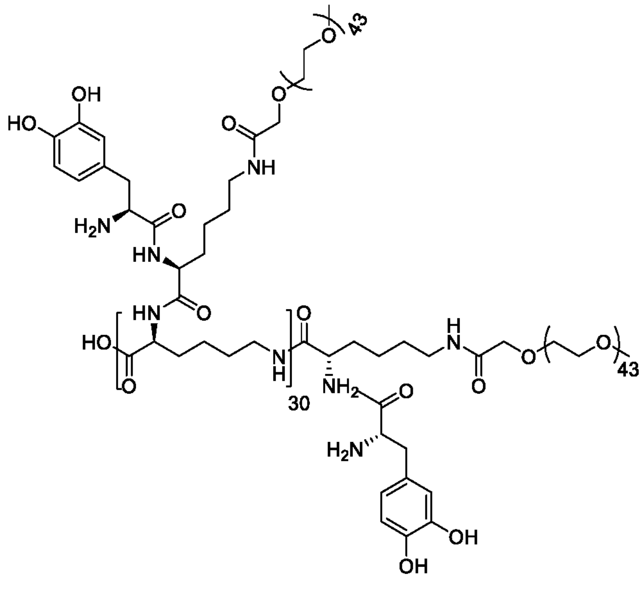

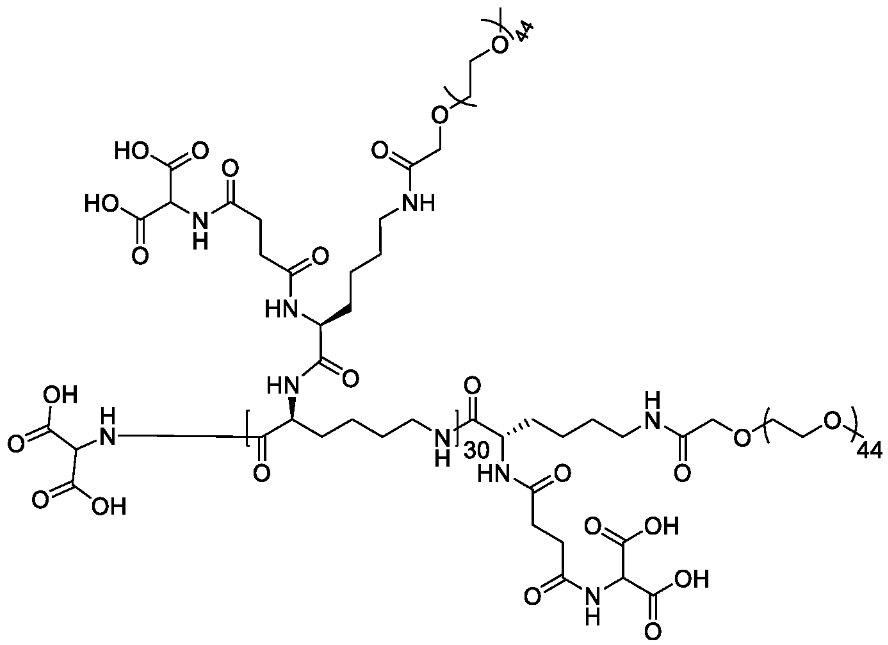

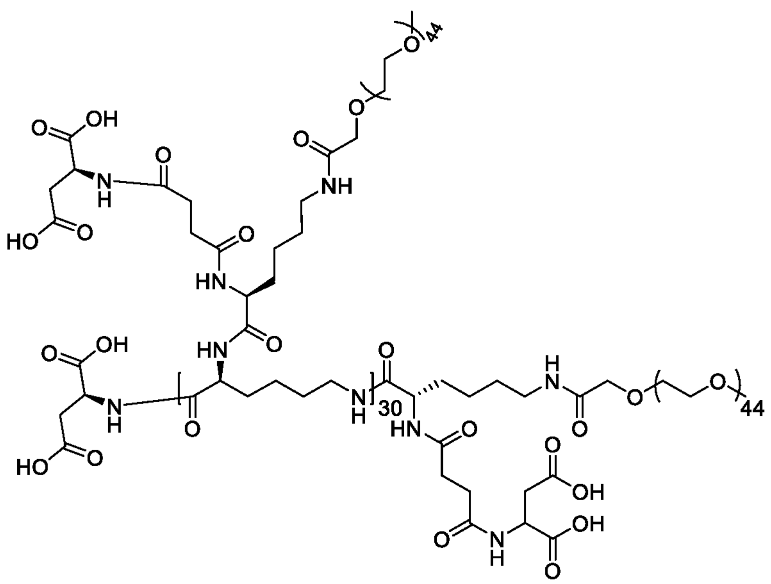

**43.** A compound as shown below:
$R^{1'}$-$L^2$-D, wherein $R^{1'}$ is H, -OH or $C_1$~$C_3$ alkyl; $L^2$ is defined as described in any of claims 4-6, 14, 16 or 38; D is defined as described in claim 25 or 38.

**44.** The compound according to claim 43, wherein the compound is selected from the following structures:

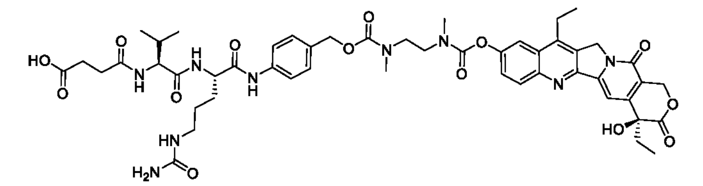

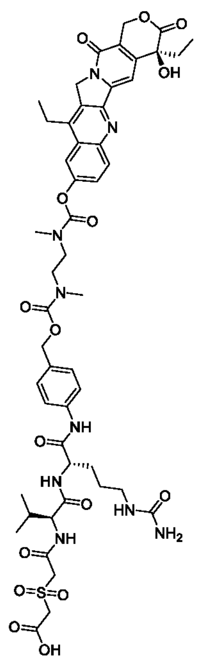

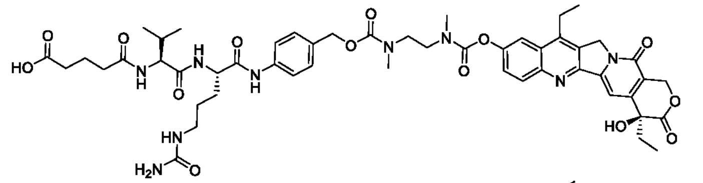

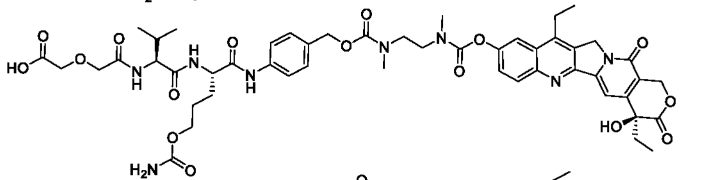

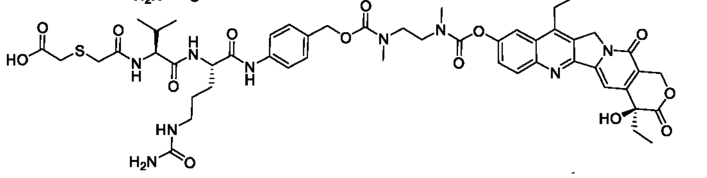

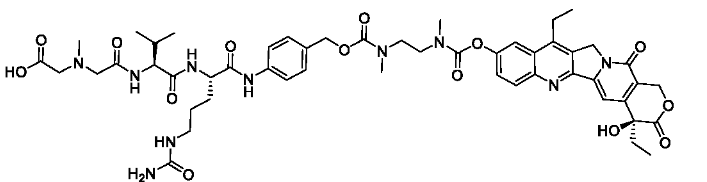

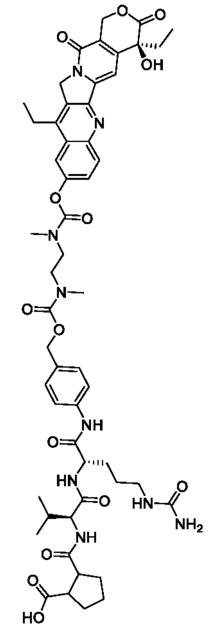

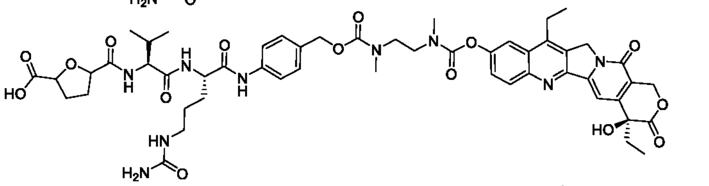

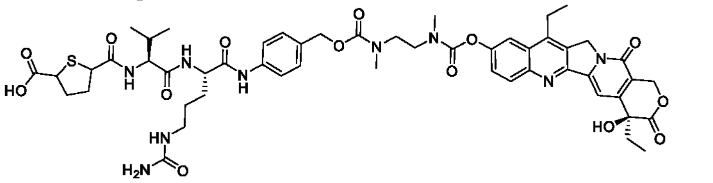

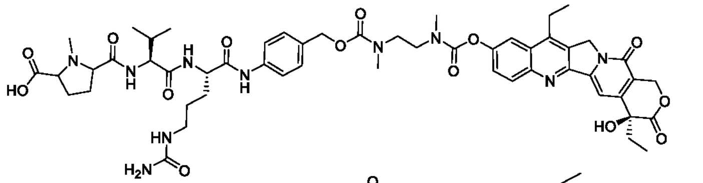

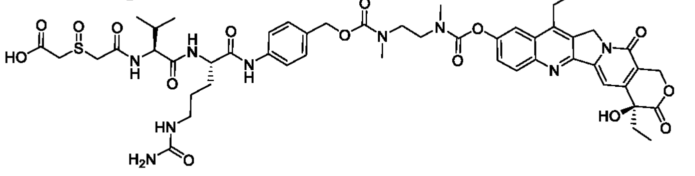

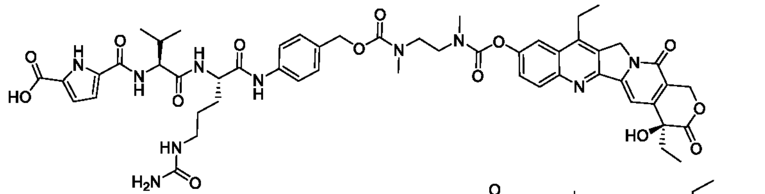

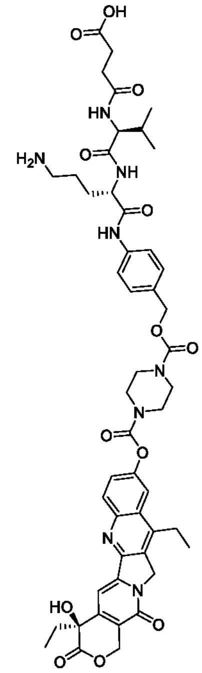

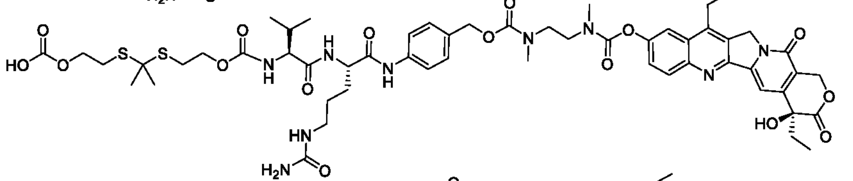

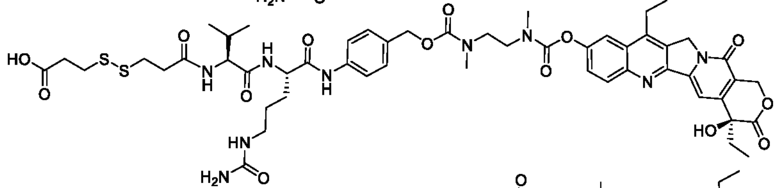

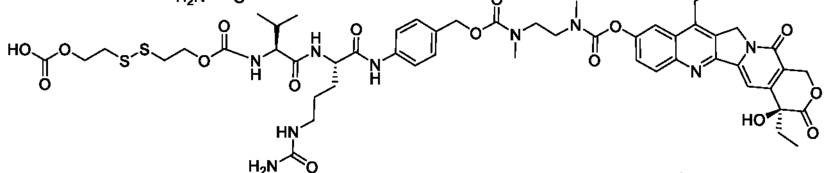

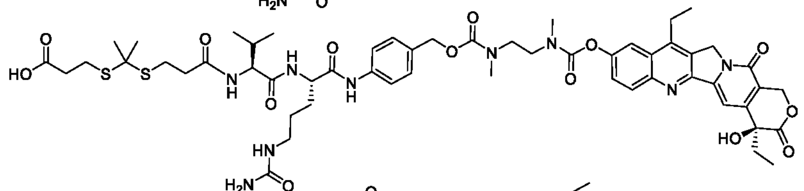

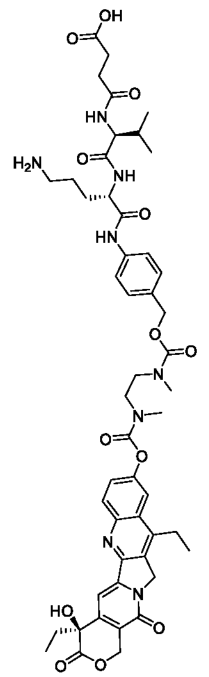

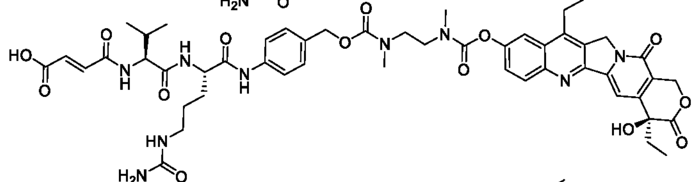

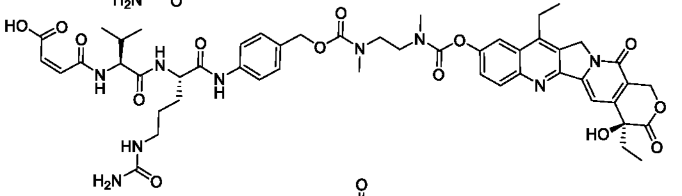

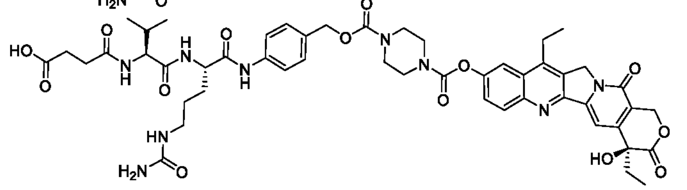

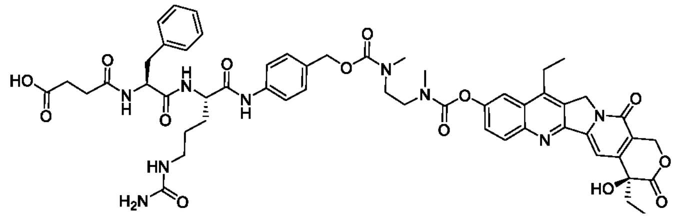

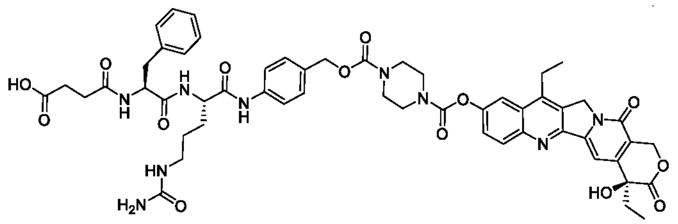

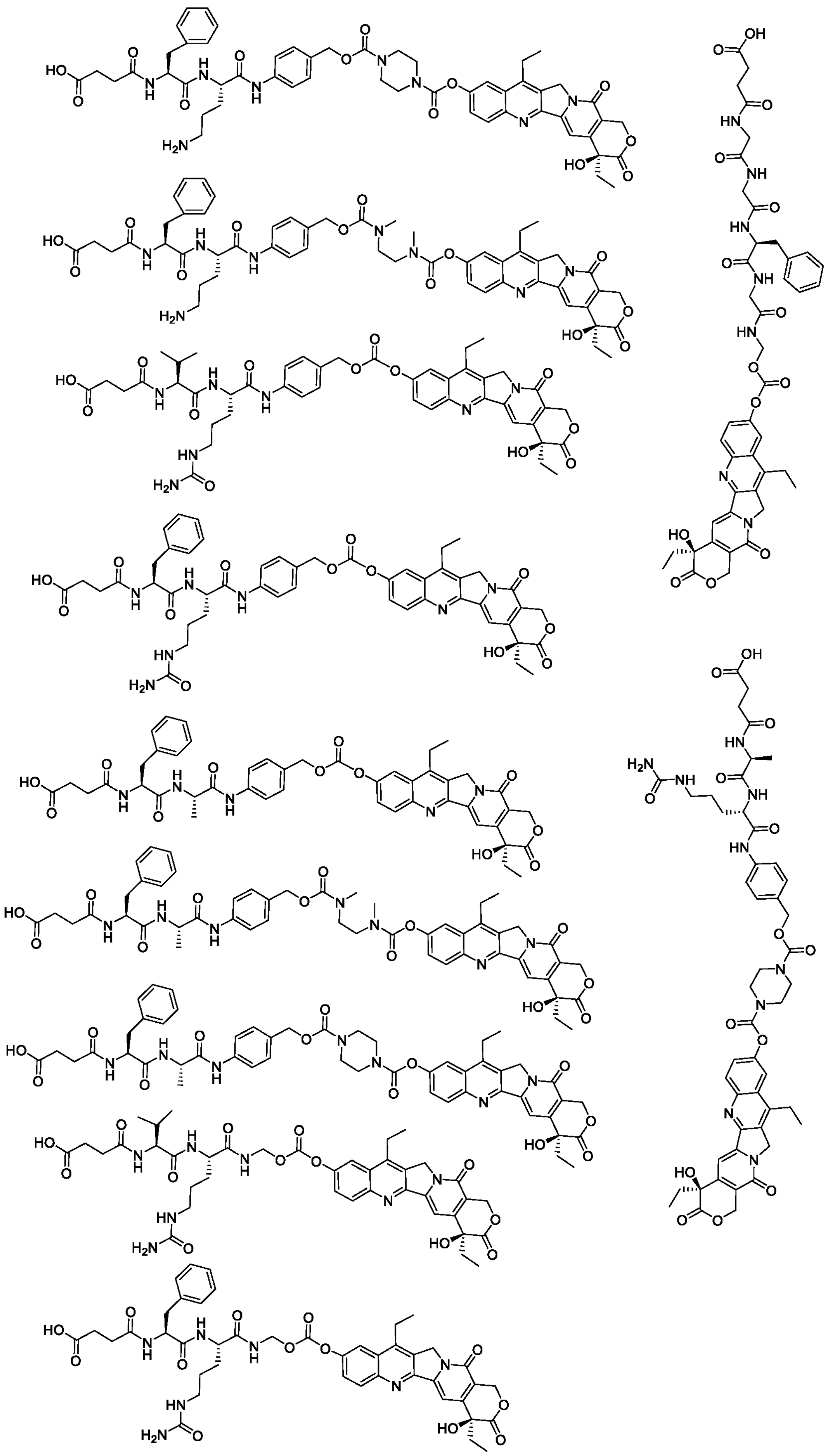

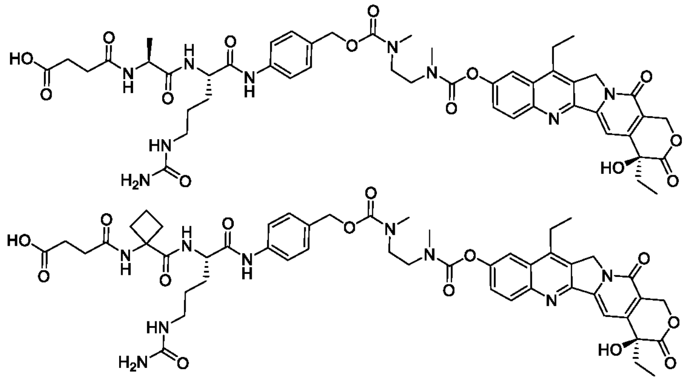

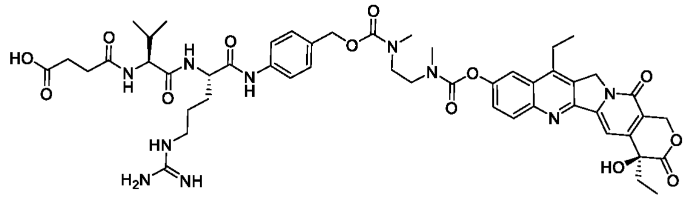

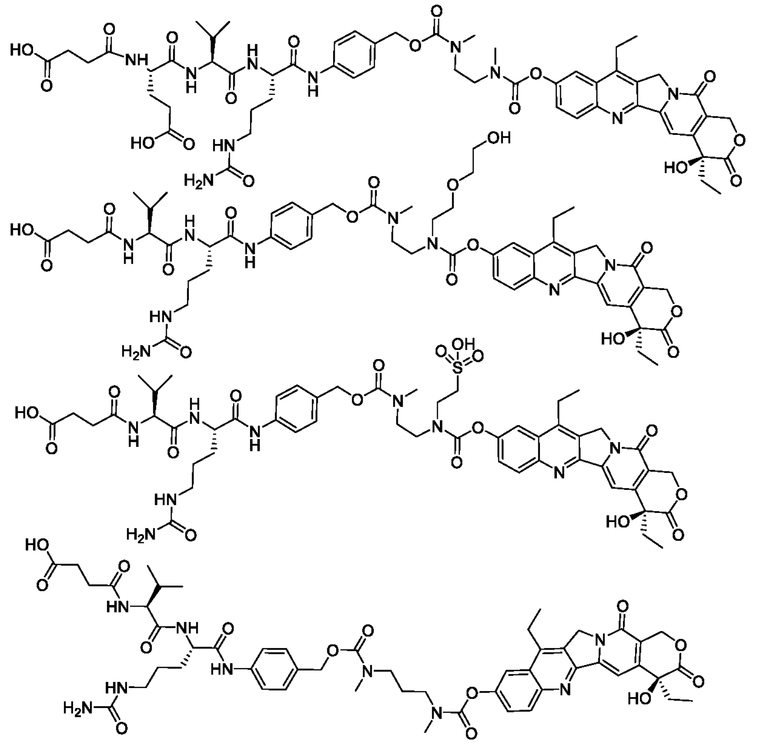

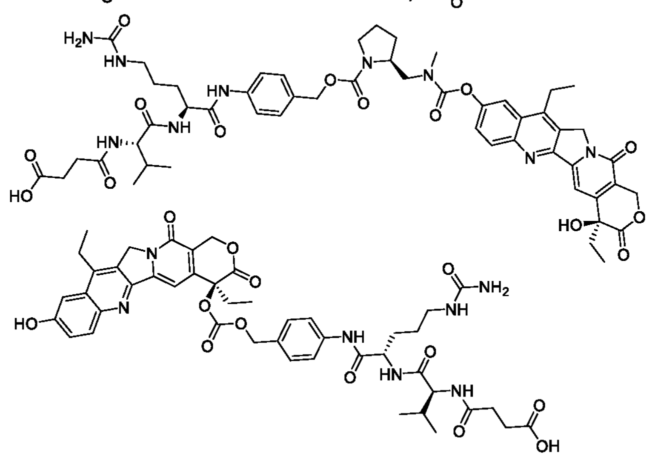

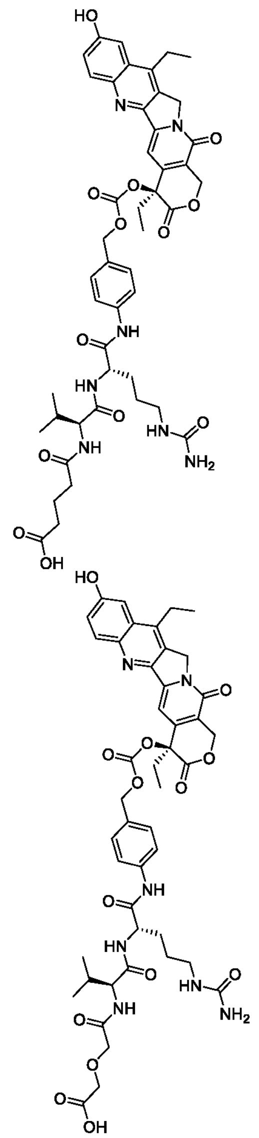

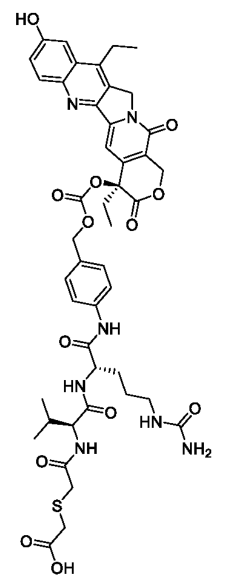

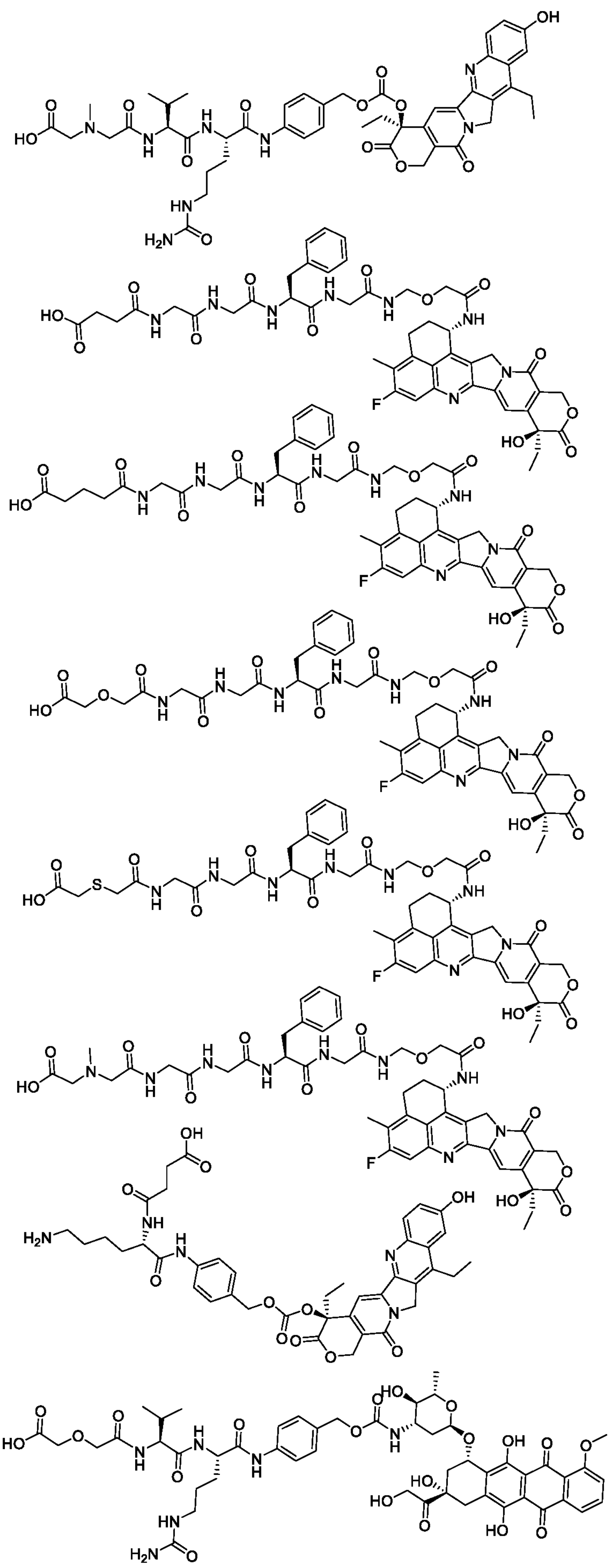

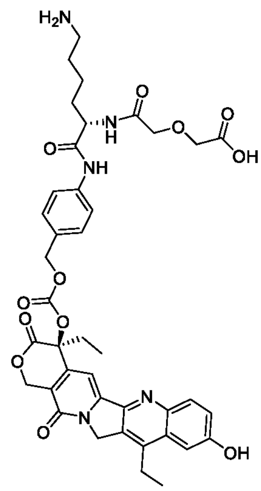

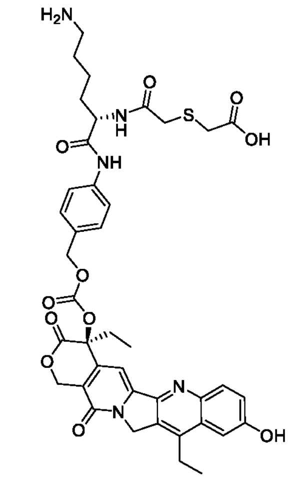

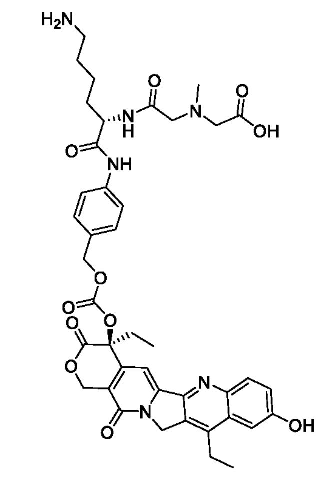

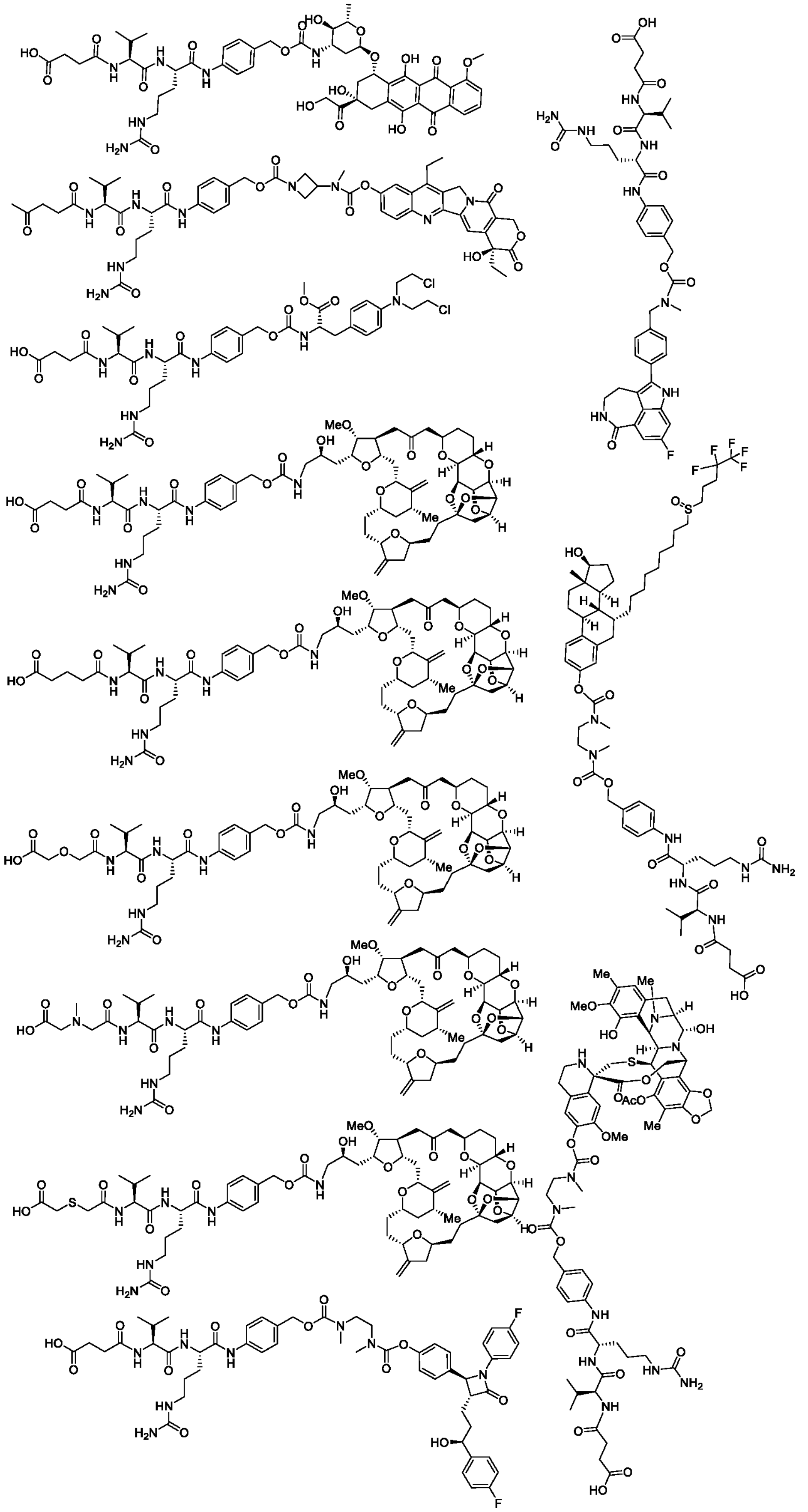

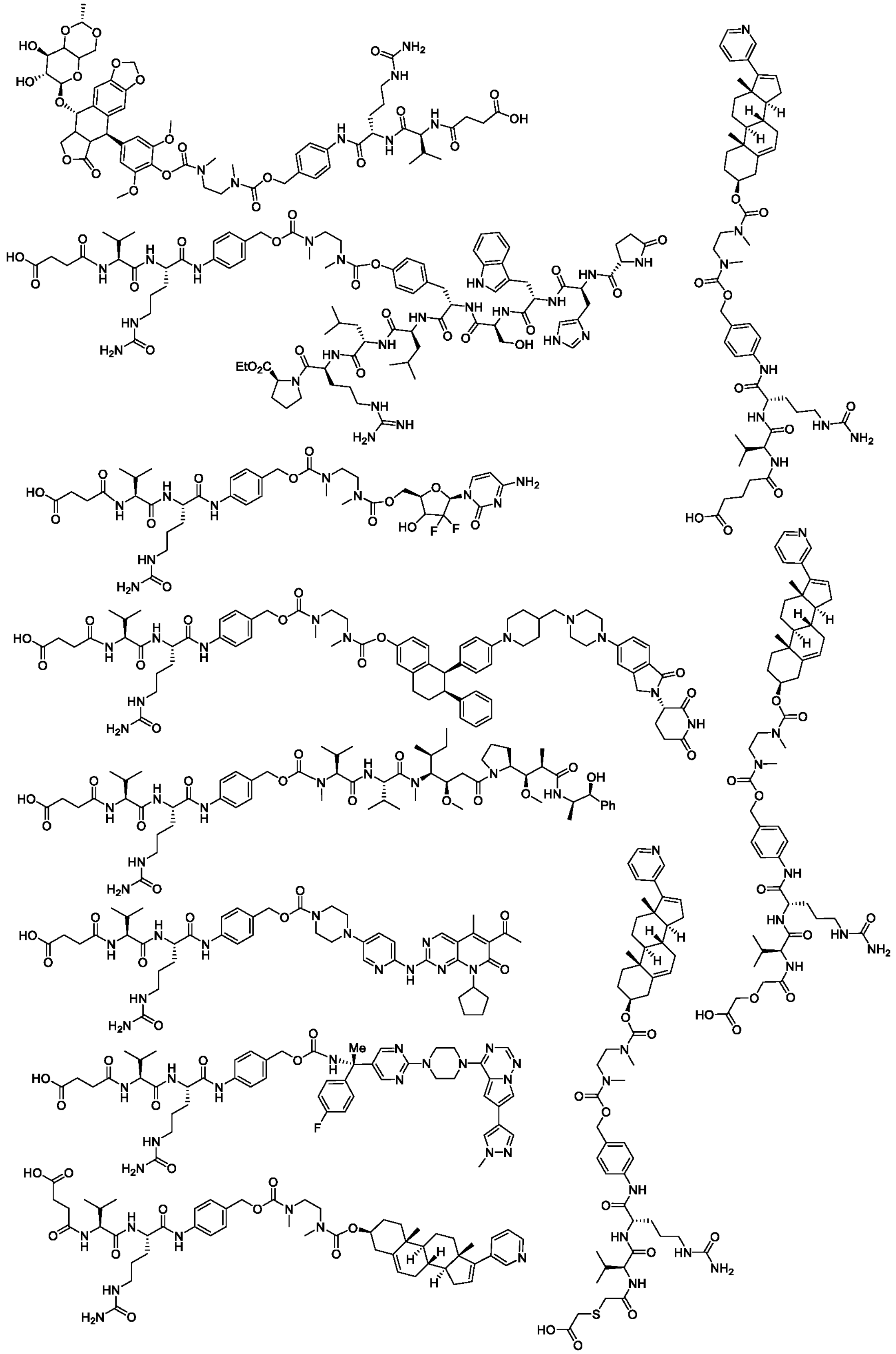

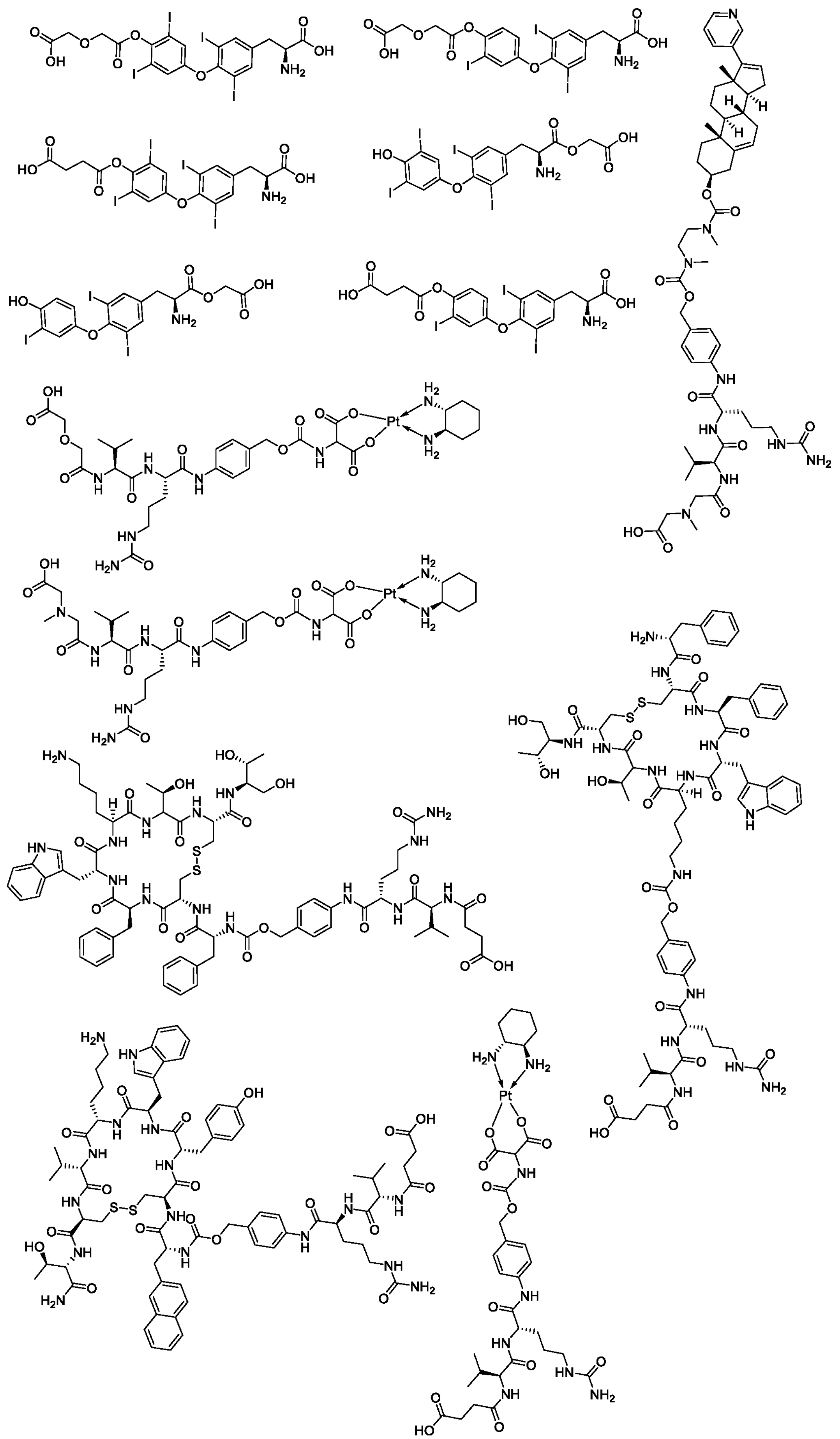

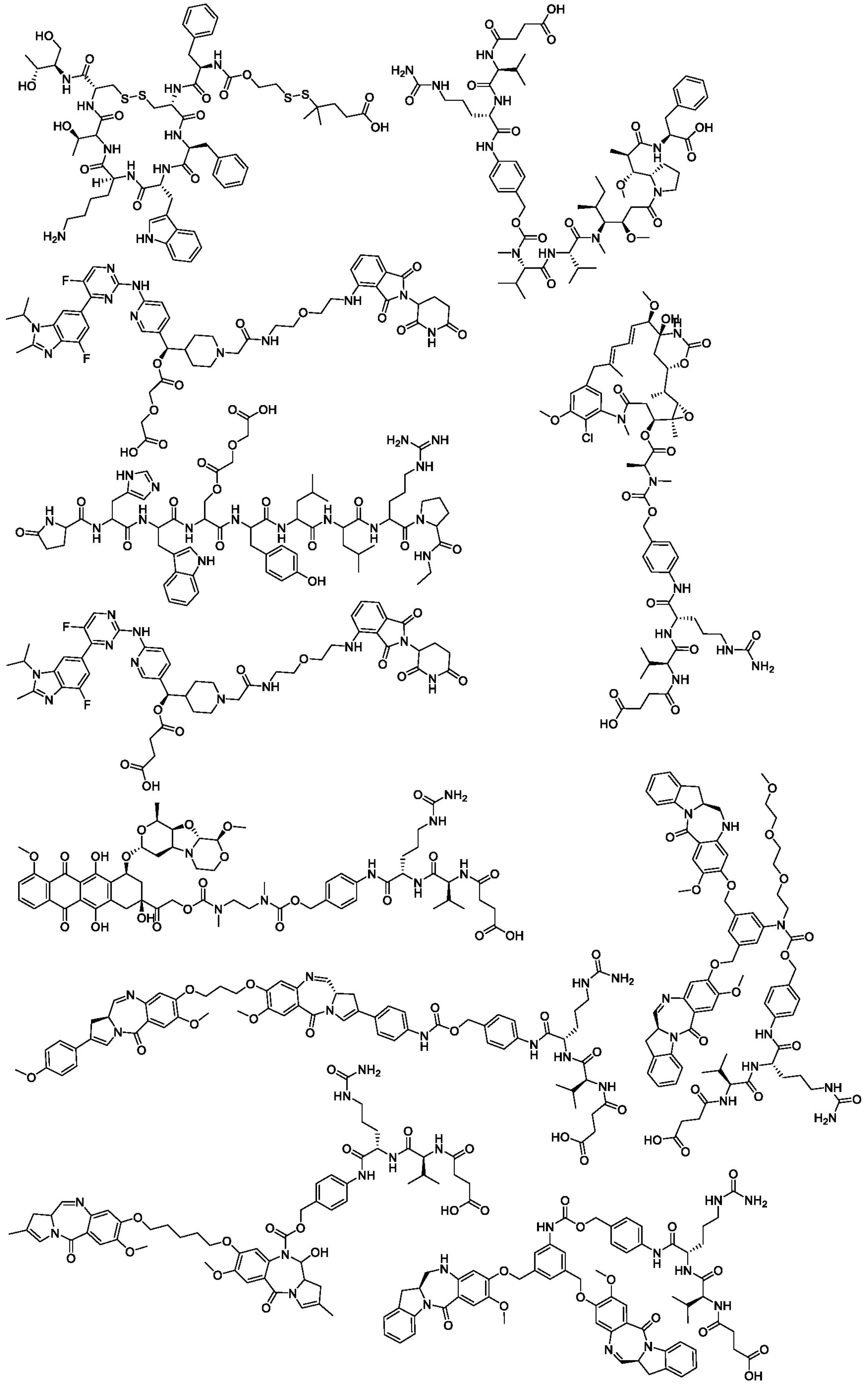

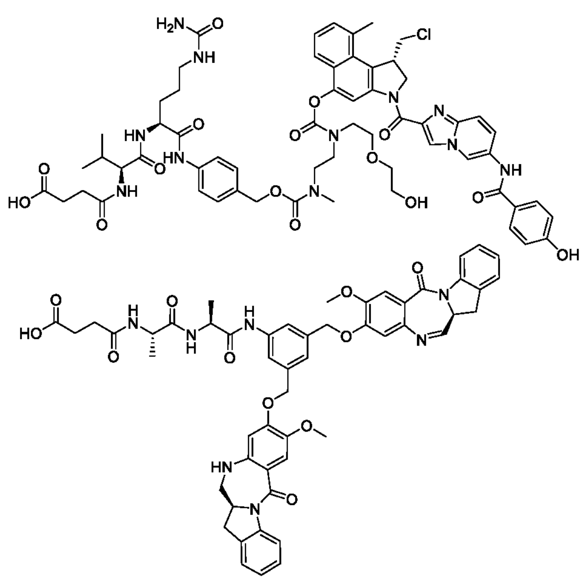

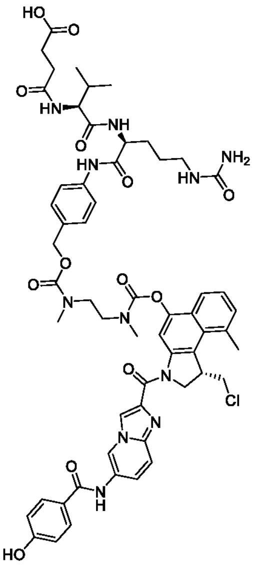

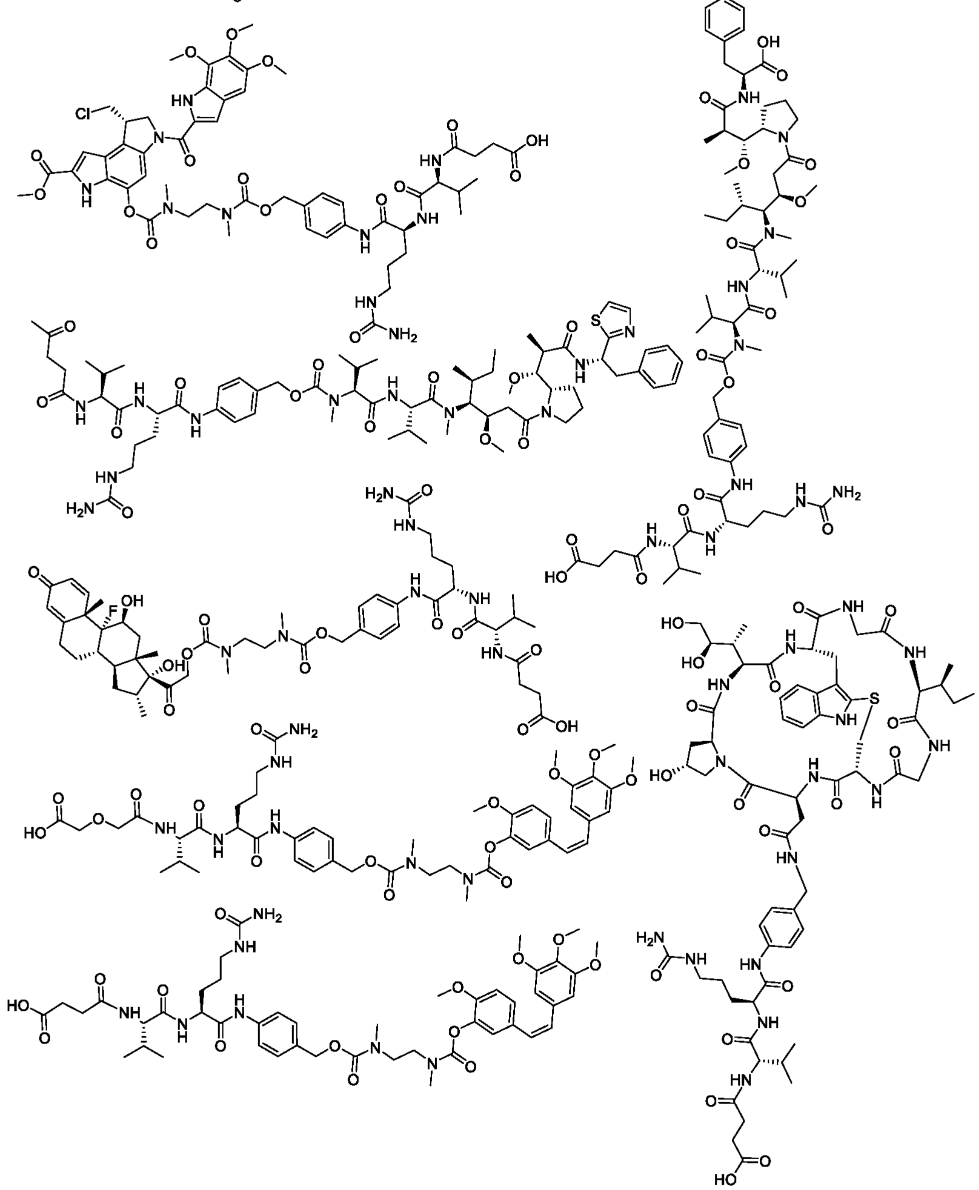

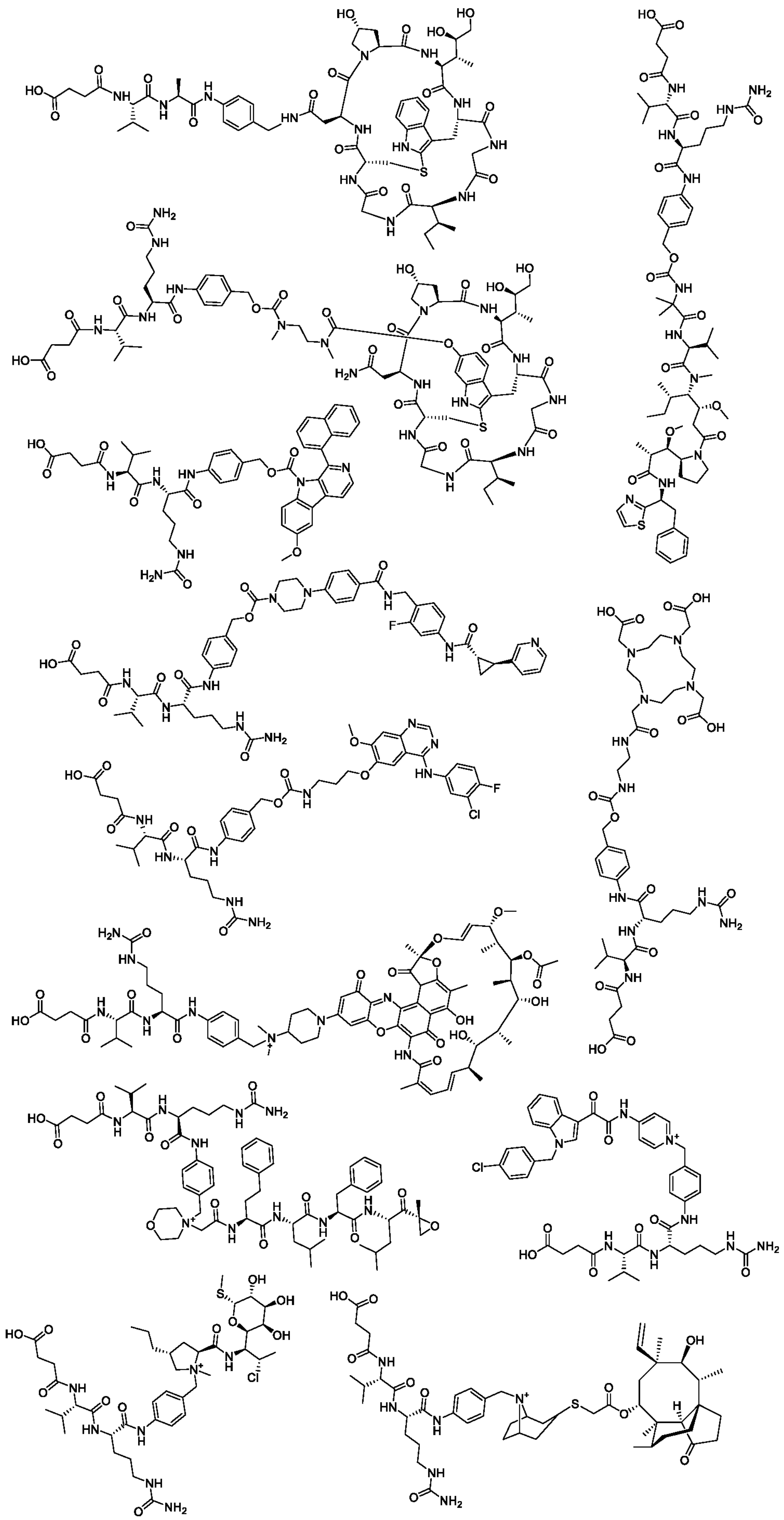

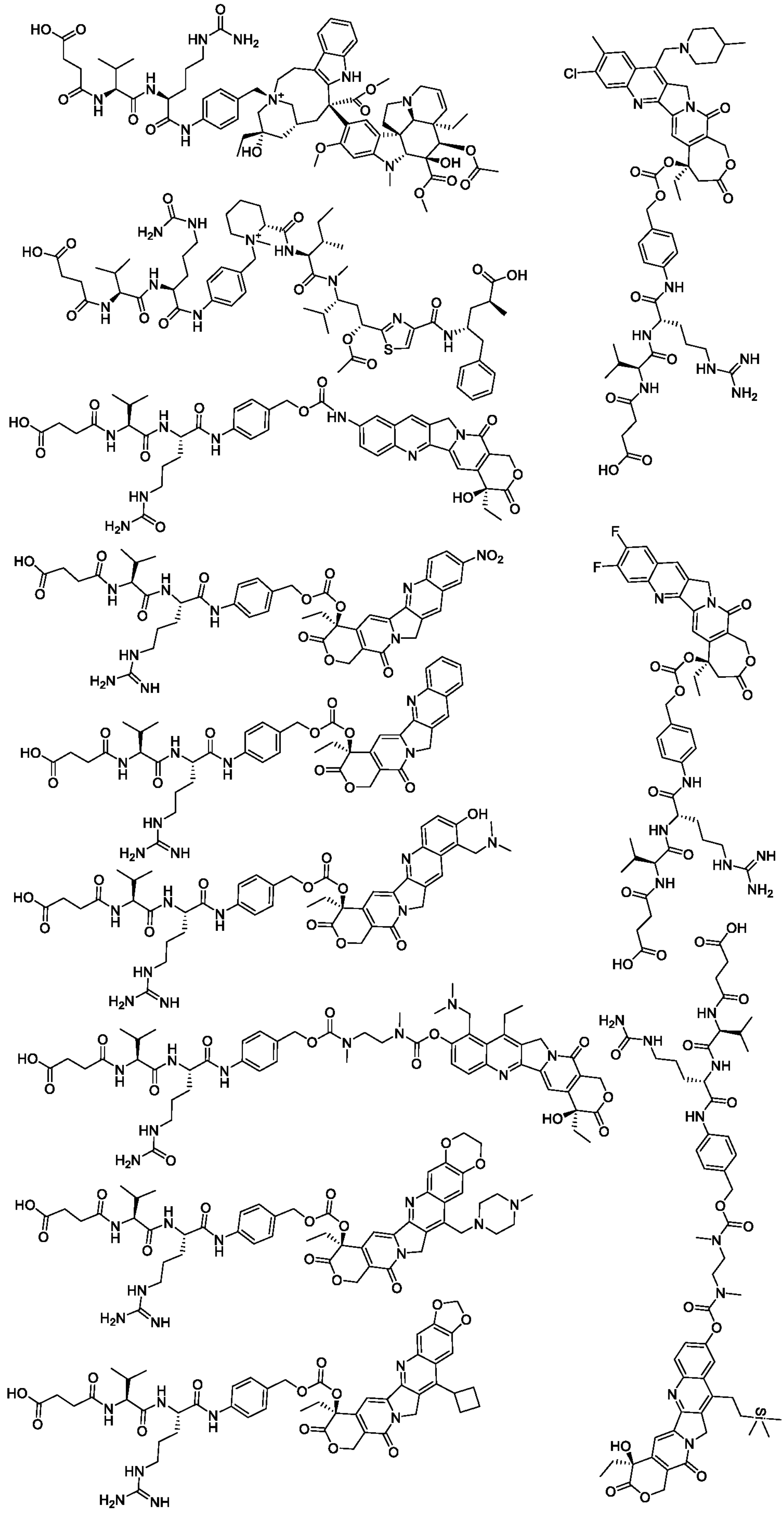

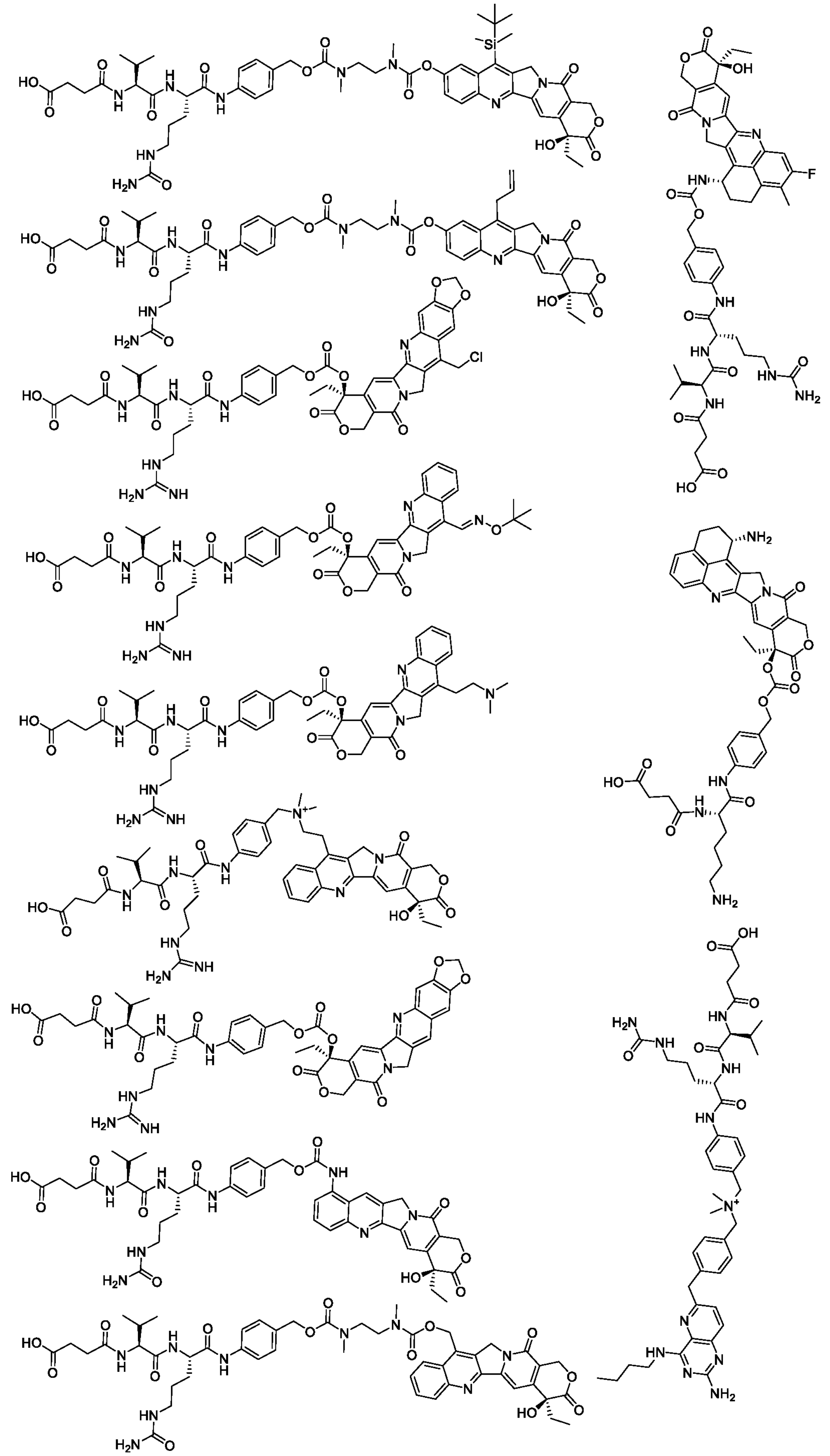

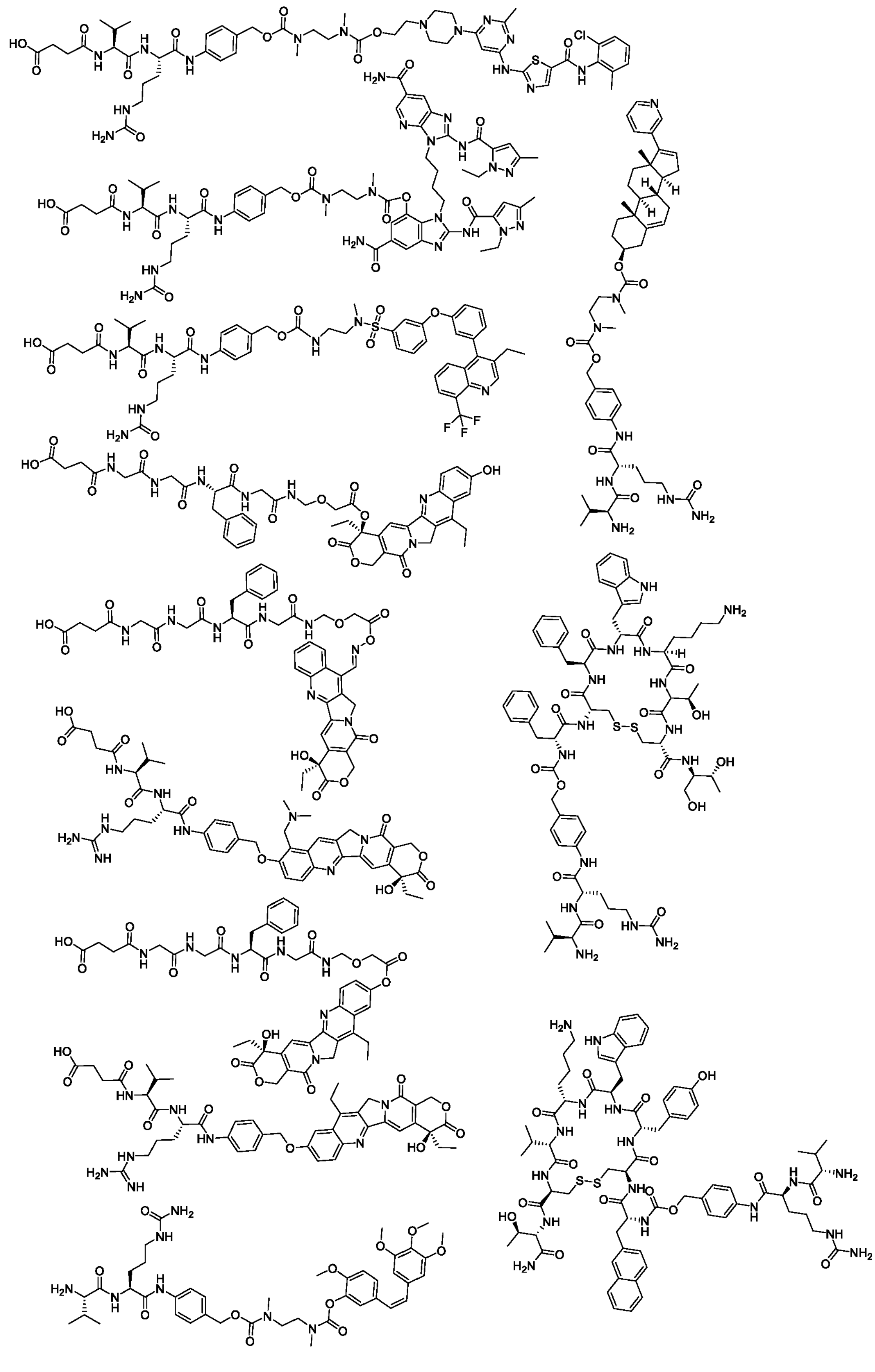

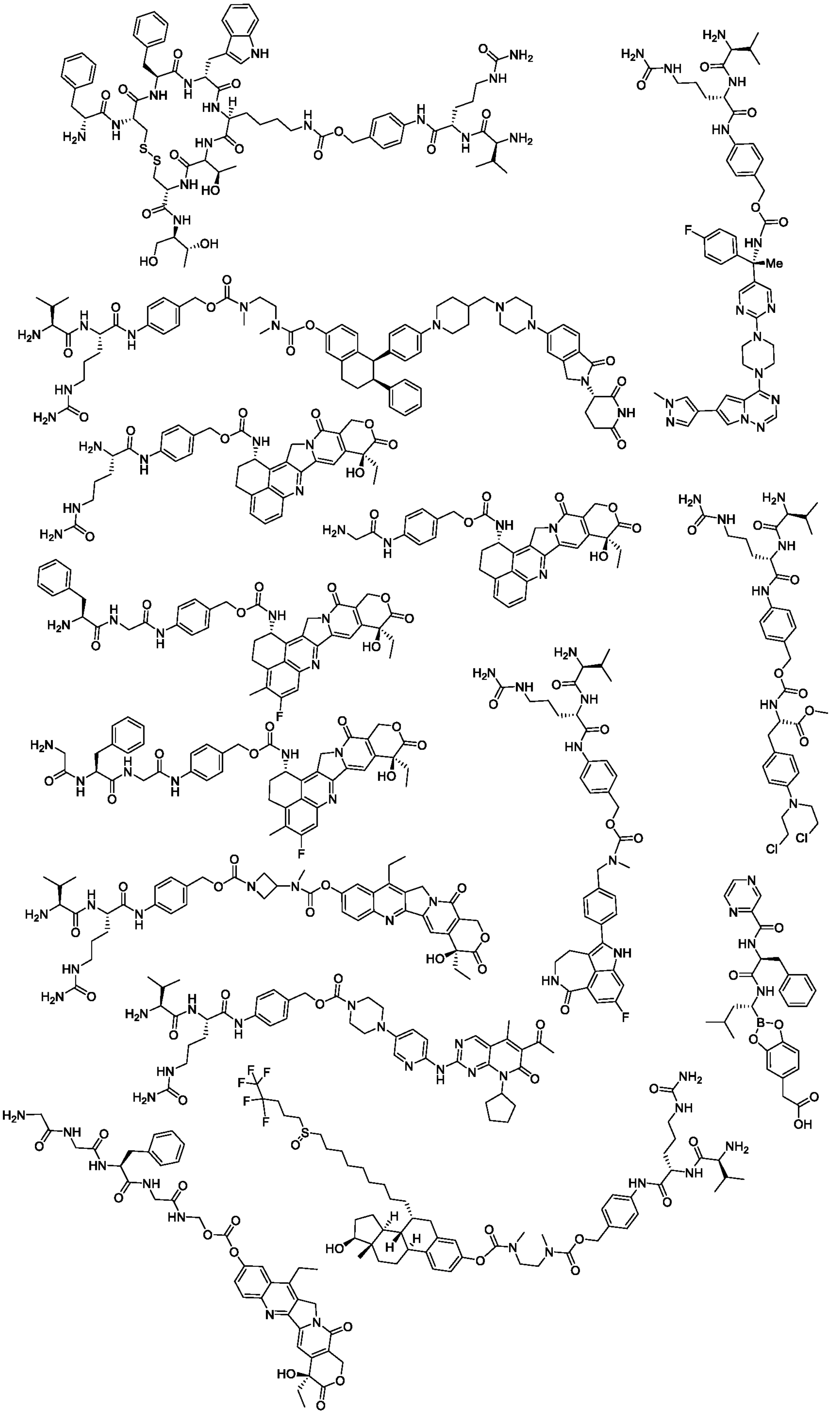

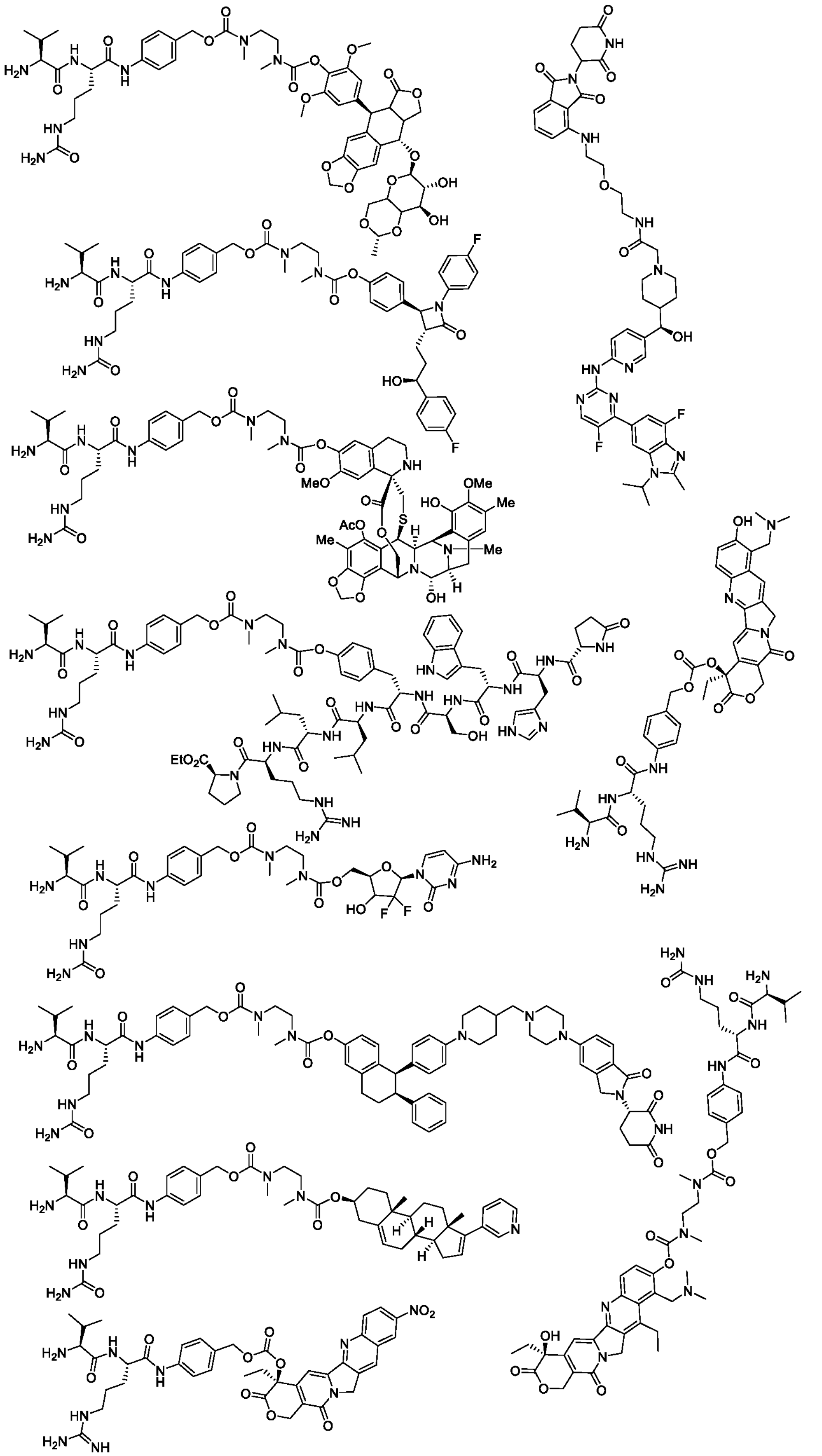

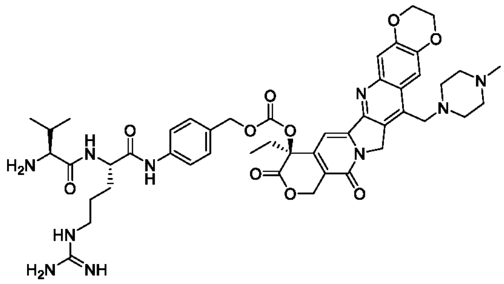

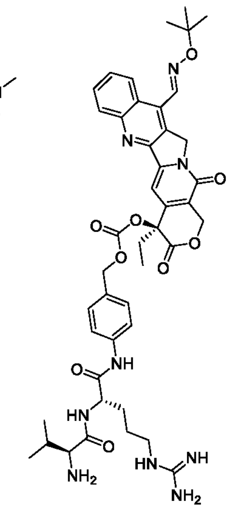

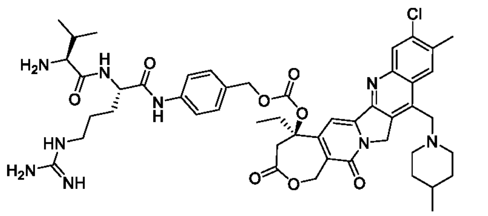

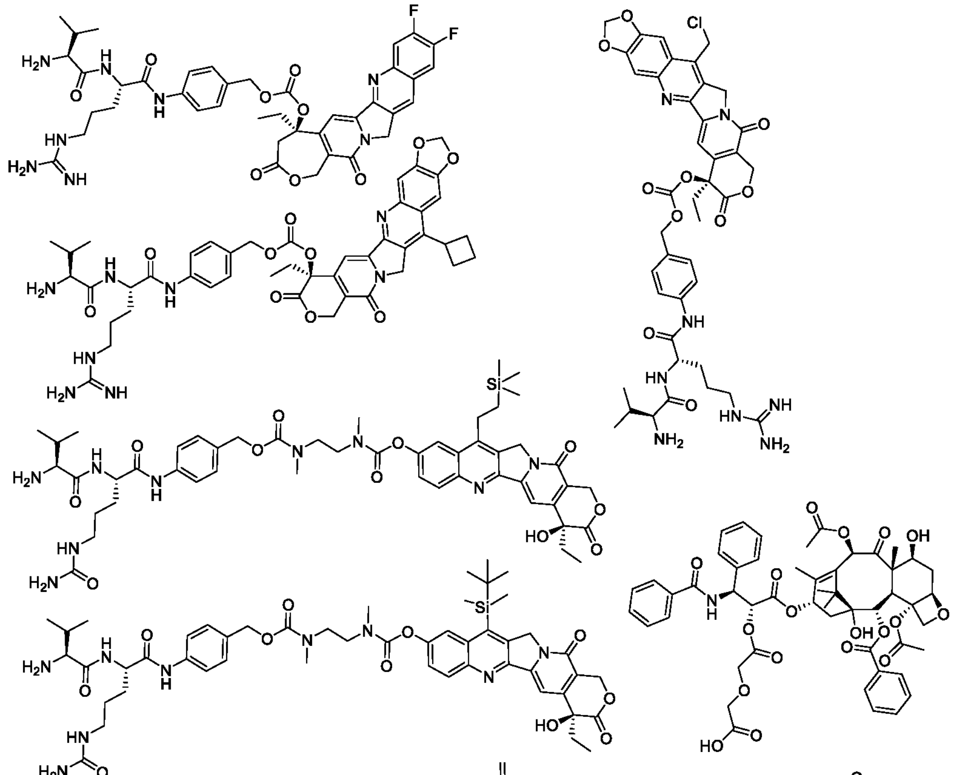

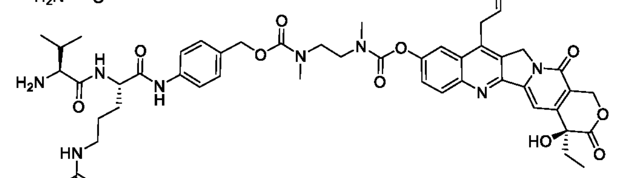

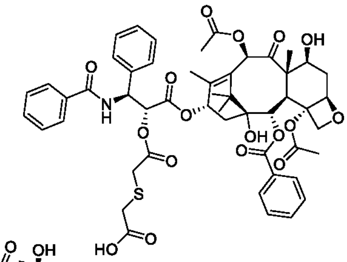

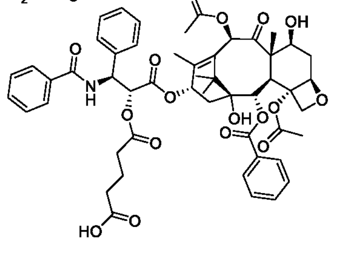

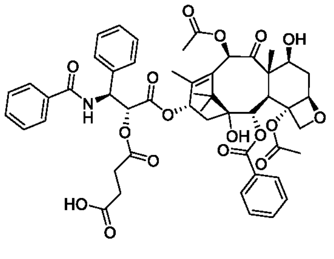

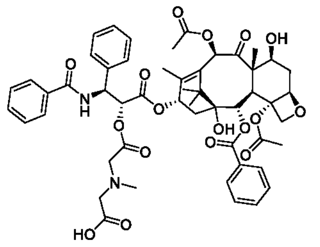
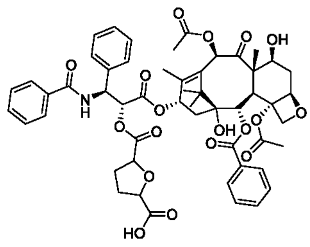
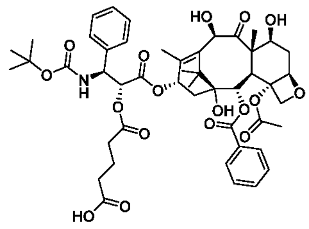
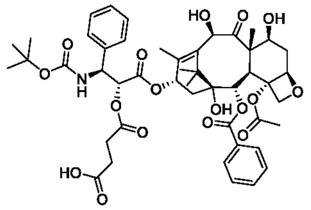
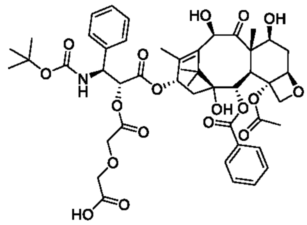
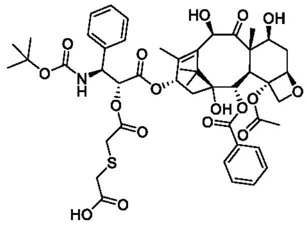
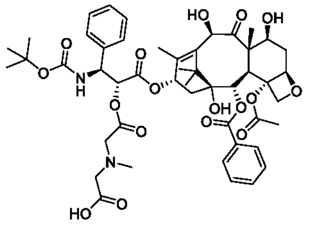
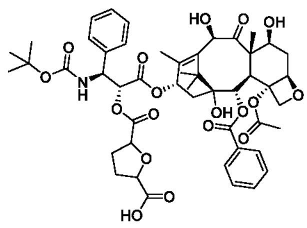
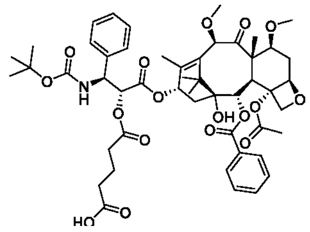

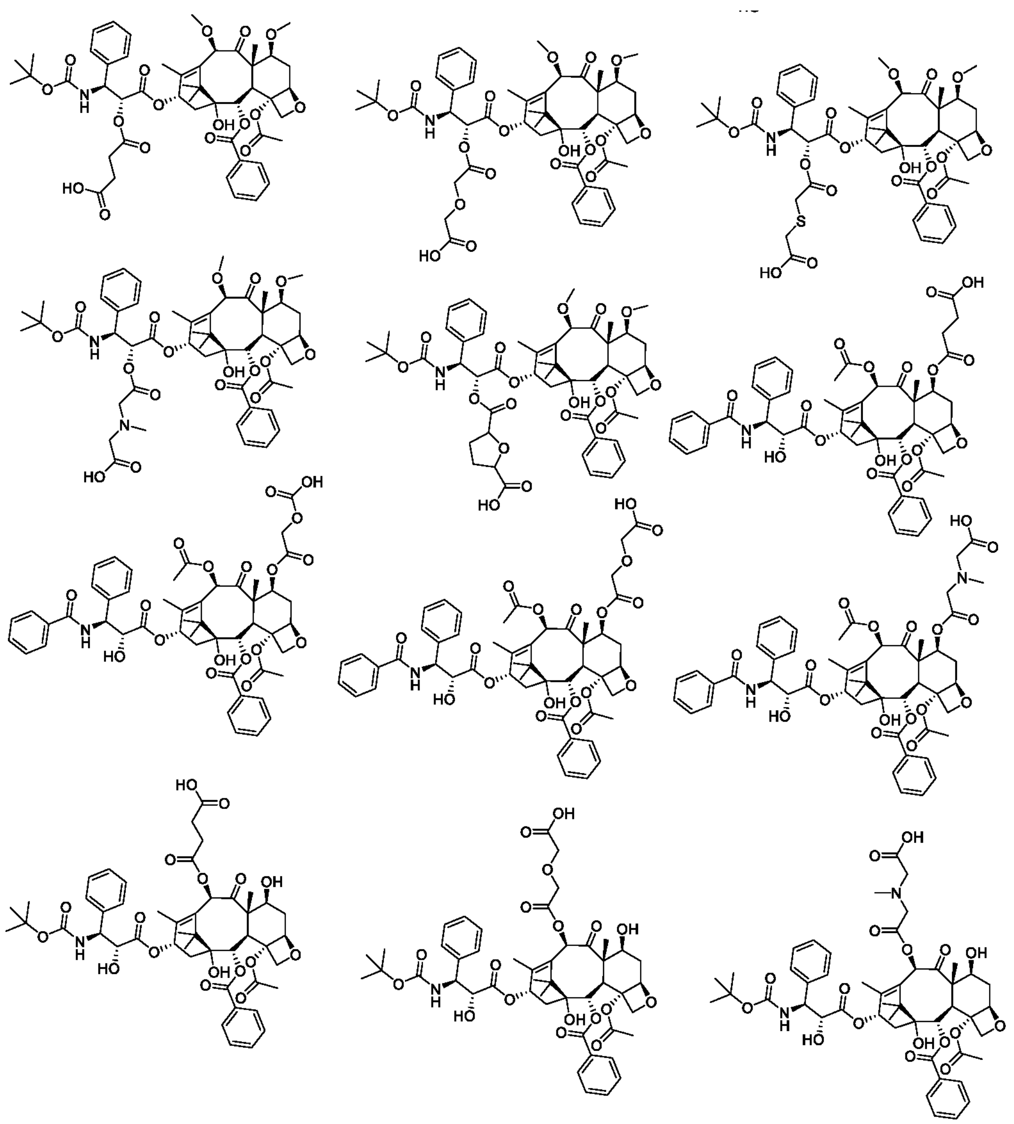

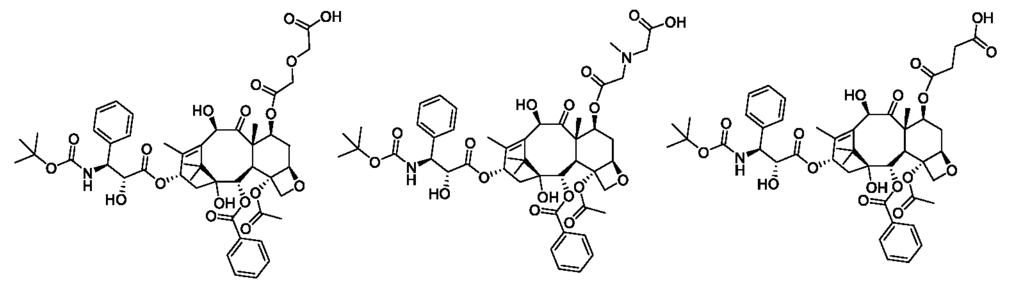

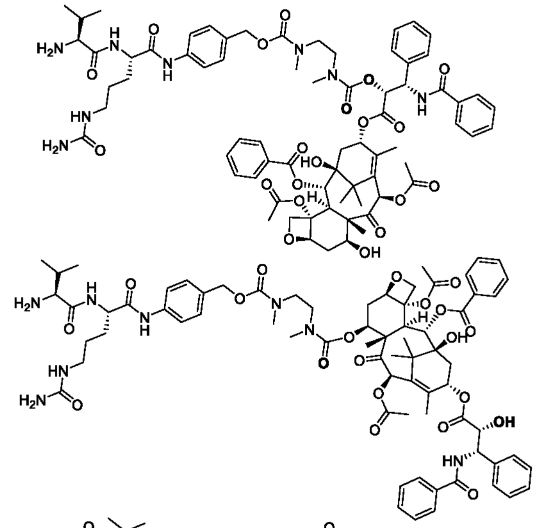

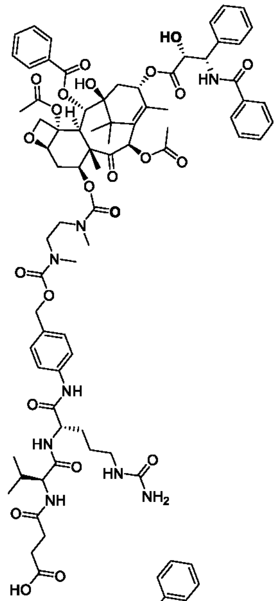

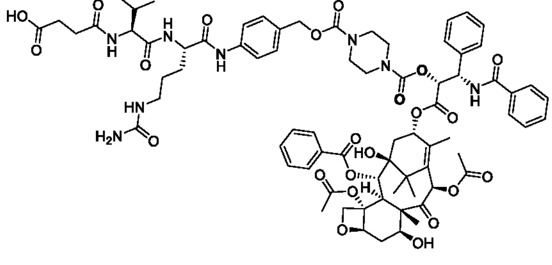

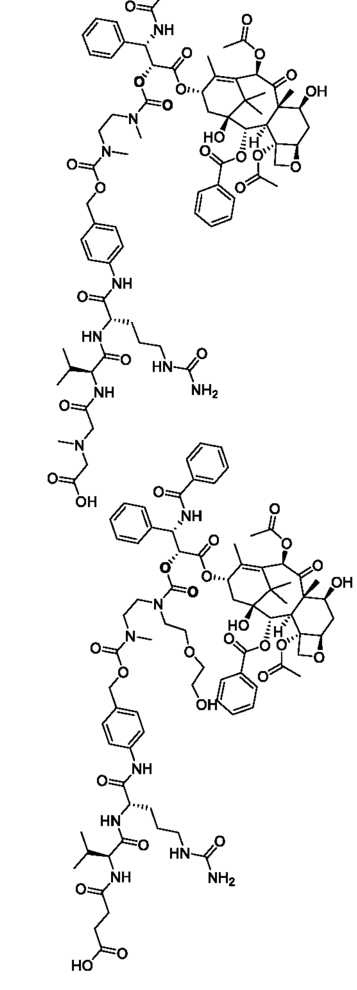

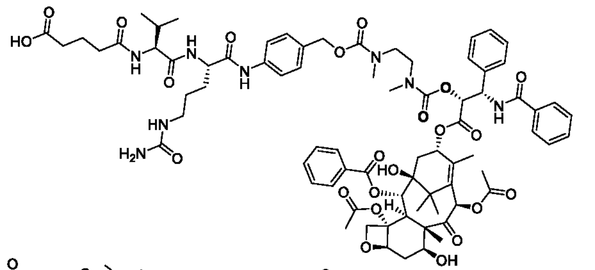

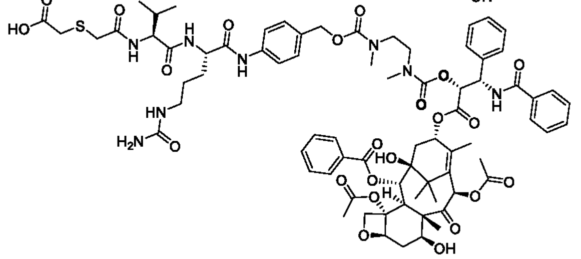

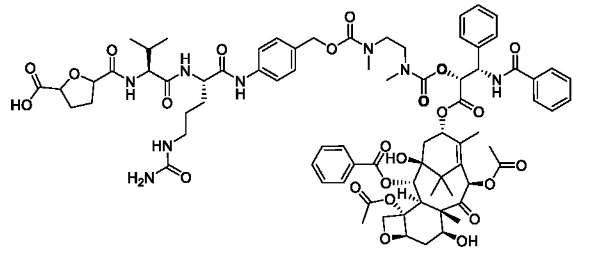

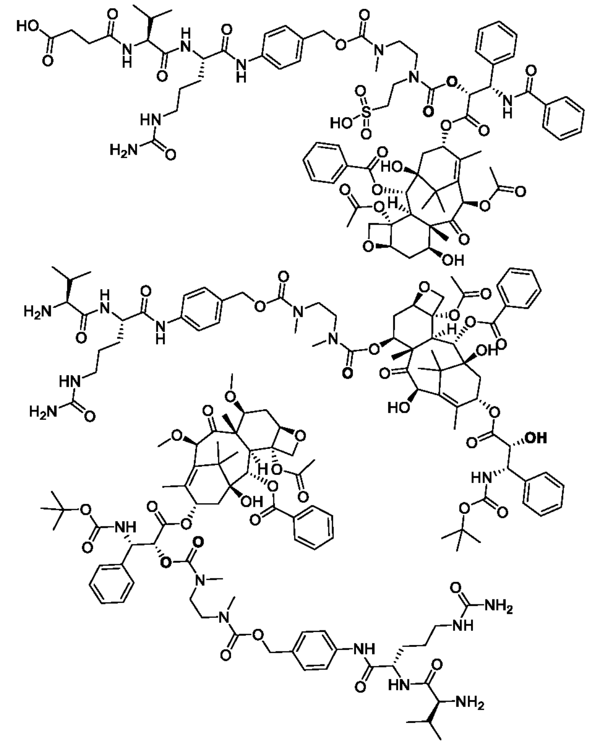

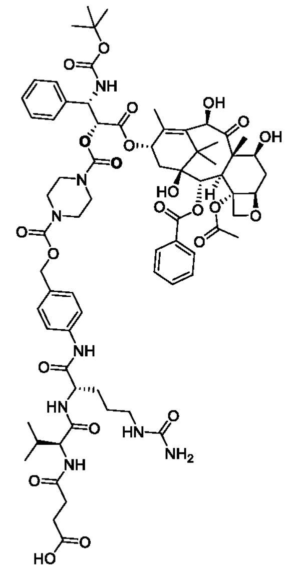

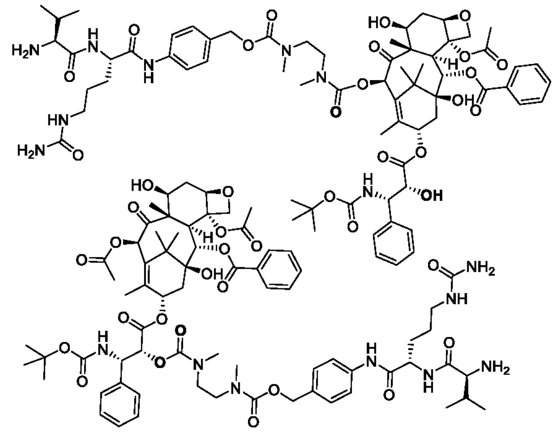

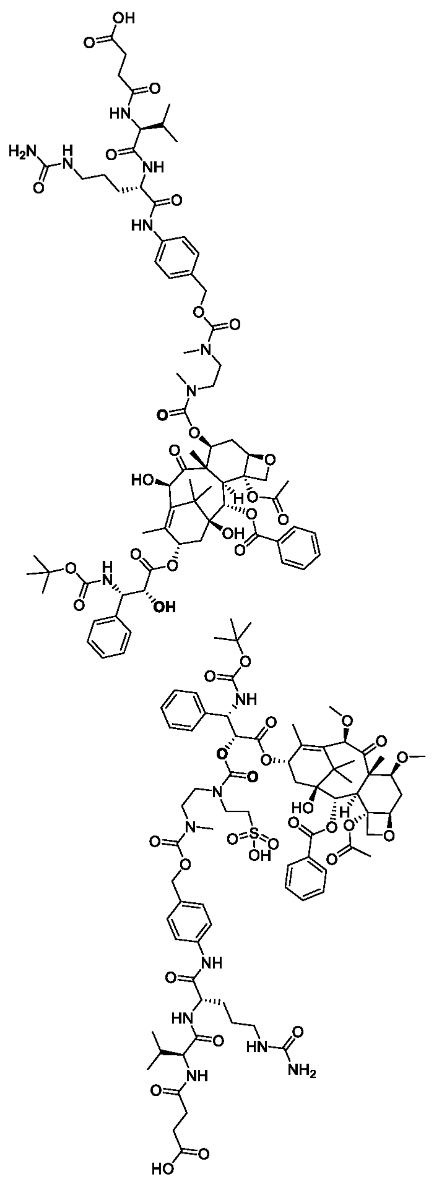

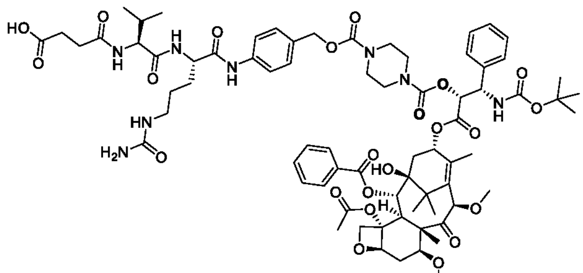

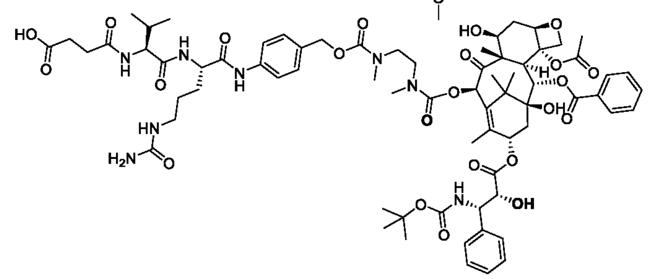

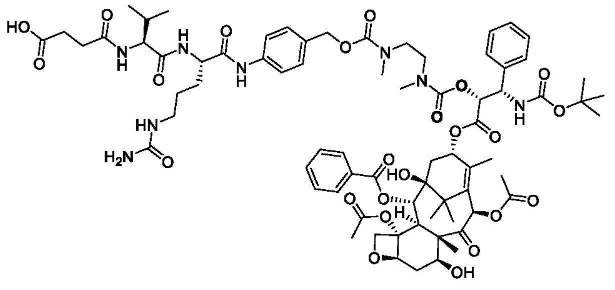

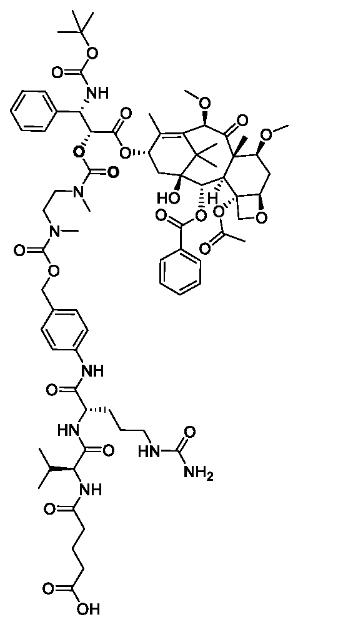

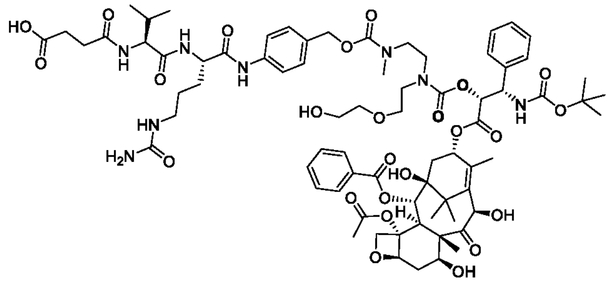

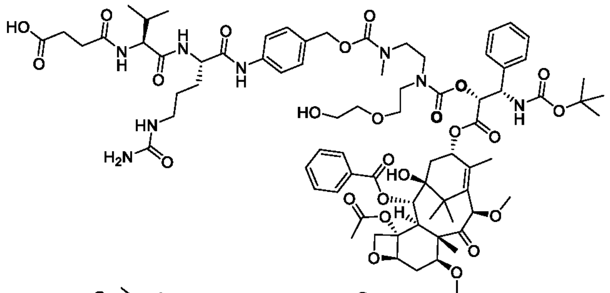

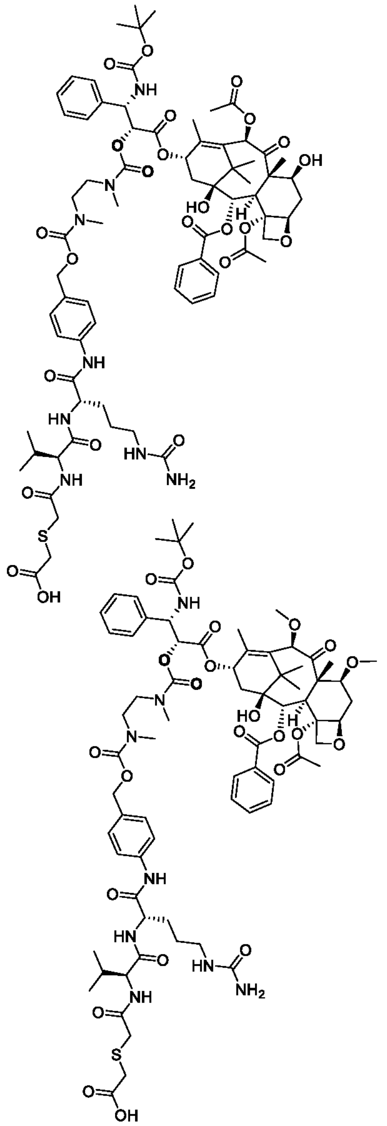

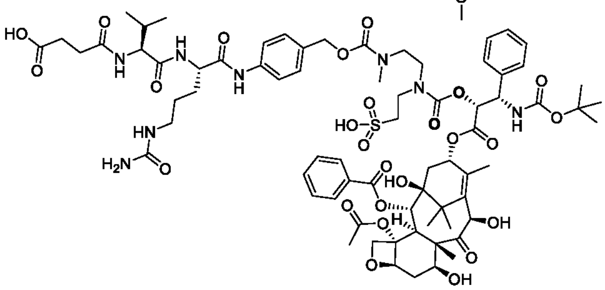

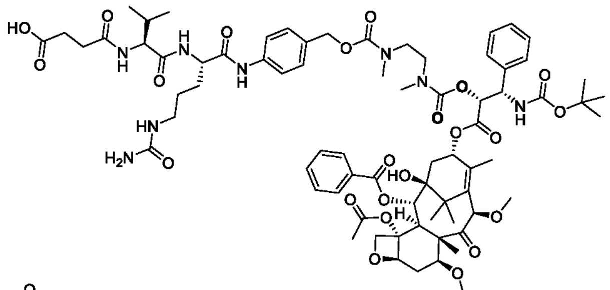

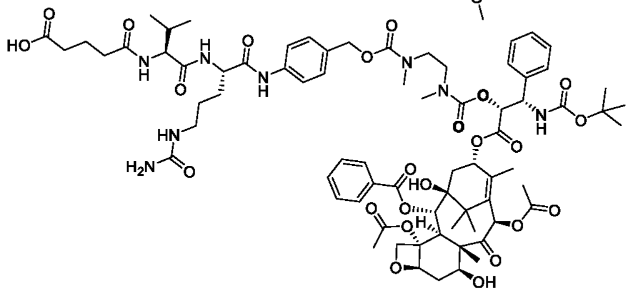

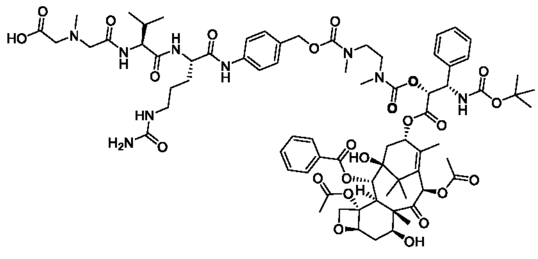

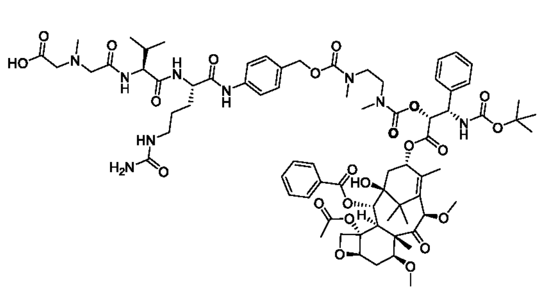

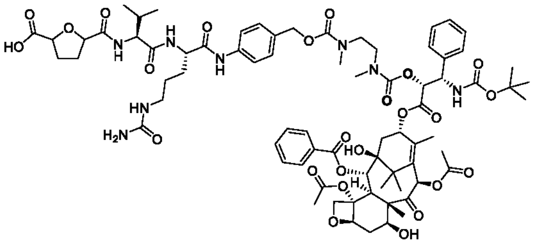

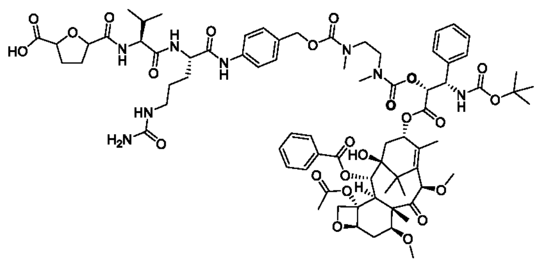

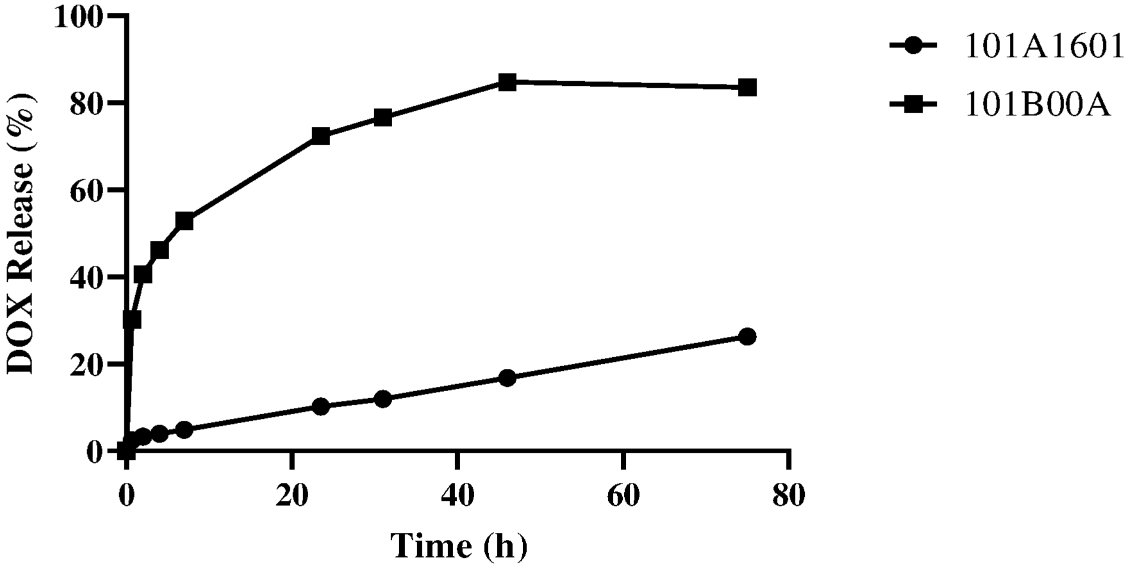


Figure 1

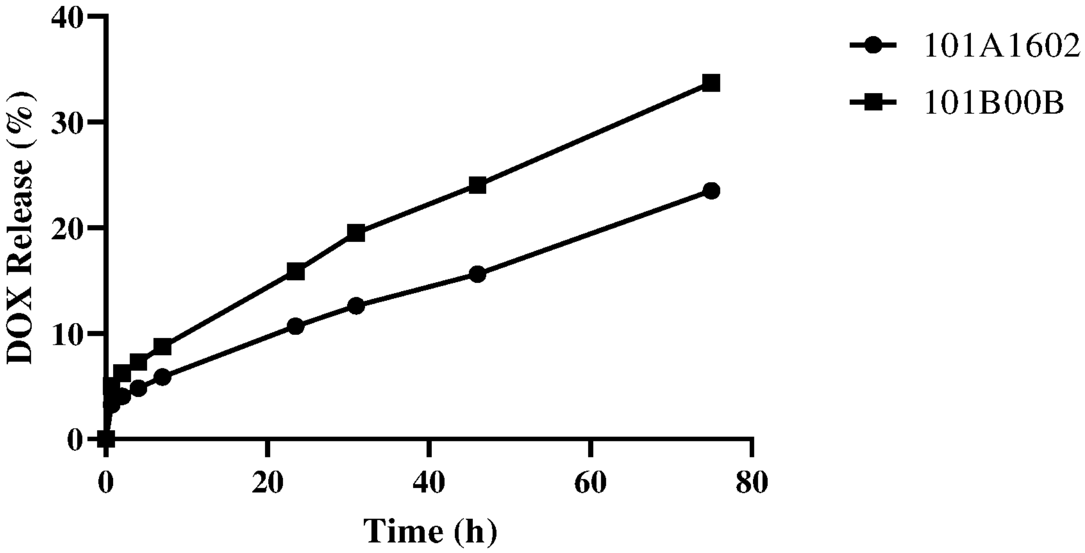


Figure 2

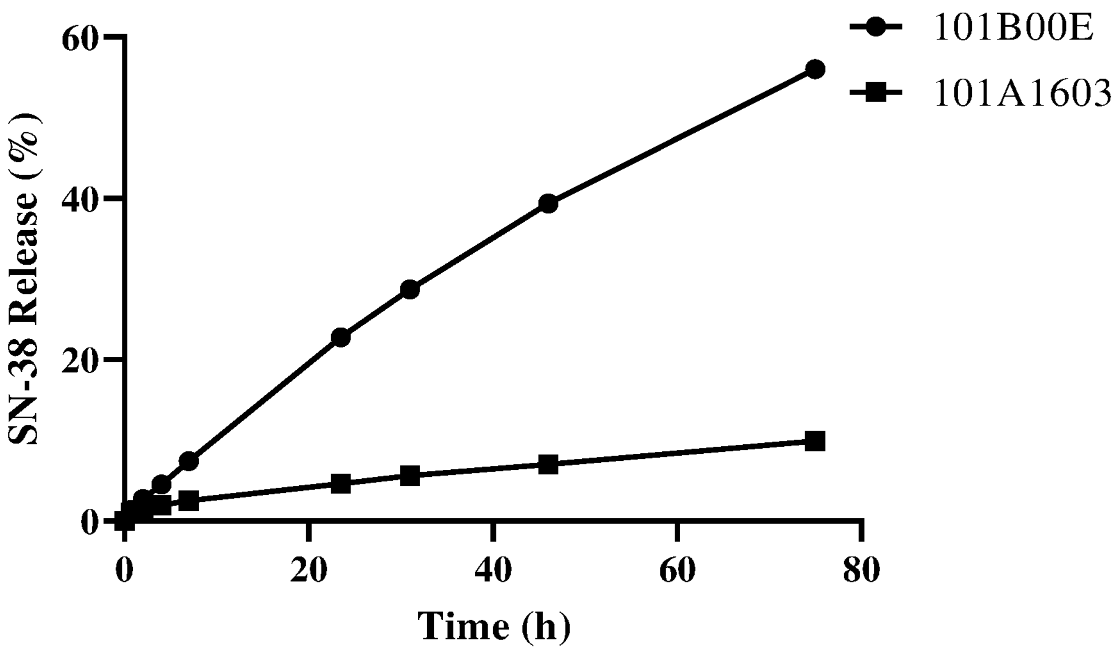


Figure 3

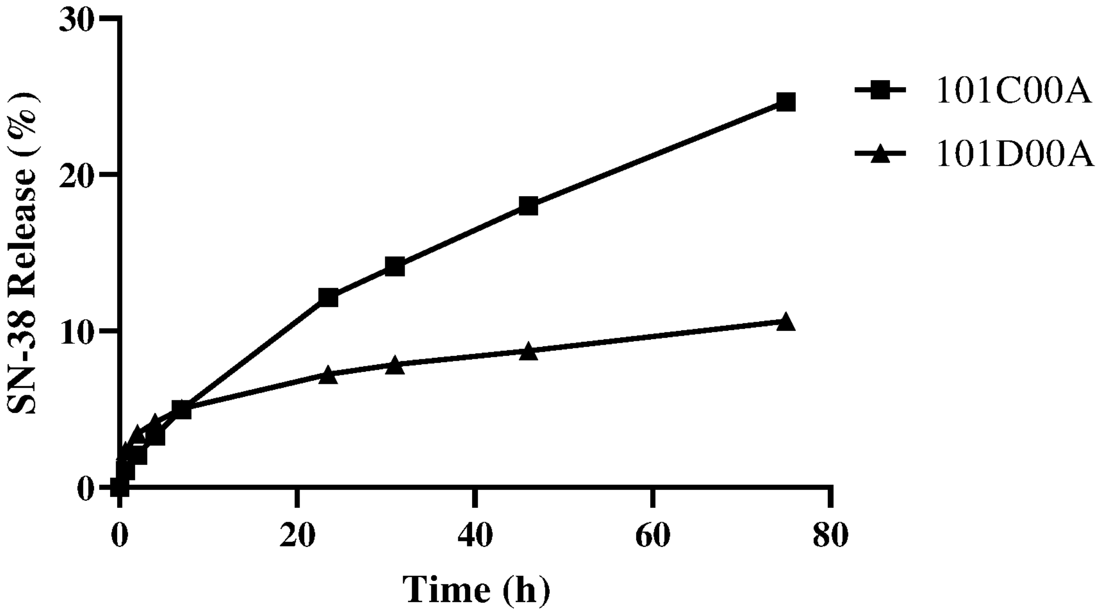


Figure 4

Irinotecan (SN-38 equivalent)

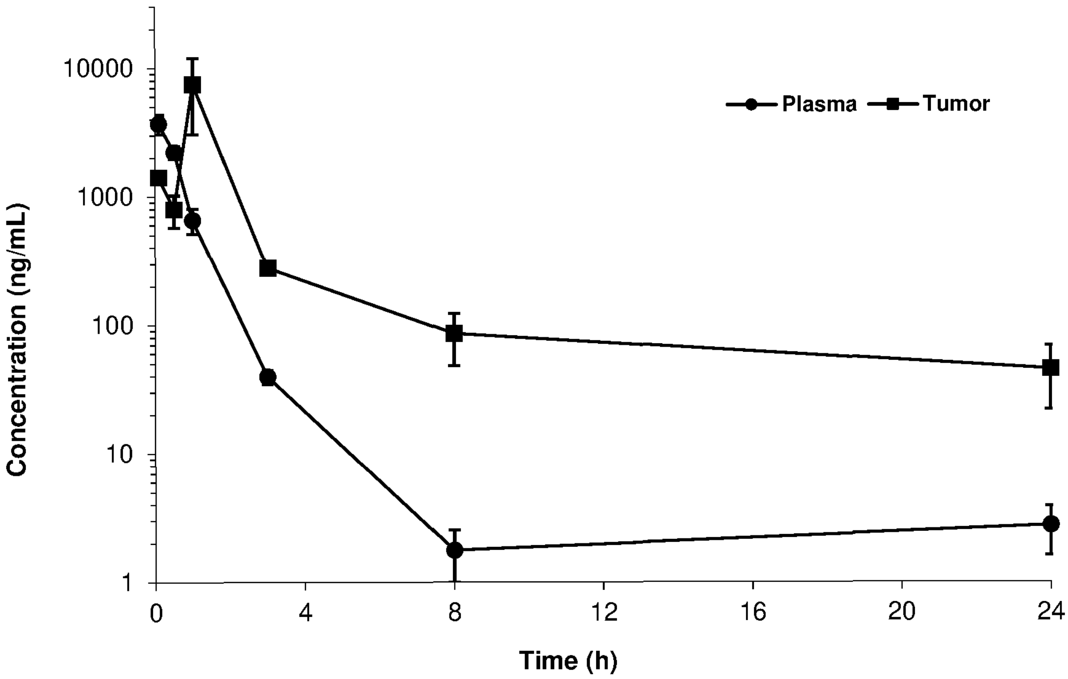


Figure 5

101B00E (SN-38 equivalent)

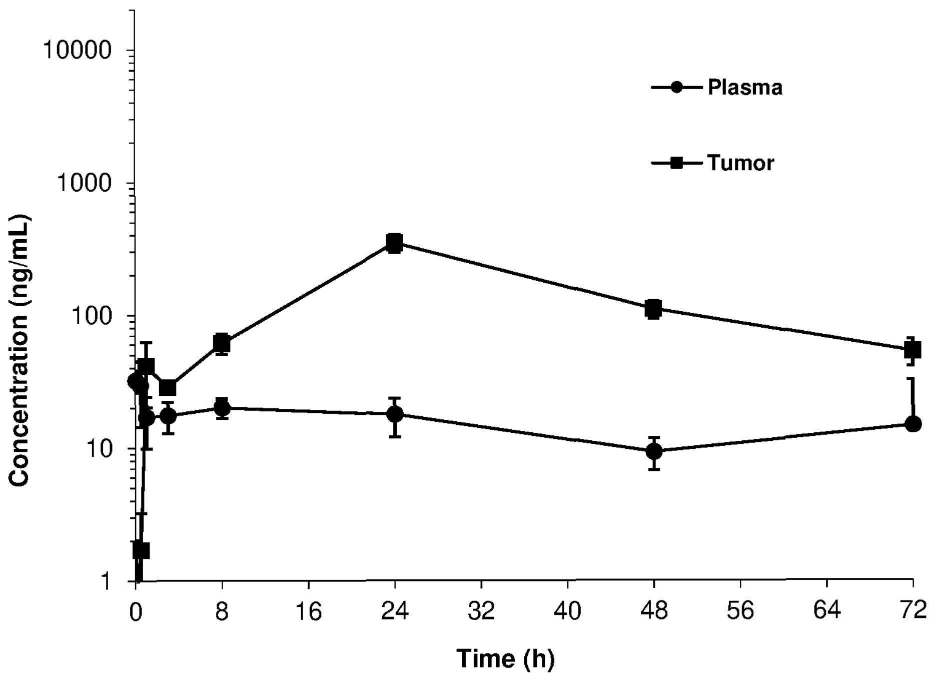


Figure 6

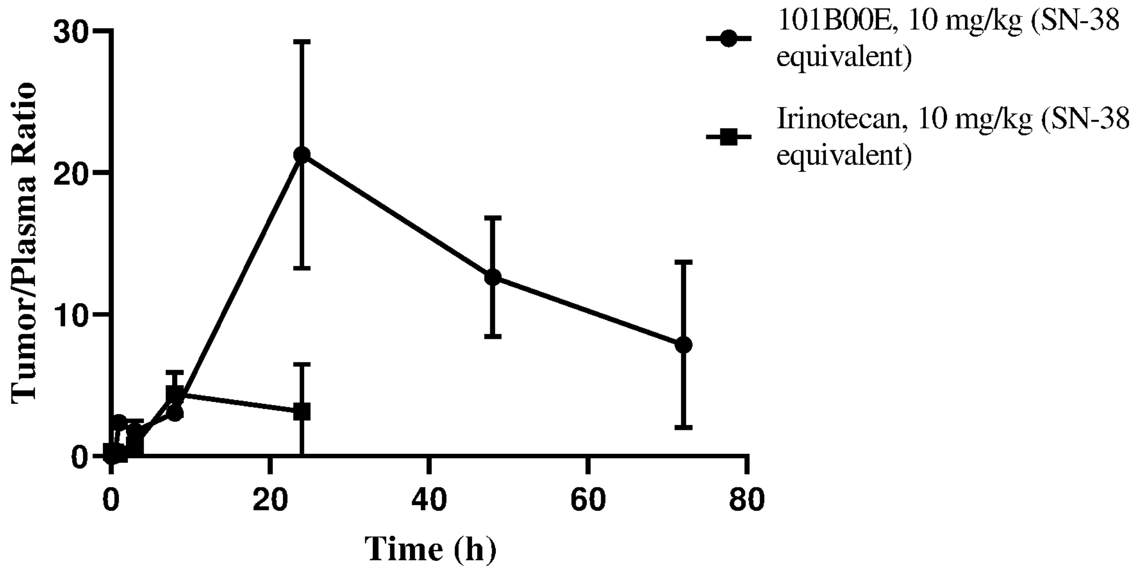


Figure 7

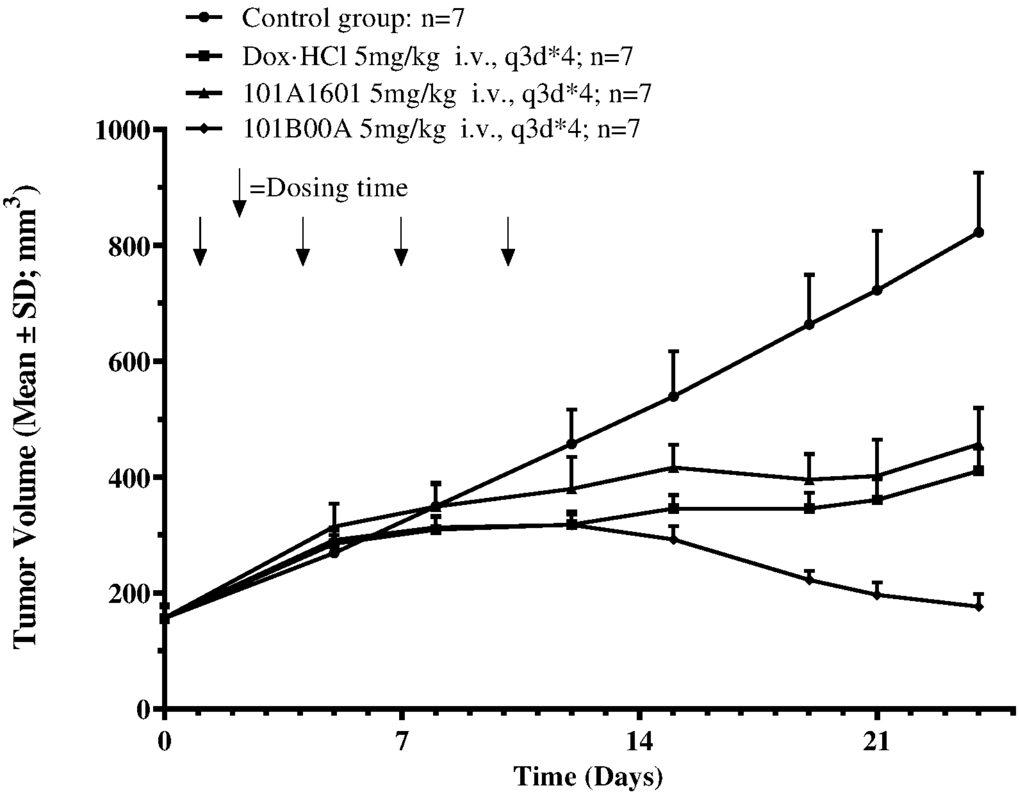


Figure 8

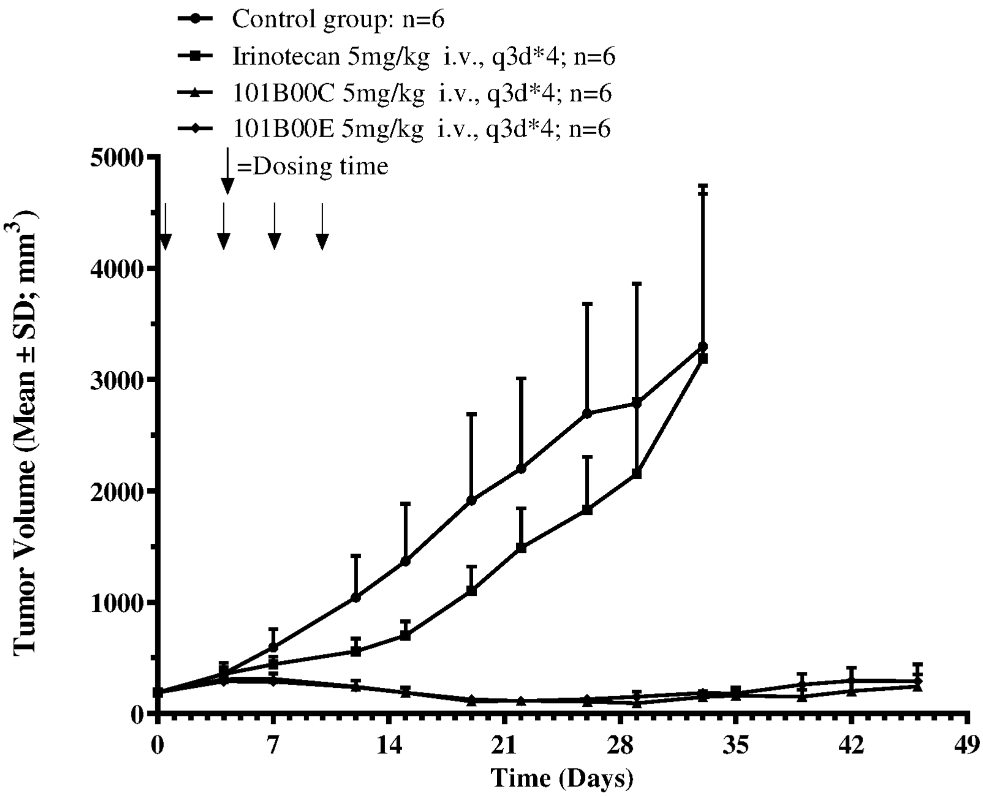


Figure 9

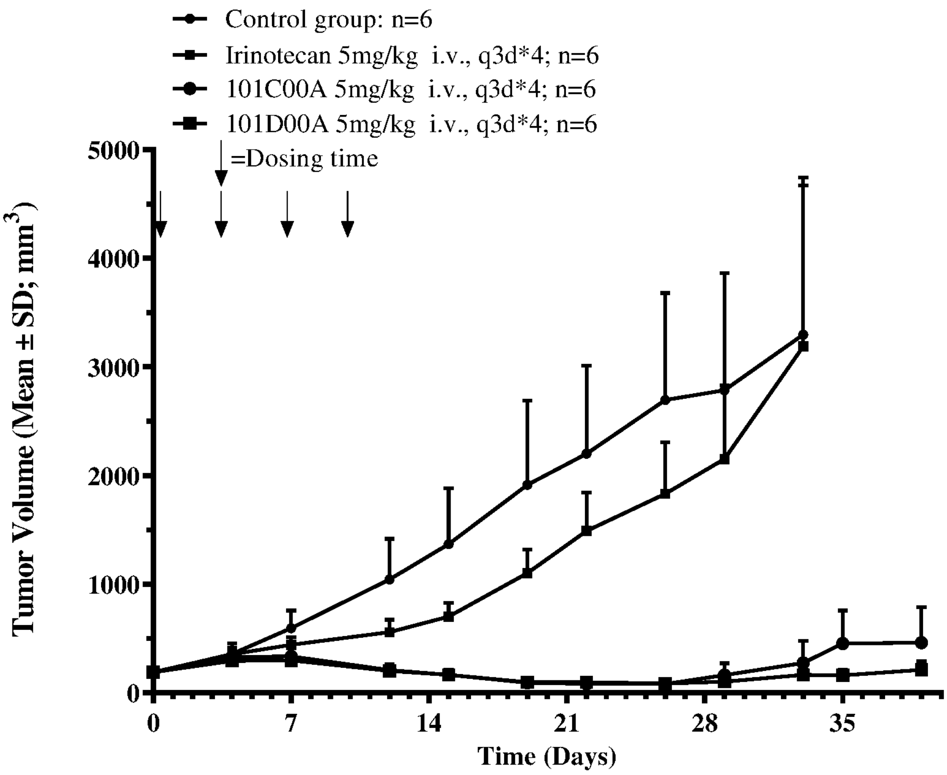


Figure 10

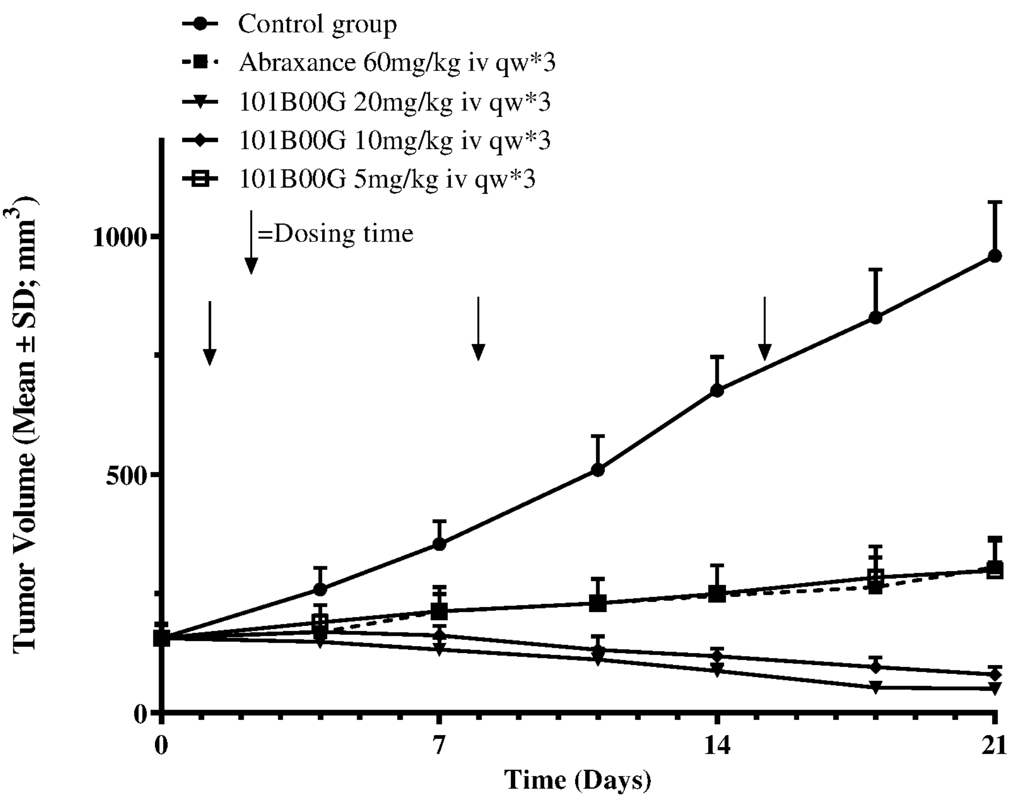


Figure 11

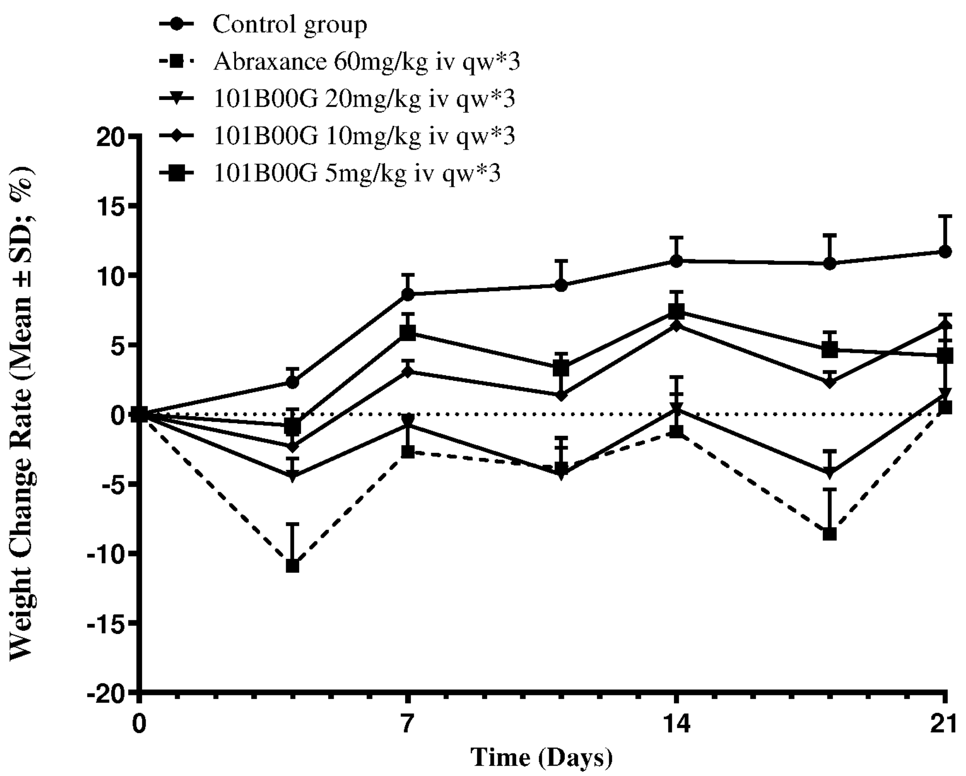


Figure 12

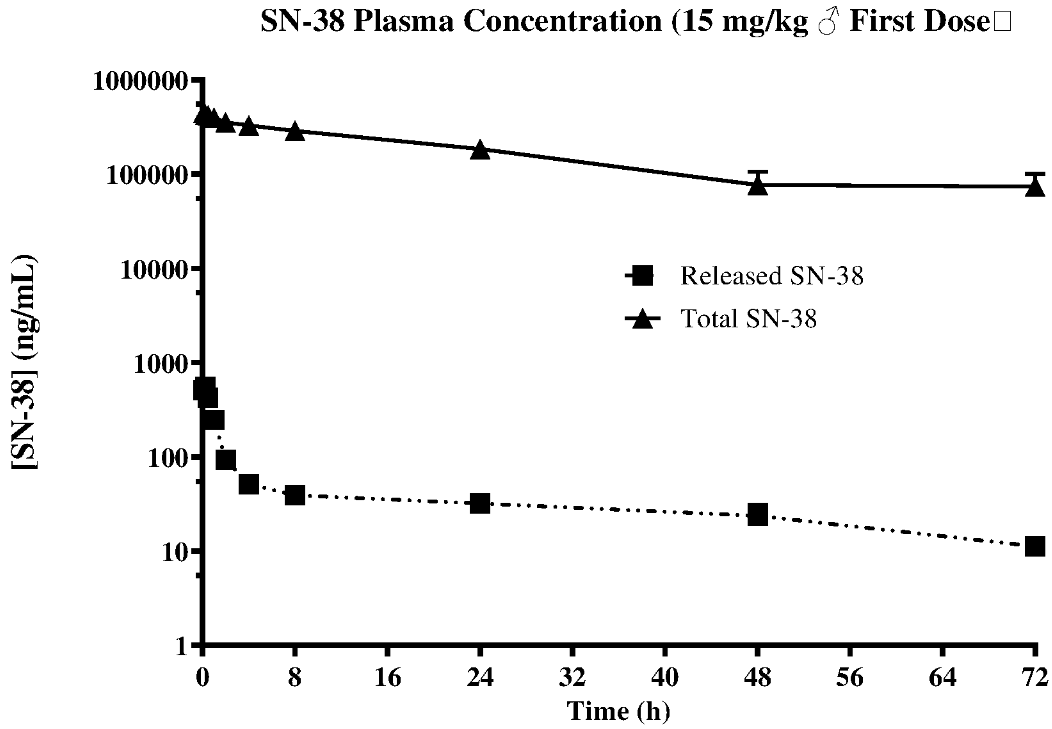


Figure 13

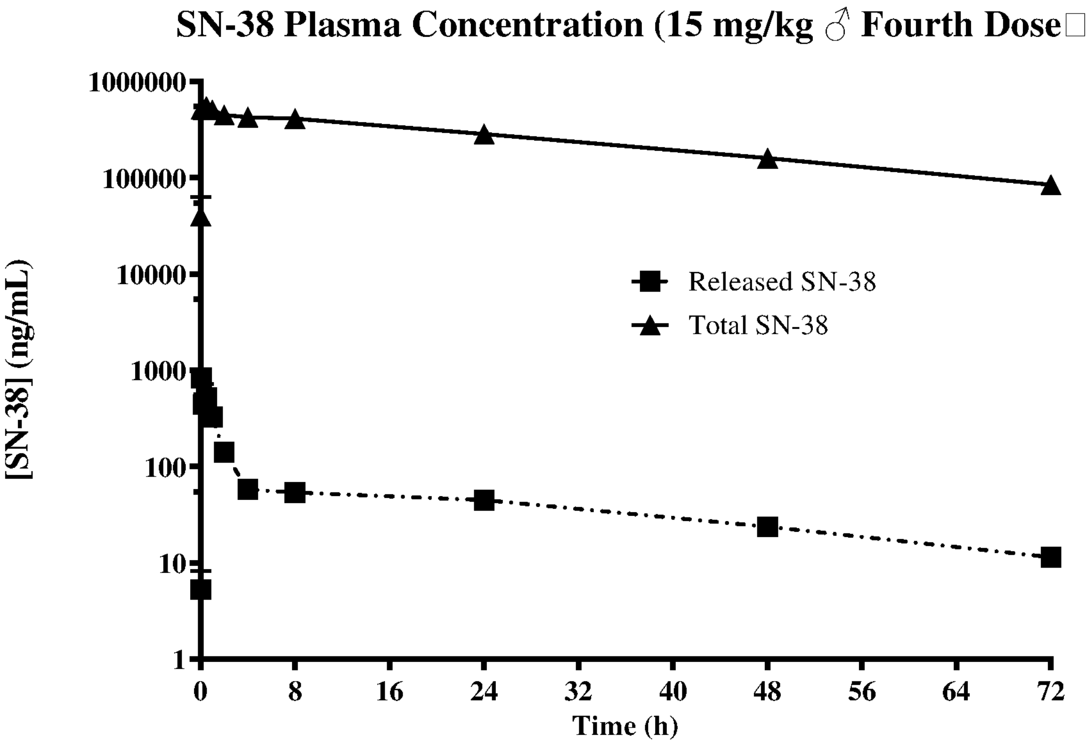


Figure 14

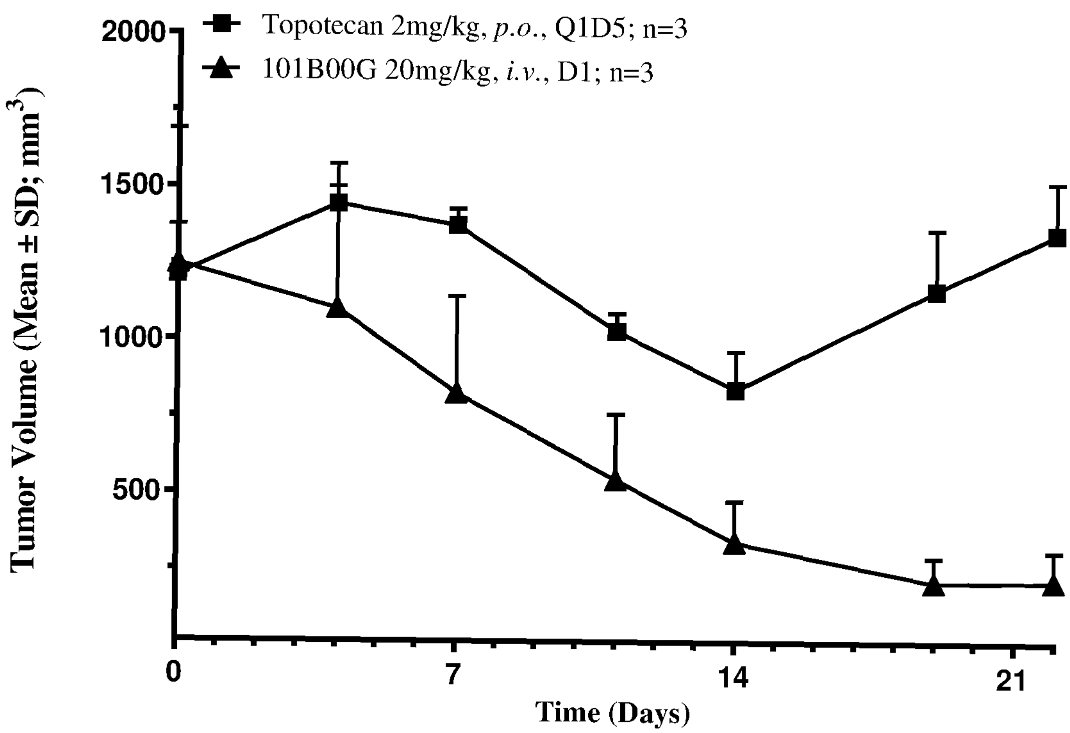


Figure 15

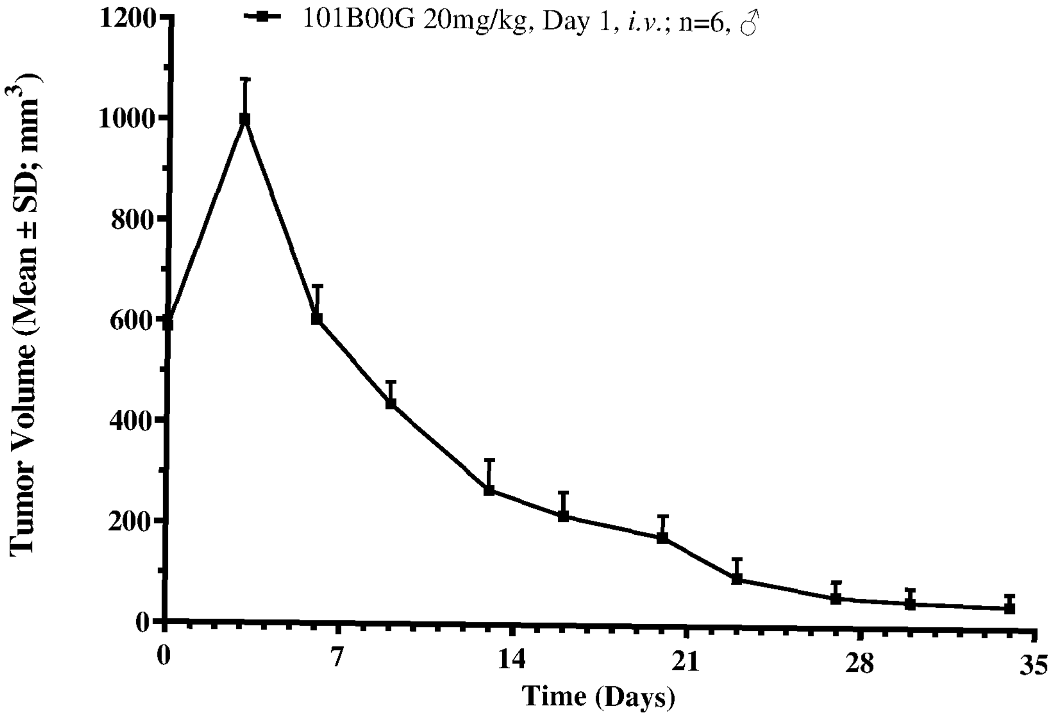


Figure 16

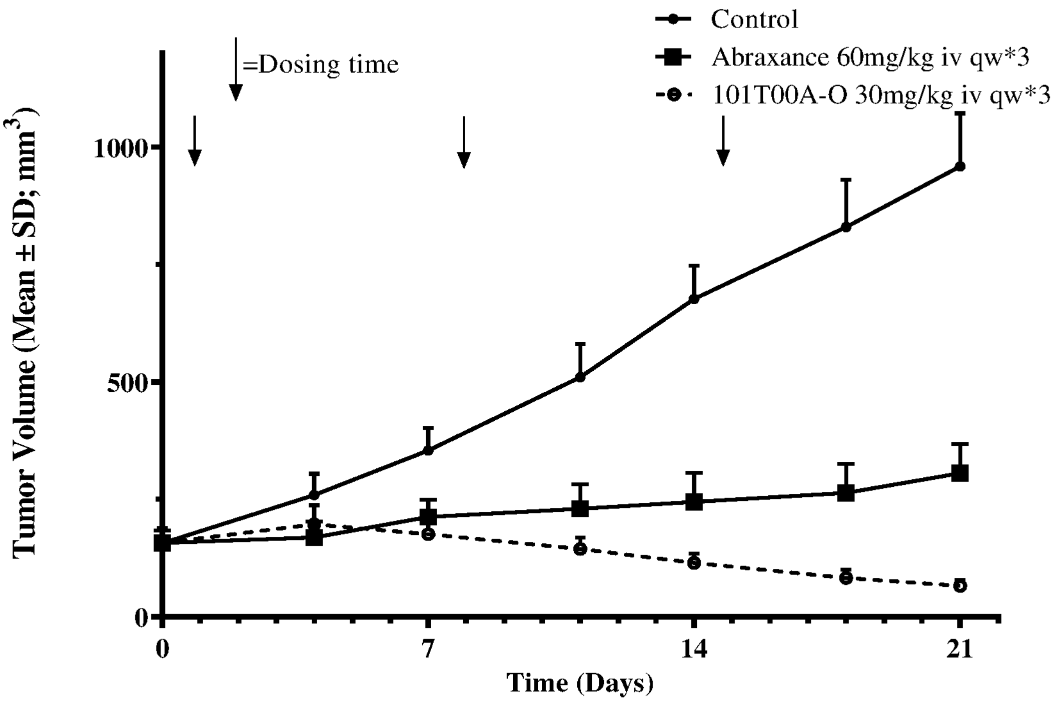

Control
Abraxance 60mg/kg iv qw*3
101T00A-O 30mg/kg iv qw*3
=Dosing time
Tumor Volume (Mean ± SD; mm3)
1000
500
0
0
7
14
21
Time (Days)


Figure 17

**INTERNATIONAL SEARCH REPORT**

International application No. **PCT/CN2022/130704**

**A. CLASSIFICATION OF SUBJECT MATTER**

A61K 47/64(2017.01)i; A61K 47/65(2017.01)i; A61K 47/54(2017.01)i; A61K 45/00(2006.01)i; A61P 35/00(2006.01)i; A61P 35/02(2006.01)i; C08G 69/48(2006.01)i

According to International Patent Classification (IPC) or to both national classification and IPC

**B. FIELDS SEARCHED**

Minimum documentation searched (classification system followed by classification symbols)

A61K A61P C08G

Documentation searched other than minimum documentation to the extent that such documents are included in the fields searched

Electronic data base consulted during the international search (name of data base and, where practicable, search terms used)

CNTXT; ENTXTC; VCN; CJFD; DWPI; CNKI; WFNPL; NPTXT; NPABS; Web of Science; STNext: 上海弼领生物, 张富尧, 赖氨酸, 线性, 聚乙二醇, PEG, Lysine, Linear, 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| X | CN 110520161 A (UNIVERSITY OF ILLINOIS) 29 November 2019 (2019-11-29) description, embodiment 5 | 43, 44 |
| A | Dharmini Mehta et al. "Reducing Dendrimer Generation and PEG Chain Length Increases Drug Release and Promotes Anticancer Activity of PEGylated Polylysine Dendrimers Conjugated with Doxorubicin via a Cathepsin-Cleavable Peptide Linker" *Molecular Pharmaceutics,* Vol. 15, 14 August 2018 (2018-08-14), pages 4568-4576 | 1-39 |
| A | Lisa M. Kaminskas et al. "The Impact of Molecular Weight and PEG Chain Length on the Systemic Pharmacokinetics of PEGylated Poly L-Lysine Dendrimers" *MOLECULAR PHARMACEUTICS,* Vol. 5, No. 3, 04 May 2008 (2008-05-04), pages 449-463 | 40-42 |

☐ Further documents are listed in the continuation of Box C. ☑ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| **06 January 2023** | **17 January 2023** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2022/130704**

| Patent document cited in search report | | | Publication date (day/month/year) | Patent family member(s) | | | Publication date (day/month/year) |
|---|---|---|---|---|---|---|---|
| CN | 110520161 | A | 29 November 2019 | WO | 2018148650 | A1 | 16 August 2018 |
| | | | | US | 2019367550 | A1 | 05 December 2019 |
| | | | | JP | 2020507584 | A | 12 March 2020 |
| | | | | KR | 20190126314 | A | 11 November 2019 |
| | | | | EP | 3595729 | A1 | 22 January 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2021113237547
- WO 2019039544 A
- CN 109265680 A
- CN 108794452 B
- WO 2016151432 A1
- WO 2015038649 A1
- WO 2017180834 A1


### Non-patent literature cited in the description


- *British Journal of Cancer,* 2011, vol. 104, 593
- *Investigational New Drugs,* 2017, vol. 35, 307
- *Biomaterials,* 2015, vol. 53, 370
- *Polymers,* 2014, vol. 6, 2186
- *Lancet Oncology,* 2015, vol. 16, 1556
- *Mol Cancer Ther,* 2018, vol. 17, 196
- *Journal of Medicinal Chemistry,* 2014, vol. 57, 2303
- *British Journal of Cancer,* 2008, vol. 98, 1608
- *Int J Oncol,* 2010, vol. 37, 563
- *Anticancer Research,* 2016, vol. 36, 6499
- *Journal of Clinical Oncology,* 2016, vol. 34 (15), 2526
- *Mol Pharm,* 2018, vol. 15, 4568
- *Mol Pharm,* 2015, vol. 12, 4226
- *Adv Mater,* 2016, vol. 28, 8162
- *Mol Pharm,* 2008, vol. 5, 449
- **T. W. GREENE ; P. G. M. WUTS.** Protective Groups in Organic Synthesis
- *Biochem.,* 1972, vol. 11, 942-944
- *Can. J. Chem.,* 1993, vol. 71 (6), 896
- *European Journal of Medicinal Chemistry,* 2021, vol. 216
- *CHEMICAL ABSTRACTS,* 644981-35-1
- *CHEMICAL ABSTRACTS,* 863971-53-3
- *J. Med. Chem.,* 2000, vol. 43, 3093-3102
- *ChemPlusChem,* 2013, vol. 78, 222-226
- *Molecules,* 2011, vol. 16, 6769-6777